(19) Europäisches Patentamt
European Patent Office
Office européen des brevets

(11) EP 4 755 399 A1

(12) EUROPEAN PATENT APPLICATION
published in accordance with Art. 153(4) EPC

(43) Date of publication:
**10.06.2026 Bulletin 2026/24**

(21) Application number: **24848183.0**

(22) Date of filing: **26.07.2024**

(51) International Patent Classification (IPC):
***A61K 47/60*** (2017.01) ***A61K 47/64*** (2017.01)
***A61K 45/00*** (2006.01) ***A61P 35/00*** (2006.01)

(52) Cooperative Patent Classification (CPC):
**A61K 45/00; A61K 47/60; A61K 47/64; A61P 35/00**

(86) International application number:
**PCT/CN2024/107866**

(87) International publication number:
**WO 2025/026226 (06.02.2025 Gazette 2025/06)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **28.07.2023 CN 202310959802**

(71) Applicant: **Chongqing Upgra Biotechnology Co., Ltd.**
**Chongqing 401120 (CN)**

(72) Inventors:
- **LI, Gaoquan**
  **Chongqing 401120 (CN)**
- **LIU, Nian**
  **Chongqing 401120 (CN)**
- **PENG, Yongchen**
  **Chongqing 401120 (CN)**
- **GAO, Yang**
  **Chongqing 401120 (CN)**
- **PENG, Yuanyuan**
  **Chongqing 401120 (CN)**
- **CHEN, Huiyu**
  **Chongqing 401120 (CN)**
- **LOU, Jie**
  **Chongqing 401120 (CN)**
- **ZENG, Xiafan**
  **Chongqing 401120 (CN)**
- **YIN, Yifeng**
  **Chongqing 401120 (CN)**
- **XU, Xia**
  **Chongqing 401120 (CN)**
- **YANG, Shuai**
  **Chongqing 401120 (CN)**
- **LIU, Jing**
  **Chongqing 401120 (CN)**
- **WU, Yongqin**
  **Chongqing 401120 (CN)**
- **GUAN, Sheng**
  **Chongqing 401120 (CN)**
- **ZHANG, Xinli**
  **Chongqing 401120 (CN)**
- **ZHANG, Qingmeng**
  **Chongqing 401120 (CN)**
- **SHI, Xichen**
  **Chongqing 401120 (CN)**
- **FANG, Leilei**
  **Chongqing 401120 (CN)**
- **MEI, Gang**
  **Chongqing 401120 (CN)**
- **MAO, Zhishuang**
  **Chongqing 401120 (CN)**
- **LI, Miao**
  **Chongqing 401120 (CN)**

(74) Representative: **Manitz Finsterwald Patent- und Rechtsanwaltspartnerschaft mbB**
**Martin-Greif-Straße 1**
**80336 München (DE)**

# (54) POLYETHYLENE GLYCOL SMALL MOLECULE DRUG, PREPARATION METHOD THEREFOR AND USE THEREOF

(57) The present invention relates to a polyethylene glycol small molecule drug, a preparation method therefor and the use thereof. Specifically, the present invention relates to a compound as shown in formula I or a pharmaceutically acceptable salt thereof, intermediate compounds of formula II and formula V thereof, a method for preparing the compound, and the use thereof in fighting tumors. Biological tests show that the compound has a significant inhibitory effect on tumors such as breast cancer, intestinal cancer, non-small cell lung cancer,



Processed by Luminess, 75001 PARIS (FR)

(Cont. next page)

melanoma, oral squamous cell carcinoma and osteosarcoma.

## Description

The present application is based on and claims priority to the Chinese application with application number 202310959802.4 and application date July 28, 2023, the content of which is hereby incorporated by reference in its entirety.

## Technical Field

The present invention belongs to the field of pharmaceutical technology, specifically to a polyethylene glycol small molecule drug with a fatty polyamine core.

## Background Art

Since 1990, the U.S. FDA has approved 30 drugs in the polyethylene glycol (PEG) drug category for marketing. More than 40 new clinical drug candidates in Phase I, II, and III clinical trials or the NDA process, with half being PEGylated small-molecule drugs. It is clear that PEGylated drugs are shifting from large-molecules to small-molecules, with small-molecule drugs accounting for 80-90% of the drug market. Chongqing Upgra Biotechnology Co., Ltd. has successfully developed PEGylated small-molecule dual drugs, as well as those incorporating active targeting moieties or enhancers. In addition, PEGylation technology is applicable not only in the field of anti-tumor drugs but also in areas such as anti-infection, gout, arthritis, mental disease, pain, and other inflammatory conditions, and can also be applied in the field of diabetes. It has the potential to derive hundreds of product pipelines, which could form a massive industry worth hundreds of billions of US dollars eventually.

In recent years, the polyethylene glycol-lysine dendritic polypeptide-chemotherapy single drugs (including cabazitaxel, docetaxel, irinotecan, etc.) developed by Australia's STARPHARMA have demonstrated excellent efficacy in Phase II clinical trials, with significantly reduced toxicity and side effects. Pharmaceutical giants such as ASTRAZENECA, MERCK, and ROCHE/GENENTECH have begun participating in the research and development of this field. Chongqing Upgra Biotechnology Co., Ltd. has also synthesized multiple pipelines of PEG-polypeptide (including glutamic acid, aspartic acid, lysine, etc.)-small molecule drugs, (including small-molecule single drugs, dual drugs, multiple drugs, enhancers, and active targeting moieties, etc.) and has begun the corresponding clinical transformation. Recently, scientists from WINDOW Tx/Massachusetts Institute of Technology (MIT) and Harvard University published preclinical animal results of bottlebrush-like PEG-triplet drugs synthesized via ring-opening metathesis polymerization (ROMP) for the treatment of multiple myeloma. On the basis of these studies and related literature reports, to further enhance the water solubility and cell membrane permeability of PEG drugs, the inventors of the present invention have designed a variety of products, including PEG-peptide-small molecule drugs with a fatty amine core structure, active targeting moieties, and boron reagents. The biological tests have demonstrated that these compounds have significant therapeutic efficacy.

## Contents of the present invention

One objective of the present invention is to design and synthesize a PEG-peptide-based small molecule drug with a fatty polyamine core structure to further improve drug solubility and enhance clinical efficacy.

Therefore, in one aspect, the present invention provides a compound represented by formula I or a pharmaceutically acceptable salt thereof:

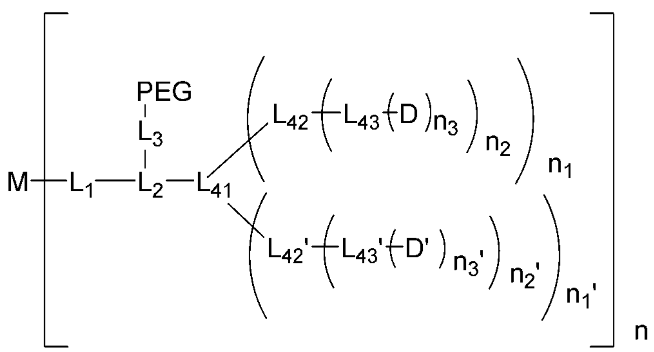

formula I

wherein,

M is a chain or cyclic structure containing one or more identical or different heteroatoms, and M is connected to $L_1$ via the heteroatom;

each $L_1$ is independently selected from the group consisting of a bond,

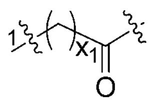

,

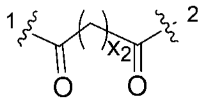

,

and a group formed by one or two identical or different $L_1$ and one or more identical or different M in the following manner: $L_1$-M-$(L_1)_n$, $L_1$-M-$(L_1$-M-$(L_1)_n)_n$, or $L_1$-M-$(L_1$-M-$(L_1$-M-$(Li)_n)_n)_n$, wherein the terminus 1 is connected to M, the terminus 2 is connected to $L_2$, wherein each of $x_1$ and $x_2$ is independently selected from the group consisting of 0, 1, 2, 3, 4, 5, 6, 7, 8, 9, and 10;

each $L_2$ is independently an amino acid residue containing in its side chain a functional group selected from the group consisting of $-NH_2$, -OH, -SH, and -COOH, and $L_2$ is connected to $L_1$, $L_3$, and $L_{41}$ via the N-terminus and C-terminus of the amino acid and the functional group; preferably, $L_2$ is connected to $L_3$ via the functional group;

each $L_3$ is independently

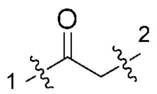

or

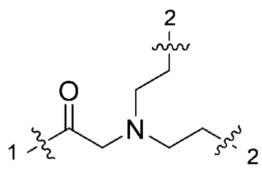

,

wherein the terminus 1 is connected to $L_2$, and the terminus 2 is connected to PEG;

each PEG is independently selected from the group consisting of polyethylene glycol, amino-polyethylene glycol, methoxy-polyethylene glycol, carboxyl-polyethylene glycol, and cholesterol-polyethylene glycol;

each $L_{41}$ is independently

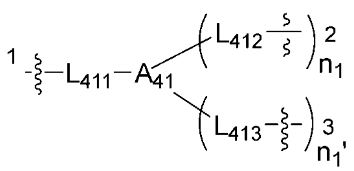

,

wherein the terminus 1 is connected to $L_2$, the terminus 2 is connected to $L_{42}$, and the terminus 3 is connected to $L_{42}$'; wherein each of $L_{411}$, $L_{412}$ and $L_{413}$ is independently selected from the group consisting of a bond, an amino acid residue or derivatives thereof, a polypeptide fragment consisting of two or more amino acids or derivatives thereof, $-NH(CH_2)_{x3}C(O)-$, $-CO(CH_2)_{x3}C(O)-$, $-NH(CH_2)_{x3}NH-$, and $-NH(CH_2)_2(OCH_2CH_2)_{x3}NH-$ and any combination thereof, wherein each $x_3$ is independently selected from the group consisting of 0, 1, 2, 3, 4, 5, 6, 7, 8, 9, and 10, $A_{41}$ is a bond or an amino acid residue or derivatives thereof, or a polypeptide fragment consisting of two or more amino acids or derivatives thereof;

each of $L_{42}$ and $L_{42}$' is independently a bond or

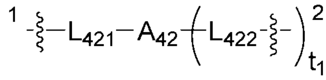,

wherein the terminus 1 is connected to $L_{41}$, and the terminus 2 is connected to $L_{43}$ or $L_{43}$'; wherein each of $L_{421}$ and $L_{422}$ is independently selected from the group consisting of a bond, an amino acid residue or derivatives thereof, or a polypeptide fragment consisting of two or more amino acids or derivatives thereof, $-NH(CH_2)x_4C(O)-$, $-CO(CH_2)x_4C(O)-$, $-NH(CH_2)x_4NH-$, and $-NH(CH_2)_2(OCH_2CH_2)x_4NH-$ and any combination thereof, each $x_4$ is independently selected from the group consisting of 0, 1, 2, 3, 4, 5, 6, 7, 8, 9, and 10, $A_{42}$ is a bond or selected from the group consisting of an amino acid residue or derivatives thereof, or a polypeptide fragment consisting of two or more amino acids or derivatives thereof, $t_1 = n_2$ or $n_2$';

each of $L_{43}$ and $L_{43}$' is independently selected from the group consisting of a bond,

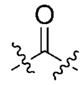,

-NH-, hydrazino (i.e., -NH-N=), an amino acid residue or derivatives thereof, a polypeptide fragment or derivatives thereofconsisting of two or more amino acids or derivatives thereof, $-NH(CH_2)_{x5}C(O)-$, $-NH(CH_2)_{x5}NH-$, $-NH(CH_2)_2(OCH_2CH_2)_{x5}NH-$, $-C(O)(CH_2)_{x5}C(O)-$,

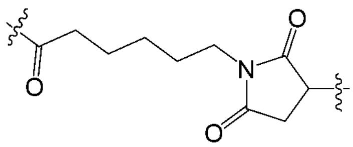

and any combination thereof, wherein each $x_5$ is independently selected from the group consisting of 0, 1, 2, 3, 4, 5, 6, 7, 8, 9, and 10;

each D is independently selected from the group consisting of cytotoxic drug moieties, preferably, the cytotoxic drug is selected from the group consisting of tubulin inhibitors, DNA intercalators, DNA topoisomerase inhibitors and RNA polymerase inhibitors; preferably, the cytotoxic drug is selected from the group consisting of PTX (paclitaxel), PCB (palbociclib), SN38 (7- and 10-hydroxy-camptothecin), NPB (Niraparib, MK-4827), AXT (Axitinib), LPT (lapatinib), DOX (doxorubicin), MI-AH-PLGLAG-iRGD, folic acid, SB7 (SB-743921), IRN (Irinotecan), sodium dodecahydrododecaborate, PPT-iRGD, disodium undecahydromercaptododecaborate, disodium undecahydromercaptododecaboride ($^{10}B$), disodium undecahydroaminododecaborate, disodium undecahydroaminododecaboride ($^{10}B$),

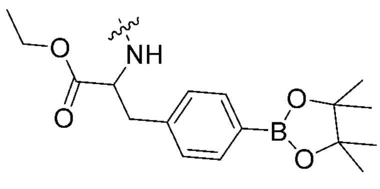, 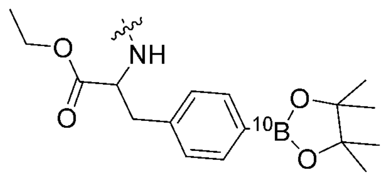 and 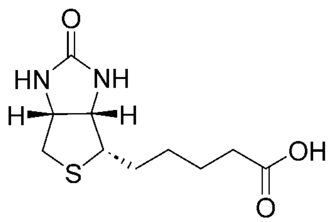 ;

each n is independently selected from the group consisting of 1, 2, 3, 4, 5, 6, 7, 8, 9, and 10;

each of $n_1$ and $n_1$' is independently selected from the group consisting of 0, 1, 2, 3, 4, 5, 6, 7, and 8;

each of $n_2$ and $n_2$' is independently selected from the group consisting of 1, 2, 3, 4, 5, 6, 7, and 8;

each of $n_3$ and $n_3$' is independently selected from the group consisting of 1, 2, 3, 4, 5, 6, 7, and 8.

In some embodiments, M is a chain or cyclic structure containing one or more identical or different heteroatoms, and M is connected to the group at its right via the heteroatom;

each $L_1$ is independently selected from the group consisting of a bond,

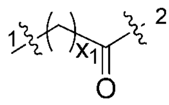

,

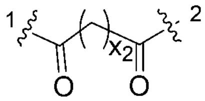

,

and a group formed by one or two identical or different $L_1$ and one or more identical or different M in the following manner: $L_1$-M-$(L_1)_n$, $L_1$-M-$(L_1$-M-$(L_1)_n)_n$, or $L_1$-M-$(L_1$-M-$(L_1$-M-$(L_1)_n)_n)_n$, in which the terminus 1 is connected to M and the terminus 2 is connected to $L_2$, wherein each of $x_1$ and $x_2$ is independently selected from the group consisting of 0, 1, 2, 3, 4, 5, 6, 7, or 8., 8, 9, and 10;

each $L_2$ is independently an amino acid residue containing in its side chain a functional group selected from the group consisting of $-NH_2$, -OH, -SH, and -COOH, and $L_2$ is connected to $L_1$, $L_3$, and $L_{41}$ via the N-terminus and C-terminus of the amino acid and the functional group; preferably, $L_2$ is connected to $L_3$ via the functional group;

each $L_3$ is independently

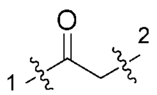

or

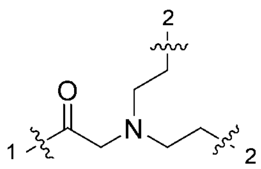

,

wherein the terminus 1 is connected to $L_2$ and the terminus 2 is connected to PEG;

each PEG is independently selected from the group consisting of polyethylene glycol, amino-polyethylene glycol, methoxy-polyethylene glycol, carboxyl-polyethylene glycol, and cholesterol-polyethylene glycol;

each $L_{41}$ is independently

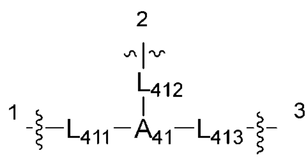

,

in which the terminus 1 is connected to $L_2$, the terminus 2 is connected to $L_{42}$, the terminus 3 is connected to $L_{42}$', wherein each of $L_{411}$, $L_{412}$ and $L_{413}$ is independently selected from the group consisting of a bond, an amino acid residue or derivatives thereof, or a polypeptide fragment consisting of two or more amino acids or derivatives thereof, $-NH(CH_2)_{x3}C(O)-$, $-CO(CH_2)_{x3}C(O)-$, $-NH(CH_2)_{x3}NH-$ and $-NH(CH_2)_2(OCH_2CH_2)_{x3}NH-$ and any combination thereof, each $x_3$ is independently selected from the group consisting of 0, 1, 2, 3, 4, 5, 6, 7, 8, 9 and 10, $A_{41}$ is a bond or selected from the group consisting of an amino acid residue or derivatives thereof, or a polypeptide fragment consisting of two or more amino acids or derivatives thereof, and any of the above amino acid residue or derivatives thereof, or the polypeptide fragment consisting of two or more amino acids or derivatives thereof, provides 1, 2, 3, 4, 5, or 6 attachment sites (for example, when the attachment site is provided by the amino acid residue or derivatives thereof, or the polypeptide fragment consisting of two or more amino acids or derivatives thereof in $L_{412}$ or $L_{413}$, the attachment site is attached to $L_{42}$ or $L_{42}$'; for another example, when the attachment site is provided by $A_{41}$, the attachment site is connected to 1, 2, 3, 4, 5, or 6 $L_{412}$ or $L_{413}$ groups);

each of $L_{42}$ and $L_{42}$' is independently

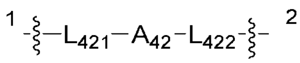

in which the terminus 1 is connected to $L_{41}$ and the terminus 2 is connected to $L_{43}$ or $L_{43}$', wherein each of $L_{421}$ and $L_{422}$ is independently selected from the group consisting of a bond, an amino acid residue or derivatives thereof, or a polypeptide fragment consisting of two or more amino acids or derivatives thereof, $-NH(CH_2)x_4C(O)-$, $-CO(CH_2)x_4C(O)-$, $-NH(CH_2)x_4NH-$ and $-NH(CH_2)_2(OCH_2CH_2)x_4NH-$ and any combination thereof, each $x_4$ is independently selected from the group consisting of 0, 1, 2, 3, 4, 5, 6, 7, 8, 9 and 10, $A_{42}$ is a bond or selected from the group consisting of an amino acid residue or derivatives thereof, or a polypeptide fragment consisting of two or more amino acids or derivatives thereof, and any of the above amino acid residue or derivatives thereof, or the polypeptide fragment consisting of two or more amino acids or derivatives thereof, provides 1, 2, 3, 4, 5, or 6 attachment sites (for example, when the attachment site is provided by the amino acid residue or derivatives thereof or the polypeptide fragment consisting of two or more amino acids or derivatives thereof in $L_{422}$, the attachment site is connected to $L_{43}$ or $L_{43}$'; for another example, when the attachment site is provided by $A_{42}$, the attachment site is connected to 1, 2, 3, 4, 5, or 6 $L_{422}$ groups);

each of $L_{43}$ and $L_{43}$' is independently selected from the group consisting of a bond,

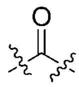,

-NH-, hydrazino (i.e., -NH-N=), an amino acid residue or derivatives thereof, a polypeptide fragment consisting of two or more amino acids or derivatives thereof, $-NH(CH_2)_{x5}C(O)-$, $-NH(CH_2)_{x5}NH-$, $-NH(CH_2)_2(OCH_2CH_2)_{x5}NH-$, $-C(O)(CH_2)_{x5}C(O)-$,

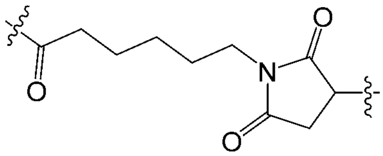

and any combination thereof, wherein each $x_5$ is independently selected from the group consisting of 0, 1, 2, 3, 4, 5, 6, 7, 8, 9, and 10;

each D is independently selected from the group consisting of cytotoxic drug moieties, preferably, the cytotoxic drug is selected from the group consisting of microtubule inhibitors, DNA intercalators, DNA topoisomerase inhibitors, and RNA polymerase inhibitors; preferably, the cytotoxic drug is selected from the group consisting of PTX (paclitaxel), PCB (palbociclib), SN38 (7- and 10-hydroxy-camptothecin), NPB (Niraparib, MK-4827), AXT (Axitinib), LPT (lapatinib), DOX (doxorubicin), MI-AH-PLGLAG-iRGD, folic acid, SB7 (SB-743921), IRN (Irinotecan), sodium dodecahydrododecaborate, PPT-iRGD, 1,2,3,4,5,6,7,8,9,10,11-undecahydro-12-mercapto-dodecaborane (BSH, sodium mercaptododecaborate), 1,2,3,4,5,6,7,8,9,10,11-undecahydro-12-mercapto dodecaborate(2-) (sodium mercaptododecaborate ($^{10}B$)),

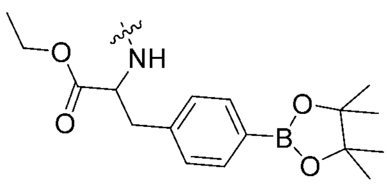,

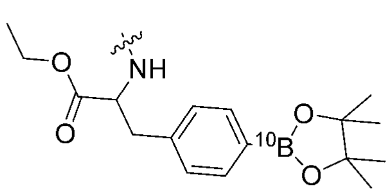 and 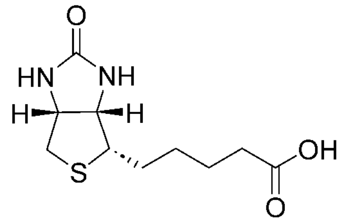 ;

each n is independently selected from the group consisting of 1, 2, 3, 4, 5, 6, 7, 8, 9, and 10;

each of $n_1$ and $n_1'$ is independently selected from the group consisting of 0, 1, 2, 3, 4, 5, 6, 7, and 8;

each of $n_2$ and $n_2'$ is independently selected from the group consisting of 1, 2, 3, 4, 5, 6, 7, and 8;

each of $n_3$ and $n_3'$ is independently selected from the group consisting of 1, 2, 3, 4, 5, 6, 7, and 8.

In some embodiments, the compound is characterized by one or more of the following:

(1) M is selected from the group consisting of groups obtained by losing one or more (e.g., 1 to 10) hydrogen atoms in the following molecules:

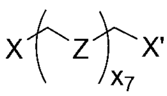 , 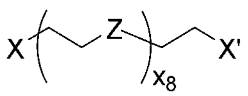 and 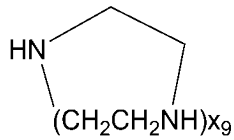 ,

wherein each of X and X' is independently selected from the group consisting of -OH, -NRR', and -SH, Z is selected from the group consisting of -C(RR')-, -O-, and -N(R)-, each of R and R' is independently selected from the group consisting of hydrogen, $C_{1-6}$ alkyl, and $C_{1-6}$ alkyl substituted with amino or $C_{1-6}$ alkylamino, each of $x_7$ and $x_8$ is independently selected from the group consisting of 0, 1, 2, 3, 4, 5, 6, 7, 8, 9, and 10, and $x_9$ is selected from the group consisting of 1, 2, 3, 4, 5, and 6;

(2) $L_1$ is selected from

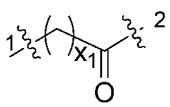 , 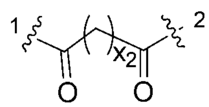 ,

and a group formed by one or more identical or different $L_1$ and one or more identical or different M in the following manner: $L_1$-M-$(L_1)_n$, wherein each of $x_1$ and $x_2$ is independently selected from the group consisting of 1, 2, 3, 4, 5 and 6; preferably, $L_1$ is selected from

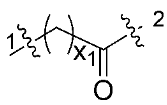 , 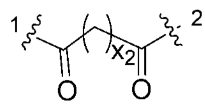

and

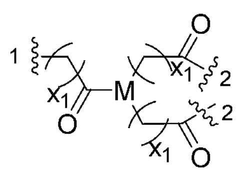

(for example,

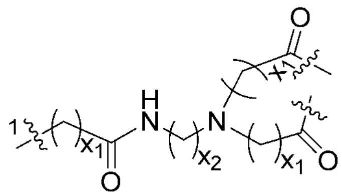

), each of $x_1$ and $x_2$ is independently selected from the group consisting of 1, 2, 3, 4, 5 and 6;

(3) $L_2$ is selected from the group consisting of the residues of the following amino acids: Lys, Cys, Thr, Ser, Asp or Glu;

(4) PEG is methoxy-polyethylene glycol;

(5) PEG has a number-average molecular weight of 5k to 40k;

(6) each $L_{411}$ is independently selected from the group consisting of a bond, $-NH(CH_2)_{x3}C(O)-$, $-CO(CH_2)_{x3}C(O)-$, $-NH(CH_2)_{x3}NH-$, $-NH(CH_2)_{x3}C(O)NH(CH_2)_{x3}C(O)-$, $-NH(CH_2)_2(OCH_2CH_2)_{x3}NH-$ and $-NH(CH_2)_2(OCH_2CH_2)_{x3}NH-$, and $x_3$ is selected from the group consisting of 1, 2, 3, 4, 5 and 6; preferably, each $L_{411}$ is independently selected from the group consisting of a bond, $-NH(CH_2)_5C(O)-$ and $-NH(CH_2)_2C(O)-$;

(7) each $L_{412}$ is independently selected from the group consisting of a bond, $-NH(CH_2)_{x3}C(O)-$, $-CO(CH_2)_{x3}C(O)-$, $-NH(CH_2)_{x3}NH-$, $-NH(CH_2)_{x3}C(O)-A-$, $-NH(CH_2)_{x3}C(O)NH(CH_2)_{x3}C(O)-$, $-NH(CH_2)_2(OCH_2CH_2)_{x3}NH-$ and $-NH(CH_2)_2(OCH_2CH_2)_{x3}NH-$, wherein A is selected from the group consisting of an amino acid residue or derivatives thereof, or a polypeptide fragment consisting of two or more amino acids or derivatives thereof, wherein the amino acid is selected from the group consisting of Glu and Asp, preferably, the amino acid residue or derivatives thereof or the polypeptide fragment or derivatives thereof provides $n_1$ (e.g., 1, 2, 3, 4, 5 or 6) attachment sites (e.g., the attachment site is optionally connected to $L_{42}$ or $L_{42}$'), each $x_3$ is independently selected from the group consisting of 1, 2, 3, 4, 5 and 6; preferably, A is selected from the group consisting of Glu, Glu-Glu, $Glu(Glu)_2$; preferably, each $L_{412}$ is independently selected from the group consisting of a bond, $-NH(CH_2)_5C(O)-Glu(Glu)_2$, $-NH(CH_2)_2NH-$ and $-NH(CH_2)_2C(O)-$;

(8) each $L_{413}$ is independently selected from the group consisting of a bond, $-NH(CH_2)_{x3}C(O)-$, $-NH(CH_2)_{x3}NH-$, $-NH(CH_2)_2(OCH_2CH_2)_{x3}NH-$, $-CO(CH_2)_{x3}C(O)-$, $-NH(CH_2)_{x3}C(O)NH(CH_2)_{x3}C(O)-$ and $-NH(CH_2)_{x3}C(O)NH(CH_2)_{x3}NH-$, each $x_3$ is independently selected from the group consisting of 1, 2, 3, 4, 5 and 6; preferably, $L_{413}$ is $-NH(CH_2)_2CO-$; preferably, each $L_{413}$ is independently selected from the group consisting of a bond and $-NH(CH_2)_2C(O)-$; preferably, $L_{413}$ is $-NH(CH_2)_2CO-$;

(9) the amino acid in $A_{41}$ or $A_{42}$ is selected from the group consisting of Glu, Gly, Asp, Lys, Cys, Thr and Ser;

(10) each $L_{421}$ is independently selected from the group consisting of a bond, $-NH(CH_2)x_4C(O)-$, $-CO(CH_2)x_4C(O)-$, $-NH(CH_2)x_4NH-$, $-NH(CH_2)_2(OCH_2CH_2)x_4NH-$ and any combination thereof, each $x_4$ is independently selected from the group consisting of 0, 1, 2, 3, 4, 5, 6, 7, 8, 9 and 10; preferably, each $L_{421}$ is independently selected from the group consisting of a bond, $-NH(CH_2)_5C(O)-$, $-CO(CH_2)_2C(O)-$, $-NH(CH_2)_2NH-$, $-NH(CH_2)_2(OCH_2CH_2)_2NH-$ and $-NH(CH_2)_2NH-CO(CH_2)_2C(O)-$;

(11) each $L_{422}$ is independently selected from the group consisting of a bond, $-NH(CH_2)_{x3}C(O)-A'-$, $-NH(CH_2)x_4C(O)-$, $-CO(CH_2)x_4C(O)-$, $-NH(CH_2)x_4NH-$ and $-NH(CH_2)_2(OCH_2CH_2)x_4NH-$, wherein A' is selected from the group consisting of an amino acid residue or derivatives thereof, or a polypeptide fragment consisting of two or more amino acids or derivatives thereof, and the amino acid is selected from the group consisting of Glu and Asp; preferably, the amino acid residue or derivatives thereof or the polypeptide fragment or derivatives thereof provides $t_1$ (e.g., 1, 2, 3, 4, 5 or 6) attachment sites (e.g., the attachment site is optionally connected to $L_{43}$), and each $x_4$ is independently selected from the group consisting of 0, 1, 2, 3, 4, 5, 6, 7, 8, 9 and 10;

(12) the amino acids in $L_{43}$ and $L_{43}$' are selected from the group consisting of Glu, Gly, Phe, Leu and Cys; preferably, each $L_{422}$ is independently selected from the group consisting of a bond, $-NH(CH_2)_2NH-$, $-NH(CH_2)_5C(O)-Glu(Glu)_2$, $-NH(CH_2)_2C(O)-$, $-CO(CH_2)_2C(O)-$ and $-NH(CH_2)_2(OCH_2CH_2)_2NH-$;

(13) the derivatives in $L_{41}$, $L_{42}$, $L_{42}$', $L_{43}$ and $L_{43}$' are selected from the group consisting of acylation (e.g., acetylation)

or alkylation (e.g., methylation) derivatives;

(14) each $x_6$ is independently selected from the group consisting of 1, 2, 3, 4, 5 and 6.

In some embodiments, A, $L_{411}$, $L_{412}$ and $L_{413}$ are not simultaneously bonds.

In some embodiments, $n_1$'=0, in which case the

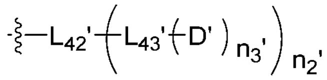

connected to $L_{41}$ in formula I is absent.

In some embodiments, the compound is characterized by one or more of the following:

(1) M is selected from the groups obtained by losing one or more (e.g., 1 to 10) hydrogen atoms in the following molecules: EDA, DETA, AMDA, TAEA, TETA, TEPA, DAPO, APDO, APD, glycerol, MPE,

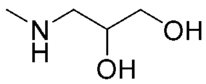 , 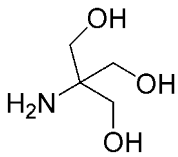 ,

TAN and TACD; preferably, M is selected from the group consisting of

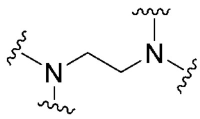 , 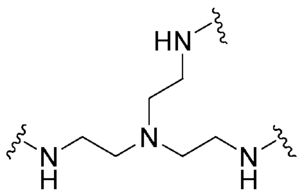 ,

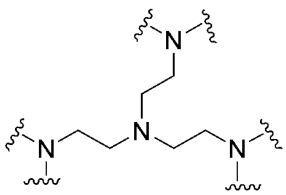 , 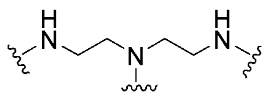 , 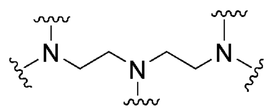 ,

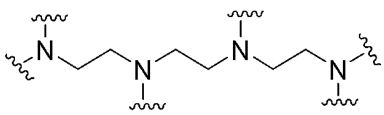 , 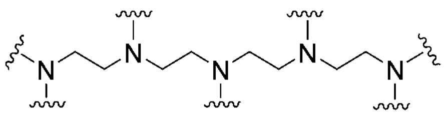 , 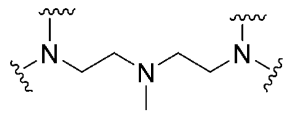 ,

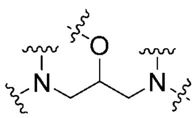 , 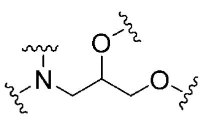 , 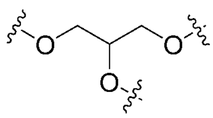 , 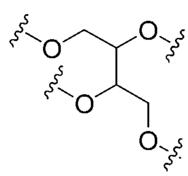 , 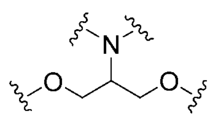 ,

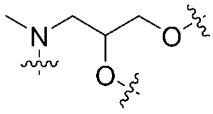, 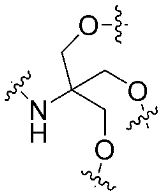, 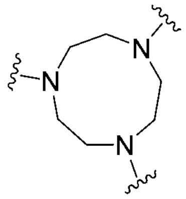 and 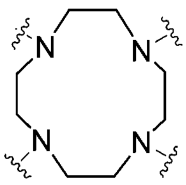;

(2) $L_1$ is selected from the group consisting of

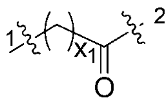, 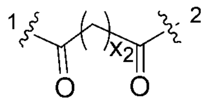

and

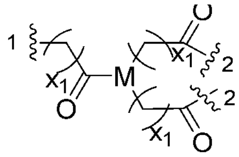,

and each of $x_1$ and $x_2$ is independently selected from the group consisting of 0, 1, 2, 3, 4, and 5;
(3) $L_2$ is selected from the residues of the following amino acids: Lys, Cys, Thr, or Ser;
(4) PEG has a number-average molecular weight of 5k to 10k, or 10k to 40k, for example, 5k or 10k;
(5) each of $A_{41}$ and $A_{42}$ is independently selected from the group consisting of a bond, Glu, Lys, Glu-Asp, Glu-Glu, $Glu(Glu)_2$, Glu(Glu)(Glu(Glu)(GlyGlu)), GluGluGly, GlyGlu;
(6) each $L_{41}$ is independently selected from the group consisting of $-NH(CH_2)_2CO-$, $-NH(CH_2)_5CO-$,

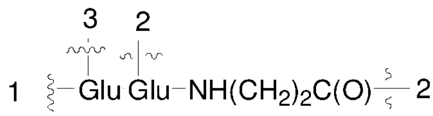, 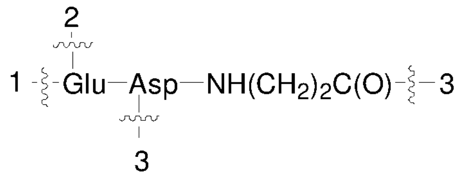,

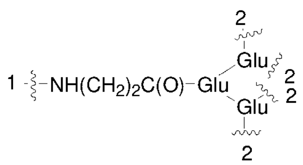, 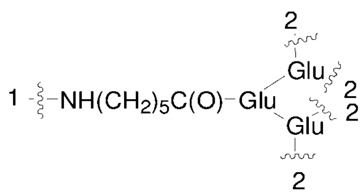, 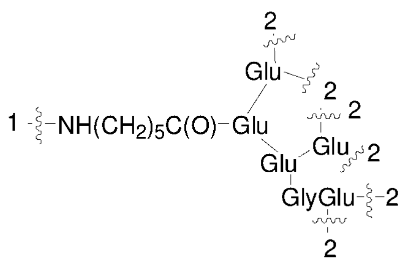,

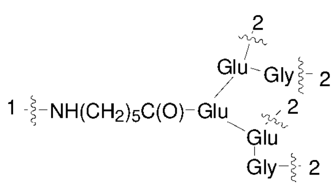, 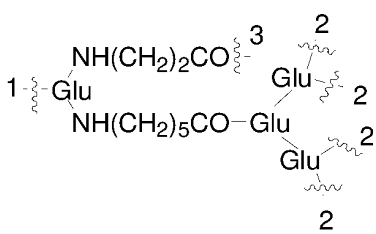, 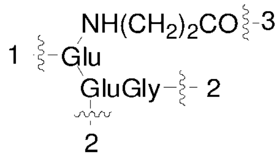,

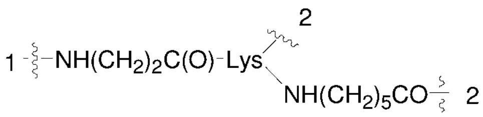,
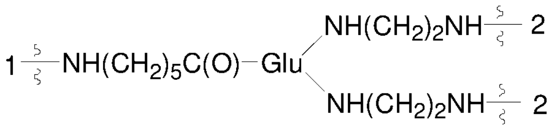,

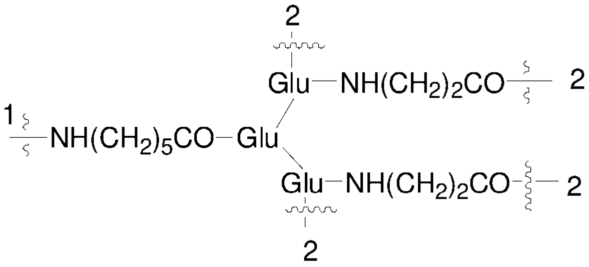,
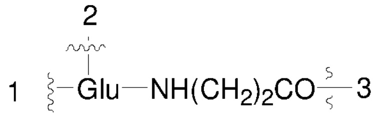

and

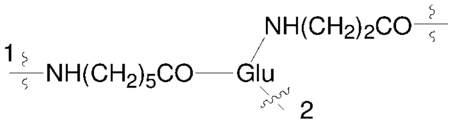
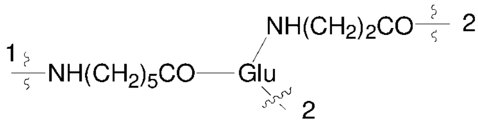;

(7) each of $L_{42}$ and $L_{42}'$ is independently selected from the group consisting of a bond, Glu, GlyGlu, $-NH(CH_2)_5CO-$, $-CO(CH_2)_2CO-$, $-NH(CH_2)_2NH-$, $-NH(CH_2)_5CO-Glu$, $Glu(Glu)_2$, $-NH(CH_2)_2(OCH_2CH_2)_2NH-$,

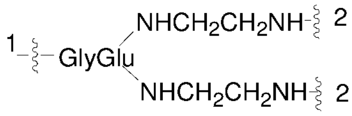,
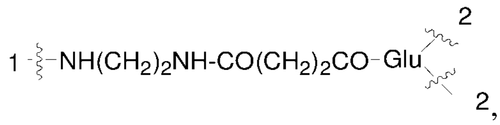,

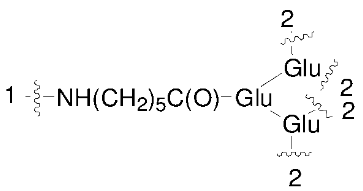,
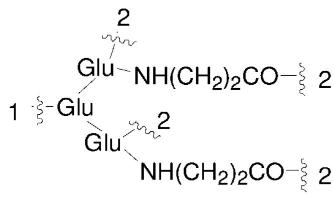,

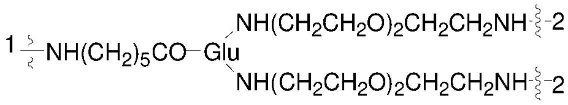,

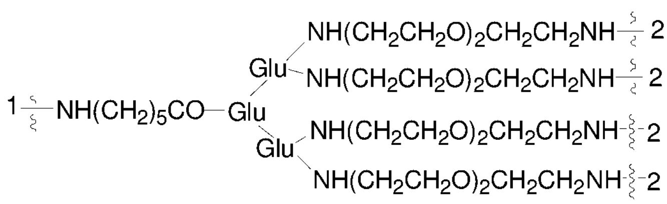,
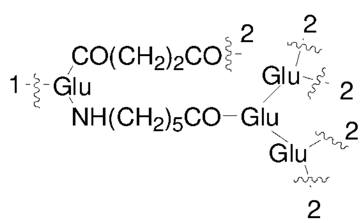

and

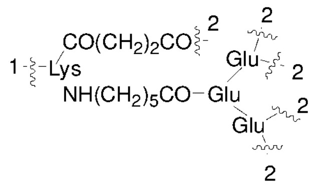;

(8) each of $L_{43}$ and $L_{43}$' is independently selected from the group consisting of a bond,

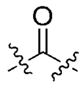,

Cys(Ac), -NH-N=, -GlyPheLeuGly-, $-C(O)(CH_2)_{x5}C(O)-$, -Gly-NH-N=, Glu(GlyPheLeuGly-)$_2$, GlyGlu(GlyPheLeuGly)$_2$, -Glu(Gly-NHN=)$_2$, $-C(O)(CH_2)_{x5}C(O)$-Glu(GlyPheLeuGly)(Glu(GlyPheLeuGly)$_2$), Glu(Glu)$_2$, $-NH(CH_2)_{x5}C(O)-$, $-NH(CH_2)_{x5}C(O)NH-$, $-NH(CH_2)_2(OCH_2CH_2)_{x5}NH-$, $-C(O)(CH_2)_{x5}C(O)-NH(CH_2)_{x5}C(O)-$, and

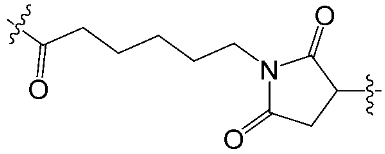,

each $x_5$ is independently selected from the group consisting of 1, 2, 3, 4, 5 and 6.

In some embodiments, the compound is characterized by one or more of the following:

(1) $L_2$ is selected from Lys residue;
(2) each of $L_{43}$ and $L_{43}$' is independently selected from the group consisting of a bond,

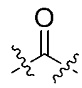,

Cys(Ac), -GlyPheLeuGly-, $-C(O)(CH_2)_2C(O)-$, Gly-NHN=, -GlyGlu(GlyPheLeuGly-)$_2$, Glu(GlyPheLeuGly-)$_2$, -Glu(Gly-NHN=)$_2$, $-C(O)(CH_2)_2C(O)$-Glu(GlyPheLeuGly)(Glu(GlyPheLeuGly)$_2$), Glu(Glu)$_2$, $-NH(CH_2)_5C(O)-$, $-NH(CH_2)_5C(O)NH-$, $-NH(CH_2)_2(OCH_2CH_2)_2NH-$, $-C(O)(CH_2)_2C(O)-NHCH_2C(O)-$, $-C(O)(CH_2)_2C(O)-NH(CH_2)_2C(O)-$, and

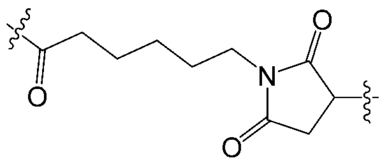.

Herein, D is a group formed by linking the cytotoxic drug to the rest of the general formula via a reactive group thereon. In some embodiments, the reactive group is an amino ($-NH_2$), a secondary amine, a hydroxyl (-OH), a thiol (-SH), a carboxyl (-COOH), or the like.

In some embodiments, D is selected from the group consisting of:

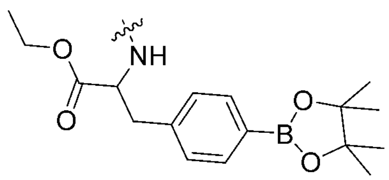 , 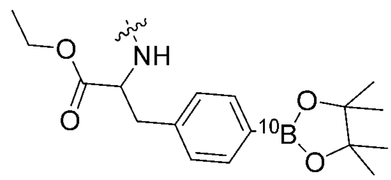 , 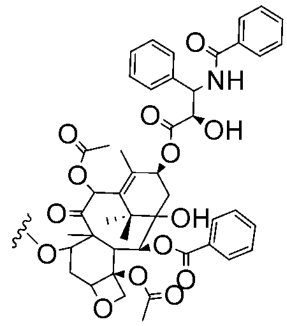 ,

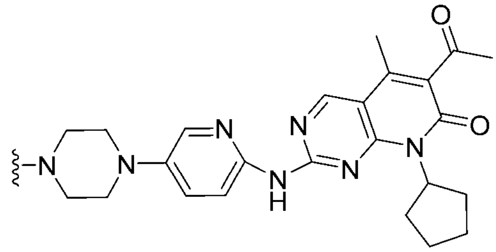 , 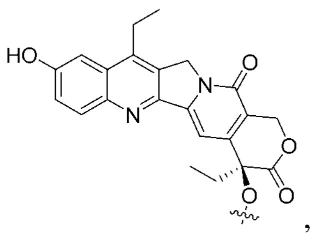 , 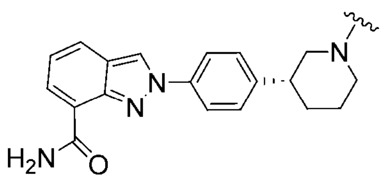 ,

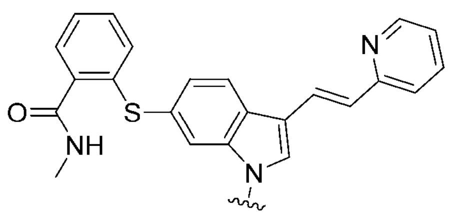 , 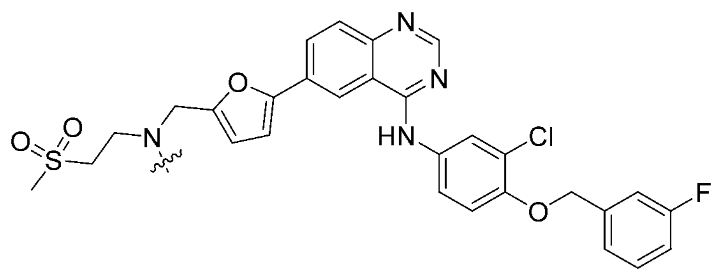 ,

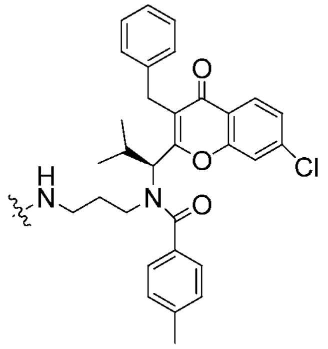 , 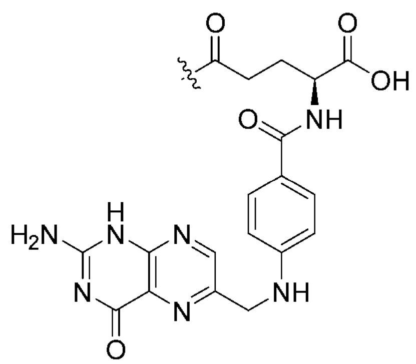 ,

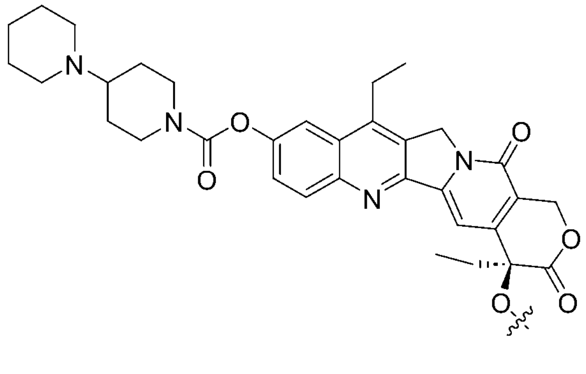 , 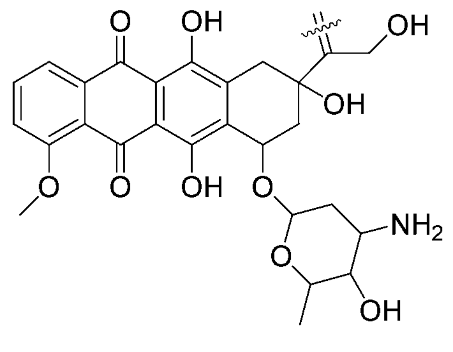 ,

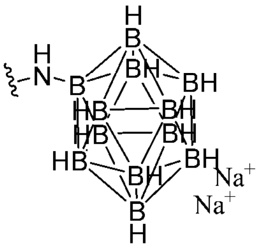 , 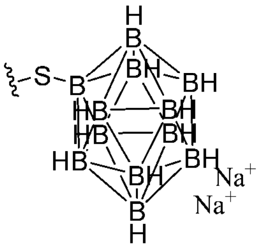 , 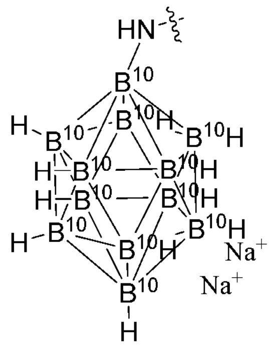 , 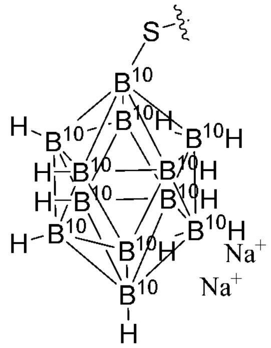 ,

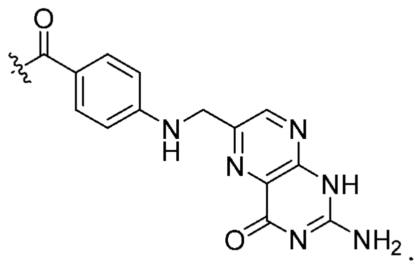

In some embodiments, the compound is selected from the group consisting of the following compounds:

| | |
|---|---|
| **71-215** | 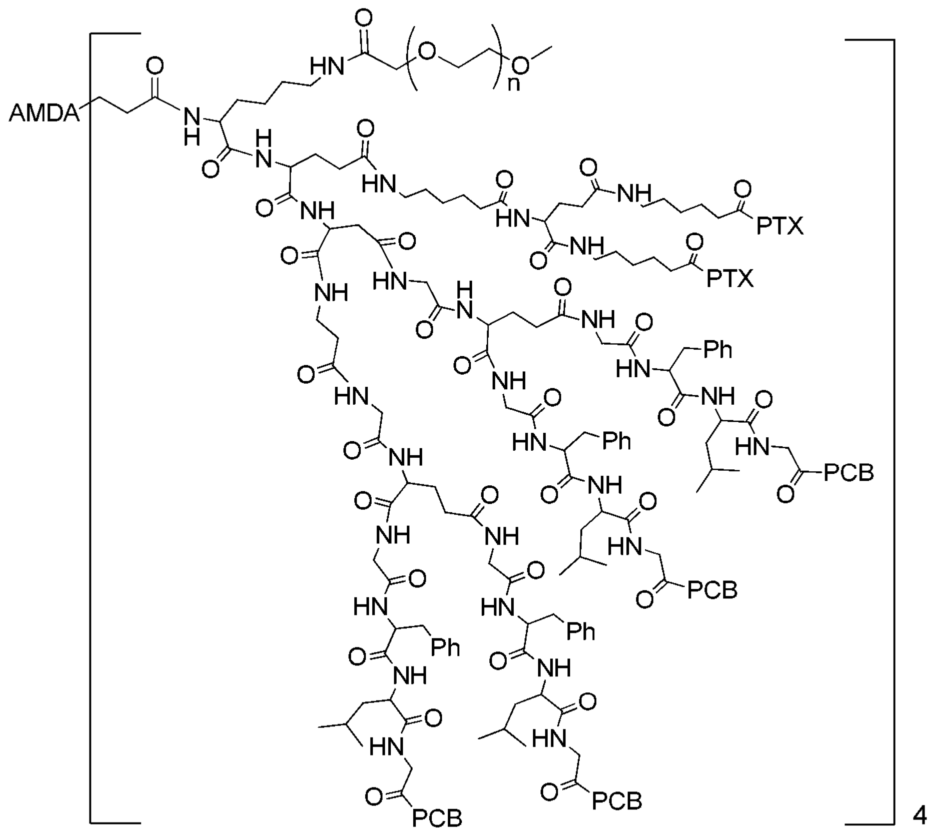 each polyethylene glycol fragment has a number-average molecular weight of 10k |

(continued)

| | |
|---|---|
| **59-126** | 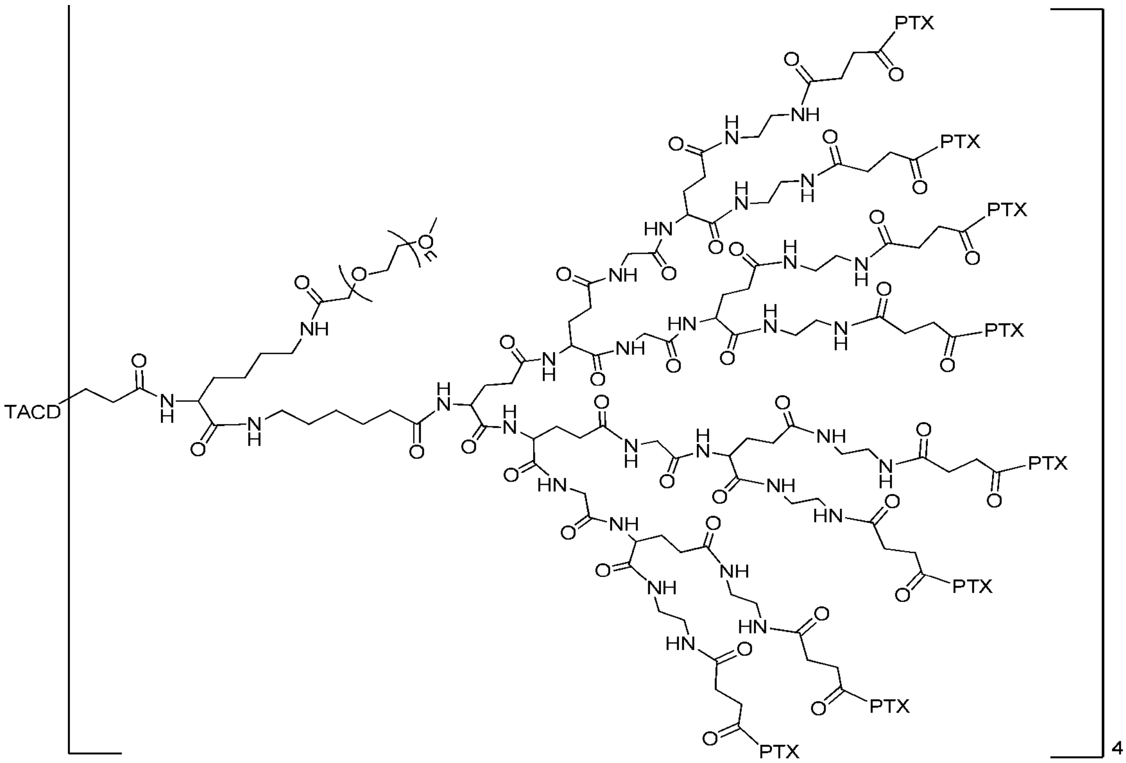<br>each polyethylene glycol fragment has a number-average molecular weight of 10k |

(continued)

| | |
|---|---|
| **67-228** | 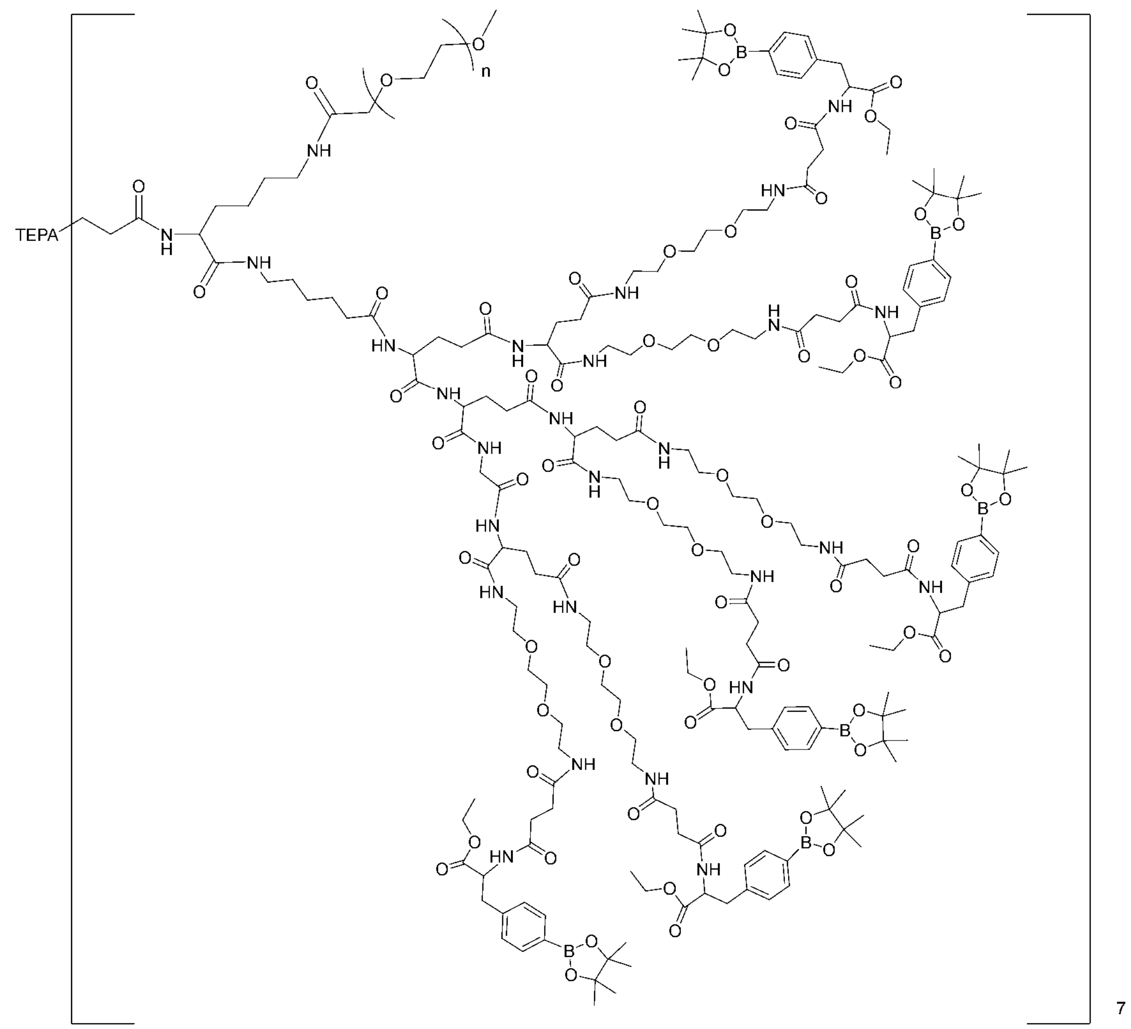<br>each polyethylene glycol fragment has a number-average molecular weight of 5k |

(continued)

| | |
|---|---|
| **81-118** | 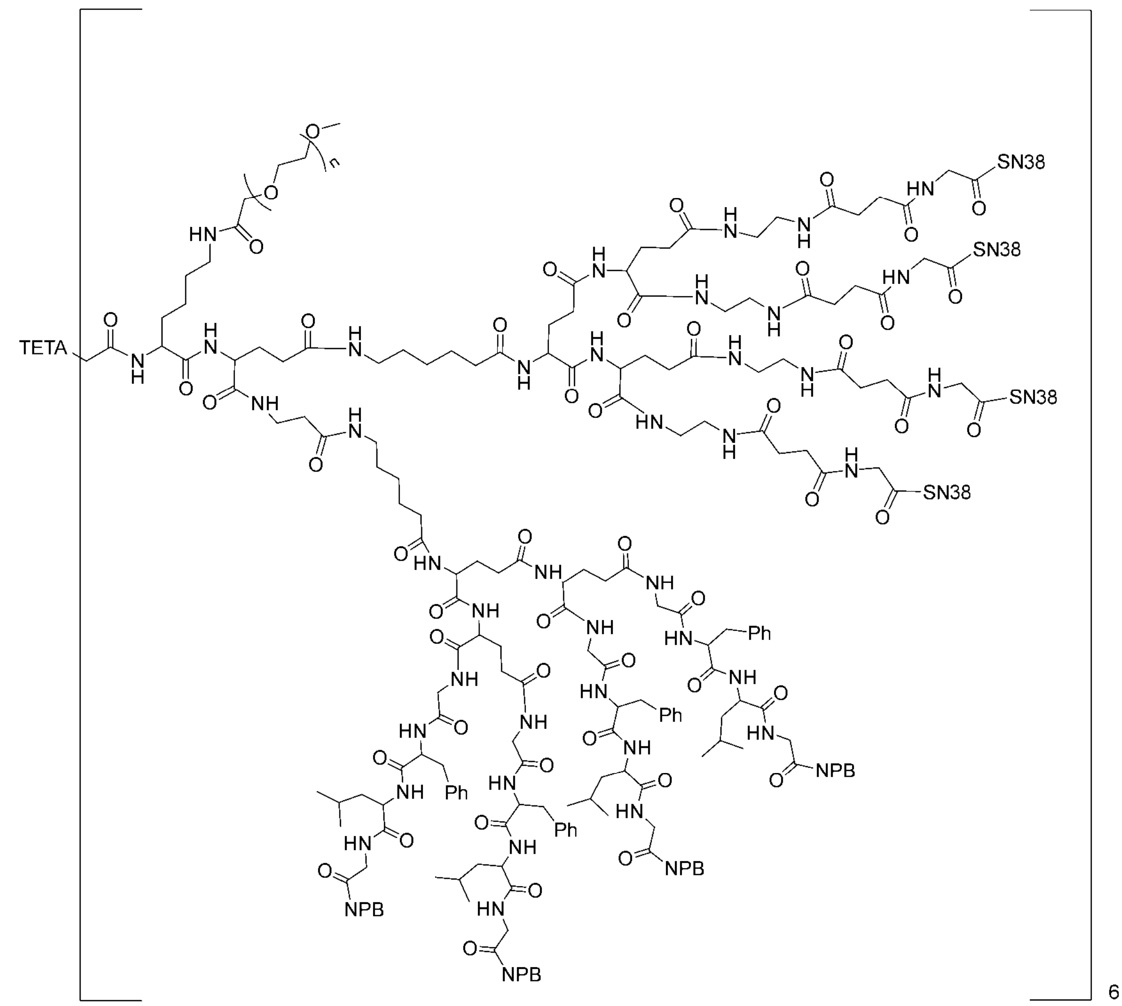 each polyethylene glycol fragment has a number-average molecular weight of 5k |
| **50-221** | 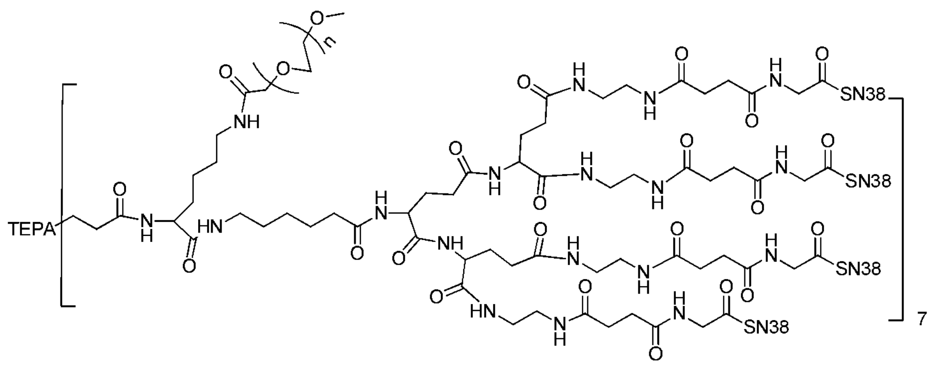 each polyethylene glycol fragment has a number-average molecular weight of 5k |

(continued)

| | |
|---|---|
| **69-113** | 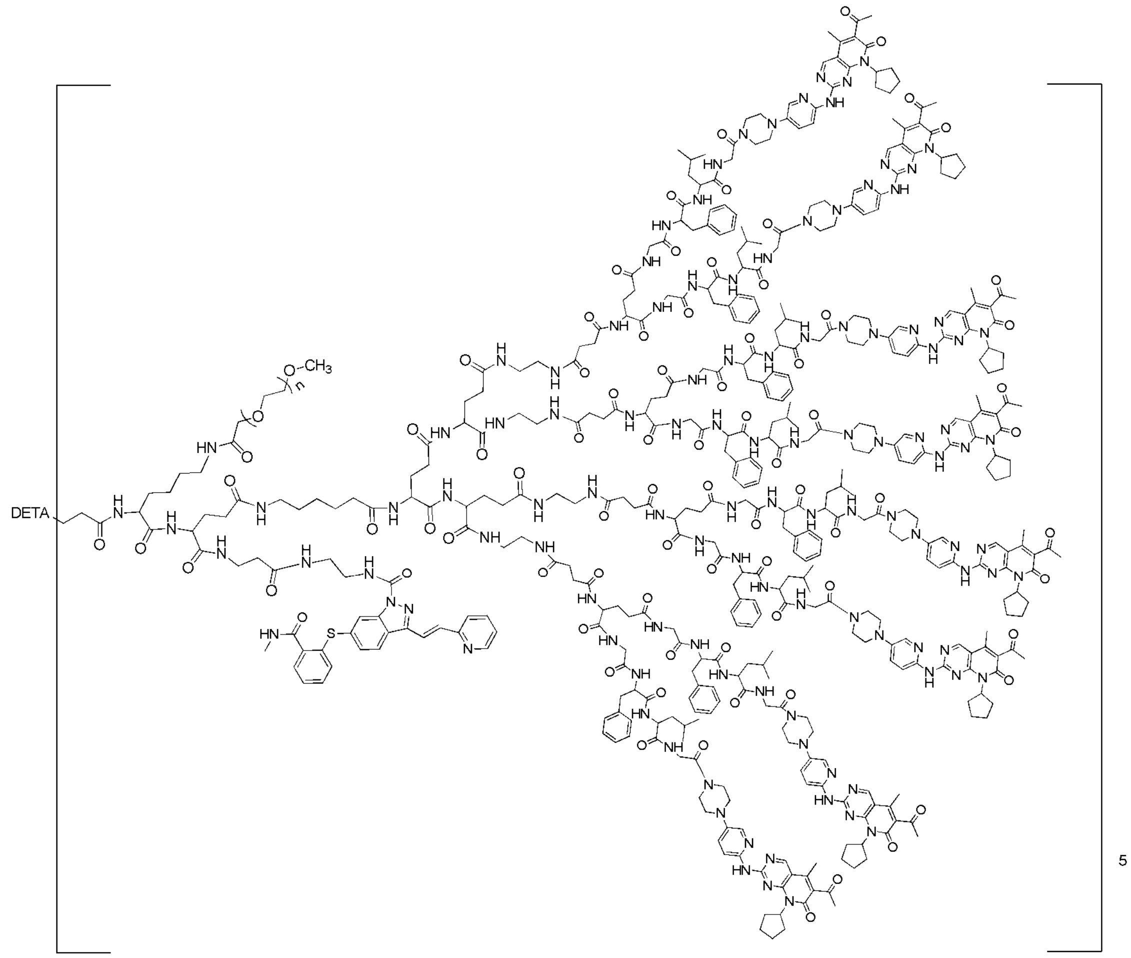 each polyethylene glycol fragment has a number-average molecular weight of 5k |

(continued)

| | |
|---|---|
| **71-246** | 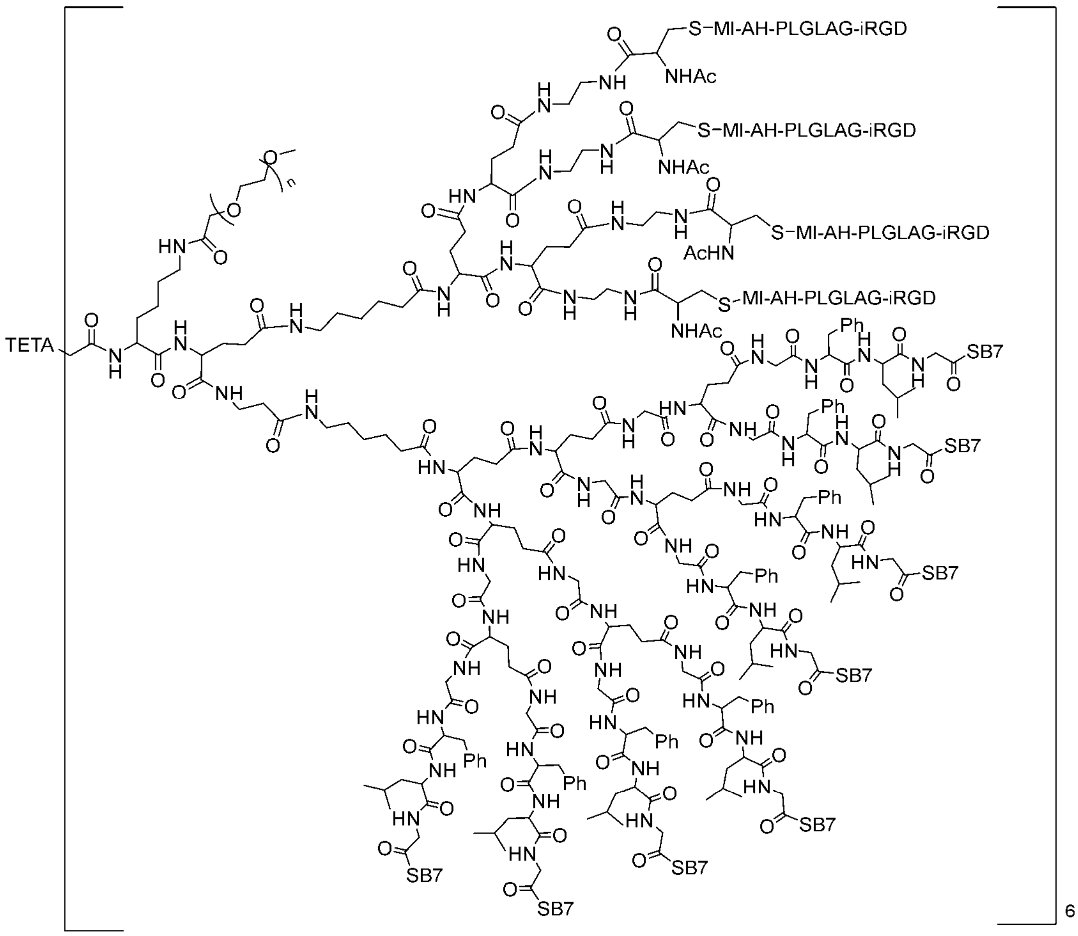<br>each polyethylene glycol fragment has a number-average molecular weight of 5k |
| **70-92** | 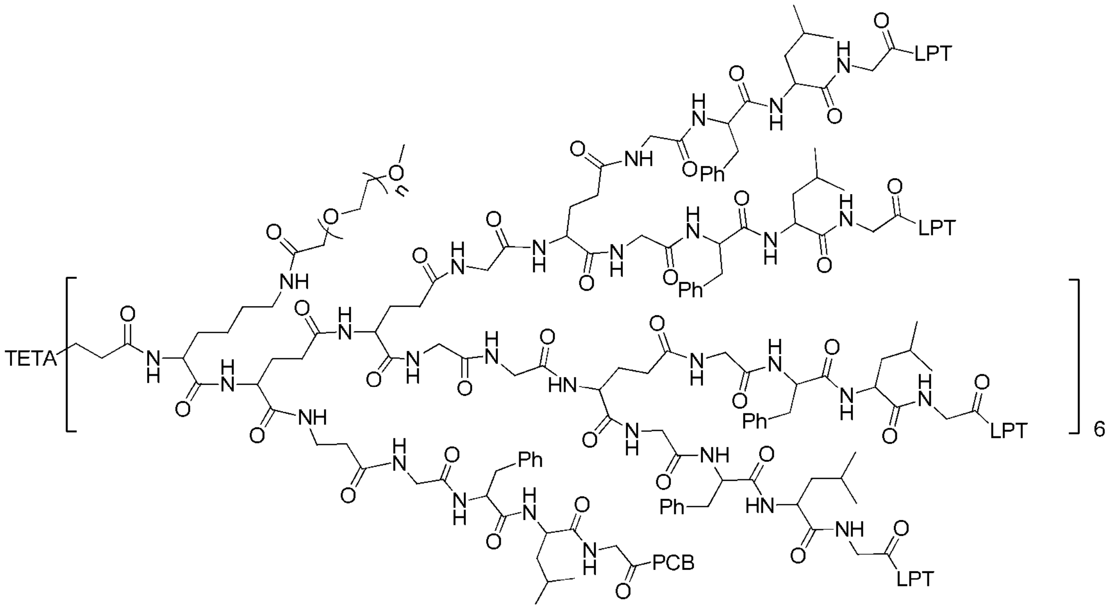<br>each polyethylene glycol fragment has a number-average molecular weight of 5k |

(continued)

| | |
|---|---|
| **77-231** | 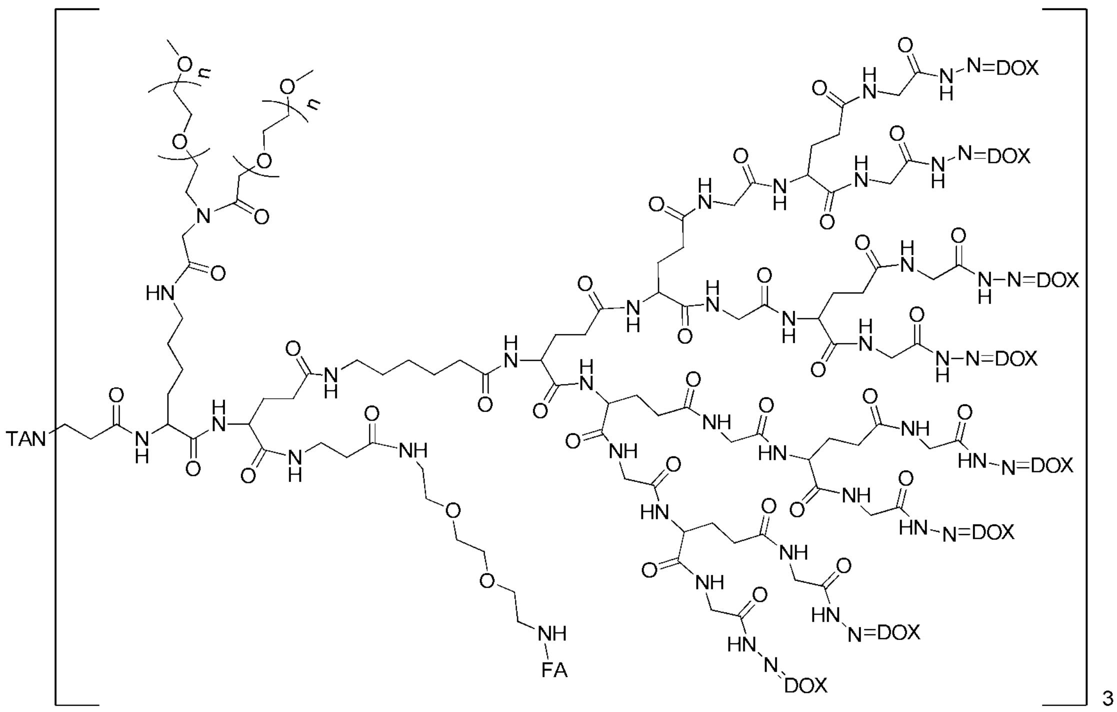<br>each polyethylene glycol fragment has a number-average molecular weight of 10k |
| **82-183** | 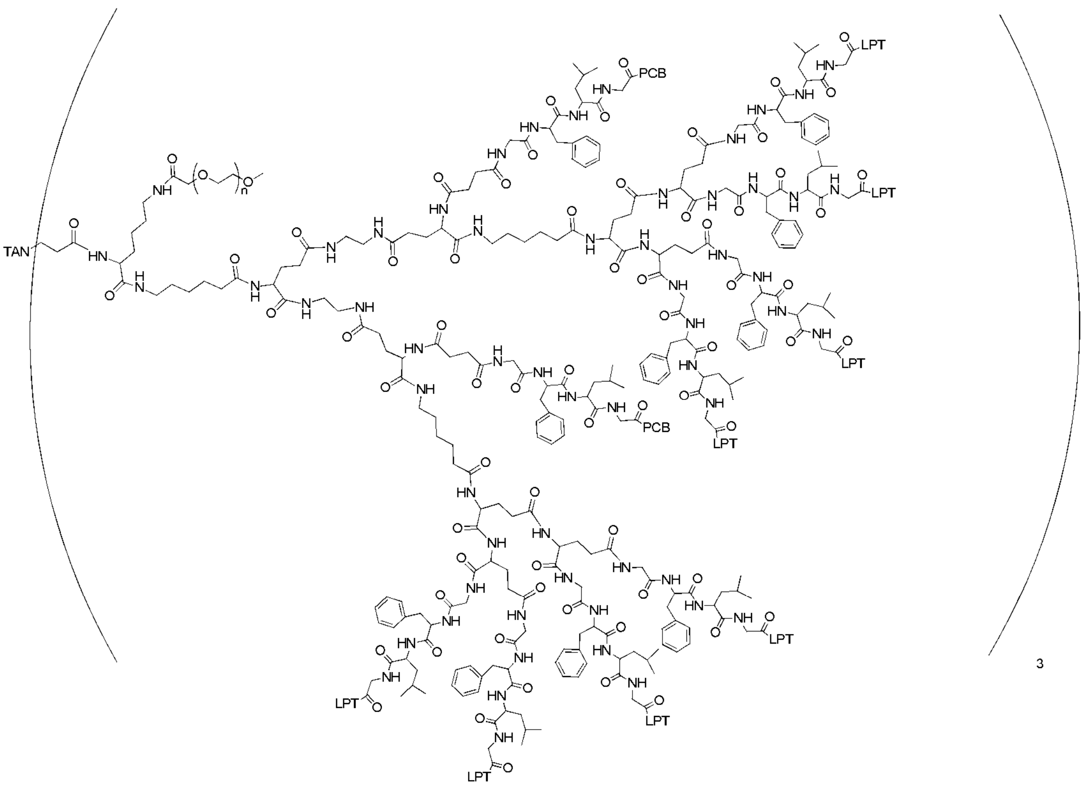<br>each polyethylene glycol fragment has a number-average molecular weight of 10k |

(continued)

| | |
|---|---|
| **82-87** | 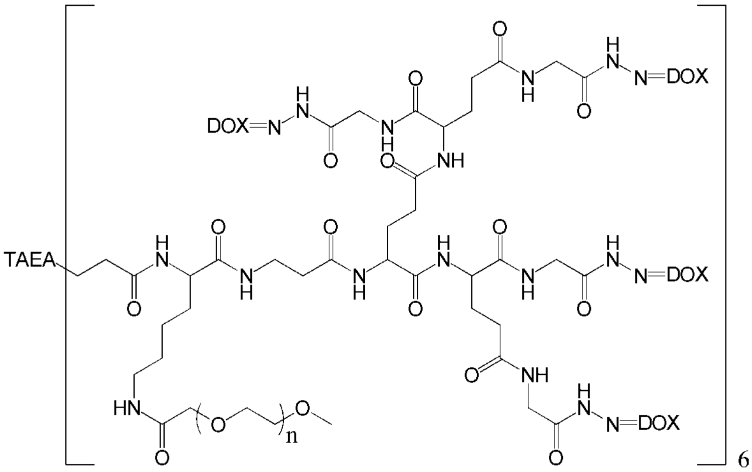 each polyethylene glycol fragment has a number-average molecular weight of 5k |
| **73-49** | 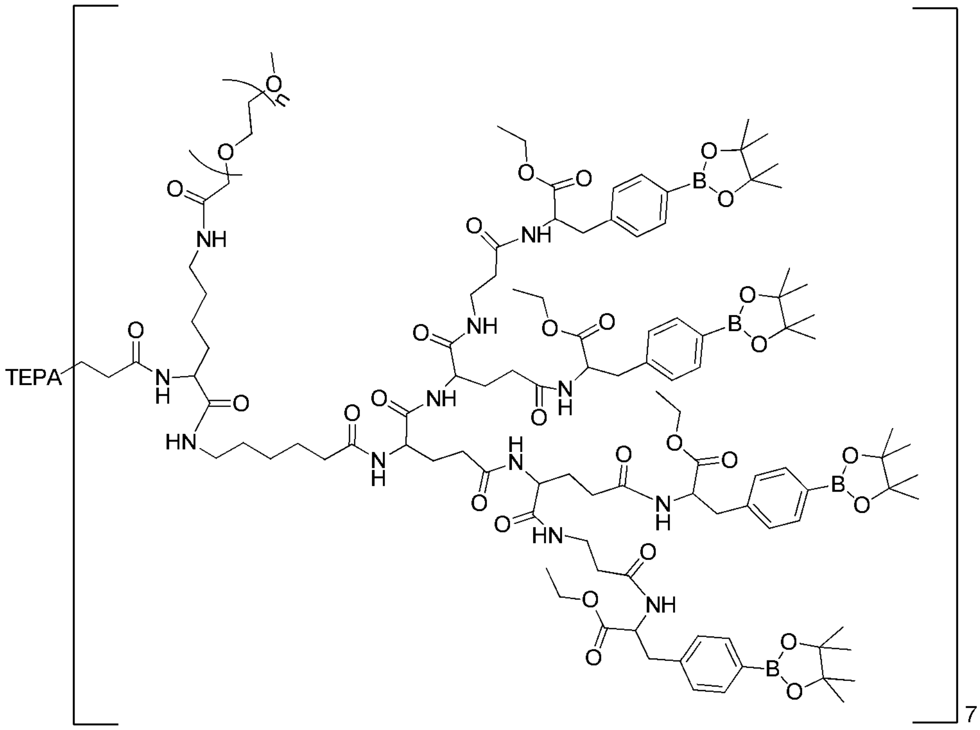 each polyethylene glycol fragment has a number-average molecular weight of 5k |

(continued)

| | |
|---|---|
| **82-226** | 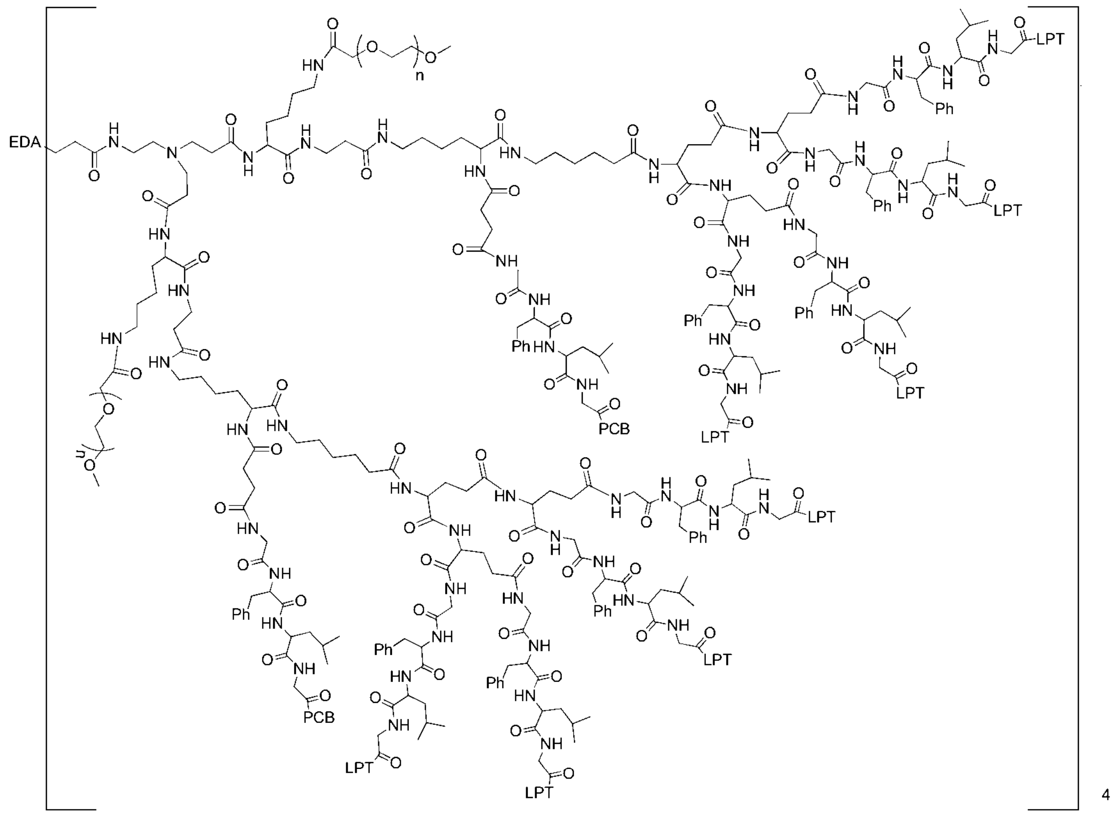 each polyethylene glycol fragment has a number-average molecular weight of 5k |

(continued)

| | |
|---|---|
| **59-169** | 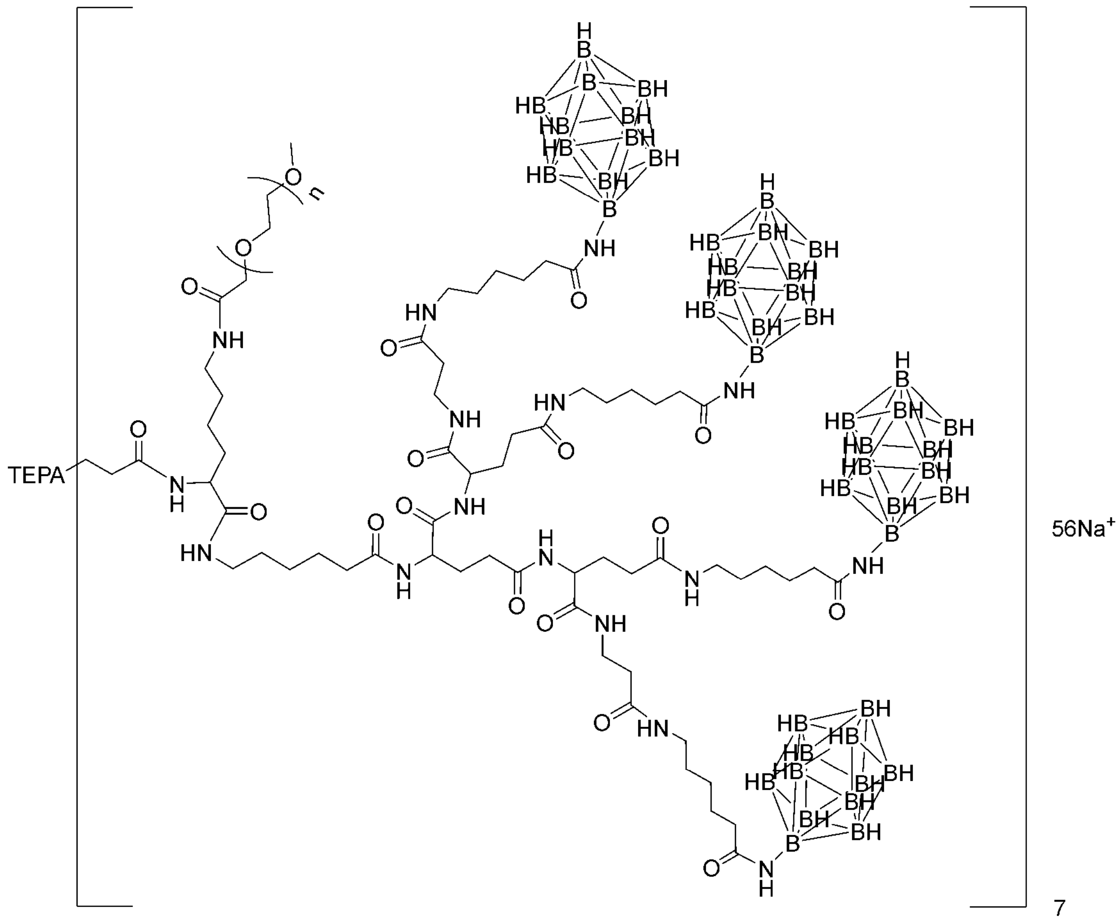<br>each polyethylene glycol fragment has a number-average molecular weight of 5k |

(continued)

| | |
|---|---|
| **67-237** | 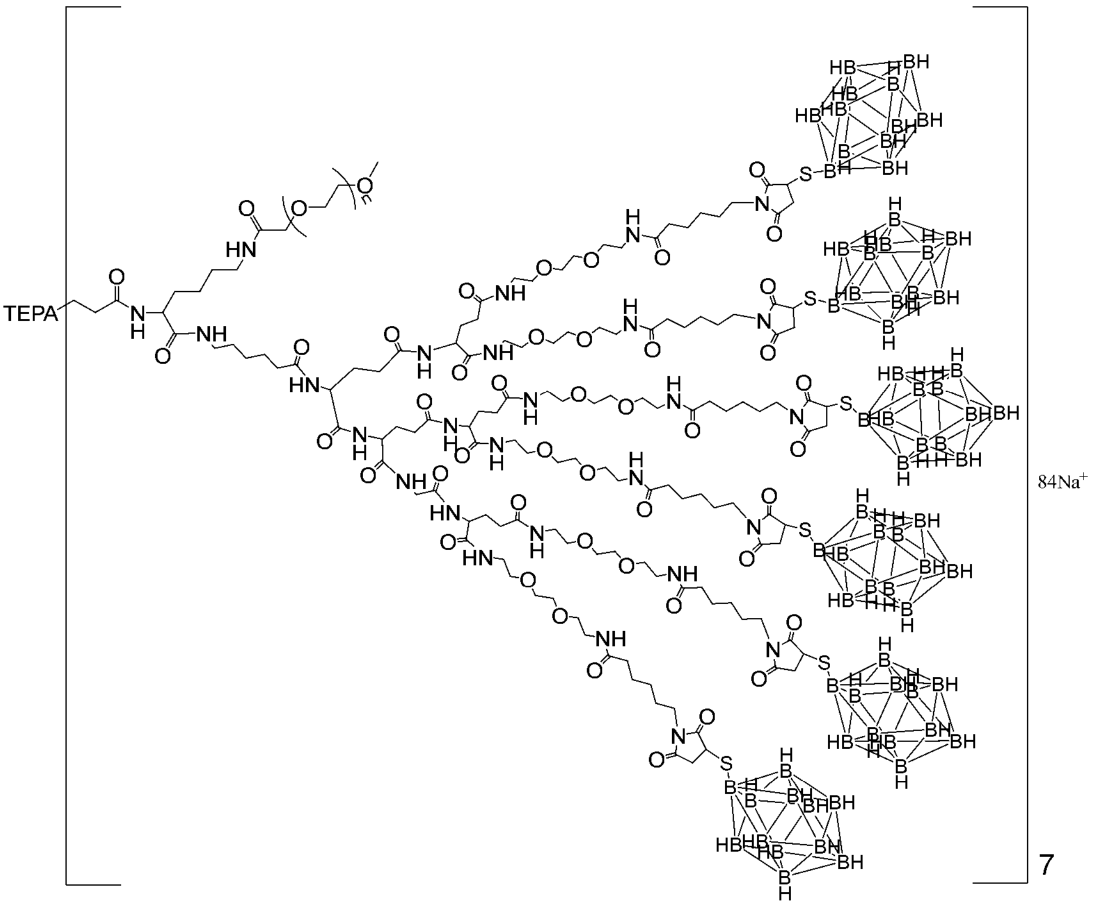<br>each polyethylene glycol fragment has a number-average molecular weight of 5k |
| **78-139** | 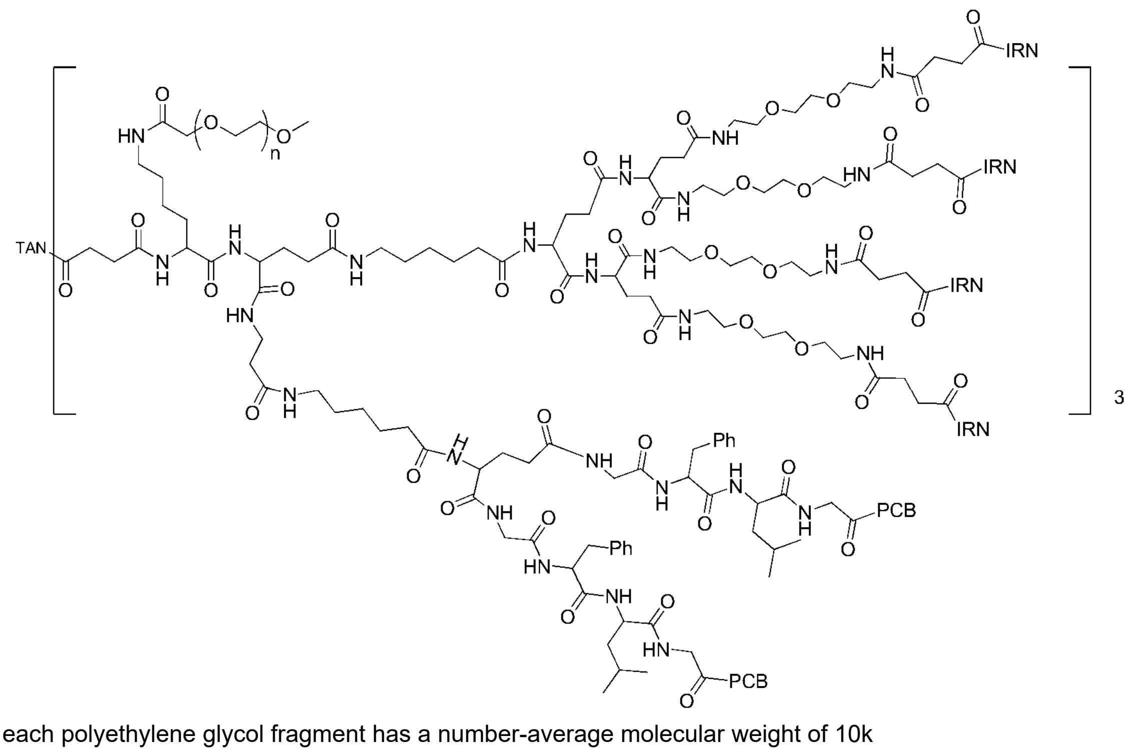<br>each polyethylene glycol fragment has a number-average molecular weight of 10k |

(continued)

| | |
|---|---|
| **82-178** | 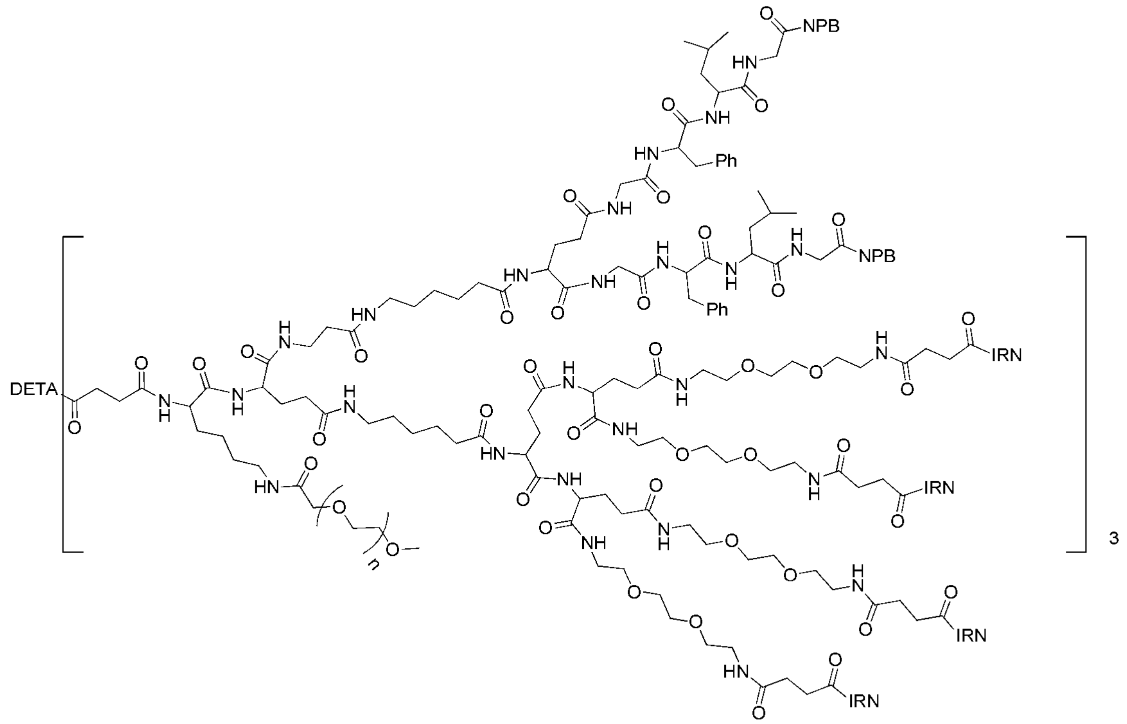<br>each polyethylene glycol fragment has a number-average molecular weight of 10k |
| **70-294** | 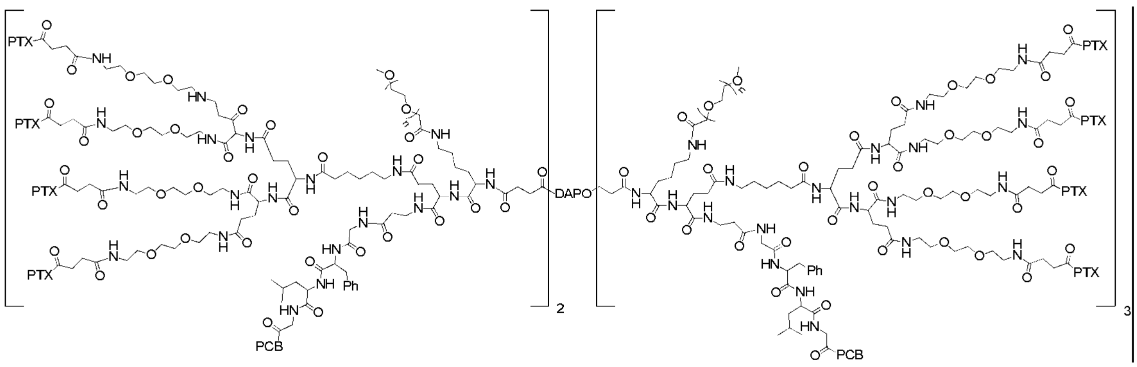<br>each polyethylene glycol fragment has a number-average molecular weight of 5k |

(continued)

| | |
|---|---|
| **81-176** | 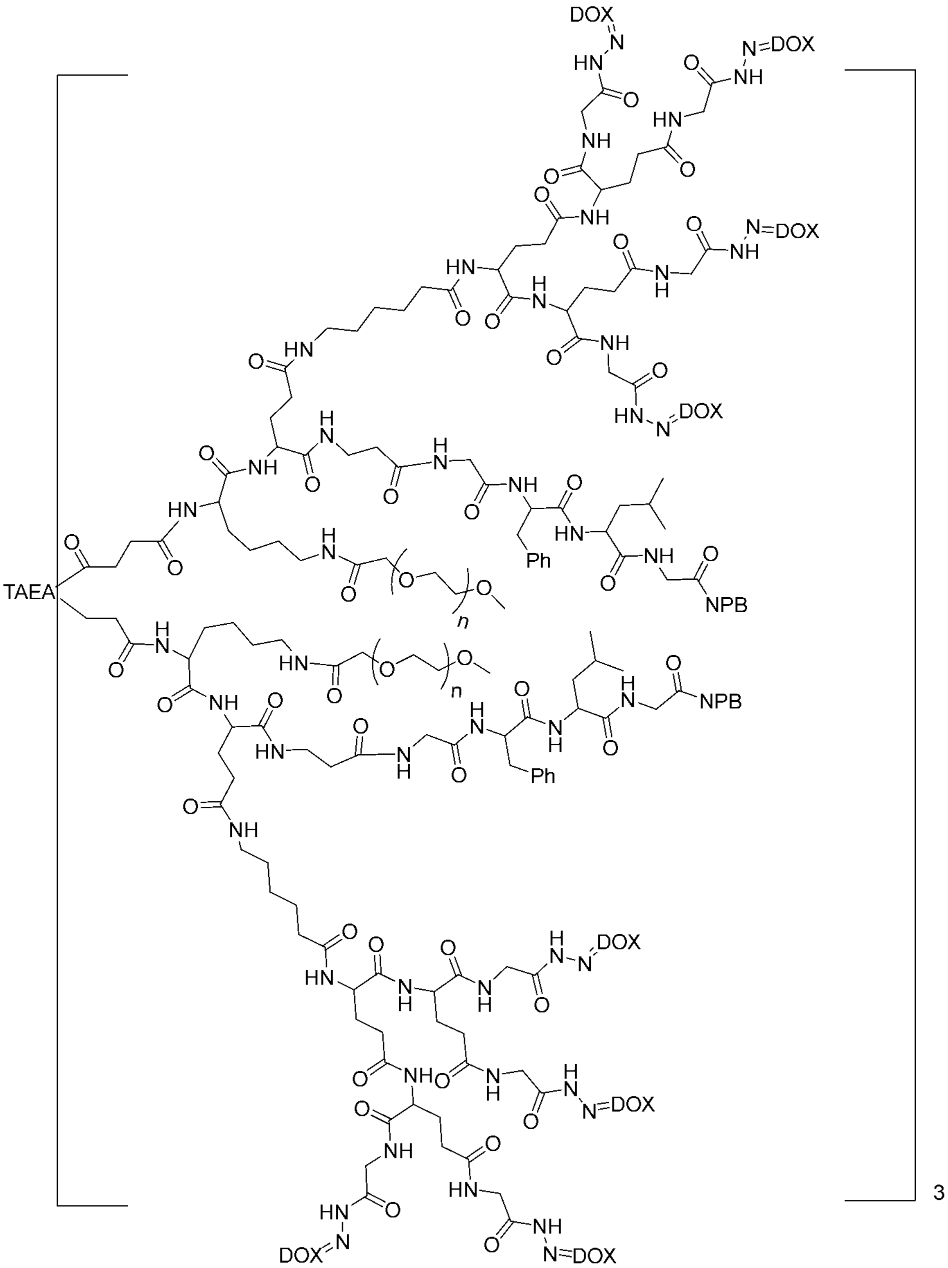 each polyethylene glycol fragment has a number-average molecular weight of 5k |

(continued)

| | |
|---|---|
| **85-71** | 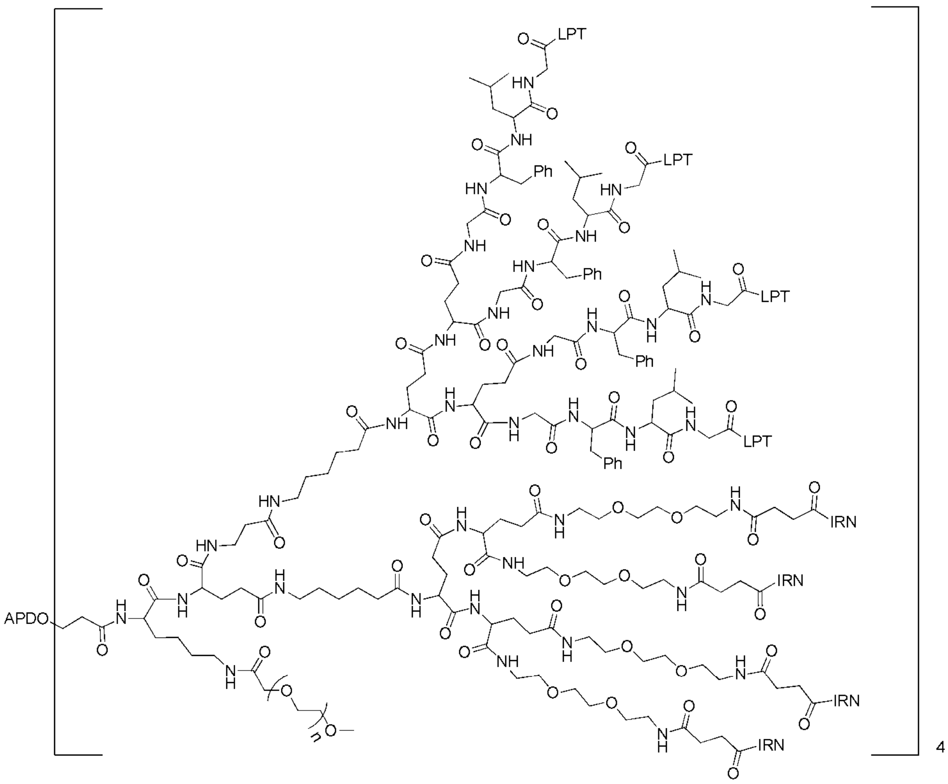<br>each polyethylene glycol fragment has a number-average molecular weight of 5k |
| **75-196** | 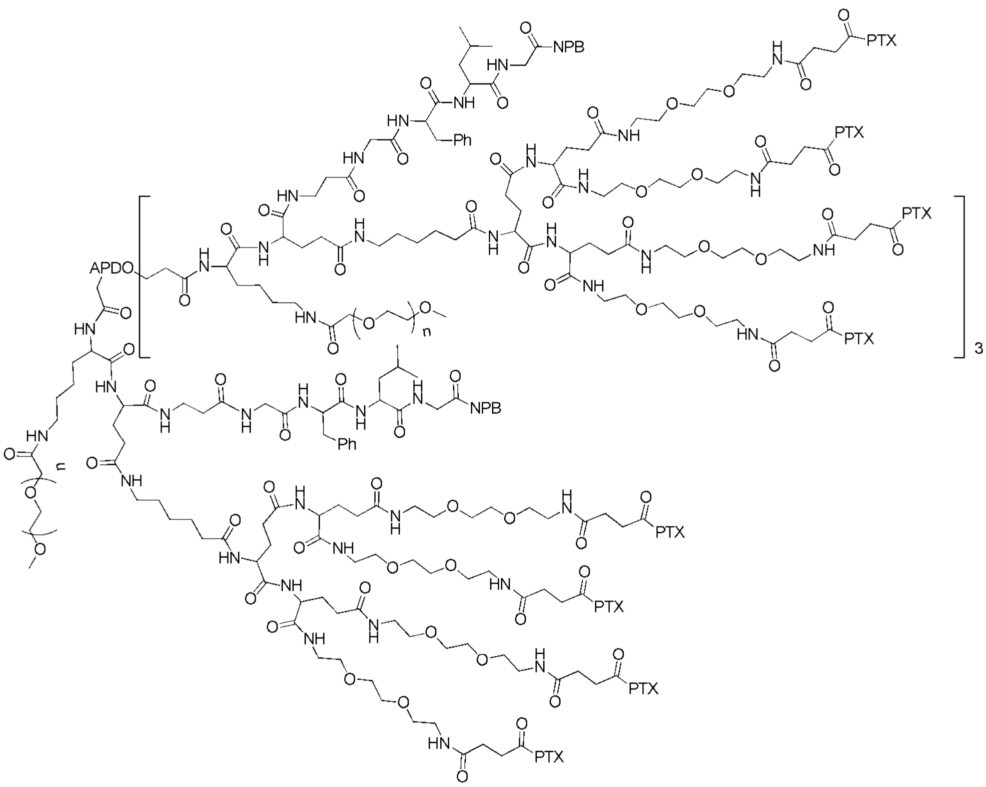<br>each polyethylene glycol fragment has a number-average molecular weight of 10k |

(continued)

| | |
|---|---|
| **86-2** | 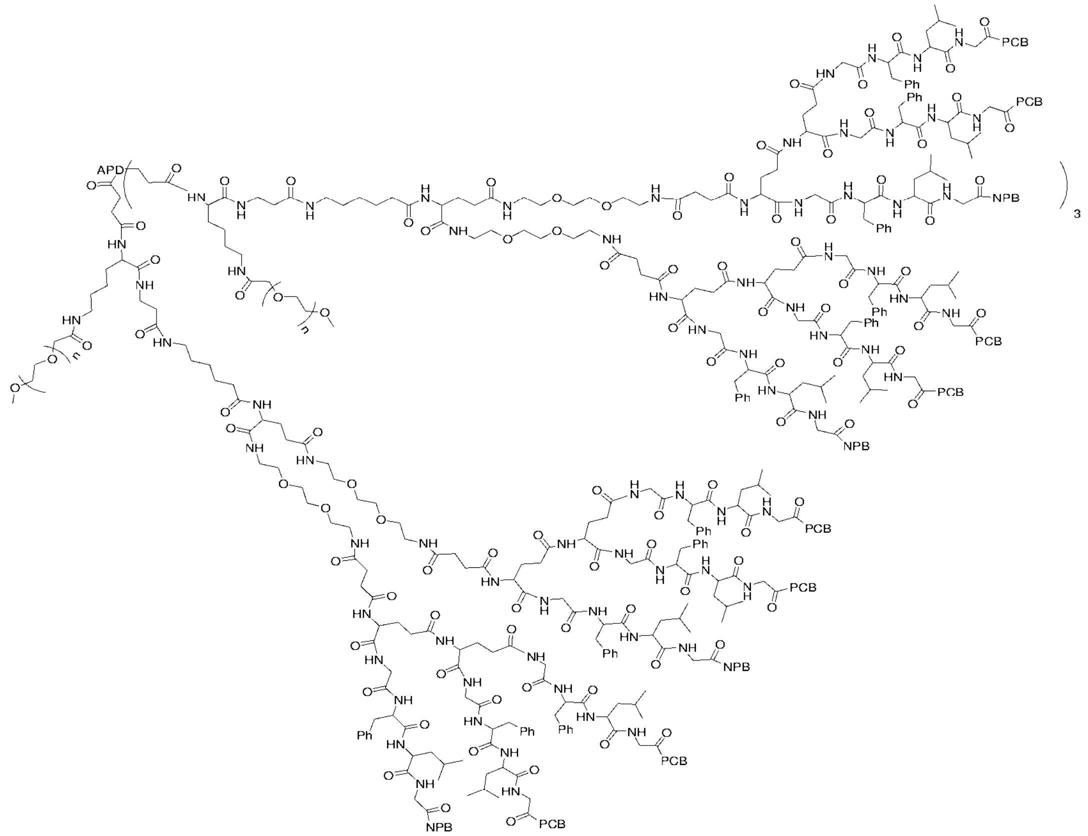 each polyethylene glycol fragment has a number-average molecular weight of 10k |
| **84-106** | 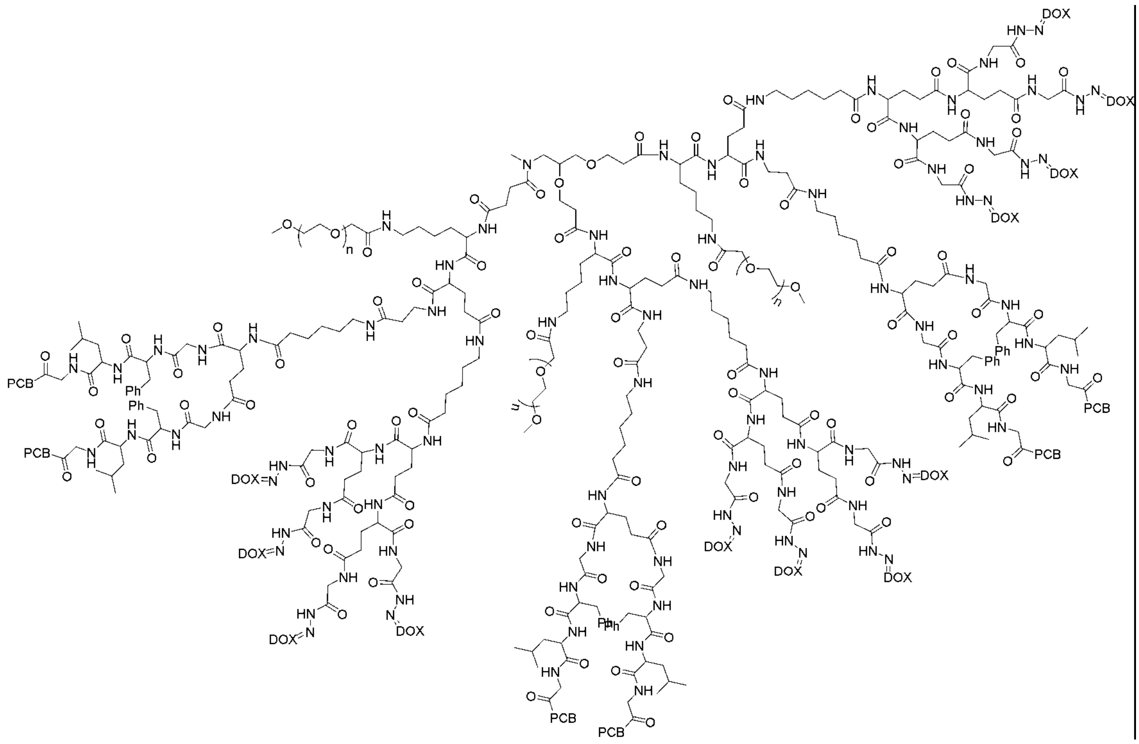 each polyethylene glycol fragment has a number-average molecular weight of 10k |

(continued)

| | |
|---|---|
| **76-207** | 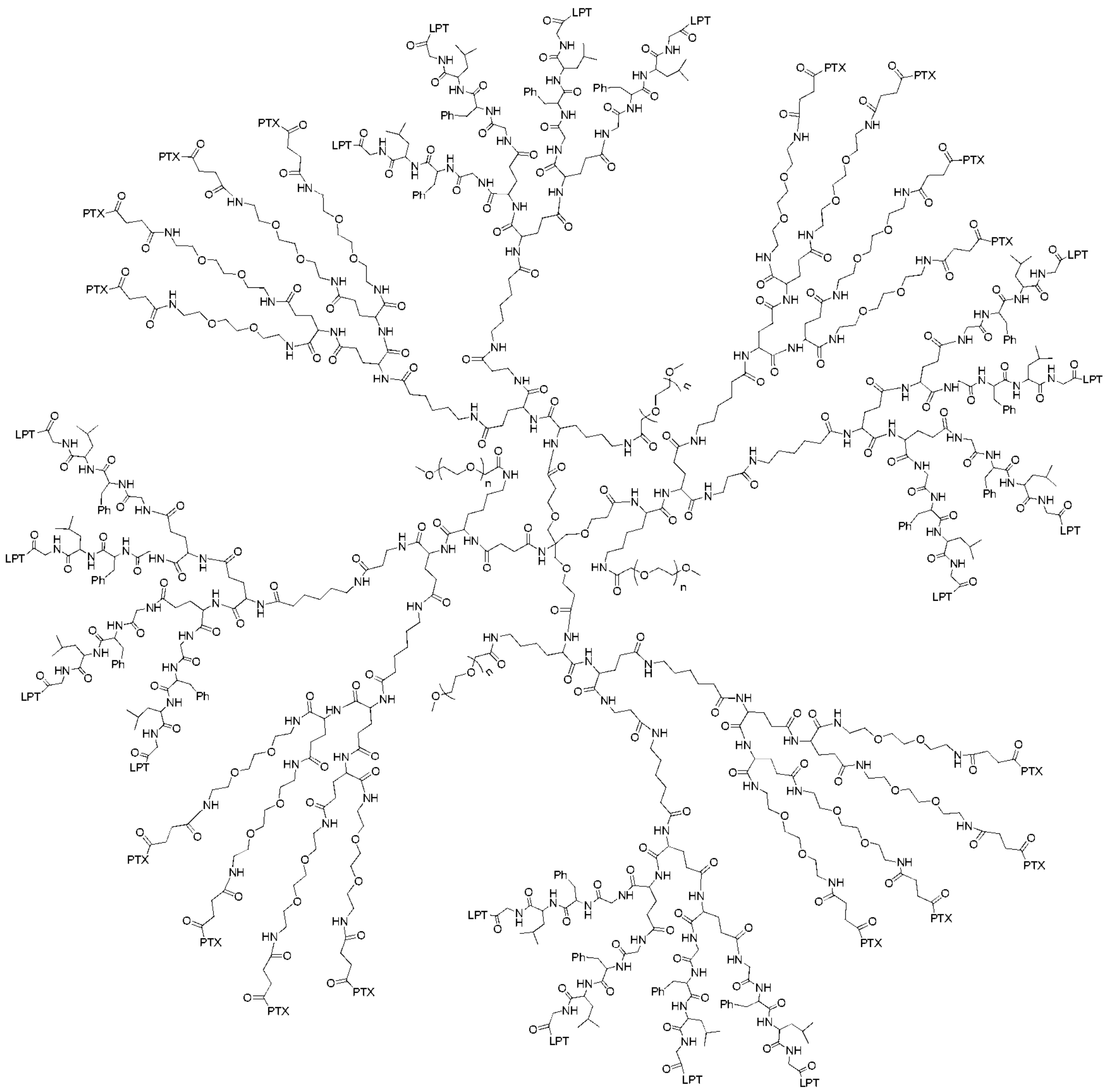<br>each polyethylene glycol fragment has a number-average molecular weight of 10k |

(continued)

| | |
|---|---|
| **82-152** | 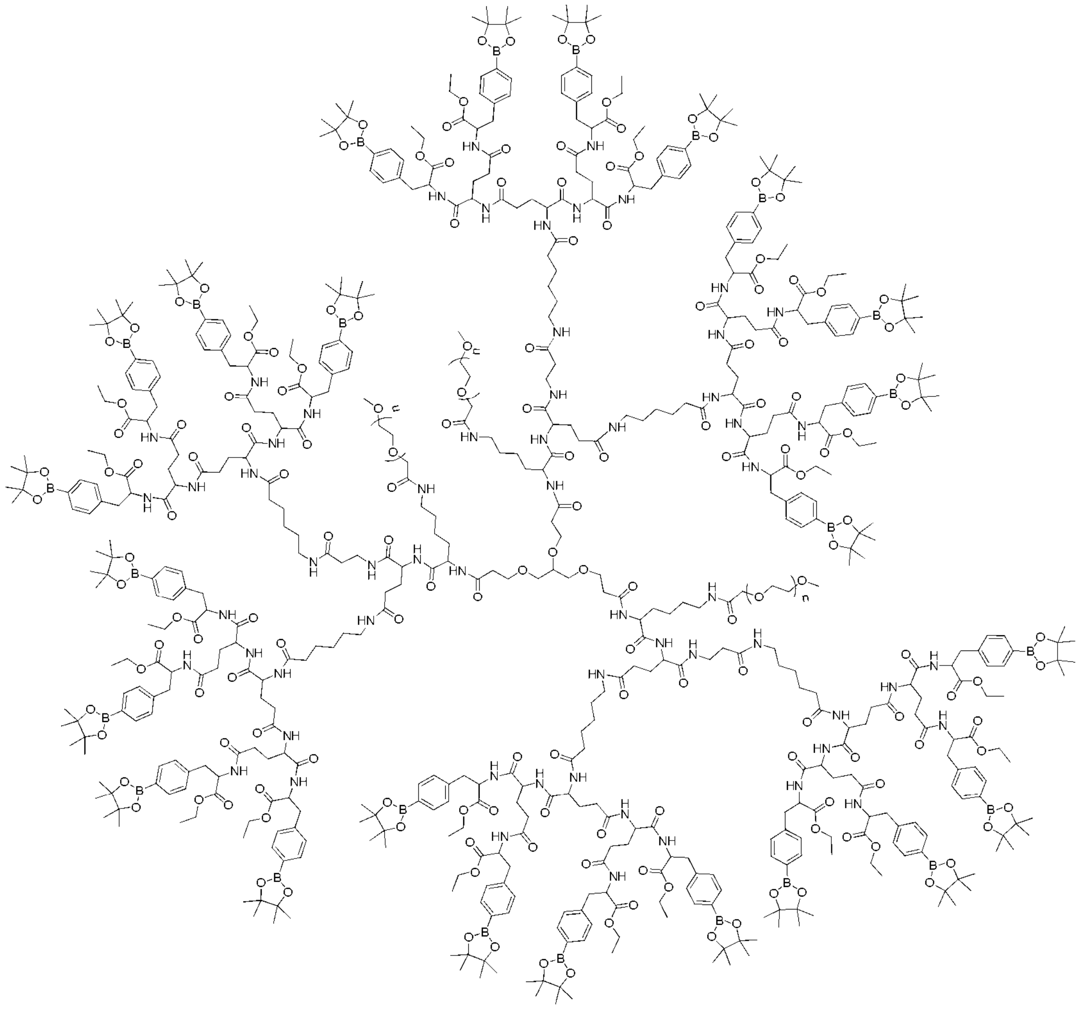<br>each polyethylene glycol fragment has a number-average molecular weight of 10k |

(continued)

| | |
|---|---|
| **82-132** | 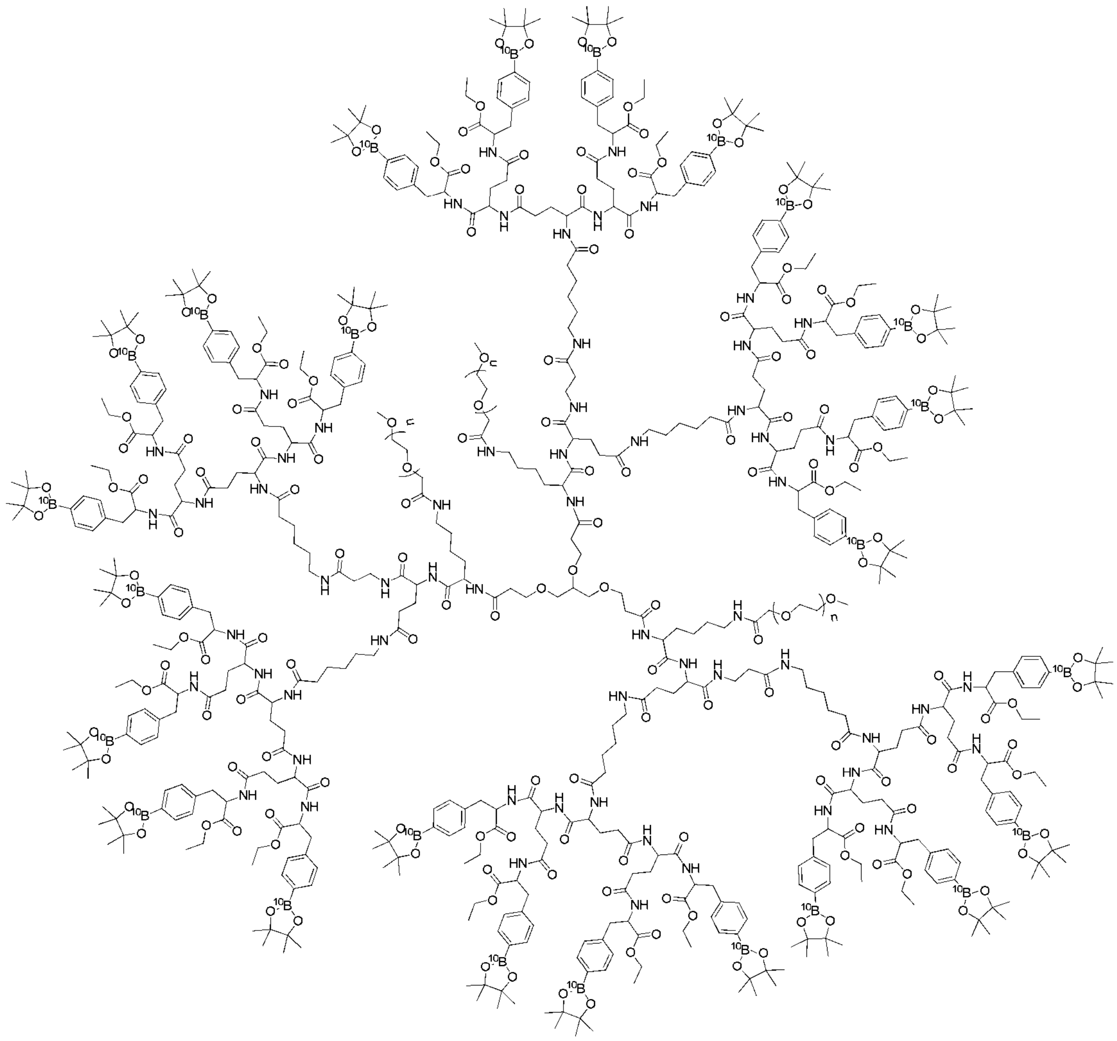 |
| | each polyethylene glycol fragment has a number-average molecular weight of 10k |

(continued)

| | |
|---|---|
| **82-208** | 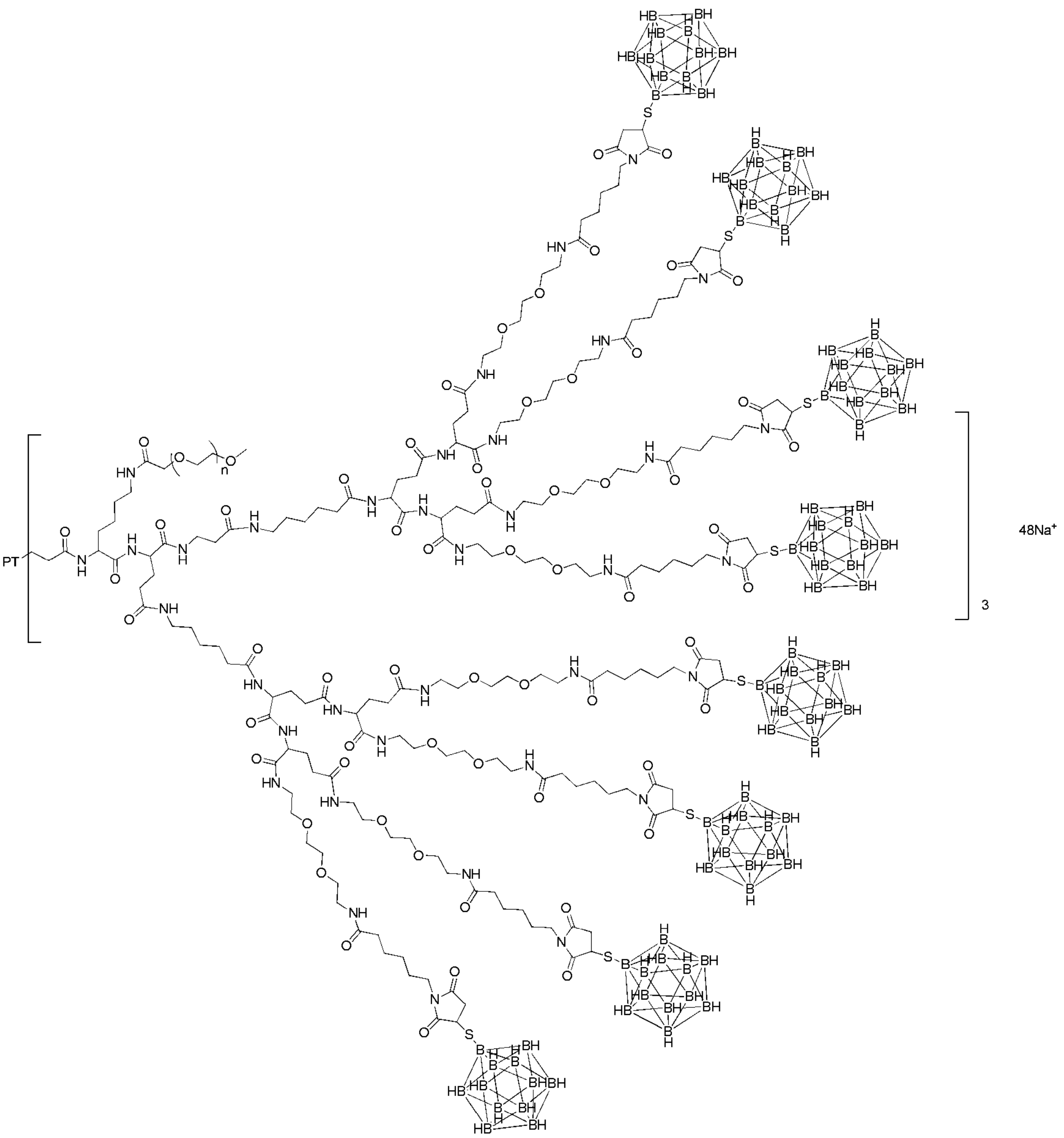<br>each polyethylene glycol fragment has a number-average molecular weight of 10k |

(continued)

| | |
|---|---|
| **82-207** | 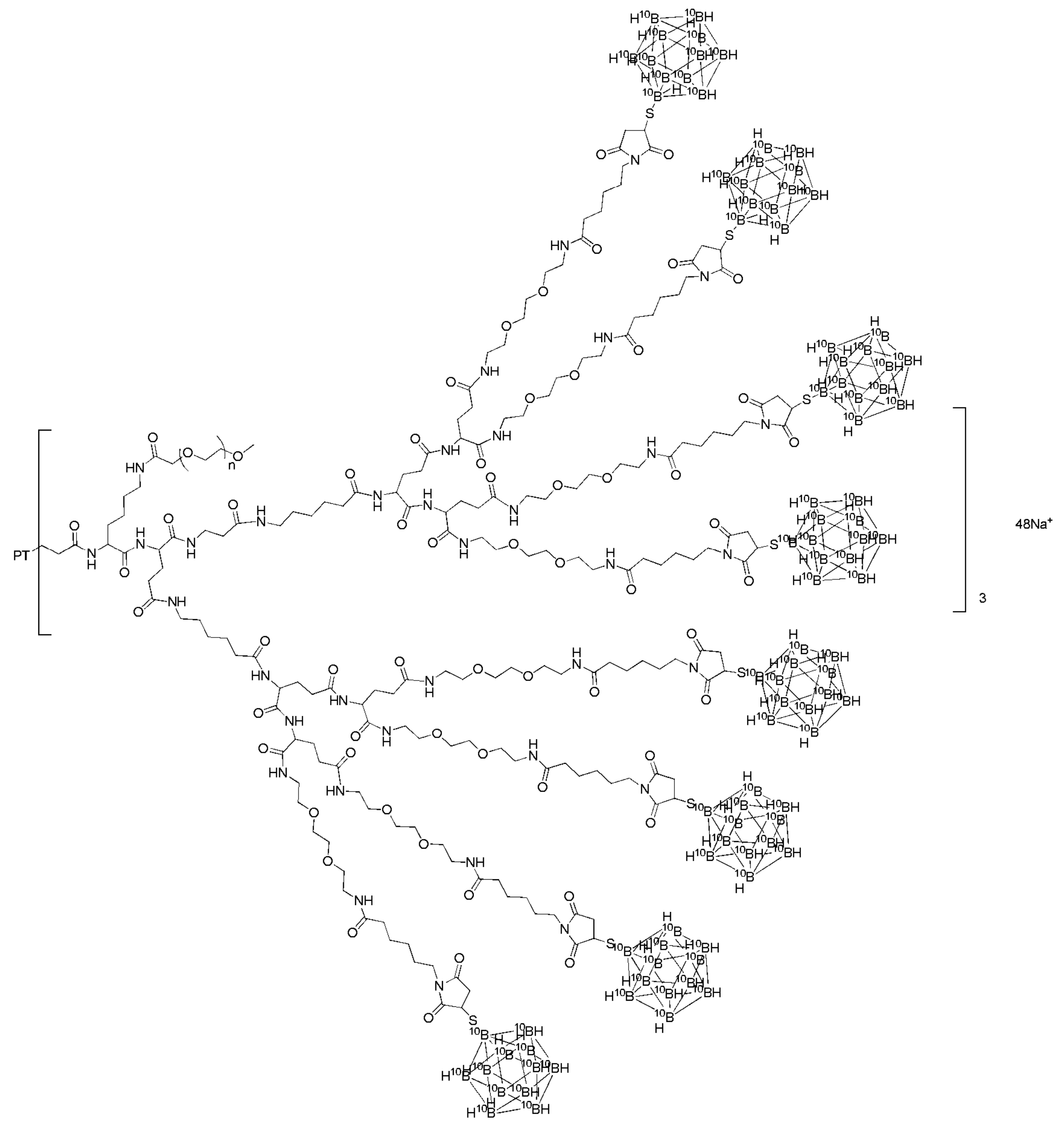<br>each polyethylene glycol fragment has a number-average molecular weight of 10k |

(continued)

| | |
|---|---|
| **78-127** | 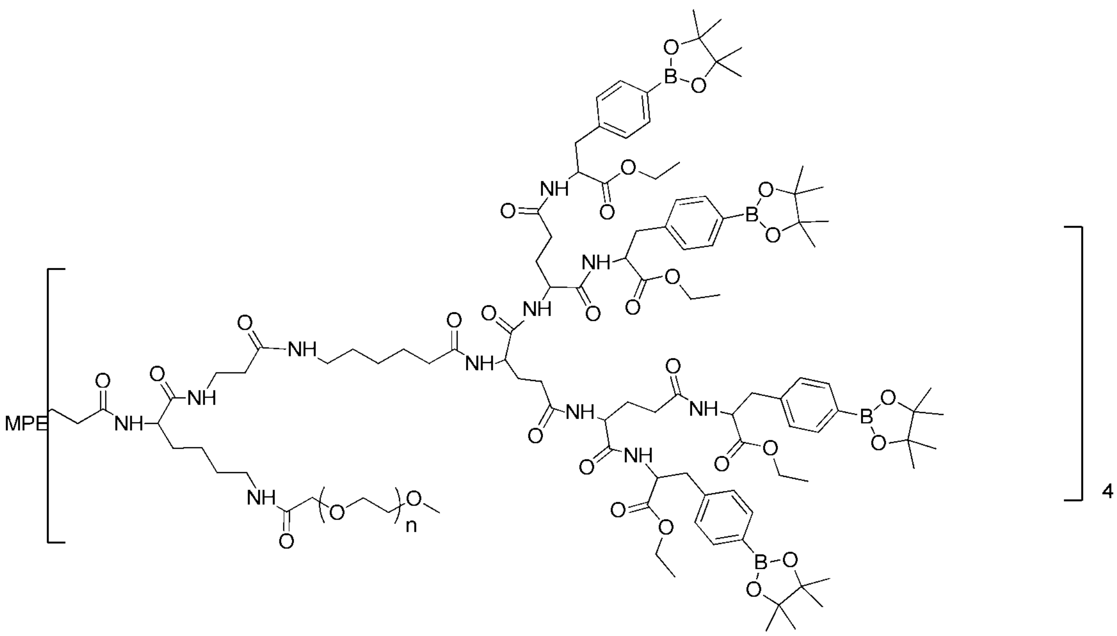 each polyethylene glycol fragment has a number-average molecular weight of 10k |
| **78-89** | 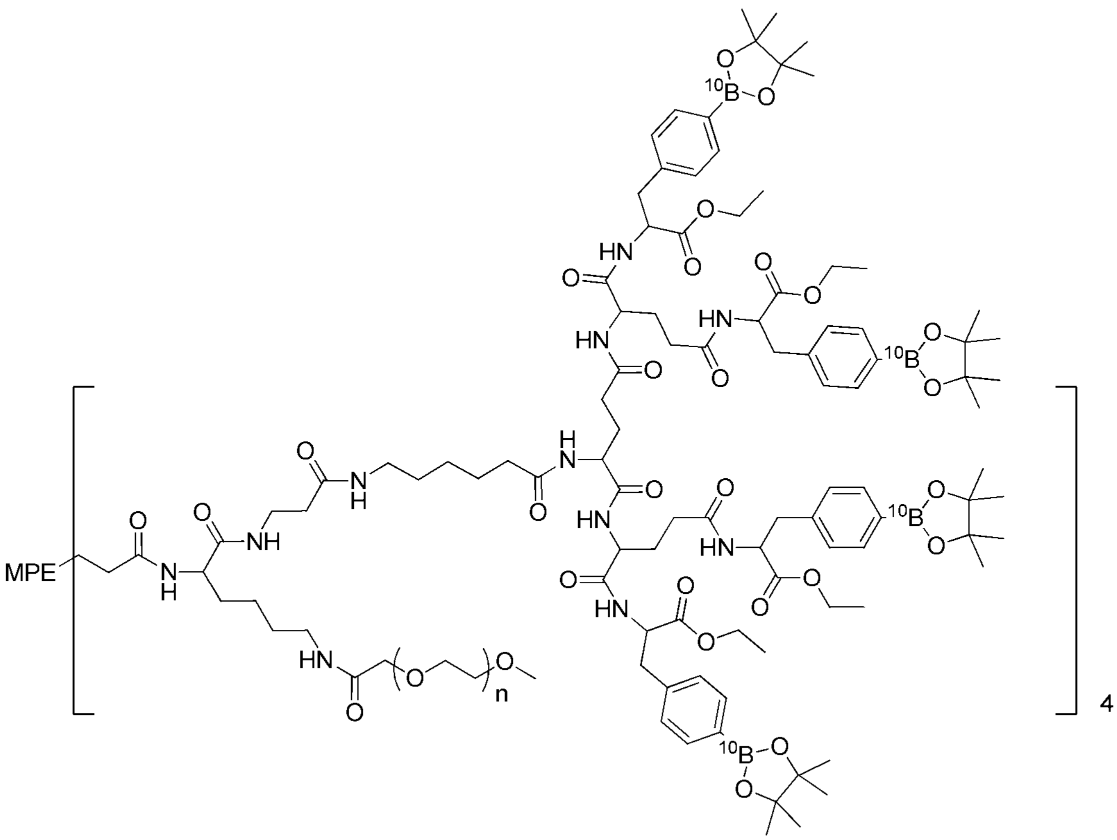 each polyethylene glycol fragment has a number-average molecular weight of 10k |

(continued)

| | |
|---|---|
| **78-152** | 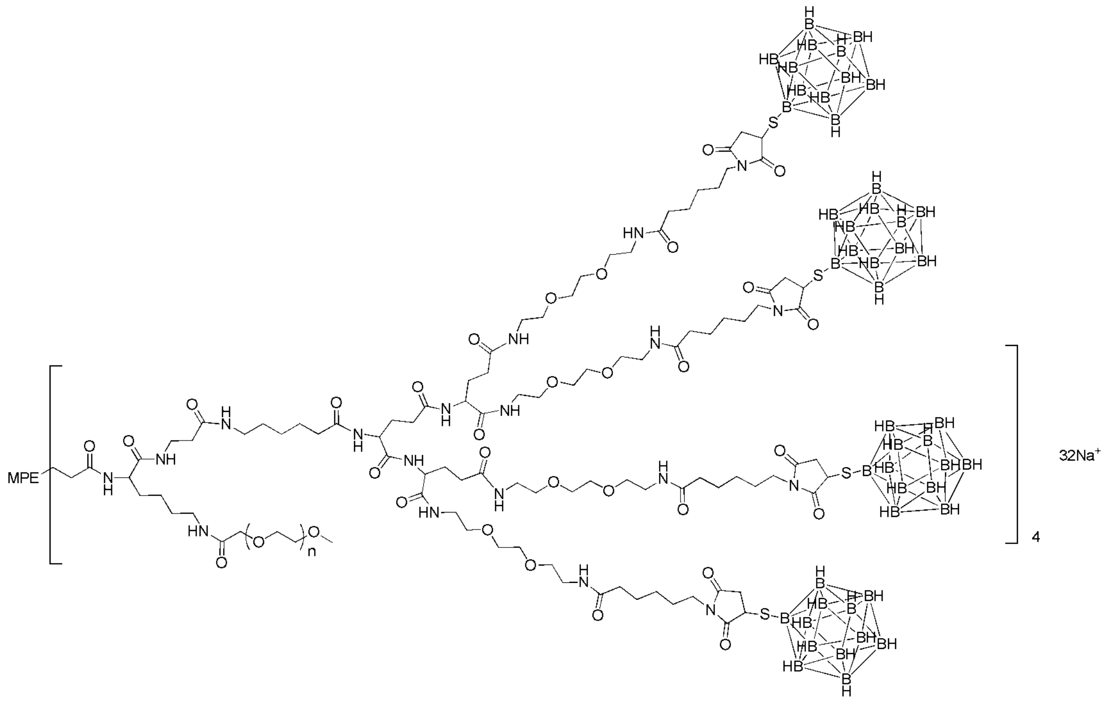 each polyethylene glycol fragment has a number-average molecular weight of 10k |
| **78-151** | 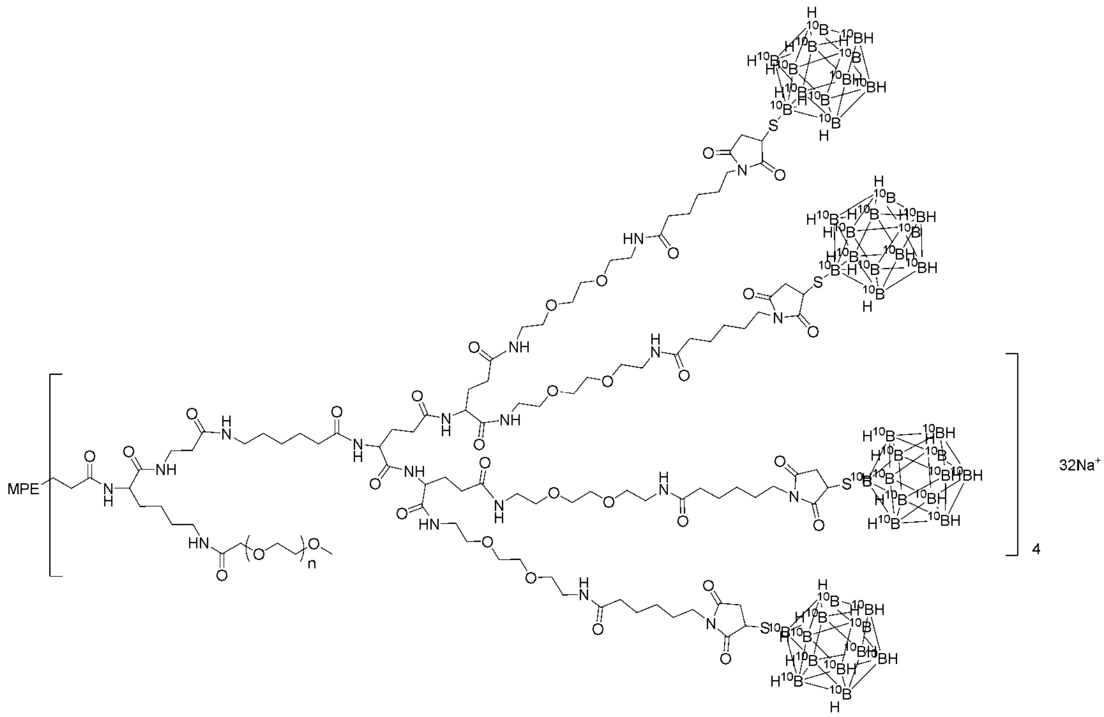 each polyethylene glycol fragment has a number-average molecular weight of 10k |

(continued)

| | |
|---|---|
| **72-183** | 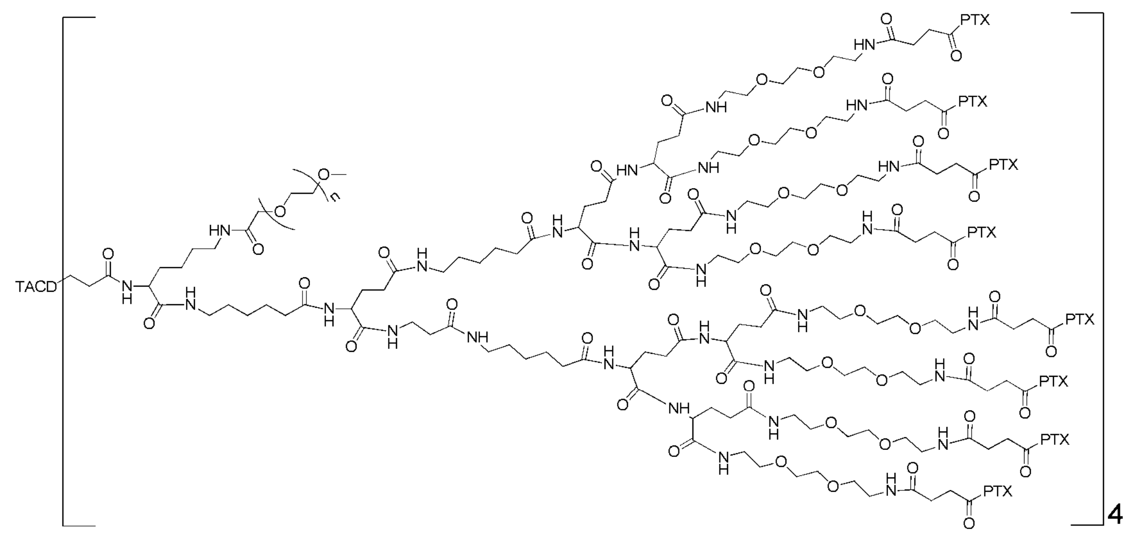 each polyethylene glycol fragment has a number-average molecular weight of 10k |

In another aspect, the present invention provides a compound represented by formula II or a pharmaceutically acceptable salt thereof, wherein:

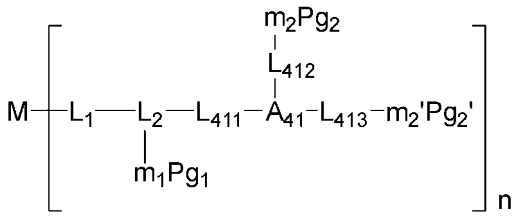

II

wherein, each of $Pg_1$, $Pg_2$, and $Pg_2'$ is independently hydrogen or selected from the group consisting of amino protecting groups and carboxyl protecting groups; the amino protecting group is selected from the group consisting of alkoxycarbonyl-based protecting group, such as Boc, Fmoc, Cbz, or Teoc; and the carboxyl protecting group is selected from the group consisting of ester-based protecting group, such as methyl ester, ethyl ester, tert-butyl ester, allyl ester, or benzyl ester;

each of $m_1$, $m_2$, and $m_2'$ is independently selected from the group consisting of 1, 2, 3, 4, 5, 6, 7, 8, 9, 10, 11, 12, 13, 14, 15, 16, 17, 18, 19, and 20;

the remaining groups are as defined in any of the preceding items.

In some embodiments, $Pg_1$ is hydrogen or an amino protecting group, such as Boc, Fmoc, or Cbz.

In some embodiments, $Pg_2$ and $Pg_2'$ are the same. In some embodiments, $Pg_2$ and $Pg_2'$ are different. In some embodiments, $Pg_2$ is hydrogen, and $Pg_2'$ is an amino protecting group or carboxyl protecting group. In some embodiments, $Pg_2'$ is hydrogen, and $Pg_2$ is an amino protecting group or carboxyl protecting group. In some embodiments, $Pg_2$ and $Pg_2'$ are both hydrogen.

In some embodiments, each $Pg_2$ is independently hydrogen, -OBn, or $O^tBu$; each $Pg_2'$ is independently hydrogen, -OBn, or $O^tBu$.

In some embodiments, each $Pg_2'$ is independently hydrogen, -OBn, or $O^tBu$; each $Pg_2$ is independently hydrogen, -OBn, or $O^tBu$.

In some embodiments, $L_{412}$ or $L_{413}$ is $-NH(CH_2)_2CO-$, and at least one $Pg_2$ or $Pg_3$ is -OBn.

In some embodiments, $A_{41}$ is selected from the group consisting of an amino acid residue or derivatives thereof, or a polypeptide fragment consisting of two or more amino acids or derivatives thereof, wherein the amino acid residue or

derivatives thereof, or the polypeptide fragment or derivatives thereof, provides 1, 2, 3, 4, 5, or 6 attachment sites; preferably, $A_{41}$ comprises a Glu or Asp residue;

$L_{412}$ is a bond;

$L_{413}$ is $-NH(CH_2)_2CO-$;

the amino acid (e.g., Glu or Asp) in $A_{41}$ directly connected to $L_{413}$ provides two attachment sites, wherein one of the attachment sites is connected to $L_{413}$ and the other is connected to $Pg_2$, and the $Pg_2$ is identical to the $Pg_2$' connected to $L_{413}$ (e.g., both are -OBn).

The compound represented by formula II allows for convenient and efficient removal of the protecting groups $Pg_2$ and/or $Pg_2$' (when they are amino protecting groups or carboxyl protecting groups) and largely avoids side reactions, which is highly advantageous for the synthesis of the target compound.

In some embodiments, the compound represented by formula II is selected from the group consisting of:

| | |
|---|---|
| **71-56** | 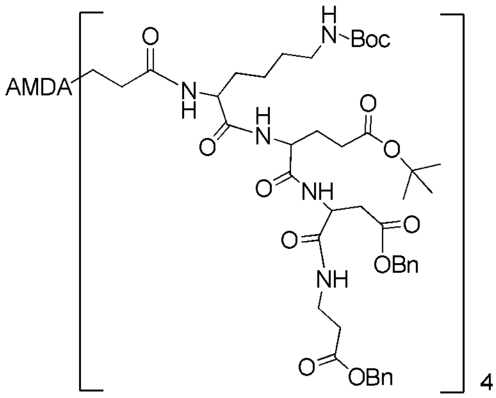 |
| **71-77** | 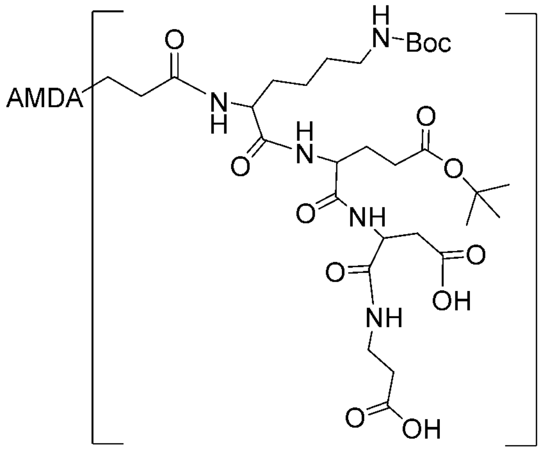 |
| **38-234** | 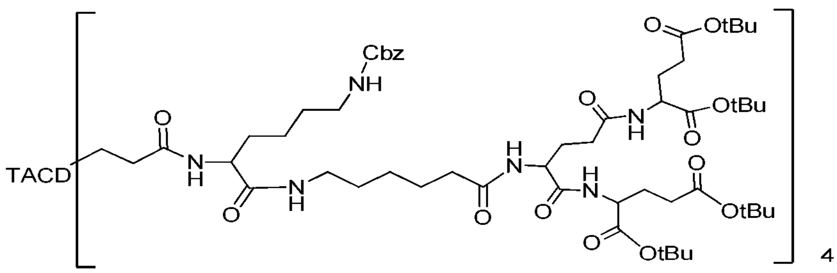 |

(continued)

| | |
|---|---|
| **38-258** | 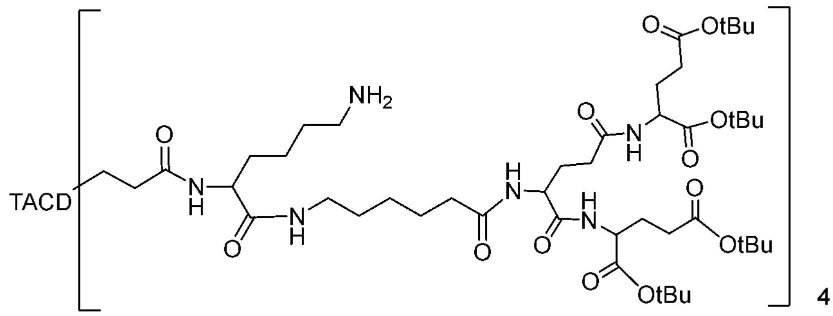 |
| **67-113** | 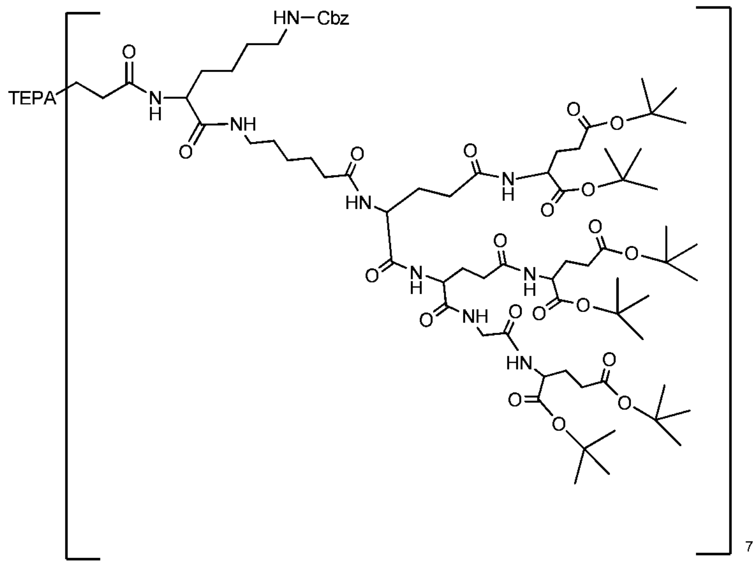 |
| **67-114** | 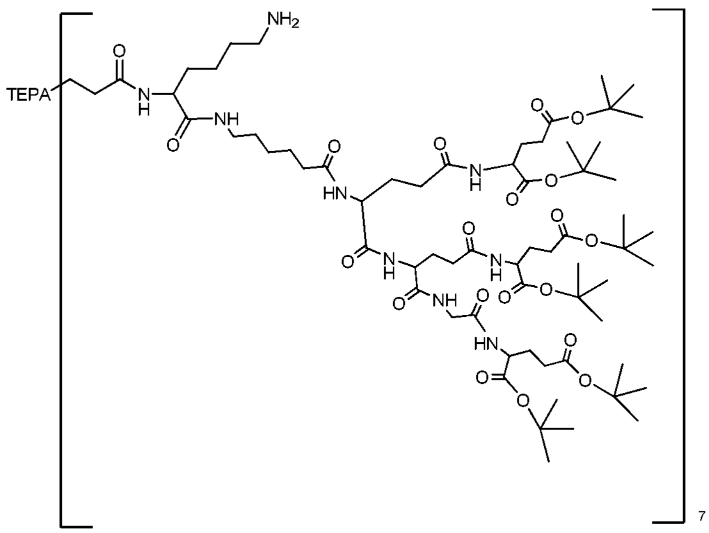 |

(continued)

| | |
|---|---|
| **81-22** | 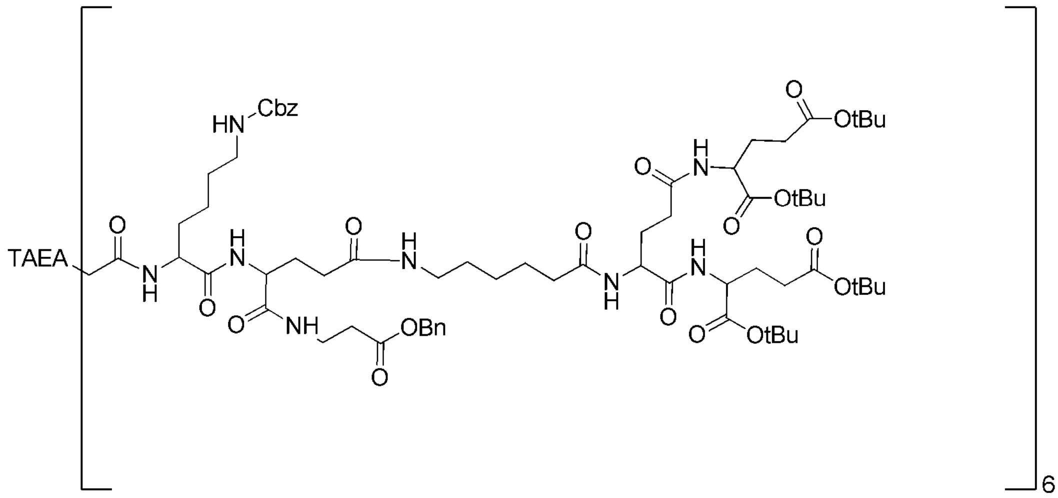 |
| **81-30** | 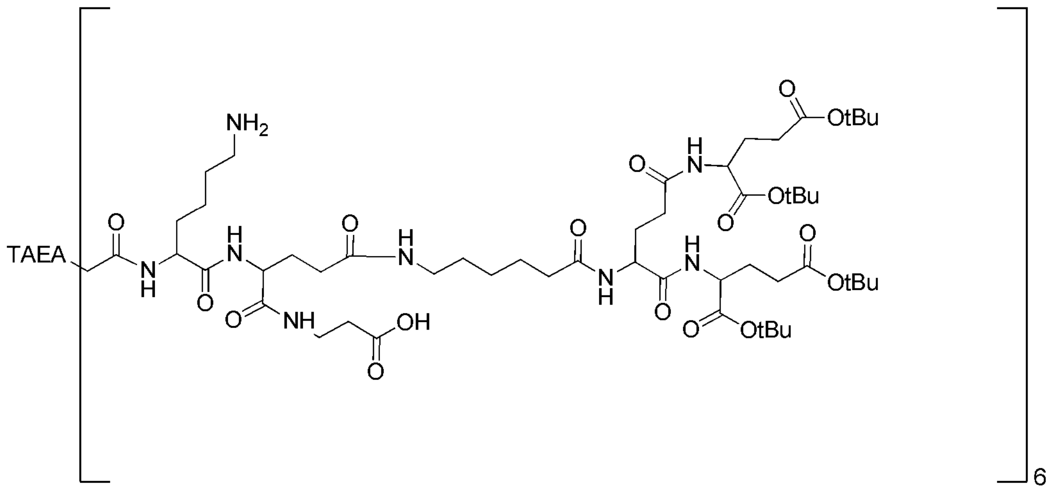 |
| **50-185** | 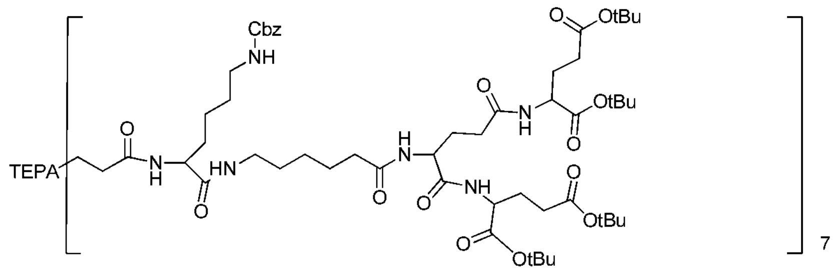 |
| **50-196** | 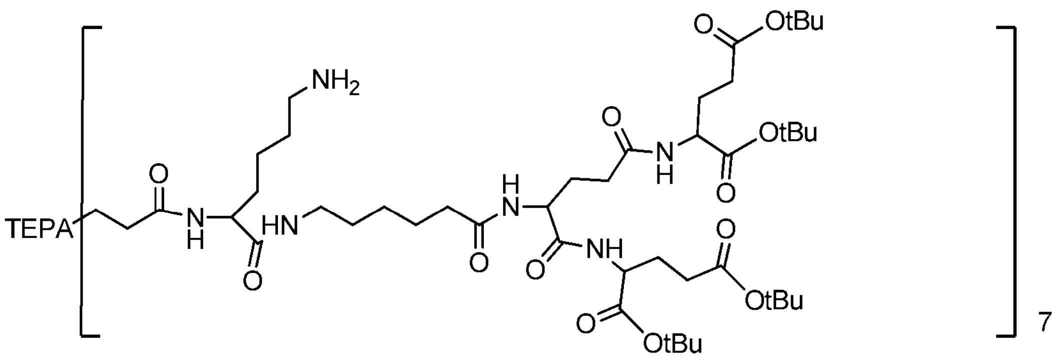 |

(continued)

| | |
|---|---|
| **69-54** | 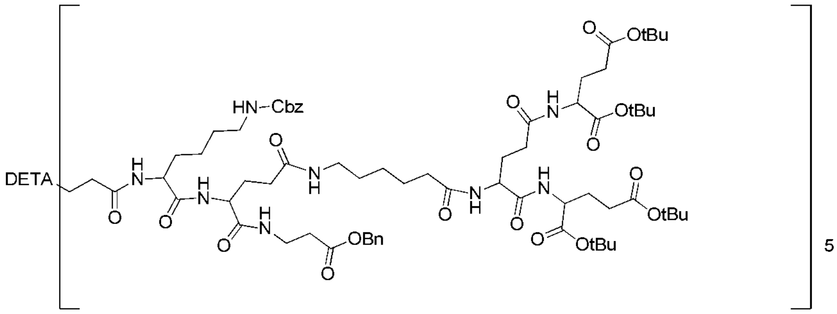 |
| **69-60** | 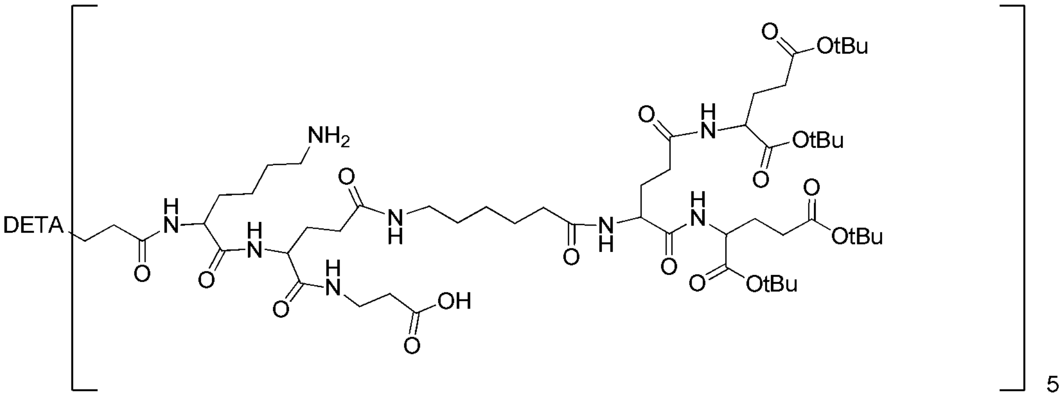 |
| **70-81** | 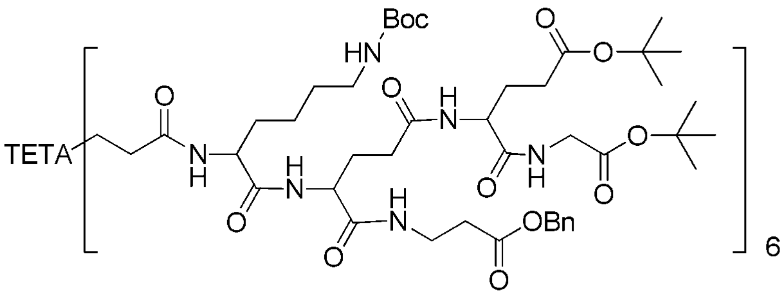 |
| **70-84** | 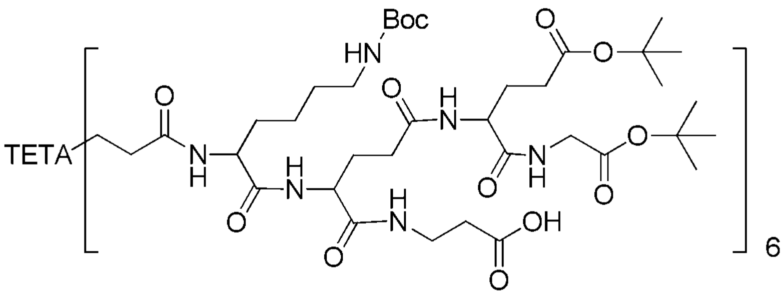 |

(continued)

| | |
|---|---|
| **68-127** | 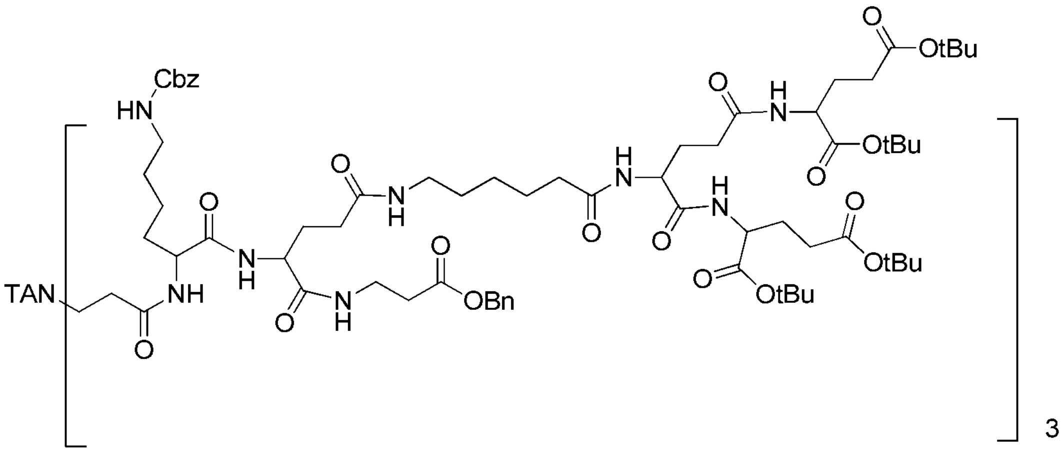 |
| **68-130** | 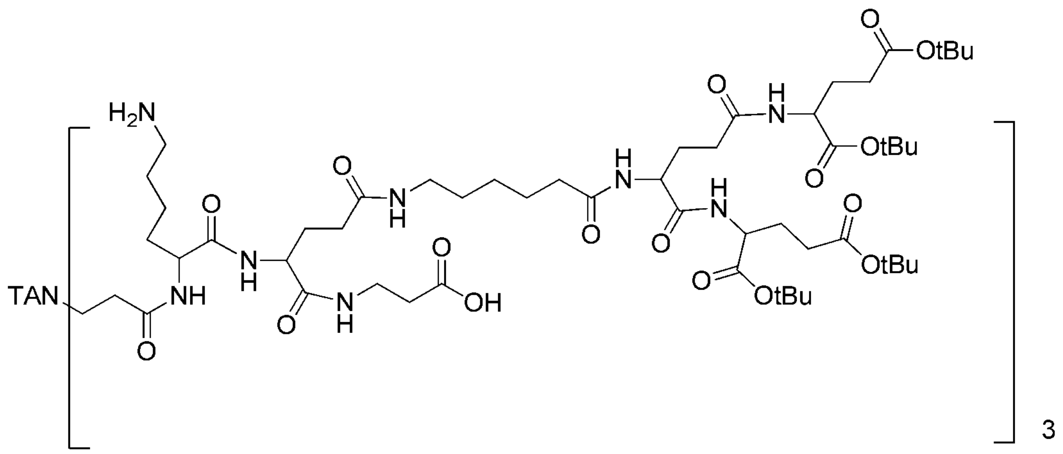<br>I-16 |
| **80-28** | 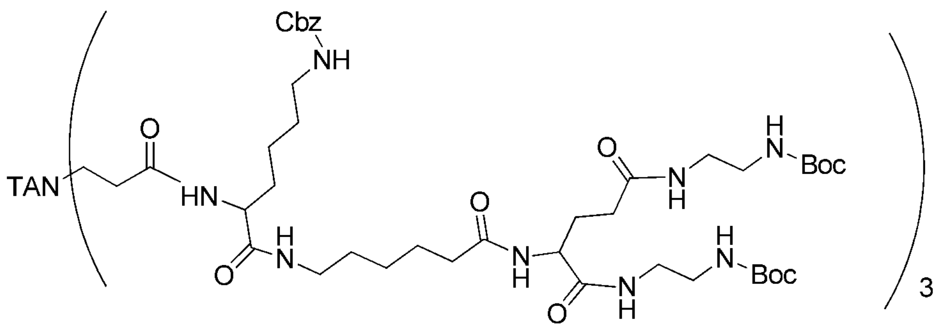 |
| **80-30** | 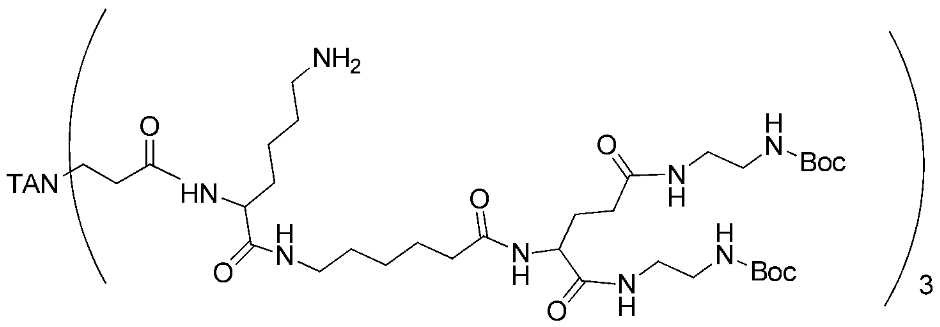 |

(continued)

| | |
|---|---|
| **58-212** | 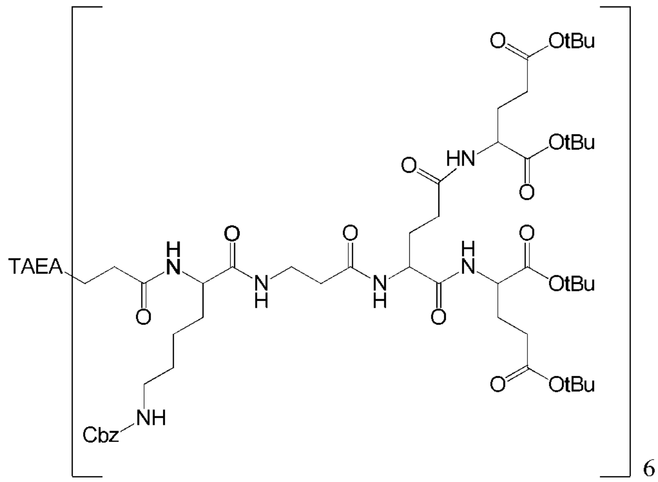 |
| **58-214** | 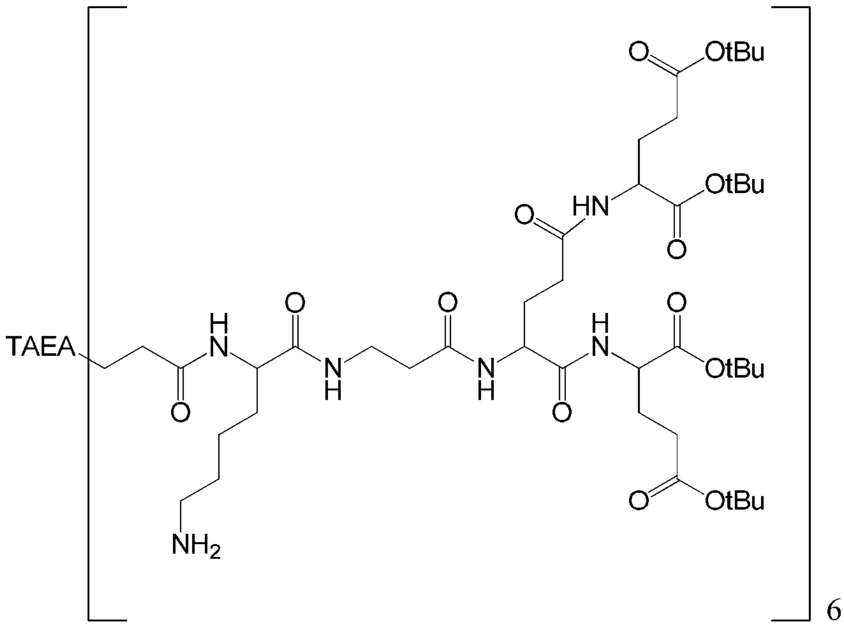 |

(continued)

| | |
|---|---|
| **73-5** | 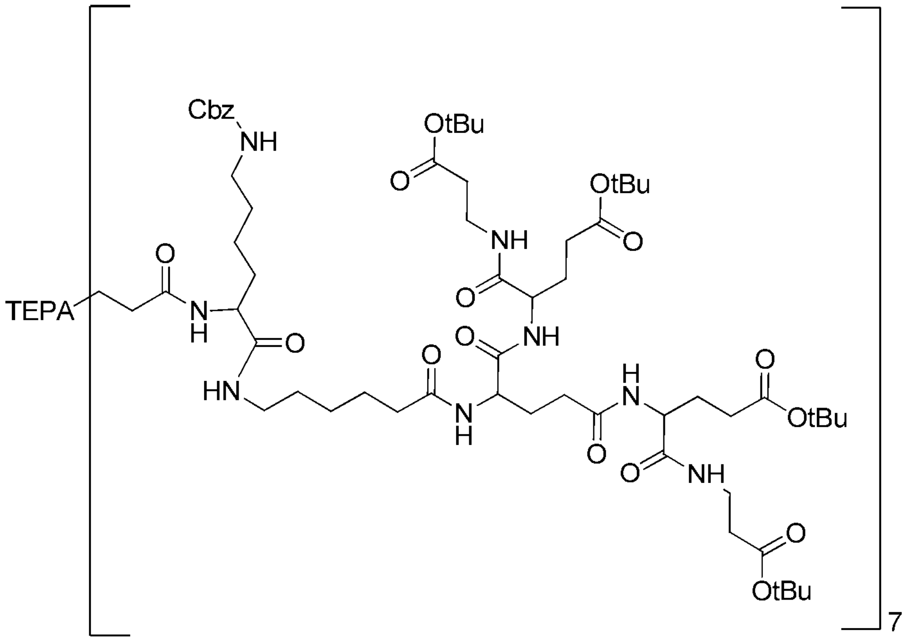 |
| **73-14** | 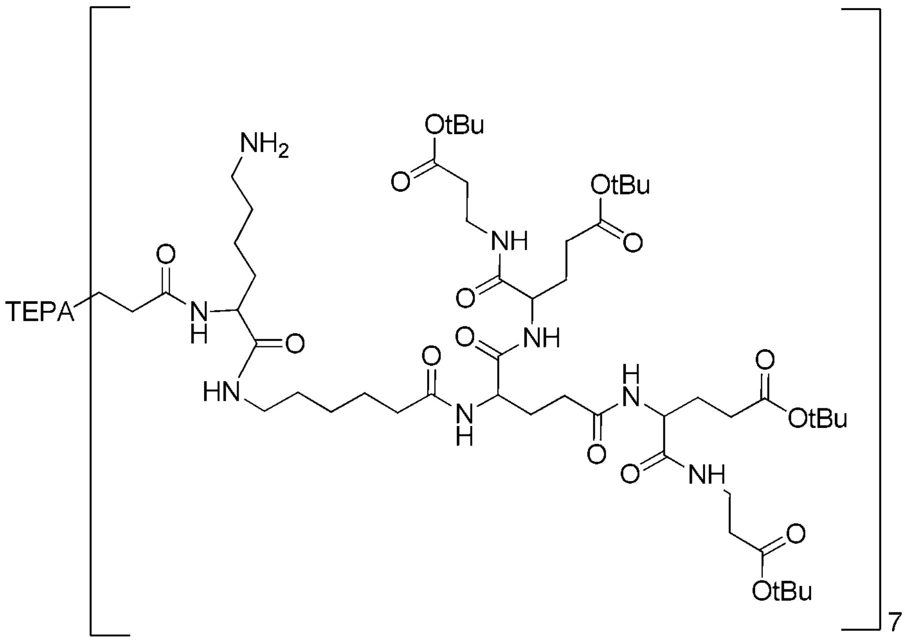 |
| **66-200** | 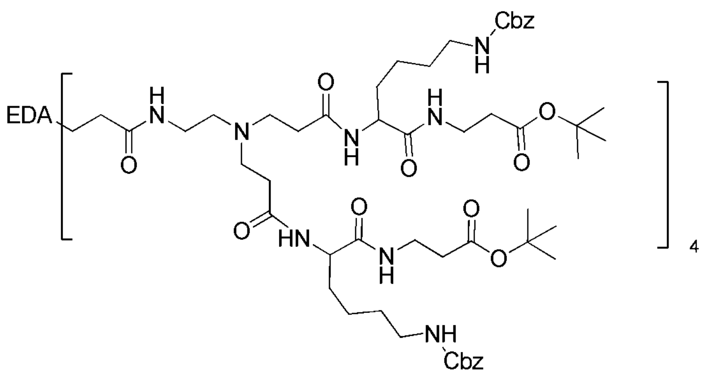 |

(continued)

| | |
|---|---|
| **66-201** | 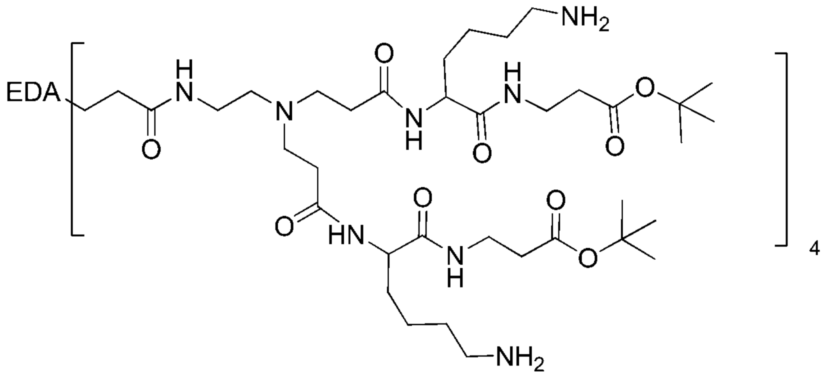 |
| **78-74** | 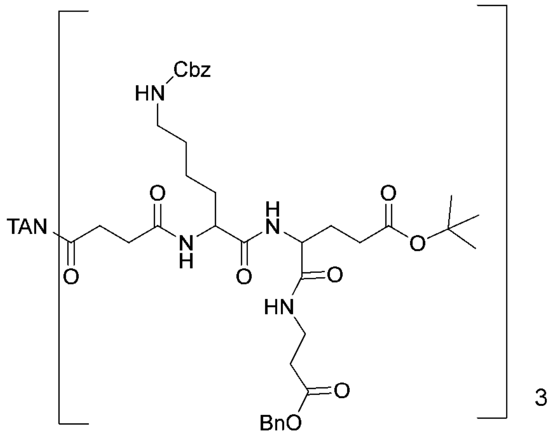 |
| **78-93** | 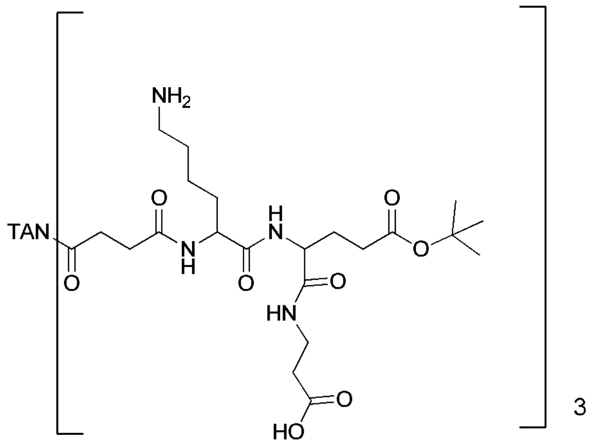 |

(continued)

| | |
|---|---|
| **82-139** | 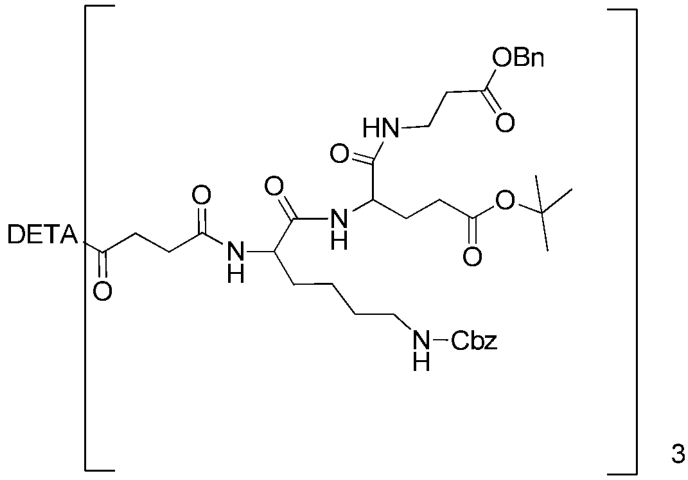 |
| **82-150** | 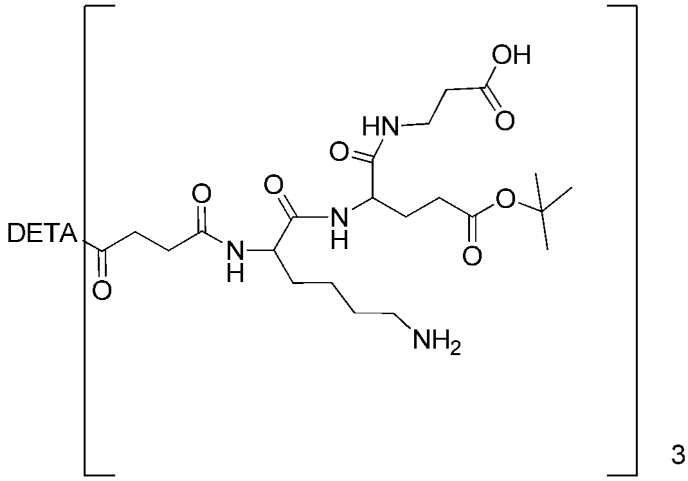 |
| **70-276** | 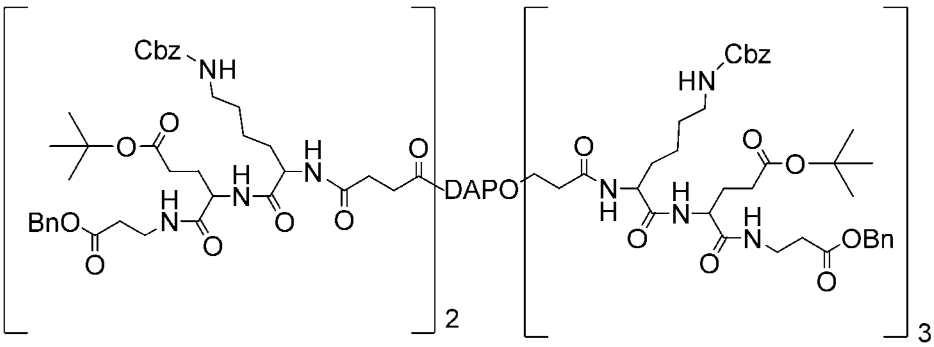 |
| **70-280** | 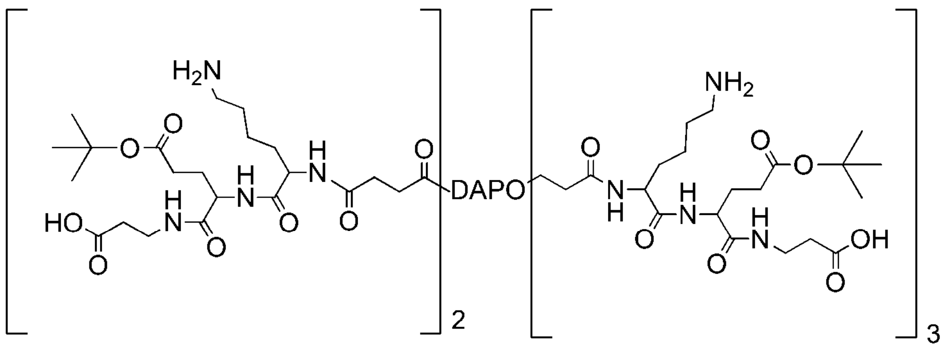 |

(continued)

| | |
|---|---|
| **81-149** | 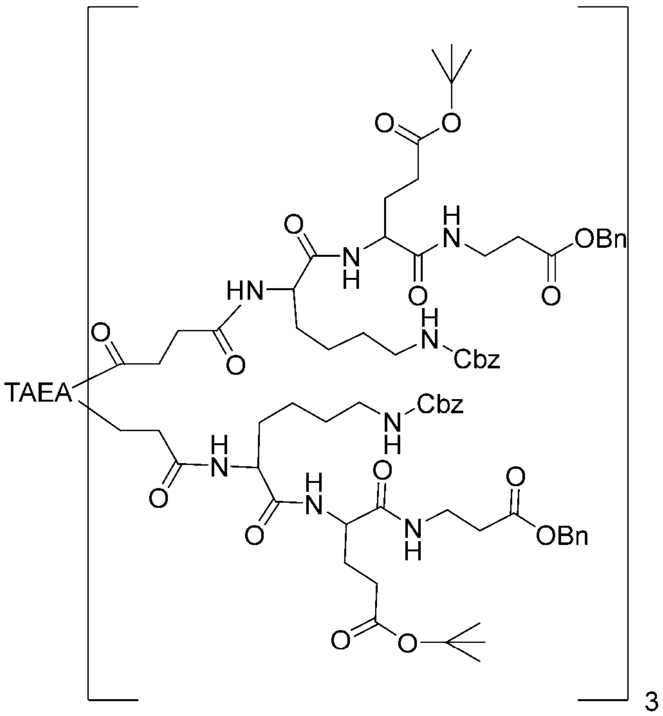 |
| **81-152** | 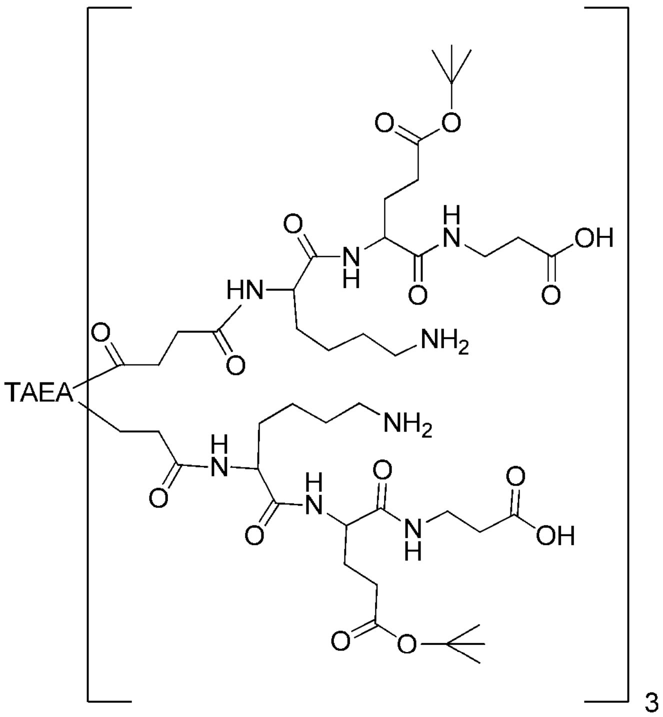 |

(continued)

| | |
|---|---|
| **71-281** | 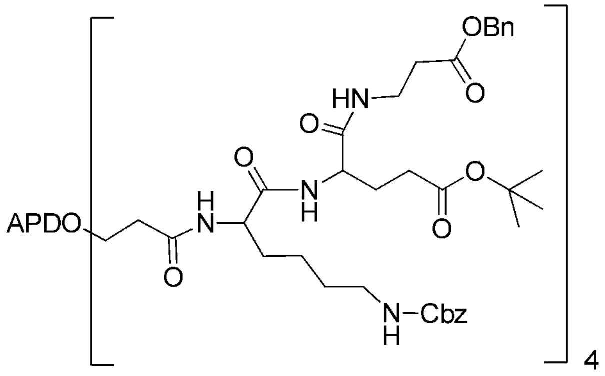 |
| **85-22** | 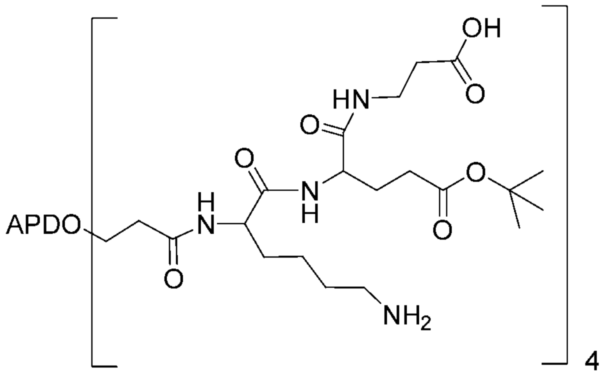 |
| **75-167** | 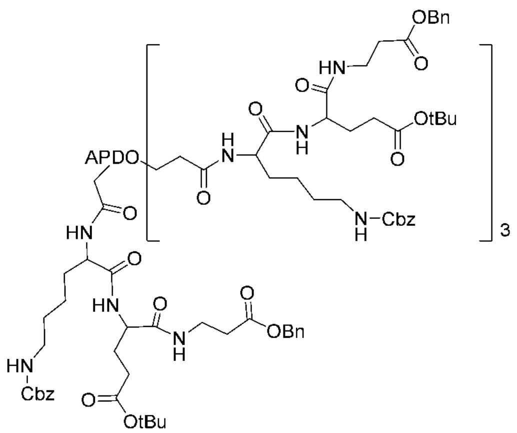 |
| **75-170** | 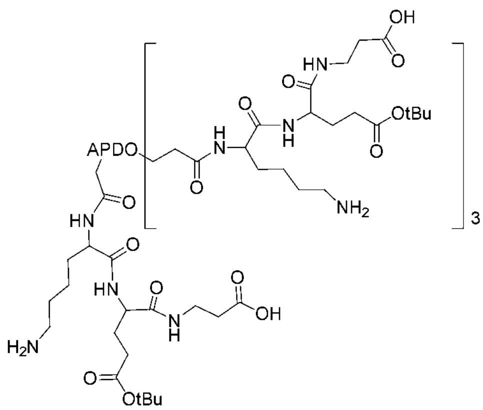 |

(continued)

| | |
|---|---|
| **69-260** | 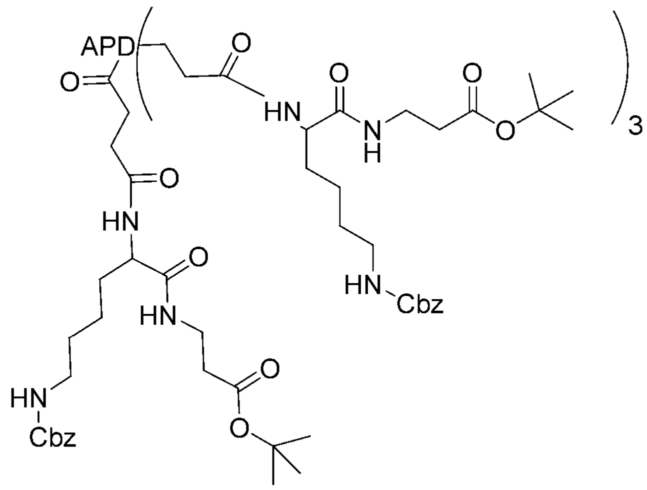 |
| **69-267** | 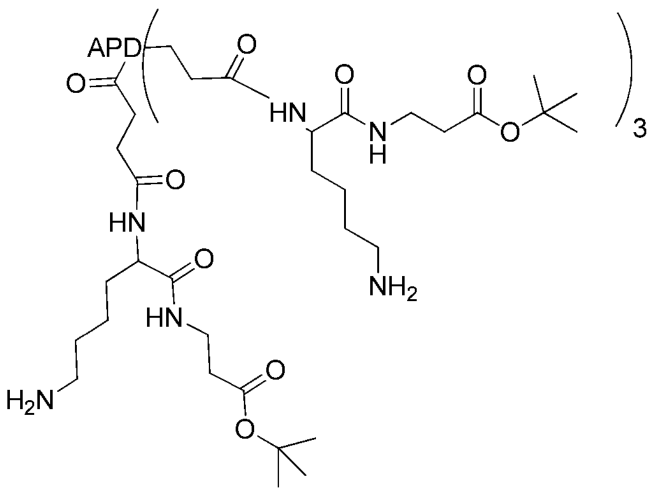 |
| **84-75** | 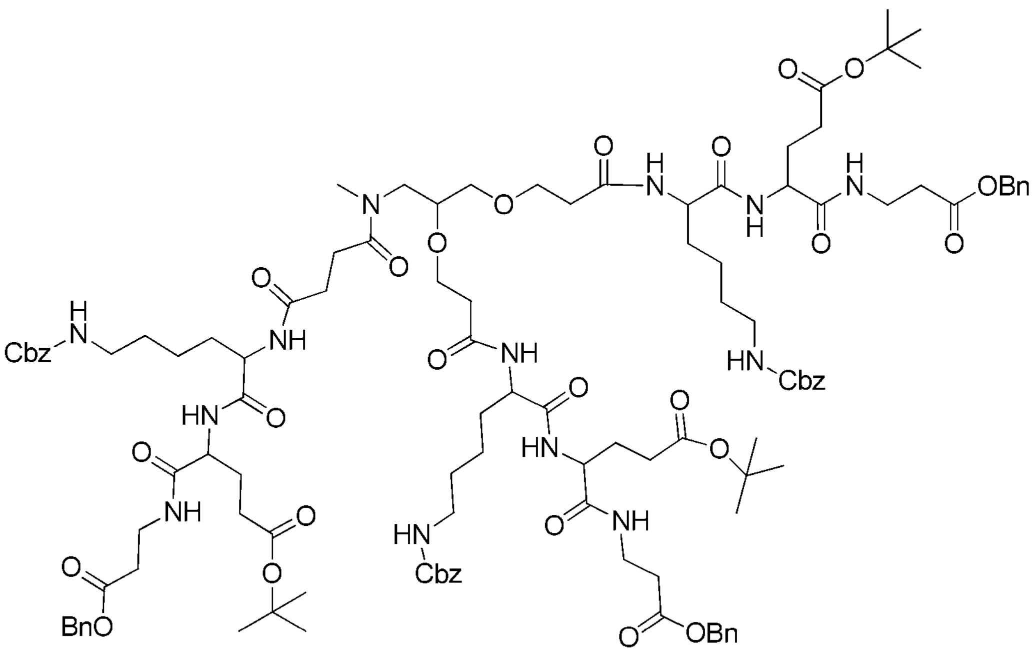 |

(continued)

| | |
|---|---|
| **84-81** | 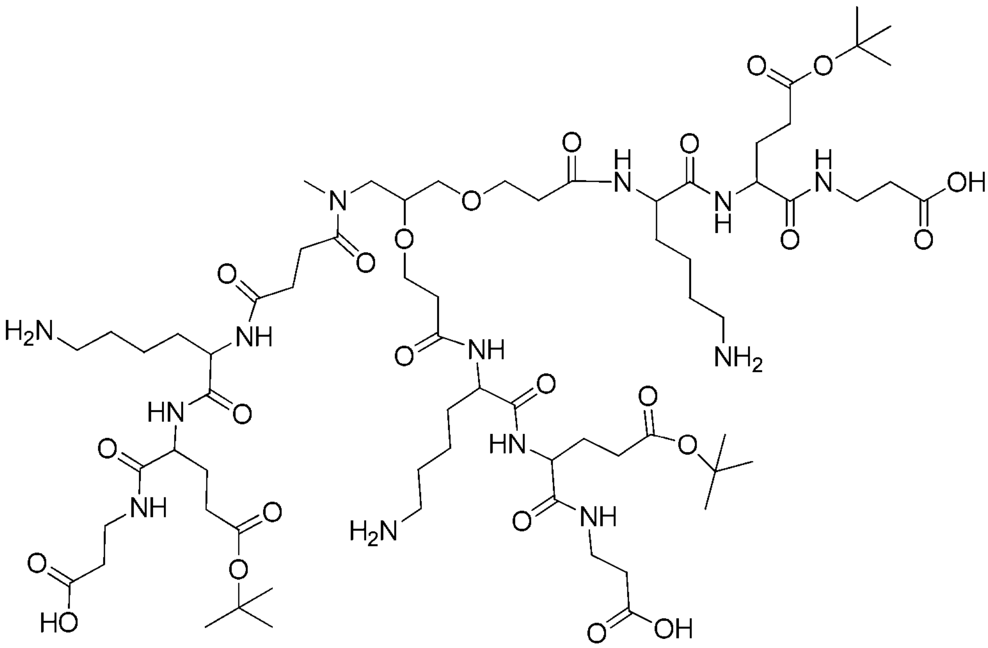 |
| **76-160** | 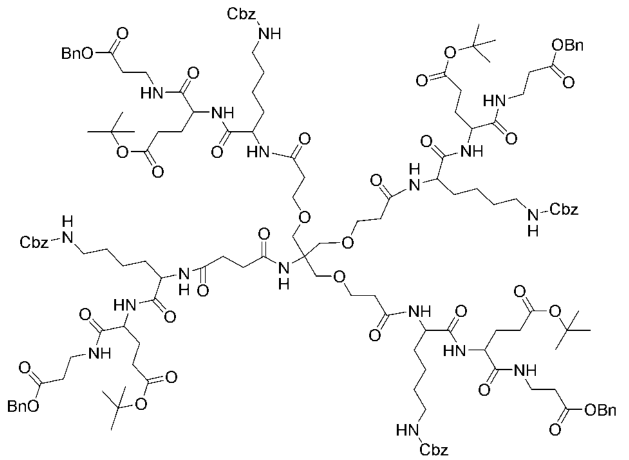 |
| **76-162** | 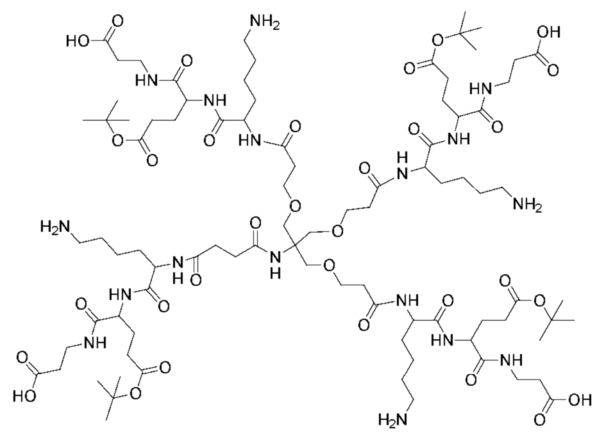 |

(continued)

| | |
|---|---|
| **82-78** | 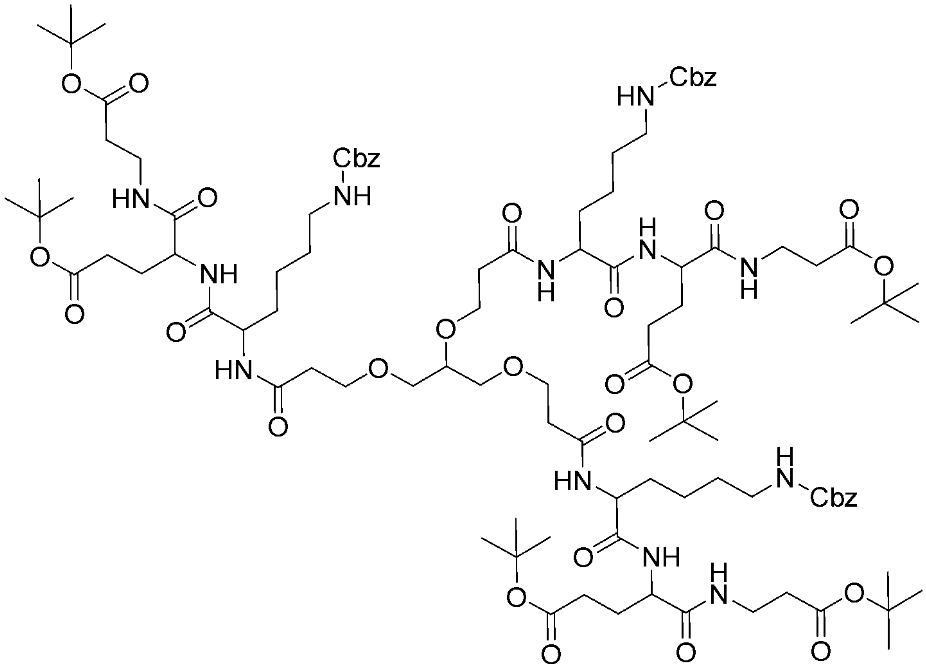 |
| **82-93** | 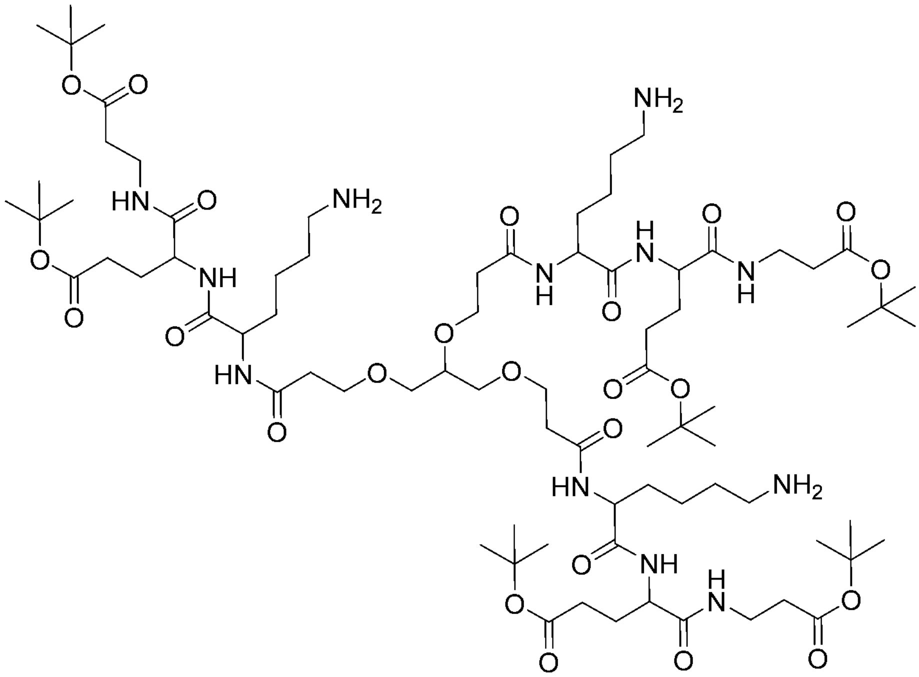 |

(continued)

| | |
|---|---|
| **80-201** | 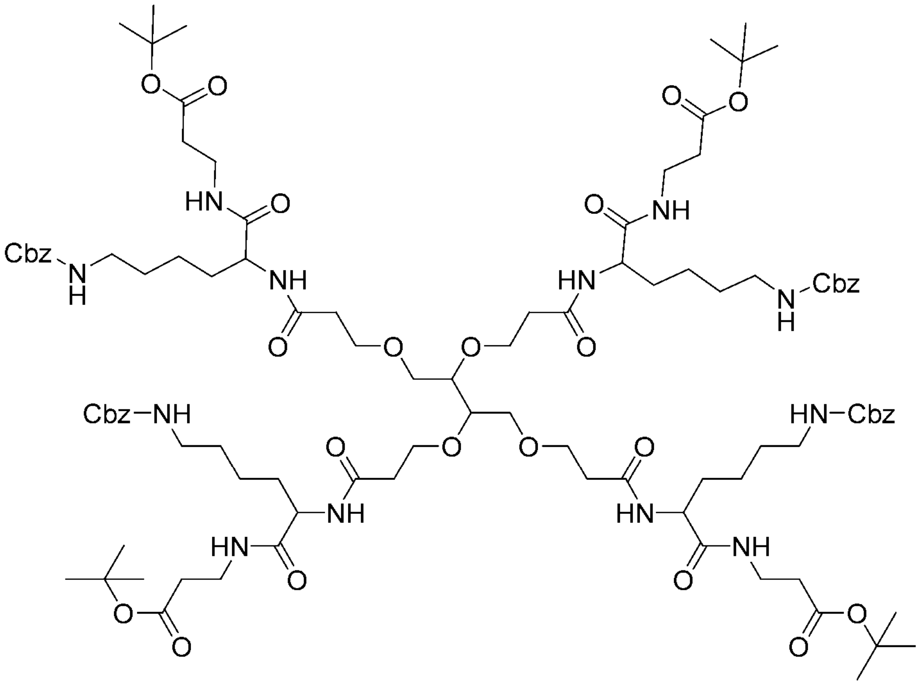 |
| **78-55** | 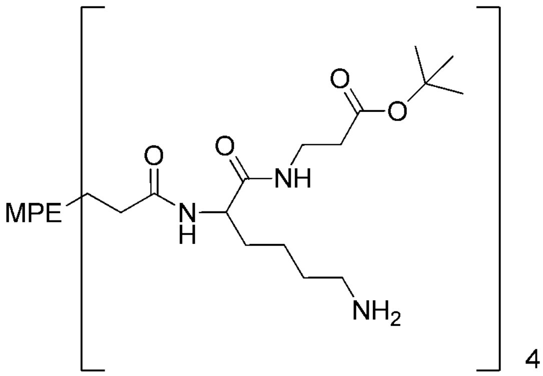 |
| **59-228** | 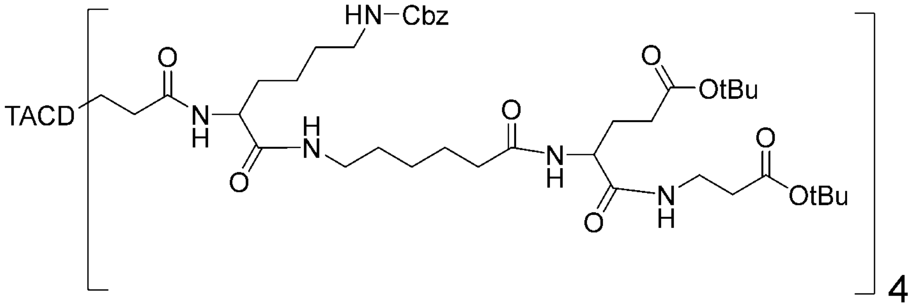 |

(continued)

| | |
|---|---|
| **59-232** | 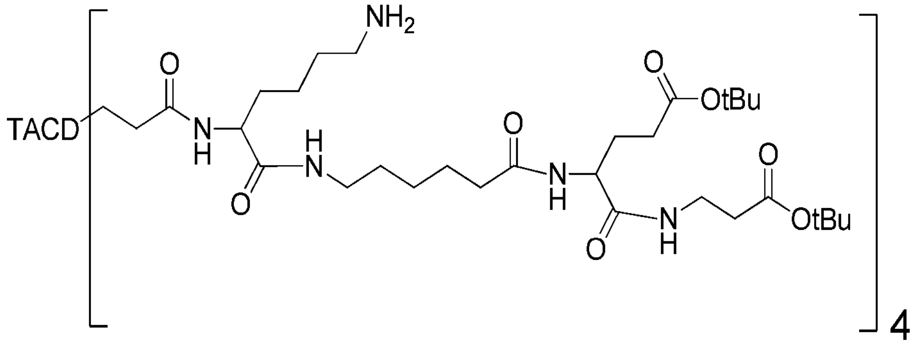 |

In another aspect, the present invention provides a compound represented by formula III or a pharmaceutically acceptable salt thereof,

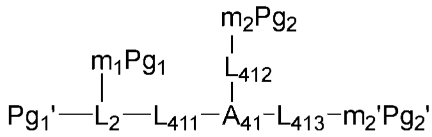

III

wherein, $Pg_1$' is hydrogen or selected from an amino protecting group, such as an alkoxycarbonyl-based protecting group, further such as Boc, Fmoc, Cbz, or Teoc;

the remaining groups are as defined in any of preceding items.

In some embodiments, $Pg_1$' is hydrogen. In some embodiments, $Pg_1$' is Fmoc. In some embodiments, $Pg_1$' is different from $Pg_1$.

In some embodiments, the compound represented by formula III is selected from the group consisting of:

| | |
|---|---|
| **71-46** | 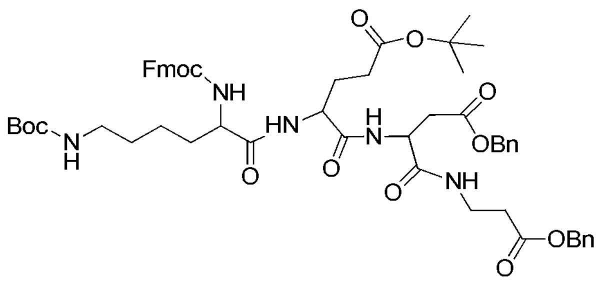 |
| **71-55** | 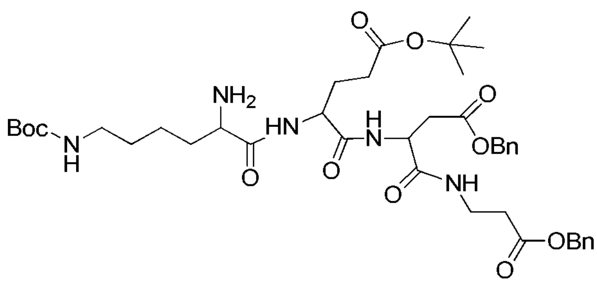 |

(continued)

| | |
|---|---|
| **38-227** | 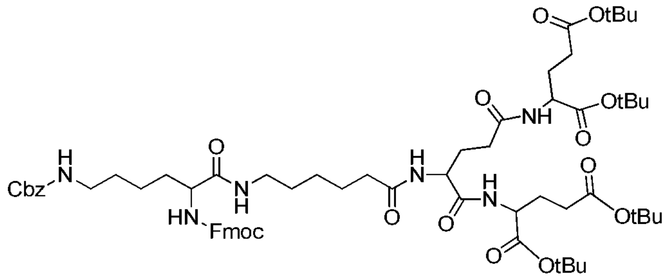 |
| **38-230** | 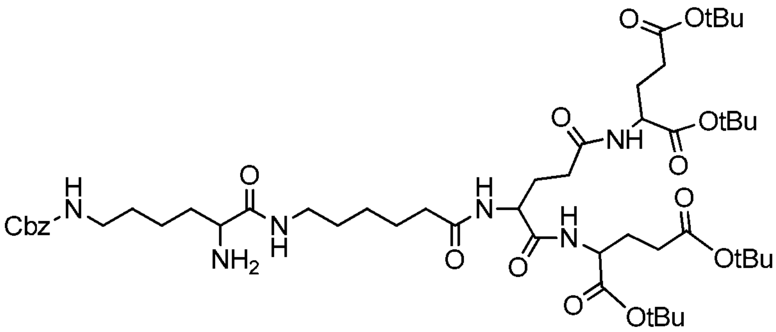 |
| **67-101** | 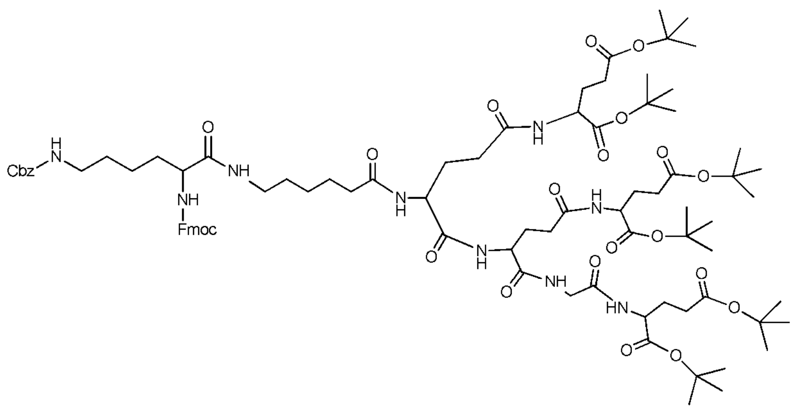 |
| **67-112** | 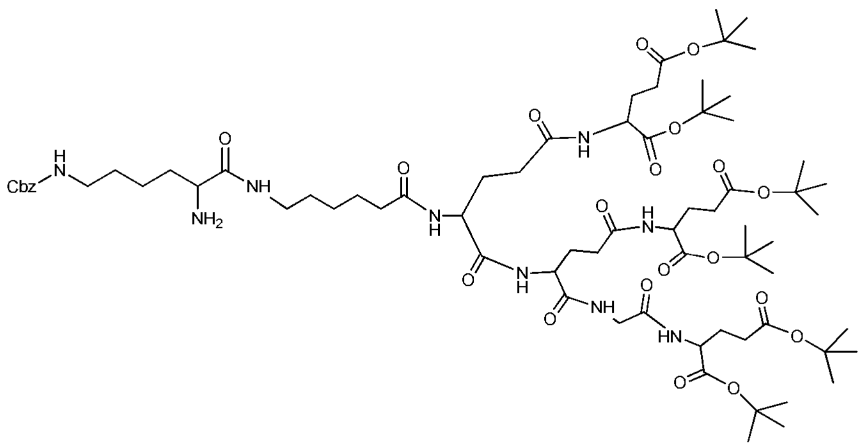 |

(continued)

| | |
|---|---|
| **69-39** | 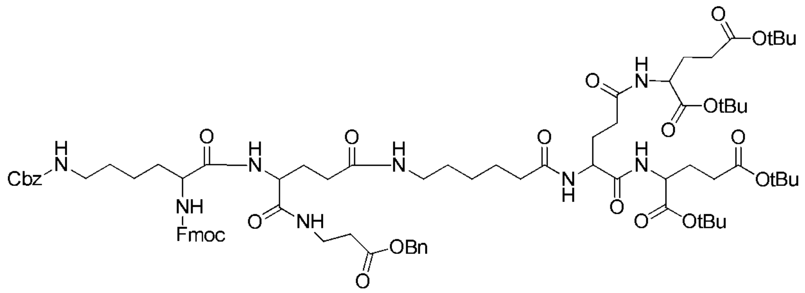 |
| **69-45** | 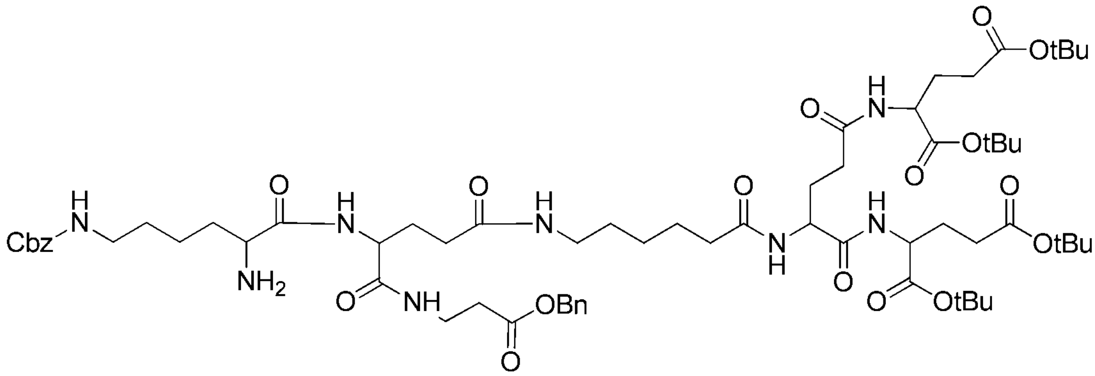 |
| **70-79** | 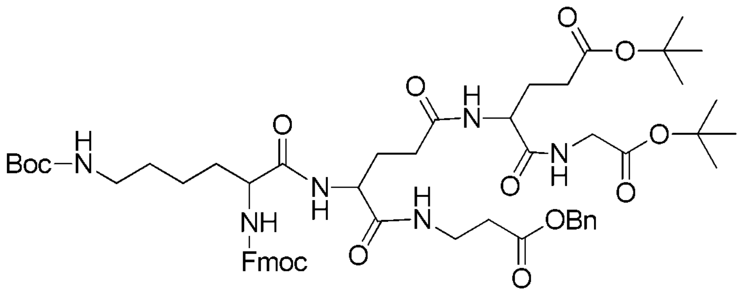 |
| **70-80** | 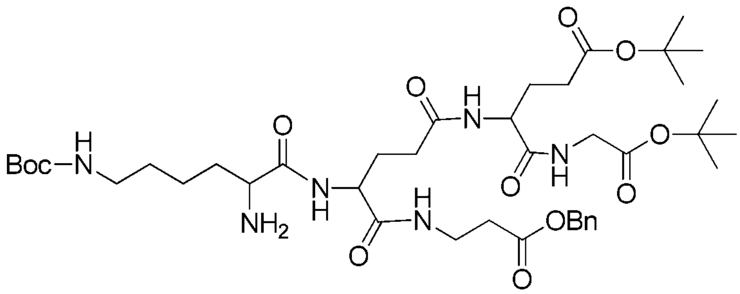 |
| **80-18** | 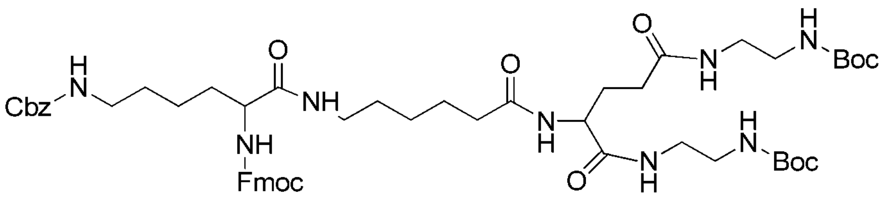 |
| **80-20** | 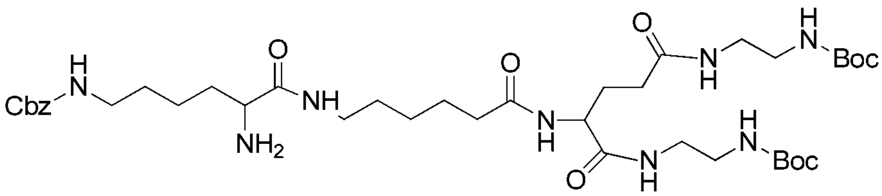 |

(continued)

| | |
|---|---|
| **58-82** | 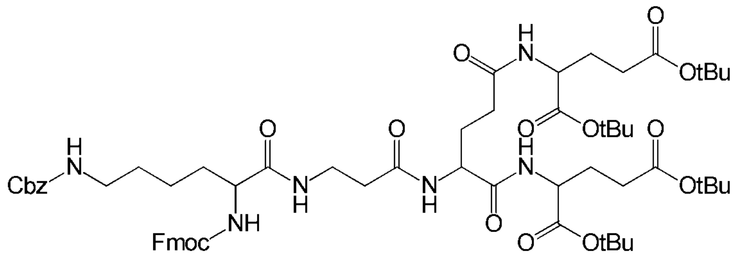 |
| **58-211** | 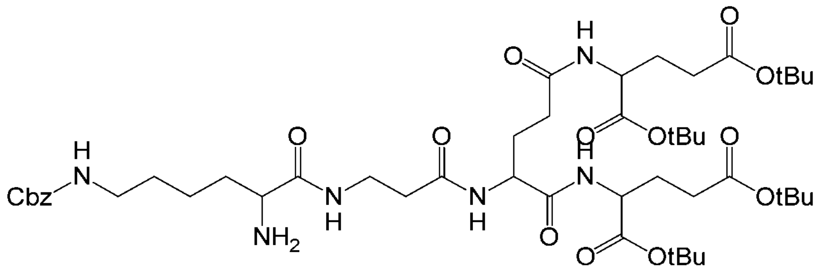 |
| **61-231** | 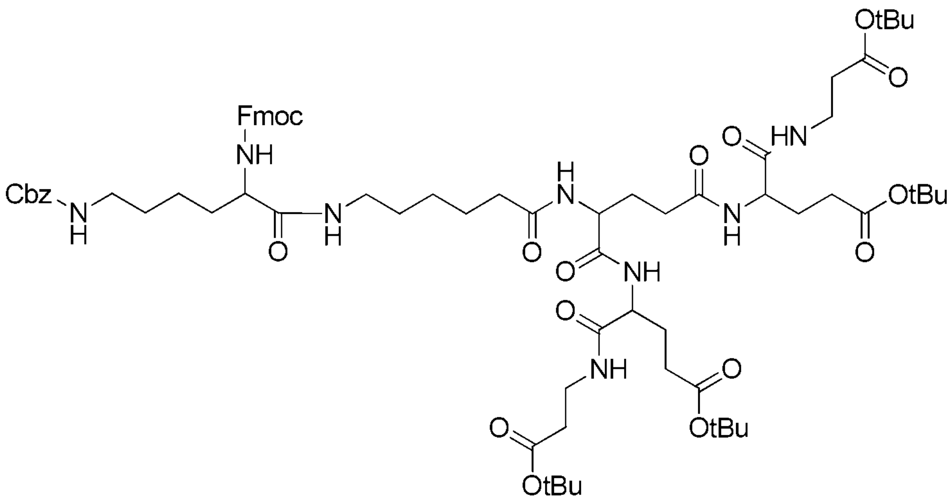 |
| **61-232** | 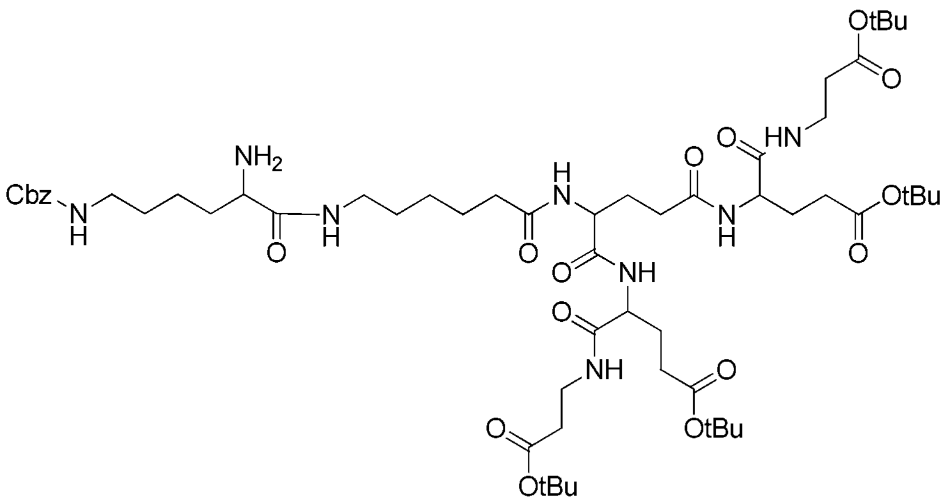 |
| **66-136** | 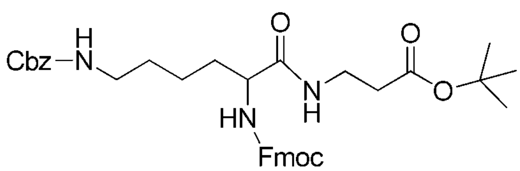 |

(continued)

| | |
|---|---|
| **66-195** | 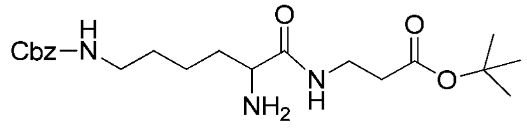 |
| **78-68** | 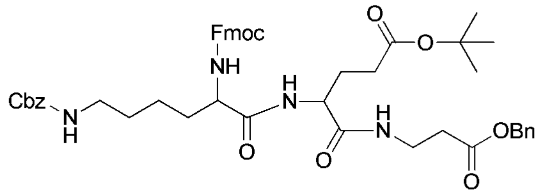 |
| **78-69** | 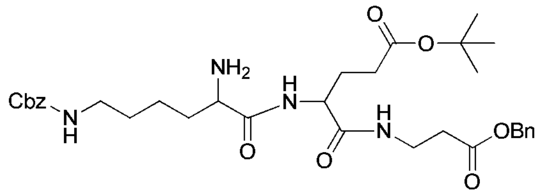 |
| **76-56** | 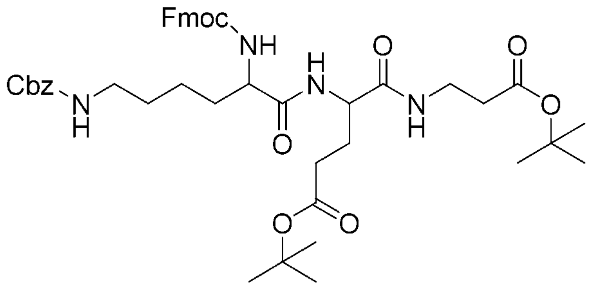 |
| **76-58** | 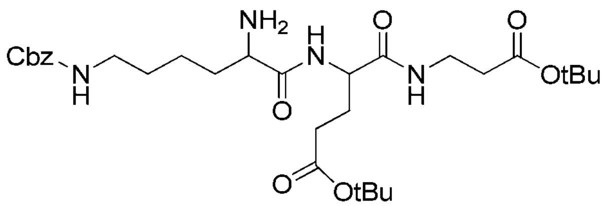 |
| **59-226** | 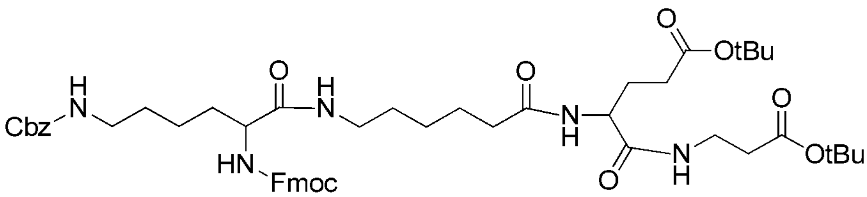 |
| **59-227** | 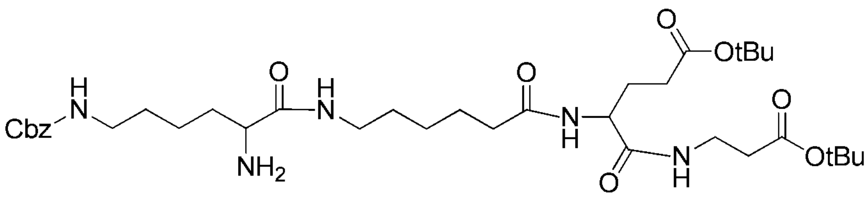 |

In another aspect, the present invention provides a compound represented by formula IV or a pharmaceutically acceptable salt thereof:

$M\text{-}[L_1\text{-}(Pg_3)n_1]n$ IV

wherein, each $Pg_3$ is independently hydrogen or selected from the group consisting of a carboxyl protecting group, such as an ester-based protecting group, preferably methyl ester, ethyl ester, tert-butyl ester, allyl ester, or benzyl ester;

the remaining groups are as defined in any of the preceding items.

In some embodiments, the compound is selected from the group consisting of:

| | |
|---|---|
| 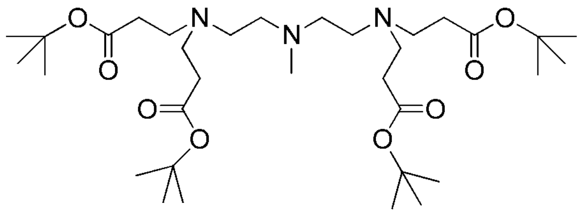 **71-6** | 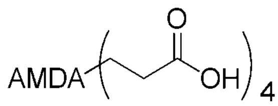 **71-54** |
| 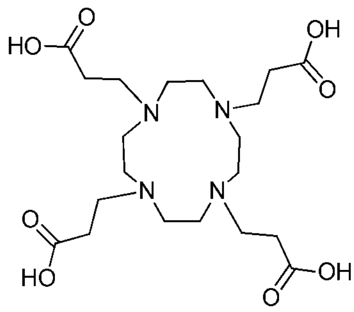 **59-108** | 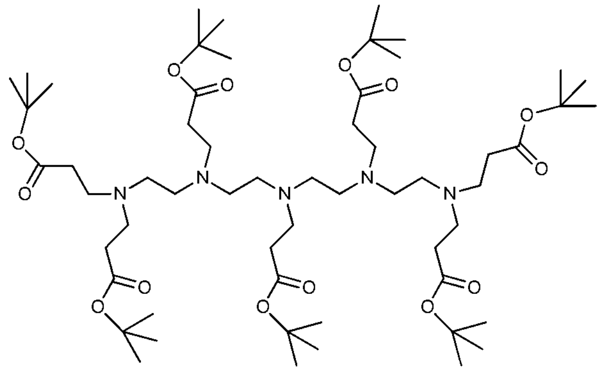 **67-34** |
| 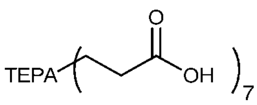 **67-53** | Triethylenetetraminehexaacetic acid |
| 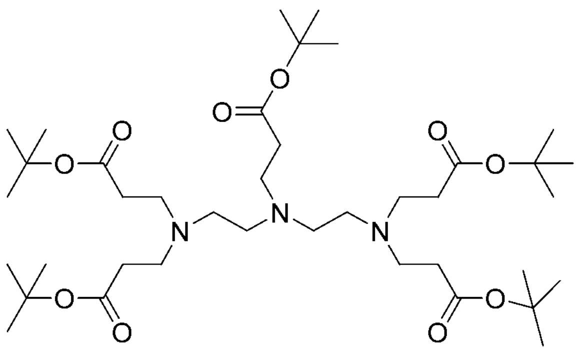 **69-9** | 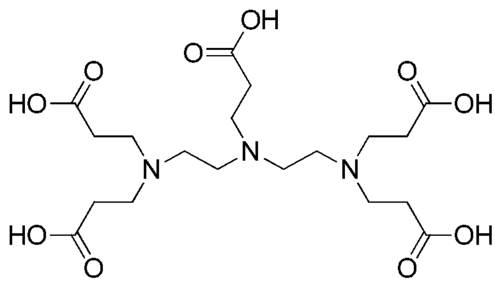 **69-38** |

(continued)

| | |
|---|---|
| 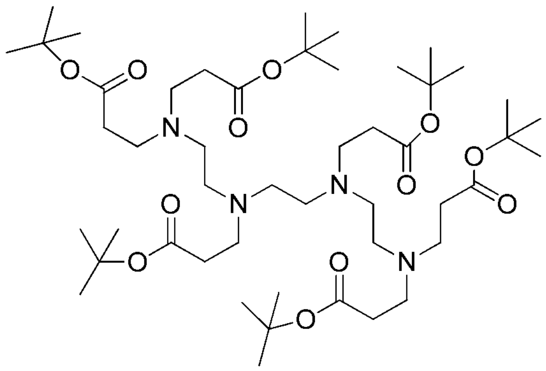 70-2 | 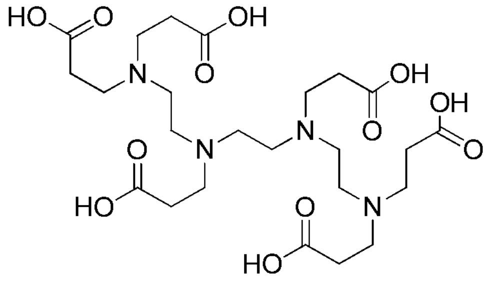 70-70 |
| 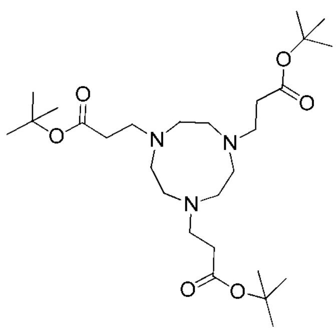 80-26 | 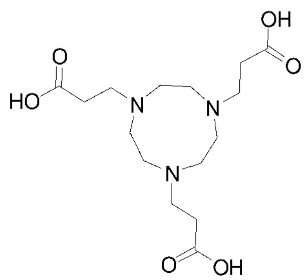 80-27 |
| 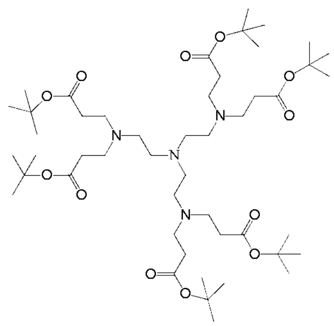 58-145 | 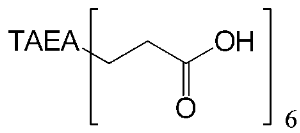 58-148 |
| 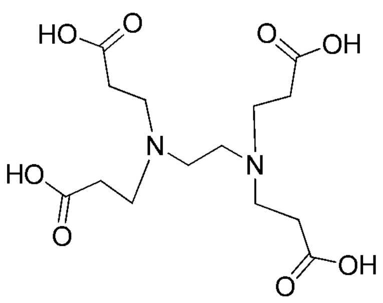 66-135 | 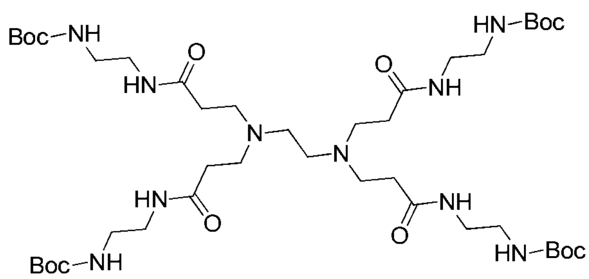 66-148 |

(continued)

| | |
|---|---|
| 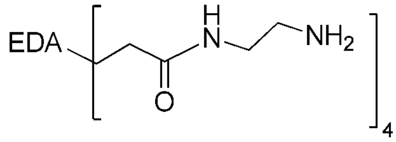 **66-168** | 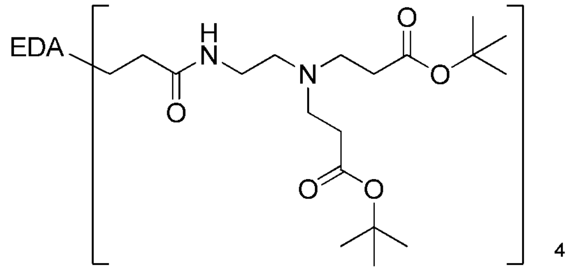 **66-171** |
| 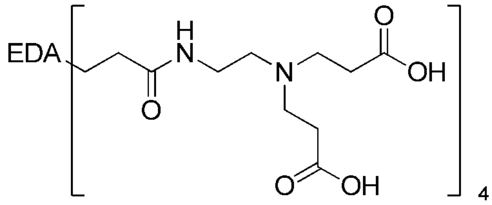 **58-179** | 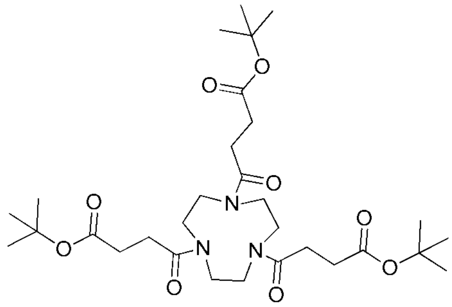 **80-234** |
| 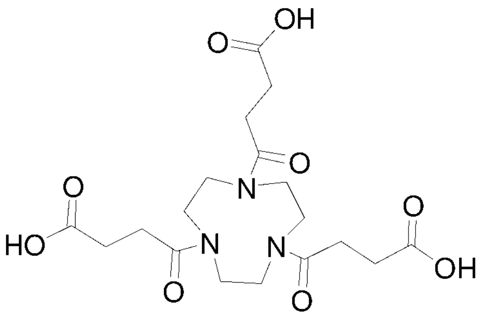 **78-70** | 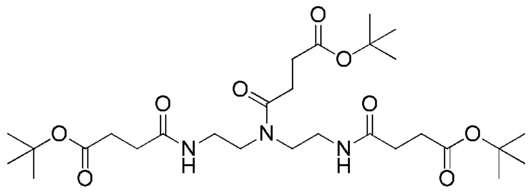 **82-112** |
| 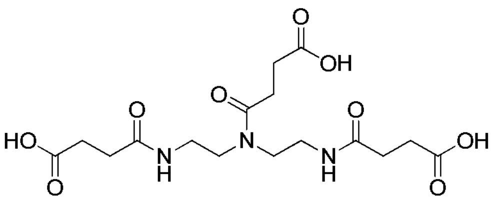 **82-133** | 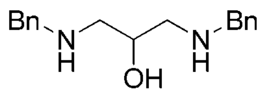 **70-218** |
| 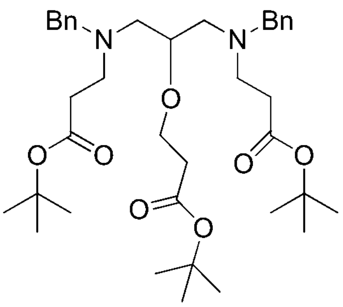 **70-247** | 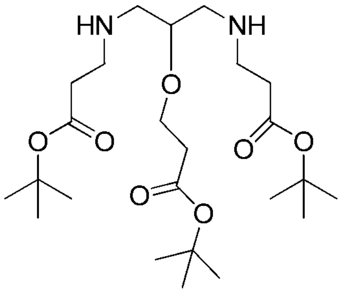 **70-255** |

(continued)

| | |
|---|---|
| 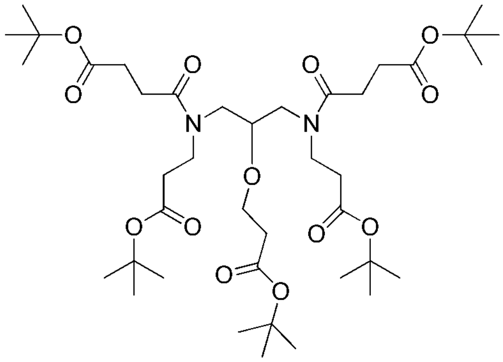<br>**70-230** | 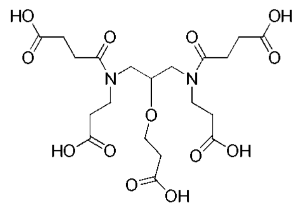<br>**70-231** |
| 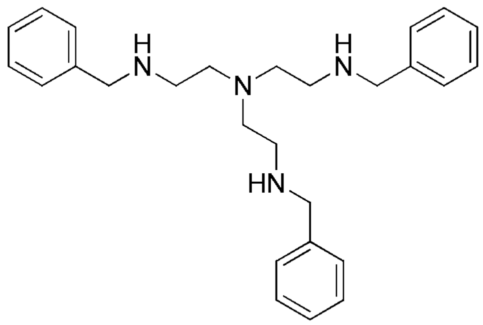<br>**81-135** | 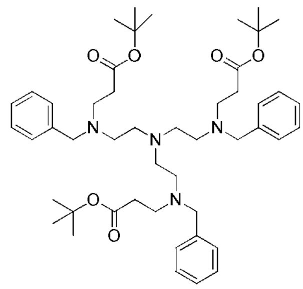<br>**81-143** |
| 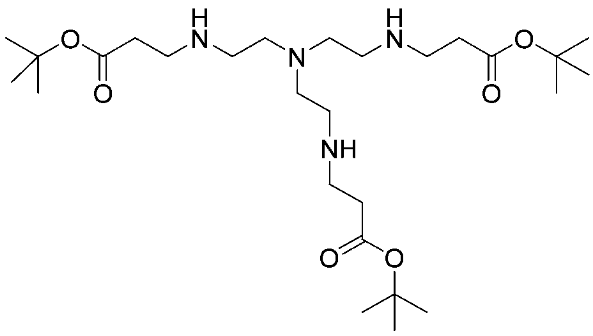<br>**81-144** | 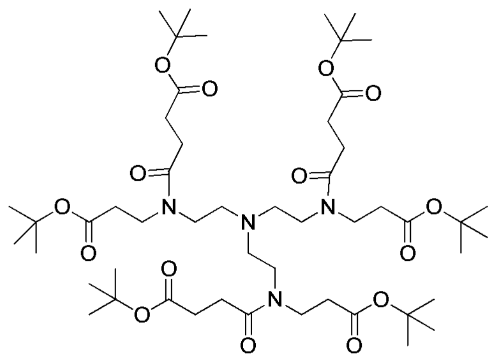<br>**81-145** |
| 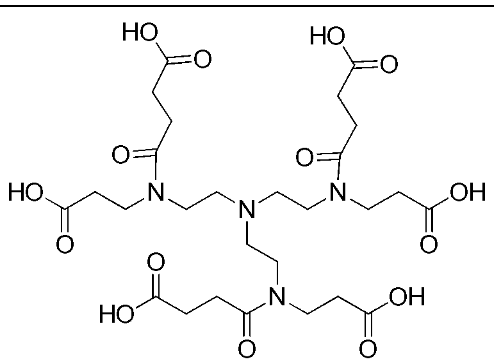<br>**81-148** | 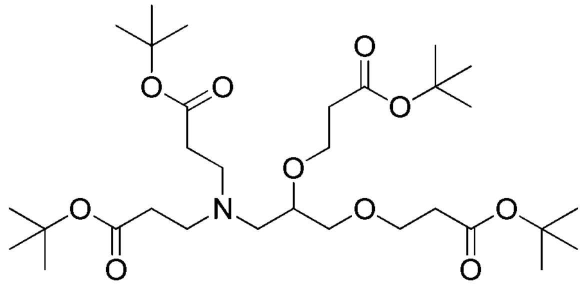<br>**82-63** |

(continued)

| | |
|---|---|
| 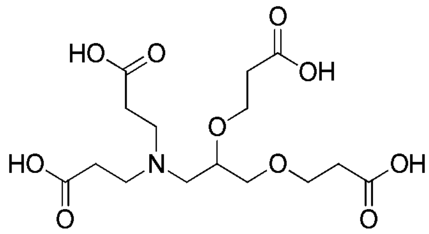 **71-273** | 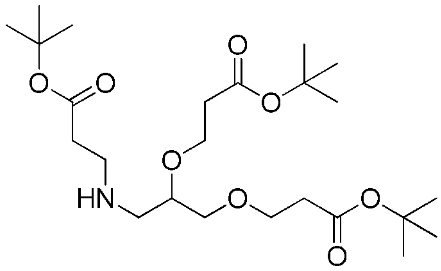 **82-50** |
| 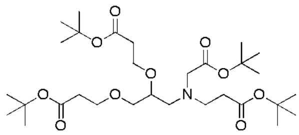 **76-138** | 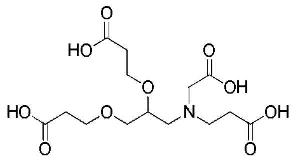 **75-165** |
| 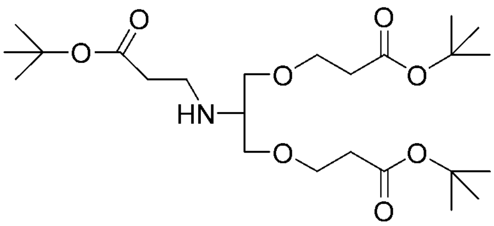 **69-237** | 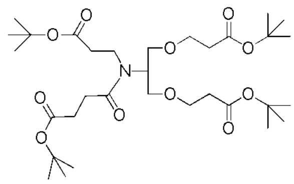 **69-242** |
| 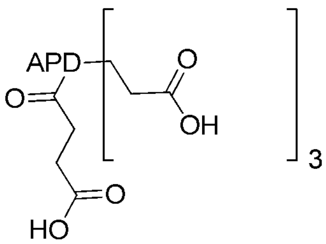 **69-256** | 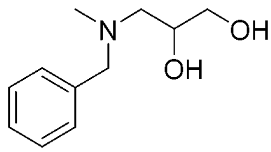 **84-29** |
| 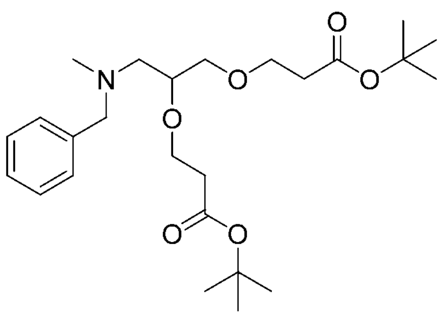 **84-49** | 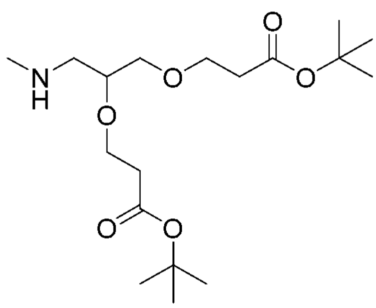 **84-50** |

(continued)

| | |
|---|---|
| 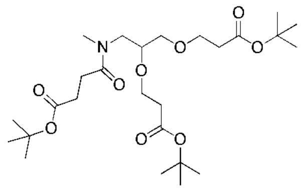 84-54 | 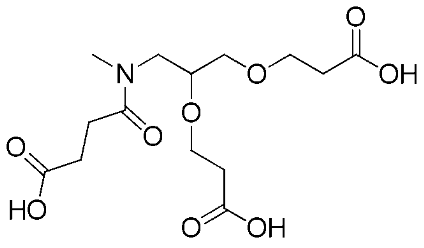 84-70 |
| 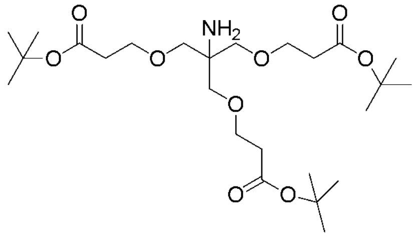 76-84 | 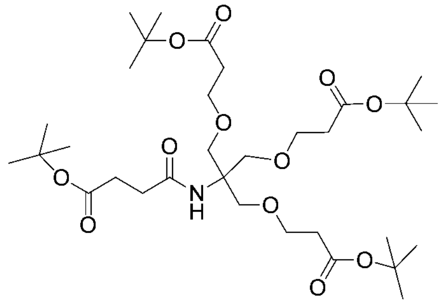 76-140 |
| 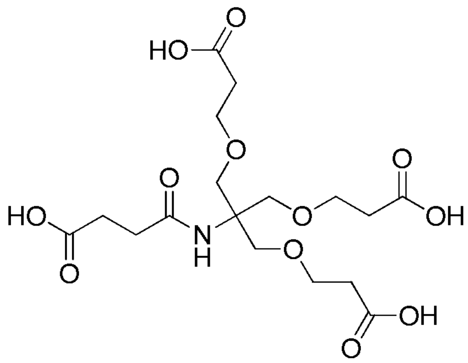 76-153 | 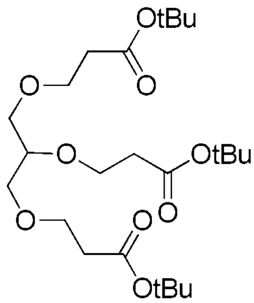 82-58 |
| 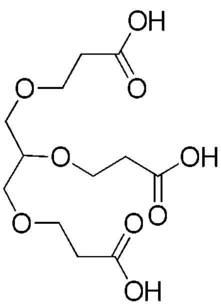 82-73 | 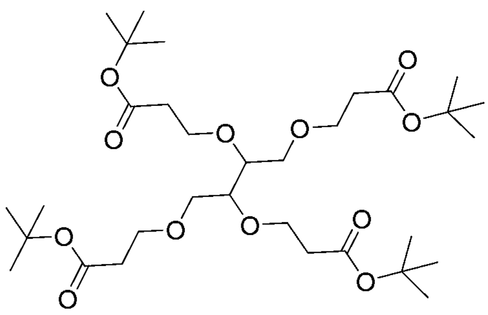 80-190 |

(continued)

| | |
|---|---|
| 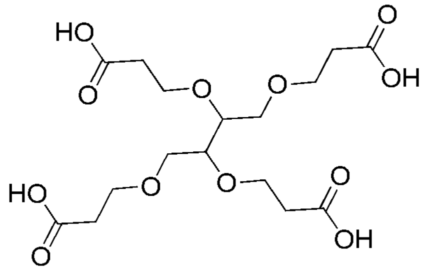 **80-196** | |

In another aspect, the present invention provides a compound represented by formula V or a pharmaceutically acceptable salt thereof:

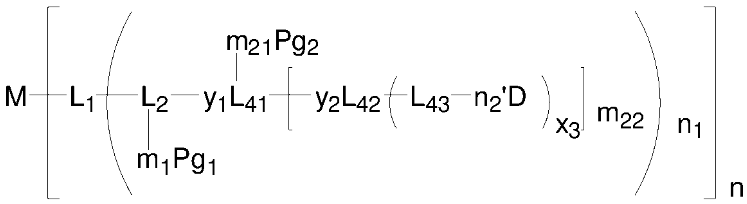

formula V

wherein, each of $m_{21}$ and $m_{22}$ is independently selected from the group consisting of 1, 2, 3, 4, 5, 6, 7, 8, 9, 10, 11, 12, 13, 14, 15, 16, 17, 18, 19, and 20, preferably, $m_{21}$ + $m_{22}$ = $x_4$;

each $n_2$' is independently selected from the group consisting of 1, 2, 3, 4, 5, 6, 7, 8, 9, 10, 11, 12, 13, 14, 15, 16, 17, 18, 19, and 20;

the remaining groups are as defined in any of the preceding items.

In some embodiments, the compound is selected from the group consisting of:

| | |
|---|---|
| **70-87** | 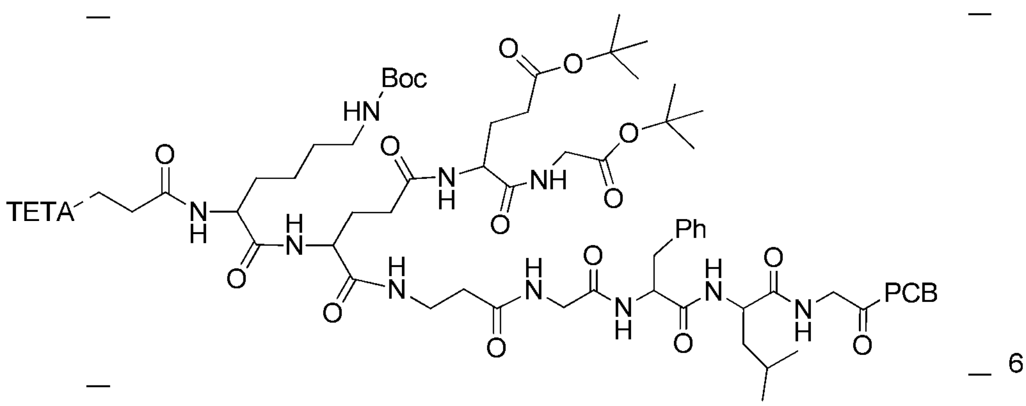 |

(continued)

| | |
|---|---|
| **70-88** | 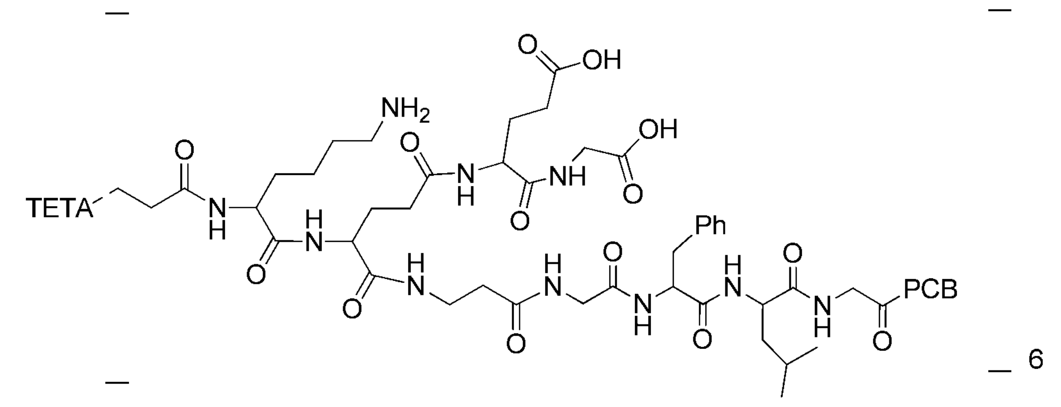 |

In another aspect, the present invention provides a pharmaceutical composition, which comprises the compound (especially the compound represented by formula I) or pharmaceutically acceptable salt thereof as described in any of the preceding items in an amount effective for treating and/or preventing a disease.

In some embodiments, the composition further comprises one or more pharmaceutically acceptable excipients.

In some embodiments, the pharmaceutical composition is formulated as an injectable formulation.

In another aspect, the present invention provides a use of the compound (especially the compound represented by formula I) or pharmaceutically acceptable salts thereof as described in any of the preceding items in the manufacture of a medicament for treating and/or preventing a disease (e.g., a cancer).

In some embodiments, the cancer is selected from the group consisting of colon cancer, leukemia, lymphoma, bladder cancer, bone cancer, brain tumor, medulloblastoma, glioma, breast cancer, adenoma/carcinoid, adrenocortical carcinoma, islet cell carcinoma, cervical cancer, endometrial cancer, ovarian cancer, colorectal cancer, skin cancer, esophageal cancer, eye cancer, gallbladder cancer, gastric cancer, head and neck cancer, liver cancer, melanoma, Kaposi's sarcoma, kidney cancer, oral cancer, lung cancer, nasopharyngeal cancer, neuroblastoma, ovarian cancer, pancreatic cancer, thyroid cancer, parathyroid gland cancer, penile cancer, prostate cancer, urethral cancer, vaginal cancer, vulvar cancer, anal cancer, sarcoma, and metastases of such cancers.

In another aspect, the present invention provides an injection, which comprises the compound (especially the compound represented by formula I) or pharmaceutically acceptable salt thereof, or the pharmaceutical composition, as described in any of the preceding items.

In some embodiments, the injection uses physiological saline as a carrier.

In another aspect, the present invention provides a method for preparing the compound represented by formula I or pharmaceutically acceptable salt thereof as described in any of the preceding items,
when the $n_1$+$n_1$' side chains connected to $L_{41}$ in formula I are all the same, the method comprises the following steps:

S1-1: reacting a compound represented by formula II with PEG connected with an activating group to provide intermediate 1-1;
S1-2: subjecting intermediate 1-1 to any of the following reactions to provide the compound:

(1) reacting directly with a precursor providing fragment

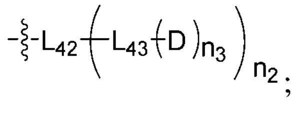

(2) reacting sequentially with precursors providing fragment $L_{42}$, fragment $L_{43}$ and fragment D,

(3) reacting sequentially with precursors providing fragment

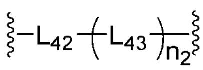

and fragment D;

(4) reacting sequentially with precursors providing fragment $L_{42}$ and fragment $L_{43}$-$n_3$D;

preferably, the step S1-1 is performed before or after S1-2, or, when S1-2 involves multiple steps, the step S1-1 is performed after any step of S1-2 is completed;
when the

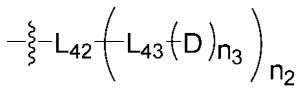

and

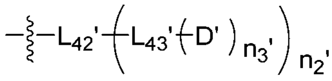

connected to $L_{41}$ in formula I are different, it comprises the following steps:
S2-1: reacting the compound represented by formula II with PEG connected with an activating group, to provide intermediate 2-1;
S2-2: subjecting intermediate 2-1 to any of the following reactions to provide the compound:

(1) reacting sequentially with precursors providing fragment

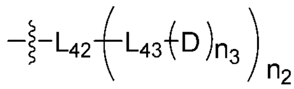

and fragment

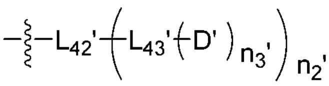
;

(2) reacting sequentially with precursors providing fragment

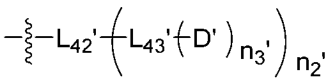

and fragment

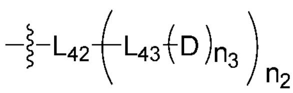
;

(3) reacting first with a precursor providing fragment

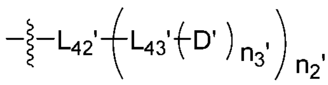
,

and then performing any of the following reactions:

1) reacting sequentially with precursors providing fragment $L_{42}$ and fragment $L_{43}$-$n_3$D;
2) reacting sequentially with precursors providing fragment $L_{42}$, fragment $L_{43}$ and fragment D;
3) reacting sequentially with precursors providing fragment

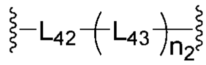

and fragment D;

preferably, the step S2-1 is performed before or after S2-2, or when S2-2 involves multiple steps, the step S2-1 is performed after any step of S2-2 is completed;
optionally, before or after any of the above steps, a step of removal of protecting groups and/or activation (e.g., activation of carbonyl or amino groups) is also included;
preferably, the precursor is a compound in free or activated form capable of providing the corresponding fragment;
preferably, the PEG activating group is preferably

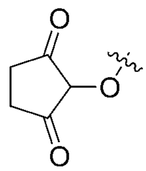

;

each of the groups is as defined in any of the preceding items.

In some embodiments, $A_{41}$ in the compound represented by formula II is selected from the group consisting of an amino acid residue or derivatives thereof, or a polypeptide fragment consisting of two or more amino acids or derivatives thereof, wherein the amino acid residue or derivatives thereof, or the polypeptide fragment or derivatives thereof, provides 1, 2, 3, 4, 5, or 6 attachment sites; preferably, $A_{41}$ comprises a Glu or Asp residue;

$L_{412}$ is a bond;

$L_{413}$ is $-NH(CH_2)_2CO-$;

the amino acid (e.g., Glu or Asp) directly connected to $L_{413}$ in $A_{41}$ provides two of the attachment sites, one of which is connected to $L_{413}$ and the other to $Pg_2$, wherein $Pg_2$ is identical to $Pg_2$' connected to $L_{413}$ (e.g., both are -OBn);

the remaining groups are as defined in any of the preceding items.

In some embodiments, the compound represented by formula II is obtained by reacting the compound represented by formula IV with the compound represented by formula III;
preferably, before conducting the reaction, a step of removing protecting groups is also included.

<u>Definition of terms</u>

Unless otherwise defined below, all technical and scientific terms used herein are intended to have the same meaning as commonly understood by those skilled in the art. References to techniques used herein are intended to refer to techniques commonly understood in the art, including those variations or substitutions of equivalent techniques readily apparent to one skilled in the art. While the following terms are believed to be well understood by those skilled in the art, the following definitions are provided to better illustrate the present invention.
The terms "comprise," "include," "have," "contain," or "involve," and their variations herein, are intended to be inclusive or open-ended and do not exclude other unrecited elements or method steps.
As used herein, the term "pharmaceutically acceptable carrier" refers to a carrier that is pharmacologically and/or physiologically compatible with the subject and the active ingredient, as is well known in the art (see, for example, Remington's Pharmaceutical Sciences. Edited by Gennaro AR, 19th ed. Pennsylvania: Mack Publishing Company, 1995), and includes, but is not limited to, pH adjusters, surfactants, adjuvants, ionic strength enhancers, diluents, agents for maintaining osmotic pressure, agents for delaying absorption, and preservatives. For example, the pH adjusters include, but are not limited to, phosphate buffer. The surfactants include, but are not limited to, cationic, anionic, or nonionic surfactants, such as Tween-80. The ionic strength enhancers include, but are not limited to, sodium chloride. The preservatives include, but are not limited to, various antibacterial and antifungal agents, such as paraben, chlorobutanol, phenol, and sorbic acid. The agents for maintaining osmotic pressure include, but are not limited to, sugar, NaCl, and analog thereof. The agents for delaying absorption include, but are not limited to, monostearate and gelatin. The diluents

include, but are not limited to, water, aqueous buffer (e.g., buffered saline), alcohol, and polyol (e.g., glycerol). The preservatives include, but are not limited to, various antibacterial and antifungal agents, such as thimerosal, 2-phenoxyethanol, paraben, chlorobutanol, phenol, and sorbic acid. The stabilizer has the meaning commonly understood by those skilled in the art and is capable of stabilizing the desired activity of the active ingredient in a pharmaceutical, including, but not limited to, sodium glutamate, gelatin, SPGA, sugar (e.g., sorbitol, mannitol, starch, sucrose, lactose, dextran, or glucose), amino acids (e.g., glutamic acid and glycine), proteins (e.g., dried whey, albumin, or casein), or degradation products thereof (e.g., lactalbumin hydrolysate).

As used herein, the term "prevention" refers to a method performed to prevent or delay the onset of a disease, condition, or symptom (e.g., a tumor) in a subject.

As used herein, the term "treatment" refers to a method performed to achieve a beneficial or desired clinical outcome. For the purposes of the present invention, beneficial or desired clinical outcomes include, but are not limited to, alleviation of symptoms, reduction in the extent of disease, stabilization (i.e., no worsening) of the disease state, delaying or slowing the progression of disease, improvement or palliation of the disease state, and alleviation of symptoms (whether partial or complete), whether detectable or undetectable. Furthermore, "treatment" may also refer to prolonging survival compared to expected survival if not receiving treatment.

As used herein, the term "subject" refers to a mammal, such as a primate mammal, such as a human. In certain embodiments, the subject (e.g., a human) has a tumor, or is at risk for such a disease.

As used herein, the term "effective amount" refers to an amount sufficient to achieve, or at least partially achieve, a desired effect. For example, an amount effective for prevention of a disease (e.g., tumor) refers to an amount sufficient to prevent, arrest, or delay the onset of a disease (e.g., tumor); an amount effective for treatment of a disease refers to an amount sufficient to cure or at least partially arrest the disease and its complications in a patient already suffering from the disease. Determination of such an effective amount is well within the capabilities of those skilled in the art. For example, the effective amount for therapeutic use will depend on the severity of the disease being treated, the overall state of the patient's own immune system, the patient's general condition such as age, weight, and sex, the mode of administration of the drug, and any other concurrently administered therapies.

The terms "cancer" and "tumor" are used interchangeably to refer to a broad class of diseases characterized by the uncontrolled growth of abnormal cells in the body. Unregulated cell division may lead to the formation of malignant tumors or cells that invade adjacent tissues and may metastasize to distant sites in the body via the lymphatic system or bloodstream. Cancers include both benign and malignant cancers, as well as dormant tumors or micrometastases. Cancers also include hematological tumors, particularly hematological malignancies.

The term "hydrocarbonyl" refers to a group derived from a corresponding hydrocarbon by losing a hydrogen atom, including monovalent, divalent, and trivalent hydrocarbonyl groups, such as $C_{2-10}$ trivalent hydrocarbonyl, $C_{2-6}$ trivalent saturated hydrocarbonyl.

The term "alkyl" is defined as a straight-chain or branched saturated aliphatic hydrocarbonyl. In some embodiments, the alkyl has 1 to 12, for example, 1 to 6, carbon atoms. For example, as used herein, the term "$C_{1-6}$ alkyl" refers to a linear or branched group of 1 to 6 carbon atoms (e.g., methyl, ethyl, n-propyl, isopropyl, n-butyl, isobutyl, sec-butyl, tert-butyl, n-pentyl, and n-hexyl).

The term "alkylamino" refers to a group having an "alkyl-NH-" structure, wherein the alkyl is defined as described above, such as, $C_{1-6}$ alkylamino, $C_{1-4}$ alkylamino, $C_{1-3}$ alkylamino, or $C_{1-2}$ alkylamino. Common alkylamino groups include (but are not limited to) methylamino, ethylamino, n-propylamino, isopropylamino, n-butylamino, isobutylamino, tert-butylamino, pentylamino, hexylamino, and the like.

The structural formula of AMDA is:

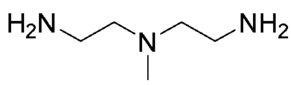

.

The structural formula of TACD is:

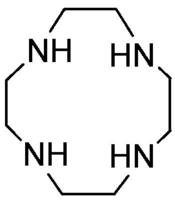

.

The structural formula of TEPA is:

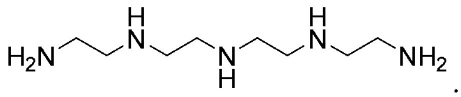

The structural formula of DETA is:

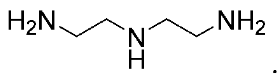

The structural formula of TETA is:

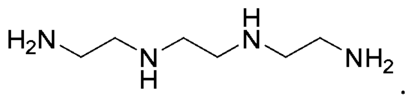

The structural formula of TAN is:

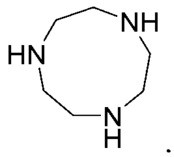

The structural formula of TAEA is:

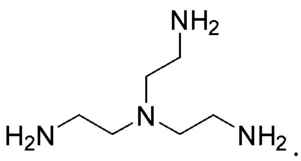

The structural formula of EDA is:

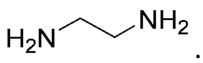

The structural formula of DAPO is:

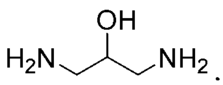

The structural formula of APDO is:

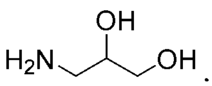

The structural formula of APD is:

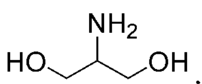

The structural formula of MPE is:

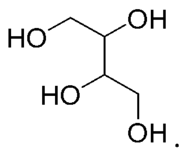

The structural formula of PT is:

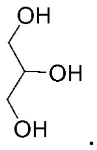

The structural formula of PTX is:

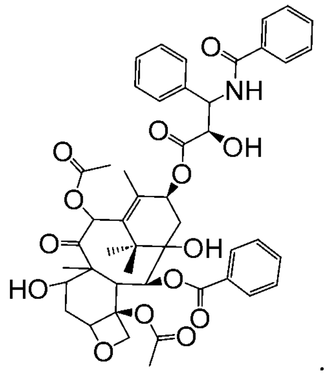

The structural formula of PCB is:

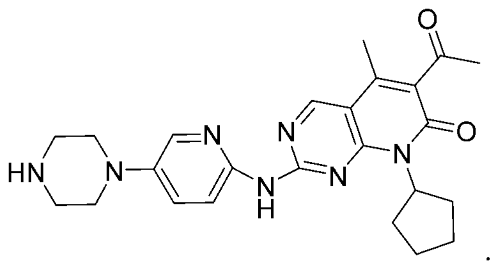

The structural formula of SN38 is:

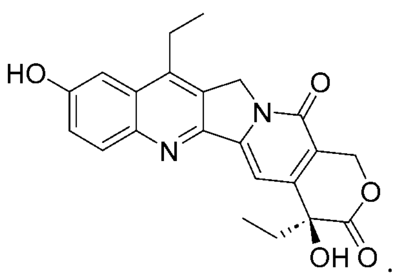

The structural formula of NPB is:

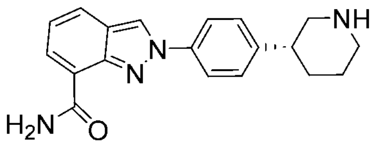

.

The structural formula of AXT is:

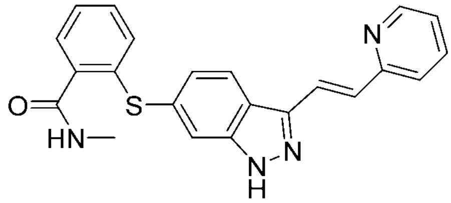

.

The structural formula of LPT is:

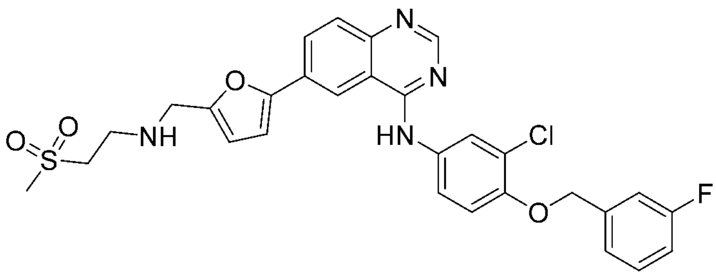

.

The structural formula of DOX is:

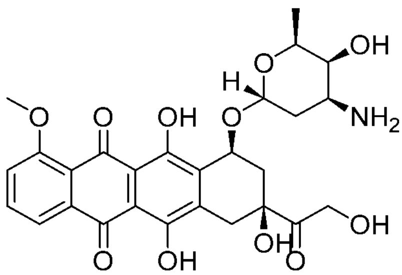

.

The structural formula of MI-AH-PLGLAG-iRGD is:

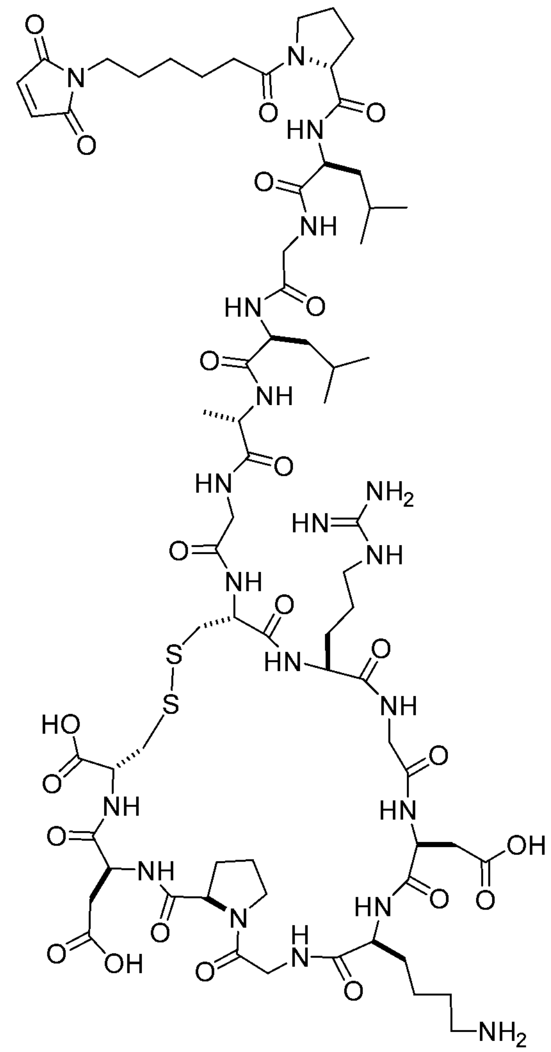

The structural formula of SB7 is:

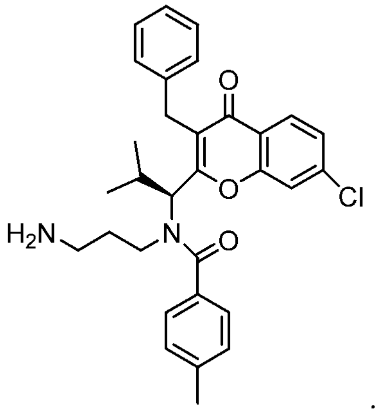

The structural formula of folic acid is:

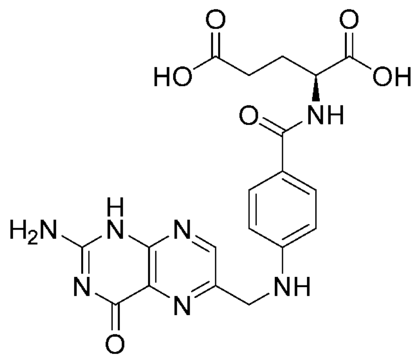

.

The structural formula of sodium dodecahydrododecaborate is:

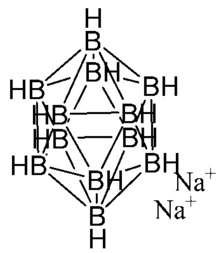

.

As used herein, the term "amino acid" mainly includes the following 20 common amino acids: alanine (Ala), arginine (Arg), aspartic acid (Asn), asparagine (Asp), cysteine (Cys), glutamine (Gln), glutamic acid (Glu), glycine (Gly), histidine (His), leucine (Leu), isoleucine (Ile), lysine (Lys), methionine (Met), phenylalanine (Phe), proline (Pro), serine (Ser), threonine (Thr), tryptophan (Trp), tyrosine (Tyr), valine (Val). It is known that amino acids contain two functional groups: amino group and carboxyl group. As used herein, "amino acid residue" refers to the residue remaining after removing a hydrogen from the amino group and/or a hydroxyl from the carboxyl group.

Beneficial effects of the invention

The present invention provides a class of polyethylene glycol small molecules with an aliphatic polyamine core, as represented by formula I, and pharmaceutically acceptable salts thereof. Furthermore, the present invention provides intermediates, preparation methods, and anti-tumor applications of such molecules. The biological tests demonstrate that the compounds of the present invention exhibit significant inhibitory effects on subcutaneous xenografts (e.g., xenografts of human breast cancer BT474 cells in NPG mice).

## Brief Description of the Drawings

The figures herein are provided to further understand the present invention and constitute a part of the present invention. The illustrative examples and their descriptions are provided for illustrative purposes only and are not intended to unduly limit the present invention. In the accompanying drawings:

Figure 1 shows the growth inhibition rate of A549 tumor cells treated with **82-178** at different concentrations;

Figure 2 shows the growth inhibition rate of COLO-205 tumor cells treated with **81-176** at different concentrations;

Figure 3 shows the growth inhibition rate of COLO-205 tumor cells treated with **71-246** at different concentrations;

Figure 4 shows the growth inhibition rate of human breast cancer BT-474 cells treated with **82-183** at different concentrations;

Figure 5 shows the growth inhibition rate of human breast cancer BT-474 cells treated with **78-139** at different concentrations;

Figure 6 shows the growth inhibition rate of human breast cancer BT-474 cells treated with **81-176** at different

concentrations;

Figure 7 shows the growth inhibition rate of human breast cancer BT-474 cells treated with **85-71** at different concentrations;

Figure 8 shows the growth inhibition rate of human breast cancer BT-474 cells treated with **84-106** at different concentrations;

Figure 9 shows the growth inhibition rate of human breast cancer BT-474 cells treated with **76-207** at different concentrations;

Figure 10 shows the growth inhibition rate of human breast cancer BT-474 cells treated with **75-196** at different concentrations;

Figure 11 shows the growth inhibition rate of human breast cancer BT-474 cells treated with **81-176** at different concentrations;

Figure 12 shows the detection results of the compounds of the present invention on tumor volume in a subcutaneous xenograft model of human breast cancer BT474 cells in NPG mice;

Figure 13 shows the detection results of the compounds of the present invention on tumor weight in a subcutaneous xenograft model of human breast cancer BT474 cells in NPG mice;

Figure 14 shows the detection results of the compounds of the present invention on body weight of tumor-bearing mice in a nude mouse xenograft model (subcutaneous tumors) of lung cancer A-549 cells;

Figure 15 shows the detection results of the compounds of the present invention on tumor volume in a nude mouse xenograft model (subcutaneous tumors) of lung cancer A-549 cells.

Figure 16 shows the detection results of the compounds of the present invention on tumor inhibition rate in a nude mouse xenograft model (subcutaneous tumors) of lung cancer A-549 cells;

Figure 17 shows the detection results of the compounds of the present invention on body weight of tumor-bearing mice in a nude mouse model of breast cancer MDA-MB-231 cells (subcutaneous tumors);

Figure 18 shows the detection results of the compounds of the present invention on tumor volume in a nude mouse model of breast cancer MDA-MB-231 cells (subcutaneous tumors);

Figure 19 shows the tumor inhibition rates of the compounds of the present invention in a nude mouse model of breast cancer MDA-MB-231 cells (subcutaneous tumors).

## Specific Models for Carrying Out the present invention

The embodiments of the present invention are described in detail in conjugation with the examples below. However, those skilled in the art will appreciate that the following examples are intended to illustrate the present invention only and should not be construed as limiting the scope of the invention. For examples, where specific conditions are not specified, the experiments were conducted under conventional conditions or manufacturer’s recommended conditions. All reagents and instruments used, for which the manufacturer was not specified, were commercially available conventional products.

Example 1: Synthesis of Compound **71-215**

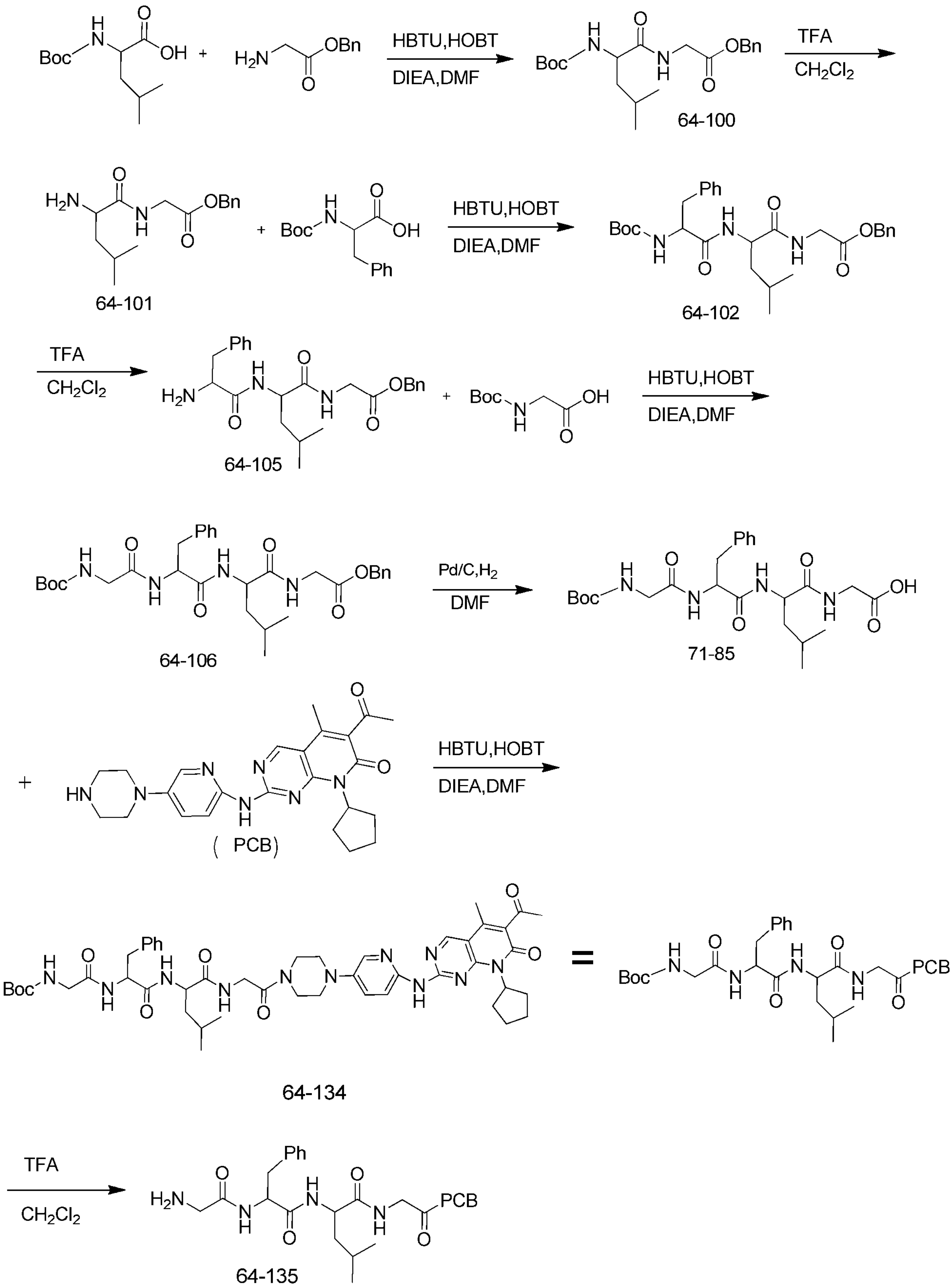


64-100

64-101

64-102

64-105

64-106

71-85

( PCB)

64-134

64-135

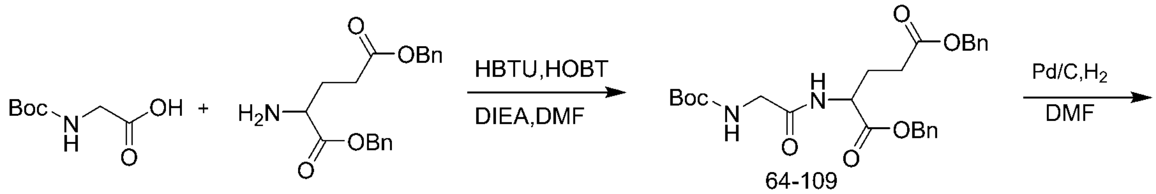


64-109

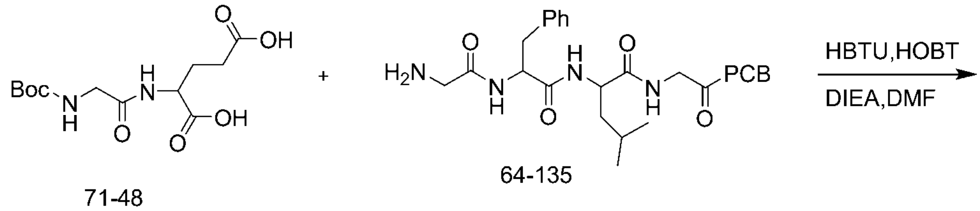


71-48 64-135

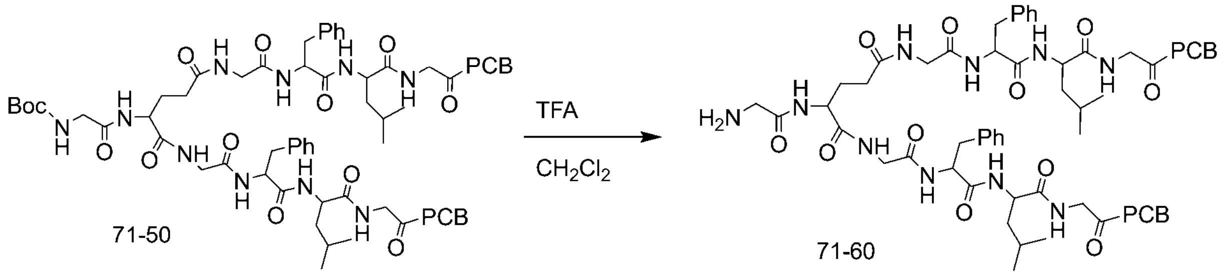


71-50 71-60

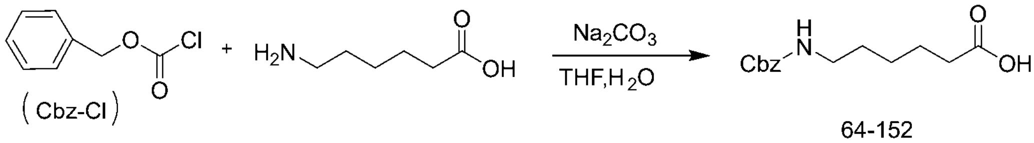


(Cbz-Cl) 64-152

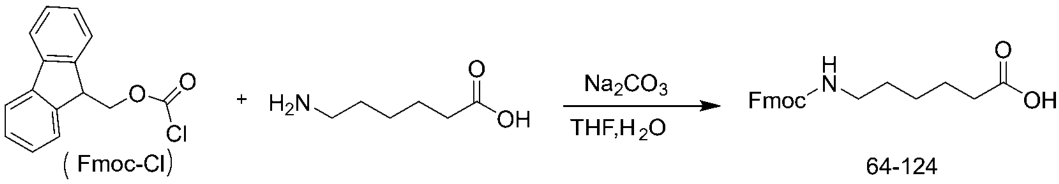


(Fmoc-Cl) 64-124

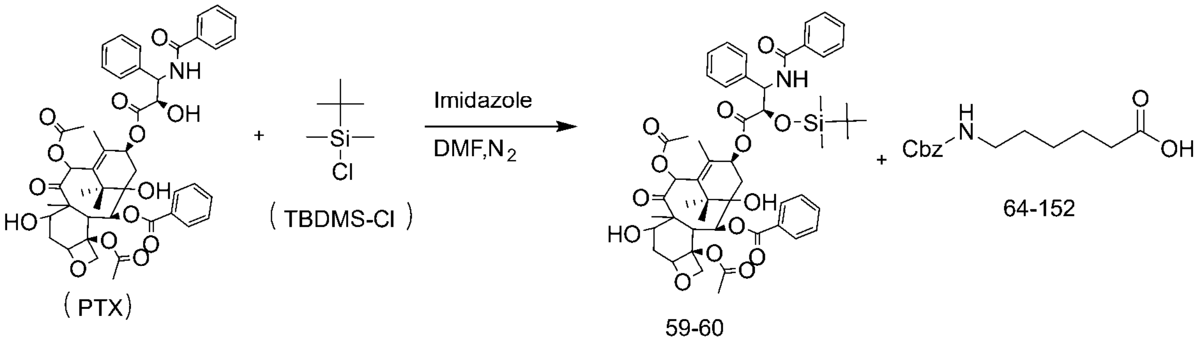


(PTX) (TBDMS-Cl) 64-152

59-60

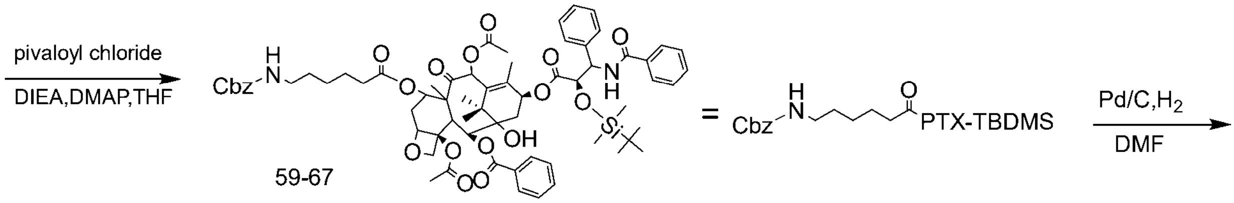


59-67

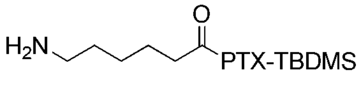

59-69

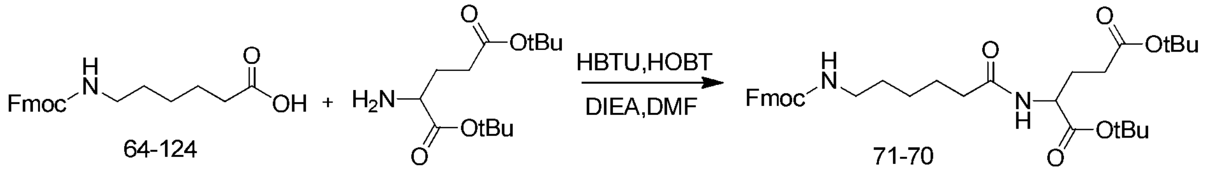


64-124

71-70

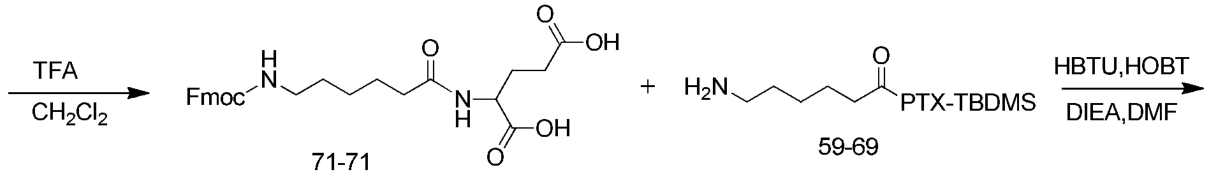


71-71

59-69

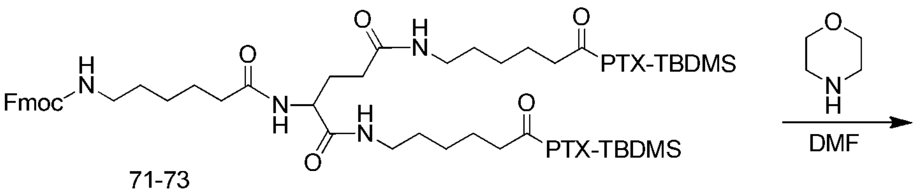


71-73

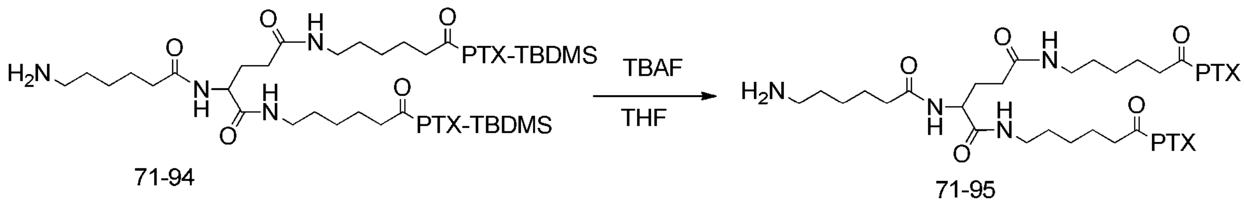


71-94

71-95

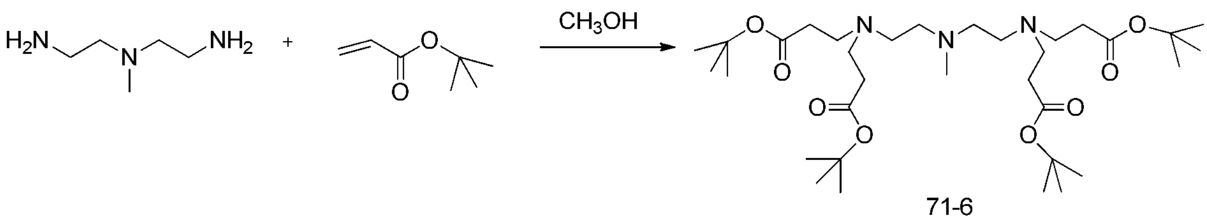


71-6

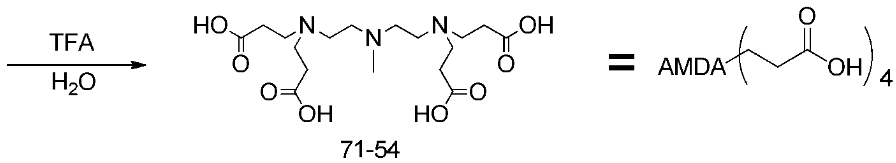


71-54

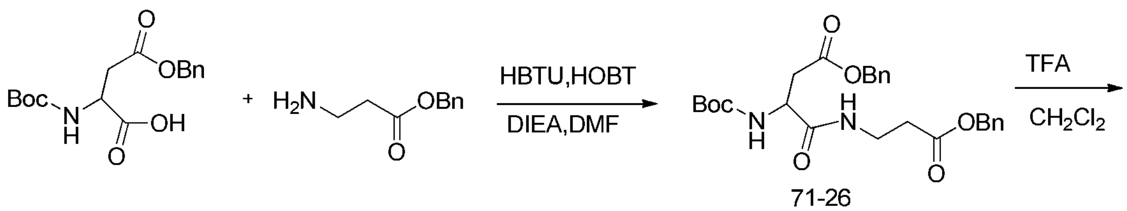


71-26

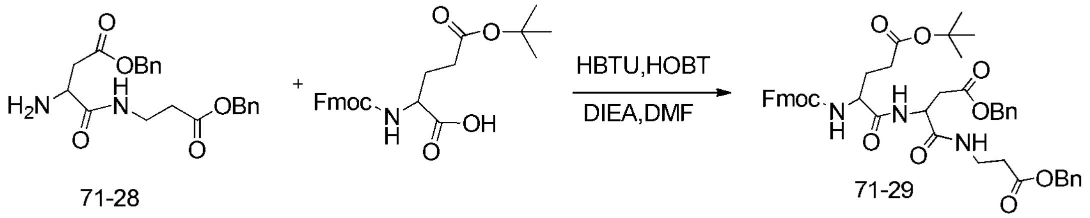


71-28

71-29

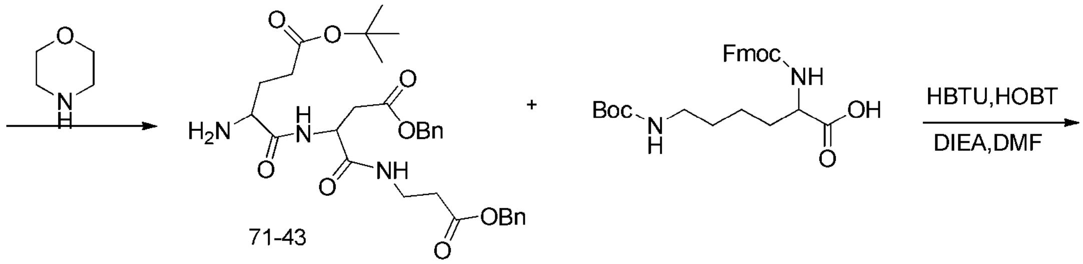


71-43

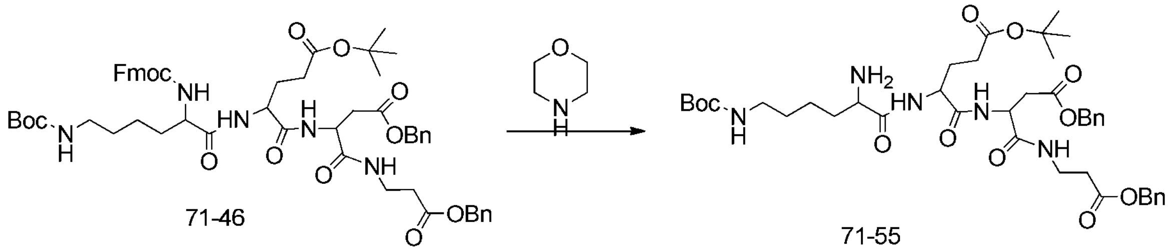

71-46

71-55

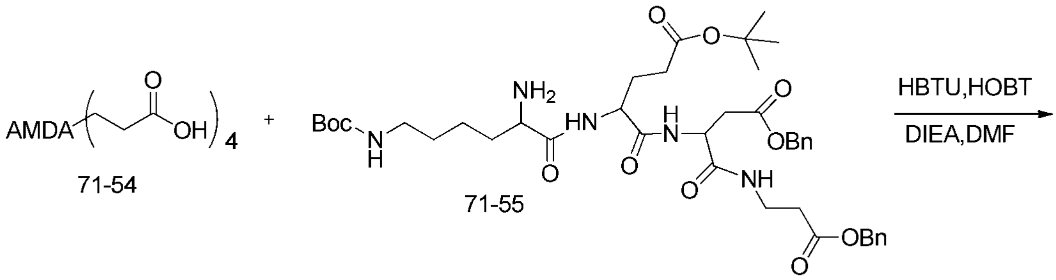


71-54

71-55

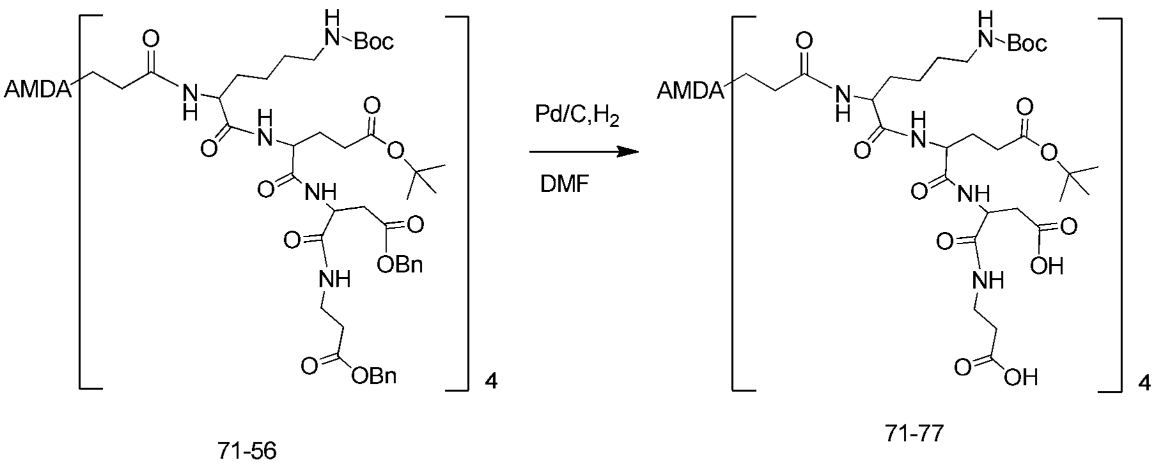


71-56

71-77

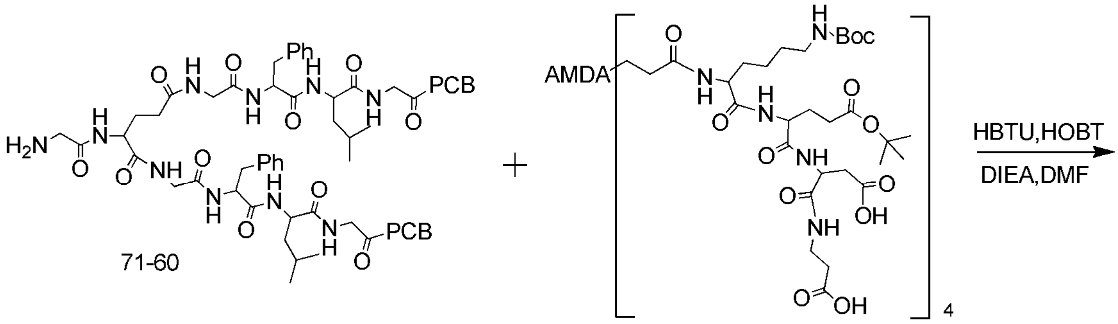


71-60

71-77

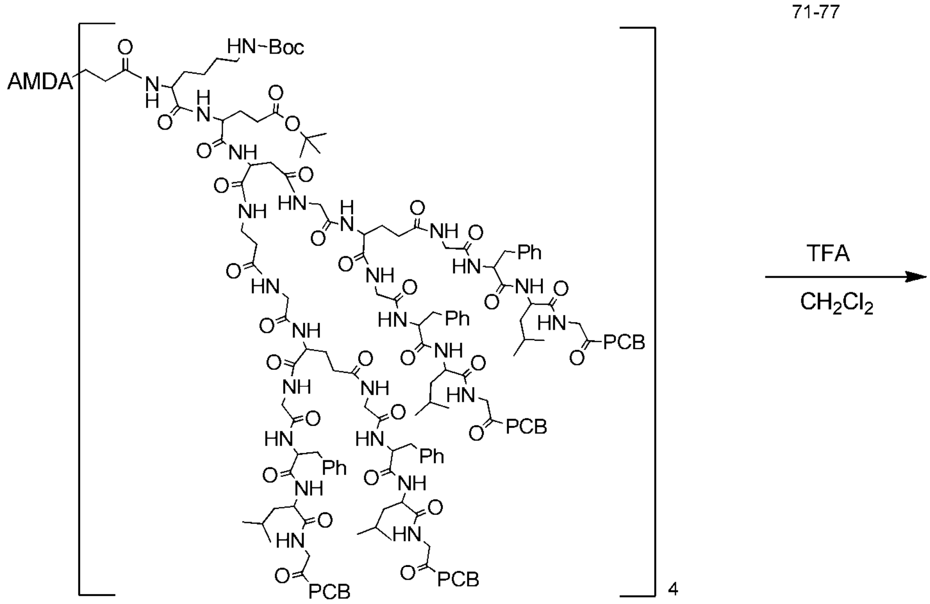


71-82

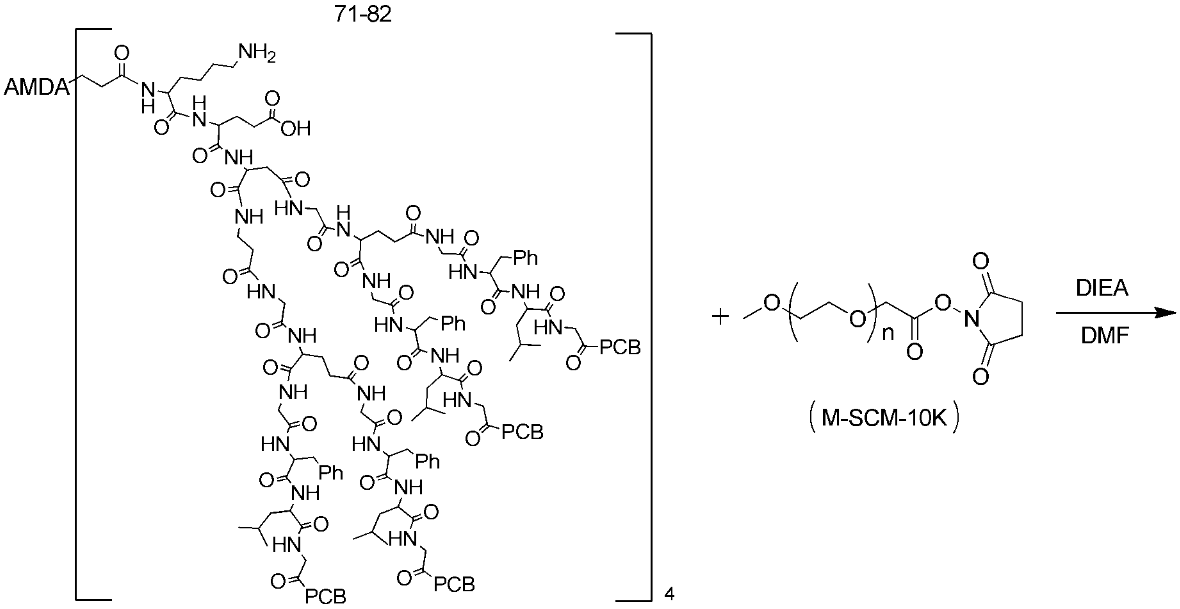


(M-SCM-10K)

71-86

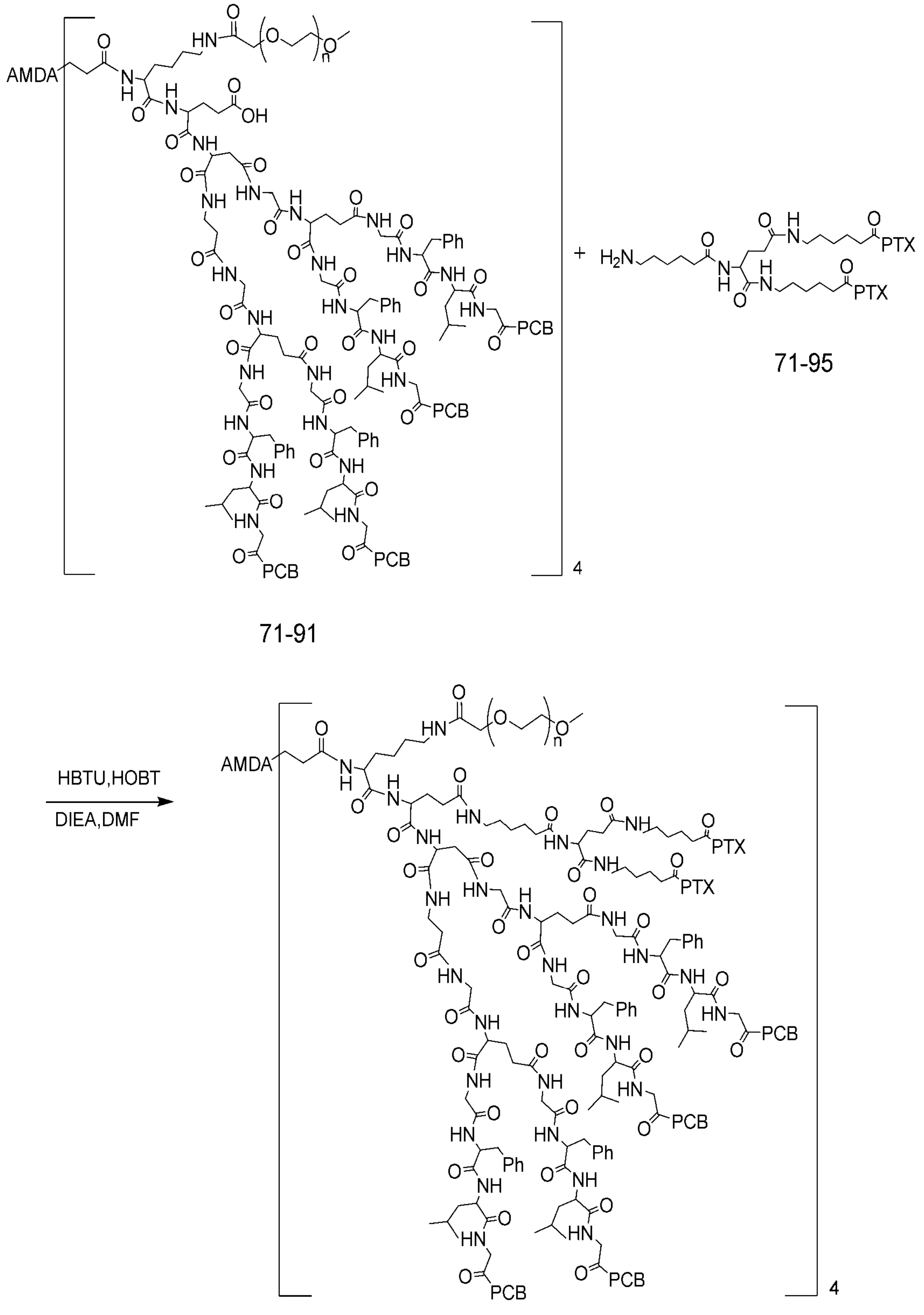


71-91

71-95

71-215

1.1 Preparation of **64-100**

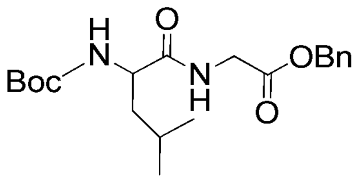

Boc-L-Leucine (30 g, 129.7073 mmol, purchased from Innochem), benzyl glycine hydrochloride (28.7710 g, 142.6780 mmol, purchased from Ark Pharm), HBTU (73.7853 g, 194.5610 mmol, purchased from Aladdin), and HOBT (26.2891 g, 194.5610 mmol, purchased from Aladdin) were added to a 1000 mL flask, then DMF (250 mL) was added to dissolve the mixture, and the mixture was stirred at -5°C for approximately 20 minutes. DIEA (96.5 mL, 583.6829 mmol) was then slowly added dropwise. After the dropwise addition was completed, stirring was continued at -5°C for 1 hour, and the mixture was then brought to room temperature and reacted with stirring. After the reaction was completed, the reaction mixture was transferred to a 2 L separatory funnel containing saturated sodium bicarbonate solution (250 mL) and ethyl acetate (300 mL) to perform extraction. The organic phase was collected, and the aqueous phase was further extracted with ethyl acetate (200 mL × 3). The organic phases were combined, washed with saturated brine (200 mL × 2), concentrated, and dried to provide 50.9873 g of the product.

1.2 Preparation of **64-101**

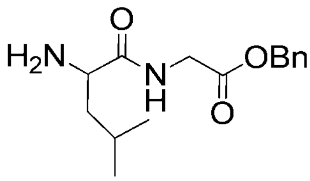

**64-100** (49.0890 g, 129.7073 mmol) was placed in a 1 L flask, and dichloromethane (60 mL) was added for dissolution, then TFA (96.3 mL, 1297.073 mmol) was added while stirring. The reaction flask was finally allowed to stand at room temperature and the reaction was stirred overnight. After the reaction was completed, the reaction mixture was transferred to a 2 L separatory funnel containing saturated sodium bicarbonate solution (350 mL) and ethyl acetate (300 mL) for extraction. The organic phase was collected, and the aqueous phase was further extracted with ethyl acetate (1500 mL × 2). The organic phases were combined, concentrated, and evaporated to dryness to provide 29.3 g of the product.

1.3 Preparation of **64-102**

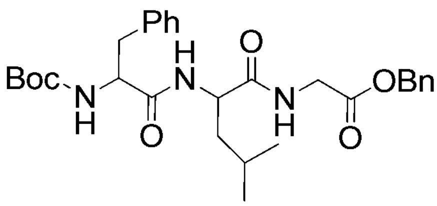

**64-101** (29.3 g, 105.2632 mmol), Boc-L-phenylalanine (33.5116 g, 126.3158 mmol, purchased from Accela), HBTU (59.8800 g, 157.8948 mmol), and HOBT (21.3347 g, 157.8948 mmol) were added to a 1 L flask, then DMF (200 mL) was added to dissolve the mixture, and the mixture was stirred at -5°C for approximately 20 minutes. DIEA (104.4 mL, 631.5792 mmol) was then slowly added dropwise. After the dropwise addition was completed, stirring was continued at -5°C for 1 hour, and then the mixture was brought to room temperature and the reaction was carried out with stirring overnight. After the reaction was completed, the reaction mixture was transferred to a 2 L separatory funnel containing saturated sodium bicarbonate solution (350 mL) and ethyl acetate (300 mL) for extraction. The organic phase was collected, and the aqueous phase was further extracted with ethyl acetate (200 mL × 3). The organic phases were combined, washed with saturated brine (250 mL × 2), concentrated, and evaporated to dryness to provide 49.4 g of the product.

1.4 Preparation of 64-105

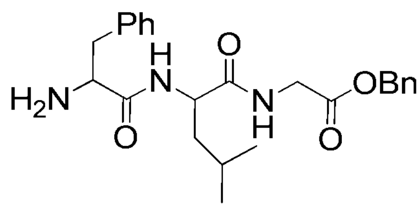

**64-102** (49.4 g, 93.9807 mmol) was placed in a 1 L flask, and dichloromethane (60 mL) was added for dissolution, then TFA (71.0 mL, 939.807 mmol) was added with stirring. The reaction flask was finally allowed to stand at room temperature, and the reaction was stirred overnight. After the reaction was completed, the reaction mixture was transferred to a 2 L separatory funnel containing saturated sodium bicarbonate solution (350 mL) and ethyl acetate (300 mL) for extraction. The organic phase was collected, and the aqueous phase was further extracted with ethyl acetate (150 mL × 2). The organic phases were combined, concentrated, and evaporated to dryness to provide 32.7 g of the product.

1.5 Preparation of **64-106**

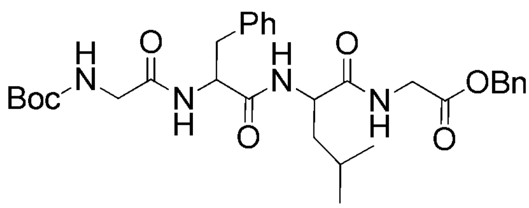

**64-105** (32.7 g, 76.8472 mmol), Boc-Gly-OH (16.1545 g, 92.2166 mmol, purchased from Ark Pharm), HBTU (45.7153 g, 115.2708 mmol), and HOBT (15.5754 g, 115.2708 mmol) were added to a 1 L flask, then DMF (200 mL) was added to dissolve the mixture, and the mixture was stirred at -5°C for approximately 20 minutes. DIEA (76.2 mL, 461.0832 mmol) was then slowly added dropwise. After the dropwise addition was completed, stirring was continued at -5°C for 1 hour, and then the mixture was brought to room temperature and the reaction was carried out with stirring overnight. After the reaction was completed, the reaction mixture was transferred to a 2 L separatory funnel containing saturated sodium bicarbonate solution (300 mL) and ethyl acetate (350 mL) for extraction. The organic phase was collected, and the aqueous phase was further extracted with ethyl acetate (200 mL × 3). The organic phases were combined and washed with saturated brine (250 mL × 2). After concentration, the organic phase was allowed to stand at room temperature for 1.5 hours to precipitate a solid. The solid was filtered, and the filter cake was washed with an ethyl acetate/petroleum ether (3/7) mixture (150 mL × 5) to provide 34.6 g of the product.

1.6 Preparation of **71-85**

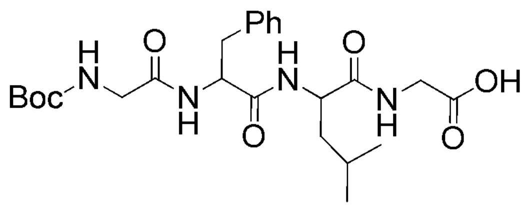

**64-106** (10 g, 17.1618 mmol) was added to a hydrogenation reactor, then 10% Pd/C catalyst (0.1 g) was added, and DMF (40 mL) was added for dissolution. The hydrogenation reactor was sealed, evacuated with a water pump, and then filled with hydrogen gas. This process was repeated three times. The pressure of the hydrogenation reactor was

adjusted to 2 MPa, and the mixture was stirred at room temperature overnight. After the reaction was completed, the reaction mixture was filtered through Celite, and the filter cake was washed with DMF (15 mL × 3). The filtrate was placed in a 500 mL round-bottom flask and used as the raw material for the next step.

1.7 Preparation of **64-134**

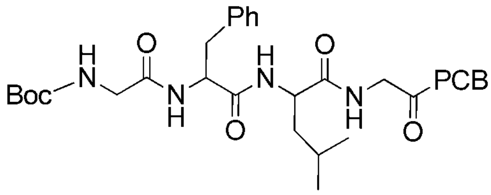

**71-85** (8.4534 g, 17.1618 mmol), PCB (6.4012 g, 14.3015 mmol, purchased from Tianjin Pharmacn), HBTU (8.1355 g, 21.4523 mmol), and HOBT (2.8986 g, 21.4523 mmol) were added to a 500 mL round-bottom flask, then DMF (80 mL) was added to dissolve the mixture, and the mixture was stirred at -5°C for approximately 20 minutes. DIEA (11.8 mL, 71.5076 mmol) was then slowly added dropwise, and stirring was continued at -5°C for 1 hour. The flask was then taken out, and brought to room temperature, and the reaction was carried out with stirring. After the reaction was completed, the reaction mixture was transferred to a 1 L separatory funnel containing deionized water (300 mL) and ethyl acetate (200 mL) for extraction. The organic phase (with precipitated solid) was collected, and the aqueous phase was further extracted with ethyl acetate (200 mL × 2). The organic phases were combined, and concentrated by rotary evaporation under reduced pressure. The solid was washed with deionized water (200 mL) and filtered. The filter cake was washed with n-hexane (40 mL × 4) and methyl tert-butyl ether (10 mL × 4). The filter cake was collected and dried to provide 13.7 g of the product.

1.8 Preparation of **64-135**

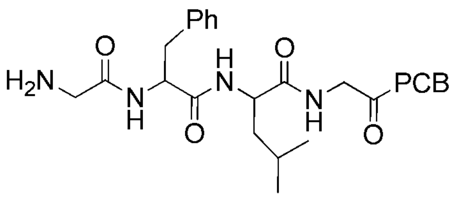

To a flask containing **64-134** (13.1871 g, 14.3015 mmol), dichloromethane (30 mL) was added for dissolution with the assistance of ultrasonication, then TFA (10.6 mL, 143.015 mmol) was added, and the mixture was reacted with stirring at room temperature overnight. After the reaction was completed, the reaction mixture was concentrated and evaporated to dryness to remove dichloromethane and most of the TFA. Methyl tert-butyl ether (300 mL) was added for precipitation. A solid product precipitated and was filtered. The filter cake was washed with methyl tert-butyl ether (40 mL × 3). The filter cake was collected and dried under vacuum to provide 16.6 g of the product.

1.9 Preparation of **64-109**

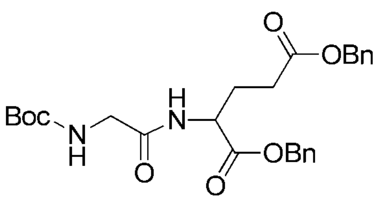

Boc-glycine (10.0 g, 57.0841 mmol, purchased from Aladdin), H-Glu(Obzl)-Obzl.TsOH (31.3699 g, 62.7926

mmol, purchased from Ark Pharm), HBTU (32.4729 g, 85.6262 mmol), and HOBT (11.5698 g, 85.6262 mmol) were added to a 1 L round-bottom flask, then DMF (180 mL) was added for dissolution, and the mixture was stirred at -5°C for approximately 20 minutes. DIEA (42.5 mL, 256.8785 mmol) was then slowly added dropwise. After the dropwise addition was completed, the reaction flask was allowed to stand at -5°C and the reaction was carried out with stirring for 1 hour, then the reaction flask was taken out and brought to room temperature, and the stirring was continued. After the reaction was completed, the reaction mixture was transferred to a 2 L separatory funnel containing saturated brine (400 mL) and ethyl acetate (300 mL) for extraction. The organic phase was collected, and the aqueous phase was further extracted with ethyl acetate (200 mL). The organic phases were combined, washed with deionized water (250 mL × 2), concentrated and evaporated to dryness, and then dissolved in a 20% methanol/dichloromethane mixture. 200-300 mesh silica gel powder was added. The mixture was dry-loaded onto the column and subjected to column chromatography using a 0-40% ethyl acetate/petroleum ether mixture as the eluent. The product fraction was collected and evaporated to dryness to provide 27.1661 g of the product.

1.10 Preparation of **71-48**

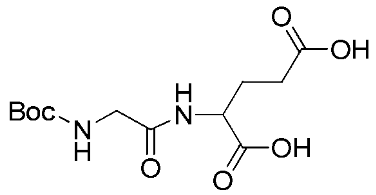

**64-109** (2.3624 g, 4.8753 mmol) and 10% Pd/C catalyst (0.04 g) were added to a hydrogenation reactor, and then DMF (40 mL) was added for dissolution. The hydrogenation reactor was sealed, evacuated with a water pump, and then filled with hydrogen gas. This process was repeated three times. The pressure of the hydrogenation reactor was adjusted to 2 MPa, and stirring was performed at room temperature overnight. After the reaction was completed, the reaction mixture was filtered through Celite, the filter cake was rinsed with DMF (15 mL × 3), and the filtrate was transferred to a 500 mL round-bottom flask to serve as the raw material for the next step.

1.11 Preparation of **71-50**

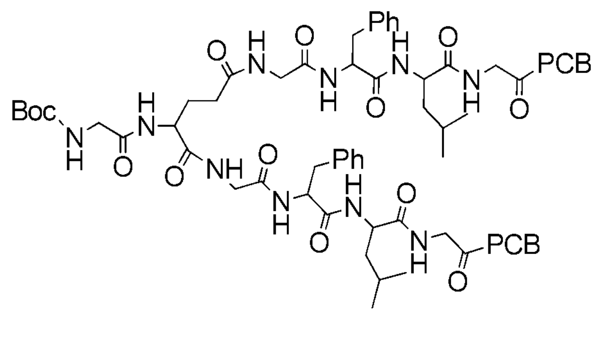

**71-48** (1.4836 g, 4.8753 mmol), **64-135** (8.8162 g, 10.7257 mmol), HBTU (5.5467 g, 14.6259 mmol), and HOBT (1.9763 g, 14.6259 mmol) were placed in a 500 mL flask, then DMF (40 mL) was added for dissolution, and the mixture was stirred at -5°C for approximately 20 minutes. DIEA (9.6 mL, 58.5036 mmol) was then slowly added dropwise. After the dropwise addition was completed, the reaction was continued at -5°C for 1 hour. Then the flask was taken out and brought to room temperature, and the stirring was continued. After the reaction was completed, n-hexane (300 mL) and methyl tert-butyl ether (50 mL) were added for precipitation, and the supernatant was discarded. This process was repeated five times. A solid product was obtained and filtered. The solid product was collected and dried in a vacuum oven to provide 8.9 g of the product. $^1$H-NMR (600MHz, DMSO-$d_6$) δ 8.08-7.90 (m, 10H), 7.51-6.90 (m, 18H), 4.56-4.58 (m, 2H), 4.37-4.36 (m, 3H), 4.22-3.97 (m, 8H), 3.70-3.68 (m, 2H), 3.63-3.59 (m, 12H), 3.20-3.08 (m, 12H), 2.43 (s, 6H), 2.31 (s, 6H), 2.25-2.24 (m, 4H), 1.92-1.84 (m, 6H), 1.79-1.72 (m, 6H), 1.59-1.58 (m, 6H), 1.51 (s, 4H), 1.36 (s, 9H), 0.9-0.84 (m, 12H)

1.12 Preparation of **71-60**

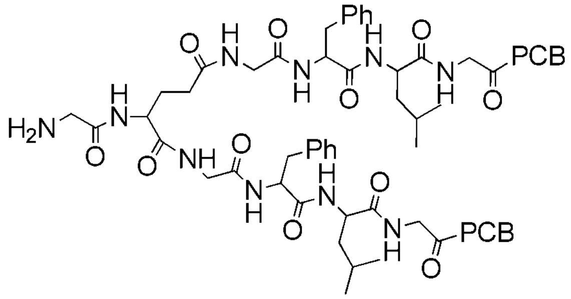

To a flask containing **71-50** (8.9 g, 4.6543 mmol), dichloromethane (30 mL) was added for dissolution with the assistance of ultrasonication, then TFA (3.5 mL, 46.543 mmol) was added, and the mixture was stirred at room temperature overnight. After the reaction was completed, the reaction mixture was concentrated and evaporated to dryness to remove dichloromethane and most of the TFA. Methyl tert-butyl ether (300 mL) was added for precipitation. A solid product precipitated and was filtered. The filter cake was washed with methyl tert-butyl ether (40 mL × 3). The filter cake was collected and dried under vacuum to provide 8.7 g of the product.

1.13 Preparation of **64-152**

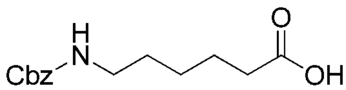

6-Aminohexanoic acid (9.19 g, 70.06 mmol, purchased from Innochem) was placed in a 1 L round-bottom flask, then a THF/$H_2O$ (1/1) solution (200 mL) was added for dissolution, and the mixture was stirred in a water bath at 0°C for 1 hour. Anhydrous sodium carbonate (14.85 g, 140.12 mmol) was then added, dissolved with the assistance of ultrasonication, and stirred in a water bath at 0°C for 30 minutes. Cbz-Cl (12.5494 g, 73.56 mmol) was then slowly added dropwise to the reaction flask. After the dropwise addition was completed, the reaction flask was taken out from the water bath and brought to room temperature, and stirring was continued for 2.5 hours. Finally, 1N aqueous hydrochloric acid (115 mL) was added dropwise into the reaction flask to adjust the pH of the reaction mixture to 3.0. After the reaction was completed, the reaction mixture was transferred to a 1 L separatory funnel and extracted with ethyl acetate (130 mL). The organic phase was collected, and the aqueous phase was further extracted with ethyl acetate (200 mL). The organic phases were combined, washed with 1N hydrochloric acid (200 mL × 2), concentrated, and evaporated to dryness. The residue was then dissolved in dichloromethane (100 mL). 200-300 mesh silica gel powder was added and the mixture was evaporated to dryness. The resulting product was dry-loaded onto the column and subjected to column chromatography using a 15-30% ethyl acetate/petroleum ether mixture as the eluent. The product fraction was collected and evaporated to dryness to provide 11.6 g of the product with a yield of 62.41%.

1.14 Preparation of **64-124**

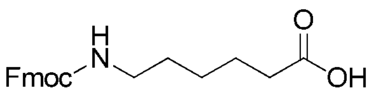

6-Aminohexanoic acid (4.6094 g, 35.1407 mmol) was placed in a 1 L round-bottom flask, then a THF/$H_2O$ (1/1) solution (150 mL) was added for dissolution, and the mixture was stirred in a water bath at 0°C for 1 hour. Anhydrous sodium carbonate (7.4491 g, 70.2814 mmol) was then added to the reaction flask, dissolved with the assistance of ultrasonication, and stirred in a water bath at 0°C for 30 minutes. 9-Fluorenylmethyl chloroformate (Fmoc-Cl, 10.0 g, 38.6548 mmol) was then dissolved in THF (30 mL) and slowly added dropwise to the reaction flask. After the dropwise

addition was completed, the reaction flask was taken out from the water bath and brought to room temperature, and stirring was continued for 2.5 hours. Finally, 1.0 M aqueous hydrochloric acid (115 mL) was added dropwise to the reaction flask to adjust the pH to 3.0. After the reaction was completed, the reaction mixture was transferred to a 1 L separatory funnel and extracted with ethyl acetate (150 mL). The organic phase was collected, and the aqueous phase was further extracted with ethyl acetate (200 mL). The organic phases were combined, washed with 1.0 M aqueous hydrochloric acid (250 mL × 2), and concentrated and evaporated to dryness. The residue was then dissolved in dichloromethane (100 mL), then 200-300 mesh silica gel powder was added, and the mixture was evaporated to dryness. The resulting product was dry-loaded onto the column and subjected to column chromatography using a 20-50% ethyl acetate/petroleum ether mixture as the eluent. The product fraction was collected and evaporated to dryness to provide 12.0 g of the product with a yield of 96.63%.

1.15 Preparation of 59-60

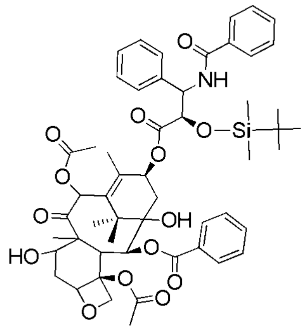

Paclitaxel (20 g, 23.42 mmol, purchased from Wuhan Yingyuanbei, abbreviated as PTX) and imidazole (7.98 g, 117.12 mmol, purchased from Innochem) were dissolved in DMA (50 mL) with the assistance of ultrasonication. $N_2$ was then introduced to expel the air. TBDMS-Cl (21.2 g, 142.54 mmol, purchased from Innochem) was then added, the nitrogen flow was stopped, and the reaction was carried out with stirring at room temperature. After the reaction was completed, the reaction mixture was transferred to a 1 L separatory funnel containing ethyl acetate (200 mL) and purified water (200 mL) for extraction. The organic phase was collected, and the aqueous phase was further extracted with ethyl acetate (150 mL × 2). The organic phases were combined, washed with saturated brine (100 mL × 2), concentrated, and evaporated to dryness to provide 22.7 g of the product.

1.16 Preparation of **59-67**

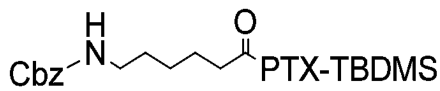

**59-60** (2 g, 7.8387 mmol) was dissolved in dichloromethane (35 mL) and stirred at 0°C. DIEA (1.5 mL, 9.04569 mmol) was then slowly added dropwise. After the dropwise addition was completed, pivaloyl chloride (1.1 mL, 9.04569 mmol) was added dropwise. After the dropwise addition was completed, the mixture was stirred at room temperature for 3 hours. The reaction progress was monitored by TLC. The reaction mixture was then stirred at 0°C. **64-152** (3.65 g, 3.7693 mmol) was dissolved in 20 mL of dichloromethane and slowly added dropwise to the reaction mixture. DIEA (0.69 mL, 4.1459 mmol) was then added dropwise. After the dropwise addition was completed, DMAP (0.57 g, 4.64805 mmol) was added. The reaction flask was allowed to stand at room temperature, and the reaction was carried out with stirring. After the reaction was completed, the mixture was evaporated to dryness using a vacuum rotary evaporator. Ethyl acetate (200 mL) was added for dissolution, and the mixture was transferred to a 2 L separatory funnel containing pure water (300 mL) for extraction. The organic phase was obtained, and the aqueous phase was further extracted with ethyl acetate (100 mL × 2). The organic phases were combined, concentrated to 150 mL, washed with dilute hydrochloric acid (200 mL × 2), and then washed with saturated sodium bicarbonate solution (200 mL × 2). The organic phase was collected and evaporated to dryness to provide 4.58 g of the product.

1.17 Preparation of Compound **59-69**

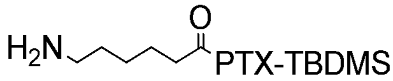

**59-67** (4.58 g, 3.769 mmol) was dissolved in 90 mL of activated carbon-treated DMA in a hydrogenation reactor. 10% Pd/C catalyst (80 mg) was added. The hydrogenation reactor was sealed, evacuated with a water pump, and then filled with hydrogen gas. This process was repeated three times. The pressure in the hydrogenation reactor was adjusted to 1.8 MPa, and the reaction was carried out with stirring at high speed at room temperature. After the reaction was completed, the reaction mixture was filtered through Celite, and the filter cake was washed with DMA (10 mL × 3). The filtrate was poured into a 1 L separatory funnel containing pure water (300 mL) and ethyl acetate (300 mL) for extraction. The organic phase was collected, and the aqueous phase was further extracted with ethyl acetate (100 mL × 2). The organic phases were combined, and washed with saturated brine (200 mL × 3). The organic phases were collected and evaporated to dryness. The resulting solid was dissolved in a dichloromethane/methanol (4/1) mixture, then silica gel powder was added, and the mixture was evaporated to dryness. The resulting product was dry-loaded onto the column and subjected to column chromatography using a 0.2% triethylamine/3% methanol/dichloromethane mixture as the eluent. The product fraction was collected and evaporated to dryness to provide 4.08 g of the product.

1.18 Preparation of **71-70**

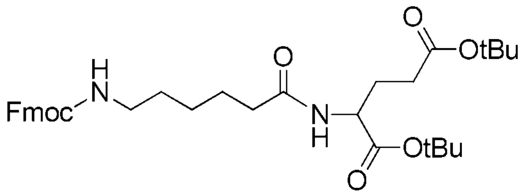

**64-124** (6.5712 g, 18.5936 mmol), di-tert-butyl L-glutamate hydrochloride (5.0 g, 16.9033 mmol, purchased from Innochem), HBTU (7.0514 g, 18.5936 mmol), and HOBT (2.5124 g, 18.5936 mmol) were added to a 1 L round-bottom flask, and DMF (70 mL) was added for dissolution. The reaction flask was allowed to stand at -5°C and the mixture was stirred for approximately 20 minutes. DIEA (6.1 mL, 37.1873 mmol) was then slowly added dropwise. After the dropwise addition was completed, the reaction was carried out with stirring at -5°C for 1 hour, and then the reaction flask was brought to room temperature and stirring continued. After the reaction was completed, the reaction mixture was transferred to a 1 L separatory funnel containing saturated brine (250 mL) and ethyl acetate (200 mL) for extraction. The organic phase was collected, and the aqueous phase was further extracted with ethyl acetate (200 mL). The organic phases were combined, washed with deionized water (250 mL × 2), concentrated and evaporated to dryness, and then dissolved in a 20% methanol/dichloromethane mixture. Silica gel powder was added, and the mixture was evaporated to dryness. The resulting product was dry-loaded onto the column and subjected to column chromatography using a 0-50% ethyl acetate/petroleum ether mixture as the eluent. The product fraction was collected, concentrated, and evaporated to dryness to provide 9.06 g of the product with a yield of 90.13%.

1.19 Preparation of **71-71**

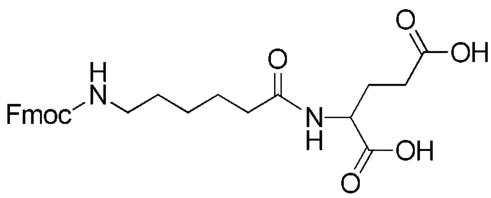

To a flask containing **71-70** (5.0 g, 8.4070 mmol), dichloromethane (20 mL) was added for dissolution with the

assistance of ultrasonication. TFA (12.5 mL, 168.1407 mmol) was added, and the mixture was stirred at room temperature overnight. After the reaction was completed, the reaction mixture was evaporated to dryness to remove dichloromethane and most of the TFA. Then, n-hexane (30 mL) and methyl tert-butyl ether (200 mL) were added for precipitation. The supernatant was disclosed, resulting in an oily solid. The solid was dissolved in ethyl acetate (20 mL), and then methyl tert-butyl ether (100 mL) was added for precipitation. The solid product was filtered to provide a solid product. This process was repeated six times. The filter cake was collected and vacuum dried to provide 4.5526 g of the product.

1.20 Preparation of **71-73**

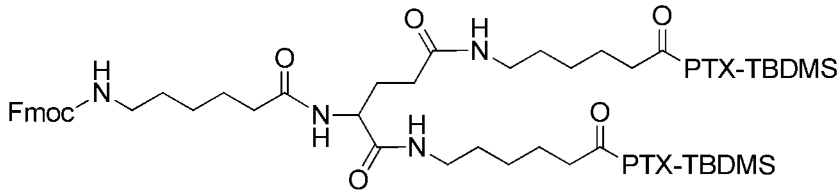

**71-71** (0.8276 g, 1.7151 mmol), **59-69** (4.08 g, 3.7732 mmol), HBTU (1.9513 g, 5.1453 mmol), and HOBT (0.6952 g, 5.1453 mmol) were added to a 1 L round-bottom flask, and DMF (70 mL) was added for dissolution. The reaction flask was allowed to stand at -5°C, and the mixture was stirred for approximately 20 minutes. DIEA (2.3 mL, 13.7208 mmol) was then slowly added dropwise. After the dropwise addition was completed, the reaction flask was allowed to stand at -5°C, the reaction was carried out with stirring for 1 hour, and then the reaction flask was brought to room temperature and the stirring was continued. After the reaction was completed, the reaction mixture was transferred to a 1 L separatory funnel containing saturated brine (250 mL) and ethyl acetate (200 mL) for extraction. The organic phase was collected, and the aqueous phase was further extracted with ethyl acetate (200 mL). The organic phases were combined, washed with deionized water (250 mL × 2), concentrated and evaporated to dryness, and then dissolved in a dichloromethane/-methanol (4/1) mixture. 200-300 mesh silica gel powder was added, and the mixture was evaporated to dryness. The resulting product was dry-loaded onto the column and subjected to column chromatography using a 1-3% methanol/dichloromethane mixture as the eluent. The product fraction was collected, evaporated to dryness, and dried in a vacuum oven to provide 3.6 g of the product. $^1$H-NMR (600MHz, DMSO-$d_6$) δ 8.0-7.81 (m, 13H), 7.75-7.61 (m, 10H), 7.55-7.47 (m, 10H), 7.43-7.39 (m, 6H), 7.34-7.31 (m, 2H), 7.26-7.20 (m, 3H), 6.04 (s, 2H), 5.88-5.85 (m, 2H), 5.54-5.39 (m, 6H), 5.00-4.98 (m, 2H), 4.77-4.75 (m, 4H), 4.29-4.28 (m, 2H), 4.21-4.15 (m, 2H), 4.07 (s, 2H), 3.75-3.73 (m, 2H), 3.03-2.95 (m, 6H), 2.45 (s, 6H), 2.18-2.03 (m, 16H), 1.65 (s, 10H), 1.57 (s, 6H), 1.48-1.43 (m, 6H), 1.40-1.34 (m, 6H), 1.24-1.20 (m, 12H), 1.02-0.98 (m, 12H), 0.80 (s, 18H), 0.08-0.03 (m, 12H)

1.21 Preparation of **71-94**

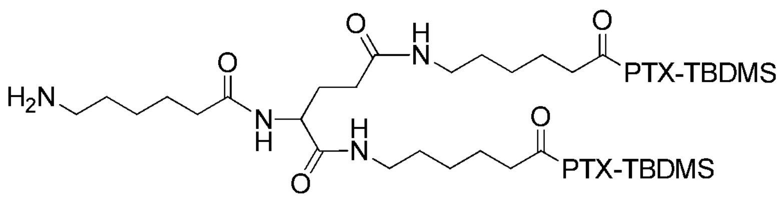

**71-73** (1.0 g, 0.3832 mmol) was added into a 250 mL flask, then DMF (6 mL) was added for dissolution. Morpholine (0.4 mL, 3.832 mmol) was then added and stirred at room temperature for 2 hours. After the reaction was completed, n-hexane (25 mL) and methyl tert-butyl ether (200 mL) were added for precipitation. The supernatant was discarded. This process was repeated four times. The mixture was filtered to provide a solid product, which was dried in a vacuum oven to provide 0.65 g of the product with a yield of 71.42%.

1.22 Preparation of **71-95**

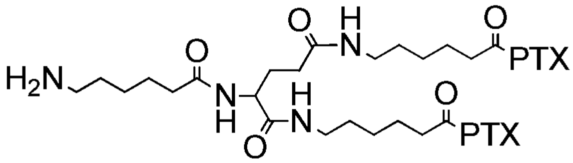

71-94 (0.32 g, 0.1345 mmol) and TBAF (0.85 g, 2.6913 mmol) were placed in a 250 mL flask. Tetrahydrofuran (10 mL) was added to dissolve the product and stirred at room temperature for 3 hours. After the reaction was completed, the reaction mixture was evaporated to dryness using a rotary evaporator under reduced pressure. Ethyl acetate (60 mL) was added to dissolve the mixture, and the mixture was transferred to a 1 L separatory funnel containing deionized water (70 mL × 2) for extraction. The organic phase was evaporated to dryness using a rotary evaporator under reduced pressure and dried in a vacuum oven to provide 0.12 g of the product with a yield of 41.37%.

1.23 Preparation of 71-6

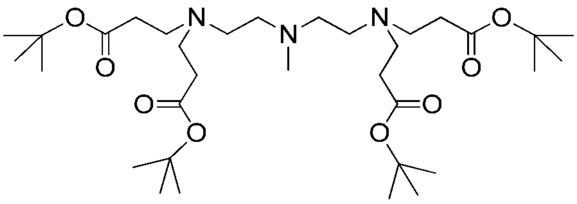

Tert-butyl acrylate (6.5622 g, 51.1992 mmol) was added to a reaction flask, and methanol (15 mL) was added for dissolution. The reaction mixture was then placed in the dark and cooled to 0°C. N-methyl-2,2'-diaminoethylenediamine (1 g, 8.5332 mmol) was dissolved in methanol (5 mL) and slowly added dropwise to the reaction flask. The mixture was then stirred at 1°C for 45 hours. The reaction mixture was then warmed to room temperature and the reaction was continued at room temperature for 24 hours. After the reaction was completed, the reaction mixture was evaporated to dryness under reduced pressure to remove the solvent and excess tert-butyl acrylate, and then n-hexane was added to the reaction mixture for dissolution. The mixture was evaporated to dryness under reduced pressure. This process was repeated 4 times. The resulting solid product was dissolved in a dichloromethane/methanol (4/1) mixture. 200-300 mesh silica gel powder was added, and the mixture was evaporated to dryness. The resulting product was dry-loaded onto the column and subjected to column chromatography using a 0-4% methanol/dichloromethane mixture as the eluent. The product fraction was collected, evaporated to dryness, and dried in a vacuum oven to provide 4.6 g of the product with a yield of 85.69%. $^1$H-NMR (600MHz, DMSO-$d_6$) δ 2.65-2.63 (m, 8H), 2.51-2.50 (m, 1H), 2.46-2.44 (m, 4H), 2.37 (s, 3H), 2.29-2.27 (m, 8H), 2.16 (s, 3H), 1.39 (s, 36H)

1.24 Preparation of **71-54**

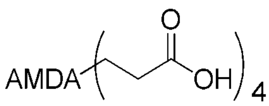

**71-6** (1.0 g, 1.5876 mmol) was added to a 100 mL flask, then 1,4-dioxane hydrochloride (31.7 mL, 127.008 mmol) was added for dissolution, and the mixture was reacted with stirring in a water bath at 50°C overnight. After the reaction was completed, the reaction mixture was evaporated to dryness by rotary evaporation under reduced pressure, then dichloromethane (10ml) was added for dissolution, and then the mixture was evaporated again to dryness under reduced pressure. This process was repeated three times to provide a solid, which was then dried in an oven to provide 0.64g of the product.

1.25 Preparation of **71-26**

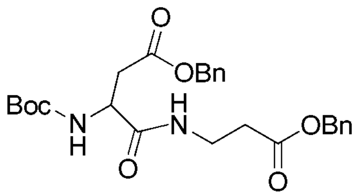

Boc-Asp(Obzl.)-OH (10.2010 g, 31.5488 mmol), H-β-Ala-Obzl.TsOH (11.0868 g, 31.5488 mmol), HBTU (13.161 g, 34.7036 mmol), and HOBT (4.6892 g, 34.7036 mmol) were added to a 500 mL round-bottom flask, then DMF (40 mL) was added for dissolution, and the mixture was stirred at -5°C for approximately 20 minutes. DIEA (11.4 mL, 69.4072 mmol) was then slowly added dropwise. The reaction was carried out with stirring at -5°C for 1 hour, and then with stirring at room temperature overnight. After the reaction was completed, the reaction mixture was transferred to a 1 L separatory funnel containing saturated sodium bicarbonate solution (300 mL) and ethyl acetate (200 mL) for extraction. The organic phase was collected, and the aqueous phase was further extracted with ethyl acetate (200 mL). The organic phases were combined, washed with deionized water (100 mL × 2), concentrated, and evaporated to dryness to provide 15.686 g of the product.

1.26 Preparation of **71-28**

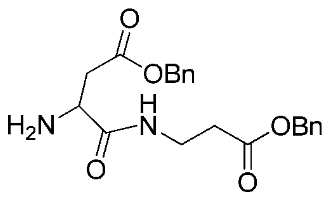

**71-26** (13.7881 g, 28.4560 mmol) was added into a 250 mL round-bottom flask, then dichloromethane (40 mL) was added for dissolution, and TFA (21.1 mL, 284.560 mmol) was added while stirring. Finally, the reaction flask was allowed to stand at room temperature and the reaction was carried out with stirring. After the reaction was completed, the reaction mixture was concentrated and evaporated to dryness to remove dichloromethane and most of the TFA. The reaction mixture was then transferred to a 1 L separatory funnel containing saturated brine (200 mL) and ethyl acetate (200 mL) for extraction. The organic phase was collected, and the aqueous phase was further extracted with ethyl acetate (200 mL). The organic phases were combined, washed with deionized water (200 mL × 2), concentrated, and dried to provide 10.6 g of the product.

1.27 Preparation of **71-29**

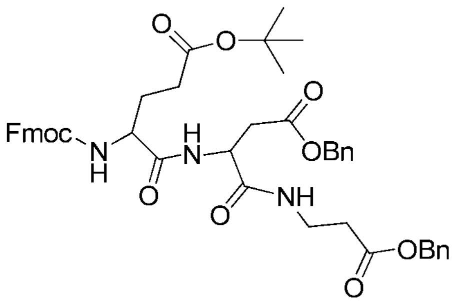

**71-28** (10.6 g, 28.4560 mmol), Fmoc-Glu(OtBu)-OH (13.3178 g, 31.3016 mmol), HBTU (11.8708 g, 31.3016 mmol, purchased from Adamas), and HOBT (4.2295 g, 31.3016 mmol, purchased from Innochem) were placed in 500 mL flask, then DMF (50 mL) was added for dissolution, and the mixture was stirred at -5°C for approximately 20 minutes. DIEA (15.0 mL, 91.0592 mmol) was then slowly added dropwise. The reaction was continued at -5°C for 1 hour. The reaction flask was then brought to room temperature and the stirring was continued overnight. After the reaction was completed, the reaction mixture was transferred to a 1 L separatory funnel containing saturated brine (200 mL) and ethyl acetate (200 mL)

for extraction. The organic phase was collected, and the aqueous phase was further extracted with ethyl acetate (200 mL). The organic phases were combined, washed with deionized water (200 mL × 2) and evaporated to dryness. The resulting solid product was dissolved in a dichloromethane/methanol (4/1) mixture. 200-300 mesh silica gel powder was added. The mixture was evaporated to dryness, dry-loaded onto the column and subjected to column chromatography using a 0-1% methanol/dichloromethane mixture as the eluent. The product fraction was collected, evaporated to dryness, and dried in a vacuum oven to provide 20.9 g of the product.

1.28 Preparation of **71-43**

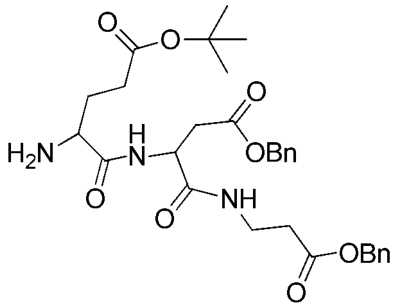

**71-29** (8 g, 10.1025 mmol) was placed in a 500 mL flask. DMF (30 mL) was added for dissolution, then morpholine (8.8 mL) was added, and the mixture was stirred for reaction at room temperature for 1 hour. After the reaction was completed, the reaction mixture was transferred to a 1 L separatory funnel containing saturated brine (200 mL) and ethyl acetate (200 mL) for extraction. The organic phase was collected, and the aqueous phase was further extracted with ethyl acetate (200 mL). The organic phases were combined, washed with deionized water (200 mL × 2), evaporated to dryness, and dried in a vacuum oven to provide 6.2 g of the product.

1.29 Preparation of **71-46**

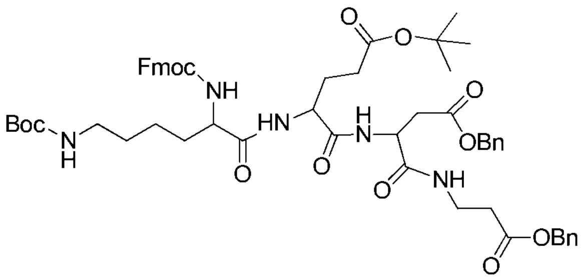

**71-43** (5.7549 g, 10.1025 mmol), Fmoc-Lys(Boc)-OH (5.2068 g, 11.1126 mmol), HBTU (4.2144 g, 11.1126 mmol), and HOBT (1.5016 g, 11.1126 mmol) were placed in a 500 mL flask, then DMF (40 mL) was added for dissolution, and the reaction was stirred at -5°C for approximately 20 minutes. DIEA (3.7 mL, 22.2255 mmol) was then slowly added dropwise, and the reaction was continued at -5°C for 1 hour. The reaction flask was then brought to room temperature, and the mixture was stirred overnight. After the reaction was completed, the reaction mixture was transferred to a 1 L separatory funnel containing saturated brine (200 mL) and ethyl acetate (200 mL) for extraction. The organic phase was obtained, and the aqueous phase was further extracted with ethyl acetate (200 mL). The organic phases were combined, washed with deionized water (200 mL × 2) and evaporated to dryness. The obtained solid product was dissolved in a dichloromethane/methanol (4/1) mixture, then 200-300 mesh silica gel powder was added, and the mixture was evaporated to dryness. The resulting product was dry-loaded onto the column and subjected to column chromatography using a 0-1.5% methanol/dichloromethane mixture as the eluent. The product fraction was collected, evaporated to dryness, and dried in a vacuum oven to provide 8.9 g of the product. $^1$H-NMR (600 MHz, DMSO-$d_6$) δ 8.17 (d, *J* = 7.9 Hz, 1H), 8.05 (d, *J* = 7.4 Hz, 1H), 7.97-7.87 (m, 3H), 7.71 (d, *J* = 16.8 Hz, 2H), 7.37 (d, *J* = 72.0 Hz, 16H), 5.12 (d, *J* = 99.6 Hz, 5H), 4.56 (dd, *J* = 14.1, 7.7 Hz, 1H), 4.26 (d, *J* = 53.6 Hz, 5H), 4.00 (t, *J* = 26.4 Hz, 2H), 3.28 (d, *J* = 44.2 Hz, 2H), 2.74 (d, *J* = 202.6 Hz, 5H), 2.22 (d, *J* = 54.9 Hz, 2H), 1.87 (d, *J* = 35.3 Hz, 1H), 1.74 (d, *J* = 37.5 Hz, 1H), 1.61 (d, *J* = 27.6 Hz, 1H), 1.52 (d, *J* =

36.1 Hz, 1H), 1.35 (d, *J* = 12.8 Hz, 18H), 1.22 (d, *J* = 11.9 Hz, 2H)

1.30 Preparation of **71-55**

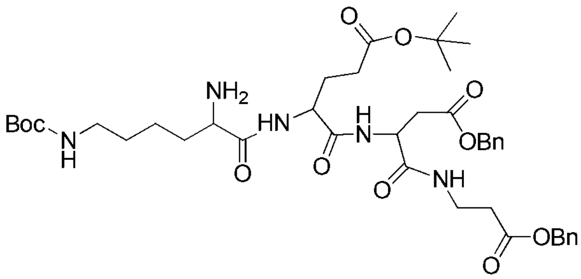

**71-46** (5 g, 4.9011 mmol) was placed in a 250 mL flask. DMF (20 mL) was added for dissolution, then morpholine (4.3 mL) was added, and the mixture was stirred for reaction at room temperature for 1 hour. After the reaction was completed, the reaction mixture was transferred to a 1 L separatory funnel containing saturated brine (200 mL) and ethyl acetate (200 mL) for extraction. The organic phase was collected, and the aqueous phase was further extracted with ethyl acetate (200 mL). The organic phases were combined, washed with deionized water (200 mL × 2), evaporated to dryness, then dissolved in a dichloromethane/methanol (4/1) mixture. 200-300 mesh silica gel powder was added, and the mixture was evaporated to dryness. The resulting product was dry-loaded onto the column and subjected to column chromatography using a 1-3% methanol/dichloromethane mixture as the eluent. The product fraction was collected, evaporated to dryness, and dried in a vacuum oven to provide 4.1 g of the product.

1.31 Preparation of **71-56**

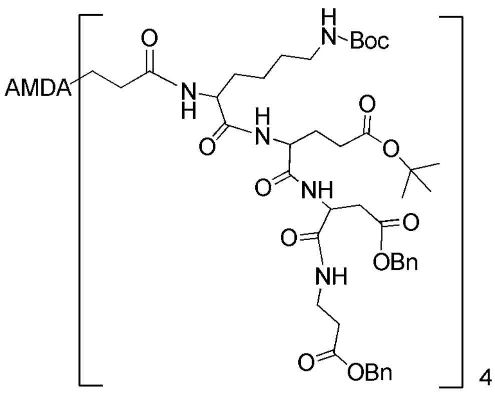

**71-54** (0.4516 g, 1.1139 mmol), **71-55** (3.9082 g, 4.9011 mmol), HBTU (2.5346 g, 6.6834 mmol), and HOBT (0.9031 g, 6.6834 mmol) were placed in a 500 mL flask, then DMF (25 mL) was added for dissolution, and the mixture was stirred at -5°C for approximately 20 minutes. DIEA (2.2 mL, 13.3668 mmol) was then slowly added dropwise, and the reaction was continued at -5°C for 1 hour. The reaction flask was brought to room temperature, and the mixture was stirred overnight. After the reaction was completed, the reaction mixture was transferred to a 1 L separatory funnel containing saturated brine (200 mL) and ethyl acetate (200 mL) for extraction. The organic phase was obtained, and the aqueous phase was further extracted with ethyl acetate (200 mL). The organic phases were combined, washed with deionized water (200 mL × 2), and evaporated to dryness. The resulting solid product was dissolved in a dichloromethane/methanol (4/1) mixture. 200-300 mesh silica gel powder was added, and the mixture was evaporated to dryness. The resulting product was dry-loaded onto the column and subjected to column chromatography using a 2-4% methanol/dichloromethane mixture as the eluent. The product fraction was collected, evaporated to dryness, and dried in a vacuum oven to provide 2.1 g of the product. $^1$H-NMR (600MHz, DMSO-$d_6$) δ 8.16-7.98 (m, 20H), 7.36-7.34 (m, 40H), 5.08-5.06 (m, 20H), 4.57-4.56 (m, 6H), 4.21-4.20 (m, 8H), 3.34-3.31 (m, 19H), 3.29-3.25 (m, 10H), 2.75-2.70 (m, 16H), 2.49-2.48 (m, 8H), 2.35-2.31 (m, 8H), 2.22-2.20 (m, 8H), 1.88-1.83 (m, 4H), 1.77-1.71 (m, 4H), 1.61-1.60 (m, 4H), 1.48-1.46 (m, 4H), 1.35 (s,

72H), 1.24-1.21 (m, 8H)

1.32 Preparation of **71-77**

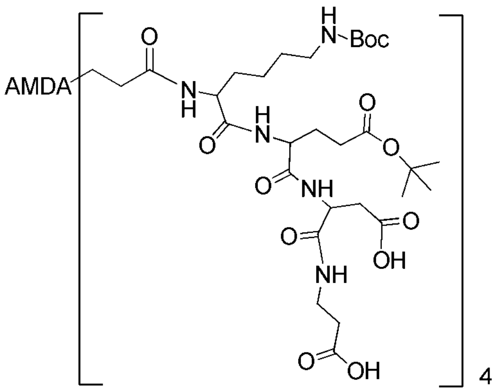

**71-56** (1 g, 0.2837 mmol) and 10% Pd/C catalyst (0.1 g) were added to a hydrogenation reactor, and NMP (30 mL) was added for dissolution. The hydrogenation reactor was sealed, evacuated with a water pump, and then filled with hydrogen gas. This process was repeated three times. The pressure in the hydrogenation reactor was adjusted to 2 MPa, and the mixture was stirred at room temperature overnight. After the reaction was completed, the reaction mixture was filtered through Celite, and the filter cake was washed with DMF (15 mL × 3). The filtrate was then placed in a 250 mL round-bottom flask and used as the raw material for the next step.

1.33 Preparation of **71-82**

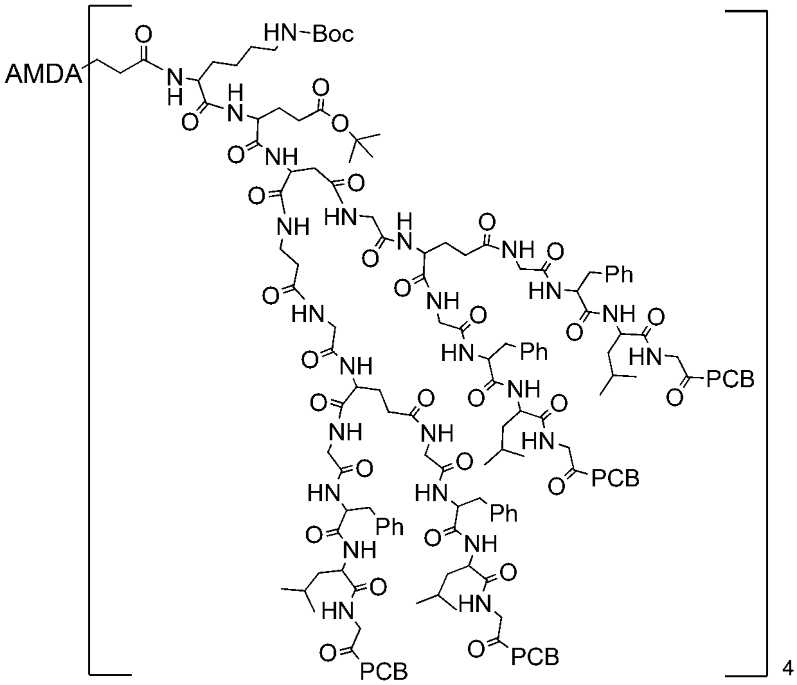

**71-77** (0.1 g, 0.0357 mmol), **71-60** (0.5686 g, 0.3138 mmol), HBTU (0.1623 g, 0.4279 mmol), and HOBT (0.0578 g, 0.4279 mmol) were added to a 1 L round-bottom flask, then NMP (40 mL) was added to dissolve the mixture, and the mixture was stirred at room temperature for approximately 20 minutes. DIEA (0.3 mL, 11.5691 mmol) was then slowly added dropwise. After the dropwise addition was completed, the reaction flask was allowed to stand at room temperature,

and the reaction was carried out with stirring. After the reaction was completed, n-hexane (30 mL) and methyl tert-butyl ether (200 mL) were added for precipitation, and the supernatant was discarded. This process was repeated three times. The solid product was filtered to provide a solid. The solid was washed with a 4% methanol/dichloromethane mixture, filtered, collected, and dried in a vacuum oven to provide 0.5 g of the product. $^1$H-NMR (600MHz, DMSO-$d_6$) δ 8.19-8.00 (m, 100H), 7.89-7.88 (m, 20H), 7.51-7.49 (m, 20H), 7.23-7.16 (m, 104H), 4.57-4.56 (m, 20H), 4.37-4.36 (m, 20H), 4.21-4.19 (m, 14H), 4.05-3.97 (m, 40H), 3.69-3.59 (m, 126H), 3.19-3.06 (m, 112H), 2.88-2.85 (m, 8H), 2.42 (s, 56H), 2.31-2.24 (m, 115H), 1.88 (s, 40H), 1.77 (s, 40H), 1.67-1.45 (m, 128H), 1.35 (s, 72H), 1.24-1.22 (m, 8H), 0.89-0.83 (m, 96H)

1.34 Preparation of **71-86**

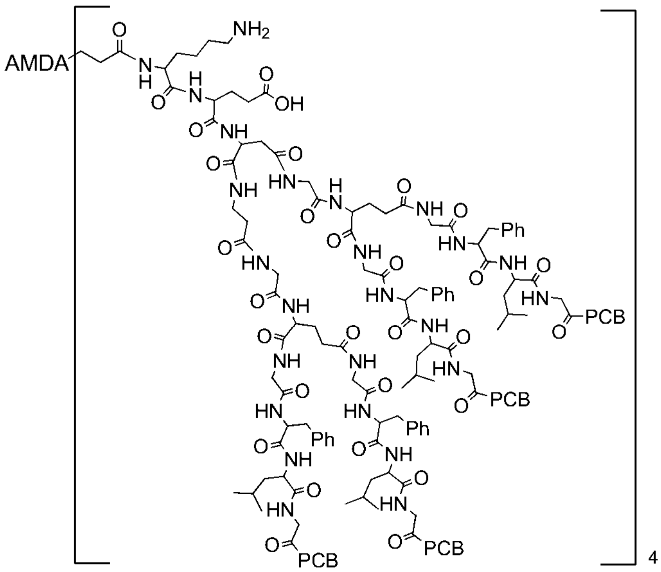

To a flask containing **71-82** (0.25 g, 0.0145 mmol), TFA (0.2 mL, 2.3315 mmol) was added, dissolved with the assistance of ultrasonication, and stirred in a water bath at 50°C overnight. After the reaction was completed, the reaction mixture was concentrated and evaporated to dryness, and methyl tert-butyl ether (300 mL) was added for precipitation. A solid product precipitated and was filtered. The filter cake was dissolved in DMF (10 mL), and methyl tert-butyl ether (300 mL) was added again to precipitate a solid product. This process was repeated six times. The filter cake was washed with methyl tert-butyl ether (40 mL × 3). The filter cake was collected and dried under vacuum to provide 0.2 g of the product.

1.35 Preparation of **71-91**

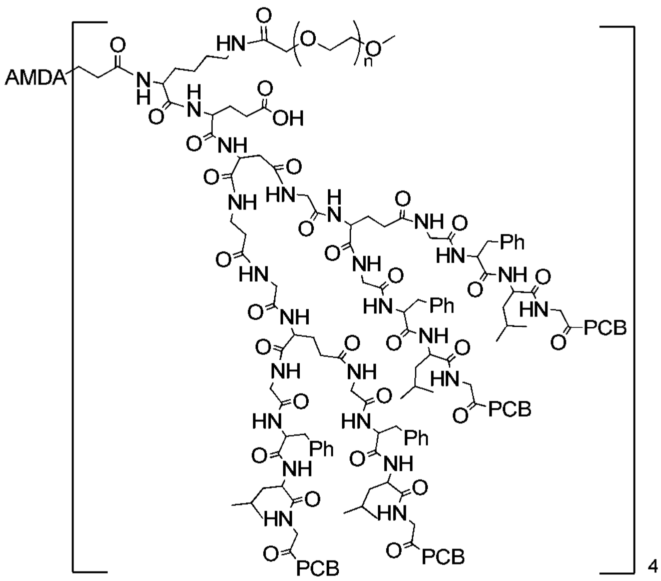

**71-86** (0.13 g, 0.0083 mmol) was placed in a 250 mL flask, then NMP (20 mL) was added for dissolution, and the mixture was stirred in a water bath at 50°C for 30 minutes. DIEA (0.2 mL, 0.996 mmol) was then slowly added dropwise, and the stirring was continued for 30 minutes. M-SCM-10K (0.3825 g, 0.0366 mmol, purchased from Jenkem, Lot Number: ZZ390P163) was added, the reaction mixture was then placed in a water bath at 40°C, and the mixture was stirred at low-speed for one week in the dark. After the reaction was completed, methyl tert-butyl ether (200 mL) and n-hexane (70 mL) were added. A solid precipitated and was filtered. The filter cake was washed with methyl tert-butyl ether (40 mL × 3). The filter cake was collected and dried in a vacuum oven to provide 0.47 g of the product.

1.36 Preparation of **71-215**

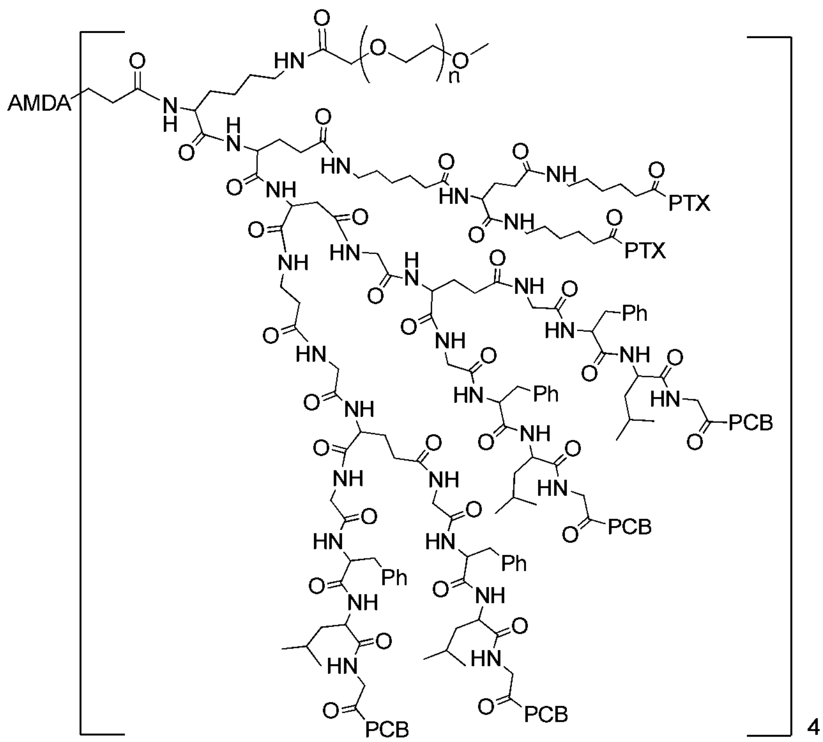

**71-91** (0.47 g, 0.0083 mmol), **71-95** (0.10 g, 0.0496 mmol), HBTU (0.0188 g, 0.0496 mmol), and HOBT (0.0067 g, 0.0496 mmol) were added to a 1 L round-bottom flask, then DMF (30 mL) was added for dissolution, and the mixture was

stirred at room temperature for approximately 20 minutes. DIEA (0.1 mL, 0.1489 mmol) was then slowly added dropwise. After the dropwise addition was completed, the reaction flask was stirred at room temperature. After the reaction was completed, n-hexane (30 mL) and methyl tert-butyl ether (100 mL) were added for precipitation, and the supernatant was discarded. This process was repeated twice. The mixture was filtered to provide a solid product, which was dissolved in a dichloromethane/methanol (4/1) mixture, then 100-200 mesh silica gel powder was added, and the mixture was evaporated to dryness. The resulting product was dry-loaded onto the column and subjected to column chromatography using a 1% triethylamine/5-10% methanol/dichloromethane mixture as the eluent. The product fraction was collected and concentrated and evaporated to dryness. The resultant solid product was dissolved in an anhydrous ethanol/dichloromethane (4/1) mixture, then methyl tert-butyl ether (200 mL) was added for precipitation, and the mixture was filtered to provide a powdery solid. This process was repeated three times. The filter cake was collected and dried in a vacuum oven to provide 0.3 g of the product. $^1$H-NMR (600MHz, DMSO-$d_6$) δ 8.23-8.01 (m, 156H), 7.89-7.88 (m, 68H), 7.39-7.23 (m, 164H), 5.37-5.28 (m, 32H), 4.61-4.52 (m, 52H), 4.36-4.35 (m, 24H), 4.03-3.99 (m, 46H), 3.63-3.62 (m, 134H), 3.51-3.50 (m, 3748H), 3.08-3.05 (m, 136H), 2.79-2.76 (m, 32H), 2.45-2.40 (m, 96H), 2.31-2.23 (m, 155H), 1.93-1.84 (m, 64H), 1.82-1.73 (m, 72H), 1.59-1.51 (m, 184H), 1.25-1.24 (m, 112H), 0.9-0.89 (m, 96H)

Example 2: Synthesis of Compound **59-126**

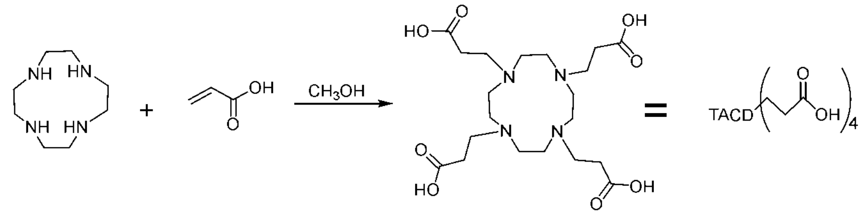


59-108

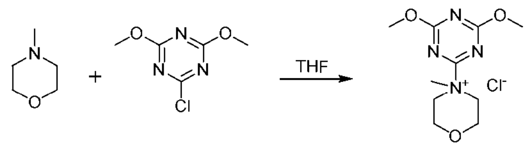


68-54

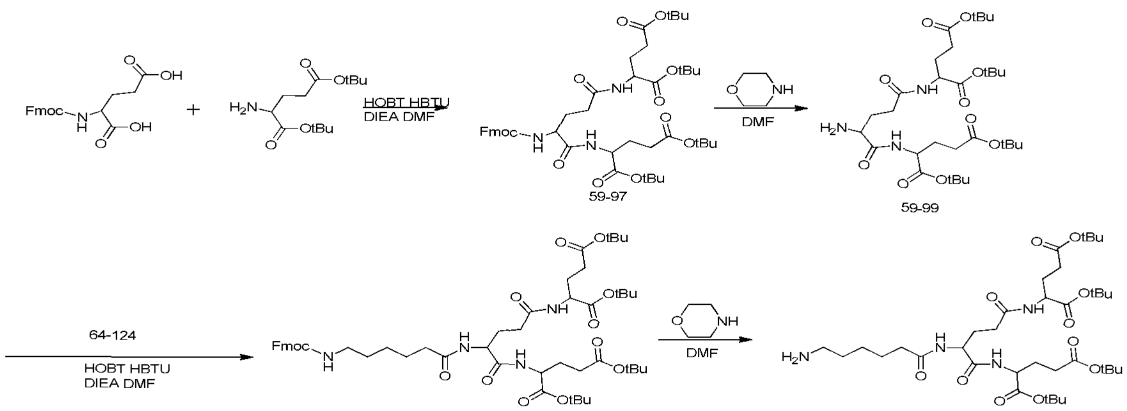


59-97

59-99

59-106

38-224

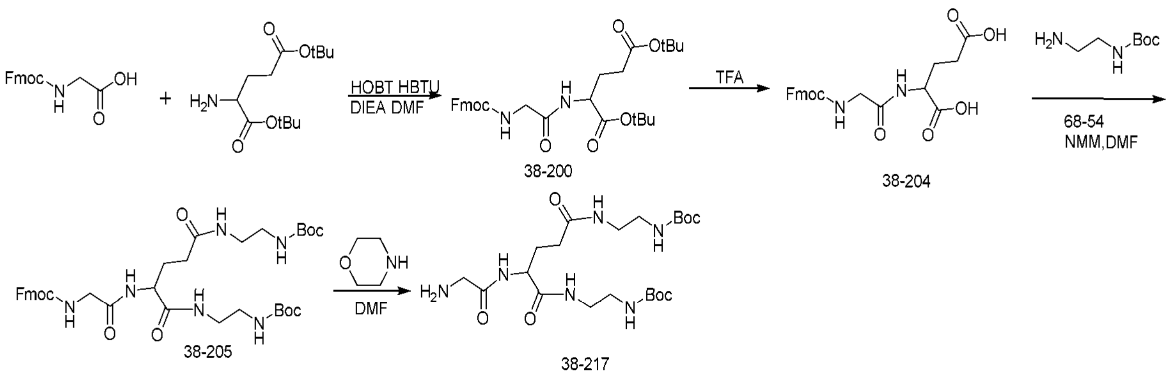


38-200

38-204

38-205

38-217

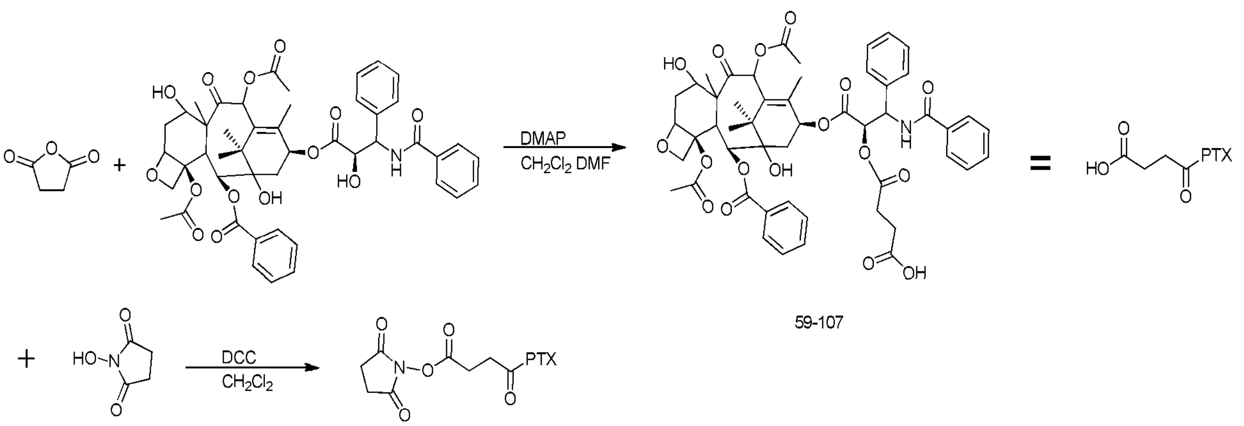


59-107

59-121

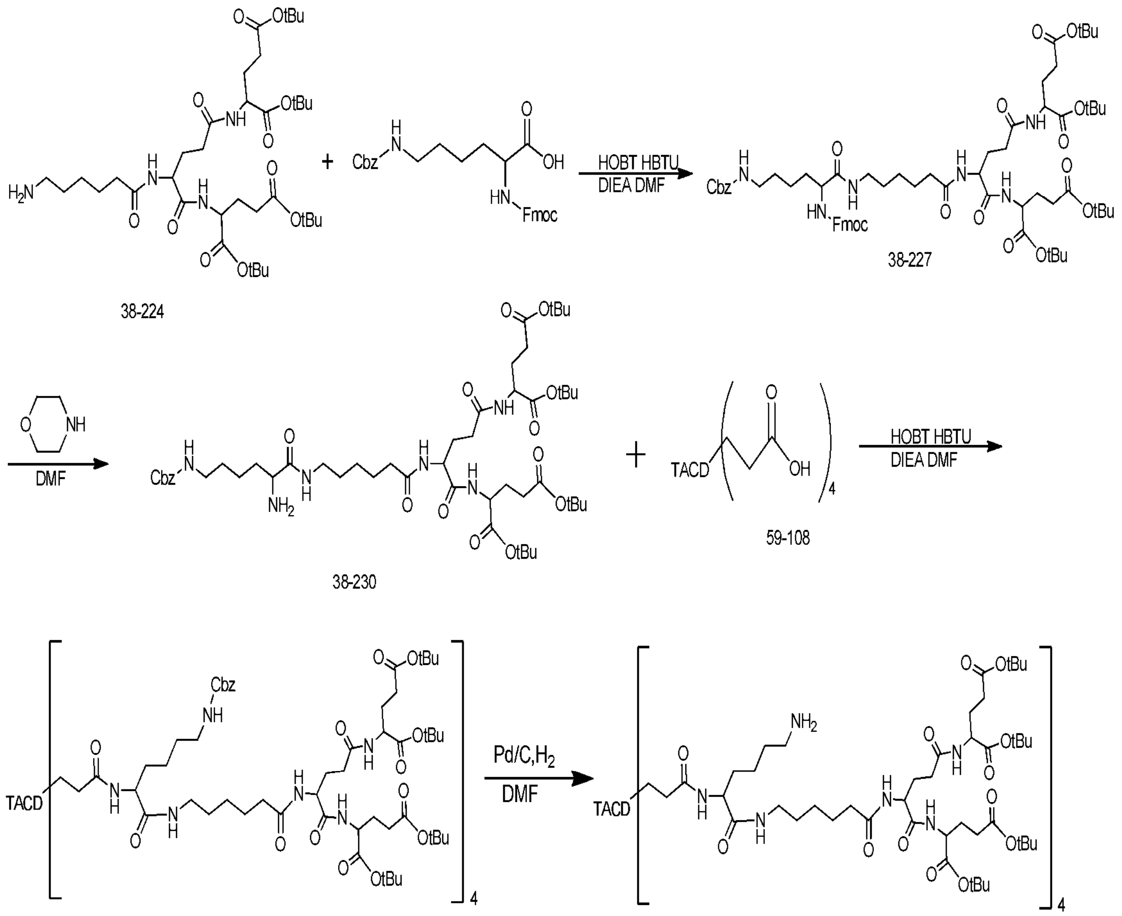


38-224

38-227

38-230

59-108

38-234

38-258

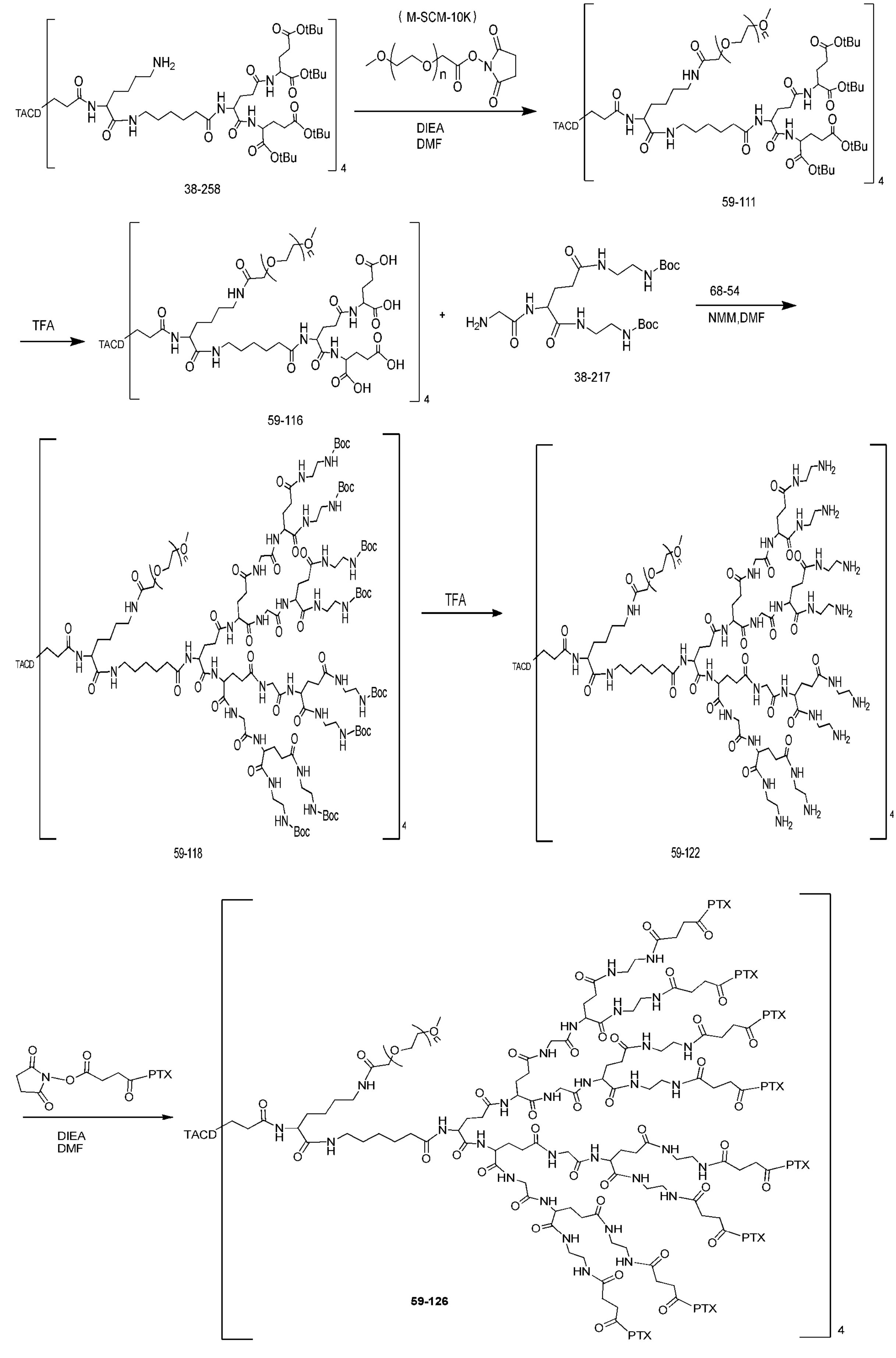


38-258

59-111

59-116

38-217

59-118

59-122

**59-126**

2.1 Preparation of **59-108**

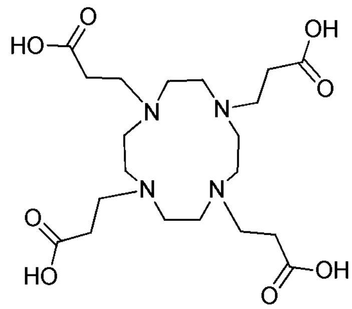

Cyclen (3.4454 g, 20 mmol) was placed in a 250 mL flask, then anhydrous ethanol was added for dissolution with the assistance of ultrasonication, and the mixture was stirred at room temperature. Acrylic acid (28.1034 g, 390 mmol) was dissolved in anhydrous ethanol and added dropwise to the reaction mixture. Stirring was continued at room temperature for 1 hour. The reaction mixture was then placed at 85°C and refluxed for 2 hours. The mixture became turbid and continued to reflux for another 2 hours. Heating was then stopped, and the mixture was cooled to room temperature. After the reaction was completed, the product was adsorbed onto the wall of the flask. The liquid was poured out, a small amount of anhydrous ethanol was added, and ultrasonic treatment was performed for 10 minutes. The filter cake was collected by suction. This process was repeated three times. The solid product was collected and dried in an oven to provide 1.79 g of the product. $^1$H-NMR (600 MHz, DMSO-$d_6$) δ 11.0 (s, 4H), 2.90 (t, $J$ = 7.0 Hz, 8H), 2.76 (d, $J$ = 25.8 Hz, 16H), 2.42 (t, $J$ = 7.1 Hz, 8H)

2.2a Preparation of **68-54**

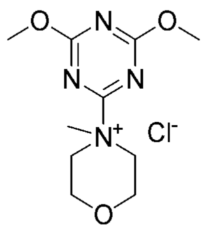

2-Chloro-4,6-dimethoxy-1,3,5-triazine (22.1 g, 125.9 mmol, purchased from Accela) was added to a 1 L flask, and THF (300 mL) was added. After complete dissolution under ultrasonication, N-methylmorpholine (12 mL, 114.4 mmol) was slowly added dropwise. After the dropwise addition was completed, the reaction was carried out with stirring at room temperature for 30 minutes. A large amount of white solid precipitated, which was collected by filtration and dried to provide the product.

2.2 Preparation of **59-97**

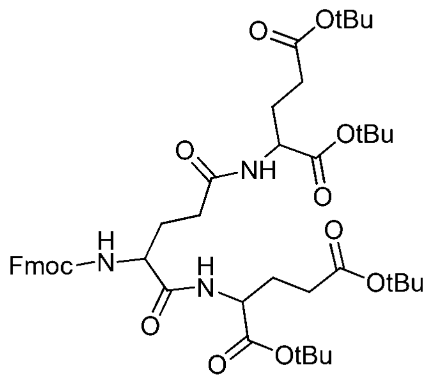

Fmoc-Glu(OH)$_2$ (6.5 g, 17.66 mmol, purchased from Adamas) and Glu(OtBu)$_2$ (10.08 g, 38.87 mmol, purchased from Innochem) were added to a 500 mL flask, then DMF (150 mL) was added for dissolution, and the mixture was stirred at room temperature for approximately 10 minutes. Then, **68-54** (10.76 g, 38.87 mmol) was added, followed by slow dropwise addition of N-methylmorpholine (3.58 mL, 155.48 mmol). After the dropwise addition was completed, the reaction was carried out with stirring at room temperature for 3 hours. After the reaction was completed, the reaction mixture was transferred to a 1 L separatory funnel containing saturated sodium bicarbonate solution (200 mL) and ethyl acetate (300 mL). The organic phase was collected, and the aqueous phase was further extracted with ethyl acetate (100 mL). The organic phases were combined, washed twice with pure water, concentrated, and evaporated to dryness to provide 10 g of the product.

2.3 Preparation of **59-99**

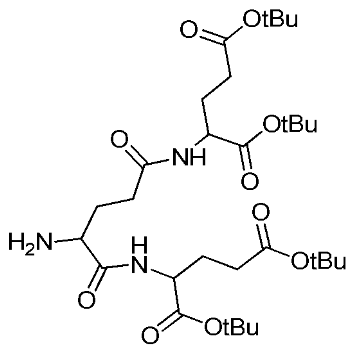

To a flask containing **59-97** (10 g, 11.7368 mmol), DMF was added. After dissolution with the assistance of ultrasonication, morpholine (20 mL, 229.7576 mmol) was added, and the reaction was stirred at room temperature for 2 hours. After the reaction was completed, the reaction mixture was poured into saturated sodium bicarbonate solution (200 mL) and ethyl acetate (200 mL) for extraction. After standing for phase separation, the organic phase was collected, and the aqueous phase was further extracted with ethyl acetate (200 mL × 3). The organic phases were combined, evaporated to dryness to provide a solid, which was dissolved in a methanol/dichloromethane (1/4) mixture (100 mL), then silica gel powder (100 mL) was added, and the mixture was evaporated to dryness to provide a powdery solid. The powdery solid was dry-loaded onto the column and subjected to column chromatography using a 0-5% methanol/dichloromethane mixture as the eluent. The desired product was collected, concentrated, and dried in a vacuum oven to provide 7 g of the product with a yield of 94.72%.

2.4 Preparation of **59-106**

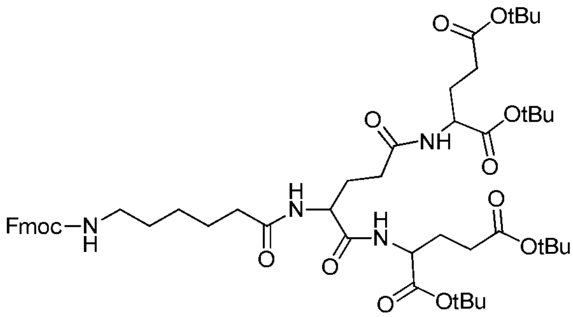

**59-99** (7 g, 11.274 mmol), HOBT (2.1 g, 15.374 mmol), and HBTU (5.8 g, 15.374 mmol) were weighed and added to a flask containing **64-124** (3.6 g, 10.25 mmol), then an appropriate amount of DMA (40 mL) was added to dissolve the mixture, and the mixture was stirred at room temperature for approximately 10 minutes. DIEA (7.6 mL, 46.125 mmol) was then slowly added dropwise. After the dropwise addition was completed, the reaction mixture was stirred for 3 hours. After the reaction was completed, the reaction mixture was transferred to a 1 L separatory funnel containing ethyl acetate (200 mL) and saturated sodium bicarbonate solution (150 mL) for extraction. The extraction was performed using the same ethyl acetate (150 mL × 2). The organic phases were combined, concentrated to 150 mL and washed with pure water (150 mL × 2). The ethyl acetate phase was collected and evaporated to dryness to provide a solid. The solid was then dissolved in a methanol/dichloromethane (1/4) mixture (100 mL), then silica gel powder (100 mL) was added, and the mixture was evaporated to dryness to provide a powdery solid. The powdery solid was dry-loaded onto the column and subjected to column chromatography using a 0.5-5% methanol/dichloromethane mixture as the eluent. The desired product was collected, concentrated, and dried in a vacuum oven to provide 10 g of the product.

2.5 Preparation of **38-224**

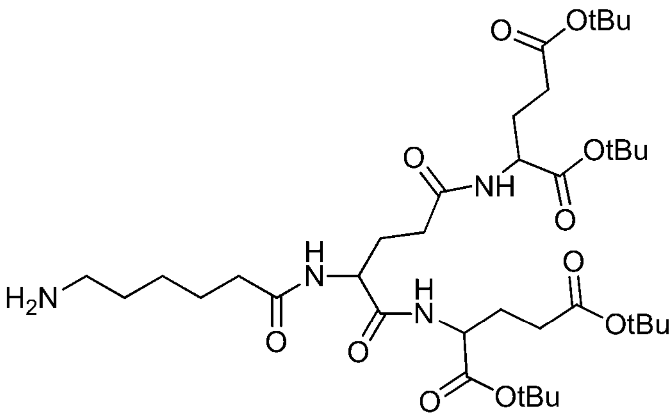

**59-106** (10 g, 10.3607 mmol) was added to a 250 mL round-bottom flask, then an appropriate amount of DMF was added for dissolution with the assistance of ultrasonication. Then morpholine (9 mL, 103.3607 mmol) was added, and the mixture was reacted with stirring at room temperature for 2 hours. After the reaction was completed, ethyl acetate (200 mL) and saturated sodium bicarbonate solution (200 mL) were added for extraction. After standing for phase separation, the organic phase was collected, and the aqueous phase was further extracted with ethyl acetate (200 mL × 3). The organic phases were combined, evaporated to dryness to provide a solid, then the solid was dissolved in a methanol/dichloromethane (1/4) mixture (100 mL). Silica gel powder (60 mL) was added, and the mixture was evaporated to dryness, resulting in a powdery solid. The powdery solid was dry-loaded onto the column and subjected to column chromatography using a 1% ammonia solution/5-10% methanol/dichloromethane mixture as the eluent. The desired product was collected, concentrated, and dried in a vacuum oven to provide 6.9 g of the product with a yield of 89.65%.

2.6 Preparation of **38-200**

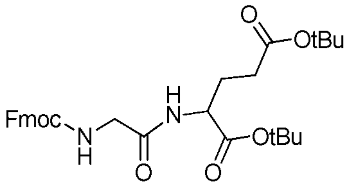

Fmoc-Gly-OH (8 g, 26.91 mmol, purchased from Accela), Glu(OtBu)$_2$ (7.676 g, 29.601 mmol), HOBT (5.45 g, 40.365 mmol), and HBTU (15.308 g, 40.365 mmol) were added to a 500 mL flask, then DMF (80 mL) was added for dissolution, and the mixture was reacted with stirring at room temperature. DIEA (20 mL, 121.095 mmol) was slowly added dropwise, and the reaction was allowed to proceed with stirring at room temperature for 3 hours. After the reaction was completed, the reaction mixture was transferred to a 1 L separatory funnel containing deionized water (200 mL) and ethyl acetate (300 mL) for extraction. The organic phase was collected, and the aqueous phase was further extracted with ethyl acetate (100 mL). The organic phases were combined, washed with saturated brine (150 mL), concentrated, and evaporated to dryness to provide 12 g of the product.

2.7 Preparation of **38-204**

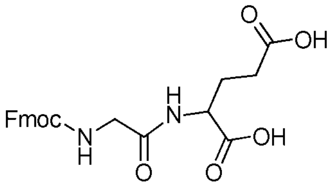

**38-200** (12 g, 22.279 mmol) was placed in a 250 mL round-bottom flask, then dichloromethane (5 mL) and TFA (60 mL, 891.16 mmol) were added for dissolution, and the mixture was reacted with stirring at room temperature overnight. After the reaction was completed, the reaction mixture was evaporated to dryness, then dichloromethane (5 mL) was added for dissolution. Then, n-hexane (100 mL) was added for precipitation, and the supernatant was discarded. This process was repeated twice. The precipitate was evaporated to dryness to provide 9 g of the product with a yield of 94.7%.

2.8 Preparation of **38-205**

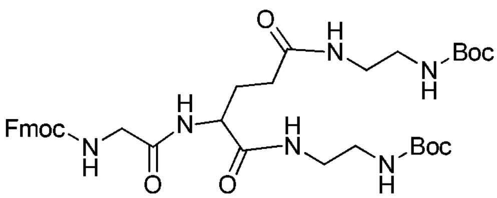

**38-204** (9 g, 21.106 mmol) and Boc-ethylenediamine (7.3 mL, 46.4332 mmol, purchased from Aladdin) were added to a 500 mL flask, then DMF (150 mL) was added for dissolution, and the mixture was stirred at room temperature for approximately 10 minutes. **68-54** (12.85 g, 46.4332 mmol) was then added, and N-methylmorpholine (20.6 mL, 185.7328 mmol) was then slowly added dropwise. After the dropwise addition was completed, stirring was continued at room temperature for 3 hours. After the reaction was completed, the reaction mixture was transferred to a 1 L separatory funnel containing deionized water (200 mL) and ethyl acetate (300 mL) for extraction. The organic phase was collected, and the aqueous phase was further extracted with ethyl acetate (100 mL). The organic phases were combined, washed with saturated brine (150 mL), concentrated. Then silica gel powder was added, and the mixture was evaporated to dryness. The resulting product was dry-loaded onto the column and subjected to column chromatography using a 3-5% methanol/dichloromethane solution as the eluent. The desired product was collected, concentrated, and dried to provide 12.5 g of the product.

2.9 Preparation of **38-217**

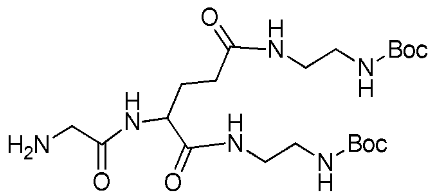

To a flask containing **38-205** (10 g, 10.36 mmol), an appropriate amount of DMF was added. After dissolution with the assistance of ultrasonication, morpholine (20 mL, 229.7576 mmol) was added, and the mixture was stirred at room temperature for 2 hours. After the reaction was completed, the reaction mixture was transferred to a 1 L separatory funnel containing deionized water (200 mL) and ethyl acetate (300 mL) for extraction. The organic phase was collected, and the aqueous phase was further extracted with ethyl acetate (100 mL). The organic phases were combined, washed with saturated brine (150 mL), concentrated. Then silica gel powder was added, and the mixture was evaporated to dryness. The resulting product was dry-loaded onto the column and subjected to column chromatography using 0-5% methanol/dichloromethane solution and 1% ammonia water/5-8% methanol/dichloromethane solution as the eluents. The desired product was collected, concentrated, and dried in a vacuum oven to provide 12.5 g of the product with a yield of 83.33%. $^1$H-NMR (600 MHz, DMSO-$d_6$) δ 8.02 (t, *J* = 5.5 Hz, 2H), 7.81 (t, *J* = 5.4 Hz, 1H), 6.80 (dd, *J* = 13.6, 5.7 Hz, 2H), 4.22 (s, 1H), 3.12 (d, *J* = 1.8 Hz, 2H), 3.09-3.06 (m, 2H), 3.04-2.94 (m, 6H), 2.04 (t, *J* = 7.9 Hz, 2H), 1.92-1.87 (m, 1H), 1.73 (dd, *J* = 13.7, 7.8 Hz, 1H), 1.37 (s, 20H)

2.10 Preparation of **59-107**

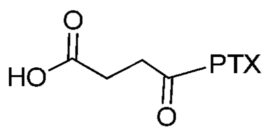

Paclitaxel (5 g, 5.86 mmol, abbreviated as PTX), succinic anhydride (2.1 g, 21.096 mmol), and DMAP (0.024 g, 0.3876 mmol) were placed in a 250 mL flask, then a mixture of dichloromethane and DMA (11/1) (24 mL) was added for dissolution with the assistance ultrasonication, and the mixture was stirred at room temperature overnight. After the reaction was completed, the mixture was rotary-evaporated and dissolved in ethyl acetate (100 mL). The reaction mixture was transferred to a 500 mL separatory funnel and washed with dilute hydrochloric acid (35 mL). The ethyl acetate phase was collected and then washed with purified water (40 mL). The organic phase was collected. The organic phase was dried over anhydrous sodium sulfate and filtered. Finally, the resulting product was concentrated and dried to provide 3.13 g of the product with a yield of 71.5%. $^1$H-NMR (600 MHz, DMSO-$d_6$) δ 12.26 (s, 1H), 8.00-7.98 (m, 2H), 7.86-7.84 (m, 2H), 7.74 (t, *J* = 7.4 Hz, 1H), 7.67 (t, *J* = 7.6 Hz, 2H), 7.56 (d, *J* = 7.4 Hz, 1H), 7.52-7.44 (m, 7H), 7.20 (td, *J* = 5.9, 3.0 Hz, 1H), 6.30 (s, 1H), 5.82 (t, *J* = 8.8 Hz, 1H), 5.55 (t, *J* = 8.8 Hz, 1H), 4.93-4.89 (m, 2H), 4.64 (s, 1H), 4.12 (dd, *J* = 6.6, 4.1 Hz, 1H), 4.06-3.98 (m, 3H), 3.59 (d, *J* = 7.2 Hz, 1H), 2.63 (t, *J* = 6.5 Hz, 3H), 2.33 (d, *J* = 1.3 Hz, 2H), 2.25 (s, 3H), 2.11 (s, 3H), 1.76 (s, 3H), 1.51 (d, *J* = 11.3 Hz, 4H), 1.01 (t, *J* = 13.8 Hz, 9H)

2.11 Preparation of **59-121**

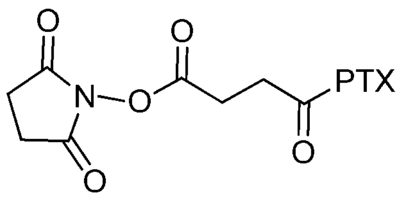

**59-107** (2.24 g, 2.34 mmol), NHS (0.355 g, 3.0888 mmol), and DCC (0.618 g, 2.9952 mmol) were placed in a 100 mL flask, then dichloromethane (30 mL) was added, and the mixture was reacted with stirring at room temperature overnight. After the reaction was completed, the mixture was filtered. Silica gel powder was added to the filtrate, and the mixture was evaporated to dryness. The resulting product was subjected to column chromatography using a 0-2% methanol/dichloromethane mixture as the eluent. The desired product was collected, concentrated, and dried to provide 2.47 g of the product.

2.12 Preparation of **38-227**

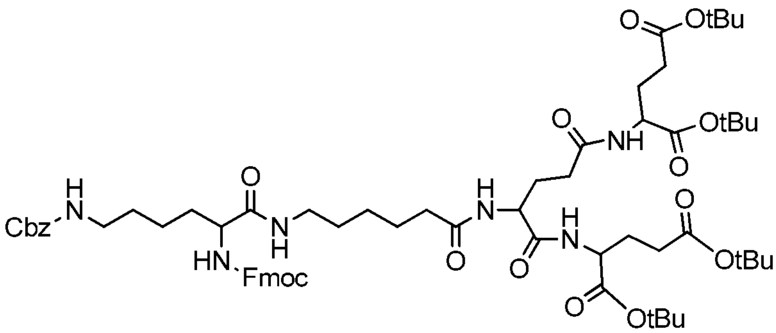

**38-224** (3.5 g, 4.711 mmol), N'-fluorenylmethyloxycarbonyl-N-benzyloxycarbonyl-L-lysine (2.15 g, 4.283 mmol), HOBT (0.87 g, 6.4245 mmol), and HBTU (2.436 g, 6.4245 mmol) were added to a 250 mL flask, then DMF (20 mL) was added for dissolution, and the mixture was stirred at room temperature for 10 minutes. DIEA (1.1 mL, 12.849 mmol) was slowly added dropwise. After the dropwise addition was completed, stirring was continued at room temperature for 3 hours. After the reaction was completed, the mixture was poured into a separatory funnel containing ethyl acetate (200 mL) and saturated sodium bicarbonate solution (200 mL) for extraction. After standing for phase separation, the organic phase was collected, and the aqueous phase was further extracted with ethyl acetate (200 mL × 3). The organic phases were combined, evaporated to dryness and dried in a vacuum oven to provide 5.3 g of the product with a yield of 100%.

2.13 Preparation of **38-230**

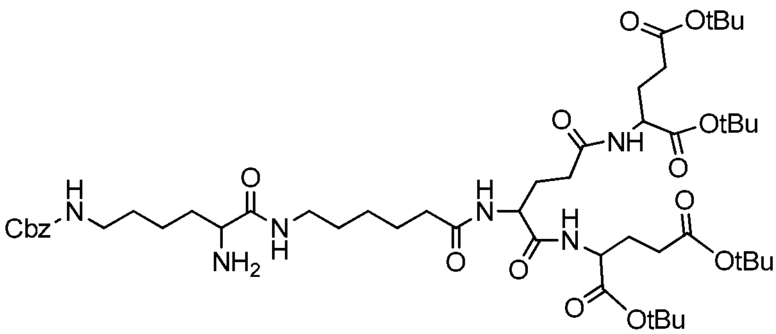

To a flask containing **38-227** (5.3 g, 4.283 mmol), DMF (20 mL) was added for dissolution with the assistance of ultrasonication, then morpholine (7.5 mL, 85.66 mmol) was added, and the mixture was reacted with stirring at room temperature for 2 hours. After the reaction was completed, the mixture was poured into a separatory funnel containing ethyl acetate (200 mL) and saturated sodium bicarbonate solution (200 mL) for extraction. After standing for phase separation, the organic phase was collected. The aqueous phase was further extracted with ethyl acetate (200 mL × 3). The organic phases were combined, evaporated to dryness to provide a solid, which was then dissolved in a methanol/dichloromethane (1/4) mixture (100 mL), then silica gel powder (60 mL) was added, and the mixture was evaporated to dryness to provide a powdery solid. The powdery solid was dry-loaded onto the column and subjected to column chromatography using a mixture of 1% aqueous ammonia/1-10% methanol/dichloromethane as the eluent. The desired product was collected, concentrated, and dried in a vacuum oven to provide 3.9 g of the product with a yield of 90.7%. $^1$H-NMR (600MHz, DMSO-$d_6$) δ 8.17-8.16 (m, 1H), 8.09-8.08 (m, 1H), 7.93-7.92 (m, 1H), 7.79-7.76 (m, 1H), 7.36-7.31 (m,

5H), 7.23-7.21 (m, 1H), 5.12-5.09 (m, 2H), 5.00 (s, 2H), 4.29-4.25 (m, 1H), 4.13-4.10 (m, 2H), 3.06-2.95 (m, 5H), 2.26-2.07 (m, 8H), 1.94-1.87 (m, 3H), 1.78-1.69 (m, 5H), 1.51-1.46 (m, 4H), 1.39 (s, 36H), 1.32-1.21 (m, 6H)

2.14 Preparation of **38-234**

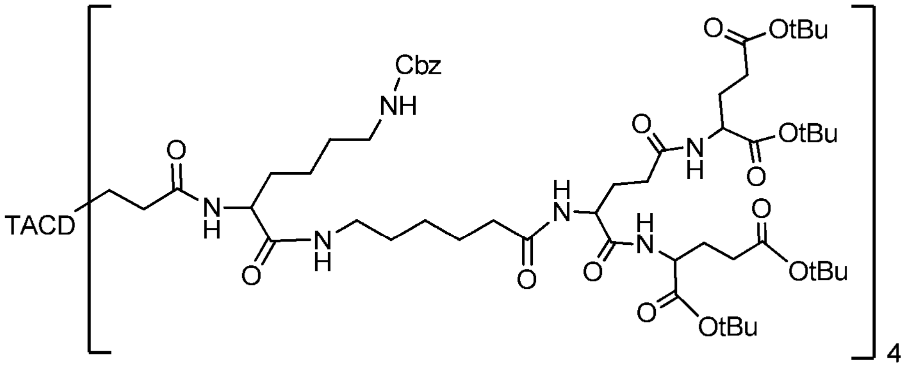

**59-108** (0.1125 g, 0.2475 mmol), **38-230** (1.5 g, 1.4925 mmol), HOBT (0.2025 g, 1.4925 mmol), and HBTU (0.5625 g, 1.4925 mmol) were added to a 250 mL flask, then DMF (8 mL) was added for dissolution, and the mixture was stirred at room temperature for approximately 10 minutes. DIEA (0.75 mL, 4.4775 mmol) was then slowly added dropwise. After the dropwise addition was completed, stirring was continued at room temperature for 3 hours. After the reaction was completed, the reaction mixture was poured into a separatory funnel containing ethyl acetate (100 mL) and saturated sodium bicarbonate solution (100 mL) for extraction. After standing for phase separation, the organic phase was collected, and the aqueous phase was further extracted with ethyl acetate (50 mL × 3). The organic phases were combined, evaporated to dryness to provide a solid, which was then dissolved in a methanol/dichloromethane (1/4) mixture (50 mL), then silica gel powder (30 mL) was added, and the mixture was evaporated to dryness. The resulting product was dry-loaded onto the column and subjected to column chromatography using a 3-8% methanol/dichloromethane mixture as the eluent. The desired product was collected, concentrated, and dried in a vacuum oven to provide 1.02 g of the product with a yield of 93.58%. $^1$H-NMR (600MHz, DMSO-$d_6$) δ8.21-8.17 (m, 8H), 8.08 (d, $J$ = 7.6 Hz, 4H), 7.92 (d, $J$ = 8.1 Hz, 4H), 7.54 (d, $J$ = 7.1 Hz, 4H), 7.38-7.32 (m, 20H), 7.27 (t, $J$ = 5.6 Hz, 4H), 5.01 (s, 8H), 4.28-4.25 (m, 4H), 4.14-4.10 (m, 8H), 3.12-2.92 (m, 44H), 2.31-2.08 (m, 40H), 1.92-1.87 (m, 12H), 1.76-1.68 (m, 20H), 1.52-1.44 (m, 16H), 1.39-1.384 (m, 144H), 1.35-1.20 (m, 24H)

2.15 Preparation of **38-258**

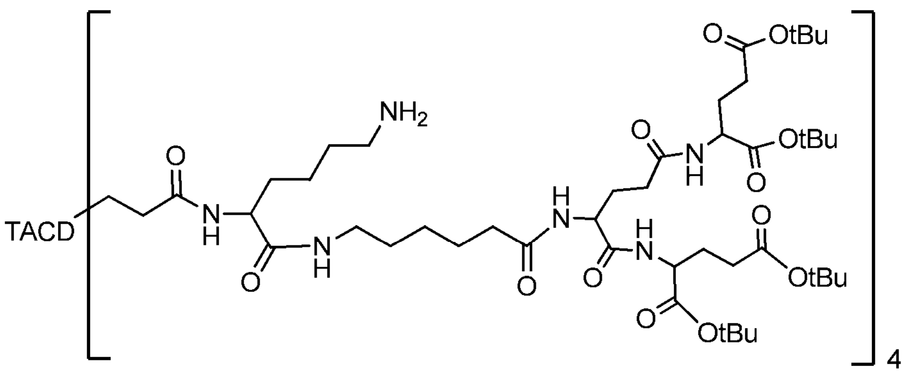

**38-234** (0.52 g, 0.118 mmol) and 10% Pd/C catalyst (0.3 g) were placed in a hydrogenation reactor, and DMF (30 mL) was added to dissolve the mixture. The hydrogenation reactor was sealed, evacuated with a water pump, and then filled with hydrogen gas. This process was repeated three times. The pressure in the hydrogenation reactor was adjusted to 2 MPa, and the reaction was carried out with stirring at room temperature overnight. After the reaction was completed, the reaction mixture was filtered through Celite, and the Celite was washed with DMF (20 mL × 3). The DMF filtrates were

combined and repeatedly precipitated with n-hexane and methyl tert-butyl ether until the reaction mixture became an oily solid, which was dried to provide 0.29 g of the product with a yield of 63.04%.

2.16 Preparation of **59-111**

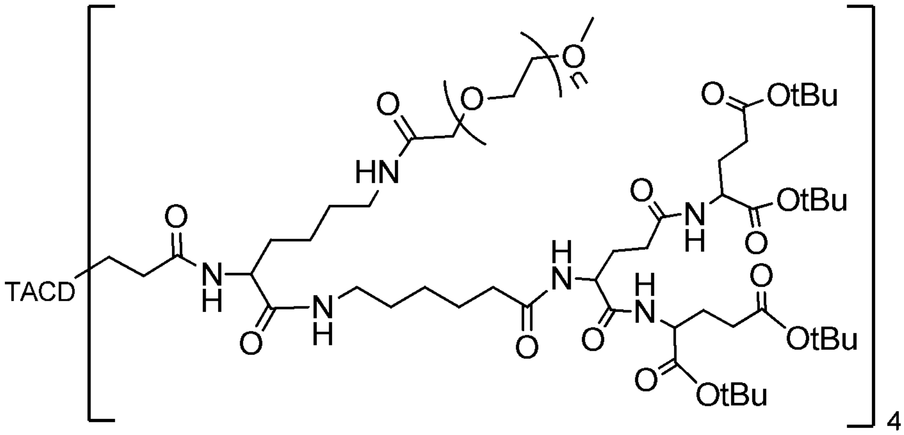

**38-258** (0.1686 g, 0.0436 mmol) was placed in a 250 mL round-bottom flask, then DMF (20 mL) was added for dissolution, and the mixture was stirred at -5°C for 30 minutes. DIEA (0.3 mL, 1.8152 mol) was then slowly added dropwise. After the dropwise addition was completed, the mixture was stirred at low temperature for 10 minutes, then stirred slowly at room temperature. M-SCM-10K (2.00 g, 0.19166 mmol, purchased from Jenkem, Lot Number: ZZ390P163) was added and dissolved with the assistance of ultrasonication. The reaction was then continued at room temperature with stirring at low-speed in the dark for 4 days. After the reaction was completed, methyl tert-butyl ether and n-hexane were added for precipitation. A solid precipitated and was filtered. The obtained solid product was dissolved in a methanol/dichloromethane (1/4) mixture, then silica gel powder (30 mL) was added, and the mixture was evaporated to dryness. The resulting product was dry-loaded onto the column and subjected to column chromatography using a mixture of 1% aqueous ammonia and 5-7% methanol/dichloromethane as the eluent. The desired product was collected, concentrated, and dried in a vacuum oven to provide 0.93 g of the product with a yield of 62%. $^1$H-NMR (600 MHz, DMSO-$d_6$) δ 8.32-8.15 (m, 16H), 8.09-8.07 (m, 4H), 7.94-7.90 (m, 4H), 4.28-4.25 (m, 4H), 4.20-4.14 (m, 8H), 3.51-3.50 (m, 3748H), 3.21-3.05 (m, 44H), 2.48-1.95 (m, 40H), 1.93-1.85 (m, 12H), 1.83-1.53 (m, 20H), 1.50-1.42 (m, 16H), 1.40-1.38 (m, 144H), 1.37-1.28 (m, 24H)

2.17 Preparation of **59-116**

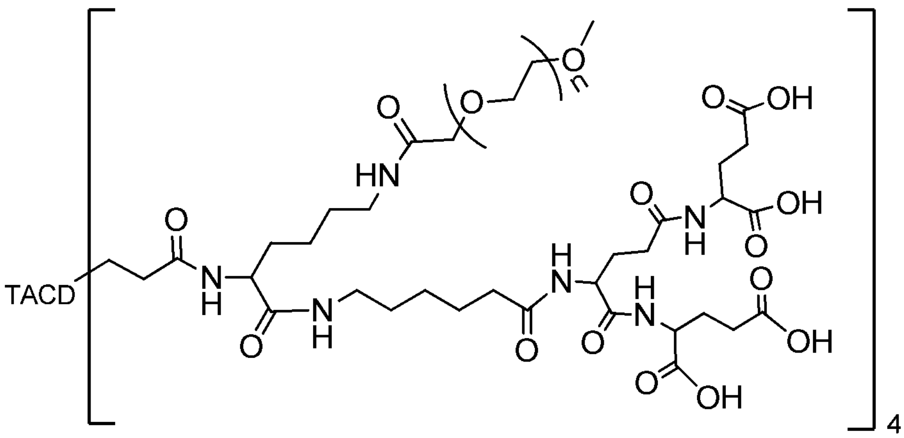

**59-111** (0.93 g, 0.0206 mmol) was placed in a 250 mL round-bottom flask, then TFA (0.4 mL, 5.056 mmol) was added for dissolution, and the mixture was reacted with stirring at room temperature overnight. After the reaction was

completed, the reaction mixture was evaporated to dryness, then dichloromethane (5 mL) was added for dissolution, and the mixture was rotavapped. This process was repeated three times. Methyl tert-butyl ether was added for precipitation, ultrasonically treated for 10 minutes, allowed to stand, and the supernatant was discarded. This process was repeated twice. Dichloromethane was added to the mixture for dissolution, and methyl tert-butyl ether was added for precipitation. The mixture was rotavapped, and then methyl tert-butyl ether was added again for precipitation. The mixture was ultrasonically treated, filtered, and the filter cake was collected. The filter cake was further dissolved in dichloromethane, then DIEA was added dropwise until the reaction mixture became neutral or weakly alkaline. The resulting product was concentrated and dried to provide 0.76 g of the product with a yield of 83.4%.

2.18 Preparation of **59-118**

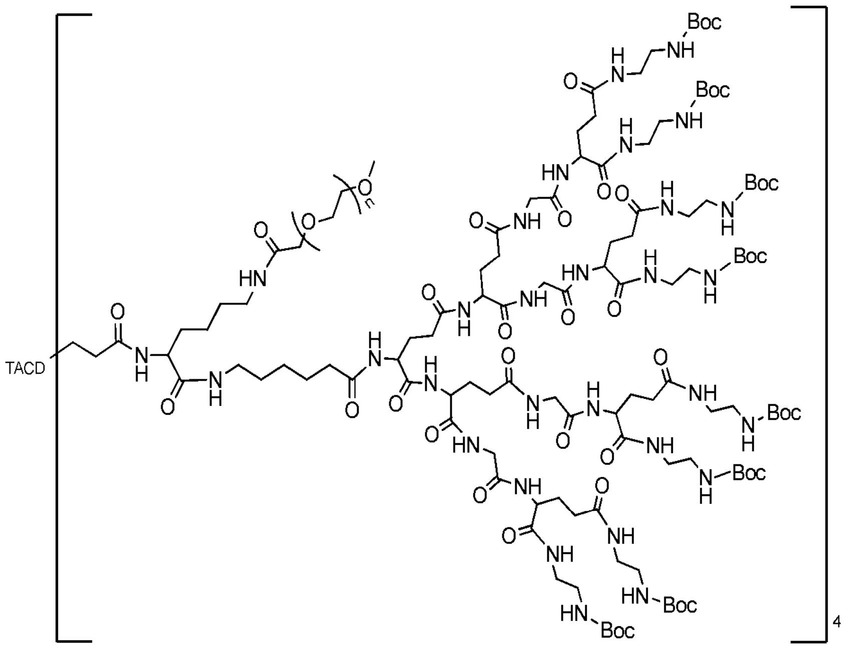

**59-116** (0.76 g, 0.0171 mmol) and **38-217** (0.2 g, 0.4104 mmol) were added to a 500 mL flask, then DMF (50 mL) was added for dissolution, and the mixture was stirred at room temperature for approximately 10 minutes. **68-54** (0.10 g, 0.4104 mmol) was then added, followed by slow dropwise addition of N-methylmorpholine (0.17 mL, 1.6416 mmol). After the dropwise addition was completed, stirring was continued at room temperature for 3 hours. After the reaction was completed, methyl tert-butyl ether (100 mL) and n-hexane (200 mL) were added for precipitation. The mixture was allowed to stand, the supernatant was discarded, and n-hexane and methyl tert-butyl ether were added again for precipitation. This process was repeated twice. The mixture was filtered to provide a solid product. The obtained solid product was dissolved in a methanol/dichloromethane (1/4) mixture, then silica gel powder (30 mL) was added, and the mixture was evaporated to dryness. The resulting product was dry-loaded onto the column and subjected to column chromatography using a 1% ammonia water/4-10% methanol/dichloromethane mixture solution as the eluent. The desired product was collected, concentrated, and dried in a vacuum oven to provide 0.33 g of the product with a yield of 37.29%. $^1$H-NMR (600 MHz, DMSO-$d_6$) δ 8.33-8.23 (m, 8H), 8.15-8.01 (m, 32H), 7.99-7.92 (m, 22H), 7.82-7.77 (m, 16H), 7.7-7.64 (m, 4H), 6.83-6.74 (m, 32H), 6.51-6.38 (m, 4H), 4.205-4.150(m, 28H), 3.51-3.50 (m, 3748H), 3.44-3.38 (m, 44H), 3.13-2.93 (m, 162H), 2.25-2.04 (m, 72H), 1.935-1.840 (m, 32H), 1.81-1.68 (m, 32H), 1.53-1.46 (m, 16H), 1.45-1.41 (m, 16H), 1.38-1.36 (m, 288H), 1.34-1.33 (m, 8H)

2.19 Preparation of **59-122**

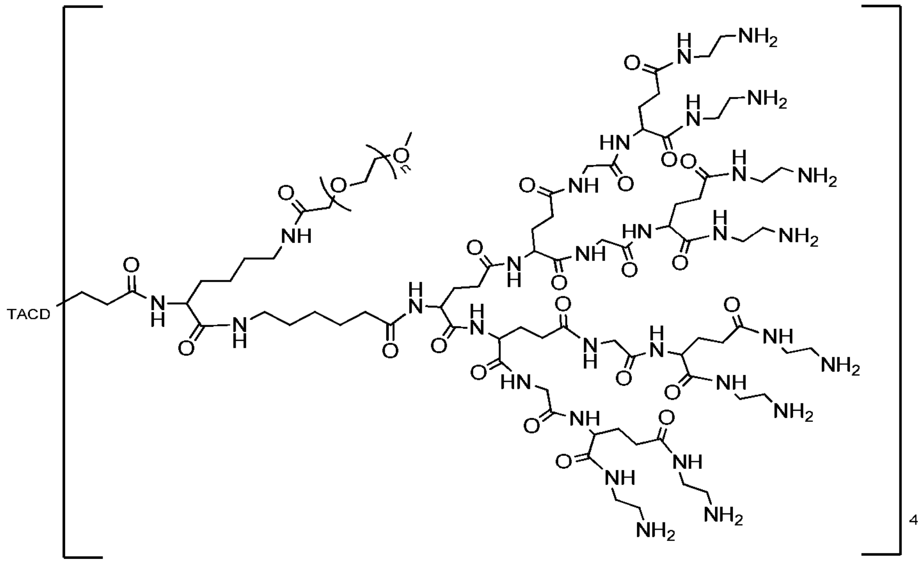

To a flask containing **59-118** (0.33 g, 0.00637 mmol), TFA (3.5 mL, 46.543 mmol) was added. Dissolution was performed with the assistance of ultrasonication, and the mixture was reacted with stirring at room temperature overnight. After the reaction was completed, the reaction mixture was evaporated to dryness, then dichloromethane (10 mL) was added for dissolution, and neutralized with excess DIEA. Then, methyl tert-butyl ether (300 mL) was added for precipitation. A solid product precipitated and was filtered. The filter cake was washed three times with methyl tert-butyl ether (40 mL). The filter cake was collected and dried under vacuum to provide 0.29 g of the product with a yield of 93.54%.

2.20 Preparation of **59-126**

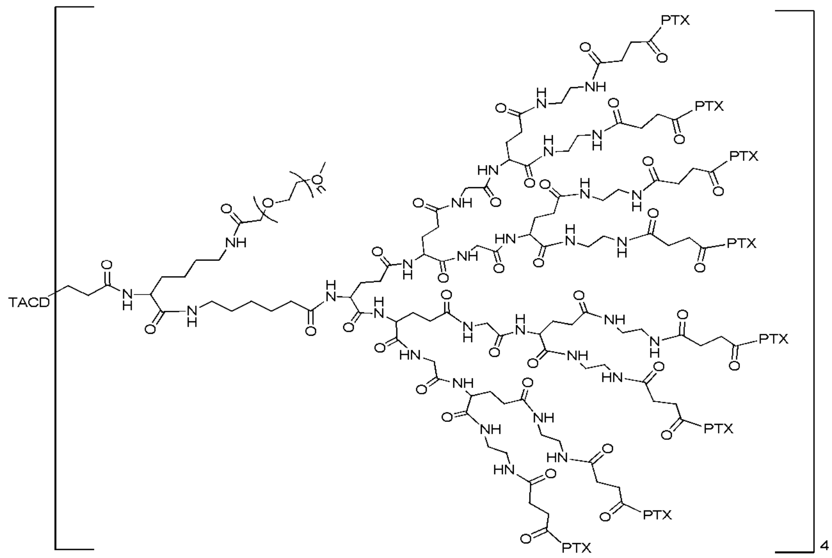

**59-122** (0.29 g, 0.00597 mmol) was dissolved in DMF (5 mL), then DIEA (0.06 mL, 0.38208 mmol) was added dropwise, and followed by the addition of **59-121** (0.403 g, 0.38208 mmol). Dissolution was performed with the assistance of ultrasonication, and the reaction was carried out at room temperature for 2 days. After the reaction was completed, the reaction mixture was repeatedly precipitated with n-hexane and tert-butyl ether until the reaction mixture became a solid. The solid was then dissolved in a small amount of a 2% methanol/dichloromethane mixture, and methyl tert-butyl ether was added for precipitation. After standing, the supernatant was discarded. This process was repeated several times until all impurities in the reaction mixture were removed. Dichloromethane was added to dissolve the product, and then methyl tert-butyl ether was added for precipitation. After standing, the supernatant was discarded. This process was repeated 3 times. The resulting product was dried on a rotary evaporator to provide 0.28 g of the product with a yield of 59.57%. $^1$H-NMR (600

MHz, DMSO-$d_6$) δ 8.26-8.21 (m, 8H), 8.152-8.146 (m, 32H), 8.10-8.03 (m, 32H), 7.994-7.792 (m, 64H), 7.92-7.90 (m, 22H), 7.876-7.827(m, 84H), 7.746-7.712 (m, 32H), 7.68-7.644 (m, 64H), 7.56-7.43(m, 256H), 7.203-7.166 (m, 32H), 6.315-6.273 (m, 32H), 6.099-5.982 (m, 4H), 5.85-5.79 (m, 32H), 5.565-5.520 (m, 32H), 4.94-4.88 (m, 64H), 4.66-4.61 (m, 32H), 4.20-4.14 (m, 28H), 4.127-4.093 (m, 32H), 4.07-3.93 (m, 96H), 3.630-3.618 (m, 32H), 3.51-3.50 (m, 3758H), 3.444-3.428 (m, 44H), 3.09-2.99 (m, 162H), 2.63-2.595 (m, 64H), 2.58-2.55 (m, 32H), 2.395-2.358 (m, 64H), 2.27-2.20 (m, 132H), 2.13-2.07 (m, 132H), 1.91-1.85 (m, 32H), 1.786-1.757 (m, 96H), 1.663-1.630 (m, 32H), 1.55-1.45 (m, 160H), 1.40-1.37 (m, 8H), 1.30-0.82 (m, 288H)

Example 3: Synthesis of Compound **67-228**

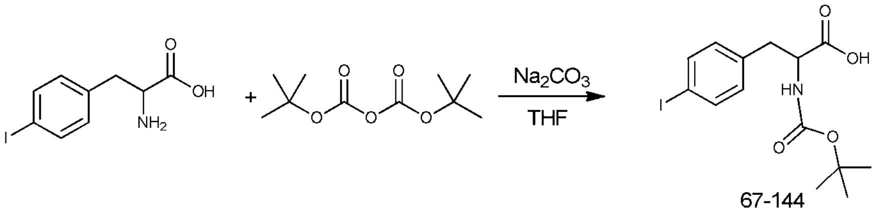


67-144

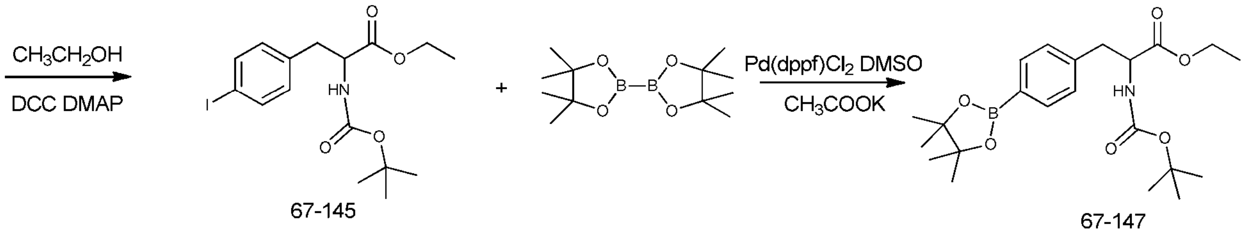


67-145

67-147

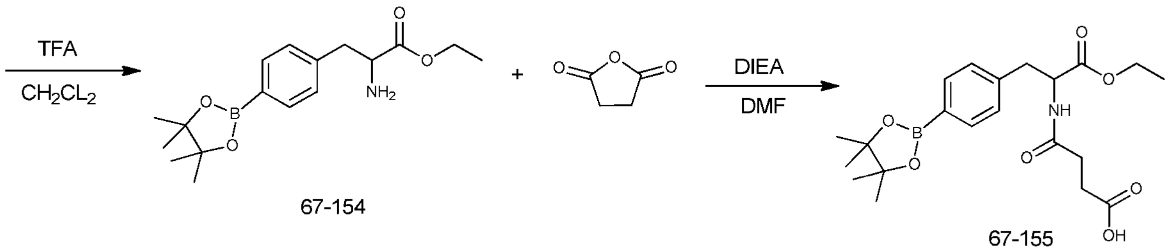


67-154

67-155

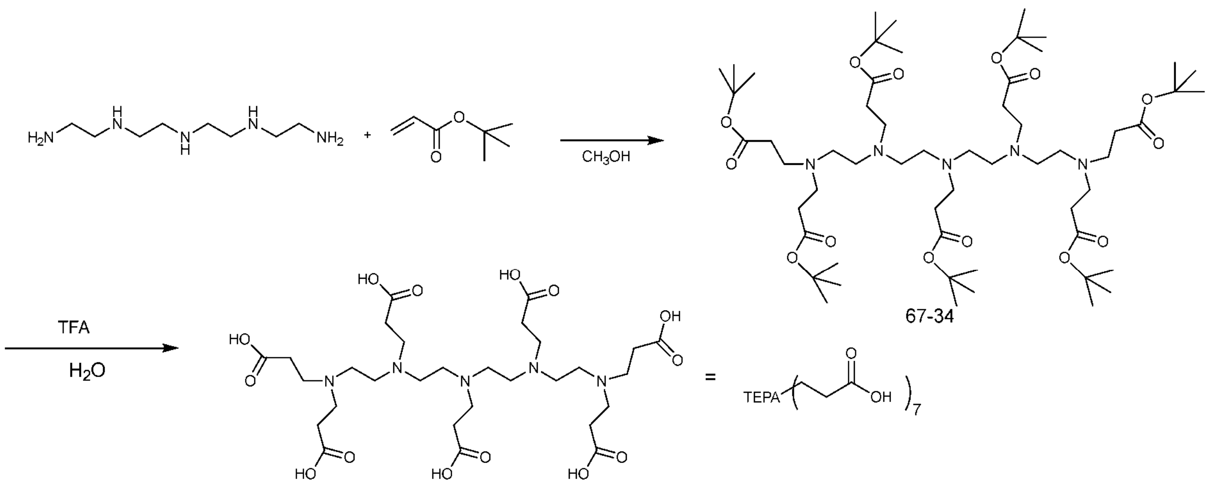


67-34

67-53

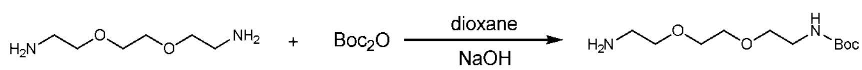


67-82

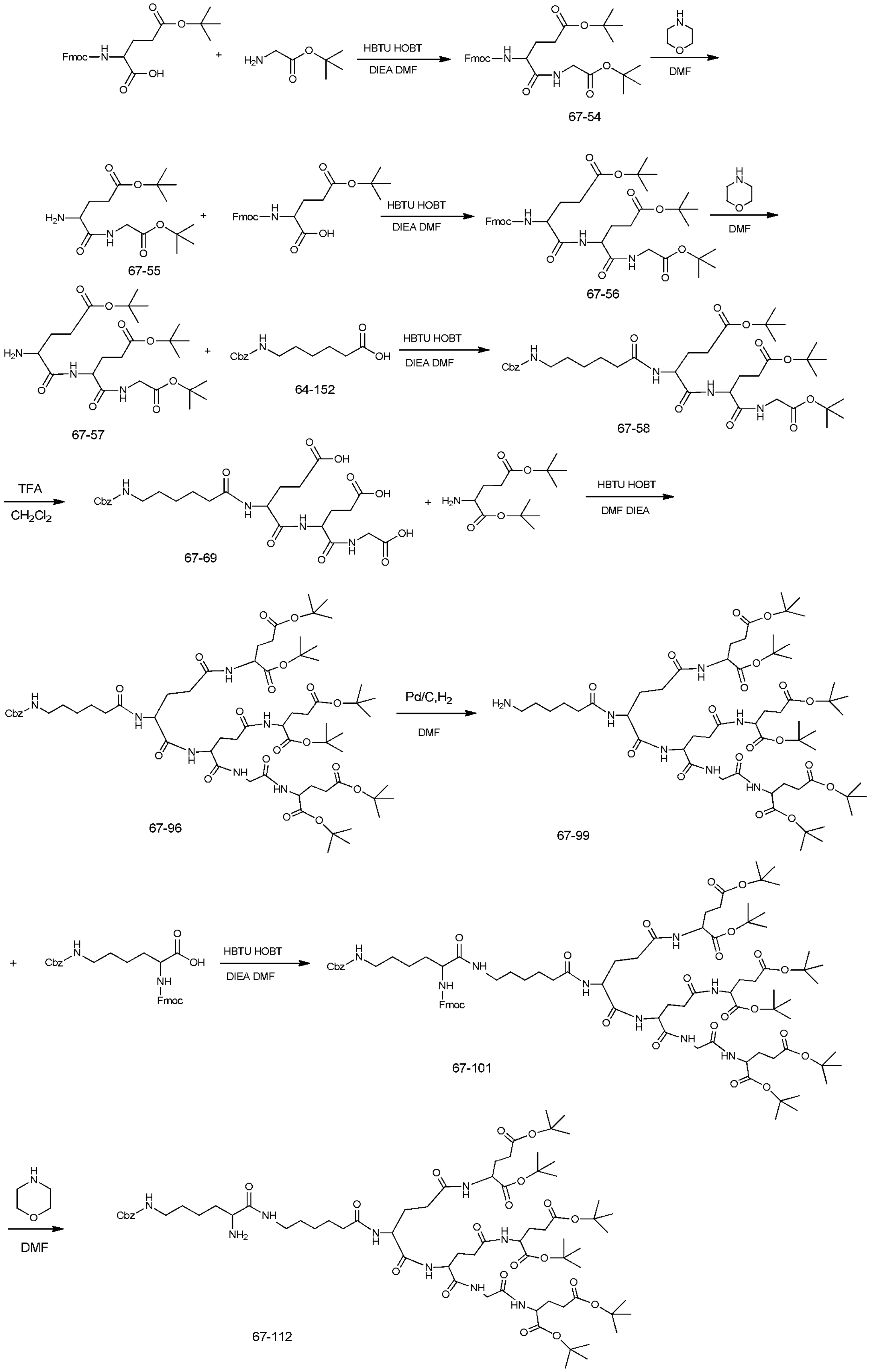


67-54

67-55

67-56

67-57

64-152

67-58

67-69

67-96

67-99

67-101

67-112

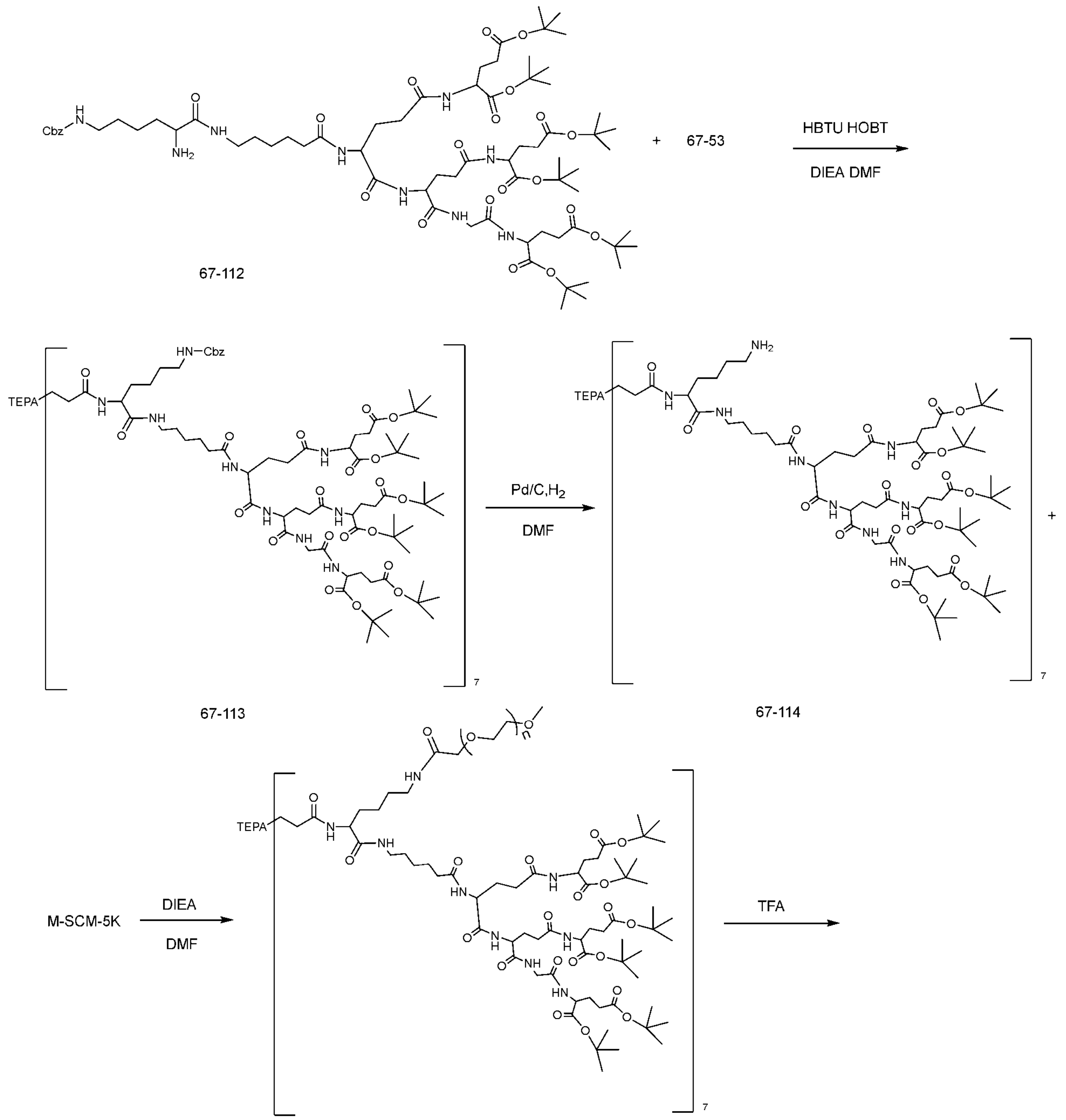


67-112

67-113

67-114

67-116

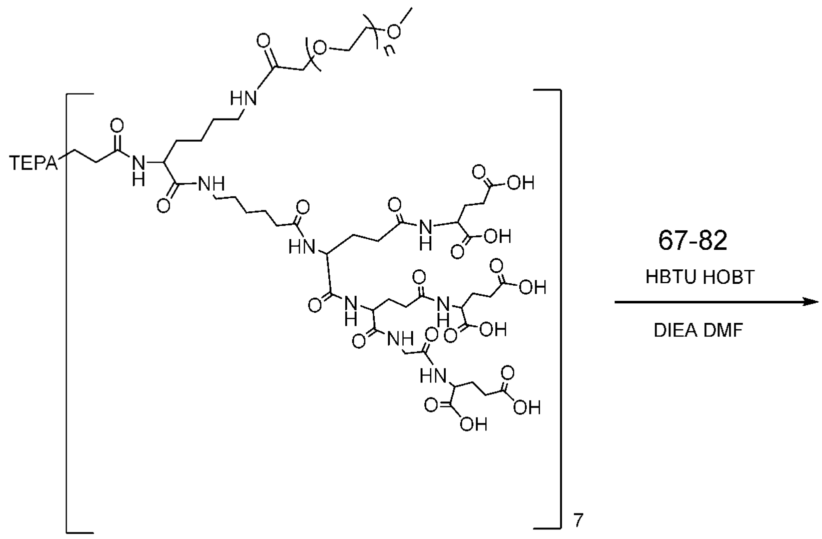


67-210

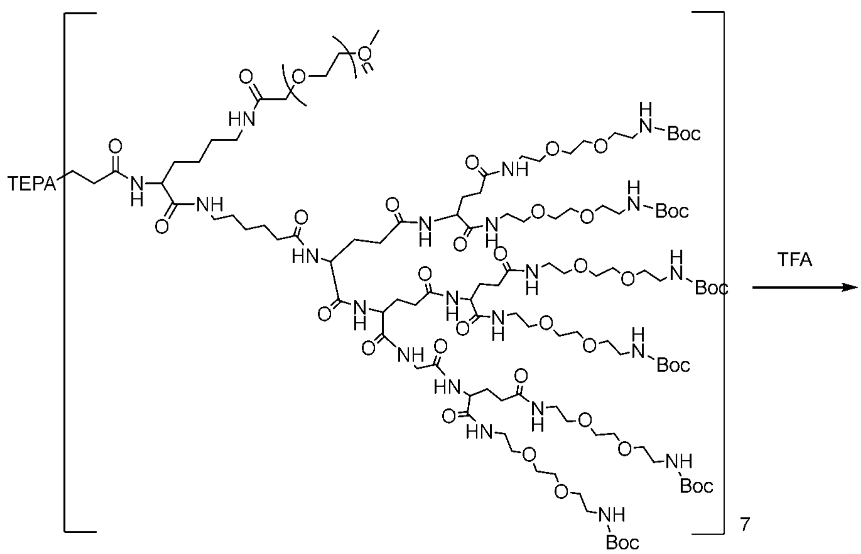


67-213

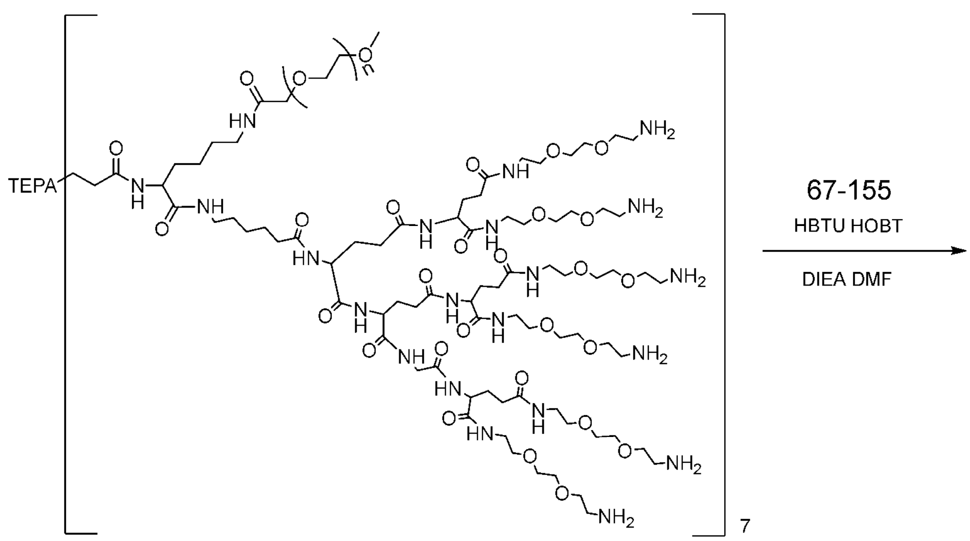


67-216

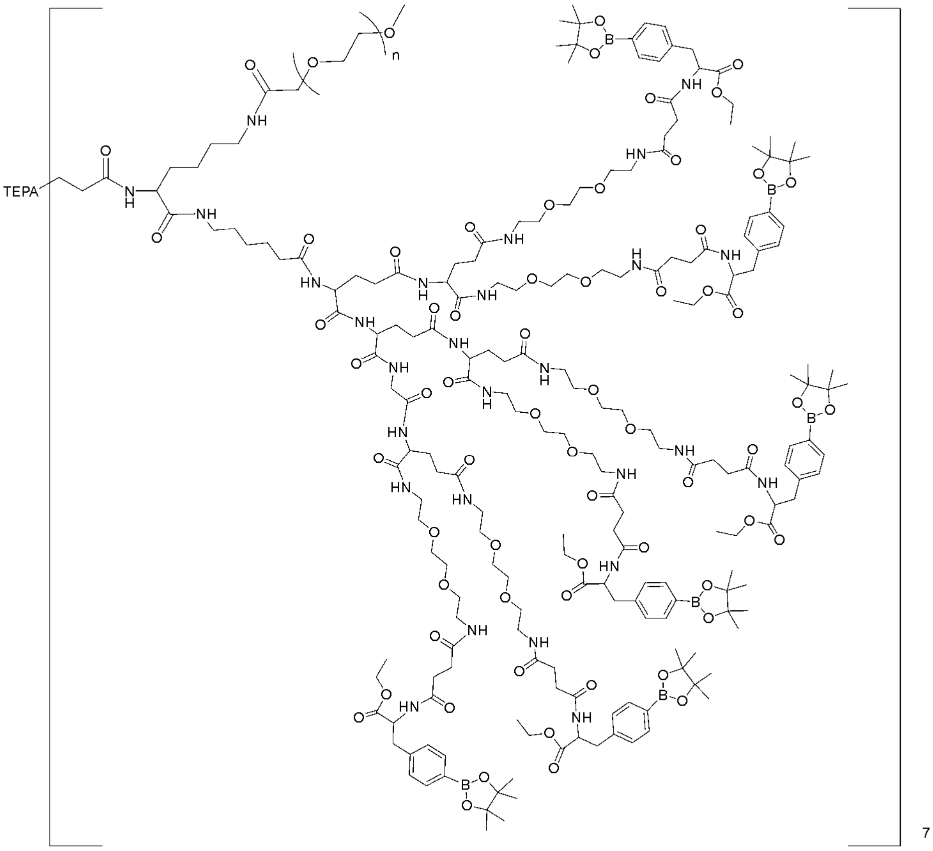

67-228

3.1 Preparation of **67-144**

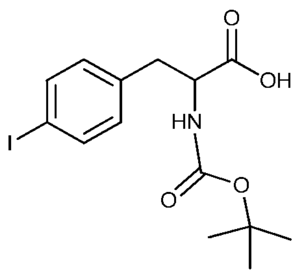

H-Phe(4-I)-OH (5.00 g, 17.176 mmol) was placed in a 500 mL round-bottom flask, then THF/$H_2O$ solution (150 mL) was added for dissolution, and the mixture was stirred in a reaction bath at 0 °C for 1 hour. Anhydrous sodium carbonate (3.64 g, 34.353 mmol) was then added, and dissolved with the assistance of ultrasonication. The reaction flask was kept in a reaction bath at 0 °C, and stirring was continued for 30 minutes. Subsequently, di-tert-butyl dicarbonate (7.49 g, 34.353 mmol) was dissolved in THF (30 mL) and slowly added dropwise to the reaction flask. After the dropwise addition was completed, the reaction flask was taken out from the reaction bath, and stirring was continued at room temperature for 3 hours. After the reaction was completed, the THF was evaporated to dryness, and methyl tert-butyl ether (150 mL) was added for extraction. The organic and aqueous phases were separated. 1N aqueous hydrochloric acid (70 mL) was added dropwise to the aqueous phase to adjust the pH to 2.0. Ethyl acetate (150 mL) was added for extraction, and the aqueous and organic phases were separated. The organic phases were combined, concentrated under reduced pressure, and then dissolved in dichloromethane (100 mL). Silica gel powder (30 mL) was added, and the mixture was evaporated to dryness. The resulting product was dry-loaded onto the column and subjected to column chromatography using 20-50% ethyl acetate/petroleum ether as the eluent. The desired product was collected, concentrated, and dried to provide 6.0 g of the product with a yield of 89.41%.

3.2 Preparation of **67-145**

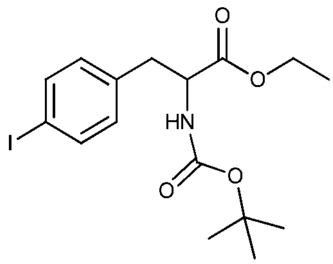

DMAP (0.37 g, 3.067 mmol) and ethanol (0.7 mL, 15.337 mmol) were added to a 500 mL round-bottom flask containing **67-144** (6.0 g, 15.337 mmol), then dichloromethane (200 mL) was added for dissolution with the assistance of ultrasonication, and the mixture was stirred at -5°C for 30 minutes. DCC (15.822 g, 76.685 mmol) was then added in three batches with an interval of 5 minutes. The reaction was continued for 3 hours. The reaction progress was monitored by TLC. After the reaction was completed, the mixture was filtered, the filtrate was collected and concentrated, then a n-hexane/petroleum ether (4/1) mixture was added. The mixture was filtered, and a crude solid product was collected. This process was repeated three times. After being evaporated to dryness, the mixture was dissolved in a methanol/dichloromethane mixture, then 200-300 mesh silica gel powder (40 mL) was added, and the mixture was evaporated to dryness. The resulting product was dry-loaded onto the column and subjected to column chromatography using 10% ethyl acetate/petroleum ether as the eluent. The desired product was collected, concentrated, and dried to provide 6.1 g of the product.

3.3 Preparation of **67-147**

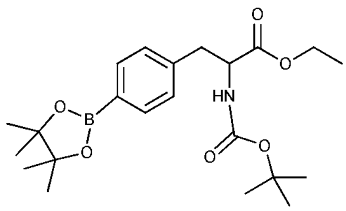

**67-145** (6.1 g, 14.549 mmol) and bis(pinacolato)diboron (8.12 g, 32.009 mmol) were dissolved in DMSO (60 mL), then Pd(dppf)$Cl_2$ solution (1.06 g, 1.454 mmol) and potassium acetate (5.99 g, 61.109 mmol) were added, and the mixture was stirred at 80°C under nitrogen for 5 hours. After the reaction was completed, ethyl acetate (300 mL × 3) was added for extraction. The organic phases were combined, washed with pure water (200 mL) and saturated brine (200 mL × 2), dried over anhydrous sodium sulfate, filtered and concentrated, and then dissolved in a methanol/dichloromethane mixture. 200-300 mesh silica gel powder (60 mL) was added, and the mixture was evaporated to dryness. The resulting product was dry-loaded onto the column and subjected to column chromatography using 30-50% ethyl acetate/petroleum ether as the eluent. The desired product was collected, concentrated, and dried to provide 5.56 g of the product with a yield of 91.14%.

3.4 Preparation of **67-154**

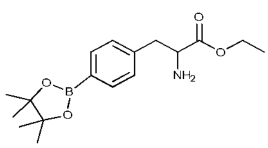

**67-147** (5.56 g, 13.259 mmol) was dissolved in 4M 1,4-dioxane hydrochloric acid (66.2 mL, 265.18 mmol) and stirred for 2 hours. After the reaction was completed, dichloromethane (30 mL) was added and evaporated to dryness.

When the rotary evaporation stopped, dichloromethane (30 mL) was further added for dissolution and then evaporated to dryness. This process was repeated eight times to provide 4.12 g of the product.

3.5 Preparation of **67-155**

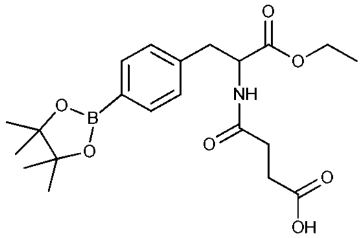

To a 500 mL flask containing **67-154** (4.12 g, 12.907 mmol), DIEA (8.53 mL, 51.629 mmol), acetonitrile (20 mL), and succinic anhydride (3.87 g, 38.721 mmol, purchased from Innochem) was added, and the reaction was stirred for 12 hours. The reaction progress was monitored by TLC plate. After the reaction was completed, the acetonitrile was evaporated, then a methanol/dichloromethane mixture was added for dissolution. 200-300 mesh silica gel powder (40 mL) was added, and the mixture was evaporated to dryness. The resulting product was dry-loaded onto the column and subjected to column chromatography using 2-5% methanol/dichloromethane as the eluent. The desired product was collected, concentrated, and dried to provide 4.9 g of the product. $^1$H-NMR (600 MHz, DMSO-$d_6$) δ 12.09 (s, 1H), 8.33 (d, *J* = 7.7 Hz, 1H), 7.59 (d, *J* = 8.0 Hz, 2H), 7.23 (d, *J* = 8.0 Hz, 2H), 4.42 (td, *J* = 8.3, 6.0 Hz, 1H), 4.06-4.01 (m, 2H), 2.96 (ddd, *J* = 22.6, 13.7, 7.4 Hz, 2H), 2.37-2.30 (m, 4H), 1.29 (s, 12H), 1.11 (t, *J* = 7.1 Hz, 3H)

3.6 Preparation of **67-34**

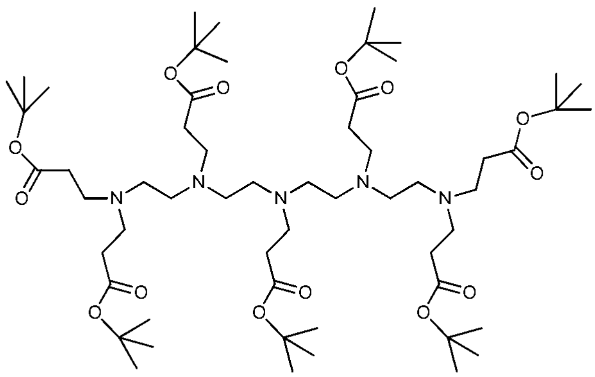

Tert-butyl acrylate (10.7242 g, 83.672 mmol) was added to a reaction flask and dissolved in 20 mL of methanol. The reaction mixture was placed in the dark and cooled to 0°C. Tetraethylenepentamine (1.98 g, 10.459 mmol) was then dissolved in 5 mL of methanol, and slowly added dropwise to the reaction flask. The reaction mixture was allowed to react overnight at 1°C. The reaction mixture was then warmed to room temperature and the reaction was continued at room temperature for 24 hours. After the reaction was completed, the reaction mixture was evaporated to dryness, then dissolved in a dichloromethane/methanol mixture. 200-300 mesh silica gel powder was added, and the mixture was evaporated to dryness. The resulting product was dry-loaded onto the column and subjected to column chromatography using a 0-4% methanol/dichloromethane mixture as the eluent. The desired product was collected, concentrated, and dried to provide 8.9 g of the product with a yield of 78.34%. $^1$H-NMR (600 MHz, DMSO-$d_6$) δ 2.67-2.60 (m, 14H), 2.43-2.42 (d, *J* = 3.8 Hz, 16H), 2.28-2.26 (t, *J* = 6.8 Hz, 14H), 1.39 (d, *J* = 1.3 Hz, 63H)

3.7 Preparation of **67-53**

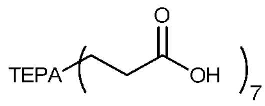

**67-34** (2.4 g, 2.208 mmol) was added to a 100 mL flask, then one drop of water and TFA (22 mL, 309.255 mmol) were added for dissolution, and the mixture was reacted with stirring at room temperature. After the reaction was completed, most of the TFA was evaporated using a rotary evaporator to provide a viscous oily liquid, which was dissolved in methanol (10 mL) and evaporated using a rotary evaporator. This process was repeated 6 times. Then, n-hexane and methyl tert-butyl ether were added for precipitation to provide a white solid. The solid was filtered and dried in an oven to provide 1.53 g of the product. $^1$H-NMR (600 MHz, DMSO-$d_6$) δ11.0 (s, 7H), 3.33-3.07 (m, 18H), 2.89 (dd, $J$ = 29.8, 5.2 Hz, 10H), 2.72 (s, 8H), 2.51 (s, 8H)

3.8 Preparation of **67-82**

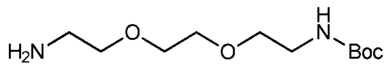

1,2-Bis(2-aminoethoxy)ethane (20.2 g, 136.2931 mmol) was weighed into a 500 mL flask, then dichloromethane (100 mL) was added for dissolution, and then triethylamine (40 mL) was added. The mixture was placed in a reaction bath at 0 °C. A solution of $Boc_2O$ (17.85 g, 81.7758 mmol) in dichloromethane (100 mL) was slowly added dropwise. After the dropwise addition was completed, the reaction was carried out with stirring at room temperature overnight. After the reaction was completed, the reaction mixture was concentrated under reduced pressure, and then a mixture of methanol and dichloromethane was added for dissolution. The resulting product was dry-loaded onto the column and subjected to column chromatography using 0.5% aqueous ammonia/1-3% methanol/dichloromethane as the eluent. The desired product was collected, concentrated, and dried to provide 12.4 g of the product with a yield of 36.64%.

3.9 Preparation of **67-54**

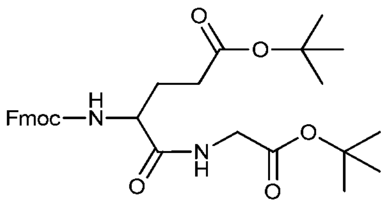

Fmoc-L-glutamic acid-5-tert-butyl ester (10 g, 23.514 mmol), HBTU (13.376 g, 35.271 mmol), and HOBT (4.765 g, 35.271 mmol) were weighed and added to a flask containing glycine tert-butyl ester hydrochloride (3.94 g, 23.514 mmol), then DMF (300 mL) was added to dissolve the mixture, and the mixture was allowed to stand at -5°C. DIEA (17.5 mL, 105.813 mmol) was slowly added dropwise. After the dropwise addition was completed, the reaction was carried out for half an hour, the flask was then taken out, and the reaction was carried out with stirring at room temperature overnight. After the reaction was completed, saturated NaCl solution (300 mL) and ethyl acetate (300 mL) were added for extraction. After standing for phase separation, the organic phase was collected, and the aqueous phase was further extracted with ethyl acetate (200 mL × 3). The organic phases were combined, evaporated to dryness to provide a solid, which was dried in a vacuum oven to provide the product.

3.10 Preparation of **67-55**

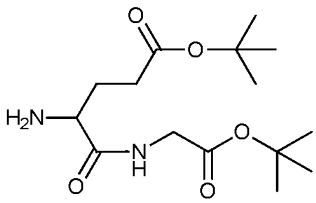

**67-54** (12.66 g, 23.514 mmol) was placed in a 500 mL round-bottom flask. DMF (150 mL) was added for dissolution, then morpholine (40.97 mL, 470.28 mmol) was added, and the mixture was reacted with stirring at room temperature for 2 hours. After the reaction was completed, saturated NaCl solution (300 mL) and ethyl acetate (300 mL) were added for extraction. After standing for phase separation, the organic phase was collected, and the aqueous phase was further extracted with ethyl acetate (200 mL × 3). The organic phases were combined, evaporated to dryness to provide a solid, which was dried in a vacuum oven to provide the product.

3.11 Preparation of **67-56**

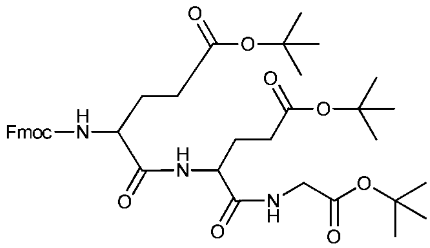

Fmoc-L-glutamic acid-5-tert-butyl ester (9.68 g, 22.756 mmol), HBTU (12.945 g, 34.135 mmol), and HOBT (4.61 g, 34.135 mmol) were weighed and added to a flask containing **67-55** (7.2 g, 22.756 mmol), then DMF (300 mL) was added to dissolve the mixture. The mixture was allowed to stand at -5°C. DIEA (8.2 mL, 50.063 mmol) was slowly added dropwise. After the dropwise addition was completed, the reaction was carried out for half an hour, then the flask was then taken out, and the reaction was carried out with stirring at room temperature overnight. After the reaction was completed, saturated NaCl solution (300 mL) and EA (ethyl acetate) (300 mL) were added for extraction. After standing for phase separation, the organic phase was collected, and the aqueous phase was further extracted with EA (ethyl acetate) (200 mL × 3). The organic phases were combined, evaporated to dryness to provide a solid, which was dried in a vacuum oven to provide the product.

3.12 Preparation of **67-57**

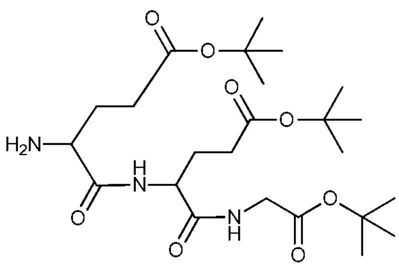

**67-56** (16.47 g, 22.756 mmol) was added into a 500 mL round-bottom flask. DMF (200 mL) was added for dissolution, then morpholine (39.65 mL, 455.12 mmol) was added, and the mixture was reacted with stirring at room temperature for 2 hours. After the reaction was completed, saturated NaCl solution (300 mL) and ethyl acetate (300 mL) were added for extraction. After standing for phase separation, the organic phase was collected, and the aqueous phase was further extracted with ethyl acetate (200 mL × 3). The organic phases were combined, evaporated to dryness to provide a solid, which was dried in a vacuum oven to provide the product.

3.13 Preparation of **67-58**

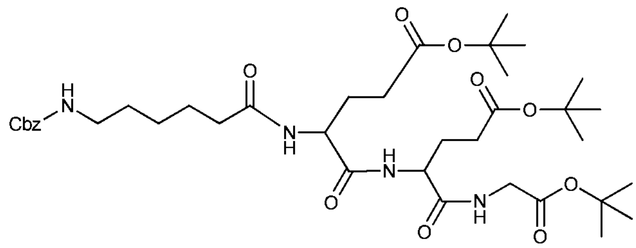

**67-57** (5.71 g, 11.378 mmol), HBTU (6.47 g, 17.067 mmol), and HOBT (2.31 g, 17.067 mmol) were weighed and added to a flask containing **64-152** (3.02 g, 11.378 mmol), then DMF (200 mL) was added to dissolve the mixture. The mixture was allowed to stand at -5°C. DIEA (5.64 mL, 34.134 mmol) was slowly added dropwise. After the dropwise addition, the reaction was carried out for half an hour, then the flask was then taken out, and the reaction was carried out with stirring at room temperature overnight. After the reaction was completed, saturated NaCl solution (300 mL) and ethyl acetate (300 mL) were added for extraction. After standing for phase separation, the organic phase was collected, and the aqueous phase was further extracted with ethyl acetate (200 mL $\times$ 3). The organic phases were collected and combined, evaporated to dryness to provide a solid, which was then dissolved in a methanol/dichloromethane (1/4) mixture, dry-loaded onto the column and subjected to column chromatography using 40-60% ethyl acetate/petroleum ether as the eluent. The desired product was collected, concentrated, and dried in a vacuum oven to provide 4.5 g of the product with a yield of 52.94%.

3.14 Preparation of **67-69**

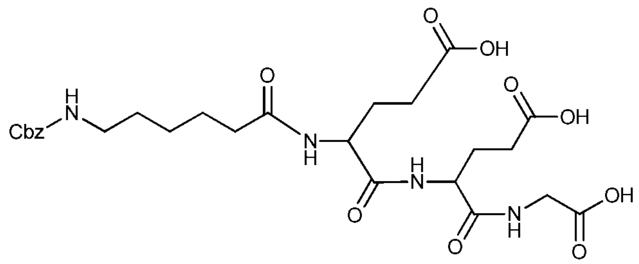

To a flask containing **67-58** (4.5 g, 6.008 mmol), dichloromethane (20 mL) was added. After dissolution was performed with the assistance of ultrasonication, TFA (26.77 mL, 360.52 mmol) was added, and the reaction was carried out with stirring at room temperature overnight. After the reaction was completed, the reaction mixture was concentrated under reduced pressure to remove dichloromethane and most of the TFA. Then, n-hexane (50 mL) and methyl tert-butyl ether (200 mL) were added for precipitation, resulting in an oily solid. The supernatant was discarded, and methyl tert-butyl ether (200 mL) was added for precipitation. This process was repeated three times. A powdery solid precipitated and was filtered. The filter cake was washed three times with methyl tert-butyl ether (60 mL). The filter cake was collected and dried under vacuum to provide 1.6 g of the product with a yield of 45.98%.

3.15 Preparation of **67-96**

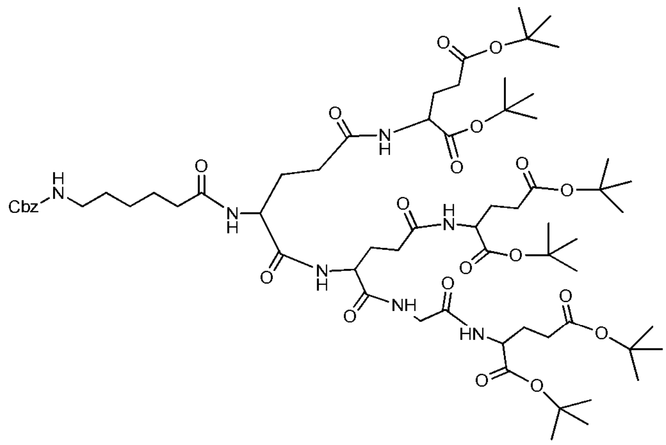

**67-69** (1.6 g, 2.392 mmol), HOBT (1.454 g, 10.764 mmol), and HBTU (4.082 g, 10.764 mmol) were weighed and added to a flask containing di-tert-butyl glutamate (2.22 g, 7.534 mmol, purchased from Adamas), and then DMF (150 mL) was added for dissolution, and the mixture was allowed to stand at -5°C. DIEA (3.5 mL, 21.528 mmol) was slowly added dropwise. After the dropwise addition, the reaction was carried out for half an hour, then the flask was taken out, and the reaction was carried out with stirring at room temperature overnight. After the reaction was completed, saturated NaCl solution (300 mL) and ethyl acetate (300 mL) were added for extraction. After standing for phase separation, the organic phase was collected, and the aqueous phase was further extracted with ethyl acetate (200 mL × 3). The organic phases were combined, evaporated to dryness and dried in a vacuum oven to provide 3.6 g of the product.

3.16 Preparation of **67-99**

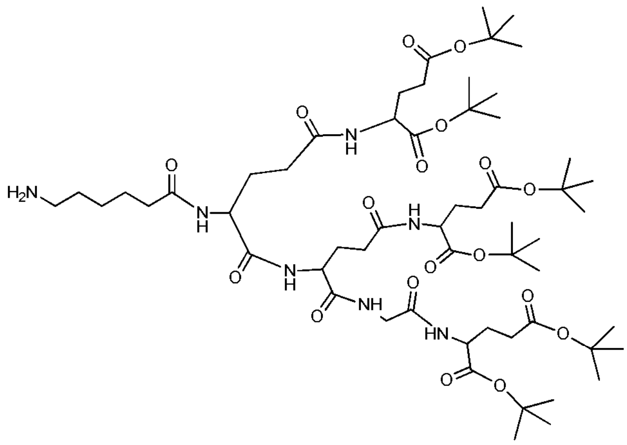

**67-96** (3.2 g, 2.297 mmol) was placed in a hydrogenation reactor, and 10% Pd/C catalyst (0.04 g) and DMF (30 mL) were added. The reactor was evacuated with a water pump, and then hydrogen gas was introduced. This process was repeated three times. The pressure reading in the hydrogenation reactor was adjusted to 2 MPa, and the mixture was stirred at room temperature overnight. After the reaction was completed, the reaction mixture was filtered through Celite, and the filter cake was washed with DMF (5 mL). The filtrate was transferred to a 500 mL round-bottom flask as the raw material for the next step.

3.17 Preparation of **67-101**

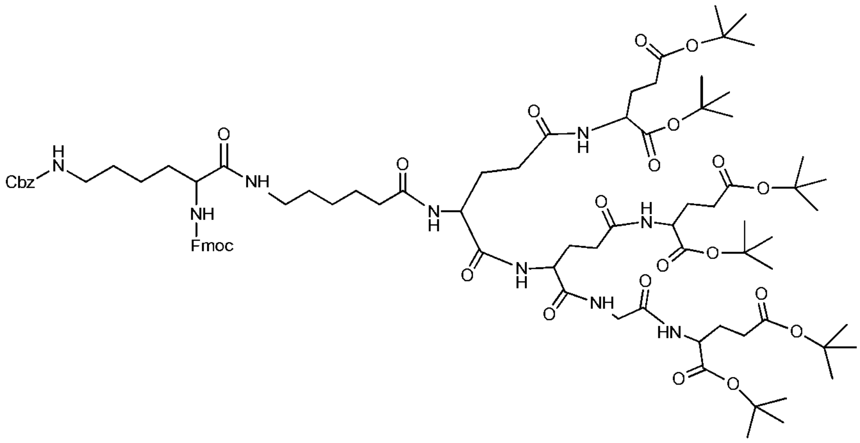

**67-99** (2.5 g, 2.135 mmol, converted), HOBT (0.43 g, 3.203 mmol), and HBTU (1.21 g, 3.203 mmol) were weighed and added to a flask containing N'-Fmoc-N-benzyloxycarbonyl-L-lysine (1.07 g, 2.135 mmol, purchased from Adamas), then DMF (100 mL) was added to dissolve the mixture. The mixture was allowed to stand at -5°C. DIEA (1.0 mL, 6.405 mmol) was slowly added dropwise. After the dropwise addition was completed, the reaction was carried out for half an hour, the flask was then taken out, and the reaction was carried out with stirring at room temperature overnight. After the reaction was completed, n-hexane (50 mL) and methyl tert-butyl ether (200 mL) were added for precipitation, resulting in an oily solid. The supernatant was discarded, and methyl tert-butyl ether (200 mL) was added for precipitation. This process was repeated three times. A powdery solid precipitated and was filtered. The filter cake was washed three times with methyl tert-butyl ether (60 mL). The filter cake was collected and dissolved in a methanol/dichloromethane mixture, dry-loaded onto the column and subjected to column chromatography using 1-5% methanol/dichloromethane as the eluent. The desired product was collected, concentrated, and dried in a vacuum oven to provide 2.3 g of the product with a yield of 64.61%.

3.18 Preparation of **67-112**

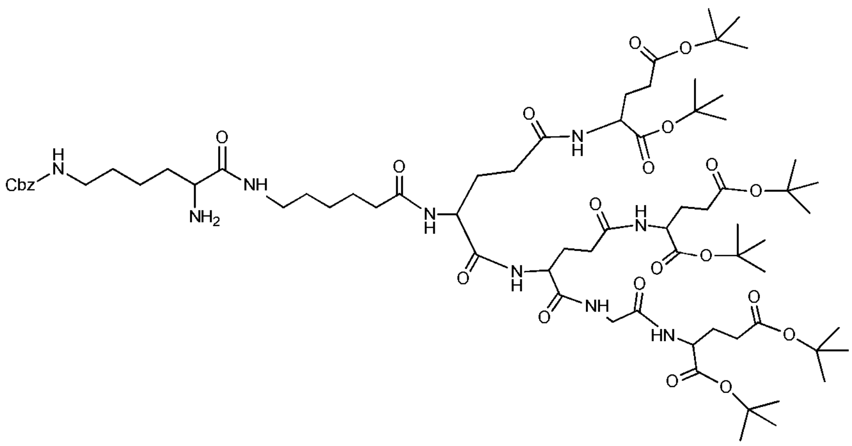

**67-101** (1.1 g, 0.647 mmol) was placed in a 500 mL round-bottom flask. DMF (50 mL) was added for dissolution, then morpholine (1.2 mL, 12.948 mmol) was added, and the mixture was reacted with stirring at room temperature for 2 hours. After the reaction was completed, saturated NaCl solution (300 mL) and ethyl acetate (300 mL) were added for extraction. After standing for phase separation, the organic phase was collected, and the aqueous phase was further extracted with ethyl acetate (200 mL × 3). The organic phases were combined, evaporated to dryness to provide a solid, which was dried in a vacuum oven to provide the product.

3.19 Preparation of **67-113**

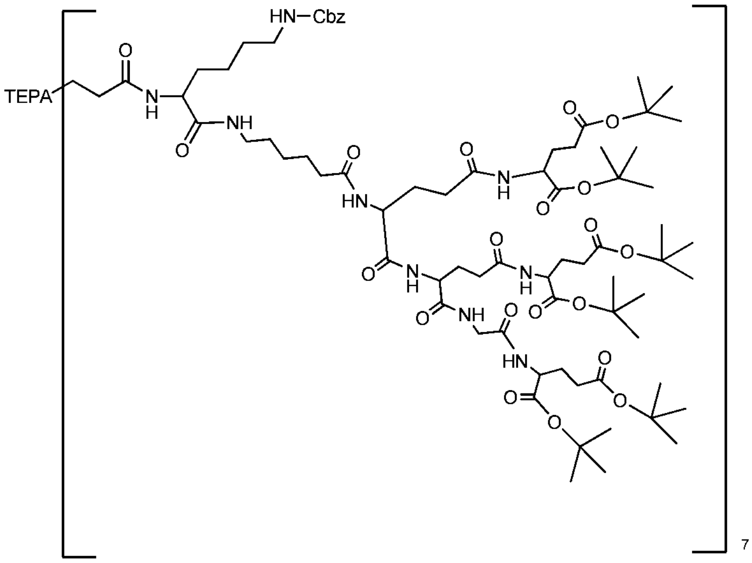

**67-112** (0.936 g, 0.647 mmol), HOBT (0.087 g, 0.647 mmol), and HBTU (0.245 g, 0.647 mmol) were weighed and added to a flask containing **67-53** (0.042 g, 0.0616 mmol), and then DMF (50 mL) was added to dissolve the mixture. The mixture was allowed to stand at -5°C. DIEA (0.3 mL, 1.941 mmol) was slowly added dropwise. After the dropwise addition was completed, the reaction was carried out for half an hour, then the flask was taken out, and the reaction was carried out with stirring at room temperature overnight. After the reaction was completed, n-hexane (50 mL) and methyl tert-butyl ether (200 mL) were added for precipitation, resulting in an oily solid. The supernatant was discarded, and methyl tert-butyl ether (200 mL) was added for precipitation. This process was repeated three times. A powdery solid precipitated and was filtered. The filter cake was washed three times with methyl tert-butyl ether (60 mL). The filter cake was collected and dried in a vacuum oven to provide 0.6 g of the product.

3.20 Preparation of **67-114**

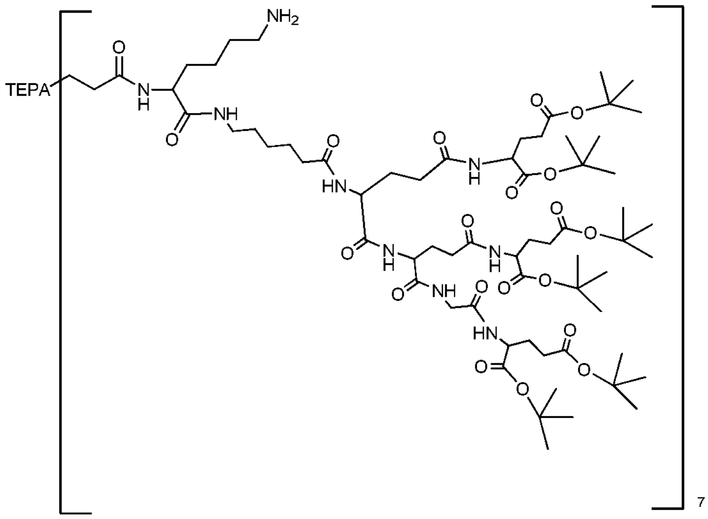

**67-113** (0.6 g, 0.0566 mmol) was placed in a hydrogenation reactor, then 10% Pd/C catalyst (0.05 g) was added, and DMF (10 mL) was added for dissolution. The reactor was evacuated with a water pump, and then hydrogen was introduced. This process was repeated three times. The pressure reading in the hydrogenation reactor was adjusted to 2 MPa, and the reaction was carried out with stirring at room temperature overnight. After the reaction was completed, the reaction mixture was filtered through Celite, the filter cake was washed with DMF (10 mL × 3). The filtrate was collected, and n-hexane (50 mL) and methyl tert-butyl ether (200 mL) were added for precipitation to provide an oily solid. The supernatant was discarded, and then methyl tert-butyl ether (200 mL) was added for precipitation. This process was

repeated three times. A powdery solid precipitated and was filtered. The filter cake was washed three times with methyl tert-butyl ether (60 mL). The filter cake was collected and dried in a vacuum oven to provide 0.19 g of the product. $^1$H-NMR (600 MHz, DMSO-$d_6$) δ 8.30-7.79 (m, 55H), 5.11 (s, 14H), 4.25-4.09 (m, 39H), 3.89-3.77 (m, 10H), 3.73-3.65 (m, 8H), 3.63-3.55(m, 7H), 3.48-3.42 (m, 14H), 2.97-2.92 (m, 7H), 2.80-2.71 (m, 28H), 2.31-2.07 (m, 48H), 2.19-2.06 (m,46H), 1.96-1.82 (m,40H), 1.81-1.66 (m, 44H), 1.53-1.45 (m, 34H), 1.39 (s, 378H), 1.28-1.15 (m, 42H)

3.21 Preparation of **67-116**

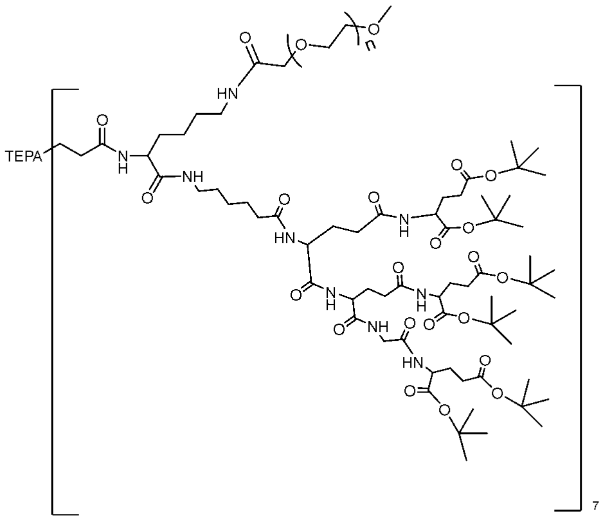

**67-114** (0.19 g, 0.0196 mmol) was dissolved in anhydrous DMF (20 mL) in a 250 mL round-bottom flask, followed by a dropwise addition of DIEA (0.9 mL, 5.4488 mmol), and the mixture was stirred for 30 minutes. M-SCM-5K (0.8 g, 0.151 mmol, purchased from Jenkem, Lot Number: ZZ363P139) was then added, dissolved with the assistance of ultrasonication, and reacted with stirring at low speed at room temperature for 4 days in the dark. After the reaction was completed, n-hexane (50 mL) and methyl tert-butyl ether (200 mL) were added for precipitation, resulting an oily solid. The supernatant was discarded, and methyl tert-butyl ether (200 mL) was added for precipitation. This process was repeated three times. A powdery solid precipitated and was filtered. The obtained solid was dissolved in a methanol/dichloromethane mixture, then silica gel powder was added, and then the mixture was dried. The resulting product was dry-loaded onto the column and subjected to column chromatography using 1% ammonia water/5-10% methanol/dichloromethane as the eluent. The desired product was collected, concentrated, and dried in a vacuum oven to provide 0.45 g of the product. $^1$H-NMR (600 MHz, DMSO-$d_6$) δ 8.27-7.85 (m, 63H), 4.26-4.09 (m, 56H), 3.75-3.72 (m, 14H), 3.52-3.50 (m, 3269H), 3.24-3.20 (m, 28H), 2.90-2.69 (m, 30H), 2.32-2.30 (m, 84H), 2.16-2.10 (m, 56H), 1.795-1.757 (m, 14H), 1.517-1.471 (m, 28H), 1.402-1.378 (s, 378H), 1.25-1.23 (m, 56H)

3.22 Preparation of **67-210**

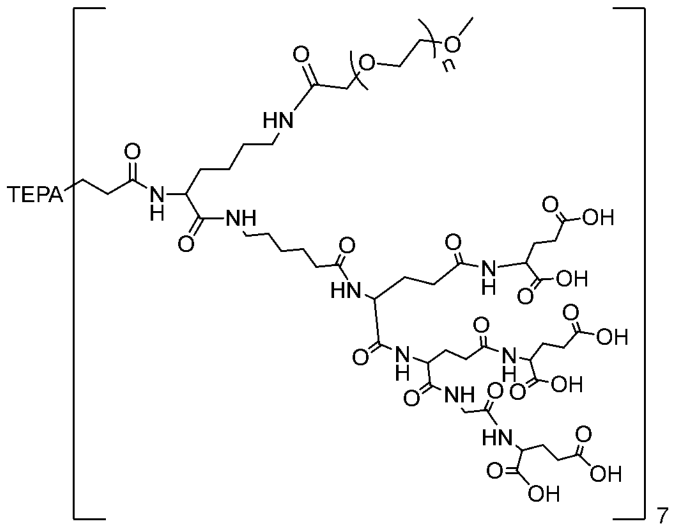

**67-116** (2.69 g, 0.0588 mmol) was dissolved in dichloromethane (4 mL), then TFA (3.6 mL, 49.405 mmol) was added, and the mixture was reacted with stirring at room temperature for two days. After the reaction was completed, the reaction mixture was concentrated under reduced pressure to remove the dichloromethane and most of the TFA. Then, n-hexane (50 mL) and methyl tert-butyl ether (200 mL) were added for precipitation, resulting in an oily solid. The supernatant was discarded, and methyl tert-butyl ether (200 mL) was then added for precipitation. This process was repeated three times. A powdery solid precipitated and was filtered. The filter cake was washed three times with methyl tert-butyl ether (60 mL). The filter cake was collected and dried under vacuum to provide 2.27 g of the product with a yield of 89.01%. $^1$H-NMR (600 MHz, DMSO-$d_6$) δ 12.84-11.70 (m, 42H), 8.31-8.02 (m, 63H), 4.25-4.16 (m, 56H), 3.85-3.80 (m, 14H), 3.735-3.687 (m, 14H), 3.52-3.50 (m, 3269H), 2.60-2.53(m, 14H), 2.37-2.34 (m, 16H), 2.31-2.08 (m, 154H), 1.792-1.770 (m, 14H), 1.52-1.47 (m, 28H), 1.25-1.23 (m, 56H)

3.23 Preparation of **67-213**

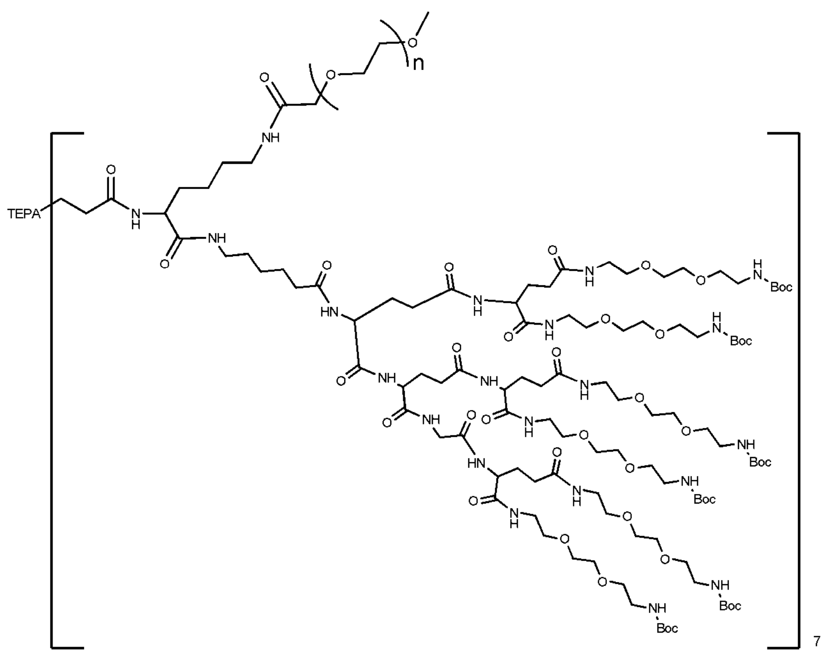

**67-210** (2.27 g, 0.052 mmol), **67-82** (0.81 g, 3.293 mmol), HBTU (1.24 g, 3.293 mmol), and HBTU (0.44 g, 3.293 mmol) were mixed in a 250 mL round-bottom flask. DMF (50 mL) was added to dissolve the mixture, then DIEA (1.6 mL, 9.879 mmol) was added, and the mixture was reacted with stirring at room temperature overnight. After the reaction was

completed, n-hexane (50 mL) and methyl tert-butyl ether (200 mL) were added for precipitation, resulting an oily solid. The supernatant was discarded, and dichloromethane (10 mL) was added for dissolution. Then, methyl tert-butyl ether (200 mL) was added for precipitation. This process was repeated three times. A powdery solid precipitated and was filtered. The filter cake was washed three times with methyl tert-butyl ether (60 mL). The filter cake was collected and vacuum dried to provide the product. $^1$H-NMR (600 MHz, DMSO-$d_6$) δ 8.03-7.91 (m, 147H), 4.18-4.15 (m, 49H), 3.63-3.55 (m, 350H), 3.52-3.50 (m, 3269H), 3.37-3.32 (m, 49H), 3.28-3.19 (m, 42H), 3.24-3.23 (m, 14H), 3.07-3.04 (m, 84H), 2.89-2.86 (m, 154H), 2.64-2.53 (m, 44H), 1.83-1.80 (m, 14H), 1.46-1.40 (m, 28H), 1.40-1.34 (m, 378H), 1.25-1.22 (m, 42H)

3.24 Preparation of **67-216**

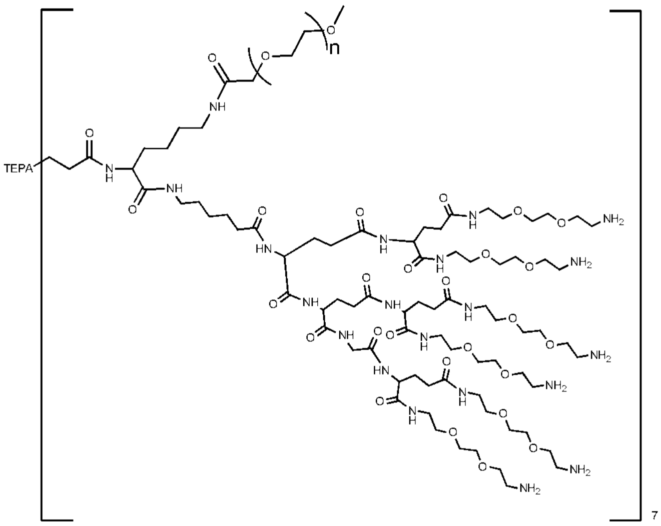

**67-213** (1.01 g, 0.019 mmol) was weighed and added into a 250 mL flask. TFA (11 mL, 159.66 mmol) was added, then a stirrer was added, and the mixture was reacted with stirring at room temperature overnight. After the reaction was completed, the reaction mixture was concentrated under reduced pressure, then dichloromethane (10 mL) was added for dissolution, and the mixture was neutralized with excess DIEA (0.5 mL). Methyl tert-butyl ether (100 mL) and n-hexane (50 mL) were added for precipitation, and an oily product was obtained and then dissolved in a methanol/dichloromethane mixture. Then, methyl tert-butyl ether (100 mL) and n-hexane (50 mL) were added for precipitation. This process was repeated five times to provide the product as an oil. $^1$H-NMR (600 MHz, DMSO-$d_6$) δ 8.05-7.98 (m, 105H), 5.11-4.99 (m, 84H), 4.18-4.12 (m, 49H), 3.62-3.59 (m, 350H), 3.52-3.50 (m, 3269H), 3.47-3.37 (m, 49H), 3.39-3.34 (m, 56H), 3.24-3.19 (m, 84H), 2.97-2.90 (m, 14H), 2.09-2.00 (m, 184H), 1.76-1.72 (m, 14H), 1.55-1.45 (m, 28H), 1.25-1.22 (m, 42H)

3.25 Preparation of **67-228**

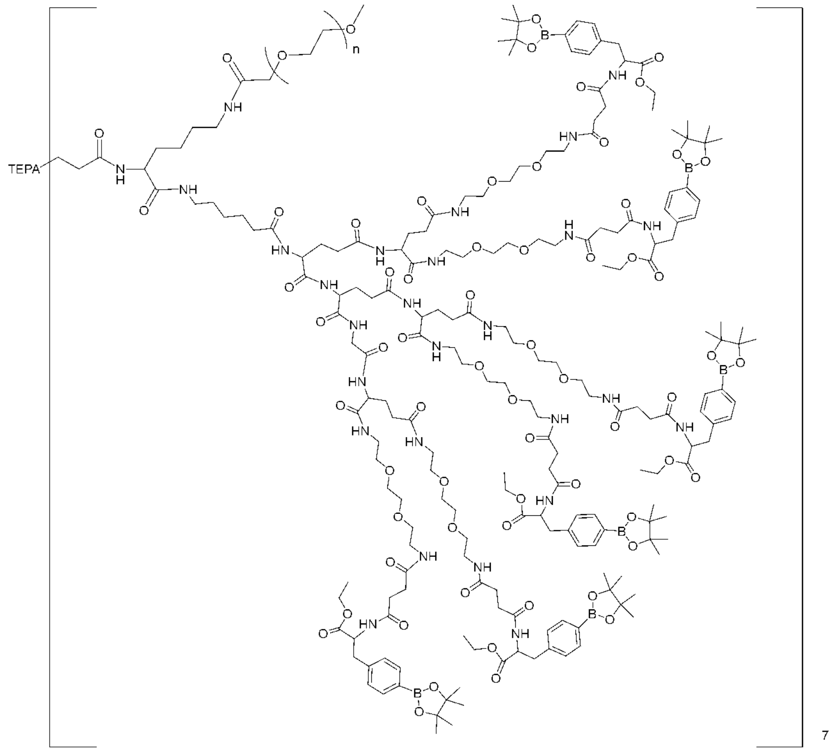

**67-216** (0.5 g, 0.0101 mmol), **67-155** (0.26 g, 0.6397 mmol), HOBT (0.086 g, 0.6397 mmol), and HBTU (0.24 g, 0.6397 mmol) were mixed in a 100 mL flask, and DMF (20 mL) was added for dissolution. DIEA (0.3 mL, 1.9191 mmol) was then added dropwise, and the mixture was reacted with stirring at room temperature overnight. After the reaction was completed, n-hexane (50 mL) and methyl tert-butyl ether (200 mL) were added for precipitation, resulting an oily solid. The supernatant was discarded, and methyl tert-butyl ether (200 mL) was added again for precipitation. This process was repeated three times. A powdery solid precipitated and was filtered. The filter cake was collected, dissolved with a methanol/dichloromethane mixture, then silica gel powder was added and the mixture was dried. The resulting product was dry-loaded onto the column and subjected to column chromatography using 1% ammonia water/8-12% methanol/dichloromethane as the eluent. The desired product was collected, concentrated, and dried in a vacuum oven to provide 0.24 g of the product. $^1$H-NMR (600 MHz, DMSO-$d_6$) δ 8.24-8.15 (m, 189H), 7.14-7.05 (m, 84H), 6.68-6.60 (m, 84H), 4.61-4.47 (m, 84H), 4.09-4.03 (m, 91H), 3.64-3.59 (m,350H), 3.53-3.49 (m, 3269H), 3.437-3.375 (m, 49H), 3.28-3.26 (m, 84H), 3.24-3.20 (m, 28H), 3.16-3.09 (m, 84H), 2.94-2.85 (m, 42H), 2.75-2.71 (m, 114H), 2.40-2.31 (m, 84H), 2.00-1.96 (m, 154H), 1.52-1.43 (m, 42H), 1.295-1.287 (m, 42H), 1.25-1.20 (m, 504H), 1.215-1.187 (m, 126H)

Example 4: Synthesis of Compound **81-118**

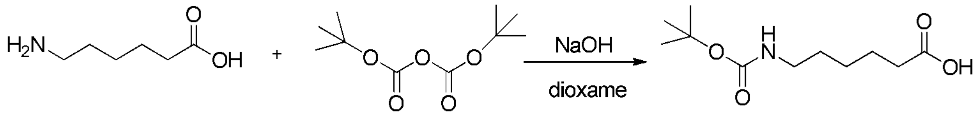


62-13

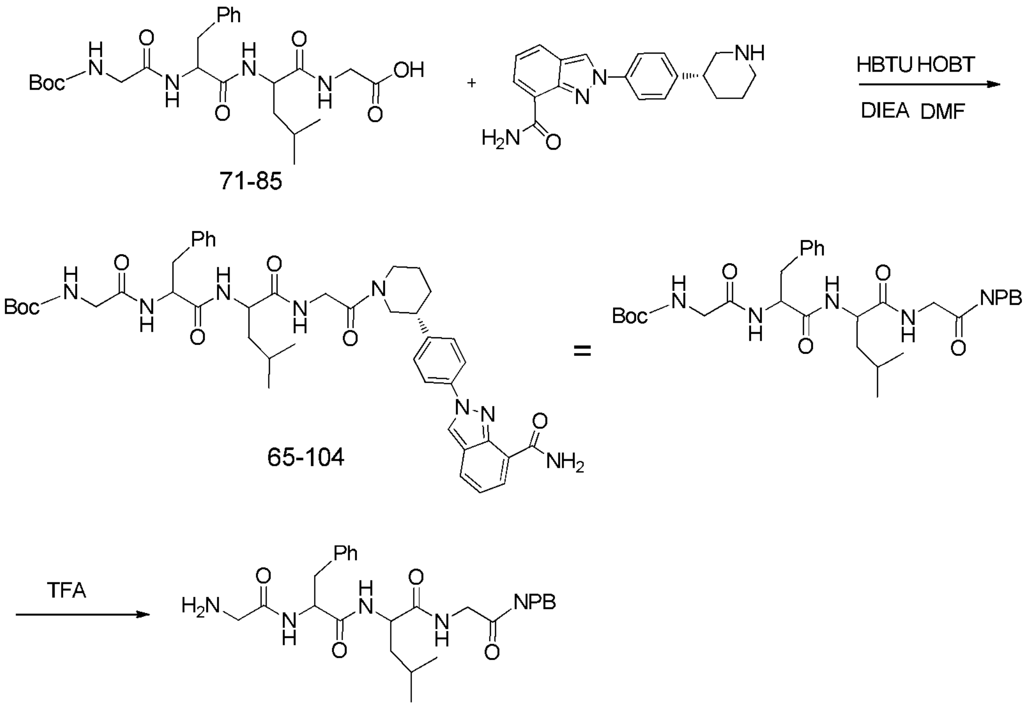


71-85

65-104

62-174

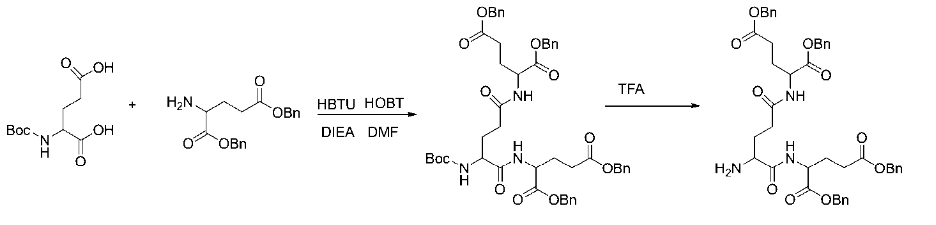


62-1

62-2

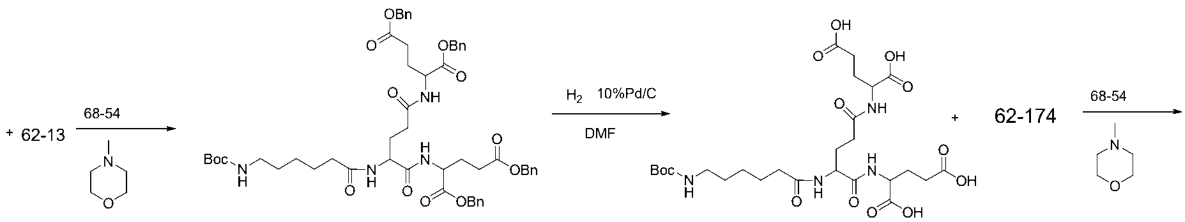


61-213

62-156

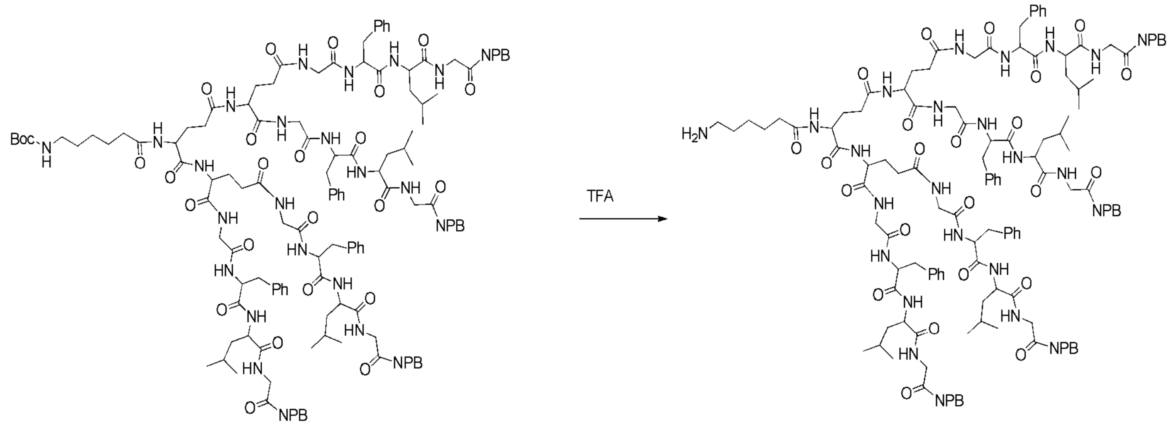


62-180

81-43

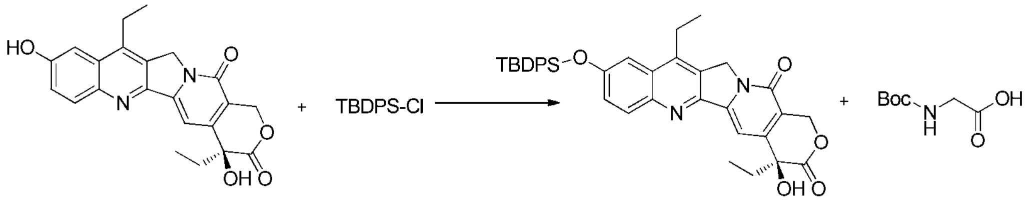


62-150

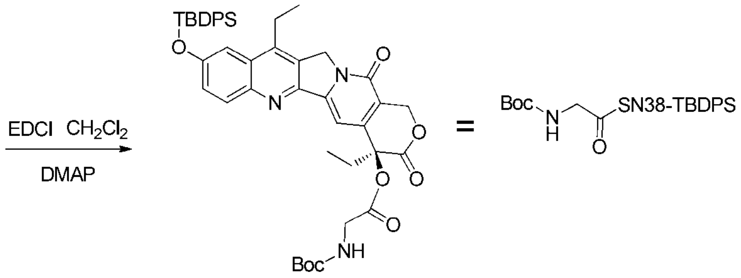


62-153

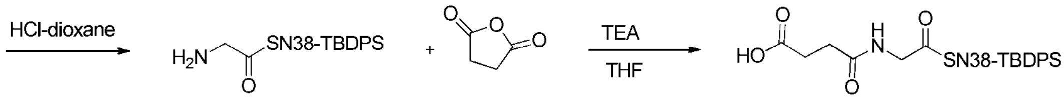


62-155

65-131

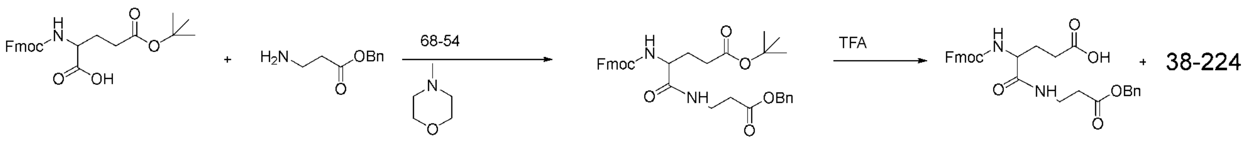


+ 38-224

69-33

69-34

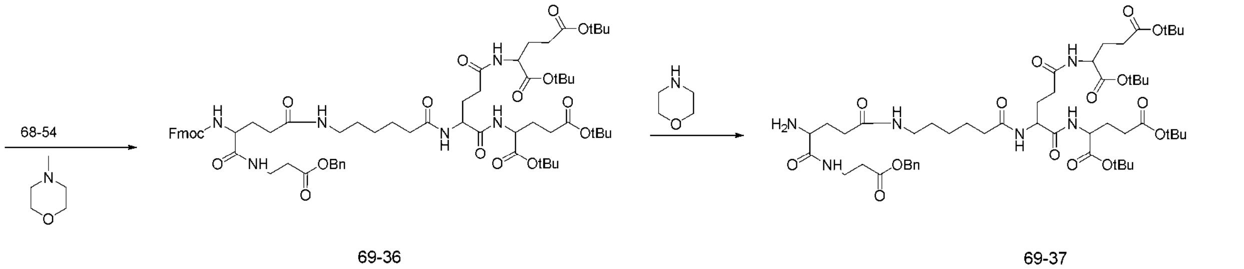


69-36

69-37

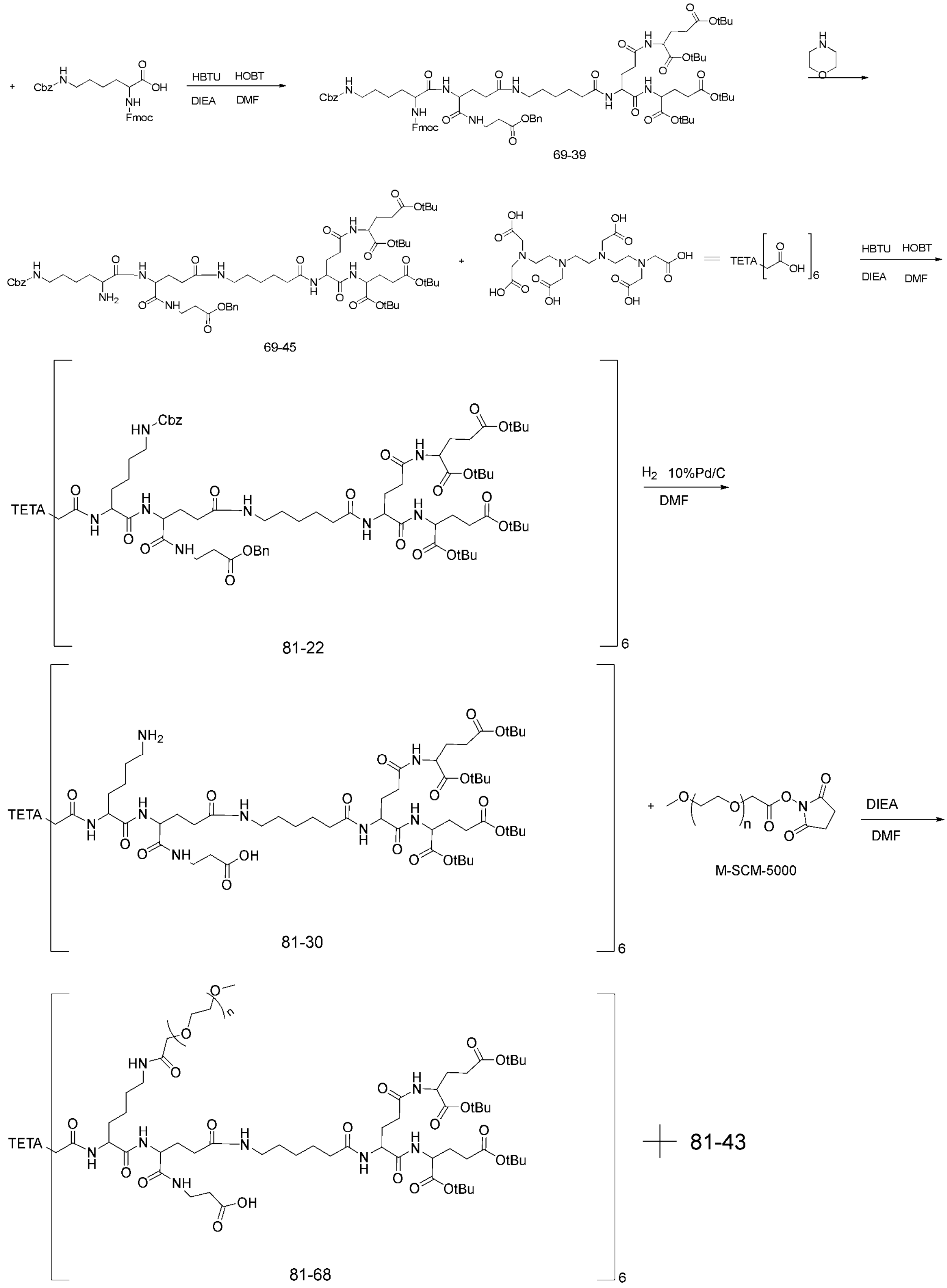


69-39

69-45

81-22

81-30

M-SCM-5000

81-68

+ 81-43

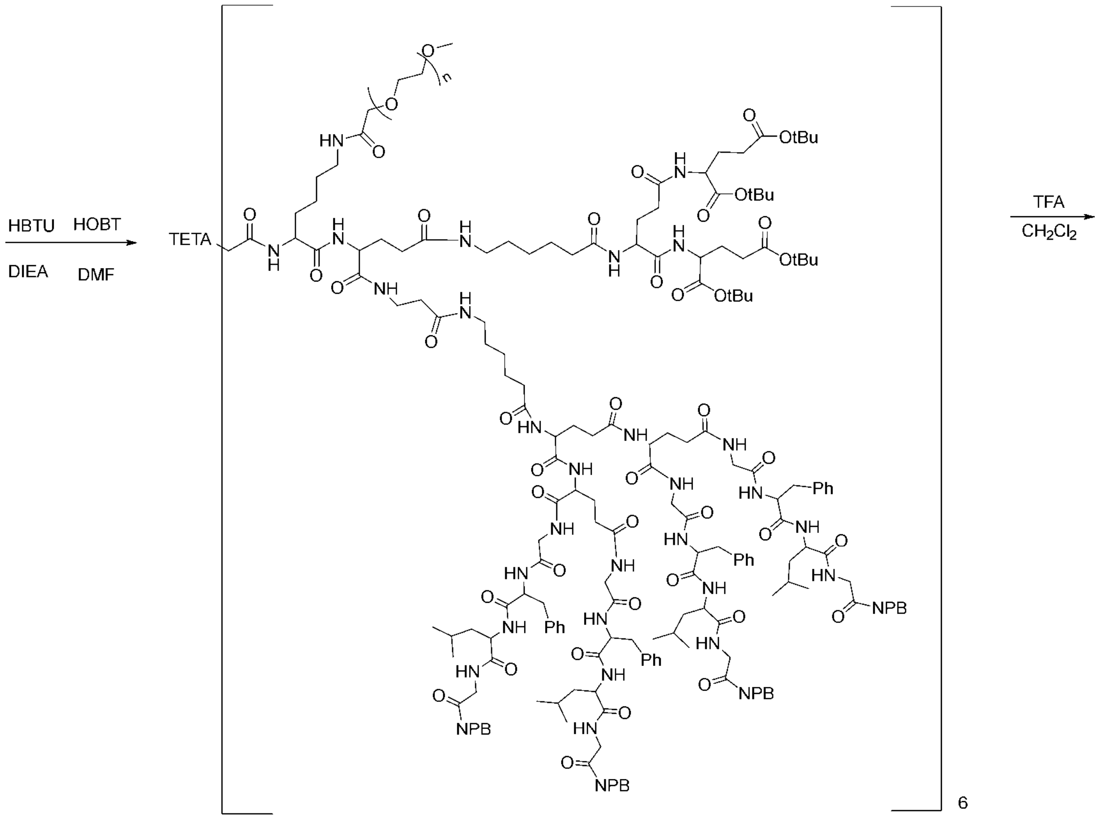


81-48

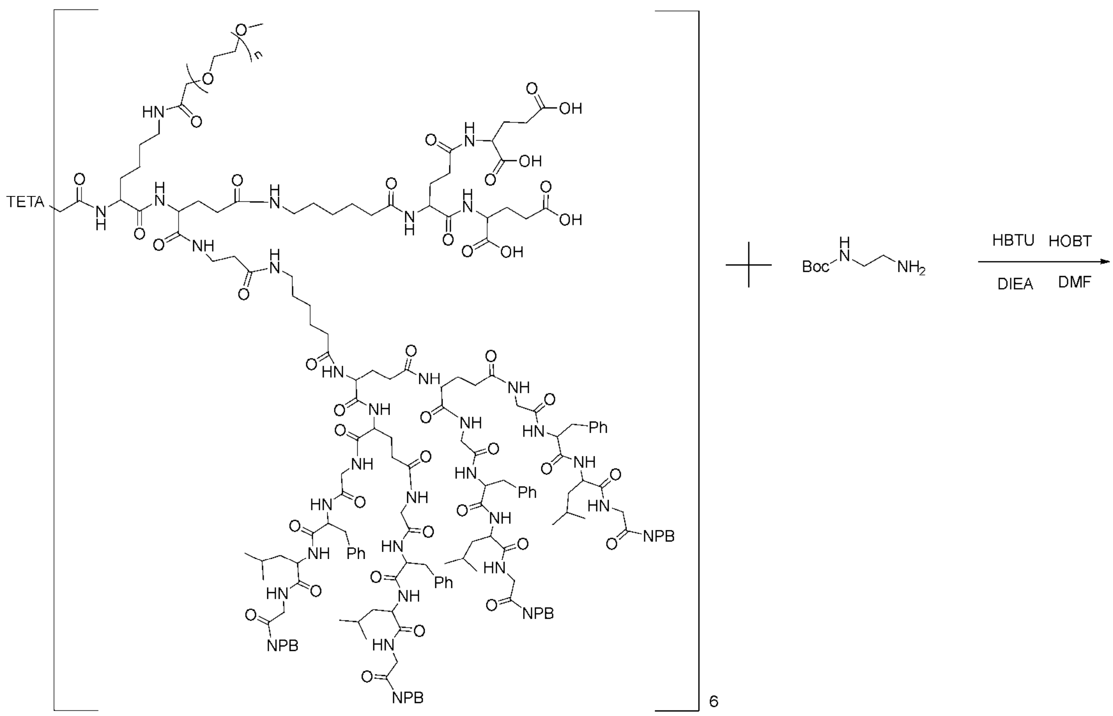


81-106

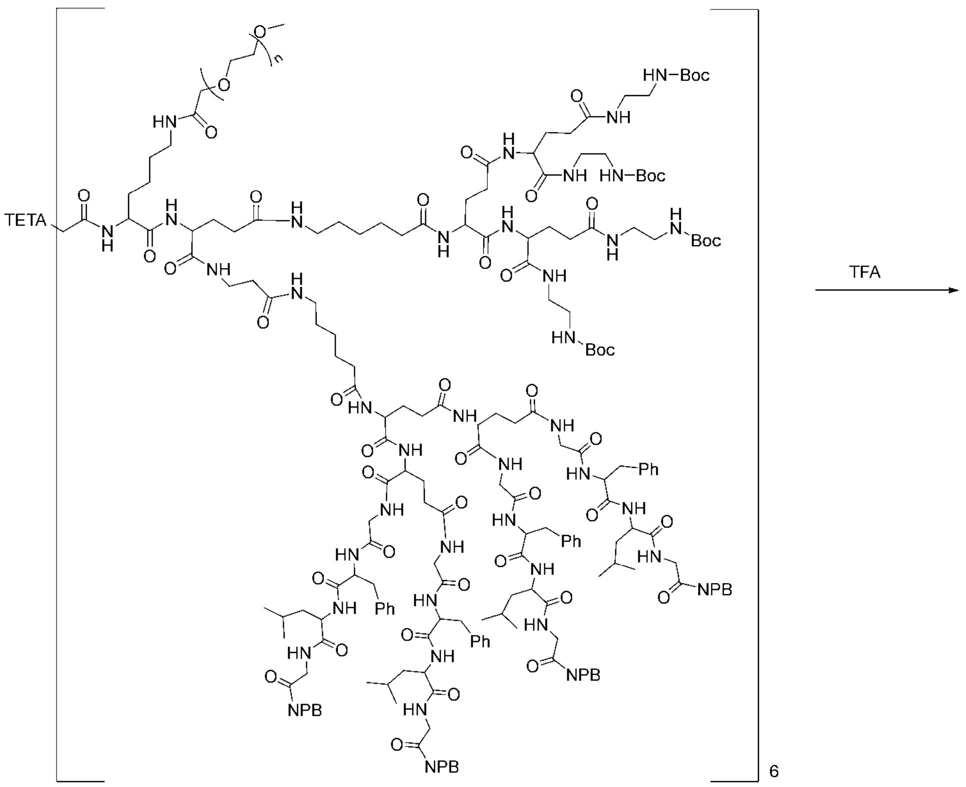


81-110

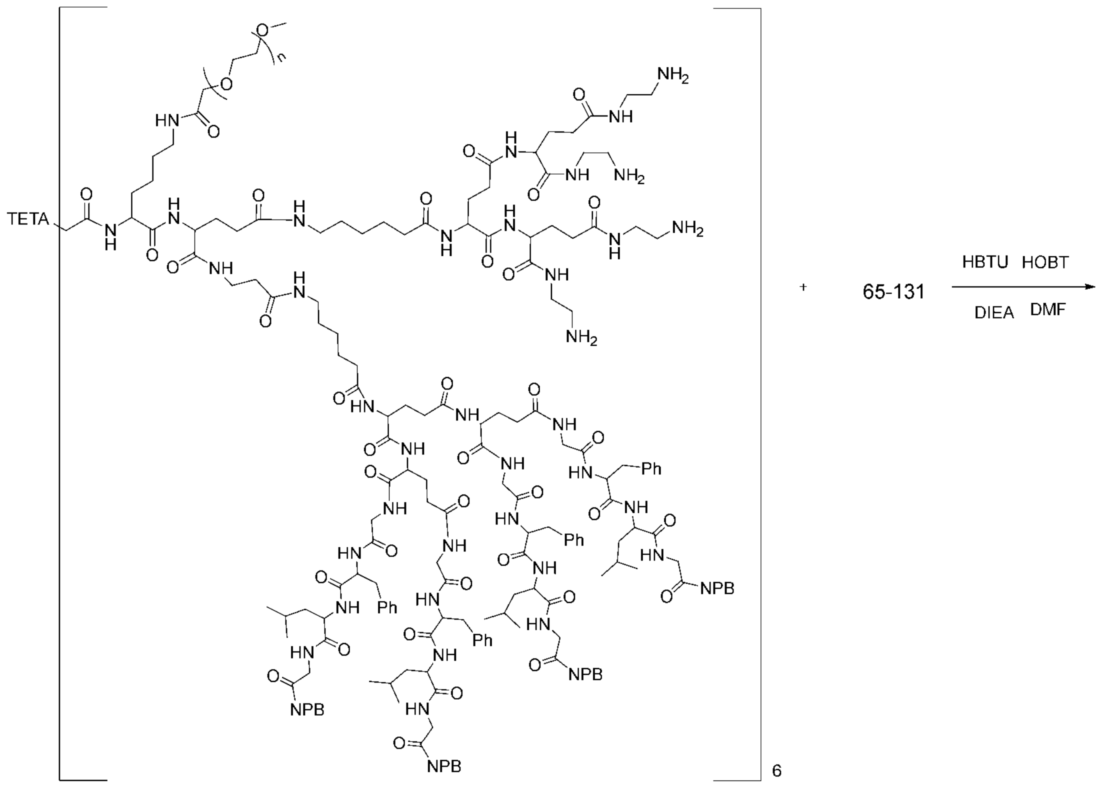


81-113

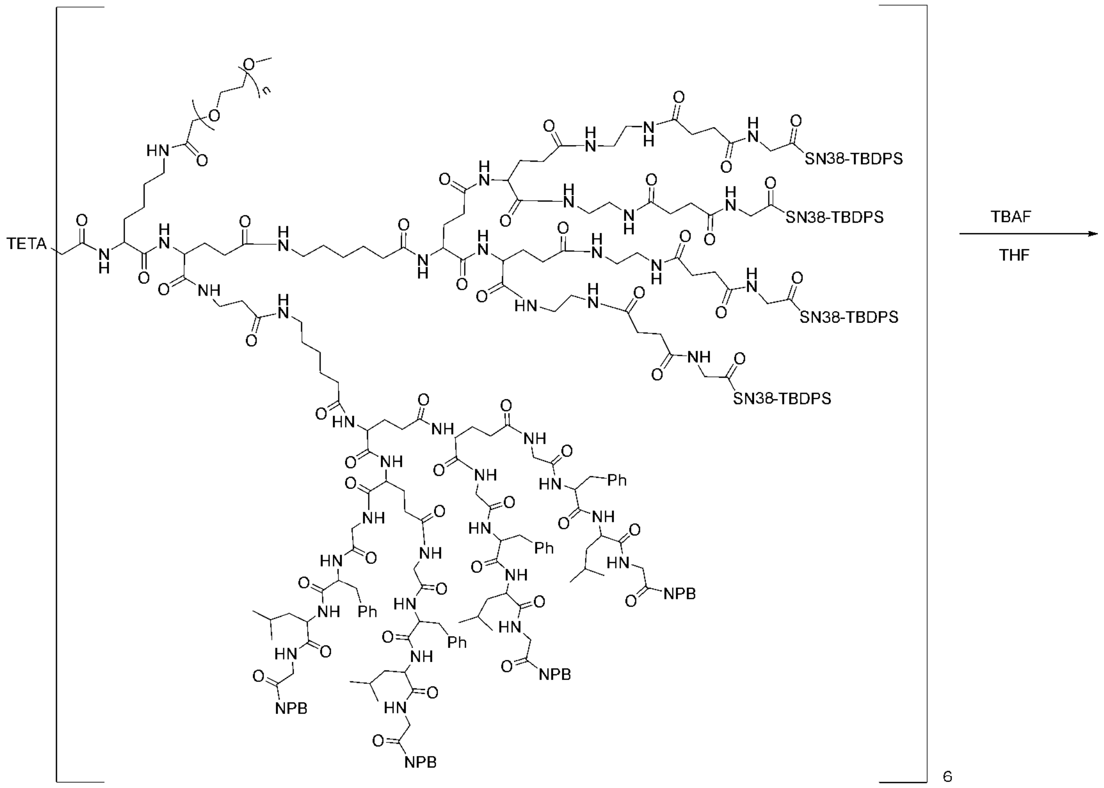


81-116

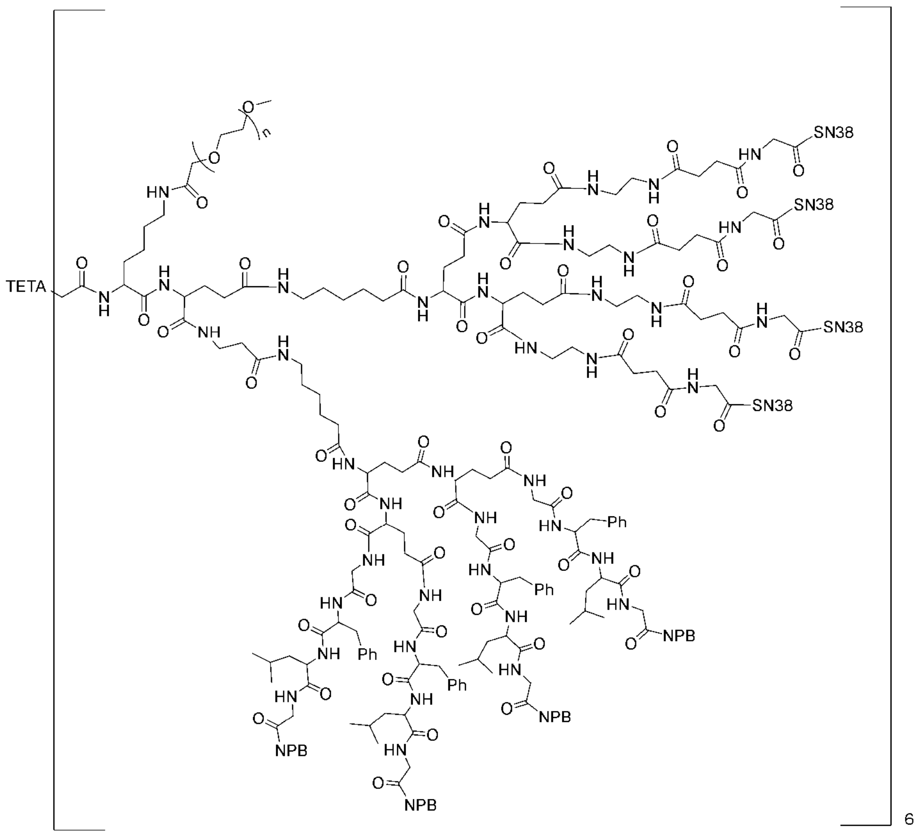

81-118

4.1 Preparation of **62-13**

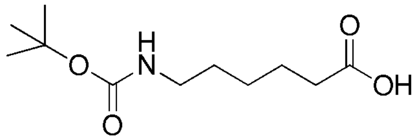

Hexaaminocaproic acid (15 g, 114.3554 mmol), 1,4-dioxane (200 mL), and pure water (100 mL) were added to a 1 L round-bottom flask. Dissolution was performed with the assistance of ultrasonication. After the mixture was stirred at -5°C for 30 minutes, an aqueous sodium hydroxide solution (5 g NaOH/20 mL pure water) was added, and the mixture was stirred at -5°C for 30 minutes. Then, di-tert-butyl dicarbonate (34.9 g, 160.09 mmol) was added. The reaction mixture was then brought to room temperature and stirred overnight. After the reaction was completed, the reaction mixture was transferred to a 2 L separatory funnel, pure water (300 mL) and ethyl acetate (350 mL) were added for extraction, and 36% dilute hydrochloric acid was added dropwise to adjust the pH to acidic. The organic phase was collected, and the aqueous phase was further extracted with ethyl acetate (200 mL × 3). The organic phases were combined, concentrated and evaporated to dryness to provide 24 g of the product.

4.2 Preparation of **65-104**

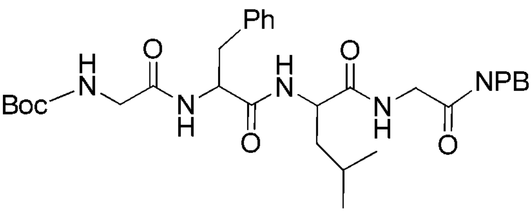

**71-85** (8.45 g, 17.16 mmol), Niraparib (3.93 g, 12.25 mmol, purchased from PharmaBlock, NPB), HBTU (6.97 g, 18.38 mmol), and HOBT (2.5 g, 18.38 mmol) were added to a 500 mL round-bottom flask, and then DMF (50 mL) was added for dissolution with the assistance of ultrasonication. The mixture was stirred at -5°C for 3 minutes, and then DIEA (8.1 mL, 49.03 mmol) was slowly added dropwise to continue the reaction. After the dropwise addition was completed, stirring was continued at -5°C for 1 hour, then the mixture was brought to room temperature, and the reaction was carried out with stirring overnight. After the reaction was completed, the reaction mixture was transferred to a 2 L separatory funnel, and purified water (300 mL) and ethyl acetate (350 mL) were added for extraction. The organic phase was collected, and the aqueous phase was further extracted with ethyl acetate (200 mL × 3). The organic phases were combined, washed with saturated brine (250 mL × 2), collected, concentrated under reduced pressure. Then 200-300 mesh silica gel powder was added, and the mixture was evaporated to dryness. The resulting product was dry-loaded onto the column and subjected to column chromatography using 2% methanol/dichloromethane as the eluent. The desired product was collected, concentrated, and evaporated to dryness to provide 10.51 g of the product.

4.3 Preparation of **62-174**

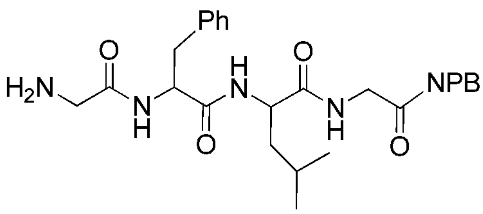

**65-104** (2.5 g, 3.14 mmol) was added to a 500 mL round-bottom flask containing dichloromethane (5 mL), and dissolved with the assistance of ultrasonication. TFA (5.37 mL, 47.17 mmol) was then added, and reacted with stirring at

room temperature. After the reaction was completed, dichloromethane and most of the TFA were removed by rotary evaporation. Methyl tert-butyl ether (200 mL) was then added for precipitation. A solid precipitated and was filtered to provide 2.08 g of the product.

4.4 Preparation of **62-1**

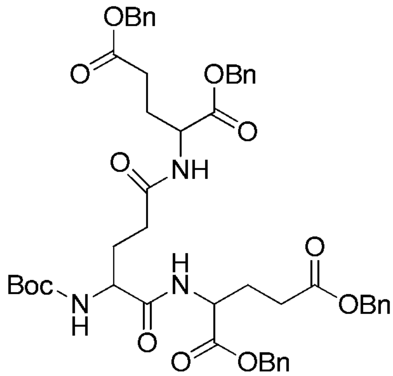

Boc-Glu-OH (3 g, 12.1 mmol), L-glutamic acid dibenzyl ester p-toluenesulfonate (13.29 g, 26.62 mmol), HBTU (13.76 g, 36.3 mmol), and HOBT (4.79 g, 36.3 mmol) were added to a 500 mL round-bottom flask, then DMF (100 mL) was added to dissolve the mixture with the assistance of ultrasonication. The flask was placed in an ice-water bath, and DIEA (17.99 mL, 108.9 mmol) was added dropwise. After the dropwise addition was completed, the flask was then taken out, and the reaction was carried out with stirring at room temperature. After the reaction was completed, the reaction mixture was transferred to a 2 L separatory funnel containing pure water (600 mL) and ethyl acetate (600 mL) for extraction. The organic phase was collected, and the aqueous phase was further extracted with ethyl acetate (200 mL × 3). The organic phases were combined, washed with saturated sodium bicarbonate solution (30 mL × 2). The organic phase was collected and concentrated under reduced pressure, then 200-300 mesh silica gel powder (25 g) was added, and the mixture was evaporated to dryness. The resulting product was dry-loaded onto the column and subjected to column chromatography using 1% methanol/dichloromethane as the eluent. The desired product was collected, concentrated, and evaporated to dryness to provide 10.47 g of the product.

4.5 Preparation of **62-2**

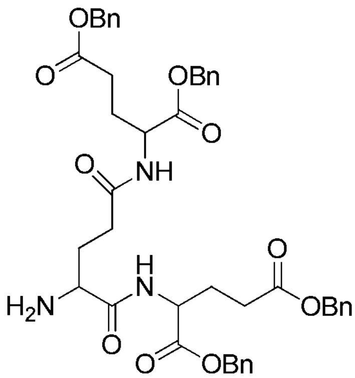

**62-1** (10.47 g, 12.1 mmol) was placed in a 500 mL round-bottom flask, then dichloromethane (15 mL) was added for dissolution with the assistance of ultrasonication. TFA (13.47 mL, 181.5 mmol) was added, and the mixture was reacted with stirring at room temperature. After the reaction was completed, the dichloromethane and most of the TFA were removed by rotary evaporation. Methyl tert-butyl ether (200 mL) was then added for precipitation. A solid precipitated was filtered and dried to provide 9.2 g of the product.

4.6 Preparation of **61-213**

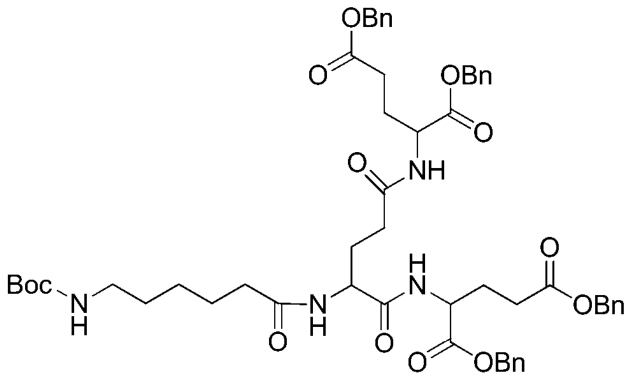

**62-13** (2.716 g, 11.75 mmol) and **62-2** (9 g, 11.75 mmol) were added to a 500 mL round-bottom flask, then DMF (50 mL) was added for dissolution with the assistance of ultrasonication. After the mixture was stirred at room temperature for 1 minutes, **68-54** (3.57 g, 12.925 mmol) and N-methylmorpholine (3.8 mL, 35.25 mmol) were added, and the reaction was continued with stirring at room temperature. After the reaction was completed, the reaction mixture was transferred to a 2 L separatory funnel containing purified water (300 mL) and ethyl acetate (350 mL) for extraction. The organic phase was collected, and the aqueous phase was further extracted with ethyl acetate (200 mL × 3). The organic phases were combined, washed with saturated sodium bicarbonate solution (250 mL × 2). The organic phases were collected and concentrated under reduced pressure, then 200-300 mesh silica gel powder (25 g) was added, and the mixture was evaporated to dryness. The resulting product was dry-loaded onto the column and subjected to column chromatography using 2.5% methanol/dichloromethane as the eluent. The desired product was collected, concentrated, and evaporated to dryness to provide 10.38 g of the product.

4.7 Preparation of **62-156**

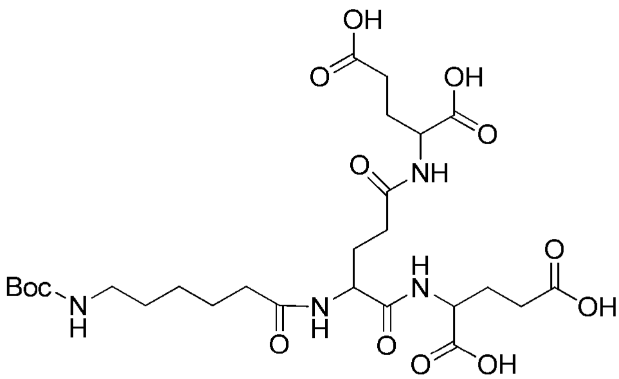

**61-213** (0.77 g, 0.786 mmol) and 10% Pd/C catalyst (0.12 g) were added to a hydrogenation reactor, and then DMF (15 mL) was added. The reactor was sealed, evacuated with a water pump, and then hydrogen was introduced. This process was repeated three times. The hydrogen pressure was then adjusted to 300 psi, and the mixture was stirred at room temperature overnight. After the reaction was completed, the reaction mixture was filtered through Celite, and the Celite was rinsed with DMF (3 mL × 3). The filtrates were collected and used as the raw material for the next reaction.

4.8 Preparation of **62-180**

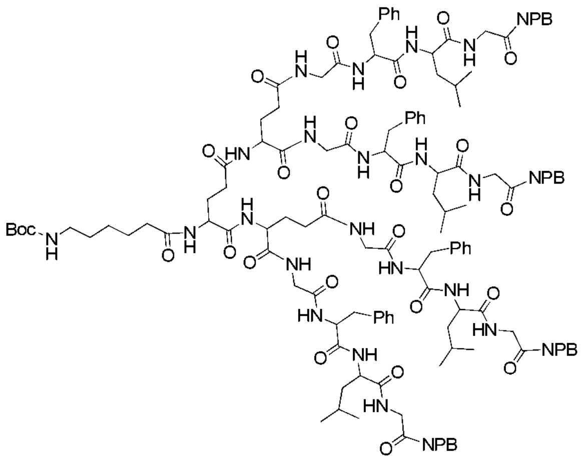

**62-174** (1.2 g, 1.72 mmol) and **62-156** (0.236 g, 0.38 mmol) were added to a 250 mL round-bottom flask, then DMF (10 mL) was added for dissolution with the assistance of ultrasonication. After the mixture was stirred at 0°C for 2 minutes, **68-54** (0.47 g, 1.72 mmol) and N-methylmorpholine (0.38 mL, 3.45 mmol) were added, and the reaction was continued with stirring at room temperature. After the reaction was completed, n-hexane (50 mL) and methyl tert-butyl ether (450 mL) were added for precipitation. A solid precipitated and was filtered. The filter cake was transferred to a 2 L round-bottom flask, then a methanol/dichloromethane (1/1) mixture (100 mL) was added for dissolution with the assistance of ultrasonication. Then, n-hexane (50 mL) and methyl tert-butyl ether (900 mL) were added for precipitation. The mixture was then filtered. This process, i.e., dissolution with mixture/precipitation with methyl tert-butyl ether, was repeated three times. After the final filtration, the resultant product was transferred to a 250 mL round-bottom flask, then ethyl acetate (50 mL) was added, and the mixture was subjected to treatment with ultrasonication for 10 minutes and filtration. This process, i.e., addition of ethyl acetate/treatment with ultrasonication/filtration, was repeated three times. After the final filtration, the filter cake was dried to provide 1.18 g of product.

4.9 Preparation of **81-43**

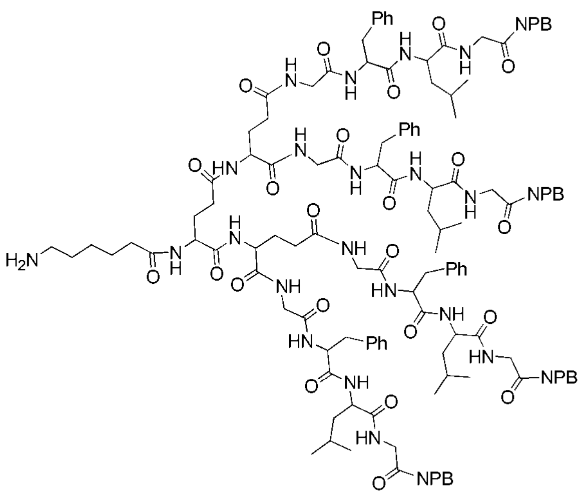

**62-180** (1.7 g, 0.51 mmol) was added to a 250 mL round-bottom flask, then dichloromethane (10 mL) was added for dissolution with the assistance of ultrasonication. Then TFA (5.7 mL, 76.6 mmol) was added, and the mixture was reacted with stirring at room temperature. After the reaction was completed, the reaction mixture was first concentrated under reduced pressure to remove dichloromethane and most of the TFA, and then n-hexane (50 mL) and methyl tert-butyl ether (200 mL) were added for precipitation, resulting in an oily solid. The supernatant was discarded, and methyl tert-butyl ether (200 mL) was added again for precipitation. This process was repeated three times. A powdery solid precipitated and was filtered. The filter cake was collected and then dissolved in a methanol/dichloromethane (1/4) mixture. The resulting product was dry-loaded onto the column and subjected to column chromatography using 1% ammonia water/8% methanol/dichloromethane as the eluent. The desired product was collected, concentrated, and evaporated to dryness

to provide 1.2 g of the product. $^1$H-NMR (600 MHz, DMSO-$d_6$) δ 9.34-9.23 (m, 4H), 8.63-8.51 (m, 4H), 8.28-8.15 (m, 6H), 8.14-7.92 (m, 24H), 7.93-7.79 (m, 7H), 7.64-7.45 (m, 11H), 7.31-7.06 (m, 23H), 4.63-4.51 (m, 4H), 4.49-4.31 (m, 8H), 4.26-4.12 (m,3H), 4.10-3.88 (m, 8H), 3.88-3.77 (m, 4H), 3.76-3.55 (m, 7H), 3.19-3.10 (m, 2H), 3.10-2.97 (m, 5H), 2.87-2.57 (m, 18H), 2.22-2.02 (m, 8H), 1.99-1.66 (m, 20H), 1.65-1.33 (m, 20H), 0.94-0.65 (m, 24H)

4.10 Preparation of **62-150**

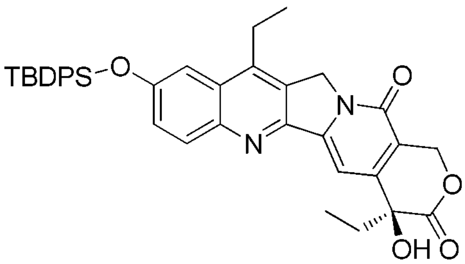

7-Ethyl-10-hydroxycamptothecin (2 g, 5.1 mmol, referred to as SN38) was added to a 500 mL round-bottom flask, then dichloromethane (100 mL) was added for dissolution with the assistance of ultrasonication. TBDPS-Cl (5.2 mL, 20.39 mmol) and triethylamine (3.2 mL, 22.93 mmol) were added, and the mixture was reacted with stirring in an oil bath at 30°C overnight. After the reaction was completed, the reaction mixture was transferred to a 1 L separatory funnel, and washed sequentially with 0.2N HCl solution (150 mL × 2), saturated sodium bicarbonate solution (300 mL), and saturated brine (300 mL). The resulting dichloromethane organic phase was dried over anhydrous magnesium sulfate and concentrated to dryness to provide 3 g of the product.

4.11 Preparation of **62-153**

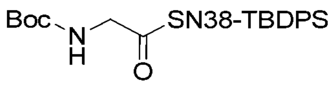

**62-150** (3 g, 4.75 mmol) and Boc-Gly-OH (1.249 g, 7.13 mmol) were added to a 500 mL round-bottom flask, then dichloromethane (100 mL) was added for dissolution with the assistance of ultrasonication. The mixture was stirred at 0°C for 5 minutes. EDCI (1.36 g, 7.13 mmol) and DMAP (0.26 g, 2.13 mmol) were then added, and the reaction was continued with stirring at 0°C. After the reaction was completed, the reaction mixture was transferred to a 1 L separatory funnel, and washed sequentially with 0.5% sodium bicarbonate solution (150 mL), purified water (150 mL), 0.1N HCl solution (150 mL), and saturated brine (150 mL). The resulting dichloromethane organic phase was dried over anhydrous magnesium sulfate, then 200-300 mesh silica gel powder (30 g) was added, and the mixture was evaporated to dryness. The resulting product was dry-loaded onto the column and subjected to column chromatography using 1.5% methanol/dichloromethane as the eluent. The desired product was collected and concentrated to dryness to provide 2.9 g of the product.

4.12 Preparation of **62-155**

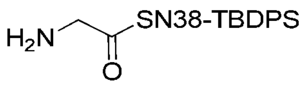

**62-153** (2.9 g, 3.68 mmol) was added to a 500 mL round-bottom flask. 1,4-dioxane (10 mL) was added for dissolution with the assistance of ultrasonication, then 4M HCl-dioxane (10 mL) was added, and the mixture was reacted with stirring at room temperature for 5 hours. After the reaction was completed, HCl-dioxane was removed by rotary evaporation under reduced pressure, and methyl tert-butyl ether (300 mL) was added for precipitation. A solid precipitated and was collected by suction filtration. The filter cake was dried to provide 2.08 g of the product. $^1$H-NMR (600 MHz, DMSO-$d_6$) δ 8.67-8.64 (m, 1H), 8.06 (d, $J$ = 8.9 Hz, 1H), 7.80-7.64 (m, 4H), 7.56-7.42 (m, 6H), 7.26 (s, 1H), 7.14 (s, 1H),

5.59-5.44 (m, 2H), 5.30-5.13 (m, 2H), 4.48-4.40 (m, 2H), 4.33-4.22 (m, 1H), 4.08-3.94 (m, 1H), 2.72-2.56 (m, 2H), 2.22-2.06 (m, 2H), 1.11 (s, 9H), 0.93 (t, *J* = 7.0 Hz, 3H), 0.83 (t, *J* = 7.2 Hz, 3H)

4.13 Preparation of **65-131**

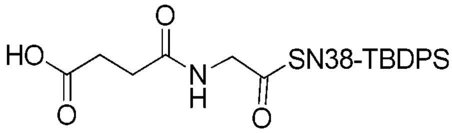

**62-155** (0.5 g, 0.72 mmol) was placed in a 500 mL round-bottom flask, then THF (15 mL) was added for dissolution with the assistance of ultrasonication. The mixture was stirred at 25°C for 5 minutes. Succinic anhydride (0.18 g, 1.81 mmol) was then added, followed by the slow dropwise addition of triethylamine (0.25 mL, 1.74 mmol), and reacted with stirring at room temperature for 12 hours. After the reaction was completed, methyl tert-butyl ether (350 mL) was added for precipitation. A solid precipitated and was filtered. The filter cake was dissolved in dichloromethane (200 mL), then 200-300 mesh silica gel powder (25 g) was added, and the mixture was evaporated to dryness. The resulting product was dry-loaded onto the column and subjected to column chromatography using 0.5% formic acid/3-6% methanol/dichloromethane as the eluent. The desired product was collected, concentrated, and evaporated to dryness to provide 0.1 g of the product. $^1$H-NMR (600 MHz, DMSO-$d_6$) δ 12.03 (s, 1H), 8.44-8.37 (m, 1H), 8.06 (d, *J* = 8.9 Hz, 1H), 7.79-7.71 (m, 4H), 7.61-7.55 (m, 1H), 7.56-7.44 (m, 6H), 7.12 (s, 1H), 7.03 (s, 1H), 5.47-5.44 (m, 2H), 5.24-5.14 (m, 2H), 4.17-4.10 (m, 1H), 4.02-3.95 (m, 1H), 2.66-2.58 (m, 2H), 2.45-2.4 (m, 2H), 2.39-2.31 (m, 2H), 2.17-2.09 (m, 2H), 1.11 (s, 9H), 0.93 (t, *J* = 7.0 Hz, 3H), 0.83 (t, *J* = 7.2 Hz, 3H)

4.14 Preparation of **69-33**

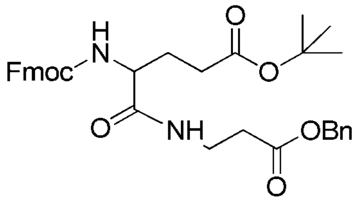

Fmoc-Glu(OtBu)-OH (10 g, 23.50 mmol) and β-alanine benzyl ester p-toluenesulfonate (7.86 g, 22.38 mmol) were added to a 500 mL round-bottom flask, and then DMF (50 mL) was added for dissolution with the assistance of ultrasonication. The mixture was stirred at -5°C for 3 minutes, followed by the addition of **68-54** (6.813 g, 24.62 mmol) and N-methylmorpholine (3.71 mL, 33.57 mmol), and the reaction was stirred at room temperature. After the reaction was completed, the reaction mixture was transferred to a 2 L separatory funnel containing purified water (300 mL) and ethyl acetate (350 mL) for extraction. The organic phase was collected, and the aqueous phase was further extracted with ethyl acetate (200 mL × 3). The organic phases were combined, and washed with saturated sodium bicarbonate solution (250 mL × 2). The organic phase was collected, then 200-300 mesh silica gel powder (50 g) was added, and the mixture was evaporated to dryness. The resulting product was dry-loaded onto the column and subjected to column chromatography using 0.5% methanol/dichloromethane as the eluent. The desired product was collected, concentrated, and evaporated to dryness to provide 11.64 g of the product.

4.15 Preparation of **69-34**

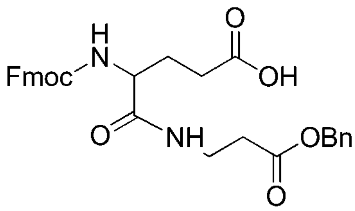

**69-33** (11.64 g, 19.84 mmol) was added to a 500 mL round-bottom flask, then dichloromethane (20 mL) was added for dissolution with the assistance of ultrasonication. Then TFA (39.58 mL, 347.13 mmol) was added, and the mixture was reacted with stirring at room temperature. After the reaction was completed, TFA was removed by rotary evaporation. The reaction mixture was transferred with ethyl acetate to a 2 L separatory funnel containing purified water (300 mL) and ethyl acetate (350 mL). The organic phase was collected, and the aqueous phase was further washed with ethyl acetate (200 mL × 3). The organic phases were combined, then washed with saturated brine (250 mL × 2), and the organic phase was collected, concentrated, and evaporated to dryness to provide 8.95 g of the product.

4.16 Preparation of **69-36**

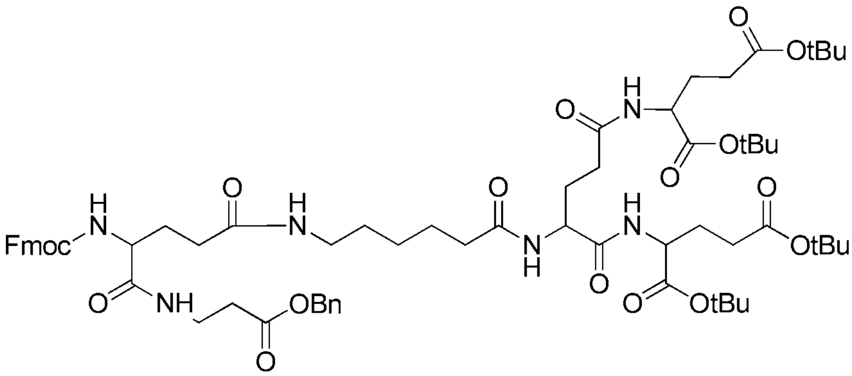

**69-34** (7.05 g, 13.31 mmol) and **38-224** (9.89 g, 13.375 mmol) were added to a 500 mL round-bottom flask, then DMF (15 mL) was added for dissolution with the assistance of ultrasonication, and the mixture was stirred at room temperature for 2 minutes. **68-54** (4.05 g, 14.64 mmol) and N-methylmorpholine (2.94 mL, 26.63 mmol) were then added, and the reaction was continued with stirring at room temperature. After the reaction was completed, methyl tert-butyl ether (420 mL) was added for precipitation. A solid precipitated and was filtered. The filter cake was dissolved in a methanol/dichloromethane (1/1) mixture (300 mL), then 200-300 mesh silica gel powder (30 g) was added, and the mixture was evaporated to dryness. The resulting product was dry-loaded onto the column and subjected to column chromatography using 5% methanol/dichloromethane as the eluent. The desired product was collected, concentrated, and evaporated to dryness to provide 14.97 g of the product.

4.17 Preparation of **69-37**

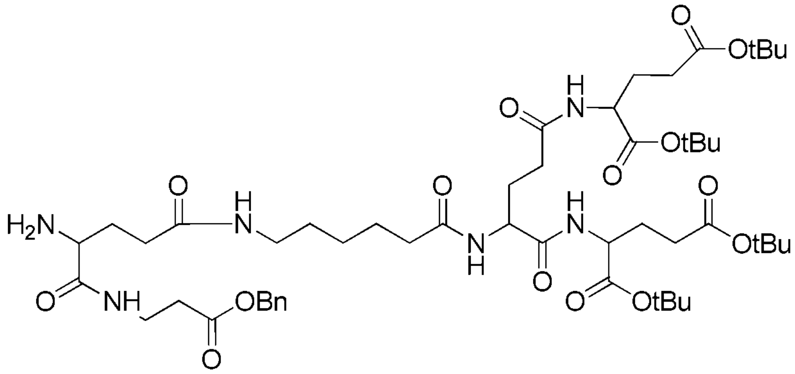

**69-36** (10 g, 7.965 mmol) was added to a 500 mL round-bottom flask. DMF (10 mL) was added for dissolution with the assistance of ultrasonication, then morpholine (15 mL) was added, and the mixture was reacted with stirring at room temperature. After the reaction was completed, methyl tert-butyl ether (400 mL) was added for precipitation. A solid precipitated and was filtered. The filter cake was washed with petroleum ether (200 mL), and dried to provide 8.27 g of the product.

4.18 Preparation of **69-39**

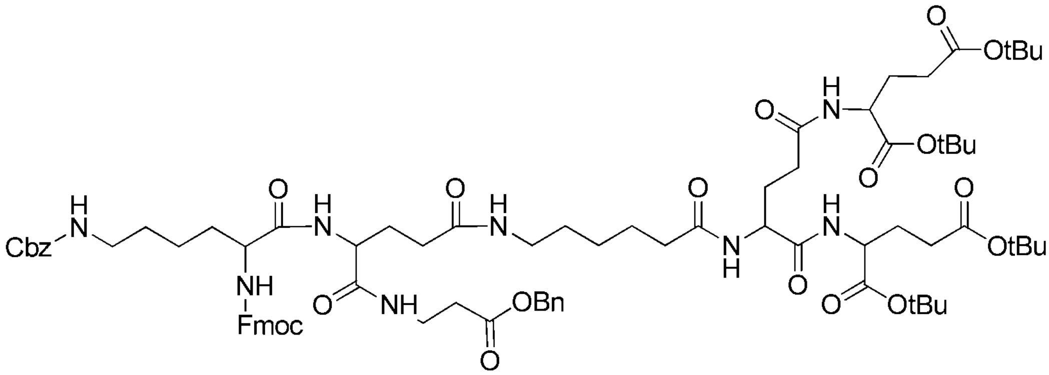

**69-37** (8.22 g, 7.965 mmol), N'-Fmoc-N-benzyloxycarbonyl-L-lysine (4.20 g, 8.36 mmol), HBTU (3.32 g, 8.76 mmol), and HOBT (1.18 g, 8.76 mmol) were placed in a 500 mL round-bottom flask, then DMF (20 mL) was added to dissolve the mixture with the assistance of ultrasonication. After stirring at 0°C for 3 minutes, DIEA (1.97 mL, 11.94 mmol) was added, and the reaction was carried out with stirring at room temperature. After the reaction was completed, methyl tert-butyl ether (400 mL) was added for precipitation. A solid precipitated and was filtered. The filter cake was dissolved in a methanol/dichloromethane (1/1) mixture (300 mL), then 200-300 mesh silica gel powder (35 g) was added, and the mixture was evaporated to dryness. The resulting product was dry-loaded onto the column and subjected to column chromatography using 8% methanol/dichloromethane as the eluent. The desired product was collected, concentrated, and evaporated to dryness to provide 10.85 g of the product. $^1$H-NMR (600 MHz, DMSO-$d_6$) δ 8.16 (d, *J* = 7.5 Hz, 1H), 8.08 (d, *J* = 7.6 Hz, 1H), 8.01-7.94 (m, 2H), 7.93-7.85 (m, 3H), 7.75-7.66 (m, 3H), 7.52-7.45 (m, 1H), 7.43-7.27 (m, 14H), 7.23 (t, *J* = 5.7 Hz, 1H), 5.15-4.94 (m, 4H), 4.34-4.06 (m, 7H), 3.97 (td, *J* = 4.5, 8.5 Hz, 1H), 3.39-3.22 (m, 2H), 3.03-2.91 (m, 4H), 2.53-2.47 (m, 2H), 2.31-2.00 (m, 10H), 1.95-1.57 (m, 9H), 1.57-1.13 (m, 47H)

4.19 Preparation of **69-45**

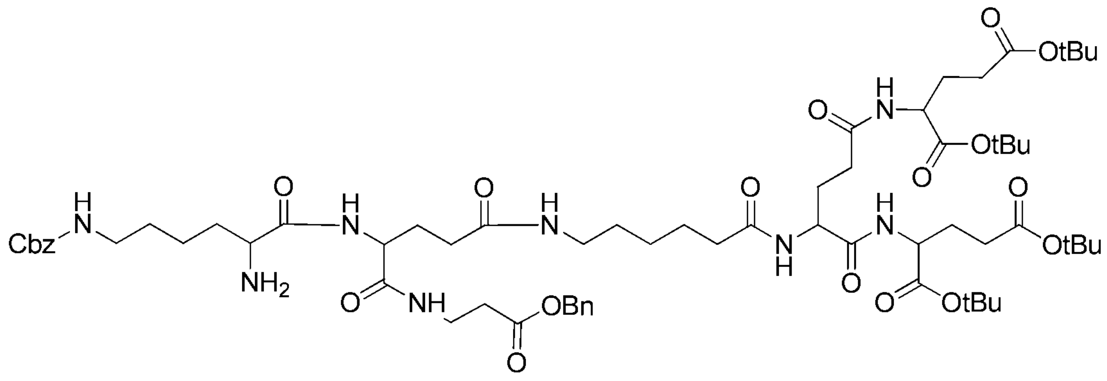

**69-39** (3.0 g, 1.926 mmol) was placed in a 500 mL round-bottom flask. DMF (20 mL) was added for dissolution with the assistance of ultrasonication, then morpholine (8.44 mL, 96.8 mmol) was added, and the mixture was reacted with stirring at room temperature. After the reaction was completed, methyl tert-butyl ether (400 mL) was added for precipitation. A solid precipitated and was filtered. The filter cake was dissolved in a methanol/dichloromethane (1/1) mixture (300 mL), then 200-300 mesh silica gel powder (30 g) was added, and the mixture was evaporated to dryness. The resulting product was dry-loaded onto the column and subjected to column chromatography using 1% ammonia water/5% methanol/dichloromethane as the eluent. The desired product was collected, concentrated, and evaporated to dryness to provide 2.3 g of the product. $^1$H-NMR (600 MHz, DMSO-$d_6$) δ 8.16 (d, $J$ = 7.5 Hz, 1H), 8.08 (d, $J$ = 7.6 Hz, 1H), 8.01-7.94 (m, 2H), 7.93-7.85 (m, 1H), 7.75-7.66 (m, 1H),7.52-7.45 (m, 1H),7.43-7.27 (m, 9H), 7.23 (t, $J$ = 5.7 Hz, 1H), 5.15-4.94 (m, 4H), 4.34-4.06 (m, 4H), 3.97 (td, $J$ = 4.5, 8.5 Hz, 1H), 3.39-3.22 (m, 2H), 3.03-2.91 (m, 4H), 2.53-2.47 (m, 4H), 2.31-2.00 (m, 10H), 1.95-1.57 (m, 9H), 1.57-1.13 (m, 47H)

4.20 Preparation of **81-22**

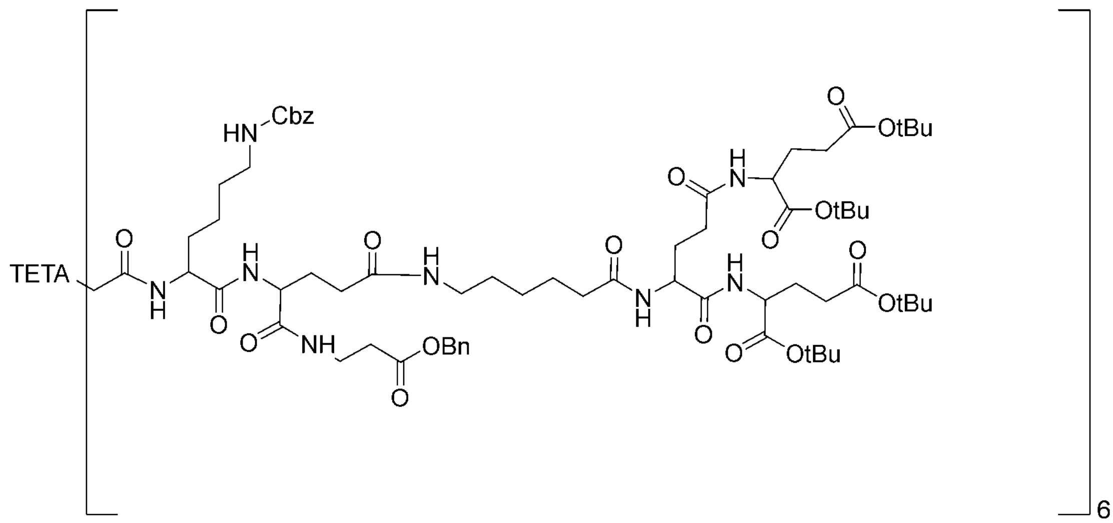

**69-45** (1.5 g, 1.157 mmol), triethylenetetraamine hexaacetic acid (0.063 g, 0.1256 mmol, purchased from TCI), HBTU (0.438 g, 1.157 mmol), and HOBT (0.156 g, 1.157 mmol) were added to a 100 mL round-bottom flask, then DMF (15 mL) was added to dissolve the mixture with the assistance of ultrasonication. After stirring at 0°C for 5 minutes, DIEA (1.13 mL, 6.864 mmol) was added, and the reaction was carried out with stirring at room temperature. After the reaction was completed, ethyl acetate (300 mL) and purified water (300 mL) were added for extraction. The organic phase was collected, and the aqueous phase was further extracted with ethyl acetate (200 mL). The organic phases were combined, concentrated, then a methanol/dichloromethane (1/4) mixture (50 mL) was added for dissolution with the assistance of ultrasonication. Then 200-300 mesh silica gel powder (10 g) was added, and the mixture was evaporated to dryness. The resulting product was dry-loaded onto the column and subjected to column chromatography using 4% methanol/dichloromethane as the eluent. The desired product was collected, concentrated, and evaporated to dryness to provide 0.49 g of the product with a yield of 74%.

4.21 Preparation of **81-30**

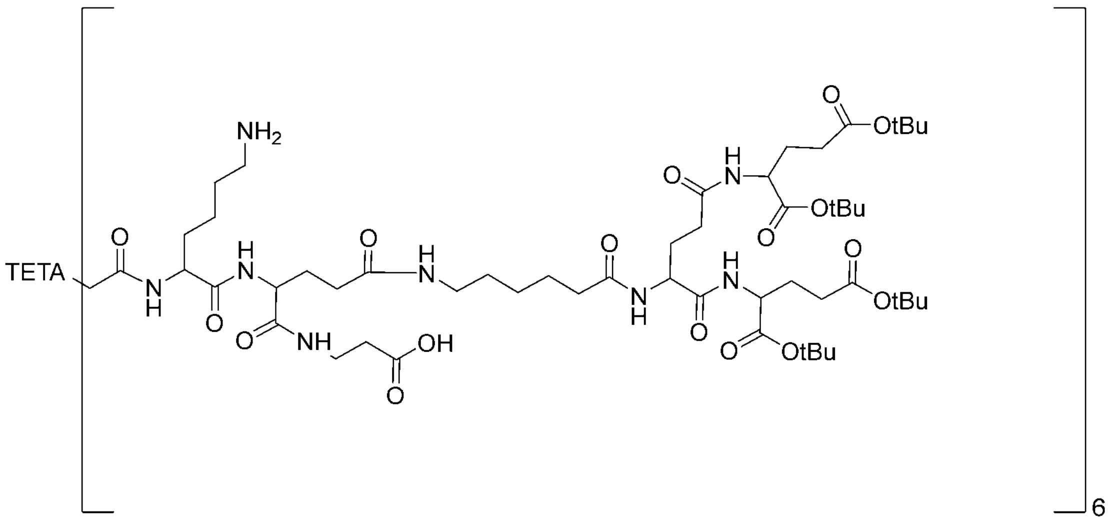

**81-22** (1 g, 0.122 mmol) and 10% Pd/C catalyst (250 mg) were added to a hydrogenation reactor, and DMF (10 mL) and acetic acid (0.5 mL) were added. The hydrogenation reactor was sealed, evacuated with a water pump, and then filled with hydrogen gas. This process was repeated three times. The hydrogen pressure was adjusted to 2 MPa. The reaction was then carried out at 35°C for 72 hours. After the reaction was completed, the mixture was subjected to suction filtration using a Buchner funnel containing filter paper, and the filtrate was collected. Methyl tert-butyl ether (100 mL) and n-hexane (100 mL) were added, shaken, allowed to stand, and the supernatant was discarded. This process was repeated several times until the product became a viscous oil, which was dried to provide 0.57 g of the product. $^1$H-NMR (600 MHz, DMSO-$d_6$) δ 8.54-8.45 (m, 3H), 8.23-7.90 (m, 33H), 7.90-7.68 (m, 6H), 4.27-4.23 (m, 6H), 4.20-4.17 (m, 5H), 4.13-4.07 (m, 14H), 4.05-3.99 (m, 6H), 3.89-3.43 (m, 8H), 3.31-3.23 (m, 5H), 3.23-3.15 (m, 6H), 3.11-3.03 (m, 12H), 3.03-2.92 (m, 20H), 2.87-2.75 (m, 20H), 2.51-2.44 (m, 4H), 2.29-1.98 (m, 74H), 1.93-1.65 (m, 57H), 1.51-1.13 (m, 279H)

4.22 Preparation of **81-68**

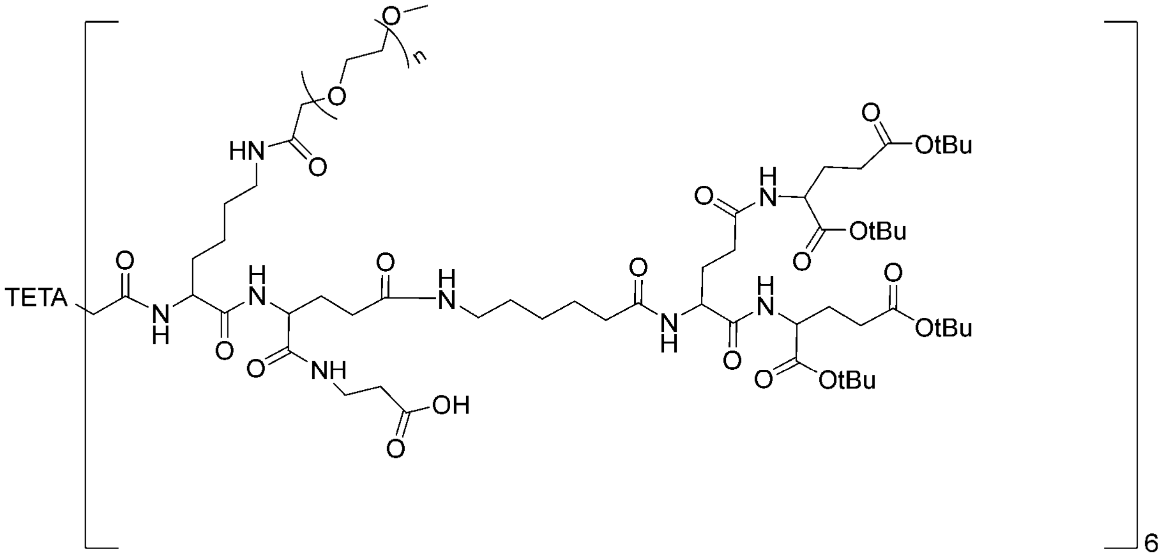

**81-30** (0.59 g, 0.0865 mmol) was placed in a round-bottom flask, and then extra-dry DMF (15 mL) was added for dissolution with the assistance of ultrasonication. The mixture was stirred at -5°C for 5 minutes. DIEA (0.85 mL, 5.19 mmol)

was then slowly added dropwise. The reaction was carried out with stirring for 2 minutes. Then, the flask was taken out, and M-SCM-5000 (2.87 g, 0.545 mmol, purchased from Jenkem, Lot Number: ZZ403P152) was added, and reacted with stirring at low speed at 35°C in the dark for 48 hours. After the reaction was completed, methyl tert-butyl ether (500 mL) was added for precipitation. A solid precipitated and was filtered. The filter cake was dissolved in a methanol/dichloromethane (1/4) mixture (30 mL) under the assistance of ultrasonication, then 100-200 mesh silica gel powder (10 g) was added, and the mixture was evaporated to dryness. The resulting product was dry-loaded onto the column and subjected to column chromatography using 5% methanol/dichloromethane to 1% ammonia water/15% methanol/dichloromethane as the eluents. The desired product was collected, concentrated, and evaporated to dryness to provide 0.37 g of the product. $^1$H-NMR (600 MHz, DMSO-$d_6$) δ 8.79-7.48 (m, 48H), 4.27-4.23 (m, 6H), 4.20-4.17 (m, 5H), 4.13-4.07 (m, 14H), 4.05-3.99 (m, 6H), 3.89-3.43 (m, 2798H), 3.31-3.23 (m, 5H), 3.23-3.15 (m, 6H), 3.11-3.03 (m, 12H), 3.03-2.92 (m, 14H), 2.87-2.75 (m, 14H), 2.51-2.44 (m, 4H), 2.29-1.98 (m, 74H), 1.93-1.65 (m, 57H), 1.51-1.13 (m, 279H)

4.23 Preparation of **81-48**

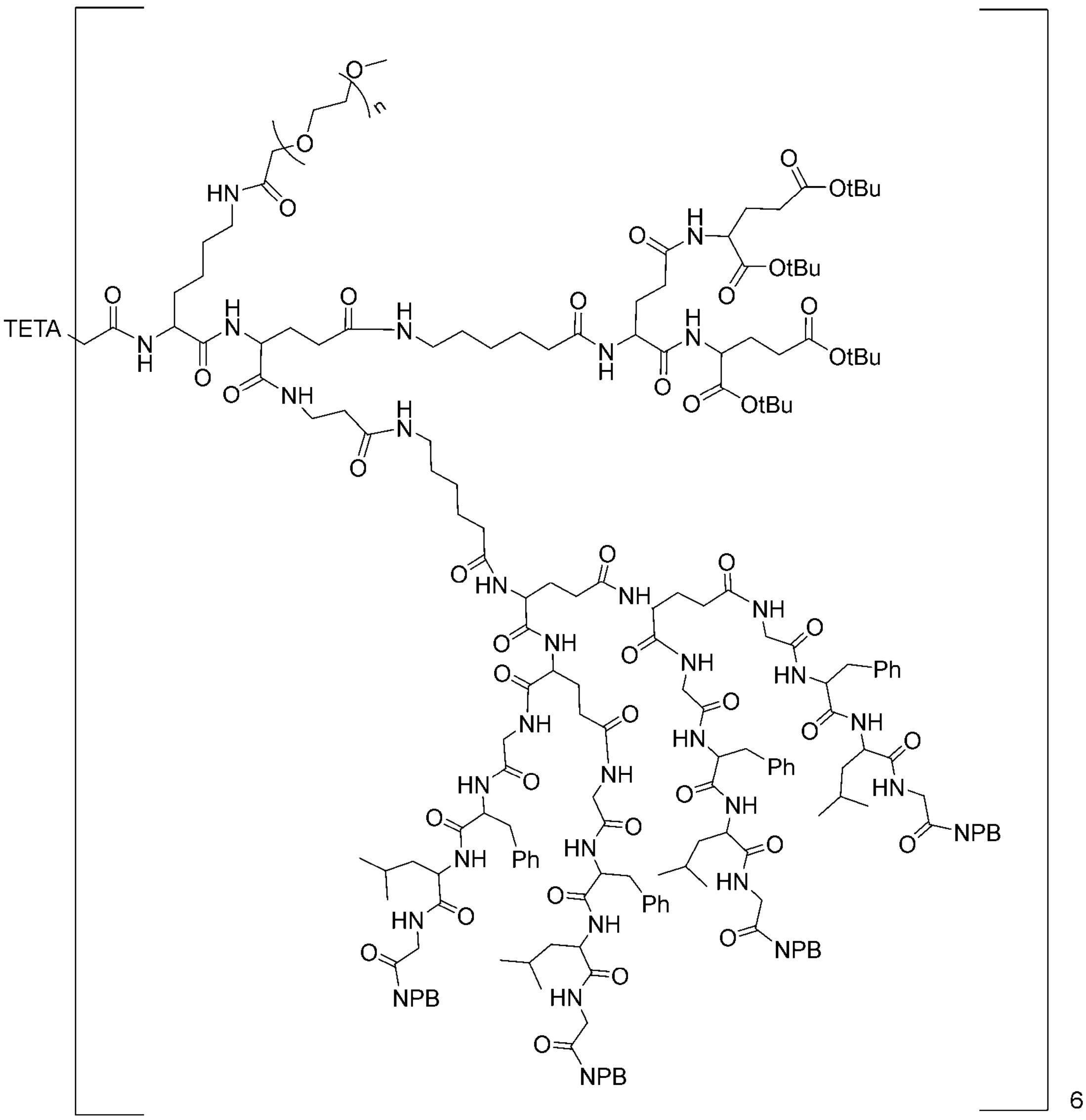

**81-68** (0.6 g, 0.0158 mmol), **81-43** (0.46 g, 0.143 mmol), HBTU (0.054 g, 0.143 mmol), and HOBT (0.019 g, 0.143 mmol) were added to a 50 mL round-bottom flask, then NMP (7 mL) was added to dissolve the mixture with the assistance of ultrasonication, and the mixture was stirred at 0°C for 10 minutes. DIEA (0.14 mL, 0.8532 mmol) was then added dropwise. The reaction was continued with stirring at 0°C for 5 minutes. The flask was then taken out, and the reaction was carried out with stirring at room temperature. After the reaction was completed, methyl tert-butyl ether (500 mL) was added

for precipitation. A solid precipitated and was filtered. The filter cake was dissolved in a methanol/dichloromethane (1/1) mixture (100 mL) with the assistance of ultrasonication, then 100-200 mesh silica gel powder (10 g) was added, and the mixture was evaporated to dryness. The resulting product was dry-loaded onto the column and subjected to column chromatography using 0-1% ammonia water/5-10% methanol/dichloromethane as the eluent. The desired product was collected, concentrated, and evaporated to dryness to provide 0.4 g of the product. $^1$H-NMR (600 MHz, DMSO-$d_6$) δ 9.34-9.23 (m, 24H), 8.79-7.48 (m, 360H), 7.31-7.06 (m, 138H), 4.63-4.51 (m, 24H), 4.49-4.31 (m, 48H), 4.27-4.12 (m, 29H), 4.13-3.88 (m, 68H), 3.88-3.43 (m, 2864H), 3.31-3.23 (m, 5H), 3.23-3.10 (m, 18H), 3.10-2.92 (m, 56H), 2.87-2.75(m, 110H), 2.51-2.44 (m, 4H), 2.29-1.65 (m, 299H), 1.65-1.13 (m, 399H), 0.94-0.65 (m, 144H)

4.24 Preparation of **81-106**

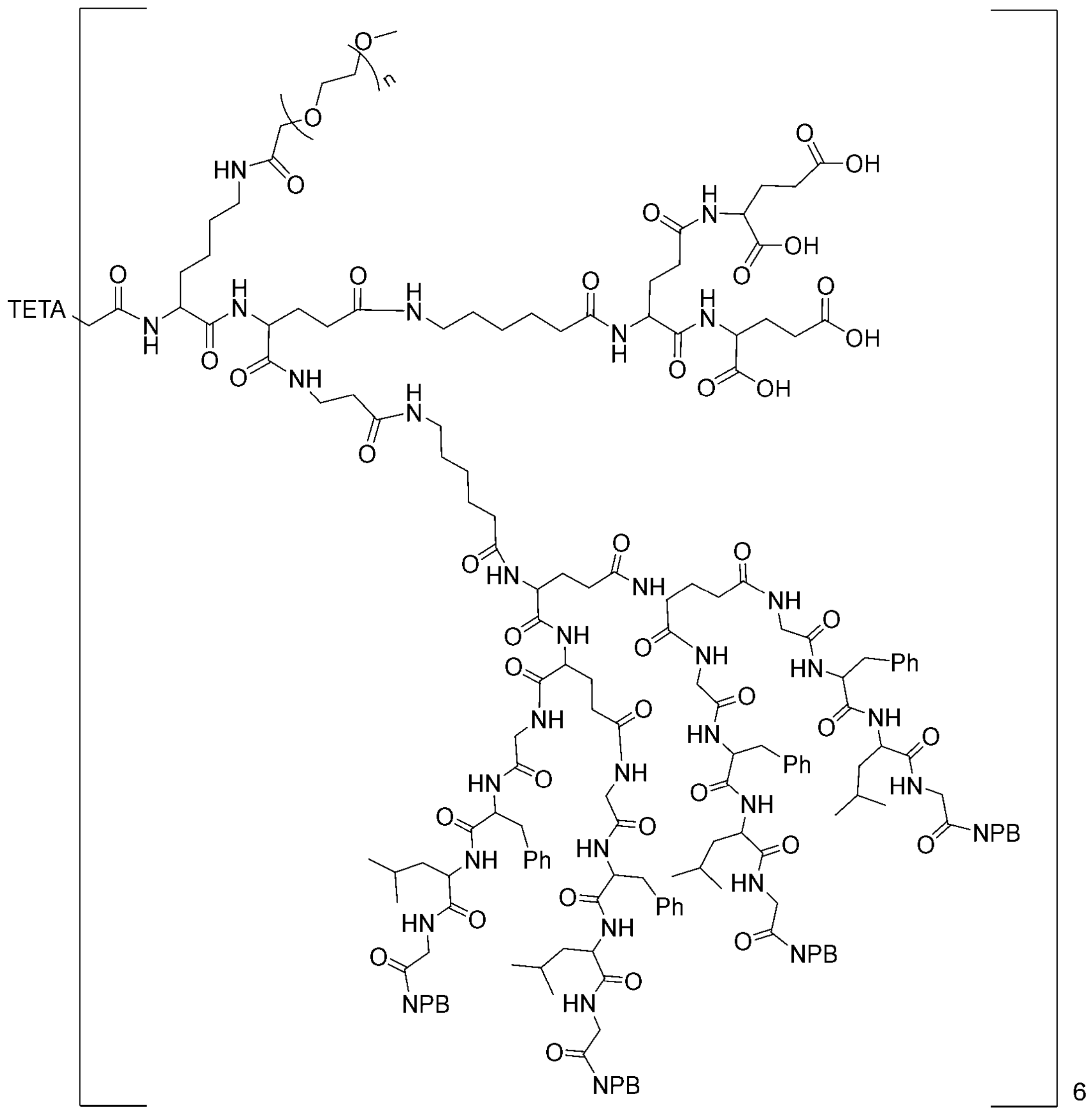

**81-48** (1.5 g, 0.026 mmol) was added to a 50 mL round-bottom flask, then TFA (10 mL, 134.6 mmol) was added, and the mixture was reacted with stirring at room temperature. After the reaction was completed, an appropriate amount of dichloromethane was added, and the TFA and dichloromethane were removed by rotary evaporation. An appropriate amount of methyl tert-butyl ether was then added, and the methyl tert-butyl ether was removed by rotary evaporation. Methyl tert-butyl ether (100 mL) was added again, and the mixture was ultrasonically treated. The mixture was allowed to stand, and the supernatant was discarded. This process was repeated several times. The mixture was concentrated and dried to provide 1.38 g of a solid as the product. $^1$H-NMR (600 MHz, DMSO-$d_6$) δ 9.34-9.23 (m, 24H), 8.79-7.48 (m, 360H), 7.31-7.06 (m, 138H), 4.63-4.51 (m, 24H), 4.49-4.31 (m, 48H), 4.27-4.12 (m, 29H), 4.13-3.88 (m, 68H), 3.88-3.43 (m,

2888H), 3.31-3.10 (m, 23H), 3.10-2.92 (m, 56H), 2.87-2.75 (m, 110H), 2.52-2.43 (m, 4H), 2.29-1.66 (m, 299 H), 1.66-1.13 (m, 183H), 0.94-0.64 (m, 144H)

4.25 Preparation of **81-110**

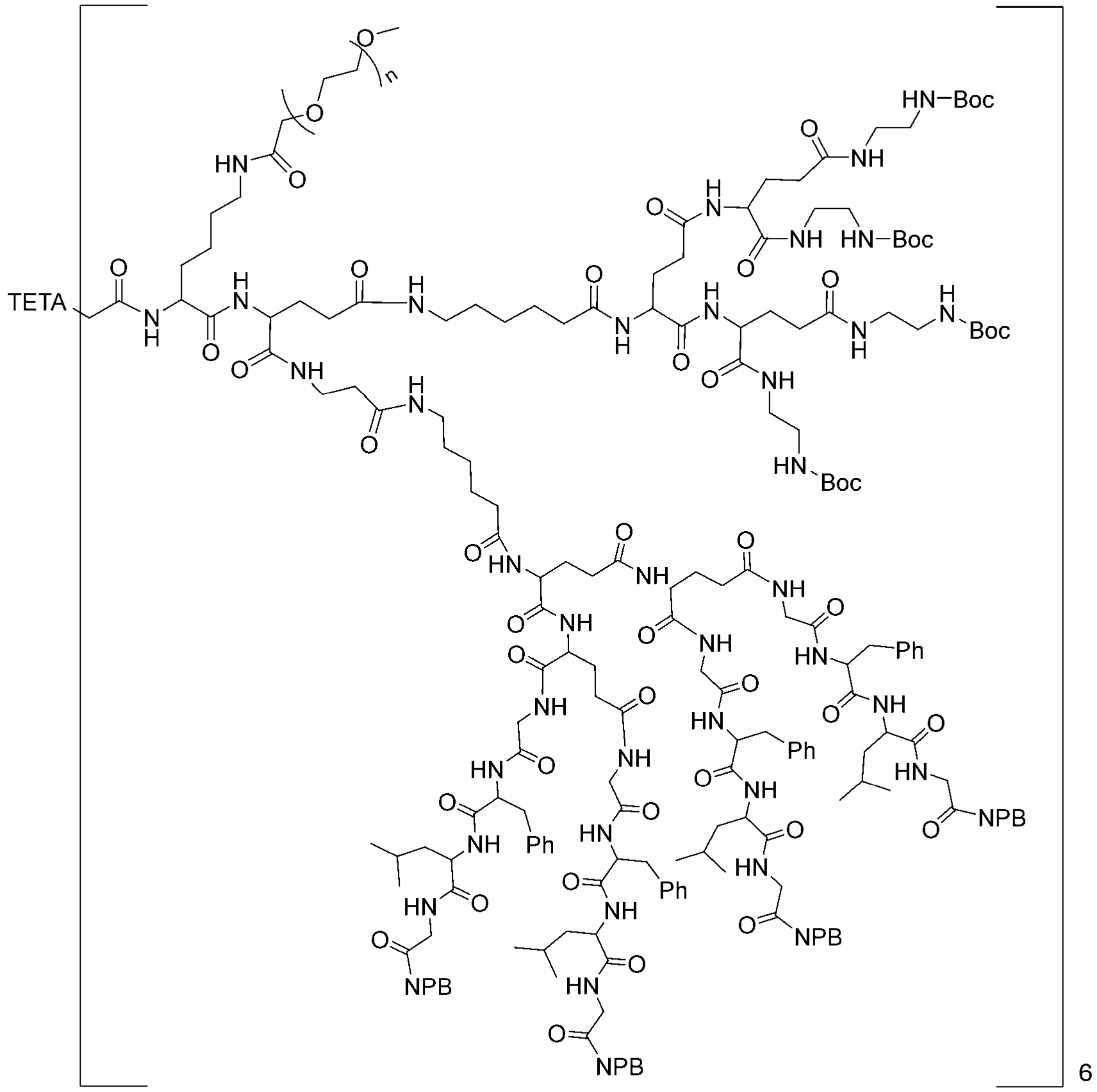

**81-106** (0.87 g, 0.0156 mmol), N-Boc-ethylenediamine hydrochloride (0.12 g, 0.75 mmol), HBTU (0.28 g, 0.75 mmol), and HOBT (0.1 g, 0.75 mmol) were placed in a 50 mL round-bottom flask, then DMF (7 mL) was added to dissolve the mixture with the assistance of ultrasonication, and the mixture was stirred at 0°C for 1 minutes. DIEA (0.3 mL, 1.872 mmol) was added dropwise, and the reaction was carried out with stirring at 35°C. After the reaction was completed, methyl tert-butyl ether (500 mL) was added for precipitation. A solid precipitated and was filtered. The filter cake was dissolved in a methanol/dichloromethane (1/4) mixture (50 mL), then 100-200 mesh silica gel powder (10 g) was added, and the mixture was evaporated to dryness. The resulting product was dry-loaded onto the column and subjected to column chromatography using 1% ammonia water/2-10% methanol/dichloromethane as the eluent. The desired product was collected, concentrated, and evaporated to dryness to provide 0.9 g of the product. $^1$H-NMR (600 MHz, DMSO-$d_6$) δ 9.34-9.22 (m, 24H), 8.79-7.45 (m, 384H), 7.31-7.06 (m, 162H), 4.63-4.51 (m, 24H), 4.49-4.30 (m, 48H), 4.27-4.12 (m, 29H), 4.13-3.88 (m, 68H), 3.88-3.43 (m, 2960H), 3.31-3.10 (m, 23H), 3.10-2.92 (m, 56H), 2.87-2.73 (m, 110H), 2.52-1.66 (m, 303H), 1.66-1.13 (m, 399H), 0.94-0.64 (m, 144H)

4.26 Preparation of **81-113**

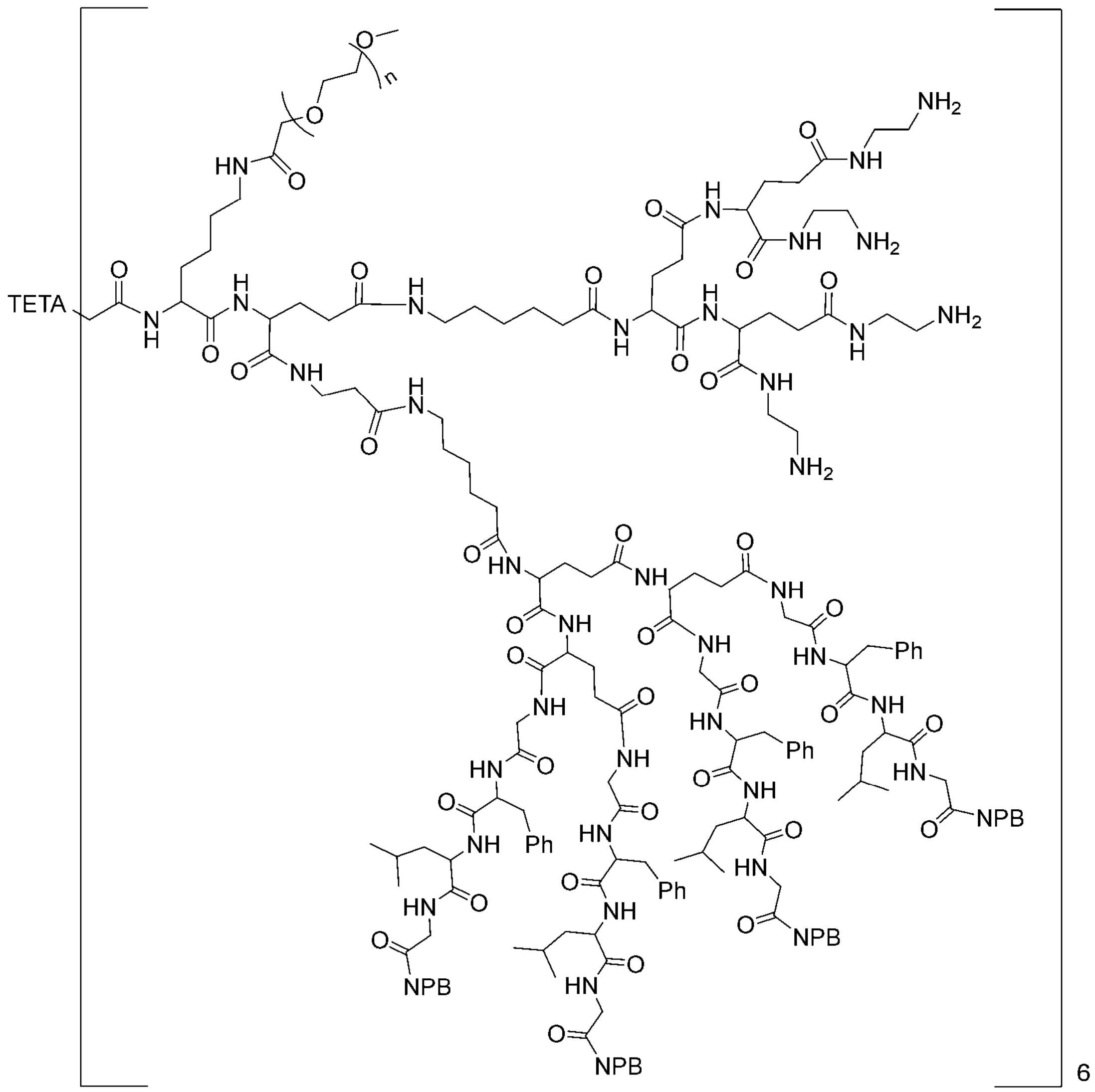

**81-110** (0.9 g, 0.015 mmol) was added to a 50 mL round-bottom flask, then TFA (5 mL, 67.3 mmol) was added, and the mixture was reacted with stirring at room temperature. After the reaction was completed, the TFA was removed by rotary evaporation, and methyl tert-butyl ether (500 mL) was added for precipitation. A solid precipitated and was then treated ultrasonically. The methyl tert-butyl ether was removed by rotary evaporation. The residue was dissolved by adding a methanol/dichloromethane (1/4) mixture (50 mL), and then methyl tert-butyl ether (200 mL) was added for precipitation. A solid precipitated and was allowed to stand. The supernatant was discarded, and the residual solvent was removed by rotary evaporation. This process, i.e., dissolution, precipitation and rotary evaporation under reduced pressure, was repeated three times. The obtained solid was dried, then dissolved in an appropriate amount of dichloromethane, and DIEA was added to adjust the pH to weak alkaline. The resulting product was evaporated to dryness, and dried to provide 0.44 g of the product.

4.27 Preparation of **81-116**

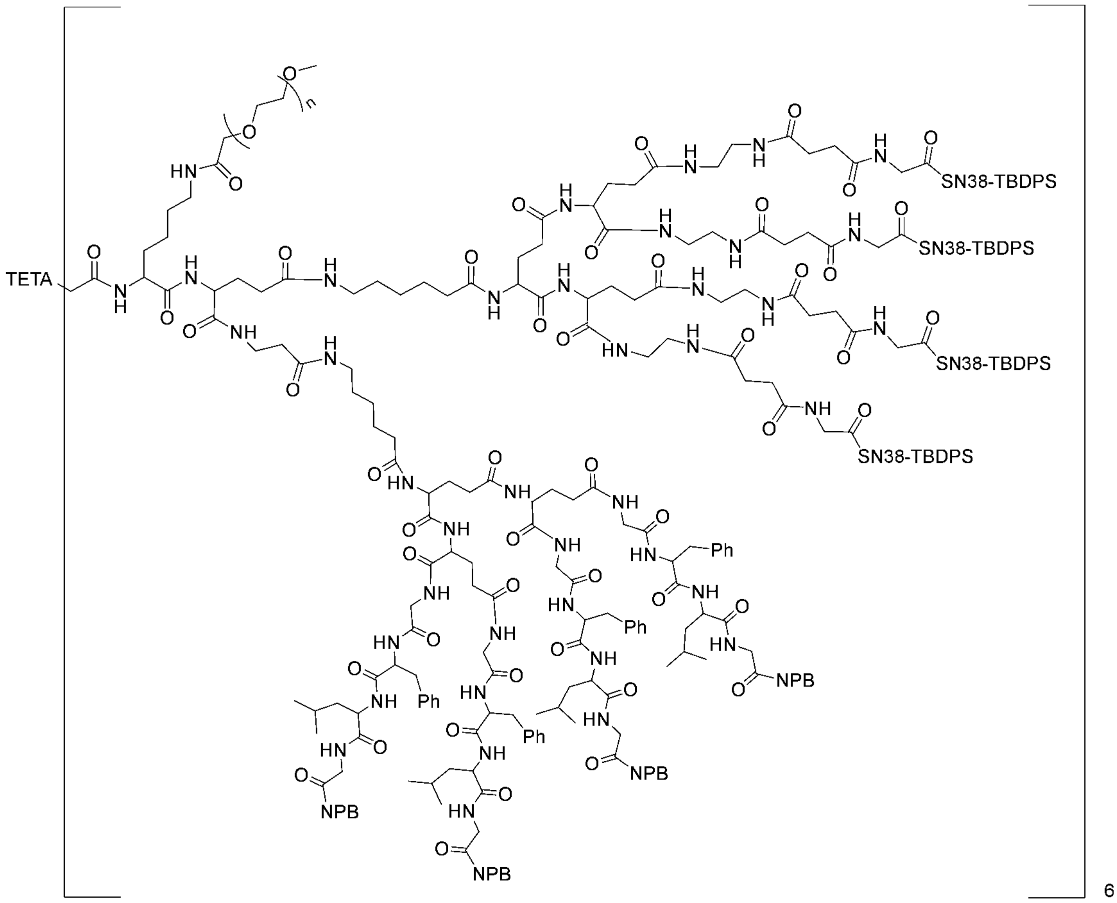

**81-113** (0.44 g, 0.0077 mmol), **65-131** (0.44 g, 0.559 mmol), HBTU (0.21 g, 0.559 mmol), and HOBT (0.075 g, 0.559 mmol) were added to a 500 mL round-bottom flask, then DMF (10 mL) was added to dissolve the mixture with the assistance of ultrasonication, and the mixture was stirred at 0°C for 30 minutes. DIEA (0.28 mL, 1.677 mmol) was then slowly added dropwise. After the dropwise addition was completed, the reaction was carried out with stirring at room temperature overnight. After the reaction was completed, n-hexane (50 mL) and methyl tert-butyl ether (200 mL) were added for precipitation, resulting in an oily solid. The supernatant was discarded, and methyl tert-butyl ether (200 mL) was added for precipitation. This process was repeated three times. A powdery solid precipitated and was filtered. The filter cake was collected, then dissolved in a methanol/dichloromethane (1/4) mixture, dry-loaded onto the column and subjected to column chromatography using 1% triethylamine/8-10% methanol/dichloromethane as the eluent. The desired product was collected, concentrated, and evaporated to dryness to provide 0.18 g of the product. $^1$H-NMR (600 MHz, DMSO-$d_6$) δ 9.34-9.22 (m, 24H), 8.79-7.37 (m, 720H), 7.31-7.03 (m, 186H), 5.50-5.42 (m, 48H), 5.31-5.12 (m, 48H), 4.63-4.51 (m, 24H), 4.49-4.30 (m, 48H), 4.27-4.12 (m, 53H), 4.13-3.87 (m, 92H), 3.87-3.43 (m, 2960H), 3.31-3.10 (m, 23H), 3.10-2.92 (m, 56H), 2.87-2.73 (m, 110H), 2.73-2.55 (m, 48H), 2.52-1.64 (m, 447H), 1.64-0.99 (m, 399H), 0.94-0.64 (m, 288H)

4.28 Preparation of **81-118**

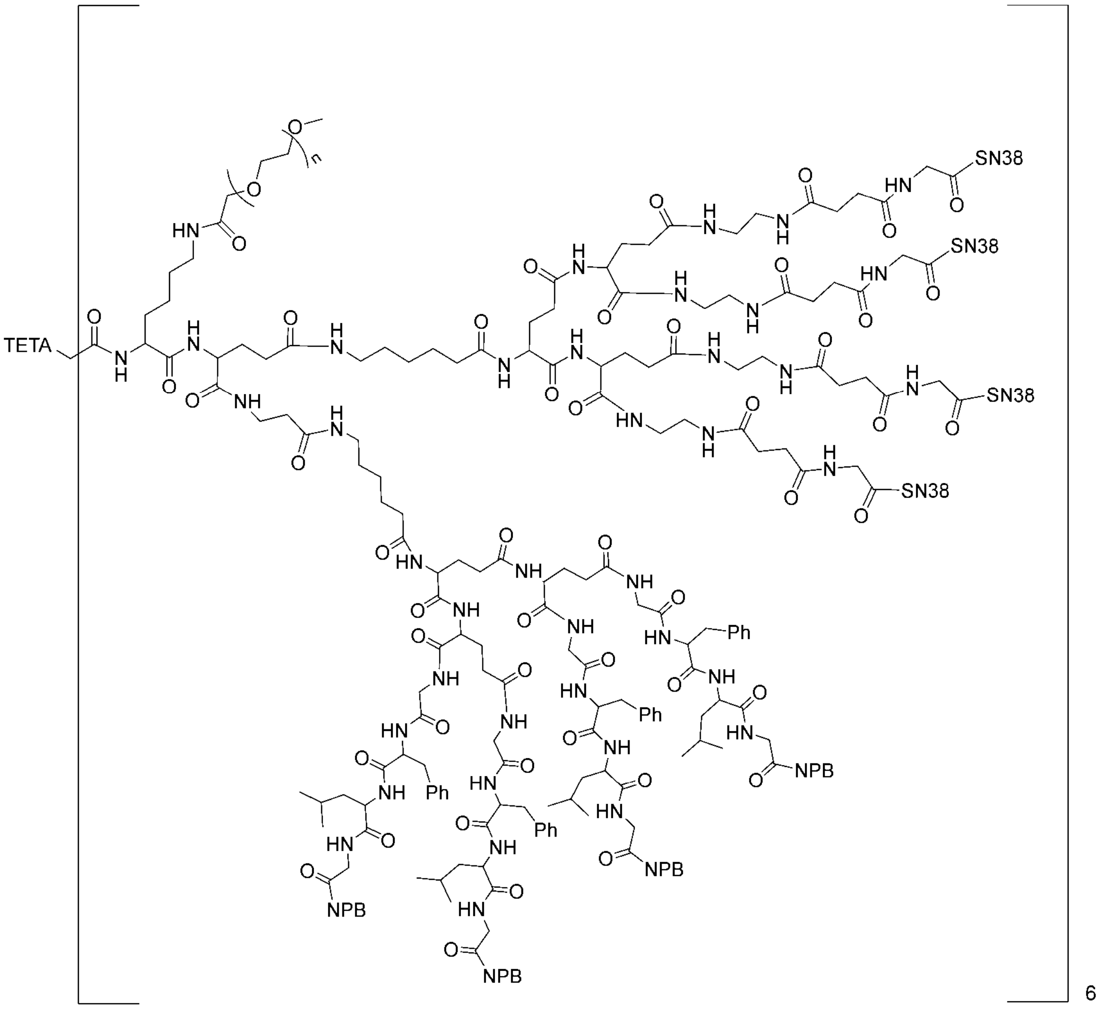

**81-116** (0.08 g, 0.00106 mmol) was placed in a 50 mL round-bottom flask. THF (7 mL) was added for dissolution with the assistance of ultrasonication, then TBAF (0.032 g, 0.102 mmol) and 0.1 N dilute hydrochloric acid (0.1 mL) were added, and the mixture was reacted with stirring at room temperature for 12 hours. After the reaction was completed, the THF was removed by rotary evaporation. Methyl tert-butyl ether (50 mL) was then added for precipitation. A solid precipitated and was filtered. The filter cake was dissolved in an appropriate amount of DMF, and then methyl tert-butyl ether (200 mL) was added for precipitation. This process, i.e., dissolving the product with DMF and adding methyl tert-butyl ether to precipitate the product, was repeated four times. The mixture was filtered to provide a solid, which was dried to provide 0.028 g of the product. $^1$H-NMR (600 MHz, DMSO-$d_6$) δ 9.34-9.22 (m, 24H), 8.79-7.45 (m, 504H), 7.31-7.03 (m, 186H), 5.50-5.42 (m, 48H), 5.31-5.12 (m, 48H), 4.63-4.51 (m, 24H), 4.49-4.30 (m, 48H), 4.27-4.12 (m, 53H), 4.13-3.87 (m, 92H), 3.87-3.43 (m, 2960H), 3.31-3.10 (m, 23H), 3.10-2.92 (m, 56H), 2.87-2.73 (m, 110H), 2.73-2.55 (m, 48H), 2.55-1.64 (m, 447H), 1.64-0.99 (m, 183H), 0.94-0.64 (m, 288H)

Example 5: Synthesis of Compound **50-221**

38-230 + 67-53

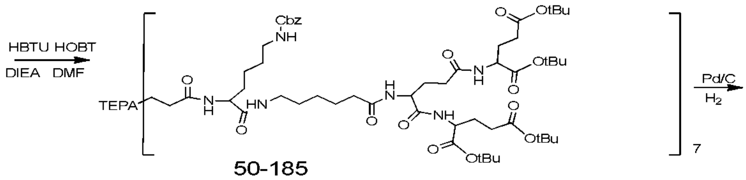


50-185

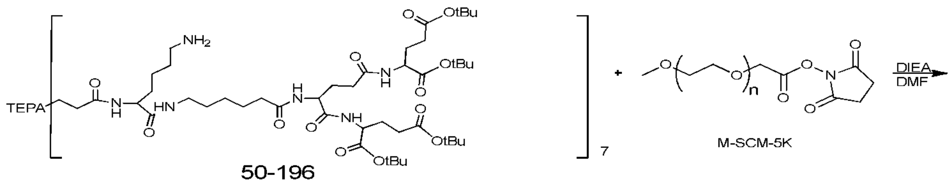


50-196

M-SCM-5K

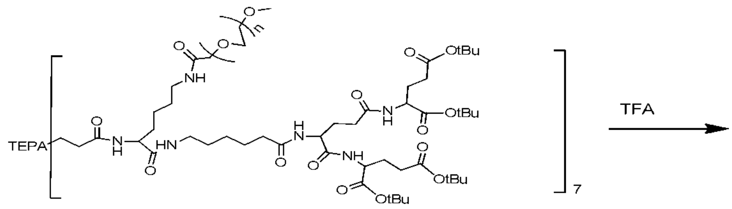


50-205

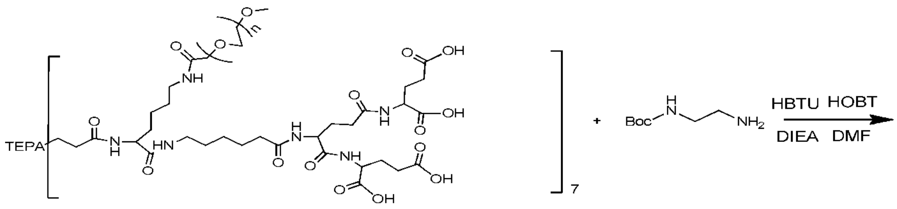


50-215

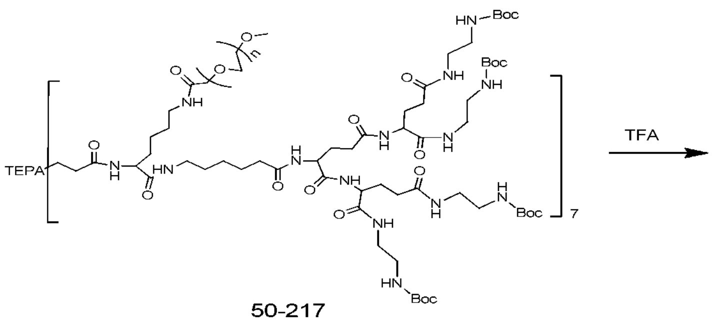


50-217

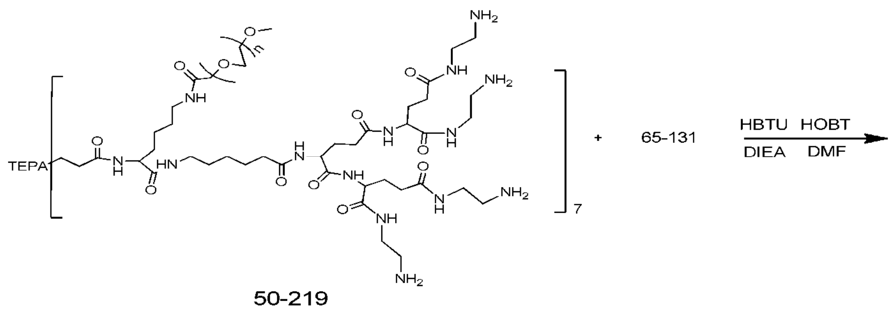


65-131

50-219

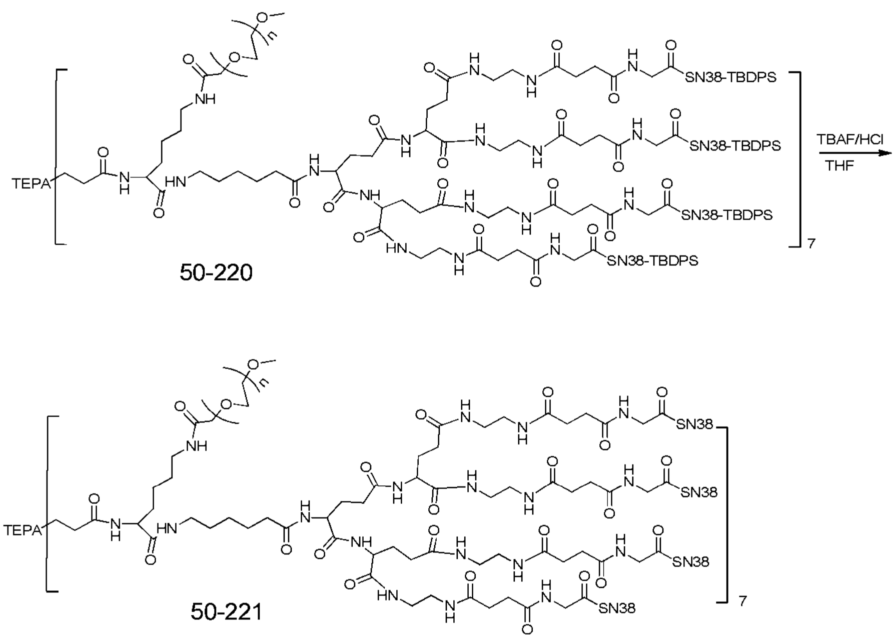


50-220

50-221

5.1 Preparation of **50-185**

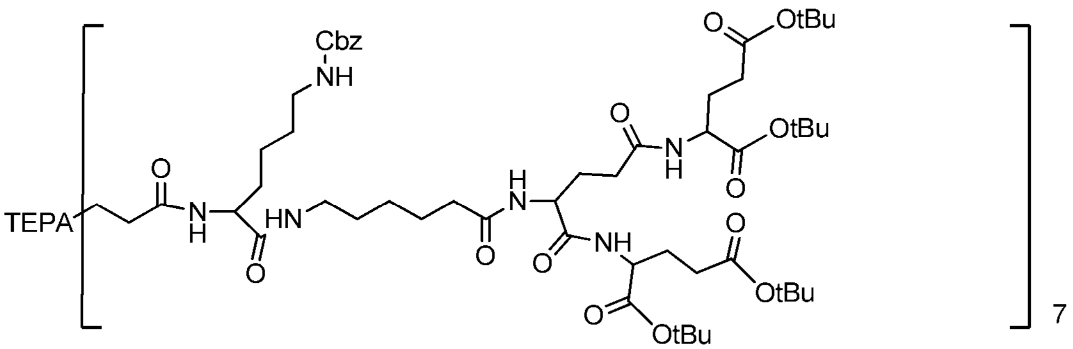

**38-230** (6.23 g, 6.23 mmol), **67-53** (0.474 g, 0.684 mmol), HBTU (2.72 g, 7.182 mmol), HOBT (0.97 g, 7.182 mmol) and DMF (30 mL) were placed in a 500 mL flask, and dissolved with the assistance of ultrasonication, and stirred at 0°C for 10 minutes. DIEA (3.56 mL, 21.546 mmol) was then added dropwise, and the reaction was carried out with stirring at room temperature for 30 hours. After the reaction was completed, the reaction mixture was transferred to a 2 L separatory funnel containing ethyl acetate (300 mL) and saturated sodium bicarbonate solution (500 mL) for extraction. The aqueous phase was further extracted with ethyl acetate (300 mL). The organic phases were combined and concentrated, then 200-300 mesh silica gel powder was added, and the mixture was evaporated to dryness. The resulting product was dry-loaded onto the column and subjected to column chromatography using 4-6% methanol/dichloromethane as the eluent. The desired product was collected, concentrated and evaporated to dryness to provide 5.25 g of the product. $^1$H-NMR (600 MHz, DMSO-$d_6$) δ 8.23-8.14 (m, 14H), 8.12-8.04 (m, 7H), 7.98-7.89 (m, 7H), 7.58-7.51 (m, 7H), 7.40-7.29 (m, 35H), 7.29-7.21 (m, 7H), 5.07-4.97 (m, 14H), 4.32-4.22 (m, 7H), 4.16-4.08 (m, 14H), 3.90-3.82 (m, 5H), 3.27-3.13 (m, 9H), 3.13-3.05 (m, 11H), 3.05-2.89 (m, 33H), 2.89-2.66 (m, 22H), 2.32-2.20 (m, 28H), 2.20-2.05 (m, 27H), 1.96-1.84 (m, 21H), 1.81-1.64 (m, 34H), 1.63-1.16 (m, 323H); ESI [M+H$^+$] 7601.33, [M+Na$^+$] 7624.07, [M+K$^+$] 7638.58

5.2 Preparation of **50-196**

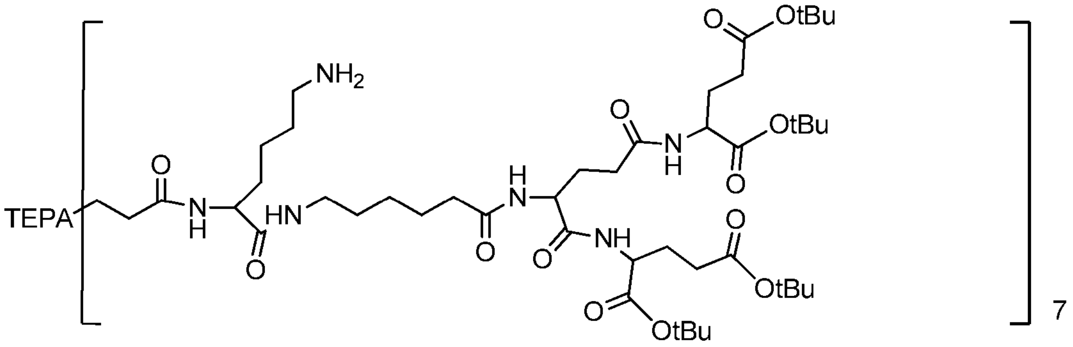

**50-185** (0.65 g, 0.0855 mmol) was added to a hydrogenation reactor, then 10% Pd/C catalyst (0.6 g) was added, and then DMF (30 mL) was added for dissolution. The reactor was sealed, evacuated with a water pump, and then filled with hydrogen gas. This process was repeated three times. The hydrogen pressure was finally adjusted to 1.8 MPa, and the reaction was stirred at room temperature overnight. After the reaction was completed, the reaction mixture was filtered through Celite, and the Celite was then washed with DMF (15 mL). The DMF solutions were combined, and n-hexane (200 mL) was added for precipitation. The supernatant was discarded. Methyl tert-butyl ether (150 mL × 3) was added again for precipitation. Finally, the obtained solid was dried in a vacuum oven to provide 0.4 g of the product. $^1$H-NMR (600 MHz, DMSO-$d_6$) δ 8.25-8.15 (m, 7H), 8.14-8.05 (m, 7H), 8.02-7.90 (m, 14H), 7.82-7.24 (m, 7H), 7.24-7.12(m, 7H), 4.32-4.22 (m, 7H), 4.16-4.08 (m, 14H), 3.90-3.82 (m, 5H), 3.56-3.13 (m, 3264H), 3.13-2.88 (m, 15H), 2.88-2.64 (m, 22H), 2.32-2.20 (m, 28H), 2.20-2.06 (m, 56H), 1.96-1.84 (m, 21H), 1.81-1.64 (m, 34H), 1.63-1.16 (m, 323H)

5.3 Preparation of **50-205**

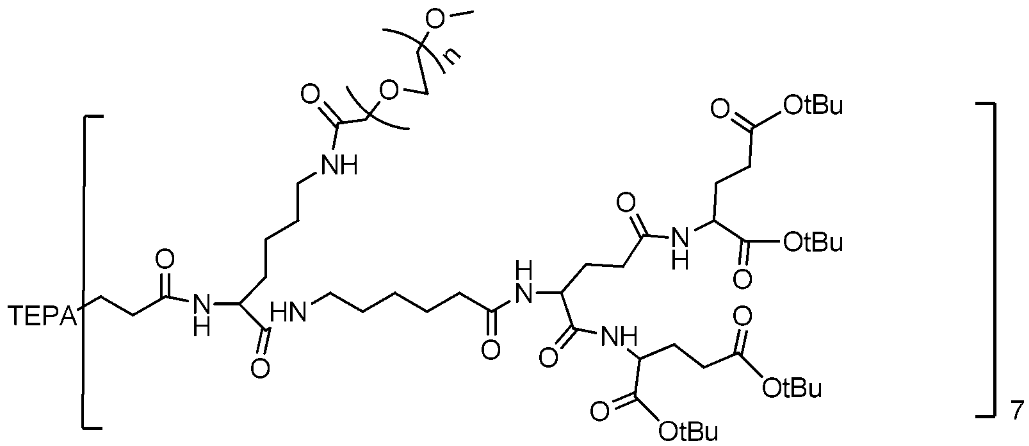

**50-196** (0.291 g, 0.0437 mmol) and M-SCM-5K (1.95 g, 0.3667 mmol, Lot Number: ZZ363P139, purchased from Tianjin Jenkem) were placed in a 100 mL round-bottom flask, and DMF (10 mL) was added to dissolve the mixture. The mixture was stirred at -5°C for 30 minutes. DIEA (1.5 mL, 9.177 mmol) was then slowly added dropwise. After the dropwise addition was completed, the reaction was carried out at low temperature for 2 hours, then the reaction was carried out with stirring at low speed at room temperature. The reaction was monitored by TLC. After the reaction was completed, methyl tert-butyl ether (150 mL) was added to the reaction mixture for precipitation. The supernatant was discarded, and n-hexane (150 mL) was added again for precipitation. This process was repeated three times. The mixture was filtered, and the obtained solid was dissolved in dichloromethane, then 100-200 mesh silica gel powder was added, and the mixture was evaporated to dryness. The resulting product was dry-loaded onto the column and subjected to column chromatography using 1% ammonia/4-6% methanol/dichloromethane as the eluent. The desired product was collected, concentrated, and evaporated to dryness to provide 1.207 g of the product. $^1$H-NMR (600 MHz, DMSO-$d_6$) δ 8.22-7.92 (m, 42H), 7.66-7.12 (m, 42H), 4.57 (s, 12H), 4.11 (s, 13H), 3.74-3.62 (m, 18H), 3.51 (s, 3269H), 3.22-3.08 (m, 16H), 3.07 (t, 15H), 3.01-2.96 (t, 22H), 2.26-2.20 (q, 18H), 2.11-2.07 (d, 10H), 1.93-1.88 (m, 10H), 1.67-1.63 (m, 26H), 1.57 (s, 22H), 1.49 (s, 22H), 1.39 (s, 252H), 1.36-1.32 (m, 29H)

5.4 Preparation of **50-215**

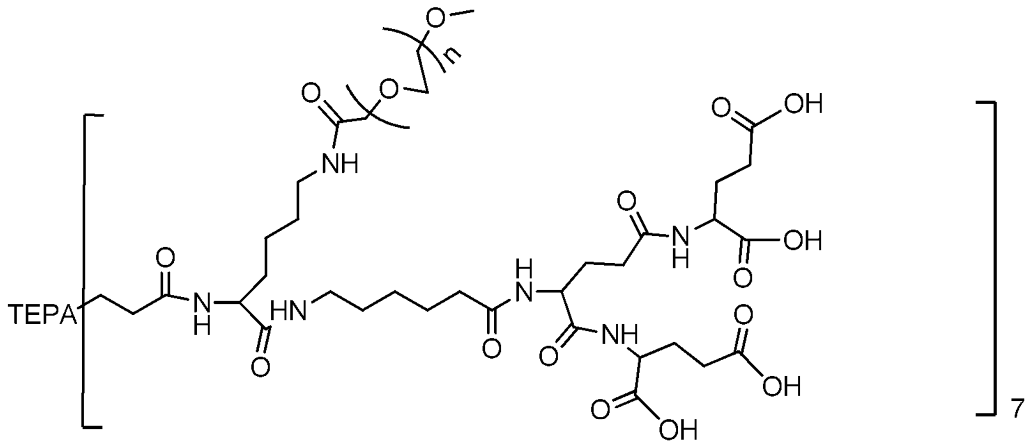

**50-205** (0.4 g, 0.0093 mmol) was placed in a 100 mL round-bottom flask, then TFA (0.4 mL, 5.208 mmol) was added for dissolution, and the mixture was reacted with stirring at room temperature overnight. After the reaction was completed, the reaction mixture was concentrated and evaporated to dryness under reduced pressure. Dichloromethane (5 mL) was then added for dissolution, followed by the addition of n-hexane (100 mL) for precipitation. The supernatant was discarded. This process was repeated twice. The precipitate was evaporated to dryness, dissolved in dichloromethane, and DIEA (0.3 mL) was added to make the solution weakly alkaline. The resulting product was then rotavapped to dryness to provide 0.38 g of the product. $^1$H-NMR (600 MHz, DMSO-$d_6$) δ 12.12-11.98(m, 28H), 8.25-8.15 (m, 7H), 8.14-8.05 (m, 7H), 8.02-7.90 (m, 14H), 7.82-7.24 (m, 7H),7.24-7.12(m, 7H), 4.32-4.22 (m, 7H), 4.16-4.08 (m, 14H), 3.90-3.82 (m, 5H), 3.56-3.13 (m, 3264H), 3.13-2.88 (m, 15H), 2.88-2.64 (m, 22H),2.32-2.06 (m, 84H), 1.96-1.84 (m, 21H), 1.81-1.64 (m, 34H), 1.63-1.16 (m, 71H)

5.5 Preparation of **50-217**

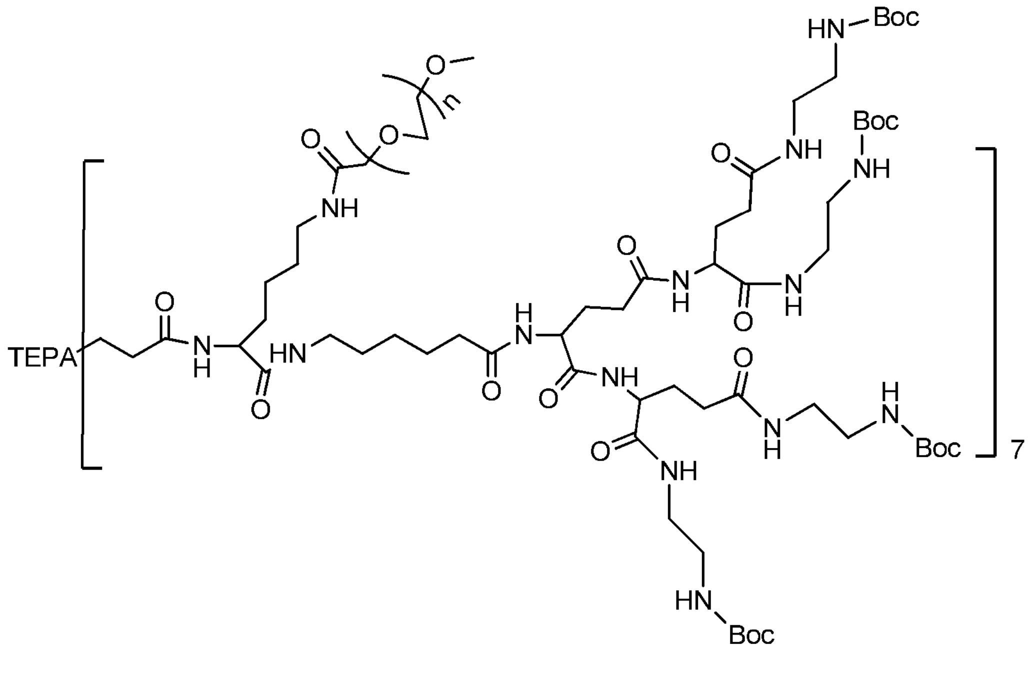

**50-215** (0.38 g, 0.0093 mmol), N-Boc-ethylenediamine (0.063 g, 0.3906 mmol), HBTU (0.15 g, 0.3906 mmol), HOBT (0.05 g, 0.3906 mmol), and DMF (10 mL) were placed in a 250 mL flask, and dissolved with the assistance of

ultrasonication. The mixture was stirred at 0°C for 10 minutes. DIEA (0.2 mL, 1.1718 mmol) was then added dropwise, and the reaction was stirred at room temperature until completion. Methyl tert-butyl ether (50 mL) and n-hexane (100 mL) were added to the reaction mixture for precipitation. The supernatant was discarded, and the residue was dried. Dichloromethane was added to the obtained solid for dissolution, then 100-200 mesh silica gel powder was added, and the mixture was evaporated to dryness. The resulting product was dry-loaded onto the column and subjected to column chromatography using 1% ammonia water/4-5% methanol/dichloromethane as the eluent. The desired product was collected, concentrated, and dried to provide 0.21 g of the product. $^1$H-NMR (600 MHz, DMSO-$d_6$) δ 8.26-8.14 (m, 7H), 8.14-8.05 (m, 7H), 8.05-7.24 (m, 49H), 7.24-6.95 (m, 35H), 4.32-4.20 (m, 7H), 4.16-4.08 (m, 14H), 3.90-3.82 (m, 5H), 3.56-3.13 (m, 3376H), 3.13-2.88 (m, 15H), 2.88-2.64 (m, 22H), 2.32-2.06 (m, 84H), 1.96-1.84 (m, 21H), 1.81-1.64 (m, 34H), 1.64-1.14 (m, 323H)

5.6 Preparation of **50-219**

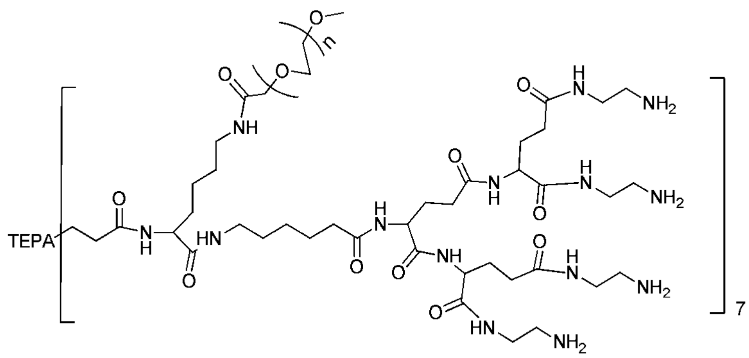

**50-217** (0.21 g, 0.0046 mmol) was placed in a 100 mL round-bottom flask, then TFA (0.5 mL, 6.44 mmol) was added for dissolution, and the mixture was reacted with stirring at room temperature overnight. After the reaction was completed, the reaction mixture was concentrated and evaporated to dryness, then dichloromethane (30 mL) was added for dissolution, and excess DIEA was added for neutralization. Then, n-hexane (100 mL) was added for precipitation, and the supernatant was discarded. This process was repeated twice. The precipitate was evaporated to dryness to provide 0.196 g of the product. $^1$H-NMR (600 MHz, DMSO-$d_6$) δ 8.26-8.12 (m, 7H), 8.12-8.05 (m, 7H), 8.05-7.22 (m, 49H), 7.22-6.95 (m, 7H), 4.32-4.18 (m, 8H), 4.16-4.07 (m, 13H), 3.90-3.82 (m, 5H), 3.56-3.13 (m, 3432H), 3.13-2.87 (m, 15H), 2.87-2.64 (m, 22H), 2.34-2.06 (m, 84H), 1.96-1.84 (m, 21H), 1.82-1.64 (m, 34H), 1.64-1.14 (m, 71H)

5.7 Preparation of **50-220**

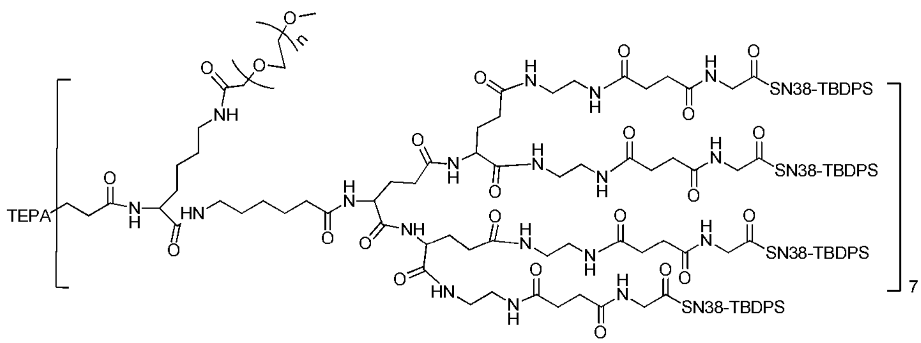

**50-219** (0.15 g, 0.0035 mmol), **65-131** (0.129 g, 0.1459 mmol), HBTU (0.055 g, 0.1459 mmol), HOBT (0.02 g, 0.1459 mmol), and DMF (3 mL) were placed in a 50 mL flask, and dissolved with the assistance of ultrasonication. The mixture was stirred at 0°C for 10 minutes. DIEA (0.03 mL, 0.196 mmol) was then added, and the reaction was carried out with stirring at room temperature until completion. Methyl tert-butyl ether (50 mL) and n-hexane (50 mL) were added to the reaction mixture for precipitation. The precipitate was evaporated to dryness to provide 0.1 g of the product. $^1$H-NMR (600 MHz, DMSO-$d_6$) δ 8.44-8.37 (m, 28H), 8.26-8.05 (m, 70H), 8.05-7.22 (m, 317H), 7.22-6.95 (m, 63H), 5.47-5.44 (m, 56H),

5.30-5.10 (m, 56H), 4.32-4.07 (m, 49H), 4.02-3.82 (m, 33H), 3.56-3.13 (m, 3404H), 3.13-2.64 (m, 37H), 2.64-2.58 (m, 56H), 2.45-2.40 (m, 56H), 2.39-2.06 (m, 196H), 1.96-1.64 (m, 55H), 1.64-1.10 (m, 323H), 0.95-0.83 (m, 84H)

5.8 Preparation of **50-221**

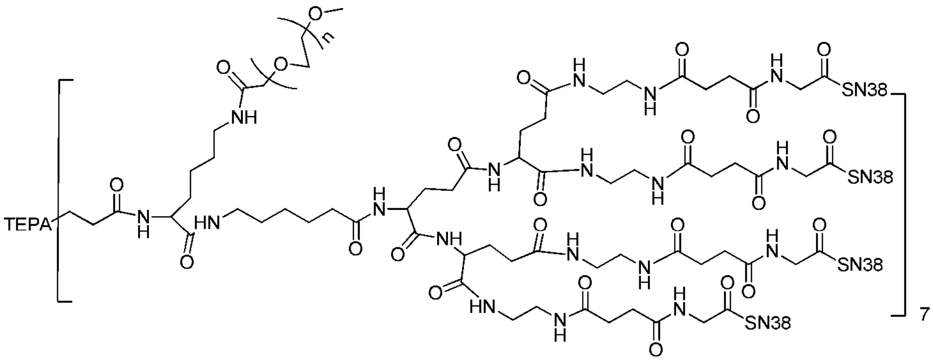

**50-220** (0.1 g, 0.0016 mmol), TBAF (0.044 g, 0.1792 mmol), THF (1 mL), and 1N dilute HCl solution (1 mL) were placed in a 50 mL flask, dissolved with the assistance of ultrasonication, and reacted with stirring at room temperature. After the reaction was completed, the THF was removed by rotary evaporation. Anhydrous ethanol (20 mL) was added for dissolution, followed by rotary evaporation to dryness. This process was repeated three times. DMF (3 mL) was then added for dissolution. Isopropanol was added for precipitation, and the mixture was filtered. This process was repeated two times. $CH_2Cl_2$ (4 mL) was added for dissolution, and methyl tert-butyl ether (30 mL) was added for precipitation. The precipitate was filtered, and dried to provide 0.05 g of the product. $^1$H-NMR (600 MHz, DMSO-$d_6$) δ 8.44-8.37 (m, 56H), 8.26-8.05 (m, 70H), 8.05-7.22 (m, 37H), 7.22-6.95(m, 63H), 5.47-5.44 (m, 56H), 5.30-5.10 (m, 56H), 4.32-4.07 (m, 49H), 4.02-3.82 (m, 33H), 3.56-3.13 (m, 3404H), 3.13-2.64 (m, 37H), 2.64-2.58 (m, 56H), 2.45-2.4(m, 56H), 2.39-2.06 (m, 196H), 1.96-1.64 (m, 55H), 1.64-1.10 (m, 71H), 0.95-0.82 (m, 84H)

Example 6: Synthesis of Compound **69-113**

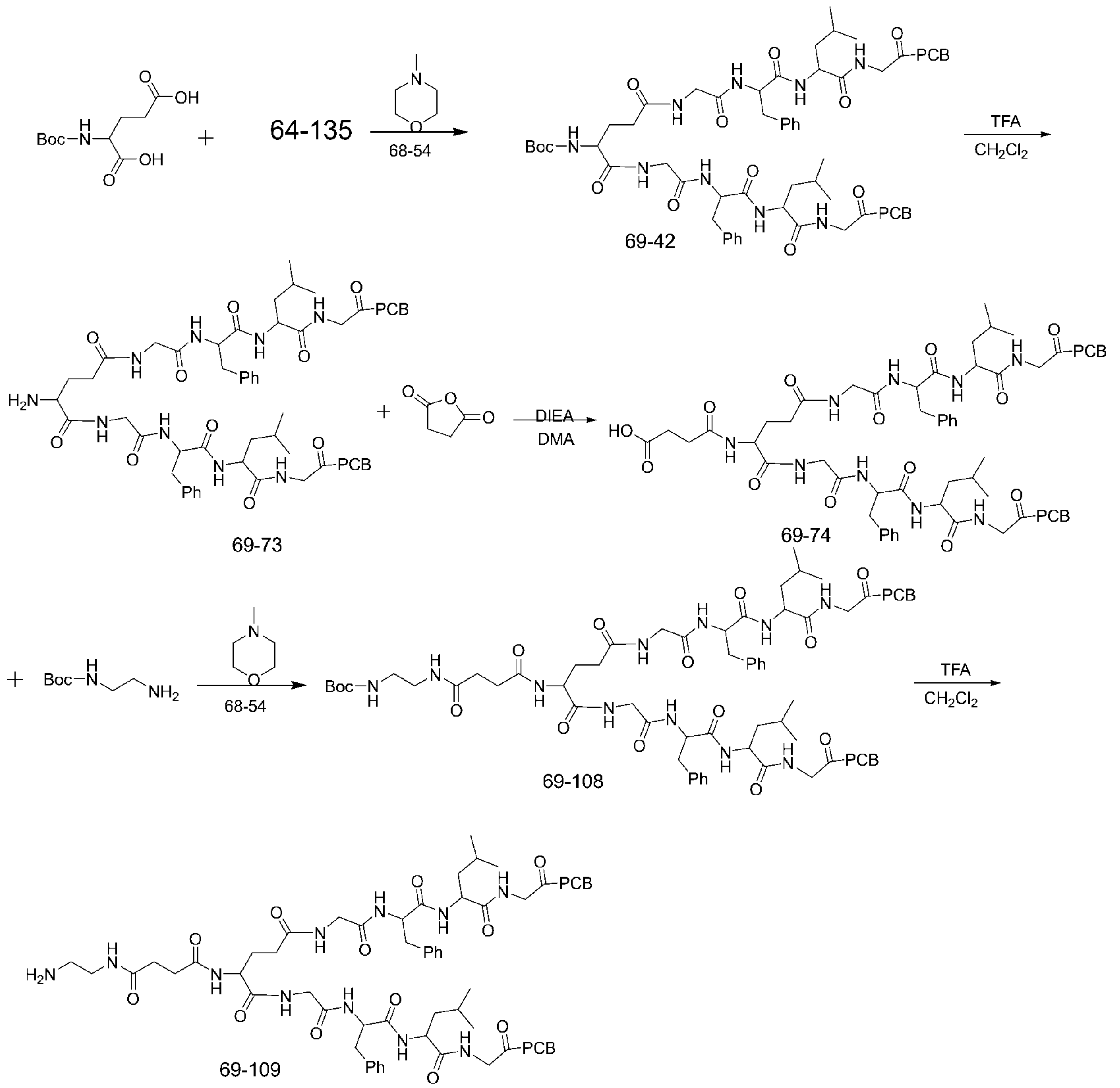


69-42

69-73

69-74

69-108

69-109

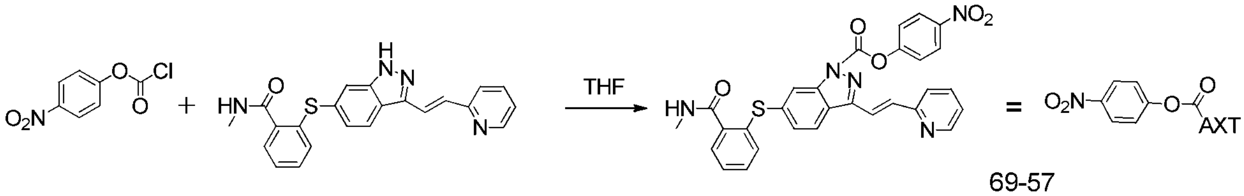


69-57

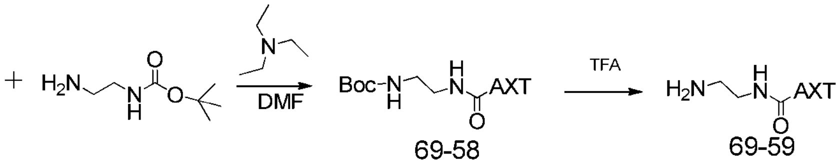


69-58

69-59

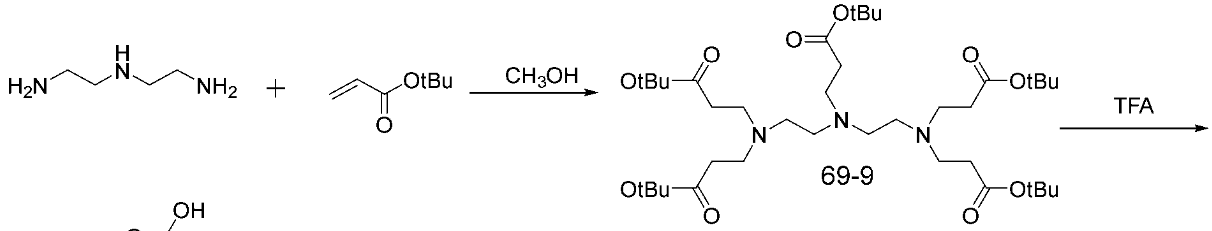


69-9

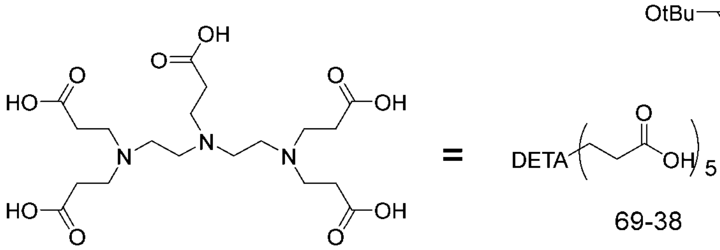

69-38

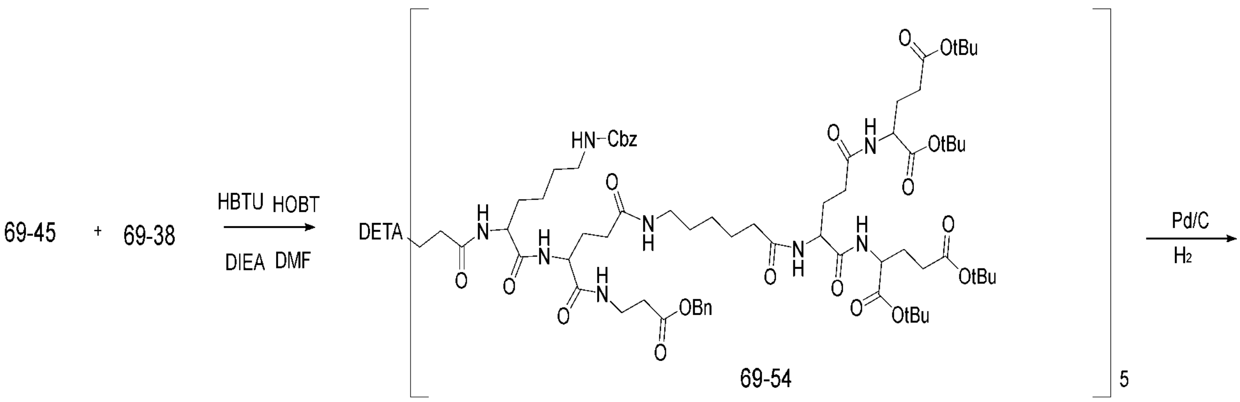


69-54

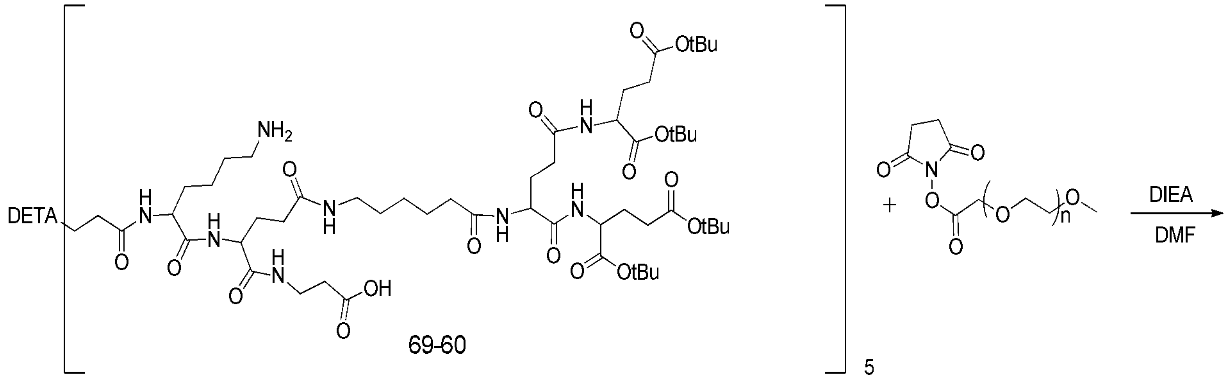


69-60

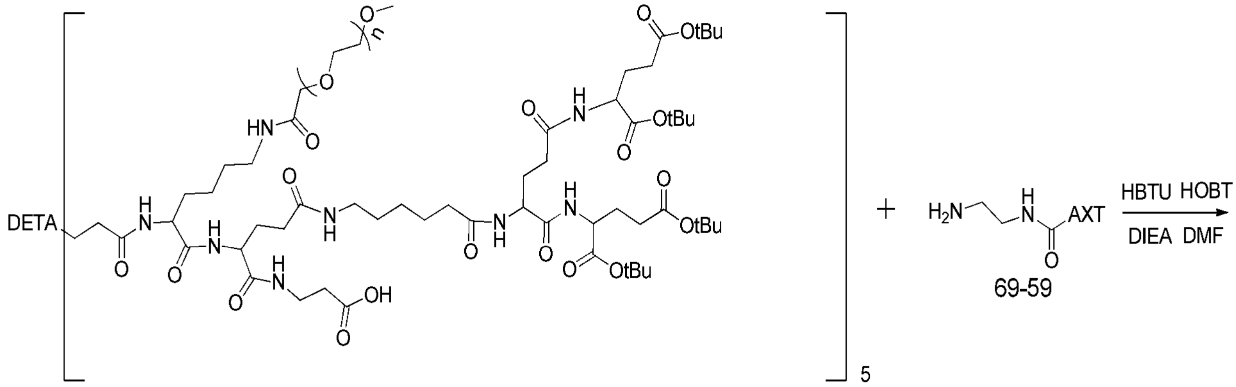


69-59

69-61

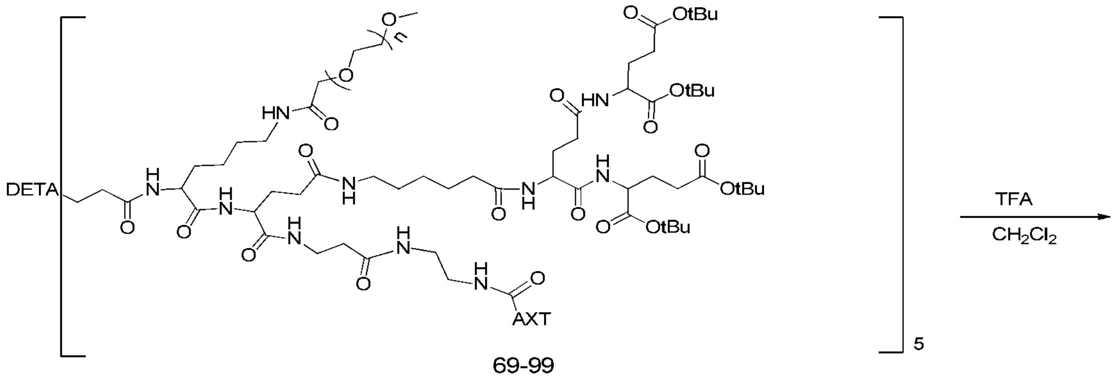


69-99

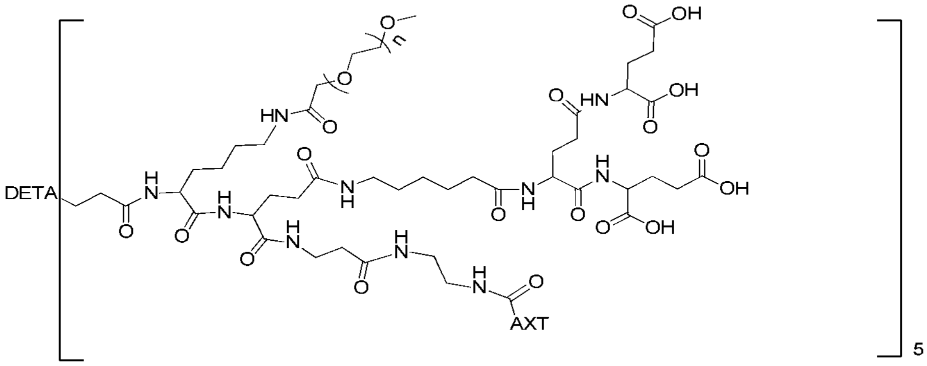

\+ 69-109

69-101

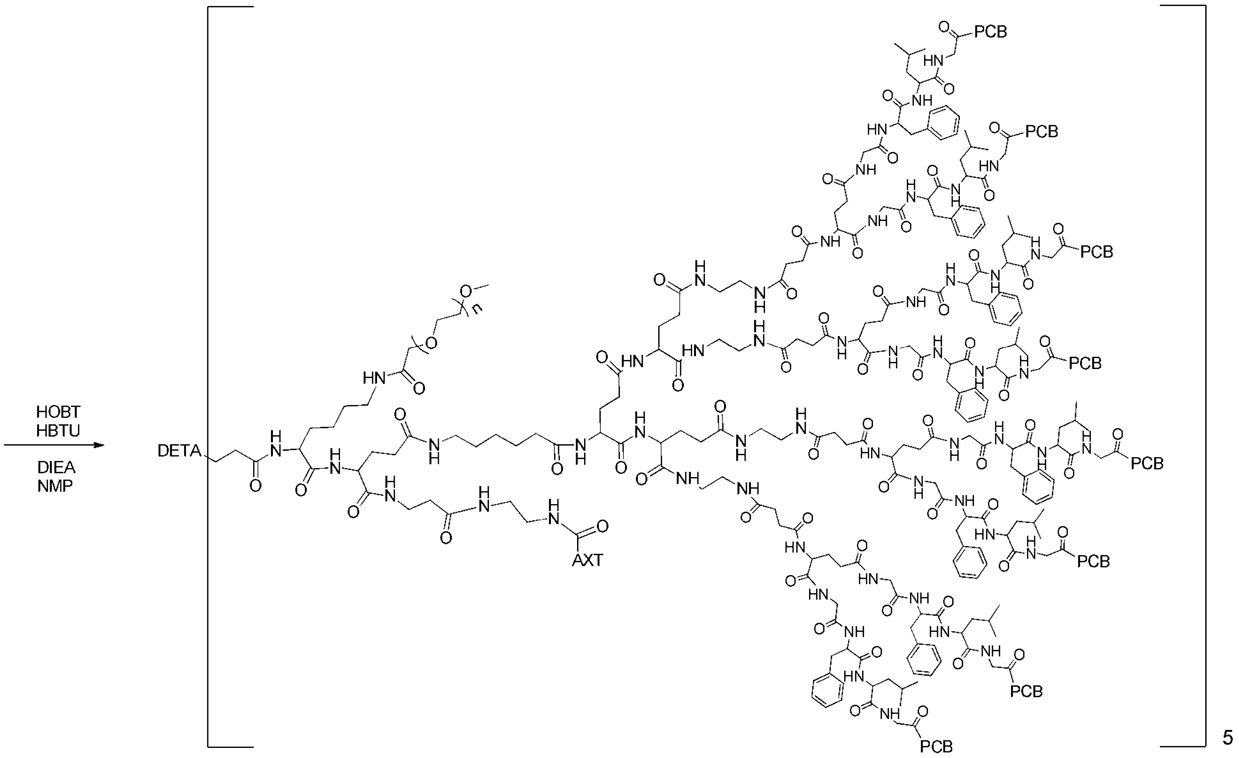


69-113

6.1 Preparation of **69-42**

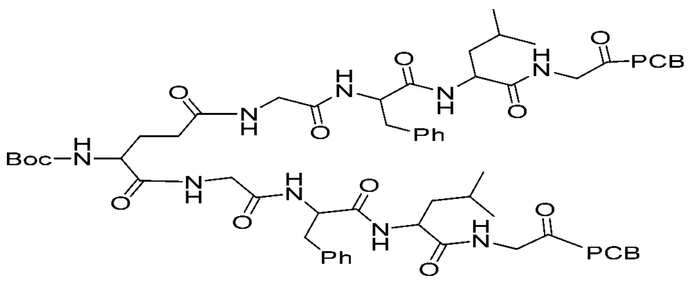

Boc-Glu-OH (0.683 g, 2.317 mmol, purchased from Ark Pharm) and **64-135** (5 g, 4.866 mmol) were added to a 500 mL flask, then DMF (30 mL) was added for dissolution, and stirred at room temperature for approximately 10 minutes. Then, **68-54** (1.606 g, 5.80 mmol) was added, and N-methylmorpholine (1.2 mL, 11.05 mmol, purchased from Aladdin) was slowly added dropwise. After the dropwise addition was completed, the mixture was stirred at room temperature for 3 hours. After the reaction was completed, methyl tert-butyl ether (150 mL) and n-hexane (100 mL) were added to the reaction mixture for precipitation. A solid product precipitated and was filtered and dried under vacuum to provide 3.9 g of the product.

6.2 Preparation of **69-73**

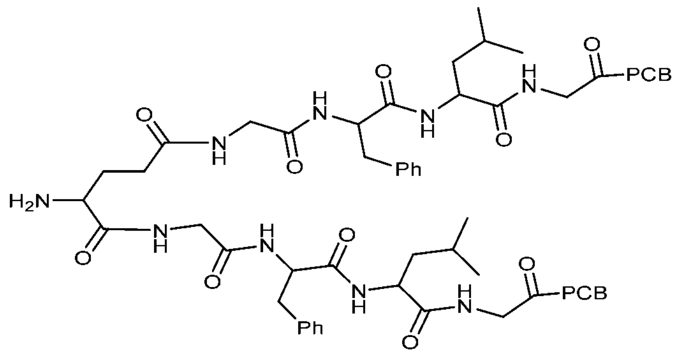

**69-42** (3.7 g, 1.994 mmol) was weighed and placed in a 250 mL round-bottom flask, then dichloromethane (5 mL) and trifluoroacetic acid (2.222 mL, 29.916 mmol) were added for dissolution, and the mixture was reacted with stirring at room temperature overnight. After the reaction was completed, the reaction mixture was concentrated under reduced pressure and evaporated to dryness. Dichloromethane (10 mL) and methanol (3 mL) were then added for dissolution. Methyl tert-butyl ether (150 mL) and n-hexane (100 mL) were added for precipitation. A solid product precipitated and was filtered and dried under vacuum to provide 3.5 g of the product.

6.3 Preparation of **69-74**

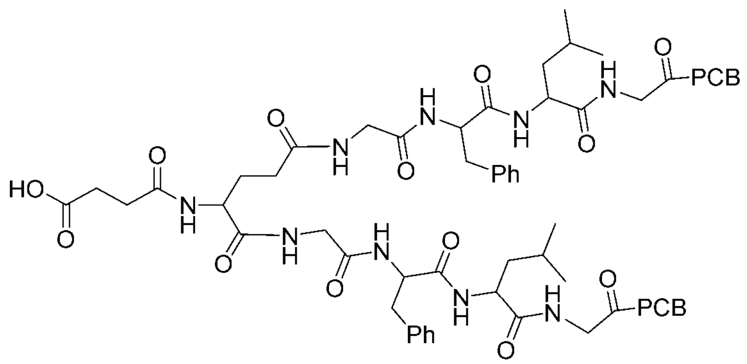

**69-73** (3.50 g, 1.99 mmol), DMF (20 mL), and DIEA (1.32 mL, 7.97 mmol) were placed in a 250 mL flask, and stirred at room temperature for 30 minutes. Then, succinic anhydride (0.598 g, 5.98 mmol, purchased from Innochem) was added, and the reaction was continued with stirring. After the reaction was completed, methyl tert-butyl ether (150 mL) and n-hexane (100 mL) were added to the reaction mixture for precipitation. A solid product precipitated and was filtered, and dried to provide 3.58 g of the product.

6.4 Preparation of **69-108**

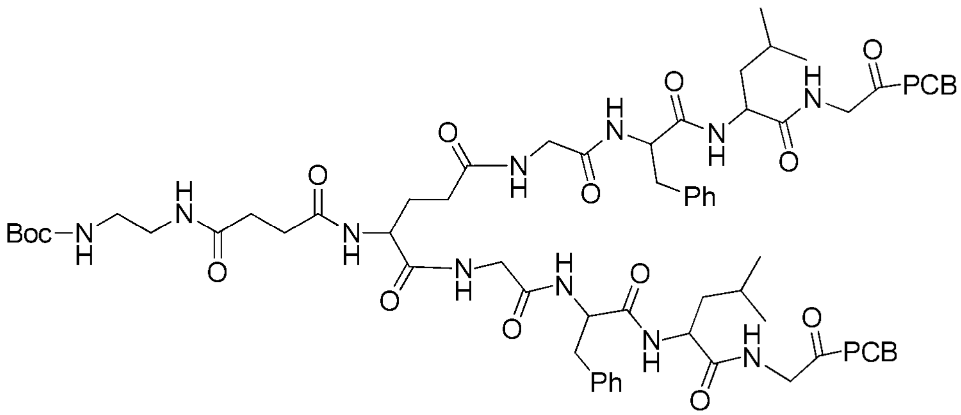

**69-74** (4.6 g, 2.48 mmol) and Boc-ethylenediamine (0.372 g, 2.361 mmol) were placed into a 250 mL flask, then NMP (30 mL) was added for dissolution with the assistance of ultrasonication, and the mixture was stirred at room temperature for 10 minutes. Then, **68-54** (0.563 g, 0.598 mmol) was added, and N-methylmorpholine (0.611 mL, 4.722 mmol, purchased from Aladdin) was slowly added dropwise. After the dropwise addition was completed, the reaction was continued with stirring at room temperature for 5 hours. The reaction progress was monitored by TLC. After the reaction was completed, methyl tert-butyl ether (100 mL) and n-hexane (200 mL) were added to the reaction mixture for precipitation. A solid product precipitated and was filtered and dried under vacuum to provide 4.98 g of the product.

6.5 Preparation of **69-109**

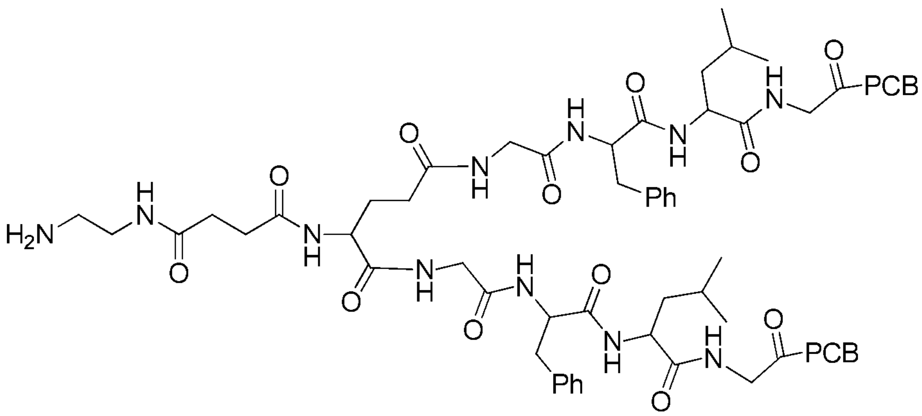

**69-108** (4.983 g, 2.48 mmol) was weighed and placed in a 250 mL round-bottom flask, then dichloromethane (5 mL) and TFA (2.787 mL, 37.2 mmol) were added for dissolution, and the mixture was reacted with stirring at room temperature overnight. After the reaction was completed, the reaction mixture was concentrated under reduced pressure and evaporated to dryness. Dichloromethane (10 mL) and methanol (3 mL) were then added for dissolution. Methyl tert-butyl ether (150 mL) and n-hexane (100 mL) were added for precipitation. A solid product precipitated and was filtered. The crude product was dissolved in a methanol/dichloromethane (1/4) mixture, then 100-200 mesh silica gel powder (20 g) was added, and the mixture was evaporated to dryness. The resulting product was dry-loaded onto the column and subjected to column chromatography using 1% aqueous ammonia/8% methanol/dichloromethane as the eluent. The desired product was collected, concentrated, and evaporated to dryness to provide 2.75 g of the product. $^1$H-NMR (600 MHz, DMSO-$d_6$) δ 10.41-9.88 (m, 2H), 9.07-8.78 (m, 2H), 8.25-8.11 (m, 4H), 8.11-8.04 (m, 2H), 8.04-7.98 (m, 2H), 7.93-7.80 (m, 6H), 7.56-7.46 (m, 2H), 7.31-7.07 (m, 10H), 5.89-5.77 (m, 2H), 4.59-4.52 (m, 2H), 4.40-4.31 (m, 2H), 4.18-4.09 (m, 1H), 4.08-3.94 (m, 4H), 3.76-3.52 (m, 13H), 3.24-2.99 (m, 14H), 2.88-2.70 (m, 2H), 2.67-2.57 (m, 1H), 2.44-2.41 (m, 6H), 2.40-2.32 (m, 4H), 2.32-2.29 (m, 6H), 2.28-2.18 (m, 4H), 2.18-2.10 (m, 2H), 1.95-1.83 (m, 5H), 1.83-1.68 (m, 5H), 1.67-1.44 (m, 10H), 0.92-0.79 (m, 12H)

6.6 Preparation of **69-57**

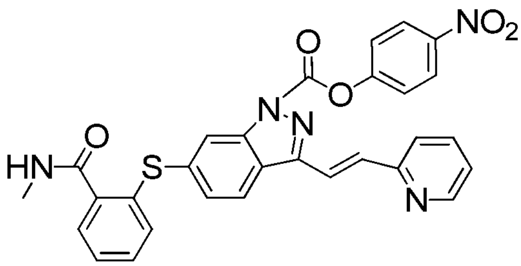

Axitinib (5 g, 12.938 mmol, abbreviated as AXT) and 4-nitrobenzene chloroformate (5.215 g, 25.875 mmol) were weighed and added to THF (approximately 500 mL), dissolved with the assistance of ultrasonication, and stirred in an oil bath under reflux at 75°C for 4 hours. After the reaction was completed, methyl tert-butyl ether (150 mL) and n-hexane (100 mL) were added to the reaction mixture for precipitation. A solid product precipitated, and was filtered and dried under vacuum to provide 7.13 g of the product.

6.7 Preparation of **69-58**

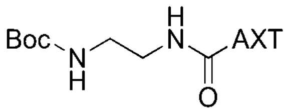

To **69-57** (7.136 g, 12.938 mmol) were added Boc-ethylenediamine (2.04 mL, 12.938 mmol, purchased from Aladdin) and triethylamine (5.4095 mL, 38.814 mmol). The mixture was dissolved in DMF (30 mL), and reacted with stirring at room temperature. After the reaction was completed, deionized water (200 mL) and ethyl acetate (200 mL) were added for extraction. After standing for phase separation, the organic phase was collected, and the aqueous phase was further extracted with ethyl acetate (200 mL × 3). The organic phases were combined, concentrated to a solid, which was dried in a vacuum oven to provide 7.4 g of the product.

6.8 Preparation of **69-59**

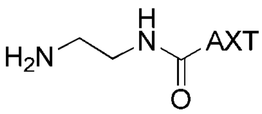

**69-58** (7.409 g, 12.938 mmol) was weighed, then dichloromethane (10 mL) and trifluoroacetic acid (9.61 mL, 129.38 mmol) were added, and the mixture was stirred at room temperature. After the reaction was completed, the reaction mixture was concentrated, and methyl tert-butyl ether (150 mL) and n-hexane (100 mL) were added for precipitation product. A solid precipitated and was filtered. The solid was dissolved in a methanol/dichloromethane (1/5) mixture, then 200-300 mesh silica gel powder (20 g) was added, and the mixture was evaporated to dryness. The resulting product was dry-loaded onto the column and subjected to column chromatography using 1% aqueous ammonia/5% methanol/dichloromethane as the eluent. The desired product was concentrated and evaporated to dryness to provide 5.1 g of the product. $^1$H-NMR (600 MHz, DMSO-$d_6$) δ 8.67-8.59 (m, 1H), 8.46-8.18 (m, 3H), 8.01-7.30 (m, 1H), 7.89-7.55 (m, 4H), 7.53-7.48 (m, 1H), 7.40-7.02 (m, 5H), 6.28-5.96 (m, 1H), 3.44-3.12 (m, 4H), 2.81-2.73 (m, 4H)

6.9 Preparation of **69-9**

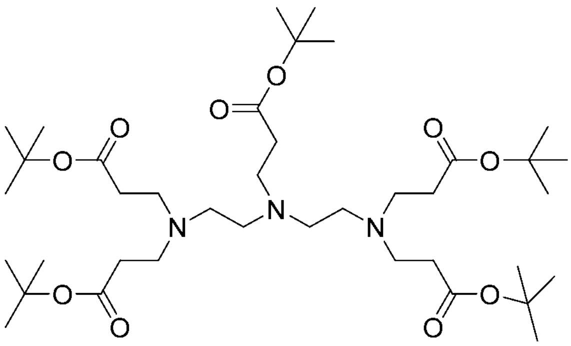

Diethylenetriamine (2 g, 19.385 mmol) was weighed and placed in a 500 mL flask, then methanol (20 mL) was added for dissolution with the assistance of ultrasonication. Tert-butyl acrylate (18.6 g, 145.38 mmol) was added dropwise. After the dropwise addition was completed, the flask was then taken out, and the reaction was carried out at room temperature in the dark for 24 hours. After the reaction was completed, methanol was removed by rotary evaporation. The reaction mixture was transferred to a 1 L separatory funnel containing deionized water (200 mL) and ethyl acetate (300 mL) for extraction. The organic phase was collected, and the aqueous phase was further extracted with ethyl acetate (100 mL). The organic phases were combined, washed with saturated brine (150 mL), concentrated, and evaporated to dryness. The crude solid product was dissolved in a methanol/dichloromethane (1/4) mixture, then 200-300 mesh silica gel powder (30 g) was added, and the mixture was evaporated to dryness. The resulting product was dry-loaded onto the column and subjected to column chromatography using 40% ethyl acetate/petroleum ether as the eluent. The desired product was collected, concentrated, and evaporated to dryness to provide 7.76 g of the product.

6.10 Preparation of **69-38**

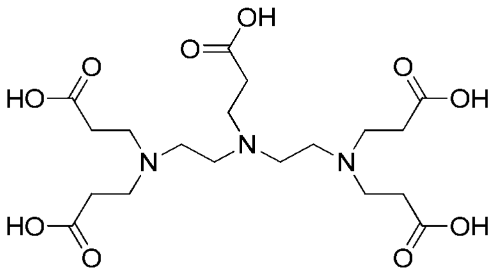

**69-9** (7.76 g, 12.98 mmol) was weighed, then dichloromethane (10 mL) was added for dissolution with the assistance of ultrasonication. Then trifluoroacetic acid (21.8 mL, 194.7 mmol) was added, and the mixture was reacted with stirring at room temperature. After the reaction was completed, the dichloromethane and most of the trifluoroacetic acid were removed by rotary evaporation. Dichloromethane (20 mL) was added for dissolution with the assistance of ultrasonication, and then the dichloromethane was removed by rotary evaporation. This process was repeated three times. Finally, the residue was dried to provide 4.86 g of the product. $^1H$-NMR (600 MHz, DMSO-$d_6$) δ 12.74-11.64 (m, 5H), 3.09-2.83 (m, 18H), 2.64-2.48 (m, 10H)

6.11 Preparation of Compound **69-54**

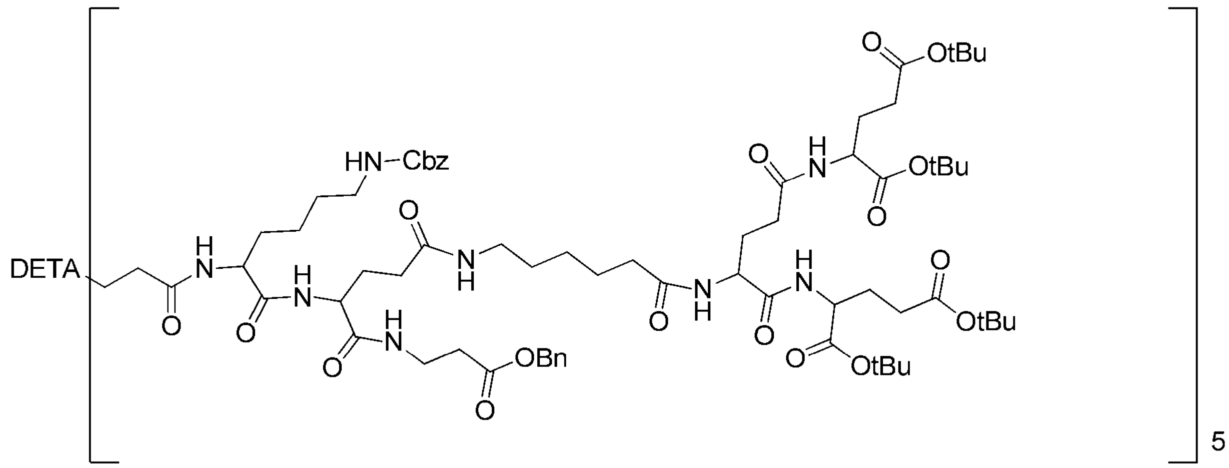

**69-45** (2.5 g, 1.929 mmol), **69-38** (0.12 g, 0.257 mmol), HBTU (0.732 g, 1.929 mmol), and HOBT (0.26 g, 1.929 mmol) were weighed and placed in a 500 mL flask, then DMF (15 mL) was added for dissolution with the assistance of ultrasonication, and the mixture was stirred at room temperature for 2 minutes. DIEA (0.85 mL, 5.14 mmol, purchased from Aladdin) was then slowly added dropwise. After the dropwise addition was completed, the reaction was carried out with stirring at room temperature for 24 hours. After the reaction was completed, methyl tert-butyl ether (100 mL) and n-hexane (200 mL) were added to the reaction mixture for precipitation. A solid product precipitated and was filtered. The obtained solid was dissolved with a methanol/dichloromethane (1/4) mixture (100 mL), then 200-300 mesh silica gel powder (35 g) was added, and the mixture was evaporated to dryness. The resulting product was dry-loaded onto the column and subjected to column chromatography using 3% methanol/dichloromethane as the eluent. The desired product was collected and concentrated to provide 1.58 g of the product. $^1$H-NMR (600 MHz, DMSO-$d_6$) δ 8.48-8.39 (m, 5H), 8.21-8.10 (m, 10H), 8.10-8.04 (m, 5H), 7.95-7.86 (m, 5H), 7.81-7.73 (m, 5H), 7.59-7.51 (m, 5H), 7.42-7.29 (m, 50H), 7.28-7.21 (m,5H), 5.11-4.97 (m, 20H), 4.30-4.24 (m, 5H), 4.24-4.17 (m, 5H), 4.15-4.07 (m, 10H), 4.06-3.97 (m, 5H), 3.41-3.24 (m, 10H), 3.09-2.69 (m, 44H), 2.57-2.46 (m, 10H), 2.32-2.06 (m, 43H), 2.06-1.98 (m, 11H), 1.97-1.85 (m, 16H), 1.85-1.62 (m, 37H), 1.51-1.14 (m, 227H)

6.12 Preparation of **69-60**

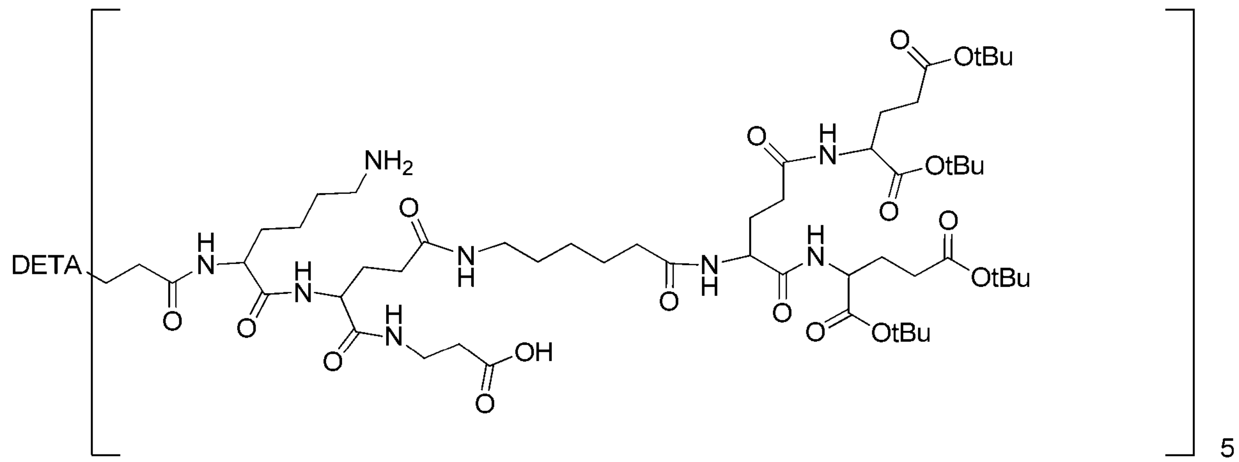

**69-54** (0.25 g, 0.0364 mmol) was placed in a hydrogenation reactor, 10% Pd/C catalyst (0.1 g) was added, and then DMF (4 mL) was added for dissolution. The hydrogenation reactor was sealed, evacuated with a water pump, and then filled with hydrogen gas. This process was repeated three times. Finally, the pressure reading of the hydrogenation reactor was adjusted to 2 MPa. The reaction was carried out at room temperature overnight. After the reaction was completed, the reaction mixture was filtered through Celite, and the Celite was rinsed with DMF (3 mL × 3). The DMF solutions were combined and used as the raw material for the next reaction.

6.13 Preparation of **69-61**

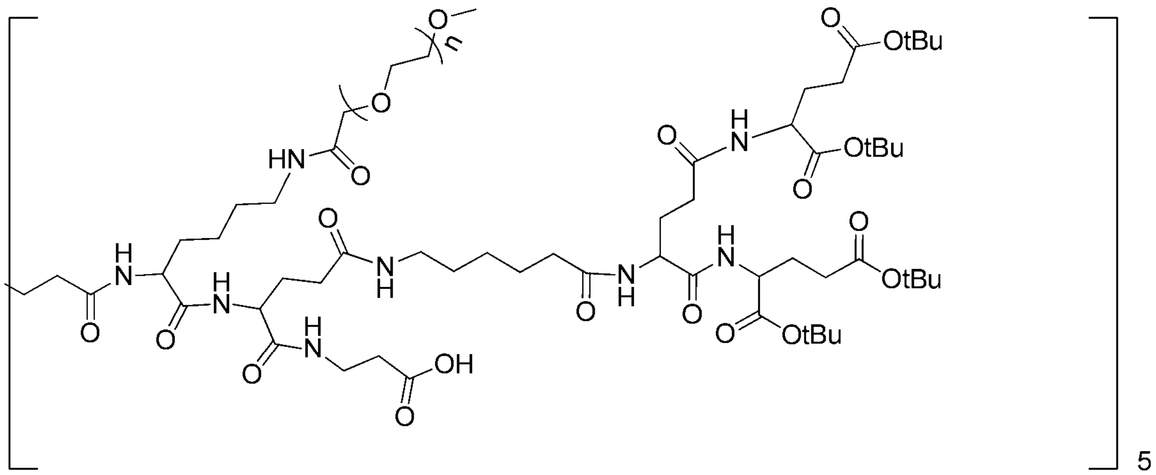

**69-60** (0.208 g, 0.0364 mmol) was weighed and added into a 50 mL round-bottom flask, and then dry DMF was added for dissolution. The mixture was stirred at -5°C for 2 minutes. DIEA (0.146 mL, 0.887 mol) was slowly added dropwise. The reaction was carried out with stirring for another 2 minutes, then M-SCM-5K (1.034 g, 0.182 mmol, purchased from Jenkem, Lot Number: ZZ363P139) was added, and the reaction was continued with stirring at low speed in the dark at room temperature for 72 hours. After the reaction was completed, methyl tert-butyl ether (500 mL) was added for precipitation. A solid precipitated and was filtered. The filter cake was dried to provide 0.86 g of the product. $^1$H-NMR (600 MHz, DMSO-$d_6$) δ 12.05-11.89 (m, 5H), 8.47-8.35 (m, 5H), 8.22-8.04 (m, 20H), 7.95-7.86 (m, 5H), 7.81-7.72 (m, 5H), 7.59-7.51 (m, 5H), 4.30-4.24 (m, 5H), 4.24-4.17 (m, 5H), 4.15-4.07 (m, 10H), 4.06-3.96 (m, 5H), 3.60-3.24 (m, 2350H), 3.11-2.68 (m, 44H), 2.57-2.46 (m, 10H), 2.32-2.06 (m, 43H), 2.06-1.98 (m, 11H), 1.97-1.62 (m, 53H), 1.51-1.12 (m, 227H)

6.14 Preparation of **69-99**

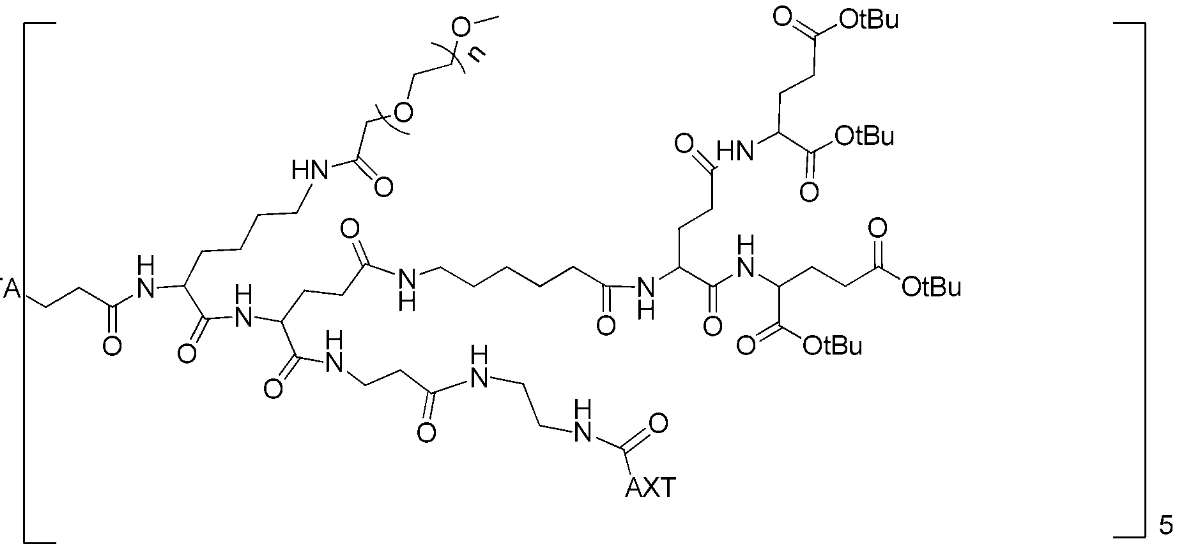

**69-61** (2.48 g, 0.088 mmol), **69-59** (0.2295 g, 0.4856 mmol), HBTU (0.184 g, 0.4856 mmol), and HOBT (0.065 g, 0.4856 mmol) were weighed and placed in a 500 mL flask, then DMF (30 mL) was added for dissolution with the assistance of ultrasonication, and the mixture was stirred at room temperature for 10 minutes. DIEA (0.327 mL, 1.98 mmol, purchased from Aladdin) was slowly added dropwise. After the dropwise addition was completed, the reaction was carried out with stirring at room temperature for 5 hours. The reaction progress was monitored by TLC. After the reaction was completed,

methyl tert-butyl ether (100 mL) and n-hexane (200 mL) were added to the reaction mixture for precipitation. A solid product precipitated and was filtered. The filter cake was added to a methanol/dichloromethane (1/1) mixture (200 mL) for dissolution with the assistance of ultrasonication, then 100-200 mesh silica gel powder (10 g) was added, and the mixture was evaporated to dryness. The resulting product was dry-loaded onto the column and subjected to column chromatography using 1% ammonia/6% methanol/dichloromethane as the eluent. The desired product was collected and concentrated to provide 2.41 g of the product.

6.15 Preparation of **69-101**

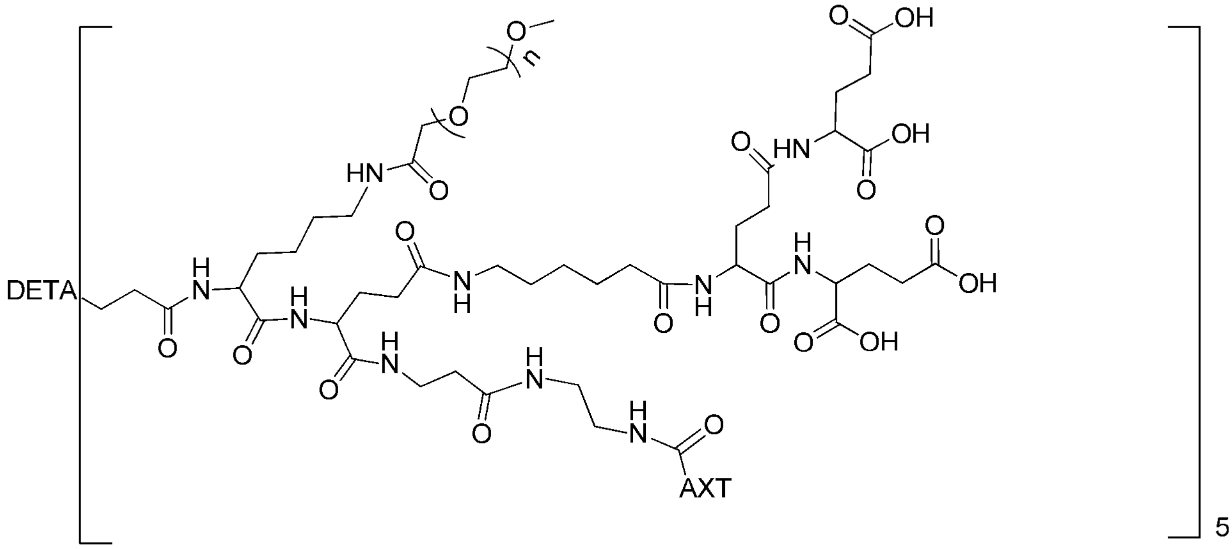

**69-99** (2.41 g, 0.072 mmol) was weighed. Dichloromethane (5 mL) was added, then trifluoroacetic acid (5 mL, 67 mmol) was added, and the mixture was stirred at room temperature. After the reaction was completed, the reaction mixture was concentrated, and methyl tert-butyl ether (150 mL) and n-hexane (100 mL) were added for precipitation. A solid product precipitated and was filtered. The filter cake was dried to provide 2.05 g of the product.

6.16 Preparation of **69-113**

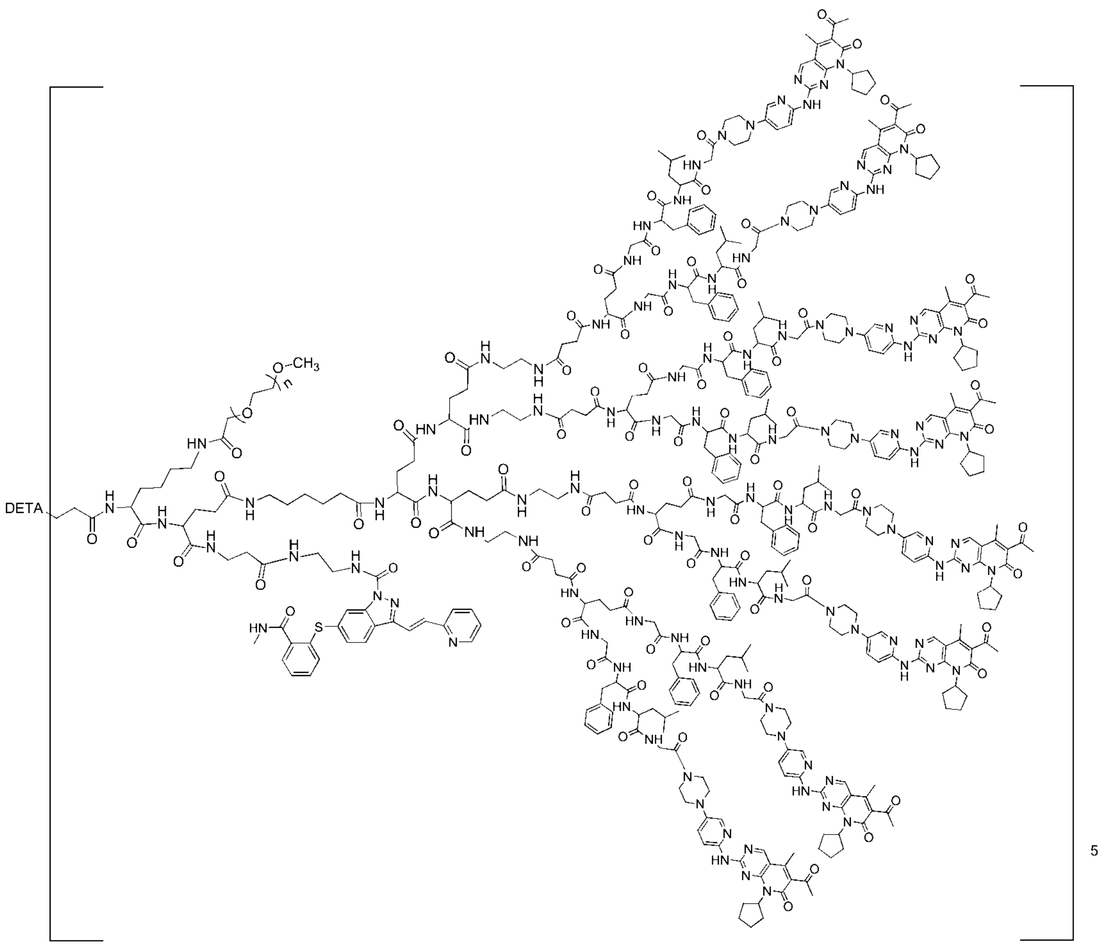

**69-101** (0.239 g, 0.00714 mmol), **69-109** (0.322 g, 0.171 mmol), HBTU (0.064 g, 0.171 mmol), and HOBT (0.023 g, 0.171 mmol) were weighed and placed in a 500 mL flask, then NMP (15 mL) was added for dissolution with the assistance of ultrasonication, and the mixture was stirred at room temperature for 10 minutes. DIEA (0.1061 mL, 0.641 mmol, purchased from Aladdin) was slowly added dropwise. The progress of the reaction was monitored by TLC. After the reaction was completed, methyl tert-butyl ether (100 mL) and n-hexane (200 mL) were added to the reaction mixture for precipitation. A solid precipitated and was filtered. A methanol/dichloromethane (1/1) mixture (200 mL) was added to the filter cake for dissolution with the assistance ultrasonication, then 100-200 mesh silica gel powder (10 g) was added, and the mixture was evaporated to dryness. The resulting product was dry-loaded onto the column and subjected to column chromatography using 1% ammonia water/10% methanol/dichloromethane as the eluent. The desired product was collected, concentrated, and dried under vacuum to provide 0.32 g of the product. $^1$H-NMR (600 MHz, DMSO-*d*$_6$) $\delta$ 10.45-9.86 (m, 40H), 9.07-8.78 (m, 40H), 8.67-8.59 (m, 5H), 8.47-8.30 (m, 10H), 8.25-7.86 (m, 316H), 7.81-7.48 (m, 77H), 7.44-7.07 (m,225H), 6.28-5.96 (m, 5H), 5.89-5.77 (m, 40H), 4.59-4.52 (m, 40H), 4.40-4.24 (m, 45H), 4.24-4.07 (m, 35H), 4.07-3.94 (m, 76H), 3.76-3.24 (m, 2630H), 3.24-2.68 (m, 375H), 2.68-2.46 (m, 30H), 2.44-2.41 (m, 120H), 2.40-2.32 (m, 80H), 2.32-2.06 (m, 283H), 2.06-1.62 (m, 264H), 1.62-1.12 (m, 247H), 0.92-0.79 (m, 240H)

Example 7: Synthesis of Compound **71-246**

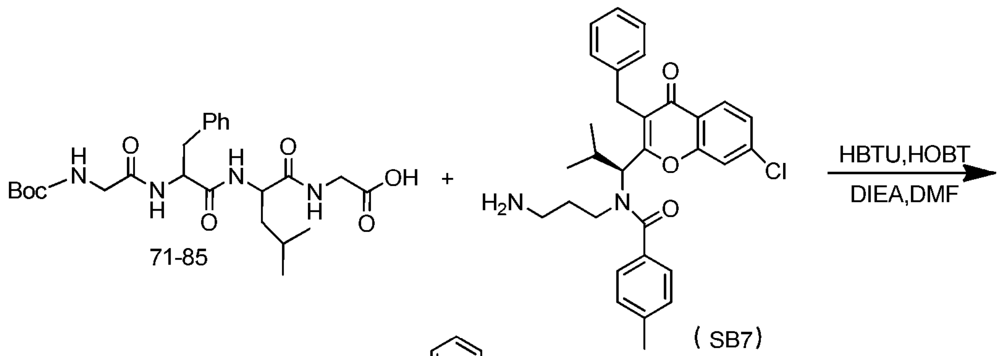


71-85

(SB7)

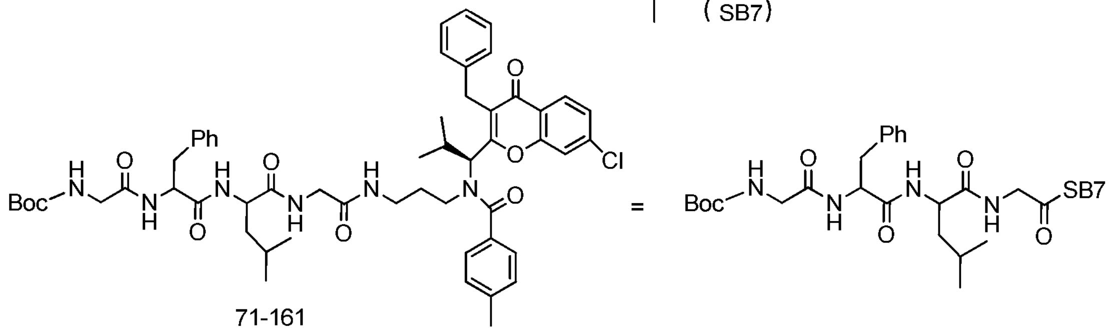

71-161

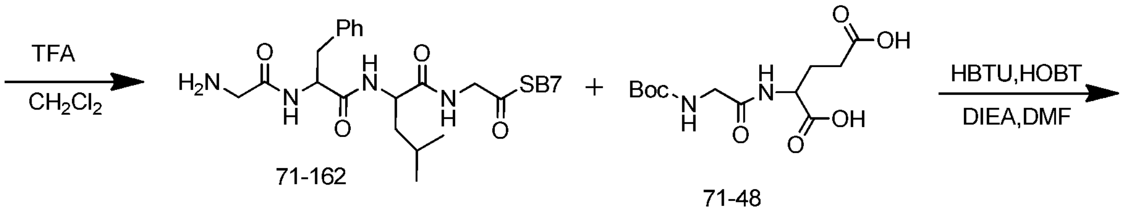


71-162

71-48

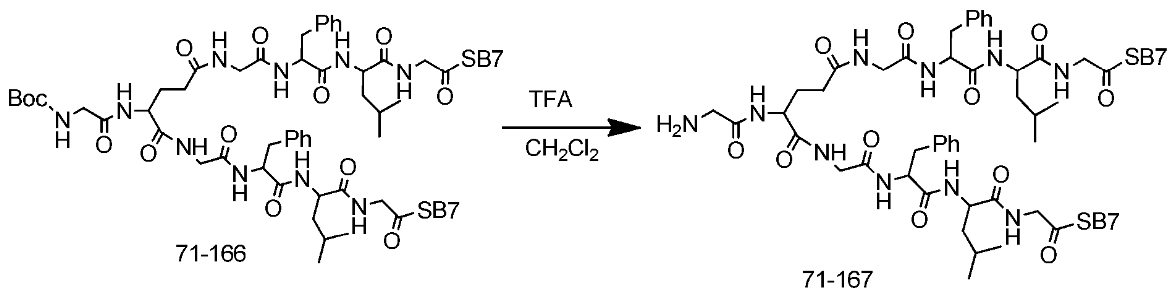


71-166

71-167

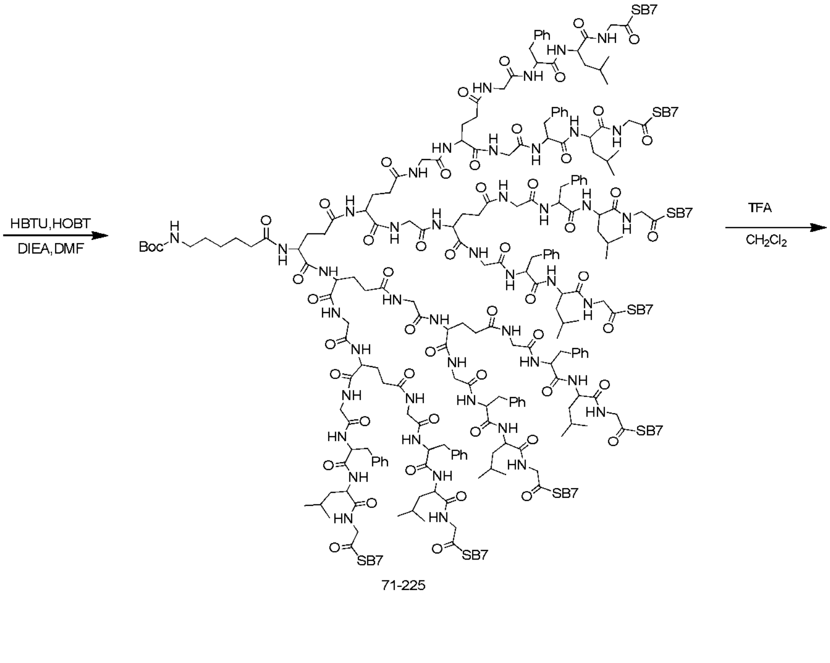


62-156 + 71-167

71-225

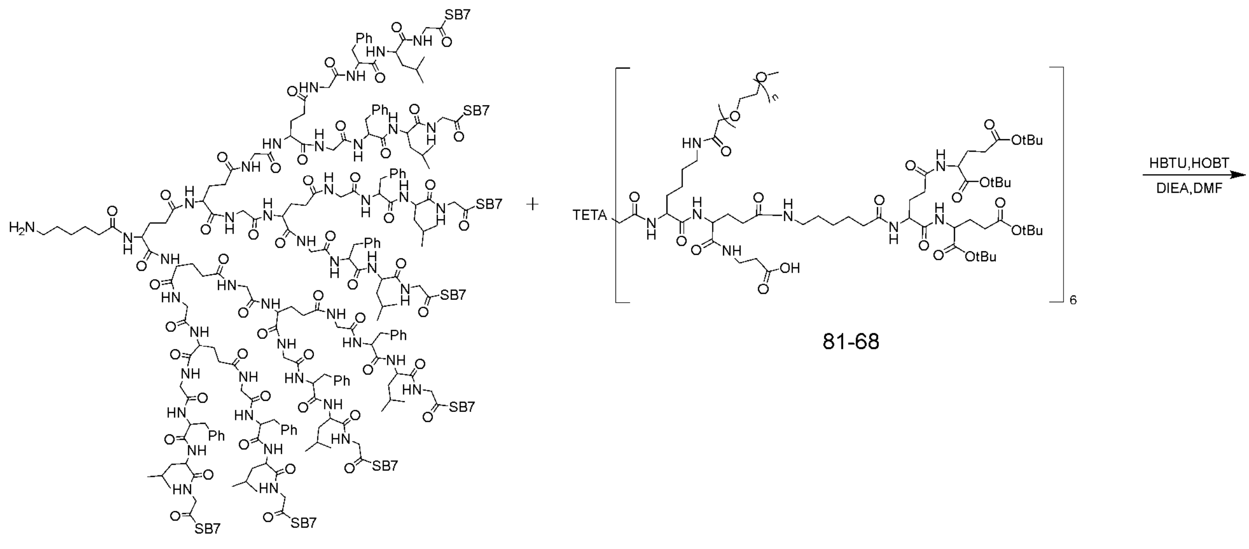


71-226

81-68

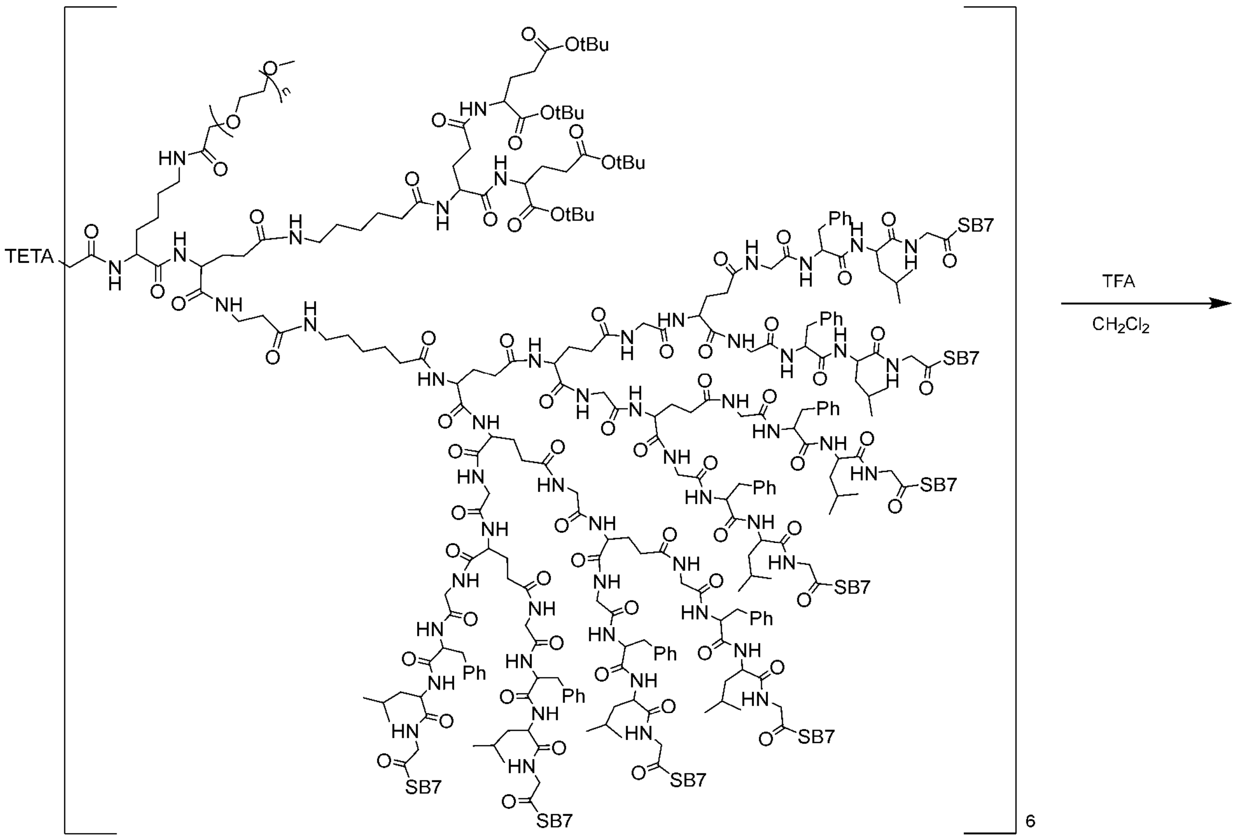


71-228

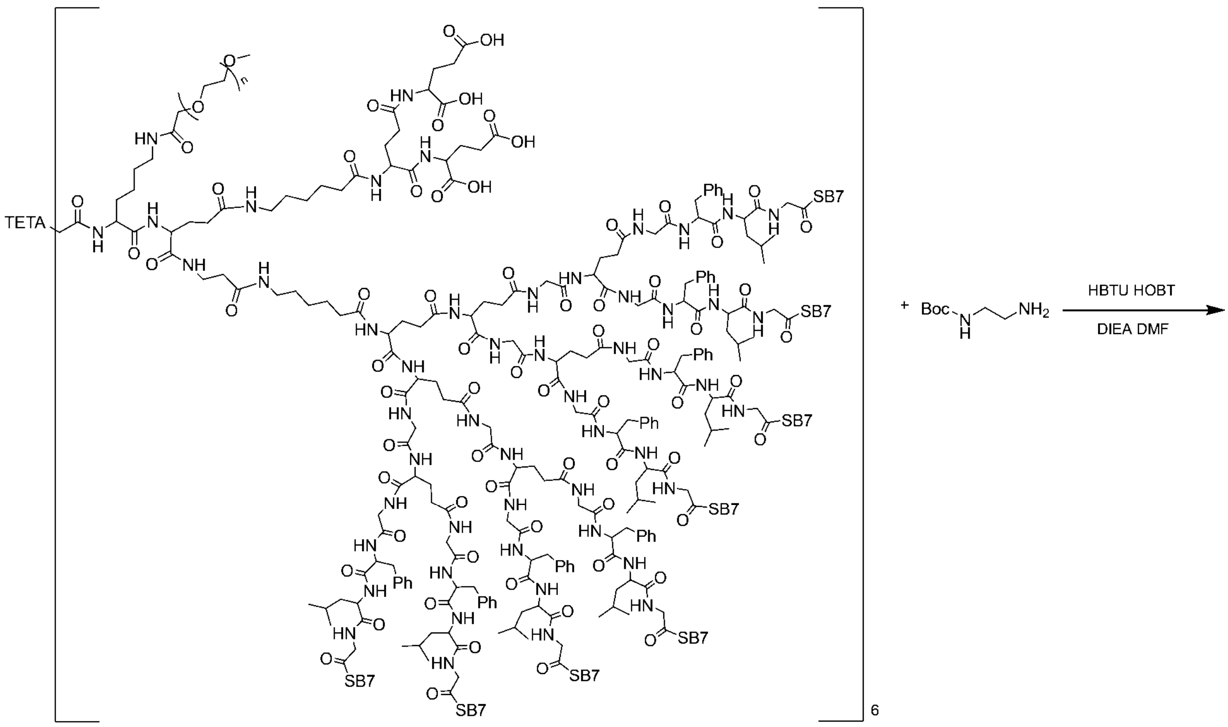


71-230

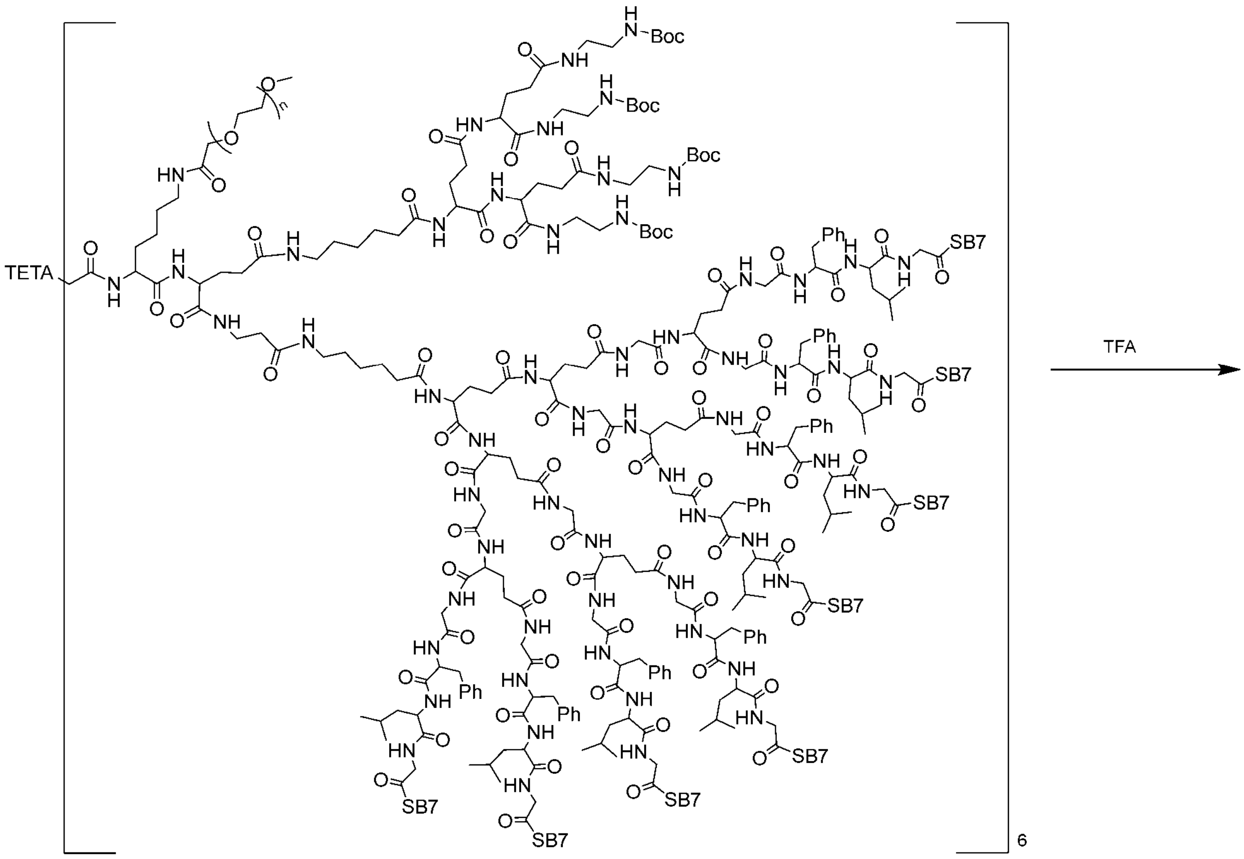


71-231

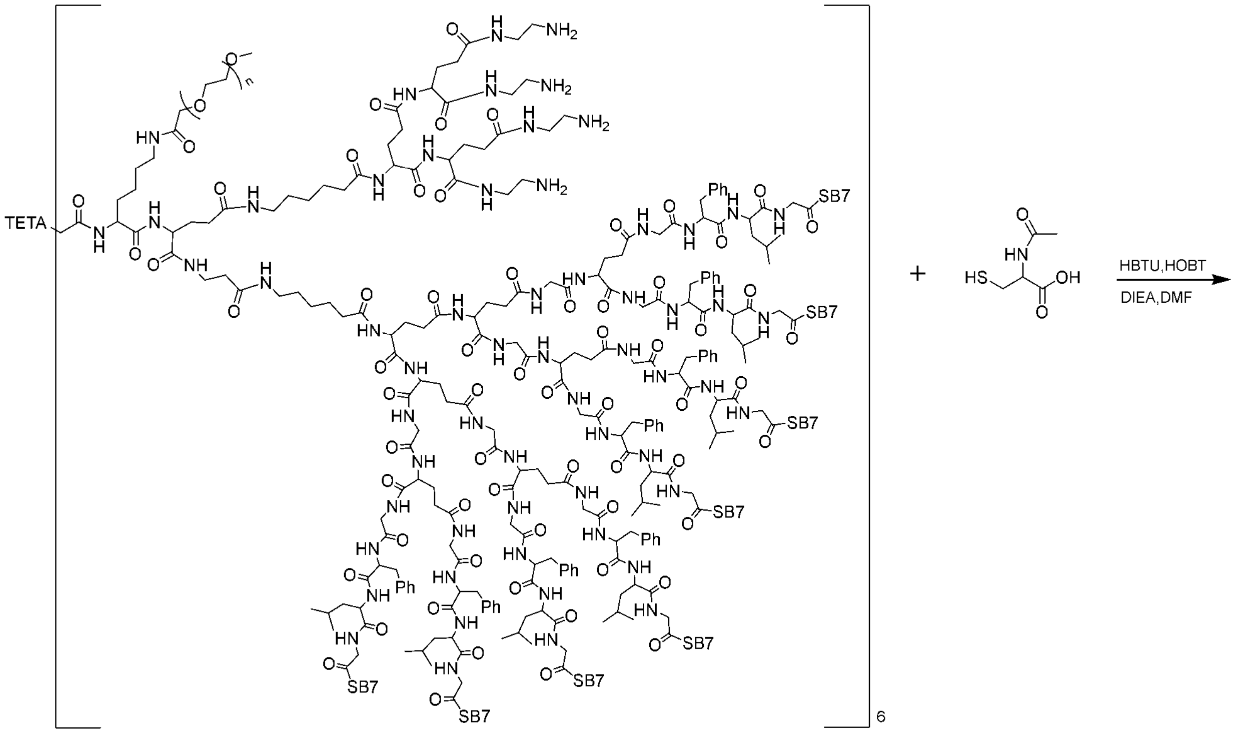


71-240

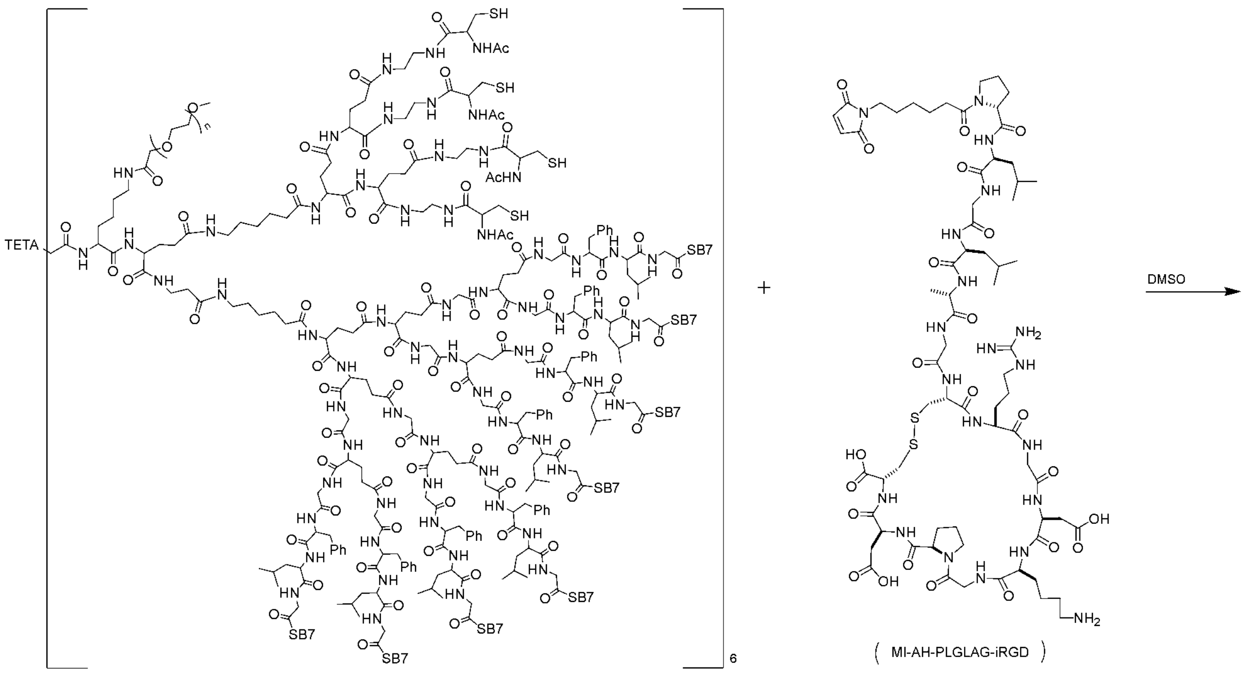


( MI-AH-PLGLAG-iRGD )

71-241

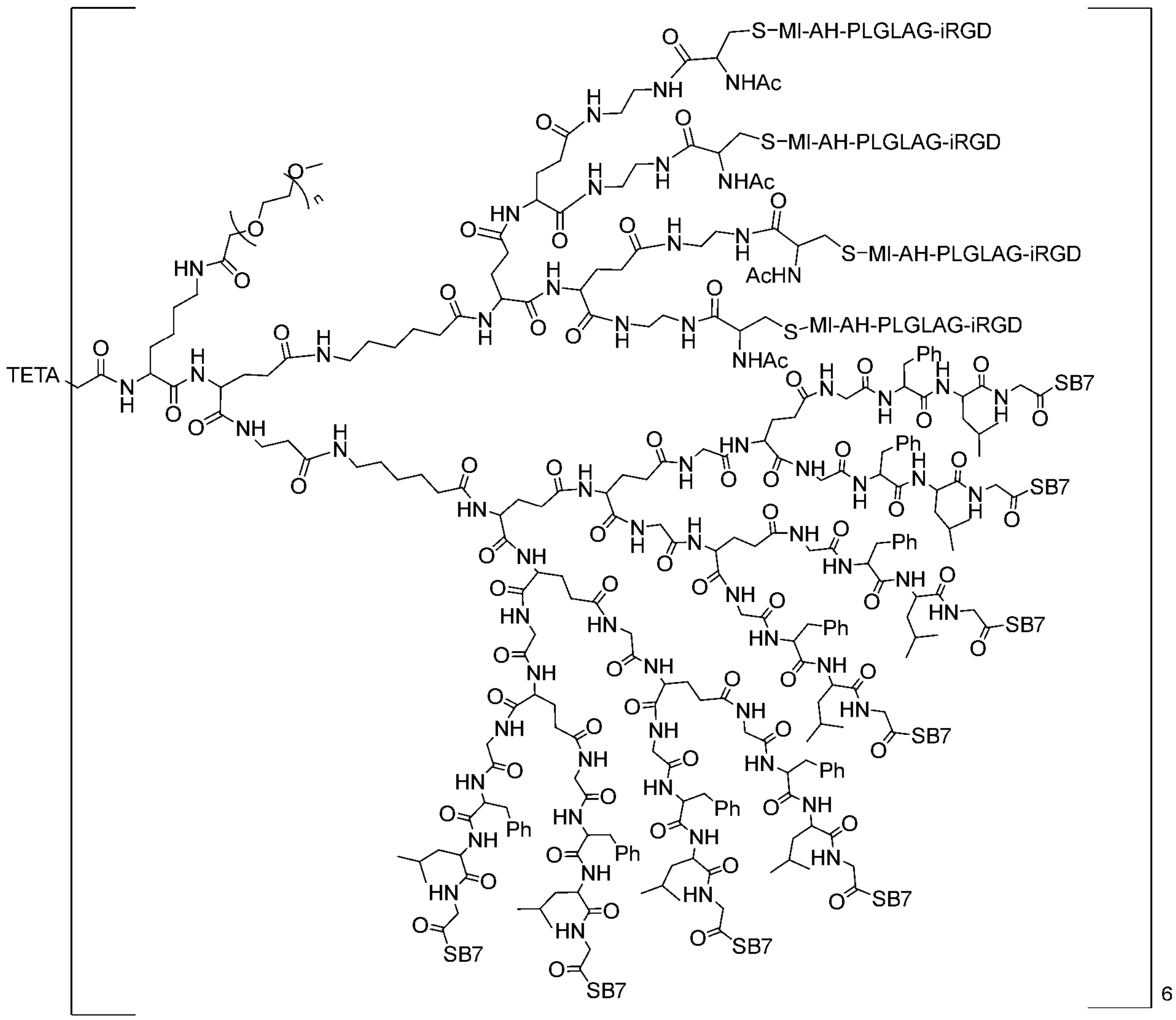

71-246

7.1 Preparation of **71-161**

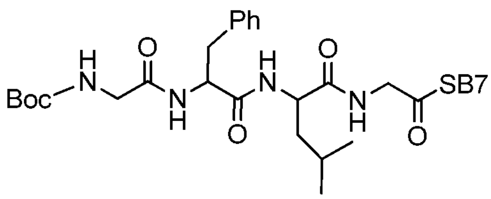

**71-85** (4.7631 g, 9.6699 mmol), SB-743921 (5.0 g, 9.6699 mmol, abbreviated as SB7, purchased from Tianjin Pharmacn), HBTU (5.5008 g, 14.5049 mmol), and HOBT (1.9599 g, 14.5049 mmol) were added to a 500 mL round-bottom flask. DMF (60 mL) was added for dissolution, and the mixture was stirred at -5°C for approximately 20 minutes. DIEA (7.2 mL, 43.5145 mmol) was then slowly added dropwise. The reaction was continued at -5°C with stirring for 1 hour. The mixture was then brought to room temperature, and the reaction was carried out with stirring. After the reaction was completed, the reaction mixture was transferred to a 1 L separatory funnel containing saturated brine (250 mL) and ethyl acetate (200 mL) for extraction. The organic phase was collected, and the aqueous phase was further extracted with ethyl acetate (200 mL). The organic phases were combined, washed with deionized water (200 mL), concentrated, and evaporated to dryness. A methanol/dichloromethane (1/4) mixture was added to the crude product for dissolution, then silica gel powder was added, and the mixture was evaporated to dryness. The resulting product was dry-loaded onto the

column and subjected to column chromatography using 1-4% methanol/dichloromethane as the eluent. The desired product was collected, concentrated, and dried to provide 8.62 g of the product with a yield of 90.0%.

7.2 Preparation of **71-162**

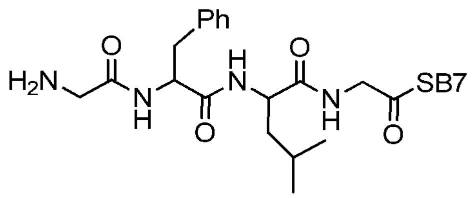

To a flask containing **71-161** (8.62 g, 8.6929 mmol), dichloromethane (20 mL) was added for dissolution with the assistance of ultrasonication, then TFA (6.5 mL, 86.929 mmol) was added, and the mixture was reacted with stirring at room temperature overnight. After the reaction was completed, the reaction mixture was concentrated and evaporated to dryness to remove the dichloromethane and most of the TFA. Methyl tert-butyl ether (300 mL) was added for precipitation. A solid product precipitated and was filtered. The filter cake was washed with methyl tert-butyl ether (40 mL $\times$ 3). The filter cake was collected and dried under vacuum to provide 7.74 g of the product.

7.3 Preparation of **71-166**

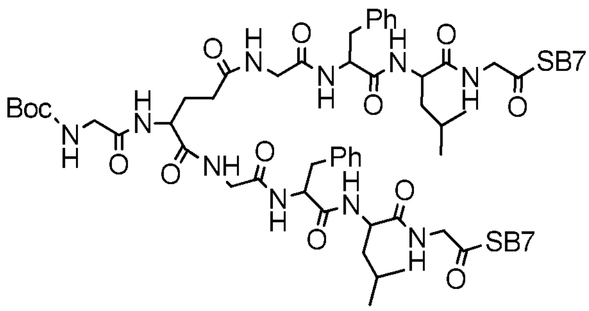

**71-48** (1.2 g, 3.9513 mmol), **71-162** (7.74 g, 8.6929 mmol), HBTU (3.59 g, 9.4831 mmol), and HOBT (1.28 g, 9.4831 mmol) were placed in a 500 mL solution, then DMF (30 mL) was added to dissolve the mixture, and the mixture was stirred at -5°C for approximately 20 minutes. DIEA (6.5 mL, 39.513 mmol) was then slowly added dropwise. The reaction was continued at -5°C for 1 hour, and then the mixture was brought to room temperature and the reaction was carried out with stirring. After the reaction was completed, n-hexane (250 mL) and methyl tert-butyl ether (70 mL) were added for precipitation. The supernatant was discarded. This process was repeated five times. The mixture was filtered to provide a solid product, which was dissolved in a dichloromethane/methanol (4/1) mixture, then 200-300 mesh silica gel powder was added, and the mixture was evaporated to dryness. The resulting product was dry-loaded onto the column and subjected to column chromatography using a 2-5% methanol/dichloromethane mixture as the eluent. The desired product was collected, concentrated, and dried in a vacuum oven to provide 8.10 g of the product. $^1$H-NMR (600 MHz, DMSO-$d_6$) $\delta$ 8.15-8.04 (m, 12H), 8.00-7.93 (m, 4H), 7.70-7.65 (m, 2H), 7.26-7.20 (m, 28H), 4.53-4.51 (m, 2H), 4.27-4.19 (m, 5H), 3.66-3.55 (m, 10H), 3.53-3.47 (m, 4H), 3.44-3.39 (m, 4H), 2.34-2.28 (m, 8H), 2.17-2.15 (m, 4H), 1.90-1.80 (m, 2H), 1.75-1.67 (m, 2H), 1.61-1.42 (m, 14H), 1.34 (s, 9H), 0.89-0.79 (m, 24H)

7.4 Preparation of **71-167**

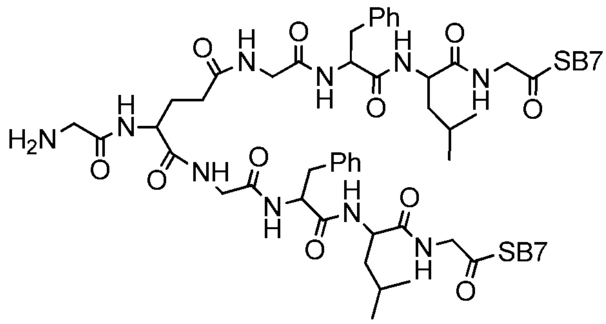

To a flask containing **71-166** (8.1 g, 3.9513 mmol), dichloromethane (15 mL) was added for dissolution with the assistance of ultrasonication, then TFA (2.9 mL, 39.513 mmol) was added, and the mixture was reacted with stirring at room temperature overnight. After the reaction was completed, the reaction mixture was concentrated and evaporated to dryness to remove dichloromethane and most of the TFA. Methyl tert-butyl ether (300 mL) was added for precipitation. A solid product precipitated and was filtered. The filter cake was washed with methyl tert-butyl ether (40 mL × 3). The filter cake was collected and dried under vacuum to provide 7.71 g of the product.

7.5 Preparation of **71-225**

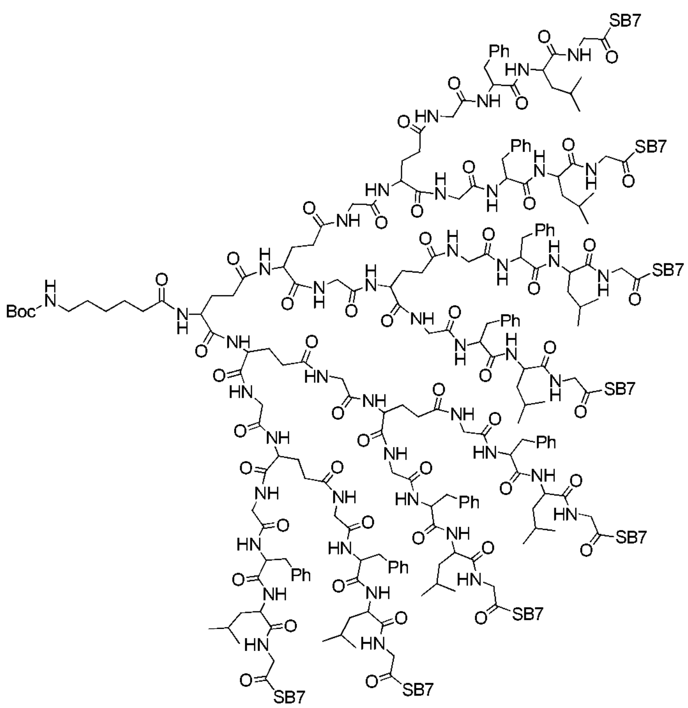

**62-156** (0.3159 g, 0.5107 mmol), **71-167** (4.3843 g, 2.2471 mmol), HBTU (1.1621 g, 3.0642 mmol), and HOBT (0.4140 g, 3.0642 mmol) were added to a 500 mL round-bottom flask, then DMF (70 mL) was added for dissolution, and the mixture was stirred at -5°C for approximately 20 minutes. DIEA (1.0 mL, 6.1284 mmol) was then slowly added dropwise. After the dropwise addition was completed, the reaction flask was allowed to stand at -5°C, and the reaction was carried out with stirring for 1 hour. Finally, the flask was brought to room temperature, and the mixture was stirred. After the reaction was completed, n-hexane (30 mL) and methyl tert-butyl ether (200 mL) were added for precipitation. The supernatant was discarded. This process was repeated three times. The mixture was subjected to suction filtration to provide a solid product, which was dissolved in a dichloromethane/methanol (4/1) mixture, and subjected to column chromatography using a 4-5% methanol/dichloromethane mixture as the eluent. The desired product was collected, evaporated to dryness, and dried in a vacuum oven to provide 3.8 g of the product with a yield of 89.1%.

7.6 Preparation of **71-226**

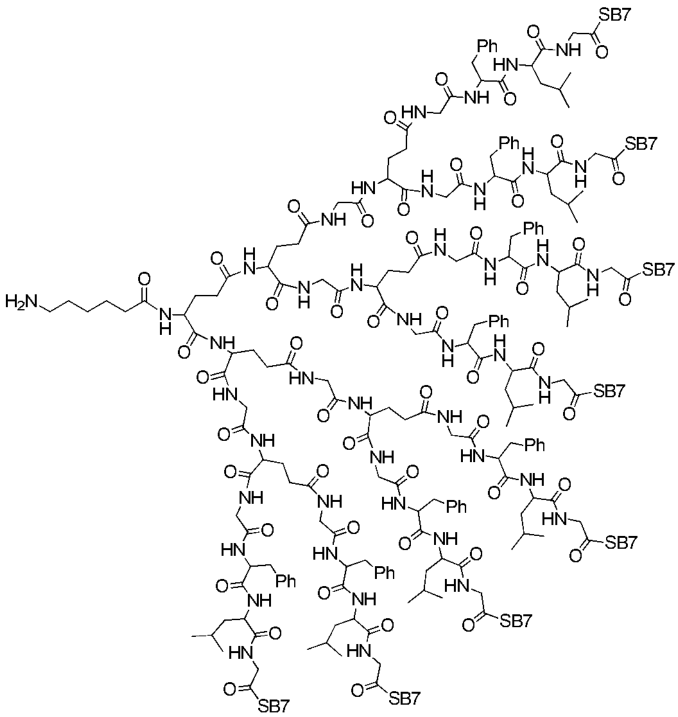

To a flask containing **71-225** (1.0 g, 0.1197 mmol), dichloromethane (5 mL) was added for dissolution with the assistance of ultrasonication, then TFA (0.2 mL, 2.394 mmol) was added, and the mixture was reacted with stirring at room temperature overnight. After the reaction was completed, the reaction mixture was concentrated and evaporated to dryness to remove the dichloromethane and most of the TFA. Methyl tert-butyl ether (300 mL) was added for precipitation. A solid product precipitated and was filtered. The filter cake was washed with methyl tert-butyl ether (40 mL × 3). The filter cake was collected and dried under vacuum to provide 0.9 g of the product. $^1$H-NMR (600 MHz, DMSO-$d_6$) δ 8.18-8.06 (m, 52 H), 7.81-7.80 (m, 8 H), 7.54-7.49 (m, 16 H), 7.24-7.20 (m, 112 H), 4.54-4.53 (m, 8 H), 4.25-4.14 (m, 24 H), 3.90-3.88 (m, 10H), 3.75-3.61 (m, 32 H), 3.55-3.38 (m, 32 H), 3.01-2.99 (m, 10H), 2.76-2.75 (m, 12H), 2.39-2.27 (m, 32 H), 2.21-2.06 (m, 20H), 1.89-1.68 (m, 18H), 1.55-1.46 (m, 34H), 1.36-1.23 (m, 14H), 0.91-0.80 (m, 96H)

7.7 Preparation of **71-228**

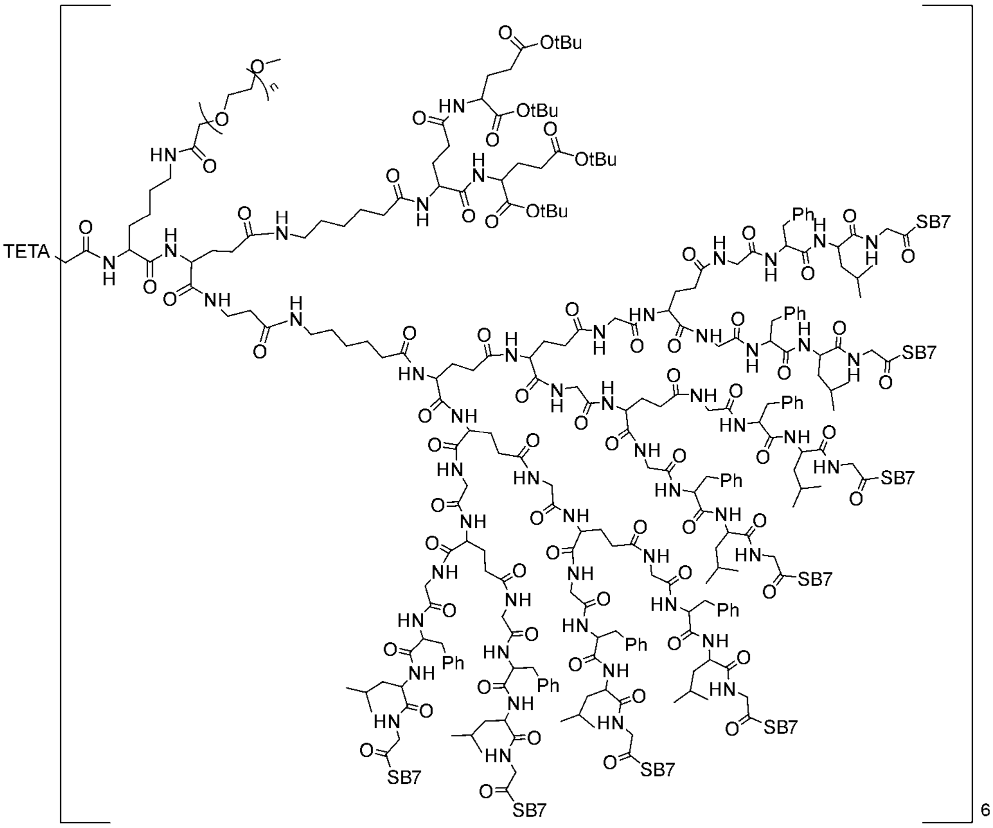

**81-68** (0.15 g, 0.004 mmol), **71-226** (0.3 g, 0.0363 mmol), HBTU (0.0137 g, 0.0363 mmol), and HOBT (0.0049 g, 0.0363 mmol) were placed in a 250 mL solution, then DMF (20 mL) was added, and the mixture was stirred at room temperature for approximately 20 minutes. DIEA (0.1 mL, 0.552 mmol) was then slowly added dropwise. After the dropwise addition was completed, the reaction was continued with stirring at room temperature. After the reaction was completed, n-hexane (250 mL) and methyl tert-butyl ether (70 mL) were added for precipitation. The supernatant was discarded. This process was repeated five times. The mixture was subjected to suction filtration to provide a solid product. The obtained solid product was dissolved in a dichloromethane/methanol (4/1) mixture, dry-loaded onto the column and subjected to column chromatography using a 1% aqueous ammonia solution and a 4-7% methanol/dichloromethane mixture as the eluents. The desired product was collected, concentrated, and dried in a vacuum oven to provide 0.22 g of the product with a yield of 64.7%. $^1$H-NMR (600 MHz, DMSO-*d$_6$*) δ 8.16-8.06 (m, 312H), 7.83-7.785 (m, 48H), 7.54-7.49 (m, 96H), 7.24-7.20 (m, 672H), 4.55-4.53 (m, 68H), 4.25-4.09 (m, 226H), 3.90-3.86 (m, 90H), 3.66-3.63 (m, 148H), 3.53-3.49 (m, 2802H), 3.44-3.42 (m, 24H), 3.40-3.38 (m, 52H), 3.01-2.99 (m, 106H), 2.83-2.69 (m, 200H), 2.66-2.57 (m, 90H), 2.31-2.30 (m, 150H), 2.18-2.10 (m, 140H), 1.91-1.63 (m, 198H), 1.60-1.52 (m, 84H), 1.49-1.47 (m, 164H), 1.387-1.363 (s, 216H), 1.24-1.23 (m, 60H), 0.91-0.8 (m, 576H)

7.8 Preparation of **71-230**

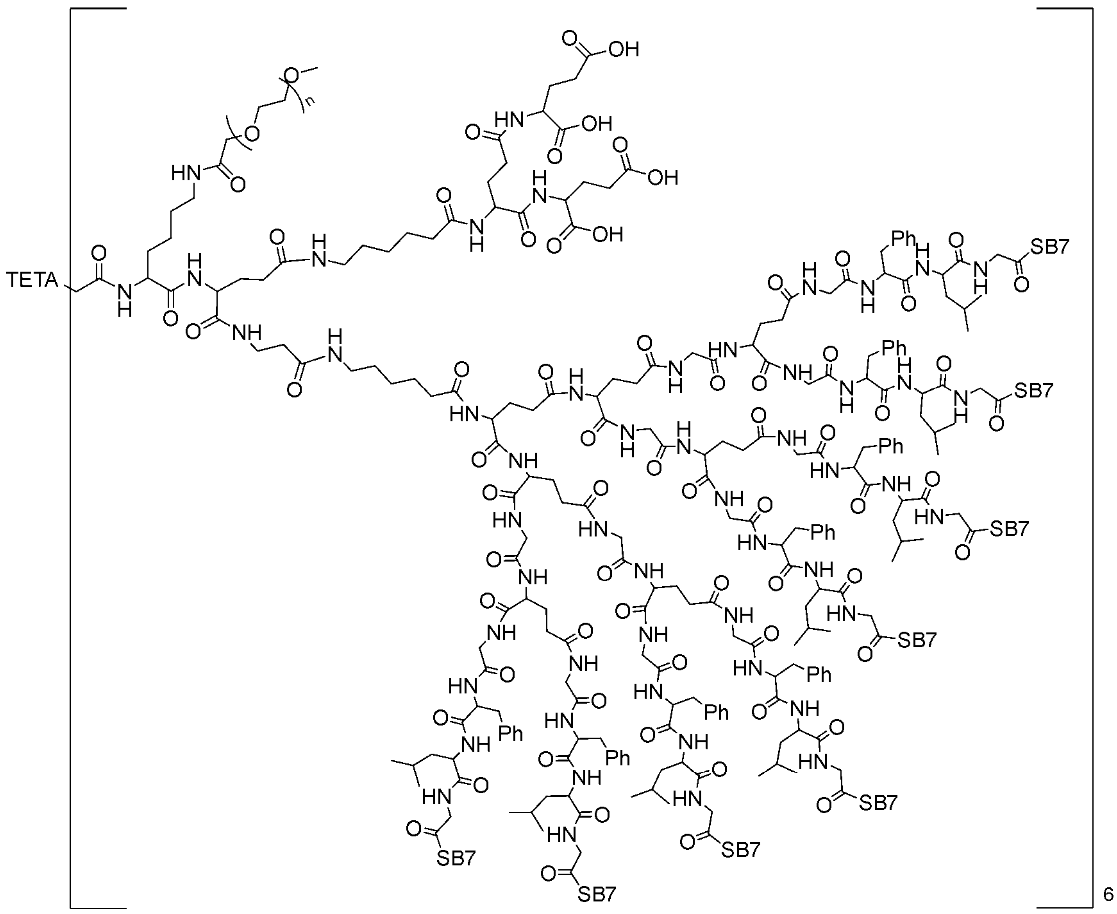

To a flask containing **71-228** (0.22 g, 0.0025 mmol), TFA (10 mL) was added for dissolution with the assistance of ultrasonication, and reacted with stirring in a water bath at 37°C overnight. After the reaction was completed, dichloromethane was added to the reaction mixture, which was then concentrated and evaporated to dryness to remove dichloromethane and most of the TFA. Methyl tert-butyl ether (300 mL) was added for precipitation. A solid product precipitated and was filtered. The filter cake was washed with methyl tert-butyl ether (40 mL $\times$ 8). The filter cake was collected and dried under vacuum to provide 0.2 g of the product. $^{1}$H-NMR (600 MHz, DMSO-$d_6$) $\delta$ 10.01-9.89 (m, 24H), 8.18-8.05 (m, 312H), 7.82-7.81 (m, 48H), 7.54-7.49 (m, 96H), 7.24-7.29 (m, 672H), 4.54-4.53 (m, 68H), 4.24-4.14 (m, 226H), 3.90-3.85 (m, 90H), 3.66-3.62 (m, 224H), 3.520-3.495 (m, 2802H), 3.03-2.97 (m, 106H), 2.84-2.72 (m, 200H), 2.64-2.61 (m, 90H), 2.33-2.27 (s, 150H), 2.18-2.10 (m, 140H), 1.93-1.67 (m, 198H), 1.58-1.51 (m, 84H), 1.50-1.43 (m, 164H), 1.17-1.11 (m, 60H), 0.91-0.80 (m, 576H)

7.9 Preparation of **71-231**

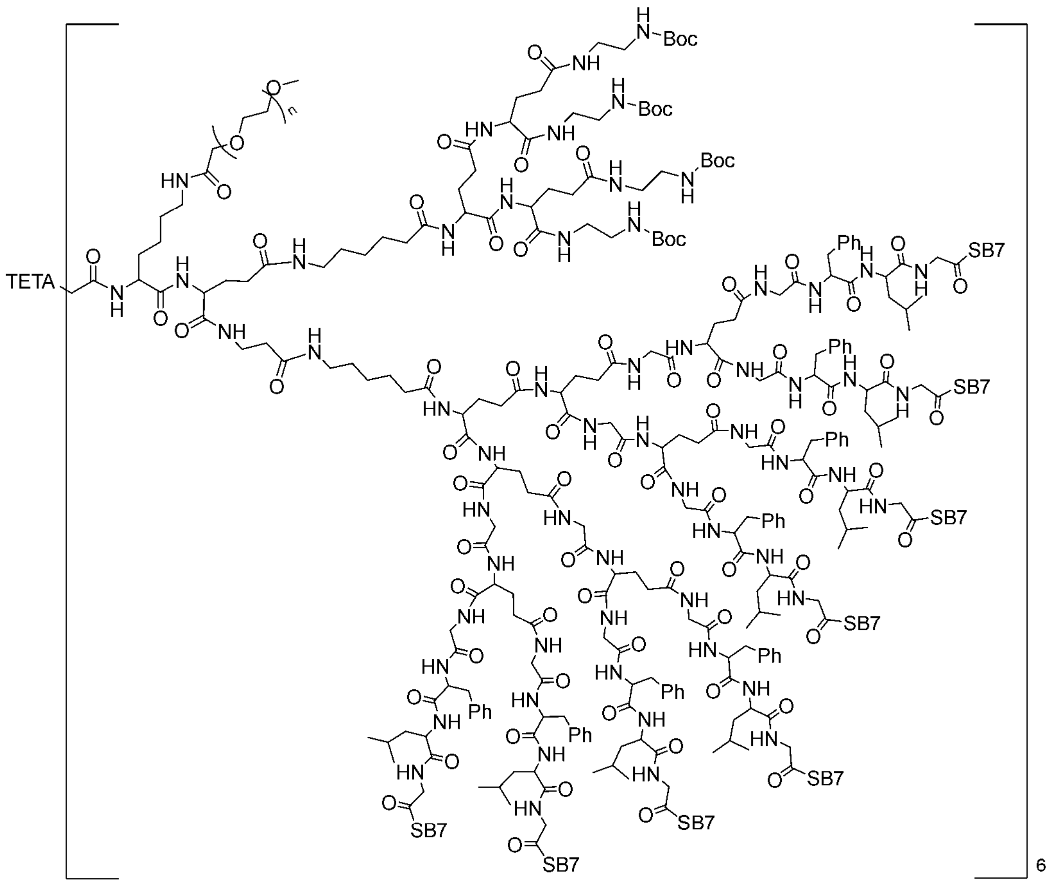

**71-230** (0.2 g, 0.0023 mmol), N-Boc-ethylenediamine (0.0132 g, 0.0828 mmol), HBTU (0.031 g, 0.0828 mmol), and HOBT (0.011 g, 0.0828 mmol) were added to a 250 mL round-bottom flask, then DMF (40 mL) was added for dissolution, and the mixture was stirred at room temperature for approximately 20 minutes. DIEA (0.1 mL, 0.6 mmol) was then slowly added dropwise. After the dropwise addition was completed, the reaction flask was allowed to stand at room temperature, and the reaction was carried out with stirring. After the reaction was completed, n-hexane (100 mL) and methyl tert-butyl ether (40 mL) were added for precipitation, and the supernatant was discarded. This process was repeated four times. The mixture was filtered to provide a solid product. The obtained solid product was dissolved in a dichloromethane/methanol (4/1) mixture, then silica gel powder was added, and the mixture was evaporated to dryness. The resulting product was dry-loaded onto the column and subjected to column chromatography using a mixture of 1% aqueous ammonia and 5-7% methanol/dichloromethane as the eluent. The desired product was collected, concentrated, and dried to provide 0.10 g of the product with a yield of 50.0%.

7.10 Preparation of **71-240**

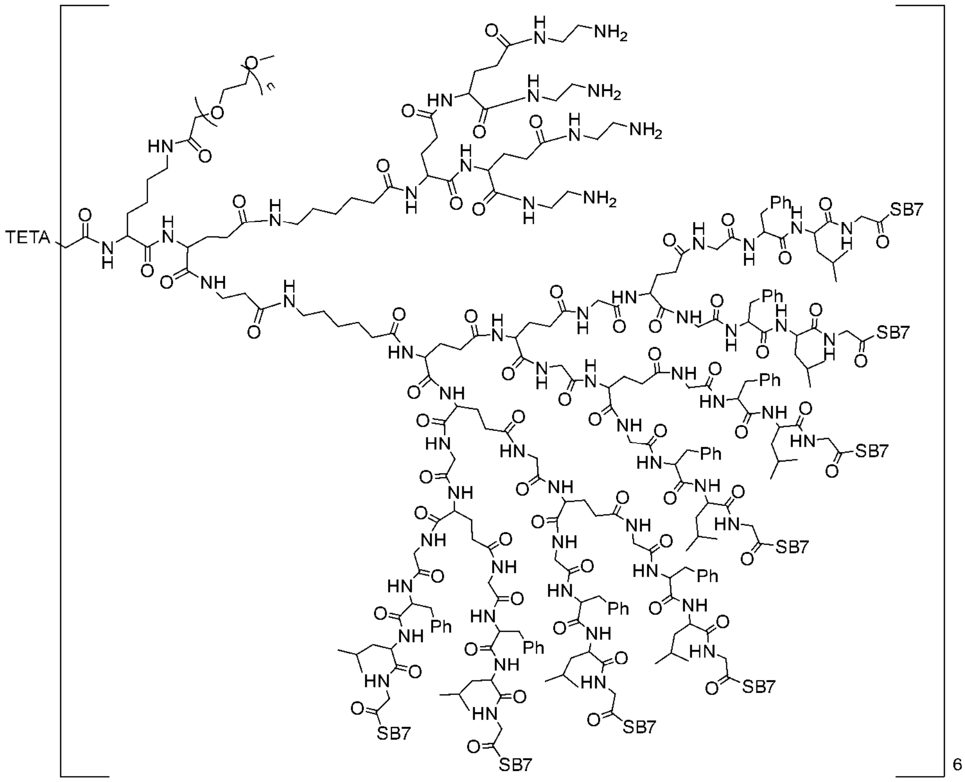

To a flask containing **71-231** (0.1 g, 0.0011 mmol), TFA (0.4 mL, 5.28 mmol) was added for dissolution with the assistance of ultrasonication, and reacted with stirring in a water bath at 35°C overnight. After the reaction was completed, the reaction mixture was concentrated and evaporated to dryness, dissolved in DMF (5 mL), and neutralized by adding excess DIEA. Methyl tert-butyl ether (100 mL) and n-hexane (100 mL) were added for precipitation, and the supernatant was discarded. This process was repeated twice. The mixture was filtered. The filter cake was washed with methyl tert-butyl ether (40 mL × 8). The filter cake was collected and dried under vacuum to provide 0.097 g of the product.

7.11 Preparation of **71-241**

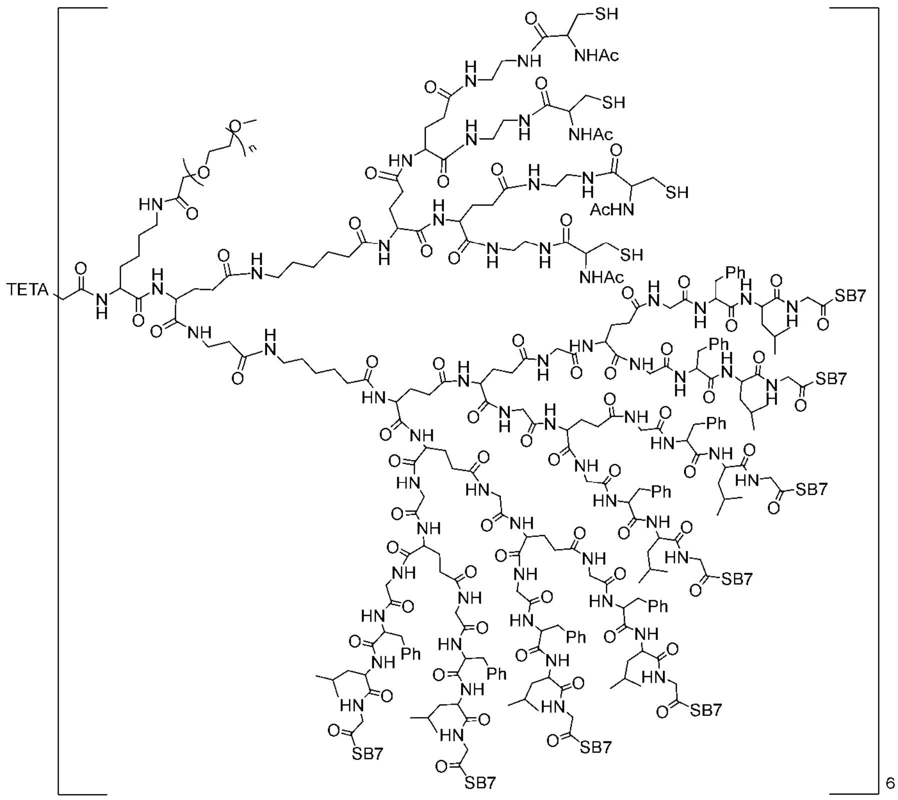

**71-240** (0.097 g, 0.0011 mmol), N-acetyl-L-cysteine (0.0064 g, 0.0396 mmol, purchased from Psaitong), HBTU (0.015 g, 0.0396 mmol), and HOBT (0.005 g, 0.0396 mmol) were added to a 250 mL round-bottom flask, then DMF (3 mL) was added for dissolution, and the mixture was stirred at room temperature for approximately 20 minutes. DIEA (0.1 mL, 0.65 mmol) was then slowly added dropwise. After the dropwise addition was completed, the mixture was stirred continuously at room temperature. After the reaction was completed, n-hexane and methyl tert-butyl ether were added for precipitation, a viscous solid was obtained, and the supernatant was discarded. The crude product was evaporated to dryness by rotary evaporation, dissolved in a dichloromethane/methanol (4/1) mixture, then 100-200 mesh silica gel powder was added, and the mixture was evaporated to dryness. The resulting product was dry-loaded onto the column and subjected to column chromatography using a mixture of 1% aqueous ammonia/5-8% methanol/dichloromethane as the eluent. The desired product was collected, concentrated, and dried to provide 0.09 g of the product.

7.12 Preparation of **71-246**

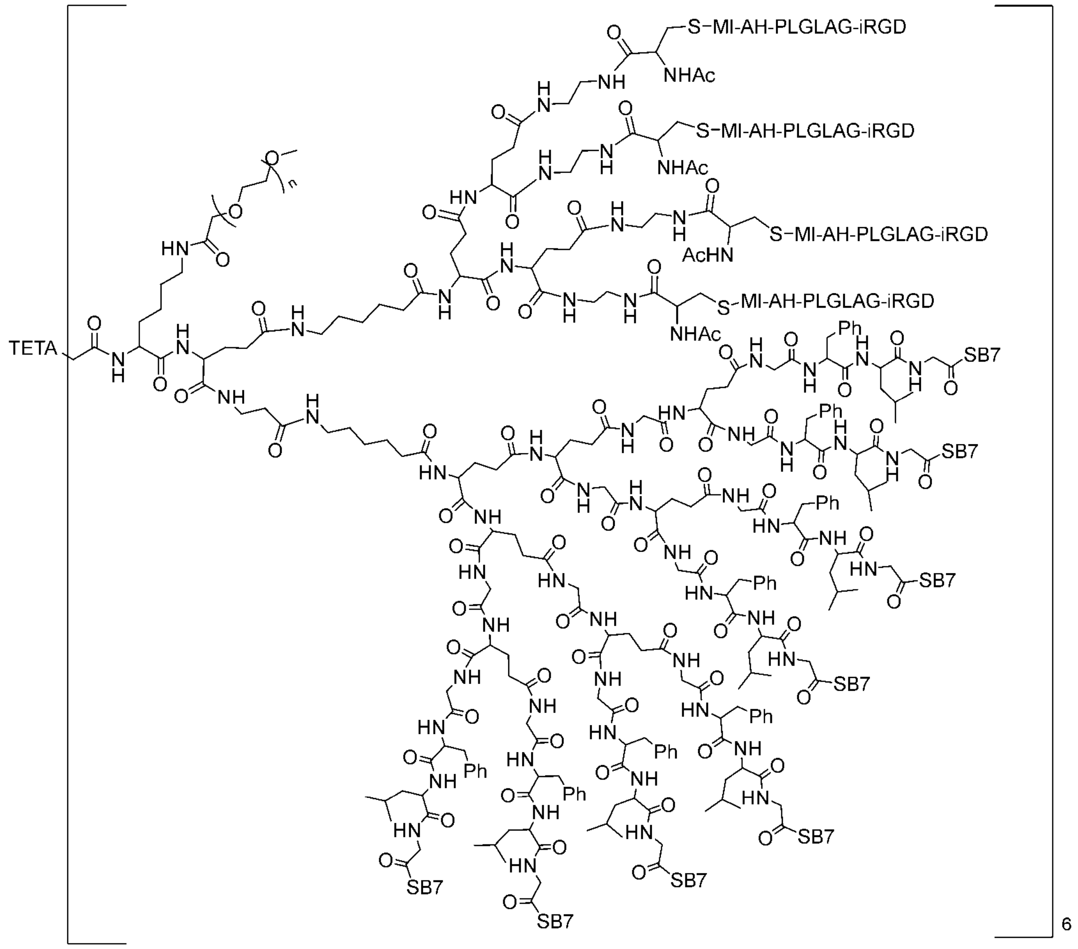

**71-241** (0.9 g, 0.0011 mmol) and MI-AH-PLGLAG-iRGD (0.052 g, 0.0316 mmol, purchased from DGpeptides) were added to a 500 mL flask, then an appropriate amount of DMSO was added for dissolution, and the mixture was reacted with stirring at room temperature overnight. After the reaction was completed, n-hexane (30 mL) and methyl tert-butyl ether (100 mL) were added for precipitation, and the supernatant was discarded. This process was repeated seven times. The mixture was filtered to provide a solid product. The obtained solid product was dissolved in a dichloromethane/methanol (4/1) mixture, then 100-200 mesh silica gel powder was added, and the mixture was evaporated to dryness. The resulting product was dry-loaded onto the column and subjected to column chromatography using a 1% ammonia solution/5-9% methanol/dichloromethane mixture as the eluent. The desired product was collected, concentrated, and dried in a vacuum oven to provide 0.04 g of the product. $^1$H-NMR (600 MHz, DMSO-$d_6$) δ 11.43-10.91 (m, 72H), 8.92-8.68 (m, 48H), 8.29-8.01 (m, 750H), 7.84-7.80 (m, 180H), 7.37-7.05 (m, 636H), 4.62-4.49 (m, 210H), 4.39-4.14 (m, 672H), 3.79-3.71 (m, 184H), 3.69-3.66 (m, 140H), 3.62-3.61 (m, 108H), 3.54-3.47 (m, 2802H), 3.01-2.97 (m, δ (m, 222H), 2.91-2.87 (m, 176H), 2.80-2.75 (m, 236H), 2.32-2.22 (m, 224H), 2.18-2.07 (m, 206H), 2.02-1.99 (m, 64H), 1.91-1.84 (m, 380H), 1.79-1.70 (m, 280H), 1.64-1.56 (m, 254H), 1.48-1.46 (m, 370H), 1.20-1.28 (m, 156H), 0.87-0.82 (m, 864H)

Example 8: Synthesis of Compound **70-92**

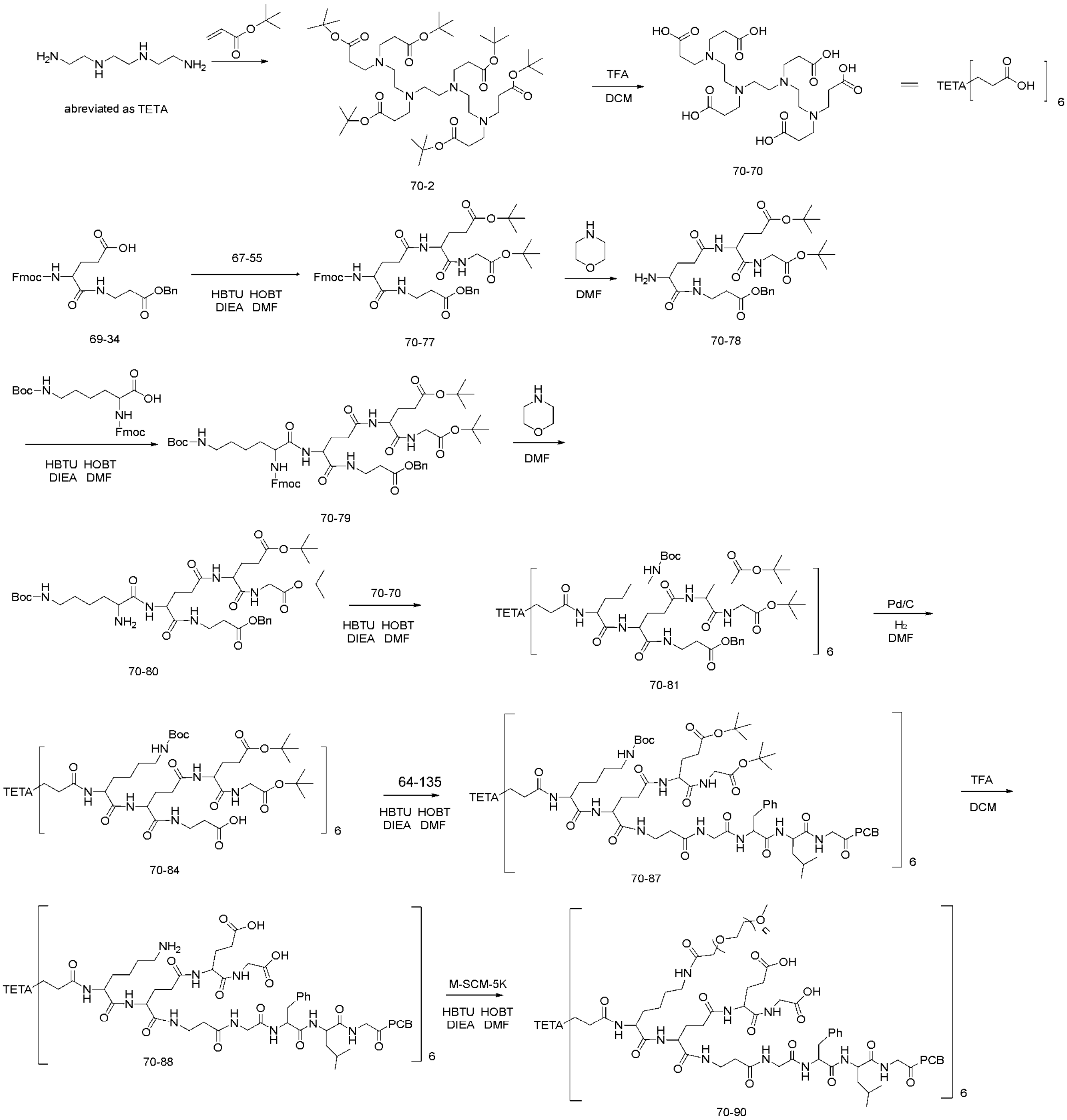


70-2

70-70

69-34

70-77

70-78

70-79

70-80

70-81

70-84

70-87

70-88

70-90

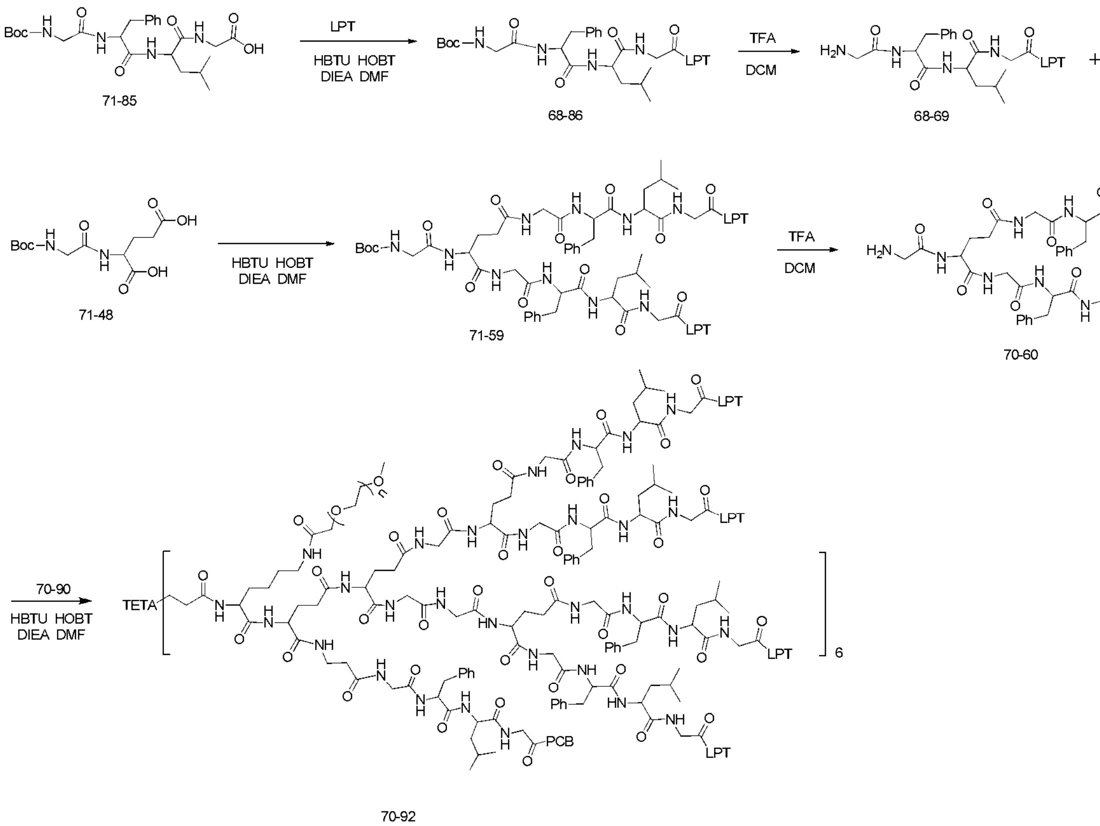


8.1 Preparation of **70-2**

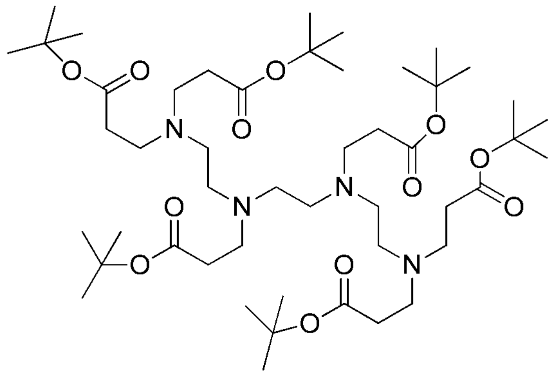

Triethylenetetramine (1 mL, 7 mmol) and tert-butyl acrylate (12.2 mL, 84 mmol) were added to a 250 mL flask, and filled with nitrogen for protection. Methanol (10 mL) was added for dissolution, and the reaction was carried out with stirring at room temperature. The reaction was monitored by TLC. After the reaction was completed, the reaction mixture was concentrated under reduced pressure, and ethyl acetate (100 mL) and saturated sodium bicarbonate solution (100 mL) were added for extraction. The organic phase was collected, and the aqueous phase was further extracted with saturated sodium chloride solution (100 mL $\times$ 2). The organic phases were combined, concentrated, and dried in a vacuum oven to provide the product. $^1$H-NMR (600 MHz, DMSO-$d_6$) $\delta$ 2.744-2.569 (m, 12H), 2.464-2.368 (m, 12H), 2.340-2.174 (m, 12H), 1.478-1.308 (m, 54H); ESI [M+H$^+$] 916.004

8.2 Preparation of **70-70**

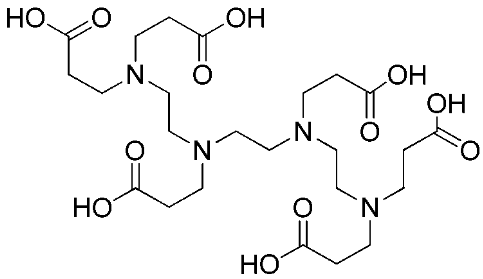

To a flask containing **70-2** (1 g, 1.1 mmol), dichloromethane (30 mL) was added for dissolution with the assistance of ultrasonication, then TFA (29 mL, 120 mmol) was added, and the mixture was reacted with stirring at room temperature overnight. After the reaction was completed, the reaction mixture was evaporated by using a rotary evaporator to provide an oil. Methyl tert-butyl ether (60 mL) was then added for precipitation. A solid precipitated from the reaction mixture and was filtered. The filter cake was washed with methyl tert-butyl ether (40 mL × 3). The filter cake was collected and dried in a vacuum oven to provide the product.

8.3 Preparation of **70-77**

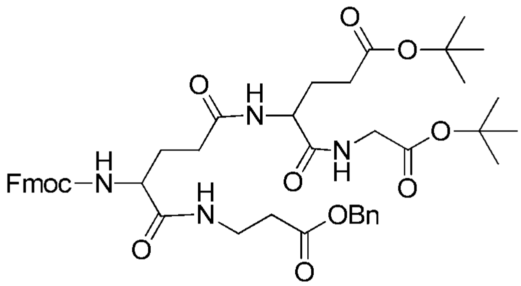

Reactants **69-34** (13.2 g, 23.6 mmol), **67-55** (7.5 g, 23.6 mmol), HOBT (3.8 g, 28.3 mmol), and HBTU (10.7 g, 28.3 mmol) were placed in a reaction flask, then DMF (200 mL) was added for dissolution with the assistance of ultrasonication, and the mixture was stirred at 0°C. DIEA (8.6 mmol, 51.9 mmol) was added dropwise. After the dropwise addition was completed, the mixture was brought to room temperature, and the reaction was carried out with stirring for 1 hour. The reaction progress was monitored by TLC. After the reaction was completed, the reaction mixture was poured into a separatory funnel containing saturated brine (300 mL) and ethyl acetate (300 mL) for extraction. The organic phase was collected, and the aqueous phase was further extracted with ethyl acetate (200 mL × 2). The organic phases were combined, evaporated to dryness, dissolved in a methanol/dichloromethane (1/4) mixture, then silica gel powder was added, and the mixture was evaporated to dryness and subjected to column chromatography using 1-2.5% methanol/dichloromethane as the eluent. The desired product was collected, concentrated, and evaporated to dryness to provide 17.8 g of the product with a yield of 90.8%.

8.4 Preparation of **70-78**

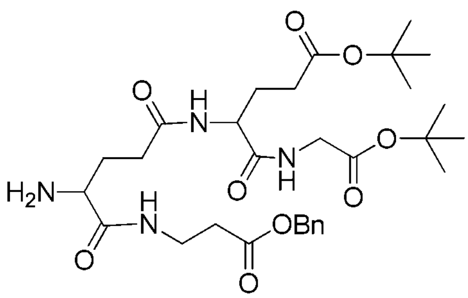

Reactant **70-77** (6.1 g, 7.5 mmol) was placed in a reaction flask. DMF (50 mL) was added for dissolution, then morpholine (13 mL, 150 mmol) was added, and the mixture was reacted with stirring at room temperature. The reaction progress was monitored by TLC. After the reaction was completed, ethyl acetate (150 mL) and saturated brine (100 mL) were added for extraction. The organic phase was collected, and the aqueous phase was further extracted twice with ethyl acetate (100 mL). The organic phases were combined, washed with deionized water, and the separated aqueous phase was further extracted twice with ethyl acetate. The organic phases were combined, concentrated and evaporated to dryness to provide 5.9 g of the product.

8.5 Preparation of **70-79**

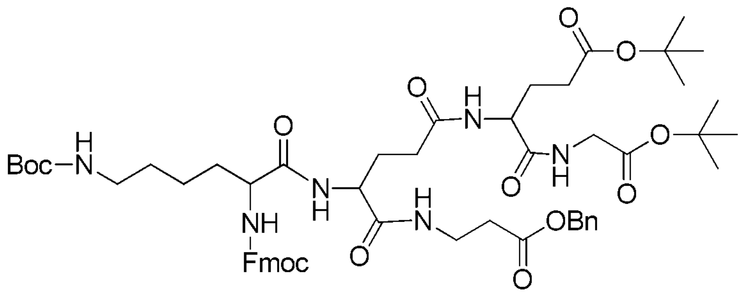

**70-78** (5.9 g, 7.5 mmol), Fmoc-Lys (Boc)-OH (3.5 g, 7.5 mmol), HOBT (1.2 g, 9.0 mmol), and HBTU (3.4 g, 9.0 mmol) were placed in a reaction flask, then DMF (200 mL) was added to dissolve the mixture with the assistance of ultrasonication, and the mixture was stirred at 0°C for 10 minutes. DIEA (3 mmol, 16.5 mmol) was then added dropwise. After the dropwise addition was completed, the mixture was brought to room temperature and the reaction was carried out with stirring overnight. The reaction progress was monitored by TLC. After the reaction was completed, n-hexane (50 mL) and methyl tert-butyl ether (200 mL) were added for precipitation, resulting in an oily solid. The supernatant was discarded, and methyl tert-butyl ether (200 mL) was added for precipitation. This process was repeated three times. A powdery solid precipitated and was filtered. The filter cake was collected and dried in a vacuum oven to provide 7.5 g of the product with a yield of 94.6%. $^1$H-NMR (600 MHz, DMSO-$d_6$) δ 8.00-7.95 (m, 3H), 7.92-7.87 (m, 2H), 7.74-7.71 (m, 3H), 7.59-7.53 (m, 2H), 7.50-7.45 (m, 1H), 7.44-7.39 (m, 4H), 7.37-7.35 (m, 2H), 7.33-7.31 (m, 2H), 5.08 (s, 2H), 4.32-4.21 (m, 3H), 4.19-4.13 (m, 2H), 4.03-3.91 (m, 3H), 3.80-3.57 (m, 4H), 3.31-3.25 (m, 2H), 3.19-3.11 (m, 2H), 2.95-2.83 (m, 2H), 2.53 (s, 1H), 2.29-2.18 (m, 2H), 2.16-2.12 (m, 1H), 1.92-1.66 (m, 4H), 1.66-1.45 (m, 2H), 1.39-1.33 (m, 27H)

8.6 Preparation of **70-80**

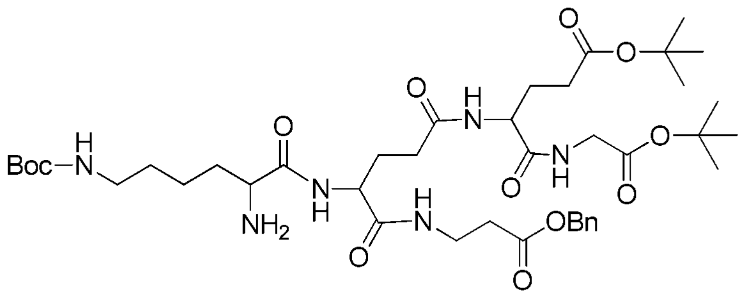

**70-79** (2 g, 1.9 mmol) was weighed and placed in a reaction flask. DMF (20 mL) was added for dissolution, then morpholine (3.3 mL, 38 mmol) was added, and the mixture was reacted with stirring at room temperature. The reaction progress was monitored by TLC. After the reaction was completed, n-hexane (50 mL) and methyl tert-butyl ether (200 mL) were added for precipitation, resulting in an oily solid. The supernatant was discarded, and methyl tert-butyl ether (200 mL) was added for precipitation. This process was repeated three times. A solid precipitated and was filtered. The filter cake was collected and dried in a vacuum oven to provide 0.88 g of the product with a yield of 55.5%.

8.7 Preparation of **70-81**

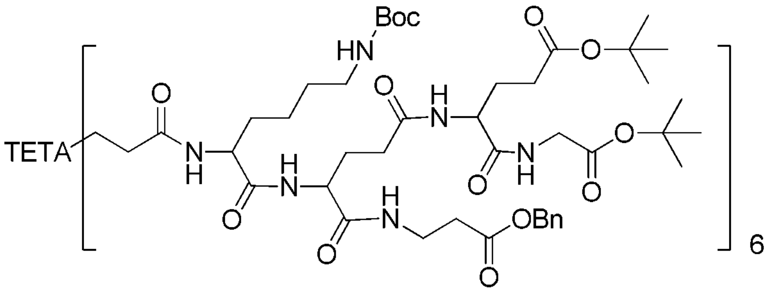

Reactants **70-80** (0.79 g, 0.94 mmol), **70-70** (76 mg, 0.13 mmol), HOBT (0.2 g, 1.4 mmol), and HBTU (0.5 g, 1.4 mmol) were placed in a reaction flask, then DMF () was added for dissolution with the assistance of ultrasonication, and the mixture was stirred at 0°C. DIEA (0.3 mmol, 1.5 mmol) was added dropwise. After the dropwise addition was completed, the mixture was brought to room temperature and reacted with stirring for 1 hour. The reaction progress was monitored by TLC. After the reaction was completed, ethyl acetate () and deionized water () were added for extraction. The organic phase was washed twice with pure water and evaporated to dryness to provide a solid crude product, which was dissolved in a methanol/dichloromethane (1/4) mixture. Silica gel powder was added, and the mixture was evaporated to dryness. The resulting product was dry-loaded onto the column and subjected to column chromatography using 0-5% methanol/dichloromethane as the eluent. The desired product was collected, concentrated, and dried to provide 0.36 g of the product with a yield of 56.8%. $^1$H-NMR (600 MHz, DMSO-$d_6$) δ 8.47-8.37 (m, 6H), 8.31-8.25 (m, 6H), 8.19-8.11 (m, 6H), 8.04-7.97 (m, 6H), 7.53-7.52 (m, 6H), 7.40-7.27 (m, 32H), 6.87-6.70 (m, 6H), 5.11-5.02 (m, 12H), 4.31-4.25 (m, 6H), 4.22-4.16 (m, 6H), 4.05-4.00 (m, 6H), 3.77-3.75 (m, 2H), 3.75-3.72 (m, 4H), 3.67-3.65(m, 4H), 3.65-3.63 (m, 2H), 3.40-3.36 (m, 10H), 3.32-3.26 (m, 12H), 2.98 (s, 32H), 2.93-2.88 (m, 18H), 2.87-2.75(m, 36H), 2.60-2.52 (m, 18H), 2.42-2.36 (m, 2H), 2.26-2.22 (m, 12H), 2.14-2.07 (m, 12H), 1.91-1.86 (m, 6H), 1.82-1.78 (m, 6H), 1.74-1.72 (m, 6H), 1.72-1.67 (m, 12H), 1.51-1.48 (m, 2H), 1.40-1.39 (m, 1H), 1.39-1.38 (m, 54H), 1.37-1.36 (s, 54H), 1.35-1.34 (m, 6H), 1.33-1.30 (m, 6H), 1.30-1.28 (m, 2H), 1.28-1.26 (m, 3H), 1.25-1.23 (m, 4H), 1.23 (s, 2H)

8.8 Preparation of **70-84**

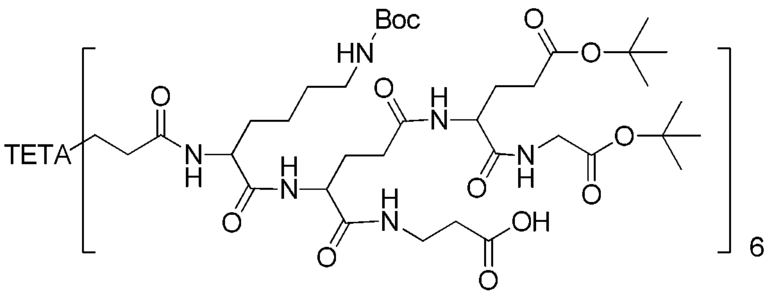

**70-81** (0.36 g, 0.067 mmol) was weighed and added to a micro hydrogenation reactor, then DMF (30 mL) was added for dissolution, and then 10% Pd/C catalyst (30 mg) was added. The reactor was sealed, evacuated with a water pump, and then filled with hydrogen gas. This process was repeated three times. The pressure reading of the hydrogenation reactor was adjusted to 2 MPa. The mixture was stirred overnight, and the reaction progress was monitored by TLC. After the reaction was completed, the mixture was filtered through Celite, and the filtrate was taken for later use.

8.9 Preparation of **70-87**

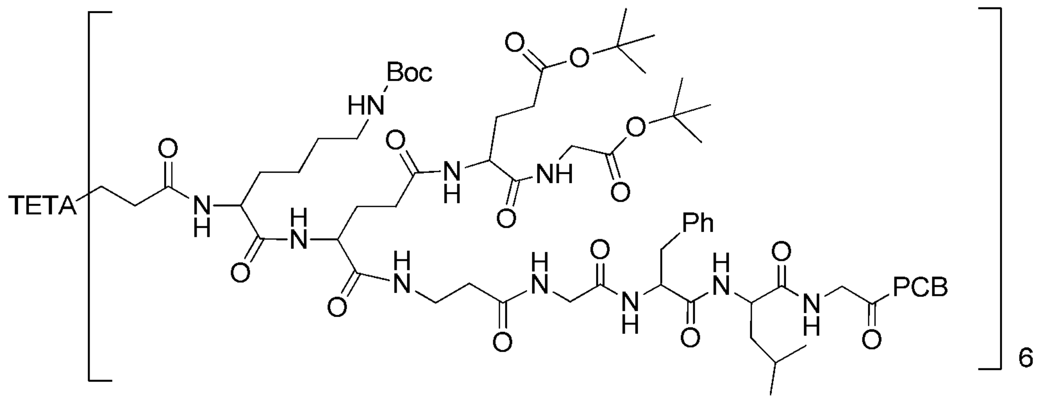

**70-84** (0.067 mmol), **64-135** (0.5 g, 0.6 mmol), HOBT (0.081 g, 0.6 mmol), and HBTU (0.23 g, 0.6 mmol) were placed in a reaction flask, then DMF (20 mL) was added for dissolution with the assistance of ultrasonication, and the mixture was stirred at 0°C. DIEA (0.2 mmol, 1.0 mmol) was added dropwise. After the dropwise addition was completed, the mixture was brought to room temperature and the reaction was carried out with stirring. The reaction progress was monitored by TLC. After the reaction was completed, methyl tert-butyl ether (100 mL) and n-hexane (20 mL) were used for precipitation, and the mixture was filtered using a sand-core funnel. The filter cake was collected, washed with methyl tert-butyl ether (100 mL × 2). The filter cake was transferred to a flask and dried to provide 0.54 g of the product with a yield of 83.07%. $^1$H-NMR (600 MHz, DMSO-$d_6$) δ 8.45-8.40 (m, 6H), 8.31-8.25 (m, 6H), 8.21-8.15 (m, 6H), 8.13-8.09 (m, 6H), 8.09-8.08 (m, 6H), 8.06 (s, 6H), 8.03-8.00 (m, 6H), 8.00-8.98 (m, 6H), 7.94-7.91 (m, 6H), 7.90-7.88 (m 6H), 7.75-7.70 (m, 6H), 7.59-7.53 (m, 12H), 7.44-7.40 (m, 6H), 7.27-7.22 (m, 24H), 7.19-7.16 (m, 6H), 4.66-4.60 (m, 2H), 4.59-4.53 (m, 6H), 4.40-4.34 (m, 6H), 4.30-4.24 (m, 6H), 4.24-4.17 (m, 6H), 4.08-3.96 (m, 20H), 3.74-3.72 (m, 6H), 3.71-3.69 (m, 6H), 3.69-3.65 (m, 6H), 3.65-3.54 (m, 38H), 3.24-3.17 (m, 20H), 3.16-3.10 (m, 18H), 3.09-3.02 (m, 12H), 3.01-2.95 (m, 22H), 2.93-2.87 (m, 26H), 2.78-2.76 (m, 4H), 2.67-2.61 (m, 12H), 2.43 (s, 20H), 2.31 (s, 22H), 2.27-2.21 (m, 26H), 2.13-2.07 (m, 10H), 1.91-1.86 (m, 18H), 1.81-1.75 (m, 24H), 1.73-1.66 (m, 18H), 1.61-1.56 (m, 14H), 1.54-1.48 (m, δ (d, 16H), 1.40 (s, 54H), 1.38 (s, 54H), 1.36 (s, 54H), 1.34 (s, 12H), 0.913-0.890 (m, 18H), 0.860-0.843 (m, 18H)

8.10 Preparation of **70-88**

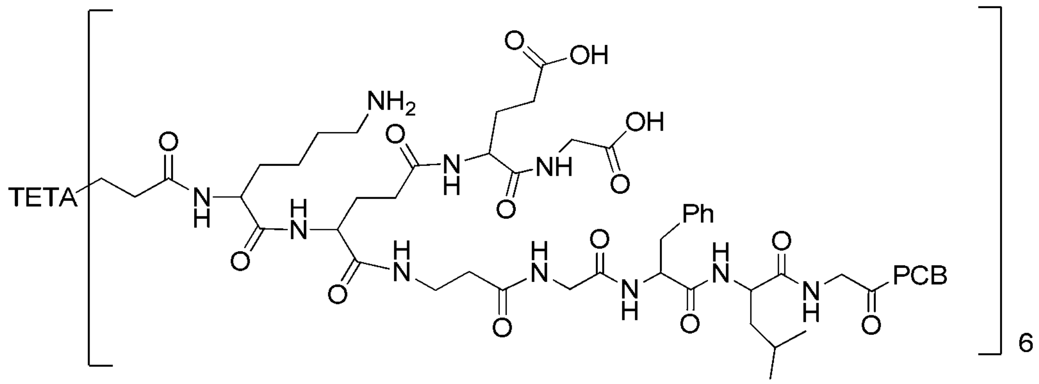

**70-87** (0.54 g, 0.056 mmol) was added to a reaction flask containing dichloromethane (13 mL) and dissolved, then TFA (1.5 mL, 20 mmol) was added, and the mixture was reacted with stirring at room temperature. The reaction progress was monitored by TLC. After the reaction was completed, the reaction mixture was concentrated under reduced pressure to remove dichloromethane and most of the TFA. Methyl tert-butyl ether (100 mL) and n-hexane (10 mL) were then added for precipitation. The mixture was then filtered using a sand-core funnel. The filter cake was collected and rinsed twice with methyl tert-butyl ether (50 mL). The filter cake was transferred to a flask and dried to provide 0.5 g of the product.

8.11 Preparation of **70-90**

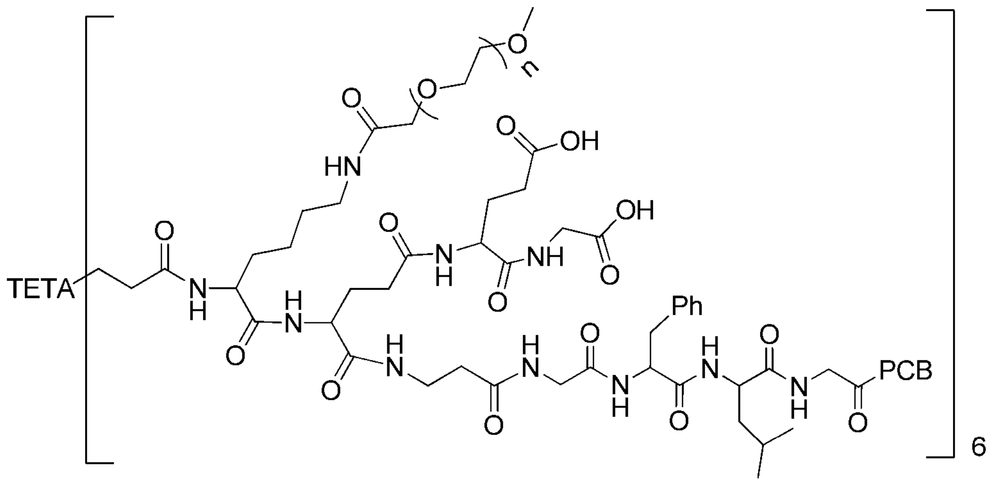

Reactant **70-88** (0.5 g, 0.056 mmol) was placed in a reaction flask, then extra-dry DMF (20 mL) was added for dissolution with the assistance of ultrasonication, and the mixture was stirred at 0°C for 10 minutes. DIEA (0.2 mmol, 1 mmol) was then added dropwise. After the dropwise addition was completed, the mixture was brought to room temperature and the reaction was carried out with stirring for 0.5 hours. M-SCM-5K (1.8 g, 0.36 mmol) was added, and the reaction was carried out with stirring in the dark at low speed. The reaction progress was monitored by TLC. After the reaction was completed, n-hexane (50 mL) and methyl tert-butyl ether (200 mL) were added for precipitation, resulting in an oily solid. The supernatant was discarded, and methyl tert-butyl ether (200 mL) was added for precipitation. This process was repeated three times. A solid precipitated and was filtered. The filter cake was collected and dried in a vacuum oven to provide 2.3 g of the product.

8.12 Preparation of **68-86**

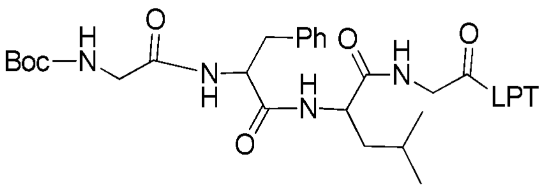

**71-85** (51.0 mmol), lapatinib (24.7 g, 42.8 mmol, purchased from Shanghai Hengxin, abbreviated as LPT), HOBT (6.3 g, 46.7 mmol), and HBTU (17.7 g, 46.7 mmol) were placed in a reaction flask, then DMF (500 mL) was added for dissolution with the assistance of ultrasonication, and the mixture was stirred at 0°C. DIEA (15.4 mmol, 93.4 mmol) was added dropwise. After the dropwise addition was completed, the mixture was brought to room temperature and the reaction was carried out with stirring for 2 hours. The reaction progress was monitored by TLC. After the reaction was completed, ethyl acetate (400 mL) and deionized water (300 mL) were added for extraction. The organic phase was collected, and the aqueous phase was further extracted twice with ethyl acetate (200 mL). The organic phases were combined, appropriately concentrated, and washed with saturated brine. The organic phase was collected, and the aqueous phase was further extracted twice with ethyl acetate (200 mL). The organic phases were combined, collected, concentrated, and dried in a vacuum oven to provide 45.0 g of the product.

8.13 Preparation of **68-69**

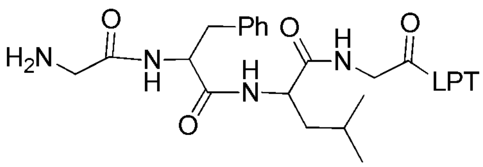

Reactant **68-86** (45.0 g, 42.8 mmol) was added to a reaction flask containing dichloromethane (100 mL), then TFA (31.5 mL, 424.8 mmol) was added, and the mixture was reacted with stirring at room temperature. The reaction progress was monitored by TLC. After the reaction was completed, the reaction mixture was concentrated under reduced pressure to remove dichloromethane and most of the TFA. Methyl tert-butyl ether (500 mL) and n-hexane (100 mL) were then added for precipitation. The mixture was filtered using a sand-core funnel. The filter cake was collected and rinsed with methyl tert-butyl ether (300 mL × 3). The filter cake was transferred to a flask and dried to provide 41 g of the product.

8.14 Preparation of **71-59**

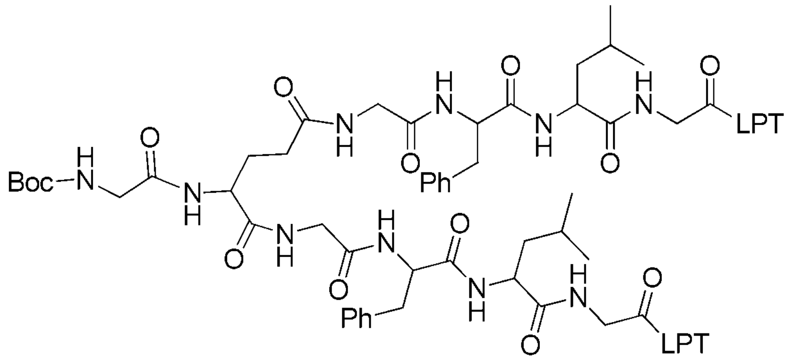

Reactants **71-48** (0.45 g, 1.48 mmol), **68-69** (3.26 mmol), HOBT (1.7 g, 4.44 mmol), and HBTU (0.6 g, 4.44 mmol) were placed in a reaction flask, then DMF (20 mL) was added for dissolution with the assistance of ultrasonication, and the mixture was stirred at 0°C. DIEA (2.2 mmL, 13.32 mmol) was added dropwise. After the dropwise addition was completed, the mixture was brought to room temperature and the reaction was continued with stirring for 1 hour. The reaction progress was monitored by TLC. After the reaction was completed, methyl tert-butyl ether (100 mL) and n-hexane (20 mL) were added for precipitation. The mixture was filtered using a sand-core funnel, and the filter cake was collected. The filter cake was rinsed twice with methyl tert-butyl ether (100 mL). The filter cake was transferred to a flask and dried to provide 2.81 g of the product with a yield of 87.8%.

8.15 Preparation of **70-60**

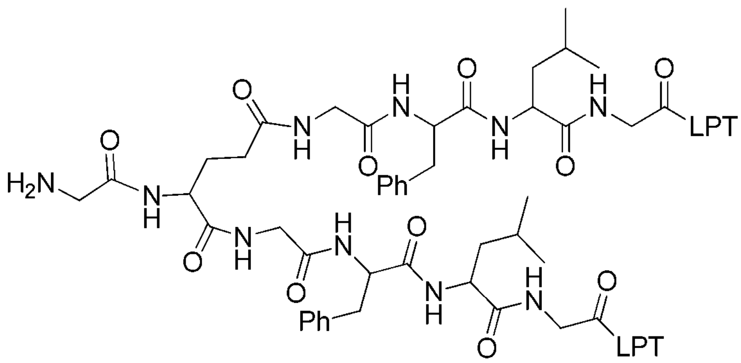

**71-59** (2.81 g, 1.29 mmol) was added to a reaction flask containing dichloromethane (20 mL) for dissolution, then TFA (1.9 mL, 25.8 mmol) was added, and the mixture was reacted with stirring at room temperature. The reaction progress was monitored by TLC. After the reaction was completed, methyl tert-butyl ether (100 mL) and n-hexane (20 mL) were added for precipitation, and the mixture was filtered using a sand-core funnel. The filter cake was collected and washed twice with methyl tert-butyl ether (80 mL). The filter cake was transferred to a flask and dried, then a methanol/dichloromethane (1/4) mixture was added for dissolution. Silica gel powder was added, and the mixture was evaporated to dryness. The resulting product was dry-loaded onto the column and subjected to column chromatography using 1% ammonia water/8% methanol/dichloromethane as the eluent. The desired product was collected, concentrated, and dried to provide 1.7 g of the product with a yield of 63.4%.

8.16 Preparation of **70-92**

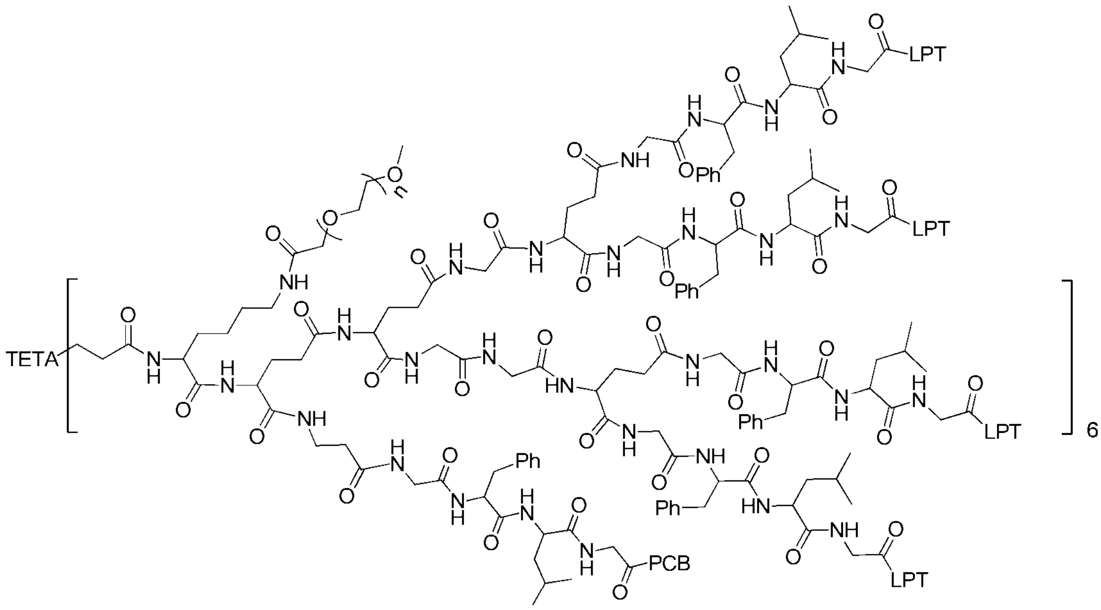

**70-60** (1.54 g, 1.8 mmol), **70-90** (2.3 g, 0.056 mmol), HOBT (0.14 g, 1.1 mmol), and HBTU (0.38 g, 1.1 mmol) were placed in a reaction flask, then DMF (30 mL) was added for dissolution with the assistance of ultrasonication, and the mixture was stirred at 0°C. DIEA (0.6 mml, 56 mmol) was added dropwise. After the dropwise addition, the mixture was brought to room temperature and the reaction was carried out with stirring for 2 hours. The reaction progress was monitored by TLC. After the reaction was completed, methyl tert-butyl ether (100 mL) and n-hexane (20 mL) were added for precipitation, and the mixture was filtered with a sand-core funnel. The filter cake was collected and washed with methyl tert-butyl ether (100 mL × 2). The filter cake was transferred to a flask, then a methanol/dichloromethane (1/4) mixture was added for dissolution. Silica gel powder was added, and the mixture was evaporated to dryness. The resulting product was dry-loaded onto the column and subjected to column chromatography using 1% ammonia water/8% methanol/dichloromethane as the eluent. The desired product was collected, concentrated, and dried to provide 1.6 g of the product with a yield of 55.17%. $^1$H-NMR (600 MHz, DMSO-$d_6$) δ 8.75-8.72 (m, 12H), 8.56-8.54 (m, 12H), 8.24-8.14 (m, 60H), 8.09-8.04 (m, 42H), 8.03-8.00 (m, 30H), 7.82-7.77 (m, 16H), 7.76-7.69 (m, 22H), 7.51-7.44 (m, 22H), 7.39-7.17 (m, 108H), 7.14-7.09 (m, 28H), 7.06-7.03 (m, 6H), 6.705-6.662 (m, 10H), 6.555-6.525 (m, 4H), 5.268-5.252 (m, 24H), 4.79-4.67 (m, 28H), 4.60-4.53 (m, 20H), 4.40-4.33 (m, 20H), 4.295-4.170 (m, 40H), 4.13-3.94 (m, 24H), 3.880-3.846 (m, 18H), 3.807-3.740 (m, 42H), 3.627-3.602 (m, 74H), 3.520-3.497 (m, 2789H), 3.17-3.19 (m, 58H), 3.08-3.05 (m, 30H), 2.98-2.93 (m, 36H), 2.83-2.79 (m, 30H), 2.637-2.600 (m, 18H), 2.403-2.381 (m, 10H), 2.32-2.27 (m, 32H), 2.14-2.08 (m, 30H), 1.79-1.70 (m, 40H), 1.65-1.56 (m, 48H), 1.528-1.460 (m, 44H), 1.307-1.290 (m, 12H), 0.895-0.830 (m, 108H)

Example 9: Synthesis of Compound **77-231**

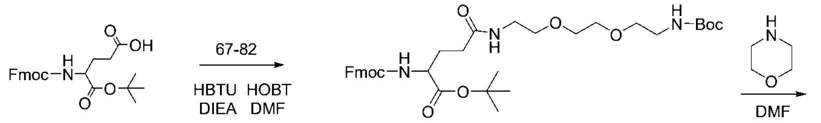


68-138

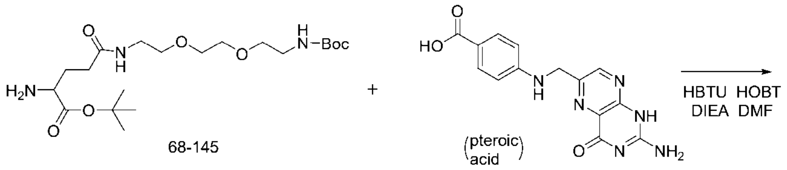


68-145

(pteroic acid)

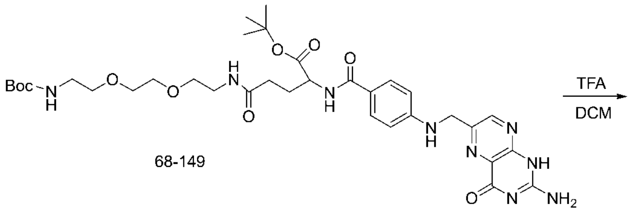


68-149

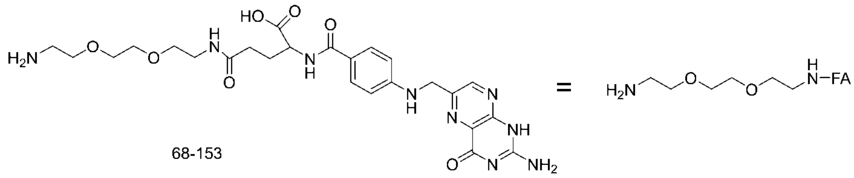

68-153

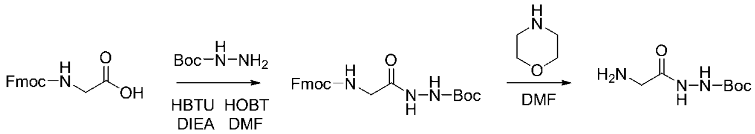


77-12

77-11

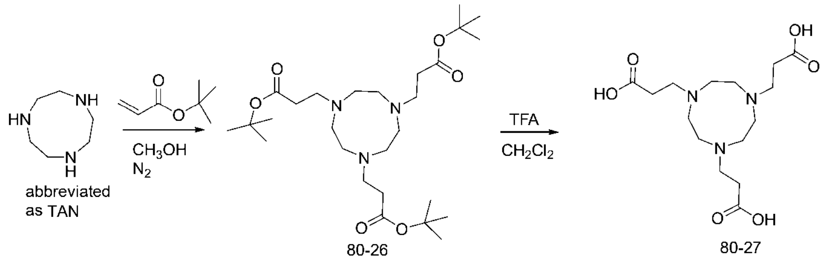


80-26

80-27

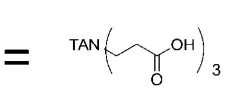

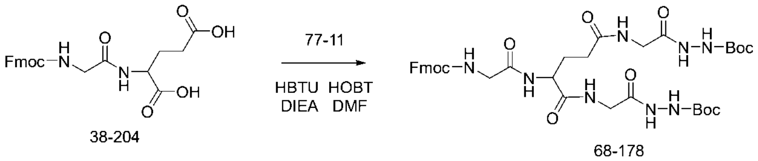


38-204

68-178

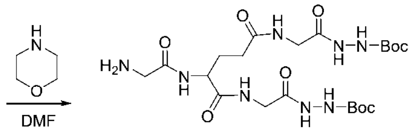


68-179

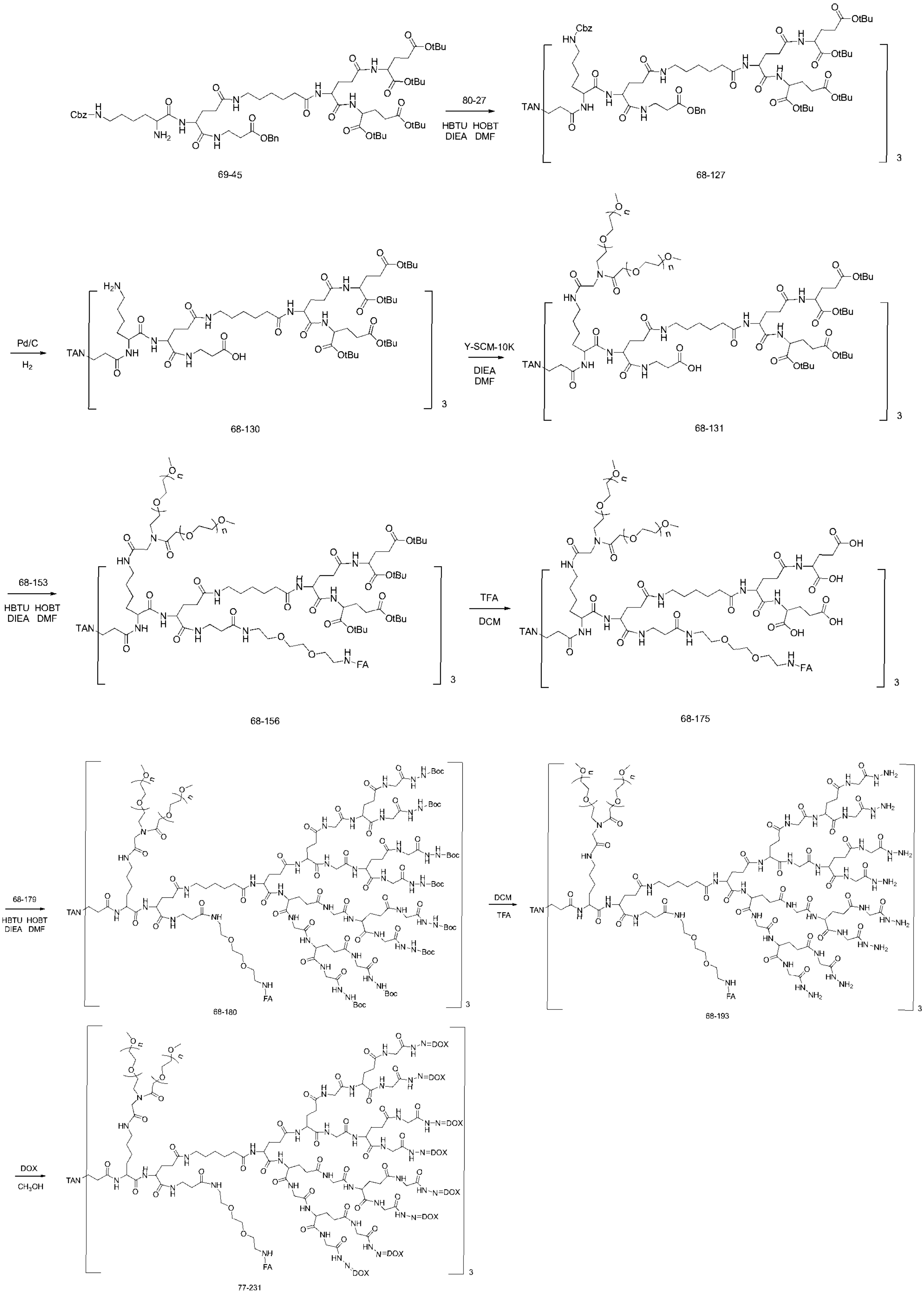


69-45

68-127

68-130

68-131

68-156

68-175

68-180

68-193

77-231

9.1 Preparation of **68-138**

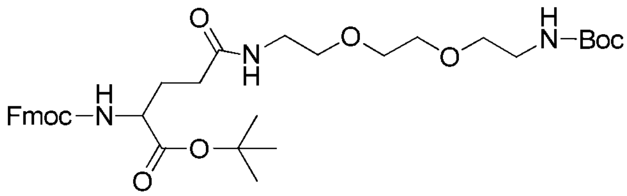

Fmoc-L-glutamic acid-1-tert-butyl ester (3.34 g, 7.8528 mmol, purchased from Innochem), HBTU (3.28 g, 8.6380 mmol), and HOBT (1.17 g, 8.6380 mmol) were weighed and added to a flask containing **67-82** (1.95 g, 7.8528 mmol), then DMF (40 mL) was added to dissolve the mixture. The mixture was allowed to stand at -5°C, and DIEA (2.86 mL, 17.2760 mmol) was slowly added dropwise. After the dropwise addition, the reaction was carried out for half an hour, the flask was then taken out, and the reaction was carried out with stirring at room temperature overnight. After the reaction was completed, saturated brine (200 mL) and ethyl acetate (200 mL) were added for extraction. After standing for phase separation, the organic phase was collected, and the aqueous phase was further extracted with ethyl acetate (200 mL × 3). The organic phases were combined, concentrated, evaporated to dryness, and dried in a vacuum oven to provide the product.

9.2 Preparation of **68-145**

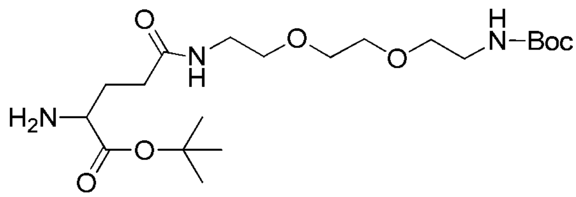

To a flask containing **68-138** (7.8528 mmol), DMF (50 mL) was added for dissolution with the assistance of ultrasonication, then morpholine (6.84 mL, 78.528 mmol) was added, and the mixture was reacted with stirring at room temperature for 2 hours. After the reaction was completed, saturated brine (200 mL) and ethyl acetate (200 mL) were added for extraction. After standing for phase separation, the organic phase was collected, and the aqueous phase was further extracted with ethyl acetate (200 mL × 3). The organic phases were combined, concentrated, evaporated to dryness, and dried in a vacuum oven to provide the product.

9.3 Preparation of **68-149**

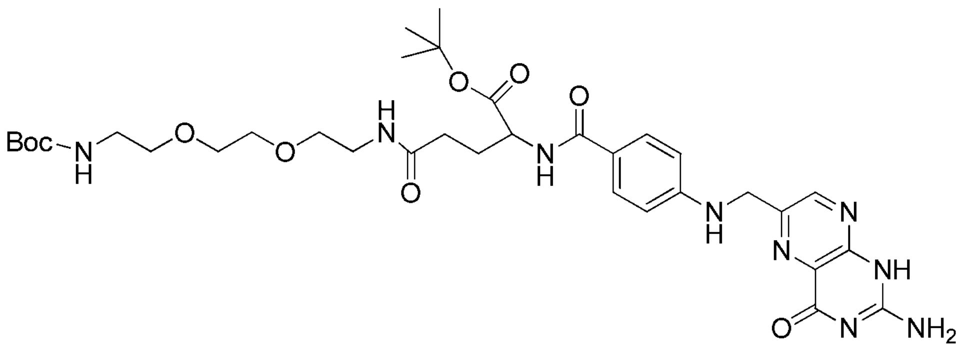

Pteroic acid (0.98 g, 3.1382 mmol, purchased from Innochem), HBTU (2.98 g, 4.7073 mmol), and HOBT (1.06 g, 4.7073 mmol) were weighed and added to a flask containing **68-145** (4.7073 mmol), then DMF (50 mL) was added to

dissolve the mixture, and the mixture was stirred at -5°C for 5 minutes. DIEA (1.55 mL, 9.4147 mmol) was slowly added dropwise. After the dropwise addition was completed, the reaction was carried out for half an hour, the flask was then taken out, and the reaction was carried out with stirring at room temperature overnight. After the reaction was completed, saturated brine (200 mL) and ethyl acetate (200 mL) were added for extraction. After standing for phase separation, the organic phase was collected, and the aqueous phase was further extracted with ethyl acetate (200 mL × 3). The organic phases were combined, concentrated, evaporated to dryness, and dried in a vacuum oven to provide 0.75 g of the product with a yield of 33%.

9.4 Preparation of **68-153**

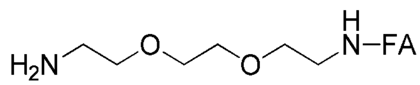

To a flask containing **68-149** (0.75 g, 1.0305 mmol), dichloromethane (10 mL) was added for dissolution with the assistance of ultrasonication, then TFA (0.77 mL, 10.305 mmol) was added, and the mixture was reacted with stirring at room temperature overnight. After the reaction was completed, n-hexane (100 mL) and methyl tert-butyl ether (600 mL) were added for precipitation. The supernatant was discarded, and n-hexane and methyl tert-butyl ether were added again for precipitation. This process was repeated three times. The mixture was filtered to provide a solid product, which was dried to provide 0.55 g of the product with a yield of 93.22%.

9.5 Preparation of **77-12**

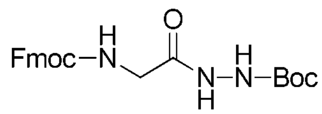

Fmoc-glycine (5 g, 16.817 mmol, purchased from Innochem), HBTU (10.5 g, 27.6635 mmol), and HOBT (3.74 g, 27.6635 mmol) were weighed and added to a flask containing tert-butyloxyformylhydrazide (2.89 g, 21.8621 mmol, purchased from Innochem), then DMF (300 mL) was added to dissolve the mixture, and the mixture was stirred at -5°C for several minutes. DIEA (9.15 mL, 55.3269 mmol) was slowly added dropwise. After the dropwise addition was completed, the reaction was carried out for half an hour, the flask was then taken out, and the reaction was carried out with stirring at room temperature overnight. After the reaction was completed, saturated brine (200 mL) and ethyl acetate (200 mL) were added for extraction. After standing for phase separation, the organic phase was collected, and the aqueous phase was further extracted with ethyl acetate (200 mL × 3). The organic phases were combined, concentrated, evaporated to dryness, and dried in a vacuum oven to provide the product.

9.6 Preparation of **77-11**

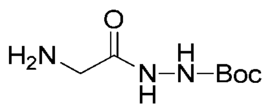

To a flask containing **77-12** (7.17 g, 16.817 mmol), acetonitrile (50 mL) was added for dissolution with the assistance of ultrasonication, then diethylamine (25.98 mL, 252.255 mmol) was added, and the mixture was reacted with stirring at room temperature for 2 hours. After the reaction was completed, saturated brine (200 mL) and ethyl acetate (200 mL) were added for extraction. After standing for phase separation, the organic phase was collected, and the aqueous phase was further extracted with ethyl acetate (200 mL × 3). The organic phases were combined, evaporated to dryness and dried in a vacuum oven to provide 2.6 g of the product with a yield of 81.7%.

9.7 Preparation of **80-26**

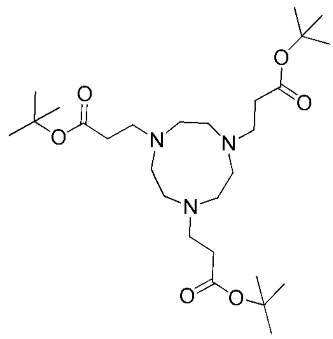

1,4,7-Triazacyclononane (0.5 g, 3.8699 mmol) was weighed and placed in a 500 mL flask. Methanol (100 mL) was added for dissolution, then tert-butyl acrylate (2.25 mL, 15.4799 mmol) was added under nitrogen protection, and the mixture was reacted with stirring at room temperature overnight. After the reaction was completed, saturated brine (200 mL) and ethyl acetate (200 mL) were added for extraction. After standing for phase separation, the organic phase was collected, and the aqueous phase was further extracted with ethyl acetate (200 mL × 3). The organic phases were combined, evaporated to dryness, then a methanol/dichloromethane (1/4) mixture was added for dissolution. Silica gel powder was added, and the mixture was evaporated to dryness to provide a powdery solid. The powdery solid was dry-loaded onto the column and subjected to column chromatography using a 2-4% methanol/dichloromethane mixture as the eluent. The desired product was collected, concentrated, and dried in a vacuum oven to provide 1.35 g of the product with a yield of 68.18%. ESI [$M+Na^+$] 514.705

9.8 Preparation of **80-27**

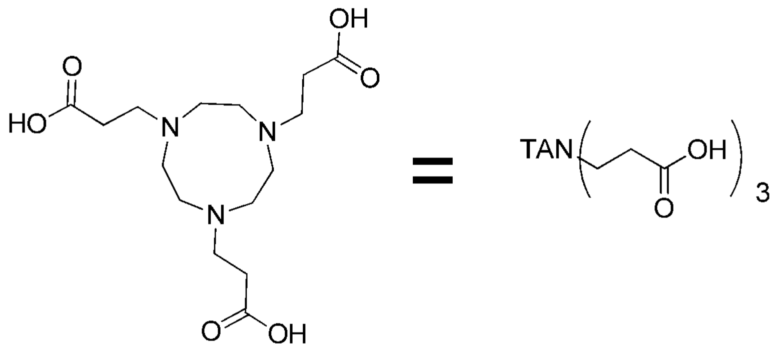

To a flask containing **80-26** (1.35 g, 2.5 mmol), dichloromethane (10 mL) was added for dissolution with the assistance of ultrasonication, then TFA (0.2 mL) was added, and the mixture was reacted with stirring at room temperature overnight. After the reaction was completed, the reaction mixture was evaporated to an oily state using a rotary evaporator. Methyl tert-butyl ether (60 mL) was then added for precipitation. A solid precipitated from the reaction mixture and was filtered. The filter cake was washed with methyl tert-butyl ether (40 mL × 3). The filter cake was collected and dried in a vacuum oven to provide the product. $^1$H-NMR (600 MHz, DMSO-$d_6$) δ 3.03-3.00 (m, 6H), 2.87-2.78 (m, 12H), 2.56-2.50 (m, 6H)

9.9 Preparation of **68-178**

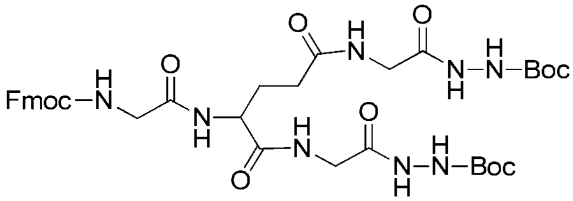

**77-11** (1.33 g, 3.6054 mmol), HBTU (3.0 g, 7.9319 mmol), and HOBT (1.07 g, 7.9319 mmol) were weighed and added to a flask containing **38-204** (1.5 g, 7.9319 mmol), then DMF (100 mL) was added to dissolve the mixture, and the flask was allowed to stand at -5°C. DIEA (11.12 mL, 67.2700 mmol) was slowly added dropwise. After the dropwise addition was completed, the reaction was carried out for half an hour, the flask was then taken out, and the reaction was carried out with stirring at room temperature overnight. After the reaction was completed, n-hexane (25 mL) and methyl tert-butyl ether (200 mL) were added for precipitation. The supernatant was discarded, and n-hexane and methyl tert-butyl ether were added again for precipitation. This process was repeated three times. The mixture was filtered to provide a solid product, which was dissolved in dichloromethane and methanol, then silica gel powder was added, and the mixture was evaporated to dryness. The resulting product was dry-loaded onto the column and subjected to column chromatography using 5-12% methanol/dichloromethane as the eluent. The desired product was collected, concentrated, and dried to provide the product.

9.10 Preparation of **68-179**

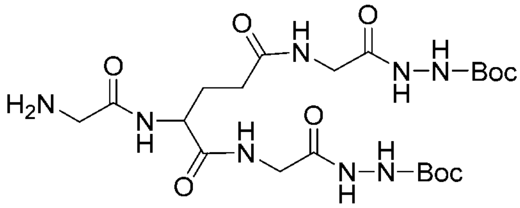

To a flask containing **68-178** (3.8309 mmol), DMF (20 mL) was added for dissolution with the assistance of ultrasonication, then morpholine (3.3 mL, 38.309 mmol) was added, and the mixture was reacted with stirring at room temperature for 2 hours. After the reaction was completed, n-hexane (25 mL) and methyl tert-butyl ether (200 mL) were added for precipitation. The supernatant was discarded, and n-hexane and methyl tert-butyl ether were added again for precipitation. This process was repeated three times. The mixture was filtered to provide a solid product, which was dried in a vacuum oven to provide 1.45 g of the product with a yield of 69.38%.

9.11 Preparation of **68-127**

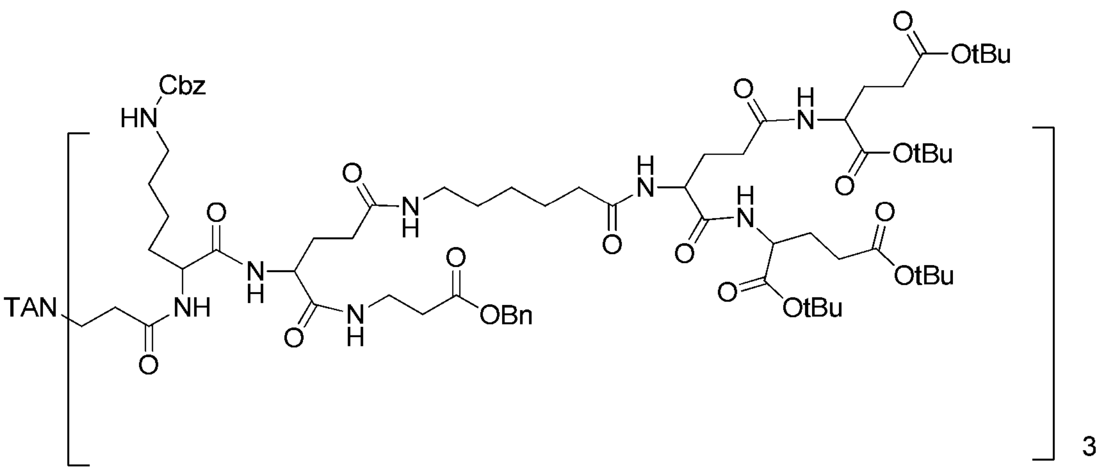

**69-45** (1.33 g, 0.9883 mmol), HBTU (0.38 g, 0.9883 mmol), and HOBT (0.14 g, 0.9883 mmol) were weighed and added to a flask containing **80-27** (0.1 g, 0.2895 mmol), then DMF (20 mL) was added to dissolve the mixture, and the flask was allowed to stand at -5°C. DIEA (0.33 mL, 1.9766 mmol) was slowly added dropwise. After the dropwise addition was completed, the reaction was carried out for half an hour, the flask was then taken out, and the reaction was carried out with stirring at room temperature overnight. After the reaction was completed, n-hexane (25 mL) and methyl tert-butyl ether (200 mL) were added for precipitation. The supernatant was discarded, and n-hexane and methyl tert-butyl ether were added again for precipitation. This process was repeated three times. The mixture was filtered to provide a solid product, which was dissolved in dichloromethane and methanol, then silica gel powder was added, and the mixture was evaporated to dryness. The resulting product was dry-loaded onto the column and subjected to column chromatography using 8-12% methanol/dichloromethane as the eluent. The desired product was collected, concentrated, and dried to provide 0.46 g of the product with a yield of 38.05%. $^1$H-NMR (600 MHz, DMSO-$d_6$) δ 8.48-8.40 (m, 3H), 8.21-8.13 (m, 6H), 8.11-8.05 (m, 3H), 7.94-7.88 (m, 3H), 7.81-7.74 (m, 3H), 7.58-7.53 (m, 3H), 7.42-7.29 (m, 33H), 7.28-7.22 (m, 3H), 5.09 (s, 6H), 5.02-4.97 (m, 6H), 4.31-4.19 (m, 6H), 4.13-4.09 (m, 6H), 4.05-3.98 (m, 3H), 3.30-3.25 (m, 3H), 3.03-2.94 (m, 27H), 2.82 (s, 15H), 2.28-2.22 (m, 12H), 2.19-2.14 (m, 6H), 2.13-2.08 (m, 6H), 2.04-1.99 (m, 6H), 1.93-1.87 (m, 9H), 1.76-1.66 (m, 18H), 1.50-1.44 (m, 9H), 1.40-1.38 (m, 108H), 1.36-1.31 (m, 12H), 1.28-1.19 (m, 15H); ESI [M+Na$^+$] 4183.41

9.12 Preparation of **68-130**

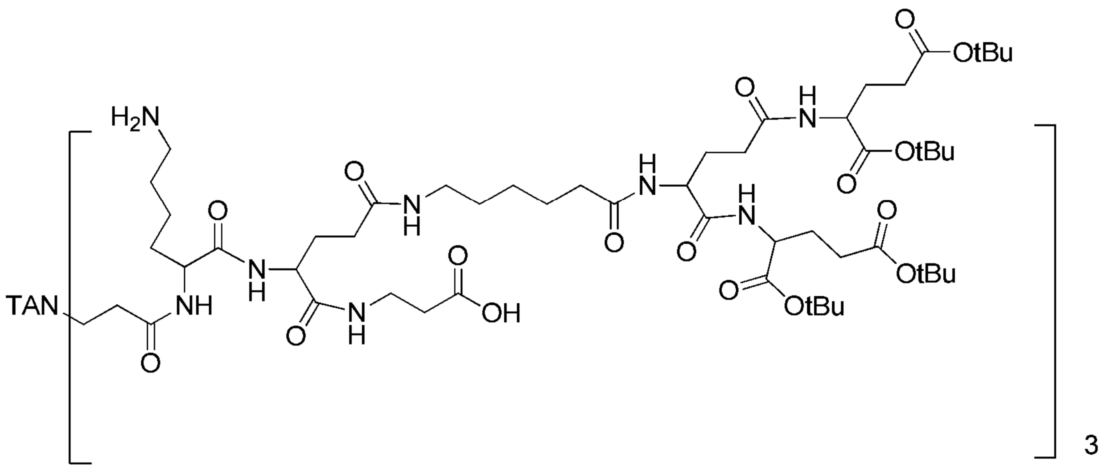

**68-127** (0.25 g, 0.0598 mmol) and 10% Pd/C catalyst (0.01 g) were placed in a hydrogenation reactor, and DMF (30 mL) was added for dissolution. The hydrogenation reactor was sealed, evacuated with a water pump, and filled with hydrogen gas. This process was repeated three times. The pressure reading of the hydrogenation reactor was adjusted to 1.8 MPa. The reaction was then carried out at room temperature overnight. After the reaction was completed, the reaction mixture was filtered through Celite, and the filter cake was washed with DMF (20 mL × 3). The DMF solution of the product was obtained and used as the raw material for the next reaction.

9.13 Preparation of **68-131**

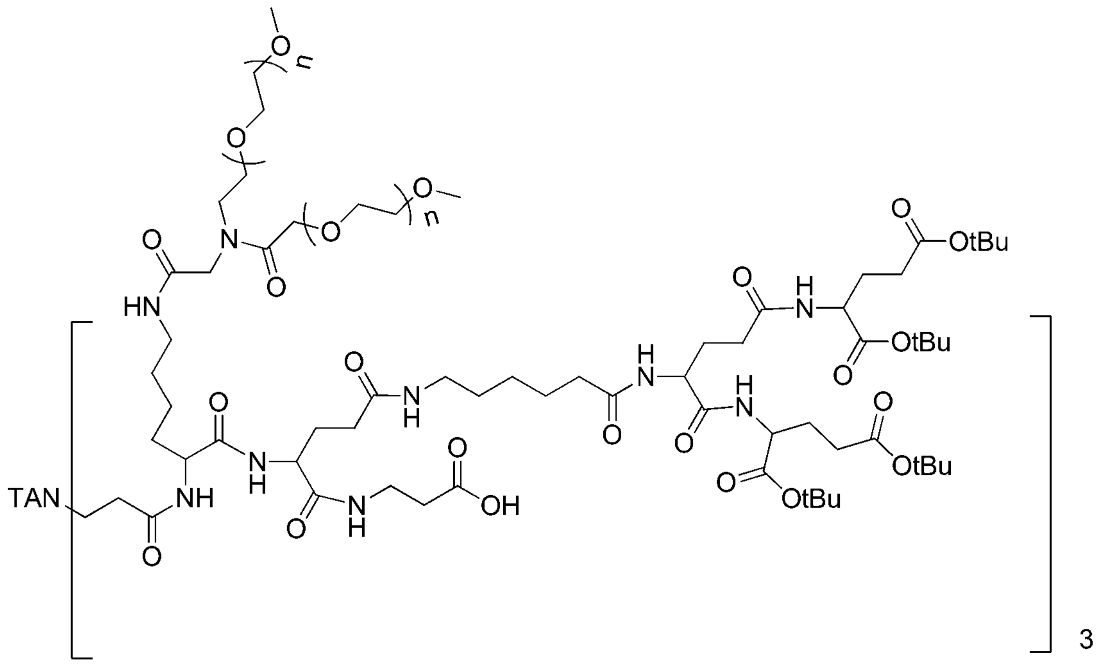

**68-130** (0.21 g, 0.0598 mmol) was placed in a 250 mL flask, then DMF (20 mL) was added for dissolution, and the flask was allowed to stand at -5°C. DIEA (0.13 mL, 0.0873 mmol) was slowly added dropwise. After the dropwise addition was completed, the mixture was stirred for 30 minutes. The flask was then taken out, Y-SCM-10K (2.0 g, 0.1975 mmol, purchased from Jenkem) was added, and the reaction was carried out with stirring in the dark at low speed at room temperature for one week. After the reaction was completed, n-hexane (25 mL) and methyl tert-butyl ether (200 mL) were added for precipitation. The supernatant was discarded, and n-hexane (20 mL) and methyl tert-butyl ether (100 mL) were added again for precipitation. This process was repeated three times. The mixture was filtered to provide a solid product, which was then washed by adding methyl tert-butyl ether (100 mL × 3). The solid was collected and dried in a vacuum oven to provide 2.1 g of the product.

9.14 Preparation of **68-156**

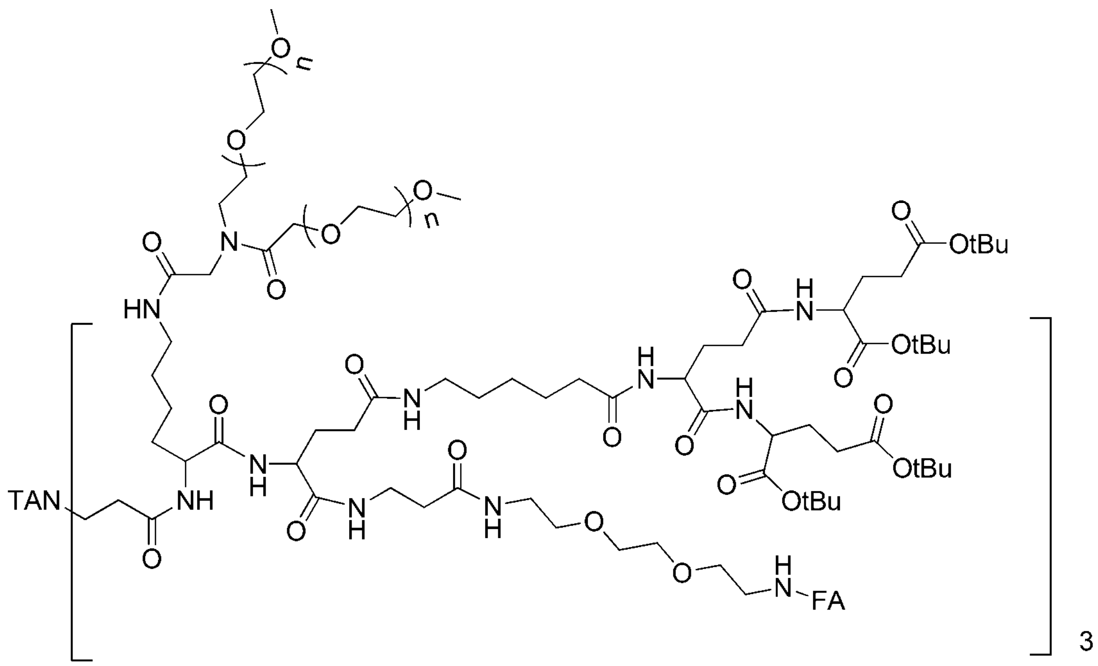

**68-131** (1 g, 0.0294 mmol), HBTU (0.066 g, 0.1763 mmol), and HOBT (0.024 g, 0.1763 mmol) were weighed and added to a flask containing **68-153** (0.10 g, 0.1763 mmol), then DMF (20 mL) was added to dissolve the mixture, and the flask was allowed to stand at -5°C. DIEA (0.058 mL, 0.3526 mmol) was slowly added dropwise. After the dropwise addition was completed, the reaction was carried out for half an hour, the flask was then taken out, and the reaction was carried out with stirring at room temperature overnight. After the reaction was completed, n-hexane (25 mL) and methyl tert-butyl ether (200 mL) were added for precipitation, the supernatant was discarded, and n-hexane and methyl tert-butyl ether were added again for precipitation. This process was repeated three times. The mixture was filtered to provide a solid product, which was dissolved in dichloromethane and methanol, then silica gel powder was added, and the mixture was evaporated to dryness. The resulting product was dry-loaded onto the column and subjected to column chromatography using 8-12% methanol/dichloromethane as the eluent. The desired product was collected, concentrated, and dried to provide 0.43 g of the product with a yield of 40.99%. $^1$H-NMR (600 MHz, DMSO-$d_6$) δ 8.51-8.38 (m, 3H), 8.20-8.11 (m, 6H), 8.11-7.95 (m, 9H), 7.95-7.89 (m, 5H), 7.81-7.75 (m, 7H), 7.56-7.52 (m, 3H), 7.38-7.30 (m, 2H), 7.21-7.17 (m, 3H), 7.11-7.01 (m, 16H), 4.29-4.20 (m, 14H), 4.13-4.09 (m, 14H), 3.99-3.87 (m, 9H), 3.56-3.49 (m, 3191H), 3.02-2.94 (m, 38H), 2.88-2.78 (m, 27H), 2.40-2.38 (m, 6H), 2.30-2.21 (m, 18H), 2.18-2.11 (m, 21H), 2.06-2.01 (m, 8H), 1.98-1.89 (m, 16H), 1.78-1.70 (m, 25H), 1.45-1.36 (m, 108H), 1.26-1.21 (m, 28H)

9.15 Preparation of **68-175**

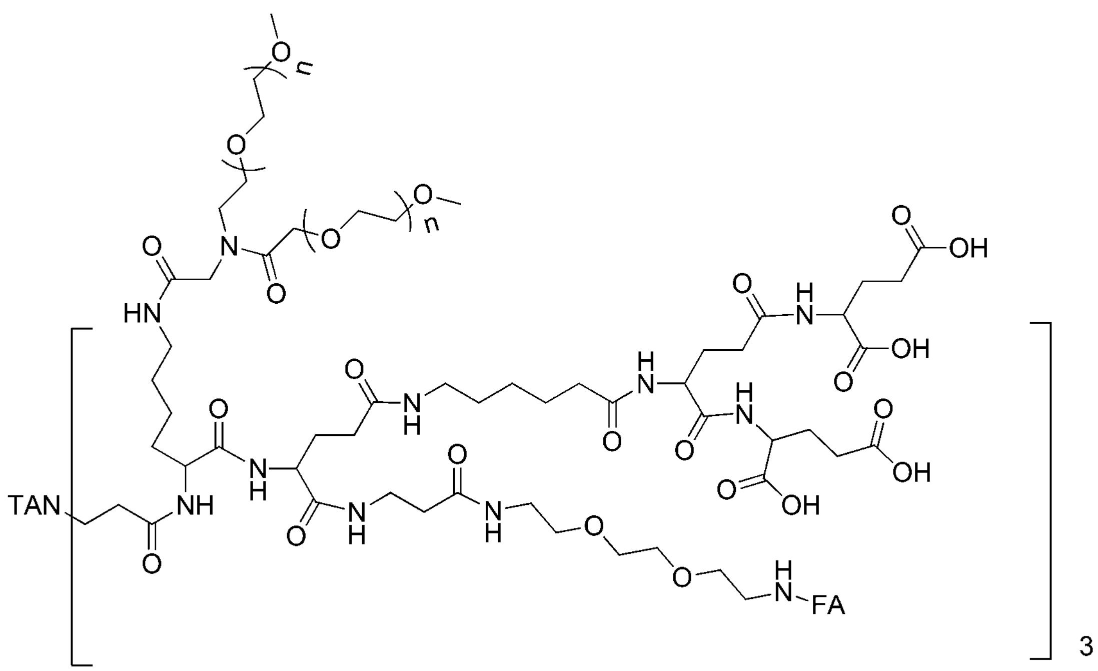

To a flask containing **68-156** (0.43 g, 0.0120 mmol), dichloromethane () was added. The mixture was dissolved with the assistance of ultrasonication, then TFA (0.2 mL) was added, and the mixture was reacted with stirring at room temperature overnight. After the reaction was completed, the reaction mixture was evaporated to an oily state using a rotary evaporator. Methyl tert-butyl ether (60 mL) was then added for precipitation. A powdery solid precipitated and was filtered. The filter cake was washed with methyl tert-butyl ether (40 mL × 3). The filter cake was collected and dried in a vacuum oven to provide the product.

9.16 Preparation of **68-180**

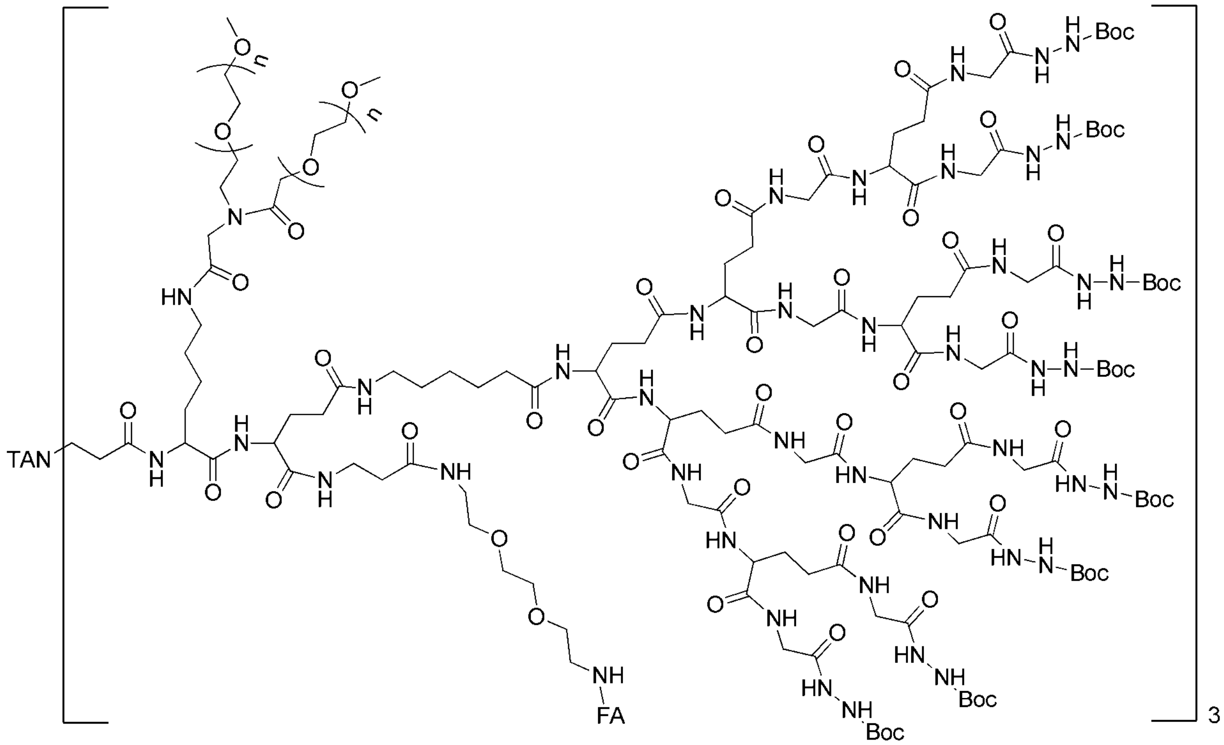

**68-175** (0.0120 mmol), HBTU (0.1 g, 0.2647 mmol), and HOBT (0.36 g, 0.2647 mmol) were weighed and added to a flask containing **68-179** (0.088 g, 0.1584 mmol), then DMF (10 mL) was added to dissolve the mixture, and the flask was allowed to stand at -5°C. DIEA (0.54 mL, 3.2481 mmol) was slowly added dropwise. After the dropwise addition was completed, the reaction was carried out for half an hour, the flask was then taken out, and the reaction was carried out with stirring at room temperature overnight. After the reaction was completed, n-hexane (25 mL) and methyl tert-butyl ether (200 mL) were added for precipitation. The supernatant was discarded, and n-hexane and methyl tert-butyl ether were added again for precipitation. This process was repeated three times. The mixture was filtered to provide a solid product, which was dissolved in dichloromethane and methanol, then silica gel powder was added, and the mixture was evaporated to dryness. The resulting product was dry-loaded onto the column and subjected to column chromatography using 5-12% methanol/dichloromethane as the eluent. The desired product was collected, concentrated, and dried to provide 0.32 g of the product.

9.17 Preparation of **68-193**

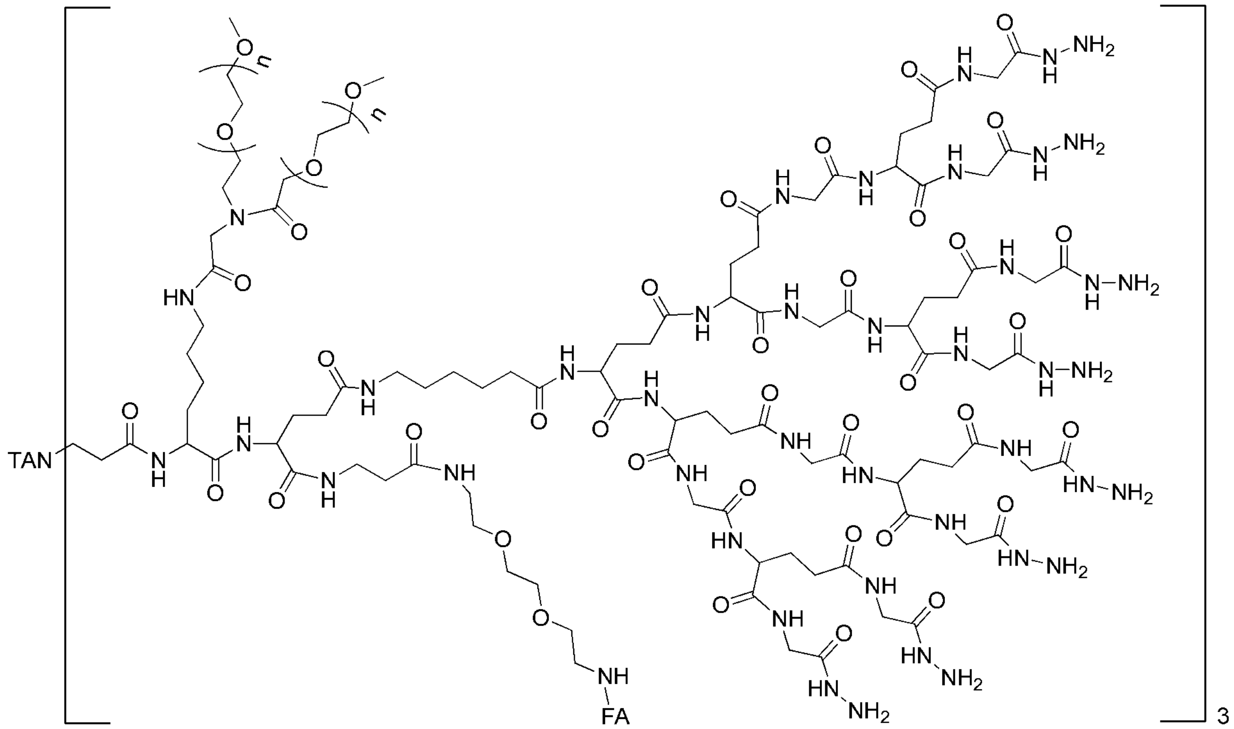

**68-180** (0.32 g, 0.0077 mmol) was added to a 250 mL flask, then dichloromethane (8 mL) and TFA (8 mL) were added for dissolution, and the mixture was reacted with stirring at room temperature overnight. After the reaction was completed, the reaction mixture was evaporated to an oily state using a rotary evaporator. Methyl tert-butyl ether (60 mL) was then added for precipitation. A powdery solid precipitated from the reaction mixture and was filtered. The filter cake was washed with methyl tert-butyl ether (40 mL × 3). The filter cake was collected and dried in a vacuum oven to provide 0.35 g of the product.

9.18 Preparation of **77-231**

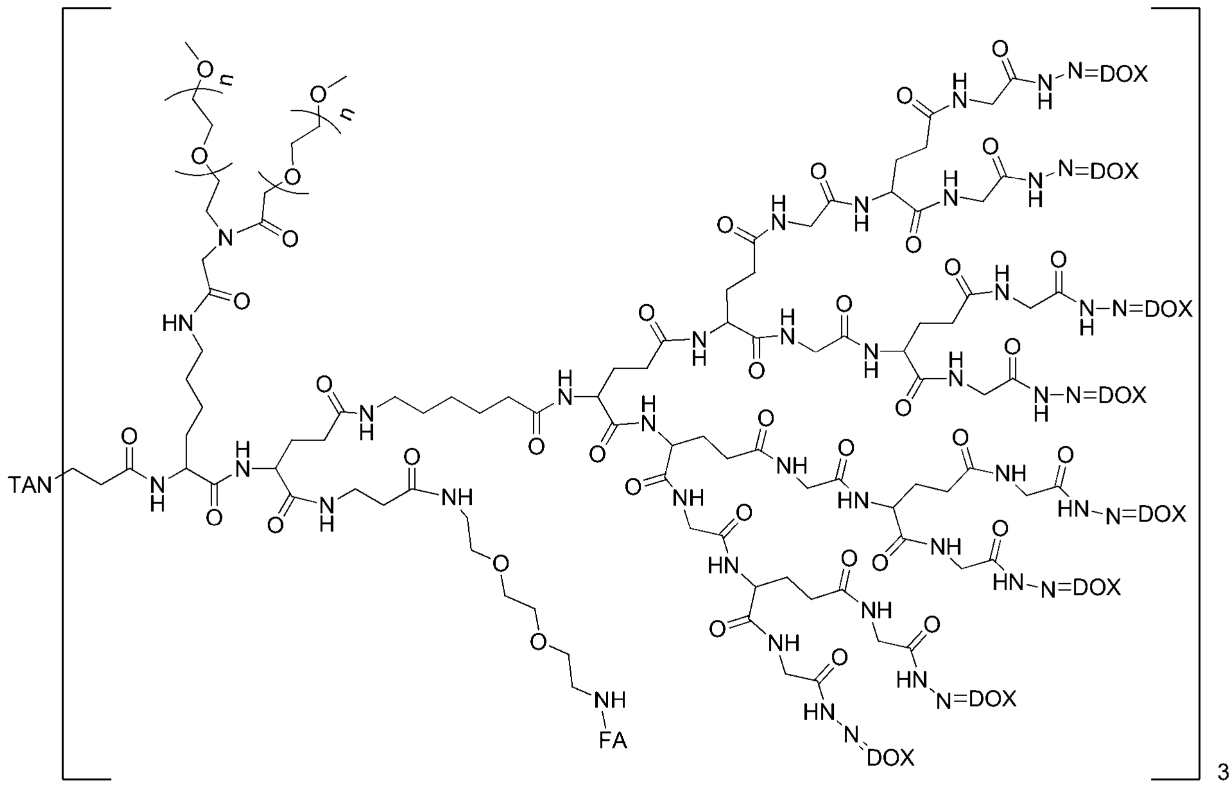

**68-193** (0.30 g, 0.0077 mmol) was placed in a dry 500 mL round-bottom flask, then anhydrous methanol (10 mL) was added for dissolution. TFA (0.27 mL, 3.6 mmol) and doxorubicin hydrochloride (0.16 g, 0.2772 mmol, referred to as DOX.HCl) were added, and the mixture was reacted with stirring at room temperature overnight. After the reaction was completed, n-hexane (25 mL) and methyl tert-butyl ether (200 mL) were added for precipitation, the supernatant was discarded, and n-hexane and methyl tert-butyl ether were added again for precipitation. This process was repeated three times. The mixture was filtered to provide a solid product. The obtained solid product was dissolved in dichloromethane and methanol, then silica gel powder was added, and the mixture was evaporated to dryness. The resulting product was dry-loaded onto the column and subjected to column chromatography using 6-10% methanol/dichloromethane as the eluent. The desired product was collected, concentrated, and dried to provide 0.13 g of the product with a yield of 32.02%. $^1$H-NMR (600 MHz, DMSO-$d_6$) δ 14.10-14.00 (m, 24H), 13.40-13.21 (m, 24H), 8.51-8.38 (m, 3H), 8.20-8.11 (m, 6H), 8.11-7.95 (m, 90H), 7.96-7.89 (m, 55H), 7.78-7.61 (m, 33H), 7.53-7.34 (m, 6H), 7.10-7.01 (m, 16H), 5.5-5.21(m, 73H), 5.01-4.26 (m, 108H), 4.19-4.01 (m, 84H), 3.96-3.75 (m, 32H), 3.58-3.49 (m, 3524H), 3.01-2.95 (m, 81H), 2.85-2.81 (m, 27H), 2.40-2.14 (m, 80H), 2.06-2.01 (m, 8H), 1.91-1.70 (m, 42H), 1.25-1.19 (m, 76H)

Example 10: Synthesis of Compound **82-183**

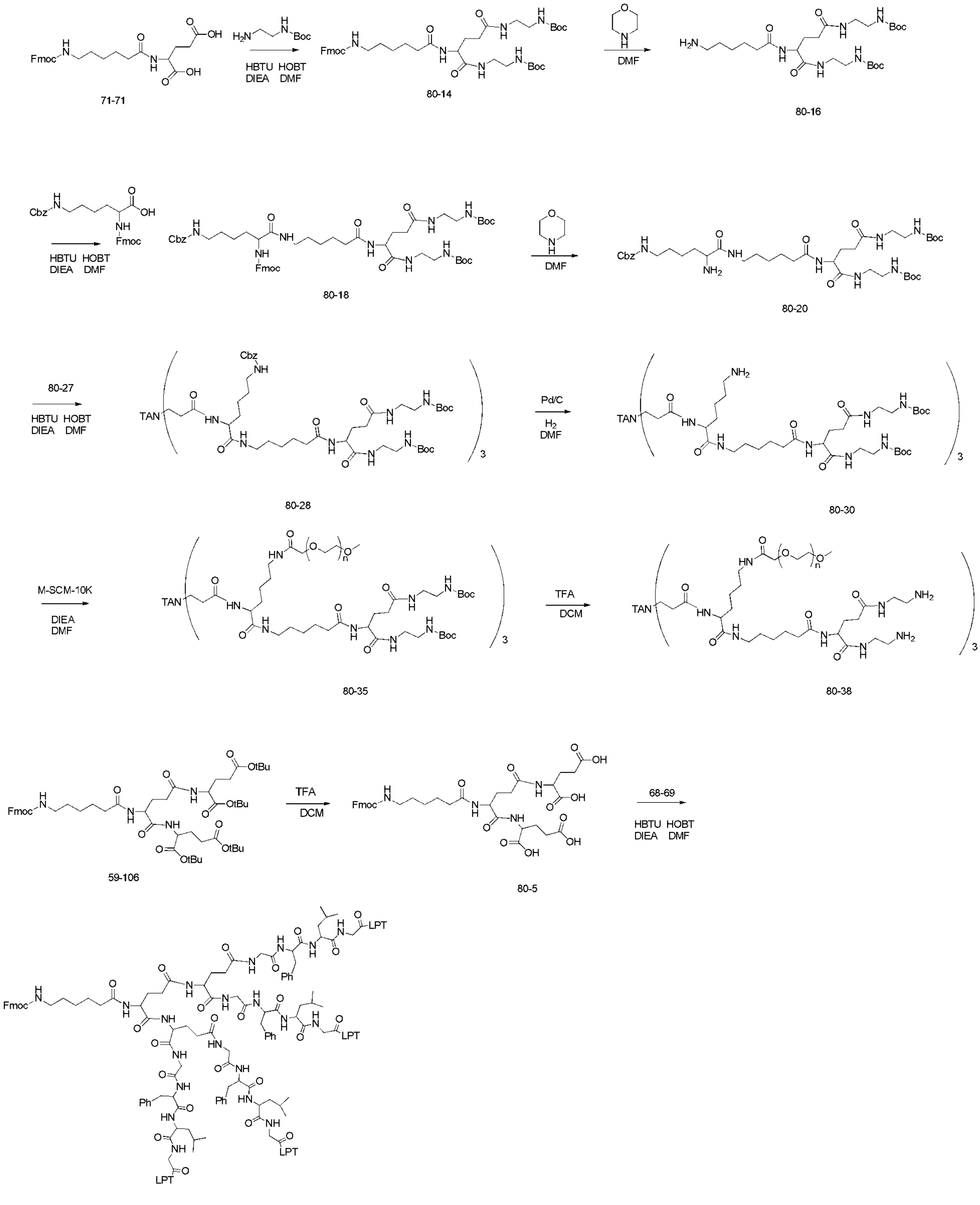
71-71
HBTU HOBT
DIEA DMF
80-14
DMF
80-16
HBTU HOBT
DIEA DMF
80-18
DMF
80-20
80-27
HBTU HOBT
DIEA DMF
80-28
Pd/C
$H_2$
DMF
80-30
M-SCM-10K
DIEA
DMF
80-35
TFA
DCM
80-38
59-106
TFA
DCM
80-5
68-69
HBTU HOBT
DIEA DMF
80-7

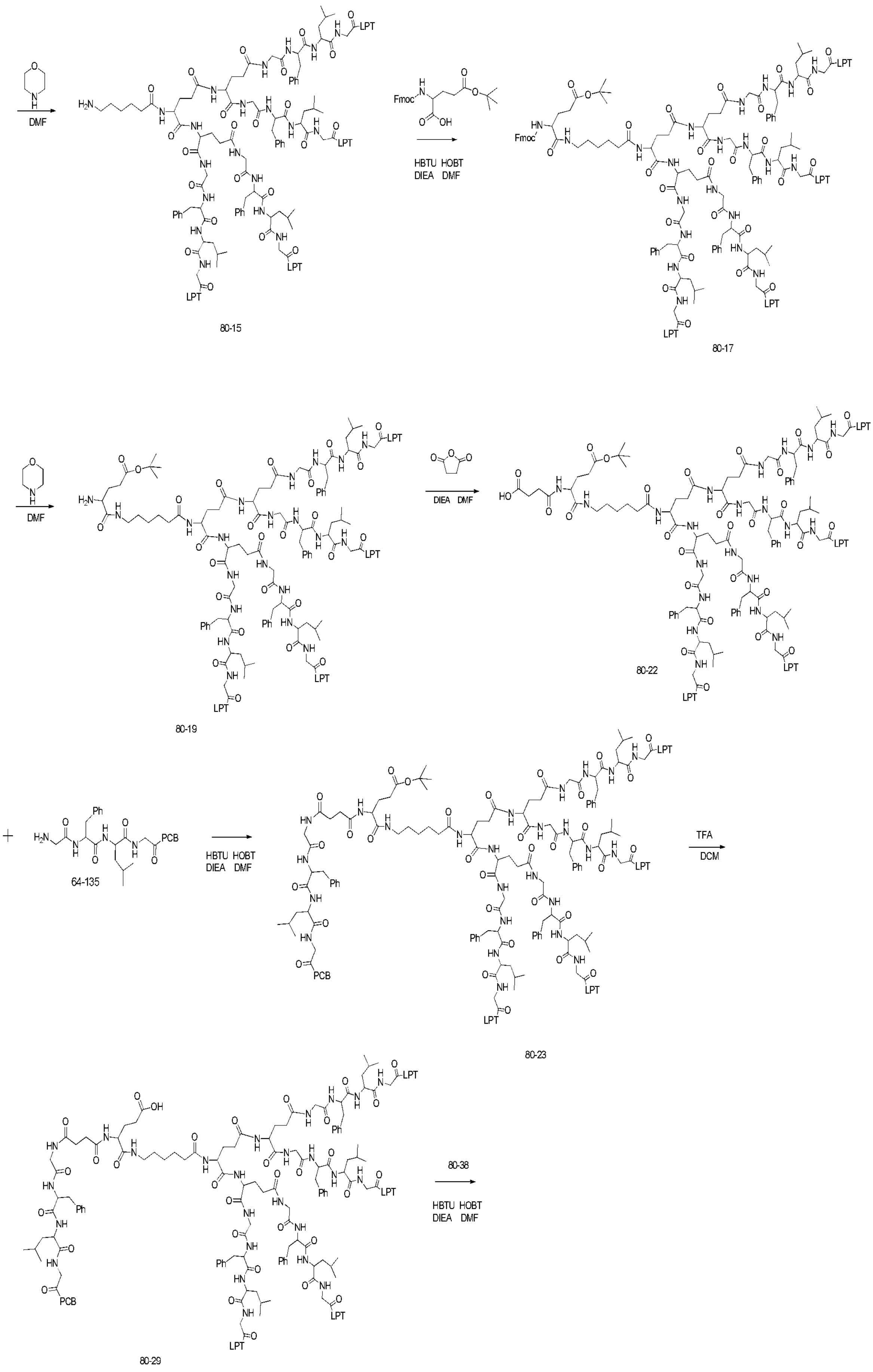


80-15

80-17

80-19

80-22

64-135

80-23

80-29

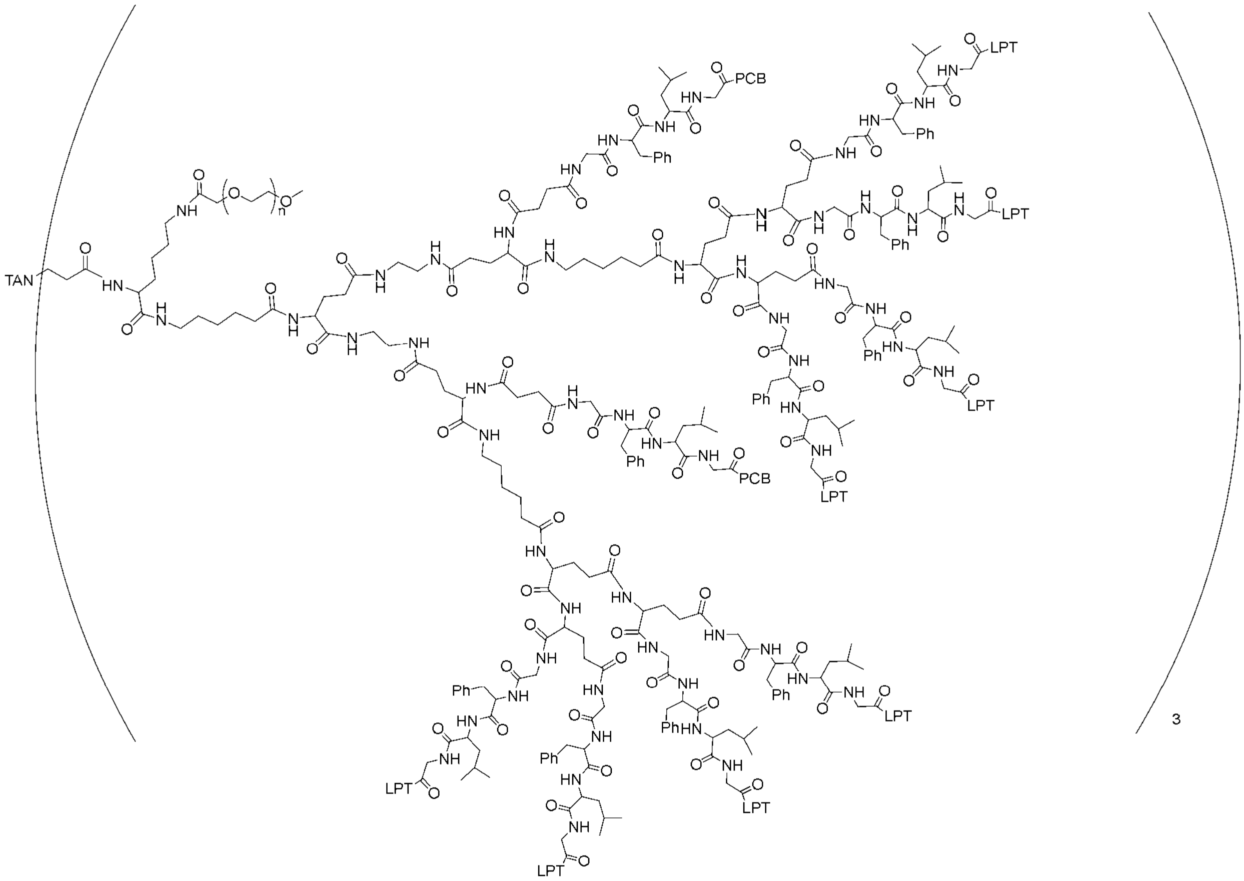

82-183

10.1 Preparation of **80-14**

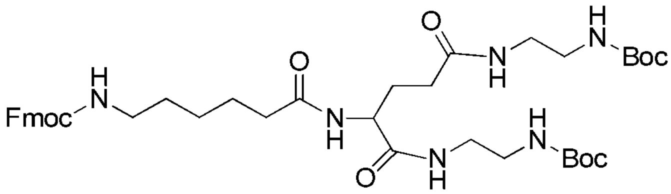

Boc-ethylenediamine (1.65 g, 10.3 mmol), HBTU (5.57 g, 14.7 mmol), and HOBT (1.99 g, 14.7 mmol) were weighed and added to a flask containing **71-71** (4 g, 8.2896 mmol), then DMF (200 mL) was added to dissolve the mixture, and the mixture was stirred at -5°C for several minutes. DIEA (7.3 mL, 44.1 mmol) was slowly added dropwise. After the dropwise addition was completed, the reaction was carried out for half an hour, the flask was then taken out, and the reaction was carried out with stirring at room temperature overnight. After the reaction was completed, saturated brine (200 mL) and ethyl acetate (200 mL) were added for extraction. After standing for phase separation, the organic phase was collected, and the aqueous phase was further extracted with ethyl acetate (200 mL × 3). The organic phases were combined, concentrated, evaporated to dryness, and dried in a vacuum oven to provide 3.76 g of the product with a yield exceeding 100%.

10.2 Preparation of **80-16**

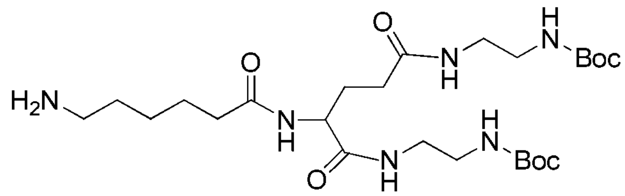

To a flask containing **80-14** (4.9 mmol), DMF (30 mL) was added for dissolution with the assistance of ultrasonication, then morpholine (5.5 mL, 73.5 mmol) was added, and the mixture was reacted with stirring at room temperature for 2 hours. After the reaction was completed, saturated brine (200 mL) and ethyl acetate (200 mL) were added for extraction. After standing for phase separation, the organic phase was collected, and the aqueous phase was further extracted with ethyl acetate (3 times 200 mL). The organic phases were combined, evaporated to dryness and dried in a vacuum oven to provide 2.67 g of the product with a yield exceeding 100%.

10.3 Preparation of **80-18**

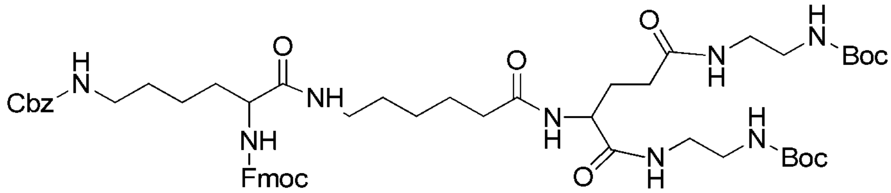

**80-16** (2.67 g, 4.9 mmol), HBTU (2.79 g, 7.35 mmol), and HOBT (0.99 g, 7.35 mmol) were weighed and added to a flask containing N'-Fmoc-N-benzyloxycarbonyl-L-lysine (2.46 g, 4.9 mmol, purchased from Innochem), then DMF (60 mL) was added to dissolve the mixture, and the flask was allowed to stand at -5°C. DIEA (2.5 mL, 14.7 mmol) was slowly added dropwise. After the dropwise addition was completed, the reaction was carried out for half an hour, the flask was then taken out, and the reaction was carried out with stirring at room temperature overnight. After the reaction was completed, n-hexane (25 mL) and methyl tert-butyl ether (200 mL) were added for precipitation. The supernatant was discarded, and n-hexane and methyl tert-butyl ether were added again for precipitation. This process was repeated three times. The mixture was filtered to provide a solid product, which was dissolved in dichloromethane and methanol, then silica gel powder was added, and the mixture was evaporated to dryness. The resulting product was dry-loaded onto the column and subjected to column chromatography using 8-12% methanol/dichloromethane as the eluent. The desired product was collected, concentrated, and dried to provide 3.66 g of the product with a yield of 72%.

10.4 Preparation of **80-20**

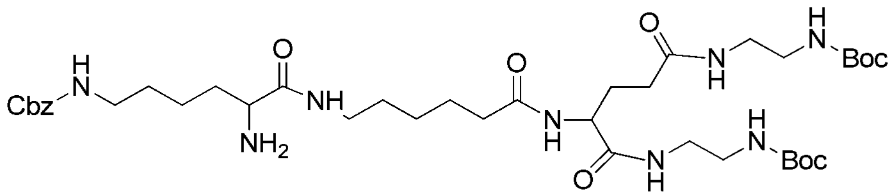

To a flask containing **80-18** (3.56 mmol), DMF (60 mL) was added for dissolution with the assistance of ultrasonication, then morpholine (6.7 mL, 71.2 mmol) was added, and the mixture was reacted with stirring at room temperature for 2 hours. After the reaction was completed, saturated brine (200 mL) and ethyl acetate (200 mL) were added for extraction. After standing for phase separation, the organic phase was collected, and the aqueous phase was further extracted with ethyl acetate (200 mL × 3). The organic phases were combined, evaporated to provide a solid, which was dried in a vacuum oven to provide 2.25 g of the product with a yield of 78%.

10.5 Preparation of **80-28**

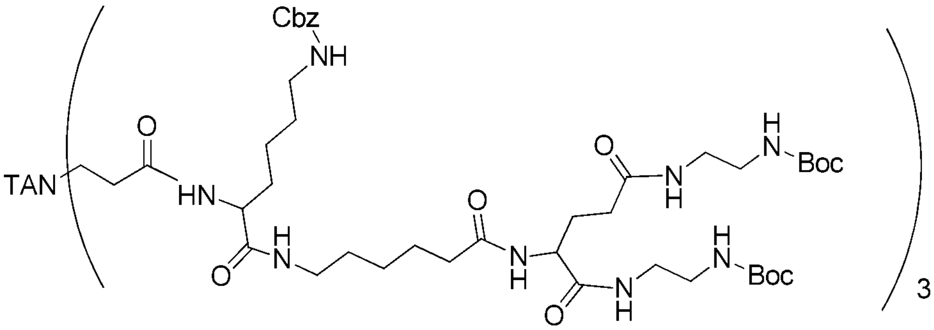

**80-20** (0.72 g, 0.8975 mmol), HBTU (0.494 g, 1.302 mmol), and HOBT (0.176 g, 1.302 mmol) were weighed and added to a flask containing **80-27** (0.1 g, 0.2895 mmol), then DMF (20 mL) was added to dissolve the mixture, and the mixture was stirred at -5°C for several minutes. DIEA (0.72 mL, 4.34 mmol) was slowly added dropwise. After the dropwise addition was completed, the reaction was carried out for half an hour, the flask was then taken out, and the reaction was carried out with stirring at room temperature overnight. After the reaction was completed, n-hexane (25 mL) and methyl tert-butyl ether (200 mL) were added for precipitation, the supernatant was discarded, and n-hexane and methyl tert-butyl ether were added again for precipitation. This process was repeated three times. The mixture was filtered to provide a solid product, which was dissolved in dichloromethane and methanol, then silica gel powder was added, and the mixture was evaporated to dryness. The resulting product was dry-loaded onto the column and subjected to column chromatography using 8-12% methanol/dichloromethane as the eluent. The desired product was collected, concentrated, and dried to provide 0.47 g of the product with a yield of 59%. $^1$H-NMR (600 MHz, DMSO-*d$_6$*) δ 8.13-7.71 (m, 15H), 7.43-7.15 (m, 18H), 6.94-6.67 (m, 6H), 5.10-4.90 (m, 6H), 4.36-3.91 (m, 6H), 3.24-2.89 (m, 48H), 2.86-2.77 (m, 7H), 2.72-2.62 (m, 9H), 2.16-2.01 (m, 13H), 1.89-1.82 (m, 4H), 1.77-1.65 (m, 4H), 1.61-1.54 (m, 3H), 1.51-1.43 (m, 12H), 1.42-1.31 (m, 54H), 1.29-1.15 (m, 14H); ESI [M+H$^+$] 2713.99

10.6 Preparation of **80-30**

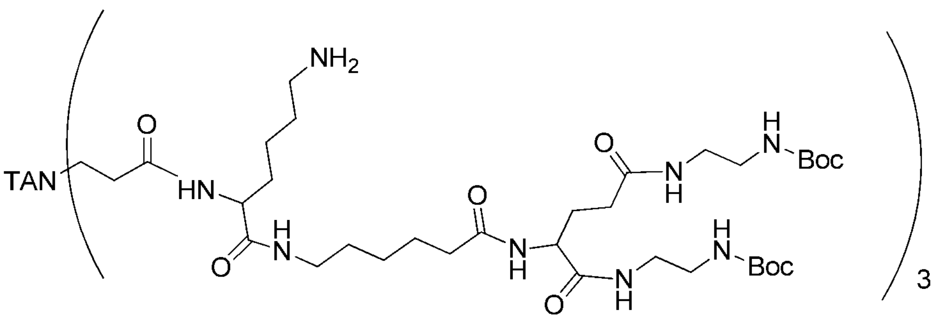

**80-28** (0.47 g, 2.2137 mmol) and 10% Pd/C catalyst (0.04 g) were placed in a hydrogenation reactor, and DMF (30 mL) was added for dissolution. The hydrogenation reactor was sealed, evacuated with a water pump, and then filled with hydrogen gas. This process was repeated three times. The pressure reading of the hydrogenation reactor was adjusted to 2 MPa. The reaction was carried out at room temperature overnight. After the reaction was completed, the reaction mixture was filtered through Celite, and the filter cake was washed with DMF (20 mL × 3). The DMF solution of the product was obtained and used as the raw material for the next reaction.

10.7 Preparation of **80-35**

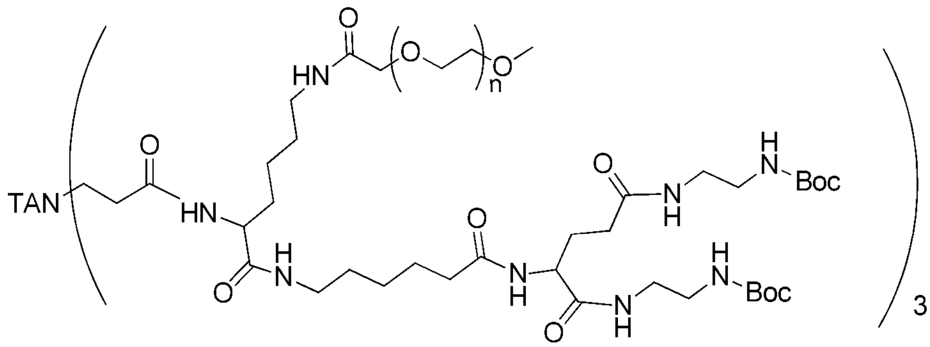

**80-30** (0.1 g, 0.043 mmol) was placed in a 250 mL flask. DMF (20 mL) was added for dissolution, and then DIEA (0.118 mL, 0.714 mmol) was slowly added dropwise at -5°C. After the dropwise addition was completed, the reaction was carried out for 30 minutes, the flask was then taken out, M-SCM-10K (1.49 g, 0.143 mmol, purchased from Jenkem) was added, and the reaction was carried out with stirring at low speed in the dark at room temperature for one week. After the reaction was completed, n-hexane (25 mL) and methyl tert-butyl ether (200 mL) were added for precipitation. The supernatant was discarded, and n-hexane (20 mL) and methyl tert-butyl ether (100 mL) were added again for precipitation. This process was repeated three times. The mixture was filtered to provide a solid product, which was filtered and washed three times with methyl tert-butyl ether (100 mL). The solid was collected and dried in a vacuum oven to provide 1.0 g of the product with a yield of 71%.

10.8 Preparation of **80-38**

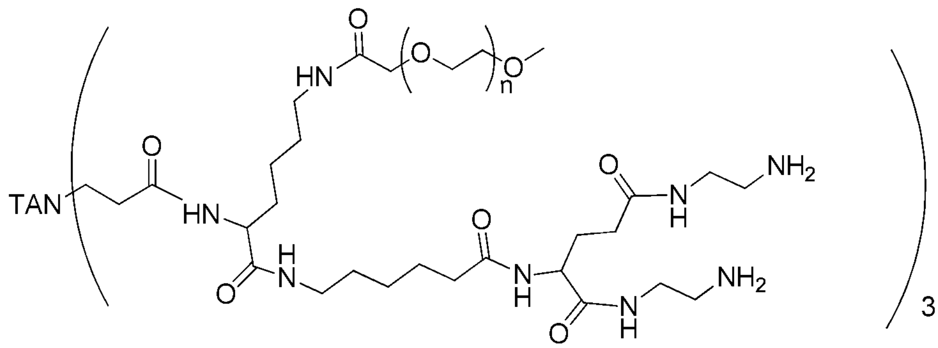

**80-35** (0.5 g, 0.0154 mmol) was added to a 250 mL flask, then dichloromethane (8 mL) and TFA (10 mL, 26.9 mmol) were added for dissolution, and the mixture was reacted with stirring at room temperature overnight. After the reaction was completed, the reaction mixture was evaporated to an oily state using a rotary evaporator. Methyl tert-butyl ether (60 mL) was then added for precipitation. A powdery solid precipitated from the reaction mixture and filtered. The filter cake was washed with methyl tert-butyl ether (40 mL × 3). The filter cake was collected and dried in a vacuum oven to provide 0.5 g of the product with a yield exceeding 100%.

10.9 Preparation of **80-5**

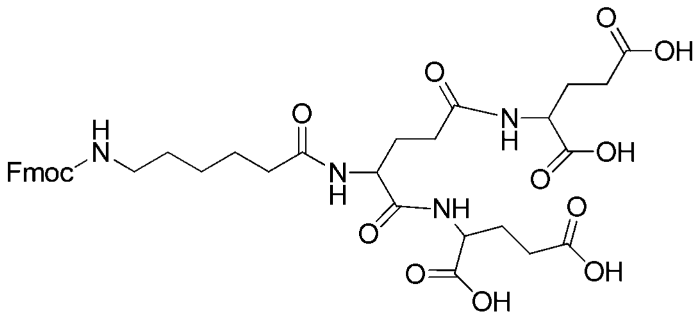

**59-106** (60.13 g, 6.35 mmol) was added to a 250 mL flask, then dichloromethane (8 mL) and TFA (43.43 mL, 381 mmol) were added for dissolution, and the mixture was reacted with stirring at room temperature overnight. After the reaction was completed, the reaction mixture was evaporated to an oily state using a rotary evaporator. Methyl tert-butyl ether (60 mL) was then added for precipitation. A powdery solid precipitated from the reaction mixture and was filtered. The filter cake was washed with methyl tert-butyl ether (40 mL × 3). The filter cake was collected and dried in a vacuum oven to provide 5.1 g of the product with a yield exceeding 100%.

10.10 Preparation of **80-7**

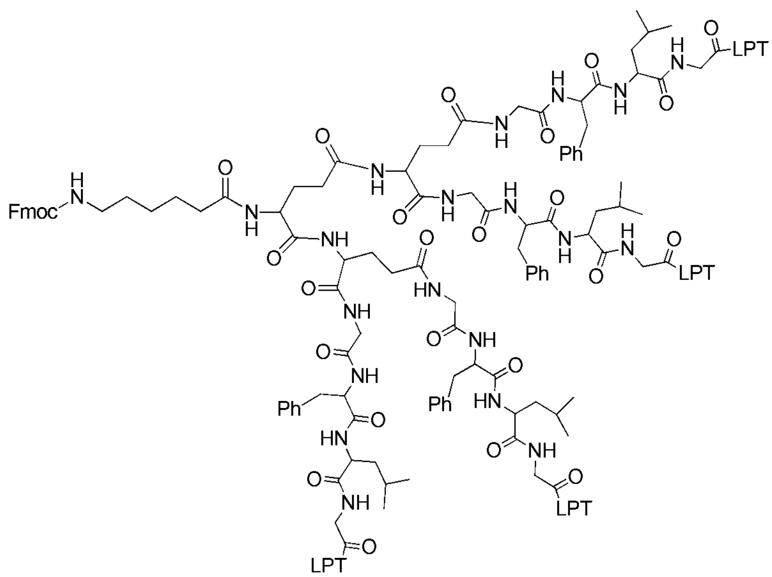

**80-5** (0.828 g, 1.43 mmol), **68-54** (2.0 g, 7.15 mmol), and NMM (1.6 mL, 14.3 mmol) were weighed and added to a flask containing **68-69** (6.0 g, 6.29 mmol), then DMF (50 mL) was added to dissolve the mixture, and the mixture was reacted with stirring at room temperature overnight. After the reaction was completed, n-hexane (25 mL) and methyl tert-butyl ether (200 mL) were added for precipitation. The supernatant was discarded, and n-hexane and methyl tert-butyl ether were added again for precipitation. This process was repeated three times. The mixture was filtered to provide a solid product, which was dried in a vacuum oven to provide 6.24 g of the product with a yield exceeding 100%.

10.11 Preparation of **80-15**

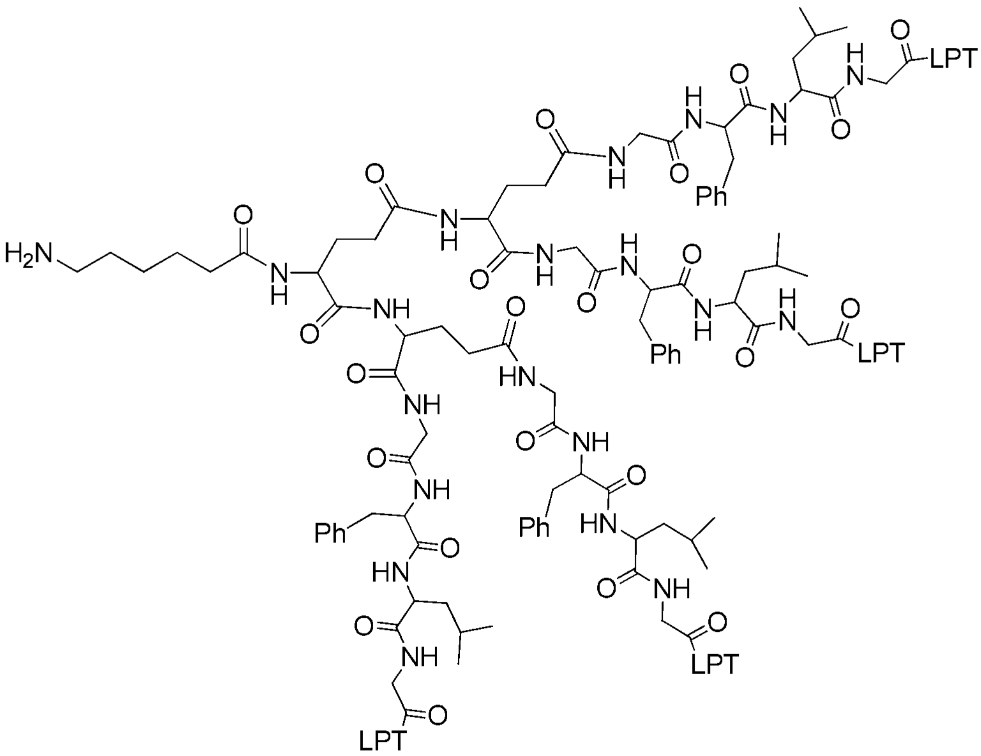

**80-7** (6.24 g, 1.43 mmol) was placed in a 250 mL flask, then DMF (20 mL) and morpholine (1.1 mL, 14.3 mmol) were added for dissolution, and the mixture was reacted with stirring at room temperature overnight. After the reaction was completed, methyl tert-butyl ether (60 mL) was added for precipitation. A powdery solid precipitated from the reaction mixture and was filtered. The filter cake was washed with methyl tert-butyl ether (40 mL × 3). The filter cake was collected and dried in a vacuum oven to provide 3.8 g of the product with a yield of 62%. ESI [M+Na$^+$] 4291

10.12 Preparation of **80-17**

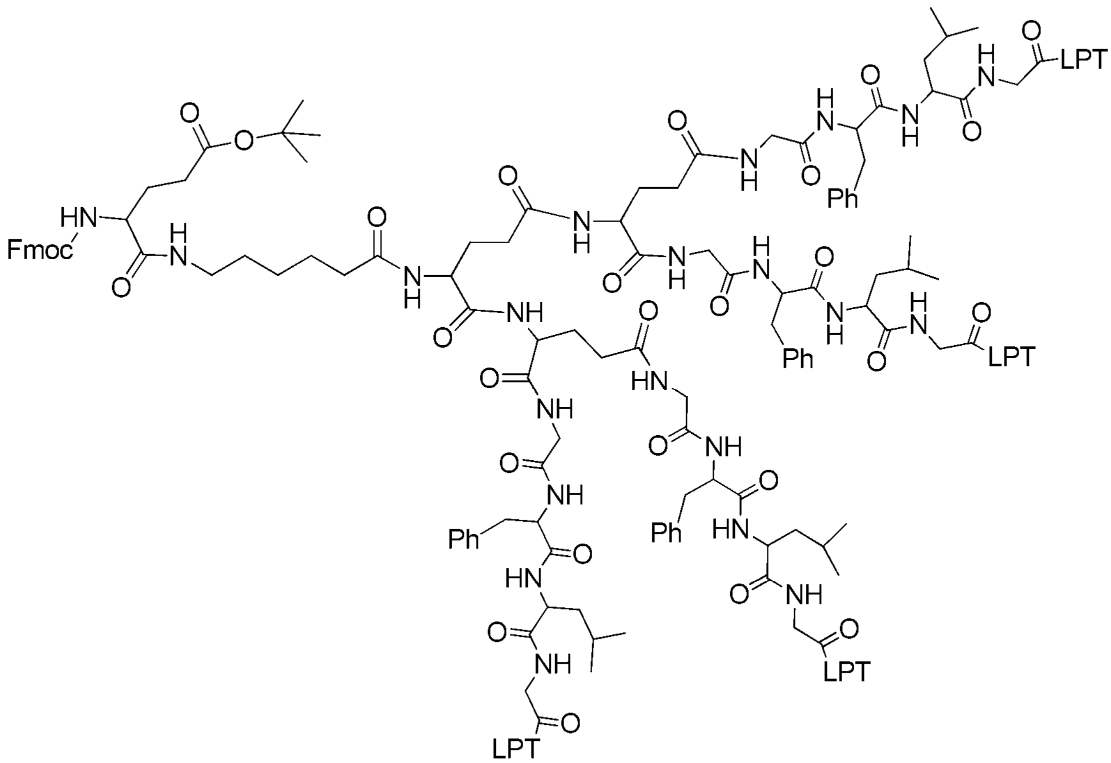

**80-15** (1.0 g, 0.234 mmol), HBTU (0.13 g, 0.351 mmol), HOBT (0.05 g, 0.351 mmol), and Fmoc-Glu(OtBu)-OH (0.1 g, 0.234 mmol) were weighed and placed in a flask, then DMF (15 mL) was added for dissolution, and the mixture was stirred at -5°C for several minutes. DIEA (0.12 mL, 0.702 mmol) was slowly added dropwise. After the dropwise addition was completed, the reaction was carried out for half an hour, the flask was then taken out, and the reaction was carried out with stirring at room temperature overnight. After the reaction was completed, n-hexane (25 mL) and methyl tert-butyl ether (200 mL) were added for precipitation. The supernatant was discarded, and n-hexane and methyl tert-butyl ether were added again for precipitation. This process was repeated three times. The mixture was filtered to provide a solid product, which was dissolved in dichloromethane and methanol, then silica gel powder was added, and the mixture was evaporated to dryness. The resulting product was then dry-loaded onto the column and subjected to column chromatography using 6-10% methanol/dichloromethane as the eluent. The desired product was collected, concentrated, and dried to provide 0.9 g of the product with a yield of 82%.

10.13 Preparation of **80-19**

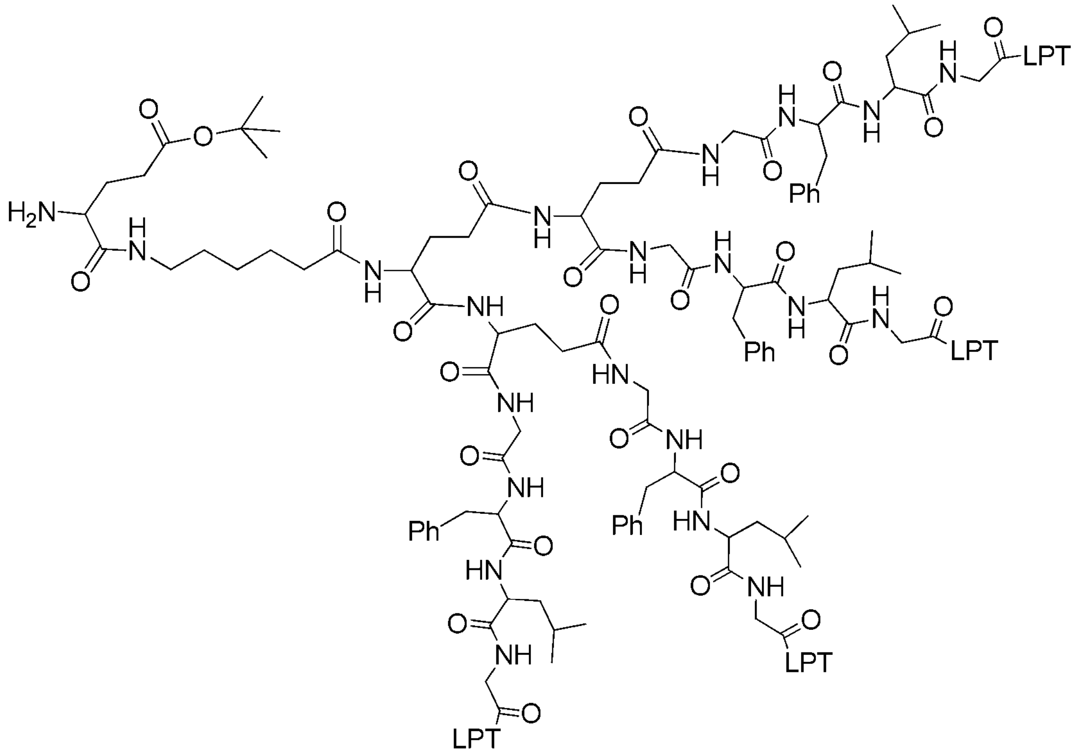

To a flask containing **80-17** (0.9 g, 0.19 mmol), DMF (10 mL) was added for dissolution with the assistance of ultrasonication, then morpholine (0.28 mL, 3.85 mmol) was added, and the mixture was reacted with stirring at room temperature for 2 hours. After the reaction was completed, n-hexane (25 mL) and methyl tert-butyl ether (200 mL) were added for precipitation. The supernatant was discarded, and n-hexane and methyl tert-butyl ether were added again for precipitation. This process was repeated three times. The mixture was filtered to provide a solid product, which was dried in a vacuum oven to provide 0.8 g of the product with a yield of 94%.

10.14 Preparation of **80-22**

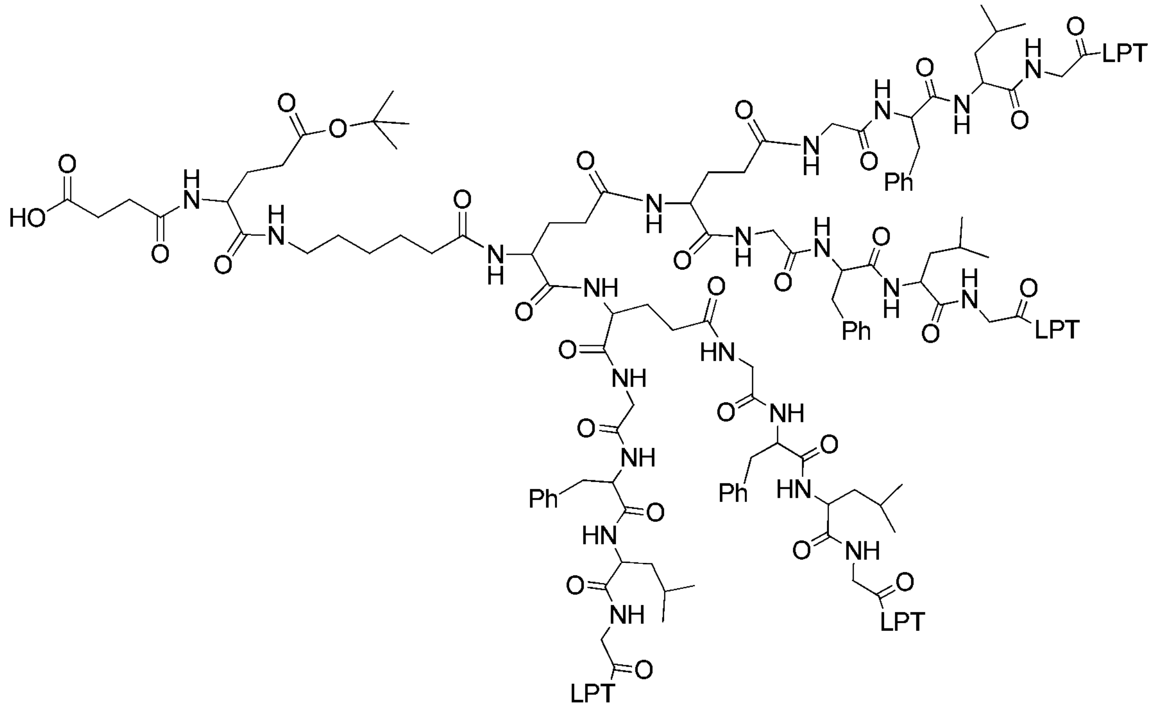

**80-19** (0.8 g, 0.18 mmol) was weighed and added to a 250 mL flask, and then DMF (15 mL) was added for dissolution. DIEA (0.13 mL, 0.81 mmol) was slowly added dropwise. After the dropwise addition was completed, the reaction was carried out with stirring at room temperature for 1 h, and succinic anhydride (0.1 g, 1.08 mmol) was added. After the reaction was completed, n-hexane (25 mL) and methyl tert-butyl ether (200 mL) were added for precipitation. The supernatant was discarded, and n-hexane and methyl tert-butyl ether were added again for precipitation. This process was repeated three times. The mixture was filtered to provide a solid product, which was dried in a vacuum oven to provide 0.8 g of the product with a yield of 97%.

10.15 Preparation of **80-23**

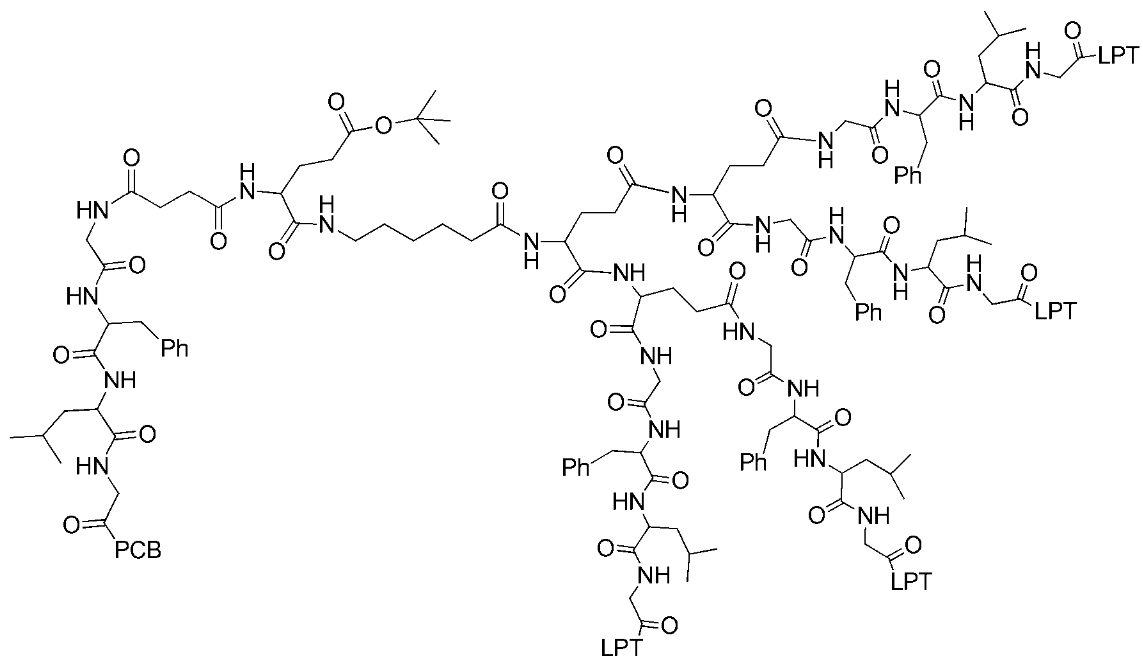

**80-22** (0.8 g, 0.175 mmol), HBTU (0.098 g, 0.26 mmol), and HOBT (0.035 g, 0.26 mmol) were weighed and added to a flask containing **64-135** (0.156 g, 0.19 mmol), then DMF (20 mL) was added to dissolve the mixture, and the mixture was stirred at -5°C for several minutes. DIEA (0.087 mL, 0.53 mmol) was slowly added dropwise. After the dropwise addition was completed, the reaction was carried out for half an hour, the flask was then taken out, and the reaction was carried out with stirring at room temperature overnight. After the reaction was completed, n-hexane (25 mL) and methyl tert-butyl ether (200 mL) were added for precipitation. The supernatant was discarded, and n-hexane and methyl tert-butyl ether were added again for precipitation. This process was repeated three times. The mixture was filtered to provide a solid product, which was collected, and dried in a vacuum oven to provide 0.9 g of the product with a yield exceeding 100%. ESI $[M+Na^+]$ 5380

10.16 Preparation of **80-29**

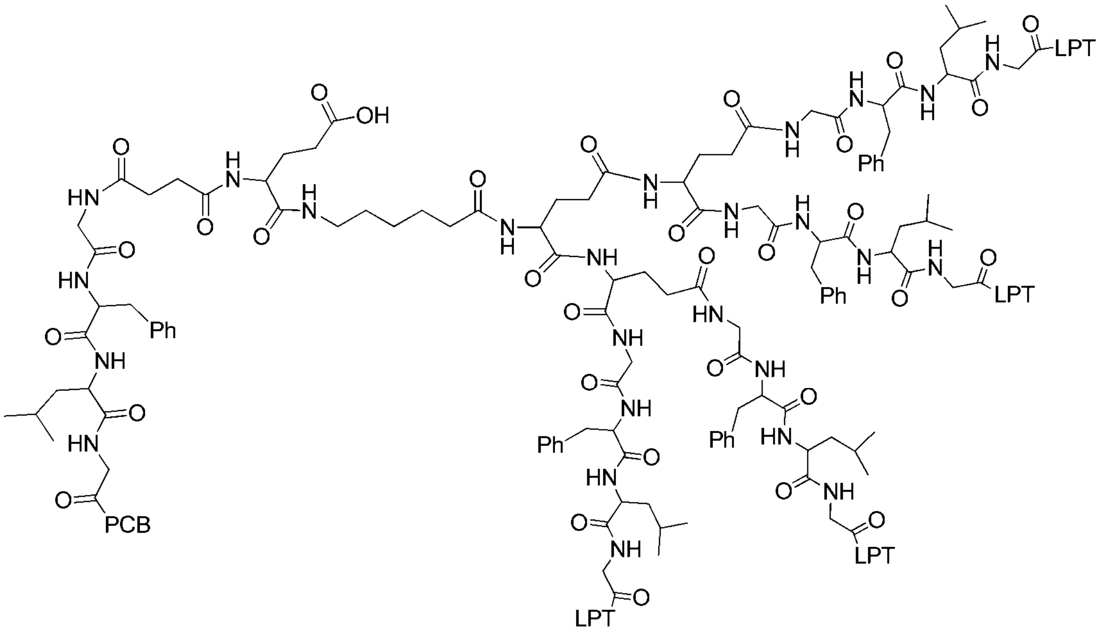

**80-23** (0.9 g, 0.168 mmol) was added to a 250 mL flask, then dichloromethane (20 mL) and TFA (5 mL) were added for dissolution, and the mixture was reacted with stirring at room temperature overnight. After the reaction was completed, the reaction mixture was evaporated to an oily state using a rotary evaporator. Methyl tert-butyl ether (60 mL) was then added for precipitation. A powdery solid precipitated from the reaction mixture and was filtered. The filter cake was washed with methyl tert-butyl ether (40 mL × 3), collected, and dried in a vacuum oven to provide 0.79 g of the product (88%).

10.17 Preparation of **82-183**

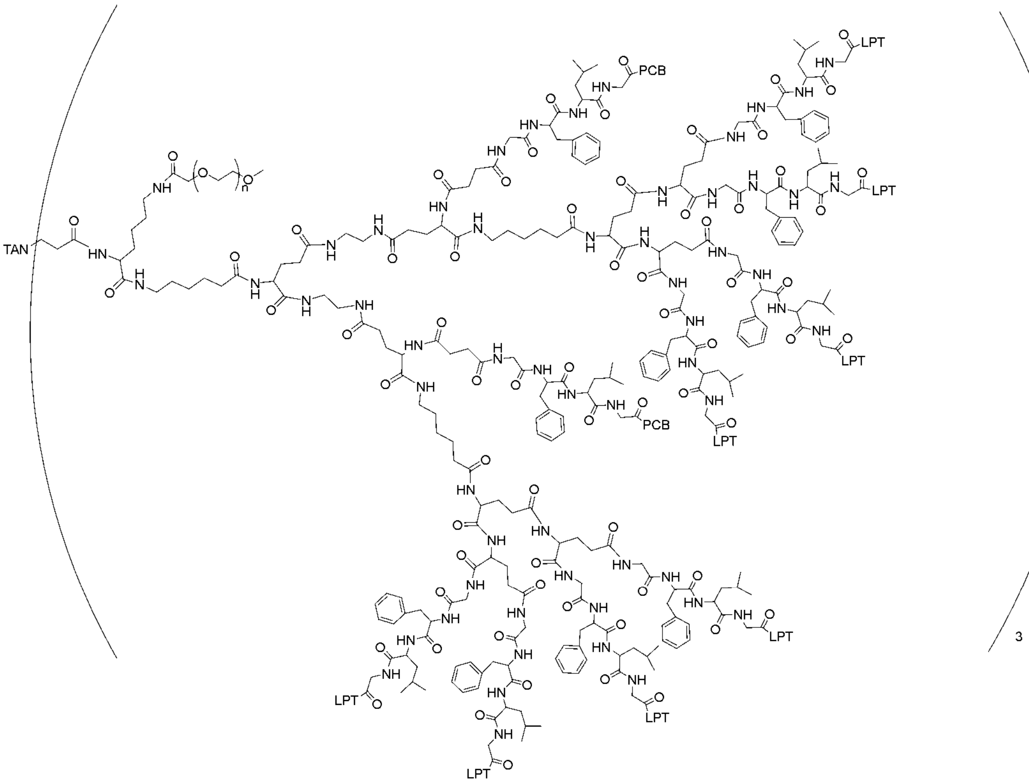

**80-29** (0.219 g, 0.0414 mmol), HBTU (0.02 g, 0.0565 mmol), and HOBT (0.007 g, 0.0565 mmol) were weighed and added to a flask containing **80-38** (0.2 g, 0.00628 mmol), then DMF (20 mL) was added to dissolve the mixture, and the mixture was stirred at -5°C for several minutes. DIEA (0.003 mL, 0.1695 mmol) was slowly added dropwise. After the dropwise addition was completed, the reaction was carried out for half an hour, the flask was then taken out, and the reaction was carried out with stirring at room temperature overnight. After the reaction was completed, n-hexane (25 mL) and methyl tert-butyl ether (200 mL) were added for precipitation, the supernatant was discarded, and n-hexane and methyl tert-butyl ether were added again for precipitation. This process was repeated three times. The mixture was filtered to provide a solid product, which was dissolved in dichloromethane and methanol, then silica gel powder was added, and the mixture was evaporated to dryness. The resulting product was dry-loaded onto the column and subjected to column chromatography using 6-10% methanol/dichloromethane as the eluent. The desired product was collected, concentrated, and dried to provide 0.2 g of the product with a yield of 51.3%. $^1$H-NMR (600 MHz, DMSO-$d_6$) δ 8.82-8.68 (m, 24H), 8.63-8.49 (m, 24H), 8.23-7.96 (m, 192H), 7.91-7.76 (m, 36H), 7.77-7.64 (m, 24H), 7.55-7.39 (m, 48H), 7.36-7.28 (m, 48H), 7.25-7.17 (m, 102H), 7.12-7.05 (m, 18H), 6.72-6.49 (m, 24H), 5.33-5.19 (m, 48H), 4.81-4.64 (m, 48H), 4.62-4.50 (m, 32H), 4.43-4.31 (m, 40H), 4.29-4.04 (m, 96H), 3.86-3.80 (m, 48H), 3.79-3.70 (m, 96H), 3.69-3.58 (m, 282H), 3.53-3.45(m, 2849H), 3.12-2.95 (m, 137H), 2.91-2.84 (m, 12H), 2.77-2.67 (m, 24H), 2.44-2.34 (m, 26H), 2.30-2.25 (m, 15H), 2.16-2.02 (m, 45H), 1.95-1.80 (m, 27H), 1.78-1.70 (m, 22H), 1.66-1.57 (m, 36H), 1.55-1.43 (m, 75H), 1.36-1.30 (m, 21H), 1.30-1.16 (m, 92H), 0.98-0.67 (m, 180H)

Example 11: Synthesis of Compound **82-87**

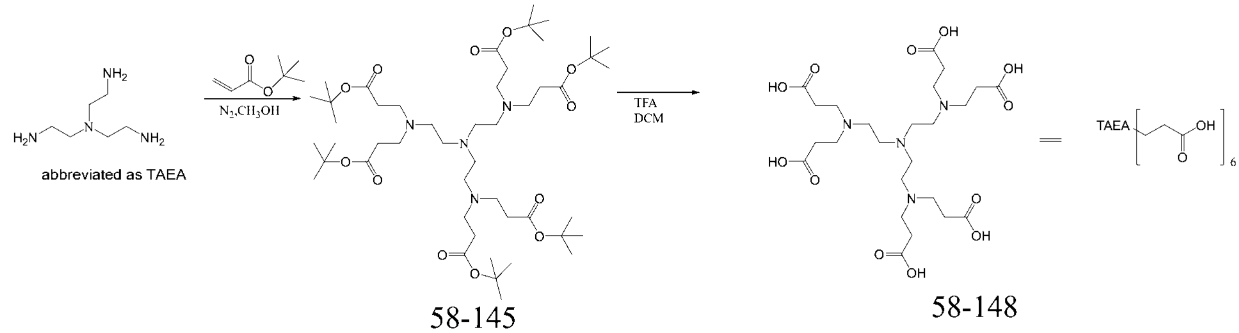


58-145

58-148

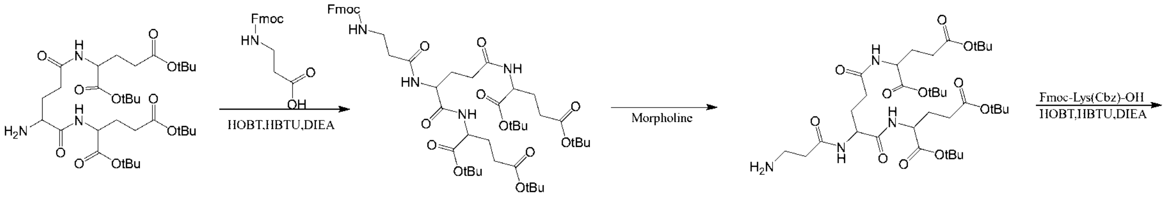


59-99

58-77

58-81

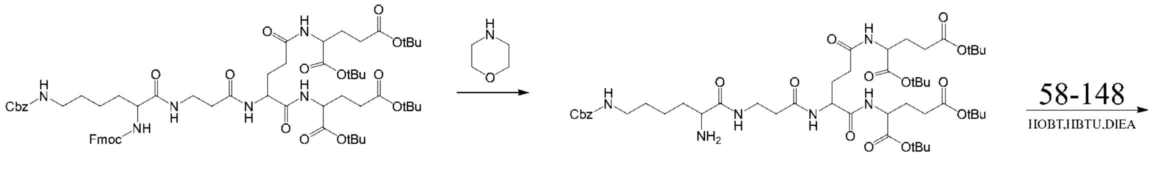


58-82

58-211

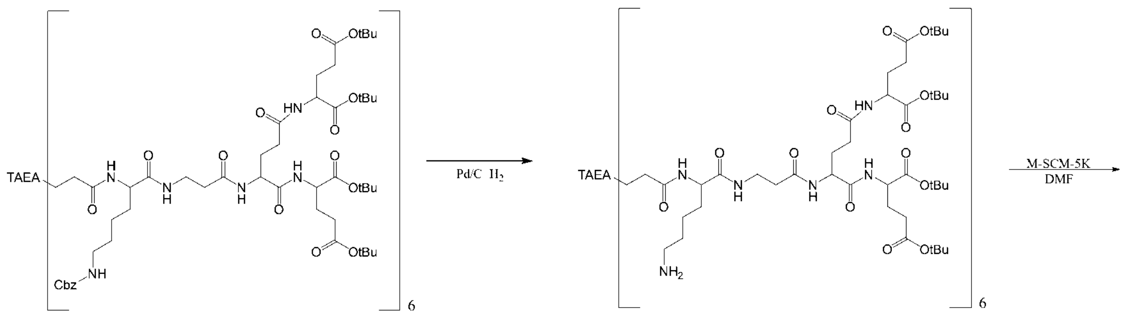


58-212

58-214

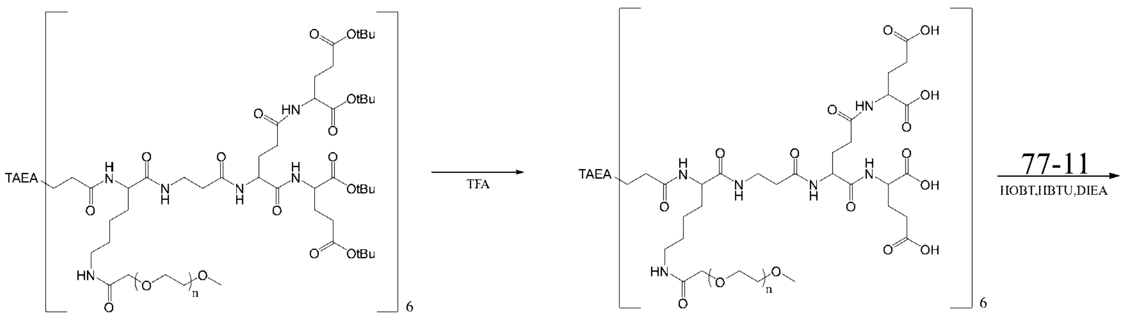


58-215

58-216

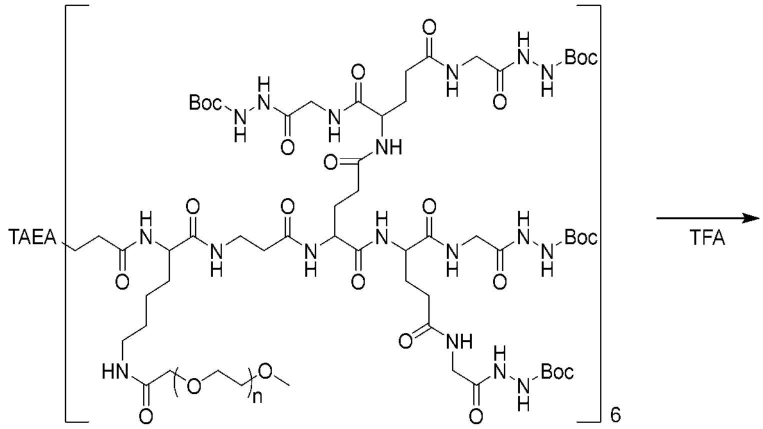


58-217

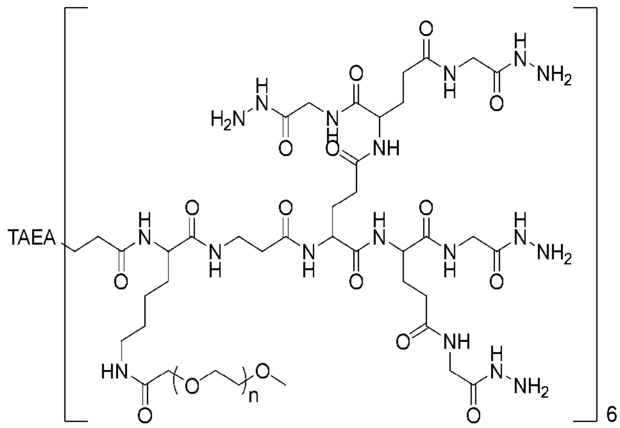

58-218

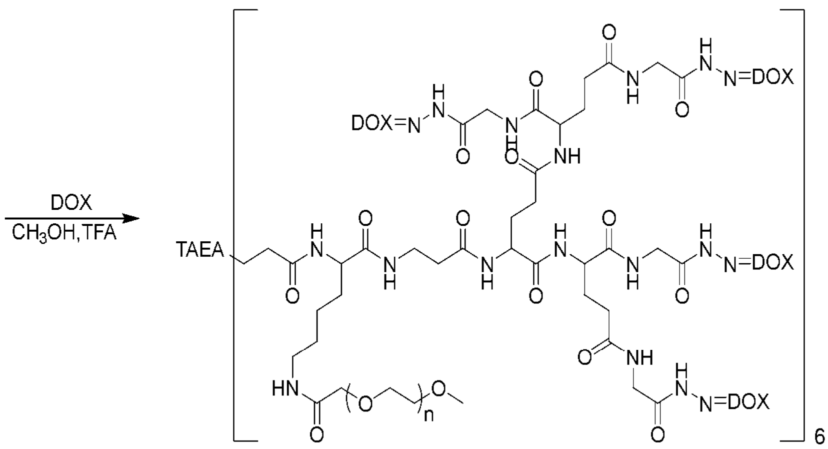


82-87

11.1 Preparation of **58-145**

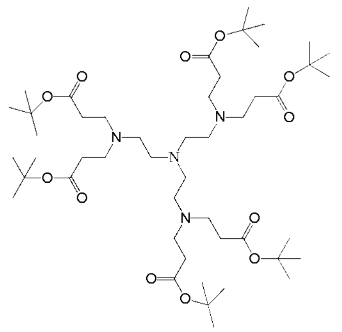

Tris(2-aminoethyl)amine (1 mL, 6.67 mmol, abbreviated as TAEA) was weighed and added to a 500 mL flask, then methanol (100 mL) was added for dissolution. Tert-butyl acrylate (7.76 mL, 53.40 mmol) was added under nitrogen protection, and the mixture was reacted with stirring at room temperature overnight. After the reaction was completed, saturated brine (200 mL) and ethyl acetate (200 mL) were added for extraction. After standing for phase separation, the organic phase was collected, and the aqueous phase was further extracted with ethyl acetate (200 mL × 3). The organic phases were combined, evaporated to dryness to provide a solid, which was then dissolved in a methanol/dichloromethane (1/4) mixture, then silica gel powder (100 mL) was added, and the mixture was evaporated to dryness to provide a powdery solid. The powdery solid was dry-loaded onto the column and subjected to column chromatography using a 40% ethyl acetate/petroleum ether mixture as the eluent. The desired product was collected, concentrated, and dried in a vacuum oven to provide 5.91 g of the product with a yield of 96.7%.

11.2 Preparation of **58-148**

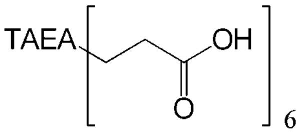

To a flask containing **58-145** (1.5 g, 1.64 mmol), 1,4-dioxane (10 mL) was added for dissolution with the assistance of ultrasonication, then 4M hydrochloric acid-1,4-dioxane (49 mL) was added, and the mixture was reacted with stirring at 70°C for 5 hours. After the reaction was completed, a solid precipitated from the reaction mixture and was filtered. The filter cake was washed with methyl tert-butyl ether (40 mL × 3). The filter cake was collected and dried in a vacuum oven to provide 0.9 g of the product with a yield of 75.8%. $^1$H-NMR (600 MHz, DMSO-$d_6$) δ 3.39-3.29 (m, 18H), 3.03-2.97 (m, 6H), 2.93-2.84 (m, 12H)

11.3 Preparation of **58-77**

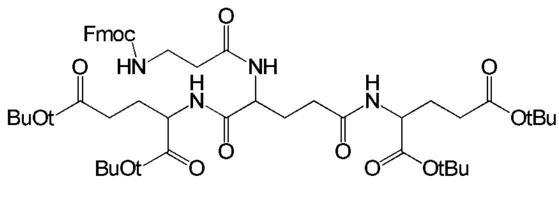

Fmoc-β-aminopropionic acid (1.22 g, 3.93 mmol, purchased from Innochem), HBTU (2.24 g, 5.90 mmol), and HOBT (0.80 g, 5.90 mmol) were weighed and added to a flask containing **59-99** (2.50 g, 3.97 mmol), then DMF (50 mL) was added to dissolve the mixture, and the mixture was stirred at -5°C for several minutes. DIEA (2.92 mL, 17.69 mmol) was slowly added dropwise. After the dropwise addition was completed, the reaction was carried out for half an hour, the flask was then taken out, and the reaction was carried out with stirring at room temperature overnight. After the reaction was completed, saturated brine (200 mL) and ethyl acetate (200 mL) were added for extraction. After standing for phase separation, the organic phase was collected, and the aqueous phase was further extracted with ethyl acetate (200 mL × 3). The organic phases were combined, concentrated, evaporated to dryness, and dried in a vacuum oven to provide 3.63

g of the product.

11.4 Preparation of **58-81**

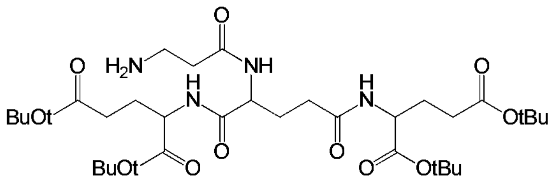

To a flask containing **58-77** (3.63 g, 3.93 mmol), DMF (30 mL) was added for dissolution with the assistance of ultrasonication, then morpholine (6.85 mL, 78.60 mmol) was added, and the mixture was reacted with stirring at room temperature for 2 hours. After the reaction was completed, saturated brine (200 mL) and ethyl acetate (200 mL) were added for extraction. After standing for phase separation, the organic phase was collected, and the aqueous phase was further extracted with ethyl acetate (200 mL × 3). The organic phases were combined, concentrated, evaporated to dryness, and dried in a vacuum oven to provide 3.69 g of the product with a yield exceeding 100%.

11.5 Preparation of **58-82**

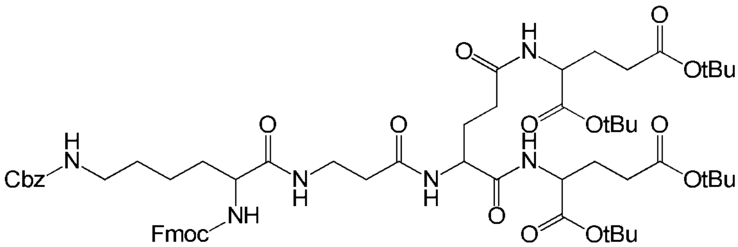

N'-Fmoc-N-benzyloxycarbonyl-L-lysine (1.87 g, 3.74 mmol, purchased from Innochem), HBTU (2.13 g, 5.61 mmol), and HOBT (0.76 g, 5.61 mmol) were weighed and added to a flask containing **58-81** (3.69 g, 3.93 mmol), then DMF (100 mL) was added to dissolve the mixture, and the mixture was stirred at -5°C for several minutes. DIEA (2.79 mL, 16.84 mmol) was slowly added dropwise. After the dropwise addition was completed, the reaction was carried out for half an hour, the flask was then taken out, and the reaction was carried out with stirring at room temperature overnight. After the reaction was completed, saturated brine (200 mL) and ethyl acetate (200 mL) were added for extraction. After standing for phase separation, the organic phase was collected, and the aqueous phase was further extracted with ethyl acetate (200 mL × 3). The organic phases were combined, evaporated to dryness to a solid, then dissolved in a methanol/dichloromethane (1/4) mixture. Silica gel powder (20 mL) was added, and the mixture was evaporated to dryness to provide a powdery solid. The powdery solid was dry-loaded onto the column and subjected to column chromatography using a 3% methanol/dichloromethane mixture as the eluent. The desired product was collected, concentrated, and dried in a vacuum oven to provide 3.05 g of the product with a yield of 68.8%. $^1$H-NMR (400 MHz, DMSO-$d_6$) δ 8.24-8.16 (m, 1H), 8.11-8.02 (m, 2H), 7.94-7.85 (m, 3H), 7.77-7.66 (m, 2H), 7.46-7.38 (m, 3H), 7.37-7.28 (m, 7H), 7.26-7.19 (m, 1H), 5.06-4.96 (m, 2H), 4.32-4.19 (m, 3H), 4.15-4.07 (m, 2H), 3.95-3.83 (m, 1H), 3.29-3.18 (m, 2H), 3.02-2.92 (m, 2H), 2.35-2.13 (m, 8H), 1.96-1.84 (m, 3H), 1.78-1.65 (m, 3H), 1.61-1.48 (m, 3H), 1.37 (s, 36H), 1.33-1.16 (m, 4H)

11.6 Preparation of **58-211**

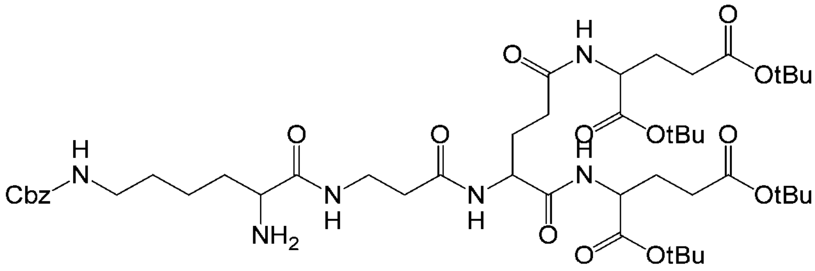

**58-82** (1.1 g, 0.93 mmol) was added to DMF (10 mL) for dissolution with the assistance of ultrasonication, then morpholine (1.62 mL, 18.56 mmol) was added, and the mixture was reacted with stirring at room temperature for 2 hours. After the reaction was completed, saturated brine (200 mL) and ethyl acetate (200 mL) were added for extraction. After standing for phase separation, the organic phase was collected, and the aqueous phase was further extracted with ethyl acetate (200 mL × 3). The organic phases were combined, concentrated, evaporated to dryness, and then dried in a vacuum oven to provide 0.86 g of the product with a yield of 97.1%.

11.7 Preparation of **58-212**

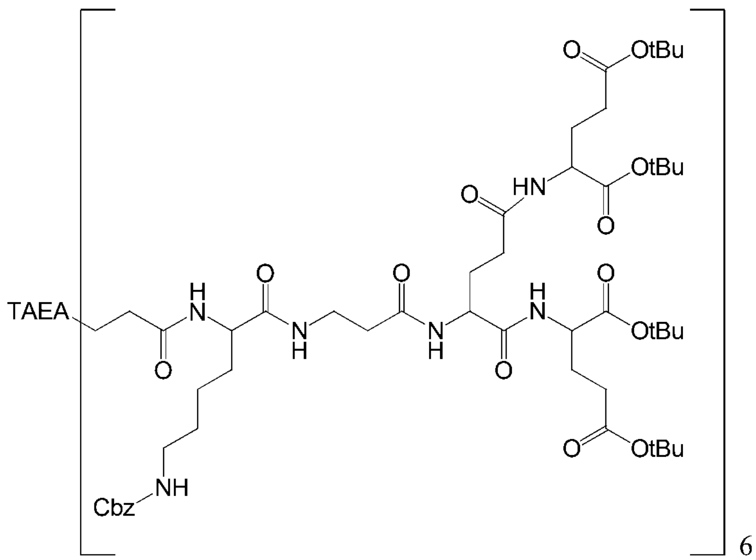

**58-148** (0.10 g, 0.14 mmol), HBTU (0.37 g, 0.97 mmol), and HOBT (0.13 g, 0.97 mmol) were weighed and added to a flask containing **58-211** (0.86 g, 0.89 mmol), then DMF (15 mL) was added to dissolve the mixture, and the mixture was stirred at -5°C for several minutes. DIEA (0.49 mL, 2.92 mmol) was slowly added dropwise. After the dropwise addition was completed, the reaction was carried out for half an hour, the flask was then taken out, and the reaction was carried out with stirring at room temperature overnight. After the reaction was completed, saturated brine (200 mL) and ethyl acetate (200 mL) were added for extraction. After standing for phase separation, the organic phase was collected, and the aqueous phase was further extracted with ethyl acetate (200 mL × 3). The organic phases were combined, evaporated to dryness to provide a solid, which was dissolved in dichloromethane and methanol, then silica gel powder was added, and the mixture was evaporated to dryness. The resulting product was dry-loaded onto the column and subjected to column chromatography using 1% ammonia water/7% methanol/dichloromethane as the eluent. The desired product was collected, concentrated, and dried to provide 0.33 g of the product with a yield of 39%. $^1$H-NMR (600 MHz, DMSO-$d_6$) δ 8.27-7.89 (m, 30H), 7.38-7.12 (m, 37H), 5.12-5.01 (m, 12H), 4.31-4.06 (m, 25H), 2.95 (s, 16H), 2.62 (s, 16H), 2.38-2.07 (m, 66H), 1.78-1.69 (m, 40H), 1.64-1.42 (m, 23H), 1.46-1.32 (m, 216H), 1.28-1.11 (m, 28H); ESI [M+H$^+$] 6246.206

11.8 Preparation of **58-214**

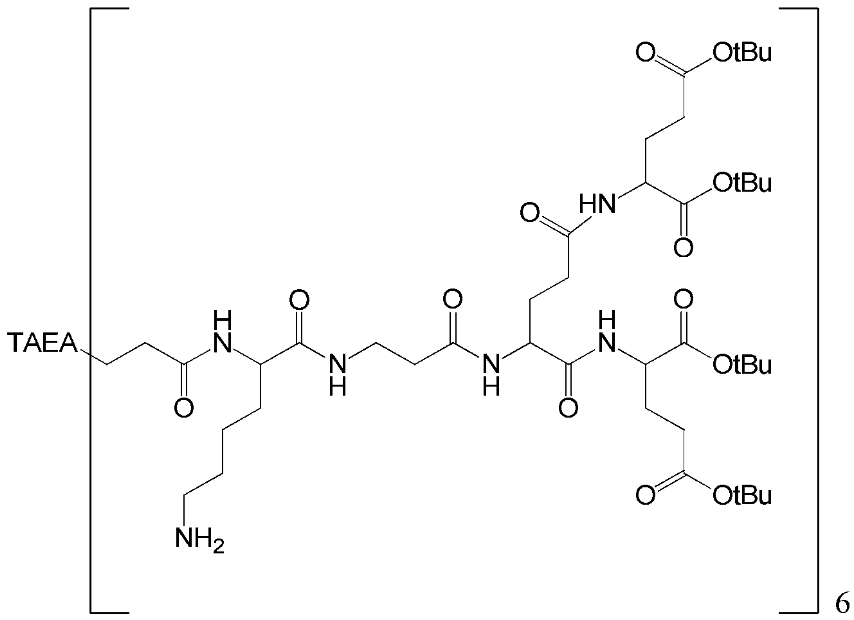

**58-212** (0.19 g, 0.0314 mmol) and 10% Pd/C catalyst (0.04 g) were placed in a hydrogenation reactor, and DMF (30 mL) was added for dissolution. The hydrogenation reactor was sealed, evacuated with a water pump, and filled with hydrogen gas. This process was repeated three times. The pressure reading of the hydrogenation reactor was adjusted to 1.8 MPa. The mixture was stirred at room temperature overnight. After the reaction was completed, the reaction mixture was filtered through Celite, and the filter cake was washed with DMF (20 mL × 3). The DMF solution of the product was obtained and used as the raw material for the next reaction.

11.9 Preparation of **58-215**

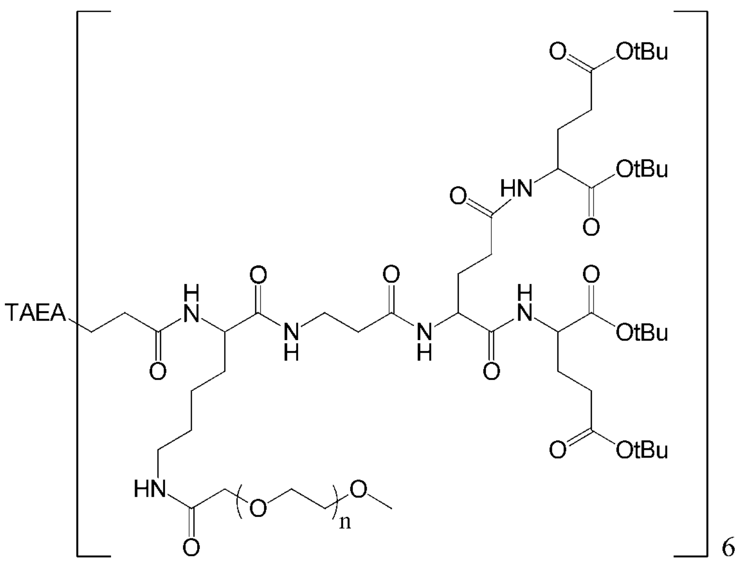

**58-214** (0.17 g, 0.0314 mmol) was placed in a 250 mL flask. DMF (20 mL) was added for dissolution, then a DMF solution of M-SCM-5K (1.00 g, 0.1883 mmol, purchased from Jenkem) was added, and the mixture was reacted with stirring at low speed in the dark at room temperature for one week. After the reaction was completed, n-hexane (25 mL) and methyl tert-butyl ether (200 mL) were added for precipitation. The supernatant was discarded, and n-hexane and methyl tert-butyl ether were added again for precipitation. This process was repeated three times. The mixture was filtered to provide a solid product, which was collected and dried in a vacuum oven to provide 1.1 g of the product with a yield of 95.7%.

11.10 Preparation of **58-216**

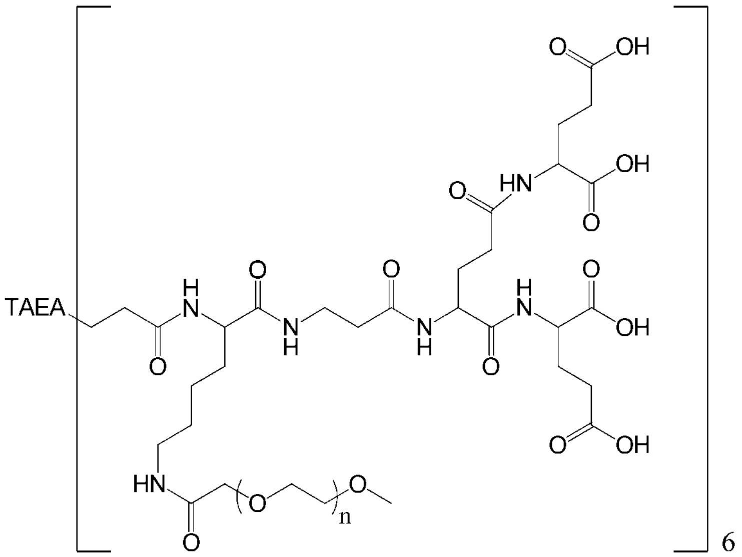

**58-215** (1.1 g, 0.03 mmol) was dissolved in dichloromethane (8 mL) and TFA (8 mL), and the reaction was stirred at room temperature overnight. After the reaction was completed, n-hexane (25 mL) and methyl tert-butyl ether (200 mL) were added for precipitation. The supernatant was discarded, and n-hexane and methyl tert-butyl ether were added again for precipitation. This process was repeated three times. The mixture was filtered to provide a solid product, which was collected and dried in a vacuum oven to provide 1.0 g of the product with a yield of 90.3%.

11.11 Preparation of **58-217**

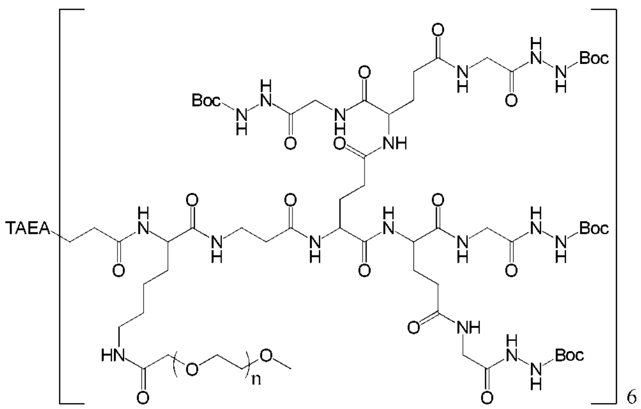

**58-216** (1.0 g, 0.0284 mmol), HBTU (0.39 g, 1.02 mmol), and HOBT (0.14 g, 1.02 mmol) were weighed and added to a flask containing **77-11** (0.14 g, 0.75 mmol), then DMF (20 mL) was added to dissolve the mixture, and the mixture was stirred at -5°C for several minutes. DIEA (0.51 mL, 3.07 mmol) was slowly added dropwise. After the dropwise addition was completed, the reaction was carried out for half an hour, the flask was then taken out, and the reaction was carried out with stirring at room temperature overnight. After the reaction was completed, n-hexane (25 mL) and methyl tert-butyl ether (200 mL) were added for precipitation. The supernatant was discarded, and n-hexane and methyl tert-butyl ether were added for precipitation. This process was repeated three times. The mixture was filtered to provide a solid product, which was dried in a vacuum oven to provide 1.23 g of the product with a yield exceeding 100%.

11.12 Preparation of **58-218**

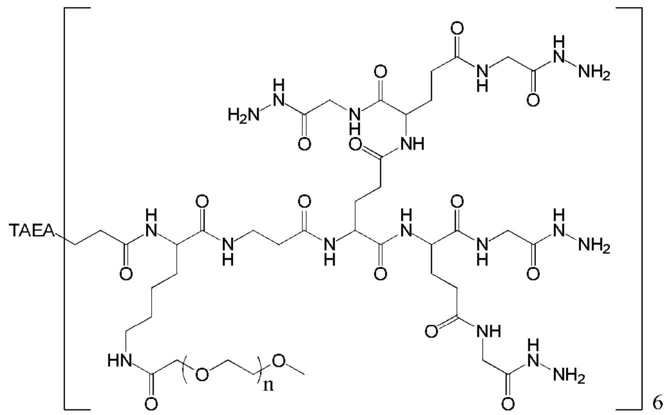

**58-217** (1.23 g, 0.0325 mmol) was added to a 250 mL flask, then dichloromethane (8 mL) and TFA (8 mL) were added, and the mixture was stirred at room temperature overnight. After the reaction was completed, the reaction mixture was evaporated to an oily state using a rotary evaporator. Methyl tert-butyl ether (60 mL) was then added for precipitation. A powdery solid precipitated from the reaction mixture and filtered. The filter cake was washed with methyl tert-butyl ether (40 mL × 3). The filter cake was collected and dried in a vacuum oven to provide 1.18 g of the product with a yield exceeding 100%.

11.13 Preparation of **82-87**

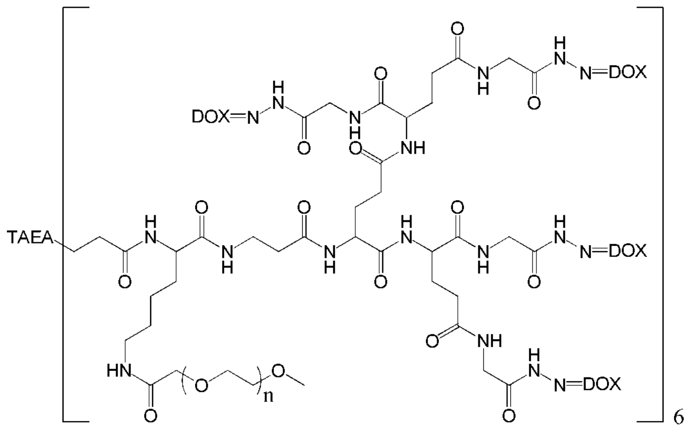

**58-218** (3.1 g, 0.0838 mmol) was placed in a dry 500 mL round-bottom flask. Anhydrous methanol (30 mL) was added for dissolution, then TFA (0.27 mL, 3.6 mmol) and DOX.HCl (1.16 g, 2.0112 mmol) were added, and the mixture was reacted with stirring at room temperature overnight. After the reaction was completed, n-hexane (25 mL) and methyl tert-butyl ether (200 mL) were added for precipitation. The supernatant was discarded, and n-hexane and methyl tert-butyl ether were added again for precipitation. This process was repeated three times. The mixture was filtered to provide a solid product, which was dissolved in dichloromethane and methanol, then silica gel powder was added, and the mixture was evaporated to dryness. The resulting product was dry-loaded onto the column and subjected to column chromatography using 6-10% methanol/dichloromethane as the eluent. The desired product was collected, concentrated, and dried to provide 2.3 g of the product with a yield of 55.42%. $^1$H-NMR (600 MHz, DMSO-$d_6$) δ 14.04-14.00 (m, 24H), 9.16-9.07 (m, 24H), 7.96-7.94 (m, 14H), 7.92-7.85 (m, 97H), 7.68-7.59 (m, 50H), 7.16-7.10 (m, 21H), 7.02-6.87 (m, 22H), 6.75-6.67 (m, 21H), 5.56-5.50 (m, 48H), 5.47-5.44 (m, 48H), 5.31-5.25 (m, 48H), 4.92-4.88 (m, 49H), 4.62-4.57 (m, 81H), 4.22-4.17 (m, 46H), 3.98-3.97 (m, 80H), 3.64-3.60 (m, 68H), 3.56-3.46 (m, 3076H), 3.21-3.15 (m, 49H), 3.02-2.96 (m, 51H), 2.76-2.71 (m, 31H), 2.19-2.06 (m, 101H), 1.91-1.84 (m, 57H), 1.73-1.66 (m, 57H), 1.16-1.12 (m, 73H)

Example 12: Synthesis of Compound **73-49**

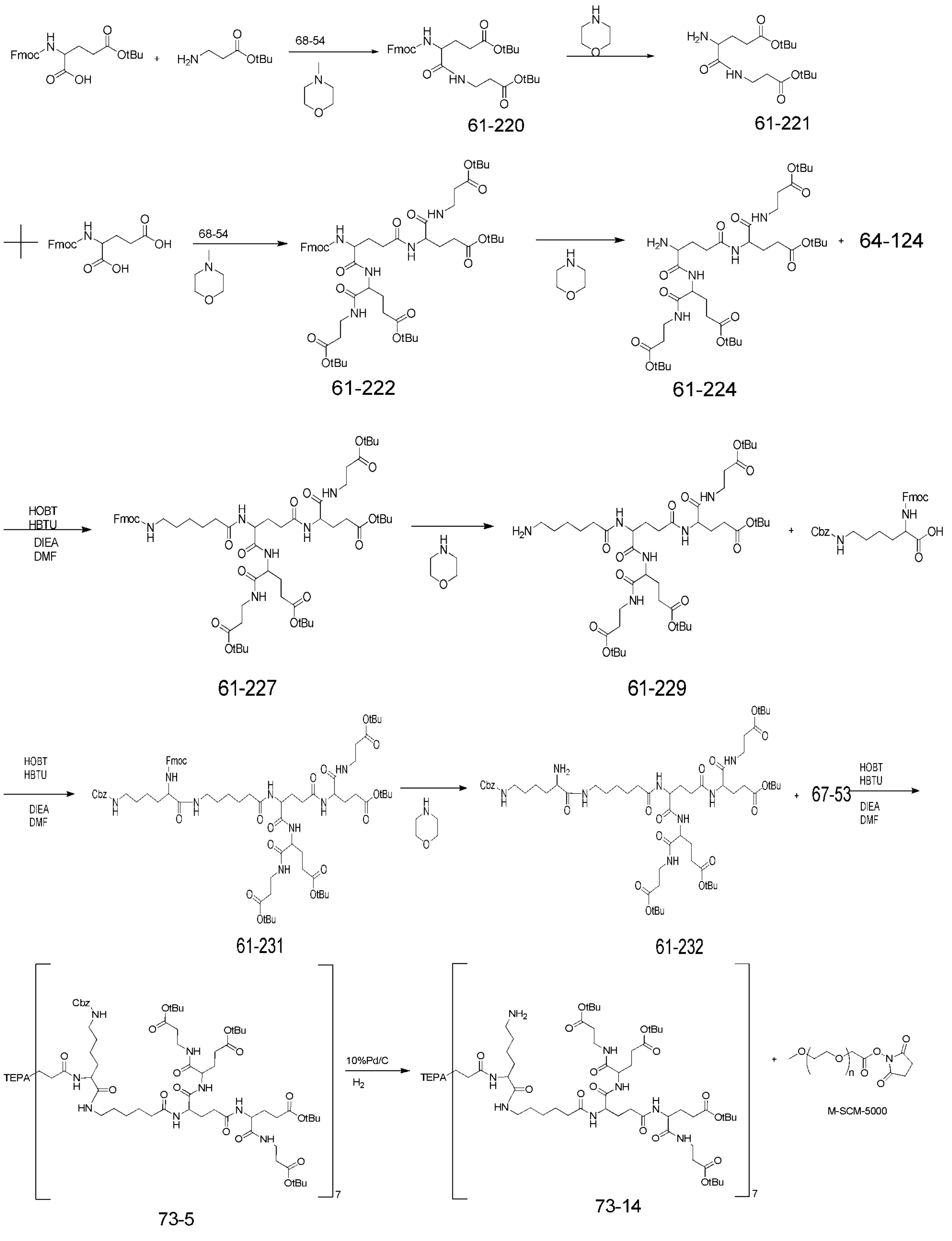


61-220

61-221

61-222

61-224

+ 64-124

61-227

61-229

61-231

61-232

+ 67-53

73-5

73-14

M-SCM-5000

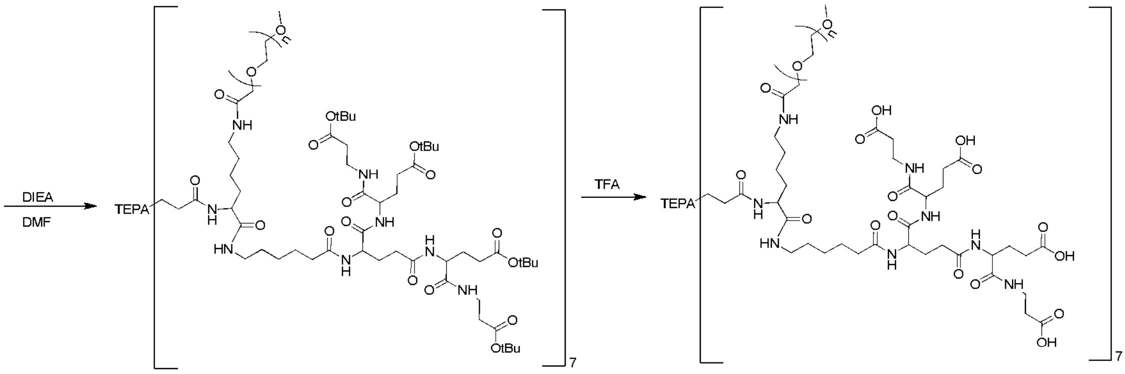


+ 67-154

73-17

73-47

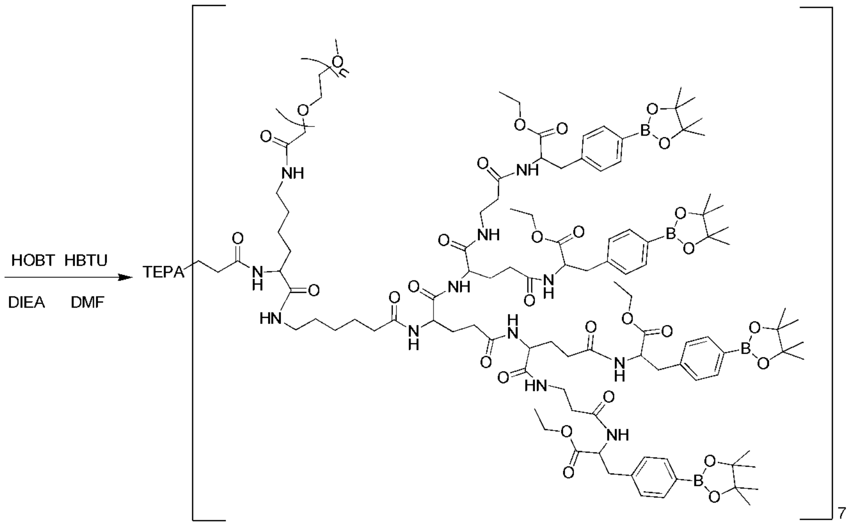


73-49

12.1 Preparation of **61-220**

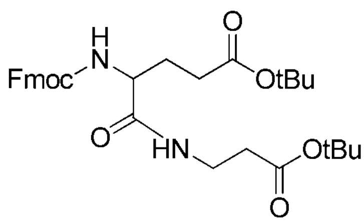

Fmoc-L-glutamic acid 5-tert-butyl ester (10 g, 23.5 mmol) and β-alanine tert-butyl ester hydrochloride (4.27 g, 23.5 mmol) were weighed and added to a 500 mL round-bottom flask, then DMF (25 mL) was added for dissolution with the assistance of ultrasonication, and the mixture was stirred at -5°C for 2 minutes. **68-54** (7.15 g, 25.85 mmol) was then added, and N-methylmorpholine (7.8 mL, 70.5 mmol) was slowly added dropwise to continue the reaction. After the reaction was completed, the reaction mixture was transferred to a 2 L separatory funnel containing pure water (300 mL) and ethyl acetate (300 mL) for extraction. The organic phase was collected, and the aqueous phase was further extracted with ethyl acetate (100 mL × 2). The organic phases were combined, washed with saturated sodium bicarbonate solution (200 mL × 2). The resulting organic phase was collected, concentrated, and evaporated to dryness to provide 12.98 g of the product.

12.2 Preparation of **61-221**

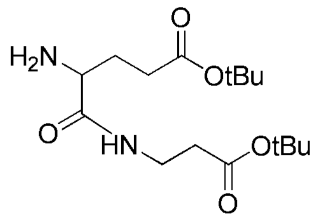

**61-220** (12.98 g, 23.5 mmol) was added to a 500 mL round-bottom flask. DMF (20 mL) was added for dissolution with the assistance of ultrasonication, then morpholine (30.7 mL, 352.5 mmol) was added, and the mixture was reacted with stirring at room temperature. After the reaction was completed, the reaction mixture was poured into a 2 L separatory funnel containing purified water (300 mL) and ethyl acetate (300 mL) for extraction. The organic phase was collected, and the aqueous phase was further extracted with ethyl acetate (100 mL × 2). The organic phases were combined, washed with saturated sodium bicarbonate solution (200 mL × 2). The resulting organic phase was collected and concentrated, then 200-300 mesh silica gel powder (90 g) was added, and the mixture was evaporated to dryness. The resulting product was dry-loaded onto the column and subjected to column chromatography using 1% ammonia/7% methanol/dichloromethane as the eluent. The desired product was collected, concentrated, and evaporated to dryness to provide 6.2 g of the product with a yield of 80%.

12.3 Preparation of **61-222**

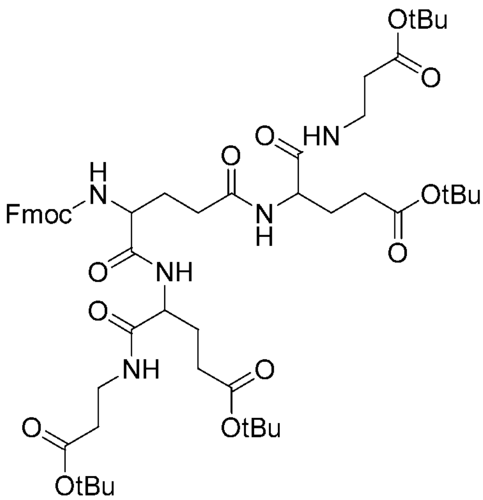

Fmoc-L-glutamic acid (3.15 g, 8.527 mmol) and **61-221** (6.2 g, 18.76 mmol) were weighed and added to a 500 mL round-bottom flask, then DMF (15 mL) was added for dissolution with the assistance of ultrasonication, and the mixture was stirred at -5°C for 2 minutes. **68-54** (5.19 g, 18.76 mmol) was added, and N-methylmorpholine (5.6 mL, 51.16 mmol) was then slowly added dropwise to continue the reaction. After the reaction was completed, the reaction mixture was transferred to a 2 L separatory funnel containing pure water (300 mL) and ethyl acetate (300 mL) for extraction. The organic phase was collected, and the aqueous phase was further extracted with ethyl acetate (100 mL × 2). The organic phases were combined, washed with saturated sodium bicarbonate solution (200 mL × 2). The resulting organic phase was collected and concentrated, then 200-300 mesh silica gel powder (40 g) was added, and the mixture was evaporated to dryness. The resulting product was dry-loaded onto the column and subjected to column chromatography using 1-3% methanol/dichloromethane as the eluent. The desired product was collected, concentrated, and evaporated to dryness to provide 8.1 g of the product with a yield of 95%.

12.4 Preparation of **61-224**

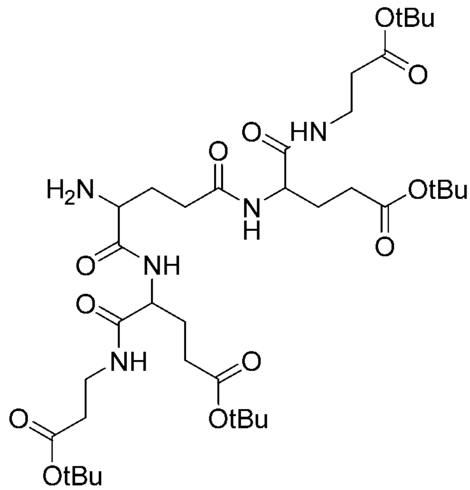

**61-222** (8.1 g, 8.147 mmol) was added to a 500 mL round-bottom flask, DMF (20 mL) was added for dissolution with the assistance of ultrasonication, then morpholine (10.65 mL, 122.21 mmol) was added, and the mixture was reacted with stirring at room temperature. After the reaction was completed, the reaction mixture was taken out, and poured into a 2 L separatory funnel containing pure water (300 mL) and ethyl acetate (300 mL) for extraction. The organic phase was collected, and the aqueous phase was further extracted with ethyl acetate (100 mL × 2). The organic phases were combined, washed with saturated sodium bicarbonate solution (200 mL × 2). The resulting organic phase was collected and concentrated, then 200-300 mesh silica gel powder (30 g) was added, and the mixture was evaporated to dryness. The resulting product was dry-loaded onto the column and subjected to column chromatography using 1% ammonia/9% methanol/dichloromethane as the eluent. The desired product was collected, concentrated, and evaporated to dryness to provide 6.2 g of the product with a yield of 98%.

12.5 Preparation of **61-227**

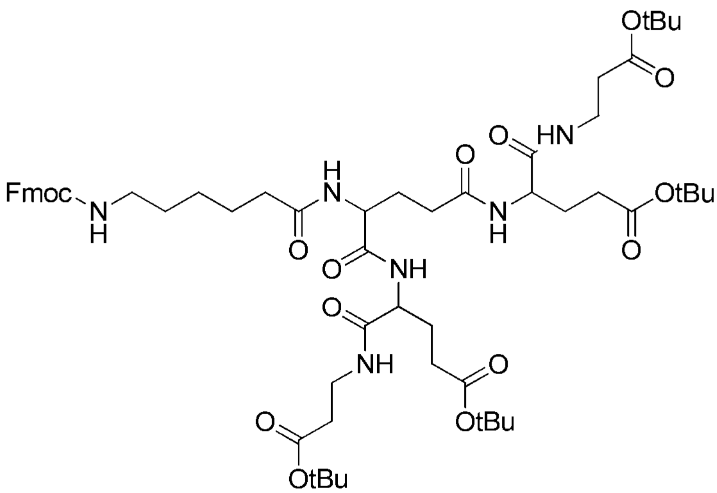

**61-224** (6.2 g, 8.03 mmol), **64-124** (2.83 g, 8.03 mmol), HBTU (4.56 g, 12.04 mmol), and HOBT (1.62 g, 12.04 mmol) were placed in a 500 mL flask, then DMF (15 mL) was added to dissolve the mixture with the assistance of ultrasonication, and the mixture was stirred at room temperature for 2 minutes. DIEA (4.64 mL, 28.105 mmol, purchased from Aladdin) was then slowly added dropwise. After the dropwise addition was complete, the reaction was continued with stirring at room temperature. After the reaction was completed, the reaction mixture was added to methyl tert-butyl ether (100 mL) and n-hexane (200 mL) for precipitation. A solid product precipitated and was filtered. The obtained solid was dissolved in a methanol/dichloromethane (1/4) mixture, then 200-300 mesh silica gel powder (35 g) was added, and the mixture was evaporated to dryness. The resulting product was dry-loaded onto the column and subjected to column chromatography using 3% methanol/dichloromethane as the eluent. The desired product was collected, concentrated, and evaporated to dryness to provide 7.48 g of the product with a yield of 83%.

12.6 Preparation of **61-229**

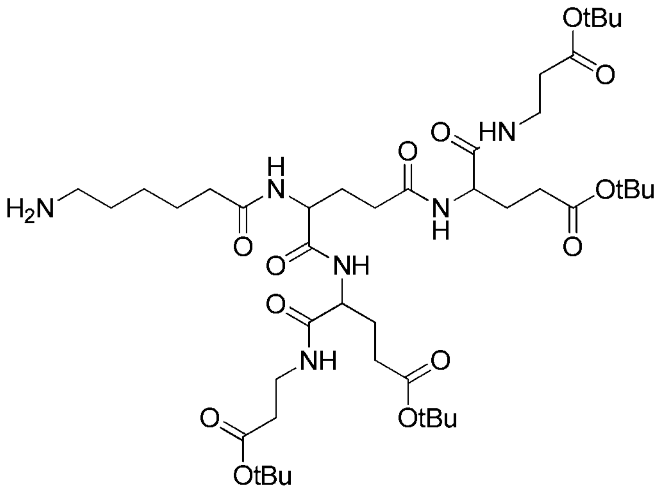

**61-227** (7.4 g, 6.68 mmol) was added to a 500 mL round-bottom flask. DMF (20 mL) was added for dissolution with the assistance of ultrasonication, then morpholine (8.73 mL, 100.24 mmol) was added, and the mixture was reacted with stirring at room temperature. After the reaction was completed, ethyl acetate (200 mL) and water (300 mL) were added for extraction. The organic phase was collected, and the aqueous phase was further extracted with ethyl acetate (150 mL × 2). The organic phases were combined and washed with saturated sodium bicarbonate solution (200 mL × 2). The resulting organic phase was collected, dehydrated over anhydrous magnesium sulfate, and filtered. The filtrate was collected, concentrated, and dried to provide 5.91 g of the product.

12.7 Preparation of **61-231**

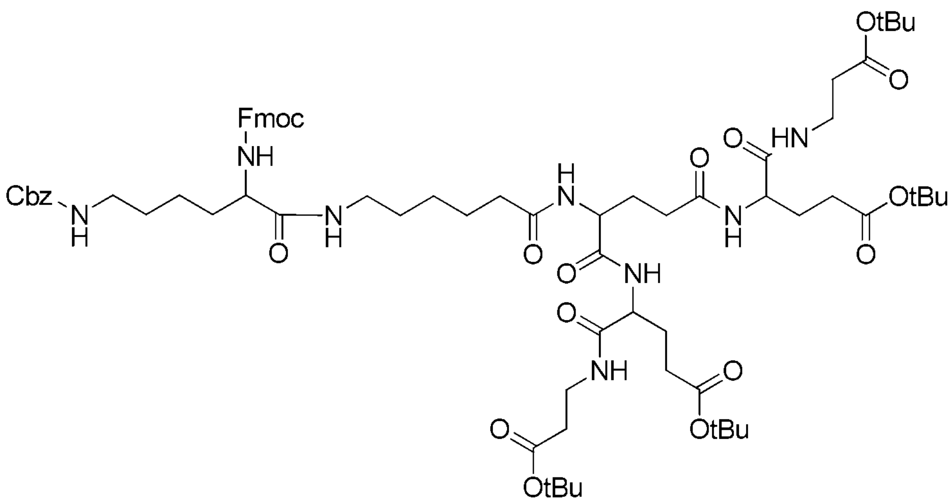

**61-229** (5.91 g, 6.68 mmol), N'-Fmoc-N-benzyloxycarbonyl-L-lysine (3.35 g, 6.68 mmol), HBTU (3.8 g, 10.02 mmol), and HOBT (1.354 g, 10.02 mmol) were placed in a 500 mL flask, then DMF (15 mL) was added for dissolution with the assistance of ultrasonication, and the mixture was stirred at room temperature for 10 minutes. DIEA (3.86 mL, 23.38 mmol, purchased from Aladdin) was then slowly added dropwise. After the dropwise addition was completed, the reaction was continued with stirring at room temperature. After the reaction was completed, n-hexane (50 mL) and methyl tert-butyl ether (450 mL) were added for precipitation. A solid precipitated and was filtered. The filter cake was dissolved in DMF (20 mL), then n-hexane (50 mL) and methyl tert-butyl ether (450 mL) were added for precipitation. This process was repeated three times. The resulting crude solid product was dissolved in a methanol/dichloromethane (1/4) mixture, then 200-300

mesh silica gel powder (30 g) was added, and the mixture was evaporated to dryness. The resulting product was dry-loaded onto the column and subjected to column chromatography using 3% methanol/dichloromethane as the eluent. The desired product was collected, concentrated, and evaporated to dryness to provide 6.3 g of the product with a yield of 68.8%. $^1$H-NMR (600 MHz, DMSO-$d_6$) δ 8.19 (d, *J* = 8.1 Hz, 1H), 8.14 (d, *J* = 8.0 Hz, 1H), 8.09 (q, *J* = 5.2 Hz, 2H), 7.94 (d, *J* = 7.5Hz, 1H), 7.89 (d, *J* = 7.6 Hz, 2H), 7.83 (t, *J* = 5.7 Hz, 1H), 7.73 (dd, *J* = 3.2, 7.8 Hz, 2H), 7.45-7.26 (m, 10H), 7.23 (t, *J* = 5.8 Hz, 1H), 5.00 (s, 2H), 4.32-4.16 (m, 5H), 4.09 (q, *J* = 7.5 Hz, 1H), 3.91 (td, *J* = 5.1, 8.7 Hz, 1H), 3.47-3.24 (m, 2H), 3.21-3.11 (m, 2H), 3.10-2.91 (m, 4H), 2.42-2.30 (m, 4H), 2.26-2.03 (m, 8H), 1.89-1.65 (m, 6H), 1.62-1.16 (m, 48H)

12.8 Preparation of **61-232**

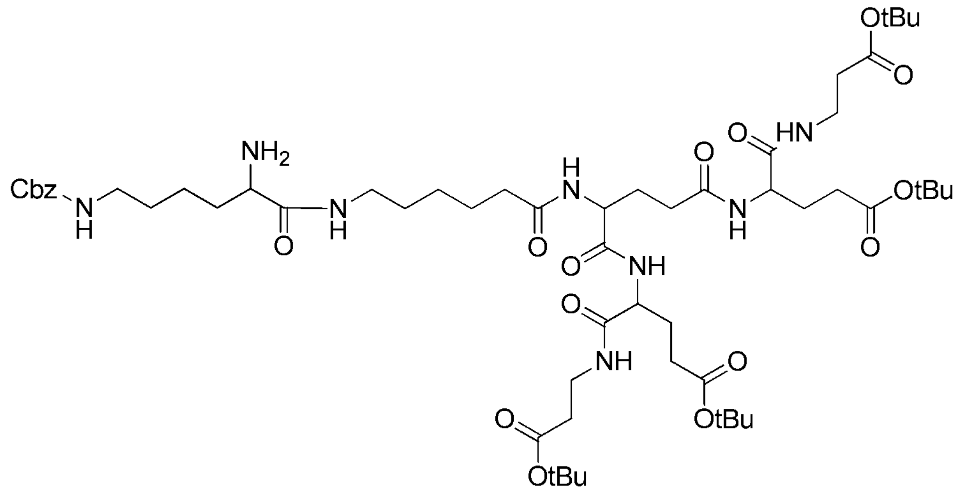

**61-231** (3 g, 2.19 mmol) was placed in a 250 mL round-bottom flask. DMF (10 mL) was added for dissolution with the assistance of ultrasonication, then morpholine (2.86 mL, 32.85 mmol) was added, and the mixture was reacted with stirring at room temperature. After the reaction was completed, n-hexane (50 mL) and methyl tert-butyl ether (450 mL) were added for precipitation. A solid precipitated and was filtered. The filter cake was redissolved in DMF (20 mL), and then methyl tert-butyl ether (500 mL) was added for precipitation. This process was repeated three times. The filter cake was collected, dissolved in a methanol/dichloromethane (1/1) mixture (100 mL), then 200-300 mesh silica gel powder (30 g) was added, and the mixture was evaporated to dryness. The resulting product was dry-loaded onto the column and subjected to column chromatography using 1% ammonia water/4% methanol/dichloromethane as the eluent. The desired product was collected, concentrated, and evaporated to dryness to provide 2.2 g of the product with a yield of 88%. $^1$H-NMR (600 MHz, DMSO-$d_6$) δ 8.19 (d, *J* = 8.2 Hz, 1H), 8.14 (d, *J* = 8.0 Hz, 1H), 8.10 (q, *J* = 5.9 Hz, 2H), 7.95 (d, *J* = 7.4 Hz, 1H), 7.80 (t, *J* = 5.8 Hz, 1H), 7.40-7.28 (m, 5H), 7.22 (t, *J* = 5.7 Hz, 1H), 5.00 (s, 2H), 4.27-4.16 (m, 2H), 4.15-4.01 (m, 3H), 3.38-3.27 (m, 1H), 3.21-3.12 (m, 1H), 3.08 (dd, *J* = 5.3, 7.5 Hz, 1H), 3.06-3.00 (m, 2H), 2.99-2.93 (q, *J* = 6.7 Hz, 2H), 2.42-2.30 (m, 5H), 2.26-2.05 (m, 9H), 1.90-1.66 (m, 6H), 1.57-1.18 (m, 48H)

12.9 Preparation of **73-5**

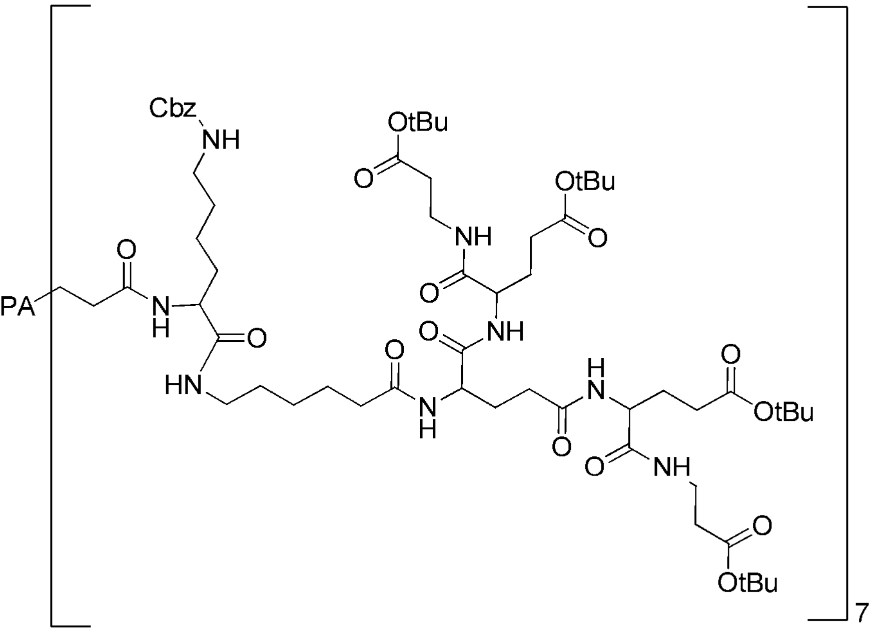

**61-232** (1 g, 0.8715 mmol), **67-53** (0.066 g, 0.095 mmol), HBTU (0.3814 g, 1.00 mmol), and HOBT (0.135 g, 1.00 mmol) were placed in a 500 mL flask, then DMF (10 mL) was added to dissolve the mixture with the assistance of ultrasonication, and the mixture was stirred at room temperature for 2 minutes. DIEA (0.63 mL, 3.83 mmol, purchased from Aladdin) was slowly added dropwise. After the dropwise addition was completed, the reaction was continued at room temperature with stirring. After the reaction was completed, n-hexane (50 mL) and methyl tert-butyl ether (450 mL) were added for precipitation. A solid precipitated and was filtered. The filter cake was redissolved in DMF (10 mL), and then methyl tert-butyl ether (500 mL) was added for precipitation. A solid precipitated and was filtered. This process was repeated three times. The filter cake was collected, dissolved in a methanol/dichloromethane (1/1) mixture (100 mL), then 200-300 mesh silica gel powder (30 g) was added, and the mixture was evaporated to dryness. The resulting product was dry-loaded onto the column and subjected to column chromatography using 1% ammonia water/3-16% methanol/dichloromethane as the eluent. The desired product was collected, concentrated, and evaporated to dryness to provide 0.12 g of the product with a yield of 18%. $^{1}$H-NMR (600 MHz, DMSO-$d_6$) δ 8.22-8.05 (m, 35H), 7.99-7.92 (m, 14H), 7.40-7.27 (m, 35H), 7.25-7.19 (m, 7H), 5.08-4.94 (m, 14H), 4.26-4.16 (m, 14H), 4.13-4.04 (m, 14H), 3.89-3.80 (m, 7H), 3.56-3.38 (m, 18H), 3.38-3.10 (m, 14H), 3.07-2.85 (m, 35H), 2.81-2.69 (m, 26H), 2.44-2.27 (m, 28H), 2.27-2.04 (m, 56H), 1.91-1.62 (m, 42H), 1.61-1.10 (m, 336H)

12.10 Preparation of 73-14

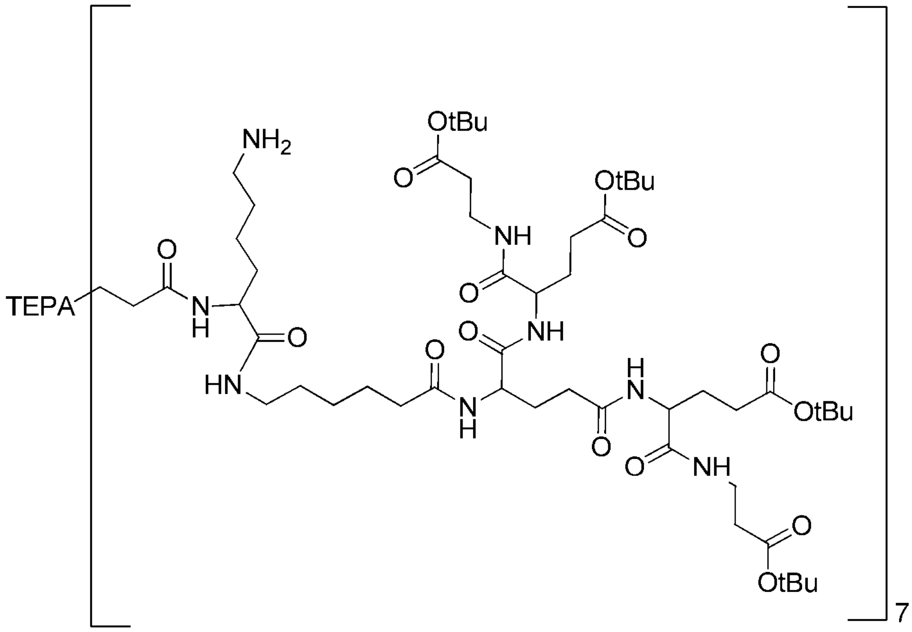

Raw material **73-5** (0.60 g, 0.0703 mmol) and 10% Pd/C catalyst (130 mg) were added to a hydrogenation reactor, and DMF (10 mL) was added for dissolution. The hydrogenation reactor was sealed, evacuated with a water pump, and then filled with hydrogen gas. This process was repeated three times. The hydrogen pressure was adjusted to 2 MPa. The reaction was then carried out at room temperature for 72 hours. After the reaction was completed, the mixture was filtered through a Buchner funnel containing filter paper. The reactor was rinsed with DMF (5 mL × 3). The filtrate was collected and used as the raw material for the next reaction.

12.11 Preparation of **73-17**

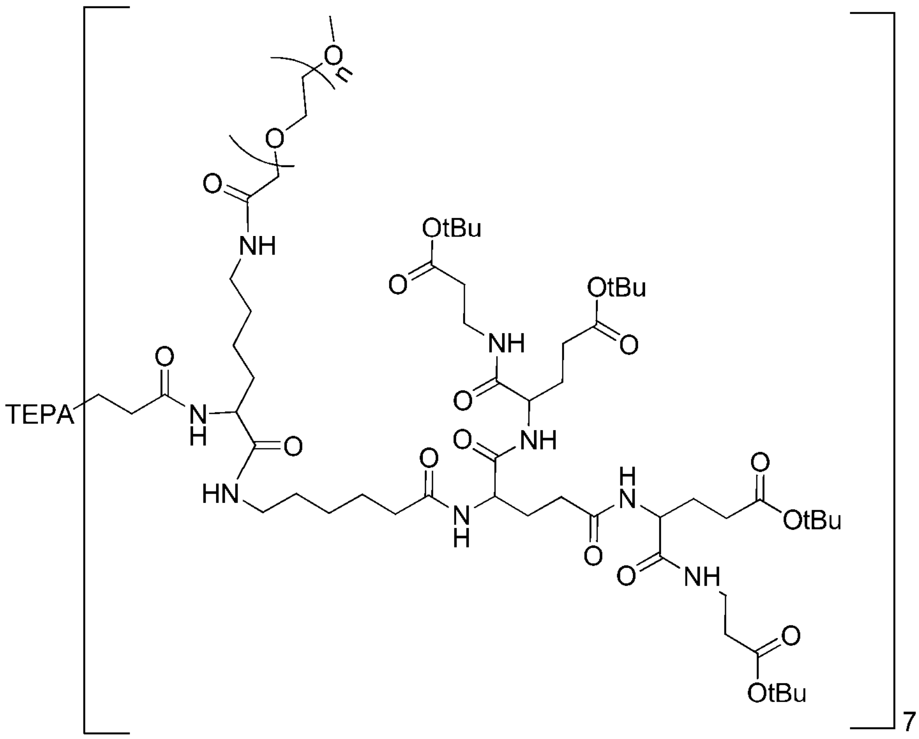

First, a solution of **73-14** (0.0703 mol) was added to a 500 mL round-bottom flask, then DMF (10 mL) was added

for dilution, and the mixture was stirred at -5°C for 2 minutes. DIEA (0.32 mL, 1.96 mmol) was then slowly added dropwise, and the reaction was continued with stirring for 3 minutes. M-SCM-5000 (2.9 g, 0.54 mmol, Lot Number ZZ348P126, purchased from Tianjin Jenkem) was then added, and the reaction was carried out with stirring at low speed in the dark for 72 hours. After the reaction was completed, n-hexane (50 mL) and methyl tert-butyl ether (450 mL) were added for precipitation. A solid precipitated and was filtered. A methanol/dichloromethane (1/1) mixture (20 mL) was added to the filter cake for dissolution with the assistance of ultrasonication, then 100-200 mesh silica gel powder (20 g) was added, and the mixture was evaporated to dryness. The resulting product was dry-loaded onto the column and subjected to column chromatography using 1% ammonia water/5% methanol/dichloromethane as the eluent. The desired product was collected, concentrated, and evaporated to dryness to provide 1.51 g of the product with a yield of 53%. $^1$H-NMR (600 MHz, DMSO-$d_6$) δ 8.23-7.99 (m, 42H), 7.99-7.92 (m, 14H), 4.26-4.16 (m, 14H), 4.13-4.04 (m, 14H), 3.89-3.80 (m, 7H), 3.63-3.38 (m, 3301H), 3.38-3.10 (m, 14H), 3.10-2.85 (m, 35H), 2.81-2.68 (m, 26H), 2.44-2.27 (m, 28H), 2.27-2.04 (m, 56H), 1.91-1.62 (m, 42H), 1.61-1.09 (m, 336H)

12.12 Preparation of **73-47**

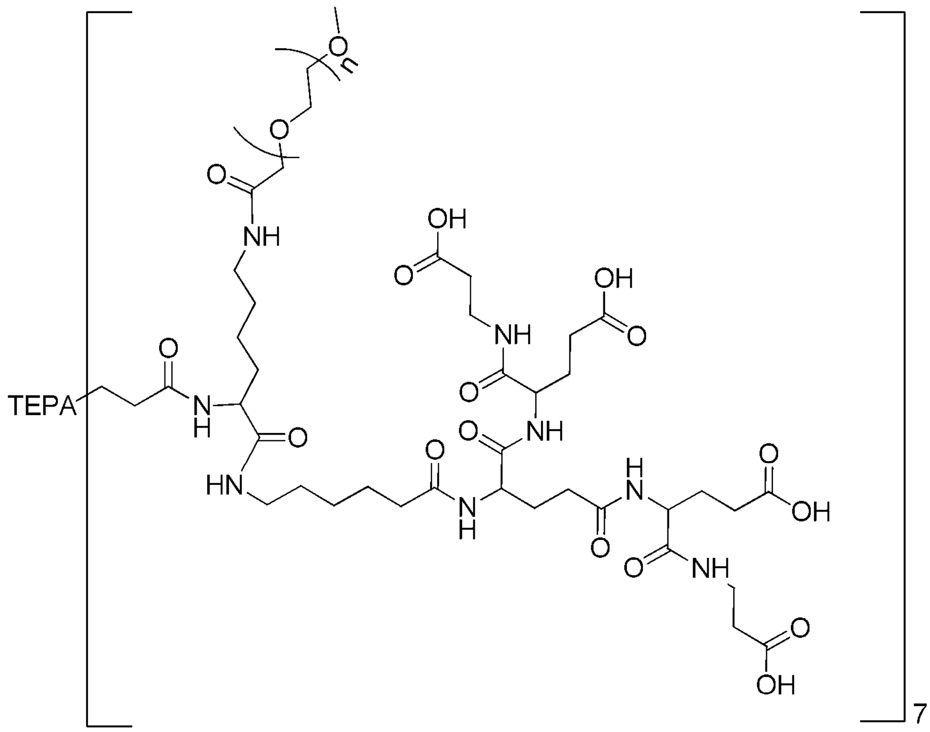

**73-17** (0.35 g, 0.0081 mmol) was added to a 50 mL round-bottom flask, then TFA (5.2 mL) was added, and the mixture was reacted with stirring at room temperature. After the reaction was completed, most of the TFA was removed using a rotary evaporator, and methyl tert-butyl ether (50 mL) was added for precipitation. A solid precipitated and was filtered. The filter cake was redissolved in DMF (5 mL), and then methyl tert-butyl ether (45 mL) was added for precipitation. This process was repeated three times. The filter cake was redissolved in DMF (5 mL), followed by a slowly dropwise addition of DIEA (0.165 mL, 1 mmol) with stirring for neutralization. Methyl tert-butyl ether (45 mL) was then added for precipitation. A solid precipitated and was filtered. The filter cake was dried to provide 0.19 g of the product with a yield of 57%. $^1$H-NMR (600 MHz, DMSO-$d_6$) δ 12.1-11.89 (m, 28H), 8.23-7.99 (m, 42H), 7.99-7.92 (m, 14H), 4.26-4.16 (m, 14H), 4.13-4.04 (m, 14H), 3.89-3.80 (m, 7H), 3.63-3.38 (m, 3301H), 3.38-3.10 (m, 14H), 3.10-2.85 (m, 35H), 2.82-2.68 (m, 26H), 2.44-2.24 (m, 28H), 2.24-2.03 (m, 56H), 1.91-1.62 (m, 42H), 1.61-1.09 (m, 84H)

12.13 Preparation of **73-49**

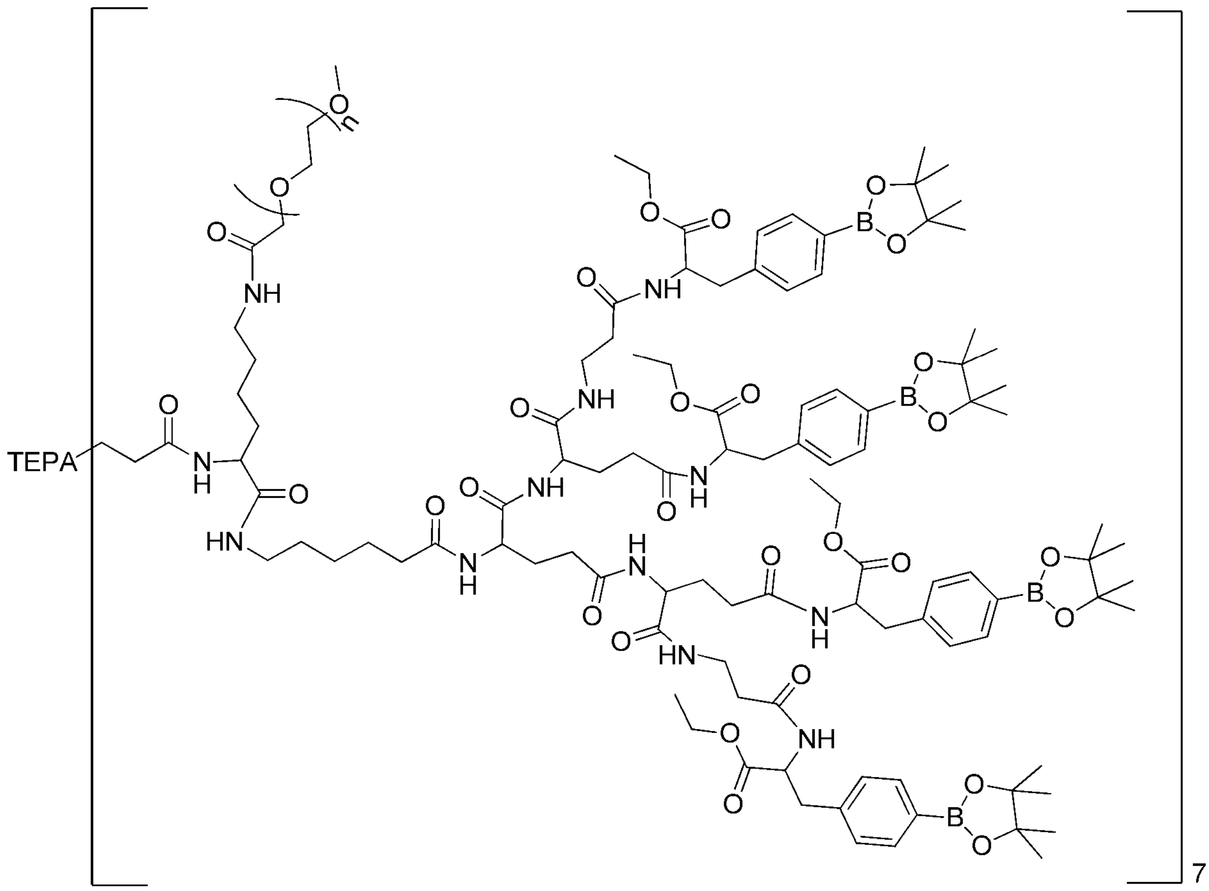

**73-47** (0.1 g, 0.0023 mmol), HBTU (0.0376 g, 0.099 mmol), HOBT (0.013 g, 0.099 mmol), and **67-154** (0.0317 g, 0.099 mmol) were added to a 50 mL round-bottom flask, then DMF (7 mL) was added to dissolve the mixture with the assistance of ultrasonication, and the mixture was stirred at room temperature for 1 minute. DIEA (0.05 mL, 0.298 mmol) was slowly added dropwise to continue the reaction. After the reaction was completed, methyl tert-butyl ether (45 mL) was added for precipitation. A solid precipitated and was filtered. The filter cake was redissolved in an anhydrous ethanol/dichloromethane (1/1) mixture (10 mL), and then methyl tert-butyl ether (45 mL) was added for precipitation. A solid precipitated and was collected by suction filtration. This process was repeated three times. The resulting filter cake was dried to provide 0.034 g of the product. $^1$H-NMR (600 MHz, DMSO-$d_6$) δ 8.23-7.99 (m, 70H), 7.99-7.92 (m, 14H), 7.70-7.52 (m, 56H), 7.33-7.20 (m, 56H), 4.26-4.04 (m, 84H), 3.89-3.80 (m, 7H), 3.63-3.38 (m, 3301H), 3.38-3.10 (m, 14H), 3.10-2.85 (m, 91H), 2.85-2.68 (m, 26H), 2.44-2.22 (m, 28H), 2.22-2.03 (m, 56H), 1.90-1.62 (m, 42H), 1.62-1.09 (m, 504H)

Example 13: Synthesis of Compound **82-226**

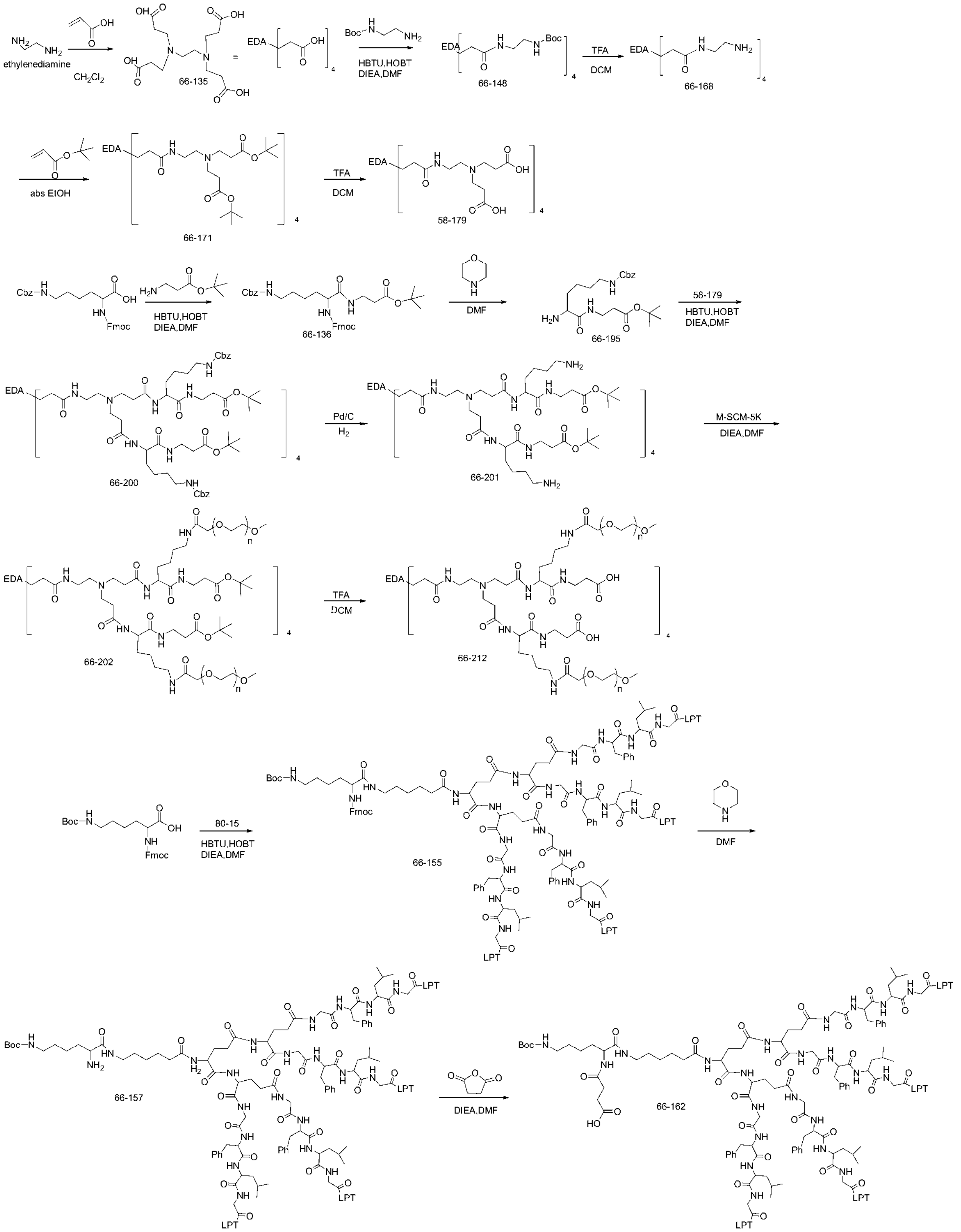
ethylenediamine
CH2Cl2
66-135
HBTU,HOBT
DIEA,DMF
66-148
TFA
DCM
66-168
abs EtOH
66-171
TFA
DCM
58-179
HBTU,HOBT
DIEA,DMF
66-136
DMF
66-195
58-179
HBTU,HOBT
DIEA,DMF
66-200
Pd/C
H2
66-201
M-SCM-5K
DIEA,DMF
66-202
TFA
DCM
66-212
80-15
HBTU,HOBT
DIEA,DMF
66-155
DMF
66-157
DIEA,DMF
66-162

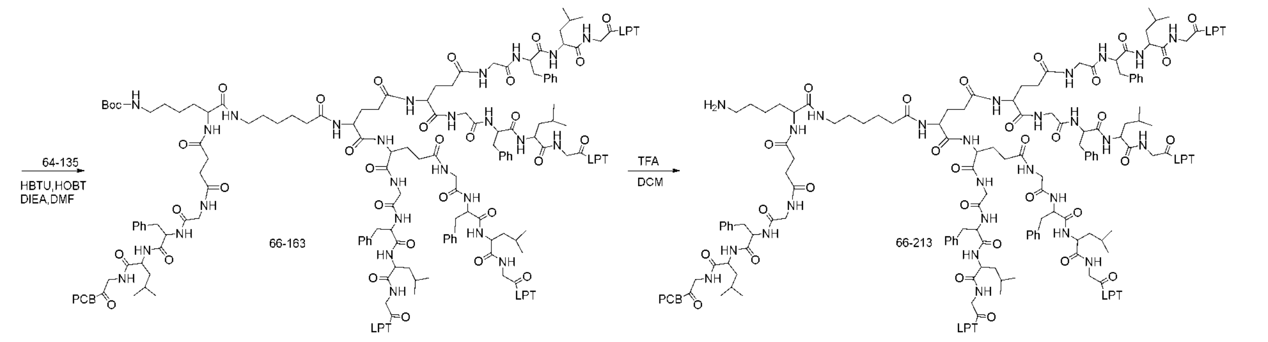


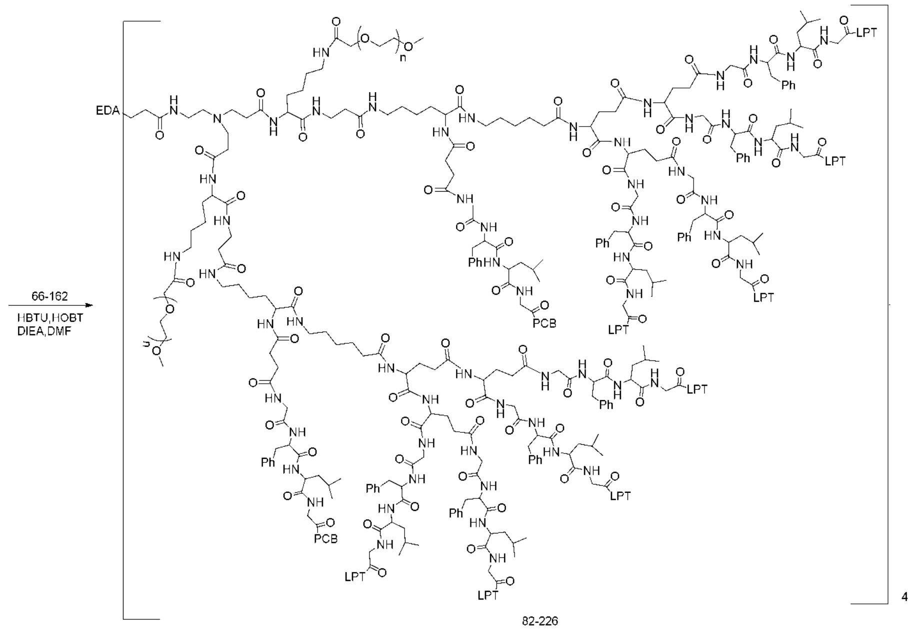


13.1 Preparation of **66-135**

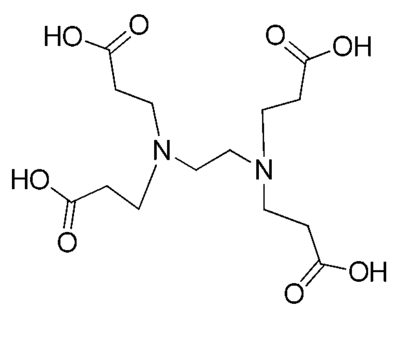

Ethylenediamine (1 g, 12.8041 mmol) was added to a reaction flask containing dichloromethane (10 mL), and the mixture was stirred at low temperature. Acrylic acid (10 g, 138.7732 mmol) was dissolved in dichloromethane (20 mL) and then slowly added dropwise to the reaction flask. After the dropwise addition was completed, the flask was filled with nitrogen for protection, and transferred to an oil bath at 55°C. The mixture was stirred under reflux for 3 days, and a white solid precipitated. After the reaction was completed, the mixture was filtered to provide a filter cake as a white solid, which was washed three times with dichloromethane (100 mL) and dried to provide 2.4434 g of the crude product with a yield of 55%. $^1$H-NMR (600 MHz, DMSO-$d_6$) $\delta$ 12.01 (s, 4H), 2.80-2.68 (m, 8H), 2.57-2.53 (m, 4H), 2.36-2.31 (m, 8H)

13.2 Preparation of **66-148**

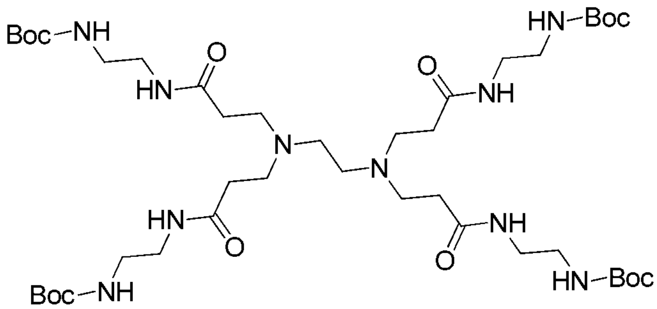

**66-135** (0.8 g, 2.2965 mmol), Boc-ethylenediamine (2.9434 mL, 18.3723 mmol), HOBT (5.2255 g, 13.7790 mmol), and HBTU (1.8618 g, 13.7790 mmol) were placed in a reaction flask, then DMF (100 mL) was added to dissolve the mixture with the assistance of ultrasonication, and the mixture was stirred at 0°C. DIEA (6.0 mmol, 36.744 mmol) was slowly added dropwise. After the dropwise addition was completed, the mixture was brought to room temperature and the reaction was carried out with stirring, and the reaction progress was monitored by TLC. After the reaction was completed, n-hexane (50 mL) and methyl tert-butyl ether (200 mL) were added for precipitation, resulting in an oily solid. The supernatant was discarded, and methyl tert-butyl ether (200 mL) was added for precipitation. This process was repeated five times. The crude product was dissolved in ethyl acetate (15 mL) and refrigerated at 2-8°C overnight. A powdery solid precipitated and was filtered. The filter cake was washed three times with methyl tert-butyl ether (60 mL). The filter cake was collected and dissolved in a methanol/dichloromethane mixture. The resulting product was dry-loaded onto the column and subjected to column chromatography using 1-8% methanol/dichloromethane as the eluent. The desired product was collected, concentrated, and dried in a vacuum oven to provide 0.6488 g of the product with a yield of 30.89%. $^1$H-NMR (600 MHz, DMSO-$d_6$) δ 8.02-7.94 (m, 4H), 7.19-7.04 (m, 1H), 6.94-6.55 (m, 3H), 3.12-3.03 (m, 8H), 3.01-2.92 (m, 8H), 2.63-2.57 (m, 4H), 2.49-2.45 (m, 8H), 2.28-2.16 (m, 8H), 1.45-1.30 (m, 36H); ESI [M+H$^+$] 917.106

13.3 Preparation of **66-168**

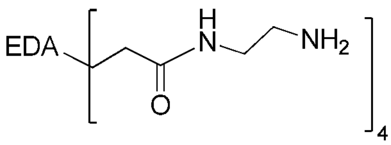

**66-148** (1.1 g, 1.1994 mmol) was added to a 250 mL round-bottom flask, then dichloromethane (20 mL) was added for dissolution with the assistance of ultrasonication, and then TFA (5.3 mL, 71.9628 mmol) was added. Finally, the reaction flask was allowed to stand at room temperature, and the reaction was carried out with stirring overnight, and monitored by TLC. After the reaction was completed, the reaction mixture was concentrated under reduced pressure to remove dichloromethane and most of the TFA. Then, n-hexane (50 mL) and methyl tert-butyl ether (200 mL) were added for precipitation, resulting in an oily solid. The supernatant was discarded, and methyl tert-butyl ether (200 mL) was added again for precipitation. This process was repeated three times. A powdery solid precipitated and was filtered. The filter cake was collected and dried in a vacuum oven to provide 0.6197 g of the product.

13.4 Preparation of **66-171**

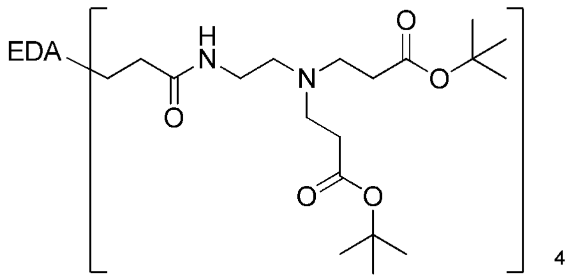

**66-168** (0.6197 g, 1.1994 mmol) was dissolved in anhydrous ethanol (20 mL). DIEA (3.2 mL, 19.1904 mmol) was slowly added dropwise at -5°C. After 10 minutes, tert-butyl acrylate (2.4596 g, 19.1904 mmol) was added. The reaction was carried out with stirring and nitrogen protection at 40°C for 2 days, and the reaction progress was monitored by TLC. After the reaction was completed, the reaction mixture was concentrated under reduced pressure, and then n-hexane (25 mL) and methyl tert-butyl ether (200 mL) were added for precipitation. The supernatant was discarded, and n-hexane and methyl tert-butyl ether were added again for precipitation. This process was repeated three times. The obtained product was dissolved in a mixture of dichloromethane and methanol, then silica gel powder was added, and the mixture was evaporated to dryness. The resulting product was dry-loaded onto the column and subjected to column chromatography using 3-10% methanol/dichloromethane as the eluent. The desired product was collected, concentrated, and dried to provide 1.14 g of the product. $^1$H-NMR (600 MHz, DMSO-$d_6$) δ 7.93-7.62 (m, 4H), 3.16-2.92 (m, 16H), 2.69-2.61 (m, 19H), 2.46-2.38 (m, 13H), 2.35-2.27 (m, 17H), 2.23-2.13 (m, 4H), 1.43-1.36 (m, 72H)

13.5 Preparation of **58-179**

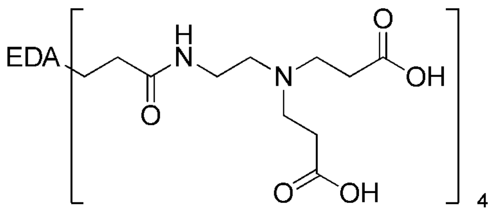

**66-171** (1.14 g, 0.739 mmol) was added to a 250 mL round-bottom flask, and 4M 1,4-dioxane hydrochloride (45 mL, 88.68 mmol) was added for dissolution. The reaction flask was placed in an oil bath at 70°C, and the reaction was carried out with stirring and reflux for 24 hours. The reaction progress was monitored by TLC. After the reaction was completed, the reaction mixture was concentrated under reduced pressure, and n-hexane (25 mL) and methyl tert-butyl ether (200 mL) were added for precipitation. The supernatant was discarded, and n-hexane (25 mL) and methyl tert-butyl ether (200 mL) were added again for precipitation. This process was repeated three times. The mixture was filtered to provide a solid product, then toluene (2 mL) and dichloromethane (10 mL) were added for dissolution with the assistance of ultrasonication, and then the mixture was evaporated to dryness and dried in a vacuum oven to provide 0.8078 g of the product with a yield exceeding 100%. $^1$H-NMR (600 MHz, DMSO-$d_6$) δ 12.18-12.11 (m, 4H), 8.44-8.41 (m, 4H), 3.69-3.65 (m, 12H), 3.47-3.40 (m, 12H), 3.38-3.34 (m, 14H), 3.14-3.09 (m, 10H), 2.86-2.82 (m, 6H), 2.78-2.70 (m, 14H)

13.6 Preparation of 66-136

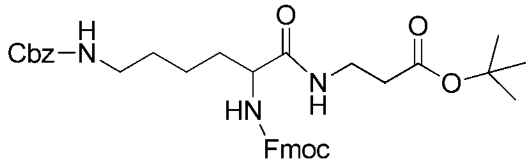

N'-Fluorenylmethyloxycarbonyl-N-benzyloxycarbonyl-L-lysine (5.5329 g, 11.0096 mmol), alanine tert-butyl ester (2.0 g, 11.0096 mmol), HOBT (1.6364 g, 12.1105 mmol), and HBTU (4.5927 g, 12.1105 mmol) were placed in a reaction flask, and DMF (30 mL) was added to dissolve the mixture with the assistance of ultrasonication. DIEA (4 mL,

24.2211 mmol) was added dropwise at 0°C. After the dropwise addition, the mixture was brought to room temperature and the reaction was continued with stirring overnight. The reaction progress was monitored by TLC. After the reaction was completed, the mixture was poured into a separatory funnel containing saturated NaCl solution (300 mL) and ethyl acetate (300 mL) for extraction. After standing for phase separation, the organic phase was collected, and the aqueous phase was further extracted with ethyl acetate (200 mL × 3). The organic phases were combined, evaporated to dryness and dried in a vacuum oven to provide 6.9332 g of the product with a yield exceeding 100%.

13.7 Preparation of **66-195**

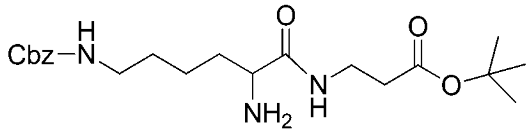

Reactant **66-136** (6.9332 g, 11.0096 mmol) was dissolved in DMF (30 mL), then morpholine (14.4 mL, 165.144 mmol) was added, and the mixture was reacted with stirring at room temperature for 3 hours. The reaction progress was monitored by TLC. After the reaction was completed, the mixture was poured into a separatory funnel containing saturated NaCl solution (100 mL) and ethyl acetate (150 mL) for extraction. After standing for phase separation, the organic phase was collected, and the aqueous phase was further extracted with ethyl acetate (100 mL × 3). The organic phases were combined, concentrated, evaporated to dryness, and dried in a vacuum oven to provide 3.9 g of the product with a yield of 86.92%. $^1$H-NMR (600 MHz, DMSO-$d_6$) δ 8.01-7.90 (m, 1H), 7.43-7.27 (m, 5H), 7.26-7.16 (m, 1H), 5.10-4.49 (m, 2H), 3.30-3.20 (m, 2H), 3.14-3.07 (m, 1H), 3.00-2.93 (m, 2H), 2.45-2.22 (m, 3H), 1.58-1.48 (m, 1H), 1.42-1.39 (m, 9H), 1.38-1.21 (m, 5H); ESI [M+H$^+$] 409.30

13.8 Preparation of **66-200**

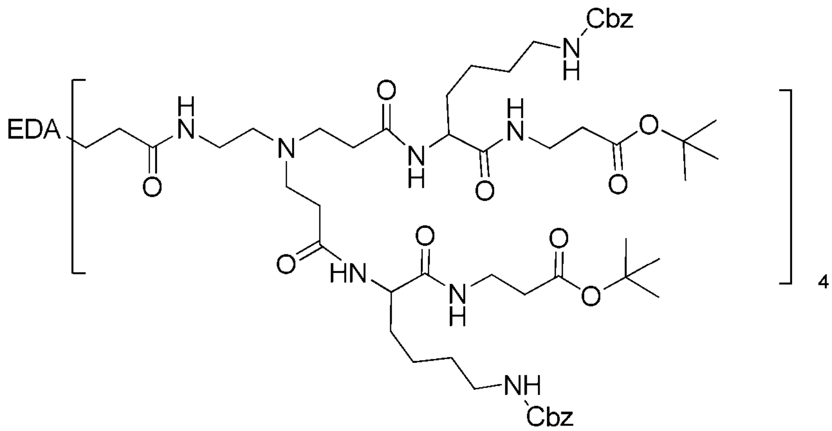

**58-179** (0.1156 g, 0.1057 mmol), **66-195** (0.656 g, 1.6099 mmol), and PyOxim (0.9727 g, 1.9022 mmol, purchased from Innochem) were placed in a reaction flask, then DMF (30 mL) was added to dissolve the mixture with the assistance of ultrasonication, and the mixture was stirred at 0°C. DIEA (1 mL, 5.8545 mmol) was added dropwise. After the dropwise addition was completed, the mixture was brought to room temperature and the reaction was continued with stirring. The reaction progress was monitored by TLC. After the reaction was completed, n-hexane (25 mL) and methyl tert-butyl ether (200 mL) were added for precipitation. The supernatant was discarded, and n-hexane (30 mL) and methyl tert-butyl ether (100 mL) were added again for precipitation. This process was repeated three times. The crude product was dissolved in a mixture of dichloromethane and methanol, then silica gel powder was added, and the mixture was evaporated to dryness. The resulting product was dry-loaded onto the column and subjected to column chromatography using 1% ammonia water/5-15% methanol/dichloromethane as the eluent. The desired product was collected, concentrated, and dried in a vacuum oven to provide 0.1868 g of the product with a yield of 42%. $^1$H-NMR (600 MHz, DMSO-$d_6$) δ 8.10-8.04 (m, 6H), 8.03-8.01 (m, 3H), 8.00-7.93 (m, 9H), 7.85-7.71 (m, 4H), 7.39-7.26 (m, 40H), 7.22-7.15 (m, 7H), 5.05-4.97 (m, 16H), 4.20-4.14 (m, 7H), 4.11-4.09 (m, 1H), 3.58-3.55 (m, 8H), 3.18-3.16 (m, 8H), 3.11-3.06 (m, 8H),

2.97-2.92 (m, 16H), 2.69-2.62 (m, 24H), 2.45-2.40 (m, 16H), 2.35-2.32 (m, 16H), 2.28-2.22 (m, 12H), 2.20-2.16 (m, 6H), 1.62-1.51 (m, 9H), 1.50-1.42 (m, 11H), 1.39-1.36 (m, 72H), 1.36-1.32 (m, 8H), 1.29-1.14 (m, 20H)

13.9 Preparation of **66-201**

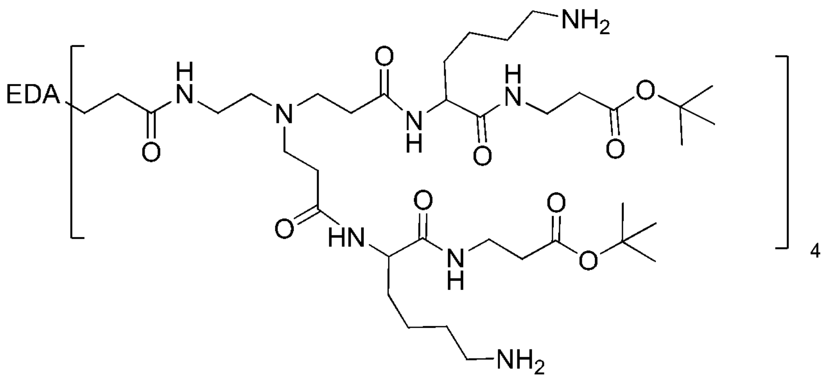

**66-200** (0.1868 g, 0.0443 mmol) was weighed and added to a micro hydrogenation reactor. DMF (30 mL) was added for dissolution, and then 10% Pd/C catalyst (30 mg) was added. A magnetic stirrer was placed. The hydrogenation reactor was sealed, evacuated with a water pump, and then filled with hydrogen gas. This process was repeated three times. The hydrogen pressure in the hydrogenation reactor was adjusted to 2 MPa. The mixture was stirred overnight, and the reaction progress was monitored by TLC. After the reaction was completed, the mixture was filtered through Celite, and the filtrate was taken for later use.

13.10 Preparation of **66-202**

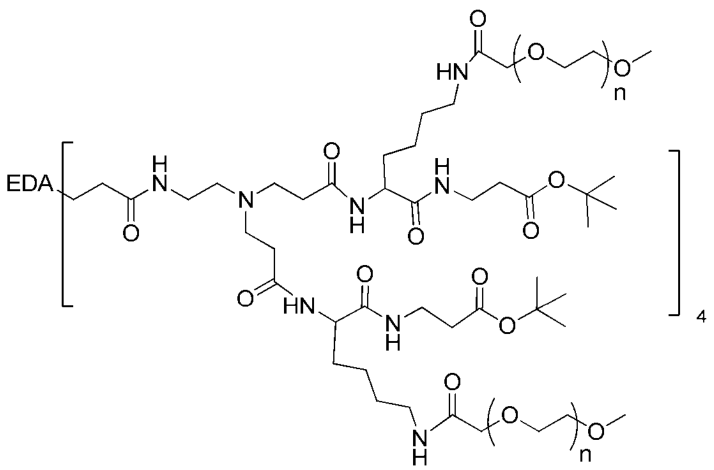

Reactant **66-201** (0.0695 g, 0.0222 mmol) was placed in a reaction flask, then DMF (30 mL) was added for dissolution with the assistance of ultrasonication, and the mixture was stirred at 0°C. DIEA (0.12 mmol, 0.7088 mmol) was added. After the dropwise addition was completed, the mixture was brought to room temperature and the reaction was continued with stirring for 0.5 hours. M-SCM-5K (1.0350 g, 0.1949 mmol, purchased from Jenkem) was added, and the reaction progress was monitored by TLC. After the reaction was completed, n-hexane (50 mL) and methyl tert-butyl ether (200 mL) were added for precipitation, resulting in an oily solid. The supernatant was discarded, and methyl tert-butyl ether (200 mL) was added again for precipitation. This process was repeated three times. A powdery solid precipitated and was filtered. The filter cake was washed three times with methyl tert-butyl ether (60 mL). The filter cake was collected and dried to provide 0.99 g of the product with a yield exceeding 100%.

13.11 Preparation of **66-212**

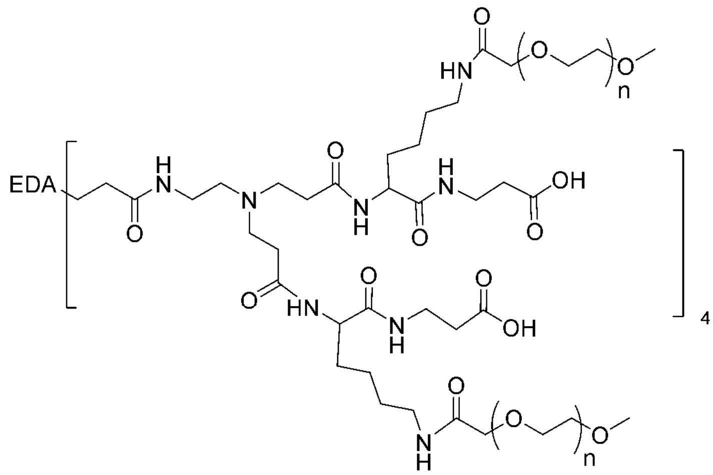

Reactant **66-202** (0.99 g, 0.0245 mmol) was dissolved in dichloromethane (10 mL), then TFA (2.7 mL, 3.675 mmol) was added, and the mixture was reacted with stirring and reflux at 50°C. The reaction progress was monitored by TLC. After the reaction was completed, the reaction mixture was concentrated under reduced pressure to remove dichloromethane and most of the TFA. Then, n-hexane (50 mL) and methyl tert-butyl ether (200 mL) were added for precipitation, resulting in an oily solid. The supernatant was discarded, and methyl tert-butyl ether (200 mL) was added again for precipitation. This process was repeated three times. A powdery solid precipitated and was filtered. The filter cake was collected and dried in a vacuum oven to provide 1.33 g of the product.

13.12 Preparation of Compound **66-155**

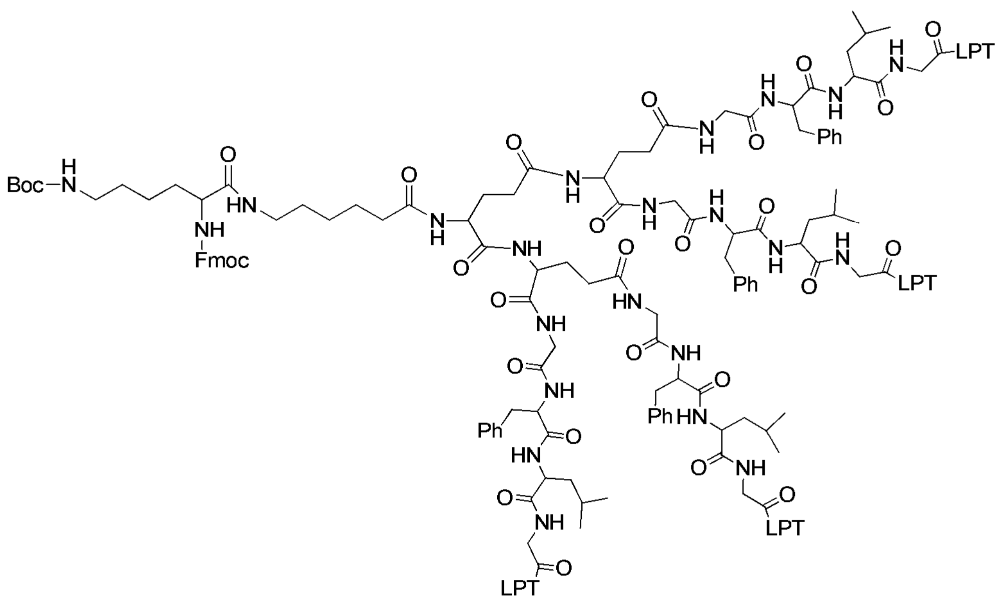

Reactants **80-15** (1.4 g, 0.3279 mmol), Boc-Lys(Fmoc)-OH (0.1613 g, 0.3607 mmol), HOBT (0.0487 g, 0.3607 mmol), and HBTU (0.1368 g, 0.3607 mmol) were placed in a reaction flask, then DMF (20 mL) was added to dissolve the mixture with the assistance of ultrasonication, and the mixture was stirred at 0°C. DIEA (0.5 mmL, 2.8112 mmol) was added dropwise. After the dropwise addition was completed, the mixture was brought to room temperature and the reaction was carried out with stirring. The reaction progress was monitored by TLC. After the reaction was completed, n-hexane (50 mL) and methyl tert-butyl ether (200 mL) were added for precipitation, resulting in an oily solid. The supernatant was discarded,

and methyl tert-butyl ether (200 mL) was added again for precipitation. This process was repeated three times. A powdery solid precipitated and was filtered. The filter cake was collected and dried in a vacuum oven to provide 1.5 g of the product with a yield of 96.94%.

13.13 Preparation of **66-157**

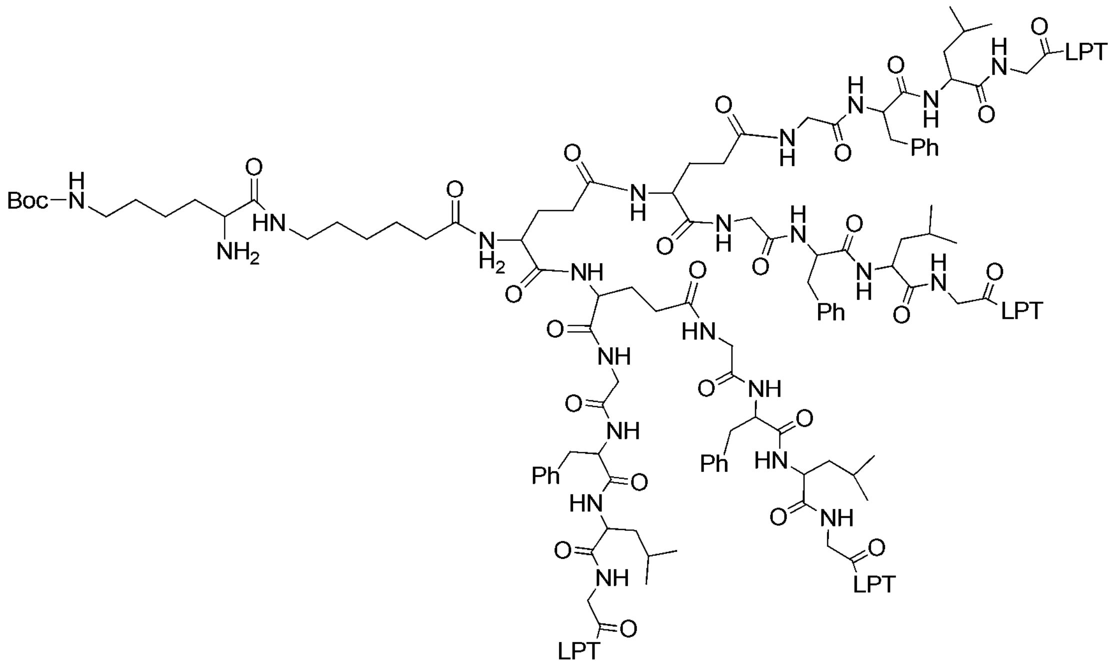

Reactant **66-155** (1.5 g, 0.3179 mmol) was dissolved in DMF (30 mL), then morpholine (0.4 mL, 4.7680 mmol) was added, and the mixture was reacted with stirring at room temperature. The reaction progress was monitored by TLC. After the reaction was completed, n-hexane (30 mL) and methyl tert-butyl ether (150 mL) were added for precipitation, resulting in an oily solid. The supernatant was discarded, and methyl tert-butyl ether (100 mL) was added again for precipitation. This process was repeated three times. A powdery solid precipitated and was filtered. The filter cake was collected and dried in a vacuum oven to provide 0.4772 g of the product with a yield of 34%. ESI [M+H$^+$] 4498.78

13.14 Preparation of **66-162**

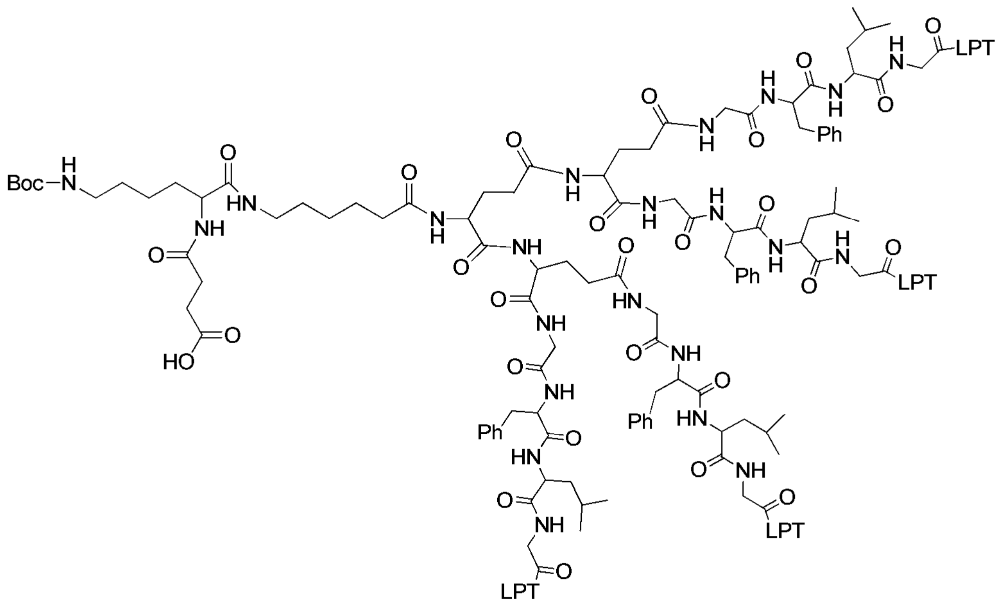

**66-157** (0.4772 g, 0.1061 mmol) was weighed and added to a 250 mL flask, and then DMF (30 mL) was added for dissolution. DIEA (0.1 mL, 0.5306 mmol) was slowly added dropwise, and reacted with stirring for 1 hour. Then, succinic anhydride (0.03 g, 0.3183 mmol) was added dropwise. After the dropwise addition was completed, the reaction was carried out with stirring at room temperature overnight. After the reaction was completed, n-hexane (30 mL) and methyl tert-butyl ether (150 mL) were added for precipitation, resulting in an oily solid. The supernatant was discarded, and methyl tert-butyl ether (100 mL) was added again for precipitation. This process was repeated three times. A powdery solid precipitated and was filtered. The filter cake was collected and dried in a vacuum oven to provide 0.3951 g of the product with a yield of 81.01%. ESI [M+Na$^+$] 4618.63

13.15 Preparation of **66-163**

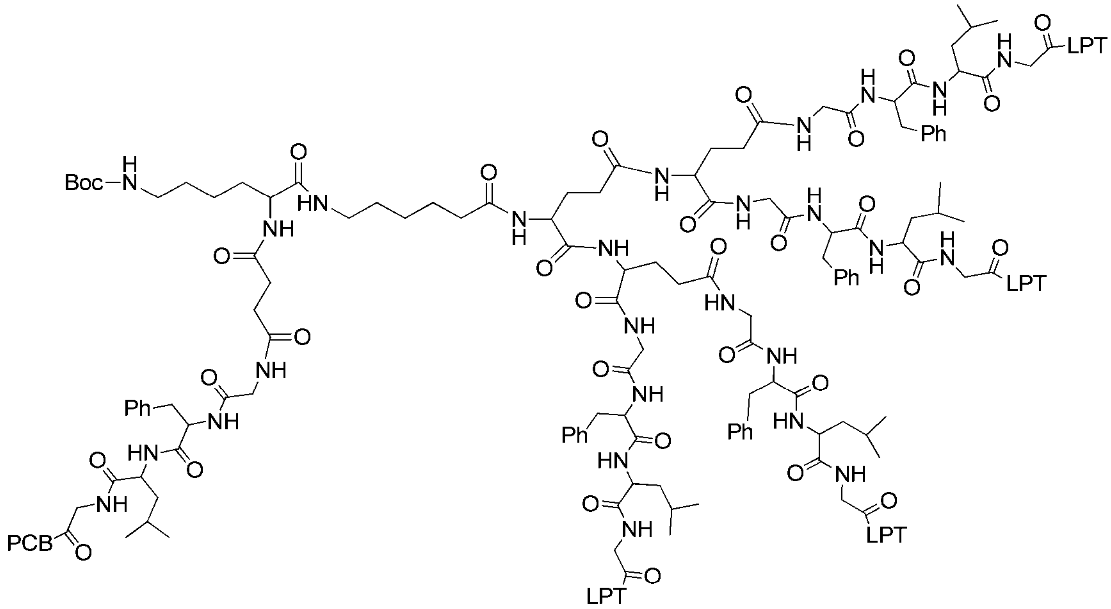

**66-162** (0.3951 g, 0.0859 mmol), **64-135** (0.0742 g, 0.0902 mmol), HOBT (0.0128 g, 0.0945 mmol), and HBTU (0.0359 g, 0.0945 mmol) were placed in a reaction flask, then DMF (20 mL) was added to dissolve the mixture with the assistance of ultrasonication, and the mixture was stirred at 0°C. DIEA (0.03 mL, 0.1891 mmol) was added dropwise. After the dropwise addition was completed, the mixture was brought to room temperature and the reaction was carried out with

stirring. The reaction progress was monitored by TLC. After the reaction was completed, n-hexane (30 mL) and methyl tert-butyl ether (150 mL) were added for precipitation, resulting in an oily solid. The supernatant was discarded, and methyl tert-butyl ether (100 mL) was added again for precipitation. This process was repeated three times. A powdery solid precipitated and was filtered. The filter cake was collected and dried in a vacuum oven to provide 0.3975 g of the product with a yield of 85.68%.

13.16 Preparation of **66-213**

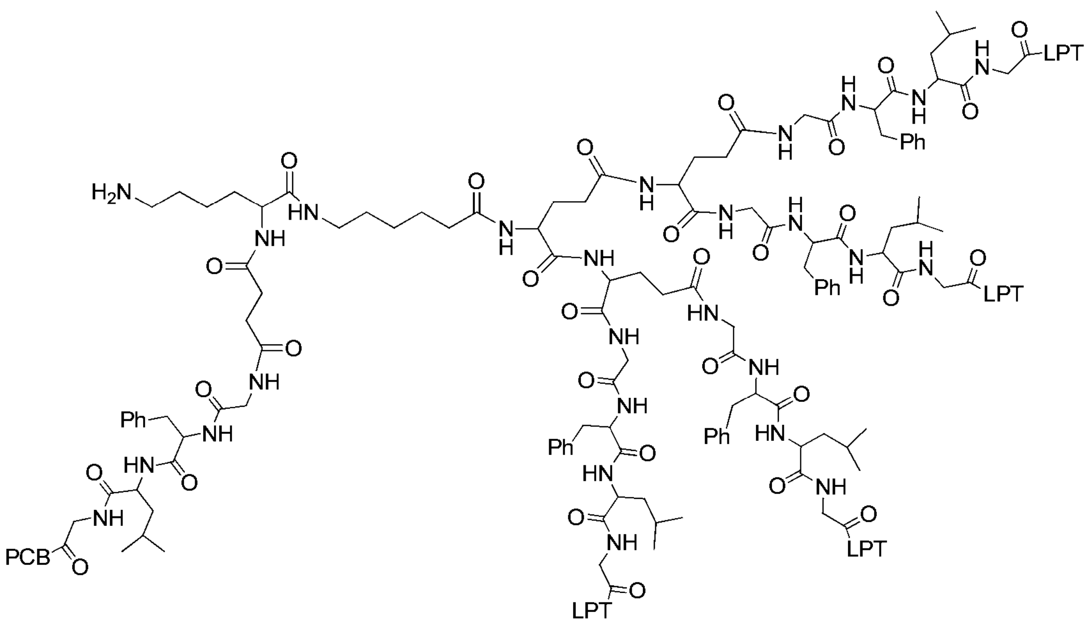

**66-163** (0.3975 g, 0.0736 mmol) was added to a 250 mL flask, then dichloromethane (5 mL) and TFA (0.8 mL, 11.04 mmol) were added for dissolution, and the mixture was reacted with stirring at room temperature overnight. The reaction progress was monitored by TLC. After the reaction was completed, the reaction mixture was evaporated to an oily state using a rotary evaporator, and then methyl tert-butyl ether (60 mL) was added for precipitation. A powdery solid precipitated and collected by suction filtration. The filter cake was washed with methyl tert-butyl ether (40 mL × 3). The filter cake was collected and dried in a vacuum oven to provide 0.3901 g of the product with a yield exceeding 100%. ESI [M+H$^+$] 5298.104

13.17 Preparation of **82-226**

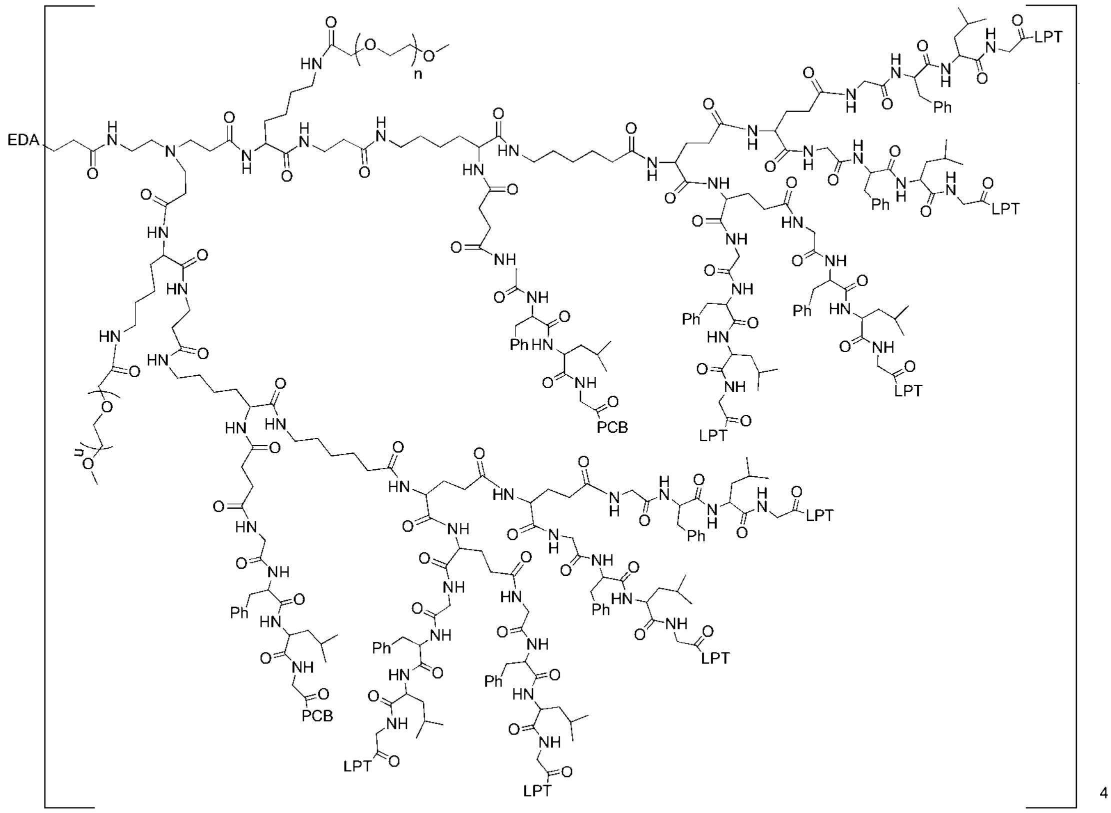

**66-162** (0.37 g, 0.084 mmol), **66-213** (0.39 g, 0.0736 mmol), HOBT (0.01 g, 0.0736 mmol), and HBTU (0.3 g, 0.0736 mmol) were placed in a reaction flask, then DMF (30 mL) was added to dissolve the mixture with the assistance of ultrasonication, and the mixture was stirred at 0°C. DIEA (0.4 mmol, 2.6880 mmol) was added dropwise. After the dropwise addition was completed, the mixture was brought to room temperature and the reaction was carried out with stirring. The reaction progress was monitored by TLC. After the reaction was completed, n-hexane (50 mL) and methyl tert-butyl ether (200 mL) were added for precipitation, resulting in an oily solid. The supernatant was discarded, and methyl tert-butyl ether (200 mL) was added for precipitation. This process was repeated three times. A powdery solid precipitated and was filtered. The filter cake was washed three times with methyl tert-butyl ether (60 mL). The filter cake was collected, dissolved in a methanol/dichloromethane mixture, dry-loaded onto the column and subjected to column chromatography using 1-8% methanol/dichloromethane as the eluent. The desired product was collected, concentrated, and dried in a vacuum oven to provide 0.09 g of the product with a yield of 12.4%. $^1$H-NMR (600 MHz, DMSO-$d_6$) δ 10.18-10.07 (m, 8H), 9.91-9.85 (m, 32H), 9.05-9.03 (m, 24H), 8.98-8.91 (m, 8H), 8.78-8.71 (m, 40H), 8.59-8.53 (m, 40H), 8.24-8.04 (m, 216H), 8.02-8.00 (m, 56H), 7.98-7.95 (m, 56H), 7.90-7.84 (m, 16H), 7.82-7.78 (m, 40H), 7.74-7.70 (m, 32H), 7.48-7.45 (m, 32H), 7.35-7.32 (m, 64H), 7.22-7.19 (m, 192H), 7.15-7.08 (m, 112H), 7.07-7.04 (m, 12H), 7.02-6.98 (m, 48H), 6.69-6.68 (m, 16H), 6.68-6.66 (m, 16H), 5.83-5.77 (m, 8H), 5.27-5.25 (m, 64H), 5.01-5.00 (m, 2H), 4.99-4.97 (m, 2H), 4.95-4.94 (m, 2H), 4.93-4.92 (m, 2H), 4.76-4.73 (m, 40H), 4.71-4.68 (m, 24H), 4.59-4.54 (m, 40H), 4.40-4.31 (m, 64H), 4.26-4.16 (m, 96H), 3.80-3.74 (m, 96H), 3.72-3.47 (m, 1864H), 3.07-2.99 (m, 184H), 2.91-2.88 (m, 64H), 2.74-2.73 (m, 64H), 2.63-2.60 (m, 24H), 2.43-2.42 (m, 16H), 2.40-2.37 (m, 16H), 2.31-2.30 (m, 16H), 2.17-2.06 (m, 80H), 1.51-1.42 (m, 142H), 1.36-1.35 (m, 64H), 1.23-1.22 (m, 196H), 1.20-1.19 (m, 16H), 1.19-1.18 (m, 16H), 1.17-1.16 (m, 16H), 1.07-1.06 (m, 2H), 1.06-1.05 (m, 4H), 1.05-1.04 (m, 2H)

Example 14: Synthesis of Compound **59-169**

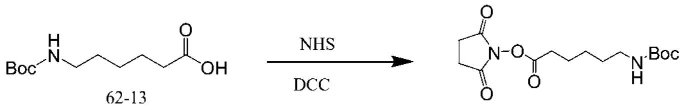


62-13

59-151

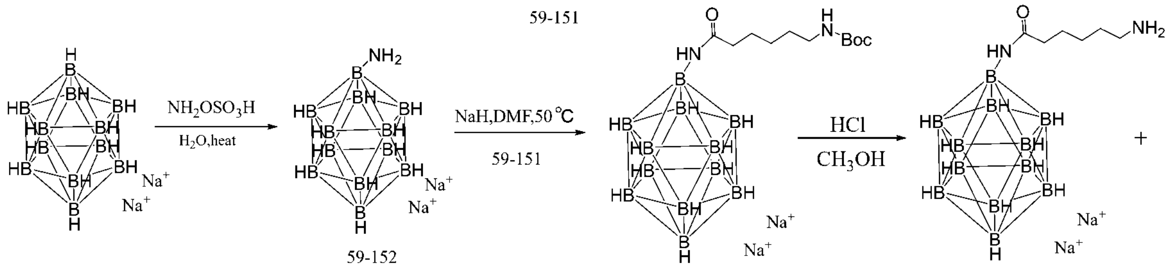


59-152

59-158

59-163

+

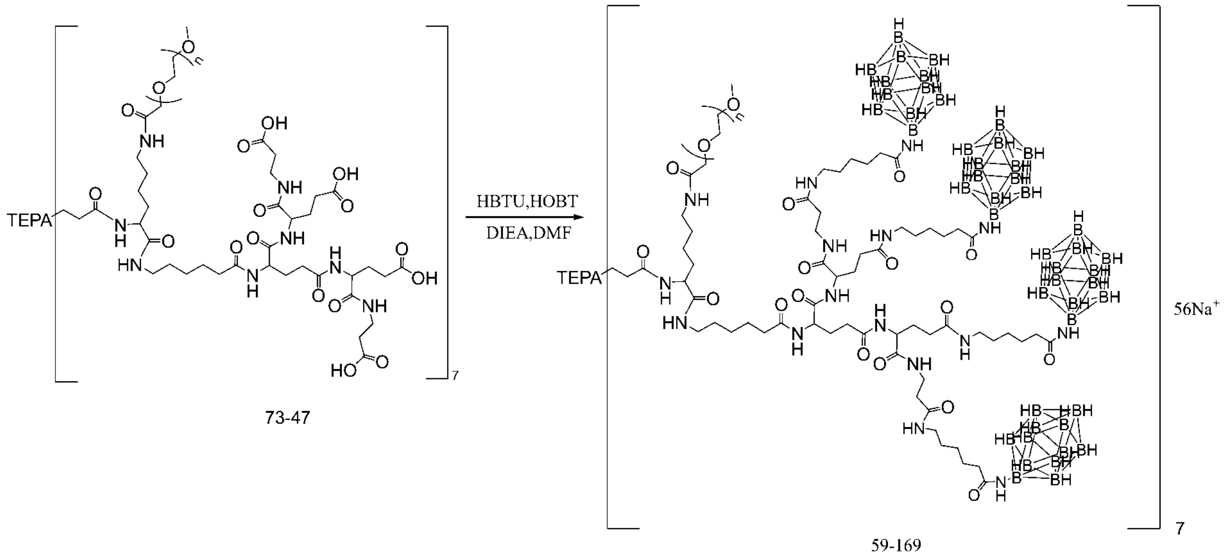


73-47

59-169

14.1 Preparation of Compound **59-151**

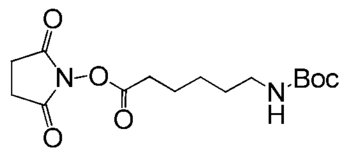

**62-13** (2 g, 8.65 mmol) and N,N-disuccinimidyl carbonate (3.3 g, 13 mmol) were added to a 100 mL round-bottom flask, then DMF (10 mL) was added to dissolve the mixture with the assistance of ultrasonication, and the mixture was stirred at 0°C. Triethylamine (1.8 mL, 13 mmol) was slowly added dropwise, and then stirred for 30 minutes. Finally, the reaction was carried out with stirring at room temperature for 3 hours. After the reaction was completed, the reaction mixture was transferred to a 1 L separatory funnel, and purified water (200 mL) and ethyl acetate (200 mL) were added for extraction. The organic phase was collected, and the aqueous phase was further extracted with ethyl acetate (200 mL). The organic phases were combined, washed with deionized water (200 mL $\times$ 2) and evaporated to dryness. The obtained solid product was dissolved in a dichloromethane/methanol (4/1) mixture, then 200-300 mesh silica gel powder was added, and the mixture was evaporated to dryness. The resulting product was dry-loaded onto the column and subjected to column chromatography using a 30% ethyl acetate/petroleum ether mixture as the eluent. The desired product was collected, concentrated, evaporated to dryness, and dried in a vacuum oven to provide 8.9 g of the product. $^1$H-NMR (600 MHz, DMSO-$d_6$) $\delta$ 6.77 (s, 1H), 2.90 (dd, *J* = 12.9, 6.6 Hz, 2H), 2.81 (s, 4H), 2.65 (t, *J* = 7.3 Hz, 2H), 1.41-1.30 (m, 15H)

14.2 Preparation of **59-152**

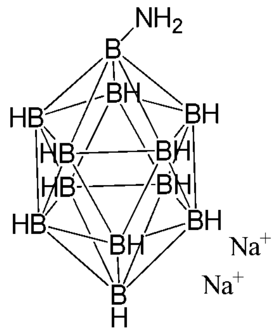

Sodium dodecahydrododecaborate (3.36 g, 0.0179 mol) was added to a 250 mL round-bottom flask, and distilled water (30 mL) was added for dissolution. The pH was measured to be approximately hydroxylamine-O-sulfonic acid (4.5 g, 0.0398 mol) was added and dissolved with the assistance of ultrasonication. The reaction mixture was reacted under nitrogen protection and reflux in an oil bath for 5 hours, and then cooled to room temperature. Tetramethylammonium chloride (2 g, 0.0182 mol) was dissolved in distilled water (5 mL), and added to the reaction mixture. After standing, a precipitate was obtained, which was filtered and dried to provide 7 g of a crude product. Distilled water (35 mL) was added to the crude product as a filter cake. The mixture was refluxed in an oil bath, then cooled to room temperature, and allowed to stand overnight for recrystallization. This process was repeated three times. The mixture was filtered, and the filter cake was dried in a vacuum oven to provide 2.46 g of the product with a yield of 60.0%.

14.3 Preparation of **59-158**

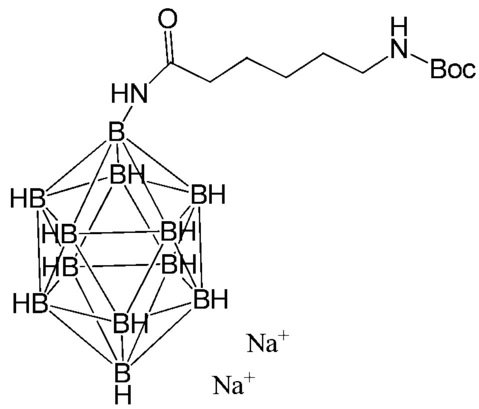

NaH (0.278 g, 11.56 mmol, dispersed in mineral oil with a content of 60%) was added to a 250 mL flask, then anhydrous DMF (10 mL) was added, and the mixture was stirred at 0°C under nitrogen protection. **59-152** (0.5 g, 2.89 mmol) was dissolved in anhydrous DMF (20 mL), slowly added dropwise to the reaction mixture, and stirred at 0°C for 30 minutes. When no bubbles were generated, the mixture was heated to 50°C, reacted for 1 hour, then cooled to room temperature, and **59-151** (1.23 g, 3.757 mmol) was added. After the dissolution with the assistance of ultrasonication, the mixture was heated to 50°C, and reacted with stirring overnight. The reaction progress was monitored by TLC. After the reaction was completed, n-hexane (25 mL) and methyl tert-butyl ether (200 mL) were added for precipitation. The supernatant was discarded, and n-hexane (20 mL) and methyl tert-butyl ether (100 mL) were added again for precipitation. This process was repeated five times. The resulting crude solid product was dissolved in a mixture of methanol and dichloromethane, and the insoluble matter was removed by filtration. The insoluble matter was then dissolved in a mixture of methanol and dichloromethane and filtered. This process was repeated twice. The filtrates were combined, concentrated to dryness using a rotary evaporator, and ethyl acetate (10 mL) was added for dissolution, and then n-hexane (150 mL) was added for precipitation. The mixture was filtered. The filter cake was collected, and dried to provide 0.808 g of the product **59-158** with a yield of 72.4%. $^1$H-NMR (600 MHz, DMSO-$d_6$) δ 6.76 (d, *J* = 5.0 Hz, 2H), 2.87 (d, *J* = 6.7 Hz, 2H),

2.37-2.34 (m, 2H), 1.45-1.40 (m, 4H), 1.37 (s, 9H), 1.33 (s, 2H), 1.18 (dd, $J$ = 8.7, 5.5 Hz, 11H)

14.4 Preparation of **59-163**

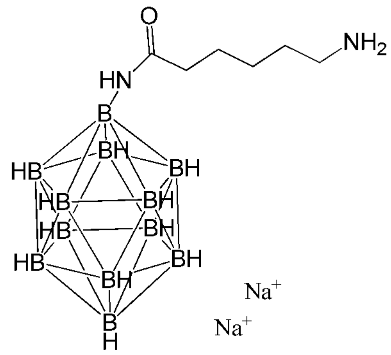

**59-158** (0.2 g, 0.518 mmol) was placed in a 100 mL flask, and TFA (0.77 mL, 10.36 mmol) was added. The reaction mixture was allowed to stand at room temperature, and the reaction was carried out with stirring. After the reaction was completed, the reaction mixture was concentrated by vacuum rotary evaporation. Methyl tert-butyl ether was then added for precipitation. The mixture was allowed to stand, and the supernatant was discarded. This process was repeated three times. A small amount of a methanol/dichloromethane (1/4) mixture was added to the resulting crude solid product for dissolution. Methyl tert-butyl ether was then added for precipitation. The mixture was allowed to stand, and the supernatant was discarded. This process was repeated twice. The resulting solid was then rotavapped using a rotary evaporator, and dried in a vacuum oven to provide 0.13 g of the product with a yield of 87.84%.

14.5 Preparation of **59-169**

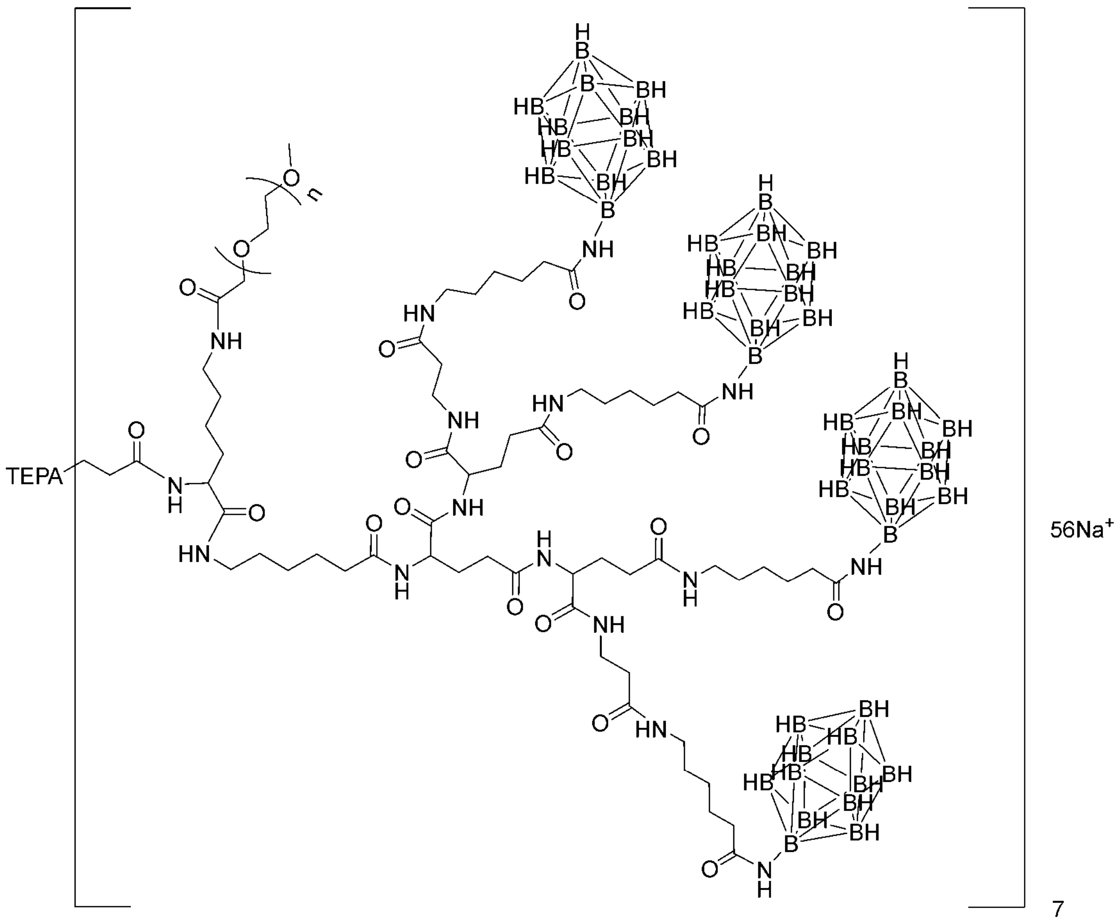

**73-47** (0.22 g, 0.00518 mmol), **59-163** (0.06 g, 0.005182 mmol), HOBT (0.0294 g, 0.2176 mmol), and HBTU (0.0825 g, 0.2176 mmol) were placed in a 100 mL round-bottom flask, and DMF (5 mL) was added for dissolution. DIEA (0.108 mL, 0.653 mmol) was slowly added dropwise with stirring at room temperature, and the reaction progress was monitored by TLC. After the reaction was completed, methyl tert-butyl ether (300 mL) and n-hexane (100 mL) were added for precipitation. The supernatant was discarded, and then methyl tert-butyl ether (300 mL) and n-hexane (100 mL) were added again for precipitation. This process was repeated three times to provide a solid precipitate. A dichloromethane/-methanol (4/1) mixture was added to the solid precipitate for dissolution, then methyl tert-butyl ether (300 mL) and n-hexane (100 mL) were added for precipitation, and the supernatant was discarded. This process, i.e., dissolution/precipitation, was repeated five times. Finally, the resulting solid was dried to provide 0.039 g of the product. $^1$H-NMR (600 MHz, DMSO-$d_6$) δ 8.02-7.926 (m, 28H), 7.898-7.849 (m, 7H), 7.747-7.705 (m, 7H), 7.682-7.641 (m, 7H), 7.419-7.391 (m, 7H), 7.167-7.116 (m, 56H), 4.64-4.58 (m, 21H), 3.636-3.63 (m, 28H), 3.525-3.501 (m, 3269H), 3.246-3.227 (m, 28H), 3.095-3.075 (m, 64H), 2.924-2.90 (m, 16H), 2.898-2.897 (m, 56H), 2.756-2.734 (m, 44H), 2.554-2.546 (m, 8H), 2.533-2.512 (m, 66H), 2.209-2.156 (m, 14H), 2.025-1.977 (m, 14H), 1.68-1.633 (m, 32H), 1.559-1.548 (m, 14H), 1.395-1.370 (m, 120H), 1.355-1.350 (m, 86H), 1.228-1.224 (m, 308H)

Example 15: Synthesis of Compound **67-237**

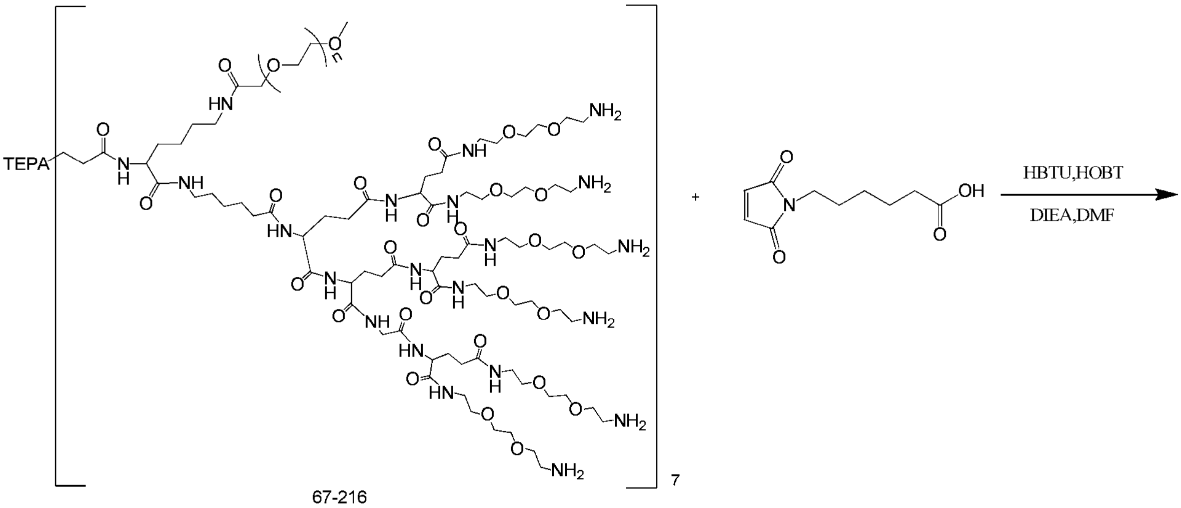


67-216

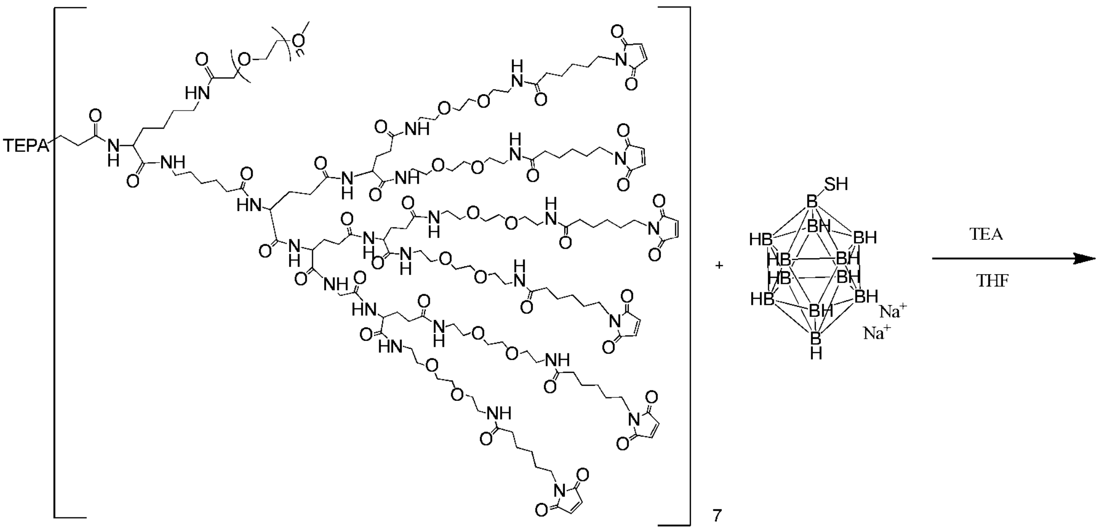


67-236

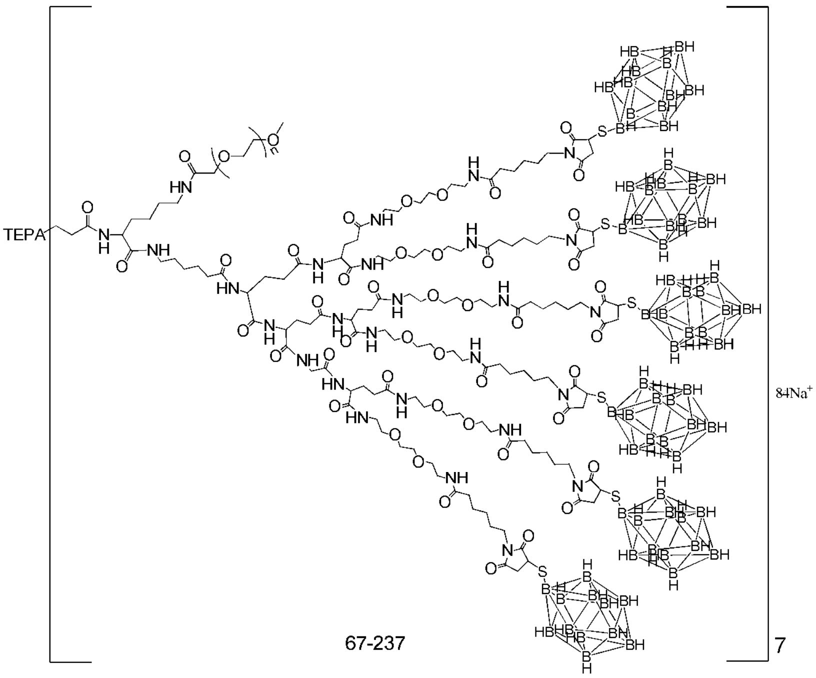

67-237

15.1 Preparation of **67-236**

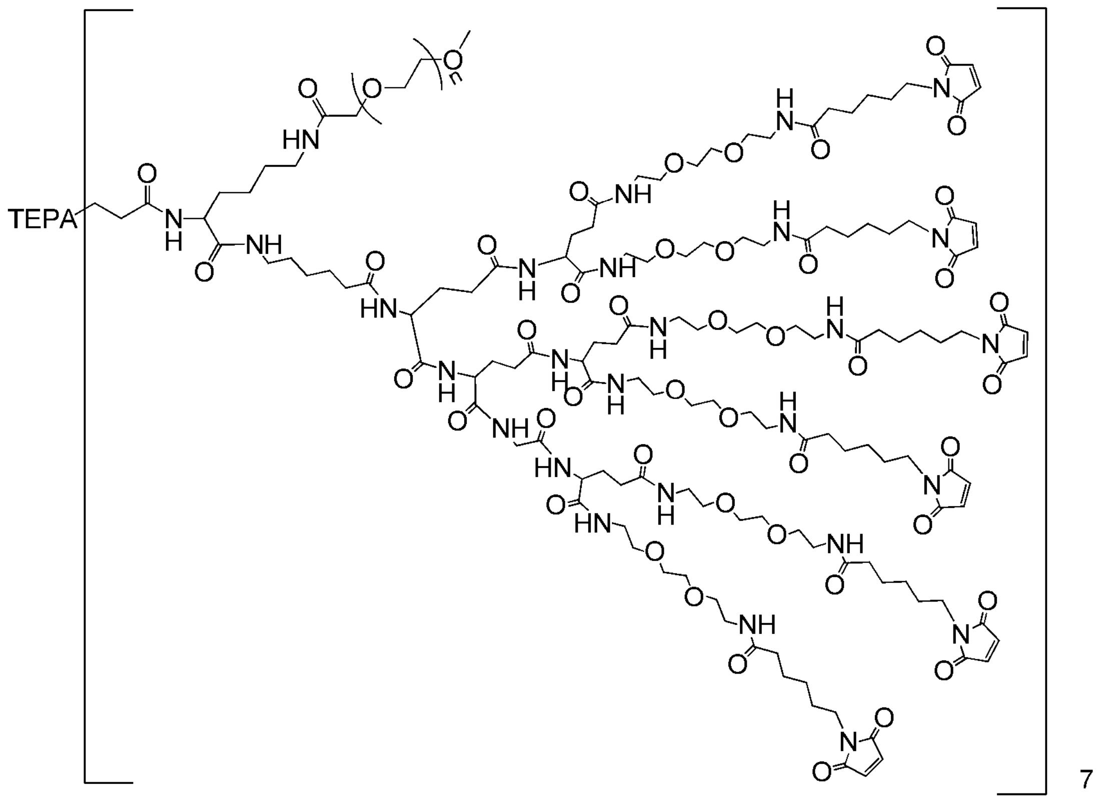

**67-216** (0.4 g, 0.00812 mmol), 5-maleimidocaproic acid (0.108 g, 0.51178 mmol), HOBT (0.069 g, 0.51178 mmol), and HBTU (0.194 g, 0.51178 mmol) were mixed in a 100 mL flask, and DMF (10 mL) was added for dissolution. DIEA (0.25 mL, 1.53535 mmol) was slowly added dropwise at -5°C. After the dropwise addition, the reaction was carried out for 30 minutes, the flask was then taken out, and the reaction was carried out with stirring at room temperature. The reaction progress was monitored by TLC. After the reaction was completed, n-hexane (25 mL) and methyl tert-butyl ether (200 mL) were added for precipitation. The supernatant was discarded, and n-hexane (20 mL) and methyl tert-butyl ether (100 mL) were added again for precipitation. This process was repeated three times. The mixture was filtered to provide a solid product, which was washed with methyl tert-butyl ether (100 mL × 3). The resulting solid product was collected and dried to provide 0.2 g of the product. $^1$H-NMR (600 MHz, DMSO-$d_6$) δ 8.27-8.13 (m, 147H), 7.01-6.96 (m, 84H), 4.19-4.10 (m, 42H), 4.08-4.06 (m, 14H), 3.88-3.78 (m, 77H), 3.62-3.54 (m, 350H), 3.509-3.497 (m, 3269H), 3.27-3.23 (m, 84H), 3.145-3.123 (m, 105H), 2.918-2.90 (m, 72H), 2.61-2.58 (m, 14H), 2.397-2.372 (m, 16H), 2.075-2.014 (m, 180H), 1.55-1.49 (m, 112H), 1.25-1.22 (m, 224H)

15.2 Preparation of **67-237**

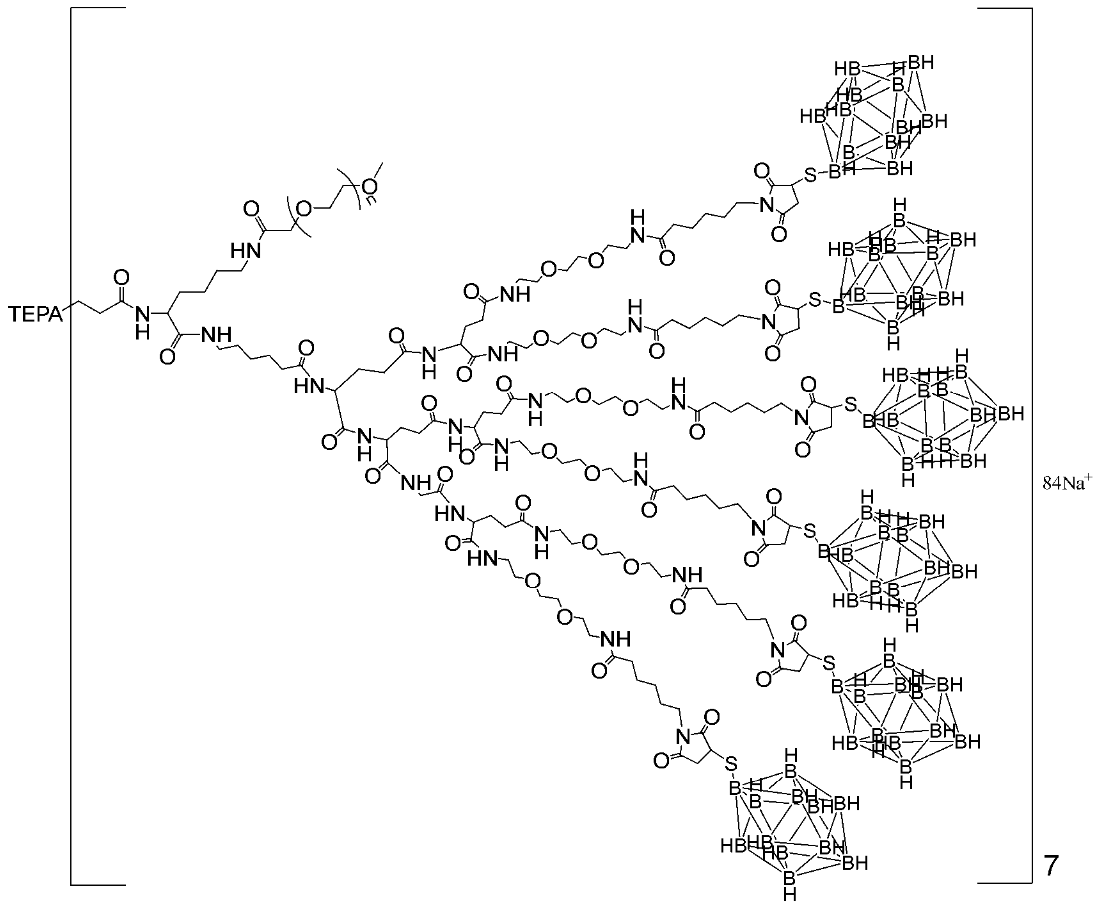

Sodium mercaptododecaborate (0.032 g, 0.147 mmol, abbreviated as N-BSH) and **67-236** (0.2 g, 0.0035 mmol) were weighed and mixed in a 100 mL flask. A stirrer was added, and methanol (10 mL) was added to dissolve the mixture. The temperature was maintained at 30°C, and the reaction progress was monitored by TLC. After the reaction was completed, the mixture was diluted by adding dichloromethane, and then concentrated to provide an oily product. Methyl tert-butyl ether (30 mL) and n-hexane (20 mL) were added for precipitation, and the supernatant was discarded. This process was repeated five times. Finally, the resulting product was dried to provide 0.2 g of the product. $^1$H-NMR (600 MHz, DMSO-*d*$_6$) δ 8.03-7.88 (m, 147H), 4.110-4.073 (m, 133H), 3.70-3.55 (m, 392H), 3.520-3.495 (m, 3269H), 3.241-3.233 (m, 42H), 3.052-2.97 (m, 84H), 2.95-2.86 (m, 147H), 2.617-2.610 (m, 42H), 2.39-2.38 (m, 30H), 2.09-2.01 (m, 212H), 1.81-1.78 (m, 14H), 1.48-1.44 (m, 112H), 1.28-1.23 (m, 208H), 1.02-0.70 (m, 462H)

Example 16: Synthesis of Compound **78-139**

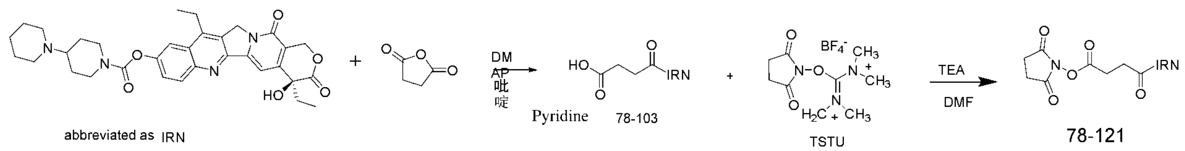

abbreviated as IRN

78-103

TSTU

78-121

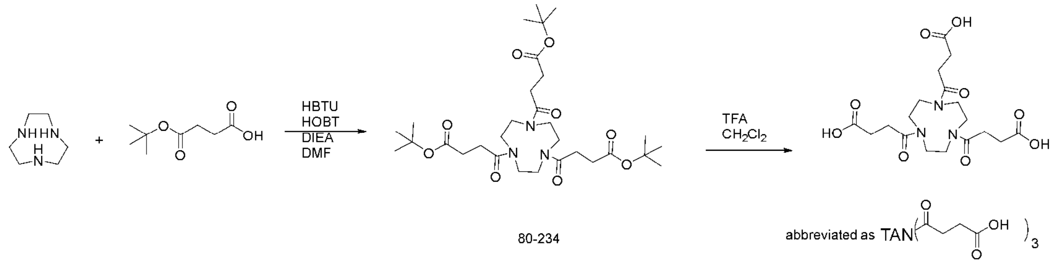

80-234

abbreviated as TAN

78-70

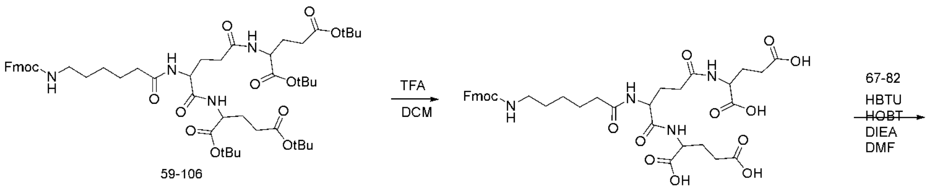

59-106

76-126

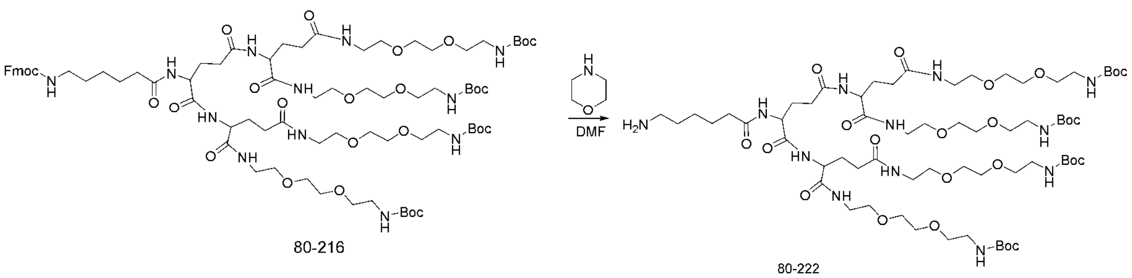

80-216

80-222

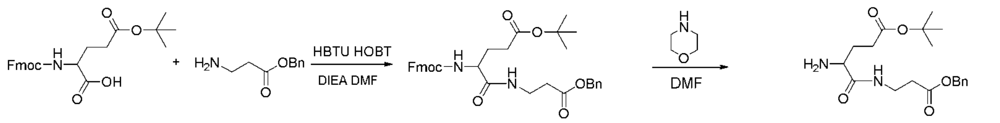

78-65

78-67

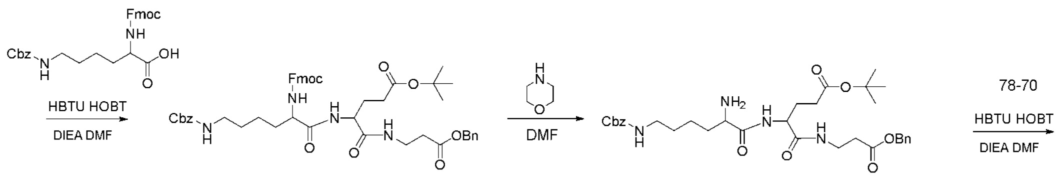

78-68

78-69

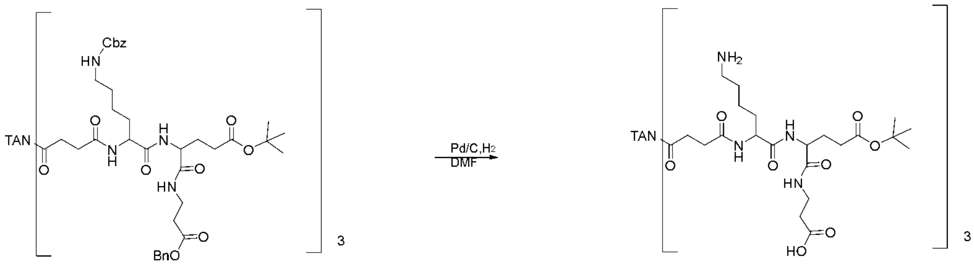

78-74

78-93

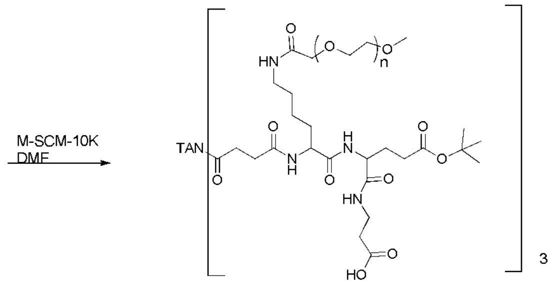


78-96

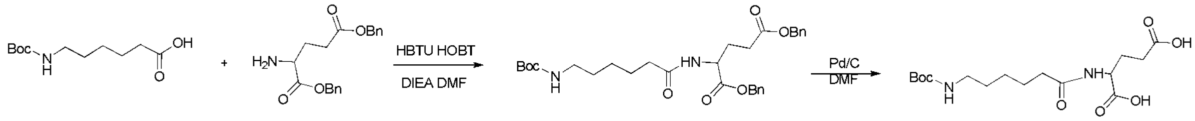


78-66

78-84

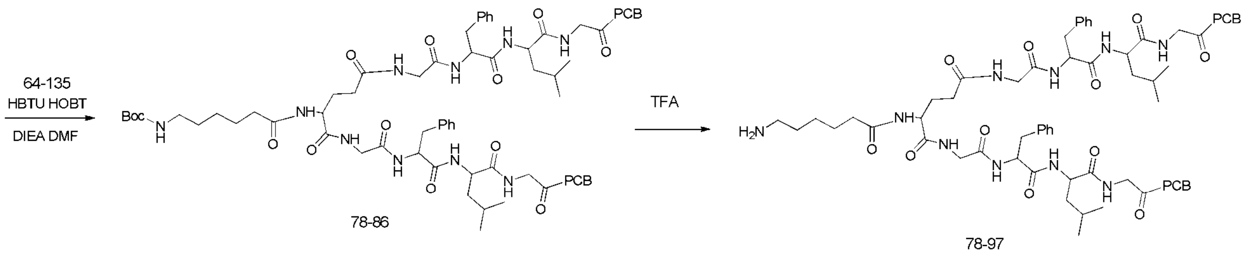


78-86

78-97

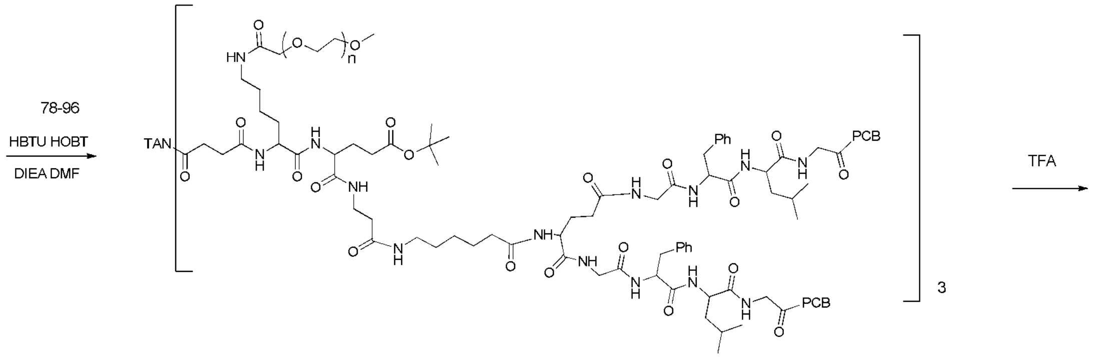


78-133

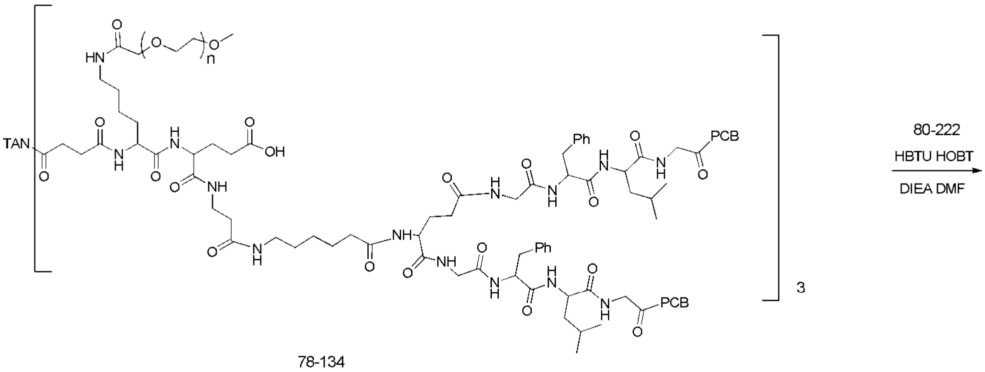


78-134

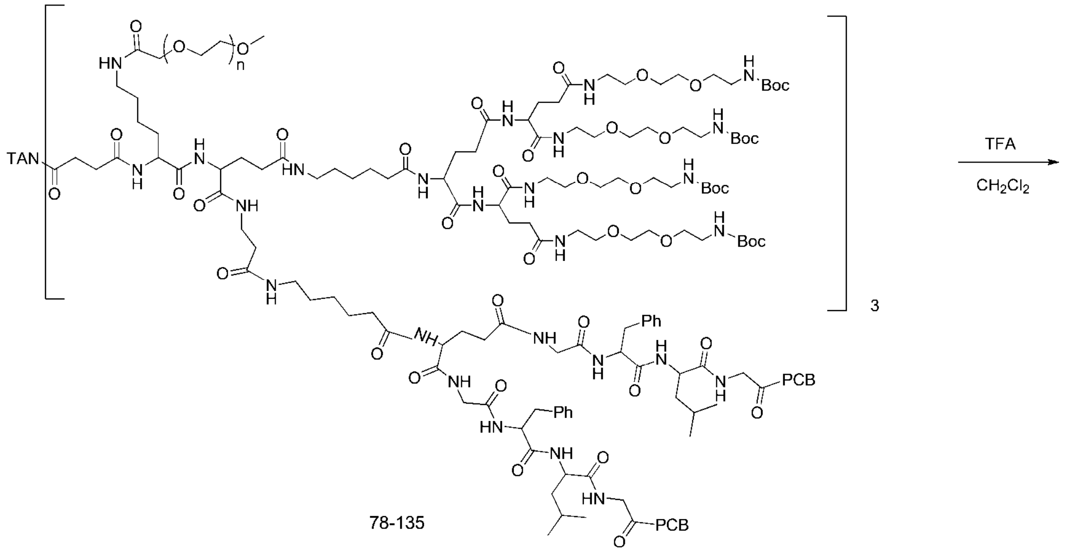


78-135

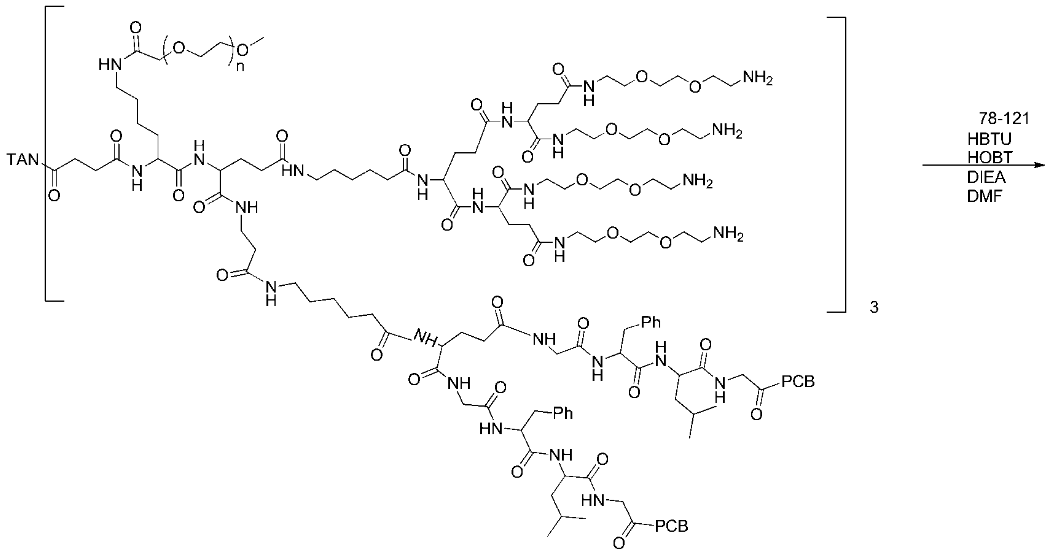


78-136

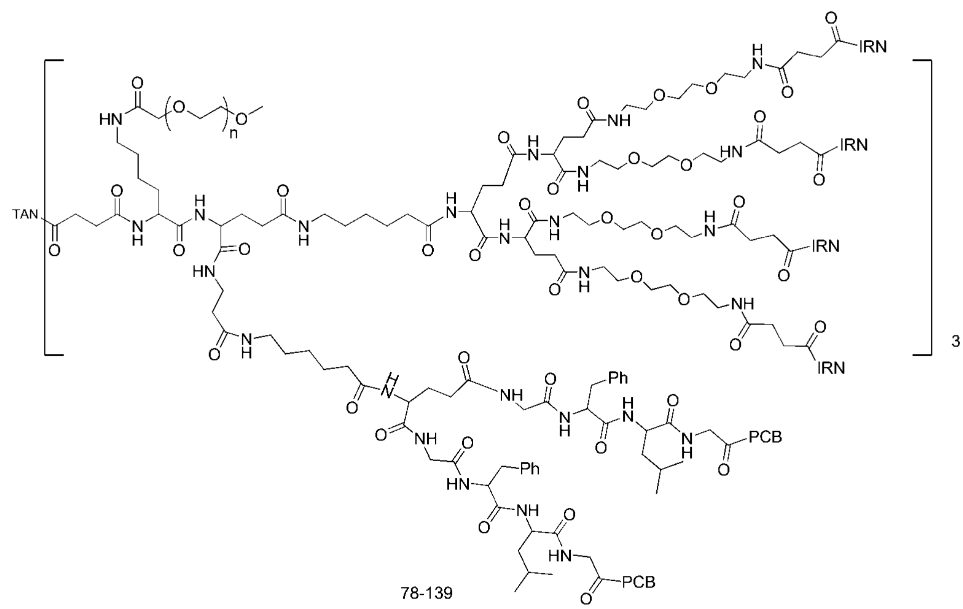

78-139

16.1 Preparation of **78-103**

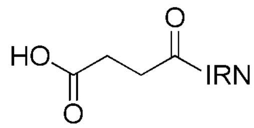

Irinotecan (3 g, 5.11 mmol) was weighed and added to a flask containing succinic anhydride (3.07 g, 30.66 mmol), then an appropriate amount of pyridine was added to dissolve the mixture. DMAP (1.25 mL, 10.22 mmol) was slowly added dropwise, and the reaction was stirred at room temperature for 4 hours. After the reaction was completed, n-hexane (25 mL) and methyl tert-butyl ether (200 mL) were added for precipitation. The supernatant was discarded, and n-hexane and methyl tert-butyl ether were added again for precipitation. This process was repeated three times. The mixture was filtered to provide a solid product. The obtained solid product was dissolved in a mixture of dichloromethane and methanol, then silica gel powder was added, and the mixture was evaporated to dryness. The resulting product was dry-loaded onto the column and subjected to column chromatography using a mixture of 1% formic acid/5-10% methanol/dichloromethane as the eluent. The desired product was collected, concentrated, and dried in a vacuum oven to provide 3.1 g of the product with a yield of 88%. $^1$H-NMR (600 MHz, DMSO-*d*$_6$) δ 8.24-8.14 (m, 1H), 8.02-7.91 (m, 1H), 7.69-7.62 (m, 1H), 7.14-7.06 (m, 1H), 5.54-5.45 (m, 2H), 5.38-5.32 (m, 2H), 4.28 (s, 1H), 4.15-4.04 (m, 1H), 3.21-3.16 (m, 2H), 3.12-3.04 (m, 1H), 2.89 (s, 1H), 2.83-2.69 (m, 2H), 2.52-2.39 (m, 6H), 2.15 (s, 2H), 1.81 (s, 2H), 1.56-1.44 (m, 6H), 1.39 (s, 2H), 1.28 (s, 3H), 0.90 (s, 3H); ESI [M+H$^+$] 687.3

16.2 Preparation of **80-234**

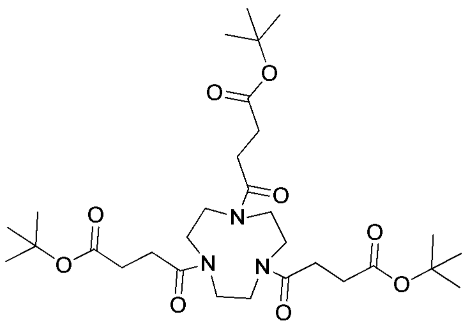

1,4,7,-Triazacyclononane (0.2 g, 1.55 mmol), tert-butyl succinate (0.89 g, 5.1 mmol), HBTU (1.9 g, 5.1 mmol), and HOBT (0.7 g, 5.1 mmol) were placed in a 500 mL flask, then DMF (15 mL) was added to dissolve the mixture, and the mixture was stirred at -5°C for approximately 20 minutes. DIEA (1.5 mL, 9.3 mmol) was then slowly added dropwise, and the reaction was continued at -5°C for 1 hour. The reaction mixture was then brought to room temperature and stirred overnight. After the reaction was completed, the reaction mixture was transferred to a 1 L separatory funnel, and saturated brine (200 mL) and ethyl acetate (200 mL) were added for extraction. The organic phase was collected, and the aqueous phase was further extracted with ethyl acetate (200 mL × 2). The organic phases were combined, washed with saturated brine (200 mL × 2), and evaporated to dryness to provide 3.1 g of the product. $^1$H-NMR (600 MHz, DMSO-*d*$_6$) δ 3.61 (s, 6H), 3.34 (s, 6H), 2.51-2.44 (m, 6H), 2.40 (s, 6H), 1.38 (s, 27H); ESI [M+Na$^+$] 620.34

16.3 Preparation of **78-70**

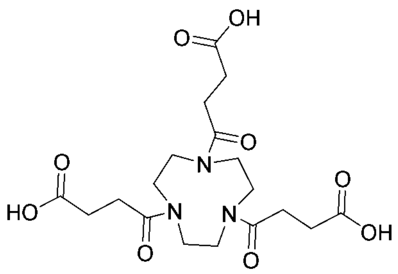

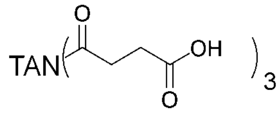

To a flask containing **80-234** (0.7 g, 1.17 mmol), dichloromethane (10 mL) was added for dissolution with the assistance of ultrasonication, then TFA (5.2 mL, 70.3 mmol) was added, and the mixture was reacted with stirring at room temperature overnight. After the reaction was completed, the reaction mixture was evaporated to an oily state using a rotary evaporator. Dichloromethane (30 mL) was then added. The reaction was treated by ultrasonication, and concentrated. This process was repeated five times. Then, methyl tert-butyl ether was added to the mixture. The mixture was treated by ultrasonication, and allowed to stand for 20 minutes. The supernatant was then discarded. This process was repeated three times. The resulting solid was evaporated to dryness and dried in a vacuum oven to provide 0.5 g of the product. $^1$H-NMR (600 MHz, DMSO-$d_6$) δ 3.61-3.54 (m, 6H), 3.38-3.26 (m, 6H), 2.50-2.44 (m, 6H), 2.45-2.39 (m, 6H); ESI [M+Na$^+$] 452

16.4 Preparation of **76-126**

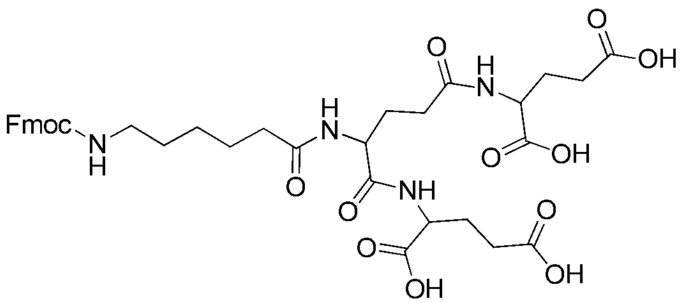

To a flask containing **59-106** (3 g, 3.1082 mmol), dichloromethane (20 mL) was added for dissolution with the assistance of ultrasonication, then TFA (15 mL) was added, and the mixture was reacted with stirring at room temperature overnight. After the reaction was completed, the reaction mixture was concentrated under reduced pressure to remove dichloromethane and most of the TFA. Methyl tert-butyl ether (500 mL) and n-hexane (100 mL) were then added for precipitation. The mixture was then filtered using a sand-core funnel and rinsed with methyl tert-butyl ether (300 mL × 3). The filter cake was collected and dried in a vacuum oven to provide the product.

16.5 Preparation of **80-216**

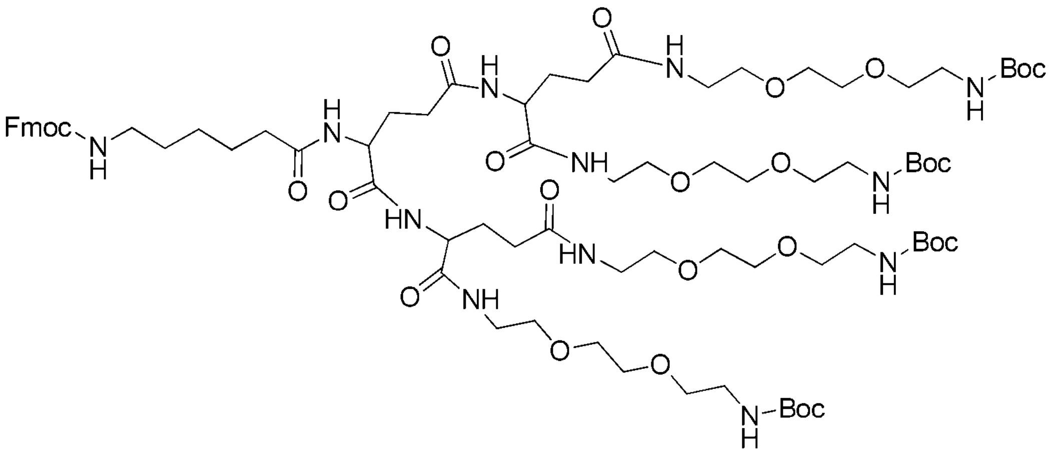

**67-82** (3.40 g, 13.6761 mmol), HBTU (5.18 g, 13.6761 mmol), and HOBT (1.85 g, 13.6761 mmol) were weighed and added to a flask containing **76-126** (2.30 g, 3.1082 mmol), then DMF (80 mL) was added to dissolve the mixture, and the mixture was allowed to stand at -5°C. DIEA (0.30 mL, 1.8040 mmol) was slowly added dropwise. After the dropwise addition was completed, the reaction was carried out for half an hour, the flask was then taken out, and the reaction was carried out with stirring at room temperature overnight. After the reaction was completed, n-hexane (25 mL) and methyl tert-butyl ether (200 mL) were added for precipitation. The supernatant was discarded, and n-hexane and methyl tert-butyl ether were added again for precipitation. This process was repeated three times. The mixture was filtered to provide a solid product, which was then dissolved in a methanol/dichloromethane (1/4) mixture, then silica gel powder (100 mL) was added, and the mixture was evaporated to dryness to provide a powdery solid. The powdery solid was dry-loaded onto the column and subjected to column chromatography using a 3-8% methanol/dichloromethane mixture as the eluent. The desired product was collected, concentrated, and dried in a vacuum oven to provide 4.56 g of the product.

16.6 Preparation of **80-222**

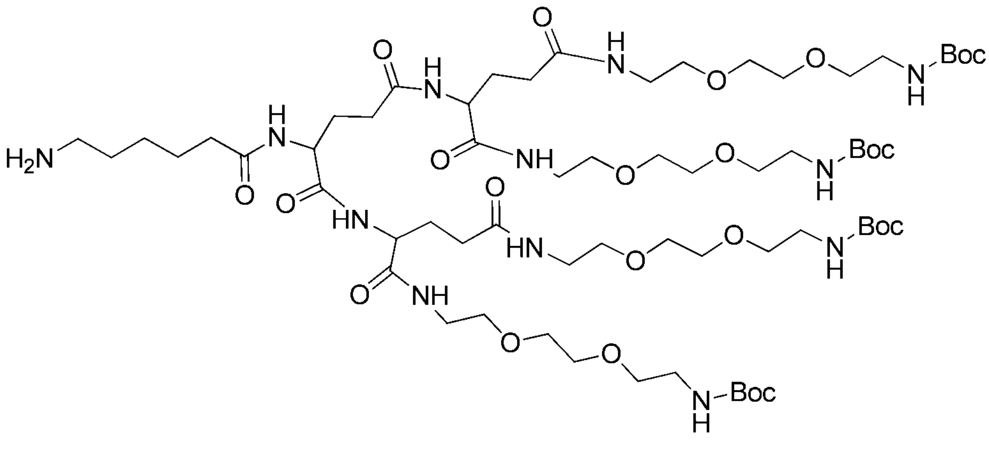

To a flask containing **80-216** (4.56 g, 2.7453 mmol), DMF (30 mL) was added for dissolution with the assistance of ultrasonication, then morpholine (2.39 mL, 27.4533 mmol) was added, and the mixture was reacted with stirring at room temperature for 2 hours. After the reaction was completed, n-hexane (25 mL) and methyl tert-butyl ether (200 mL) were added for precipitation. The supernatant was discarded, and n-hexane (40 mL) and methyl tert-butyl ether (70 mL) were added again for precipitation. This process was repeated three times. The mixture was filtered to provide a solid product, which was dried in a vacuum oven to provide 2.34 g of the product with a yield of 77%.

16.7 Preparation of **78-65**

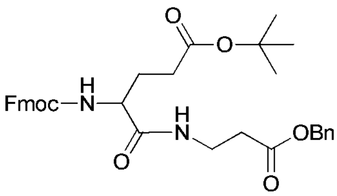

N-Fmoc-L-glutamic acid 5-tert-butyl ester (1.7 g, 3.9956 mmol), L-alanine benzyl ester p-toluenesulfonate (1.4 g, 3.9956 mmol), HBTU (1.66 g, 4.3958 mmol), HOBT (0.6 g, 4.3958 mmol), and DMF (20 mL) were placed in a 250 mL flask and dissolved with stirring. The reaction flask was then allowed to stand at -5°C, and the mixture was stirred for approximately 20 minutes. DIEA (1.3 mL, 7.9925 mmol) was then slowly added dropwise. After the dropwise addition was completed, the reaction was carried out with stirring at -5°C for 1 hour, and the mixture was then brought to room temperature and stirred. After the reaction was completed, the reaction mixture was transferred to a 1 L separatory funnel, and saturated brine (200 mL) and ethyl acetate (200 mL) were added for extraction. The organic phase was collected, and the aqueous phase was further extracted with ethyl acetate (200 mL × 3). The organic phases were combined, washed with saturated brine (200 mL × 2), concentrated, and dried to provide 2.35 g of the product with a yield exceeding 100%.

16.8 Preparation of **78-67**

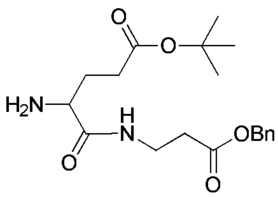

**78-65** (2.35 g, 3.9956 mmol) was dissolved in DMF (20 mL), then morpholine (5.2 mL, 59.9436 mmol) was added, and the mixture was reacted with stirring at room temperature overnight. The reaction progress was monitored by TLC. After the reaction was completed, the reaction mixture was transferred to a 1 L separatory funnel, and saturated brine (200 mL) and ethyl acetate (200 mL) were added for extraction. The organic phase was collected, and the aqueous phase was further extracted with ethyl acetate (200 mL × 3). The organic phases were combined, washed with saturated brine (200 mL × 2), concentrated, and dried to provide 1.46 g of the product with a yield exceeding 100%.

16.9 Preparation of **78-68**

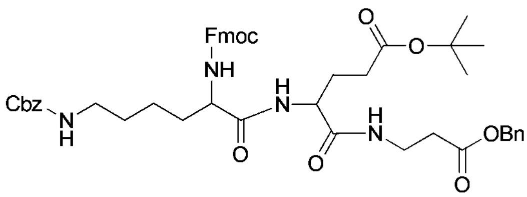

**78-67** (1.465 g, 3.9956 mmol), Fmoc-Lyz(Z)-OH (2.01 g, 3.9956 mmol), HBTU (1.67 g, 4.3958 mmol), and HOBT (0.59 g, 4.3958 mmol) were placed in a 250 mL flask, then DMF (40 mL) was added for dissolution, and the mixture was stirred at -5°C for 20 minutes. DIEA (1.3 mL, 7.9925 mmol) was then slowly added dropwise. After the dropwise addition was completed, the mixture was stirred for 1 hour, the flask was then taken out, and the reaction was carried out with stirring at room temperature overnight. After the reaction was completed, the reaction mixture was transferred to a 1 L separatory funnel containing saturated brine (200 mL) and ethyl acetate (200 mL) for extraction. The organic phase was collected, and the aqueous phase was further extracted with ethyl acetate (200 mL × 3). The organic phases were combined, washed with saturated brine (200 mL × 2), concentrated, and dried to provide 3.4 g of the product with a yield exceeding 100%.

16.10 Preparation of **78-69**

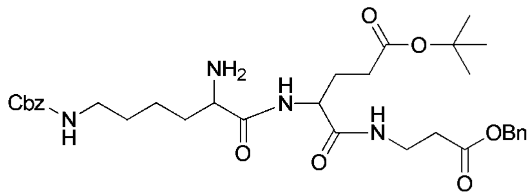

**78-68** (3.4 g, 3.9956 mmol) was dissolved in DMF (20 mL), then morpholine (5.2 mL, 59.9436 mmol) was added, and the mixture was reacted with stirring at room temperature overnight. The reaction progress was monitored by TLC. After the reaction was completed, the reaction mixture was transferred to a 1 L separatory funnel containing saturated brine (200 mL) and ethyl acetate (200 mL) for extraction. The organic phase was collected, and the aqueous phase was further extracted with ethyl acetate (200 mL × 3). The organic phases were combined, washed with saturated brine (200 mL × 2), concentrated, and dried to provide 1.98 g of the product with a yield of 78.9%. ESI [M+Na$^+$] 627

16.11 Preparation of **78-74**

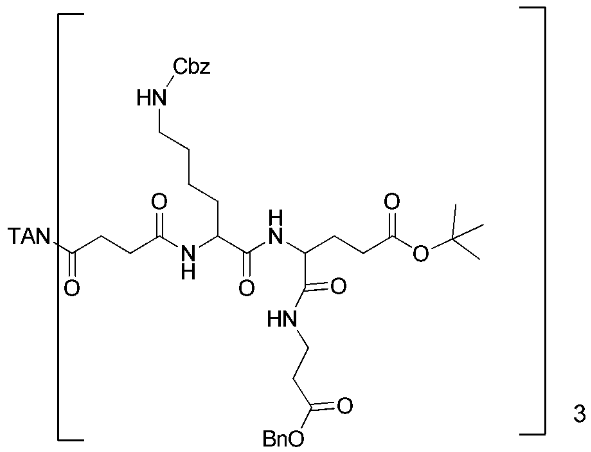

**78-70** (0.41 g, 0.96 mmol), **78-69** (1.98 g, 3.16 mmol), HBTU (1.3 g, 3.45 mmol), and HOBT (1.47 g, 3.45 mmol) were placed in a 250 mL flask, then DMF (40 mL) was added for dissolution, and the mixture was stirred at -5°C for 20 minutes. DIEA (1.4 mL, 8.61 mmol) was then slowly added dropwise. After the dropwise addition was completed, the reaction was carried out for 1 hour, the flask was then taken out, and the reaction was carried out with stirring at room temperature overnight. After the reaction was completed, the reaction mixture was transferred to a 1 L separatory funnel containing saturated brine (200 mL) and ethyl acetate (200 mL) for extraction. The organic phase was collected, and the aqueous phase was further extracted with ethyl acetate (200 mL × 3). The organic phases were combined and washed with saturated brine (200 mL × 2). The organic phases were concentrated, then 40 mL of silica gel powder was added, and the mixture was evaporated to dryness. The resulting product was dry-loaded onto the column and subjected to column chromatography using a 2-4% methanol/dichloromethane mixture as the eluent. The desired product was collected, concentrated, and dried to provide 1.55 g of the product with a yield of 71.6%. $^1$H-NMR (600 MHz, DMSO-$d_6$) δ 8.24-8.07 (m, 3H), 7.90-7.78 (m, 3H), 7.79-7.64 (m, 3H), 7.45-7.28 (m, 30H), 7.27-7.14 (m, 3H), 5.18-5.05 (m, 6H), 5.03-4.96 (m, 6H), 4.27-4.04 (m, 6H), 3.67-3.37 (m, 8H), 3.33-3.07 (m, 16H), 2.99-2.91 (m, 6H), 2.47-2.32 (m, 12H), 2.28-2.02 (m, 9H), 1.96-1.87 (m, 3H), 1.76-1.63 (m, 6H), 1.58-1.39 (m, 6H), 1.37-1.34 (m, 27H), 1.31-1.25 (m, 6H); ESI [M/2+Na$^+$] 1150, [M+Na$^+$] 2278

16.12 Preparation of **78-93**

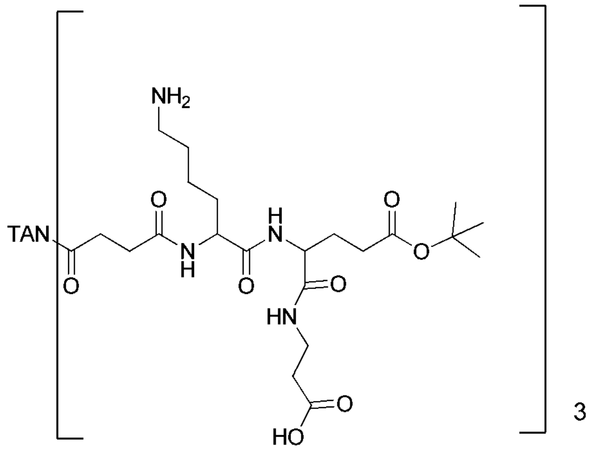

**78-74** (0.5 g, 0.222 mmol) was added to a hydrogenation reactor, and then 10% Pd/C catalyst (0.17 g) and methanol (15 mL) were added. The reactor was evacuated with a water pump and then filled with hydrogen gas. This process was repeated three times. The pressure reading of the hydrogenation reactor was adjusted to 1.8 MPa, and the mixture was stirred at room temperature overnight. After the reaction was completed, the reaction mixture was filtered through a suction funnel and rinsed with methanol (15 mL × 3). The filtrate was then transferred to a 250 mL round-bottom flask and evaporated to dryness. Dichloromethane (10 mL) and toluene (2 mL) were added, mixed with the assistance of ultrasonication, and then evaporated to dryness. This process was repeated five times. The resulting solid was dried in a vacuum oven to provide 0.35 g of the product with a yield of 99.7%. $^1$H-NMR (600 MHz, DMSO-$d_6$) δ 8.40-8.22 (m, 3H), 8.18-8.03 (m, 3H), 7.78-7.59 (m, 3H), 4.31-4.13 (m, 6H), 3.20-3.05 (m, 12H), 2.85-2.69 (m, 6H), 2.46-2.29 (m, 12H), 2.26-2.07 (m, 12H), 2.01-1.87 (m, 6H), 1.79-1.68 (m, 6H), 1.67-1.54 (m, 6H), 1.50-1.44 (m, 6H), 1.38-1.36 (m, 27H), 1.34-1.29 (m, 6H); ESI [M+H$^+$] 1582.89

16.13 Preparation of **78-96**

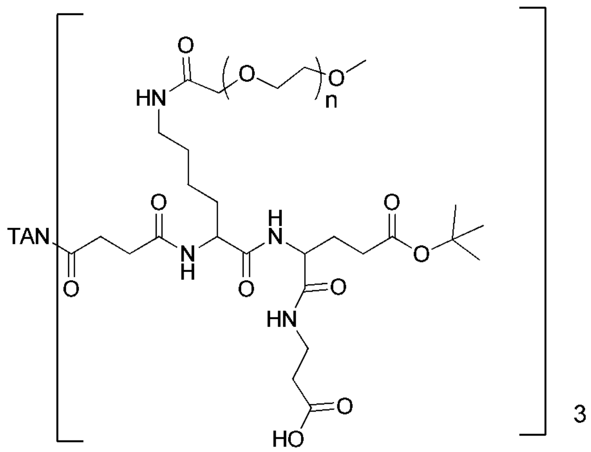

**78-93** (0.15 g, 0.095 mmol) was placed in a 250 mL flask. Then dry DMF (20 mL) was added for dissolution, followed by a slowly dropwise addition of DIEA (0.3, 1.3 mmol) at -5°C. After the dropwise addition was completed, the reaction was carried out for 10 minutes, the flask was then taken out, M-SCM-10K (3.23 g, 0.313 mmol) was added, and the reaction was stirred at low speed in the dark at room temperature for one week. After the reaction was completed, methyl tert-butyl ether (200 mL) and n-hexane (70 mL) were added for precipitation. The supernatant was discarded, and n-hexane (40 mL) and methyl tert-butyl ether (70 mL) were added again for precipitation. This process was repeated three times. The solid precipitated and was filtered. The filter cake was washed with methyl tert-butyl ether (40 mL × 3). The filter cake was collected and dried in a vacuum oven to provide 3.27 g of the product with a yield exceeding 100%.

16.14 Preparation of **78-66**

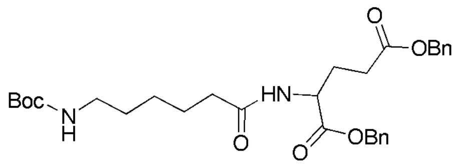

**62-13** (1.0 g, 4.3 mmol), L-glutamic acid dibenzyl ester p-toluenesulfonate (2.1 g, 4.3 mmol), HBTU (1.8 g, 4.8 mmol), and HOBT (0.65 g, 4.8 mmol) were placed in a 250 mL flask, then DMF (40 mL) was added for dissolution, and the mixture was stirred at -5°C for 20 minutes. DIEA (1.4 mL, 8.6 mmol) was then slowly added dropwise. After the dropwise addition was completed, the reaction was carried out for 1 hour, the flask was then taken out, and the reaction was carried out with stirring at room temperature overnight. After the reaction was completed, the reaction mixture was transferred to a 1 L separatory funnel containing saturated brine (200 mL) and ethyl acetate (200 mL) for extraction. The organic phase was collected, and the aqueous phase was further extracted with ethyl acetate (200 mL × 3). The organic phases were combined, washed with saturated brine (200 mL × 2). The organic phases were concentrated, then 40 mL of silica gel powder was added, and the mixture was evaporated to dryness. The resulting product was dry-loaded onto the column and subjected to column chromatography using a 50% ethyl acetate/petroleum ether mixture as the eluent. The desired product was collected, concentrated, and dried to provide 2.2 g of the product. ESI [M+H$^+$] 541

16.15 Preparation of **78-84**

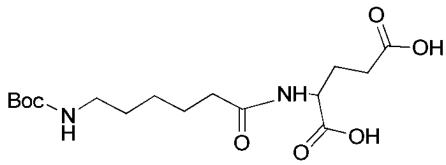

**78-66** (0.5 g, 0.925 mmol) was added to a hydrogenation reactor, and 10% Pd/C catalyst (0.08 g) and DMF (10 mL) were added. The reactor was evacuated with a water pump and then filled with hydrogen gas. This process was repeated three times. The pressure reading of the hydrogenation reactor was adjusted to 1.8 MPa, and the mixture was stirred at room temperature overnight. After the reaction was completed, the reaction mixture was filtered through Celite, and the filter cake was washed with DMF (15 mL × 3). The filtrate was then transferred to a 250 mL round-bottom flask and used as the raw material for the next step.

16.16 Preparation of **78-86**

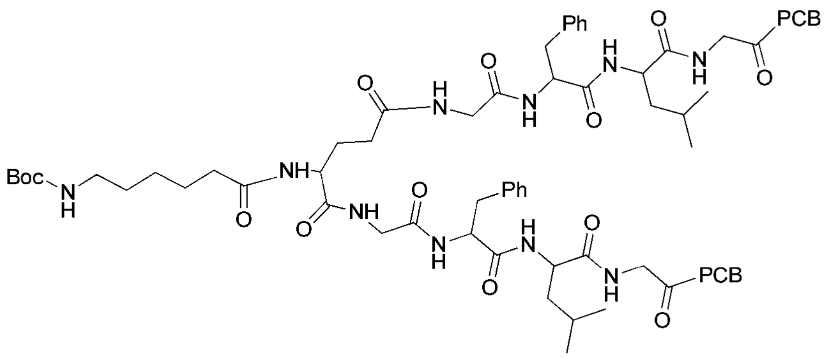

**78-84** (0.33 g, 0.925 mmol), **64-135** (1.6 g, 1.94 mmol), HBTU (0.77 g, 2.04 mmol), and HOBT (0.28 g, 2.04 mmol) were placed in a 500 mL flask, then DMF (20 mL) was added for dissolution. DIEA (0.6 mL, 3.7 mmol) was then slowly

added dropwise at -5°C. After the dropwise addition was completed, the reaction was carried out for 30 minutes, the flask was then taken out, and the reaction was carried out with stirring at room temperature overnight. After the reaction was completed, n-hexane (25 mL) and methyl tert-butyl ether (200 mL) were added for precipitation. The supernatant was discarded, and n-hexane (40 mL) and methyl tert-butyl ether (70 mL) were added again for precipitation. This process was repeated three times. The mixture was filtered to provide a solid product, which was dried in a vacuum oven to provide 1.7 g of the product.

16.17 Preparation of **78-97**

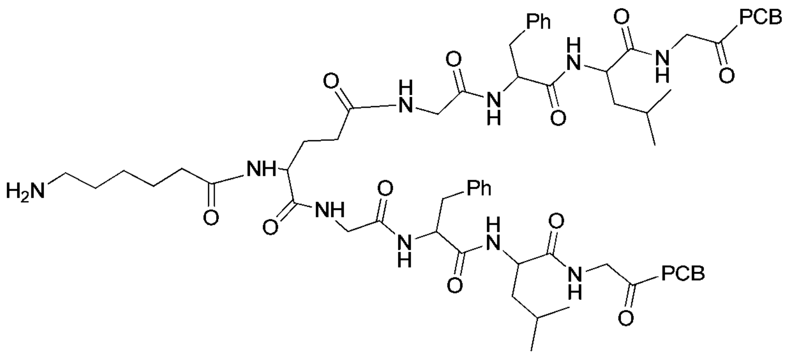

To a flask containing **78-86** (1.7 g, 0.86 mmol), dichloromethane (20 mL) was added for dissolution with the assistance of ultrasonication, then TFA (1 mL, 12.9 mmol) was added, and the mixture was reacted with stirring at room temperature overnight. After the reaction was completed, the reaction mixture was evaporated to an oily state using a rotary evaporator. Dichloromethane (30 mL) was added, treated by ultrasonication, and concentrated. This process was repeated five times. Methyl tert-butyl ether (100 mL) was then added for precipitation and treated by ultrasonication. The supernatant was discarded, and methyl tert-butyl ether was added for precipitation. This process was repeated three times. The mixture was filtered to provide a solid product, which was dried to provide 1.5 g of the product with a yield of 92.9%. $^1$H-NMR (600 MHz, DMSO-$d_6$) δ 9.01-8.92 (m, 2H), 8.22-8.16 (m, 2H), 8.13-8.06 (m, 4H), 8.05-8.00 (m, 2H), 7.93-7.85 (m, 4H), 7.64-7.50 (m, 5H), 7.32-7.13 (m, 10H), 5.86-5.79 (m, 2H), 4.57-4.51 (m, 2H), 4.41-4.32 (m, 2H), 4.05-3.99 (m, 3H), 3.64-3.58 (m, 8H), 3.22-3.18 (m, 4H), 3.15-3.12 (m, 4H), 2.98-2.95 (m, 2H), 2.92-2.87 (m, 4H), 2.80-2.71 (m, 6H), 2.45-2.39 (m, 8H), 2.34-2.30 (m, 6H), 2.28-2.19 (m, 6H), 2.15-2.08 (m, 4H), 1.92-1.87 (m, 4H), 1.80-1.75 (m, 4H), 1.63-1.56 (m, 6H), 1.53-1.46 (m, 8H), 1.27-1.24 (m, 2H), 1.13-1.09 (m, 4H), 0.93-0.78 (m, 12H); ESI [M+H$^+$] 1867.98

16.18 Preparation of **78-133**

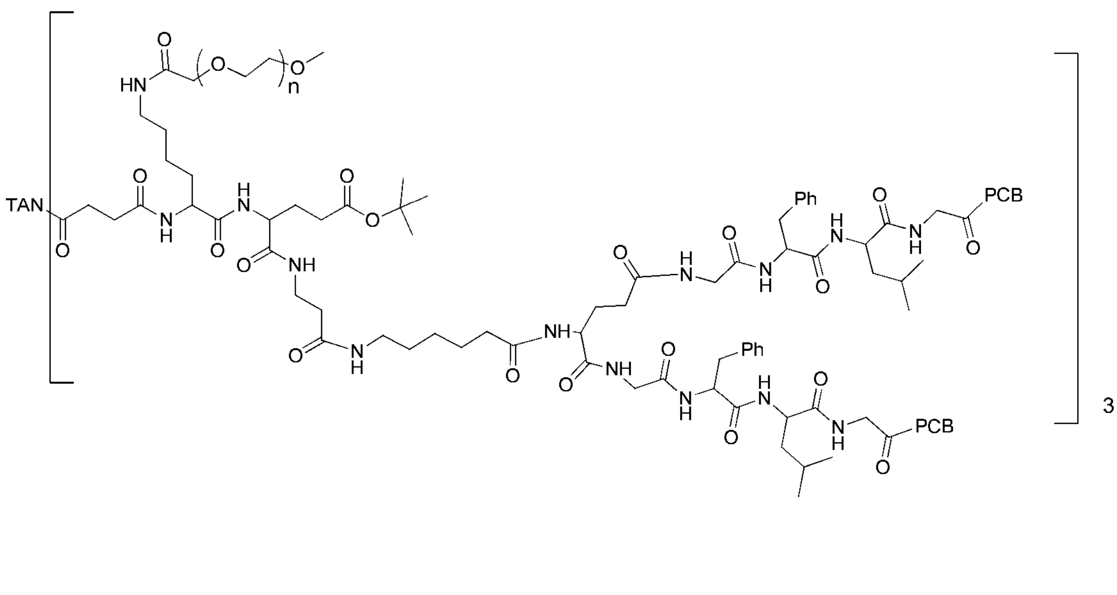

**78-97** (0.4 g, 0.21 mmol), **78-96** (2.1 g, 0.0634 mmol), HBTU (0.08 g, 0.0.21 mmol), and HOBT (0.03 g, 0.21 mmol) were placed in a 500 mL flask, and dissolved in DMF (30 mL). DIEA (0.15 mL, 0.86 mmol) was then slowly added dropwise at -5°C. After the dropwise addition was completed, the reaction was carried out for 30 minutes, the flask was taken out, and the reaction was carried out with stirring at room temperature overnight. After the reaction was completed, n-hexane (25 mL) and methyl tert-butyl ether (200 mL) were added for precipitation. The supernatant was discarded, and n-hexane (40 mL) and methyl tert-butyl ether (70 mL) were added again for precipitation. This process was repeated three times. The mixture was filtered to provide a solid product, which was dissolved in a methanol/dichloromethane (1/4) mixture, then silica gel powder was added, and the mixture was evaporated to dryness. The resulting product was dry-loaded onto the column and subjected to column chromatography using a 5-7% methanol/dichloromethane mixture as the eluent. The desired product was collected, concentrated, and dried in a vacuum oven to provide 2.29 g of the product with a yield of 93.5%.

16.19 Preparation of **78-134**

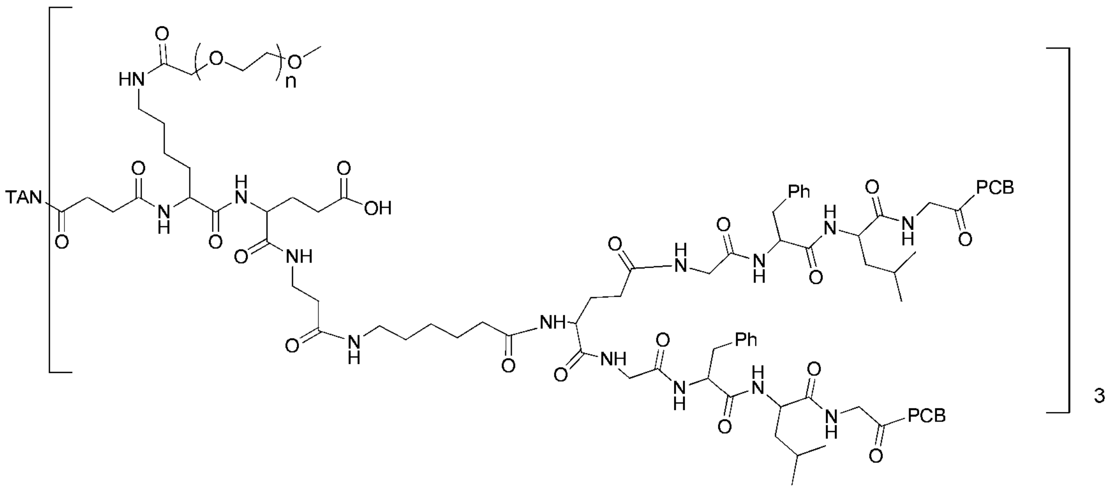

To a flask containing 78-133 (2.29 g, 0.0592 mmol), dichloromethane (20 mL) was added for dissolution with the assistance of ultrasonication, then TFA (0.3 mL, 3.55 mmol) was added, and the mixture was reacted with stirring at room temperature overnight. After the reaction was completed, the reaction mixture was evaporated to an oily state using a rotary evaporator. Dichloromethane (30 mL) was added, treated by ultrasonication, and concentrated. This process was repeated five times. Methyl tert-butyl ether (100 mL) was then added for precipitation. The filter cake was collected and dried in a vacuum oven to provide 2.18 g of the product with a yield of 95.6%.

16.20 Preparation of **78-135**

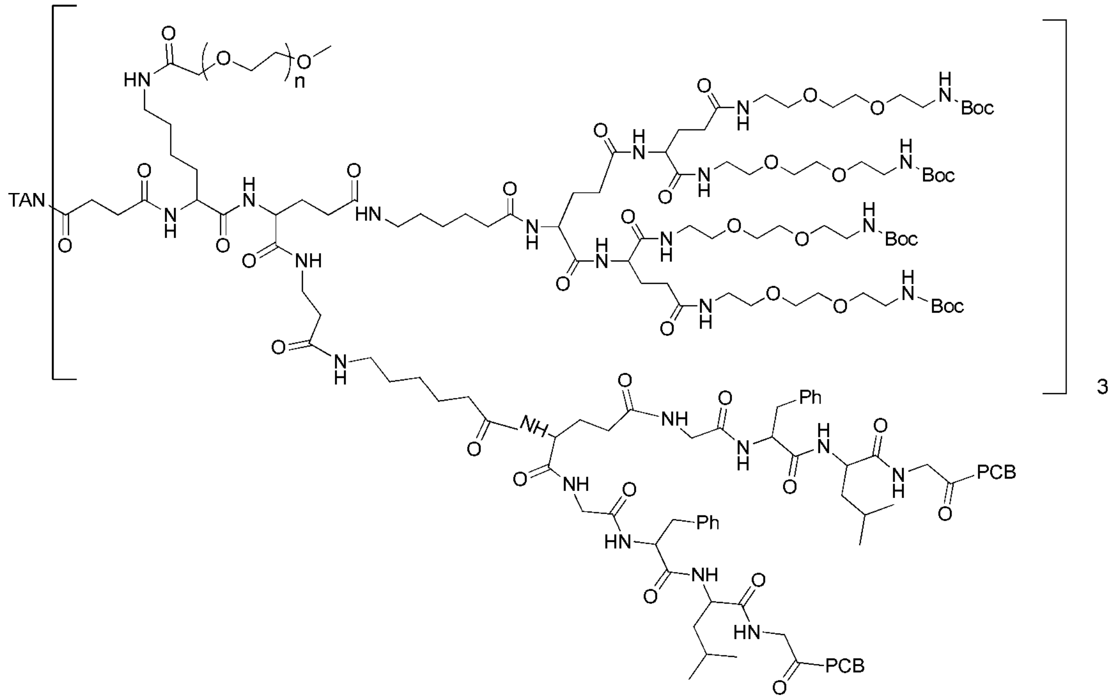

**78-134** (2.18 g, 0.057 mmol), **80-222** (0.33 g, 0.227 mmol), HBTU (0.09 g, 0.227 mmol), and HOBT (0.03 g, 0.227 mmol) were placed in a 500 mL flask, and DMF (30 mL) was added for dissolution. DIEA (0.13 mL, 0.765 mmol) was then slowly added dropwise at -5°C. After the dropwise addition was completed, the reaction was carried out for 30 minutes, the flask was then taken out, and the reaction was carried out with stirring at room temperature overnight. After the reaction was completed, n-hexane (25 mL) and methyl tert-butyl ether (200 mL) were added for precipitation. The supernatant was discarded, and n-hexane (40 mL) and methyl tert-butyl ether (70 mL) were added again for precipitation. This process was repeated three times. The mixture was filtered to provide a solid product, which was dissolved in a methanol/dichloromethane (1/4) mixture, then silica gel powder was added, and the mixture was evaporated to dryness. The resulting product was dry-loaded onto the column and subjected to column chromatography using a 5-10% methanol/dichloromethane mixture as the eluent. The desired product was collected, concentrated, and dried to provide 1.25 g of the product with a yield of 51.3%.

16.21 Preparation of **78-136**

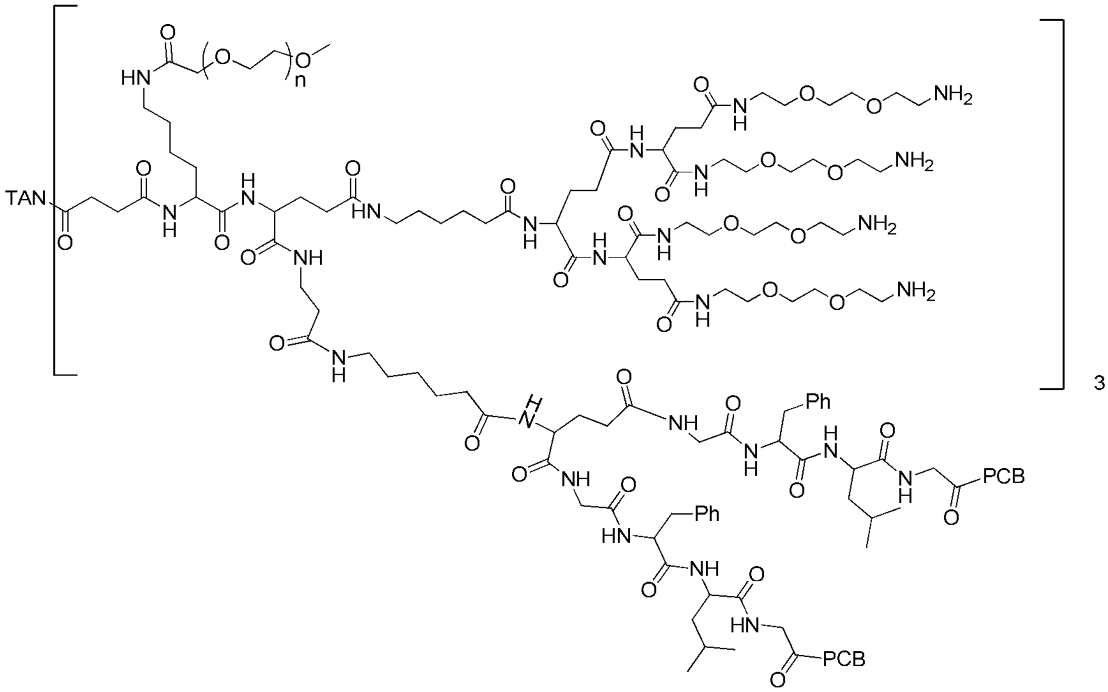

To a flask containing **78-135** (1.25 g, 0.029 mmol), dichloromethane (10 mL) was added for dissolution with the assistance of ultrasonication, then TFA (0.52 mL, 7.02 mmol) was added, and the mixture was reacted with stirring at room temperature overnight. After the reaction was completed, the reaction mixture was evaporated to an oily state using a rotary evaporator. Dichloromethane (20 mL) was added, treated by ultrasonication, and concentrated. This process was repeated five times. Methyl tert-butyl ether (200 mL) was then added for precipitation. The mixture was filtered, and the filter cake was collected and dried in a vacuum oven to provide 1.25 g of the product.

16.22 Preparation of **78-121**

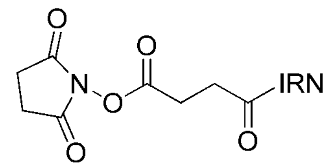

**78-103** (1.3 g, 1.94 mmol) and TSTU (0.65 g, 2.13 mmol) were placed in a 500 mL flask. DMF (15 mL) was added for dissolution, and then triethylamine (0.8 mL, 5.81 mmol) was added dropwise at room temperature. After the dropwise addition was completed, the reaction was carried out with stirring for 3 hours. After the reaction was completed, n-hexane (25 mL) and methyl tert-butyl ether (200 mL) were added for precipitation. The supernatant was discarded, and n-hexane (40 mL) and methyl tert-butyl ether (70 mL) were added again for precipitation. This process was repeated three times. The mixture was filtered to provide a solid product, which was dried to provide 1.3 g of the product with a yield of 85.5%.

16.23 Preparation of **78-139**

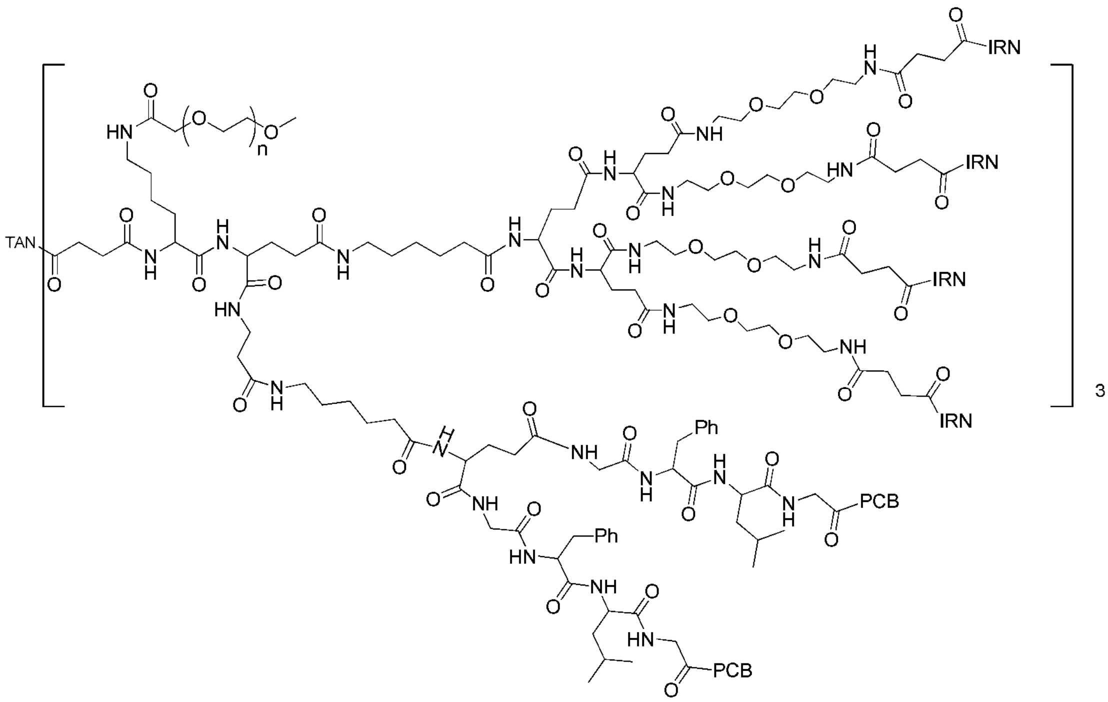

**78-136** (1.2 g, 0.024 mmol) and **78-121** (0.42 g, 0.432 mmol) were placed in a 500 mL flask. DMF (20 mL) was added for dissolution, and then DIEA (0.2 mL, 1.29 mmol) was slowly added dropwise at -5°C. After the dropwise addition was completed, the reaction was carried out for 30 minutes, the flask was then taken out, and the reaction was carried out with stirring at room temperature overnight. After the reaction was completed, n-hexane (25 mL) and methyl tert-butyl ether (200 mL) were added for precipitation. The supernatant was discarded, and n-hexane (40 mL) and methyl tert-butyl ether (70 mL) were added again for precipitation. This process was repeated three times. The mixture was filtered to provide a solid product, the filter cake was dissolved in a methanol/dichloromethane (1/4) mixture, then silica gel powder was added, and the mixture was evaporated to dryness. The resulting product was dry-loaded onto the column and subjected to column chromatography using a 5-12% methanol/dichloromethane mixture as the eluent. The desired product was collected, concentrated, and dried to provide 1.1 g of the product with a yield of 93.2%. $^1$H-NMR (600 MHz, DMSO-$d_6$) $\delta$ 10.20-10.06 (m, 6H), 9.00-8.84 (m, 6H), 8.27-8.15 (m, 20H), 8.15-8.09 (m, 20H), 8.09-8.01 (m, 27H), 8.01-7.96 (m, 23H), 7.94-7.88 (m, 23H), 7.85-7.84 (m, 6H), 7.74-7.65 (m, 12H), 7.62-7.55 (m, 12H), 7.52-7.43 (m, 6H), 7.27-7.18 (m, 28H), 7.17-7.13 (m, 6H), 7.11-7.04 (m, 18H), 5.84-5.76 (m, 6H), 5.50-5.46 (m, 24H), 5.30-5.21 (m, 24H), 4.58-4.52 (m, 6H), 4.41-4.33 (m, 18H), 4.21-4.13 (m, 30H), 4.04-3.96 (m, 12H), 3.64-3.59 (m, 48H), 3.51-3.50 (m, 2981H), 3.26-3.22 (m, 48H), 3.20-3.15 (m, 60H), 3.13-3.11 (m, 32H), 3.01-2.94 (m, 48H), 2.84-2.74 (m, 38H), 2.64-2.59 (m, 12H), 2.44-2.39 (m, 42H), 2.33-2.27 (m, 28H), 2.18-2.02 (m, 126H), 1.91-1.68 (m, 180H), 1.58-1.55 (m, 16H), 1.53-1.49 (m, 18H), 1.47-1.40 (m, 32H), 1.36-1.31 (m, 18H), 1.25-1.24 (m, 24H), 1.23-1.22 (m, 36H), 0.94-0.90 (m, 36H), 0.90-0.86 (m, 36H)

Example 17: Synthesis of Compound **82-178**

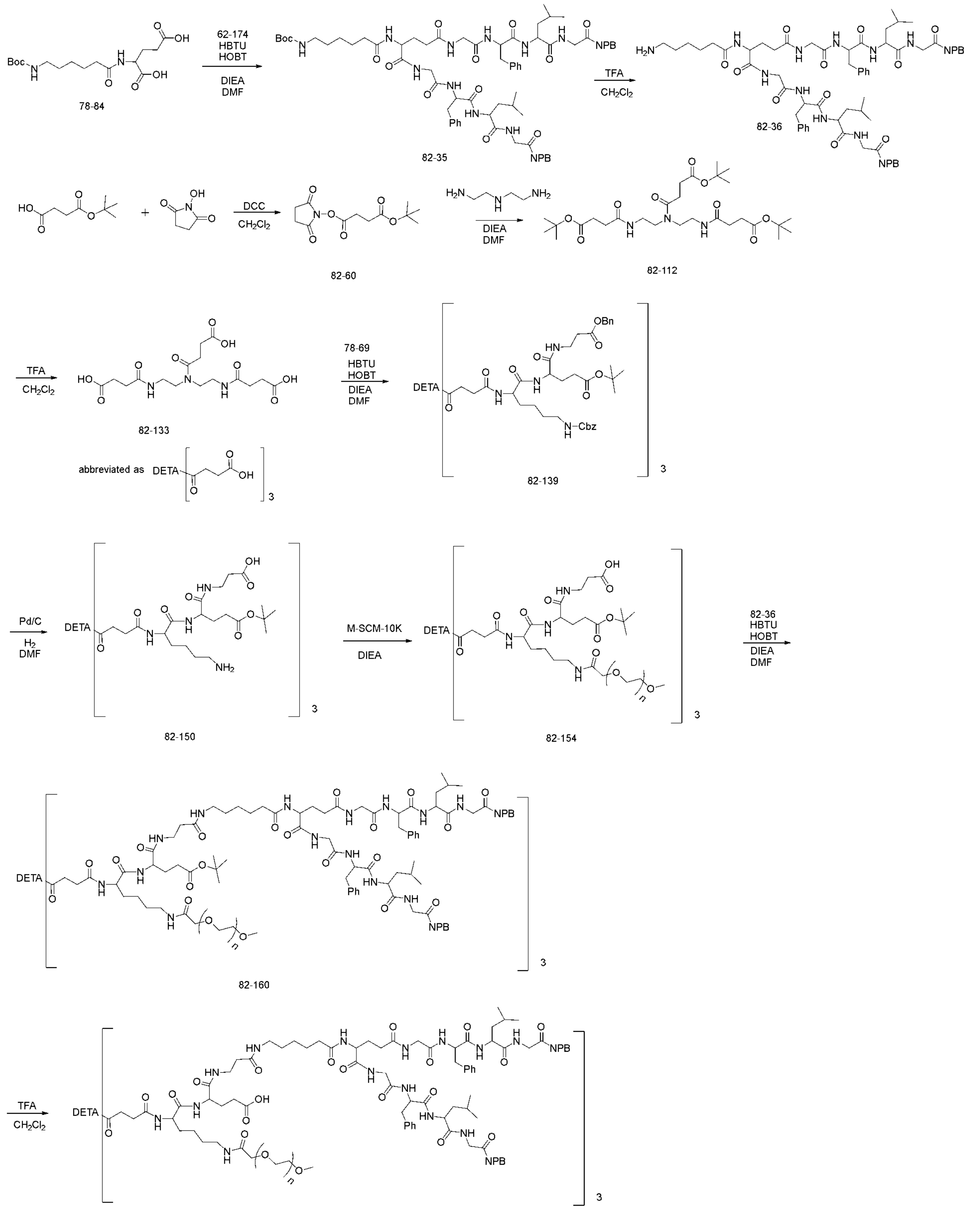
78-84
62-174
HBTU
HOBT
DIEA
DMF
82-35
TFA
$CH_2Cl_2$
82-36
DCC
$CH_2Cl_2$
82-60
DIEA
DMF
82-112
TFA
$CH_2Cl_2$
82-133
abbreviated as
78-69
HBTU
HOBT
DIEA
DMF
82-139
Pd/C
$H_2$
DMF
82-150
M-SCM-10K
DIEA
82-154
82-36
HBTU
HOBT
DIEA
DMF
82-160
TFA
$CH_2Cl_2$
82-162

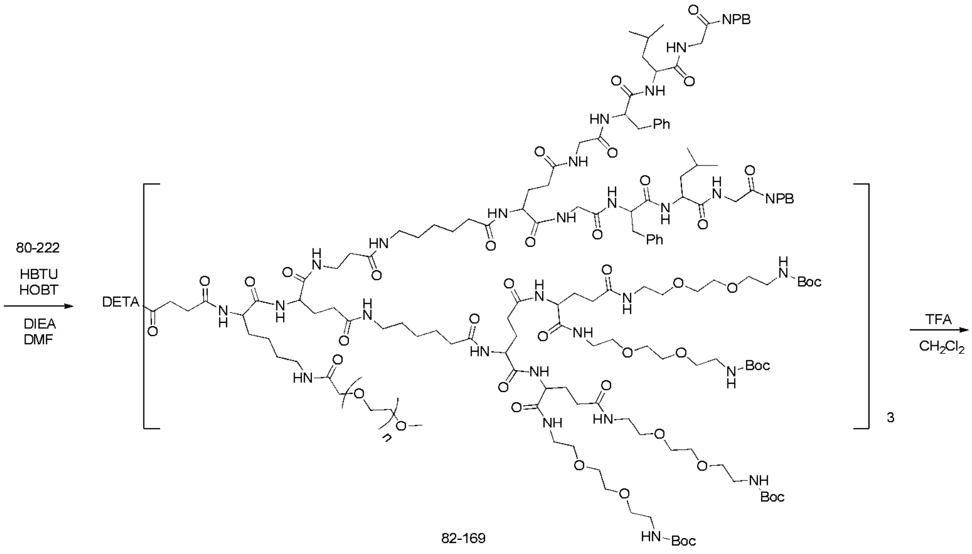


82-169

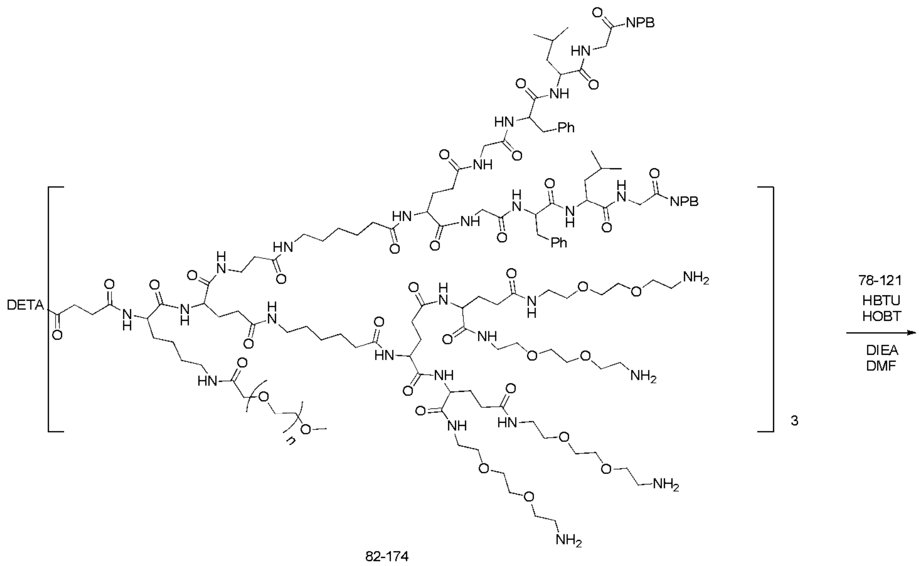


82-174

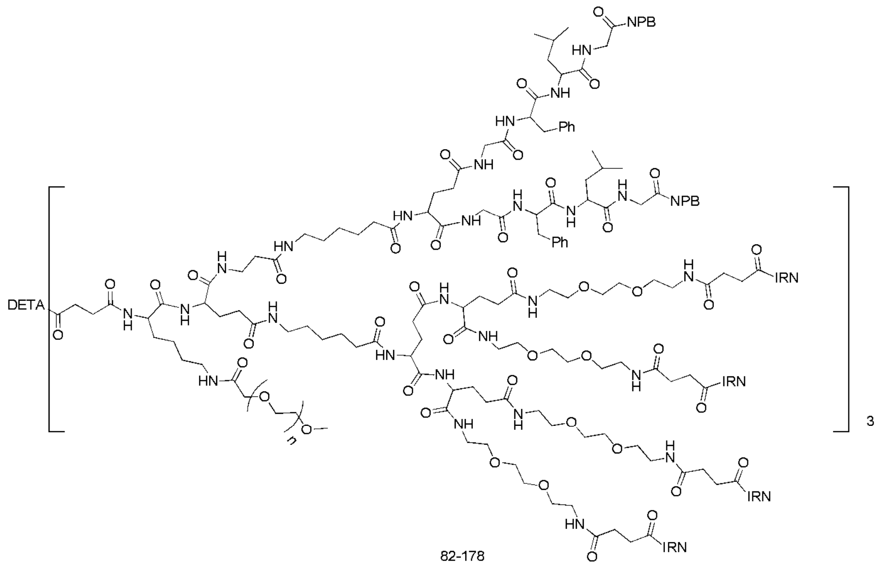

82-178

17.1 Preparation of 82-35

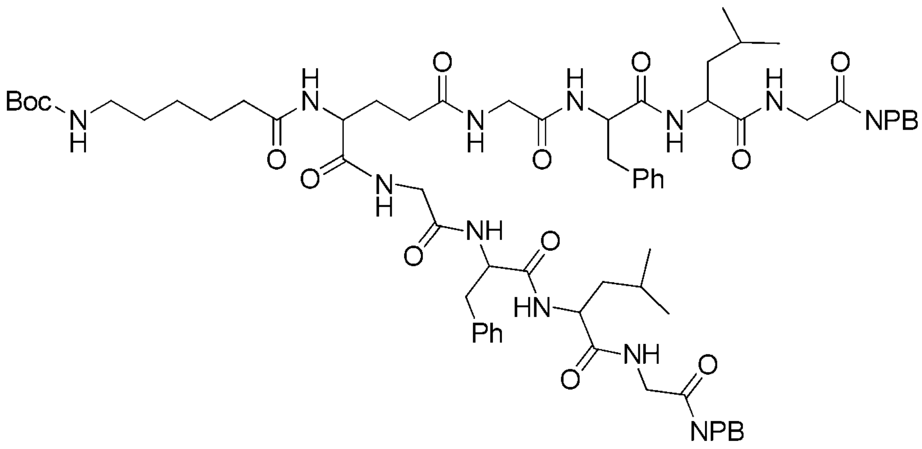

**62-174** (5 g, 10.6714 mmol), HBTU (6.07 g, 16.0072 mmol), and HOBT (2.16 g, 16.0072 mmol) were weighed and added to a flask containing **78-84** (1.88 g, 11.2050 mmol), then DMF (200 mL) was added to dissolve the mixture, and the mixture was allowed to stand at -5°C. DIEA (7.93 mL, 48.0215 mmol) was slowly added dropwise. After the dropwise addition was completed, the reaction was carried out for half an hour, the flask was then taken out, and the reaction was carried out with stirring at room temperature overnight. After the reaction was completed, saturated brine (200 mL) and ethyl acetate (200 mL) were added for extraction. After standing for phase separation, the organic phase was collected, and the aqueous phase was further extracted with ethyl acetate (200 mL × 3). The organic phases were combined, evaporated to dryness to provide a solid, which was then dissolved in a methanol/dichloromethane (1/4) mixture, then silica gel powder (100 mL) was added, and the mixture was evaporated to dryness to provide a powdery solid. The powdery solid was dry-loaded onto the column and subjected to column chromatography using a 2-4% methanol/dichloromethane mixture as the eluent. The desired product was collected, concentrated, and dried in a vacuum oven to provide 3.6 g of the product.

17.2 Preparation of **82-36**

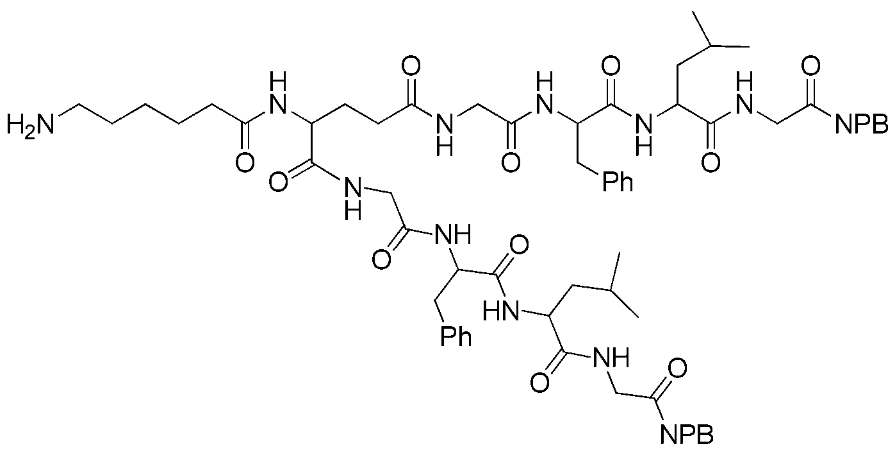

To a flask containing **82-35** (2.0068 mmol), dichloromethane (15 mL) was added for dissolution with the assistance of ultrasonication, then TFA (5 mL) was added, and the mixture was reacted with stirring at room temperature overnight. After the reaction was completed, the reaction mixture was evaporated to an oily state using a rotary evaporator. Methyl tert-butyl ether (60 mL) was then added. A powdery solid precipitated from the reaction mixture and was filtered. The filter cake was washed with methyl tert-butyl ether (40 mL × 3). The filter cake was collected and dried in a vacuum oven to provide 3.1 g of the product.

17.3 Preparation of **82-60**

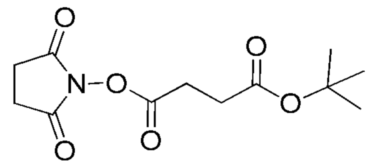

Mono-tert-butyl succinate (1.41 g, 3.3665 mmol) and N-hydroxysuccinimide (0.77 g, 6.7315 mmol) were weighed and added to a 500 mL flask containing dichloromethane (20 mL), dissolved with the assistance of ultrasonication, then DCC (1.39 g, 6.7315 mmol) was added, and the mixture was reacted with stirring at room temperature overnight. After the reaction was completed, the reaction mixture was concentrated under reduced pressure and evaporated to dryness to provide a solid, which was then dissolved in a methanol/dichloromethane (1/4) mixture, then silica gel powder (100 mL) was added, and the mixture was evaporated to dryness to provide a powdery solid. The powdery solid was dry-loaded onto the column and subjected to column chromatography using a 2-4% methanol/dichloromethane mixture as the eluent. The desired product was collected, concentrated, and dried in a vacuum oven to provide 1.6 g of the product.

17.4 Preparation of **82-112**

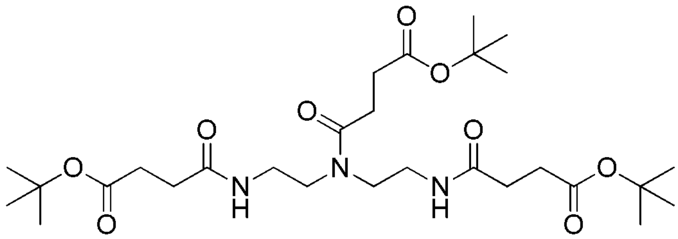

**82-60** (2 g, 7.3727 mmol) was weighed and added to a flask containing diethylenetriamine (0.23 g, 2.2341 mmol), then DMF (20 mL) was added to dissolve the mixture, and the mixture was allowed to stand at -5°C. DIEA (2.44 mL, 14.7451 mmol) was slowly added dropwise. After the dropwise addition was completed, the reaction was carried out for half an hour, the flask was then taken out, and the reaction was carried out with stirring at room temperature overnight. After the reaction was completed, saturated brine (200 mL) and ethyl acetate (200 mL) were added for extraction. After standing for phase separation, the organic phase was collected, and the aqueous phase was further extracted with ethyl acetate (200 mL × 3). The organic phases were combined, evaporated to dryness to a solid, which was then dissolved in a methanol/dichloromethane (1/4) mixture, then silica gel powder (100 mL) was added, and the mixture was evaporated to dryness to provide a powdery solid. The powdery solid was dry-loaded onto the column and subjected to column chromatography using a 2-6% methanol/dichloromethane mixture as the eluent. The desired product was collected, concentrated, and dried in a vacuum oven to provide 0.95 g of the product with a yield of 74.22%. $^1$H-NMR (600 MHz, DMSO-*$d_6$*) δ 8.06-8.00 (m, 1H), 7.90-7.83 (m, 1H), 3.32-3.28 (m, 2H), 3.27-3.23 (m, 2H), 3.21-3.16 (m, 4H), 3.12 (dd, 2H), 2.41-2.37 (m, 6H), 2.32-2.27 (m, 2H), 2.27-2.22 (m, 2H), 1.38 (s, 27H); ESI [M+H$^+$] 572.3522, [M+Na$^+$] 594.3336

17.5 Preparation of **82-133**

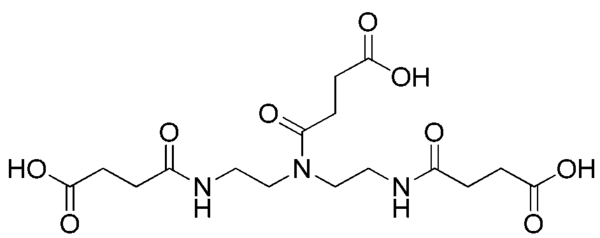

To a flask containing **82-112** (2.4020 mmol), dichloromethane (20 mL) was added for dissolution with the assistance of ultrasonication, then TFA (12 mL) was added, and the mixture was reacted with stirring at room temperature overnight. After the reaction was completed, the reaction mixture was evaporated to an oily state using a rotary evaporator. Methyl tert-butyl ether (60 mL) was then added. A powdery solid precipitated from the reaction mixture. The mixture was filtered, and the filter cake was washed with methyl tert-butyl ether (40 mL × 3). The filter cake was collected and dried in a vacuum oven to provide 1.2 g of the product with a yield exceeding 100%.

17.6 Preparation of **82-139**

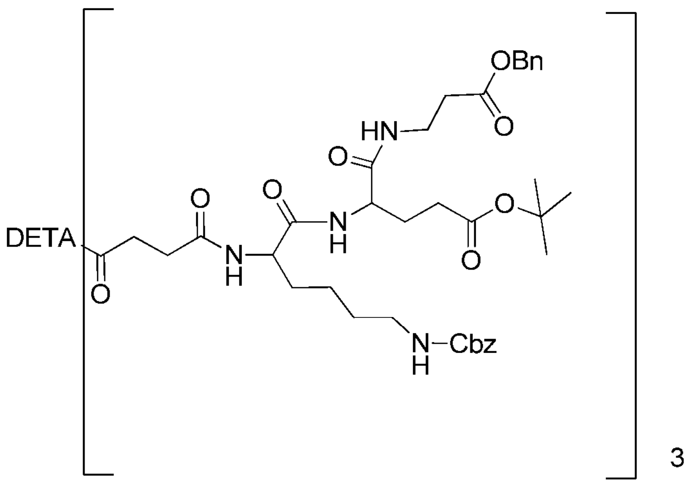

**82-133** (0.40 g, 1.0006 mmol), HBTU (1.25 g, 3.3019 mmol), and HOBT (0.45 g, 3.3019 mmol) were weighed and added to a flask containing **78-69** (2.07 g, 3.3019 mmol), then DMF (30 mL) was added to dissolve the mixture, and the mixture was stirred at -5°C for several minutes. DIEA (1.09 mL, 6.6038 mmol) was slowly added dropwise. After the dropwise addition was completed, the reaction was carried out for half an hour, the flask was then taken out, and the reaction was carried out with stirring at room temperature overnight. After the reaction was completed, n-hexane (25 mL) and methyl tert-butyl ether (200 mL) were added for precipitation. The supernatant was discarded, and n-hexane and methyl tert-butyl ether were added again for precipitation. This process was repeated three times. The mixture was filtered to provide a solid product, which was collected, concentrated, and dried to provide 1.07 g of the product with a yield of 47.91%. $^1$H-NMR (600 MHz, DMSO-$d_6$) δ 8.19-8.11 (m, 2H), 8.10-8.04 (m, 1H), 7.99-7.90 (m, 3H), 7.89-7.84 (m, 2H), 7.80-7.73 (m, 2H), 7.72-7.68 (m, 1H), 7.40-7.27 (m, 30H), 7.24-7.20 (m, 3H), 5.12-5.05 (m, 6H), 5.05-4.96 (m, 6H), 4.28-3.97 (m, 6H), 3.73-3.64 (m, 2H), 3.30-3.07 (m, 11H), 3.02-2.91 (m, 7H), 2.90-2.70 (m, 4H), 2.66-2.54 (m, 5H), 2.42-2.28 (m, 9H), 2.26-2.09 (m, 6H), 1.99-1.80 (m, 3H), 1.77-1.58 (m, 6H), 1.55-1.45 (m, 3H), 1.38-1.33 (m, 27H), 1.31-1.18 (m, 6H); ESI [M+H$^+$] 2230.1145, [M+Na$^+$] 2252.1047

17.7 Preparation of **82-150**

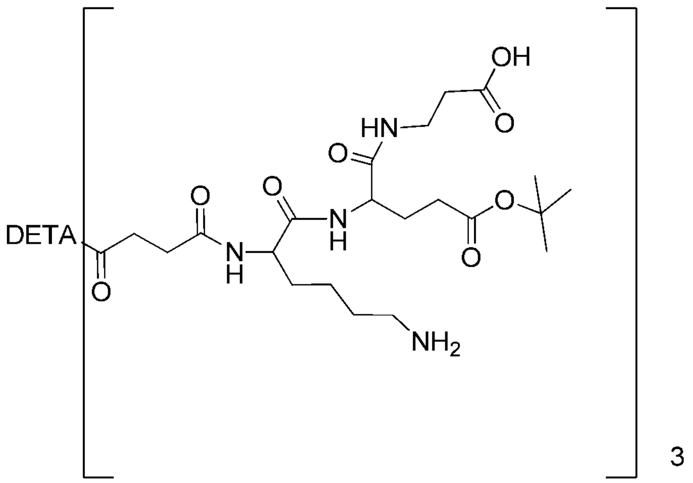

**82-139** (0.3 g, 0.1346 mmol) and 10% Pd/C catalyst (0.01 g) were placed in a hydrogenation reactor, and then DMF (30 mL) was added for dissolution. The hydrogenation reactor was sealed, evacuated with a water pump, and then filled with hydrogen gas. This process was repeated three times. The pressure reading of the hydrogenation reactor was adjusted to 1.8 MPa, and the reaction was carried out with stirring at room temperature overnight. After the reaction was completed, the reaction mixture was filtered through Celite, and the filter cake was washed with DMF (20 mL × 3). The DMF solution of the product was obtained and used as the raw material for the next reaction.

17.8 Preparation of **82-154**

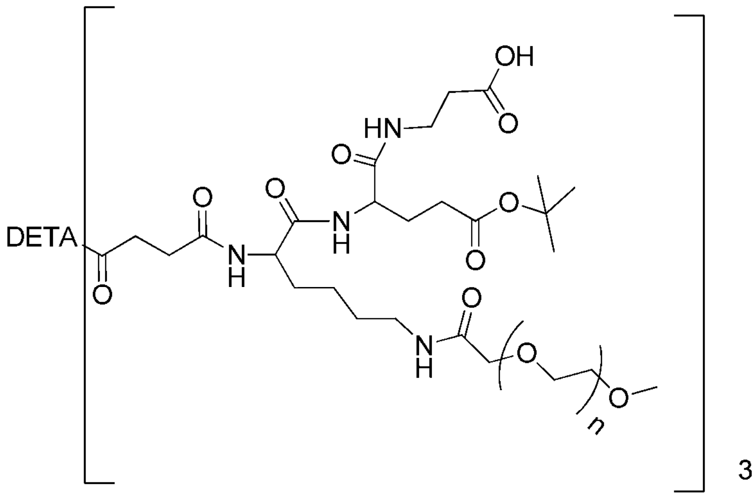

**82-150** (0.3 g, 0.1346 mmol) was placed in a 250 mL flask, then DMF (20 mL) was added for dissolution, and then a DMF solution containing DIEA (0.33 mL, 1.9988 mmol) and M-SCM-10K (4.6 g, 0.4442 mmol, purchased from Jenkem) was slowly added dropwise. The reaction was carried out with stirring at low speed in the dark at room temperature for one week. After the reaction was completed, n-hexane (25 mL) and methyl tert-butyl ether (200 mL) were added for precipitation. The supernatant was discarded, and n-hexane and methyl tert-butyl ether were added again for precipitation. This process was repeated three times. The mixture was filtered to provide a solid product, which was collected, and dried in a vacuum oven to provide 3.6 g of the product.

17.9 Preparation of **82-160**

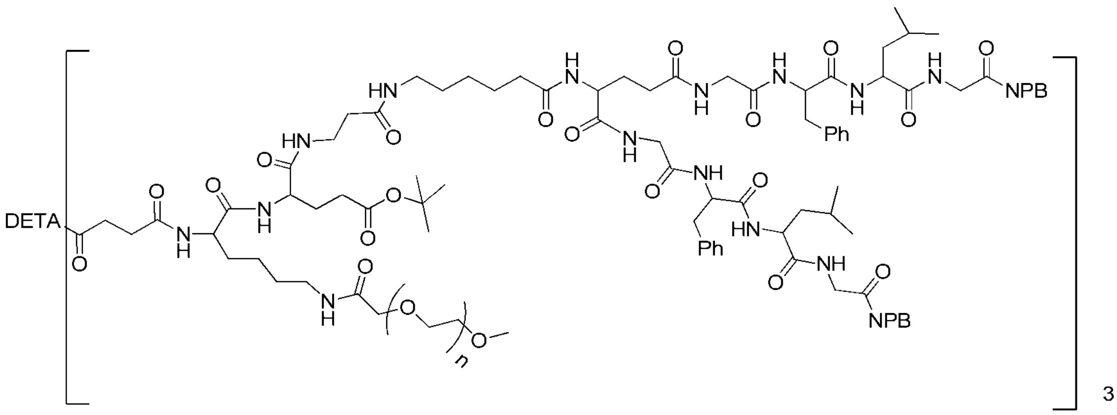

**82-154** (0.1047 mmol), HBTU (0.22 g, 0.6013 mmol), and HOBT (0.08 g, 0.0613 mmol) were weighed and added to a reaction flask containing **82-36** (1.43 g, 0.6013 mmol), then DMF (30 mL) was added to dissolve the mixture, and the mixture was stirred at -5°C for several minutes. DIEA (0.30 mL, 1.8040 mmol) was then slowly added dropwise. After the dropwise addition was completed, the reaction was carried out for half an hour, the flask was taken out, and the reaction was carried out with stirring at room temperature overnight. After the reaction was completed, n-hexane (25 mL) and methyl tert-butyl ether (200 mL) were added for precipitation. The supernatant was discarded, and n-hexane and methyl tert-butyl ether were added for precipitation. This process was repeated three times. The mixture was filtered to provide a solid product, which was collected, concentrated, and dried to provide the product.

17.10 Preparation of **82-162**

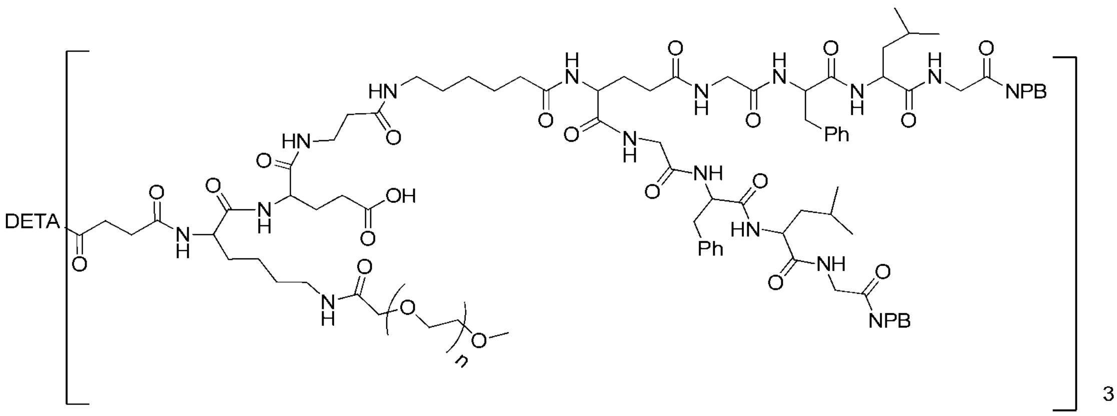

To a flask containing **82-160** (0.1047 mmol), dichloromethane (20 mL) was added for dissolution with the assistance of ultrasonication, then TFA (2 mL) was added, and the mixture was stirred at room temperature overnight. After the reaction was completed, the reaction mixture was evaporated to an oily state using a rotary evaporator. Methyl tert-butyl ether (60 mL) was then added. A powdery solid precipitated from the reaction mixture and was filtered. The filter cake was washed with methyl tert-butyl ether (40 mL × 3). The filter cake was collected and dried in a vacuum oven to provide the product.

17.11 Preparation of **82-169**

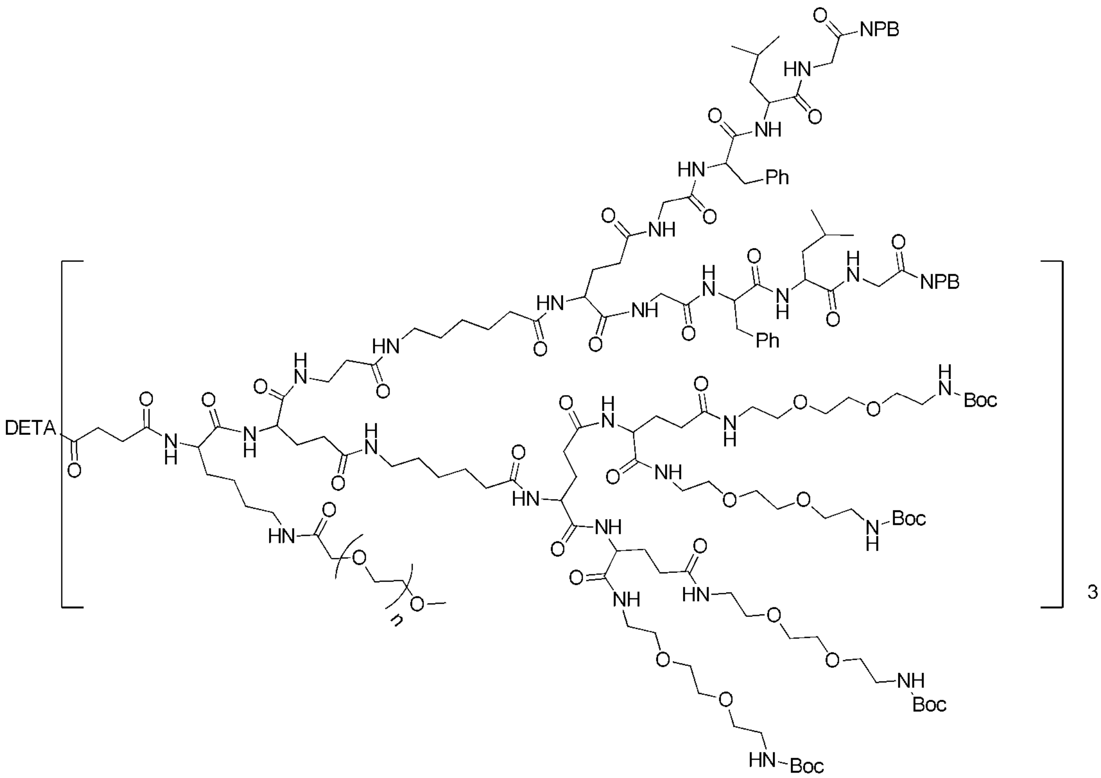

**82-162** (0.1047 mmol), HBTU (0.22 g, 0.6013 mmol), and HOBT (0.08 g, 0.0613 mmol) were weighed and added to a flask containing **80-222** (0.99 g, 0.6013 mmol), then DMF (15 mL) was added to dissolve the mixture, and the mixture was stirred at -5°C for several minutes. DIEA (0.30 mL, 1.8040 mmol) was then slowly added dropwise. After the dropwise addition was completed, the reaction was carried out for half an hour, the flask was then taken out, and the reaction was carried out with stirring at room temperature overnight. After the reaction was completed, n-hexane (25 mL) and methyl tert-butyl ether (200 mL) were added for precipitation. The supernatant was discarded, and n-hexane and methyl tert-butyl ether were added again for precipitation. This process was repeated three times. The mixture was filtered to provide a solid product, which was collected, concentrated, and dried to provide 1.4 g of the product with a yield of 32.11%.

17.12 Preparation of **82-174**

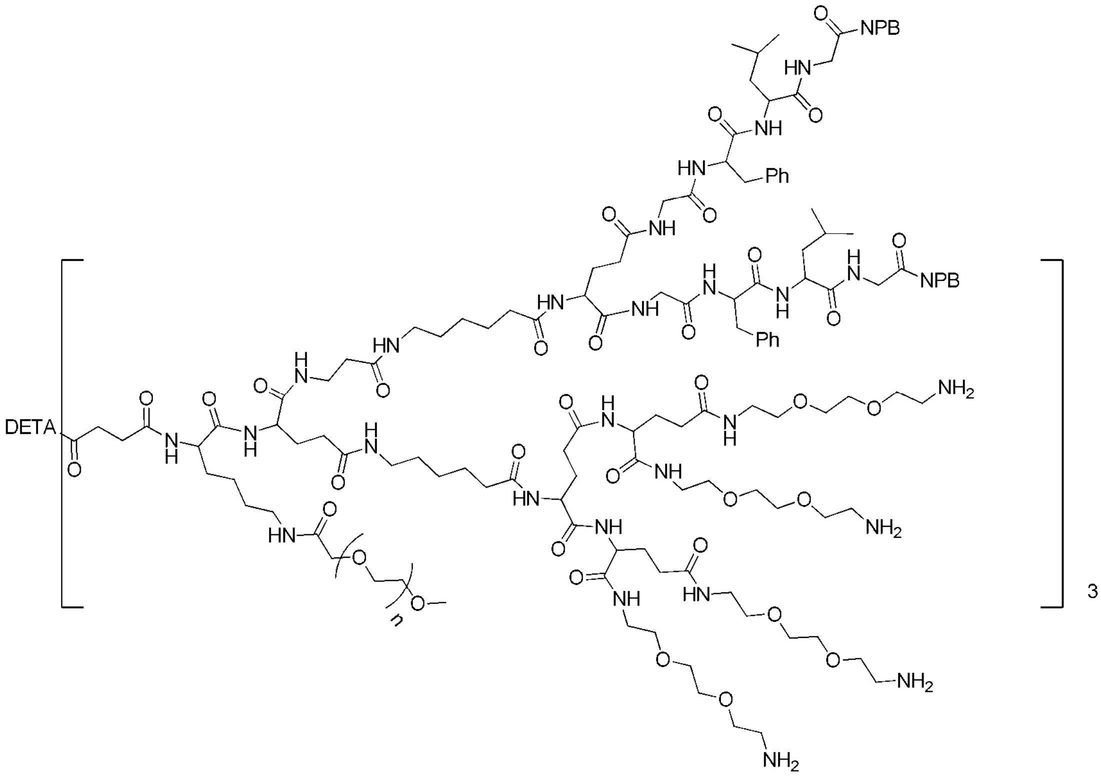

To a flask containing **82-169** (1.4 g, 0.0336 mmol), dichloromethane (20 mL) was added for dissolution with the assistance of ultrasonication, then TFA (10 mL) was added, and the mixture was reacted with stirring at room temperature overnight. After the reaction was completed, the reaction mixture was evaporated to an oily state using a rotary evaporator. Methyl tert-butyl ether (60 mL) was then added. A powdery solid precipitated from the reaction mixture and was filtered. The filter cake was washed with methyl tert-butyl ether (40 mL × 3). The filter cake was collected and dried in a vacuum oven to provide 1.6 g of the product with a yield exceeding 100%.

17.13 Preparation of **82-178**

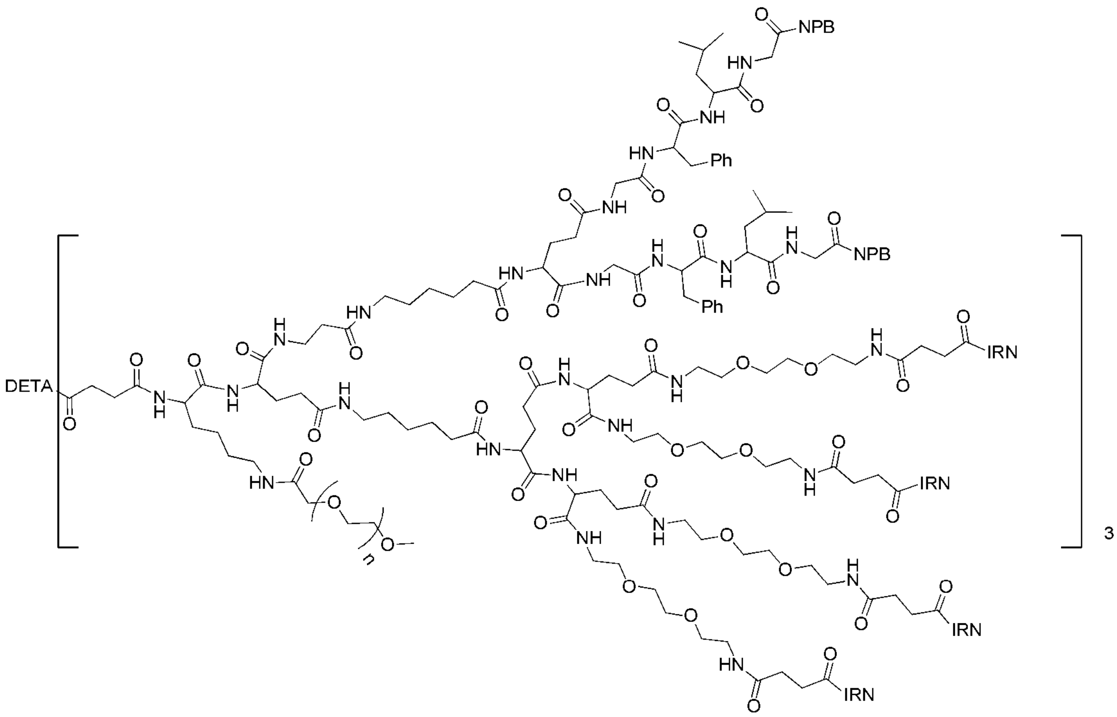

**82-174** (1.02 g, 0.0252 mmol) was weighed and added to a flask containing **78-121** (0.36 g, 0.4539 mmol), then DMF (20 mL) was added to dissolve the mixture, and the mixture was stirred at -5°C for several minutes. Triethylamine (1 mL) was slowly added dropwise. After the dropwise addition was completed, the reaction was carried out for half an hour, the flask was then taken out, and the reaction was carried out with stirring at room temperature overnight. After the reaction was completed, n-hexane (25 mL) and methyl tert-butyl ether (200 mL) were added for precipitation, the supernatant was discarded, and n-hexane and methyl tert-butyl ether were added again for precipitation. This process was repeated three times. The mixture was filtered to provide a solid product, which was collected, dissolved in a methanol/dichloromethane (1/4) mixture, then silica gel powder (100 mL) was added, and the mixture was evaporated to dryness to provide a powdery solid. The powdery solid was dry-loaded onto the column and subjected to column chromatography using a 6-12% methanol/dichloromethane mixture as the eluent. The desired product was collected, concentrated, and dried in a vacuum oven to provide 0.61 g of the product with a yield of 50.00%. $^1$H-NMR (600 MHz, DMSO-$d_6$) δ 9.34-9.20 (m, 6H), 8.63-8.52 (m, 6H), 8.33-7.73 (m, 153H), 7.70-7.55 (m, 23H), 7.54-7.38 (m, 12H), 7.35-7.05 (m, 59H), 5.54-5.41 (m, 24H), 5.33-5.21 (m, 24H), 4.68-4.50 (m, 6H), 4.48-3.91 (m, 84H), 3.88-3.77 (m, 14H), 3.70-3.43 (m, δ (m, 3079H), 3.28-3.02 (m, 238H), 2.99-2.84 (m, 50H), 2.79-2.61 (m, 74H), 2.44-2.31 (m, 52H), 2.19-2.00 (m, 100H), 1.89-1.68 (m, 86H), 1.63-1.31 (m, 189H), 1.29-1.11 (m, 80H), 0.92-0.79 (m, 78H)

Example 18: Synthesis of Compound **70-294**

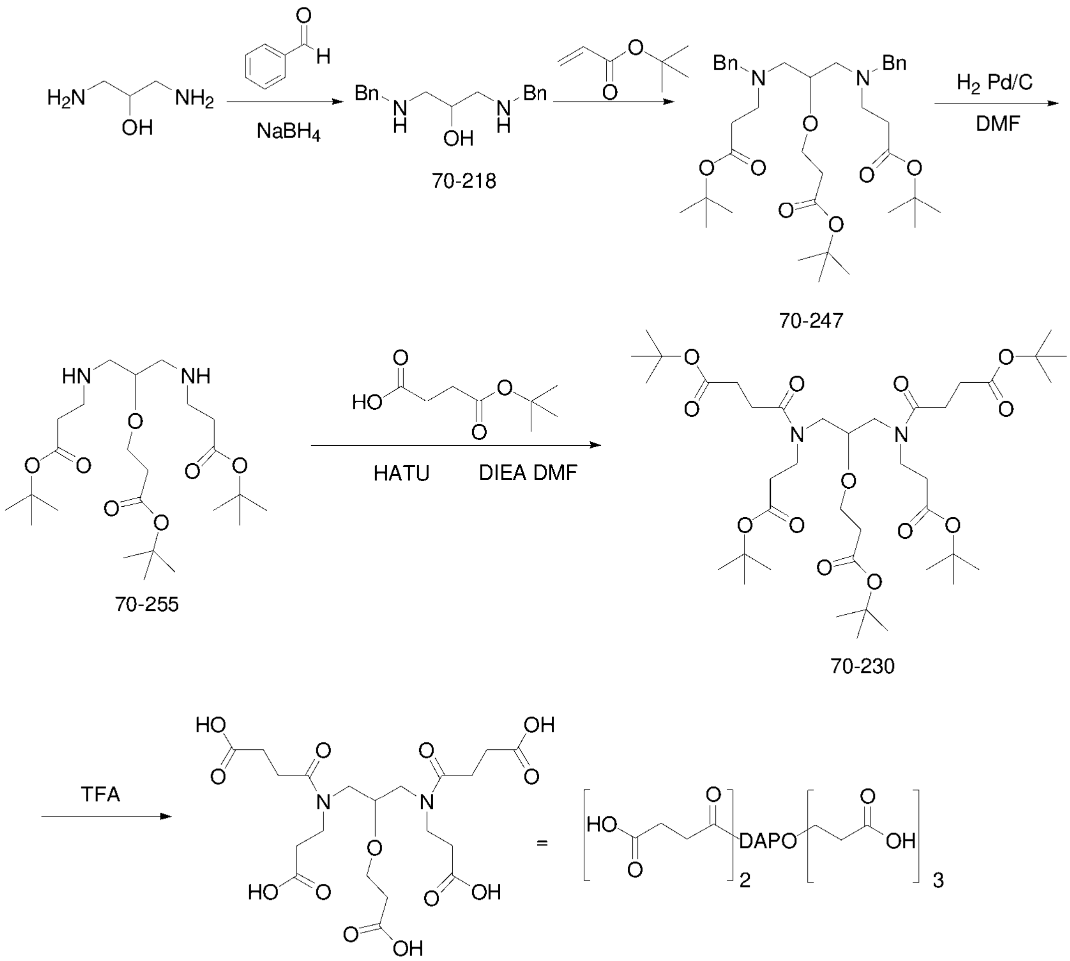


70-218

70-247

70-255

70-230

70-231

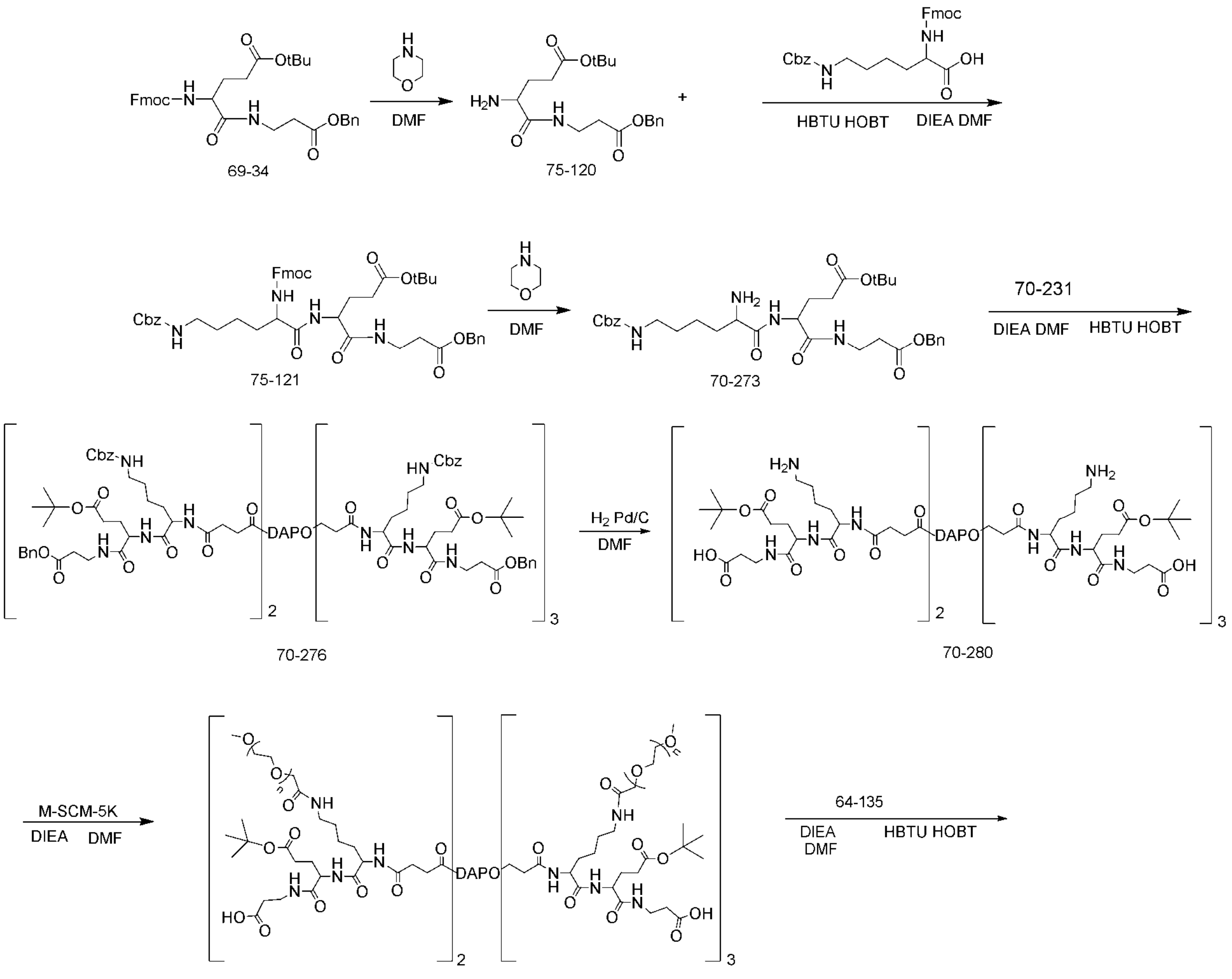
DMF
69-34
75-120
HBTU HOBT DIEA DMF
75-121
DMF
70-273
70-231
DIEA DMF HBTU HOBT
70-276
$H_2$ Pd/C
DMF
70-280
M-SCM-5K
DIEA DMF
70-283
64-135
DIEA
DMF
HBTU HOBT

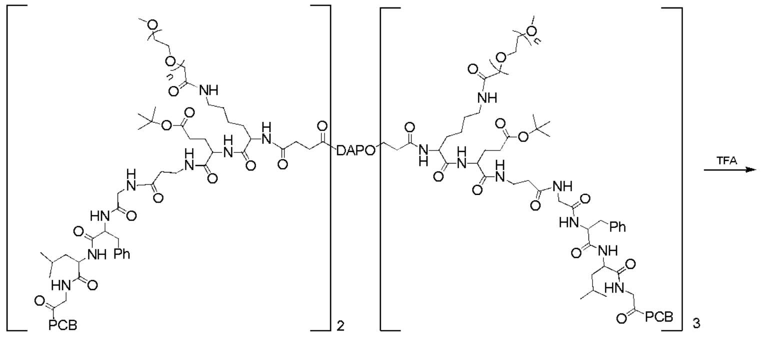


70-287

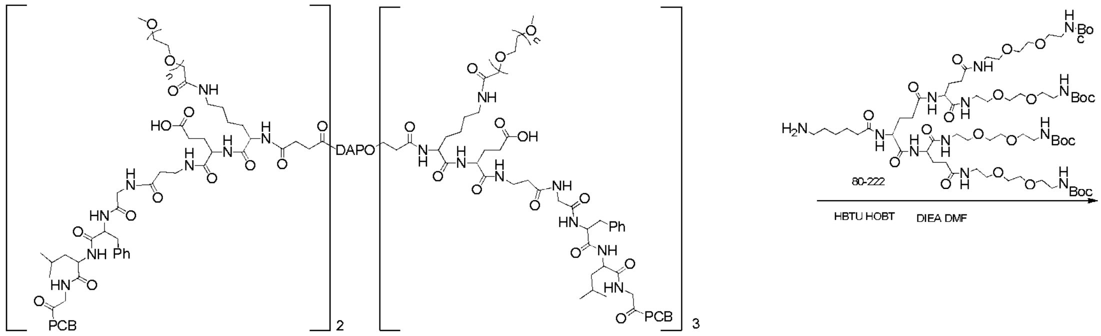


70-288

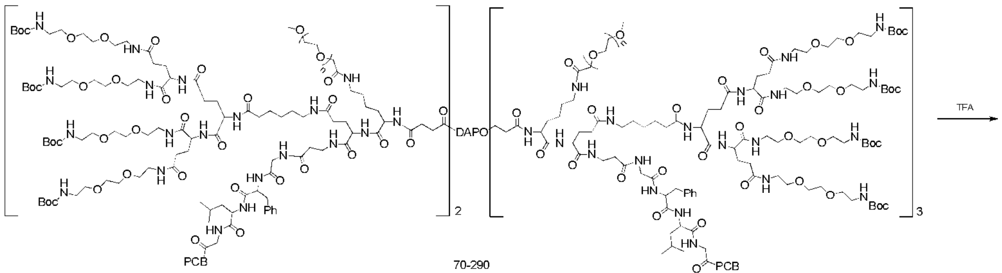


70-290

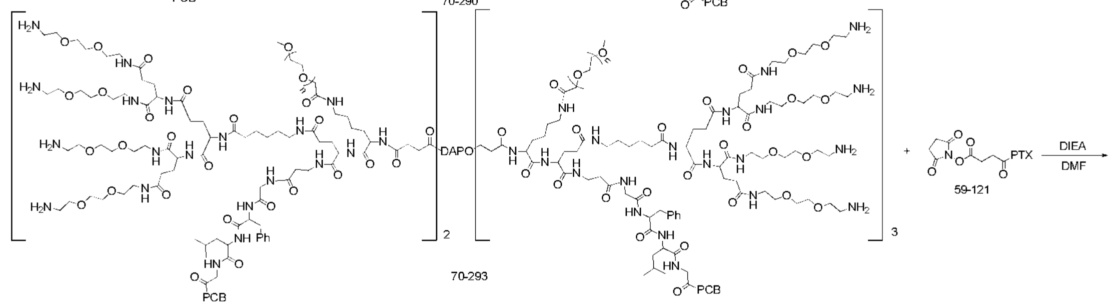


70-293

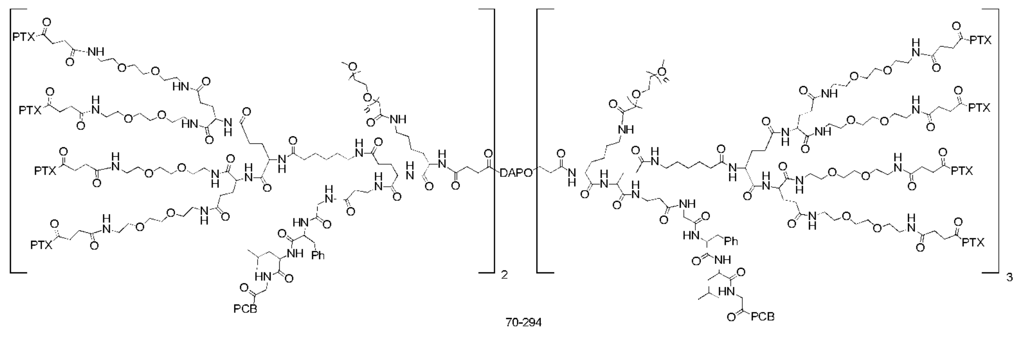

70-294

18.1 Preparation of **70-218**

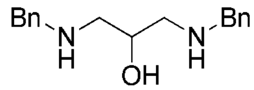

1,3-Diamino-2-propanol (3.0 g, 33 mmol) was dissolved in dichloromethane (30 mL) and stirred rapidly with a stirrer. Solid sodium sulfate (6.30 g, 44.383 mmol) and benzaldehyde (6.6 mL, 66.6 mmol) were added sequentially, and stirred at room temperature for 18 hours. The mixture was filtered, and the filtrate was concentrated. The residue was dissolved in anhydrous ethanol (20 mL) and cooled to 0°C. Solid $NaBH_4$ (3.24 g, 83.4 mmol) was added in batches, with an interval of at least 10 minutes. The mixture was stirred for 1 hour, then taken out, and added to purified water (12 mL). The reaction mixture was concentrated under reduced pressure, then ethyl acetate (60 mL) was added for dissolution. The mixture was washed with 1N aqueous hydrochloric acid (2 × 60 mL), and 3N aqueous sodium hydroxide was added to adjust pH to alkaline. The organic phase was collected, dehydrated by adding anhydrous magnesium sulfate, and filtered. The filtrate was concentrated, then silica gel powder was added, and the mixture was evaporated to dryness. The resulting product was dry-loaded onto the column and subjected to column chromatography using a 1-5% methanol/dichloromethane mixture as the eluent. The desired product was collected, concentrated under reduced pressure and dried to provide 5.87 g of the product with a yield of 64.87%. $^1$H-NMR (600 MHz, DMSO-$d_6$) δ 7.38-7.16 (m, 10H), 3.74-3.42 (m, 8H), 2.57-2.54 (m, 2H), 2.50-2.47 (m, 2H); ESI [M+H$^+$] 271.1796

18.2 Preparation of **70-247**

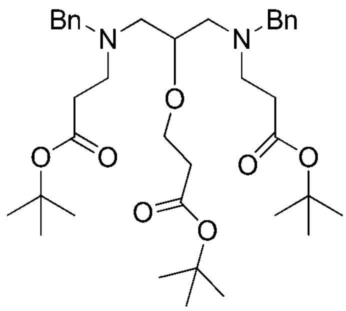

To a 250 mL flask, t-butyl acrylate (29.5 mL, 204.164 mmol) was added, then methanol (50 mL) was added for dissolution, and the mixture was stirred at 0°C for 30 minutes. A solution of **70-218** (9.2 g, 34.026 mmol) in methanol (40 mL) was then slowly added dropwise. After the dropwise addition was completed, the mixture was brought to room temperature and stirred in the dark overnight. The reaction progress was monitored by TLC. After the reaction was completed, the reaction mixture was transferred to a separatory funnel containing saturated brine (400 mL) and ethyl acetate (300 mL) for extraction. The organic phase was collected, and the aqueous phase was further extracted with ethyl acetate (200 mL). The organic phases were combined, washed with deionized water (250 mL × 2), concentrated and evaporated to dryness. The residue was then dissolved in a 20% methanol/dichloromethane mixture, then silica gel powder was added, and the mixture was evaporated to dryness. The resulting product was dry-loaded onto the column and subjected to column chromatography using a 1-6% methanol/dichloromethane mixture as the eluent. The desired product was collected, concentrated under reduced pressure, and dried in a vacuum oven to provide 5.1 g of the product. $^1$H-NMR (600 MHz, DMSO-$d_6$) δ 7.32-7.17 (m, 10H), 3.70-3.62 (m, 2H), 3.53-3.50 (m, 4H), 3.48-3.42 (m, 1H), 2.70-2.56 (m, 4H), 2.47-2.42 (m, 2H), 2.40-2.33 (m, 4H), 2.33-2.27 (m, 4H), 1.36 (s, 27H); ESI [M+H$^+$] 655.42950

18.3 Preparation of **70-255**

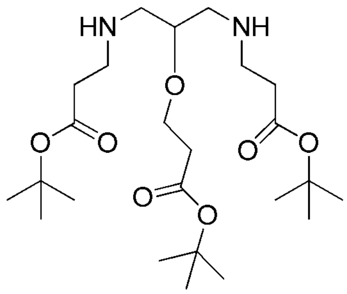

**70-247** (5.1 g, 7.7981 mmol) was added to a hydrogenation reactor, and a stirrer was placed. Methanol (20 mL) was added for dissolution, and then 10% Pd/C catalyst (0.4 g) was added. The hydrogenation reactor was sealed, evacuated with a water pump, and then refilled with hydrogen gas. This process was repeated three times. The pressure in the hydrogenation reactor was adjusted to 2 MPa, and the mixture was stirred at room temperature overnight. After the reaction was completed, the reaction mixture was filtered through Celite, and the filter cake was washed with methanol (5 mL × 3). The methanol solutions were combined and concentrated under reduced pressure. Dichloromethane (10 mL) and toluene (0.2 mL) were then added. The mixture was treated by ultrasonication and evaporated to dryness. This process was repeated five times. The resulting solid was collected and dried to provide 3.7 g of the product.

18.4 Preparation of **70-230**

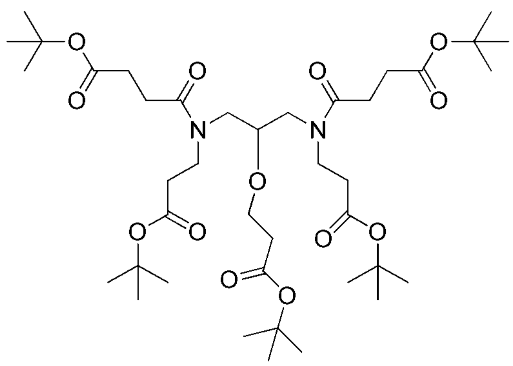

**70-255** (0.7 g, 1.47 mmol), mono-tert-butyl succinate (0.62 g, 3.24 mmol), and HATU (1.4 g, 3.54 mmol) were dissolved in DMF (10 mL) in a reaction flask. A stirrer was placed, and DIEA (1.2 mL, 7.08 mmol) was slowly added dropwise at -5°C. After the dropwise addition was completed, the reaction was carried out for 30 minutes, the flask was then taken out, and the reaction was carried out with stirring at room temperature for five hours. The reaction progress was monitored by TLC. After the reaction was completed, the mixture was poured into a separatory funnel containing ethyl acetate (60 mL) and saturated brine (60 mL) for extraction, and the aqueous phase was further extracted with ethyl acetate (50 mL × 3). The organic phases were combined and concentrated under reduced pressure, and the residue was then dissolved in a 20% methanol/dichloromethane mixture, then silica gel powder was added, and the mixture was evaporated to dryness. The resulting product was dry-loaded onto the column and subjected to column chromatography using a 10-30% ethyl acetate/petroleum ether mixture as the eluent. The desired product was collected, concentrated under reduced pressure, and dried to provide 1.1 g of the product with a yield of 95.20%. $^1$H-NMR (600 MHz, DMSO-$d_6$)δ 3.76-3.71 (m, 1H), 3.69-3.63 (m, 1H), 3.37 (d, 7.0, 2H), 3.29 (d,, 2H), 3.23-3.17 (m, 1H), 3.12-3.04 (m, 2H), 2.55-2.51 (m, J=12.0, 5.2, 3H), 2.42-2.27 (m, 12H), 2.01-1.96 (m, 1H), 1.42-1.33 (m, 45H); ESI [M+H$^+$] 787.49

18.5 Preparation of **70-231**

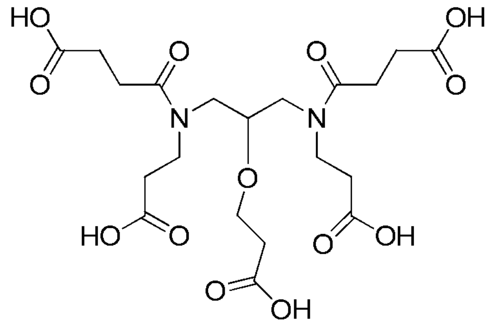

**70-230** (0.46 g, 0.58 mmol) was dissolved in dichloromethane (20 mL), then TFA (7.4 mL, 100 mmol) was added, and the mixture was stirred at room temperature for two days. The reaction progress was monitored by TLC. After the reaction was completed, n-hexane (25 mL) and methyl tert-butyl ether (200 mL) were added for precipitation. The supernatant was discarded, and n-hexane (40 mL) and methyl tert-butyl ether (70 mL) were added again for precipitation. This process was repeated three times. The mixture was filtered to provide a solid product, which was dried in a vacuum oven to provide 0.3 g of the product with a yield of 85.3%.

18.6 Preparation of **75-120**

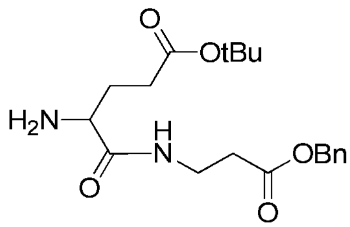

To a flask containing **69-34** (11.03 g, 18.802 mmol), DMF (100 mL) was added for dissolution with the assistance of ultrasonication, then morpholine (32 mL, 376.04 mmol) was added, and the mixture was stirred at room temperature for 2 h. The reaction progress was monitored by TLC. After the reaction was completed, the mixture was poured into a separatory funnel containing saturated sodium chloride (200 mL) and ethyl acetate (200 mL) for extraction. After standing for phase separation, the organic phase was collected, and the aqueous phase was further extracted with ethyl acetate (200 mL × 3). The organic phases were combined, evaporated to dryness, and the residue was dissolved in a 20% methanol/dichloromethane mixture, then silica gel powder was added, and the mixture was evaporated to dryness. The resulting product was dry-loaded onto the column and subjected to column chromatography using a 0.5-1.0% ammonia solution/2-10% methanol/dichloromethane mixture as the eluent. The desired product was collected, concentrated under reduced pressure, and dried to provide 9.44 g with a yield exceeding 100%.

18.7 Preparation of **75-121**

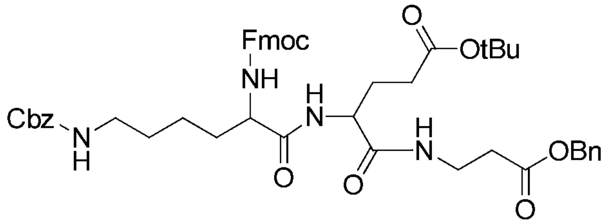

**75-120** (9.44 g, 18.802 mmol), N'-Fmoc-N-benzyloxycarbonyl-L-lysine (6.85 g, 18.802 mmol), HBTU (10.695 g, 28.23 mmol), and HOBT (3.810 g, 40.6096 mmol) were added to a 500 mL flask, and DMF (60 mL) was added for dissolution. DIEA (13.9 mL, 121.8290 mmol) was then slowly added dropwise at -5°C. After the dropwise addition was completed, the reaction was carried out with stirring at 0°C and monitored by TLC. After the reaction was completed, the

reaction mixture was transferred to a 1 L separatory funnel containing saturated sodium bicarbonate solution (200 mL) and ethyl acetate (300 mL). The organic phase was collected, and the aqueous phase was further extracted with ethyl acetate (100 mL × 3). The organic phases were combined, washed with pure water (150 mL × 2), concentrated, and dried to provide 11.9 g of the product with a yield exceeding 100%.

18.8 Preparation of **70-273**

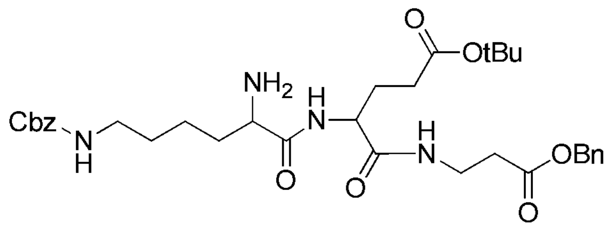

To a flask containing **75-121** (3.27 g, 3.85 mmol), DMF (30 mL) was added for dissolution with the assistance of ultrasonication, then morpholine (6.7 mL, 77.09 mmol) was added, and the mixture was reacted with stirring at room temperature for 2 hours. The reaction was monitored by TLC. After the reaction was completed, the mixture was poured into a separatory funnel containing saturated sodium chloride (200 mL) and ethyl acetate (200 mL) for extraction. After standing for phase separation, the organic phase was collected, and the aqueous phase was further extracted with ethyl acetate (200 mL × 3). The organic phases were combined, evaporated to dryness to provide a solid, which was dried in a vacuum oven to provide 2.00 g of the product with a yield of 97.61%.

18.9 Preparation of **70-276**

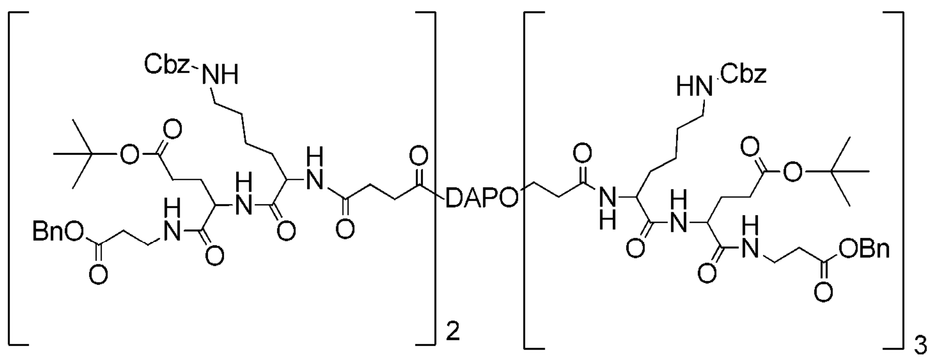

**70-273** (1.3 g, 2.57 mmol), **70-231** (0.21 g, 0.43 mmol), HBTU (0.34 g, 2.5 mmol), and HOBT (0.95 g, 2.5 mmol) were weighed and placed in a 250 mL flask, then DMF (20 mL) was added to dissolve the mixture, and then DIEA (1.7 mL, 9 mmol) was slowly added dropwise at -5°C. After the dropwise addition was completed, the reaction was carried out for 30 minutes, the flask was then taken out, and the reaction was carried out with stirring at room temperature. The reaction progress was monitored by TLC. After the reaction was completed, n-hexane (25 mL) and methyl tert-butyl ether (200 mL) were added for precipitation. The supernatant was discarded, and n-hexane (40 mL) and methyl tert-butyl ether (70 mL) were added again for precipitation. This process was repeated three times. The mixture was filtered to provide a solid product, which was dissolved in a methanol/dichloromethane mixture, then silica gel powder was added, and the mixture was evaporated to dryness. The resulting product was dry-loaded onto the column and subjected to column chromatography using a 3-5% methanol/dichloromethane mixture as the eluent. The desired product was collected, concentrated under reduced pressure, and dried to provide 0.73 g of the product with a yield of 63.94%. $^1$H-NMR (600 MHz, DMSO-*$d_6$*) δ 8.16-8.02 (m, 5H), 8.02-7.85 (m, 10H), 7.36-7.28 (m, 50H), 7.24-7.16 (m, 5H), 5.07 (s, 10H), 4.98 (s, 10H), 4.32-4.11 (m, 10H), 3.68-3.48 (m, 6H), 3.39-3.37 (m, 3H), 3.26-3.20 (m, 5H), 2.98-2.93 (m, 10H), 2.66-2.53 (m, 4H), 2.50-2.48 (m, 5H), 2.43-2.27 (m, 10H), 2.26-2.08 (m, 12H), 1.94-1.79 (m, 6H), 1.78-1.67 (m, 6H), 1.66-1.55 (m, 6H), 1.54-1.43 (m, 6H), 1.38-1.36 (m, 10H), 1.36-1.34 (m, 45H), 1.29-1.23 (m, 10H), 1.23-1.18 (m, 6H)

18.10 Preparation of **70-280**

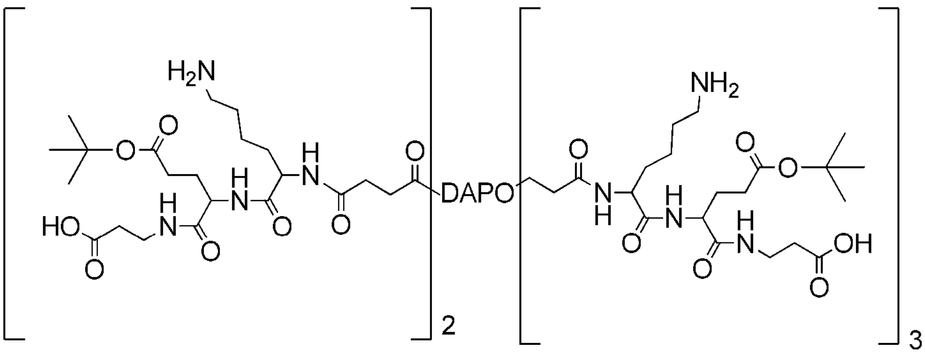

**70-276** (0.4 g, 0.012 mmol) was weighed and added to a micro hydrogenation reactor, then methanol (20 mL) was added for dissolution, and then 10% Pd/C catalyst (0.6 mg) was added. A magnetic stirrer was added. The reactor was sealed, evacuated with a water pump, and then filled with hydrogen gas. This process was repeated three times. The pressure in the hydrogenation reactor was adjusted to 2 MPa, and the mixture was stirred for 3 days. The reaction progress was monitored by TLC. After the reaction was completed, the reaction mixture was filtered through Celite, and the filter cake was washed with methanol (5 × 3 mL). The collected filtrate was concentrated, then dichloromethane (10 mL) and toluene (0.2 mL) were added, and the mixture was evaporated to dryness. This process was repeated five times. The resulting product was dried in a vacuum oven to provide 0.21 g of the product.

18.11 Preparation of **70-283**

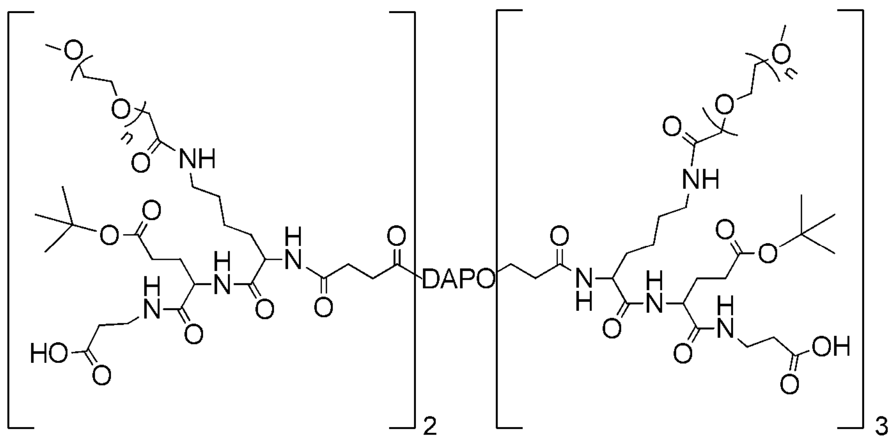

Reactant **70-280** (0.1 g, 0.041 mmol) was dissolved in DMF (15 mL), and then DIEA (0.2 mL, 1.2 mmol) was slowly added dropwise at -5°C. After the dropwise addition was completed, the mixture was stirred for 30 minutes, the flask was then taken out, M-SCM-5K (1.2 g, 0.228 mmol) was added, and reacted with stirring at low speed in the dark at room temperature. The reaction progress was monitored by TLC. After the reaction was completed, n-hexane (25 mL) and methyl tert-butyl ether (200 mL) were added for precipitation. The supernatant was discarded, and n-hexane (40 mL) and methyl tert-butyl ether (70 mL) were added again for precipitation. This process was repeated three times. The mixture was filtered to provide a solid product, which was dried to provide 1.28 g of the product.

18.12 Preparation of **70-287**

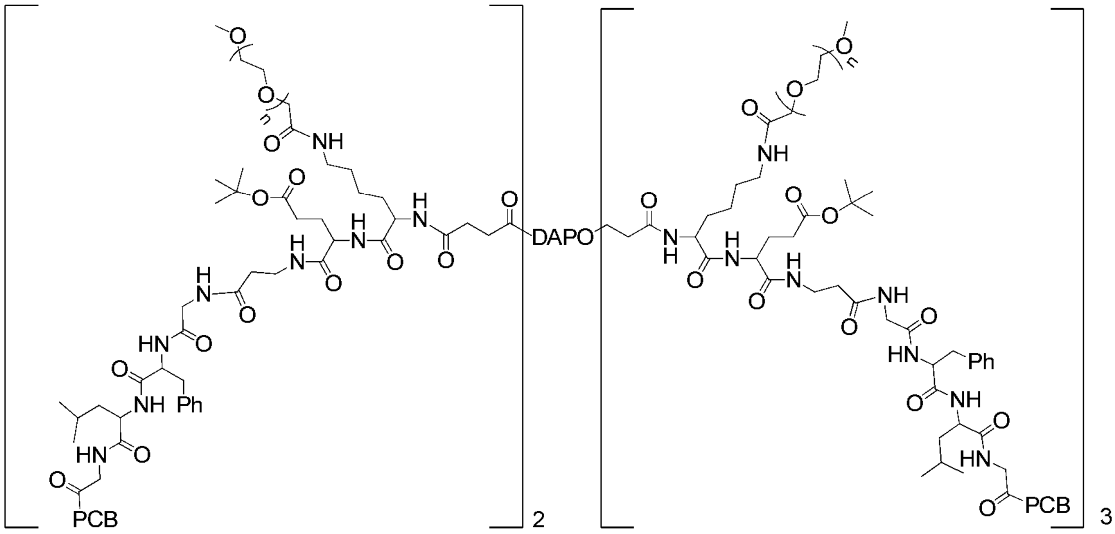

Reactants **70-283** (1.28 g, 0.045 mmol), **64-135** (0.26 g, 0.27 mmol), HOBT (0.04 g, 0.30 mmol), and HBTU (0.11 g, 0.30 mmol) were placed in a reaction flask, then DMF (30 mL) was added to dissolve the mixture with the assistance of ultrasonication. DIEA (0.2 mmol, 1.2 mmol) was then slowly added dropwise at -5°C. After the dropwise addition was completed, the mixture was brought to room temperature, and the reaction was continued with stirring. The reaction progress was monitored by TLC. After the reaction was completed, n-hexane (50 mL) and methyl tert-butyl ether (200 mL) were added for precipitation. The supernatant was discarded, and n-hexane (40 mL) and methyl tert-butyl ether (100 mL) were added again for precipitation. This process was repeated three times. The mixture was filtered to provide a solid product, which was dissolved in a methanol/dichloromethane mixture, then silica gel powder was added, and the mixture was evaporated to dryness. The resulting product was dry-loaded onto the column and subjected to column chromatography using a 1% ammonia/8% methanol/dichloromethane mixture as the eluent. The desired product was collected, concentrated under reduced pressure, and dried to provide 0.33 g of the product.

18.13 Preparation of **70-288**

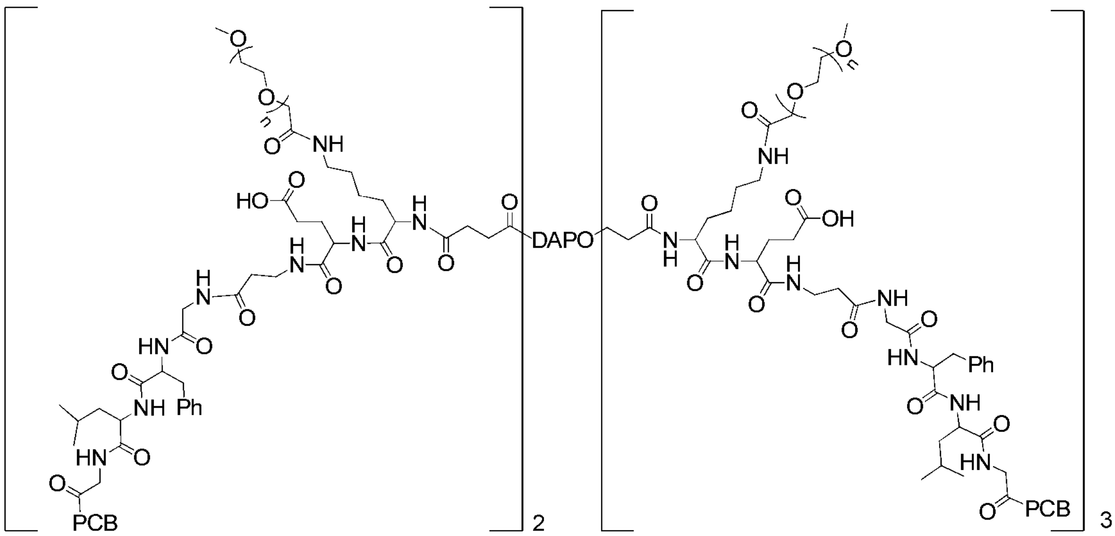

Reactant **70-287** (0.33 g, 0.01 mmol) was dissolved in dichloromethane (20 mL), then TFA (5 mL, 28.6 mmol) was added, and the mixture was reacted with stirring at room temperature. The reaction progress was monitored by TLC. After the reaction was completed, the reaction mixture was concentrated under reduced pressure to remove dichloromethane and most of the TFA. Methyl tert-butyl ether (100 mL) and n-hexane (20 mL) were then added for precipitation. The mixture was filtered using a sand-core funnel and rinsed with methyl tert-butyl ether (100 mL × 3). The filter cake was collected and dried in a vacuum oven to provide 0.3 g of the product.

18.14 Preparation of **70-290**

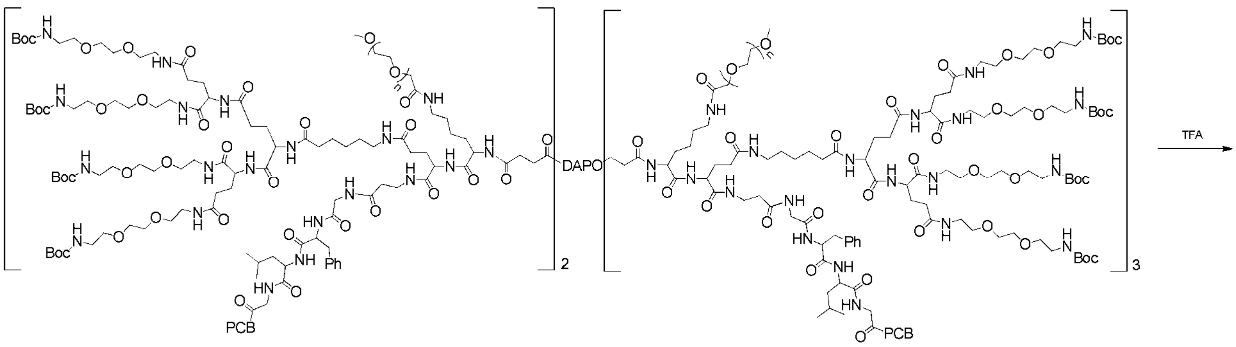


Reactants **70-288** (0.343 g, 0.0108 mmol), **80-222** (0.093 g, 0.0646 mmol), HOBT (0.013 g, 0.0969 mmol), and HBTU (0.036 g, 0.0969 mmol) were placed in a reaction flask, then DMF (5 mL) was added to dissolve the mixture with the assistance of ultrasonication. DIEA (0.3 mL, 0.1937 mmol) was slowly added dropwise at -5°C. After the dropwise addition was completed, the mixture was brought to room temperature, and the reaction was continued with stirring. The reaction progress was monitored by TLC. After the reaction was completed, n-hexane (50 mL) and methyl tert-butyl ether (200 mL) were added for precipitation. The supernatant was discarded, and n-hexane (40 mL) and methyl tert-butyl ether (100 mL) were added again for precipitation. This process was repeated three times. The mixture was filtered to provide a solid product, which was dissolved in a 20% methanol/dichloromethane mixture, then silica gel powder was added, and the mixture was evaporated to dryness. The resulting product was dry-loaded onto the column and subjected to column chromatography using a 0.8-1% aqueous ammonia/6-8% methanol/dichloromethane mixture as the eluent. The desired product was collected, concentrated under reduced pressure, and dried to provide 0.3 g of the product.

18.15 Preparation of **70-293**

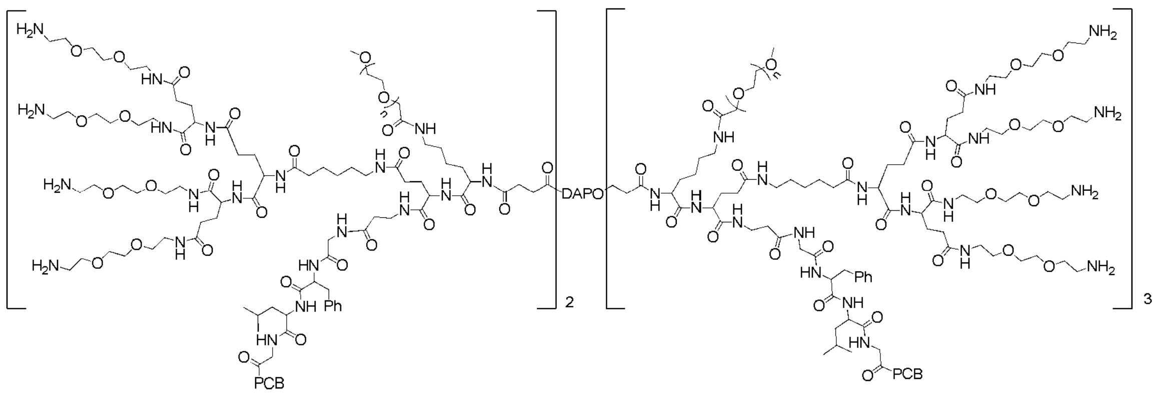

Reactant **70-290** (0.3 g, 0.0081 mmol) was placed in a reaction flask, then dichloromethane (20 mL) was added for dissolution. Then TFA (3 mL, 4 mmol) was added, and the mixture was reacted with stirring at room temperature. The reaction progress was monitored by TLC. After the reaction was completed, the reaction mixture was concentrated under reduced pressure to remove dichloromethane and most of the TFA. Methyl tert-butyl ether (100 mL) and n-hexane (20 mL) were then added for precipitation. The mixture was filtered using a sand-core funnel and rinsed with methyl tert-butyl ether (100 mL × 3). The filter cake was collected and dried in a vacuum oven to provide 0.3 g of the product.

18.16 Preparation of **70-294**

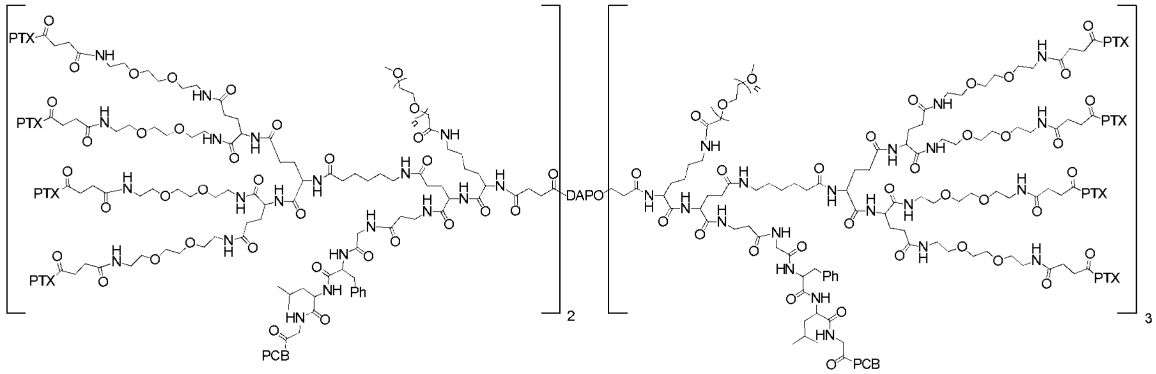

Reactant **70-293** (0.3 g, 0.0081 mmol) was placed in a reaction flask, and DMF (10 mL) was added for dissolution with the assistance of ultrasonication. DIEA (0.1 mL, 6.1 mmol) was slowly added dropwise at -5°C. After the dropwise addition was completed, **59-121** (0.25 g, 0.24 mmol) was added. The reaction mixture was brought to room temperature, and the reaction was continued with stirring. The reaction progress was monitored by TLC. After the reaction was completed, n-hexane (50 mL) and methyl tert-butyl ether (200 mL) were added for precipitation. The supernatant was discarded, and n-hexane (40 mL) and methyl tert-butyl ether (100 mL) were added again for precipitation. This process was repeated three times. The mixture was filtered to provide a solid product, which was dissolved in a methanol/dichloromethane mixture, then silica gel powder was added, and the mixture was evaporated to dryness. The resulting product was dry-loaded onto the column and subjected to column chromatography using a 1% ammonia solution/8% methanol/dichloromethane mixture as the eluent. The desired product was collected, concentrated under reduced pressure, and dried to provide 0.18 g of the product with a yield of 39.75%. $^1$H NMR (600 MHz, DMSO-$d_6$) δ 8.01-7.95 (m, 134H), 7.87-7.84 (m, 64H), 7.76-7.72 (m, 44H), 7.59-7.54 (m, 50H), 7.48-7.43 (m, 130H), 7.22-7.16 (m, 33H), 6.91-6.88 (m, 5H), 6.70-6.66 (m, 15H), 6.23-6.18 (m, 10H), 5.82-5.78 (m, 30H), 5.55-5.50 (m, 30H), 4.61-4.56 (m, 12H), 4.14-4.07 (m, 54H), 4.03-3.98 (m, 72H), 3.62-3.54 (m,161H), 3.52-3.49 (m, 2340H), 3.25-3.17 (m,178H), 3.09-3.07 (m, 14H), 2.79-2.77 (m, 82H), 2.63-2.61 (m, 80H), 2.25-2.21 (m, 133H), 2.13-2.09 (m, 129H), 1.80-1.73 (m, 168H), 1.67-1.59 (m, 62H), 1.234-1.220 (m, 190H), 0.872-0.849 (m, 30H)

Example 19: Synthesis of Compound **81-176**

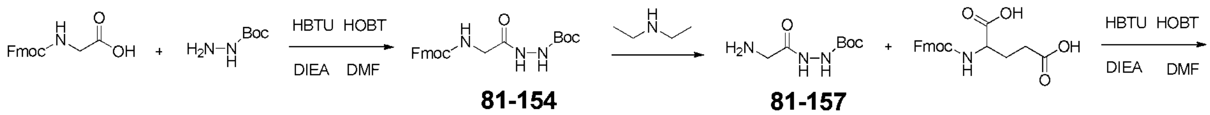


81-154 81-157

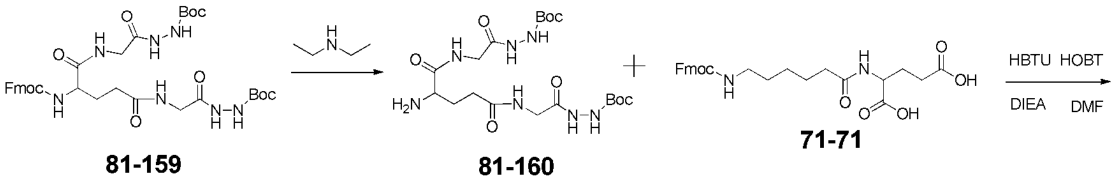


81-159 81-160 71-71

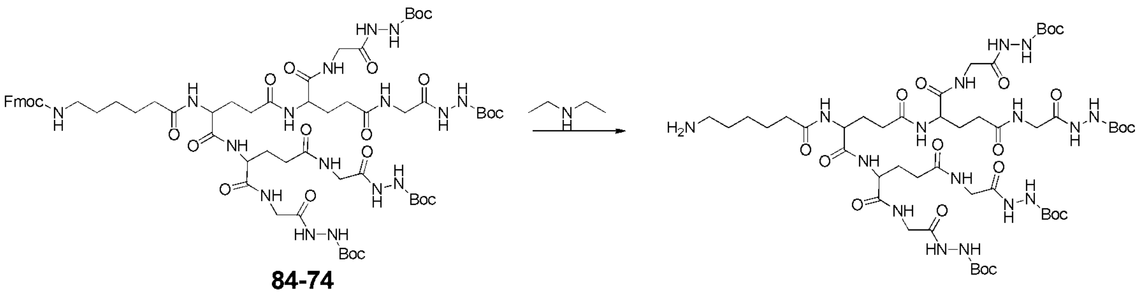

84-74 84-98

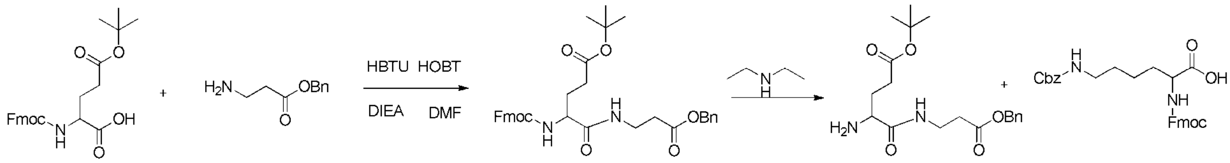


81-140 84-65

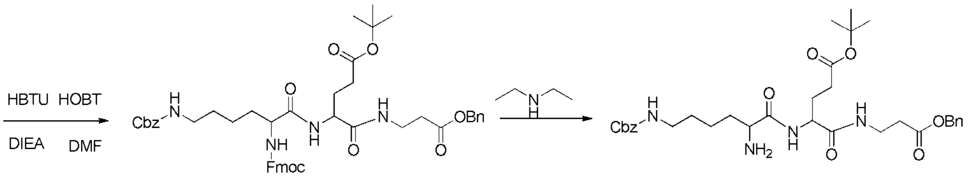


84-67 84-68

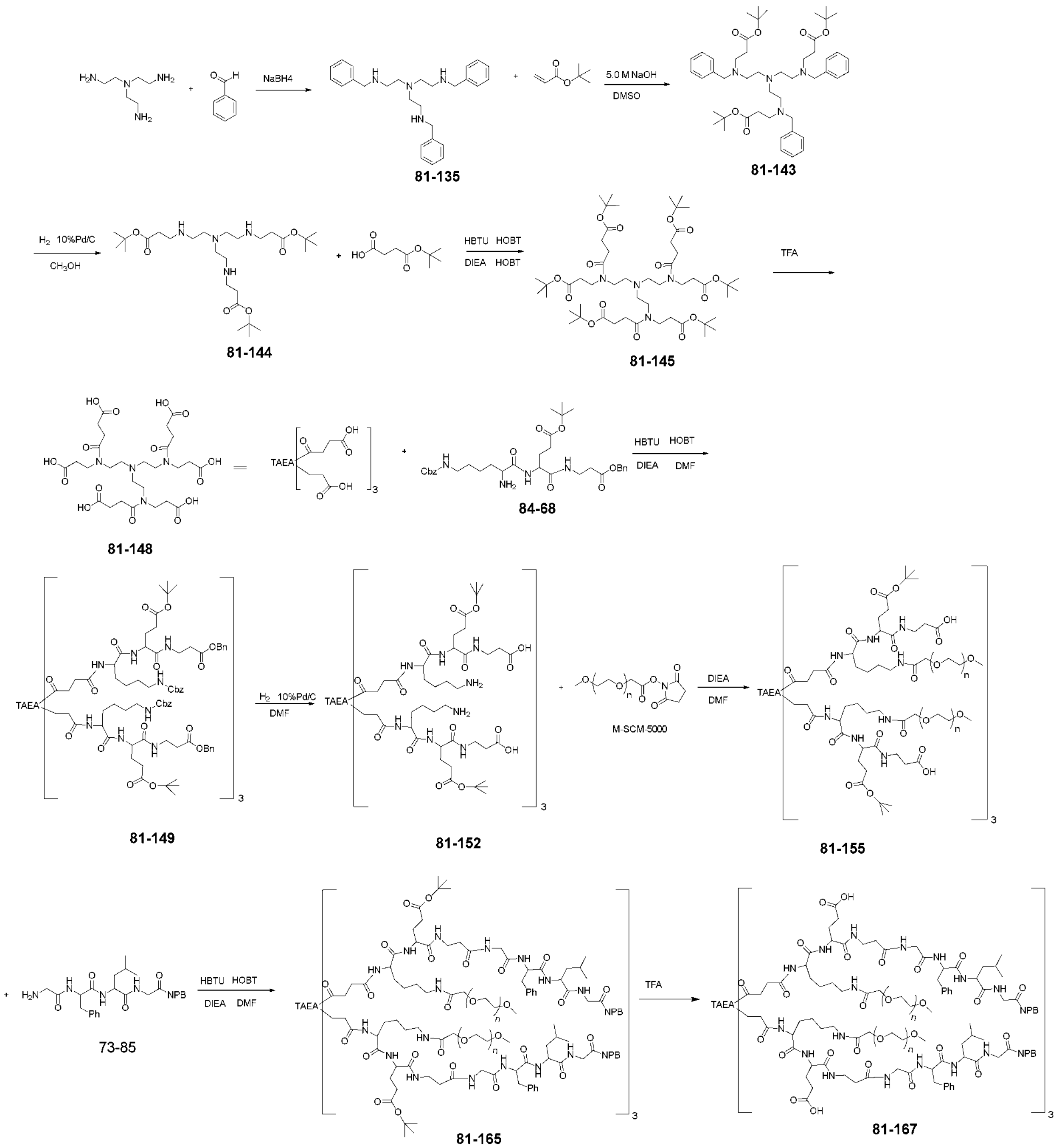


81-135

81-143

81-144

81-145

81-148

84-68

81-149

81-152

81-155

73-85

81-165

81-167

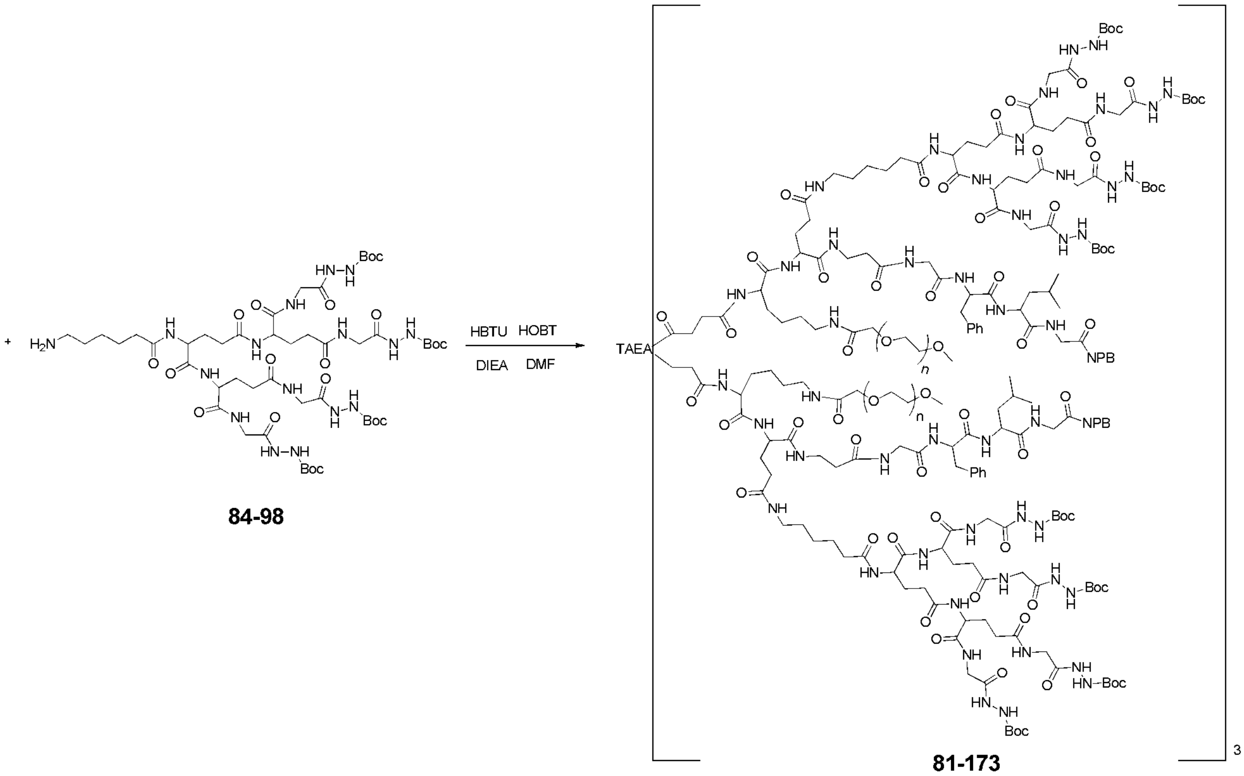


84-98

81-173

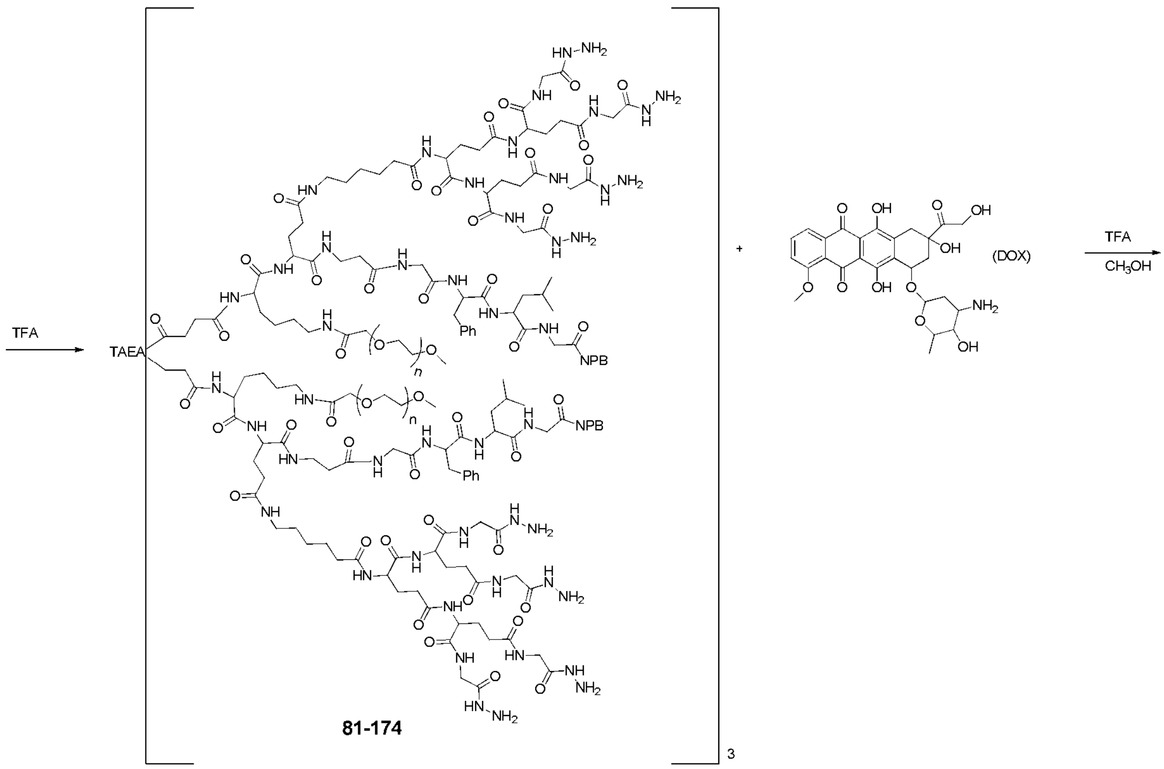


81-174

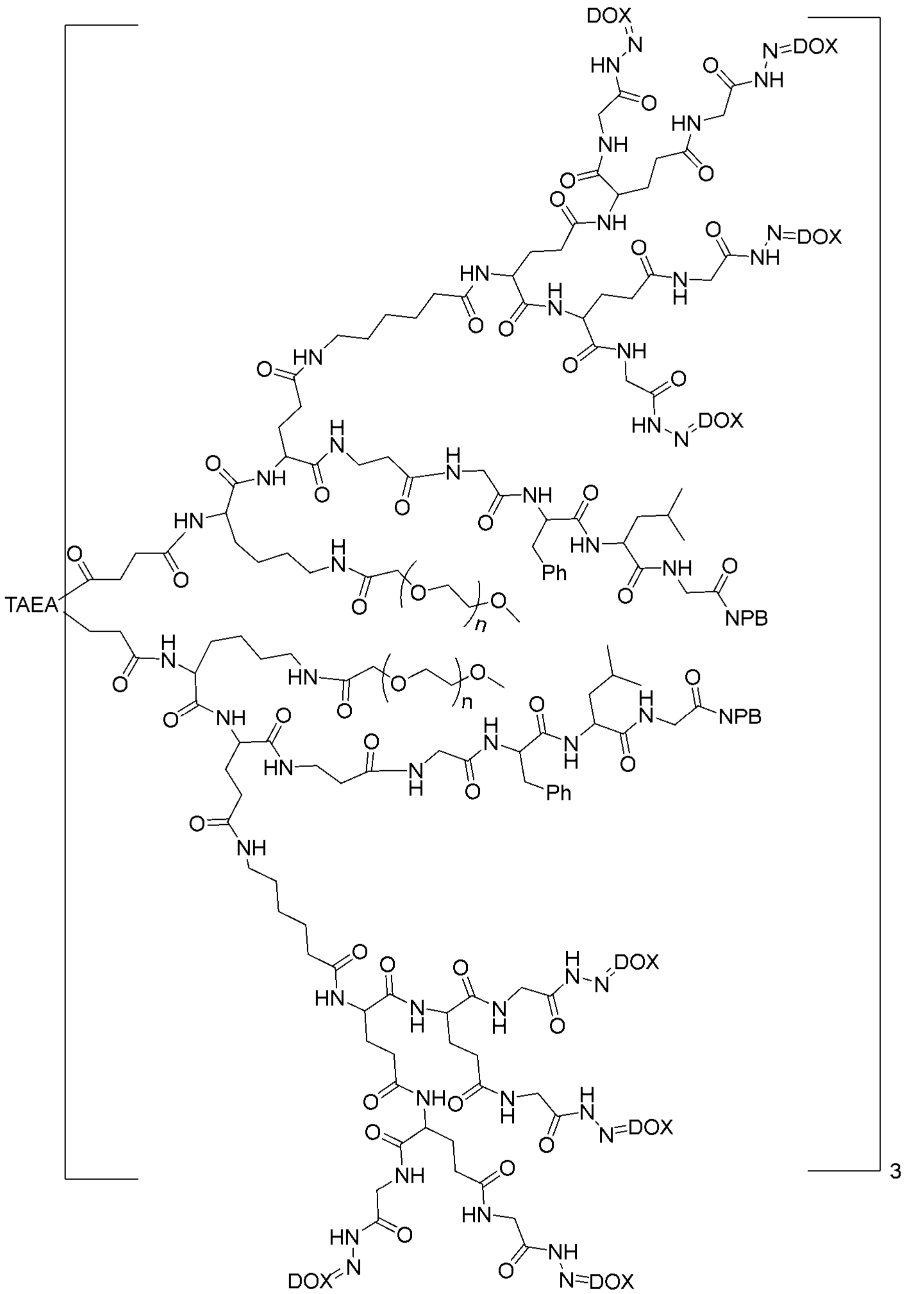

**81-176**

19.1 Synthesis of **81-154**

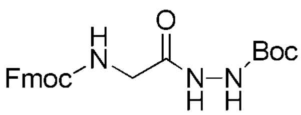

Fmoc-Gly-OH (30 g, 100.88 mmol), tert-butyl carbazate (17.33 g, 131.14 mmol), and DCC (20.81 g, 100.88 mmol) were added to a 500 mL round-bottom flask, then ethyl acetate (80 mL) was added to dissolve the mixture with the assistance of ultrasonication, and the mixture was stirred at 0°C for 2 hours. The flask was then taken out, and the reaction

was carried out with stirring at room temperature. After the reaction was completed, the solid was removed by suction filtration. Ethyl acetate (200 mL) and purified water (200 mL) were added to the filtrate for extraction. The organic phase was collected, and the aqueous phase was further extracted with ethyl acetate (100 mL × 2). The organic phases were combined, and washed with saturated brine (200 mL). The resulting organic phase was collected, dehydrated with anhydrous sodium sulfate, filtered, concentrated, and evaporated to dryness to provide 41.5 g of the product.

19.2 Synthesis of **81-157**

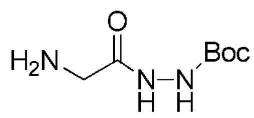

**81-154** (41.5 g, 100.88 mmol) was placed in a 500 mL round-bottom flask, then acetonitrile (50 mL) was added for dissolution with the assistance of ultrasonication, then diethylamine (196 mL, 2017.6 mmol) was added, and the mixture was reacted with stirring at room temperature. After the reaction was completed, the acetonitrile and diethylamine were removed using a rotary evaporator. An appropriate amount of a methanol/dichloromethane (1/9) mixture (200 mL) was added to the residue for dissolution, then 200-300 mesh silica gel powder (120 g) was added, and the mixture was evaporated to dryness. The resulting product was dry-loaded onto the column and subjected to column chromatography using 1% aqueous ammonia/5-7% methanol/dichloromethane as the eluent. The desired product was collected, concentrated, and evaporated to dryness to provide 13.5 g of the product.

19.3 Synthesis of **81-159**

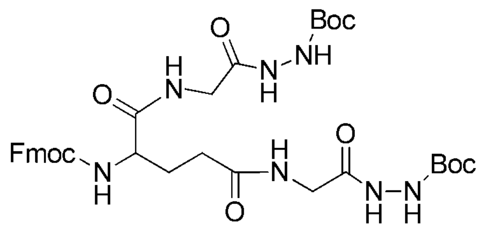

Fmoc-Glu-OH (5.57 g, 15.1 mmol), **81-157** (6 g, 31.71 mmol), HBTU (12.02 g, 31.71 mmol), and HOBT (4.28 g, 31.71 mmol) were placed in a 500 mL round-bottom flask, then DMF (20 mL) was added to dissolve the mixture with the assistance of ultrasonication, and the mixture was stirred at 0°C for several minutes. DIEA (16.22 mL, 98.15 mmol) was then added dropwise. The mixture was then brought to room temperature and reacted with stirring. After the reaction was completed, the reaction mixture was poured into a 1 L separatory funnel containing purified water (200 mL) and ethyl acetate (200 mL) for extraction. The organic phase was collected, and the aqueous phase was further extracted with ethyl acetate (100 mL × 2). The organic phases were combined, and washed with saturated sodium bicarbonate solution (200 mL × 2). The resulting organic phase was collected, dried over anhydrous magnesium sulfate, concentrated, and evaporated to dryness to provide 10.74 g of the product.

19.4 Synthesis of **81-160**

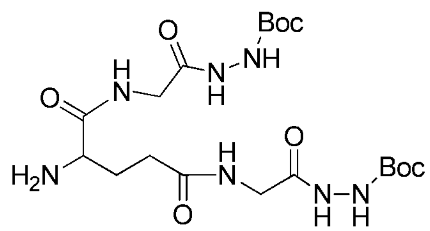

**81-159** (10.74 g, 15.1 mmol) was placed in a 500 mL round-bottom flask, then acetonitrile (20 mL) was added for dissolution with the assistance of ultrasonication. Diethylamine (29.45 mL, 302 mmol) was added, and the mixture was reacted with stirring at room temperature. After the reaction was completed, the acetonitrile and diethylamine were removed using a rotary evaporator. An appropriate amount of dichloromethane was added to the residue for dissolution, then 200-300 mesh silica gel powder (30 g) was added, and the mixture was evaporated to dryness. The resulting product was dry-loaded onto the column and subjected to column chromatography using 0-1% aqueous ammonia/5-15% methanol/dichloromethane as the eluent. The desired product was collected, concentrated, and evaporated to dryness to provide 7.39 g of the product.

19.5 Synthesis of **84-74**

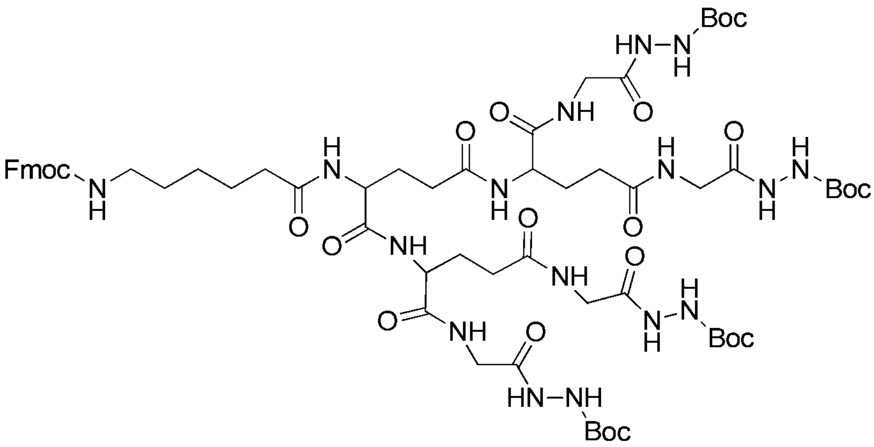

**71-71** (2.12 g, 4.40 mmol), **81-160** (4.52 g, 9.24 mmol), HBTU (4.34 g, 11.44 mmol), and HOBT (1.55 g, 11.44 mmol) were added to a 500 mL round-bottom flask, then DMF (10 mL) was added to dissolve the mixture with the assistance of ultrasonication, and the mixture was stirred at 0°C for 1 minute. DIEA (4.37 mL, 26.41 mmol) was then added dropwise. The mixture was then brought to room temperature and reacted with stirring. After the reaction was completed, methyl tert-butyl ether (200 mL) was added for precipitation. A solid precipitated and was filtered, and dried to provide 2.5 g of the product.

19.6 Synthesis of **84-98**

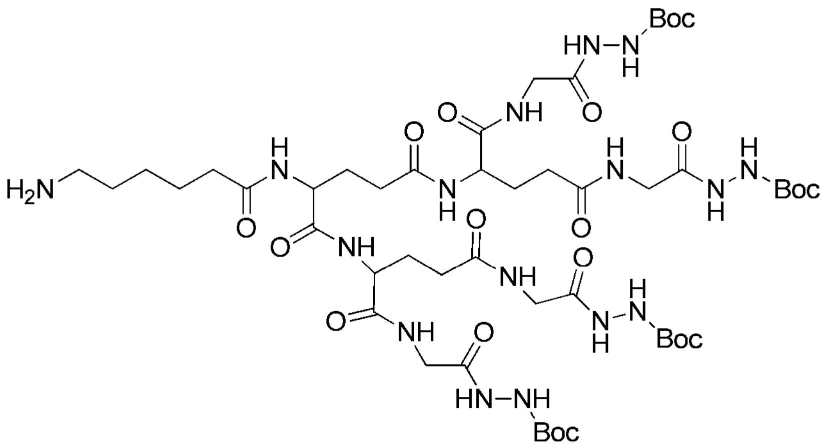

**84-74** (2.5 g, 1.75 mmol) was placed in a 500 mL round-bottom flask, then THF (15 mL) was added for dissolution with the assistance of ultrasonication. Diethylamine (30 mL) was added, and the mixture was reacted with stirring at room temperature. After the reaction was completed, the THF and diethylamine were removed by rotary evaporation. An appropriate amount of methyl tert-butyl ether was added to the residue for precipitation. After standing, the supernatant

was discarded. An appropriate amount of a methanol/dichloromethane (1/9) mixture (20 mL) was added to the resulting solid for dissolution, then 200-300 mesh silica gel powder (10 g) was added, and the mixture was evaporated to dryness. The resulting product was dry-loaded onto the column and subjected to column chromatography using 1% aqueous ammonia/10-50% methanol/dichloromethane as the eluent. The desired product was collected, concentrated, and evaporated to dryness to provide 1.58 g of the product.

19.7 Synthesis of **81-140**

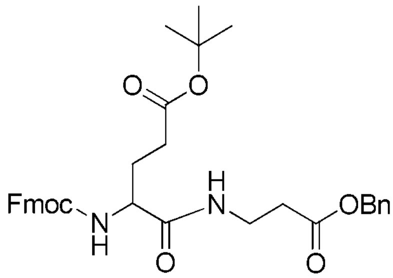

Fmoc-Glu(OtBu)-OH (8.47 g, 19.91 mmol), β-alanine benzyl ester p-toluenesulfonate (7 g, 19.91 mmol), HBTU (9.06 g, 23.9 mmol), and HOBT (3.22 g, 23.9 mmol) were added to a 500 mL round-bottom flask, then DMF (30 mL) was added for dissolution with the assistance of ultrasonication, and the mixture was stirred at 0°C for 2 minutes. DIEA (11.51 mL, 69.68 mmol) was then added dropwise. The mixture was brought to room temperature and reacted with stirring. After the reaction was completed, the reaction mixture was poured into a 1 L separatory funnel containing purified water (200 mL) and ethyl acetate (200 mL) for extraction. The organic phase was collected, and the aqueous phase was further extracted with ethyl acetate (100 mL × 2). The organic phases were combined, washed with saturated sodium bicarbonate solution (200 mL × 2). The resulting organic phase was collected, dried over anhydrous magnesium sulfate, concentrated, and evaporated to dryness to provide 11.68 g of the product.

19.8 Synthesis of **84-65**

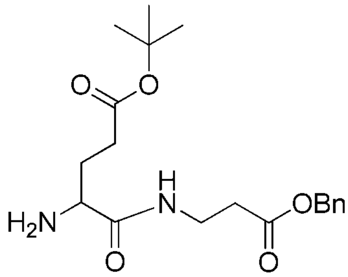

**81-140** (11.68 g, 19.91 mmol) was placed in a 500 mL round-bottom flask, then acetonitrile (30 mL) was added for dissolution with the assistance of ultrasonication. Diethylamine (41 mL, 398.2 mmol) was added, and the mixture was stirred at room temperature. After the reaction was completed, the acetonitrile and diethylamine were removed using a rotary evaporator. An appropriate amount of dichloromethane was added to the mixture for dissolution, then 200-300 mesh silica gel powder (30 g) was added, and the mixture was evaporated to dryness. The resulting product was dry-loaded onto the column and subjected to column chromatography using 1% ammonia/2% methanol/dichloromethane as the eluent. The desired product was collected, concentrated, and evaporated to dryness to provide 6.8 g of the product.

19.9 Synthesis of **84-67**

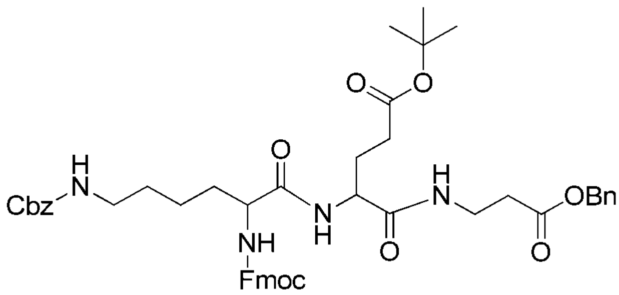

**84-65** (6.8 g, 19.40 mmol), N'-Fmoc-N-benzyloxycarbonyl-L-lysine (9.7 g, 19.40 mmol), HBTU (9.56 g, 25.22 mmol), and HOBT (3.4 g, 25.22 mmol) were placed in a 250 mL round-bottom flask, then DMF (20 mL) was added for dissolution with the assistance of ultrasonication, and the mixture was stirred at 0°C for 2 minutes. DIEA (9.6 mL, 58.21 mmol) was then added dropwise. The mixture was brought to room temperature and reacted with stirring. After the reaction was completed, the reaction mixture was poured into a 1 L separatory funnel containing pure water (200 mL) and ethyl acetate (200 mL) for extraction. The organic phase was collected, and the aqueous phase was further extracted with ethyl acetate (100 mL × 2). The organic phases were combined, washed with saturated sodium bicarbonate solution (200 mL × 2), dried over anhydrous magnesium sulfate, concentrated. 200-300 mesh silica gel powder (25 g) was added, and the mixture was evaporated to dryness. The resulting product was dry-loaded onto the column and subjected to column chromatography using 1-3% methanol/dichloromethane as the eluent. The desired product was collected, concentrated, and evaporated to dryness to provide 26.21 g of the product.

19.10 Synthesis of **84-68**

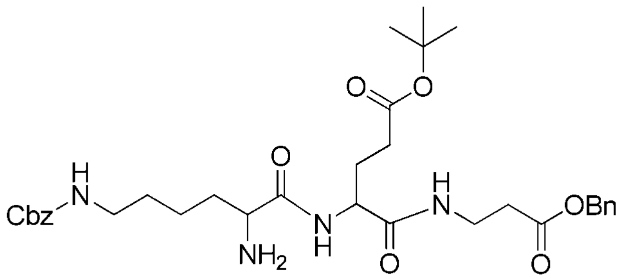

**84-67** (16.2 g, 19.40 mmol) was placed in a 500 mL round-bottom flask, then acetonitrile (30 mL) was added for dissolution with the assistance of ultrasonication. Diethylamine (40 mL, 388.07 mmol) was added, and the mixture was reacted with stirring at room temperature. After the reaction was completed, acetonitrile and diethylamine were removed using a rotary evaporator. An appropriate amount of dichloromethane was added to the residue for dissolution, then 200-300 mesh silica gel powder (40 g) was added, and the mixture was evaporated to dryness. The resulting product was dry-loaded onto the column and subjected to column chromatography using 1-4% methanol/dichloromethane as the eluent. The desired product was collected, concentrated, and evaporated to dryness to provide 11.8 g of the product.

19.11 Synthesis of **81-135**

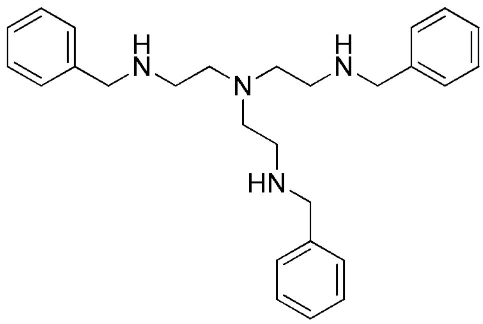

Tris(2-aminoethyl)amine (2 g, 13.67 mmol) was added to a 500 mL round-bottom flask, then dichloromethane (30 mL) was added for dissolution with the assistance of ultrasonication. Anhydrous sodium sulfate (6.41 g, 45.13 mmol) and benzaldehyde (4.78 g, 45.13 mmol) were added, and the mixture was reacted with stirring at room temperature for 18 hours. The mixture was then filtered, and the filtrate was concentrated, then methanol (15 mL) was added, and the mixture was cooled to 0°C. Sodium borohydride (2.06 g, 54.68 mmol) was added in batches with small amounts at 0°C and reacted for 1 hour. Pure water (5 mL) was then added, and the mixture was concentrated on a rotary evaporator. Ethyl acetate (30 mL) was added for dissolution, and the mixture was washed with 1N HCl solution (20 mL × 2). The organic phase was collected and neutralized with 3N NaOH solution, and the pH was adjusted to alkaline. The organic phase was collected and dehydrated with anhydrous magnesium sulfate, then 200-300 mesh silica gel powder (8 g) was added, and the mixture was evaporated to dryness. The resulting product was dry-loaded onto the column and subjected to column chromatography using 1% ammonia/2-3% methanol/dichloromethane as the eluent. The desired product was collected, concentrated, and evaporated to dryness to provide 2.62 g of the product with a yield of 46%. $^1$H-NMR (600 MHz, $CDCL_3$) δ7.39-7.18 (m, 15H), 3.78 (s, 6H), 2.71 (t, *J* = 6.0 Hz, 6H), 2.62 (t, *J* = 6.0 Hz, 6H), 1.89 (s, 3H); FT-MS ESI m/z [M+H$^+$] 417.29

19.12 Synthesis of **81-143**

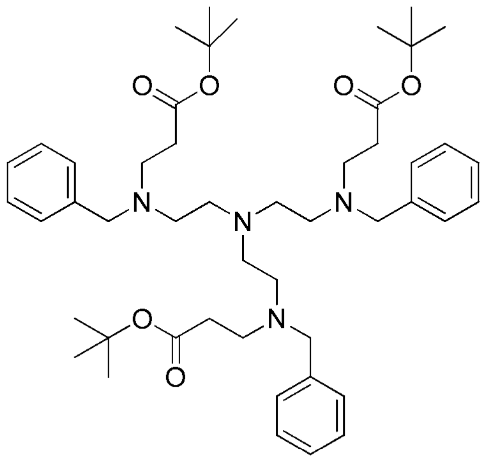

**81-135** (2.25 g, 5.4 mmol) and tert-butyl acrylate (2.76 g, 21.6 mmol) were added to a 100 mL round-bottom flask, then methanol (15 mL) was added for dissolution with the assistance of ultrasonication, and the mixture was reacted with stirring and nitrogen protection at room temperature. After the reaction was completed, 200-300 mesh silica gel powder (15 g) was added, and the mixture was evaporated to dryness. The resulting product was dry-loaded onto the column and subjected to column chromatography using 40% ethyl acetate/petroleum ether as the eluent. The desired product was collected, concentrated, and evaporated to dryness to provide 3.04 g of the product with a yield of 70.37%. $^1$H-NMR (600 MHz, $CDCL_3$) δ 7.38-7.14 (m, 15H), 3.53 (s, 6H), 2.74 (t, *J* = 7.2 Hz, 6H), 2.47-2.38 (m, 12H), 2.36 (t, *J* = 7.2 Hz, 6H), 1.46 (s, 27H); FT MS ESI m/z [M+H$^+$] 801.55

19.13 Synthesis of **81-144**

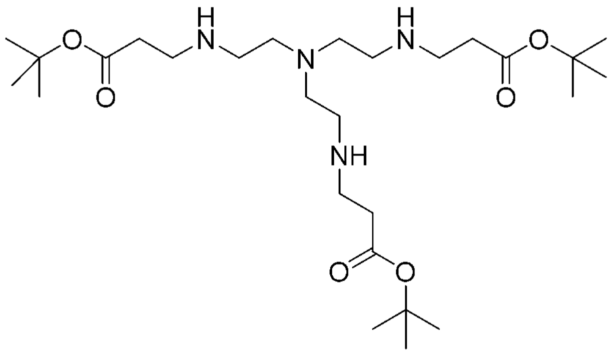

Raw material **81-143** (2 g, 2.49 mmol) and 10% Pd/C catalyst (150 mg) were added to a hydrogenation reactor, and then methanol (15 mL) was added for dissolution. The hydrogenation reactor was sealed, evacuated with a water pump, and then filled with hydrogen gas. This process was repeated three times. Finally, the hydrogen pressure was adjusted to 2 MPa, and the reaction was carried out with stirring at 30°C for 24 hours. After the reaction was completed, the mixture was filtered using a Buchner funnel containing filter paper. The filtrate was collected and concentrated to provide 1.32 g of the product. $^1$H-NMR (600 MHz, $CDCL_3$) δ 2.78-2.71 (m, 6H), 2.61-2.54 (m, 6H), 2.52-2.44 (m, 6H), 2.39-2.31 (m, 6H), 2.20 (s, 3H), 1.37 (s, 27H); FT MS ESI m/z [M+H$^+$] 531.40

19.14 Synthesis of **81-145**

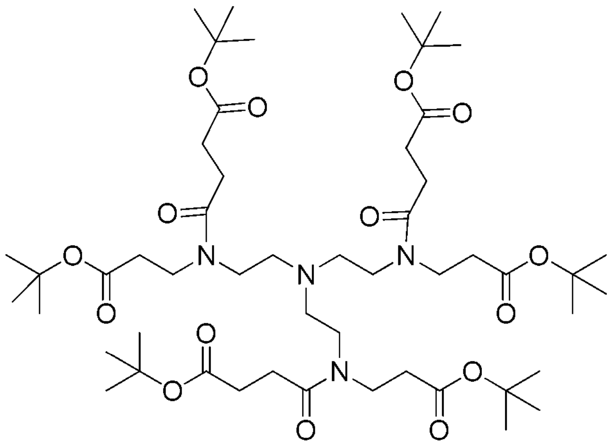

**81-144** (1 g, 1.88 mmol), mono-tert-butyl succinate (1.14 g, 6.59 mmol), HBTU (2.49 g, 6.59 mmol), and HOBT (0.89 g, 6.59 mmol) were added to a 100 mL round-bottom flask, then DMF (15 mL) was added to dissolve the mixture with the assistance of ultrasonication, and the mixture was stirred at 0°C for 1 minute. DIEA (3.72 mL, 22.56 mmol) was then added dropwise. The mixture was then brought to room temperature and reacted with stirring. After the reaction was completed, the mixture was poured into a separatory funnel containing pure water (100 mL) and ethyl acetate (100 mL) for extraction. The organic phase was separated, and the aqueous phase was extracted again with ethyl acetate (100 mL × 2). The organic phases were combined and washed with saturated sodium bicarbonate solution (50 mL × 2). The resulting organic phase was collected, dried over anhydrous magnesium sulfate, and concentrated. 200-300 mesh silica gel powder (8 g) was added to the residue, and the mixture was evaporated to dryness. The resulting product was dry-loaded onto the column and subjected to column chromatography using 40-60% ethyl acetate/petroleum ether as the eluent. The desired product was collected, concentrated, and evaporated to dryness to provide 1.65 g of the product. $^1$H-NMR (600 MHz, $CDCL_3$) δ 3.58-3.50 (m, 4H), 3.49-3.44 (m, 2H), 3.44-3.36 (m, 2H), 3.36-3.26 (m, 4H), 2.69-2.61 (m, 2H), 2.61-2.38 (m, 22H), 1.38-1.33 (m, 54H)

19.15 Synthesis of **81-148**

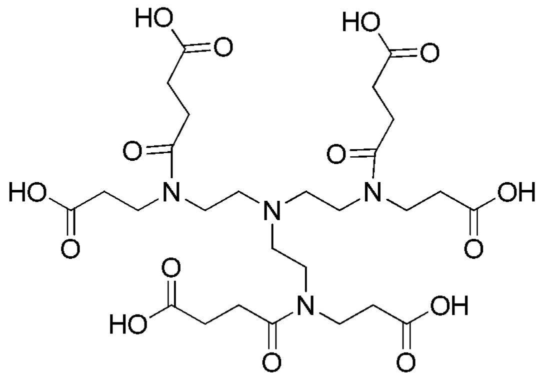

**81-145** (1.65 g, 1.65 mmol) was placed in a 500 mL round-bottom flask, then TFA (15 mL) was added, and the mixture was reacted with stirring at room temperature. After the reaction was completed, an appropriate amount of dichloromethane was added to the mixture, and then the dichloromethane and most of the TFA were removed using a rotary evaporator. Then, an appropriate amount of dichloromethane was added, and the dichloromethane and TFA were removed using a rotary evaporator. This process was repeated several times until the product became a viscous oil. An appropriate amount of dichloromethane was added to the resulting product for dissolution, then DIEA was added to adjust the pH to alkaline. The mixture was concentrated under reduced pressure and evaporated to dryness to provide 1.09 g of the product.

19.16 Synthesis of **81-149**

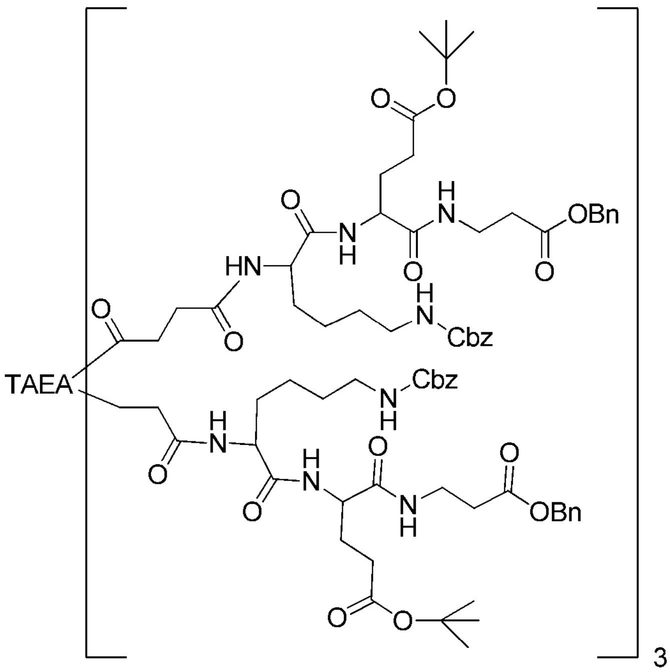

**81-148** (1 g, 1.5 mmol), **84-68** (7.04 g, 11.55 mmol), HBTU (4.38 g, 11.55 mmol), and HOBT (1.56 g, 11.55 mmol) were added to a 100 mL round-bottom flask, then DMF (10 mL) was added for dissolution with the assistance of ultrasonication, and the mixture was stirred at 0°C for 1 minute. DIEA (9.81 mL, 59.4 mmol) was then added dropwise. The mixture was brought to room temperature and reacted with stirring. After the reaction was completed, methyl tert-butyl ether (200 mL) was added for precipitation. A solid precipitated and was filtered. The filter cake was dissolved in an appropriate amount of a methanol/dichloromethane (1/4) mixture, then 200-300 mesh silica gel powder (20 g) was added, and the mixture was evaporated to dryness. The resulting product was dry-loaded onto the column and subjected to column chromatography using 3-5% methanol/dichloromethane as the eluent. The desired product was collected, concentrated, and evaporated to dryness to provide 0.91 g of the product. $^1$H-NMR (600 MHz, DMSO-$d_6$) δ 8.42-7.55

(m, 20H), 7.54-6.70 (m, 64H), 5.31-4.63 (m, 24H), 4.37-3.88(m, 6H), 3.59-3.03 (m, 28H), 3.03-2.83(m, 23H),2.81-2.66 (m, 13H), 2.64-2.25 (m, 24H), 2.23-2.01 (m, 14H), 2.01-1.75 (m, 12H), 1.74-1.41 (m, 12H), 1.41-0.98 (m, 66H)

19.17 Synthesis of **81-152**

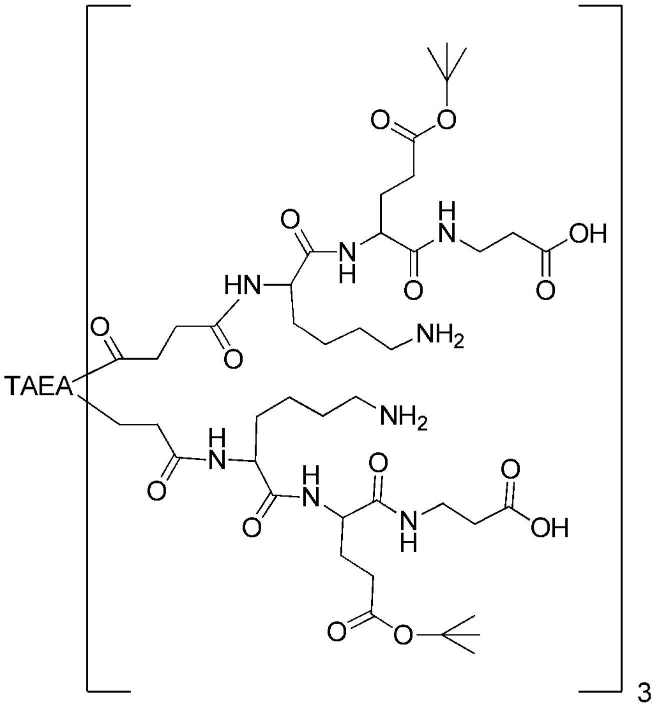

Raw material **81-149** (0.91 g, 0.21 mmol) and 10% Pd/C catalyst (150 mg) were added to a hydrogenation reactor, then methanol (20 mL) was added for dissolution. The hydrogenation reactor was sealed, evacuated with a water pump, and then filled with hydrogen gas. This process was repeated three times. The hydrogen pressure was adjusted to 2 MPa, and the reaction was carried out with stirring at room temperature. After the reaction was completed, the mixture was filtered through a Buchner funnel containing filter paper. The reactor was rinsed with methanol (5 mL × 3). The filtrate was collected, concentrated under reduced pressure, and evaporated to dryness to provide 0.62 g of the product.

19.18 Synthesis of **81-155**

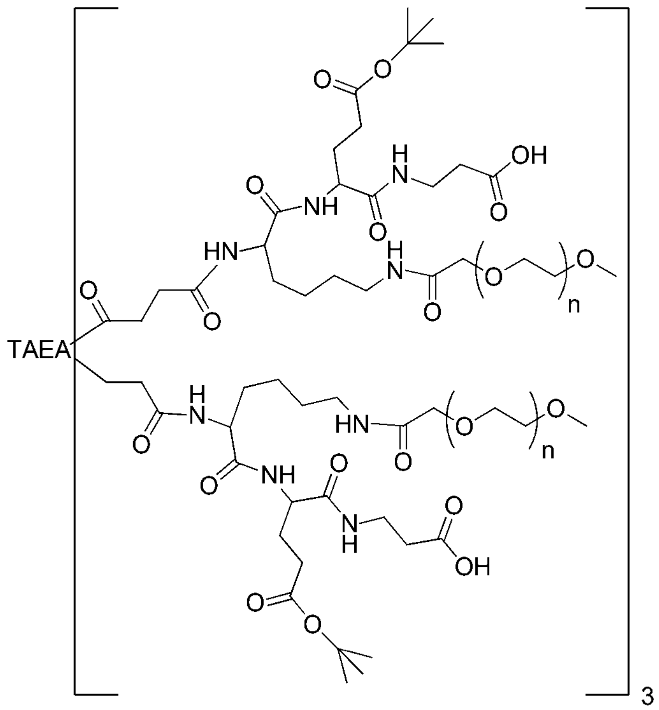

**81-152** (0.55 g, 0.185 mmol) was placed in a 500 mL round-bottom flask, then extra-dry DMF (18 mL) was added for dissolution with the assistance of ultrasonication, and the mixture was stirred at 0°C for 5 minutes. DIEA (3.66 mL, 22.2 mmol) was added dropwise, followed by the addition of M-SCM-5000 (5.9 g, 1.12 mmol, purchased from Jenkem, Lot Number: ZZ403P152). The air in the flask was replaced with nitrogen gas, and the reaction was carried out with stirring at 30°C in the dark. After the reaction was completed, methyl tert-butyl ether (200 mL) was added for precipitation. A solid precipitated and was filtered, then 100-200 mesh silica gel powder (20 g) was added, and the mixture was evaporated to dryness. The resulting product was dry-loaded onto the column and subjected to column chromatography using 1% ammonia water/5-10% methanol/dichloromethane as the eluent. The silica gel powder containing the uneluted product in the column chromatography was then dissolved with DMF, then an appropriate amount of methyl tert-butyl ether was added for precipitation. A solid precipitated and was filtered. The solution containing the product and the filter cake were collected, and dried to provide 4 g of the product. $^1$H-NMR (600 MHz, DMSO-$d_6$) $\delta$ 12.14-11.82 (m, 6H), 8.28-7.85 (m, 16H), 7.81-7.62 (m,8H), 4.49-3.83 (m, 16H), 3.65-3.00 (m, 2826H), 3.00-2.82 (m, 23H), 2.82-2.64 (m, 13H), 2.61-2.38 (m, 12H), 2.38-2.03 (m, 11H), 2.03-1.75 (m, 19H), 1.75-1.50 (m, 15H), 1.47-0.93 (m, 77H); MALDI-TOF M structures m/z 33887

19.19 Synthesis of **81-165**

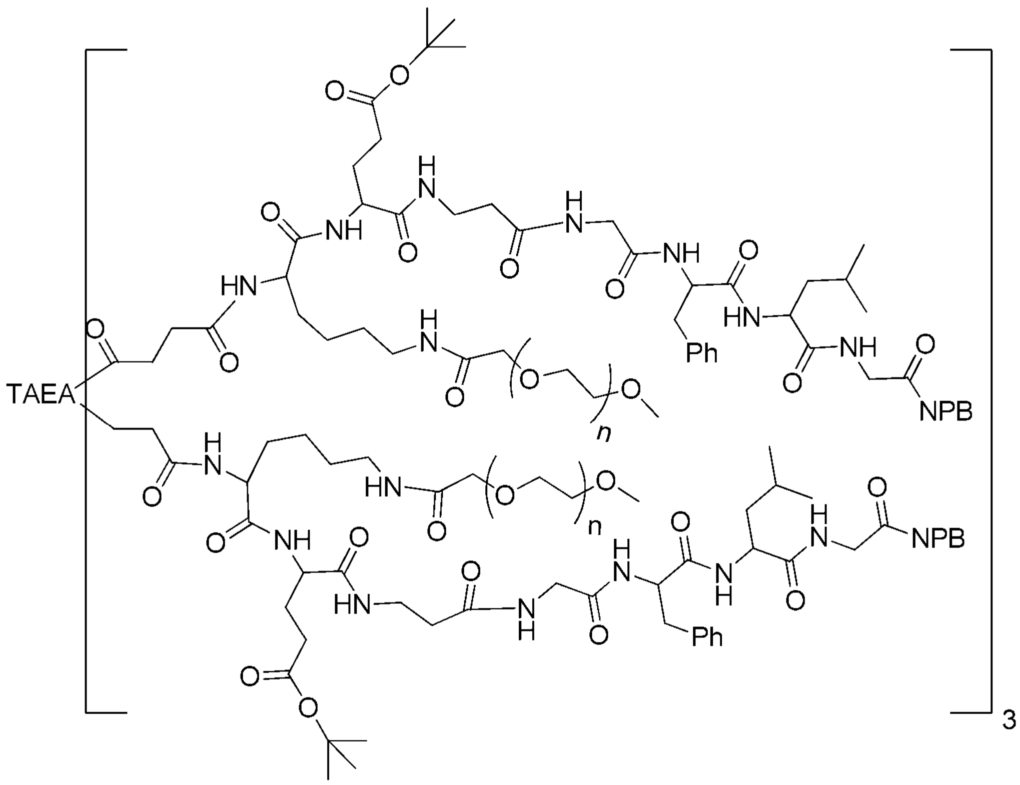

**81-155** (3 g, 0.088 mmol), **73-85** (1.84 g, 2.65 mmol), HBTU (1.00 g, 2.65 mmol), and HOBT (0.35 g, 2.65 mmol) were added to a 250 mL round-bottom flask, then DMF (20 mL) was added for dissolution with the assistance of ultrasonication, and the mixture was stirred at 0°C for 2 minutes. DIEA (0.87 mL, 5.31 mmol) was then added dropwise. The mixture was brought to room temperature and reacted with stirring. After the reaction was completed, methyl tert-butyl ether (200 mL) was added for precipitation. A solid precipitated and was filtered. The filter cake was dissolved, then 100-200 mesh silica gel powder (15 g) was added, and the mixture was evaporated to dryness. The resulting product was dry-loaded onto the column and subjected to column chromatography using 1% ammonia water/5-8% methanol/dichloromethane as the eluent. The desired product was collected, concentrated, and evaporated to dryness to provide 1.91 g of the product. $^1$H-NMR (600MHz, DMSO-$d_6$) δ9.31-9.22 (m, 6H), 8.64-8.49 (m, 6H), 8.42-8.31 (m, 6H), 8.28-7.83 (m, 76H), 7.81-7.44 (m, 20H), 7.44-7.21 (m, 36H), 4.63-4.50 (m, 6H), 4.49-3.79(m, 36H), 3.65-3.00 (m, 2838H), 3.00-2.82 (m, 45H), 2.82-2.64 (m, 25H), 2.61-2.38 (m, 18H),2.38-2.03 (m, 11H), 2.03-1.75 (m, 25H), 1.75-0.93 (m, 116H), 0.89-0.74 (m, 36H); MALDI-TOF M structures m/z 37943

19.20 Synthesis of **81-167**

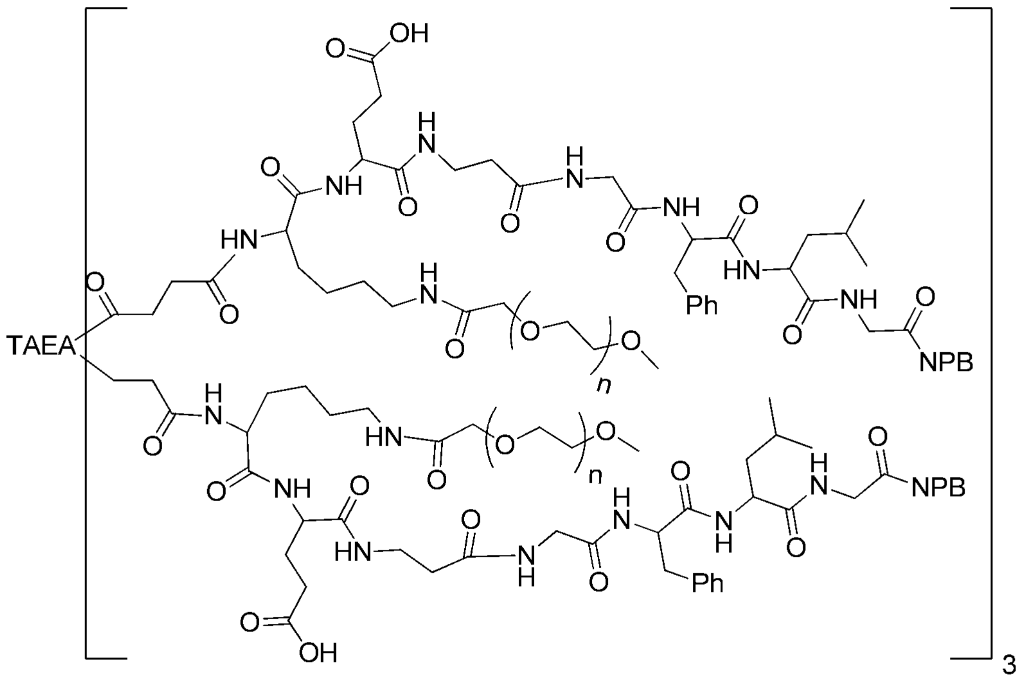

**81-165** (1.91 g, 0.05 mmol) was placed in a 500 mL round-bottom flask, then TFA (10 mL) was added, and the mixture was reacted with stirring at room temperature. After the reaction was completed, TFA was removed by rotary evaporation, and methyl tert-butyl ether (300 mL) was added for precipitation. A solid precipitated and was filtered. The filter cake was dried to provide 1.85 g of the product. $^1$H-NMR (600MHz, DMSO-$d_6$) δ 12.32-11.87 (m, 6H), 9.31-9.22 (m, 6H), 8.64-8.49 (m, 6H), 8.42-8.29 (m, 6H), 8.28-7.83 (m, 76H), 7.81-7.44 (m,20H), 7.44-7.21 (m, 36H), 4.63-4.50 (m, 6H), 4.49-3.79(m, 36H), 3.65-3.01 (m, 2838H), 3.01-2.82 (m, 45H), 2.82-2.64 (m, 25H), 2.62-2.38 (m, 18H), 2.38-2.03 (m, 11H), 2.03-1.74 (m, 25H), 1.74-0.92 (m, 62H), 0.90-0.74 (m, 36H); MALDI-TOF M structures m/z 37607

19.21 Synthesis of **81-173**

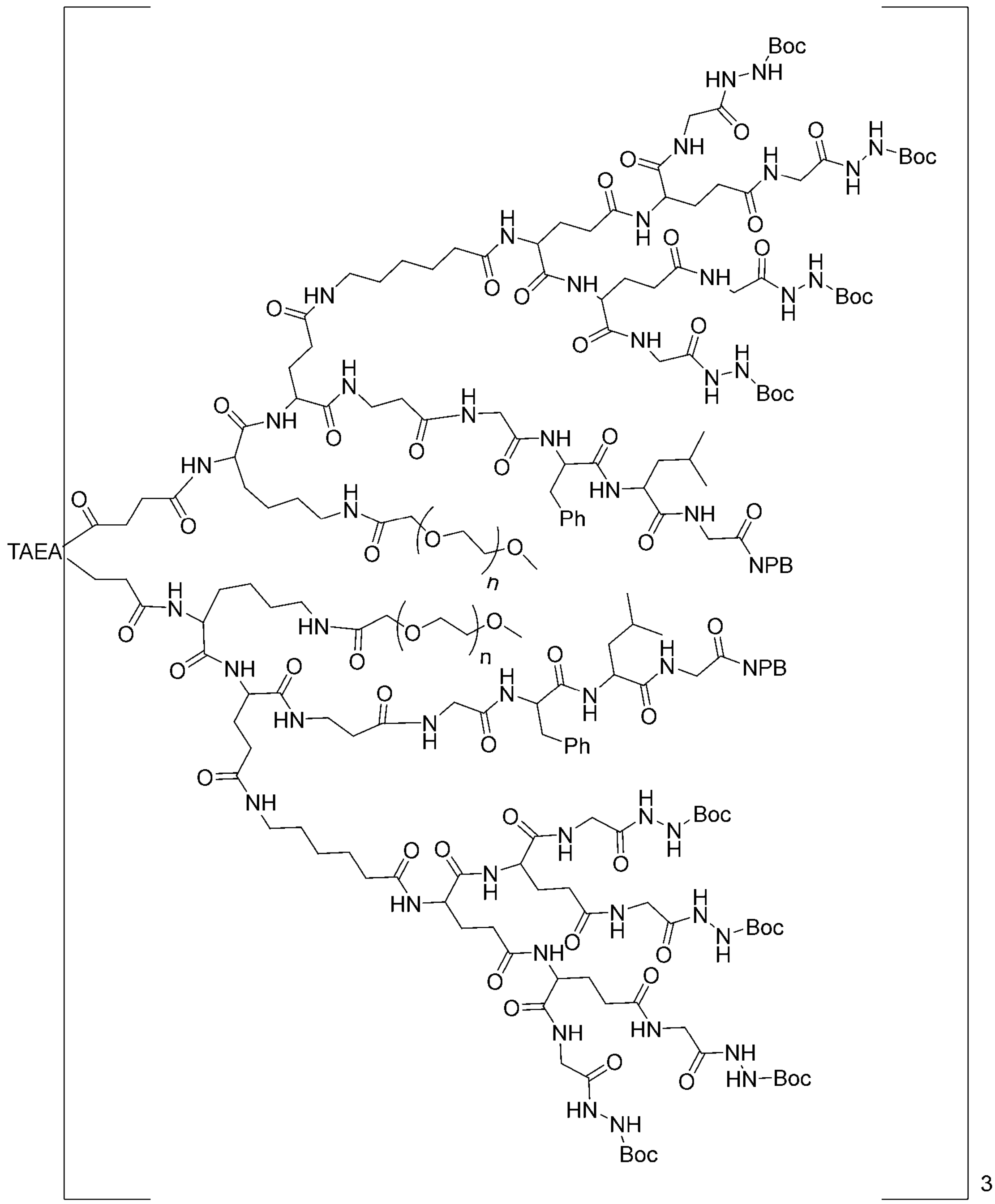

**81-167** (1.59 g, 0.04 mmol), **84-98** (0.91 g, 0.76 mmol), and HATU (0.289 g, 0.761 mmol) were added to a 250 mL round-bottom flask, then DMF (15 mL) was added for dissolution with the assistance of ultrasonication, and the mixture was stirred at 0°C for 1 minute. DIEA (0.20 mL, 1.26 mmol) was then added dropwise, and the mixture was brought to room temperature and reacted with stirring. After the reaction was completed, methyl tert-butyl ether (200 mL) was added for precipitation. A solid precipitated and was filtered, then 200-300 mesh silica gel powder (8 g) was added, and the mixture was evaporated to dryness. The resulting product was dry-loaded onto the column and subjected to column chromatography using 2% ammonia water/7% methanol/dichloromethane as the eluent. The desired product was collected, concentrated, and evaporated to dryness to provide the product. $^1$H-NMR (600 MHz, DMSO-$d_6$) δ 9.31-9.20 (m, 30H), 8.66-8.45 (m, 6H), 8.42-8.28 (m, 6H), 8.28-7.83 (m, 124H), 7.83-7.44 (m, 44H), 7.44-7.20 (m, 36H), 4.63-4.50 (m, 6H), 4.49-3.79(m, 102H), 3.65-3.01 (m, 2868H), 3.01-2.82 (m, 63H), 2.82-2.64 (m, 25H), 2.64-2.38 (m, 18H), 2.38-2.03 (m, 11H), 2.03-1.73 (m, 73H), 1.73-0.92 (m, 314H), 0.92-0.74 (m, 36H); MALDI-TOF M structures m/z 44717

19.22 Synthesis of **81-174**

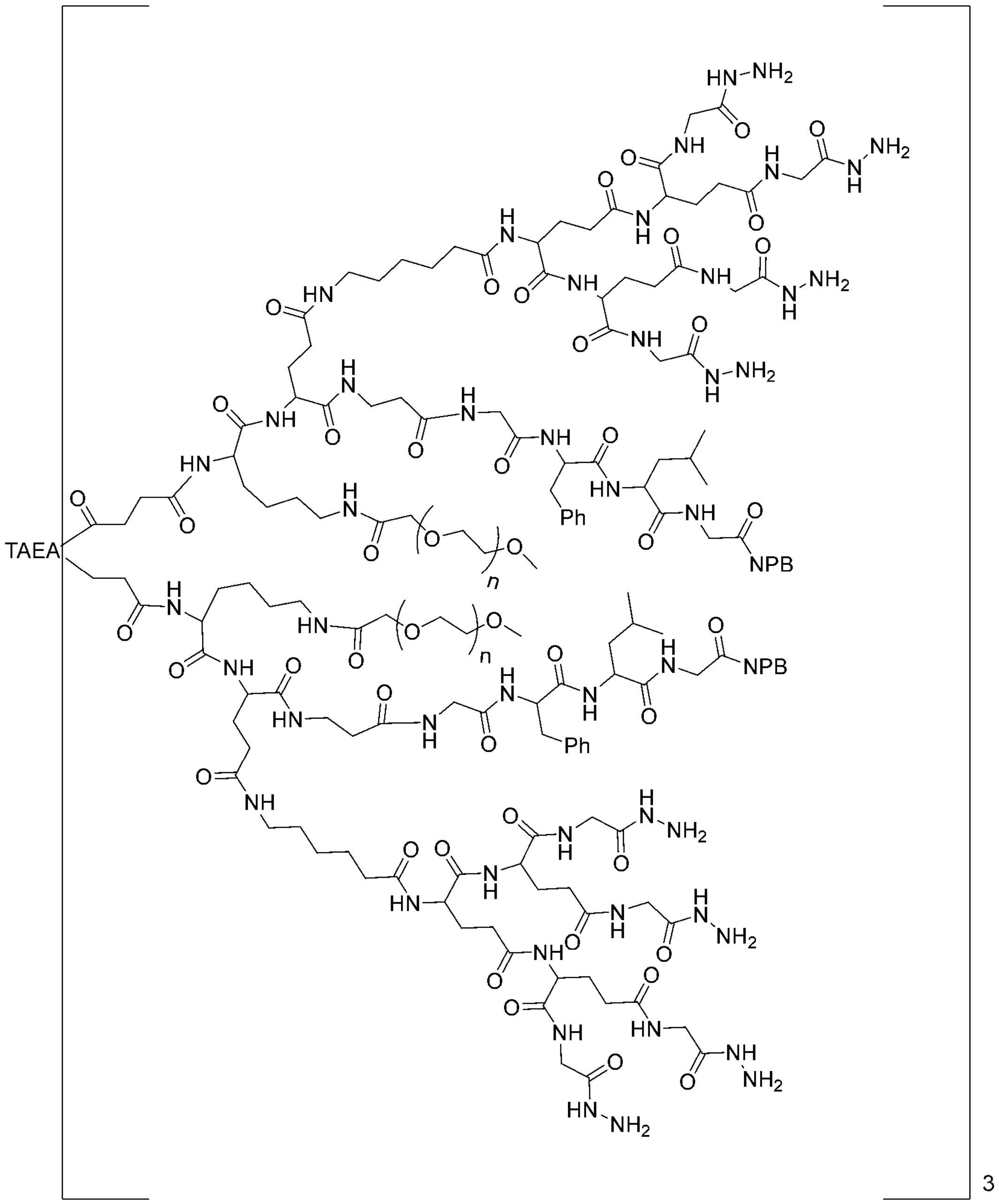

**81-173** (1.6 g, 0.035 mmol) was placed in a 500 mL round-bottom flask, then TFA (15 mL) was added, and the mixture was reacted with stirring at room temperature. After the reaction was completed, an appropriate amount of dichloromethane was added, and the mixture was evaporated under reduced pressure. Then methyl tert-butyl ether (200 mL) was added. The mixture was treated by ultrasonication and then allowed to stand. The upper layer turbid white liquid was discarded, and the resulting solid was evaporated to dryness using a rotary evaporator and then dried in a vacuum oven to provide 1.5 g of the product.

19.21 Synthesis of **81-176**

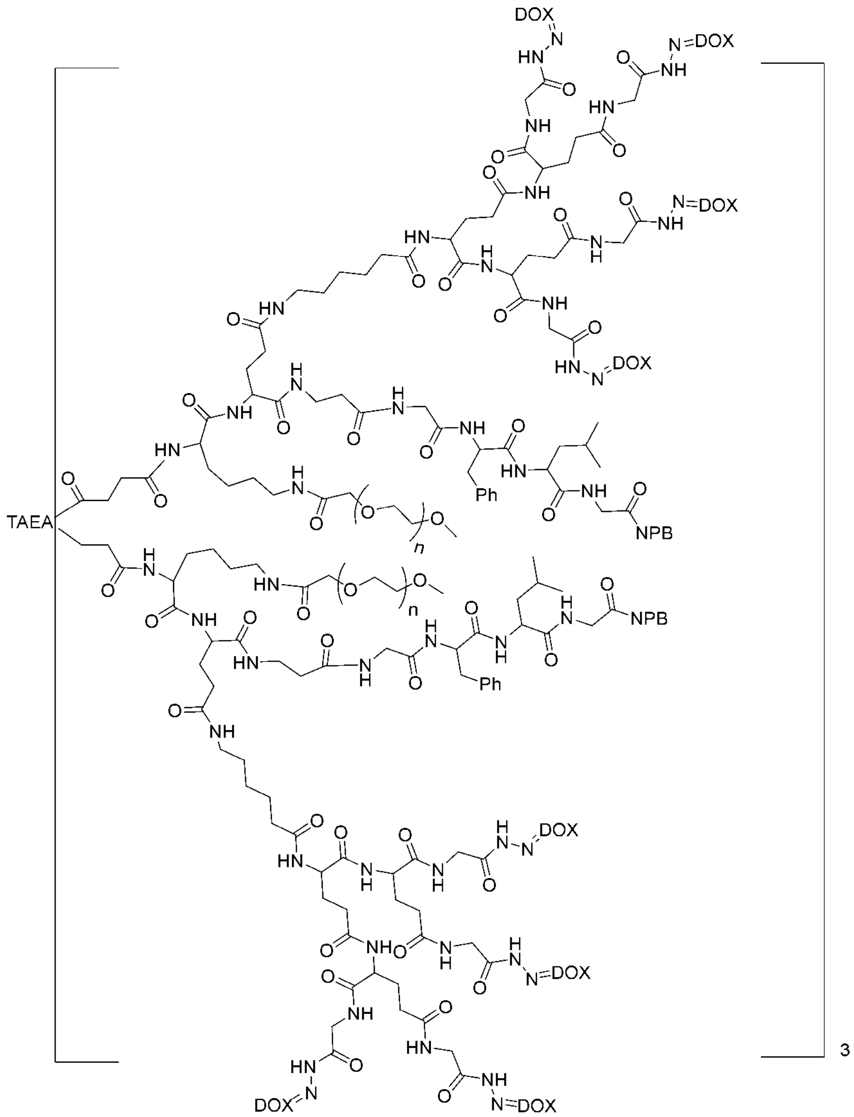

**81-174** (1.51 g, 0.0357 mmol) was added to a 100 mL round-bottom flask, then methanol (10 mL) was added for dissolution with the assistance of ultrasonication. TFA (0.2 mL) was added, and the mixture was stirred for 3 minutes. Doxorubicin hydrochloride (0.62 g, 1.07 mmol) was then added, and the mixture was reacted in the dark at room temperature. After the reaction was completed, the mixture was filtered through a Buchner funnel containing filter paper. The filtrate was collected and evaporated to dryness, an ethanol/dichloromethane (1/9) mixture (30 mL) was added for dissolution, then methyl tert-butyl ether (300 mL) was added for precipitation. A solid precipitated and was filtered. The filter cake was collected, then dissolved in an ethanol/dichloromethane mixture, and methyl tert-butyl ether was added for precipitation. This process was repeated three times. The resulting filter cake was dried in a vacuum oven to provide 1.1 g of the product. $^1$H-NMR (600 MHz, DMSO-$d_6$) δ 14.05-13.98 (m, 24H), 13.21-13.10 (m, 24H), 9.30-9.28 (m, 7H), 8.58-8.56 (m, 7H), 8.24-7.63 (m, 301H), 7.63-7.42 (m, 17H), 7.41-6.96 (m, 46H), 5.51-5.46 (m, 48H), 5.28(m, 24H), 4.94-4.89 (m, 48H), 4.63-4.40 (m, 125H), 4.39-4.01 (m, 56H), 4.01-3.60 (m, 116H), 3.59-3.43 (m, 2862H), 3.43-3.25 (m, 27H), 3.25-3.22 (m, 18H), 3.21-3.10 (m, 6H), 3.09-2.57 (m, 132H), 2.56-2.22 (m, 42H), 2.21-2.05 (m, 60H), 2.02-1.89 (m, 36H), 1.87-1.13 (m, 156H), 0.91-0.79 (m, 36H)

Example 20: Synthesis of Compound **85-71**

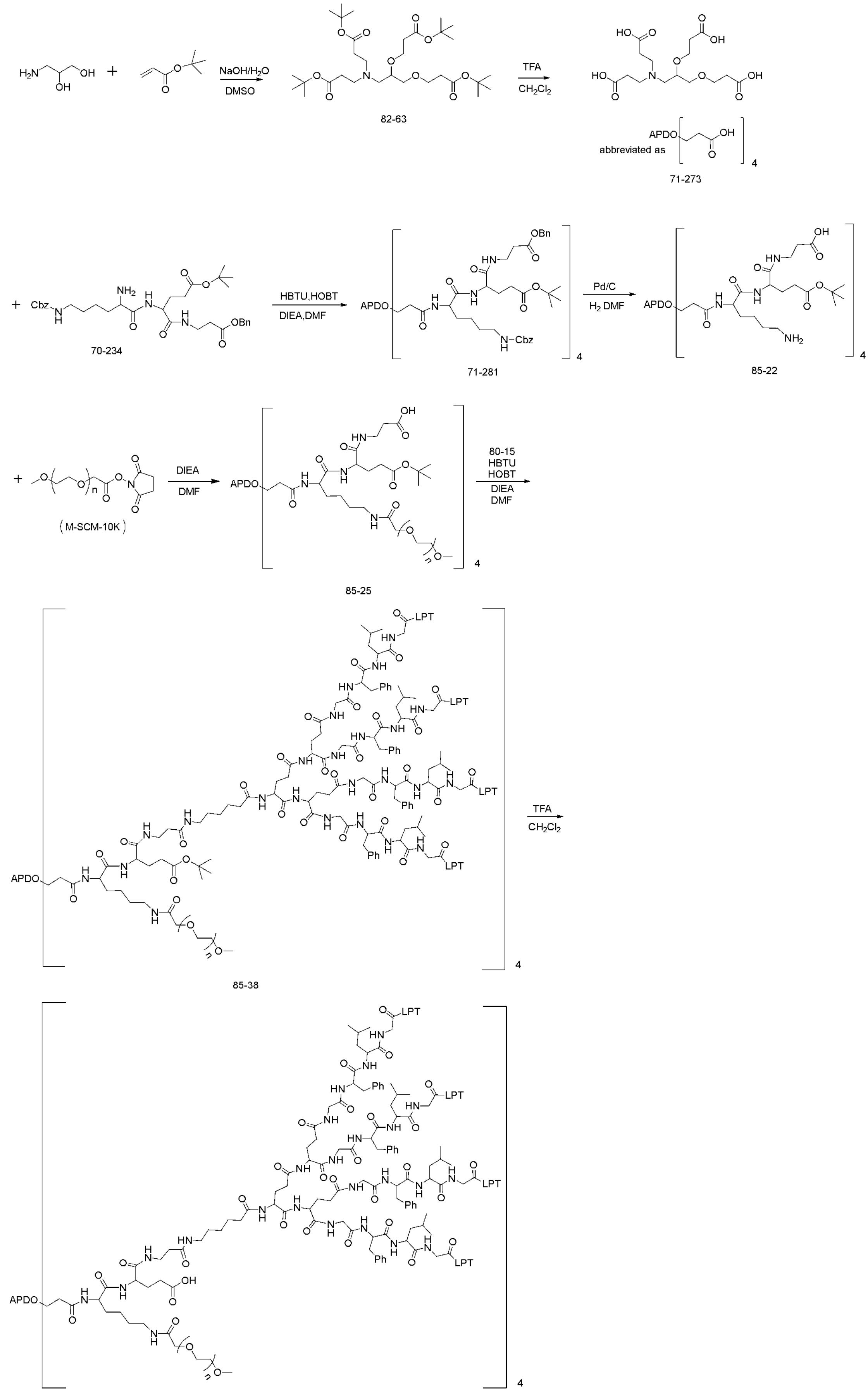


82-63

71-273

70-234

71-281

85-22

(M-SCM-10K)

85-25

85-38

85-48

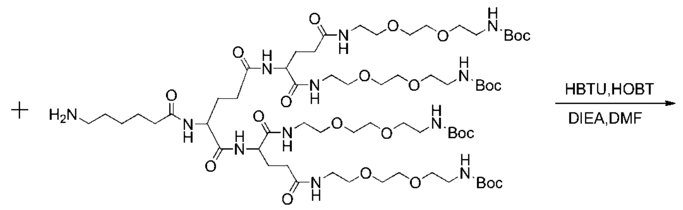


80-222

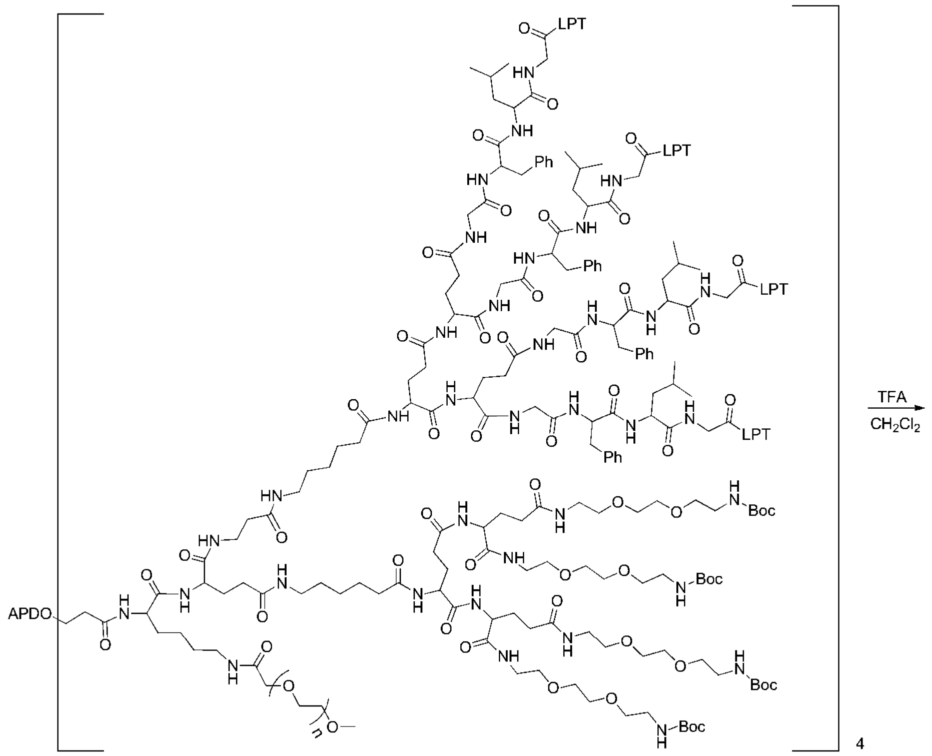


85-59

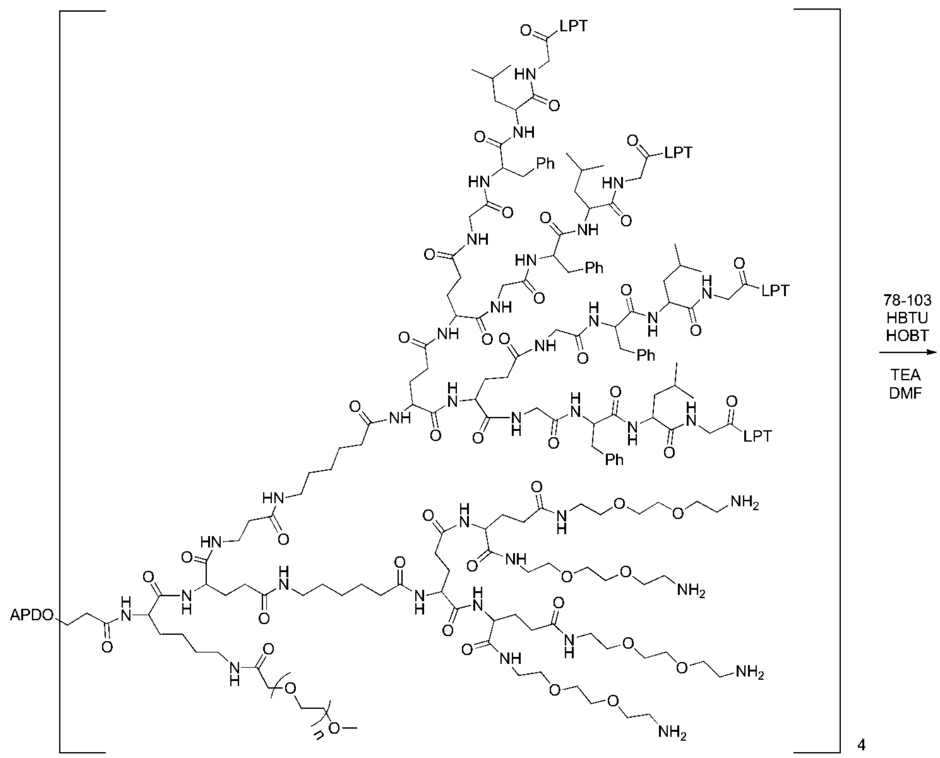


85-69

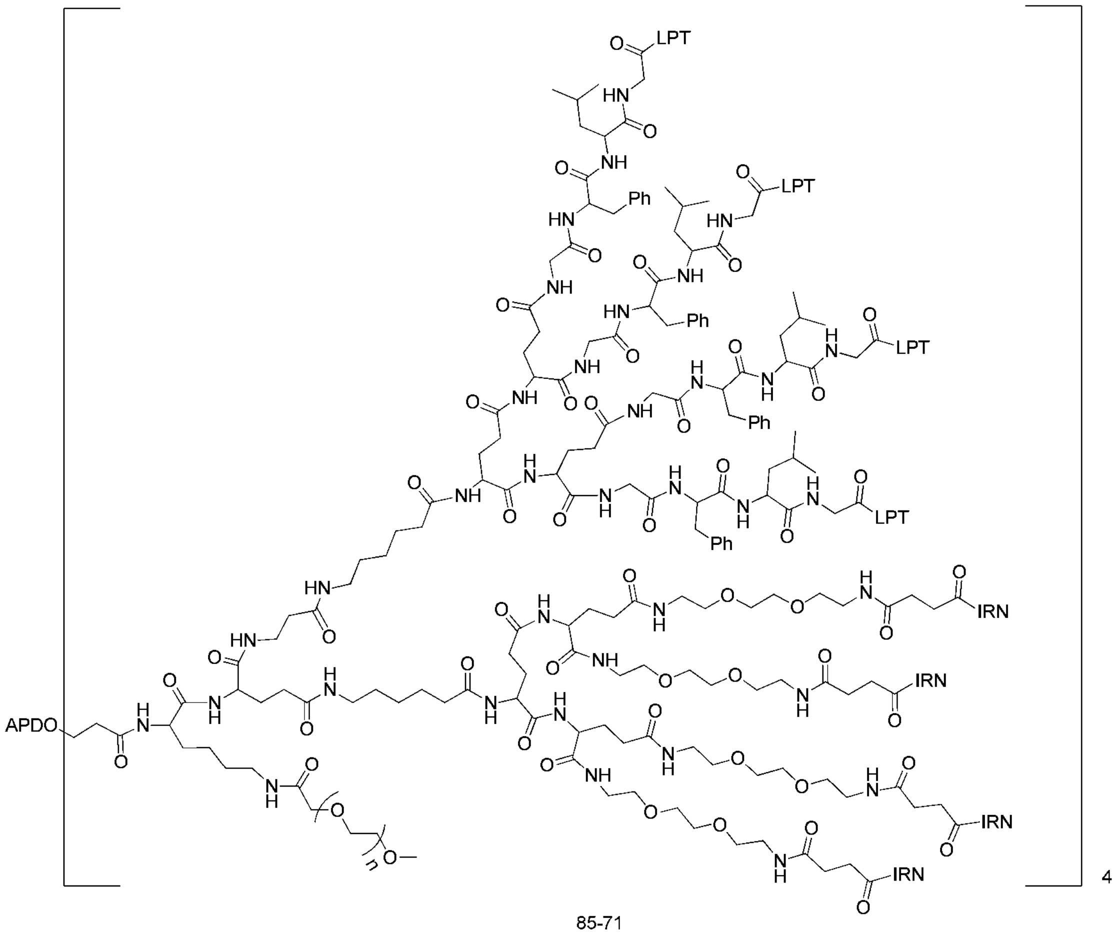

85-71

20.1 Preparation of **82-63**

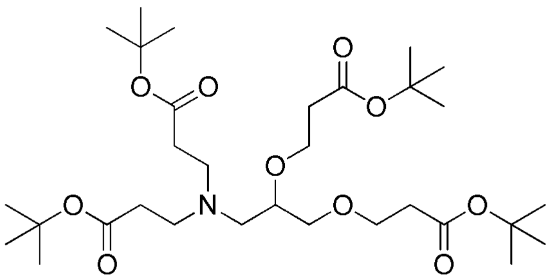

3-Amino-1,2-propanediol (1.0 g, 10.98 mmol) was weighed and added to a 500 mL flask, then DMSO (2 mL) was added, and the flask was allowed to stand at 15°C. The flask was filled with nitrogen gas, and then a 5M aqueous sodium hydroxide solution (0.2 mL) was added. After complete dissolution with the assistance of ultrasonication, tert-butyl acrylate (4.78 mL, 32.93 mmol) was slowly added dropwise. After the dropwise addition was completed, the mixture was stirred and allowed to react at room temperature overnight. After the reaction was completed, the mixture was poured into a separatory funnel containing saturated brine (200 mL) and ethyl acetate (200 mL) for extraction. The organic phase was collected, and the aqueous phase was further extracted with ethyl acetate (200 mL × 3). The organic phases were combined, evaporated to dryness to a solid, which was then dissolved in a methanol/dichloromethane (1/4) mixture. 200-300 mesh silica gel powder was added, and the mixture was evaporated to dryness to provide a powdery solid. The powdery solid was dry-loaded onto the column and subjected to column chromatography using a 5-50% ethyl acetate/petroleum ether mixture as the eluent. The desired product was collected, concentrated, and dried in a vacuum oven to provide 1.6 g of the product with a yield of 24.24%. $^{1}$H-NMR (600 MHz, DMSO-$d_6$) δ 3.71-3.53 (m, 4H), 3.42-3.39 (m, 2H), 3.32-3.31 (m, 1H), 2.70-2.56 (m, 4H), 2.43-2.31 (m, 6H), 2.30-2.23 (m, 4H), 1.38 (s, 36H); ESI [M+H$^+$] 604.40454

20.2 Preparation of **71-273**

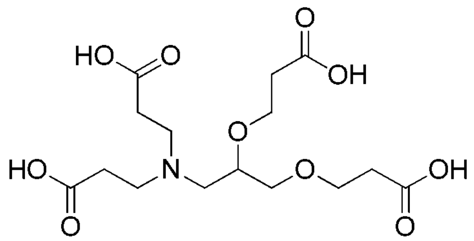

To a flask containing **82-63** (0.5 g, 0.8281 mmol), dichloromethane (10 mL) was added for dissolution with the assistance of ultrasonication, then TFA (5 mL) was added, and the mixture was reacted with stirring at room temperature overnight. After the reaction was completed, the reaction mixture was evaporated to an oily state using a rotary evaporator, and then methyl tert-butyl ether (60 mL) was added for precipitation. A powdery solid precipitated from the reaction liquid and was filtered. The filter cake was washed with methyl tert-butyl ether (40 mL × 6). The resulting product had poor solubility. The filter cake was collected and dried in a vacuum oven to provide 0.52 g of the product. ESI [M+H$^+$] 380.15351

20.3 Preparation of **71-281**

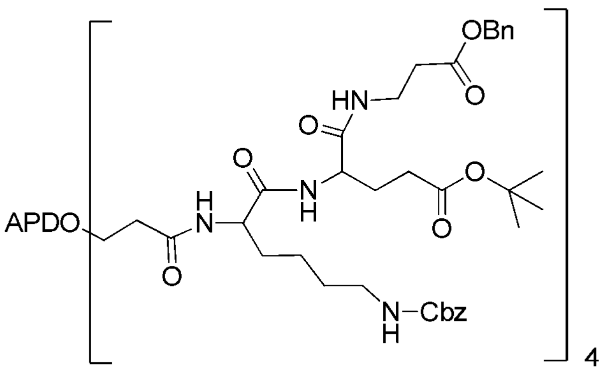

**71-273** (0.25 g, 0.6624 mmol), HBTU (1.50 g, 3.9744 mmol), and HOBT (0.53 g, 3.9744 mmol) were weighed and added to a flask containing **70-234** (1.82 g, 2.9145 mmol), then DMF (20 mL) was added to dissolve the mixture, and the flask was allowed to stand in a reaction bath at 0 °C. DIEA (2.2 mL, 13.248 mmol) was slowly added dropwise. After the dropwise addition was completed, the reaction was continued for half an hour, the flask was then taken out, and the reaction was carried out with stirring at room temperature. After the reaction was completed, the mixture was poured into a separatory funnel containing saturated brine (200 mL) and ethyl acetate (200 mL) for extraction. After standing for phase separation, the organic phase was collected, and the aqueous phase was further extracted with ethyl acetate (200 mL). The organic phases were combined and washed with deionized water (200 mL × 2). The resulting organic phase was collected, and evaporated to dryness to provide a solid product, which was dissolved in a methanol/dichloromethane (1/4) mixture, then silica gel powder was added, and the mixture was evaporated to dryness to provide a powdery solid. The powdery solid was dry-loaded onto the column and subjected to column chromatography using a 1-5% methanol/dichloromethane mixture as the eluent. The desired product was collected, concentrated, evaporated to dryness, and dried in a vacuum oven to provide 1.27 g of the product with a yield of 69.78%. $^1$H-NMR (600 MHz, DMSO-$d_6$) δ 8.03-7.95 (m, 12H), 7.36-7.29 (m, 40H), 7.19 (d, *J* = 5.3 Hz, 4H), 5.07 (s, 8H), 4.98 (s, 8H), 4.24-4.15 (m, 8H), 3.65 (d, *J* = 6.1 Hz, 2H), 3.58-3.54 (m, 2H), 3.42-3.36 (m, 6H), 3.24 (td, *J* = 12.4, 6.2 Hz, 5H), 2.97-2.92 (m, 8H), 2.80-2.51 (m, 10H), 2.42 (ddd, *J* = 20.9, 15.1, 8.7 Hz, 10H), 2.33-2.03 (m, 18H), 1.83 (dd, *J* = 15.6, 7.8 Hz, 4H), 1.71-1.66 (m, 4H), 1.61-1.56 (m, 4H), 1.49-1.45 (m, 4H), 1.35 (s, 36H), 1.24 (dd, *J* = 12.9, 6.5 Hz, 8H); ESI [M+Na$^+$] 2835.899

20.4 Preparation of **85-22**

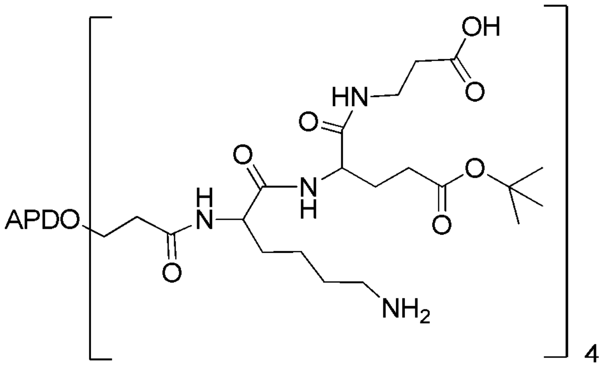

**71-281** (0.32 g, 0.1137 mmol) and 10% Pd/C catalyst (0.01 g) were placed in a hydrogenation reactor, and DMF (15 mL) was added for dissolution. The hydrogenation reactor was sealed, evacuated with a water pump, and then filled with hydrogen gas. This process was repeated three times. The pressure reading of the hydrogenation reactor was adjusted to 1.8 MPa, and the reaction was carried out with stirring at room temperature overnight. After the reaction was completed, the reaction mixture in the hydrogenation reactor was filtered and transferred to a flask. The hydrogenation reactor was then washed with DMF (5 mL × 3). The DMF solution of the product was obtained and used as the raw material for the next reaction. ESI [M+H$^+$] 1918.09912

20.5 Preparation of **85-25**

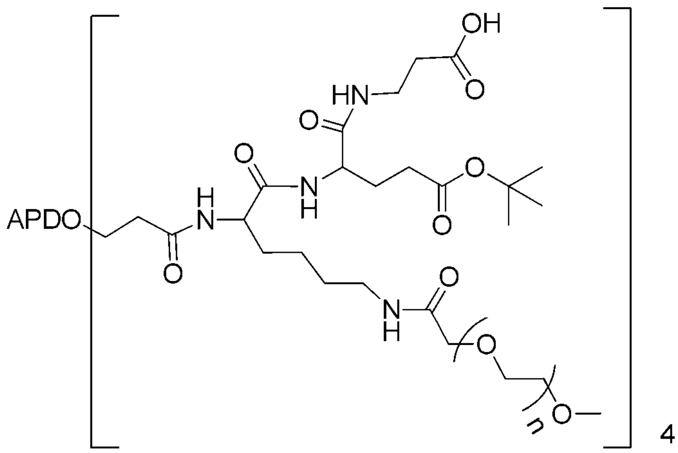

**81-22** (0.06 g, 0.0312 mmol) was placed in a 250 mL flask, then anhydrous DMF (15 mL) was added for dilution, and then DIEA (0.3 mL, 1.5647 mmol) was added dropwise. After the dropwise addition was completed, the mixture was stirred for 30 minutes. M-SCM-10K (1.33 g, 0.1251 mmol, purchased from Jenkem, Lot Number: A3016-N230101) was added and dissolved with the assistance of ultrasonication. The reaction was carried out with stirring at low speed in the dark at room temperature for one week. After the reaction was completed, n-hexane (25 mL) and methyl tert-butyl ether (200 mL) were added for precipitation. The supernatant was discarded, and n-hexane (25 mL) and methyl tert-butyl ether (200 mL) were added again for precipitation. This process was repeated three times. The mixture was filtered to provide a solid product, which was dried in a vacuum oven to provide 1.22 g of the product. $^1$H-NMR (600 MHz, DMSO-$d_6$) δ 11.39-10.94 (m, 4H), 8.20-7.79 (m, 13H), 7.73-7.60 (m, 3H), 4.25-4.13 (m, 8H), 3.87-3.84 (m, 6H), 3.537-3.475 (m, 3828H), 3.24-3.23 (m, 9H), 2.93-2.86 (m, 2H), 2.83-2.76 (m, 2H), 2.75-2.60 (m,10H), 2.39-2.31 (m, 16H), 2.29-2.13 (m, 16H), 1.87-1.79 (m, 4H), 1.73-1.65 (m, 4H), 1.64-1.55 (m, 4H), 1.52-1.44 (m, 4H), 1.393-1.356 (m, 36H), 1.27-1.19 (m, 8H)

20.6 Preparation of **85-38**

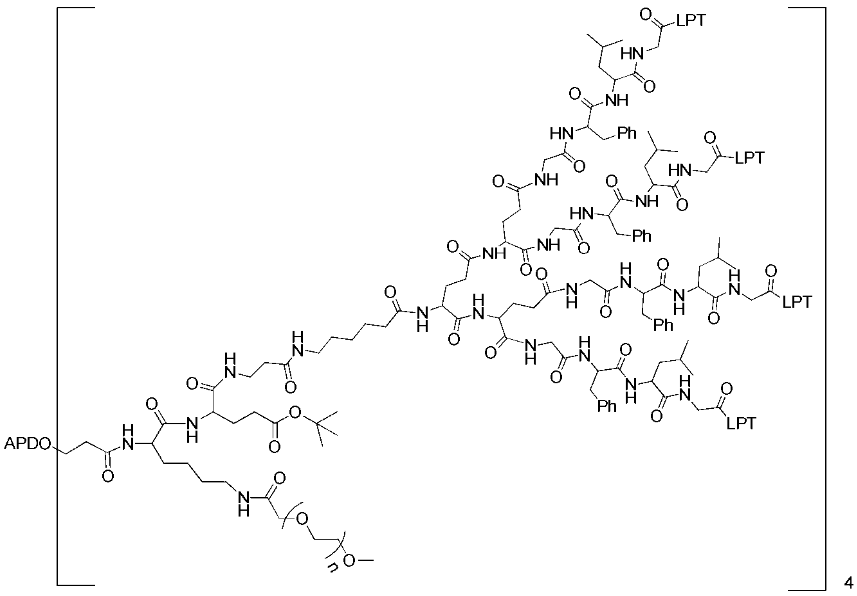

**85-25** (1.06 g, 0.0240 mmol), HBTU (0.05 g, 0.1443 mmol), and HOBT (0.019 g, 0.1443 mmol) were weighed and added to a flask containing 80-15 (0.61 g, 0.1443 mmol), then DMF (25 mL) was added to dissolve the mixture, and the mixture was stirred at room temperature for 10 minutes. DIEA (0.08 mL, 0.4810 mmol) was slowly added dropwise. After the dropwise addition was completed, the reaction was continued with stirring at room temperature. After the reaction was completed, n-hexane (25 mL) and methyl tert-butyl ether (200 mL) were added for precipitation, and the supernatant was discarded. This process was repeated three times. The mixture was filtered to provide a solid product. The solid product was dissolved in a methanol/dichloromethane (1/4) mixture, then 100-200 mesh silica gel powder was added, and the mixture was evaporated to dryness to provide a powdery solid. The powdery solid was dry-loaded onto the column and subjected to column chromatography using a 1% ammonia solution/5-9% methanol/dichloromethane mixture as the eluent. The desired product was collected, evaporated to dryness, and dried in a vacuum oven to provide 1.24 g of the product. $^1$H-NMR (600 MHz, DMSO-$d_6$) δ 8.81-8.69 (m, 18H), 8.59-8.51 (m, 16H), 8.25-7.93 (m, 130H), 7.82-7.66 (m, 34H), 7.49-7.43 (m, 16H), 7.32-7.09 (m, 160H), 6.68-6.65 (m, 10H), 5.29-5.21 (m, 32H), 4.81-4.66 (m, 34H), 4.59-4.52 (m, 17H), 4.41-4.30 (m, 24H), 4.29-4.14 (m, 58H), 3.87-3.79 (m, 29H), 3.67-3.59 (m, 102H), 3.53-3.49 (m, 3828H), 3.11-2.95 (m, 64H), 2.94-2.87 (m, 3H), 2.82-2.67 (m, 15H), 2.63-2.60 (m,2H), 2.32-2.04 (m, 36H), 1.86-1.78 (m, 8H), 1.73-1.65 (m, 10H), 1.62-1.55(m, 18H), 1.52-1.43 (m, 40H), 1.37-1.35 (m, 30H), 1.342-1.333 (m, 36H), 1.23-1.22 (m, 39H), 0.89-0.80 (m, 96H)

20.7 Preparation of **85-48**

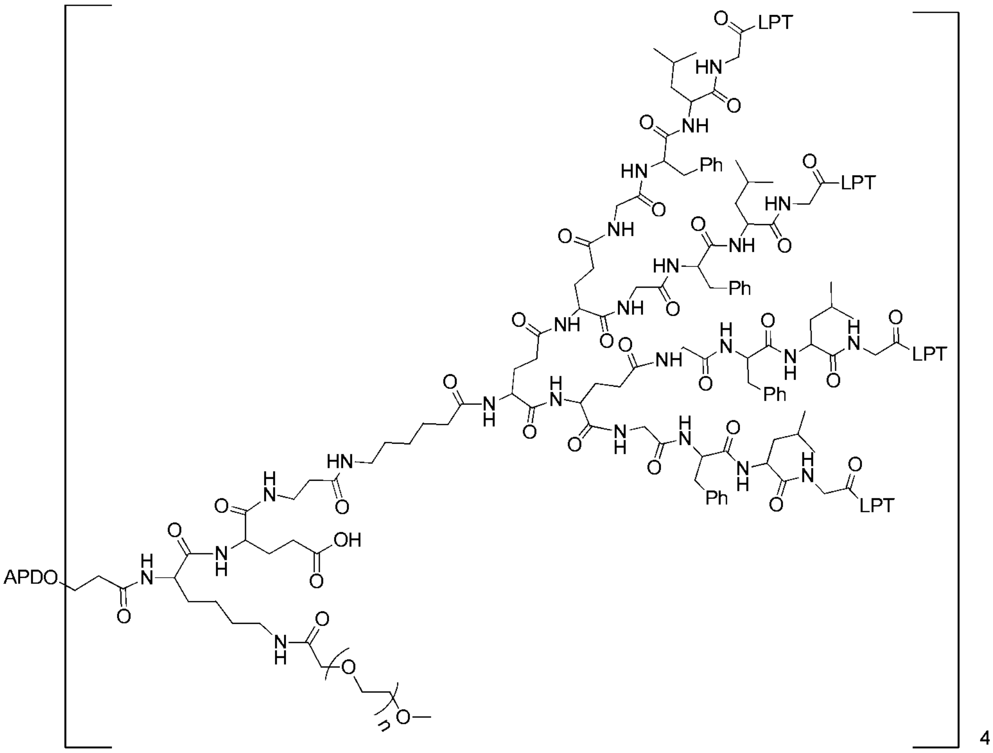

To a flask containing **85-38** (2.5 g, 0.0415 mmol), dichloromethane (3 mL) was added for dissolution with the assistance of ultrasonication, then TFA (10 mL) was added, and the mixture was reacted with stirring at room temperature overnight. After the reaction was completed, the reaction mixture was evaporated to an oily state using a rotary evaporator. Methyl tert-butyl ether (60 mL) was then added for precipitation. A powdery solid precipitated from the reaction mixture and was filtered. The filter cake was washed with methyl tert-butyl ether (40 mL × 3). The filter cake was collected and dried in a vacuum oven to provide 1.2 g of the product. $^1$H-NMR (600 MHz, DMSO-$d_6$) δ 10.00-9.79 (m, 4H), 8.87-8.69 (m, 16H), 8.66-8.53 (m, 16H), 8.29-7.96 (m, 132H), 7.84-7.76 (m, 18H), 7.74-7.76 (m, 16H), 7.50-7.40 (m, 16H), 7.32-7.09 (m, 170H), 5.32-5.19 (m, 32H), 4.82-4.64 (m, 34H), 4.60-4.54 (m, 17H), 4.40-4.31 (m, 24H), 4.29-4.13 (m, 58H), 3.87-3.81 (m, 30H), 3.67-3.59 (m, 106H), 3.53-3.49 (m, 3828H), 3.27-3.20 (m, 16H), 3.07-2.99 (m, 60H), 2.79-2.69 (m, 20H), 2.23-2.05 (m,36H), 1.87-1.78 (m, 10H), 1.76-1.66 (m, 14H), 1.64-1.55 (m, 20H), 1.53-1.29 (m, 66H), 1.27-1.14 (m, 18H), 0.87-0.77 (m, 96H)

20.8 Preparation of **85-59**

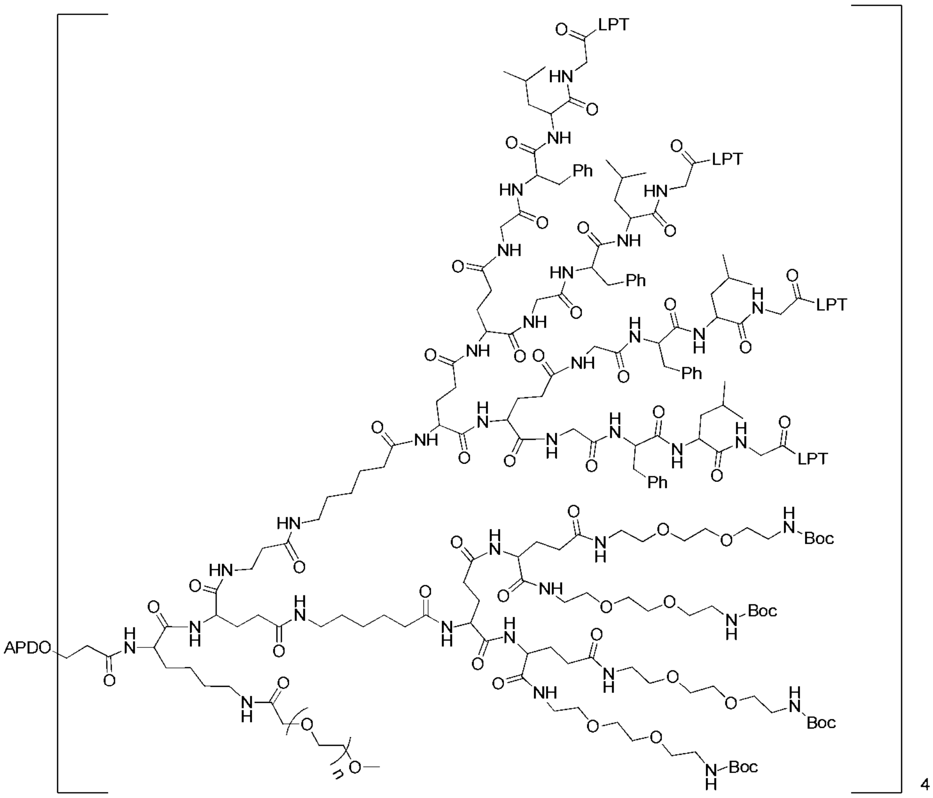

**85-48** (1.2 g, 0.0197 mmol), HBTU (0.044 g, 0.1183 mmol), and HOBT (0.015 g, 0.1183 mmol) were weighed and added to a flask containing **75-189** (0.17 g, 0.1183 mmol), then DMF (25 mL) was added to dissolve the mixture, and the mixture was stirred at room temperature for 20 minutes. DIEA (0.07 mL, 0.3944 mmol) was slowly added dropwise. After the dropwise addition, the mixture was stirred and allowed to react at room temperature overnight. After the reaction was completed, n-hexane (25 mL) and methyl tert-butyl ether (200 mL) were added for precipitation. The supernatant was discarded, and n-hexane (25 mL) and methyl tert-butyl ether (200 mL) were added again for precipitation. This process was repeated three times. The mixture was filtered to provide a solid product, which was dissolved in a methanol/dichloromethane (1/4) mixture, then 100-200 mesh silica gel powder was added, and the mixture was evaporated to dryness. The resulting product was dry-loaded onto the column and subjected to column chromatography using a 1% ammonia solution/5-9% methanol/dichloromethane mixture as the eluent. The desired product was collected, concentrated, evaporated to dryness, and dried in a vacuum oven to provide 1.0 g of the product.

20.9 Preparation of **85-69**

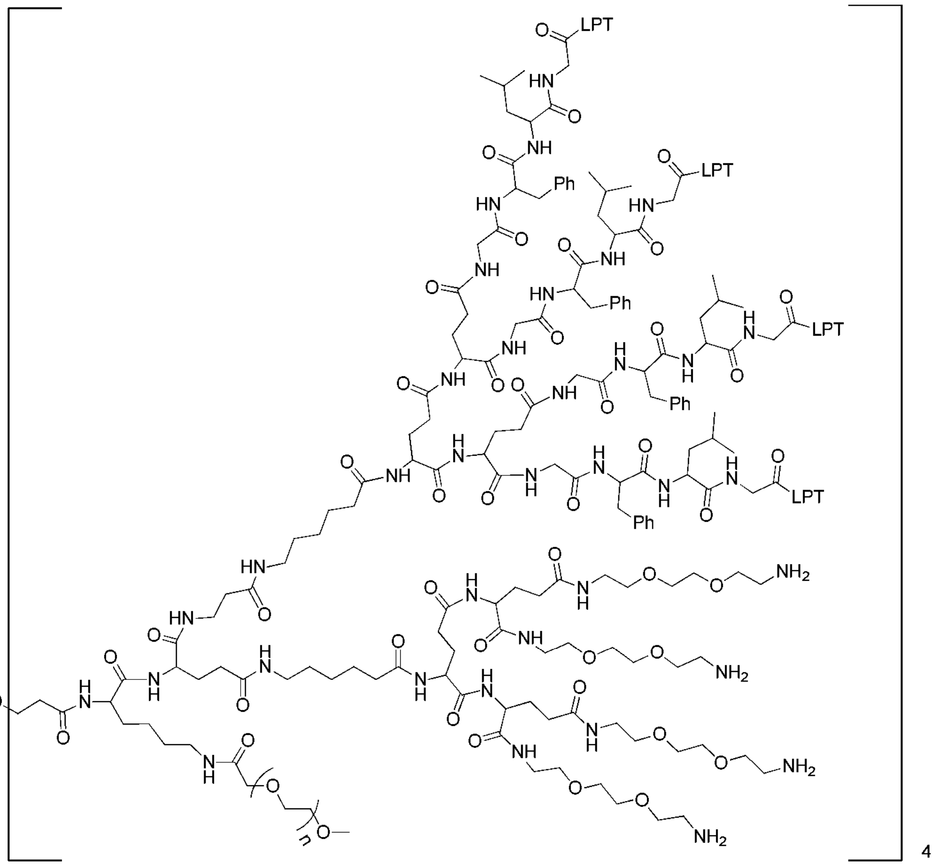

To a flask containing **85-59** (1.0 g, 0.0150 mmol), TFA (10 mL) was added for dissolution with the assistance of ultrasonication, and reacted with stirring at room temperature overnight. After the reaction was completed, the reaction mixture was evaporated to an oily state using a rotary evaporator. Methyl tert-butyl ether (60 mL) was then added for precipitation. A powdery solid precipitated from the reaction mixture and was filtered. The filter cake was washed with methyl tert-butyl ether (40 mL × 3). The filter cake was collected and dried in a vacuum oven to provide 0.97 g of the product. $^1$H-NMR (600 MHz, DMSO-$d_6$) δ 8.81-8.69 (m, 16H), 8.58-8.49 (m, 16H), 8.26-7.96 (m, 180H), 7.82-7.76 (m, 34H), 7.48-7.43 (m, 16H), 7.23-7.08 (m, 170H), 5.28-5.21 (m, 32H), 5.17-5.10 (m, 32H), 4.79-4.66 (m, 30H), 4.62-4.49 (m,27H), 4.41-4.31 (m, 22H), 4.27-4.13 (m, 66H), 3.90-3.80 (m, 28H), 3.79-3.73 (m, 30H), 3.70-3.60 (m, 128H), 3.52-3.48 (m, 3828H), 3.25-3.21 (m, 54H), 3.20-3.16 (m, 40H), 3.03-2.95 (m, 112H), 2.78-2.72 (m, 28H), 2.17-2.01 (m, 92H), 1.85-1.82 (m, 28H), 1.75-1.66 (m, 34H), 1.61-1.55 (m, 32H), 1.48-1.47 (m, 20H), 1.25-1.15 (m, 34H), 0.86-0.80 (m, 96H)

20.10 Preparation of **85-71**

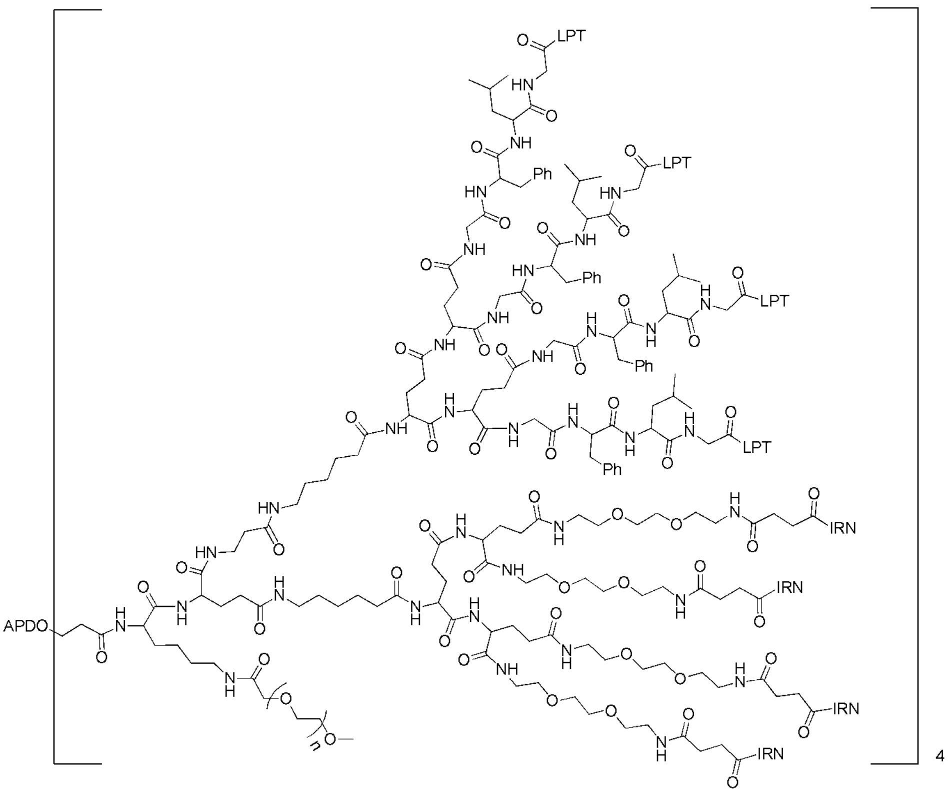

**85-69** (0.97 g, 0.0150 mmol), HBTU (0.136 g, 0.36 mmol), and HOBT (0.048 g, 0.36 mmol) were weighed and added to a reaction flask containing **78-103** (0.25 g, 0.36 mmol), then DMF (20 mL) was added to dissolve the mixture, and the mixture was stirred at room temperature for 5 minutes. Triethylamine (0.1 mL, 0.72 mmol) was slowly added dropwise. After the dropwise addition was completed, the reaction was carried out with stirring at room temperature overnight. After the reaction was completed, n-hexane (25 mL) and methyl tert-butyl ether (200 mL) were added for precipitation. The supernatant was discarded, and n-hexane (25 mL) and methyl tert-butyl ether (200 mL) were added again for precipitation. This process was repeated three times. The mixture was filtered to provide a solid product, which was dissolved in a methanol/dichloromethane (1/4) mixture, then 100-200 mesh silica gel powder was added, and the mixture was evaporated to dryness to provide a powdery solid. The powdery solid was dry-loaded onto the column and subjected to column chromatography using a 1% triethylamine/8-12% methanol/dichloromethane mixture as the eluent. The desired product was collected, concentrated, evaporated to dryness, and dried in a vacuum oven to provide 0.71 g of the product. $^1$H-NMR (600 MHz, DMSO-$d_6$) δ 8.82-8.72 (m, 16H), 8.58-8.49 (m, 16H), 8.37-7.87 (m, 180H), 7.71-7.65 (m, 50H), 7.38-7.35 (m, 16H), 7.23-7.16 (m, 218H), 6.82-6.79 (m, 16H), 5.37-5.34 (m, 32H), 4.79-4.71 (m, 62H), 4.57-4.55 (m, 27H), 4.37-4.35 (m, 22H), 4.20-4.15 (m, 98H), 3.78-3.75 (m, 58H), 3.63-3.61 (m, 128H), 3.52-3.48 (m, 3828H), 3.26-3.21 (m, 118H), 3.19-3.17 (m, 40H), 3.04-3.00 (m, 112H), 2.78-2.73 (m, 76H), 2.63-2.59 (m, 60H), 2.40-2.35 (m, 64H), 2.15-2.10 (m, 92H), 1.83-1.81 (m, 64H), 1.74-1.68 (m, 98H), 1.52-1.45 (m, 148H), 1.31-1.27 (m, 82H), 0.88-0.83 (m, 144H)

Example 21: Synthesis of Compound **75-196**

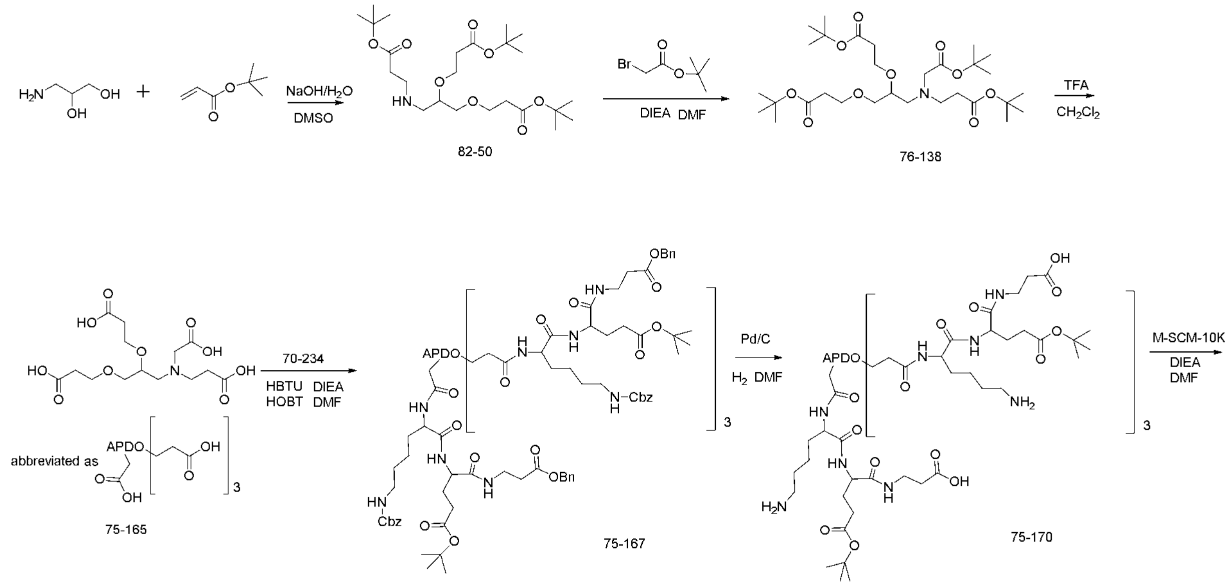
NaOH/H2O
DMSO
82-50
DIEA DMF
76-138
TFA
CH2Cl2
abbreviated as
75-165
70-234
HBTU DIEA
HOBT DMF
75-167
Pd/C
H2 DMF
75-170
M-SCM-10K
DIEA
DMF

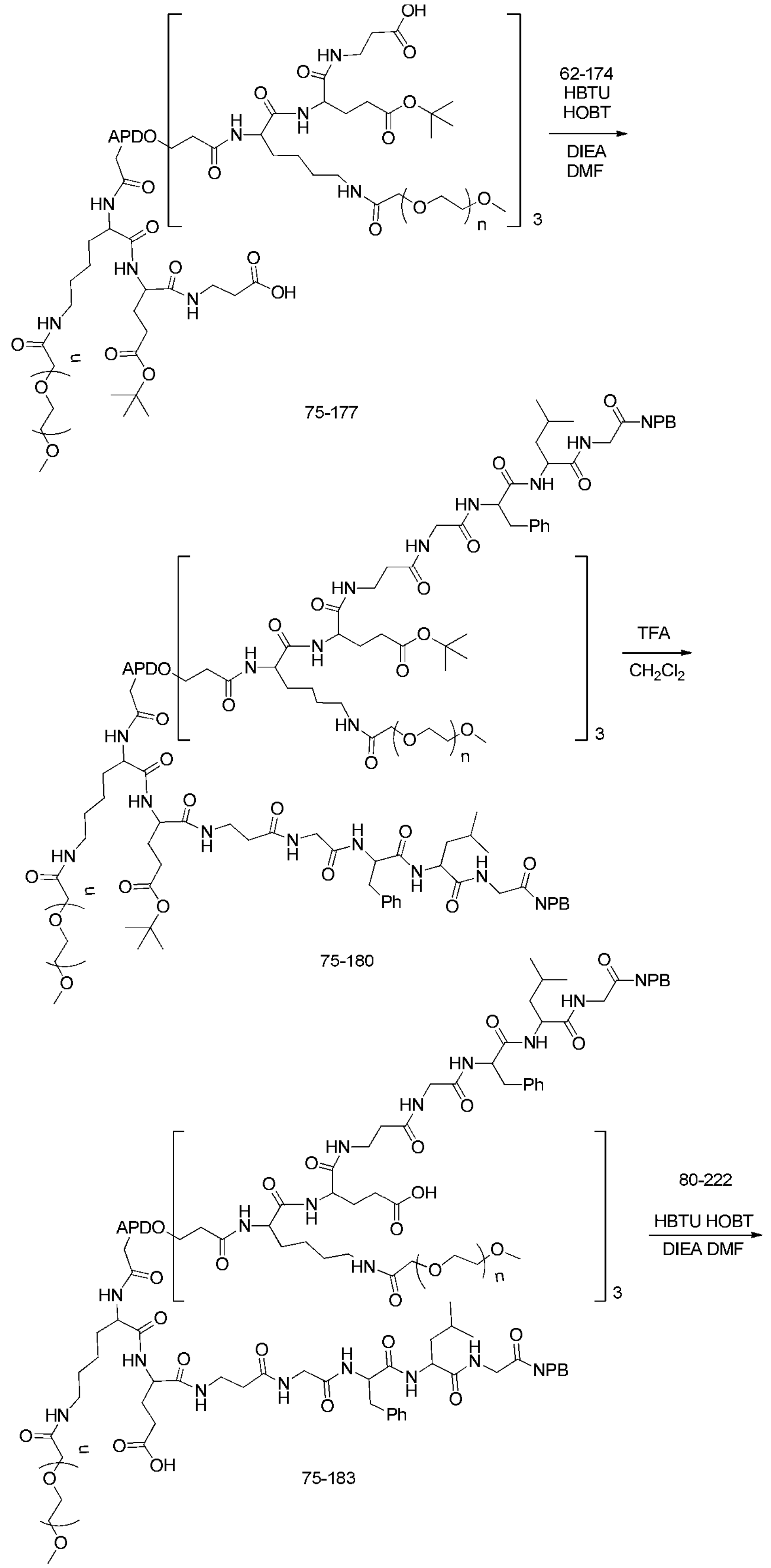


75-177

75-180

75-183

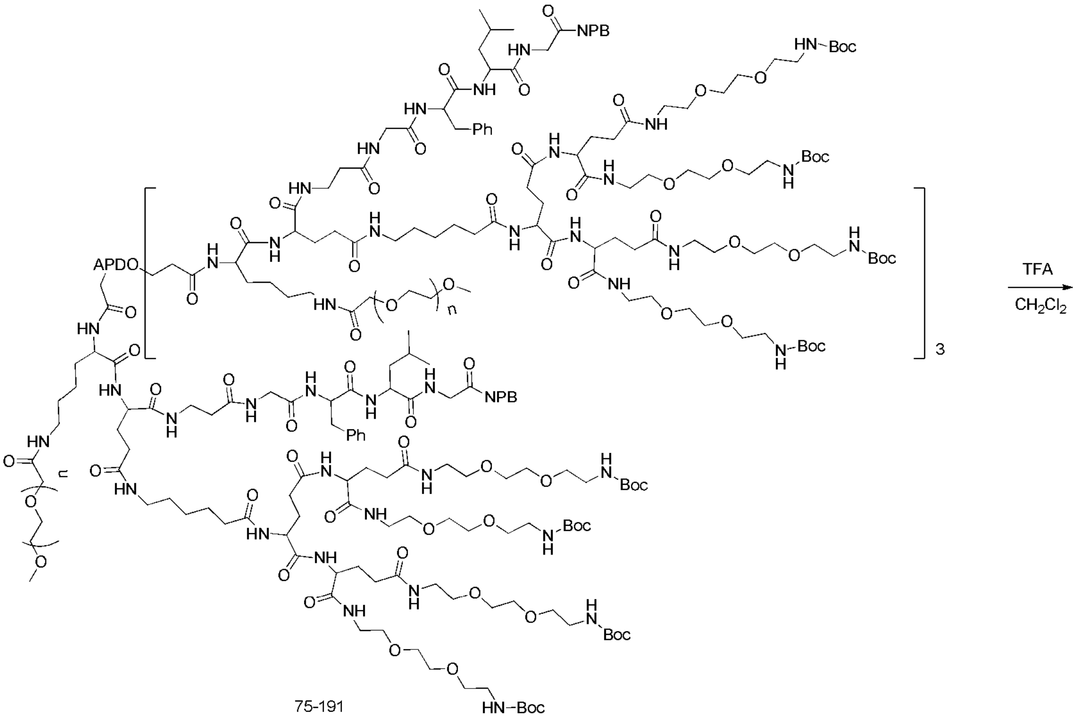


75-191

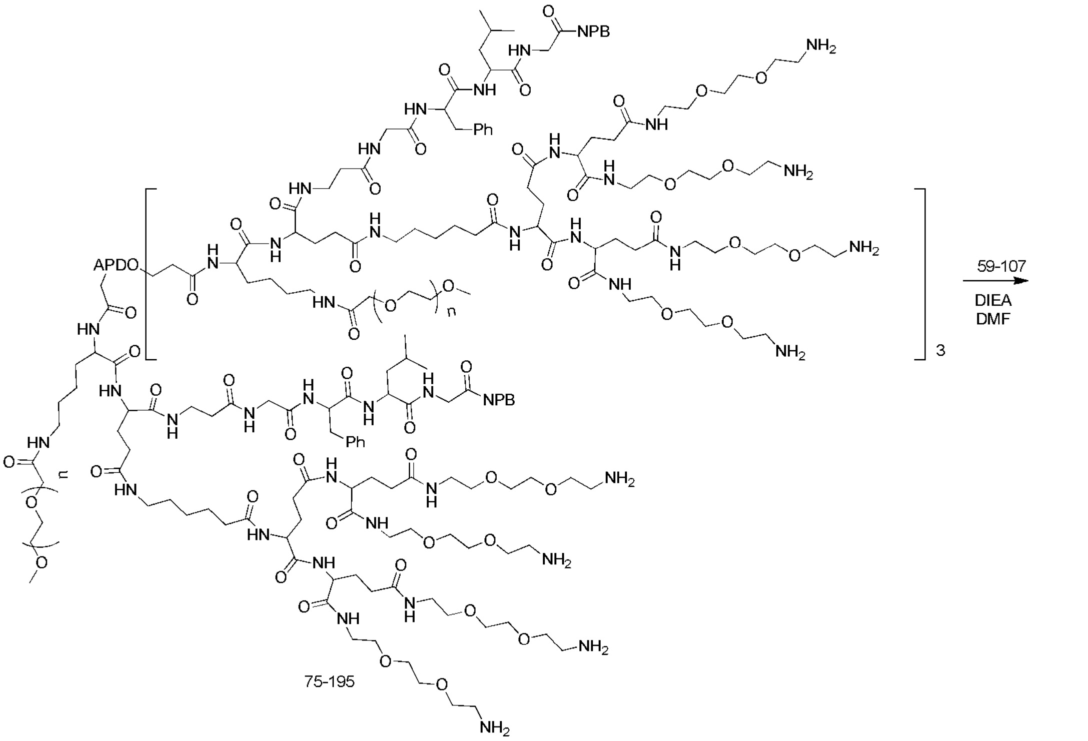


75-195

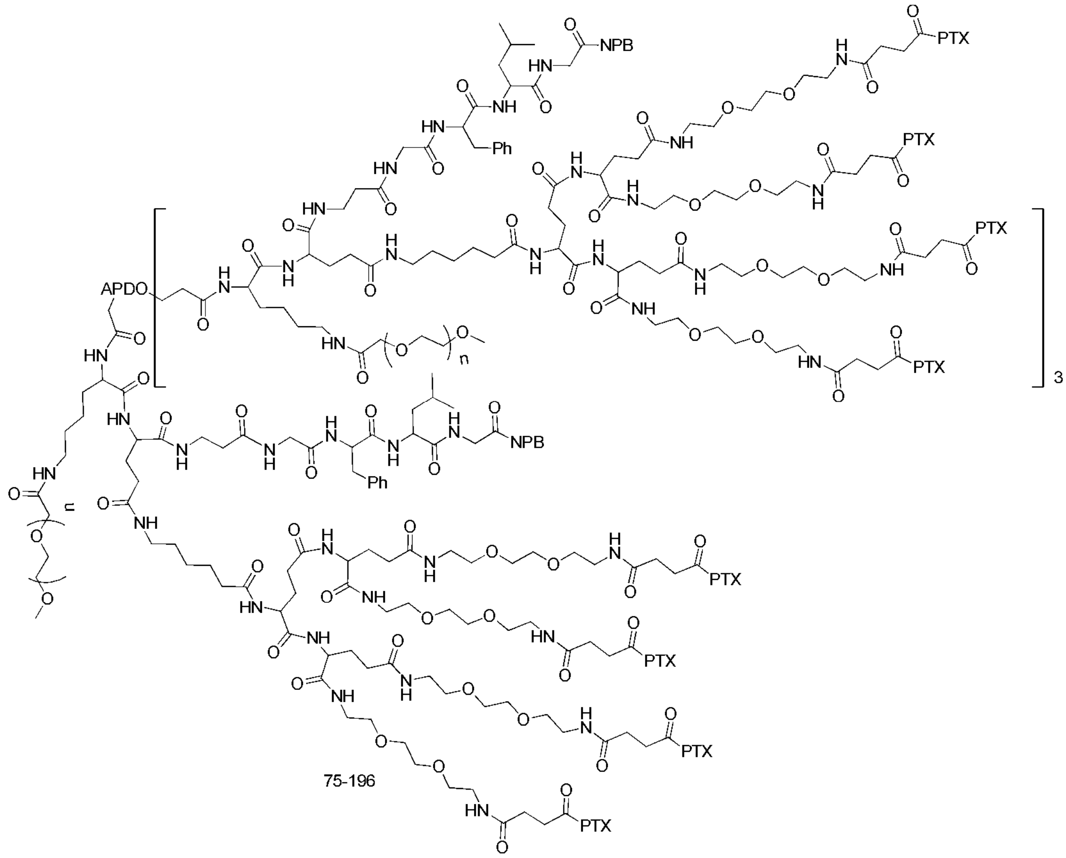

75-196

21.1 Preparation of **82-50**

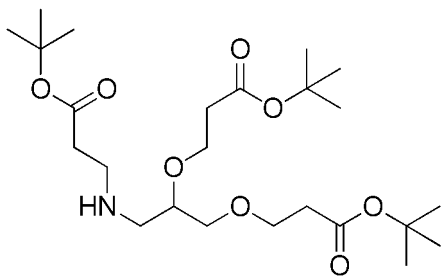

3-Amino-1,2-propanediol (1.0 g, 10.98 mmol) was weighed and added into a 500 mL flask, and then DMSO (2 mL) was added, and the flask was allowed to stand at 15°C. The flask was filled with nitrogen gas, and then 5N aqueous sodium hydroxide solution (0.2 mL) was added. After complete dissolution with the assistance of ultrasonication, tert-butyl acrylate (4.78 mL, 32.93 mmol) was slowly added dropwise. After the dropwise addition was completed, the mixture was stirred and allowed to react at room temperature overnight. After the reaction was completed, the mixture was poured into a separatory funnel containing saturated brine (200 mL) and ethyl acetate (200 mL) for extraction. After standing for phase separation, the organic phase was collected, and the aqueous phase was further extracted with ethyl acetate (200 mL × 3). The organic phases were combined, evaporated to dryness to provide a solid, which was collected and concentrated, and dried in a vacuum oven. The obtained solid was then dissolved in a methanol/dichloromethane (1/4) mixture, then silica gel powder (100 mL) was added, and the mixture was evaporated to dryness to provide a powdery solid. The powdery solid was dry-loaded onto the column and subjected to column chromatography using a 5-50% ethyl acetate/petroleum ether mixture as the eluent. The desired product was collected, concentrated, and dried in a vacuum oven to provide 1.21 g of the product.

21.2 Preparation of **76-138**

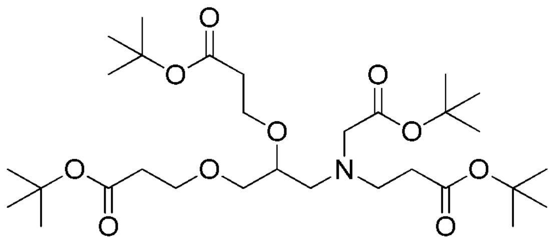

**82-50** (1.21 g, 2.1025 mmol) was weighed and placed in a 500 mL flask, then DIEA (1.39 g, 6.7315 mmol) and DMF (20 mL) were added for dissolution with the assistance of ultrasonication, then tert-butyl bromoacetate (0.41 g, 2.1025 mmol) was slowly added dropwise, and the mixture was reacted with stirring at room temperature overnight. After the reaction was completed, the mixture was poured into a separatory funnel containing saturated brine (200 mL) and ethyl acetate (200 mL) for extraction. After standing for phase separation, the organic phase was collected, and the aqueous phase was further extracted with ethyl acetate (200 mL × 3). The organic phases were combined, evaporated to dryness to provide a solid, then the obtained solid was dissolved in a methanol/dichloromethane (1/4) mixture. Silica gel powder was added, and the mixture was evaporated to dryness to provide a powdery solid. The powdery solid was dry-loaded onto the column and subjected to column chromatography using a 20-40% ethyl acetate/petroleum ether mixture as the eluent. The desired product was collected, concentrated, and dried in a vacuum oven to provide 0.47 g of the product. ESI [M+H$^+$] 590.38629

21.3 Preparation of **75-165**

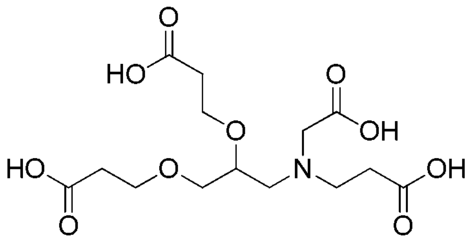

To a flask containing **76-138** (0.47 g, 0.796 mmol), dichloromethane (15 mL) was added for dissolution with the assistance of ultrasonication, then TFA (3 mL) was added, and the mixture was reacted with stirring at room temperature overnight. After the reaction was completed, the reaction mixture was evaporated to an oily state using a rotary evaporator. Methyl tert-butyl ether (60 mL) was then added for precipitation. A powdery solid precipitated from the reaction mixture and was filtered. The filter cake was washed with methyl tert-butyl ether (40 mL × 3). The resulting product had poor solubility. The filter cake was collected and dried in a vacuum oven to provide 0.22 g of the product.

21.4 Preparation of **75-167**

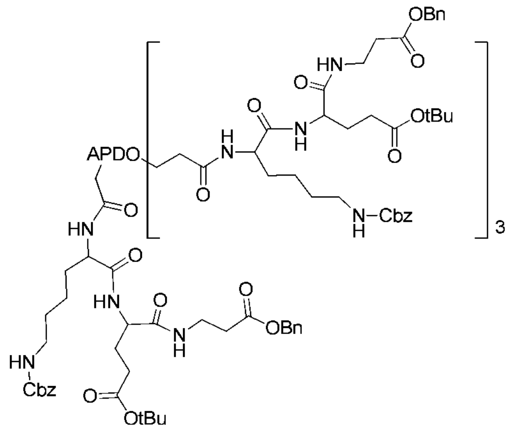

**75-165** (0.22 g, 0.609 mmol), HBTU (1.38 g, 3.655 mmol), and HOBT (0.49 g, 3.655 mmol) were weighed and added to a flask containing **70-234** (1.68 g, 2.680 mmol), then DMF (30 mL) was added to dissolve the mixture. DIEA (1.8 mL, 10.965 mmol) was slowly added dropwise at -5°C. After the dropwise addition was completed, the reaction was carried out for half an hour, the flask was then taken out, and the reaction was carried out with stirring at room temperature overnight. After the reaction was completed, n-hexane (25 mL) and methyl tert-butyl ether (200 mL) were added for precipitation. The supernatant was decanted, and n-hexane and methyl tert-butyl ether were added again for precipitation. This process was repeated three times. The mixture was filtered to provide a solid product, which was dissolved in a methanol/dichloromethane (1/4) mixture, then silica gel powder was added, and the mixture was evaporated to dryness. The resulting product was dry-loaded onto the column and subjected to column chromatography using a 1-2% methanol/dichloromethane mixture as the eluent. The desired product was collected, concentrated, and dried in a vacuum oven to provide 1.1 g of the product. $^{1}$H-NMR (600 MHz, DMSO-$d_6$) δ 8.42-8.39 (m, 4H), 8.18-8.15 (m, 4H), 7.59-7.56 (m, 4H), 7.39-7.32 (m, 40H), 7.27-7.24 (m, 4H), 5.08 (s, 8H), 5.00 (s, 8H), 4.25-4.20 (m, 4H), 4.05-4.00 (m, 4H), 3.42-3.35 (m, 8H), 3.30-3.22 (m, 7H), 3.0-2.96 (m, 20H), 2.56-2.51 (m, 16H), 2.18-2.12 (m, 8H), 1.83-1.67 (m, 16H), 1.38 (s, 36H), 1.31-1.21 (m, 8H)

21.5 Preparation of **75-170**

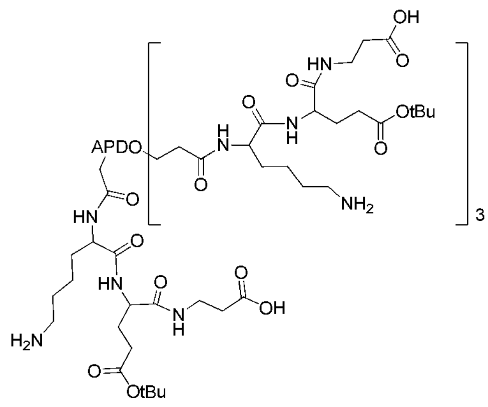

**75-165** (0.2 g, 0.0714 mmol) and 10% Pd/C catalyst (0.1 g) were placed in a hydrogenation reactor, then methanol (20 mL) was added for dissolution. The hydrogenation reactor was sealed, evacuated with a water pump, and then filled with hydrogen gas. This process was repeated three times. The pressure reading of the hydrogenation reactor was adjusted to 2 MPa, and the reaction was carried out at room temperature overnight. After the reaction was completed, the reaction mixture was filtered through Celite, and the filter cake was washed with methanol (5 mL × 3). The filtrate was collected and concentrated under reduced pressure. Dichloromethane (10 mL) and toluene (1 mL) were added to the residue, and the mixture was subjected to azeotropic evaporation to dryness. This process was repeated six times. Finally, the evaporation was performed until a solid precipitated, which was dried in a vacuum oven to provide 0.14 g of the product. ESI [M+K$^+$] 1941.914

21.6 Preparation of **75-177**

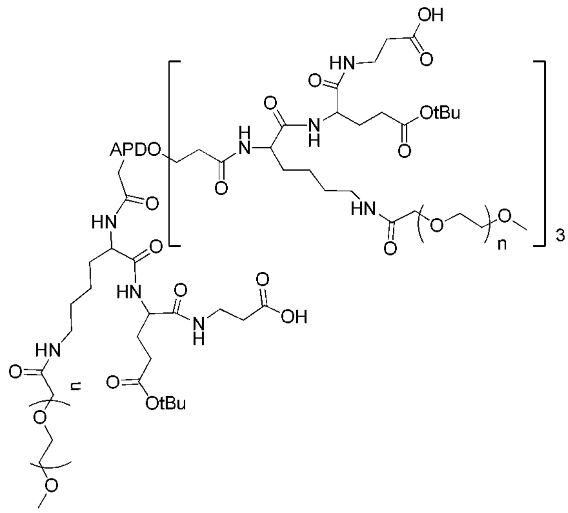

**75-170** (0.1 g, 0.0525 mmol) was placed in a 250 mL flask. DMF (10 mL) was added for dissolution, then DIEA (1.4 mL, 8.82 mmol) was slowly added dropwise at -5°C, and the mixture was reacted for 30 minutes. The flask was taken out, M-SCM-10K (2.3 g, 0.22 mmol, purchased from Jenkem, Lot Number: A3016-N230101) was added, and the reaction was carried out with stirring at low speed in the dark at room temperature for one week. After the reaction was completed, n-hexane (25 mL) and methyl tert-butyl ether (200 mL) were added for precipitation, and the mixture was filtered. The obtained solid was dissolved in dichloromethane, and then n-hexane (25 mL) and methyl tert-butyl ether (200 mL) were added for precipitation. This process was repeated three times. The mixture was filtered to provide a solid product, which was dissolved in a methanol/dichloromethane (1/4) mixture, then silica gel powder was added, and the mixture was evaporated to dryness. The resulting product was dry-loaded onto the column and subjected to column chromatography using a 1% ammonia solution/5-7% methanol/dichloromethane mixture as the eluent. The desired product was collected, concentrated, evaporated to dryness, and dried in a vacuum oven to provide 2.3 g of the product. $^1$H-NMR (600 MHz, DMSO-$d_6$) δ 9.39-9.15 (m, 4H) 8.05-8.03 (m, 16H), 4.55-4.53 (m, 8H), 3.65-3.62 (m, 5H), 3.50-3.44 (m, 3840H), 3.47-3.43 (m, 6H), 3.25-3.22 (m, 6H), 2.90-2.87 (m, 19H), 2.76-2.71 (m, 23H), 1.79-1.77 (m, 8H), 1.53-1.51 (m, 8H), 1.39-1.38 (m, 36H), 1.25-1.23 (m, 8H)

21.7 Preparation of **75-180**

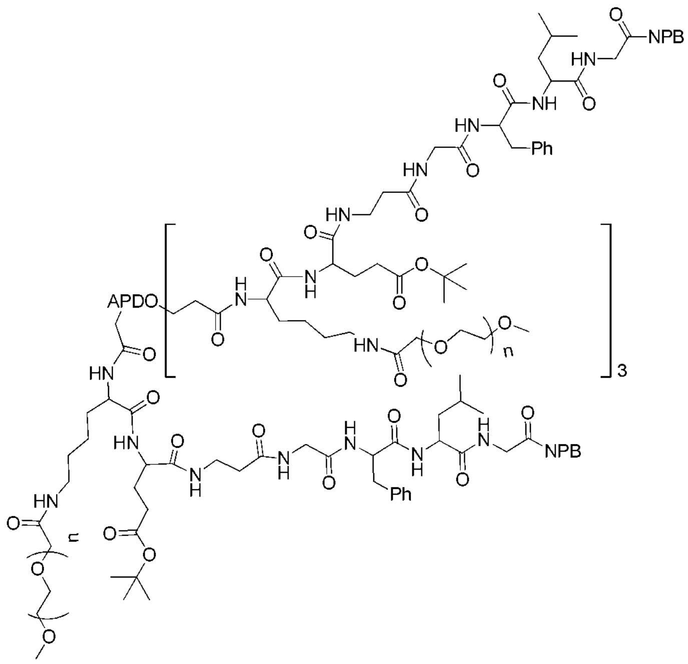

**75-177** (2.30 g, 0.0522 mmol), HBTU (0.12 g, 0.313 mmol), and HOBT (0.04 g, 0.313 mmol) were weighed and added to a flask containing **62-174** (0.22 g, 0.313 mmol), then DMF (30 mL) was added to dissolve the mixture, and DIEA (0.2 mL, 0.941 mmol) was slowly added dropwise at -5°C. After the dropwise addition was completed, the mixture was brought to room temperature and reacted with stirring overnight. After the reaction was completed, n-hexane (25 mL) and methyl tert-butyl ether (200 mL) were added for precipitation, and the mixture was filtered. The obtained solid was dissolved in dichloromethane, and then n-hexane and methyl tert-butyl ether were added for precipitation. The supernatant was discarded, and n-hexane and methyl tert-butyl ether were added again for precipitation. This process was repeated three times. The mixture was filtered to provide a solid product, which was dissolved in a methanol/dichloromethane (1/4) mixture, then 200-300 mesh silica gel powder was added, and the mixture was evaporated to dryness. The resulting product was dry-loaded onto the column and subjected to column chromatography using a 1% ammonia water/5-7% methanol/dichloromethane mixture as the eluent. The desired product was collected, concentrated and evaporated to dryness, and dried in a vacuum oven to provide 2.29 g of the product. $^1$H-NMR (600 MHz, DMSO-$d_6$) δ 8.12-8.01 (m, 36H), 7.92-7.89 (m, 8H), 7.60-7.58 (m, 4H), 7.54-7.50 (m, 8H), 7.31-7.21 (m, 36H), 4.60-4.52 (m, 4H), 4.47-4.35 (m, 12H), 4.10-4.08 (m, 8H), 3.63-3.61 (m, 21H), 3.51-3.50 (m, 3840H), 3.44-3.42 (m, 28H), 2.94-2.91 (m, 24H), 2.65-2.61 (m, 24H), 1.90-1.80 (m, 24H), 1.67-1.57 (m, 20H), 1.377-1.341 (m, 36H), 1.25-1.23 (m, 8H), 0.92-0.90 (m, 24H)

21.8 Preparation of **75-183**

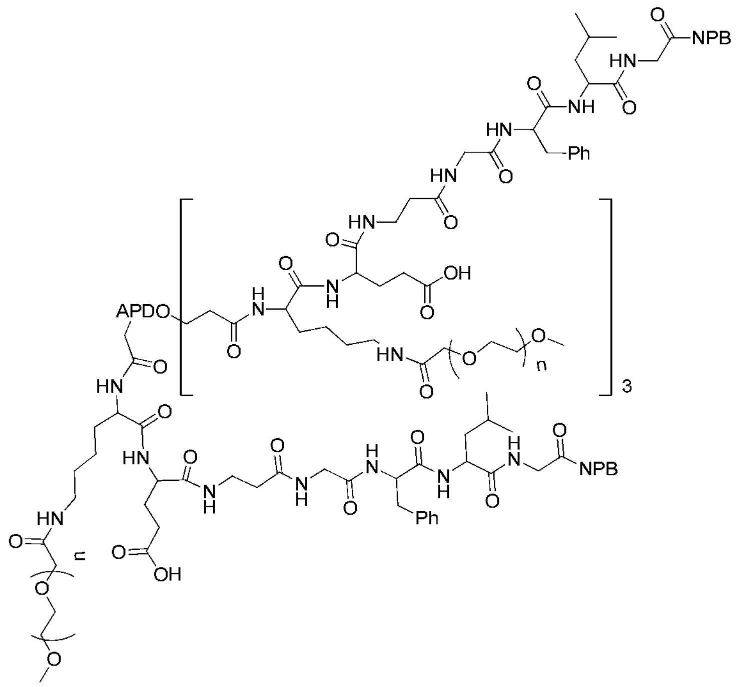

To a flask containing **75-180** (3.4 g, 0.0763 mmol), dichloromethane (20 mL) was added for dissolution with the assistance of ultrasonication, then TFA (10 mL) was added, and the mixture was reacted with stirring at room temperature overnight. After the reaction was completed, the reaction mixture was evaporated to an oily state using a rotary evaporator, and then methyl tert-butyl ether (60 mL) and n-hexane (20 mL) were added for precipitation. A powdered solid precipitated from the reaction mixture and was filtered. The filter cake was washed with methyl tert-butyl ether (40 mL×3). The filter cake was collected and dried in a vacuum oven to provide 2.08 g of the product. $^1$H-NMR (600 MHz, DMSO-$d_6$) δ 9.90-9.81 (m, 4H), 8.10-8.01 (m, 36H), 7.98-7.93 (m, 8H), 7.62-7.60 (m, 4H), 7.54-7.50 (m, 8H), 7.31-7.21 (m, 36H), 4.60-4.52 (m, 4H), 4.47-4.35 (m, 12H),4.10-4.08 (m, 8H), 3.66-3.61 (m, 21H), 3.51-3.50 (m, 3840H), 3.44-3.36 (m, 28H), 2.94-2.91 (m, 24H), 2.64-2.60 (m, 24H), 1.90-1.80 (m, 24H), 1.67-1.57 (m, 20H), 1.26-1.24 (m, 8H), 0.95-0.92 (m, 24H)

21.9 Preparation of **75-191**

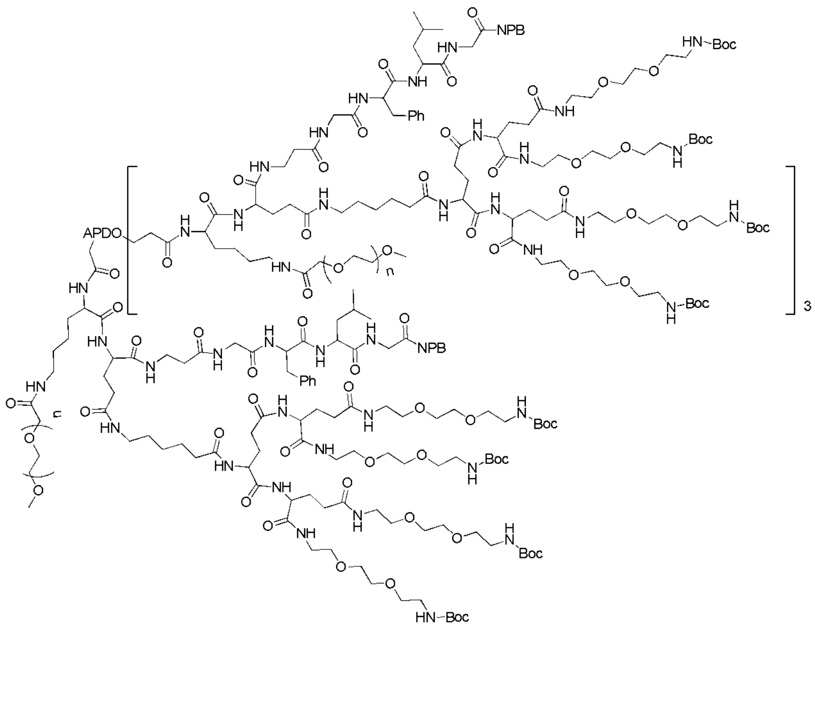

**75-183** (2.08 g, 0.0447 mmol), HBTU (0.101 g, 0.2675 mmol), and HOBT (0.035 g, 0.2675 mmol) were added to a flask containing **80-222** (0.51 g, 0.3569 mmol). DMF (20 mL) was added to dissolve the mixture, then DIEA (0.2 mL, 1.294 mmol) was slowly added dropwise at -5°C. After the dropwise addition was completed, the reaction was carried out for half an hour, the flask was then taken out, and the reaction was carried out with stirring at room temperature overnight. After the reaction was completed, n-hexane (25 mL) and methyl tert-butyl ether (200 mL) were added for precipitation. The supernatant was discarded, and n-hexane and methyl tert-butyl ether were added again for precipitation. This process was repeated three times. The mixture was filtered to provide a solid product, which was dissolved in a methanol/dichloromethane (1/4) mixture, then 200-300 mesh silica gel powder was added, and the mixture was evaporated to dryness. The resulting product was dry-loaded onto the column and subjected to column chromatography using 1% ammonia water/5-10% methanol/dichloromethane as the eluent. The desired product was collected, concentrated, and evaporated to dryness to provide 1.09 g of the product. $^1$H-NMR (600 MHz, DMSO-$d_6$) δ 8.31-8.18 (m, 8H), 8.06-7.98 (m, 84H), 7.60-7.58 (m, 4H), 7.55-7.51 (m, 8H), 7.35-7.28 (m, 36H), 4.55-4.51 (m, 16H), 4.46-4.42 (m, 12H), 4.20-4.18 (m, 4H), 4.10-4.06 (m, 8H), 3.76-3.65 (m, 72H), 3.54-3.51 (m, 77H), 3.52-3.49 (m, 3840H), 3.44-3.36 (m, 50H), 3.04-2.95 (m, 56H), 2.66-2.60 (m, 38H), 2.08-2.04 (m, 56H), 1.92-1.85 (m, 24H), 1.65-1.59 (m, 28H), 1.38-1.34 (m, 144H), 1.29-1.22 (m, 24H), 0.96-0.93 (m, 24H)

21.10 Preparation of **75-195**

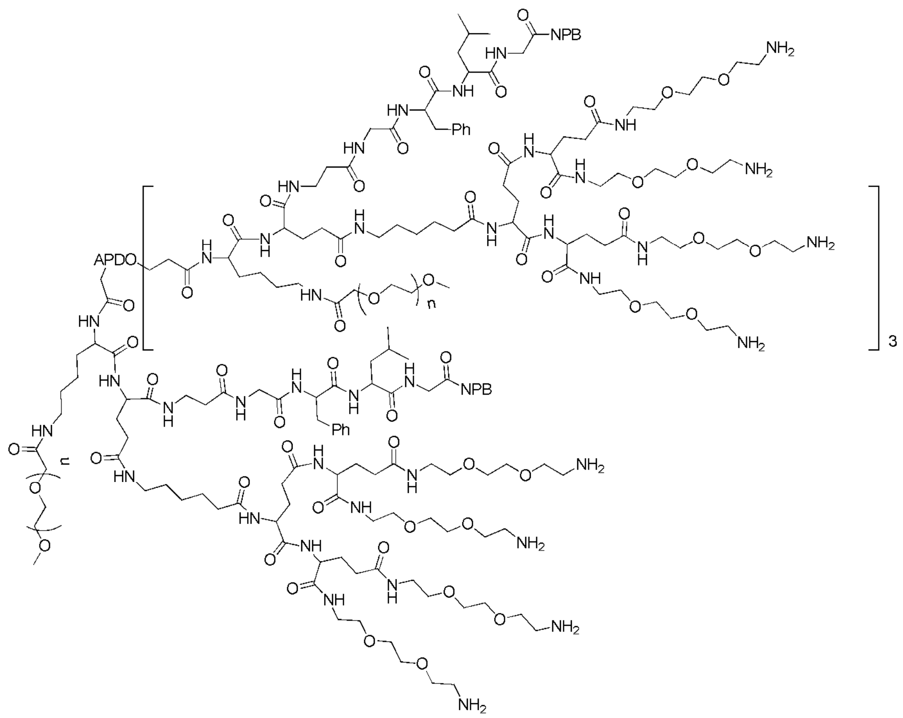

To a flask containing **75-191** (1.09 g, 0.0209 mmol), dichloromethane (10 mL) was added for dissolution with the assistance of ultrasonication, then TFA (0.5 mL) was added, and the mixture was reacted with stirring at room temperature overnight. After the reaction was completed, the reaction mixture was evaporated to an oily state using a rotary evaporator. Methyl tert-butyl ether (60 mL) was then added for precipitation. A powdery solid precipitated from the reaction mixture and was filtered. The obtained solid was dissolved in dichloromethane, and then methyl tert-butyl ether (40 mL) and n-hexane (15 mL) were added again for precipitation. This process was repeated three times. The filter cake was collected and dried to provide 0.97 g of the product. $^1$H-NMR (600 MHz, DMSO-$d_6$) δ 8.57-8.35 (m, 8H), 8.23-.01 (m, 68H), 7.60-7.58 (m, 8H), 7.54-7.50 (m, 4H), 7.31-7.21 (m, 36H), 5.11-5.02 (m, 32H), 4.58-4.50 (m, 16H), 4.47-4.35 (m, 12H), 4.14-4.10 (m, 4H), 4.10-4.08 (m, 8H), 3.76-3.65 (m, 73H), 3.55-3.51 (m, 76H), 3.52-3.49 (m, 3840H), 3.43-3.35 (m, 36H), 3.06-3.02 (m, 42H), 2.94-2.91 (m, 29H), 2.66-2.60 (m, 36H), 2.07-2.03 (m, 56H), 1.99-1.94 (m, 24H), 1.68-1.57 (m, 28H), 1.29-1.26 (m, 24H), 0.92-0.90 (m, 24H)

21.11 Preparation of **75-196**

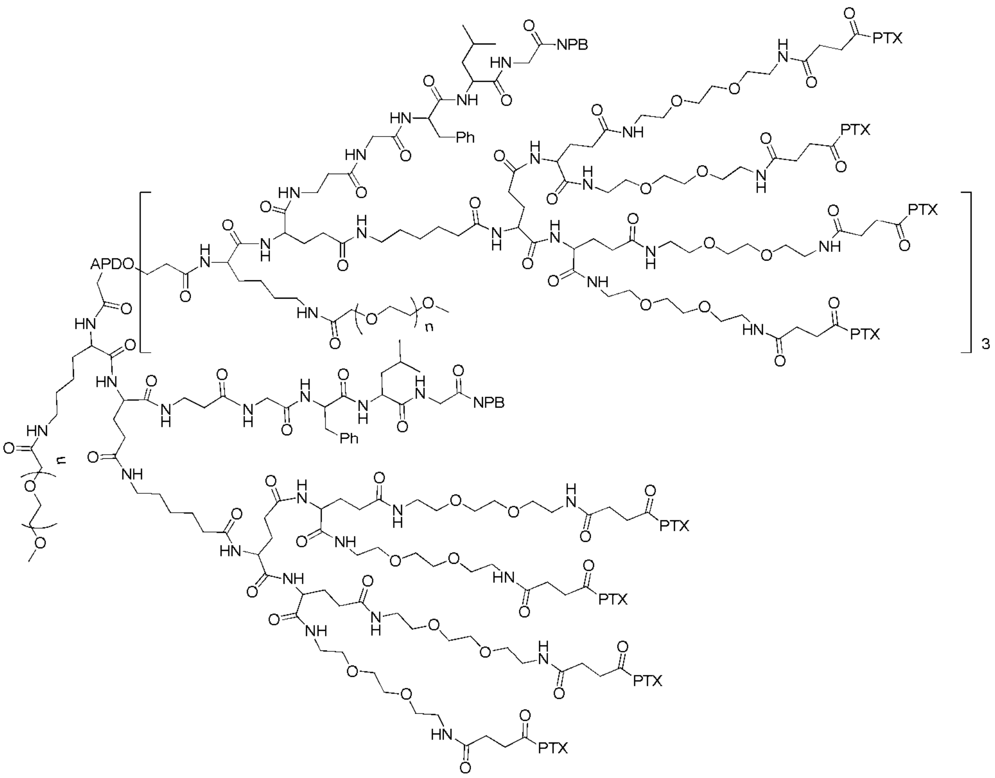

**75-195** (0.97 g, 0.0191 mmol) was dissolved in DMF (7.5 mL). DIEA was added to adjust the pH to neutral, and then the mixture was added to a flask containing **59-107** (0.64 g, 0.6133 mmol). DIEA (0.1 mL, 0.6133 mmol) was slowly added dropwise, and reacted with stirring at room temperature overnight. After the dropwise addition was completed, n-hexane (25 mL) and methyl tert-butyl ether (200 mL) were added for precipitation, and the supernatant was discarded. The obtained solid was then dissolved in dichloromethane, and then n-hexane and methyl tert-butyl ether were added for precipitation. This process was repeated three times. The mixture was filtered to provide a solid product, which was collected, and dissolved in a methanol/dichloromethane (1/4) mixture, then 200-300 mesh silica gel powder (10 mL) was added, and the mixture was evaporated to dryness to provide a powdery solid. The powdery solid was dry-loaded onto the column and subjected to column chromatography using a 1% triethylamine/6-10% methanol/dichloromethane mixture as the eluent. The desired product was collected, concentrated, and dried in a vacuum oven to provide 1.01 g of the product. $^1$H-NMR (600 MHz, DMSO-$d_6$) $\delta$ 8.16-7.81 (m, 172H), 7.78-7.63 (m, 66H), 7.53-7.36 (m, 126H), 7.25-7.13 (m, 36H), 6.30-6.27 (m, 16H), 5.83-5.78 (m, 16H), 5.41-5.38 (m, 16H), 4.98-4.94 (m, 16H), 4.92-4.87 (m, 16H), 4.68-4.63 (m, 18H), 4.22-4.14 (m, 10H), 4.04-3.95 (m, 40H), 3.85-3.81 (m, 6H), 3.64-3.525 (m, 136H), 3.52-3.47 (m, 3840H), 3.43-3.42 (m, 60H), 3.20-3.10 (m, 94H), 3.08-3.00 (m, 25H), 2.90-2.72 (m, 82H), 2.63-2.53 (m, 76H), 2.25-2.05 (m, 176H), 1.84-1.71 (m, 109H), 1.66-1.59 (m, 36H), 1.04-0.94 (m, 124H), 0.90-0.81 (m, 24H)

Example 22: Synthesis of Compound **86-2**

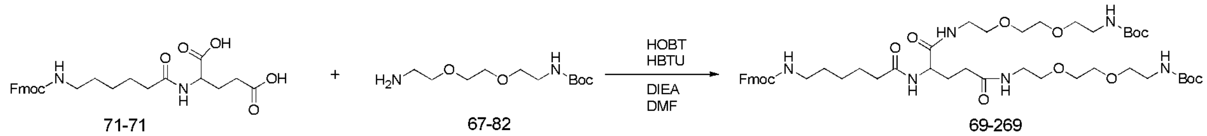


71-71 67-82 69-269

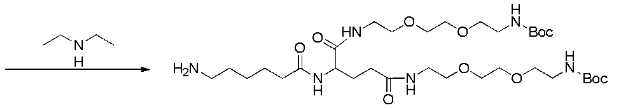

69-274

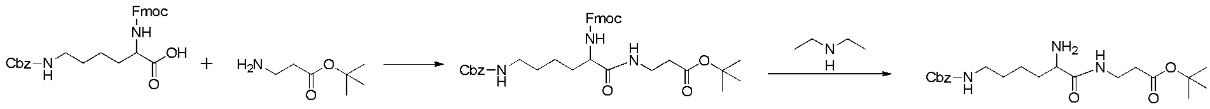

69-249 69-250

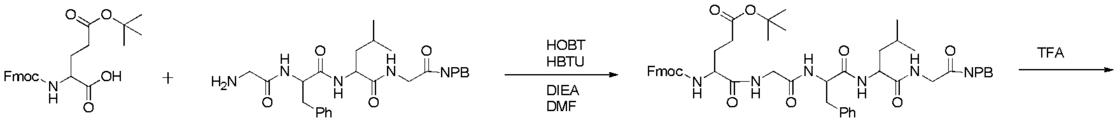


69-252

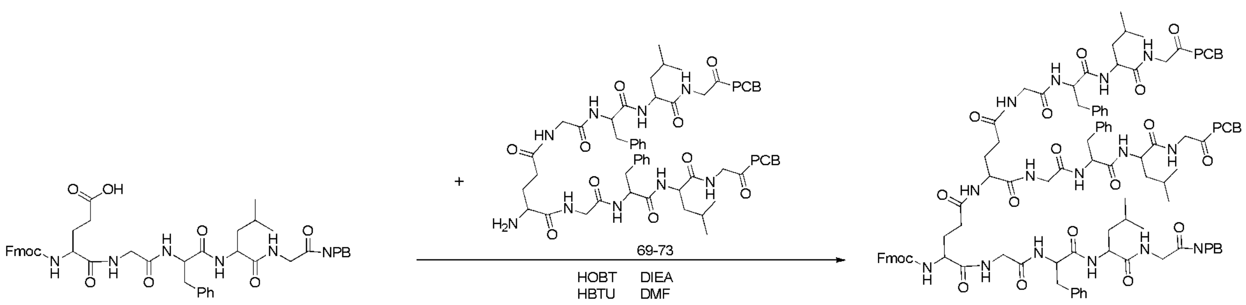


69-73 69-254 69-258

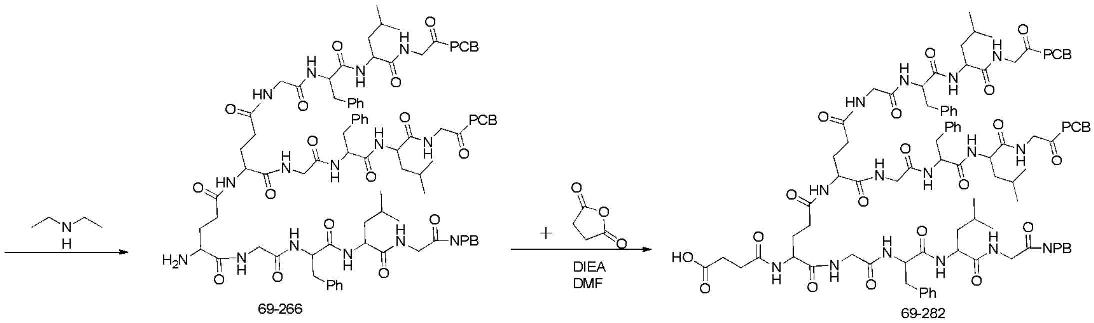


69-266 69-282

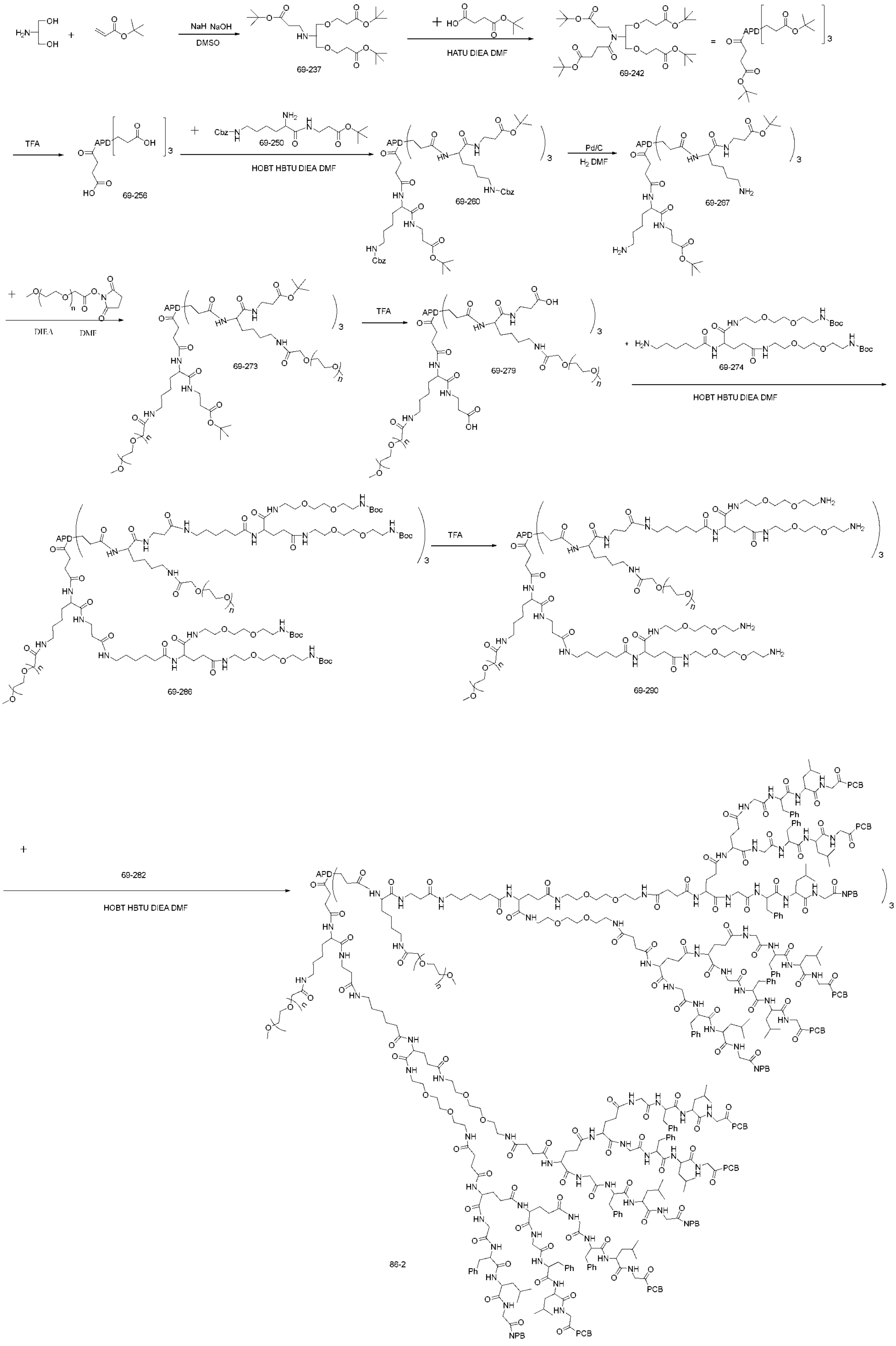
NaH NaOH
DMSO
69-237
HATU DIEA DMF
69-242
TFA
69-256
69-250
HOBT HBTU DIEA DMF
69-260
Pd/C
$H_2$ DMF
69-267
DIEA DMF
69-273
TFA
69-279
69-274
HOBT HBTU DIEA DMF
69-286
TFA
69-290
69-282
HOBT HBTU DIEA DMF
86-2

22.1 Preparation of **69-269**

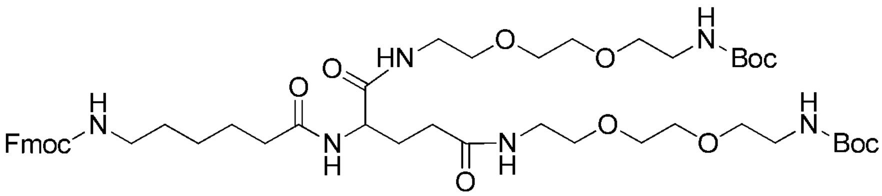

**71-71** (3.06 g, 6.341 mmol), HBTU (2.227 g, 16.486 mmol), and HOBT (6.252 g, 16.486 mmol) were weighed and added to a flask containing **67-82** (3.46 g, 13.951 mmol). DMF (150 mL) was added to dissolve the mixture, and then DIEA (6.28 mL, 38.04 mmol) was slowly added dropwise at -5°C. After the dropwise addition was completed, the reaction was carried out for half an hour, the flask was then taken out, and the reaction was carried out with stirring at room temperature overnight. After the reaction was completed, the mixture was poured into a separatory funnel containing ethyl acetate (200 mL) and purified water (200 mL) for extraction. The organic phase was collected, and the aqueous phase was further extracted with ethyl acetate (100 mL × 2). The organic phases were combined, washed once with saturated brine, dried over anhydrous magnesium sulfate, and filtered. The filtrate was concentrated under reduced pressure and dried in a vacuum oven to provide 7.25 g of the product.

22.2 Preparation of **69-274**

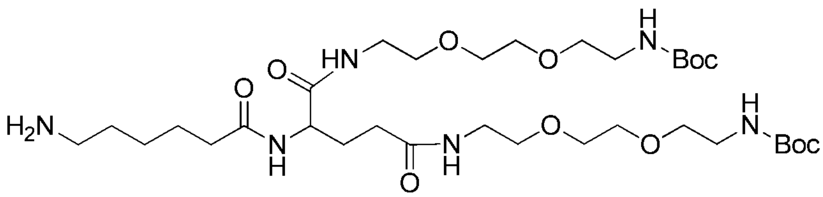

**69-269** (7.254 g, 6.341 mmol) was placed in a 500 mL round-bottom flask, then acetonitrile (20 mL) was added for dissolution with the assistance of ultrasonication. Then diethylamine (30 mL) was added, and the mixture was reacted with stirring at room temperature. After the reaction was completed, the mixture was concentrated under reduced pressure using a rotary evaporator, and the residue was dissolved in dichloromethane (30 mL), then 200-300 mesh silica gel powder was added, and the mixture was evaporated to dryness. The resulting product was dry-loaded onto the column and subjected to column chromatography using a mixture of 1% aqueous ammonia/5% methanol/dichloromethane as the eluent. The desired product was collected, concentrated, and evaporated to dryness to provide 3.25 g of the product. $^1$H-NMR (600 MHz, DMSO-$d_6$) δ 7.95-7.87 (m, 3H), 6.83-6.75 (m, 2H), 4.21-4.15 (td, $J$ = 8.3, 5.7 Hz, 1H), 3.50-3.47 (m, 8H), 3.41-3.26 (m, 12H), 3.24-3.14 (m, 2H), 3.09-3.02 (m, 4H), 2.51-2.50 (m, 2H), 2.13-2.03 (m,4H), 1.86-1.78 (m, 1H), 1.72-1.63 (m, 1H),1.50-1.44 (m, 2H), 1.39-1.34 (m, 18H), 1.34-1.29 (m, 2H), 1.27-1.20 (m, 2H); FT MS ESI m/z [M+H$^+$] 721.4689

22.3 Preparation of **69-249**

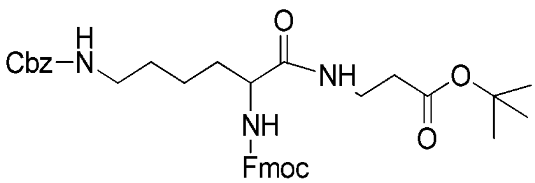

To a reaction flask, β-alanine tert-butyl ester (1.988 g, 10.943 mmol), N'-fluorenylmethyloxycarbonyl-N-benzyloxycarbonyl-L-lysine (5.0 g, 9.949 mmol), HOBT (2.016 g, 14.923 mmol), and HBTU (5.659 g, 14.923 mmol) were added, then DMF (150 mL) was added to dissolve the mixture with the assistance of ultrasonication, and the mixture was stirred at

0°C for several minutes. DIEA (7.399 mmol, 44.705 mmol) was added dropwise. After the dropwise addition was completed, the mixture was brought to room temperature and reacted with stirring for 1 hour. After the reaction was completed, methyl tert-butyl ether (200 mL) was added for precipitation. The mixture was then filtered and the filter cake collected. The filter cake was dissolved in DMF (10 mL), and then methyl tert-butyl ether (200 mL) was added for precipitation. This process was repeated twice. Then, the mixture was filtered, and the resulting filter cake was transferred to a flask and dried to provide 6.265 g of the product.

22.4 Preparation of **69-250**

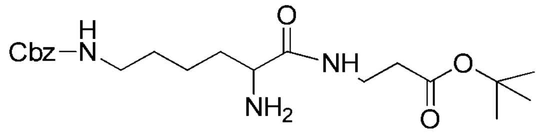

Reactant **69-249** (1.8 g, 9.5846 mmol) was taken and dissolved in DMF (30 mL), then morpholine (16.5 mL, 144 mmol) was added, and the mixture was reacted with stirring at room temperature. The reaction progress was monitored by TLC. After the reaction was completed, methyl tert-butyl ether (100 mL) and n-hexane (20 mL) were added for precipitation. The mixture was filtered using a sand-core funnel, then the filter cake was collected and washed with methyl tert-butyl ether (80 mL × 2). The filter cake was dissolved in a mixture of dichloromethane and methanol, then silica gel powder was added, and the mixture was evaporated to dryness. The resulting product was dry-loaded onto the column and subjected to column chromatography using a mixture of 1% ammonia water/4.5% methanol/dichloromethane as the eluent. The desired product was collected, concentrated and dried to provide 3.85 g of the product. $^1$H-NMR (600 MHz, DMSO-$d_6$) δ 7.96 (s, 1H), 7.34 (tt, *J* = 14.4, 7.3 Hz, 5H), 7.22 (s, 1H), 5.00 (s, 2H), 3.46-3.19 (m, 4H), 3.11 (dd, *J* = 7.1, 5.5 Hz, 1H), 2.97 (dd, *J* = 12.9, 6.7 Hz, 2H), 2.36 (t, *J* = 6.8 Hz, 2H), 1.57-1.49 (m, 1H), 1.43-1.31 (m, 12H), 1.31-1.19 (m, 2H); FT MS ESI m/z [M+H$^+$] 408.248

22.5 Preparation of **69-252**

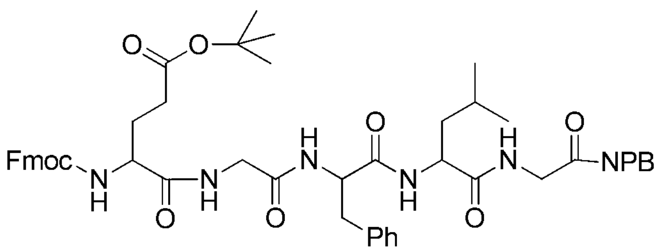

Fmoc-Glu(OtBu)-OH (0.943 g, 2.2165 mmol), **69-139** (1.4 g, 2.015 mmol), HBTU (1.146 g, 3.0225 mmol), and HOBT (0.408 g, 3.0225 mmol) were placed in a 250 mL round-bottom flask, then DMF (30 mL) was added for dissolution with the assistance of ultrasonication, and the mixture was stirred at 0°C for 10 minutes. DIEA (1.498 mL, 9.0675 mmol) was then added dropwise, and the mixture was brought to room temperature and reacted with stirring. After the reaction was completed, n-hexane (25 mL) and methyl tert-butyl ether (200 mL) were added for precipitation. The supernatant was discarded, and n-hexane (20 mL) and methyl tert-butyl ether (100 mL) were added again for precipitation. This process was repeated three times. The mixture was filtered to provide a solid product, and the filter cake was dissolved in a methanol/dichloromethane mixture, then 200-300 mesh silica gel powder was added, and the mixture was evaporated to dryness. The resulting product was dry-loaded onto the column and subjected to column chromatography using a 5% methanol/dichloromethane mixture as the eluent. The desired product was collected, concentrated, and evaporated to dryness to provide 2.02 g of the product.

22.6 Preparation of **69-254**

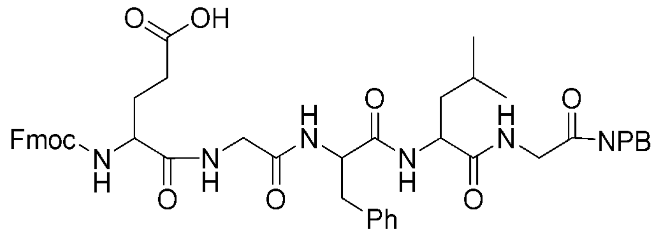

**69-252** (2.02 g, 2.015 mmol) was placed in a 500 mL round-bottom flask. Dichloromethane (5 mL) was added for dissolution, then trifluoroacetic acid (5 mL) was added, and the mixture was reacted with stirring at room temperature. After the reaction was completed, the reaction mixture was concentrated under reduced pressure to remove dichloromethane and most of the TFA. Methyl tert-butyl ether (200 mL) and n-hexane (50 mL) were then added for precipitation. The mixture was filtered using a sand-core funnel and rinsed with methyl tert-butyl ether (100 mL × 3). The filter cake was collected and dried in a vacuum oven to provide 1.89 g of the product.

22.7 Preparation of **69-258**

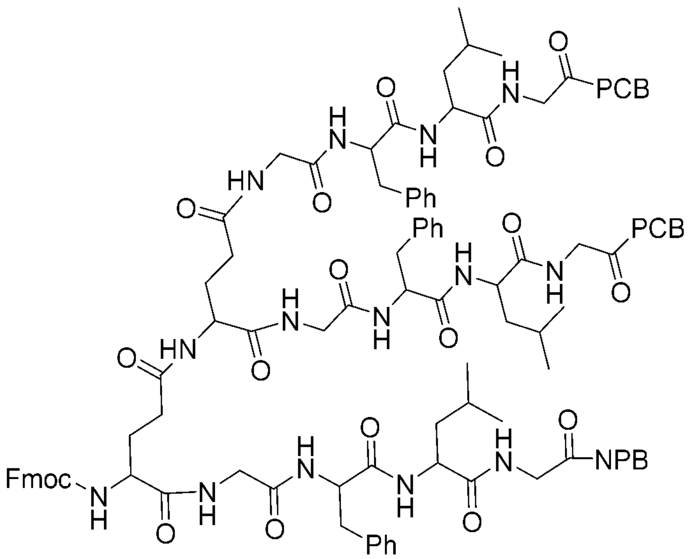

**69-254** (1.1737 g, 1.1219 mmol), **69-73** (1.79 g, 1.0199 mmol), HBTU (0.580 g, 1.5298 mmol), and HOBT (0.206 g, 1.5298 mmol) were added to a 500 mL round-bottom flask, then DMF (40 mL) was added to dissolve the mixture with the assistance of ultrasonication. DIEA (0.758 mL, 4.58 mmol) was added dropwise at 0°C. After the dropwise addition was completed, the mixture was brought to room temperature and reacted with stirring. After the reaction was completed, n-hexane (25 mL) and methyl tert-butyl ether (200 mL) were added for precipitation. The supernatant was discarded, and n-hexane (20 mL) and methyl tert-butyl ether (100 mL) were added again for precipitation. This process was repeated three times. The mixture was filtered, the filter cake was dissolved in N-methylpyrrolidone (10 mL), then methyl tert-butyl ether (200 mL) was added for precipitation, and the supernatant was discarded. This process was repeated three times. A solid precipitated, which was filtered and dried to provide 2.145 g of the product.

22.8 Preparation of **69-266**

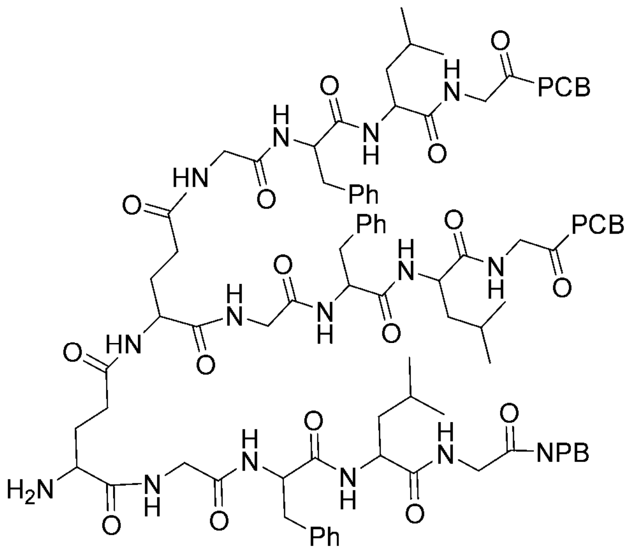

**69-258** (2.145 g, 1.0199 mmol) was placed in a 500 mL round-bottom flask. DMF (30 mL) was added for dissolution with the assistance of ultrasonication, then diethylamine (40 mL) was added, and the mixture was reacted with stirring at room temperature. After the reaction was completed, n-hexane (25 mL) and methyl tert-butyl ether (200 mL) were added for precipitation. The supernatant was discarded, and n-hexane (40 mL) and methyl tert-butyl ether (70 mL) were added again for precipitation. This process was repeated three times. Finally, the mixture was filtered to provide a solid product, which was dried in a vacuum oven to provide 1.825 g of the product.

22.9 Preparation of **69-282**

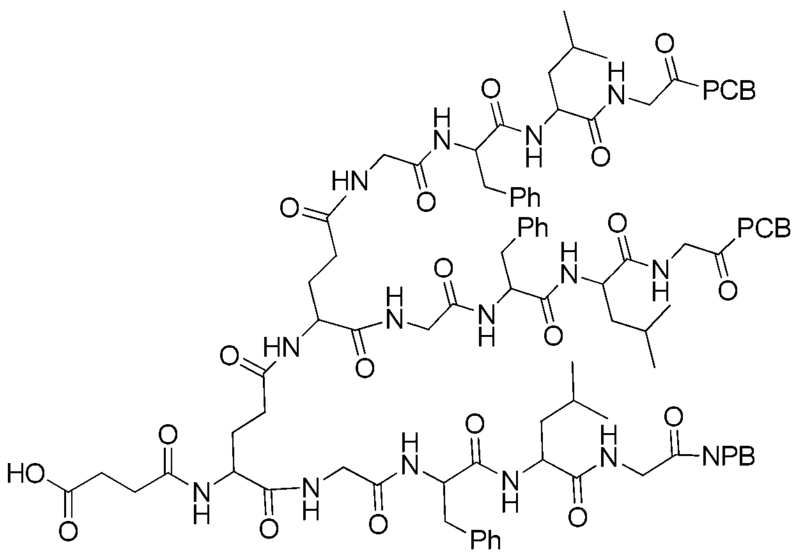

**69-266** (2.08 g, 0.8122 mmol) was placed in a 500 mL round-bottom flask, then DMF (40 mL) was added for dissolution with the assistance of ultrasonication, and the mixture was stirred at 0°C for 10 minutes. DIEA (0.53 mL, 3.24 mmol) was added dropwise, followed by the addition of succinic anhydride (0.24 g, 2.43 mmol), and the mixture was reacted with stirring at room temperature for 4 hours. After the reaction was completed, n-hexane (25 mL) and methyl tert-butyl ether (200 mL) were added for precipitation. The supernatant was discarded, and n-hexane (40 mL) and methyl tert-butyl ether (70 mL) were added again for precipitation. This process was repeated three times. The mixture was filtered to provide a solid product, which was dried in a vacuum oven to provide 0.56 g of the product. $^1$H-NMR (600 MHz, DMSO-$d_6$) δ 12.01(s, 1H), 10.28-10.02 (m, 2H), 9.39-9.17 (m, 1H), 9.04-8.83 (m, 2H), 8.66-8.49 (m, 1H), 8.40-7.71 (m, 21H), 7.63-7.37 (m, 4H), 7.34-6.97 (m,11H), 5.95-5.68 (m, 6H), 4.67-4.28 (m, 6H), 4.23-3.78 (m, 4H), 3.77-2.01(m, 67H), 2.00-1.33 (m, 26H), 1.03-0.68 (m, 18H); FT MS ESI m/z [M+Na$^+$] 2684.961

22.10 Preparation of **69-237**

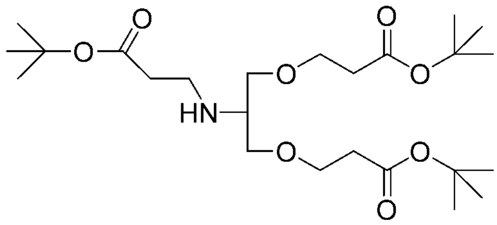

2-Amino-1,3-propanediol (3.644 g, 40.00 mmol) was weighed and placed in a reaction flask. DMSO (3 mL) was added to dissolve the mixture, and nitrogen was then purged. The mixture was stirred at 15°C. 5N NaOH solution and tert-butyl acrylate (13.16 mL, 90.6668 mmol) were added dropwise. The mixture was allowed to react at room temperature for 24 hours, and the reaction progress was monitored by TLC. After the reaction was completed, the mixture was poured into a separatory funnel containing deionized water (100 mL) and ethyl acetate (50 mL). The organic phase was collected, and the aqueous phase was further extracted with ethyl acetate (100 mL × 2). The organic phases were combined, evaporated to dryness, and then the residue was dissolved in a mixture of dichloromethane and methanol. Silica gel powder was added, and the mixture was evaporated to dryness. The resulting product was dry-loaded onto the column and subjected to column chromatography using a 20% ethyl acetate/n-hexane mixture as the eluent. The desired product was collected, concentrated, and dried in a vacuum oven to provide 2.85 g of the product. $^1$H-NMR (600 MHz, DMSO-$d_6$) δ 3.58-3.50 (m, 5H), 3.33-2.99 (m, 4H), 2.76-2.72 (m, 2H), 2.40 (t, *J* = 6.1 Hz, 4H), 2.28 (t, *J* = 6.6 Hz, 2H), 1.91 (s, 1H), 1.42-1.34 (m, 27H); FT MS ESI m/z [M+H$^+$] 476.32

22.11 Preparation of **69-242**

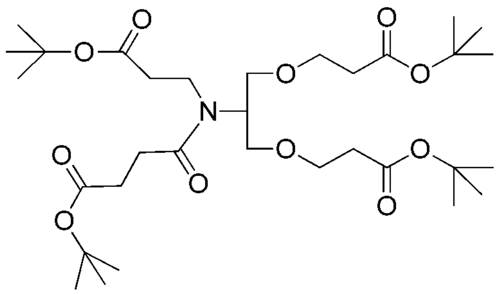

**69-237** (1.0 g, 2.1025 mmol), mono-tert-butyl succinate (0.4028 g, 2.312 mmol), HBTU (1.196 g, 3.1537 mmol), and HOBT (0.426 g, 3.1537 mmol) were placed in a 500 mL round-bottom flask, then DMF (50 mL) was added for dissolution with the assistance of ultrasonication, and the mixture was stirred at 0°C for 3 minutes. DIEA (1.563 mL, 9.4611 mmol) was then added dropwise. The mixture was then brought to room temperature and reacted with stirring. After the reaction was completed, the reaction mixture was poured into a 1 L separatory funnel containing purified water (200 mL) and ethyl acetate (200 mL). The organic phase was collected, and the aqueous phase was further extracted with ethyl acetate (100 mL × 2). The organic phases were combined and washed with saturated sodium bicarbonate solution (200 mL × 2). The resulting organic phase was collected and dried over anhydrous magnesium sulfate, filtered, concentrated, and evaporated to dryness, and the residue was dissolved in a mixture of dichloromethane and methanol. 200-300 mesh silica gel powder was added, and the mixture was evaporated to dryness. The resulting product was dry-loaded onto the column and subjected to column chromatography using a mixture of 40% ethyl acetate/petroleum ether as the eluent. The desired product was collected, concentrated, and dried to provide 1.06 g of the product. $^1$H-NMR (600 MHz, DMSO-$d_6$) δ 3.62-3.41 (m, 9H), 3.37-3.15 (m, 2H), 2.96-2.88 (m, 1H), 2.56-2.46 (m, 2H), 2.44-2.32 (m, 7H), 1.43-1.35 (m, 36H); FT-MS ESI m/z [M+H$^+$] 654.380

22.12 Preparation of **69-256**

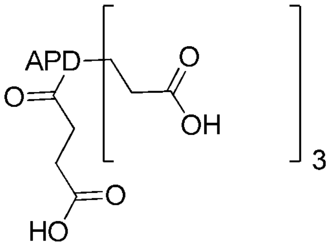

**69-242** (1.2 g, 1.899 mmol) was placed in a 500 mL round-bottom flask, then trifluoroacetic acid (8.5 mL, 113.961 mmol) was added, and the mixture was reacted with stirring at room temperature. After the reaction was completed, dichloromethane (30 mL) was added to the mixture, and then the mixture was concentrated under reduced pressure using a rotary evaporator. This operation was repeated several times until the product became a viscous oil, to which dichloromethane (10 mL) was added for dissolution, and DIEA was added to adjust pH to weakly alkaline. Methyl tert-butyl ether (100 mL) and n-hexane (20 mL) were then added for precipitation. The mixture was then filtered using a sand-core funnel and rinsed three times with methyl tert-butyl ether (100 mL). The filter cake was collected and dried to provide 0.6 g of the product. $^1$H-NMR (600 MHz, DMSO-$d_6$) δ 12.79-10.30 (m, 4H), 3.74-3.11 (m, 11H), 2.68-2.30 (m, 10H); FT-MS ESI m/z [M+H$^+$] 408.150

22.13 Preparation of **69-260**

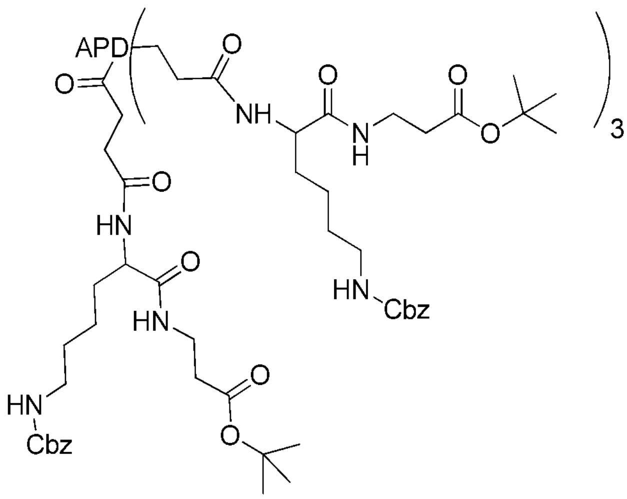

**69-256** (0.6 g, 1.472 mmol), **69-250** (2.64 g, 6.480 mmol), HBTU (3.349 g, 8.832 mmol), and HOBT (1.193 g, 8.832 mmol) were placed in a 500 mL round-bottom flask, then DMF (10 mL) was added to dissolve the mixture with the assistance of ultrasonication, and the mixture was stirred at 0°C for 2 minutes. DIEA (4.37 mL, 26.49 mmol) was then added dropwise, and the mixture was brought to room temperature and reacted with stirring. After the reaction was completed, n-hexane (25 mL) and methyl tert-butyl ether (200 mL) were added for precipitation. The supernatant was discarded, and n-hexane (40 mL) and methyl tert-butyl ether (70 mL) were added again for precipitation. This process was repeated three times. The mixture was filtered to provide a solid product. The filter cake was dissolved in a mixture of dichloromethane and methanol, then 200-300 mesh silica gel powder was added, and the mixture was evaporated to dryness. The resulting product was dry-loaded onto the column and subjected to column chromatography using a 7% methanol/dichloromethane mixture as the eluent. The desired product was collected, concentrated, and evaporated to dryness to provide 0.49 g of the product. $^1$H-NMR (600 MHz, DMSO-$d_6$) δ 8.14-7.79 (m, 8H), 7.42-7.25 (m, 20H), 7.24-7.10 (m, 4H), 5.09-4.89 (m, 8H), 4.29-4.01 (m, 4H), 3.67-3.10 (m, 19H), 3.03-2.88 (m, 8H), 2.67-2.17 (m, 18H), 1.68-1.09 (m, 60H); FT MS ESI m/z [M+Na$^+$] 1988.046

22.14 Preparation of **69-267**

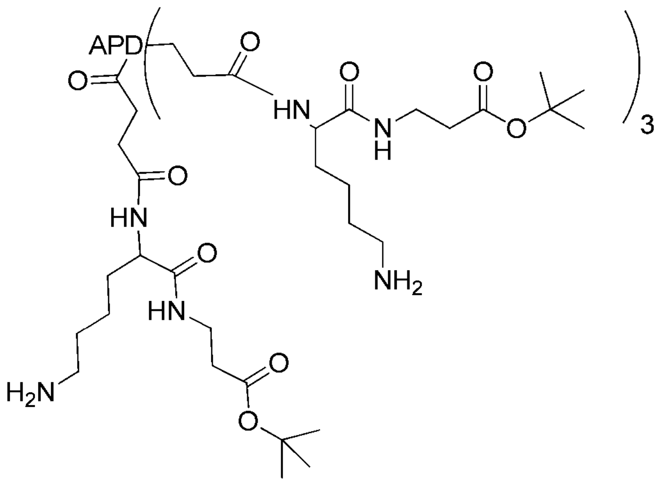

Raw material **69-260** (0.49 g, 0.249 mmol) and 10% Pd/C catalyst (200 mg) were added to a hydrogenation reactor, and then methanol (10 mL) was added for dissolution. The hydrogenation reactor was sealed, evacuated with a water pump, and then filled with hydrogen gas. This process was repeated three times. The hydrogen pressure was adjusted to 2 MPa, and the reaction was carried out with stirring at room temperature. After the reaction was completed, the mixture was filtered through a Buchner funnel containing filter paper. The reactor was rinsed with methanol (5 mL × 3). The filtrate was collected, concentrated, evaporated, and dried to provide 0.15 g of the product. FT MS ESI m/z [M+H$^+$] 1429.931

22.15 Preparation of **69-273**

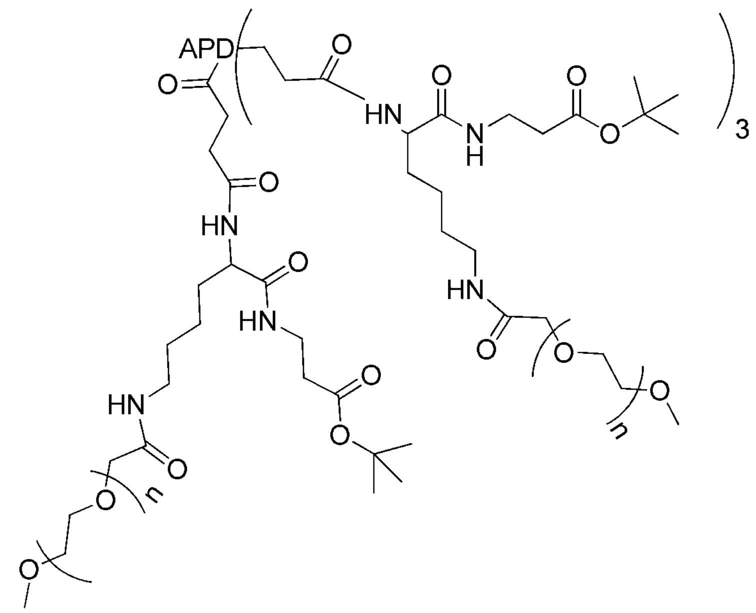

**69-267** (0.15 g, 0.10498 mmol) was placed in a 500 mL round-bottom flask, then extra-dry DMF (15 mL) was added for dissolution with the assistance of ultrasonication, and the mixture was stirred at 0°C for 5 minutes. DIEA (2.77 mL, 16.76 mmol) was added dropwise, followed by the addition of M-SCM-10K (4.47 g, 0.419 mmol, purchased from Jenkem, Lot Number: A3016-N230101), and the reaction was stirred at low speed in the dark at room temperature. After the reaction was completed, n-hexane (25 mL) and methyl tert-butyl ether (200 mL) were added for precipitation. The supernatant was discarded, and n-hexane (40 mL) and methyl tert-butyl ether (70 mL) were added again for precipitation. This process was repeated three times. The mixture was filtered to provide a solid product, and the filter cake was dissolved in a mixture of dichloromethane and methanol, then 200-300 mesh silica gel powder was added, and the mixture was evaporated to dryness. The resulting product was dry-loaded onto the column and subjected to column chromatography using a mixture of 1% ammonia water/8-10% methanol/dichloromethane as the eluent. The desired product was collected, concentrated, and evaporated to dryness to provide 4.12 g of the product. $^1$H-NMR (600 MHz, DMSO-$d_6$) δ 8.14-7.79 (m, 9H), 7.65-7.60 (m, 3H), 4.29-4.01 (m, 4H), 3.67-3.10 (m, 3831H), 3.03-2.88 (m, 8H), 2.67-2.17 (m, 18H), 1.68-1.09 (m, 60H)

22.16 Preparation of **69-279**

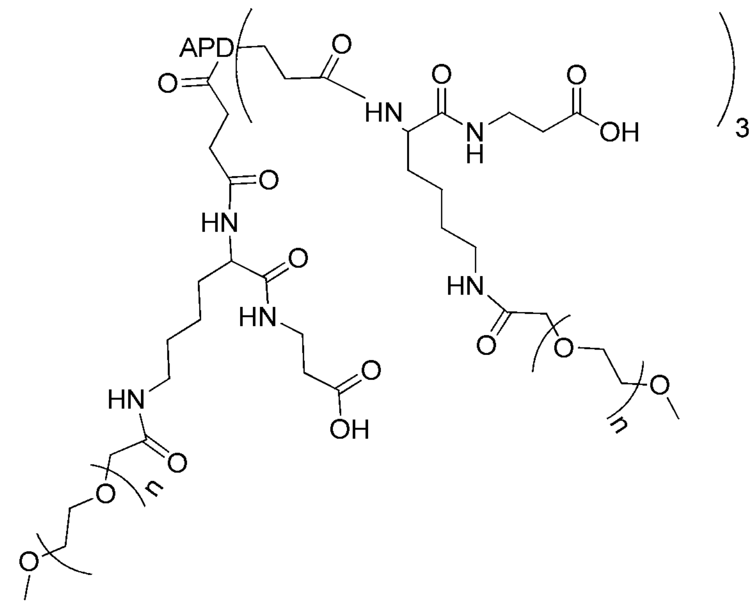

**69-273** (2.354 g, 0.4512 mmol) was placed in a 500 mL round-bottom flask, then trifluoroacetic acid (40 mL) was added, and the mixture was reacted with stirring at room temperature. After the reaction was completed, dichloromethane (60 mL) was added to the mixture for dissolution, and then the mixture was concentrated under reduced pressure. This process was repeated until the product became a viscous oil, to which dichloromethane (20 mL) was then added for dissolution, and DIEA was added to adjust pH to weakly alkaline. Methyl tert-butyl ether (200 mL) and n-hexane (50 mL) were then added for precipitation. The mixture was filtered using a sand-core funnel and rinsed with methyl tert-butyl ether (100 mL × 3). The filter cake was collected and dried to provide 2.12 g of the product.

22.17 Preparation of **69-286**

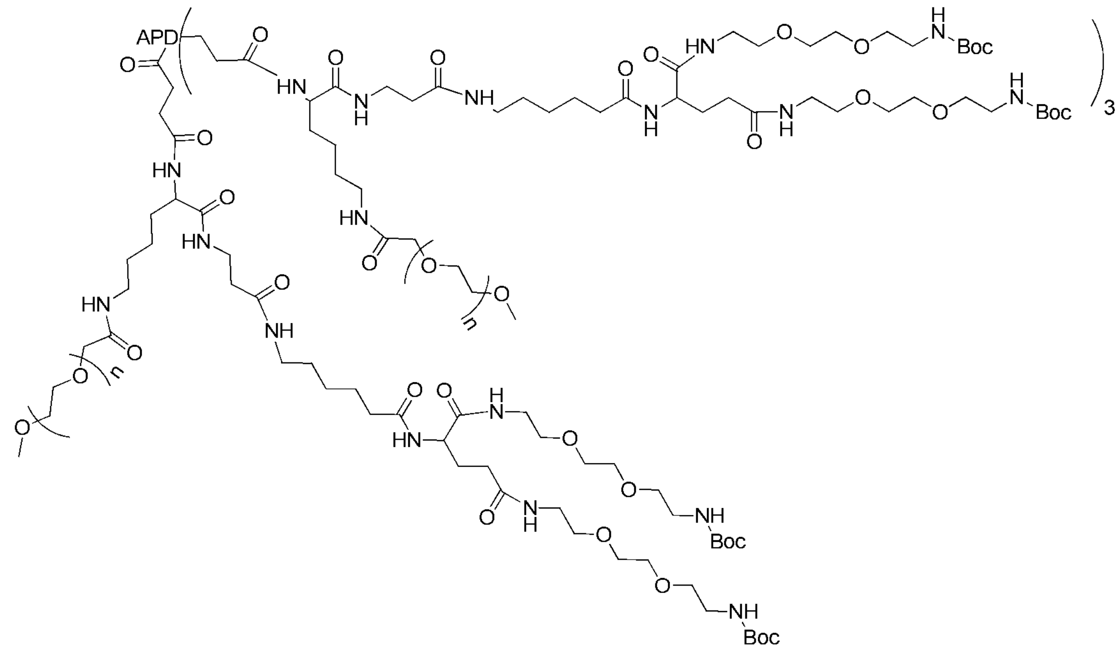

**69-279** (2.1 g, 0.0480 mmol), **69-274** (0.207 g, 0.288 mmol), HBTU (0.109 g, 0.288 mmol), and HOBT (0.0389 g, 0.288 mmol) were added to a 500 mL round-bottom flask, then DMF (15 mL) was added for dissolution with the assistance of ultrasonication, and the mixture was stirred at 0°C for 2 minutes. DIEA (0.142 mL, 0.864 mmol) was then added dropwise, and the mixture was brought to room temperature and reacted with stirring. After the reaction was completed, n-hexane (25 mL) and methyl tert-butyl ether (200 mL) were added for precipitation. The supernatant was discarded, and n-hexane (40 mL) and methyl tert-butyl ether (70 mL) were added again for precipitation. This process was repeated three times. The mixture was filtered to provide a solid product, and the filter cake was dissolved in a mixture of dichloromethane and methanol. 100-200 mesh silica gel powder was added, and the mixture was evaporated to dryness. The resulting product was dry-loaded onto the column and subjected to column chromatography using a 7% methanol/dichloromethane mixture as the eluent. The desired product was collected, concentrated, and evaporated to dryness to provide 2.15 g of the product. $^1$H-NMR (600 MHz, DMSO-$d_6$) δ 8.14-7.79 (m, 21H), 7.65-7.60 (m, 3H), 6.83-6.75 (m, 8H), 4.29-4.01 (m, 8H), 3.67-3.10 (m, 3915H), 3.10-3.03 (m, 16H), 3.03-2.88 (m, 8H), 2.69-2.17 (m, 26H), 2.13-2.02 (m, 16H), 1.86-1.78 (m, 4H), 1.74-1.09 (m, 124H)

22.18 Preparation of **69-290**

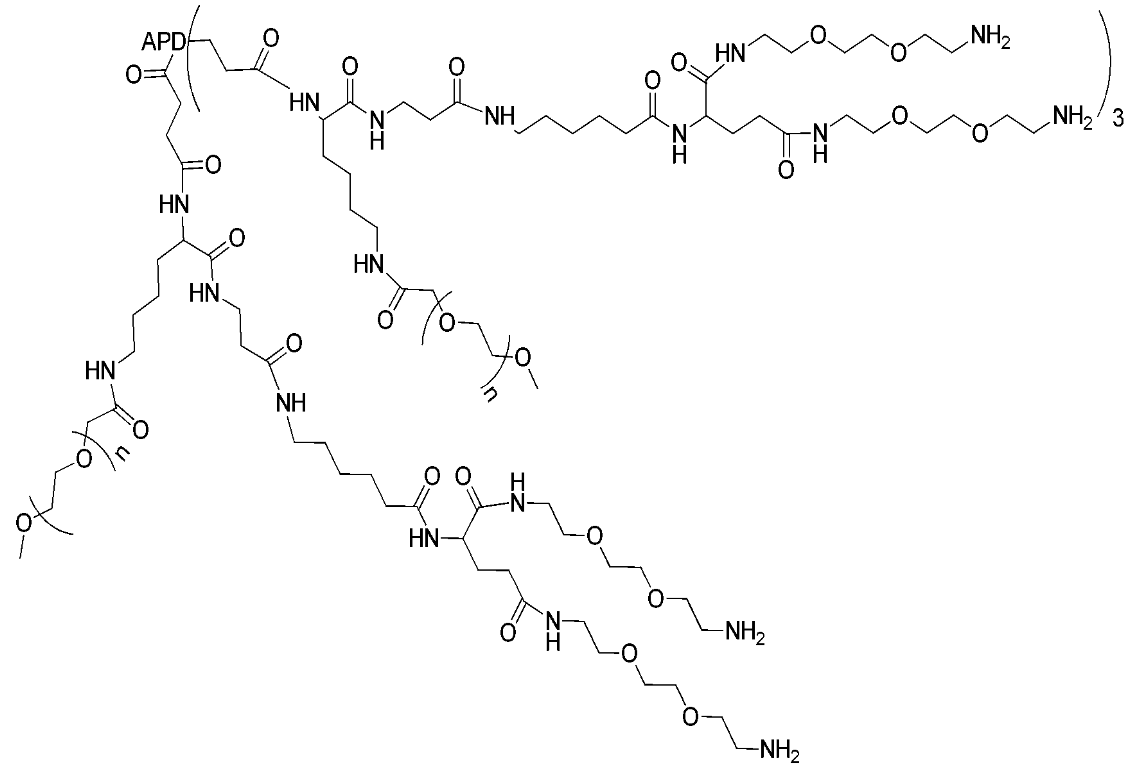

**69-286** (1.432 g, 0.035 mmol) was placed in a 500 mL round-bottom flask, then trifluoroacetic acid (20 mL) was added, and the mixture was reacted with stirring at room temperature. After the reaction was completed, dichloromethane (60 mL) was added, and the mixture was concentrated under reduced pressure. This process was repeated until the product became a viscous oil, which was then dissolved in dichloromethane (20 mL), then DIEA was added to adjust pH to weakly alkaline. Then, methyl tert-butyl ether (200 mL) and n-hexane (50 mL) were added for precipitation. The mixture was filtered using a sand-core funnel and rinsed with methyl tert-butyl ether (100 mL × 3). The filter cake was collected, and dried to provide 1.12 g of the product.

22.19 Preparation of 86-2

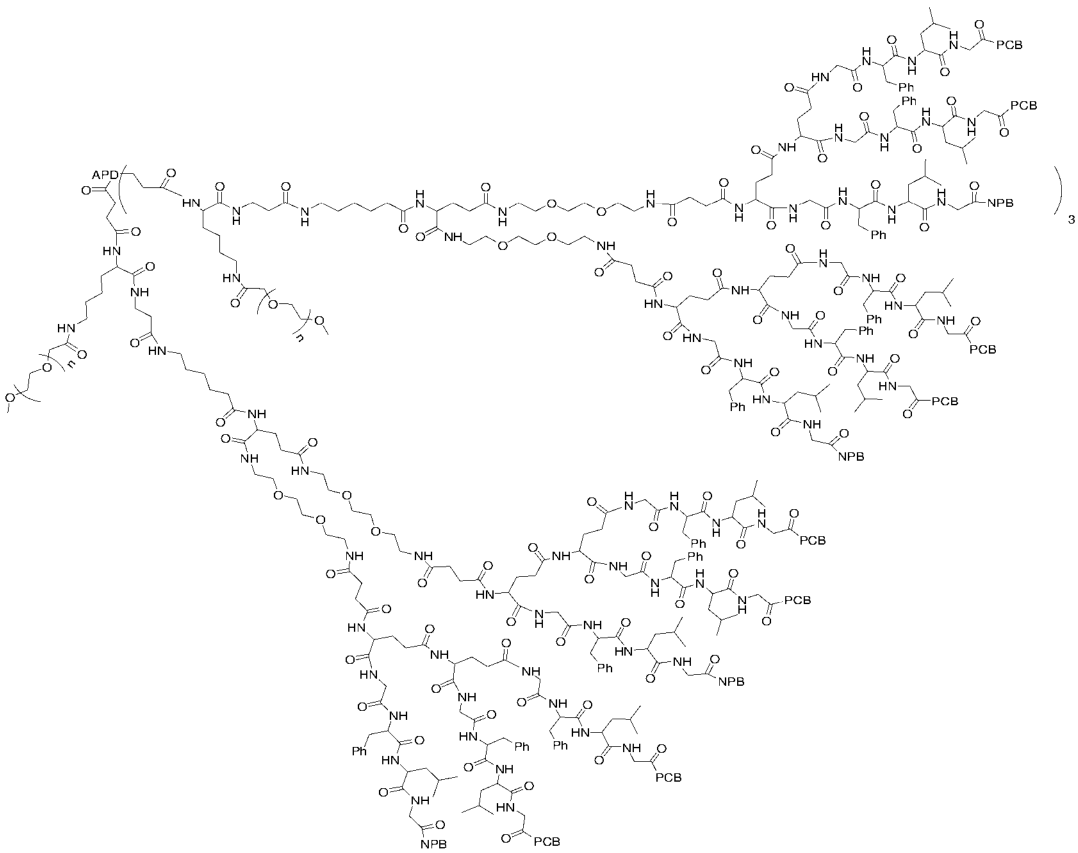

**69-290** (1.11 g, 0.0241 mmol), **69-282** (0.7714 g, 0.2899 mmol), HBTU (0.109 g, 0.2899 mmol), and HOBT (0.039 g, 0.2899 mmol) were placed in a 500 mL round-bottom flask, then DMF (50 mL) was added for dissolution with the assistance of ultrasonication, and the mixture was stirred at 0°C for 2 minutes. DIEA (0.143 mL, 0.8697 mmol) was then added dropwise, and the mixture was brought to room temperature and reacted with stirring. After the reaction was completed, n-hexane (25 mL) and methyl tert-butyl ether (200 mL) were added for precipitation. The supernatant was discarded, and n-hexane (40 mL) and methyl tert-butyl ether (70 mL) were added again for precipitation. This process was repeated three times. The mixture was filtered to provide a solid product, and the filter cake was dissolved in a mixture of dichloromethane and methanol, then 100-200 mesh silica gel powder was added, and the mixture was evaporated to dryness. The resulting product was dry-loaded onto the column and subjected to column chromatography using a 10% methanol/dichloromethane mixture as the eluent. The desired product was collected, concentrated, and evaporated to dryness to provide 1.09 g of the product. $^1$H-NMR (600 MHz, DMSO-$d_6$) δ 10.30-10.00 (m, 16H), 9.39-9.16 (m, 8H), 9.04-8.83 (m, 16H), 8.66-8.49 (m, 8H), 8.40-7.71 (m, 189H), 7.65-7.60 (m, 35H), 7.34-6.97 (m, 88H), 6.83-6.75 (m, 8H), 5.95-5.68 (m, 54H), 4.67-4.28 (m, 54H), 4.28-3.78 (m, 40H), 3.77-2.01 (m, 4505H), 2.00-1.09 (m, 264H), 1.05-0.67 (m, 144H)

Example 23: Synthesis of Compound **84-106**

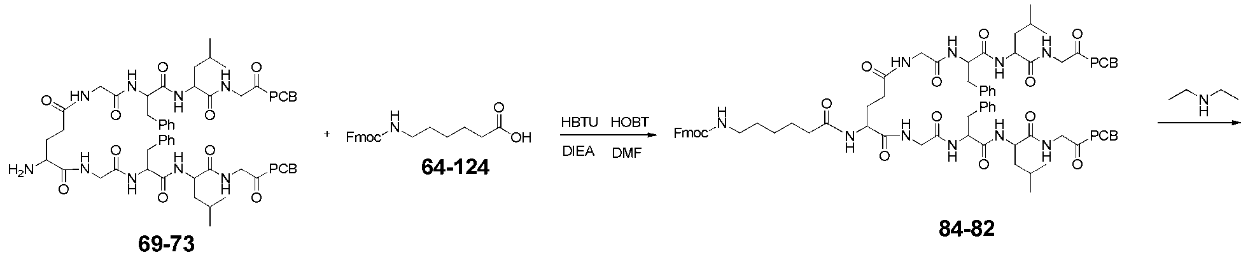


64-124

69-73

84-82

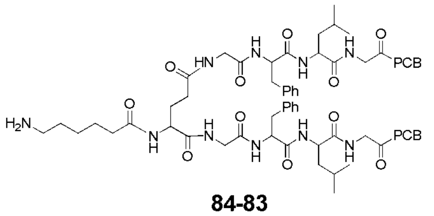

84-83

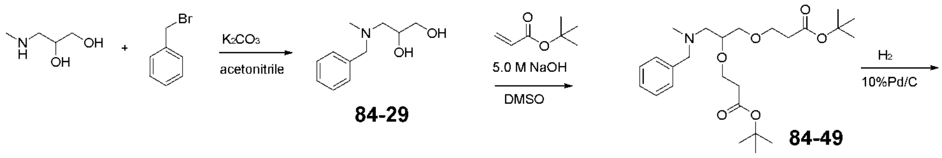


84-29

84-49

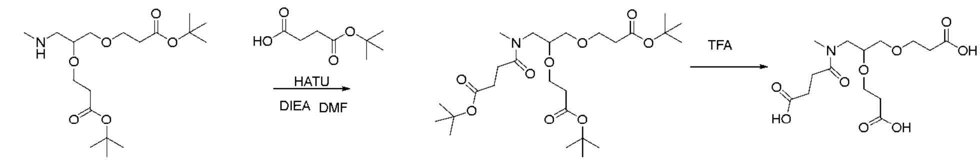


84-50

84-54

84-70

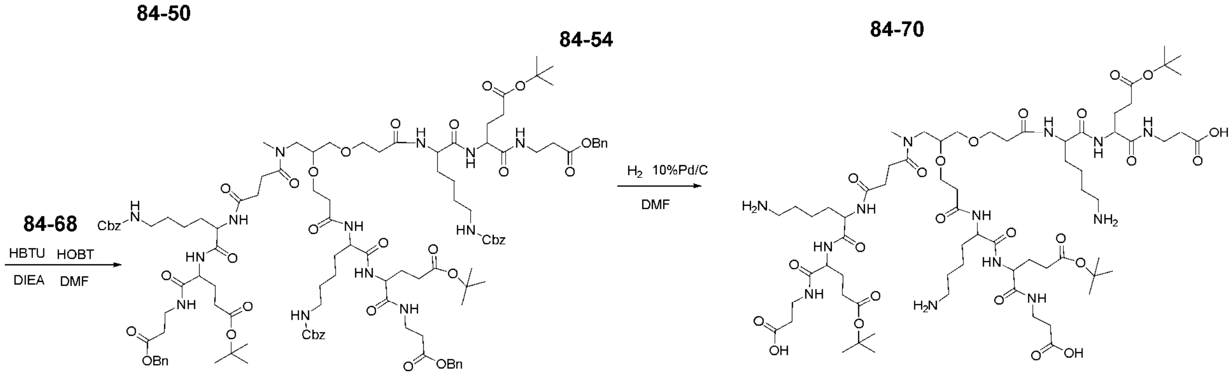


84-68

84-75

84-81

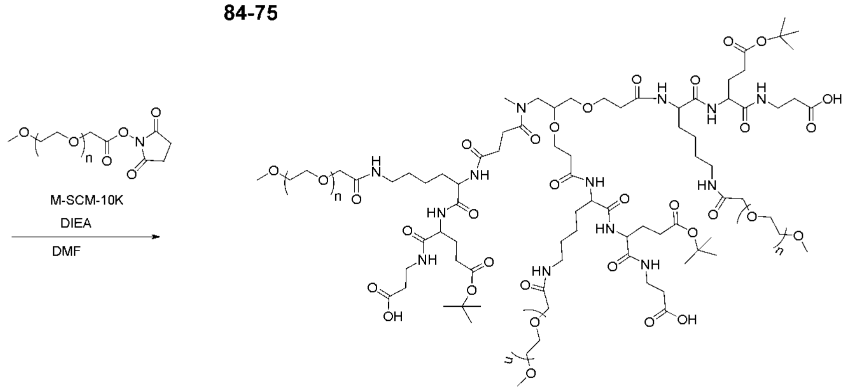


84-88

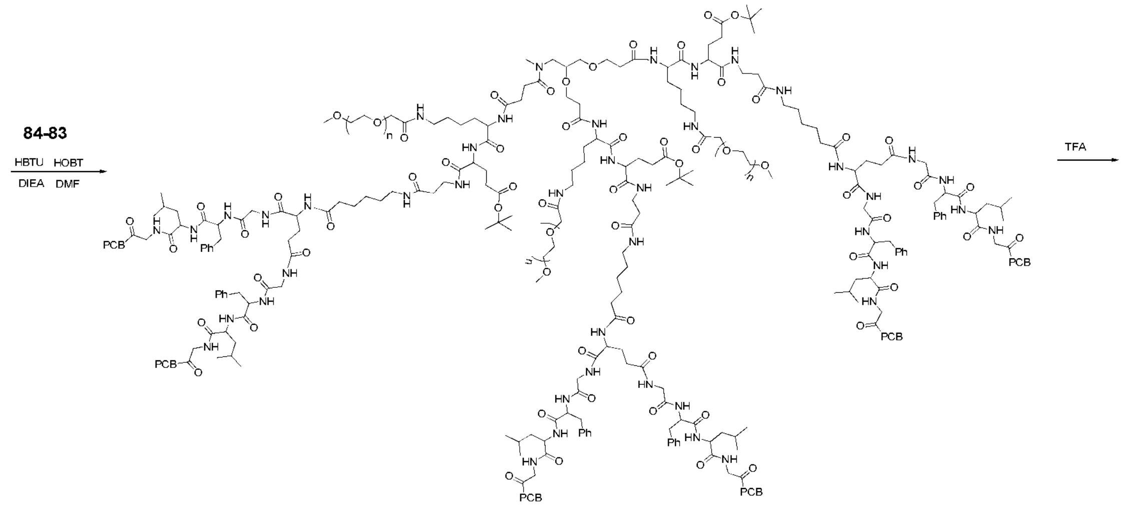


84-94

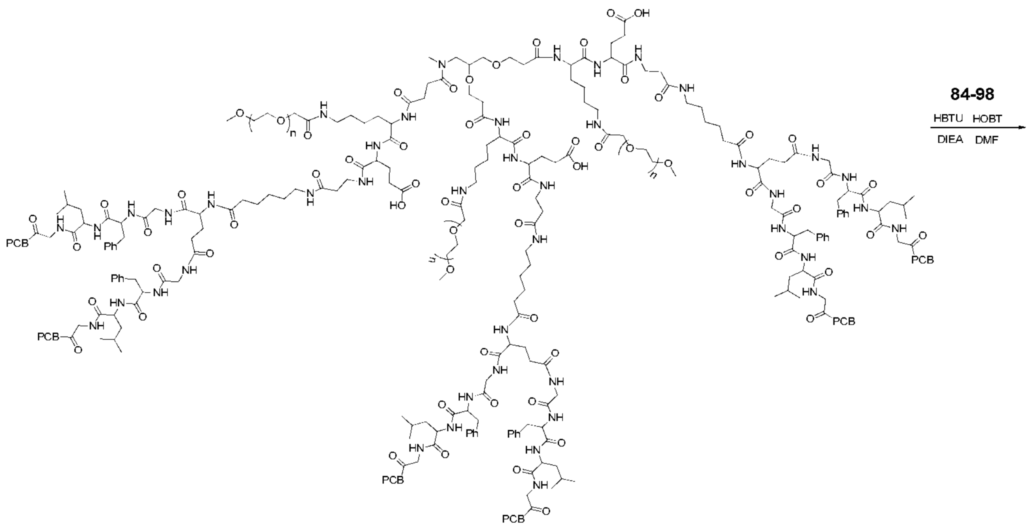


84-99

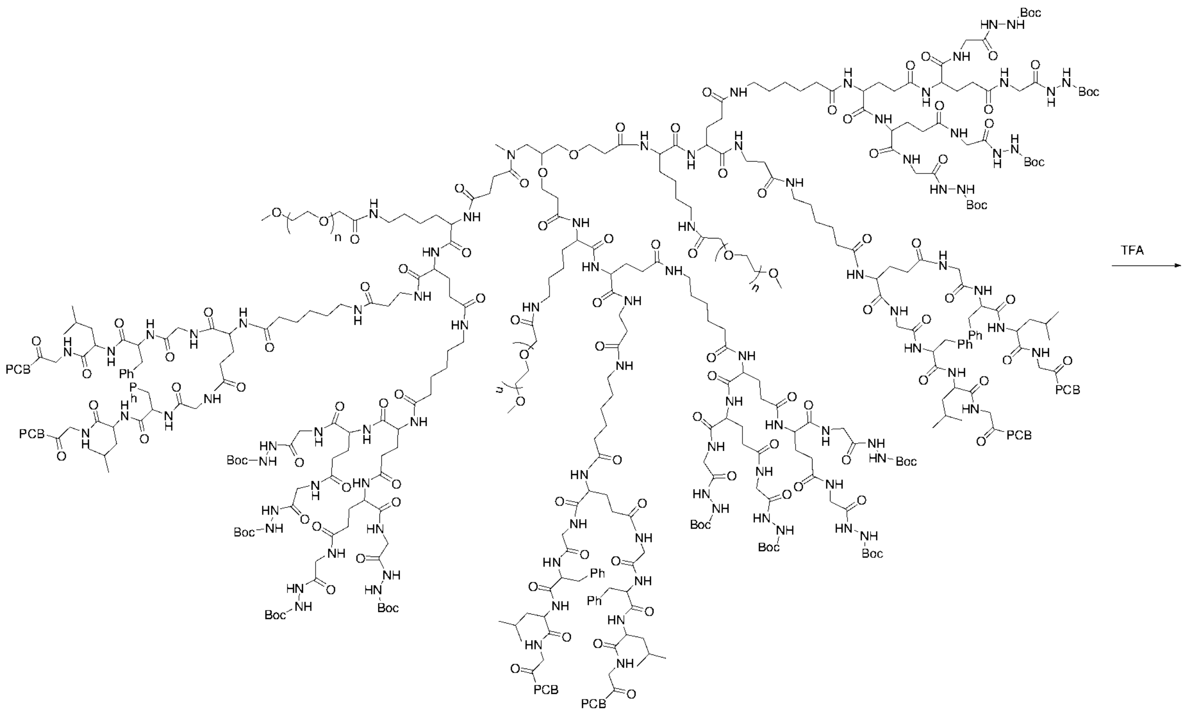


84-100

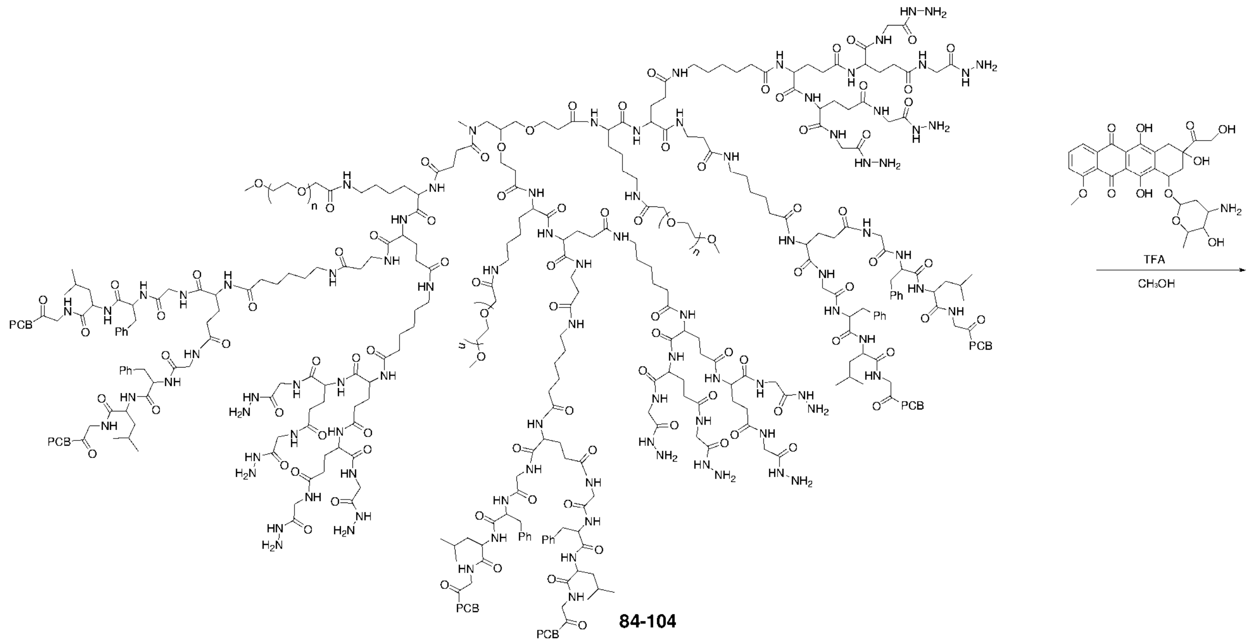


84-104

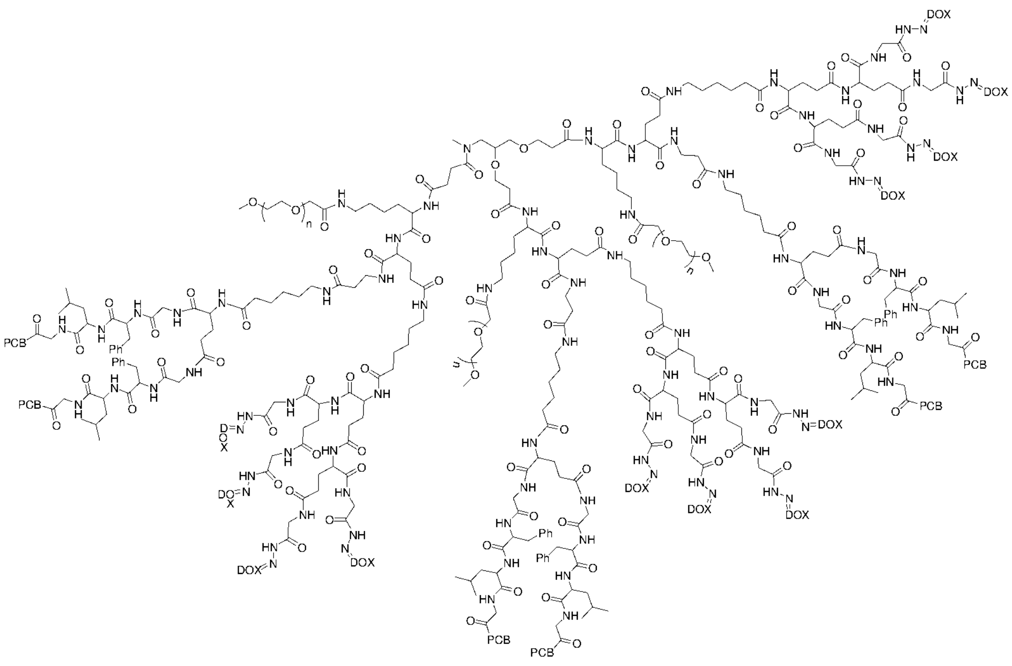

84-106

23.1 Preparation of **84-82**

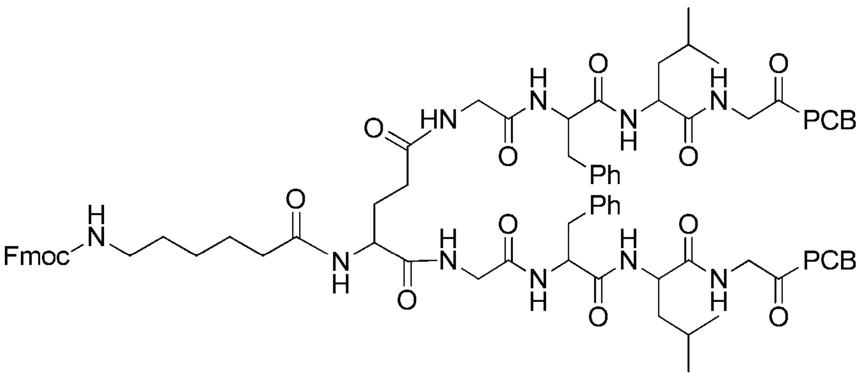

**64-124** (0.57 g, 1.18 mmol), **69-73** (2 g, 2.43 mmol), HBTU (1.17 g, 3.08 mmol), and HOBT (0.42 g, 3.08 mmol) were added to a 500 mL round-bottom flask, then DMF (10 mL) was added to dissolve the mixture with the assistance of ultrasonication, and the mixture was stirred at 0°C for 1 minute. DIEA (1.2 mL, 7.12 mmol) was then added dropwise, and the mixture was brought to room temperature and reacted with stirring. After the reaction was completed, n-hexane (25 mL) and methyl tert-butyl ether (200 mL) were added for precipitation. The supernatant was discarded, and n-hexane (20 mL) and methyl tert-butyl ether (100 mL) were added again for precipitation. This process was repeated three times. The mixture was filtered to provide a solid product, and the filter cake was dried to provide 2.48 g of the product.

23.2 Preparation of **84-83**

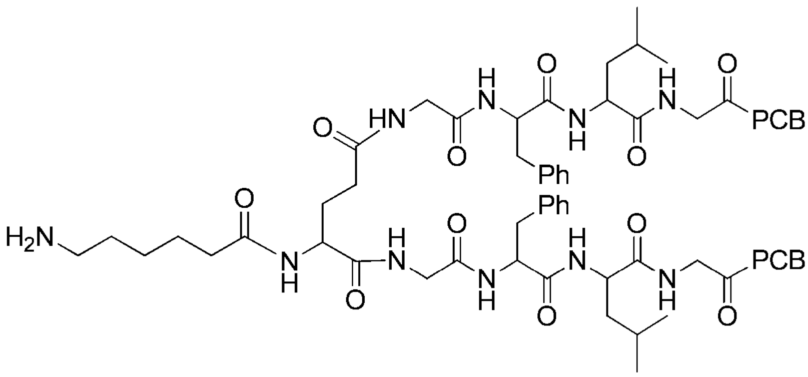

**84-82** (2.48 g, 1.18 mmol) was placed in a 500 mL round-bottom flask. DMF (30 mL) was added for dissolution with the assistance of ultrasonication, then diethylamine (30 mL) was added, and the mixture was reacted with stirring at room temperature. After the reaction was completed, methyl tert-butyl ether (300 mL) was added for precipitation. A solid precipitated and was filtered. The filter cake was redissolved in DMF (10 mL), and methyl tert-butyl ether (150 mL) was added. A solid precipitated and was filtered. The filter cake was dried to provide 2.2 g of the product.

23.3 Preparation of **84-29**

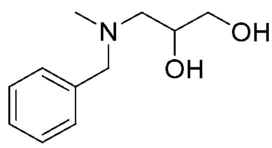

3-(Methylamino)propane-1,2-diol (5 g, 47.55 mmol), benzyl bromide (12.19 g, 71.325 mmol), and potassium carbonate (13.14 g, 95.1 mmol) were added to a 500 mL round-bottom flask, then acetonitrile (30 mL) was added for dissolution with the assistance of ultrasonication, and the mixture was stirred at 30°C. After the reaction was completed, the mixture was filtered, and the filtrate was collected, concentrated under reduced pressure, then dissolved in a

methanol/dichloromethane mixture. 200-300 mesh silica gel powder (20 g) was added, and the mixture was evaporated to dryness. The resulting product was dry-loaded onto the column and subjected to column chromatography using a 1% aqueous ammonia/1% methanol/dichloromethane mixture as the eluent. The desired product was collected, concentrated, and evaporated to dryness to provide 5.61 g of the product.

23.4 Preparation of **84-49**

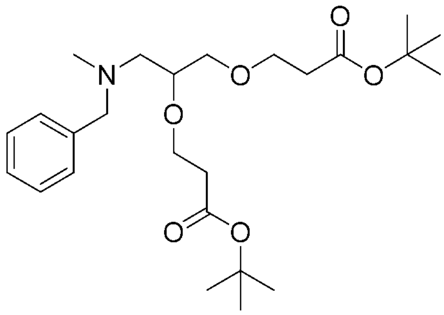

**84-29** (4.27 g, 21.8694 mmol) was added to a 250 mL round-bottom flask, then DMSO (16 mL) was added for dissolution with the assistance of ultrasonication. Then 5N sodium hydroxide solution (1 mL) was added, and the mixture was dissolved with the assistance of ultrasonication. Tert-butyl acrylate (6.72 g, 52.4865 mmol) was then added dropwise, and reacted with stirring at room temperature. After the reaction was completed, the mixture was poured into a separatory funnel containing pure water (100 mL) and ethyl acetate (100 mL) for extraction. The organic phase was collected, and the aqueous phase was further extracted with ethyl acetate (100 mL × 2). The organic phases were combined, extracted with saturated sodium bicarbonate solution (200 mL × 2). The resulting organic phase was collected, dried over anhydrous magnesium sulfate, filtered, and the filtrate was collected. 200-300 mesh silica gel powder (20 g) was added, and the mixture was evaporated to dryness. The resulting product was dry-loaded onto the column and subjected to column chromatography using a 20% ethyl acetate/petroleum ether mixture as the eluent. The desired product was collected, concentrated, and evaporated to dryness to provide 2.97 g of the product.

23.5 Preparation of **84-50**

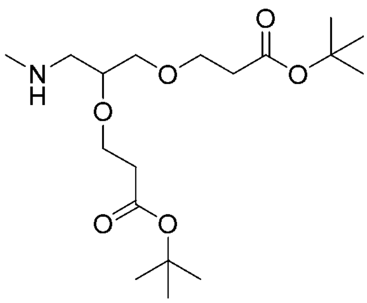

Raw material 84-49 (2.97 g, 6.5767 mmol) and 10% Pd/C catalyst (400 mg) were added to a hydrogenation reactor, then methanol (20 mL) was added for dissolution. The hydrogenation reactor was sealed, evacuated with a water pump, and then filled with hydrogen gas. This process was repeated three times. The hydrogen pressure was adjusted to 2 MPa, and the reaction was carried out with stirring at room temperature. After the reaction was completed, the mixture was filtered through a Buchner funnel containing filter paper. The reactor was rinsed with methanol (5 mL × 3). The filtrate was collected, concentrated under reduced pressure, and evaporated to dryness to provide 2.3 g of the product.

23.6 Preparation of **84-54**

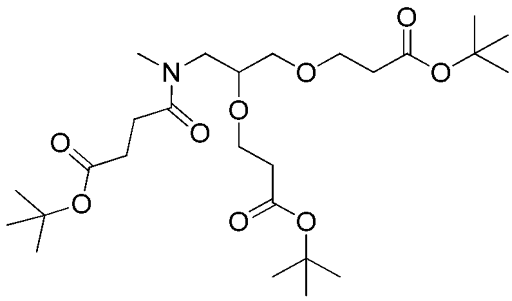

**84-50** (2.3 g, 6.3629 mmol), mono-tert-butyl succinate (1.1 g, 6.3629 mmol), HBTU (3.1 g, 8.27 mmol), and HOBT (1.12 g, 8.27 mmol) were added to a 250 mL round-bottom flask, then DMF (15 mL) was added for dissolution with the assistance of ultrasonication, and the mixture was stirred at 0°C for 2 minutes. DIEA (4.2 mL, 25.4516 mmol) was then added dropwise, and the mixture was brought to room temperature and reacted with stirring. After the reaction was completed, the mixture was poured into a separatory funnel containing pure water (100 mL) and ethyl acetate (100 mL) for extraction. The organic phase was collected, and the aqueous phase was further extracted with ethyl acetate (100 mL × 2). The organic phases were combined, washed with saturated sodium bicarbonate solution (50 mL × 2). The resulting organic phase was collected, dried over anhydrous magnesium sulfate, filtered, and the filtrate was collected, concentrated, then dissolved in a methanol/dichloromethane mixture. 200-300 mesh silica gel powder (20 g) was added, and the mixture was evaporated to dryness. The resulting product was dry-loaded onto the column and subjected to column chromatography using a 30% ethyl acetate/petroleum ether mixture as the eluent. The desired product was collected, concentrated, and evaporated to dryness to provide 2.1 g of the product. $^1$H-NMR (600 MHz, DMSO-$d_6$) δ 3.71-3.49 (m, 5H), 3.47-3.14 (m, 4H), 3.01-2.78 (s, 3H), 2.58-2.30 (m, 8H), 1.44-1.33 (m, 27H); FT-MS ESI m/z [M+H$^+$] 518.33, [M+Na$^+$] 540.31

23.7 Preparation of **84-70**

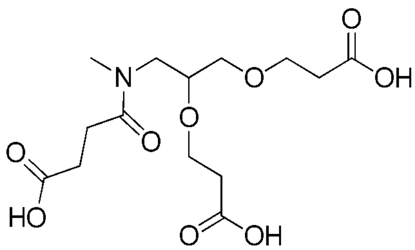

**84-54** (1 g, 1.93 mmol) was placed in a 500 mL round-bottom flask, then trifluoroacetic acid (10 mL) was added, and the mixture was reacted with stirring at room temperature. The reaction mixture was concentrated under reduced pressure to remove dichloromethane and most of the TFA. Methyl tert-butyl ether (200 mL) and n-hexane (50 mL) were then added for precipitation. The mixture was filtered using a sand-core funnel and rinsed with methyl tert-butyl ether (100 mL × 3). The filter cake was collected and dried to provide 0.67 g of the product.

23.8 Preparation of **84-75**

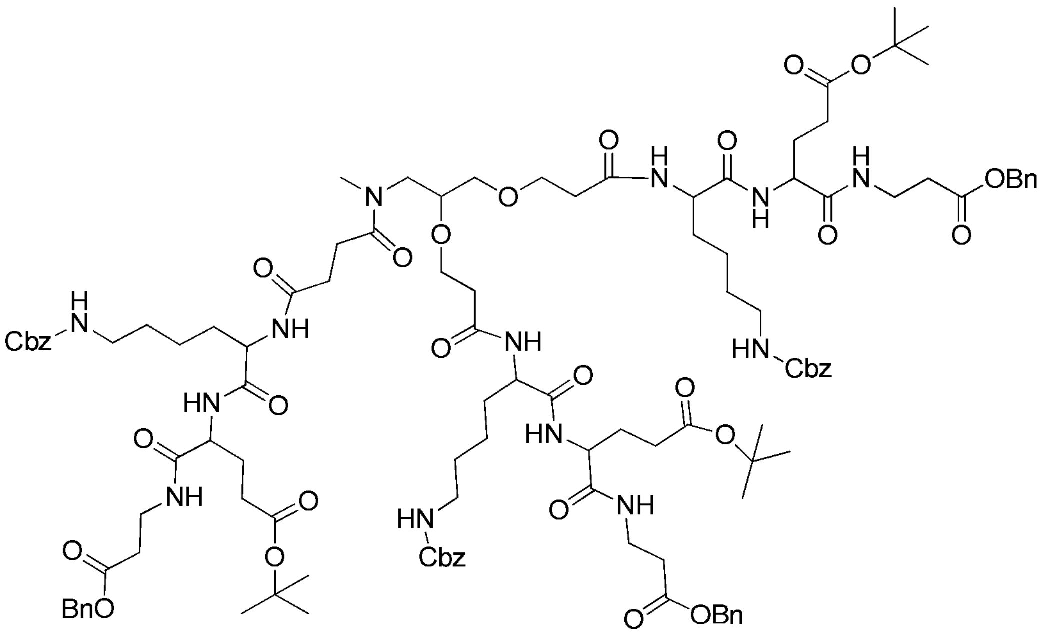

**84-70** (0.67 g, 1.9317 mmol), **84-68** (3.9 g, 6.3746 mmol), HBTU (2.86 g, 7.5336 mmol), and HOBT (1 g, 7.5336 mmol) were added to a 100 mL round-bottom flask, then DMF (15 mL) was added for dissolution with the assistance of ultrasonication, and the mixture was stirred at 0°C for 2 minutes. DIEA (8.35 mL, 50.54 mmol) was then added dropwise, and the mixture was brought to room temperature and reacted with stirring. After the reaction was completed, n-hexane (25 mL) and methyl tert-butyl ether (200 mL) were added for precipitation. The supernatant was discarded, and n-hexane (20 mL) and methyl tert-butyl ether (100 mL) were added again for precipitation. This process was repeated three times. The mixture was filtered, and the filter cake was dissolved in 10% methanol/dichloromethane (50 mL), then 200-300 mesh silica gel powder (15 g) was added, and the mixture was evaporated to dryness. The resulting product was dry-loaded onto the column and subjected to column chromatography using a 4% methanol/dichloromethane mixture as the eluent. The desired product was collected, concentrated, and dried to provide 1.56 g of the product. $^1$H-NMR (600 MHz, DMSO-$d_6$) δ 8.32-8.16 (m, 1H), 8.06-7.87 (m, 6H), 7.88-7.75 (m, 1H), 7.69-7.53 (m, 1H), 7.43-7.26 (m, 30H), 7.25-7.15 (m, 3H), 5.17-4.91 (m, 12H), 4.28-4.11 (m, 5H), 4.10-4.00 (m, 1H), 3.79-3.64 (m, 1H), 3.64-3.49 (m, 6H), 3.45-3.16 (m, 11H), 3.04-2.86 (m, 8H), 2.69-2.25 (m, 12H), 2.25-2.09 (m, 6H), 2.03-1.88 (m, 1H), 1.88-1.79 (m, 2H), 1.79-1.16 (m, 48H); FT MS ESI m/z [M+Na$^+$] 2198.077

23.9 Preparation of **84-81**

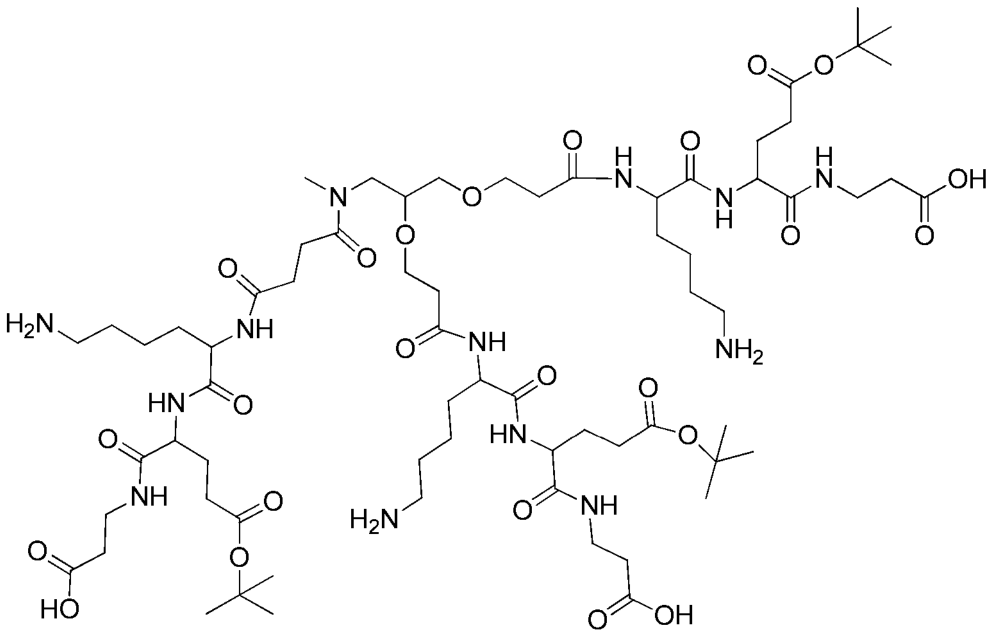

**84-75** (0.5 g, 0.22 mmol) and 10% Pd/C catalyst (120 mg) were added to a hydrogenation reactor, and then methanol (20 mL) was added for dissolution. The hydrogenation reactor was sealed, evacuated with a water pump, and then filled with hydrogen gas. This process was repeated three times, and the hydrogen pressure was adjusted to 2 MPa. The reaction was then carried out with stirring at room temperature. After the reaction was completed, the mixture was filtered through a Buchner funnel containing filter paper. The reactor was rinsed with methanol (10 mL × 3). The filtrate was collected, concentrated under reduced pressure. Then dichloromethane (10 mL) and toluene (1 mL) were added, and the mixture was subjected to azeotropic evaporation to dryness. This process was repeated five times. Finally, the resulting product was dried in a vacuum oven to provide 0.34 g of the product. $^1$H-NMR (600 MHz, DMSO-$d_6$) δ 8.58-7.99 (m, 6H), 7.85-7.65 (m, 2H), 7.65-7.50 (m, 1H), 4.38-4.08 (m, 2H), 3.81-3.08 (m, 28H), 3.01-2.91 (m, 2H), 2.84-2.65 (m, 6H), 2.63-1.87 (m, 24H), 1.81-1.19 (m, 48H)

23.10 Preparation of **84-88**

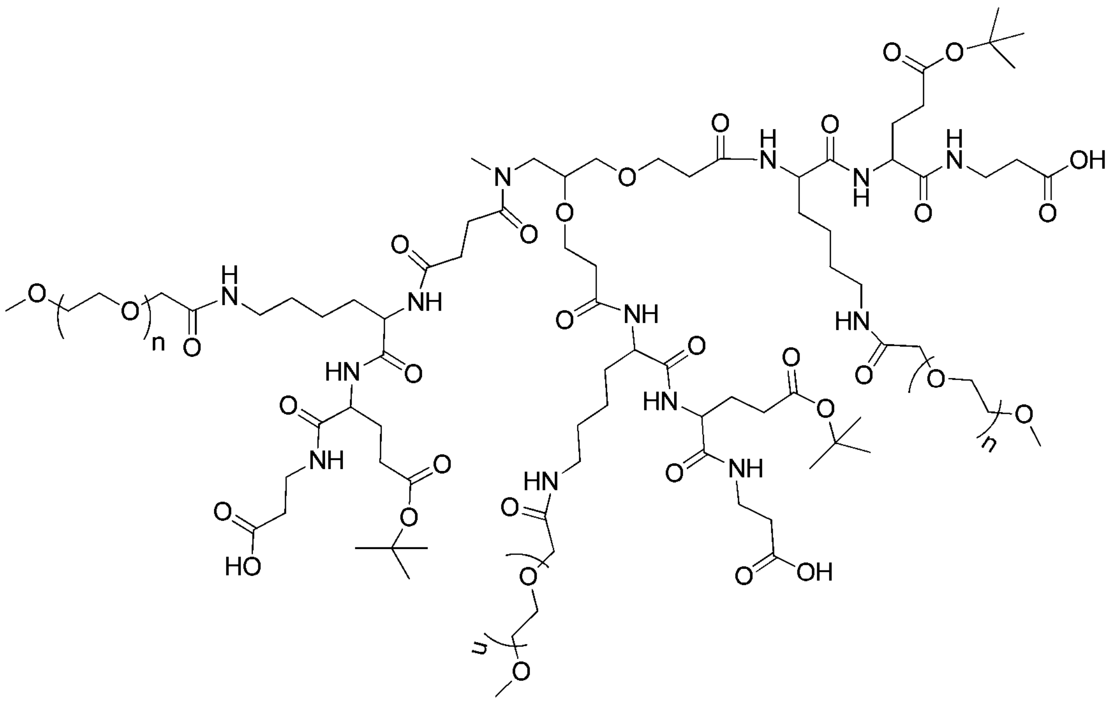

**84-81** (0.14 g, 0.0939 mmol) was placed in a 500 mL round-bottom flask, then extra-dry DMF (20 mL) was added for dissolution with the assistance of ultrasonication, and the mixture was stirred at 0°C for 5 minutes. DIEA (1 mL, 5.634 mmol) was added dropwise, followed by the addition of M-SCM-10K (3 g, 0.2816 mmol, purchased from Jenkem, Lot Number A3016-N230101). The air in the flask was replaced with nitrogen gas, and the reaction was carried out with stirring at low speed in the dark at 35°C. After the reaction was completed, n-hexane (25 mL) and methyl tert-butyl ether (200 mL) were added for precipitation. The supernatant was discarded, and n-hexane (20 mL) and methyl tert-butyl ether (100 mL) were added again for precipitation. This process was repeated three times. The mixture was subjected to suction filtration, and the filter cake was dissolved in dichloromethane (50 mL), then 100-200 mesh silica gel powder (15 g) was added, and the mixture was evaporated to dryness. The resulting product was dry-loaded onto the column and subjected to column chromatography using a mixture of 1% ammonia water/8-12% methanol/dichloromethane as the eluent. The desired product was collected, concentrated and dried to provide 1.6 g of the product. $^1$H-NMR (600 MHz, DMSO-$d_6$) δ 8.57-7.99 (m, 7H), 7.85-7.65 (m, 4H), 7.65-7.50 (m, 1H), 4.38-4.08 (m, 2H), 3.81-3.06 (m, 2881H), 3.01-2.89 (m, 2H), 2.84-2.65 (m, 6H), 2.65-1.87 (m, 24H), 1.82-1.18 (m, 48H)

23.11 Preparation **84-94**

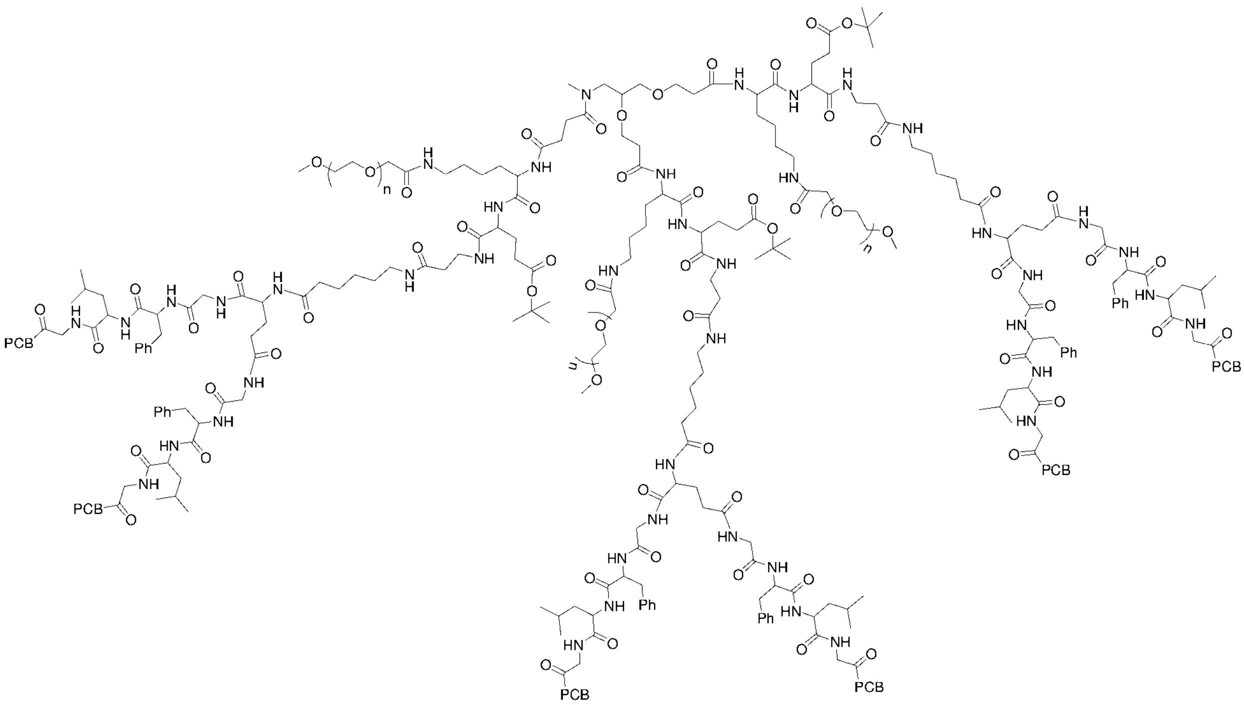

**84-88** (1.6 g, 0.0484 mmol), **84-83** (0.4 g, 0.2178 mmol), HBTU (0.08 g, 0.2178 mmol), and HOBT (0.03 g, 0.2178 mmol) were added to a 100 mL round-bottom flask, then DMF (20 mL) was added for dissolution with the assistance of ultrasonication, and the mixture was stirred at 0°C for 2 minutes. DIEA (0.1 mL, 0.58 mmol) was then added dropwise, and the mixture was brought to room temperature and reacted with stirring. After the reaction was completed, n-hexane (25 mL) and methyl tert-butyl ether (200 mL) were added for precipitation. The supernatant was discarded, and n-hexane (20 mL) and methyl tert-butyl ether (100 mL) were added again for precipitation. This process was repeated three times. The mixture was filtered, and the filter cake was dissolved in dichloromethane (50 mL), then 100-200 mesh silica gel powder (15 g) was added, and the mixture was evaporated to dryness. The resulting product was dry-loaded onto the column and subjected to column chromatography using a mixture of 1% ammonia water/8-12% methanol/dichloromethane as the eluent. The desired product was collected, concentrated, and dried to provide 1.3 g of the product. $^1$H-NMR (600 MHz, DMSO-$d_6$) δ 10.41-9.88 (m, 6H), 9.07-8.78 (m, 6H), 8.60-7.99 (m, 31H), 7.99-7.46 (m, 26H), 7.31-7.07 (m, 30H), 5.89-5.77 (m, 6H), 4.60-4.51 (m,6H), 4.42-4.08 (m, 11H), 4.08-3.06 (m, 2962H), 3.01-2.65 (m, 14H), 2.65-1.87 (m, 102H), 1.83-1.18 (m, 111H), 0.92-0.79 (m, 36H)

23.12 Preparation of **84-99**

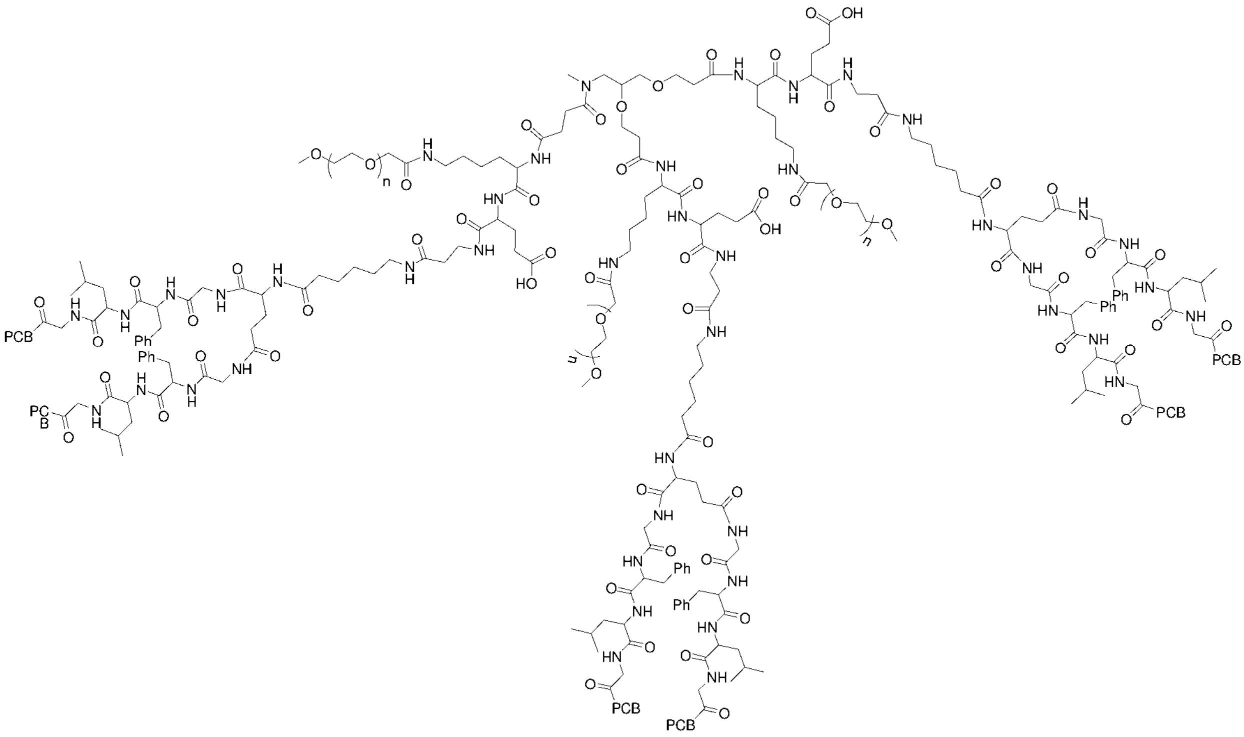

**84-94** (1.18 g, 0.0306 mmol) was placed in a 500 mL round-bottom flask, then trifluoroacetic acid (3 mL) was added, and the mixture was reacted with stirring at room temperature. After the reaction was completed, dichloromethane (30 mL) was added, and the dichloromethane and most of the trifluoroacetic acid were removed by rotary evaporation under reduced pressure. Methyl tert-butyl ether (300 mL) was then added for precipitation. A solid precipitated and was filtered. The filter cake was dissolved in 20% methanol/dichloromethane (15 mL), and then methyl tert-butyl ether (300 mL) was added for precipitation. A solid precipitated and was filtered. This process was repeated three times. The filter cake was collected and dried to provide 1.15 g of the product. $^1$H-NMR (600 MHz, DMSO-$d_6$) δ 12.23-11.80 (m, 3H), 10.42-9.87 (m, 6H), 9.07-8.78 (m, 6H), 8.60-7.99 (m, 31H), 7.99-7.46 (m, 26H), 7.31-7.07 (m, 30H), 5.89-5.76 (m, 6H), 4.60-4.51 (m, 6H), 4.42-4.08 (m, 11H), 4.08-3.06 (m, 2962H), 3.03-2.65 (m, 14H), 2.65-1.87 (m, 102H), 1.83-1.17 (m, 84H), 0.92-0.79 (m, 36H)

23.13 Preparation of **84-100**

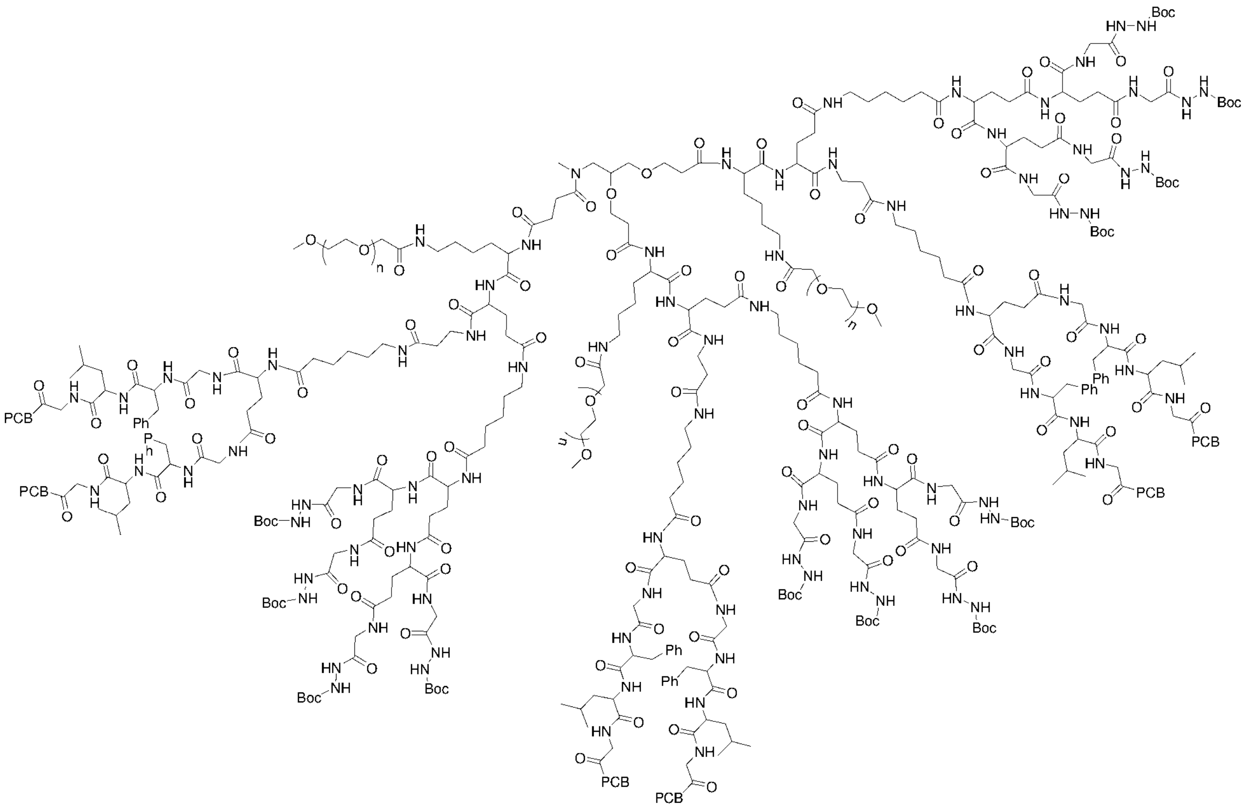

84-100

**84-99** (1.15 g, 0.0299 mmol), **84-98** (0.165 g, 0.1348 mmol), HBTU (0.05 g, 0.1168 mmol), and HOBT (0.015 g, 0.1168 mmol) were placed in a 100 mL round-bottom flask, then DMF (10 mL) was added for dissolution with the assistance of ultrasonication, and the mixture was stirred at 0°C for 2 minutes. DIEA (0.1 mL, 0.3595 mmol) was then added dropwise, and the mixture was brought to room temperature and reacted with stirring. After the reaction was completed, n-hexane (25 mL) and methyl tert-butyl ether (200 mL) were added for precipitation. The supernatant was discarded, and then n-hexane (20 mL) and methyl tert-butyl ether (100 mL) were added again for precipitation. This process was repeated three times. The mixture was subjected to suction filtration, and the filter cake was dissolved in dichloromethane (50 mL), then 100-200 mesh silica gel powder (15 g) was added, and the mixture was evaporated to dryness. The resulting product was dry-loaded onto the column and subjected to column chromatography using a 1% ammonia/5-6% methanol/dichloromethane solution as the eluent. The desired product was collected, concentrated, and dried to provide 1.06 g of the product. $^1$H-NMR (600 MHz, DMSO-$d_6$) δ 10.42-9.87 (m, 6H), 9.07-8.78 (m, 18H), 8.60-7.99 (m, 67H), 7.99-7.46 (m, 26H), 7.31-7.07 (m, 30H), 5.89-5.76 (m, 6H), 4.60-4.51 (m, 6H), 4.42-4.08 (m, 20H), 4.08-3.06 (m, 3034H), 3.03-2.65 (m, 14H), 2.65-1.87 (m, 126H), 1.83-1.10 (m, 210H), 0.92-0.79 (m, 36H)

23.14 Preparation of **84-104**

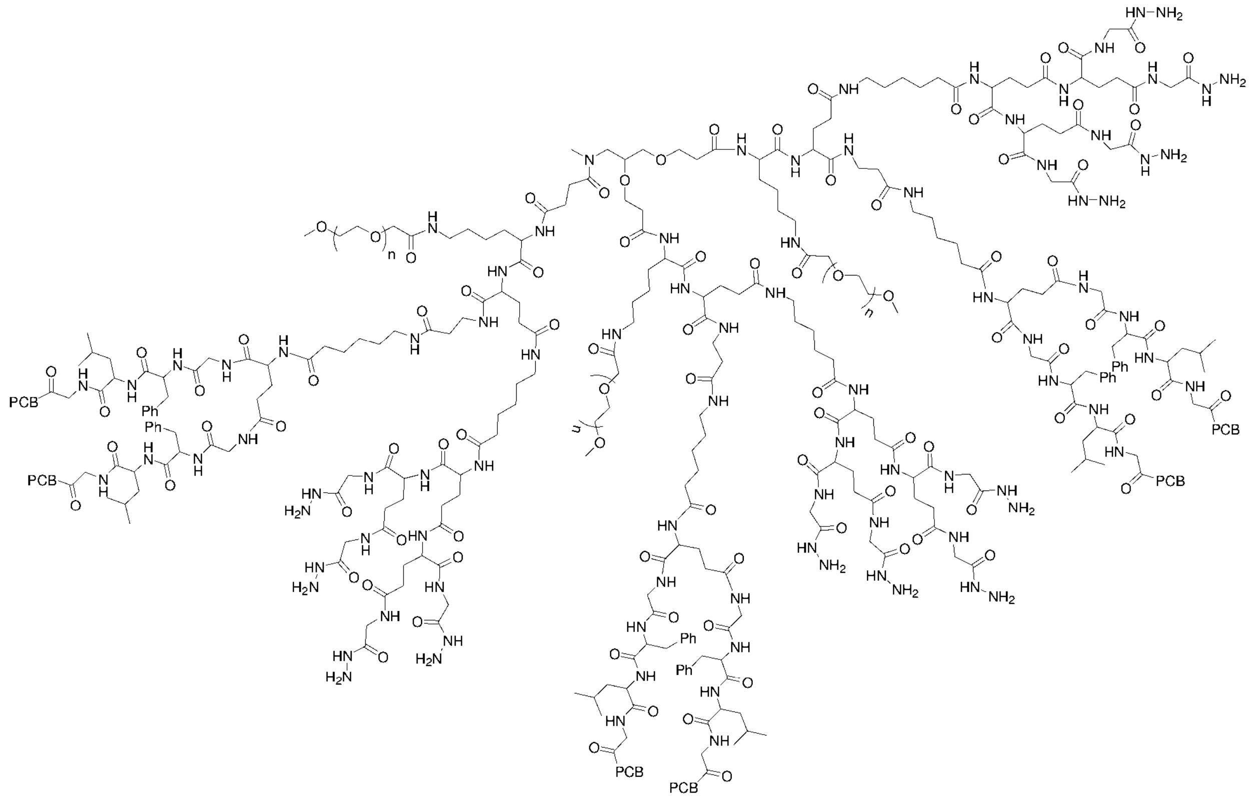

**84-100** (5 g, 0.12 mmol) was placed in a 50 mL round-bottom flask, then trifluoroacetic acid (10 mL) was added, and the mixture was reacted with stirring at room temperature. After the reaction was completed, dichloromethane (30 mL) was added, and the dichloromethane and most of the trifluoroacetic acid were removed by rotary evaporation under reduced pressure. Methyl tert-butyl ether (300 mL) was then added for precipitation. A solid precipitated and was filtered. The obtained solid was dissolved in 20% methanol/dichloromethane (15 mL), then methyl tert-butyl ether (300 mL) was added for precipitation. A solid precipitated and was filtered. This process was repeated three times. The filter cake was collected and dried to provide 0.9 g of the product. $^1$H-NMR (600 MHz, DMSO-$d_6$) δ 10.42-9.88 (m, 6H), 9.07-8.78 (m, 18H), 8.60-7.99 (m, 67H), 7.99-7.46 (m, 26H), 7.31-7.06 (m, 18H), 5.89-5.75 (m, 6H), 4.60-4.51 (m, 6H), 4.42-4.08 (m, 20H), 4.08-3.06 (m, 3034H), 3.03-1.87 (m, 164H), 1.83-1.10 (m, 102H), 0.92-0.79 (m, 36H)

23.15 Preparation of **84-106**

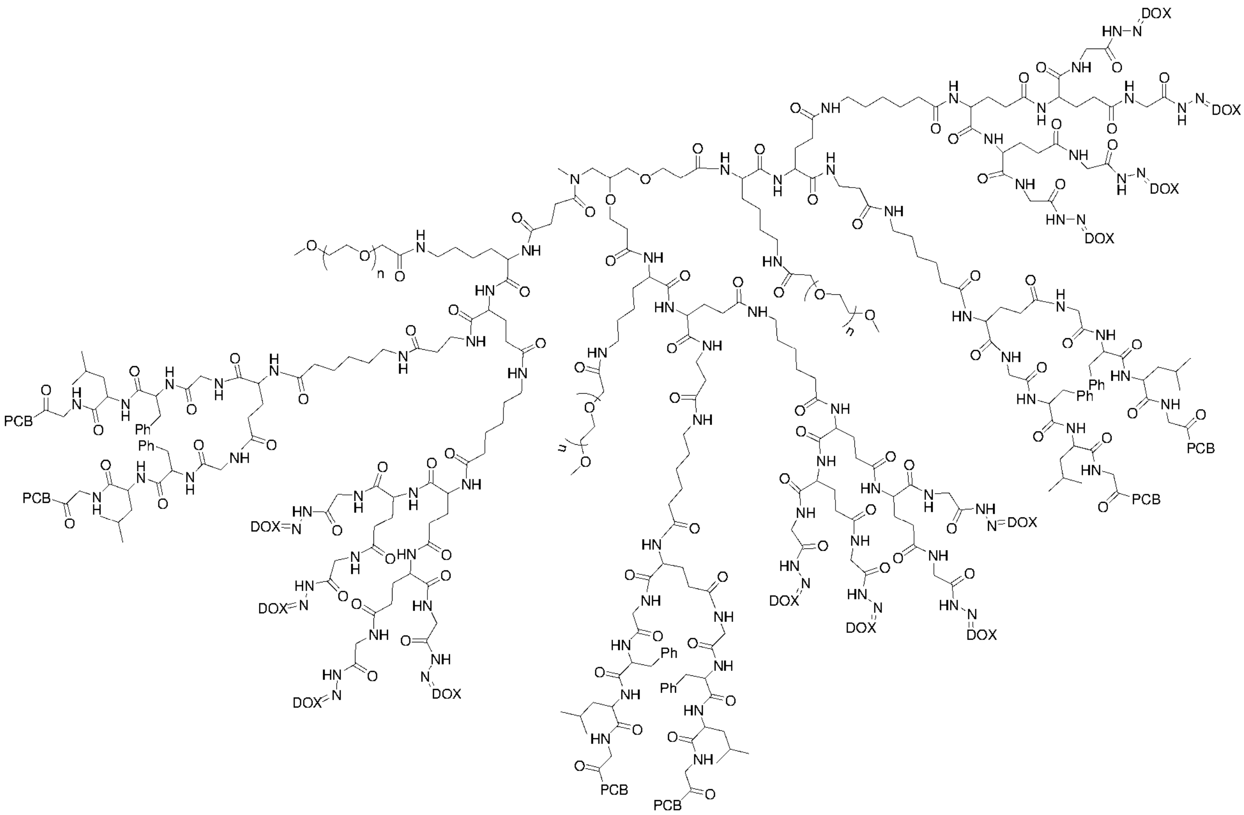

**84-104** (0.9 g, 0.0223 mmol) and doxorubicin (0.23 g, 0.4025 mmol) were added to a 50 mL round-bottom flask, then methanol (10 mL) was added for dissolution with the assistance of ultrasonication. Then trifluoroacetic acid (0.3 mL) was added, and the mixture was reacted with stirring at room temperature. After the reaction was completed, methyl tert-butyl ether (100 mL) was added. A solid precipitated and was filtered. The filter cake was redissolved in DMF (10 mL), and methyl tert-butyl ether (100 mL) was added. A solid precipitated and was filtered. This process was repeated three times. The filter cake was dried to provide 0.93 g of the product. $^1$H-NMR (600 MHz, DMSO-$d_6$) δ 14.16-13.88 (m, 12H), 13.38-13.05 (m, 12H), 10.42-9.88 (m, 6H), 9.07-8.78 (m, 18H), 8.60-7.99 (m, 103H), 7.99-7.46 (m, 62H), 7.31-7.06 (m, 18H), 5.89-5.75 (m, 6H), 5.58-5.20 (m, 36H), 5.00-4.84 (m, 24H), 4.60-4.51 (m, 30H), 4.42-4.08 (m, 32H), 4.08-3.06 (m, 3058H), 3.03-1.87 (m, 224H), 1.83-1.19 (m, 150H), 0.92-0.79 (m, 36H)

Example 24: Synthesis of Compound **76-207**

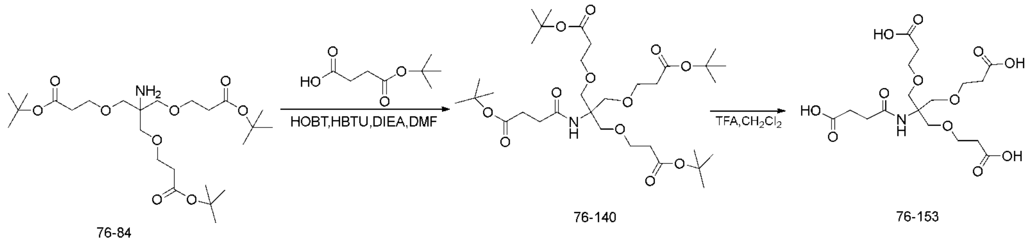


76-84 76-140 76-153

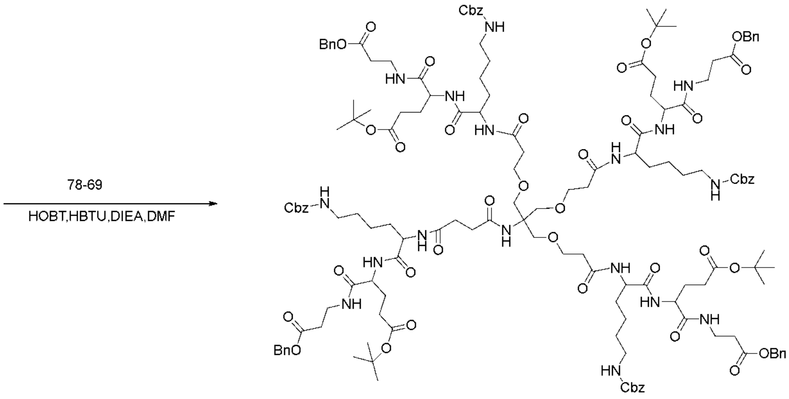


76-160

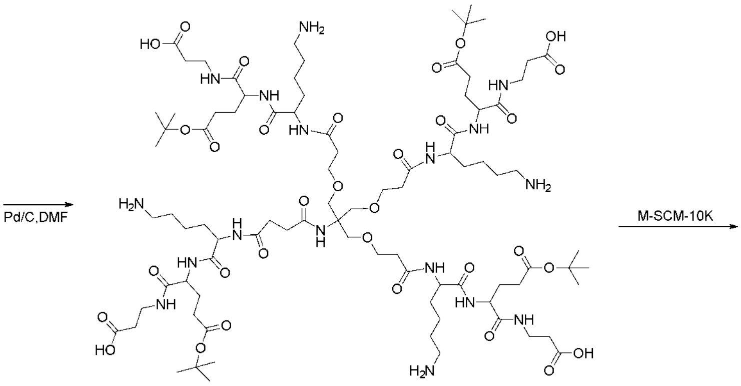


76-162

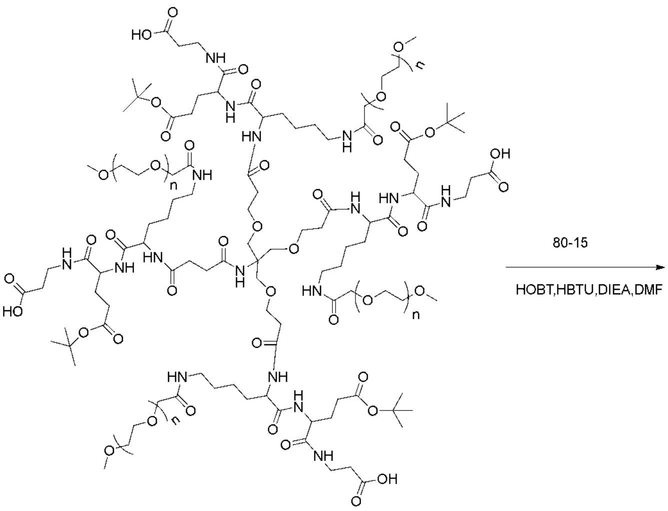


76-173

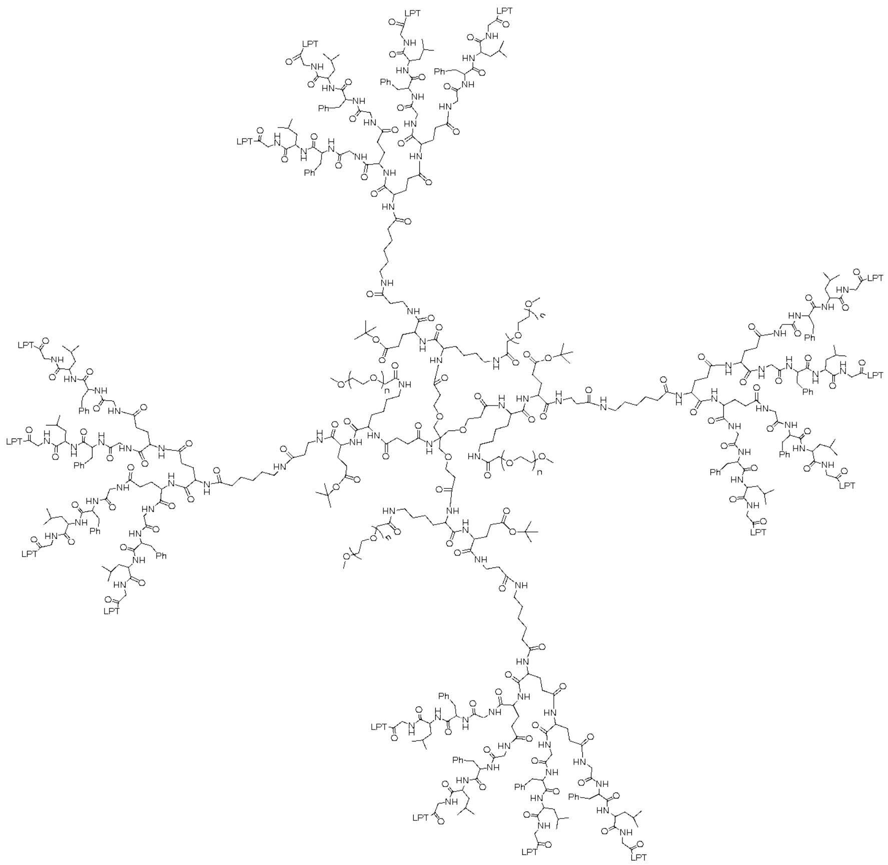

76-181

TFA $CH_2Cl_2$

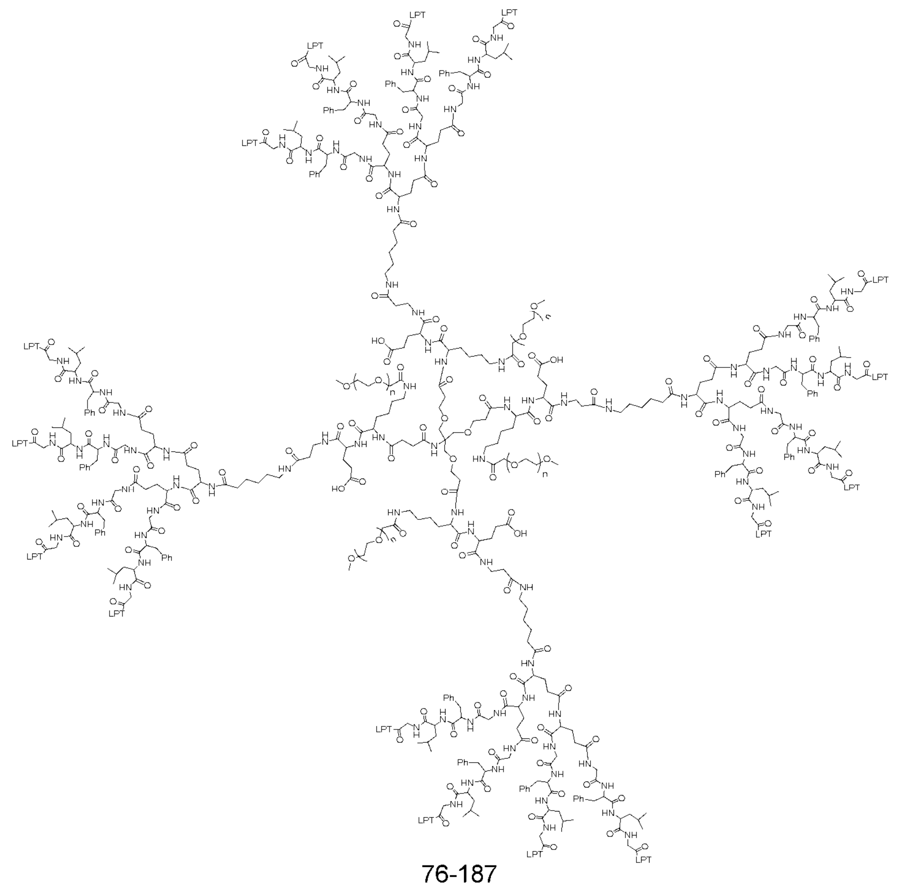

76-187

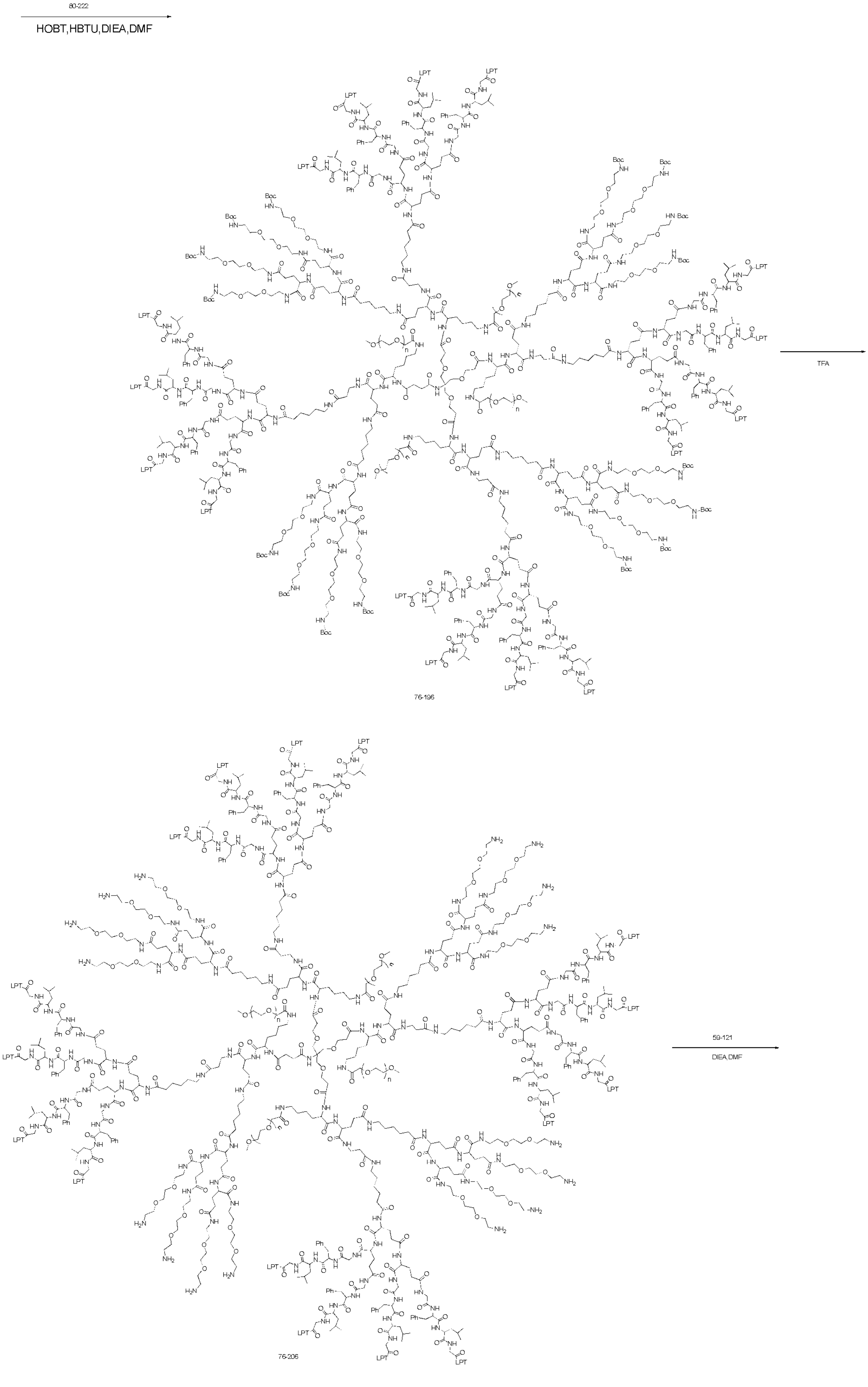


76-196

76-206

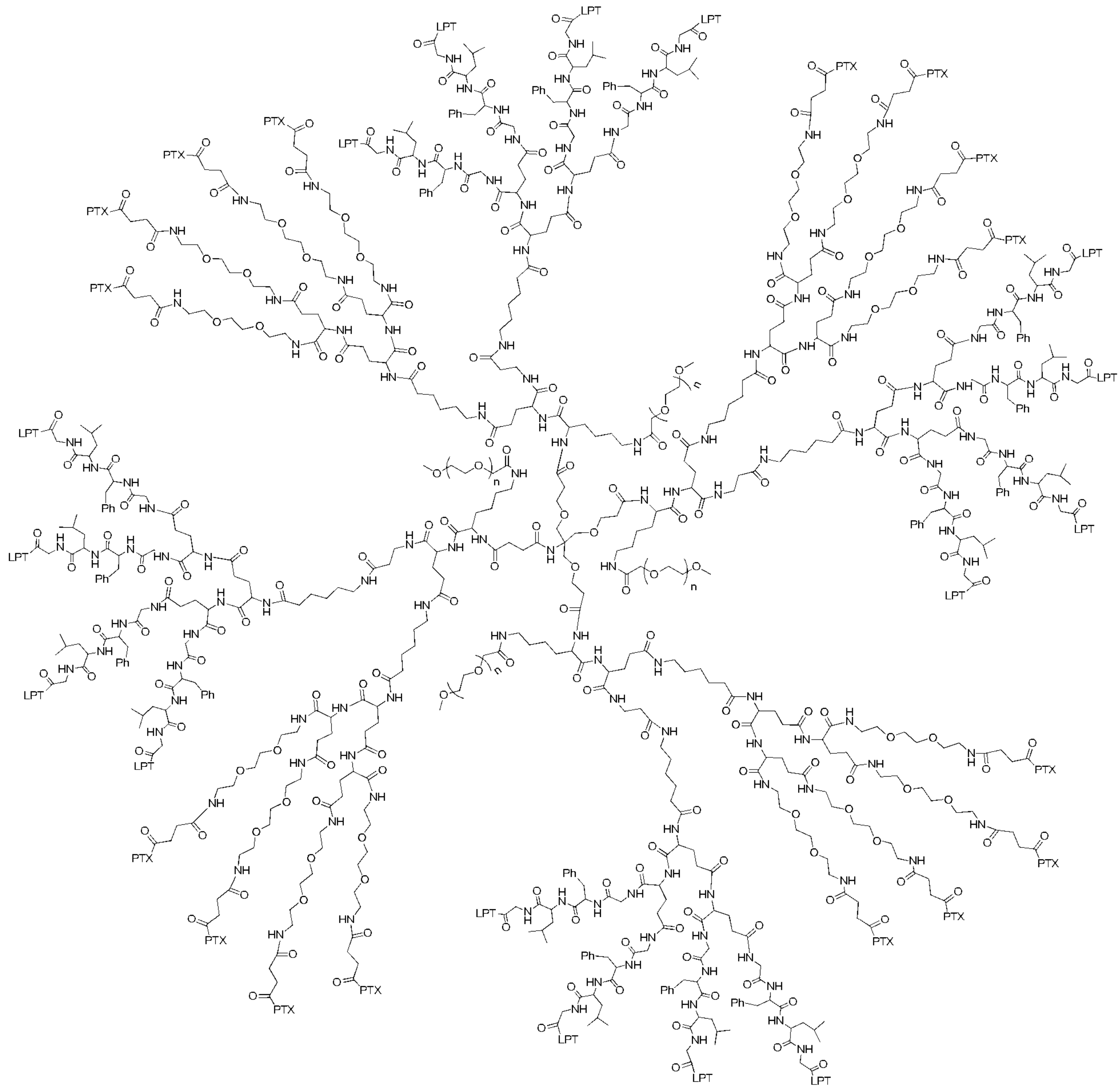

76-207

24.1 Preparation of **76-140**

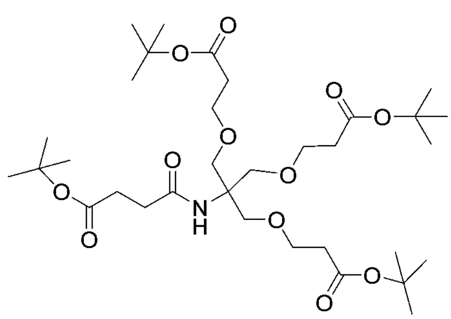

To a flask containing **76-84** (0.33 g, 0.65 mmol, synthesized according to PCT/EP2012/062505), mono-tert-butyl succinate (0.13, 0.72 mmol), HBTU (0.30 g, 0.78 mmol), and HOBT (0.11 g, 0.78 mmol) were added, and then DMF (20 mL)

was added to dissolve the mixture. DIEA (0.39 mL, 2.345 mmol) was slowly added dropwise at -5°C. After the dropwise addition was completed, the reaction was carried out for half an hour, the flask was then taken out, and the reaction was carried out with stirring at room temperature overnight. After the reaction was completed, the mixture was poured into a separatory funnel containing saturated brine (200 mL) and ethyl acetate (200 mL) for extraction. After standing for phase separation, the organic phase was collected, and the aqueous phase was further extracted with ethyl acetate (200 mL × 3). The organic phases were combined, evaporated to dryness to provide a solid, which was then dissolved in a methanol/dichloromethane (1/4) mixture, then silica gel powder (100 mL) was added, and the mixture was evaporated to dryness to provide a powdery solid. The powdery solid was dry-loaded onto the column and subjected to column chromatography using a 20% ethyl acetate/petroleum ether mixture as the eluent. The desired product was collected, concentrated, and dried in a vacuum oven to provide 0.38 g of the product. $^1$H-NMR (600 MHz, DMSO-$d_6$) δ 7.04 (s, 1H), 3.56-3.52 (m, 12H), 2.41-2.38 (m, 6H), 2.34-2.31 (m, 2H), 2.31-2.28 (m, 2H), 1.40 (s, 27H), 1.38 (s, 9H); ESI [M+H$^+$] 662.41, [M+Na$^+$] 684.39

24.2 Preparation of **76-153**

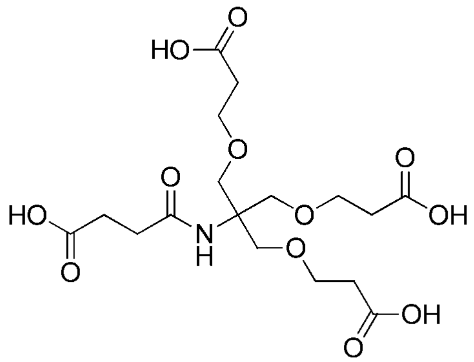

To a flask containing **76-140** (0.38 g, 0.57 mmol), dichloromethane (15 mL) was added for dissolution with the assistance of ultrasonication, then TFA (3.41 mL) was added, and the mixture was reacted with stirring at room temperature overnight. After the reaction was completed, the mixture was evaporated under reduced pressure until the product became an oil. Methyl tert-butyl ether (60 mL) was then added for precipitation. A solid precipitated and was filtered. The filter cake was washed with methyl tert-butyl ether (40 mL × 3). The filter cake was collected and dried to provide 4.34 g of the product.

24.3 Preparation of **76-160**

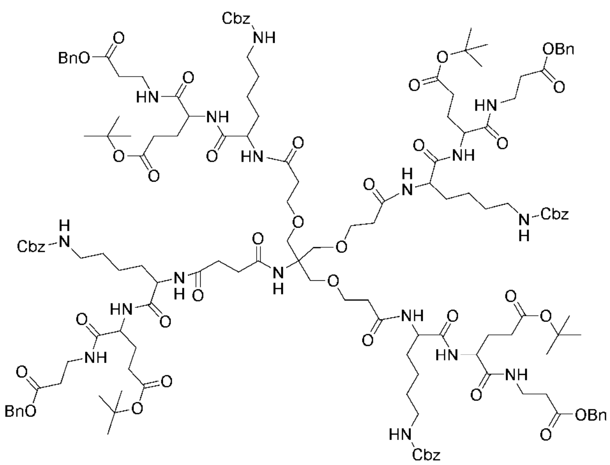

**76-153** (4.34 g, 2.18 mmol), HBTU (0.90 g, 2.37 mmol), and HOBT (0.32 g, 2.37 mmol) were weighed and added to a flask containing **78-69** (0.87 g, 1.98 mmol), then DMF (50 mL) was added to dissolve the mixture. DIEA (1.18 mL, 7.12

mmol) was slowly added dropwise at -5°C. After the dropwise addition was completed, the reaction was carried out for half an hour, the flask was then taken out, and the reaction was carried out with stirring at room temperature overnight. After the reaction was completed, the mixture was poured into a separatory funnel containing saturated brine (200 mL) and ethyl acetate (200 mL) for extraction. After standing for phase separation, the organic phase was collected, and the aqueous phase was further extracted with ethyl acetate (200 mL × 3). The organic phases were combined, and evaporated to dryness to provide a solid, which was then dissolved in a methanol/dichloromethane (1/4) mixture, then silica gel powder (100 mL) was added, and the mixture was evaporated to dryness. The resulting product was dry-loaded onto the column and subjected to column chromatography using a 20% ethyl acetate/petroleum ether mixture as the eluent. The desired product was collected, concentrated, and dried to provide 0.95 g of the product. $^1$H-NMR (600 MHz, DMSO-$d_6$) δ 8.13-8.08 (m, 1H), 8.03-7.89 (m, 11H), 7.81-7.75 (m, 1H), 7.36-7.28 (m, 40H), 7.20-7.16 (m, 4H), 5.08-5.05 (m, 8H), 5.02-4.97 (m, 8H), 4.27-4.09 (m, 9H), 3.57-3.47 (m, 15H), 3.27-3.18 (m, 8H), 2.98-2.90 (m, 9H), 2.43-2.33 (m, 10H), 2.25-2.08 (m, 10H), 1.95-1.79 (m, 5H), 1.76-1.66 (m, 5H), 1.65-1.55 (m, 5H), 1.54-1.44 (m, 6H), 1.37-1.35 (m, 36H), 1.31-1.15 (m, 12H); ESI [M+H$^+$] 2872.88, [M+Na$^+$] 2894.40

24.4 Preparation of **76-162**

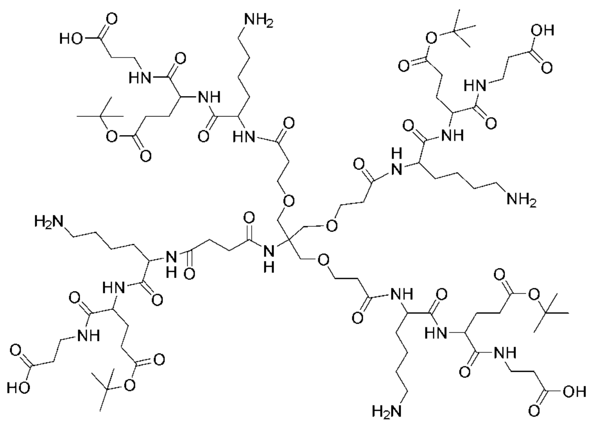

**76-160** (0.64 g, 0.22 mmol) was added to a hydrogenation reactor, and DMF (15 mL) and 10% Pd/C catalyst (60 mL) were added. The reactor was sealed, evacuated with a water pump, and then filled with hydrogen gas. This process was repeated three times. The hydrogen pressure in the reactor was adjusted to 1.6 MPa, and the reaction was carried out overnight with stirring with a magnetic stirrer. After the reaction was completed, the mixture was filtered through Celite and rinsed with DMF (10 mL × 5). The filtrate was collected for later use.

24.5 Preparation of 76-173

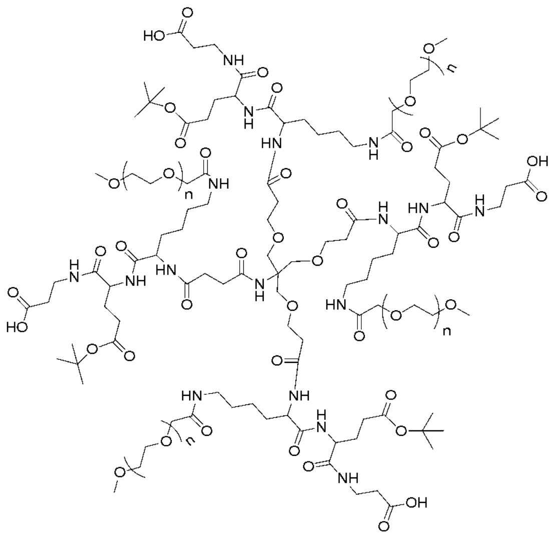

**76-162** (0.0883 g, 0.0447 mmol) was placed in a 250 mL flask, and DMF (25 mL) was added for dissolution. DIEA (0.30 mL, 1.7880 mmol) was slowly added dropwise at -5°C, followed by the addition of M-SCM-10K (2.0 g, 0.1877 mmol, purchased from Jenkem, Lot Number: A3016-N230101), and the reaction was stirred at low speed in the dark at room temperature for one week. After the reaction was completed, n-hexane (25 mL) and methyl tert-butyl ether (200 mL) were added for precipitation. The supernatant was discarded, and n-hexane (20 mL) and methyl tert-butyl ether (100 mL) were added again for precipitation. This process was repeated three times. The filter cake was filtered and dried to provide 1.97 g of the product.

24.6 Preparation of **76-181**

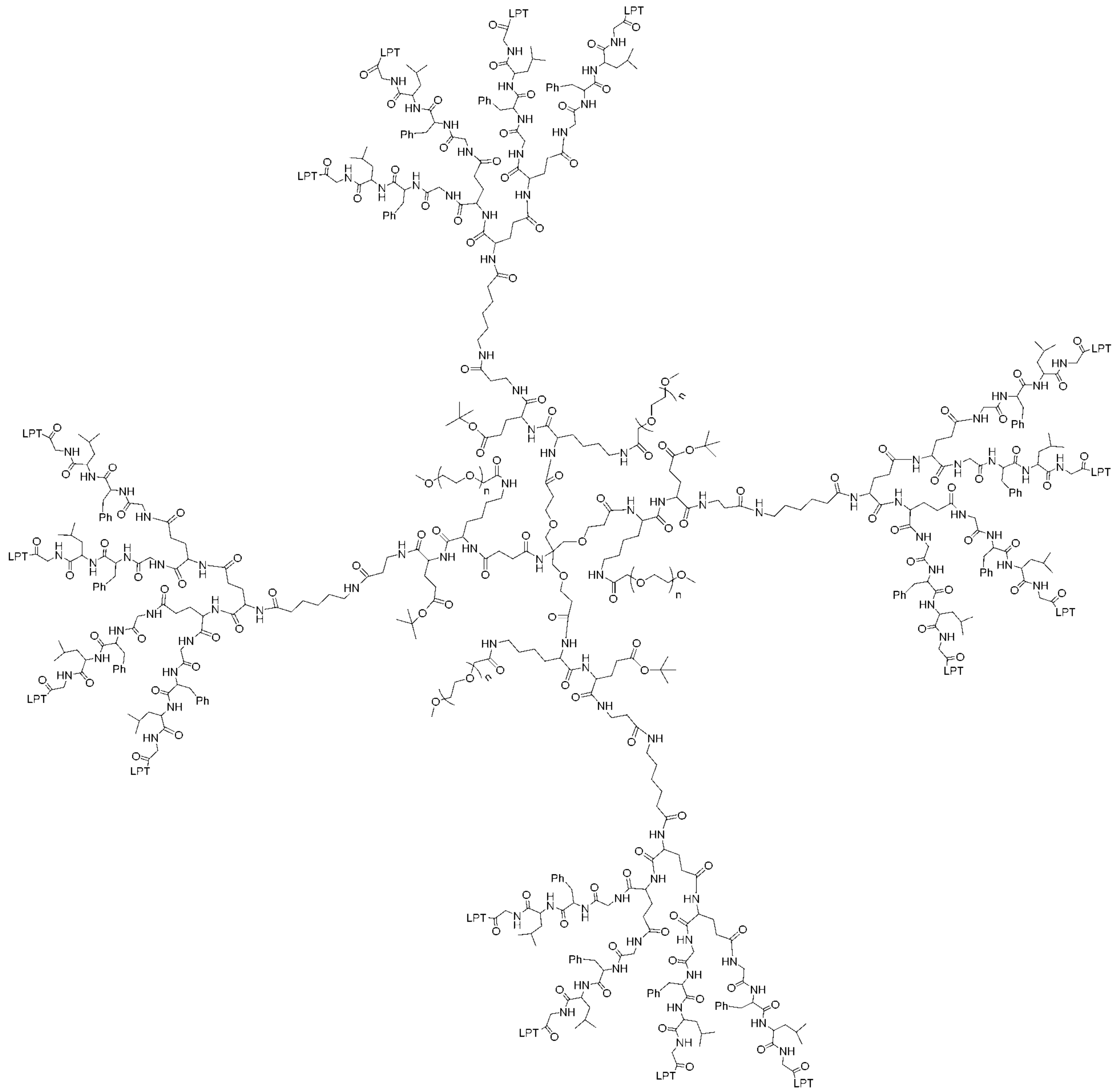

**76-173** (1.92 g, 0.0447 mmol), HBTU (0.09 g, 0.21 mmol), and HOBT (0.03 g, 0.21 mmol) were weighed and added to a flask containing **80-15** (0.84 g, 0.1967 mmol), and then DMF (20 mL) was added to dissolve the mixture. DIEA (0.11 mL, 0.64 mmol) was slowly added dropwise at -5°C. After the dropwise addition was completed, the reaction was carried out for half an hour, the flask was then taken out, and the reaction was carried out with stirring at room temperature overnight. After the reaction was completed, n-hexane (25 mL) and methyl tert-butyl ether (200 mL) were added for precipitation. The mixture was allowed to stand, and the supernatant was discarded. Then, n-hexane (25 mL) and methyl tert-butyl ether (200 mL) were added again for precipitation. This process was repeated four times. A solid precipitated and was filtered to provide a filter cake. The filter cake was dissolved in a methanol/dichloromethane (1/4) mixture, then silica gel powder (100 mL) was added, and the mixture was evaporated to dryness to provide a powdery solid. The powdery solid was dry-loaded onto the column and subjected to column chromatography using a 6-10% methanol/dichloromethane mixture as the eluent. The desired product was collected, concentrated, and dried in a vacuum oven to provide 2.16 g of the product.

24.7 Preparation of 76-187

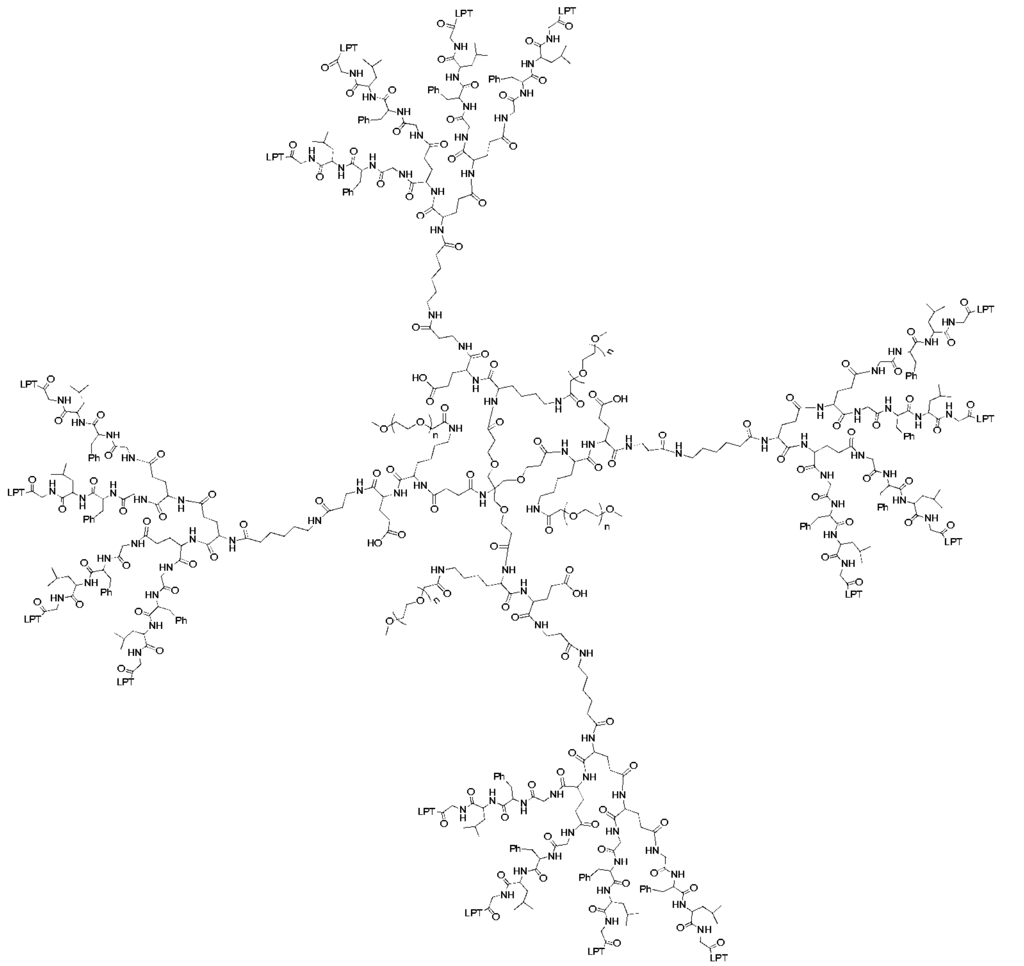

Reactant **76-181** (1.03 g, 0.0169 mmol) was placed in a reaction flask. Dichloromethane (10 mL) was added for dissolution, then TFA (20 mL) was added, and the mixture was reacted with stirring at room temperature. After the reaction was completed, the dichloromethane and most of the TFA were removed by evaporation under reduced pressure, and then methyl tert-butyl ether (200 mL) and n-hexane (50 mL) were added for precipitation. A solid precipitated and was filtered. The filter cake was collected and then dissolved in dichloromethane (10 mL), methyl tert-butyl ether (200 mL) and n-hexane (50 mL) were added for precipitation, and the mixture was filtered. This process was repeated three times. The filter cake was collected and dried to provide 1.02 g of the product.

24.8 Preparation of **76-196**

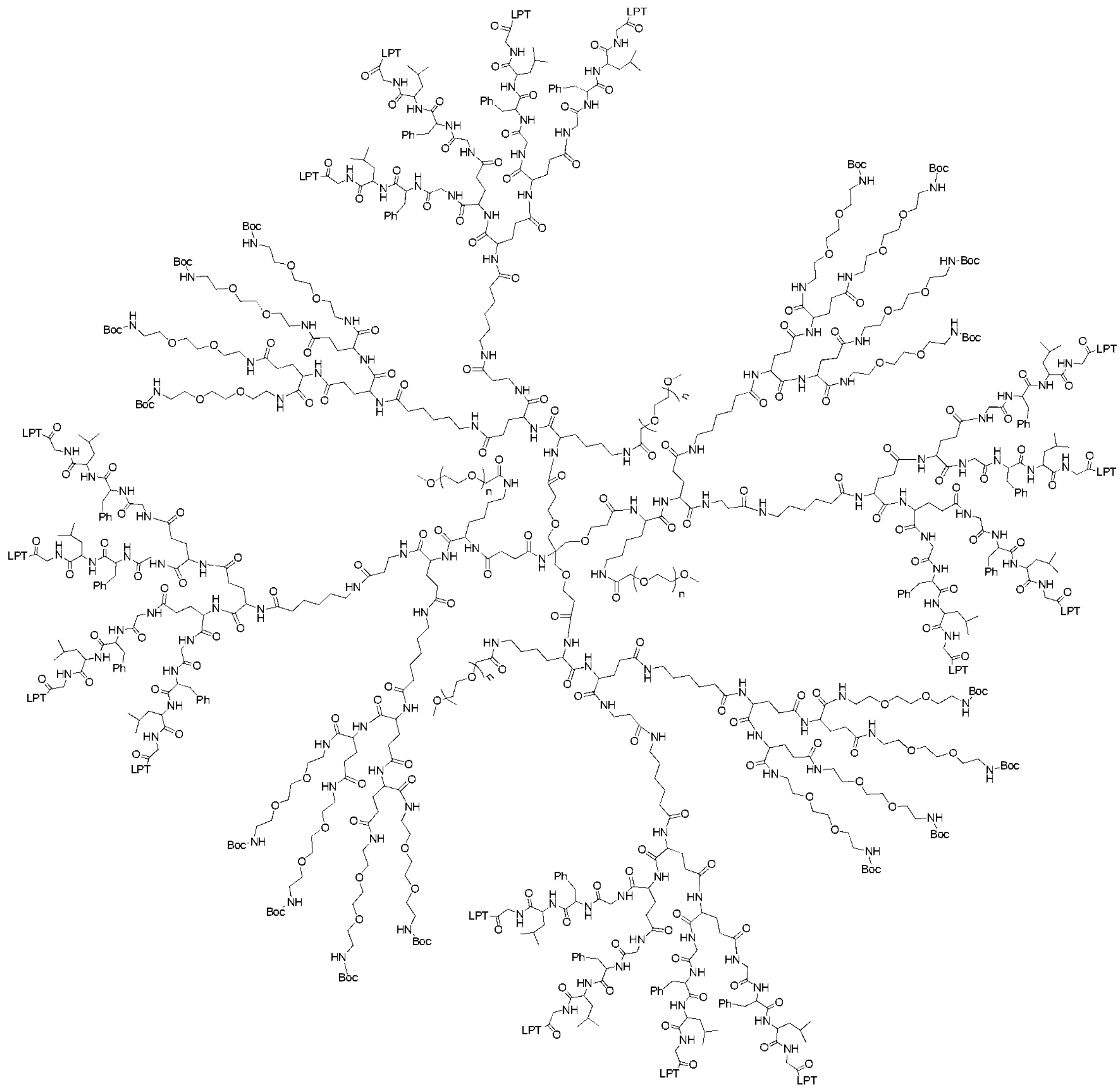

**76-187** (1.02 g, 0.0169 mmol), HBTU (0.03 g, 0.08 mmol), and HOBT (0.01 g, 0.08 mmol) were weighed and added to a flask containing **80-222** (0.12 g, 0.08 mmol), and then DMF (20 mL) was added to dissolve the mixture. DIEA (0.04 mL, 0.24 mmol) was slowly added dropwise at 0°C. After the dropwise addition was completed, the reaction was carried out for half an hour, the flask was then taken out, and the reaction was carried out with stirring at room temperature overnight. After the reaction was completed, methyl tert-butyl ether (200 mL) and n-hexane (50 mL) were added for precipitation. A solid precipitated and was filtered. The filter cake was collected and dissolved in dichloromethane (10 mL), methyl tert-butyl ether (200 mL) and n-hexane (50 mL) were added for precipitation, and a solid precipitated and was filtered. This process was repeated three times. The filter cake was collected and dried to provide 1.12 g of the product.

24.9 Preparation of **76-206**

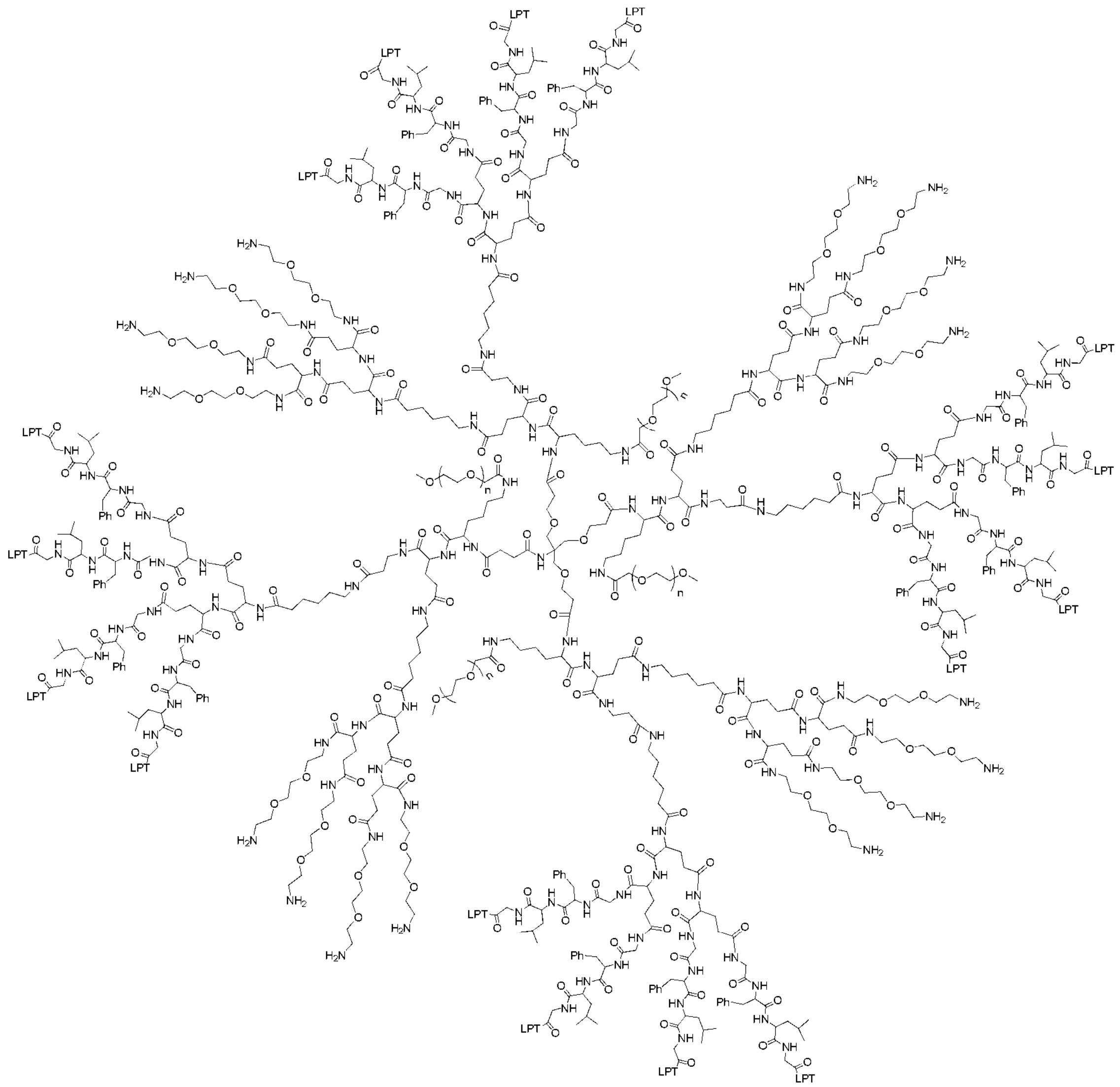

Reactant **76-196** (1.12 g, 0.0169 mmol) was dissolved in dichloromethane (10 mL), then TFA (20 mL) was added, and the mixture was reacted with stirring at room temperature. After the reaction was completed, the dichloromethane and most of the TFA were removed by evaporation using a rotary evaporator. Methyl tert-butyl ether (200 mL) and n-hexane (50 mL) were added for precipitation. A solid precipitated and was filtered. The filter cake was collected and dissolved in dichloromethane (10 mL), methyl tert-butyl ether (200 mL) and n-hexane (50 mL) were added for precipitation, and a solid precipitated and was filtered. This process was repeated three times. The filter cake was collected and dried to provide 1.09 g of the product.

24.10 Preparation of **76-207**

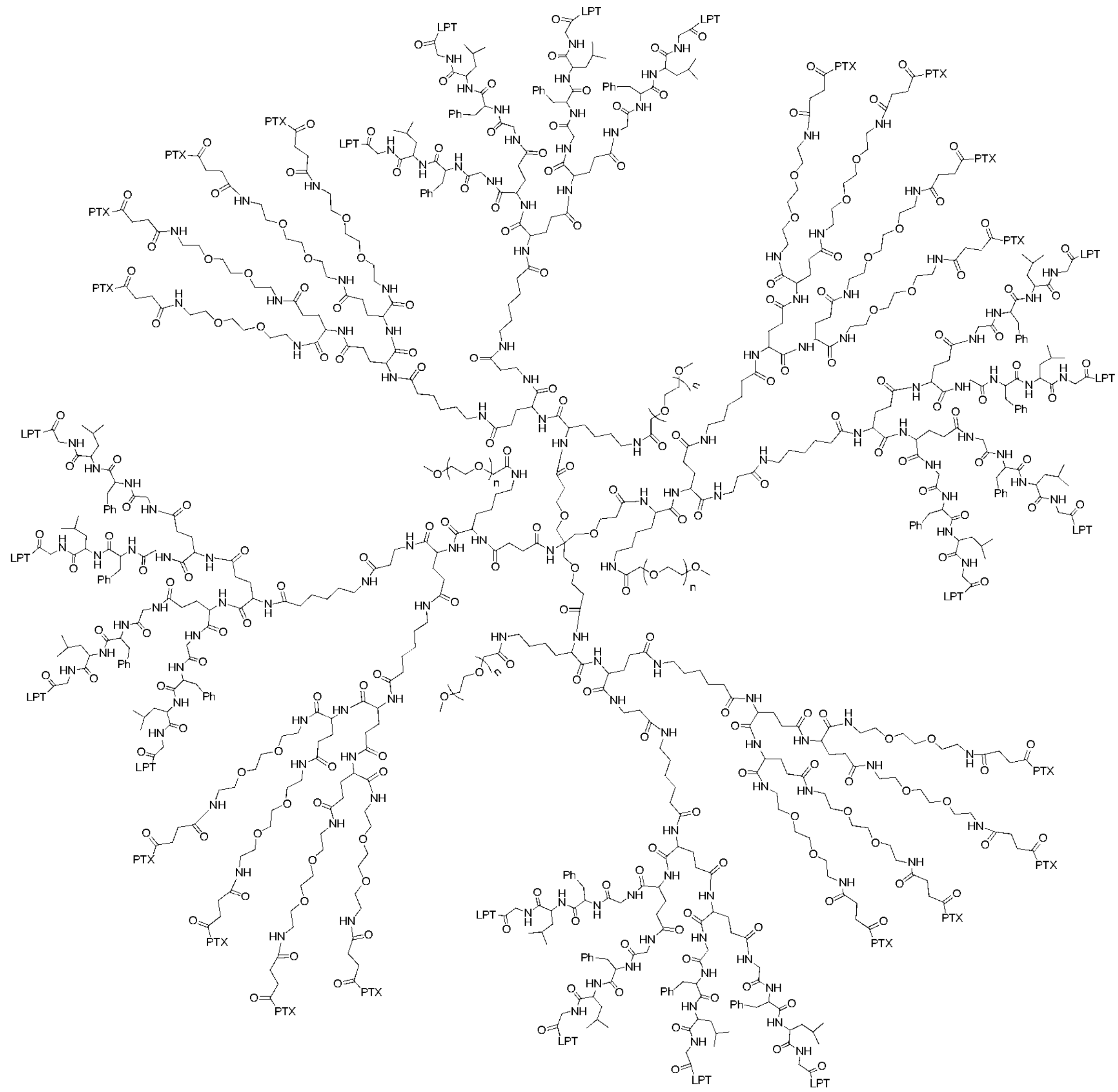

**59-121** (0.43 g, 0.41 mmol) was weighed and placed in a 250 mL flask, then DMF (20 mL) was added for dissolution, and then DIEA (0.2 mL, 1.22 mmol) was added. **76-206** (1.09 g, 0.0169 mmol) was dissolved in DMF (5 mL) and added dropwise to the flask. The reaction was carried out with stirring at room temperature overnight. After the reaction was completed, n-hexane (50 mL) and methyl tert-butyl ether (200 mL) were added for precipitation. A solid precipitated and was filtered. The filter cake was collected and then dissolved in dichloromethane (10 mL), methyl tert-butyl ether (200 mL) and n-hexane (50 mL) were added for precipitation, the mixture was filtered, and the filter cake was collected. This process was repeated three times. The filter cake was dissolved in a methanol/dichloromethane (1/4) mixture, then silica gel powder (100 mL) was added, and the mixture was evaporated to dryness. The resulting product was dry-loaded onto the column and subjected to column chromatography using a 1% triethylamine/7% methanol/dichloromethane mixture as the eluent. The desired product was collected, concentrated, and dried to provide 0.6 g of the product. $^1$H-NMR (600 MHz, DMSO-$d_6$) δ 10.28-9.79 (m, 48H), 8.80-8.49 (m, 8H), 8.12-7.92 (m, 73H), 7.92-7.78 (m, 40H), 7.56-7.40 (m, 22H), 7.35-7.02 (m, 102H), 6.90-6.59 (m, 8H), 5.64-5.49 (m, 18H), 5.48-5.28 (m, 96H), 5.28-5.15 (m, 20H), 5.10-4.84 (m, 15H), 4.81-4.46 (m, 31H), 4.29-4.04 (m, 58H), 3.59-3.41 (m, 3750H), 3.12-2.99 (m, 287H), 2.93-2.83 (m, 53H), 2.82-2.68 (m, 49H), 2.68-2.58 (m, 54H), 2.17-2.06 (m, 89H), 1.92-1.77 (m, 50H), 1.65-1.53 (m, 62H), 1.52-1.41 (m, 68H), 1.26-1.21 (m, 208H), 1.21-1.13 (m, 451H), 1.05-0.92 (m, 35H), 0.91-0.72 (m, 82H)

Example 25: Synthesis of Compound **82-152**

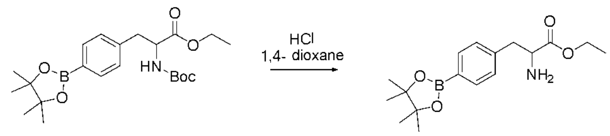


82-98

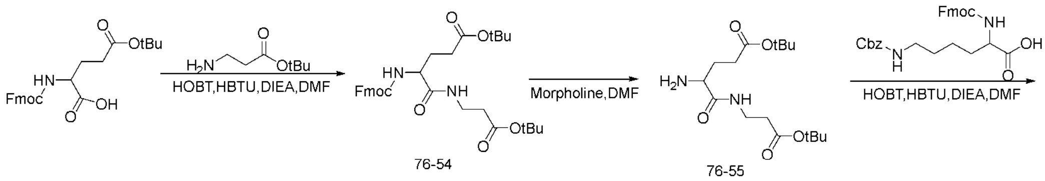


76-54

76-55

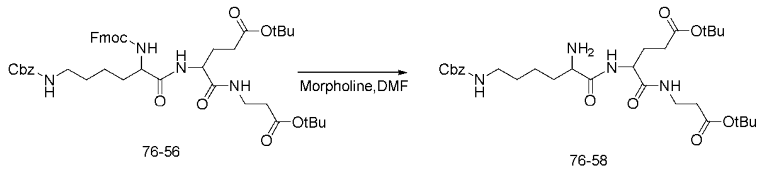


76-56

76-58

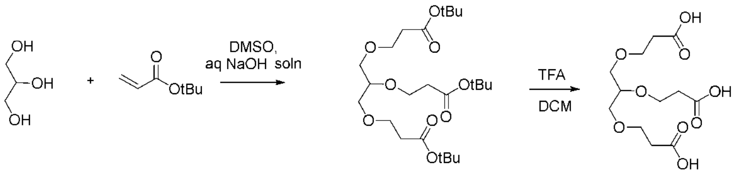


82-58

82-73

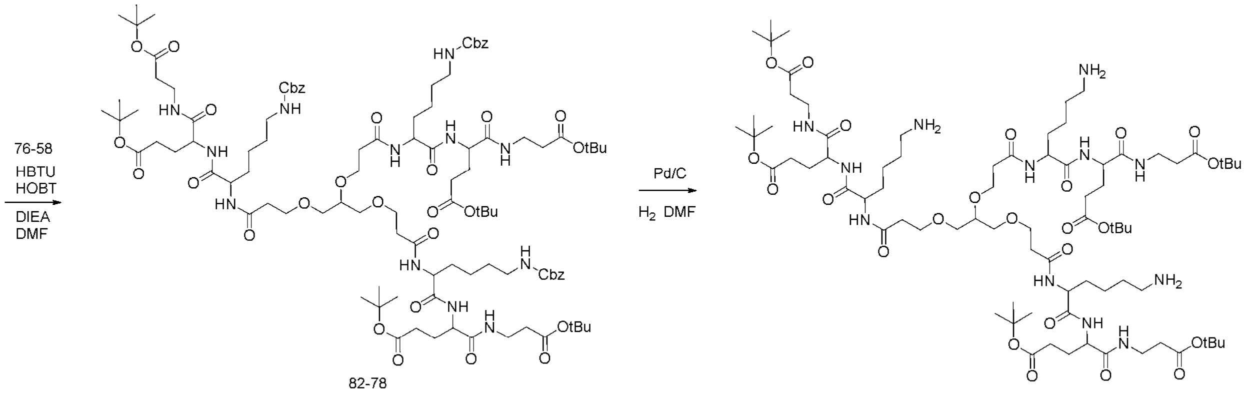


82-78

82-93

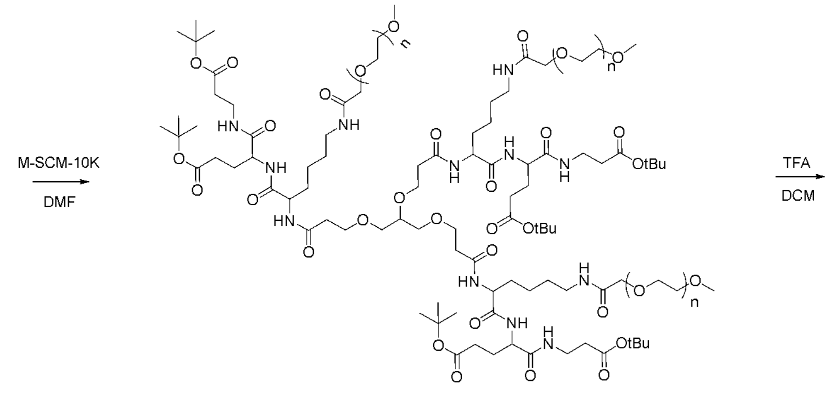


82-95

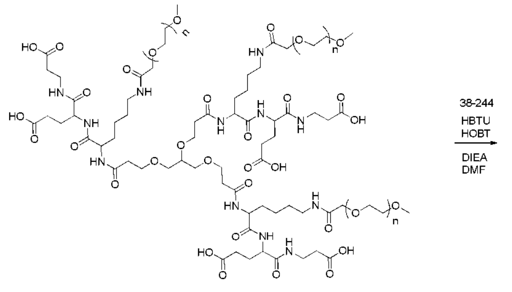


82-119

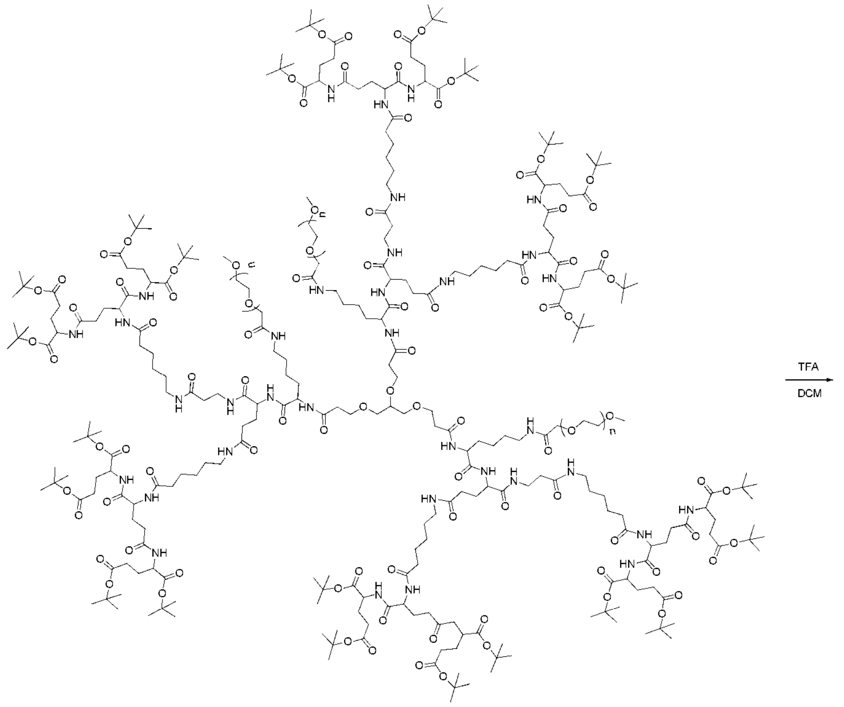


82-122

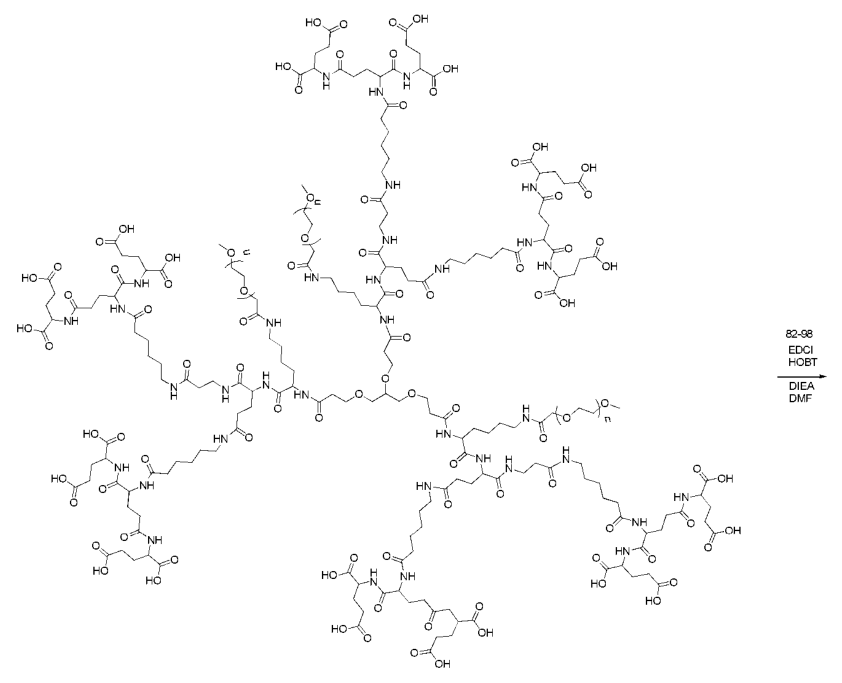


82-130

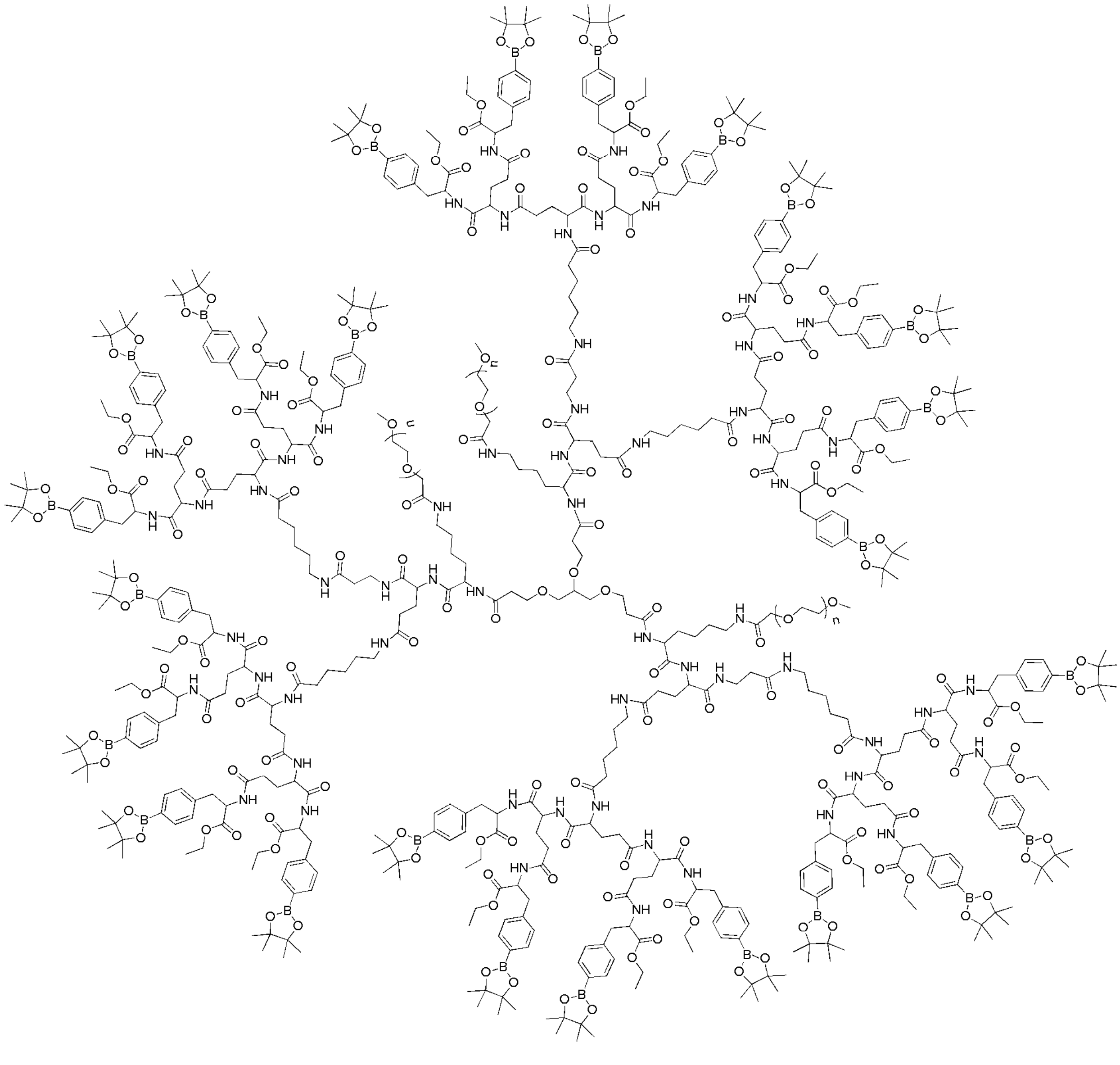

82-152

25.1 Preparation of **82-98**

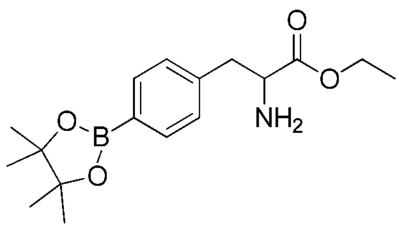

To a flask containing ethyl 2-((tert-butoxycarbonyl)amino)-3-(4-(4,4,5,5-tetramethyl-1,3,2-dioxaborolan-2-yl) phenyl)propanoate (1 g, 2.3848 mmol, purchased from Lakestar PharmaTech), dichloromethane (10 mL) was added for dissolution with the assistance of ultrasonication, then TFA (5 mL) was added, and the mixture was reacted with stirring at room temperature overnight. After the reaction was completed, the reaction mixture was evaporated to an oily state using a rotary evaporator. Methyl tert-butyl ether (60 mL) was then added. A powdery solid precipitated from the reaction

mixture and was filtered. The filter cake was washed with methyl tert-butyl ether (40 mL × 3). The filter cake was collected and dried in a vacuum oven to provide 0.95 g of the product.

25.2 Preparation of **76-54**

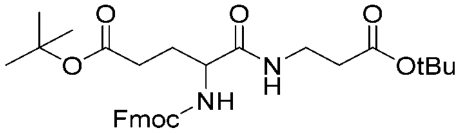

Fmoc-Glu(OtBu)-OH (10.00 g, 23.50 mmol), HBTU (10.69 g, 28.20 mmol), and HOBT (3.81 g, 28.20 mmol) were weighed and added to a flask containing β-alanine tert-butyl ester hydrochloride (4.48 g, 24.68 mmol), then DMF (200 mL) was added to dissolve the mixture, and the flask was allowed to stand at -5°C. DIEA (13.98 mL, 84.60 mmol) was slowly added dropwise. After the dropwise addition was completed, the reaction was carried out for half an hour, the flask was then taken out, and the reaction was carried out with stirring at room temperature overnight. After the reaction was completed, the mixture was poured into a separatory funnel containing saturated brine (200 mL) and ethyl acetate (200 mL) for extraction. After standing for phase separation, the organic phase was collected, and the aqueous phase was further extracted with ethyl acetate (200 mL × 3). The organic phases were combined and evaporated to dryness to provide a solid as the product.

25.3 Preparation of **76-55**

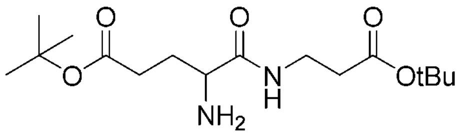

To a flask containing **76-54** (23.50 mmol), DMF (200 mL) was added for dissolution with the assistance of ultrasonication, then morpholine (40 mL, 470 mmol) was added, and the mixture was stirred at room temperature for 2 hours. After the reaction was completed, the mixture was poured into a separatory funnel containing saturated brine (200 mL) and ethyl acetate (200 mL) for extraction. After standing for phase separation, the organic phase was collected, and the aqueous phase was further extracted with ethyl acetate (200 mL × 3). The organic phases were combined, evaporated to dryness to provide a solid, which was then dissolved in a methanol/dichloromethane (1/4) mixture, then silica gel powder (100 mL) was added, and the mixture was evaporated to dryness to provide a powdery solid. The powdery solid was dry-loaded onto the column and subjected to column chromatography using a 20% ethyl acetate/petroleum ether mixture as the eluent. The desired product was collected, concentrated, and dried in a vacuum oven to provide 6.89 g of the product with a yield of 88.7%.

25.4 Preparation of **76-56**

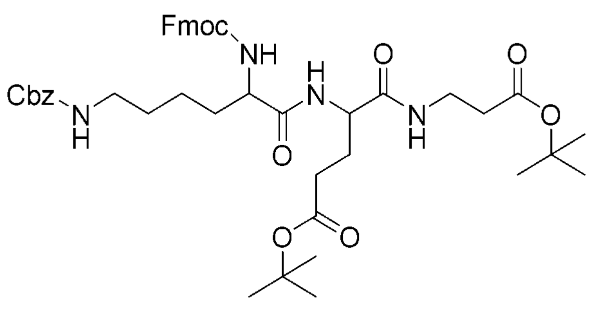

N'-Fmoc-N-benzyloxycarbonyl-L-lysine (2.90 g, 5.76 mmol), HBTU (2.62 g, 6.91 mmol), and HOBT (0.93 g, 6.91

mmol) were weighed and added to a flask containing **76-55** (2.0 g, 6.05 mmol), and then DMF (50 mL) was added to dissolve the mixture. DIEA (3.43 mL, 20.74 mmol) was slowly added dropwise at -5°C. After the dropwise addition was completed, the reaction was carried out for half an hour, the flask was then taken out, and the reaction was carried out with stirring at room temperature overnight. After the reaction was completed, the mixture was poured into a separatory funnel containing saturated brine (200 mL) and ethyl acetate (200 mL) for extraction. After standing for phase separation, the organic phase was collected, and the aqueous phase was further extracted with ethyl acetate (200 mL × 3). The organic phases were combined, evaporated to dryness to provide a solid as the product.

25.5 Preparation of **76-58**

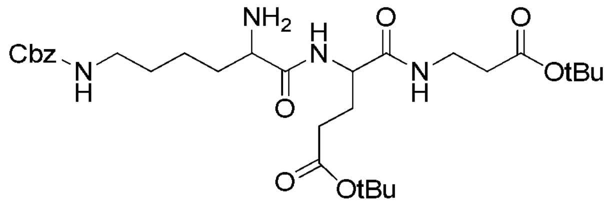

To a flask containing **76-56** (5.76 mmol), DMF (50 mL) was added for dissolution with the assistance of ultrasonication, then morpholine (10 mL, 115.21 mmol) was added, and the mixture was reacted with stirring at room temperature for 2 hours. After the reaction was completed, the mixture was poured into a separatory funnel containing saturated brine (200 mL) and ethyl acetate (200 mL) for extraction. After standing for phase separation, the organic phase was collected, and the aqueous phase was further extracted with ethyl acetate (200 mL × 3). The organic phases were combined, evaporated to dryness, and the residue was then dissolved in a methanol/dichloromethane (1/4) mixture, then silica gel powder (100 mL) was added, and the mixture was evaporated to dryness to provide a powdery solid. The powdery solid was dry-loaded onto the column and subjected to column chromatography using a 20% ethyl acetate/petroleum ether mixture as the eluent. The desired product was collected, concentrated, and dried in a vacuum oven to provide 2.26 g of the product with a yield of 66.2%. $^1$H-NMR (600 MHz, DMSO-*d*$_6$) δ 8.14-7.97 (m, 2H), 7.38-7.29 (m, 5H), 7.26-7.21 (m, 1H), 5.04-4.98(m, 2H), 4.26-4.18 (m, 1H), 3.33-3.25(m, 2H), 3.20-3.10(m, 2H),2.98-2.94 (m, 2H), 2.38-2.31 (m, 2H), 2.19-2.11 (m, 2H), 1.91-1.79 (m, 2H), 1.72-1.66 (m, 1H), 1.59-1.47 (m, 2H), 1.40-1.38 (m, 9H), 1.38-1.37 (m, 9H), 1.35-1.22 (m, 4H); ESI [M+H$^+$] 593.35, [M+Na$^+$] 615.33, [M+K$^+$] 631.30

25.6 Preparation of 82-58

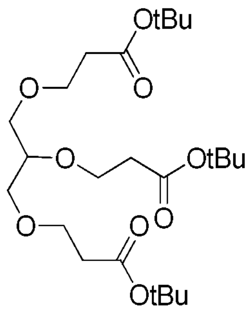

Glycerol (1 g, 10.8589 mmol) was weighed and placed in a 500 mL flask, and DMSO (10 mL) was added. Nitrogen gas was introduced at 15°C, and 5N aqueous NaOH (1 mL) was then added and dissolved with the assistance of ultrasonication. Then, tert-butyl acrylate (10.44 g, 81.4421 mmol) was slowly added dropwise, and reacted with stirring at room temperature overnight. After the reaction was completed, the mixture was poured into a separatory funnel containing saturated brine (200 mL) and ethyl acetate (200 mL) for extraction. After standing for phase separation, the organic phase was collected, and the aqueous phase was further extracted with ethyl acetate (200 mL × 3). The organic phases were combined, evaporated to dryness to provide a solid, which was then dissolved in a methanol/dichloromethane (1/4) mixture, then silica gel powder (100 mL) was added, and the mixture was evaporated to dryness to provide a powdery solid. The powdery solid was dry-loaded onto the column and subjected to column chromatography using a 5-60% ethyl acetate/petroleum ether mixture as the eluent. The desired product was collected, concentrated, and dried in a vacuum

oven to provide 2.88 g of the product with a yield of 55.70%. $^1$H-NMR (600 MHz, DMSO-$d_6$) δ 3.70-3.65 (m, 2H), 3.60-3.55 (m, 4H), 3.51-3.45 (m, 1H), 3.42-3.34 (m, 4H), 2.43-2.34 (m, 6H), 1.41 (s, 27H); ESI [M+H$^+$] 477.30

25.7 Preparation of **82-73**

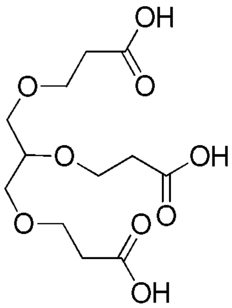

To a flask containing **82-58** (1.03 g, 2.1611 mmol), dichloromethane (10 mL) was added for dissolution with the assistance of ultrasonication, then TFA (4.67 mL, 62.946 mmol) was added, and the mixture was reacted with stirring at room temperature overnight. After the reaction was completed, the reaction mixture was evaporated to an oily state using a rotary evaporator. Methyl tert-butyl ether (60 mL) was then added. A powdery solid precipitated from the reaction mixture and was filtered. The filter cake was washed with methyl tert-butyl ether (40 mL × 3). The resulting product had poor solubility. The filter cake was collected and dried in a vacuum oven to provide the product.

25.8 Preparation of **82-78**

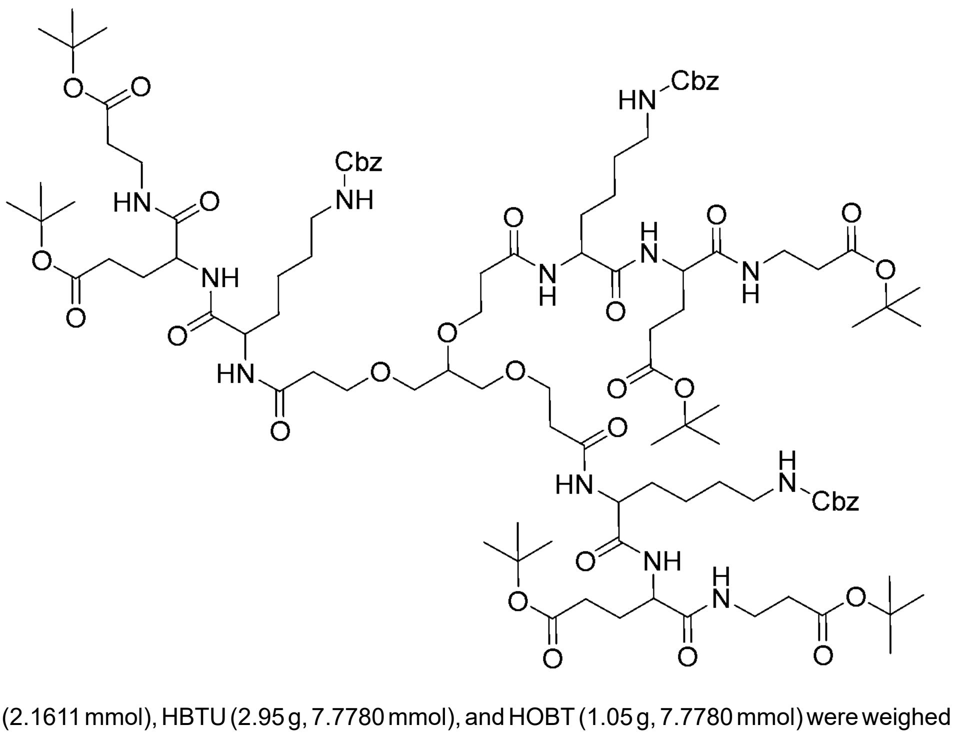

**82-73** (2.1611 mmol), HBTU (2.95 g, 7.7780 mmol), and HOBT (1.05 g, 7.7780 mmol) were weighed and added to a flask containing **76-58** (4.22 g, 7.1316 mmol), and then DMF (50 mL) was added to dissolve the mixture. DIEA (2.57 mL, 15.5600 mmol) was slowly added dropwise at -5°C. After the dropwise addition was completed, the reaction was carried out for half an hour, the flask was then taken out, and the reaction was carried out with stirring at room temperature overnight. After the reaction was completed, n-hexane (25 mL) and methyl tert-butyl ether (200 mL) were added for precipitation. The supernatant was discarded, and n-hexane and methyl tert-butyl ether were added for precipitation. This process was repeated three times. The mixture was filtered to provide a solid product, which was collected, and dried to

provide 2.1 g of the product. $^1$H-NMR (600 MHz, DMSO-$d_6$) δ 8.03-7.88 (m, 9H), 7.43-7.27 (m, 15H), 7.21 (m, 3H), 5.09-4.90 (m, 6H), 4.30-4.11 (m, 6H), 3.72-3.43 (m, 7H), 3.32-3.25 (m, 3H), 3.21-3.11 (m, 3H), 3.04-2.90 (m, 6H), 2.45-2.26 (m, 12H), 2.24-2.09 (m, 6H), 1.90-1.77 (m, 3H), 1.74-1.65 (m, 3H), 1.63-1.55 (m, 3H), 1.53-1.41 (m, 6H), 1.39-1.35 (m, 54H), 1.28-1.19 (m, 6H)

25.9 Preparation of **82-93**

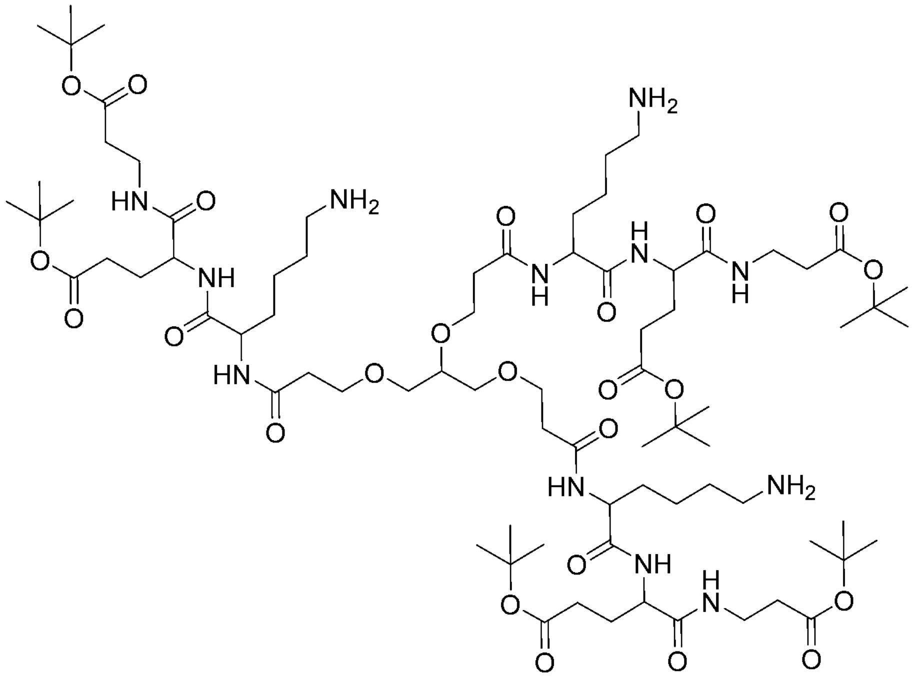

**82-78** (0.3060 g, 0.1506 mmol) and 10% Pd/C catalyst (0.01 g) were placed in a hydrogenation reactor, and DMF (30 mL) was added for dissolution. The hydrogenation reactor was sealed, evacuated with a water pump, and then filled with hydrogen gas. This process was repeated three times. The pressure reading of the hydrogenation reactor was adjusted to 1.5 MPa, and the reaction was carried out at room temperature overnight. After the reaction was completed, the reaction mixture was filtered through Celite, and the filter cake was washed with DMF (20 mL × 3). The DMF solution of the product was obtained and used as the raw material for the next reaction.

25.10 Preparation of **82-95**

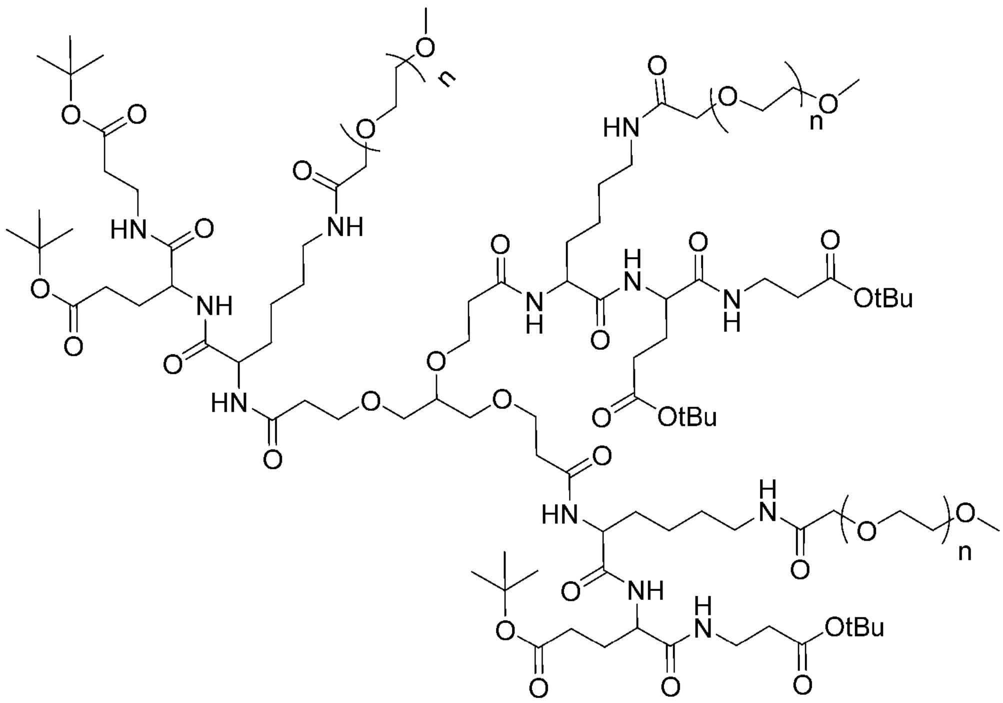

**82-93** (0.1506 mmol) was placed in a 250 mL flask. DMF (20 mL) was added for dissolution, then a DMF solution of M-SCM-10K (4.86 g, 0.4467 mmol, purchased from Jenkem) was added, and the mixture was reacted with stirring at low speed in the dark at room temperature for one week. After the reaction was completed, n-hexane (25 mL) and methyl tert-butyl ether (200 mL) were added for precipitation. A solid precipitated. The mixture was allowed to stand, the supernatant was then discarded, and n-hexane (25 mL) and methyl tert-butyl ether (200 mL) were added again for precipitation. This process was repeated three times. The filter cake was collected and dried to provide the product.

25.11 Preparation of **82-119**

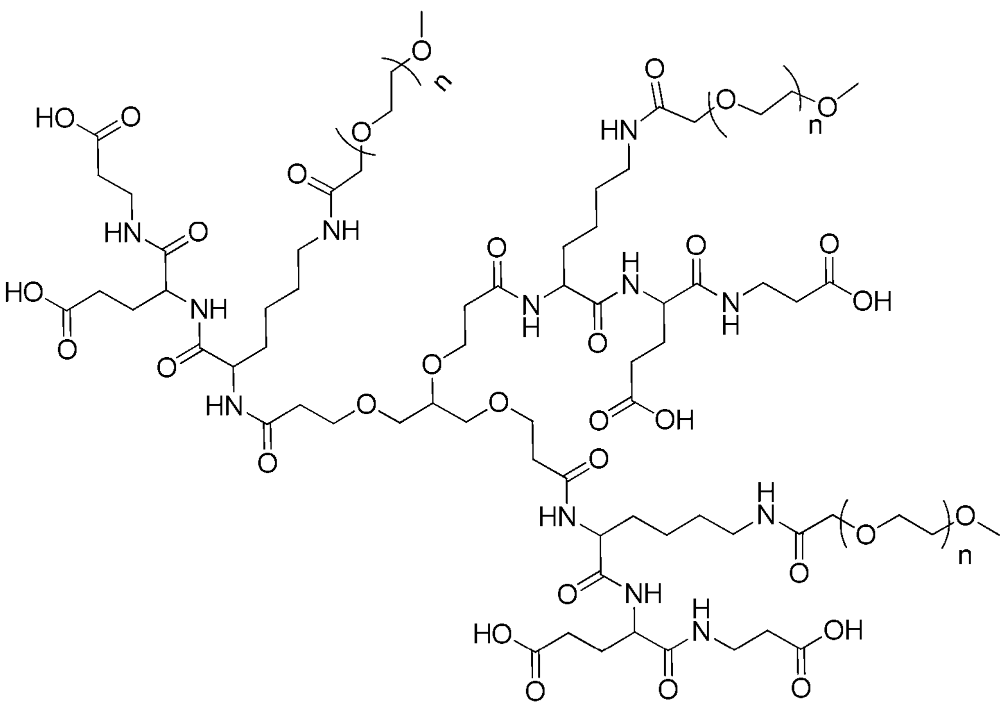

To a flask containing **82-95** (1.5 g, 0.0460 mmol), dichloromethane (10 mL) was added for dissolution with the assistance of ultrasonication, then TFA (10 mL) was added, and the mixture was reacted with stirring at room temperature overnight. After the reaction was completed, the mixture was evaporated on a rotary evaporator until a viscous oil formed. Methyl tert-butyl ether (60 mL) was then added for precipitation. A solid precipitated and were filtered. The filter cake was washed with methyl tert-butyl ether (40 mL × 3). The obtained product had poor solubility. The filter cake was collected and dried to provide the product.

25.12 Preparation of **82-122**

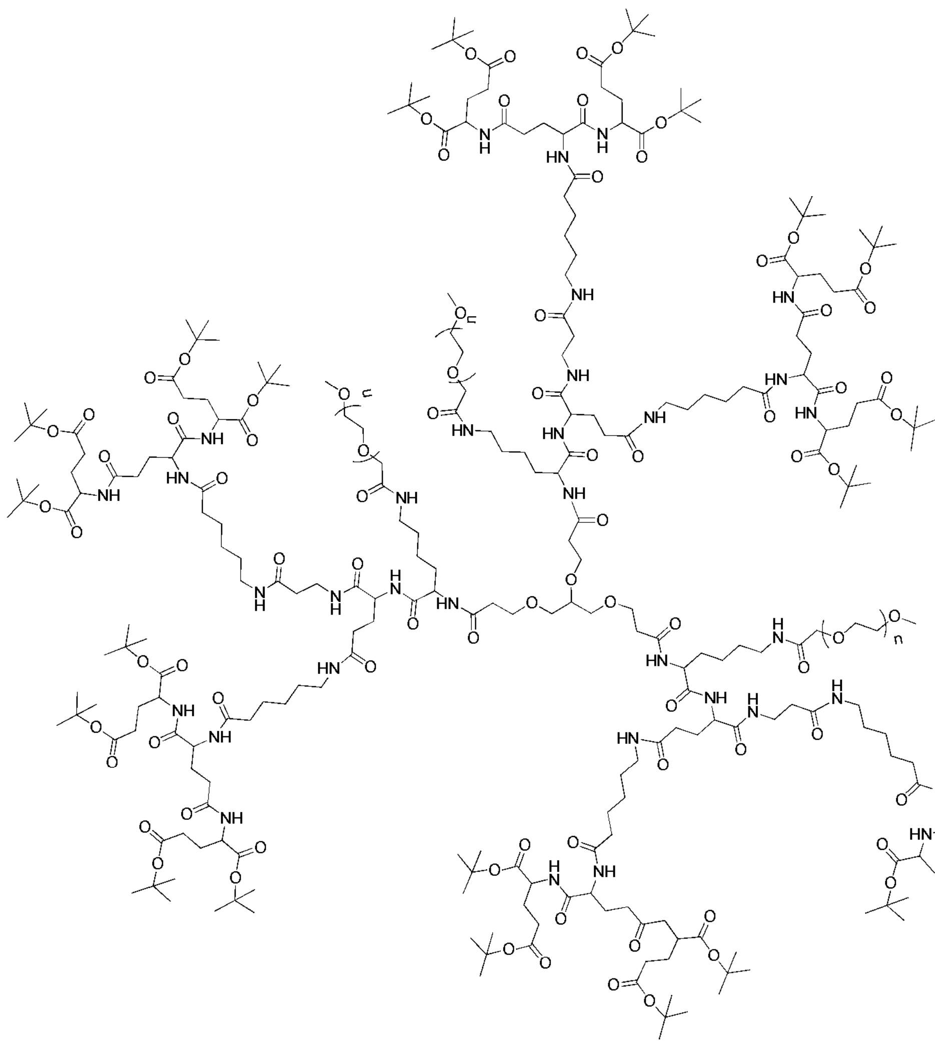

**38-244** (0.31 g, 0.4140 mmol), HBTU (0.21 g, 0.5520 mmol), and HOBT (0.07 g, 0.5520 mmol) were weighed and added to a flask containing **82-119** (0.0460 mmol), and then DMF (10 mL) was added to dissolve the mixture. DIEA (0.30 mL, 1.8040 mmol) was slowly added dropwise at -5°C. After the dropwise addition was completed, the reaction was carried out for half an hour, the flask was then taken out, and the reaction was carried out with stirring at room temperature overnight. After the reaction was completed, n-hexane (25 mL) and methyl tert-butyl ether (200 mL) were added for precipitation. After the mixture was allowed to stand, the supernatant was discarded, and n-hexane (25 mL) and methyl tert-butyl ether (200 mL) were added for precipitation. This process was repeated three times. The mixture was filtered, and the filter cake was collected, dissolved in a methanol/dichloromethane (1/4) mixture, then silica gel powder (100 mL) was added, and the mixture was evaporated to dryness. The resulting product was dry-loaded onto the column and subjected to column chromatography using a 6-12% methanol/dichloromethane mixture as the eluent. The desired product was collected, concentrated, and dried to provide 1.28 g of the product with a yield of 76.19%.

25.13 Preparation of **82-130**

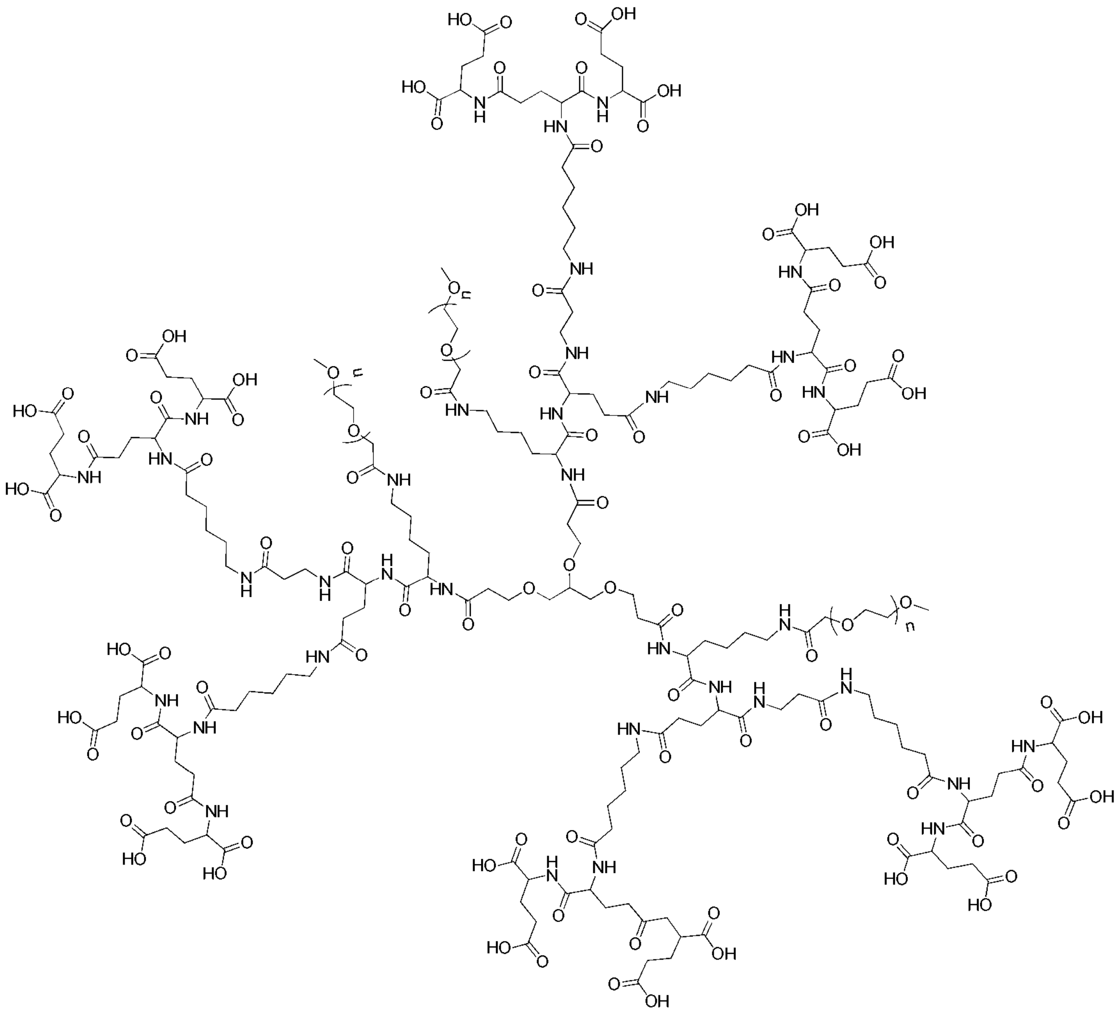

To a flask containing **82-122** (1.18 g, 0.0323 mmol), dichloromethane (15 mL) was added for dissolution with the assistance of ultrasonication, then TFA (10 mL) was added, and the mixture was reacted with stirring at room temperature overnight. After the reaction was completed, the mixture was evaporated to dryness on a rotary evaporator until an oily substance was obtained. Methyl tert-butyl ether (60 mL) was then added to the mixture to allow precipitation. A solid precipitated and was filtered. The filter cake was washed with methyl tert-butyl ether (40 mL × 3). The filter cake was collected and dried to provide 1.2 g of the product.

25.14 Preparation of **82-152**

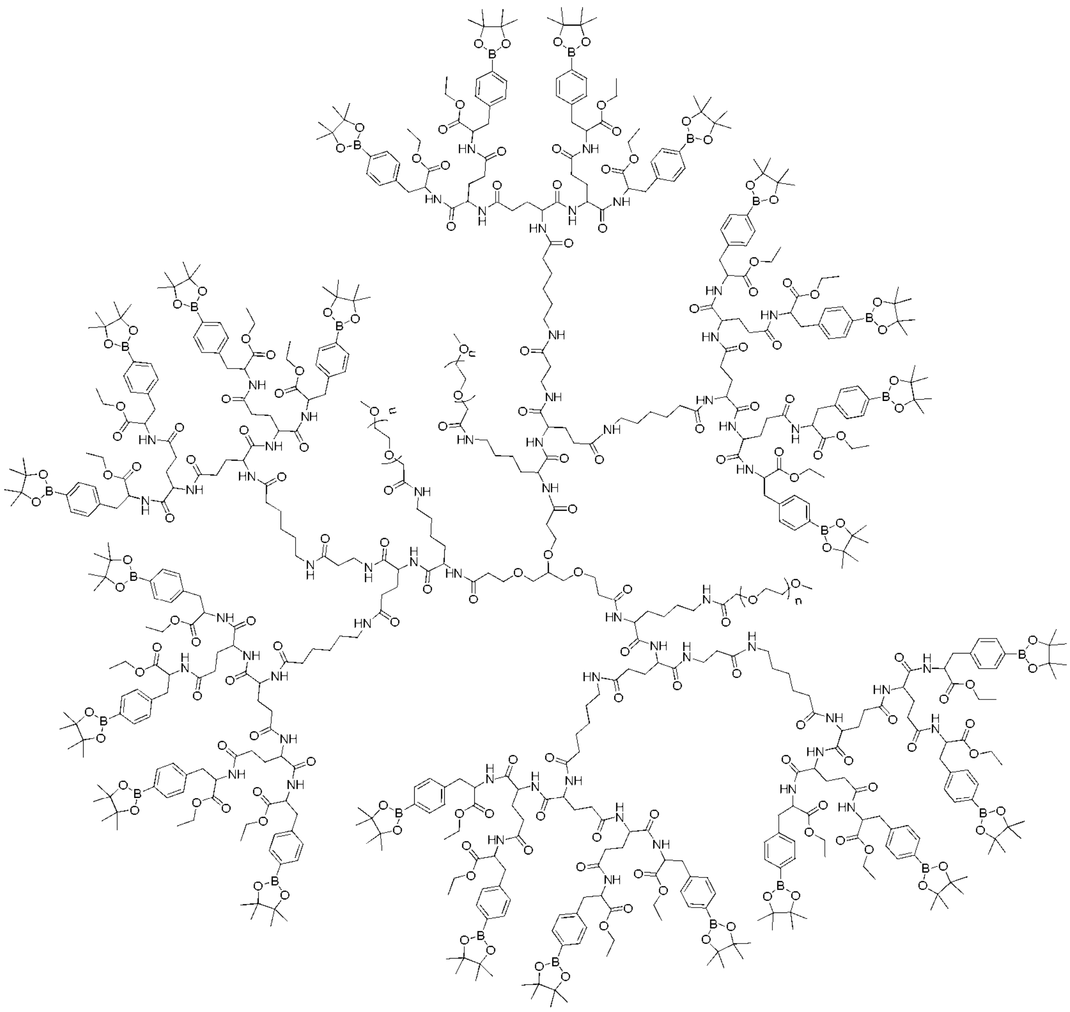

**82-130** (0.72 g, 0.0204 mmol), EDCI (0.18 g, 0.9807 mmol), and HOBT (0.13 g, 0.9807 mmol) were weighed and added to a flask containing **82-98** (0.31 g, 0.9807 mmol), and then DMF (20 mL) was added to dissolve the mixture. DIEA (0.32 mL, 1.9613 mmol) was slowly added dropwise at -5°C. After the dropwise addition was completed, the reaction was carried out for half an hour, the flask was then taken out, and the reaction was carried out with stirring at room temperature overnight. After the reaction was completed, n-hexane (25 mL) and methyl tert-butyl ether (200 mL) were added for precipitation. The supernatant was discarded, and n-hexane (25 mL) and methyl tert-butyl ether (200 mL) were added again for precipitation. This process was repeated three times. The mixture was filtered, and the filter cake was collected, then dissolved in a methanol/dichloromethane (1/4) mixture. Silica gel powder (100 mL) was added, and the mixture was evaporated to dryness to provide a powdery solid. The powdery solid was dry-loaded onto the column and subjected to column chromatography using a 6-13% methanol/dichloromethane mixture as the eluent. The desired product was collected, concentrated, and dried in a vacuum oven to provide 0.4 g of the product with a yield of 46.19%. $^1$H-NMR (600 MHz, DMSO-$d_6$) $\delta$ 8.69-8.45 (m, 33H), 8.30-8.17 (m, 25H), 8.03-7.92 (m, 22H), 7.89-7.78 (m, 15H), 7.75-7.57 (m, 14H), 7.40-7.25 (m, 18H), 4.20-4.05 (m, 21H), 4.04-3.93 (m, 79H), 3.66-3.57 (m, 28H), 3.58-3.51 (m, 3555H), 3.40-3.38 (m, 27H), 3.19-3.13 (m, 31H), 3.08-3.02 (m, 38H), 2.92-2.88 (m, 47H), 1.36-1.35 (m, 55H), 1.23-1.21 (m, 288H), 1.20-1.17 (m, 72H)

Example 26: Synthesis of Compound **82-132**

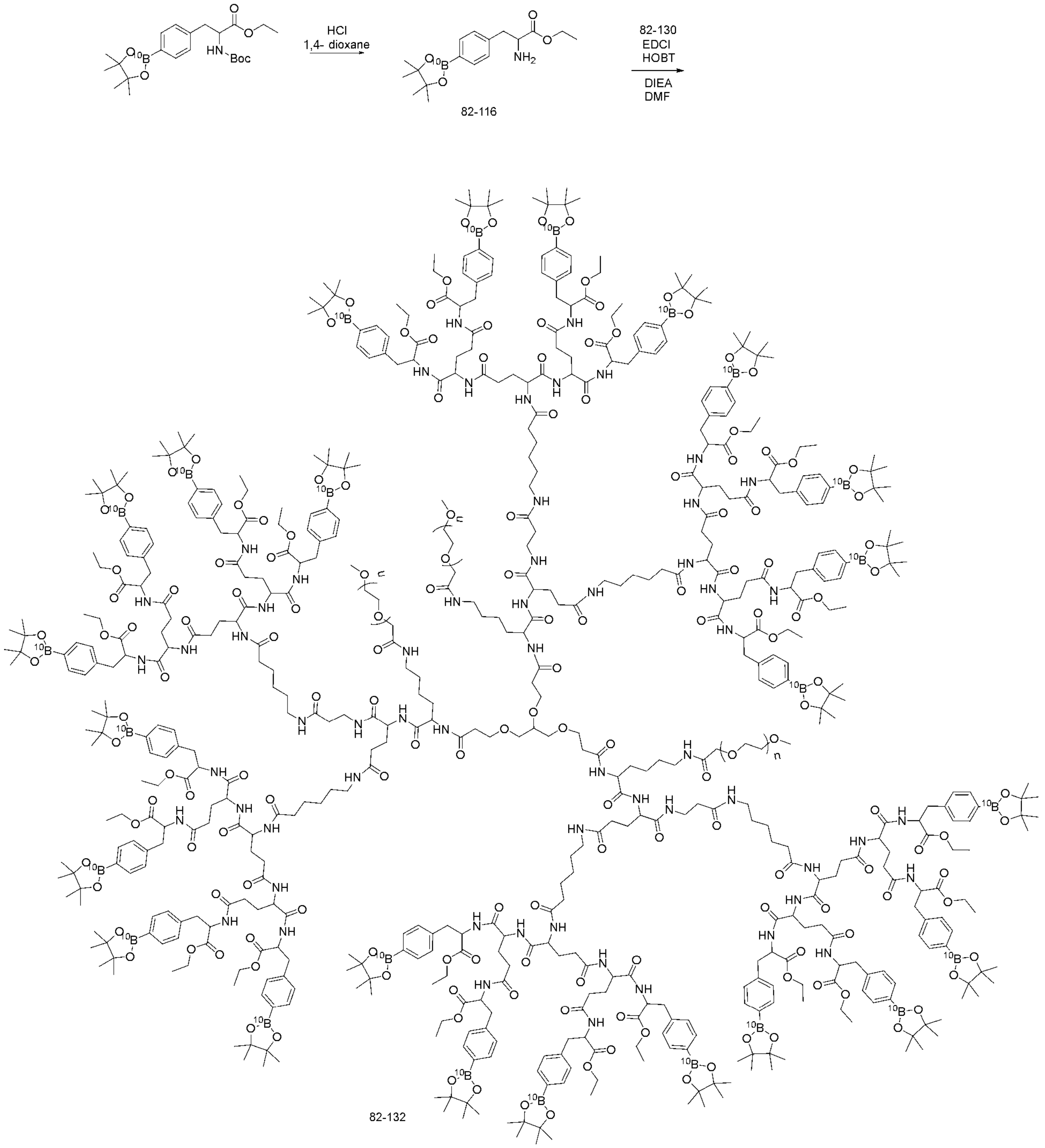


82-116

82-132

26.1 Preparation of Compound **82-116**

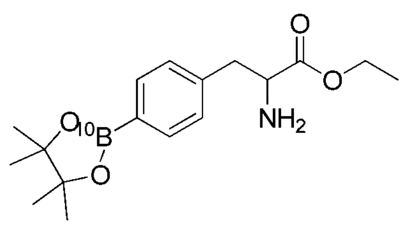

To a flask containing ethyl 2-((tert-butoxycarbonyl)amino)-3-(4-(4,4,5,5-tetramethyl-1,3,2-dioxaborolan-2-yl)

phenyl)propanoate ($^{10}B$) (1 g, 3.1328 mmol, purchased from Lakestar PharmaTech), dichloromethane (10 mL) was added for dissolution with the assistance of ultrasonication, then TFA (5 mL) was added, and the mixture was reacted with stirring at room temperature overnight. After the reaction was completed, the reaction mixture was evaporated to an oily state using a rotary evaporator. Methyl tert-butyl ether (60 mL) was then added for precipitation. A powdery solid precipitated from the reaction mixture and was filtered. The filter cake was washed with methyl tert-butyl ether (40 mL × 3). The filter cake was collected and dried in a vacuum oven to provide 0.95 g of the product. $^1$H-NMR (600 MHz, DMSO-$d_6$) δ 8.59 (s, 2H), 7.64 (d, *J* = 8.0 Hz, 2H), 7.27 (d, *J* = 7.9 Hz, 2H), 4.25 (dd, *J* = 7.5, 6.0 Hz, 1H), 4.18-4.06 (m, 2H), 3.21 (dd, *J* = 14.0, 5.8 Hz, 1H), 3.09 (dd, *J* = 14.0, 7.6 Hz, 1H), 1.29 (s, 12H), 1.10 (t, *J* = 7.1 Hz, 3H)

26.2 Preparation of **82-132**

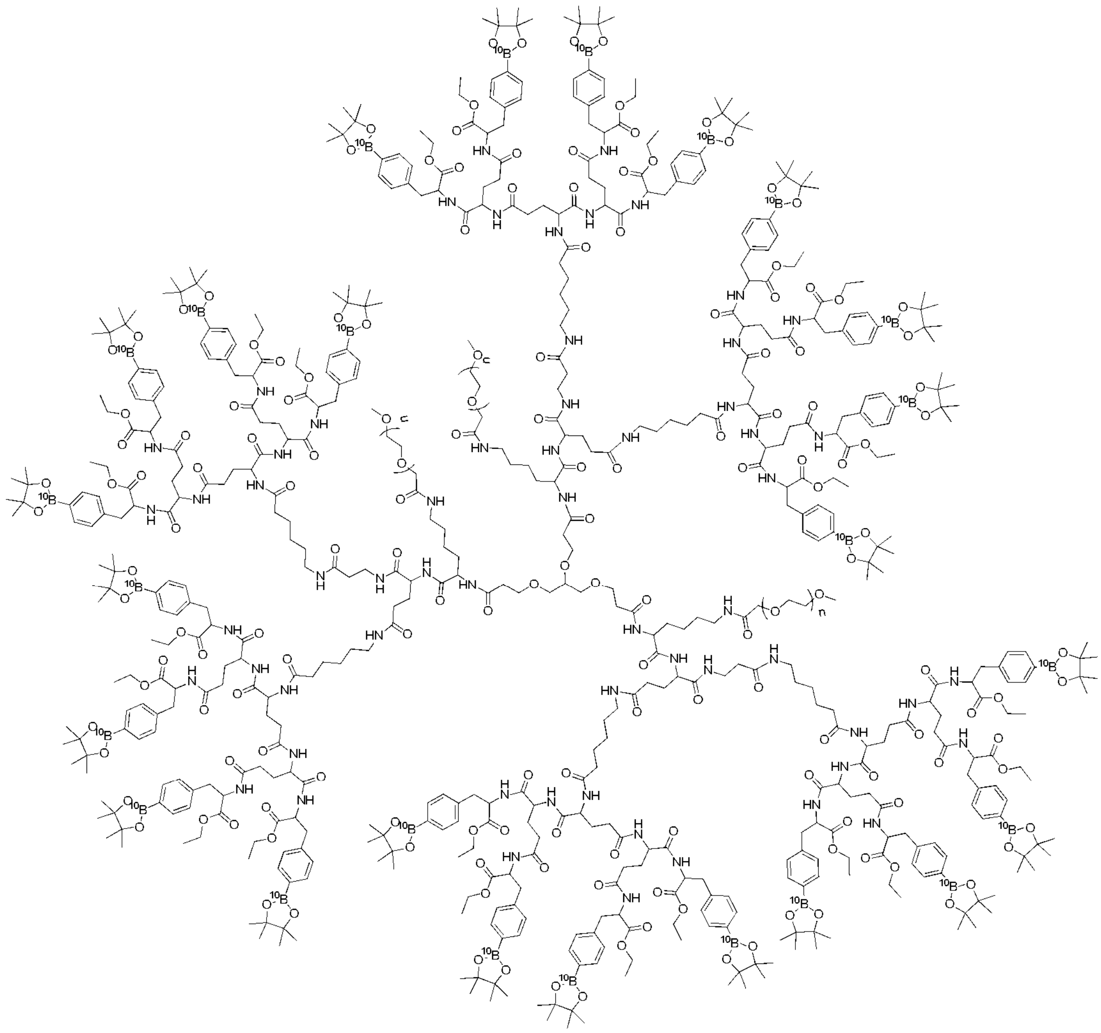

**82-130** (0.323 mmol), EDCI (0.29 g, 1.5480 mmol), and HOBT (0.21 g, 1.5480 mmol) were weighed and added to a reaction flask containing **82-116** (0.49 g, 1.5480 mmol), and then DMF (10 mL) was added to dissolve the mixture. DIEA (0.51 mL, 3.0960 mmol) was slowly added dropwise at -5°C. After the dropwise addition was completed, the reaction was carried out for half an hour, the flask was then taken out, and the reaction was carried out with stirring at room temperature overnight. After the reaction was completed, n-hexane (25 mL) and methyl tert-butyl ether (200 mL) were added for precipitation. The supernatant was discarded, and n-hexane (25 mL) and methyl tert-butyl ether (200 mL) were added again for precipitation. This process was repeated three times. The mixture was filtered to provide a solid product, which was collected, and dried to provide 0.35 g of the product with a yield of 25.73%. $^1$H-NMR (600 MHz, DMSO-$d_6$) δ 8.53-8.37 (m, 56H), 8.02-7.92 (m, 24H), 7.77-7.67 (m, 24H), 7.60-7.52 (m, 24H), 7.45-7.39 (m, 24H), 4.16-4.08 (m, 22H), 3.87-3.80 (m, 24H), 3.69-3.60 (m, 107H), 3.59-3.48 (m, 3158H), 3.43-3.41 (m, 31H), 3.27-3.21 (m, 32H), 3.16-3.07 (m, 27H), 2.96-2.86 (m, 49H), 1.30-1.28 (m, 55H), 1.24-1.21 (m, 288H), 1.21-1.17 (m, 72H)

Example 27: Synthesis of Compound **82-208**

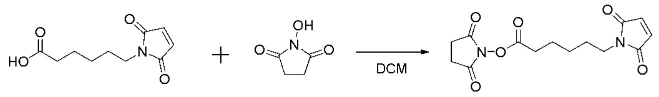


78-146

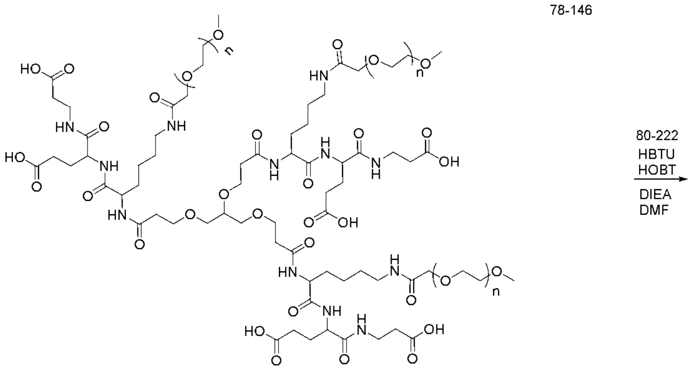


abbreviated as

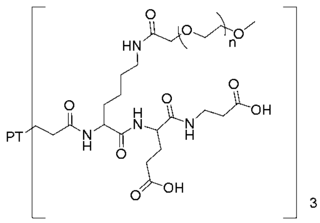

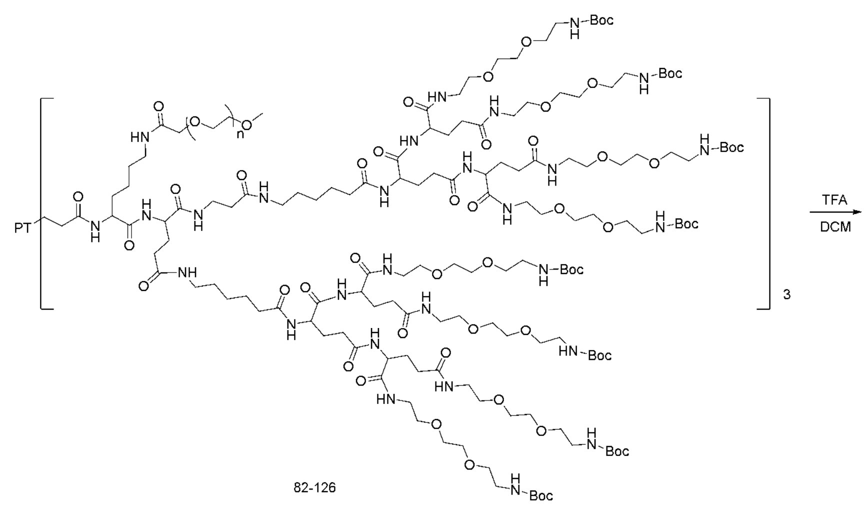


82-126

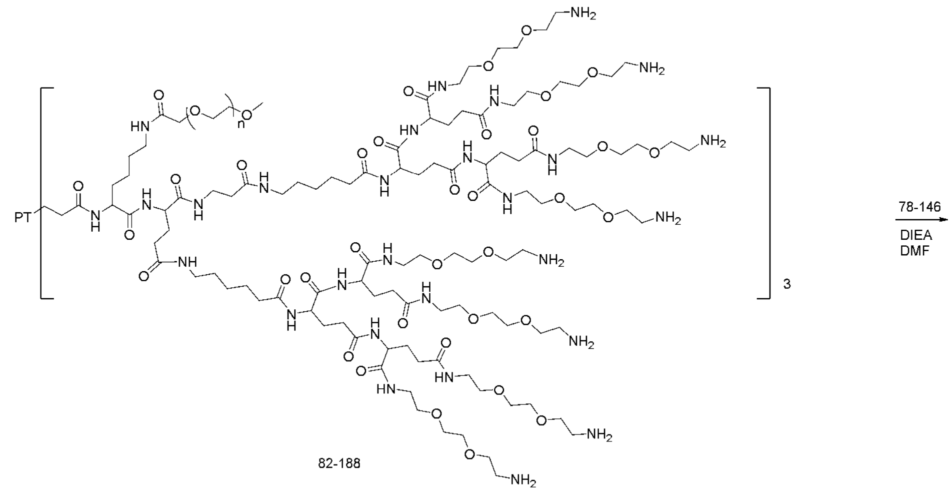


82-188

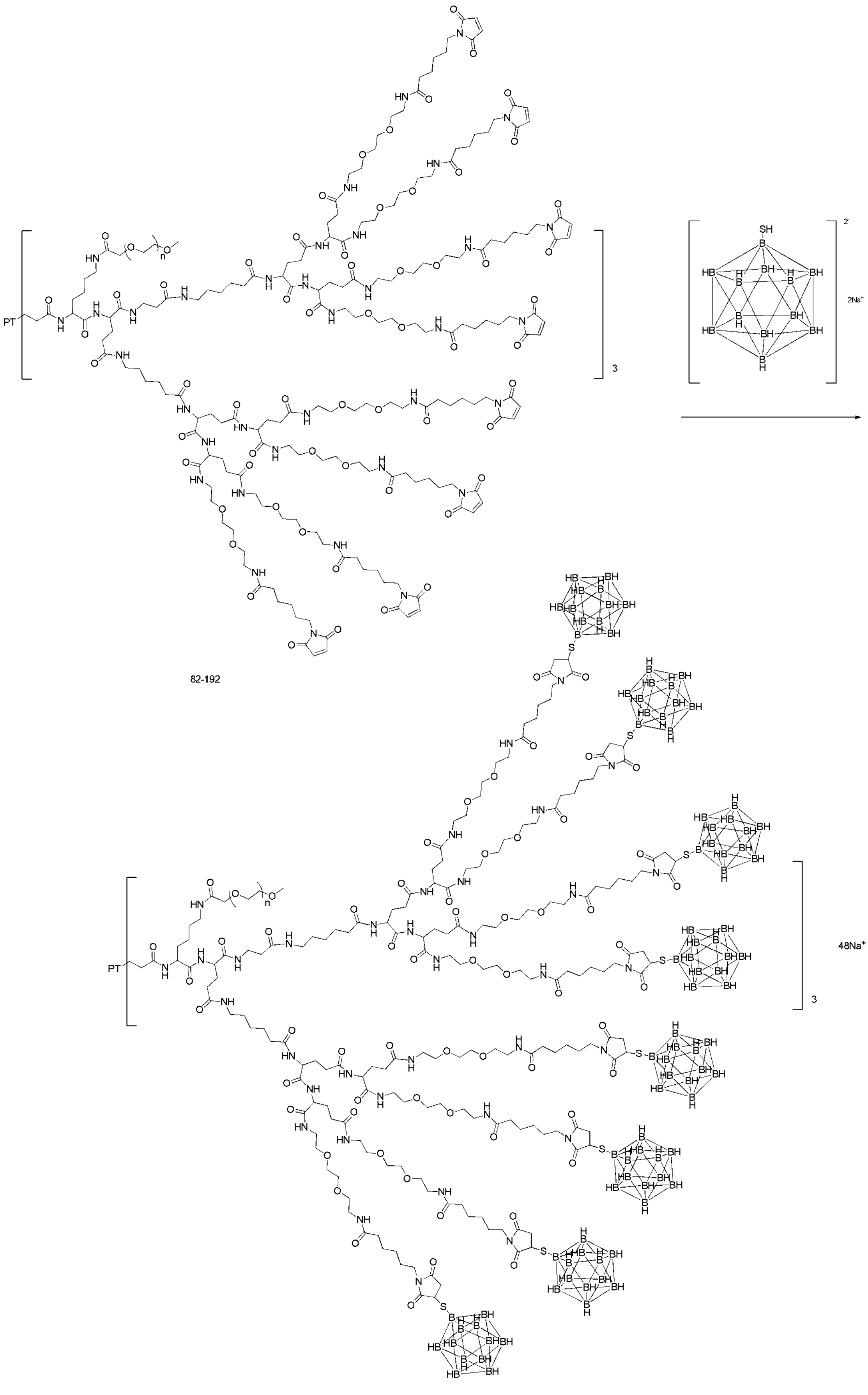

82-192

82-208

27.1 Preparation of **78-146**

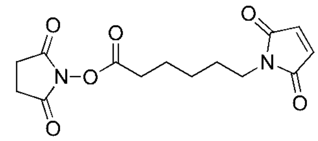

5-Maleimidocaproic acid (2 g, 9.4751 mmol, purchased from Sun Chemical Technology (Shanghai) Co.,Ltd.) and N-hydroxysuccinimide (1.42 g, 12.3176 mmol) were weighed and added to a 500 mL flask, then DCC (2.54 g, 12.3176 mmol) and dichloromethane (30 mL) were added for dissolution with the assistance of ultrasonication, and the mixture was reacted with stirring at room temperature overnight. After the reaction was completed, the reaction mixture was concentrated under reduced pressure and evaporated to dryness to provide a solid. The solid was then dissolved in a methanol/dichloromethane (1/4) mixture, then silica gel powder (100 mL) was added, and the mixture was evaporated to dryness to provide a powdery solid. The powdery solid was dry-loaded onto the column and subjected to column chromatography using a 2-4% methanol/dichloromethane mixture as the eluent. The desired product was collected, concentrated, and dried in a vacuum oven to provide 1.17 g of the product with a yield of 40%.

27.2 Preparation of **82-126**

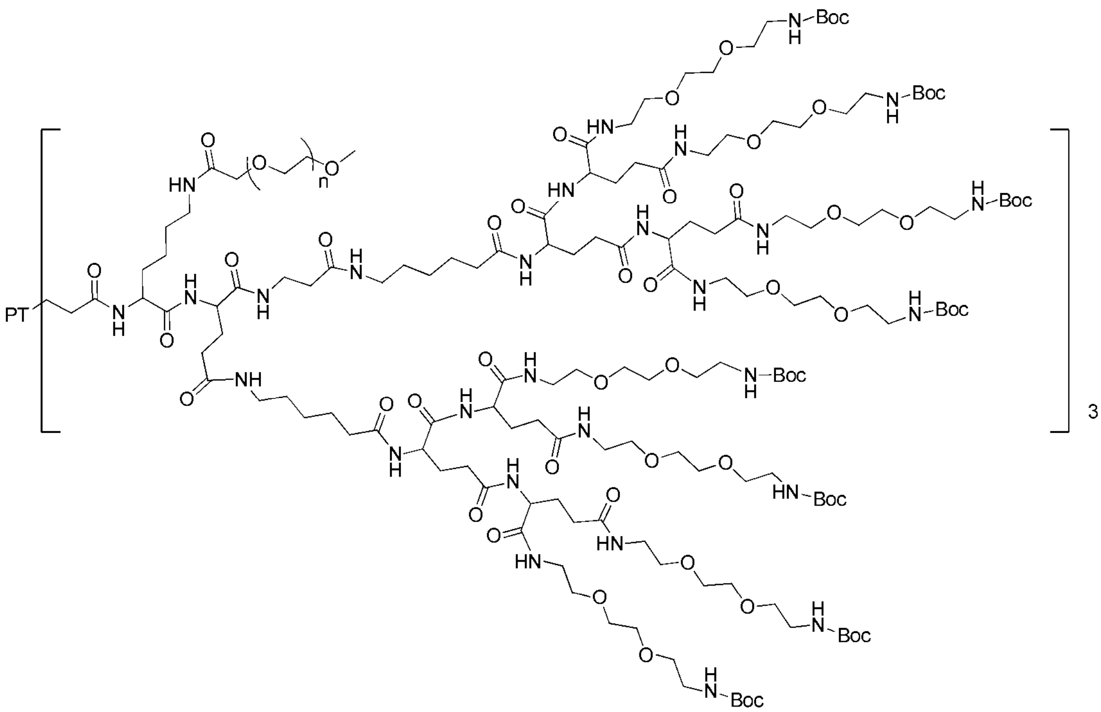

**82-119** (0.0850 mmol), HBTU (0.22 g, 0.6013 mmol), and HOBT (0.08 g, 0.0613 mmol) were weighed and added to a flask containing **80-222** (0.9 g, 0.6013 mmol), and then DMF (5 mL) was added for dissolution. DIEA (0.30 mL, 1.8040 mmol) was slowly added dropwise at -5°C. After the dropwise addition was completed, the reaction was carried out for half an hour, the flask was then taken out, and the reaction was carried out with stirring at room temperature overnight. After the reaction was completed, n-hexane (25 mL) and methyl tert-butyl ether (200 mL) were added for precipitation. The supernatant was discarded, and n-hexane and methyl tert-butyl ether were added again for precipitation. This process was repeated three times. The mixture was filtered to provide a solid product, which was collected, and dried to provide the product.

27.3 Preparation of **82-188**

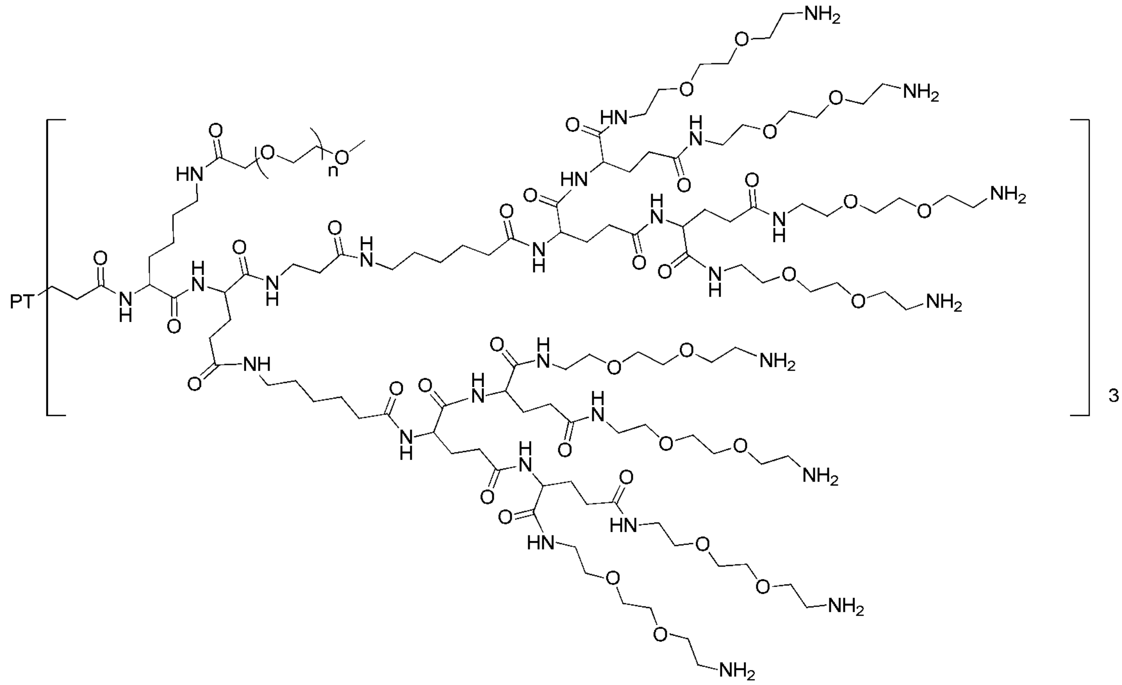

To a flask containing **82-126** (1.05 g, 0.0254 mmol), dichloromethane (10 mL) was added for dissolution with the assistance of ultrasonication, then TFA (5 mL) was added, and the mixture was reacted with stirring at room temperature overnight. After the reaction was completed, the reaction mixture was evaporated to an oily state using a rotary evaporator. Methyl tert-butyl ether (60 mL) was then added for precipitation. A powdery solid precipitated from the reaction mixture and was filtered, and the filter cake was washed with methyl tert-butyl ether (40 mL × 3). The resulting product had poor solubility. The filter cake was collected and dried in a vacuum oven to provide the product.

27.4 Preparation of **82-192**

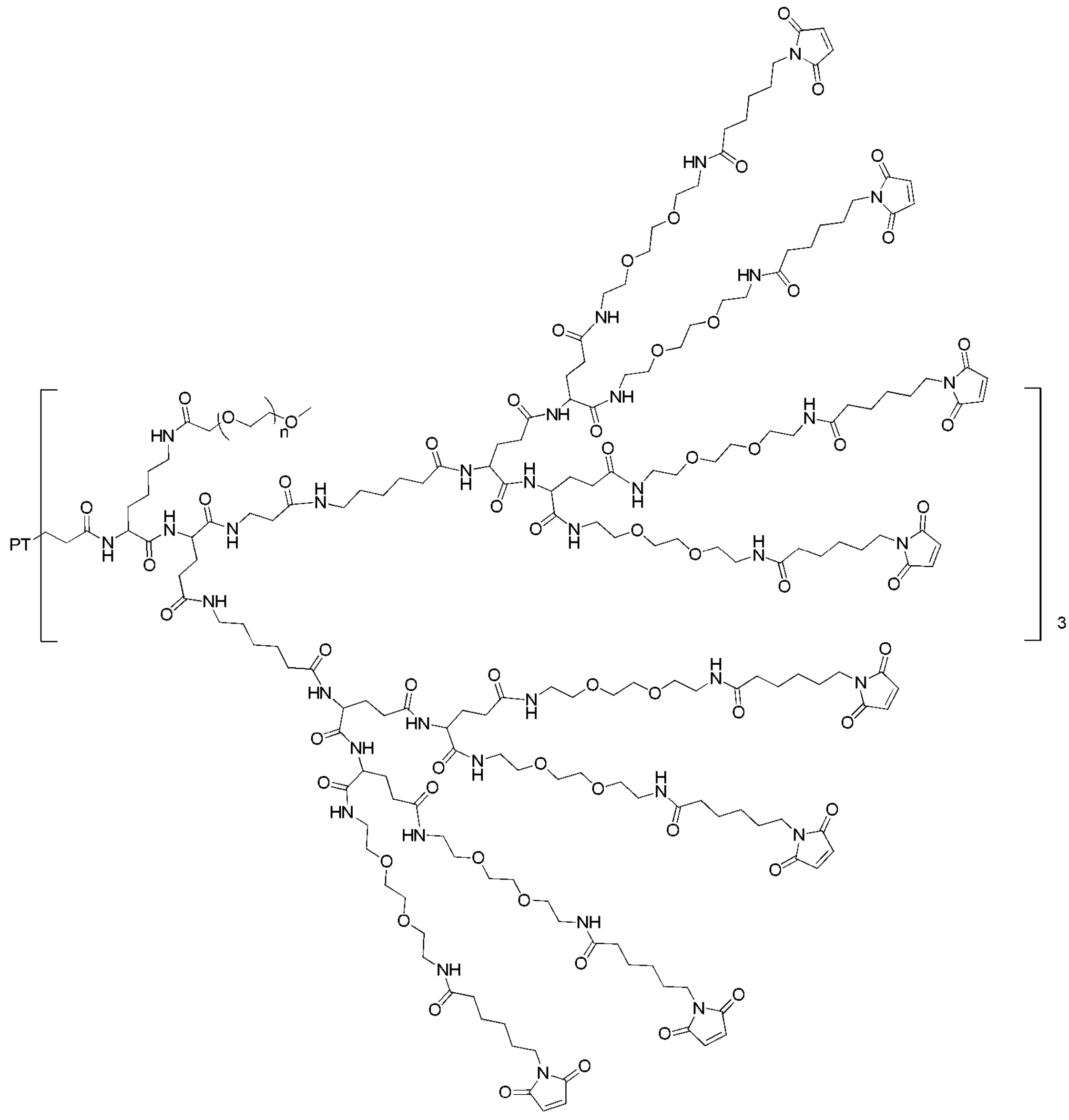

**78-146** (0.28 g, 0.9144 mmol) was weighed and added to a flask containing **82-188** (0.99 g, 0.0254 mmol), and then DMF (10 mL) was added to dissolve the mixture. DIEA (0.60 mL, 3.6080 mmol) was slowly added dropwise at -5°C. After the dropwise addition was completed, the reaction was carried out for half an hour, the flask was then taken out, and the reaction was carried out with stirring at room temperature overnight. After the reaction was completed, n-hexane (25 mL) and methyl tert-butyl ether (200 mL) were added for precipitation. The supernatant was discarded, and n-hexane and methyl tert-butyl ether were added for precipitation. This process was repeated three times. The mixture was filtered to provide a solid product, which was collected, and dried to provide the product.

27.5 Preparation of **82-208**

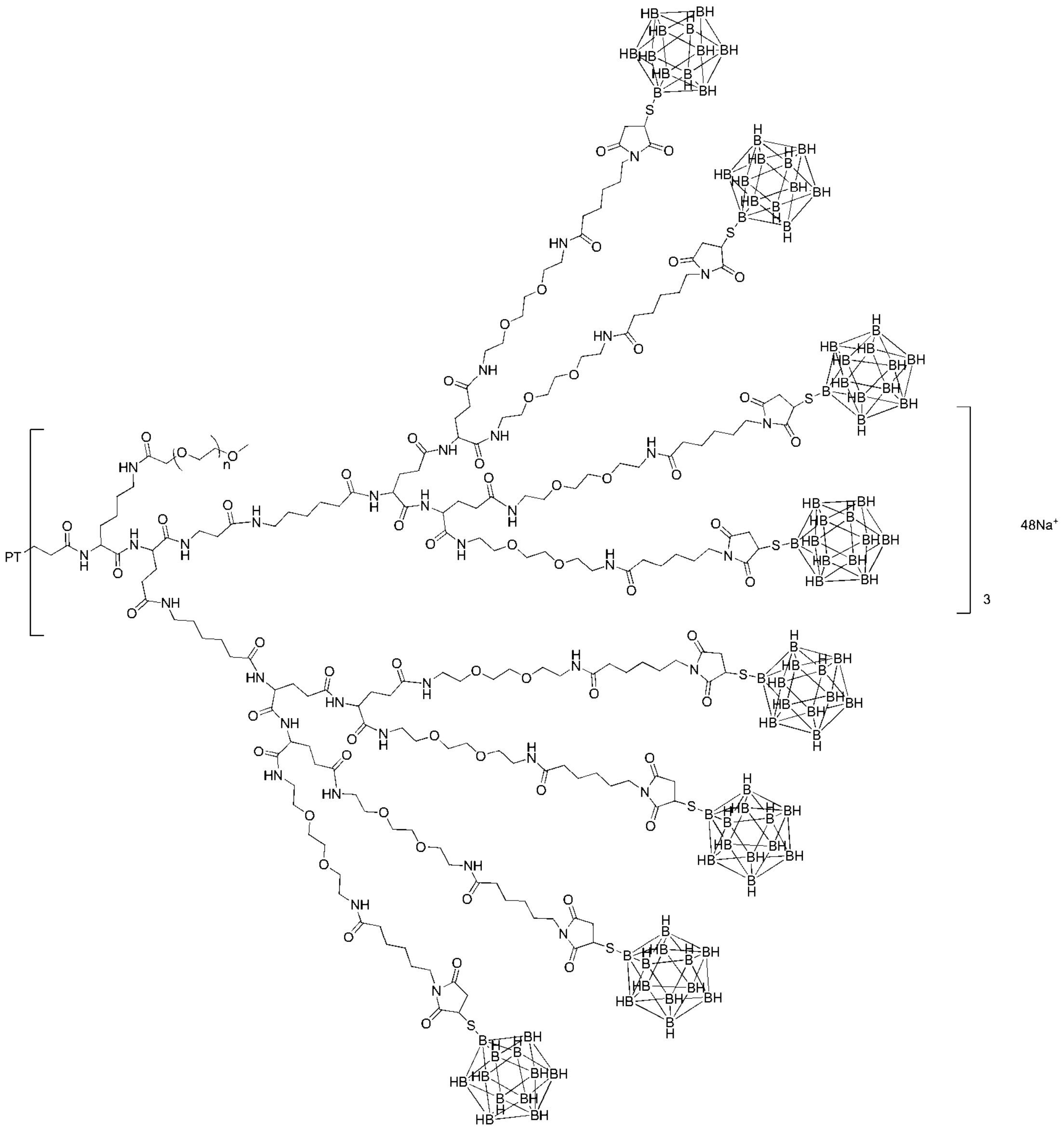

**82-192** (0.35 g, 0.0080 mmol) was placed in a 500 mL flask containing DMSO (5 mL), then disodium undecahydromercaptododecaboride (0.05 g, 0.2304 mmol, purchased from CATCHEM, Czech Republic) was added, and the mixture was reacted with stirring at room temperature overnight. After the reaction was completed, n-hexane (25 mL) and methyl tert-butyl ether (200 mL) were added to the reaction mixture for precipitation. The supernatant was discarded, and n-hexane and methyl tert-butyl ether were added again for precipitation. This process was repeated three times. The mixture was filtered to provide a solid product, which was then dissolved in a methanol/dichloromethane (1/4) mixture, and n-hexane (50 mL) and methyl tert-butyl ether (100 mL) were added for precipitation. This process was repeated five times. The mixture was filtered to provide a solid, which was collected and dried in a vacuum oven to provide 0.25 g of the product. $^1$H-NMR (600 MHz, DMSO-$d_6$) $\delta$ 8.46-7.64 (m, 87H), 4.26-4.19 (m, 24H), 3.90-3.78 (m, 12H), 3.74-3.68 (m, 3H), 3.66-3.58 (m, 32H), 3.56-3.41 (m, 2968H), 3.21-3.16 (m, 98H), 3.11-2.98 (m, 100H), 2.91-2.87 (m, 16H), 2.79-2.69 (m, 22H), 2.68-2.58 (m, 22H), 2.19-1.98 (m, 67H), 2.08-1.98 (m, 59H), 1.89-1.65 (m, 51H), 1.55-1.33 (m, 146H), 1.26-1.15 (m, 137H), 0.99-0.58 (m, 271H)

Example 28: Synthesis of Compound **82-207**

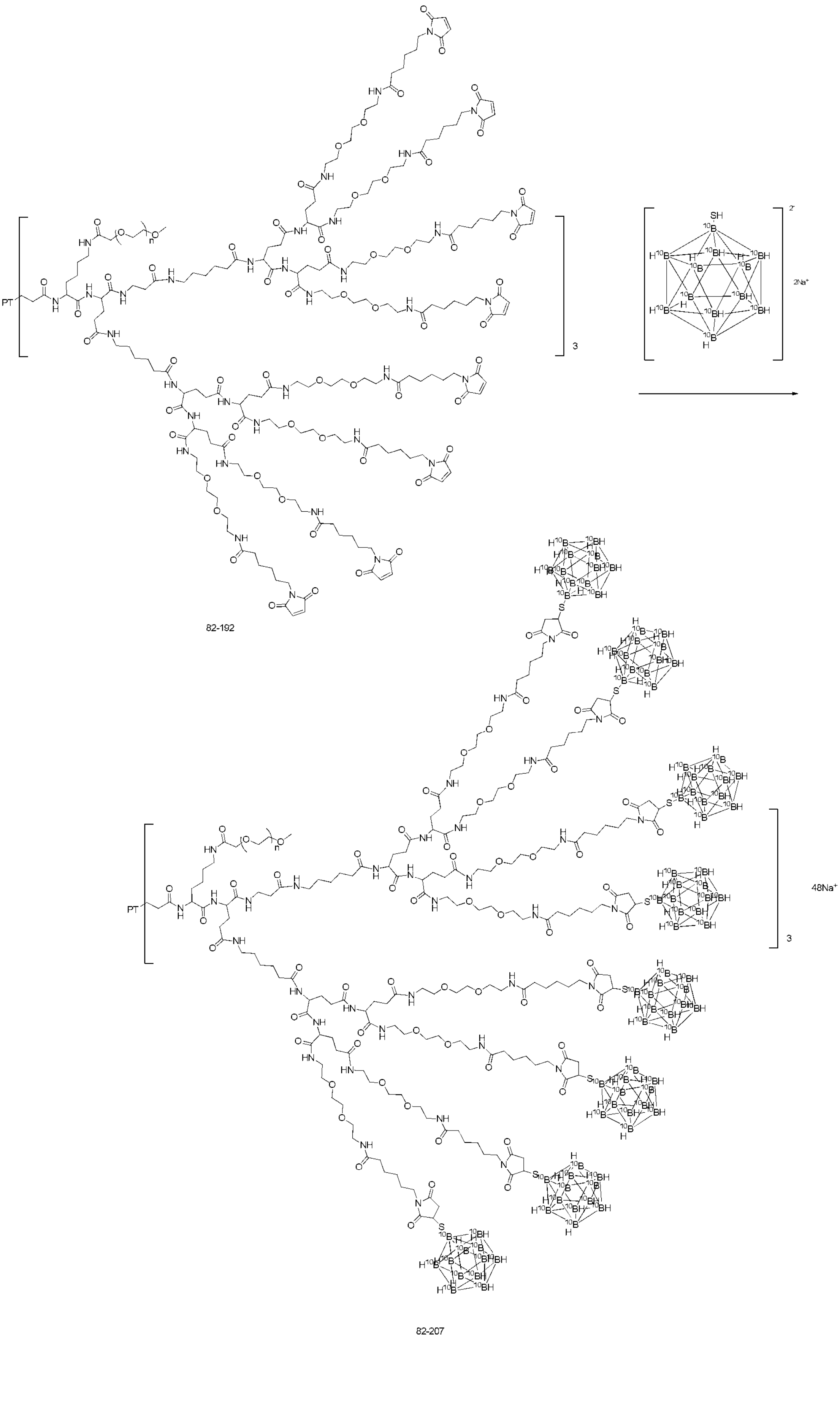

82-192

82-207

28.1 Preparation of **82-207**

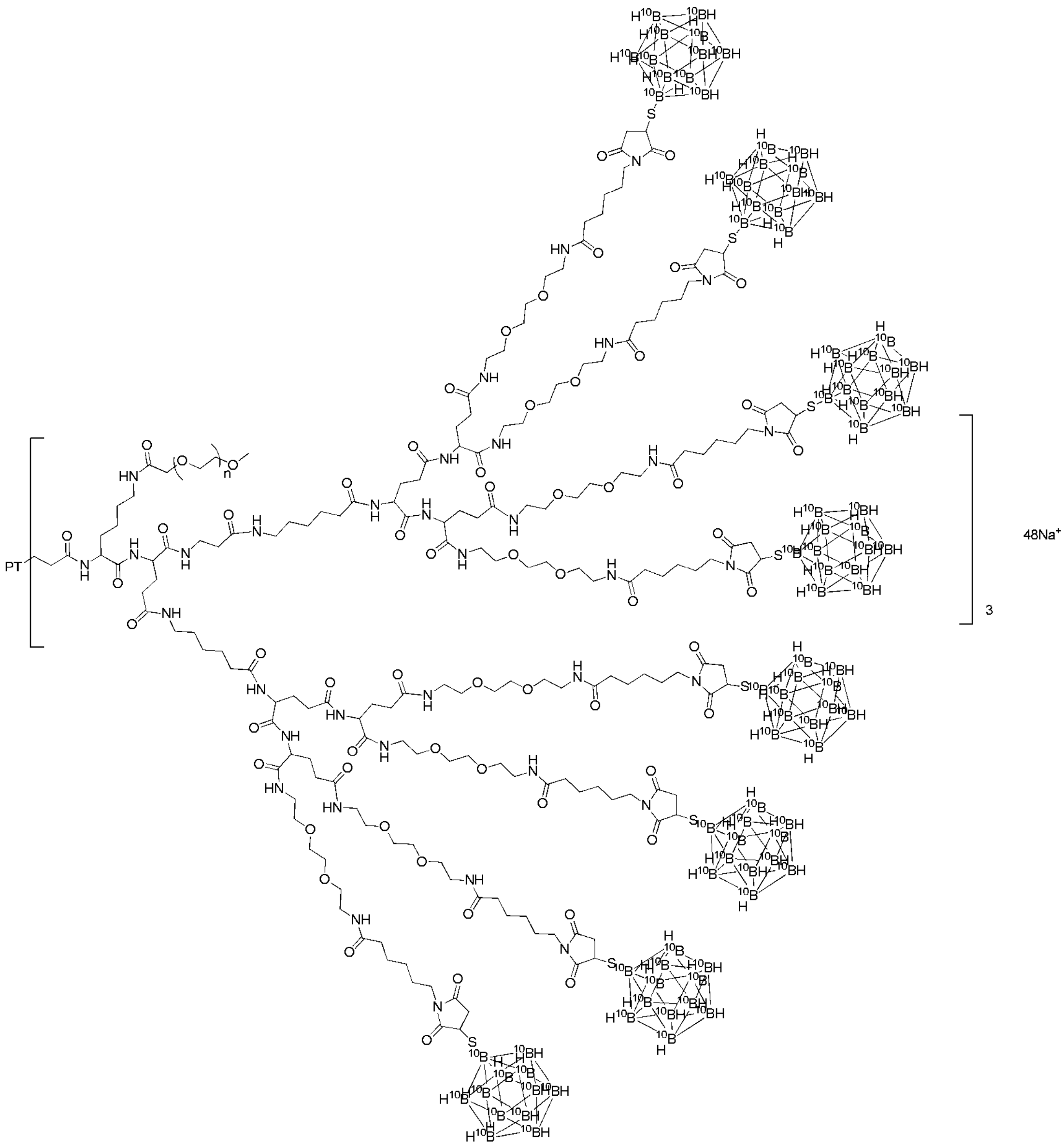

**82-192** (0.75 g, 0.0172 mmol) was placed in a 250 mL flask containing DMSO (10 mL), then disodium undecahydromercaptododecaboron ($^{10}B$) (0.10 g, 0.4954 mmol, purchased from CATCHEM, Czech Republic) was added, and the mixture was reacted with stirring at room temperature overnight. After the reaction was completed, n-hexane (25 mL) and methyl tert-butyl ether (200 mL) were added for precipitation. The supernatant was discarded, and n-hexane and methyl tert-butyl ether were added again for precipitation. This process was repeated three times. The mixture was filtered to provide a solid product, which was then dissolved in a methanol/dichloromethane (1/4) mixture, and n-hexane (50 mL) and methyl tert-butyl ether (100 mL) were added for precipitation. This process was repeated five times. The mixture was filtered, the solid was collected and dried in a vacuum oven to provide 0.66 g of the product. $^1$H-NMR (600 MHz, DMSO-$d_6$) $\delta$ 8.44-7.61 (m, 87H), 4.25-4.17 (m, 24H), 3.92-3.77 (m, 11H), 3.75-3.68 (m, 3H), 3.66-3.59 (m, 34H), 3.58-3.42 (m, 3035H), 3.22-3.17 (m, 102H), 3.10-2.98 (m, 103H), 2.91-2.88 (m, 16H), 2.78-2.69 (m, 20H), 2.67-2.57 (m, 21H), 2.19-1.97 (m, 65H), 2.07-1.97 (m, 58H), 1.89-1.63 (m, 52H), 1.57-1.33 (m, 152H), 1.28-1.14 (m, 145H), 0.98-0.60 (m, 269H)

Example 29: Synthesis of Compound **78-127**

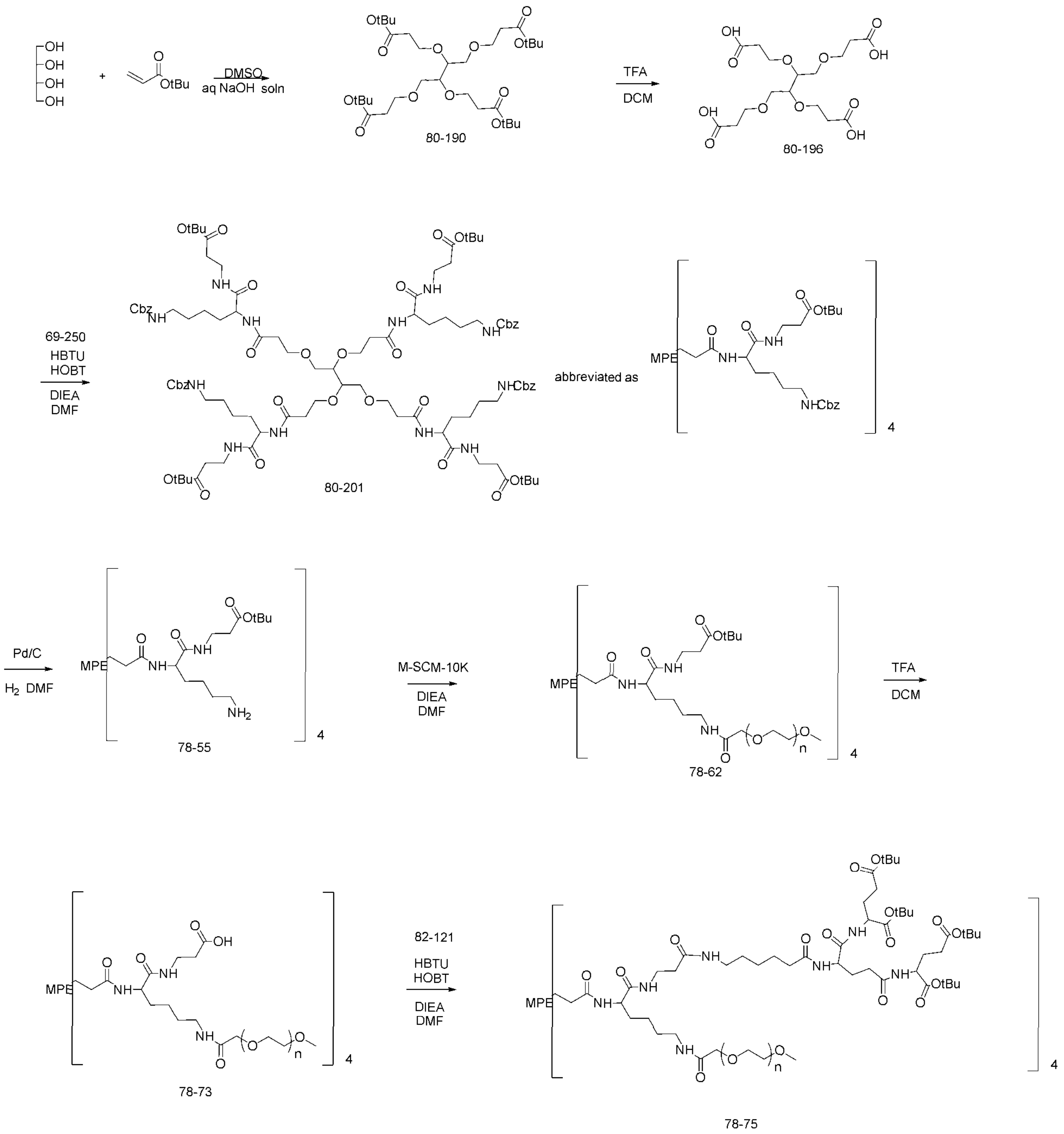

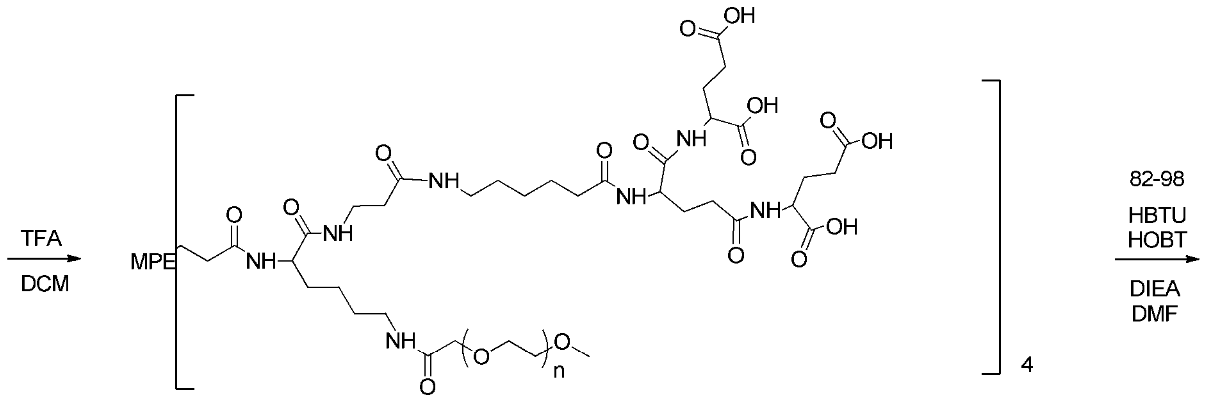


78-87

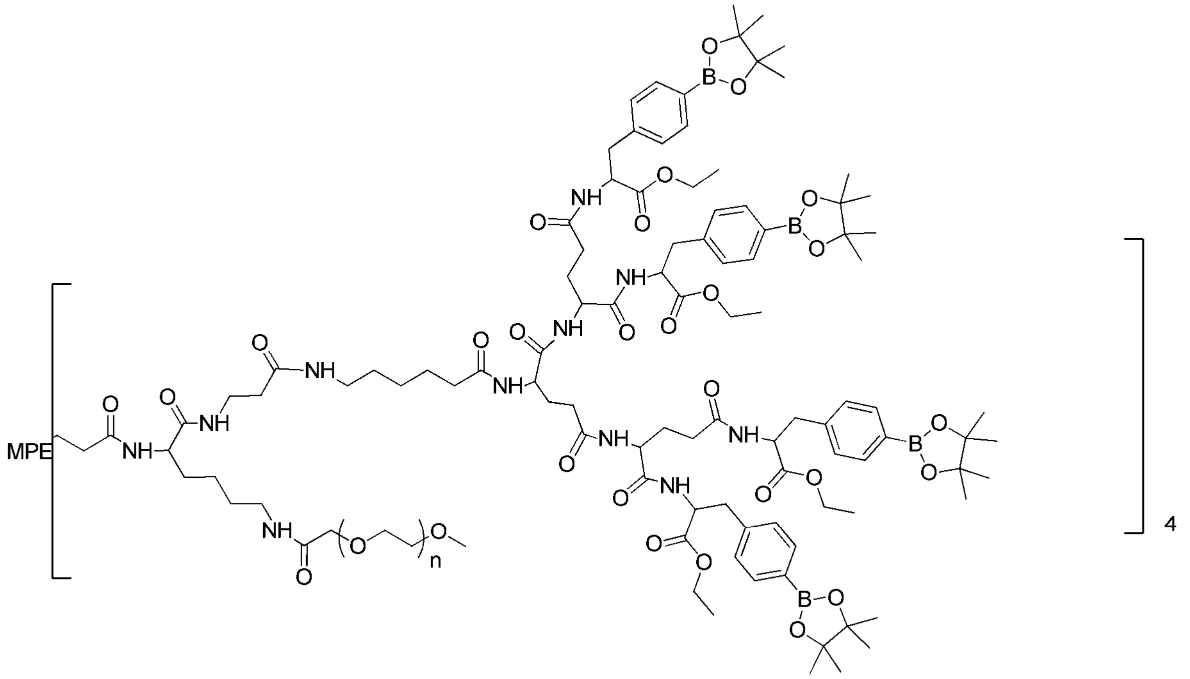

78-127

29.1 Preparation of **80-190**

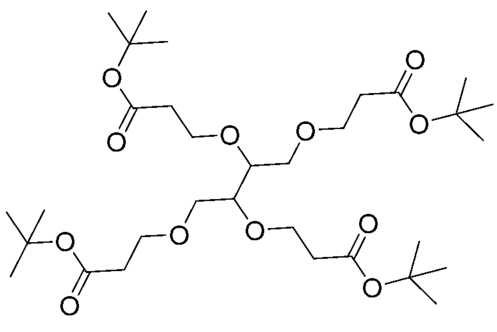

Meso-erythritol (2 g, 16.38 mmol) was weighed and added to a 250 mL flask, then DMSO (10 mL) was added for

dissolution. 5N sodium hydroxide solution (1 mL) was added dropwise with stirring and under nitrogen protection. The mixture was stirred for 10 minutes, tert-butyl acrylate (10.7 mL, 73.7 mmol) was then added dropwise. After the dropwise addition was completed, the flask was transferred to an oil bath at 35°C and the reaction was carried out with stirring overnight. After the reaction was completed, the mixture was poured into a separatory funnel containing saturated brine (200 mL) and ethyl acetate (200 mL) for extraction. After standing for phase separation, the organic phase was collected, and the aqueous phase was further extracted with ethyl acetate (200 mL × 3). The organic phases were combined, evaporated to dryness until an oily substance was obtained. The oil was then dissolved in a methanol/dichloromethane (1/4) mixture, then silica gel powder (100 mL) was added, and the mixture was evaporated to dryness to provide a powdery solid. The powdery solid was dry-loaded onto the column and subjected to column chromatography using a 5-8% ethyl acetate/petroleum ether mixture as the eluent. The desired product was collected, concentrated, and dried to provide 0.7 g of the product with a yield of 7%. $^1$H-NMR (600 MHz, DMSO-$d_6$) δ 3.69-3.62 (m, 2H), 3.64-3.60 (m, 2H), 3.59-3.55 (m, 4H), 3.51 (s, 2H), 3.40-3.36 (m, 4H), 2.42-2.39 (m, 4H), 2.38-2.32 (m, 4H), 1.40 (s, 36H); ESI [M+Na$^+$] 657.38

29.2 Preparation of **80-196**

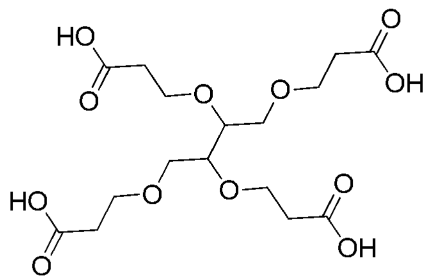

To a flask containing **80-190** (0.7 g, 1.1 mmol), dichloromethane (10 mL) was added for dissolution with the assistance of ultrasonication, then TFA (4.9 mL, 66 mmol) was added, and the mixture was reacted with stirring at room temperature overnight. After the reaction was completed, the reaction mixture was evaporated to an oily state using a rotary evaporator. The oil was dissolved in 30 mL of dichloromethane and then concentrated under reduced pressure. This process was repeated five times. Methyl tert-butyl ether (100 mL) was added for precipitation. After the mixture was allowed to stand, the supernatant was discarded. This process was repeated three times. Finally, the mixture was evaporated to dryness and dried in a vacuum oven to provide 0.45 g of the product with a yield exceeding 100%. ESI [M+Na$^+$] 433.13

29.3 Preparation of **80-201**

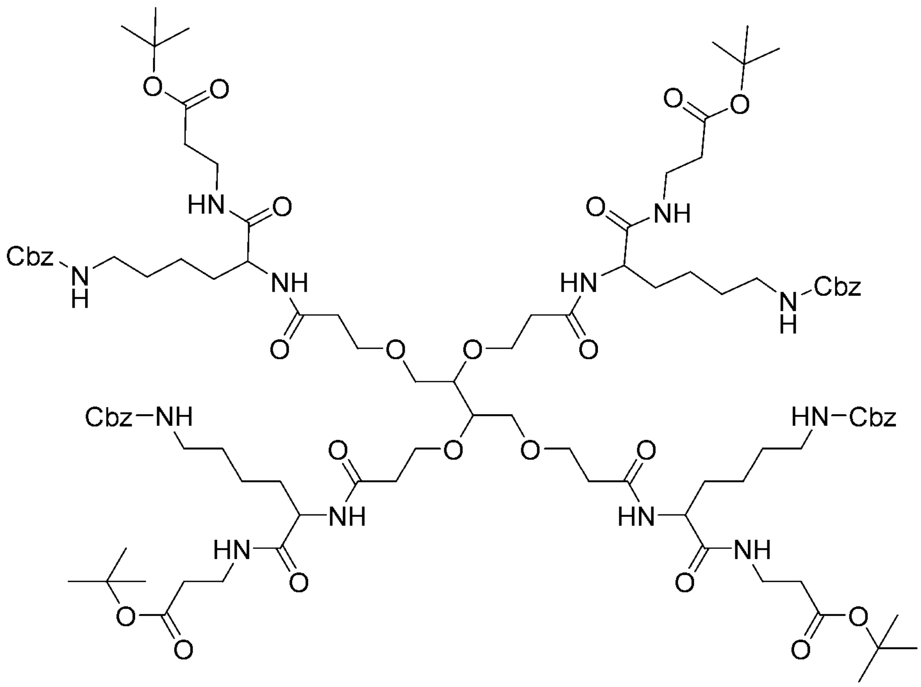

**80-196** (0.45 g, 1.1 mmol), **69-250** (2.0 g, 4.8 mmol), HBTU (1.8 g, 4.8 mmol), and HOBT (0.65 g, 4.8 mmol) were placed in a 500 mL flask, then DMF (30 mL) was added for dissolution, and the mixture was stirred at -5°C for approximately 20 minutes. DIEA (2.2 mL, 13.2 mmol) was then slowly added dropwise, and the reaction was continued at -5°C for 1 hour. The mixture was then brought to room temperature and stirred overnight. After the reaction was completed, the reaction mixture was transferred to a 1 L separatory funnel containing saturated sodium bicarbonate solution (200 mL) and ethyl acetate (200 mL) for extraction. The organic phase was collected, and the aqueous phase was further extracted with ethyl acetate (200 mL × 2). The organic phases were combined, washed with saturated brine (200 mL × 2), then concentrated to reach a volume of about 100 mL. Silica gel powder (40 mL) was added, and the mixture was evaporated to dryness. The resulting product was dry-loaded onto the column and subjected to column chromatography using a 2.5-5% methanol/dichloromethane mixture as the eluent. The desired product was collected, evaporated to dryness, and dried in a vacuum oven to provide 0.8 g of the product with a yield of 37%. $^1$H-NMR (600 MHz, DMSO-$d_6$) δ 8.04-7.82 (m, 8H), 7.44-7.26 (m, 20H), 7.25-7.08 (m, 4H), 5.07-4.95 (m, 8H), 4.22-4.12 (m, 4H), 3.71-3.65 (m, 2H), 3.64-3.59 (m, 2H), 3.58-3.52 (m, 4H), 3.50-3.45 (m, 2H), 3.36-3.33 (m, 4H), 3.30-3.24 (m, 4H), 3.19-3.13 (m, 4H), 3.00-2.89 (m, 8H), 2.42-2.25 (m, 16H), 1.61-1.53 (m, 4H), 1.49-1.43 (m, 4H), 1.40-1.36 (m, 36H), 1.35-1.32 (m, 4H), 1.30-1.11 (m, 12H); ESI [M+Na$^+$] 1991, [M/2+Na$^+$] 1007

29.4 Preparation of **78-55**

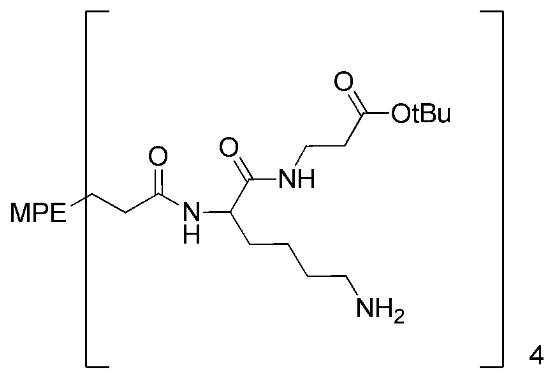

**80-201** (0.5 g, 0.254 mmol) and 10% Pd/C catalyst (0.1 g) were placed in a hydrogenation reactor, and DMF (30 mL) was added for dissolution. The hydrogenation reactor was sealed, evacuated with a water pump, and then filled with

hydrogen gas. This process was repeated three times. The pressure reading of the hydrogenation reactor was adjusted to 1.5 MPa, and the reaction mixture was stirred at room temperature overnight. After the reaction was completed, the reaction mixture was filtered through Celite, and the filter cake was washed with DMF (20 mL × 3). The DMF solution of the product was obtained and used as the raw material for the next reaction.

29.5 Preparation of **78-62**

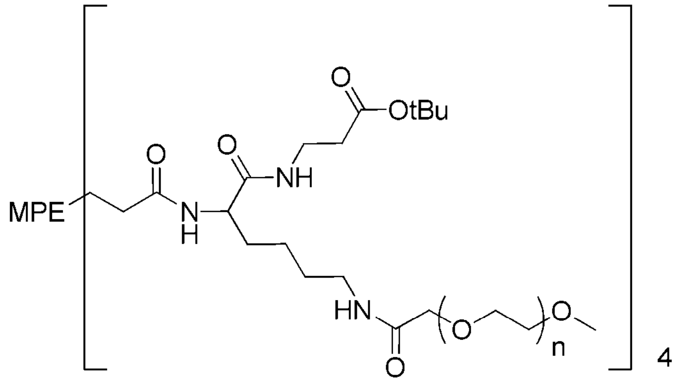

**78-55** (0.1 g, 0.0698 mmol) was placed in a 250 mL flask, then DMF (20 mL) was added for dissolution. Then M-SCM-10K (3.3 g, 0.307 mmol, purchased from Jenkem) was added, and the mixture was reacted with stirring at low speed at room temperature in the dark for one week. After the reaction was completed, n-hexane (25 mL) and methyl tert-butyl ether (200 mL) were added for precipitation. The supernatant was discarded, and n-hexane (50 mL) and methyl tert-butyl ether (100 mL) were added for precipitation. This process was repeated three times. The mixture was filtered, and the obtained solid product was then dried in a vacuum oven to provide 3.2 g of the product with a yield exceeding 100%.

29.6 Preparation of **78-73**

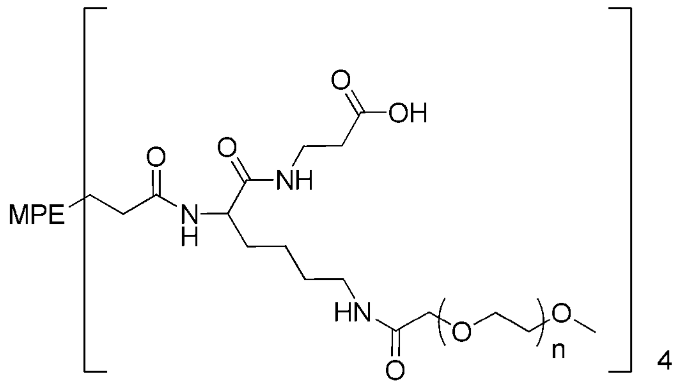

To a flask containing **78-62** (1.5 g, 0.0344 mmol), dichloromethane (20 mL) was added for dissolution with the assistance of ultrasonication, then TFA (22 mL) was added, and the mixture was reacted with stirring at room temperature overnight. After the reaction was completed, the reaction mixture was evaporated to an oily state using a rotary evaporator. Methyl tert-butyl ether (200 mL) was then added for precipitation. A powdery solid precipitated from the reaction mixture and was filtered. The filter cake was washed with methyl tert-butyl ether (100 mL × 3). The filter cake was collected and dried in a vacuum oven to provide 1.48 g of the product with a yield exceeding 100%.

29.7 Preparation of **78-75**

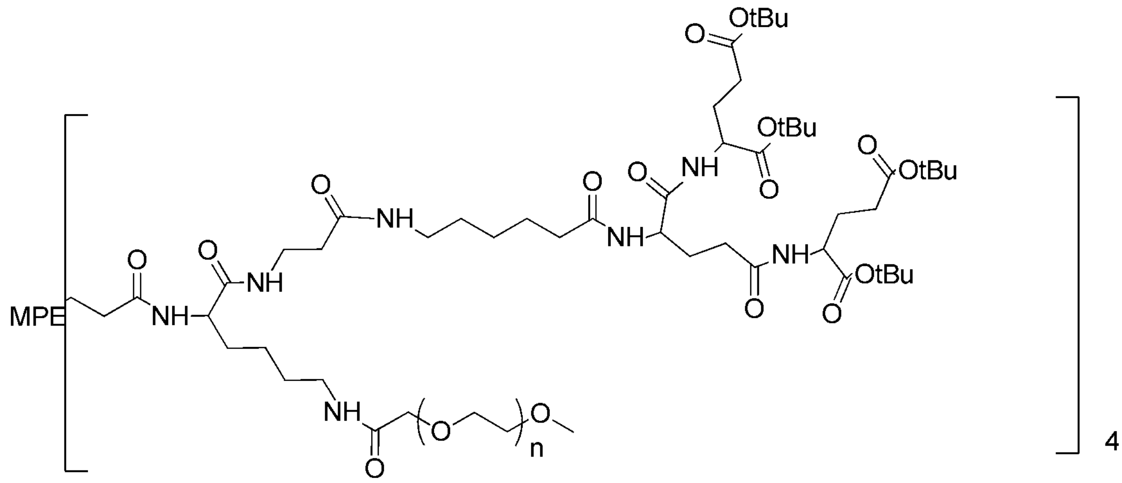

**78-73** (1.489 g, 0.0344 mmol), HBTU (0.078 g, 0.276 mmol), and HOBT (0.028 g, 0.276 mmol) were weighed and added to a flask containing **82-121** (0.154 g, 0.207 mmol), and then DMF (15 mL) was added to dissolve the mixture. DIEA (0.068 mL, 0.414 mmol) was slowly added dropwise with stirring at room temperature, and the mixture was reacted with stirring at room temperature overnight. After the reaction was completed, n-hexane (25 mL) and methyl tert-butyl ether (200 mL) were added for precipitation. The supernatant was discarded, and n-hexane (20 mL) and methyl tert-butyl ether (150 mL) were added again for precipitation. This process was repeated three times. The mixture was filtered to provide a solid product, which was dried to provide 0.9 g of the product with a yield of 56.6%.

29.8 Preparation of **78-87**

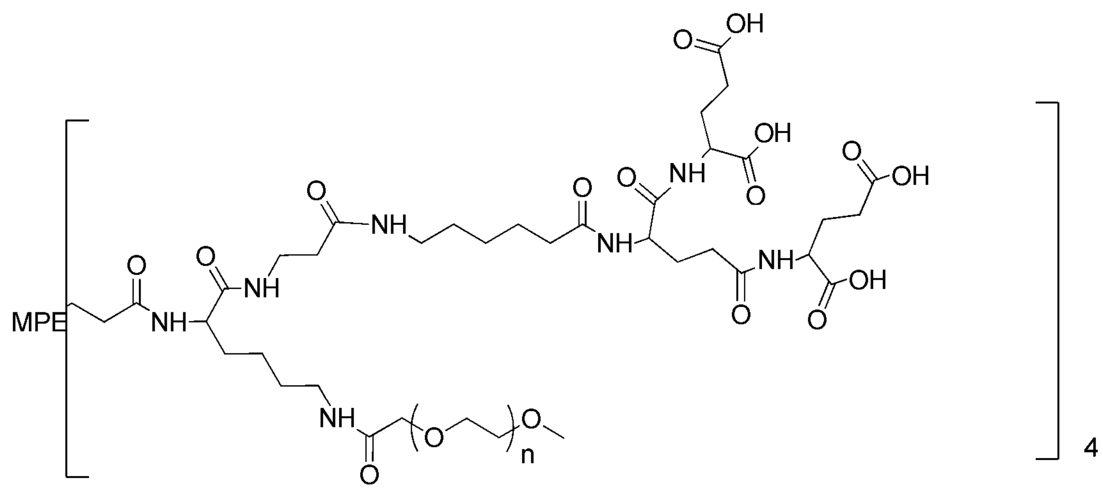

To a flask containing **78-75** (0.9 g, 0.0195 mmol), dichloromethane (15 mL) was added for dissolution with the assistance of ultrasonication, then TFA (5 mL) was added, and the mixture was reacted with stirring at room temperature overnight. After the reaction was completed, the reaction mixture was evaporated to an oily state using a rotary evaporator. Methyl tert-butyl ether (60 mL) was then added for precipitation. A powdery solid precipitated from the reaction mixture and was filtered. The filter cake was washed with methyl tert-butyl ether (40 mL × 3). The filter cake was collected and dried in a vacuum oven to provide 0.77 g of the product with a yield exceeding 100%.

29.9 Preparation of Compound **78-127**

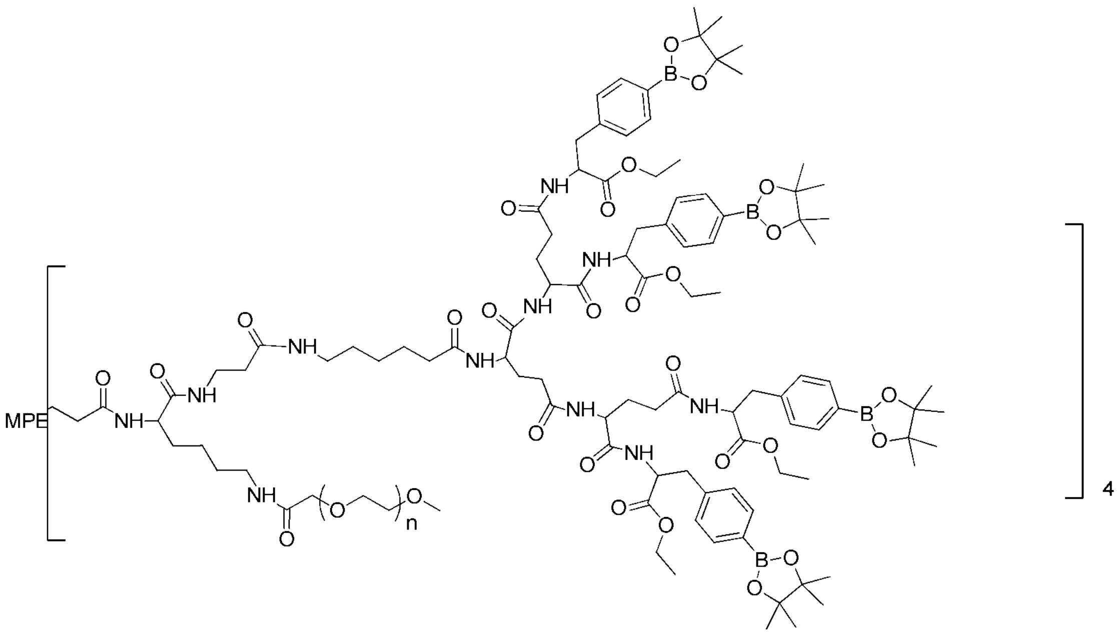

82-98 (0.225 g, 0.706 mmol), EDCI (0.142 g, 0.742 mmol), and HOBT (0.1 g, 0.742 mmol) were weighed and added to a flask containing **78-87** (1.0 g, 0.022 mmol), then DMF (20 mL) was added to dissolve the compound, and the mixture was stirred at room temperature. DIEA (0.26 mL, 1.59 mmol) was slowly added dropwise, and the reaction was stirred at room temperature overnight. After the reaction was completed, n-hexane (25 mL) and methyl tert-butyl ether (200 mL) were added for precipitation. The supernatant was discarded, and n-hexane (20 mL) and methyl tert-butyl ether (150 mL) were added again for precipitation. This process was repeated three times. The mixture was filtered to provide a solid product, which was dissolved in a methanol/dichloromethane (1/4) mixture, then silica gel powder (30 mL) was added. The mixture was mixed evenly and evaporated to dryness. The resulting product was dry-loaded onto the column and subjected to column chromatography using a 6-10% methanol/dichloromethane mixture as the eluent. The desired product was collected, concentrated, and dried to provide 0.21 g of the product with a yield of 19.1%. $^1$H-NMR (600 MHz, DMSO-$d_6$) δ 8.45-7.97 (m, 44H), 7.72-7.60 (m, 16H), 7.59-7.57 (m, 16H), 7.26-7.22 (m, 16H), 7.22-7.19 (m, 16H), 4.43-4.40 (m, 12H), 3.86-3.82 (m, 16H), 3.52-3.49 (m, 3785H), 3.27-3.22 (m, 32H), 3.11-3.03 (m, 42H), 3.02-2.95 (m, 68H), 2.76-2.73 (m, 64H), 1.76-1.69 (m, 28H), 1.30-1.25 (m, 192H), 1.04-0.96 (m, 48H)

Example 30: Synthesis of Compound **78-89**

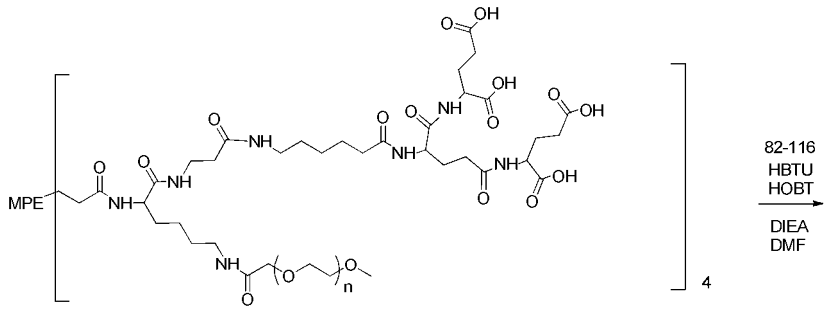


78-87

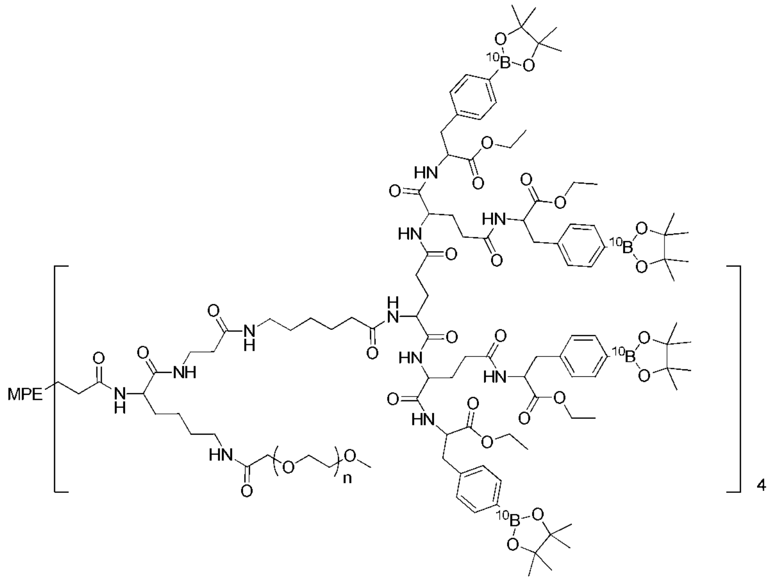

78-89

30.1 Preparation of **78-89**

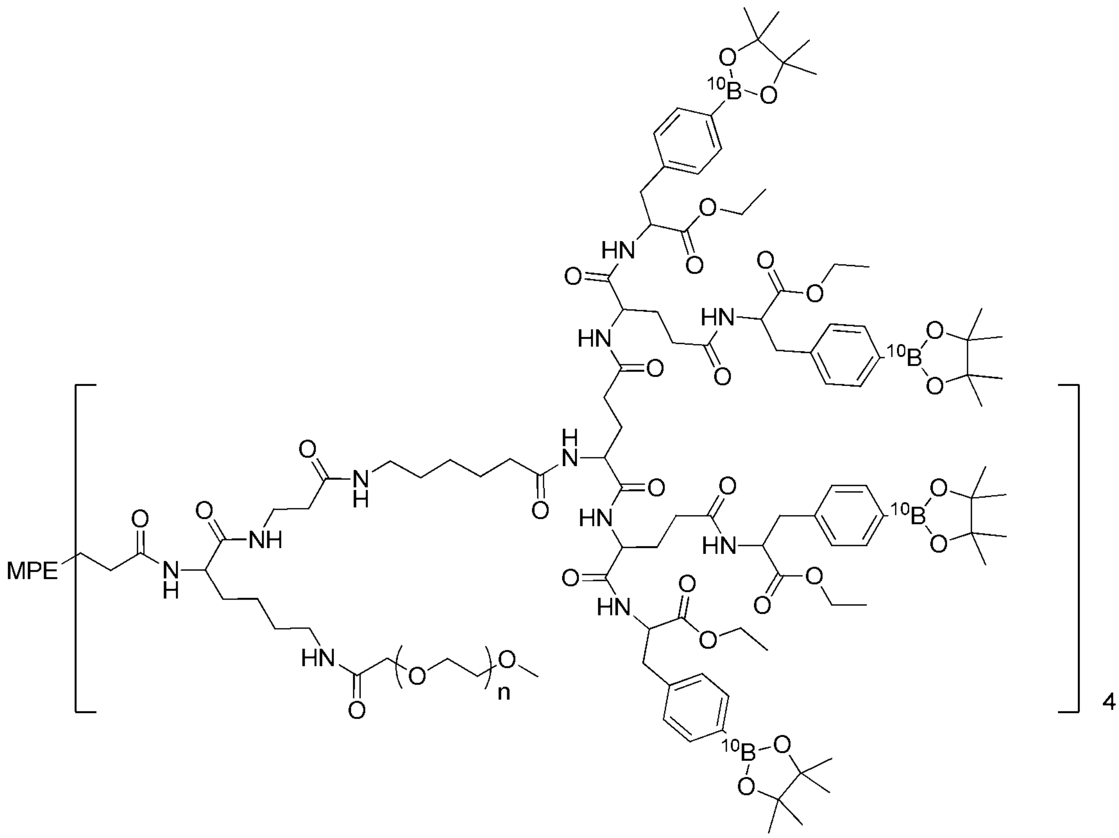

**82-116** (0.45 g, 1.412 mmol), EDCI (0.28 g, 1.478 mmol), and HOBT (0.19 g, 1.478 mmol) were weighed and added to a flask containing **78-87** (2.0 g, 0.044 mmol), then DMF (20 mL) was added to dissolve the mixture, and the mixture was stirred at room temperature. DIEA (0.47 mL, 2.816 mmol) was slowly added dropwise, and then reacted with stirring at room temperature overnight. After the reaction was completed, n-hexane (25 mL) and methyl tert-butyl ether (200 mL) were added for precipitation. The supernatant was discarded, and n-hexane (20 mL) and methyl tert-butyl ether (150 mL) were added again for precipitation. This process was repeated three times. The mixture was filtered to provide a solid product, which was collected, and dried to provide 0.85 g of the product with a yield of 38.6%. $^1$H-NMR (600 MHz, DMSO-$d_6$) $\delta$ 8.49-8.11 (m, 44H), 7.97-7.93 (m, 16H), 7.89-7.73 (m, 16H), 7.70-7.56 (m, 16H), 7.32-7.08 (m, 16H), 4.35-4.30 (m, 32H), 3.87-3.81 (m, 32H), 3.24-3.21 (m, 20H), 3.08-3.03 (m, 24H), 3.02-2.96 (m, 32H), 1.86-1.80 (m, 20H), 1.49-1.44 (m, 28H), 1.34-1.32 (m, 16H), 1.28-1.25 (m, 48H), 1.25-1.21 (m, 192H), 1.06-1.01 (m, 48H)

Example 31: Synthesis of Compound **78-152**

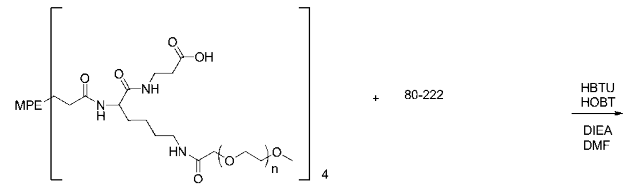


78-87

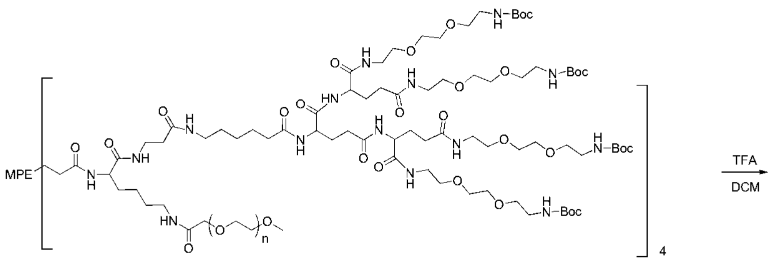


78-148

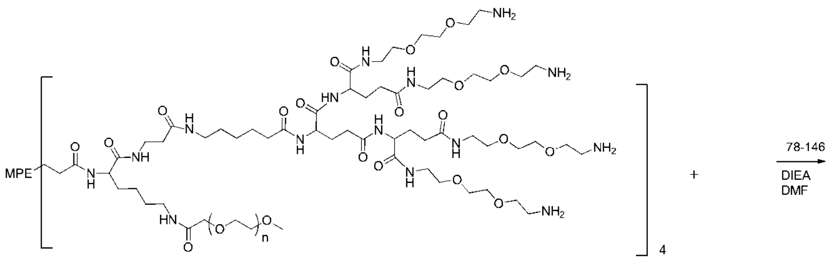


78-149

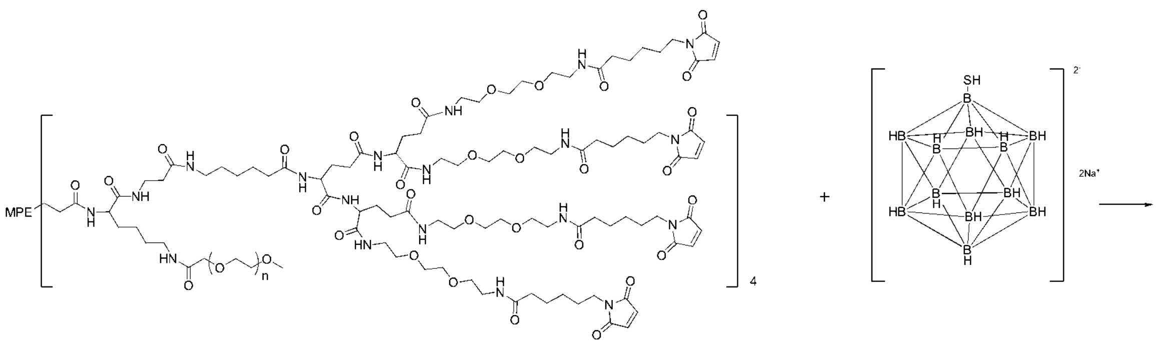


78-150

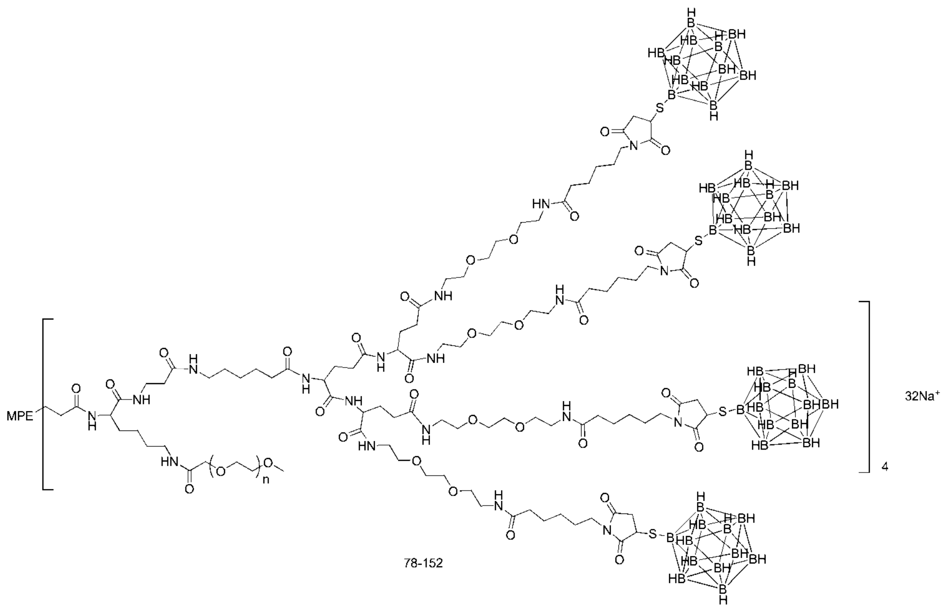


78-152

31.1 Preparation of **78-148**

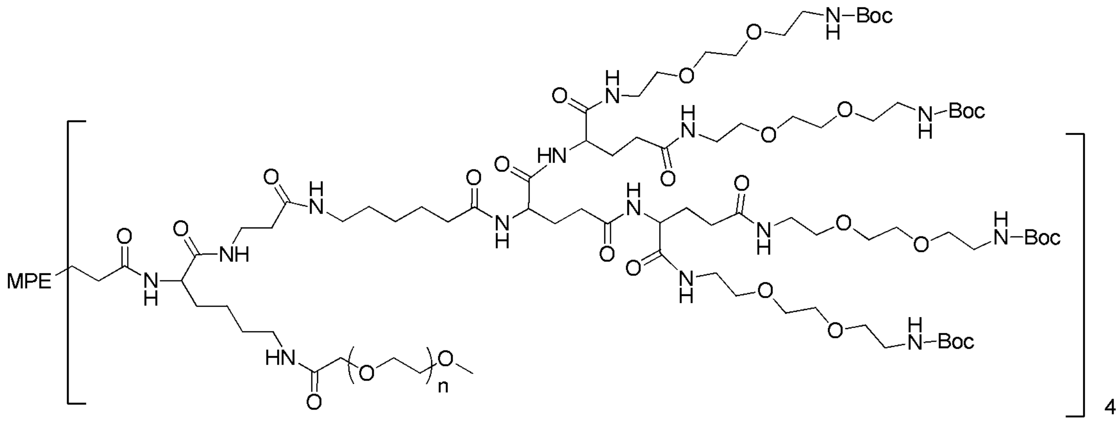

**78-87** (3.04 g, 0.07 mmol), HBTU (0.16 g, 0.42 mmol), and HOBT (0.06 g, 0.42 mmol) were weighed and added to a flask containing **80-222** (0.6 g, 0.42 mmol), and then DMF (40 mL) was added to dissolve the mixture. DIEA (0.30 mL, 1.8040 mmol) was slowly added dropwise at -5°C. After the dropwise addition was completed, the reaction was carried out for half an hour, the flask was then taken out, and the reaction was carried out with stirring at room temperature overnight. After the reaction was completed, n-hexane (25 mL) and methyl tert-butyl ether (200 mL) were added for precipitation. The supernatant was discarded, and n-hexane and methyl tert-butyl ether were added again for precipitation. This process was repeated three times. The mixture was filtered to provide a solid product, which was collected, and dried to provide 1.0 g of the product with a yield of 29.4%.

31.2 Preparation of **78-149**

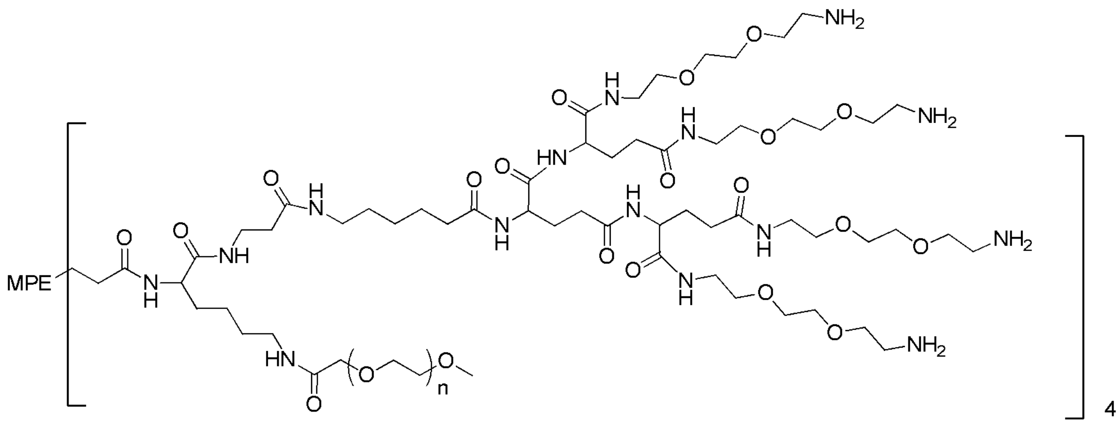

To a flask containing **78-148** (1.0 g, 0.02 mmol), dichloromethane (15 mL) was added for dissolution with the assistance of ultrasonication, then TFA (10 mL) was added, and the mixture was reacted with stirring at room temperature overnight. After the reaction was completed, the reaction mixture was evaporated to an oily state using a rotary evaporator. Methyl tert-butyl ether (60 mL) was then added for precipitation. A powdery solid precipitated from the reaction mixture and was filtered. The filter cake was washed with methyl ether (40 mL × 3). The filter cake was collected, and dried to provide 1.0 g of the product.

31.3 Preparation of **78-150**

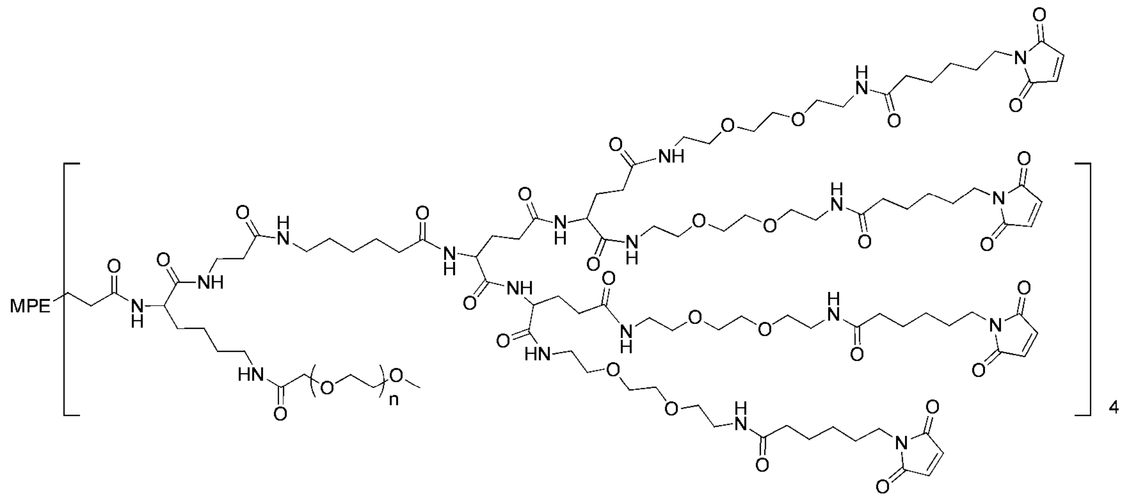

**78-149** (1.0 g, 0.0211 mmol) was placed in a 250 mL flask, and DMF (20 mL) was added for dissolution. DIEA (0.25 mL, 1.52 mmol) was then slowly added dropwise, and reacted with stirring for 20 minutes. **78-146** (0.208 g, 0.6752 mmol) was then added, and the mixture was continuously stirred and allowed to react overnight. After the reaction was completed, n-hexane (25 mL) and methyl tert-butyl ether (200 mL) were added for precipitation. The supernatant was discarded, and n-hexane and methyl tert-butyl ether were added again for precipitation. This process was repeated three times. The mixture was filtered to provide a solid product, which was dissolved in a 1/4 mixture of methanol and dichloromethane, then silica gel powder (10 mL) was added, and the mixture was evaporated to dryness. The resulting product was dry-loaded onto the column and subjected to column chromatography using a 5-8% methanol/dichloromethane mixture as the eluent. The desired product was collected, concentrated, and dried to provide 0.9 g of the product with a yield of 83.8%.

31.4 Preparation of **78-152**

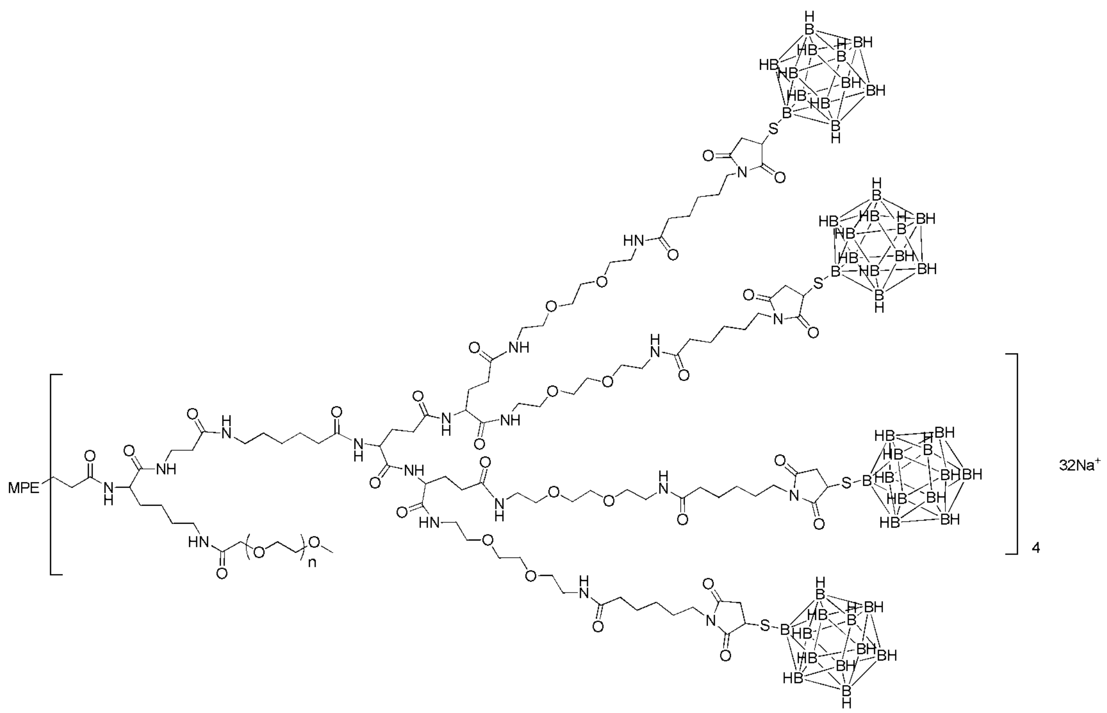

**78-150** (0.45 g, 0.0088 mmol) and sodium mercaptododecaborate (BSH) (0.062 g, 0.2826 mmol, purchased from CATCHEM) were placed in a 250 mL flask, then DMSO (10 mL) was added for dissolution, and the mixture was

reacted with stirring overnight. After the reaction was completed, n-hexane (25 mL) and methyl tert-butyl ether (200 mL) were added for precipitation. The supernatant was discarded, and n-hexane and methyl tert-butyl ether were added again for precipitation. This process was repeated three times. The mixture was filtered to provide a solid product, which was dissolved in a 2% methanol/dichloromethane mixture, and methyl tert-butyl ether (100 mL) was added for precipitation. The mixture was allowed to stand, and the supernatant was discarded. This process was repeated five times. The filter cake was collected and dried in a vacuum oven to provide 0.45 g of the product with a yield of 95.6%. $^{1}$H-NMR (600 MHz, DMSO-*d*$_6$) δ 8.35-7.62 (m, 60H), 4.21-4.16 (m, 16H), 3.89-3.82 (m, 8H), 3.72-3.63 (m, 21H), 3.58-3.41 (m, 3021H), 3.24-3.13 (m, 65H), 3.08-2.96 (m, 72H), 2.80-2.70 (m, 18H), 2.64-2.58 (m, 13H), 2.19-1.97 (m, 60H), 1.89-1.64 (m, 38H), 1.51-1.31 (m, 85H), 1.28-1.23 (m, 36H), 1.10-0.55 (m, 232H)

Example 32: Synthesis of Compound **78-151**

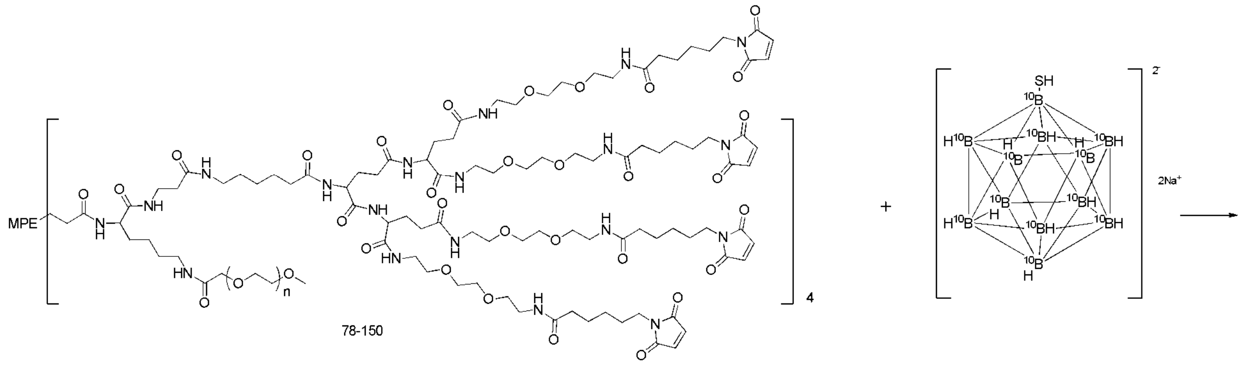

78-150

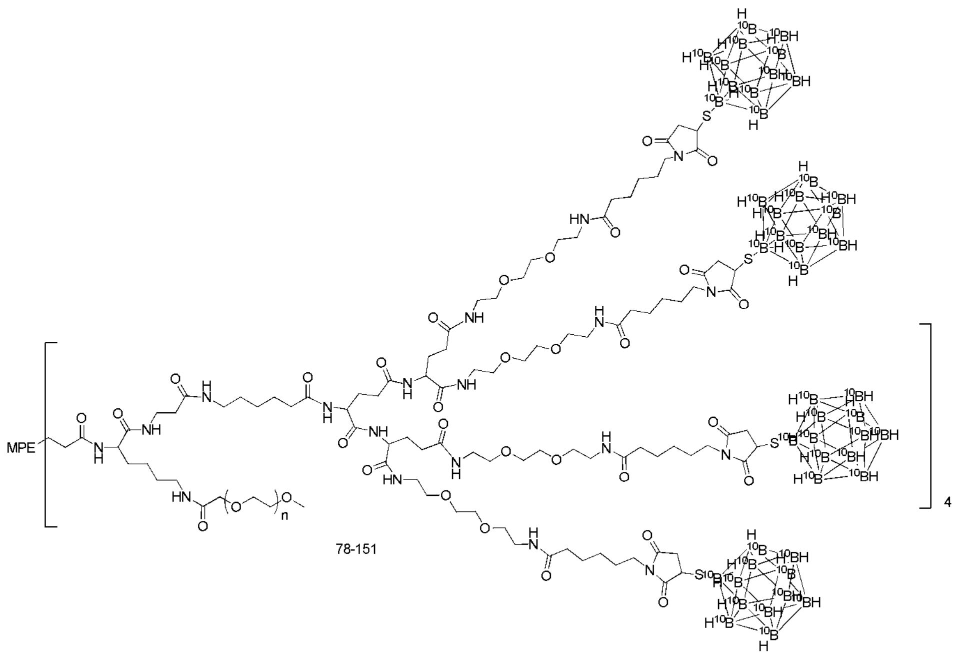

78-151

**78-150** (0.45 g, 0.088 mmol) was placed in a 250 mL flask, then DMSO (10 mL) was added for dissolution, then sodium mercaptododecaborate ($^{10}$B, BSH) (0.059 g, 0.2826 mmol, purchased from CATCHEM, Czech Republic) was added, and the mixture was reacted with stirring at room temperature overnight. After the reaction was completed, n-hexane (25 mL) and methyl tert-butyl ether (200 mL) were added for precipitation. The supernatant was discarded, and n-hexane and methyl tert-butyl ether were added again for precipitation. This process was repeated three times. The mixture was filtered to provide a solid product, which was dissolved in a 2% methanol/dichloromethane mixture, and methyl tert-butyl sulfoxide (100 mL) was added for precipitation. The mixture was allowed to stand, and then the supernatant was discarded. This process was repeated five times. The filter cake was collected and dried in a vacuum oven to provide 0.43 g of the product with a yield of 89.5%. $^{1}$H-NMR (600 MHz, DMSO-*d*$_6$) δ 8.30-7.61 (m, 60H), 4.25-4.19 (m, 16H), 3.90-3.81 (m, 8H), 3.65-3.60 (m, 23H), 3.57-3.41 (m, 3222H), 3.21-3.11 (m, 69H), 3.10-2.96 (m, 72H), 2.80-2.70 (m, 15H), 2.65-2.57 (m,

12H), 2.20-2.00 (m, 66H), 1.89-1.64 (m, 36H), 1.52-1.35 (m, 91H), 1.28-1.22 (m, 38H), 1.06-0.58 (m, 241H)

Example 33: Synthesis of Compound **72-183**

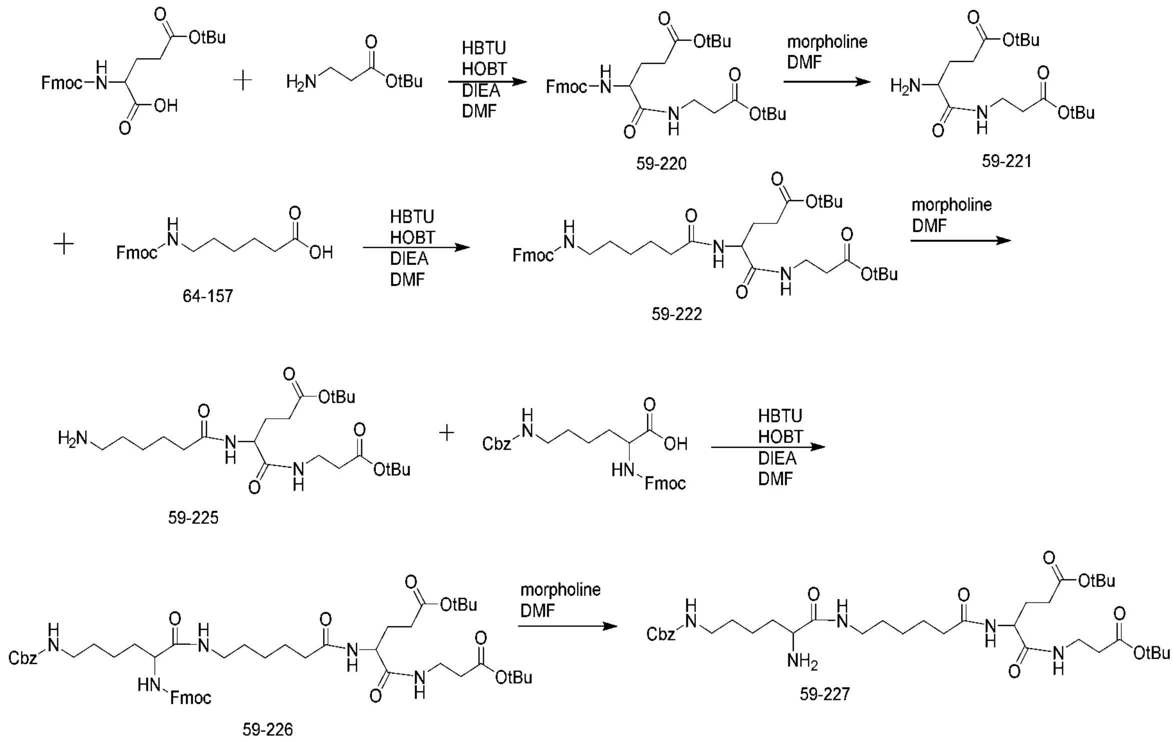


59-220 59-221 64-157 59-222 59-225 59-226 59-227

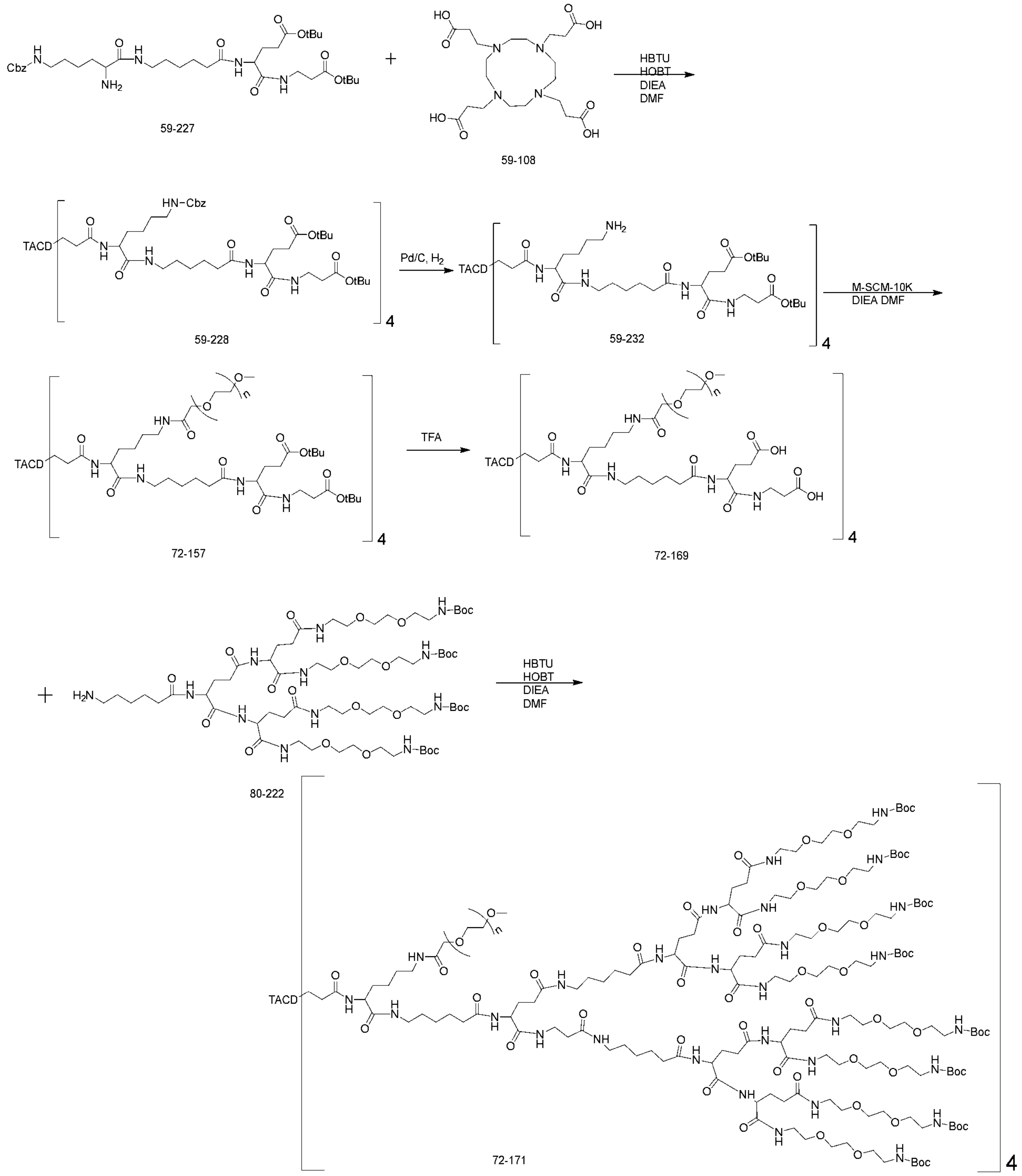


59-227

59-108

59-228

59-232

72-157

72-169

80-222

72-171

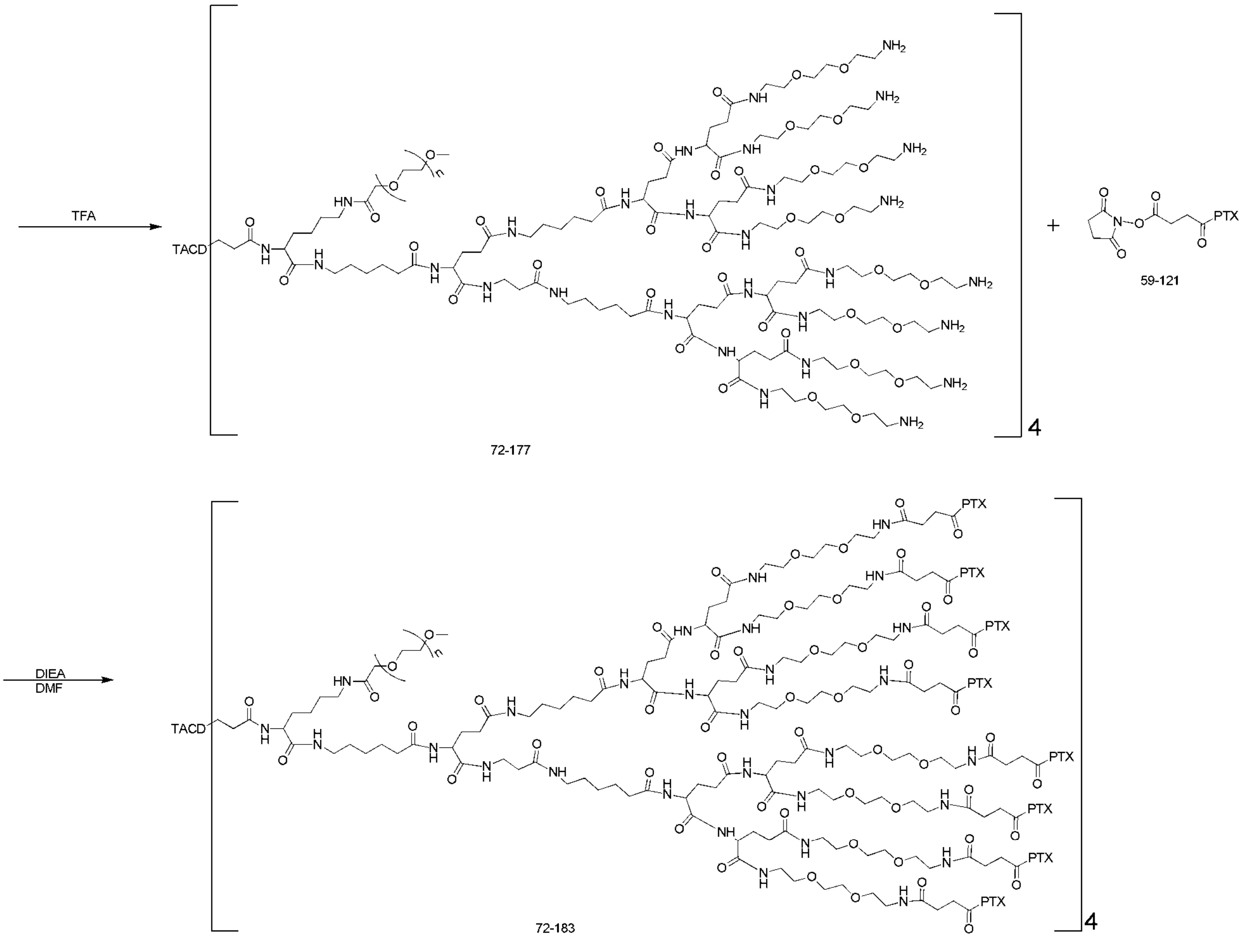


72-177

59-121

72-183

33.1 Synthesis of **59-220**

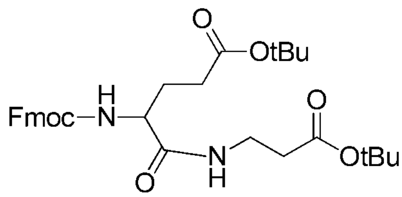

Fmoc-5-tert-butyl L-glutamate (15 g, 35.255 mmol), tert-butyl β-aminopropionate (6.72 g, 37.0178 mmol), HBTU (20.05 g, 52.8825 mmol), and HOBT (7.14 g, 52.8825 mmol) were placed in a 500 mL round-bottom flask, then DMF (65 mL) was added for dissolution with the assistance of ultrasonication, and the mixture was stirred at room temperature. DIEA (26 mL, 158.6475 mmol) was then added dropwise, and the reaction was stirred at room temperature for 2 hours. After the reaction was completed, the reaction mixture was transferred to a 1 L separatory funnel containing saturated sodium bicarbonate solution (200 mL) and ethyl acetate (200 mL) for extraction. The organic phase was collected, and the aqueous phase was further extracted with ethyl acetate (200 mL × 2). The organic phases were combined, washed with saturated brine (200 mL × 2), concentrated, evaporated to dryness, and dried in a vacuum oven to provide 19.5 g of the product.

33.2 Synthesis of **59-221**

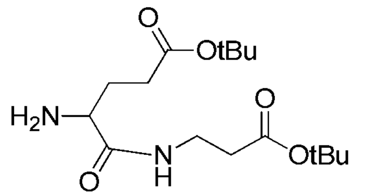

**59-220** (19.5 g, 35.255 mmol) was placed in a 500 mL round-bottom flask. DMF (40 mL) was added for dissolution, then morpholine (30 mL, 352.55 mmol) was added, and the mixture was reacted with stirring at room temperature for 3 hours. After the reaction was completed, the reaction mixture was poured into a 2 L separatory funnel containing saturated sodium bicarbonate solution (300 mL) and ethyl acetate (300 mL) for extraction. The organic phase was collected, and the aqueous phase was further extracted with ethyl acetate (100 mL × 2). The organic phases were combined, washed with saturated brine (200 mL × 2). The resulting organic phase was collected, concentrated, and dried in a vacuum oven to provide 10.6 g of the product.

33.3 Synthesis of **59-222**

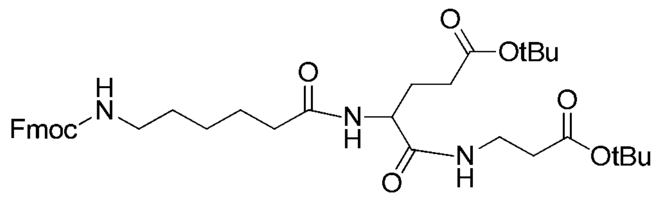

**64-157** (11.32 g, 32.05 mmol), **59-221** (10.6 g, 35.255 mmol), HBTU (18.2 g, 48.075 mmol), and HOBT (6.4 g, 48.075 mmol) were placed in a 500 mL round-bottom flask, then DMF (50 mL) was added for dissolution, and the mixture was stirred at 0°C. DIEA (23.8 mL, 144.225 mmol) was added dropwise. After the dropwise addition was completed, the reaction was carried out with stirring at room temperature overnight. After the reaction was completed, the reaction mixture was transferred to a 1 L separatory funnel containing ethyl acetate (200 mL) and purified water (200 mL) for extraction. The organic phase was collected, and the aqueous phase was further extracted with ethyl acetate (100 mL × 2). The organic phases were combined, evaporated to dryness, dissolved in ethyl acetate (30 mL), and petroleum ether (200 mL) was added for precipitation. The mixture was filtered, and the filter cake was collected and dried to provide 21.34 g of the product.

33.4 Synthesis of **59-225**

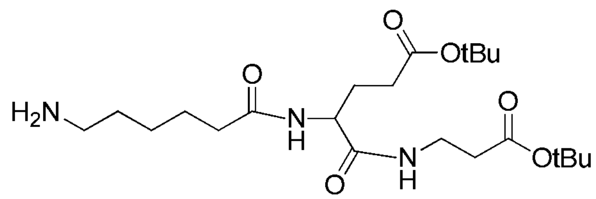

**59-222** (21.34 g, 32.05 mmol) was placed in a 500 mL round-bottom flask, then DMF (40 mL) was added for dissolution, then morpholine (30 mL, 352.55 mmol) was added, and the mixture was reacted with stirring at room temperature. After the reaction was completed, the mixture was filtered, the filtrate was collected and poured into a 2 L separatory funnel containing saturated sodium bicarbonate solution (300 mL) and ethyl acetate (300 mL) for extraction. The organic phase was collected, and the aqueous phase was further extracted with ethyl acetate (100 mL × 2). The organic phases were combined, washed with saturated brine (200 mL × 2), collected, concentrated, and dried to provide 13.7 g of the product with a yield of 96.48%.

33.5 Synthesis of **59-226**

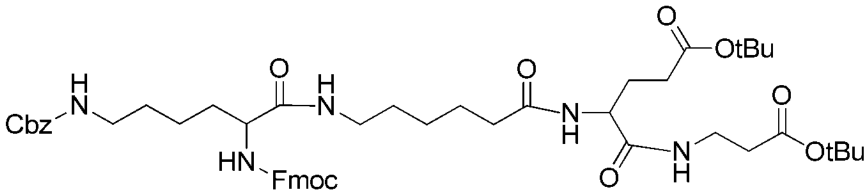

N'-Fmoc-N-benzyloxycarbonyl-L-lysine (14.1 g, 28.07 mmol), **59-225** (13.7 g, 30.88 mmol), HBTU (15.97 g, 42.105 mmol), and HOBT (5.68 g, 42.105 mmol) were placed in a 500 mL round-bottom flask, then DMF (300 mL) was added to dissolve the mixture, and the mixture was stirred at 0°C for several minutes. DIEA (20 mL, 126.315 mmol) was then added dropwise. The mixture was stirred and allowed to react at room temperature overnight. After the reaction was completed, the reaction mixture was transferred to a 1 L separatory funnel containing ethyl acetate (200 mL) and purified water (200 mL) for extraction. The organic phase was collected, and the aqueous phase was further extracted with ethyl acetate (100 mL × 2). The organic phases were combined, evaporated to dryness, dissolved in ethyl acetate (60 mL), and petroleum ether (500 mL) was added for precipitation. The mixture was filtered, and the filter cake was collected and dried to provide 25.8 g of the product with a yield of 98.85%.

33.6 Synthesis of **59-227**

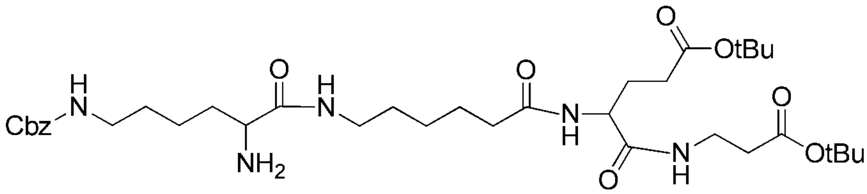

**59-226** (25.8 g, 27.8 mmol) was placed in a 500 mL round-bottom flask. DMF (200 mL) was added for dissolution, then morpholine (24 mL, 278 mmol) was added, and the mixture was reacted with stirring at room temperature. After the reaction was completed, the mixture was filtered, the filtrate was collected and poured into a 2 L separatory funnel containing saturated sodium bicarbonate solution (300 mL) and ethyl acetate (300 mL) for extraction. The organic phase was collected, and the aqueous phase was further extracted with ethyl acetate (100 mL × 2). The organic phases were combined, and washed with saturated brine (200 mL × 2). The resulting organic phase was collected, concentrated, dissolved in a dichloromethane/methanol (4/1) mixture, then silica gel powder was added, and the mixture was evaporated to dryness. The resulting product was dry-loaded onto the column and subjected to column chromatography using a 0.5% ammonia solution/3-5% methanol/dichloromethane mixture as the eluent. The desired product was collected, concentrated, and dried to provide 9.209 g of the product with a yield of 46.93%. $^1$H-NMR (600 MHz, DMSO-$d_6$) δ 7.93-7.88 (m, 2H), 7.79 (s, 1H), 7.37-7.30 (m, 5H), 7.22 (s, 1H), 5.12 (s, 2H), 5.00 (s, 2H), 4.19 (d, $J$ = 5.7 Hz, 1H), 3.29 (dd, $J$ = 13.2, 6.4 Hz, 2H), 3.17 (td, $J$ = 12.2, 6.1 Hz, 1H), 3.06-3.01 (m, 2H), 2.97 (d, $J$ = 6.1 Hz, 2H), 2.35 (td, $J$ = 6.7, 2.2 Hz, 2H), 2.24-2.04 (m, 6H), 1.85-1.79 (m, 1H), 1.66 (ddd, $J$ = 14.6, 12.1, 7.6 Hz, 1H), 1.56-1.43 (m, 4H), 1.39 (s, 9H), 1.38 (s, 9H), 1.34-1.20 (m, 6H); ESI [M+H$^+$] 706.436

33.13 Synthesis of **59-228**

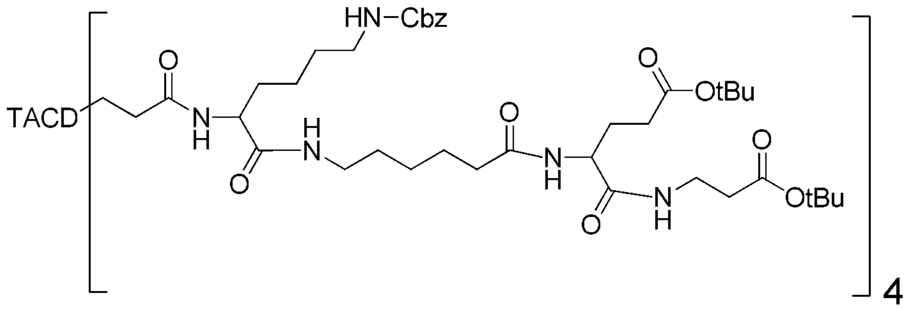

**59-108** (0.326 g, 0.708 mmol), **59-227** (3 g, 4.25 mmol), HBTU (1.61 g, 4.25 mmol), and HOBT (0.57 g, 4.25 mmol) were placed in a 100 mL round-bottom flask, then DMF (30 mL) was added for dissolution with the assistance of ultrasonication, and the mixture was stirred at room temperature. DIEA (2.1 mL, 12.75 mmol) was then added dropwise, and the reaction was stirred at room temperature overnight. After the reaction was completed, the mixture was poured into a 500 mL separatory funnel containing pure water (60 mL) and ethyl acetate (60 mL) for extraction. The organic phase was collected, and the aqueous phase was further extracted with ethyl acetate (50 mL × 2). The organic phases were combined, and washed with saturated brine (60 mL × 2). The resulting organic phase was collected, concentrated, then silica gel powder was added, and the mixture was evaporated to dryness. The resulting product was dry-loaded onto the column and subjected to column chromatography using a mixture of 1% ammonia water/5-6% methanol/dichloromethane as the eluent. The desired product was collected, concentrated, and dried to provide 1.25 g of the product with a yield of 55.06%. $^1$H-NMR (600 MHz, DMSO-$d_6$) δ 8.09 (d, *J* = 7.1 Hz, 4H), 7.93 (dd, *J* = 14.0, 8.6 Hz, 8H), 7.88 (d, *J* = 8.0 Hz, 4H), 7.33 (dd, *J* = 14.8, 7.2 Hz, 20H), 7.20 (s, 4H), 4.99 (s, 8H), 4.19 (d, *J* = 6.2 Hz, 4H), 3.30-3.26 (m, 8H), 3.18-3.15 (m, 4H), 2.99-2.90 (m, 24H), 2.69 (s, 16H), 2.40 (s, 8H), 2.34 (t, *J* = 6.6 Hz, 8H), 2.20-2.05 (m, 24H), 1.81 (s, 4H), 1.69-1.65 (m, 4H), 1.49-1.44 (m, 16H), 1.38 (s, 36H), 1.37 (s, 36H), 1.21 (d, *J* = 6.9 Hz, 24H)

33.14 Synthesis of **59-232**

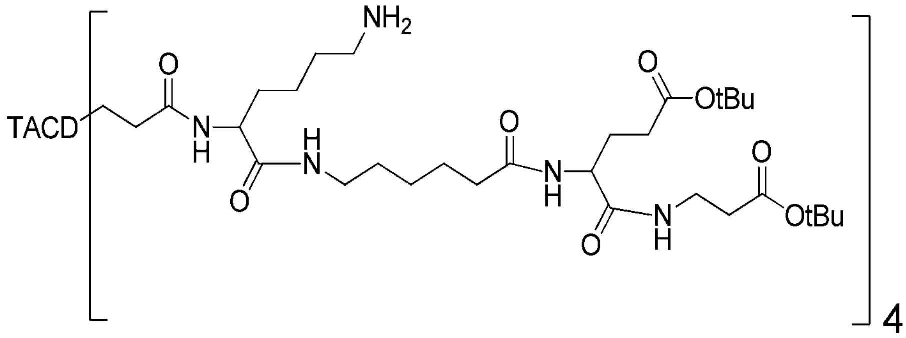

**59-228** (1.25 g, 0.389 mmol) was dissolved in DMF (30 mL) in a hydrogenation reactor, and then 10% Pd/C catalyst (600 mg) was added. The reactor was evacuated with a water pump, and then filled with hydrogen gas. This process was repeated three times. The hydrogen pressure was adjusted to 1.8 MPa, and the reaction was carried out with stirring at 50°C. After the reaction was completed, the reaction mixture was transferred to a suction funnel containing filter paper using a rubber-tipped pipette for filtration. The filtrate was collected, and then n-hexane and methyl tert-butyl ether were added for precipitation. The supernatant was discarded, and n-hexane and methyl tert-butyl ether were added again for precipitation. This process was repeated several times until the reaction mixture became an obvious solid. Dichloromethane (10 mL) and toluene (0.5 mL) were added to the solid, and the mixture was subjected to azeotropic evaporation to dryness. This process was repeated five times. Finally, the solid was dried to provide 0.91 g of the product with a yield of 87.5%.

33.15 Synthesis of **72-157**

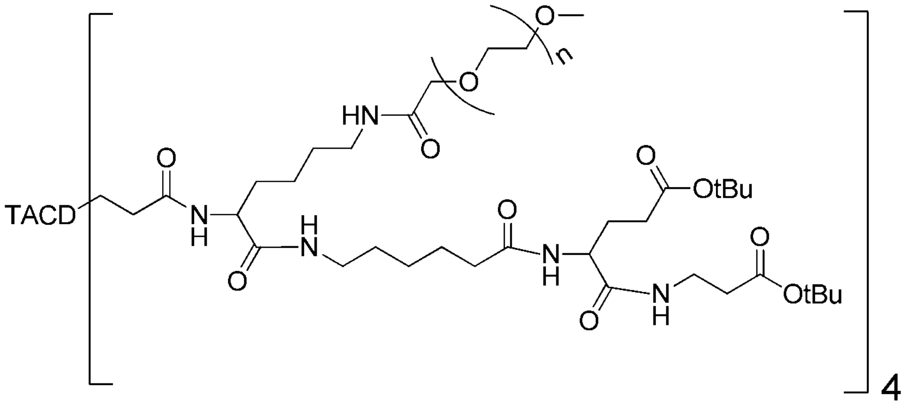

**59-232** (0.314 g, 0.117 mmol) was placed in a 100 mL flask, then anhydrous DMF (40 mL) was added for dissolution. DIEA (0.77 mL, 4.69 mmol) was added, and finally M-SCM-10K (5 g, 0.469 mmol) was added. Dissolution was performed with the assistance of ultrasonication. The reaction was carried out with stirring at low speed in the dark at room temperature. After the reaction was completed, n-hexane (25 mL) and methyl tert-butyl ether (200 mL) were added for precipitation. The supernatant was discarded, and n-hexane (50 mL) and methyl tert-butyl ether (100 mL) were added again for precipitation. This process was repeated three times. The mixture was filtered to provide a solid product, which was dissolved in a dichloromethane/methanol (4/1) mixture, then silica gel powder was added, and the mixture was evaporated to dryness. The resulting product was dry-loaded onto the column and subjected to column chromatography using 1% ammonia water/5-6% methanol/dichloromethane as the eluent. The desired product was collected, concentrated, and dried to provide 4.2 g of the product with a yield of 84%. $^1$H-NMR (600 MHz, DMSO-$d_6$) δ 8.01-7.91 (m, 16H), 7.66-7.53 (m, 4H), 4.20-4.19 (m, 4H), 3.52-3.49 (m, 3748H), 3.17-3.08 (m, 8H), 3.09-2.90 (m, 28H), 2.82-2.66 (m, 16H), 2.40-2.33 (m, 16H), 2.21-2.06 (m, 24H), 1.83-1.80 (m, 4H), 1.69-1.66 (m, 4H), 1.50-1.44 (m, 16H), 1.39-1.36 (m, 72H), 1.29-1.16 (m, 24H)

33.16 Synthesis of **72-169**

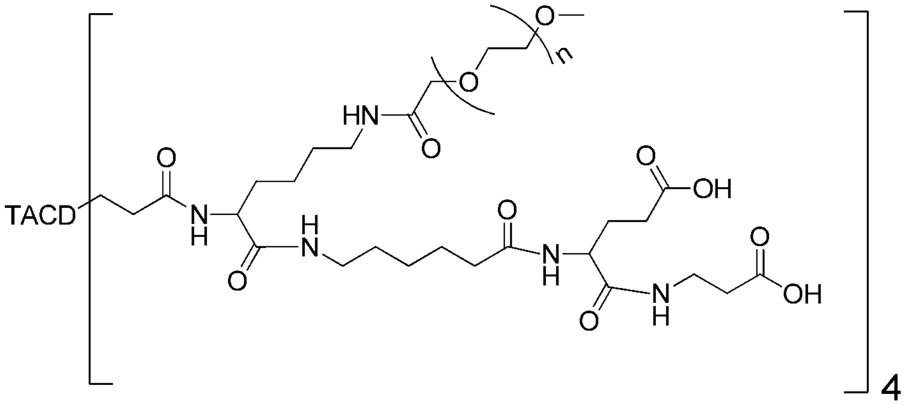

**72-157** (2 g, 0.0455 mmol) was placed in a 250 mL round-bottom flask, then TFA (10 mL) was added, and the mixture was reacted with stirring at room temperature overnight. After the reaction was completed, the reaction mixture was evaporated to dryness. Dichloromethane (20 mL) was then added for dissolution, followed by evaporation to dryness. This process was repeated three times. Methyl tert-butyl ether (100 mL) was added for precipitation. The mixture was treated by ultrasonication for 10 minutes, and allowed to stand. The supernatant was then discarded. This process was repeated three times. A solid precipitated and was filtered. The filter cake was collected, and dried to provide 1.5 g of the product with a yield of 76.53%.

33.17 Synthesis of **72-171**

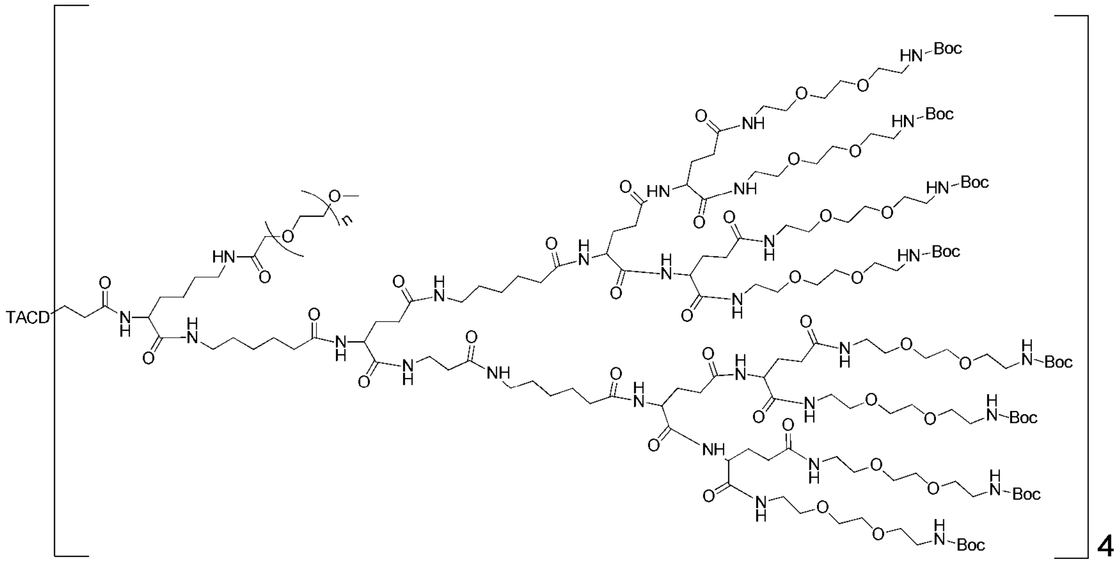

**72-169** (1.2 g, 0.0278 mmol) was placed in a 100 mL round-bottom flask, then DMF (20 mL) was added for dissolution with the assistance of ultrasonication. DIEA (2 mL) was then added to adjust the pH to approximately 8. **80-222** (0.8 g, 0.556 mmol), HBTU (0.127 g, 0.334 mmol), and HOBT (0.045 g, 0.334 mmol) were then added. Dissolution was performed with the assistance of ultrasonication. The reaction was carried out with stirring at room temperature. After the reaction was completed, n-hexane (25 mL) and methyl tert-butyl ether (200 mL) were added for precipitation. The supernatant was discarded, and n-hexane (50 mL) and methyl tert-butyl ether (100 mL) were added again for precipitation. This process was repeated three times. The mixture was filtered to provide a solid product, which was dissolved in a dichloromethane/methanol (4/1) mixture, then silica gel powder was added, and the mixture was evaporated to dryness. The resulting product was dry-loaded onto the column and subjected to column chromatography using 1% ammonia water/5-10% methanol/dichloromethane as the eluent. The desired product was collected, concentrated, and dried to provide 0.62 g of the product with a yield of 31.3%. $^1$H-NMR (600 MHz, DMSO-$d_6$) δ 8.316-8.151 (m, 32H), 8.104-8.023 (m, 28H), 8.01-7.94 (m, 16H), 7.87-7.80 (m, 24H), 7.76-7.73 (m, 8H), 7.71-7.66 (m, 8H), 4.21-4.18 (m, 28H), 3.51-4.99 (m, 3748H), 3.39-3.35 (m, 256H), 3.28-3.22 (m, 28H), 3.20-3.15 (m, 64H), 3.07-3.05 (m, 72H), 2.64-2.60 (m, 32H), 2.15-2.08 (m, 88H), 1.88-1.80 (m, 36H), 1.78-1.71 (m, 36H), 1.48-1.45 (m, 32H), 1.38-1.36 (m, 288H), 1.35-1.34 (m, 56H)

33.18 Synthesis of **72-177**

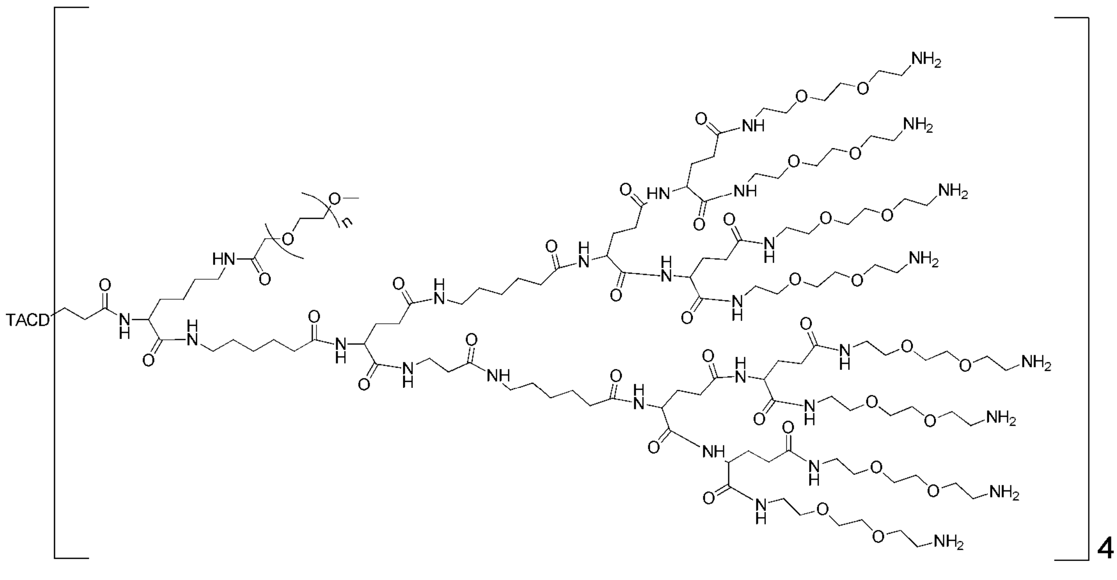

**72-171** (0.62 g, 0.01137 mmol) was weighed and placed in a 100 mL round-bottom flask, then TFA (10 mL) was

added for dissolution, and the mixture was reacted with stirring at room temperature overnight. After the reaction was completed, the reaction mixture was evaporated to dryness. Then, dichloromethane (20 mL) was added for dissolution, and the mixture was evaporated to dryness using a rotary evaporator. This process was repeated three times. Methyl tert-butyl ether (100 mL) was added for precipitation, and treated by ultrasonication for 10 minutes. After standing, the supernatant was discarded. This process was repeated three times. Dichloromethane (10 mL) was added to the mixture for dissolution, then DIEA was added to adjust the pH to neutral, and then methyl tert-butyl ether (100 mL) was added for precipitation. A solid precipitated and was filtered, and the filter cake was collected and dried to provide 0.469 g of the product with a yield of 78.17%.

33.19 Synthesis of **72-183**

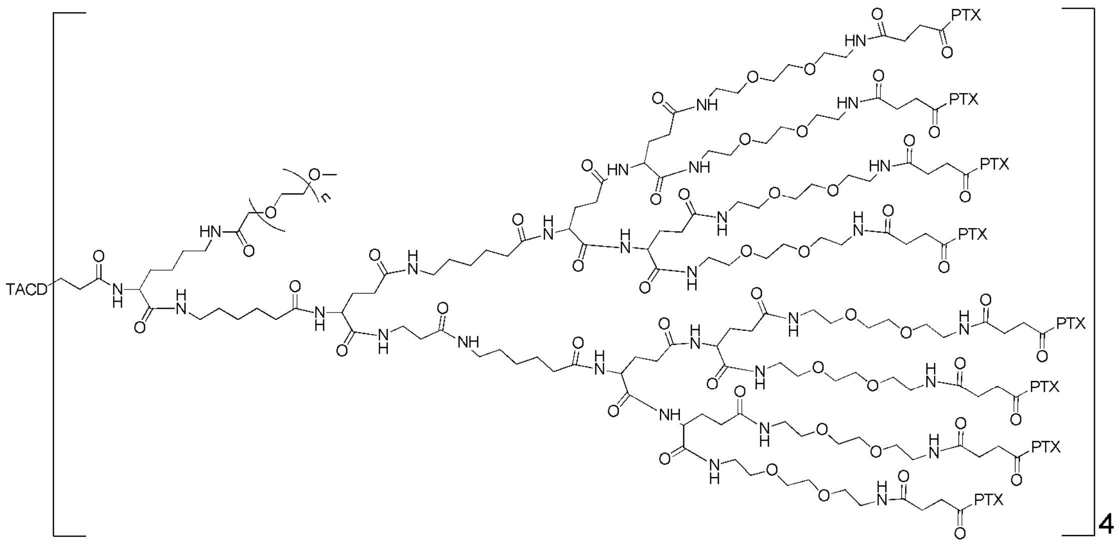

**72-177** (0.469 g, 0.00914 mmol) was dissolved in DMF (30 mL) in a reaction flask, and then DIEA (0.096 mL, 0.5851 mmol) was added dropwise to adjust the pH of the reaction mixture to neutral or slightly alkaline. **59-121** (0.62 g, 0.5851 mmol) was added, and dissolved with the assistance of ultrasonication, and the reaction was stirred at room temperature for 2 days. After the reaction was completed, n-hexane (25 mL) and methyl tert-butyl ether (200 mL) were added for precipitation. The supernatant was discarded, and n-hexane (50 mL) and methyl tert-butyl ether (100 mL) were added again for precipitation. This process was repeated three times. The mixture was filtered to provide a solid product, which was dissolved in a 2% methanol/dichloromethane mixture, then methyl tert-butyl ether (100 mL) was added for precipitation. The flask was allowed to stand, and the supernatant was discarded. This process was repeated five times. The mixture was filtered, and the filter cake was collected, and dried it in a vacuum oven to provide 0.369 g of the product with a yield of 49.8%. $^1$H-NMR (600 MHz, DMSO-$d_6$)$\delta$ 9.26-9.22 (m, 32H), 8.25-8.13 (m, 24H), 8.08-8.03 (m, 16H), 7.99-7.95 (m, 96H), 7.89-7.82 (m, 96H), 7.74-7.71 (m, 36H), 7.69-7.64 (m, 72H), 7.56-7.53 (m, 32H), 7.49-7.43 (m, 192H), 7.20-7.17 (m, 32H), 6.31-6.27 (m, 32H), 5.84-5.80 (m, 32H), 5.56-5.51 (m, 32H), 5.43-5.40 (m, 32H), 4.94-4.89 (m, 72H), 4.65-4.62 (m, 32H), 4.20-4.15 (m, 32H), 4.11-4.10 (m, 32H), 4.02-4.0 (m, 52H), 3.65-3.64 (m, 32H), 3.52-3.48 (m, 3748H), 3.40-3.38 (m, 232H), 3.27-3.25 (m, 28H), 3.17-3.16 (m, 64H), 3.02-3.00 (m, 96H), 2.90-2.89 (m, 16H), 2.74-2.73 (m, 16H), 2.63-2.61 (m, 64H), 2.40-2.37 (m,64H), 2.33-2.31 (m, 16H), 2.25-2.22 (m, 96H), 2.19-2.16 (m, 24H), 2.12-2.07 (m, 144H), 2.00-1.98 (m, 32H), 1.80-1.75 (m, 136H), 1.68-1.60 (m, 96H), 1.53-1.48 (m, 192H), 1.20-1.18 (m, 56H), 1.03-0.99 (m, 192H)

Experimental Example 1: Test of cytotoxicity of compounds

I. Materials:

1. Drugs: **82-178, 82-183, 78-139, 81-176, 85-71, 84-106, 76-207, 75-196, 71-246**

2. Cells: A-549 cells, COLO-205 cells, and BT-474 cells

3. Equipment: PerkinElmer Multi-Purpose Microplate Reader (PerkinElmer).

4. Reagents: CCK8 Cell Proliferation and Cytotoxicity Assay Kit (Dojindo); DMEM medium, fetal bovine serum, and antibiotics (GIBCO).

II. Experimental method for testing growth inhibition of A549, COLO-205, and BT-474 cells at different drug concentrations

1. The cells were cultured and trypsinized. The cells were dissociated into single-cell suspensions using the corresponding culture media, seeded into a 96-well plate at 100 μL per well with a cell density of approximately 10-15%, and cultured overnight in a 37°C, 5% $CO_2$ incubator.
2. According to the drug concentration requirements, drug-containing culture media with different concentrations were prepared by serial dilution method.
✧ The corresponding drugs for A549 cells were as follows:

| **Plate layout** | | | | | | | | | | | | |
|---|---|---|---|---|---|---|---|---|---|---|---|---|
| **82-178 [IRN] (μM)** | CCK8 back-ground | 100.00 | 50.00 | 25.00 | 12.50 | 6.25 | 3.13 | 1.56 | 0.78 | 0.39 | 0 | 2-fold dilution |
| | | 100.00 | 50.00 | 25.00 | 12.50 | 6.25 | 3.13 | 1.56 | 0.78 | 0.39 | 0 | |
| | | 100.00 | 50.00 | 25.00 | 12.50 | 6.25 | 3.13 | 1.56 | 0.78 | 0.39 | 0 | |
| | | 100.00 | 33.33 | 11.11 | 3.70 | 1.23 | 0.41 | 0.14 | 0.05 | 0.02 | 0 | 3-fold dilution |
| | | 100.00 | 33.33 | 11.11 | 3.70 | 1.23 | 0.41 | 0.14 | 0.05 | 0.02 | 0 | |
| | | 100.00 | 33.33 | 11.11 | 3.70 | 1.23 | 0.41 | 0.14 | 0.05 | 0.02 | 0 | |

✧ The corresponding drugs for COLO-205 cells were as follows:

| **Plate layout** | | | | | | | | | | | | |
|---|---|---|---|---|---|---|---|---|---|---|---|---|
| **71-246 [SB7] (μM)** | CCK8 back-ground | 100.00 | 50.00 | 25.00 | 12.50 | 6.25 | 3.13 | 1.56 | 0.78 | 0.39 | 0 | 2-fold dilution |
| | | 100.00 | 50.00 | 25.00 | 12.50 | 6.25 | 3.13 | 1.56 | 0.78 | 0.39 | 0 | |
| | | 100.00 | 50.00 | 25.00 | 12.50 | 6.25 | 3.13 | 1.56 | 0.78 | 0.39 | 0 | |
| | | 100.00 | 33.33 | 11.11 | 3.70 | 1.23 | 0.41 | 0.14 | 0.05 | 0.02 | 0 | 3-fold dilution |
| | | 100.00 | 33.33 | 11.11 | 3.70 | 1.23 | 0.41 | 0.14 | 0.05 | 0.02 | 0 | |
| | | 100.00 | 33.33 | 11.11 | 3.70 | 1.23 | 0.41 | 0.14 | 0.05 | 0.02 | 0 | |
| **Plate layout** | | | | | | | | | | | | |
| **81-176 [DOX] (μM)** | CCK8 back-ground | 100.00 | 50.00 | 25.00 | 12.50 | 6.25 | 3.13 | 1.56 | 0.78 | 0.39 | 0 | 2-fold dilution |
| | | 100.00 | 50.00 | 25.00 | 12.50 | 6.25 | 3.13 | 1.56 | 0.78 | 0.39 | 0 | |
| | | 100.00 | 50.00 | 25.00 | 12.50 | 6.25 | 3.13 | 1.56 | 0.78 | 0.39 | 0 | |
| | | 100.00 | 33.33 | 11.11 | 3.70 | 1.23 | 0.41 | 0.14 | 0.05 | 0.02 | 0 | 3-fold dilution |
| | | 100.00 | 33.33 | 11.11 | 3.70 | 1.23 | 0.41 | 0.14 | 0.05 | 0.02 | 0 | |
| | | 100.00 | 33.33 | 11.11 | 3.70 | 1.23 | 0.41 | 0.14 | 0.05 | 0.02 | 0 | |

✧ The corresponding drugs for BT-474 cells were as follows:

| | | | | | | | | | | | | |
|---|---|---|---|---|---|---|---|---|---|---|---|---|
| **Plate layout** | | | | | | | | | | | | |
| **81-176 [DOX] (μM)** | CCK8 back-ground | 100.00 | 50.00 | 25.00 | 12.50 | 6.25 | 3.13 | 1.56 | 0.78 | 0.39 | 0 | 2-fold dilution |
| | | 100.00 | 50.00 | 25.00 | 12.50 | 6.25 | 3.13 | 1.56 | 0.78 | 0.39 | 0 | |
| | | 100.00 | 50.00 | 25.00 | 12.50 | 6.25 | 3.13 | 1.56 | 0.78 | 0.39 | 0 | |
| | | 100.00 | 33.33 | 11.11 | 3.70 | 1.23 | 0.41 | 0.14 | 0.05 | 0.02 | 0 | 3-fold dilution |
| | | 100.00 | 33.33 | 11.11 | 3.70 | 1.23 | 0.41 | 0.14 | 0.05 | 0.02 | 0 | |
| | | 100.00 | 33.33 | 11.11 | 3.70 | 1.23 | 0.41 | 0.14 | 0.05 | 0.02 | 0 | |
| **Plate layout** | | | | | | | | | | | | |
| **78-139 [IRN] (μM)** | CCK8 back-ground | 100.00 | 50.00 | 25.00 | 12.50 | 6.25 | 3.13 | 1.56 | 0.78 | 0.39 | 0 | 2-fold dilution |
| | | 100.00 | 50.00 | 25.00 | 12.50 | 6.25 | 3.13 | 1.56 | 0.78 | 0.39 | 0 | |
| | | 100.00 | 50.00 | 25.00 | 12.50 | 6.25 | 3.13 | 1.56 | 0.78 | 0.39 | 0 | |
| | | 100.00 | 33.33 | 11.11 | 3.70 | 1.23 | 0.41 | 0.14 | 0.05 | 0.02 | 0 | 3-fold dilution |
| | | 100.00 | 33.33 | 11.11 | 3.70 | 1.23 | 0.41 | 0.14 | 0.05 | 0.02 | 0 | |
| | | 100.00 | 33.33 | 11.11 | 3.70 | 1.23 | 0.41 | 0.14 | 0.05 | 0.02 | 0 | |
| **Plate layout** | | | | | | | | | | | | |
| **85-71 [IRN] (μM)** | CCK8 back-ground | 100.00 | 50.00 | 25.00 | 12.50 | 6.25 | 3.13 | 1.56 | 0.78 | 0.39 | 0 | 2-fold dilution |
| | | 100.00 | 50.00 | 25.00 | 12.50 | 6.25 | 3.13 | 1.56 | 0.78 | 0.39 | 0 | |
| | | 100.00 | 50.00 | 25.00 | 12.50 | 6.25 | 3.13 | 1.56 | 0.78 | 0.39 | 0 | |
| | | 100.00 | 33.33 | 11.11 | 3.70 | 1.23 | 0.41 | 0.14 | 0.05 | 0.02 | 0 | 3-fold dilution |
| | | 100.00 | 33.33 | 11.11 | 3.70 | 1.23 | 0.41 | 0.14 | 0.05 | 0.02 | 0 | |
| | | 100.00 | 33.33 | 11.11 | 3.70 | 1.23 | 0.41 | 0.14 | 0.05 | 0.02 | 0 | |
| **Plate layout** | | | | | | | | | | | | |
| **86-2 [PCB]** | CCK8 background | 100.00 | 50.00 | 25.00 | 12.50 | 6.25 | 3.13 | 1.56 | 0.78 | 0.39 | 0 | 2-fold dilution |
| | | 100.00 | 50.00 | 25.00 | 12.50 | 6.25 | 3.13 | 1.56 | 0.78 | 0.39 | 0 | |
| **(μM)** | | 100.00 | 50.00 | 25.00 | 12.50 | 6.25 | 3.13 | 1.56 | 0.78 | 0.39 | 0 | |
| | | 100.00 | 33.33 | 11.11 | 3.70 | 1.23 | 0.41 | 0.14 | 0.05 | 0.02 | 0 | 3-fold dilution |
| | | 100.00 | 33.33 | 11.11 | 3.70 | 1.23 | 0.41 | 0.14 | 0.05 | 0.02 | 0 | |
| | | 100.00 | 33.33 | 11.11 | 3.70 | 1.23 | 0.41 | 0.14 | 0.05 | 0.02 | 0 | |
| **Plate layout** | | | | | | | | | | | | |
| **84-106 [DOX] (μM)** | CCK8 back-ground | 100.00 | 50.00 | 25.00 | 12.50 | 6.25 | 3.13 | 1.56 | 0.78 | 0.39 | 0 | 2-fold dilution |
| | | 100.00 | 50.00 | 25.00 | 12.50 | 6.25 | 3.13 | 1.56 | 0.78 | 0.39 | 0 | |
| | | 100.00 | 50.00 | 25.00 | 12.50 | 6.25 | 3.13 | 1.56 | 0.78 | 0.39 | 0 | |
| | | 100.00 | 33.33 | 11.11 | 3.70 | 1.23 | 0.41 | 0.14 | 0.05 | 0.02 | 0 | 3-fold dilution |
| | | 100.00 | 33.33 | 11.11 | 3.70 | 1.23 | 0.41 | 0.14 | 0.05 | 0.02 | 0 | |
| | | 100.00 | 33.33 | 11.11 | 3.70 | 1.23 | 0.41 | 0.14 | 0.05 | 0.02 | 0 | |

(continued)

| Plate layout | | | | | | | | | | | | |
|---|---|---|---|---|---|---|---|---|---|---|---|---|
| **76-207 [PTX] (μM)** | CCK8 back-ground | 100.00 | 50.00 | 25.00 | 12.50 | 6.25 | 3.13 | 1.56 | 0.78 | 0.39 | 0 | 2-fold dilution |
| | | 100.00 | 50.00 | 25.00 | 12.50 | 6.25 | 3.13 | 1.56 | 0.78 | 0.39 | 0 | |
| | | 100.00 | 50.00 | 25.00 | 12.50 | 6.25 | 3.13 | 1.56 | 0.78 | 0.39 | 0 | |
| | | 100.00 | 33.33 | 11.11 | 3.70 | 1.23 | 0.41 | 0.14 | 0.05 | 0.02 | 0 | 3-fold dilution |
| | | 100.00 | 33.33 | 11.11 | 3.70 | 1.23 | 0.41 | 0.14 | 0.05 | 0.02 | 0 | |
| | | 100.00 | 33.33 | 11.11 | 3.70 | 1.23 | 0.41 | 0.14 | 0.05 | 0.02 | 0 | |
| **Plate layout** | | | | | | | | | | | | |
| **75-196 [PTX] (μM)** | CCK8 back-ground | 100.00 | 50.00 | 25.00 | 12.50 | 6.25 | 3.13 | 1.56 | 0.78 | 0.39 | 0 | 2-fold dilution |
| | | 100.00 | 50.00 | 25.00 | 12.50 | 6.25 | 3.13 | 1.56 | 0.78 | 0.39 | 0 | |
| | | 100.00 | 50.00 | 25.00 | 12.50 | 6.25 | 3.13 | 1.56 | 0.78 | 0.39 | 0 | |
| | | 100.00 | 33.33 | 11.11 | 3.70 | 1.23 | 0.41 | 0.14 | 0.05 | 0.02 | 0 | 3-fold dilution |
| | | 100.00 | 33.33 | 11.11 | 3.70 | 1.23 | 0.41 | 0.14 | 0.05 | 0.02 | 0 | |
| | | 100.00 | 33.33 | 11.11 | 3.70 | 1.23 | 0.41 | 0.14 | 0.05 | 0.02 | 0 | |

3. Drug gradients: 100 μL of fresh culture medium containing the corresponding drug prepared in step 2 were added respectively (the prepared drug concentration was 2 times that in the table above, the total volume after addition was 200 μL, and the final drug concentrations were equal to the values in the table above), and the cells were cultured for 72 hours.

4. After 72 hours of drug treatment, the original culture medium was discarded.

5. 100 μL of culture medium (containing 10% CCK8) was added to each well and continuously incubated in the cell culture incubator for 1-2 hours.

6. The absorbance values at 450 nm were measured.

III. The results of the tumor cell growth inhibition experiment with drugs at different concentrations were detected. The results are shown in Table 1 and Figures 1 to 11.

Table 1-1: $IC_{50}$ values of the compounds of the present invention for inhibiting the growth of A549 tumor cells

| Compound | $IC_{50}$ (μM) for A549 cells | |
|---|---|---|
| | 2-fold dilution | 3-fold dilution |
| 82-178 | 26.08 | 28.84 |

Table 1-2: $IC_{50}$ values of the compounds of the present invention for inhibiting the growth of COLO-205 tumor cells

| Compound | $IC_{50}$ (μM) for COLO-205 cells | |
|---|---|---|
| | 2-fold dilution | 3-fold dilution |
| 81-176 | 0.1593 | 0.05514 |
| 71-246 | 4.423 | 6.112 |

Table 1-3: $IC_{50}$ values of the compounds of the present invention for inhibiting the growth of BT-474 tumor cells

| Compound | $IC_{50}$ (μM) for BT-474 cells | |
|---|---|---|
| | 2-fold dilution | 3-fold dilution |
| 81-176 | 1.568 | 1.438 |
| 78-139 | 326.7 | 269.6 |
| 85-71 | 56.27 | 50.39 |
| 84-106 | 0.4934 | 0.6302 |

(continued)

| Compound | $IC_{50}$ (μM) for BT-474 cells | |
|---|---|---|
| | 2-fold dilution | 3-fold dilution |
| 76-207 | <<0.02 | |
| 75-196 | <<0.02 | |

Experimental Example 2: Experiment on BT-474 animal tumor model

Purpose: To establish a subcutaneous xenograft tumor model of human breast cancer BT474 cells in NPG mice, investigate the antitumor effects of multiple test products on subcutaneous xenograft tumors, and provide a basis for further pharmacodynamic studies.

Method: BT474 cells were resuscitated and subjected to cell passage and amplification. When sufficient cell number was reached, the cells in the logarithmic growth phase were harvested for cell inoculation. Before inoculation, a sustained-release estrogen tablet (17β-ESTRADIOL, lot number: SE-121, Innovative Research of America) was implanted subcutaneously in the nape of NPG mice. A BT474 cell suspension at a concentration of $8.4 \times 10^7$ cells/mL was mixed with matrigel (Matrigel Basement Membrance Matrix, CORNING) in a ratio of 1:1 to provide a cell suspension with a concentration of $4.2 \times 10^7$ cells/mL. 0.2 mL of this suspension was inoculated into the right mammary pad of the mouse. Tumor growth was observed after inoculation, and 36 tumor-bearing animals with tumor volumes ranging from 133.34 to 221.88 $mm^3$ were selected for the experiment. Based on tumor volume and body weight, the animals were randomly divided into 6 groups, which respectively were: negative control (sodium chloride injection), group **82-226** (93.1 mg/kg), group **78-139** (281.4 mg/kg), group **85-71** (162.7/81.4/40.7/20.4 mg/kg), and group **76-207** (172.0/86.0 mg/kg), with six animals in each group. The drug was administered intravenously every 3 days. The dose volume for group **78-139** was 20 mL/kg, and the dose volume for the other groups was 10 mL/kg. Each group received eight doses. The first dose for group **85-71** was 162.7 mg/kg, the second dose was 81.4 mg/kg, the third and fourth doses were 40.7 mg/kg, and the fifth to eighth doses were 20.4 mg/kg. The first to fourth doses for group **85-71** were 172.0 mg/kg, and the fifth to eighth doses were 86.0 mg/kg. Animals were euthanized on Day 29. General clinical symptoms were observed twice daily during the experimental period. Body weight and tumor diameter were measured twice weekly. Tumors were removed and weighed after euthanasia. Tumor volume, relative tumor volume (RTV), relative tumor proliferation rate (T/C%), and tumor weight inhibition rate ($IR_{TW}$%) were calculated. Efficacy was defined as relative tumor proliferation rate (T/C%) of ≤40% in the treatment group and $P \leq 0.05$ for the RTV of the treatment group compared to the RTV of the negative control group. $IR_{Tw}$ of ≥60% was used as a secondary reference indicator for efficacy.

Results: During the entire experiment, one animal in the negative control group died on Day 15; one animal in Group **82-226** was found dead on Day 19 and one on Day 20, respectively; one animal in Group **78-139** was found dead on Day 7 and one on Day 19, respectively; and one animal in Group **85-71** was found dead on Day 4 and one on Day 10, respectively. Causes of death in the negative control group was presumably related to the high tumor burden. Some animals that died from the test products were light in weight and were observed to be lethargic before death, which was presumably related to the toxicity of the test products. The weight of animals in all groups showed a gradual increase, and no significant differences were observed between groups at any time point (P>0.05).

Tumors in the negative control group gradually grew throughout the experiment. By the end of the experiment (Day 29), the average tumor volume was $2518.14 \pm 984.66$ $mm^3$, and the average RTV was $13.78 \pm 5.44$. The average tumor volumes of groups **82-226, 78-139, 85-71,** and **76-207** were $876.63 \pm 388.34$ $mm^3$, $763.44 \pm 303.36$ $mm^3$, $1486.48 \pm 439.37$ $mm^3$, and $1948.23 \pm 630.67$ $mm^3$, respectively, and the average RTV values were $4.85 \pm 1.26$, $4.22 \pm 1.71$, $8.08 \pm 1.53$, and $10.80 \pm 3.96$, respectively. Both the average tumor volume and RTV decreased in each group after drug administration. The mean tumor volumes and RTV values of groups **82-226, 78-139,** and **85-71** were significantly lower than those of group 1 on Day 15 ($P \leq 0.05$), and no significant differences were observed between groups at any time point ($P > 0.05$). The tumor growth trends of all groups are shown in Figure 12.

By the end of the experiment (Day 29), the T/C% values of groups **82-226, 78-139, 85-71,** and **76-207** were 35.17%, 30.65%, 58.66%, and 78.42%, respectively, and their $IR_{TV}$% values were 64.83%, 69.35%, 41.34%, and 21.58%, respectively. The T/C% value of group **82-226** decreased to below 40% on Day 15, and the RTV was also lower than that of the negative control group ($P \leq 0.05$).

At the end of the experiment, the animals were euthanized and tumor weights were measured. The average tumor weights of the negative control group, groups **82-226, 85-71,** and **76-207** were $1.785 \pm 0.837$ g, $0.594 \pm 0.265$ g, $0.474 \pm 0.167$ g, $1.207 \pm 0.510$ g, and $1.384 \pm 0.419$ g, respectively. The $IR_{TW}$% values were 66.72%, 73.47%, 32.37%, 22.44%, respectively. The $IR_{TW}$% values of groups **82-226** and **78-139** were >60%.

A diagram showing the tumor weight inhibition rate for each group is shown in Figure 13.

Conclusion: Under the condition of the present experiment, test product **82-226** at a dose of 93.1 mg/kg, test

product **78-139** at a dose of 281.4 mg/kg, and test product **85-71** at a dose range of 20.4-162.7 mg/kg administered once every 3 days via tail vein injection significantly inhibited the growth of subcutaneous xenograft tumors of human breast cancer BT474 cells in NPG mice. **82-226** and **78-139** exhibited stronger effects than **85-71,** while **76-207** had the weakest inhibitory effect.

Experimental Example 3: Experiment on A-549 animal tumor model

Purpose:

To investigate the effects of a series of polyethylene glycol small molecule dual-drug compounds on subcutaneous tumor models of lung cancer (A-549) and breast cancer (MDA-MB-231) in mice.

Methods:

Efficacy study on nude mouse xenograft model (subcutaneous tumor) of A-549 lung cancer cells: Quarantine-passed BALB/c-nude mice were inoculated subcutaneously on the right side of their backs with A-549 cells at a final concentration of $5\times10^7$ cells/mL in a volume of 0.1 mL per mouse. When the average tumor volume reached approximately 100 $mm^3$, the mice were randomly divided into the following groups according to the tumor volume: model control group, group **82-178,** group **75-196,** and group **59-126.** Drugs were administered via tail vein injection once every 3 days for a total of 4 doses. The animals were observed daily, and their body weights and tumor volumes were measured every 3 days. Relative tumor volume and proliferation rate were calculated. At the final sampling, the appearance of animals and tumors were photographed, tumor weights were measured, and tumor inhibition rates were calculated.

Efficacy study on nude mouse xenograft model (subcutaneous tumor) of MDA-MB-231 breast cancer cells: Quarantine-passed BALB/c-nude mice were inoculated subcutaneously on the right side of their backs with MDA-MB-231 cells at a final concentration of $5\times10^7$ cells/mL in a volume of 0.1 mL per mouse. When the average tumor volume reached approximately 100 $mm^3$, the mice were randomly divided into the following groups according to the tumor volume: model control group, group **82-178,** and group **81-176.** Drugs were administered via tail vein injection once every 3 days for a total of 4 doses. The animals were observed daily, and their body weights and tumor volumes were measured every 3 days. The relative tumor volume and proliferation rate were calculated. At the final sampling, the appearance of animals and tumors were photographed, tumor weights were measured, and tumor inhibition rates were calculated.

Results:

1. Efficacy study on nude mouse xenograft model (subcutaneous tumor) of lung cancer A-549 cells

(1) General observation: During the experiment, all animals were in good mental and physical condition, with normal tumor growth, animal excretion, and diet.

(2) Body weight (Figure 14): After drug administration, no abnormal changes were observed in the body weight of the animals in each group, indicating that the test product had no significant effect on the body weight of the animals.

(3) Tumor volume, relative tumor volume and relative tumor proliferation rate (Figure 15): Compared with the model control group, the final tumor volumes after drug administration in groups **82-178, 75-196,** and **59-126** was significantly reduced. Compared with the model control group, the final relative tumor volume of group **82-178** was significantly decreased, and the relative tumor proliferation rates on Day 4 to Day 28 were 76.23%, 88.15%, 83.85%, 44.57%, 33.04%, 31.02%, 31.27%, 25.77%, and 28.03%, respectively; the final relative tumor volume of group **75-196** was significantly decreased, and the relative tumor proliferation rates on Day 4 to D28 were 71.96%, 76.49%, 98.49%, 57.55%, 37.83%, 35.25%, 28.03%, 29.68%, and 21.74%, respectively; the final relative tumor volume of group **59-126** was significantly decreased, and the relative tumor proliferation rates on Day 4 to Day 28 were 75.32%, 66.61%, 96.77%, 27.33%, 10.31%, 6.10%, 5.22%, 3.84%, and 4.96%, respectively.

(4) Tumor weight and tumor inhibition rate (Figure 16): Compared with the model control group, the tumor weights of mice in groups **82-178, 75-196,** and **59-126** were significantly reduced, and the tumor inhibition rates were 74.67%, 77.35%, and 94.23%, respectively, all greater than 40%.

2. Efficacy study on nude mouse xenograft tumor model (subcutaneous tumor) of breast cancer MDA-MB-231 cells

(1) General observation: From inoculation to administration, all animals were in good mental and general condition, with normal tumor growth, animal excretion, and diet. After administration, no obvious abnormalities were observed in the model control group, group **82-178,** and group **81-176.**

(2) Body weight (Figure 17): Compared with the model control group, the final body weight of group **82-178** was significantly reduced; the final body weight of group **81-176** was not significantly affected.

(3) Tumor volume, relative tumor volume, and relative tumor proliferation rate (Figure 18): Compared with the model control group, the final tumor volume after drug administration in groups **82-178** and **81-176** decreased significantly. Compared with the model control group, the final relative tumor volume of group **82-178** decreased significantly, and the relative tumor proliferation rates on Day 4 to Day 22 were 66.56%, 56.52%, 18.83%, 5.46%, 8.74%, 15.59%, and 18.18%, respectively; the final relative tumor volume of group **81-176** decreased significantly, and the relative tumor proliferation rates on Day 4 to Day 22 were 70.87%, 45.43%, 17.51%, 4.53%, 2.06%, 1.61%, and 0.20%, respectively.

(4) Tumor weight and tumor inhibition rate (Figure 19): At the end of the experiment, compared with the model control group, the tumor weights of mice in group **82-178** and group **81-176** were significantly reduced, and the tumor inhibition rates were 84.63% and 99.70%, respectively, both greater than 40%.

Conclusion:

1. Efficacy study on nude mouse xenograft tumor model (subcutaneous tumor) of lung cancer A-549 cells

Under the conditions of this experiment, the subcutaneous lung cancer (A-549) mouse model was prepared, and **82-178** and **81-176** were injected via tail vein once every 3 days for a total of 4 doses. The results showed that **82-178** and **81-176** could significantly reduce tumor volume, relative tumor volume, relative tumor proliferation rate, and tumor weight, and tumor inhibition rates were >40%, suggesting that **82-178** and **81-176** are promising for the treatment of lung cancer.

2. Efficacy Study on nude mouse xenograft tumor model (subcutaneous tumor) of breast cancer MDA-MB-231 cells

Under the conditions of this experiment, the subcutaneous breast cancer (MDA-MB-231) mouse model was established, and **82-178** and **81-176** were administered via tail vein injection once every 3 days for a total of 4 doses. The results showed that **82-178** and **81-176** could significantly reduce tumor volume, relative tumor volume, relative tumor proliferation rate, and tumor weight, and tumor inhibition rates were >40%, suggesting that **82-178** and **81-176** are promising for the treatment of breast cancer.

Experimental Example 3: BNCT experiments on B16-F10 melanoma model, SAS human oral squamous cell carcinoma model, MG-63 human osteosarcoma model, and MDA-MB-231 and BT-474 human breast cancer model

① Preparation and treatment of mice B16-F10 melanoma model, SAS human oral squamous cell carcinoma model, MG-63 human osteosarcoma model, and MDA-MB-231 and BT-474 human breast cancer model

All C57BL6/j, NPG, NOD-SCID, or other strains of mice (female, 4 weeks old) were acclimated for 7 days under specific breeding conditions (14-hour/10-hour light and dark cycle, with light of 200 Lx in daytime between 5:00 AM and 7:00 PM, temperature of 23.5±2.5°C, and humidity of 52.5±12.5%). Afterwards, the dorsal hair of the right hind leg of the mice was shaved. On the next day, under isoflurane anesthesia (2-2.5% in ambient air), the mice were inoculated subcutaneously in the right thigh with B16-F10 cells, SAS human oral squamous cell carcinoma cells, MG-63 human osteosarcoma cells and BT-474 human breast cancer cells ($10^7$ cells/mL; 50 μL/mouse). Tumors were allowed to grow for 7 days, and then the animals were randomly divided into control group and experimental groups. Tumor volume (V) was estimated using the following formula:

$$V = a \times b^2/2$$

wherein a and b are the lengths of long and short axes of tumor, respectively, as determined by a vernier caliper. On the day of irradiation (Day 0), the average tumor volume for all mice was approximately 80 $mm^3$, which was considered as the initial tumor volume ($V_0$). All subcutaneous injections were performed on the dorsal side of the neck using a syringe with a 27-gauge needle. All animal experiments were conducted in accordance with the submitted animal experiment plan and were

reviewed and approved by the Animal Ethics Committee.

② Boron neutron capture therapy (BNCT) studies in B16-F16 melanoma model, SAS human oral squamous cell carcinoma model, MG-63 human osteosarcoma model, and MDA-MB-231 and BT-474 human breast cancer model

The mice of B16-F10 melanoma model, SAS human oral squamous cell carcinoma model, MG-63 human osteosarcoma model, and MDA-MB-231 and BT-474 human breast cancer model were respectively divided into 9 groups, with 6 mice in each group. Normal saline solution and the normal saline solutions of compounds **67-228, 73-49, 59-169, 67-237, 82-152, 82-132, 82-208, 82-207, 78-127, 78-89, 78-152** and **78-151** (equivalent dose: 1 mg B/kg)) were injected subcutaneously: normal saline (2 hours), compounds **67-228, 73-49, 59-169, 67-237, 82-152, 82-132, 82-208, 82-207, 78-127, 78-89, 78-152,** and **78-151** (24 hours), where the hours in parentheses represent the time interval between injection and irradiation. The mice were placed in customized acrylic holders by securing their tails and thighs with autoclaved tape. These holders were attached around the holes of the acrylic plate with Velcro. The acrylic plate was then fixed to a 5 mm thick thermoplastic plate, in which the thermoplastic plate contained 40 wt% $^6$LiF ($^6$Li enrichment: 96%), and provided neutron shielding. Both the acrylic plate and the thermoplastic plate had a round hole in the center, so that the mouse's right thigh could be stretched and exposed to these holes. Through these holes, the tumors were irradiated with neutrons for 60 minutes at an average rate of $3.44 \pm 0.22 \times 10^{12}$ neutrons/cm$^2$ (n = 6). After neutron irradiation, the mice were housed for three weeks, with tumor volume measured twice weekly and tumor growth curves plotted. After the third week, the mice were sacrificed, the tumor masses were removed and weighed, and the tumor weight inhibition rate was calculated.

③ Statistical analysis

Statistical analysis was performed using the Student's t-test in GraphPad Prism 8 software. A p-value of less than 0.05 was considered statistically significant. Unless otherwise stated, all results are presented as mean ± standard deviation.

④ Experimental conclusions

Compared with the normal saline group, the tumor volumes and tumor mass weights in the groups treated with compounds **67-228, 73-49, 59-169, 67-237, 82-152, 82-132, 82-208, 82-207, 78-127, 78-89, 78-152,** and **78-151** were significantly reduced.

While specific embodiments of the present invention have been described in detail, those skilled in the art will appreciate that, based on the teachings disclosed herein, various modifications and substitutions to those details are possible, and such changes are within the scope of protection of the present invention. The full scope of the present invention is given by the appended claims and any equivalents thereof.

## Claims

1. A compound represented by formula I or a pharmaceutically acceptable salt thereof:

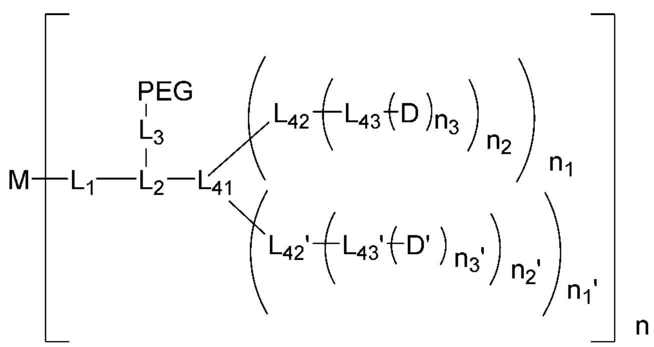

formula I

wherein,

M is a chain or cyclic structure containing one or more identical or different heteroatoms, and M is connected to $L_1$ via the heteroatom;
each $L_1$ is independently selected from the group consisting of a bond,

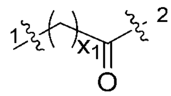
,

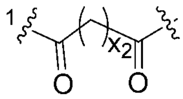
,

and a group formed by one or two identical or different $L_1$ and one or more identical or different M in the following manner: $L_1$-M-$(L_1)_n$, $L_1$-M-$(L_1$-M-$(L_1)_n)_n$, or $L_1$-M-$(L_1$-M-$(L_1$-M-$(L_1)_n)_n)_n$, wherein the terminus 1 is connected to M, the terminus 2 is connected to $L_2$, wherein each of $x_1$ and $x_2$ is independently selected from the group consisting of 0, 1, 2, 3, 4, 5, 6, 7, 8, 9, and 10;
each $L_2$ is independently an amino acid residue containing in its side chain a functional group selected from the group consisting of $-NH_2$, -OH, -SH, and -COOH, and $L_2$ is connected to $L_1$, $L_3$, and $L_{41}$ via the N-terminus and C-terminus of the amino acid and the functional group; preferably, $L_2$ is connected to $L_3$ via the functional group;
each of $L_3$ and $L_{43}$' is independently

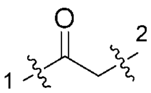
or
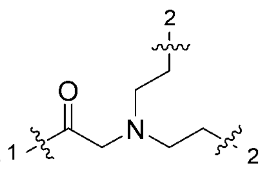
,

wherein the terminus 1 is connected to $L_2$, and the terminus 2 is connected to PEG;
each PEG is independently selected from the group consisting of polyethylene glycol, amino-polyethylene glycol, methoxy-polyethylene glycol, carboxyl-polyethylene glycol, and cholesterol-polyethylene glycol;
each $L_{41}$ is independently

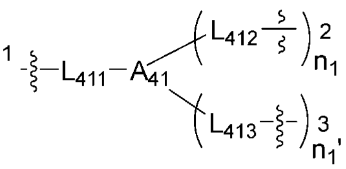
,

wherein the terminus 1 is connected to $L_2$, the terminus 2 is connected to $L_{42}$, and the terminus 3 is connected to $L_{42}$'; wherein each of $L_{411}$, $L_{412}$ and $L_{413}$ is independently selected from the group consisting of a bond, an amino acid residue or derivatives thereof, a polypeptide fragment consisting of two or more amino acids or derivatives thereof, $-NH(CH_2)_{x3}C(O)-$, $-CO(CH_2)_{x3}C(O)-$, $-NH(CH_2)_{x3}NH-$, and $-NH(CH_2)_2(OCH_2CH_2)_{x3}NH-$ and any combination thereof, wherein each $x_3$ is independently selected from the group consisting of 0, 1, 2, 3, 4, 5, 6, 7, 8, 9, and 10; $A_{41}$ is a bond or an amino acid residue or derivatives thereof, or a polypeptide fragment consisting of two or more amino acids or derivatives thereof;
each of $L_{42}$ and $L_{42}$' is independently a bond or

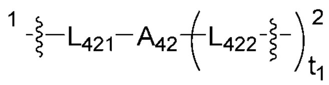
,

wherein the terminus 1 is connected to $L_{41}$, and the terminus 2 is connected to $L_{43}$ or $L_{43}$'; wherein each of $L_{421}$ and $L_{422}$ is independently selected from the group consisting of a bond, an amino acid residue or derivatives thereof, or a polypeptide fragment consisting of two or more amino acids or derivatives thereof, $NH(CH_2)x_4C(O)-$, $-CO(CH_2)x_4C(O)-$, $-NH(CH_2)x_4NH-$, and $-NH(CH_2)_2(OCH_2CH_2)x_4NH-$, and any combination thereof, each $x_4$ is

independently selected from the group consisting of 0, 1, 2, 3, 4, 5, 6, 7, 8, 9, and 10, $A_{42}$ is a bond or selected from the group consisting of amino acid residue or derivatives thereof, or a polypeptide fragment consisting of two or more amino acids or derivatives thereof, $t_1 = n_2$ or $n_2$';
each of $L_{43}$ and $L_{43}$' is independently selected from the group consisting of a bond,

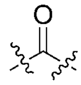,

-NH-, hydrazino (i.e., -NH-N=), an amino acid residue or derivatives thereof, a polypeptide fragment consisting of two or more amino acids or derivatives thereof, $-NH(CH_2)_{x5}C(O)-$, $-NH(CH_2)_{x5}NH-$, $-NH(CH_2)_2(OCH_2CH_2)_{x5}NH-$, $-C(O)(CH_2)_{x5}C(O)-$,

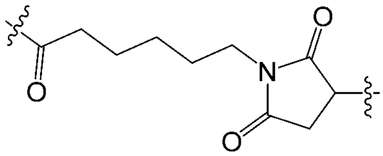

and any combination thereof, wherein each $x_5$ is independently selected from the group consisting of 0, 1, 2, 3, 4, 5, 6, 7, 8, 9, and 10;
each D is independently selected from the group consisting of cytotoxic drug moieties, preferably, the cytotoxic drug is selected from the group consisting of tubulin inhibitors, DNA intercalators, DNA topoisomerase inhibitors and RNA polymerase inhibitors; preferably, the cytotoxic drug is selected from the group consisting of PTX (paclitaxel), PCB (palbociclib), SN38 (7- and 10-hydroxy-camptothecin), NPB (Niraparib, MK-4827), AXT (Axitinib), LPT (lapatinib), DOX (doxorubicin), MI-AH-PLGLAG-iRGD, folic acid, SB7 (SB-743921), IRN (Irinotecan), sodium dodecahydrododecaborate, PPT-iRGD, disodium undecahydromercaptododecaborate, disodium undecahydromercaptododecaboride ($^{10}B$), disodium undecahydroaminododecaborate, disodium undecahydroaminododecaboride ($^{10}B$),

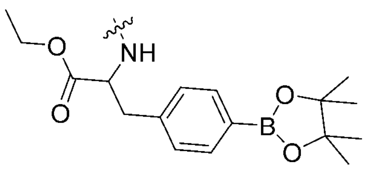, 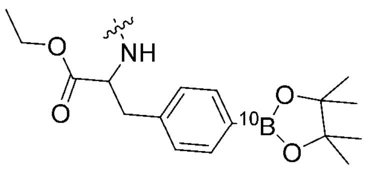 and 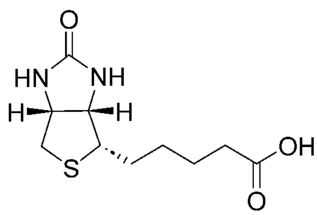 ;

each n is independently selected from the group consisting of 1, 2, 3, 4, 5, 6, 7, 8, 9, and 10;
each of $n_1$ and $n_1$' is independently selected from the group consisting of 0, 1, 2, 3, 4, 5, 6, 7, and 8;
each of $n_2$ and $n_2$' is independently selected from the group consisting of 1, 2, 3, 4, 5, 6, 7, and 8;
each of $n_3$ and $n_3$' is independently selected from the group consisting of 1, 2, 3, 4, 5, 6, 7, and 8.

**2.** The compound or pharmaceutically acceptable salt thereof according to claim 1, wherein the compound is **characterized by** one or more of the following:

(1) M is selected from the group consisting of groups obtained by losing one or more (e.g., 1 to 10) hydrogen atoms in the following molecules:

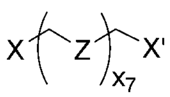, 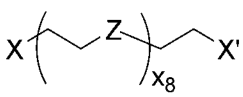 and 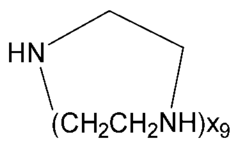,

wherein each of X and X' is independently selected from the group consisting of -OH, -NRR', and -SH, Z is selected from the group consisting of -C(RR')-, -O-, and -N(R)-, each of R and R' is independently selected from the group consisting of hydrogen, $C_{1-6}$ alkyl, and $C_{1-6}$ alkyl substituted with amino or $C_{1-6}$ alkylamino, each of $x_7$

and $x_8$ is independently selected from the group consisting of 0, 1, 2, 3, 4, 5, 6, 7, 8, 9, and 10, and $x_9$ is selected from the group consisting of 1, 2, 3, 4, 5, and 6;

(2) $L_1$ is selected from

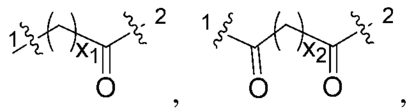

and a group formed by one or more identical or different $L_1$ and one or more identical or different M in the following manner: $L_1$-M-$(L_1)_n$, wherein each of $x_1$ and $x_2$ is independently selected from the group consisting of 1, 2, 3, 4, 5 and 6;

(3) $L_2$ is selected from the group consisting of the residues of the following amino acids: Lys, Cys, Thr, Ser, Asp or Glu;

(4) PEG is methoxy-polyethylene glycol;

(5) PEG has a number-average molecular weight of 5k to 40k;

(6) each $L_{411}$ is independently selected from the group consisting of a bond, $-NH(CH_2)_{x3}C(O)-$, $-CO(CH_2)_{x3}C(O)-$, $-NH(CH_2)_{x3}NH-$, $-NH(CH_2)_{x3}C(O)NH(CH_2)_{x3}C(O)-$, $-NH(CH_2)_2(OCH_2CH_2)_{x3}NH-$ and $-NH(CH_2)_2(OCH_2CH_2)_{x3}NH-$, and $x_3$ is selected from the group consisting of 1, 2, 3, 4, 5 and 6;

(7) each $L_{412}$ is independently selected from the group consisting of a bond, $-NH(CH_2)_{x3}C(O)-$, $-CO(CH_2)_{x3}C(O)-$, $-NH(CH_2)_{x3}NH-$, $-NH(CH_2)_{x3}C(O)-A-$, $-NH(CH_2)_{x3}C(O)NH(CH_2)_{x3}C(O)-$, $-NH(CH_2)_2(OCH_2CH_2)_{x3}NH-$ and $-NH(CH_2)_2(OCH_2CH_2)_{x3}NH-$, wherein A is selected from the group consisting of an amino acid residue or derivatives thereof, or a polypeptide fragment consisting of two or more amino acids or a derivative thereof, the amino acid is selected from the group consisting of Glu and Asp, and each $x_3$ is independently selected from the group consisting of 1, 2, 3, 4, 5 and 6;

(8) each $L_{413}$ is independently selected from the group consisting of a bond, $-NH(CH_2)_{x3}C(O)-$, $-NH(CH_2)_{x3}NH-$, $-NH(CH_2)_2(OCH_2CH_2)_{x3}NH-$, $-CO(CH_2)_{x3}C(O)-$, $-NH(CH_2)_{x3}C(O)NH(CH_2)_{x3}C(O)-$ and $-NH(CH_2)_{x3}C(O)NH(CH_2)_{x3}NH-$, each $x_3$ is independently selected from the group consisting of 1, 2, 3, 4, 5 and 6;

(9) the amino acid in $A_{41}$ or $A_{42}$ is selected from the group consisting of Glu, Gly, Asp, Lys, Cys, Thr and Ser;

(10) each $L_{421}$ is independently selected from the group consisting of a bond, $-NH(CH_2)x_4C(O)-$, $-CO(CH_2)x_4C(O)-$, $-NH(CH_2)x_4NH-$, $-NH(CH_2)_2(OCH_2CH_2)x_4NH-$ and any combination thereof, each $x_4$ is independently selected from the group consisting of 0, 1, 2, 3, 4, 5, 6, 7, 8, 9 and 10;

(11) each $L_{422}$ is independently selected from the group consisting of a bond, $-NH(CH_2)_{x3}C(O)-A'-$, $-NH(CH_2)x_4C(O)-$, $-CO(CH_2)x_4C(O)-$, $-NH(CH_2)x_4NH-$ and $-NH(CH_2)_2(OCH_2CH_2)x_4NH-$, wherein A' is selected from the group consisting of an amino acid residue or derivatives thereof, or polypeptide fragment consisting of two or more amino acids or derivatives thereof, and each $x_4$ is independently selected from the group consisting of 0, 1, 2, 3, 4, 5, 6, 7, 8, 9 and 10;

(12) the amino acids in $L_{43}$ and $L_{43}$' are selected from the group consisting of Glu, Gly, Phe, Leu and Cys;

(13) the derivatives in $L_{41}$, $L_{42}$, $L_{42}$', $L_{43}$ and $L_{43}$' are selected from the group consisting of acylation (e.g., acetylation) or alkylation (e.g., methylation) derivatives;

(14) each $x_5$ is independently selected from the group consisting of 1, 2, 3, 4, 5 and 6.

**3.** The compound or pharmaceutically acceptable salt thereof according to claim 1 or 2, wherein the compound is **characterized by** one or more of the following:

(1) M is selected from the groups obtained by losing one or more (e.g., 1 to 10) hydrogen atoms in the following molecules: EDA, DETA, AMDA, TAEA, TETA, TEPA, DAPO, APDO, APD, glycerol, MPE,

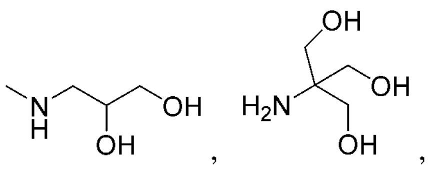

TAN and TACD; preferably, M is selected from the group consisting of

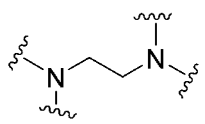 , 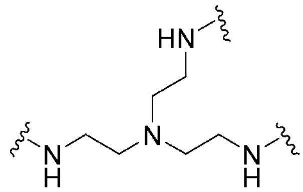 ,

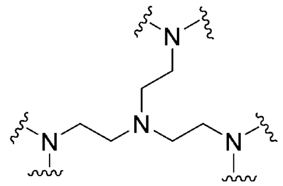 , 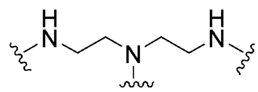 , 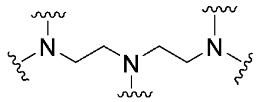 ,

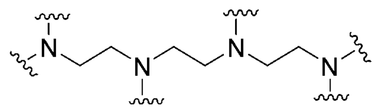 , 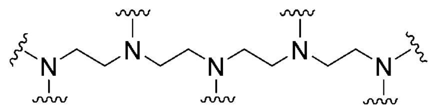 , 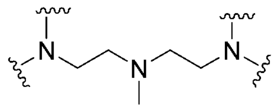 ,

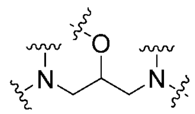 , 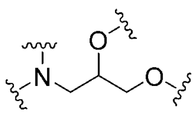 , 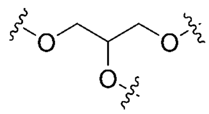 , 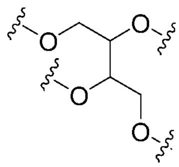 , 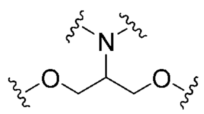 ,

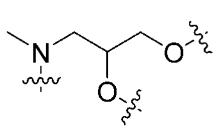 , 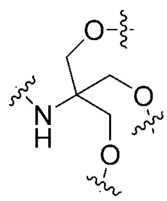 , 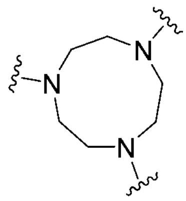 and 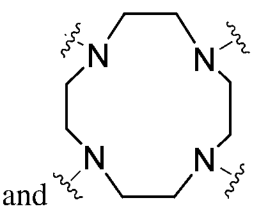 ;

(2) $L_1$ is selected from the group consisting of

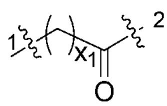 , 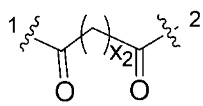

and

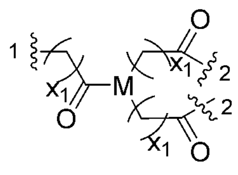 ,

and each of $x_1$ and $x_2$ is independently selected from the group consisting of 0, 1, 2, 3, 4, and 5;
(3) $L_2$ is selected from the residues of the following amino acids: Lys, Cys, Thr, or Ser;
(4) PEG has a number-average molecular weight of 5k to 10k, or 10k to 40k, for example, 5k or 10k;
(5) $A_{41}$ and $A_{42}$ are independently selected from the group consisting of a bond, Glu, Lys, Glu-Asp, Glu-Glu, $Glu(Glu)_2$, Glu(Glu)(Glu(Glu)(GlyGlu)), GluGluGly, GlyGlu;
(6) each $L_{41}$ is independently selected from the group consisting of $-NH(CH_2)_2CO-$, $-NH(CH_2)_5CO-$,

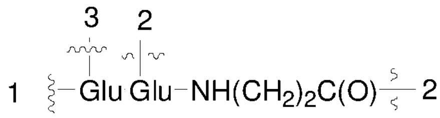, 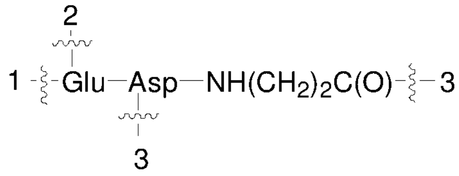,

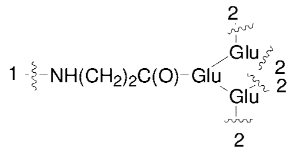, 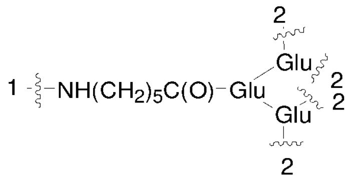, 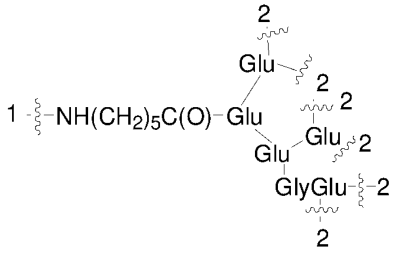,

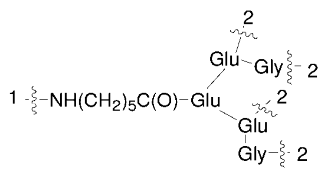, 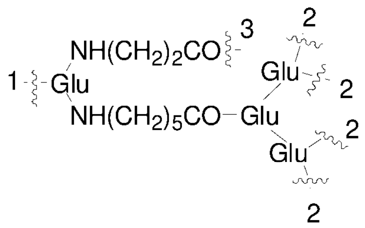, 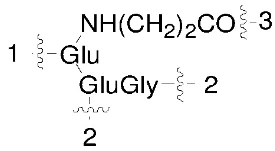,

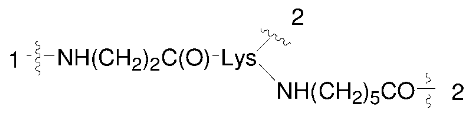, 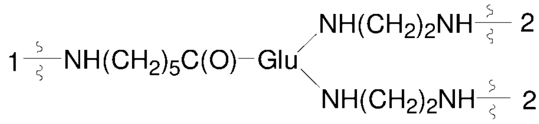,

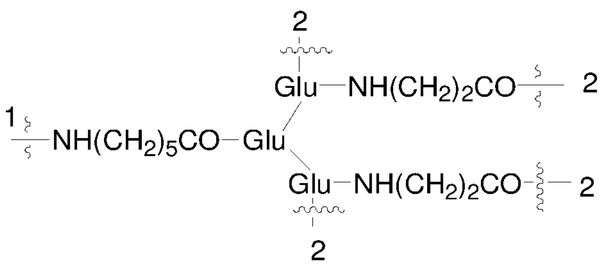, 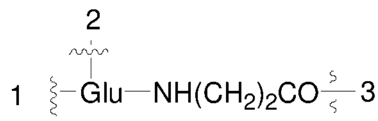

and

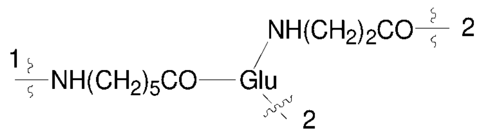;

(7) each of $L_{42}$ and $L_{42}$' is independently selected from the group consisting of a bond, Glu, GlyGlu, $-NH(CH_2)_5CO-$, $-CO(CH_2)_2CO-$, $-NH(CH_2)_2NH-$, $-NH(CH_2)_5CO-Glu$, $Glu(Glu)_2$, $-NH(CH_2)_2(OCH_2CH_2)_2NH-$,

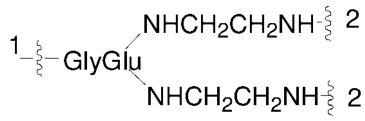,

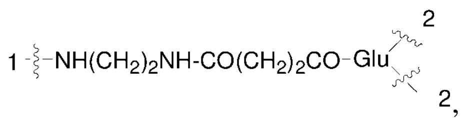, 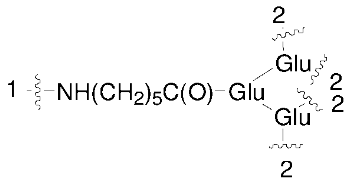, 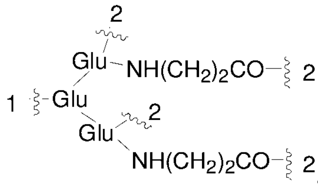,

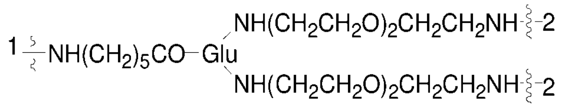,

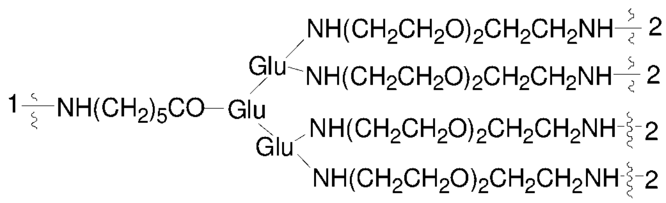, 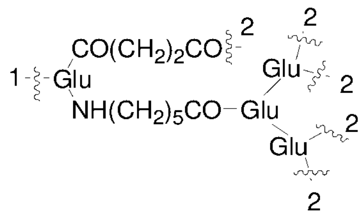

and

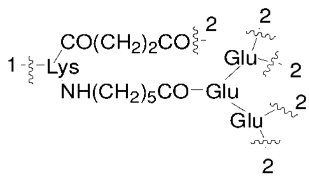;

(8) $L_{43}$ is selected from the group consisting of a bond,

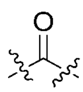,

Cys(Ac), -NH-N=, -GlyPheLeuGly-, $-C(O)(CH_2)_{x5}C(O)-$, -Gly-NH-N=, $Glu(GlyPheLeuGly-)_2$, $GlyGlu(GlyPheLeuGly)_2$, $-Glu(Gly-NHN=)_2$, $-C(O)(CH_2)_{x5}C(O)-Glu(GlyPheLeuGly)(Glu(GlyPheLeuGly)_2)$, $Glu(Glu)_2$, $-NH(CH_2)_{x5}C(O)-$, $-NH(CH_2)_{x5}C(O)NH-$, $-NH(CH_2)_2(OCH_2CH_2)_{x5}NH-$, $-C(O)(CH_2)_{x5}C(O)-NH(CH_2)_{x5}C(O)-$, and

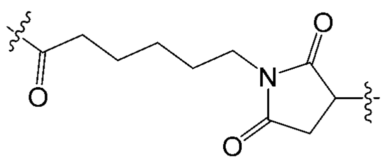,

each $x_5$ is independently selected from the group consisting of 1, 2, 3, 4, 5 and 6.

**4.** The compound or pharmaceutically acceptable salt thereof according to any one of claims 1 to 3, wherein the compound is **characterized by** one or more of the following:

(1) $L_2$ is selected from the group consisting of Lys residue;
(2) $L_{43}$ is selected from the group consisting of a bond,

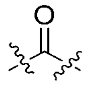,

Cys(Ac), -GlyPheLeuGly-, -C(O)$(CH_2)_2$C(O)-, Gly-NHN=, -GlyGlu(GlyPheLeuGly-$)_2$, Glu(GlyPheLeuGly-$)_2$, -Glu(Gly-NHN=$)_2$, -C(O)$(CH_2)_2$C(O)-Glu(GlyPheLeuGly)(Glu(GlyPheLeuGly$)_2$), Glu(Glu$)_2$, -NH$(CH_2)_5$C(O)-, -NH$(CH_2)_5$C(O)NH-, -NH$(CH_2)_2$$(OCH_2CH_2)_2$NH-, -C(O)$(CH_2)_2$C(O)-NHCH$_2$C(O)-, -C(O)$(CH_2)_2$C(O)-NH$(CH_2)_2$C(O)-, and

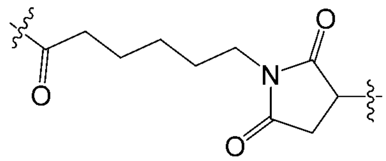.

5. The compound or pharmaceutically acceptable salt thereof according to any one of claims 1 to 4, wherein the compound is selected from the group consisting of the following compounds:

| | |
|---|---|
| 71--215 | 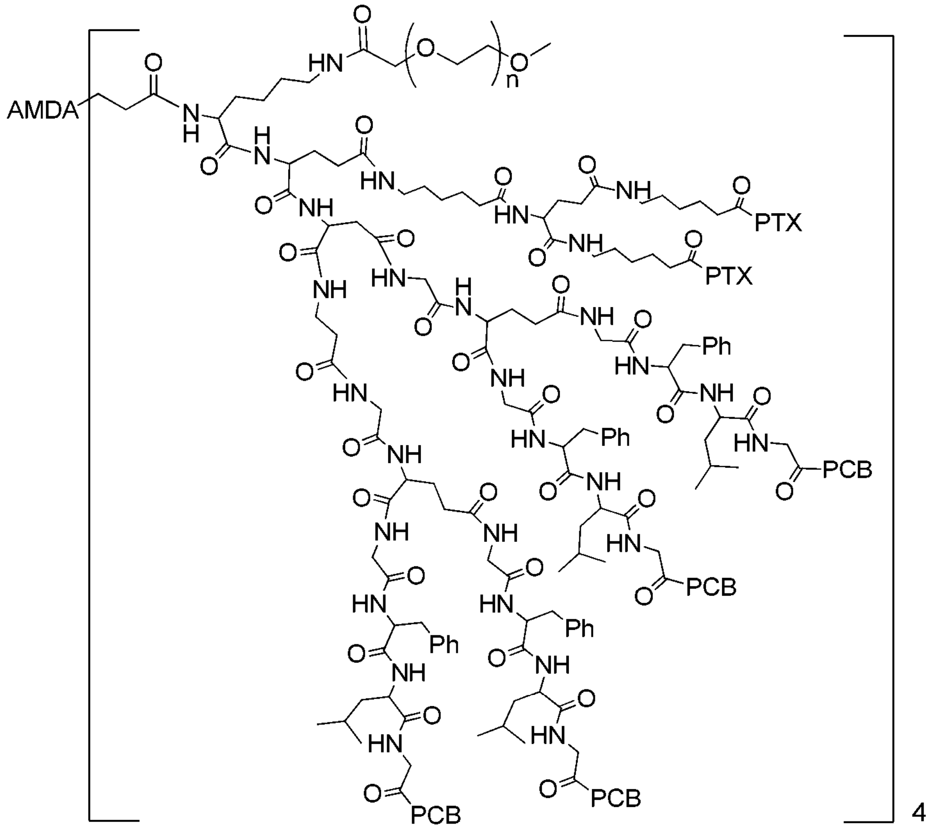<br>each polyethylene glycol fragment has a number-average molecular weight of 10k |

(continued)

| | |
|---|---|
| 59--126 | 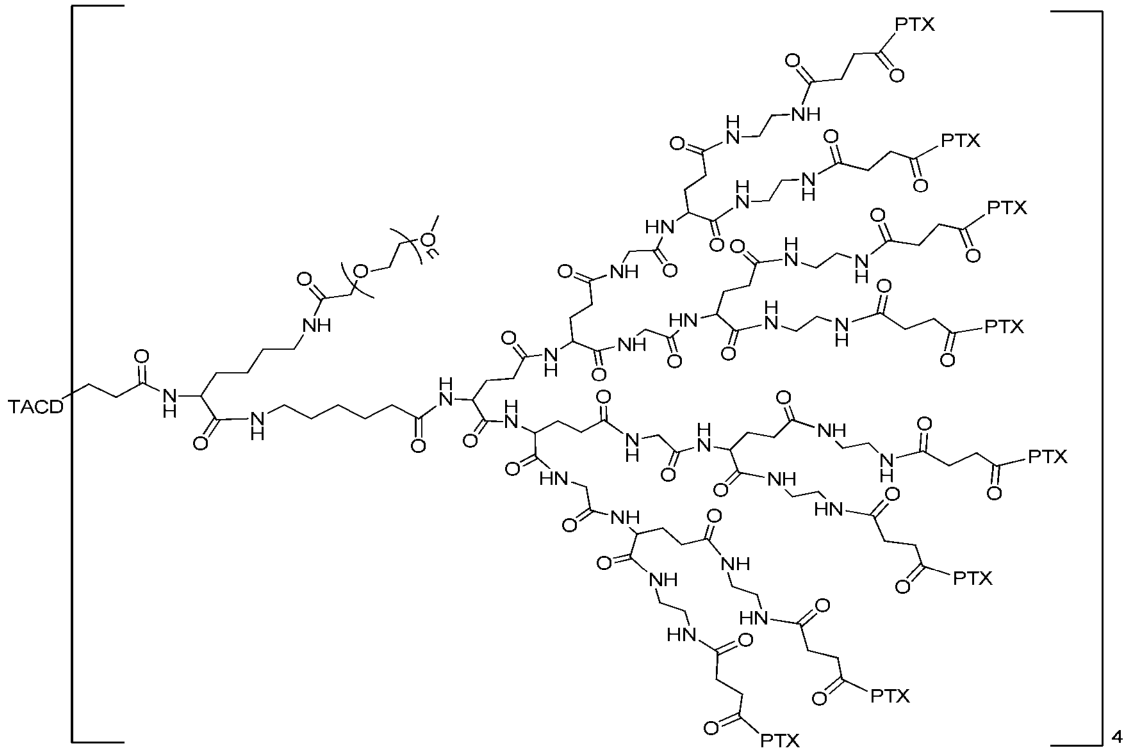 each polyethylene glycol fragment has a number-average molecular weight of 10k |

(continued)

| | |
|---|---|
| **67--228** | 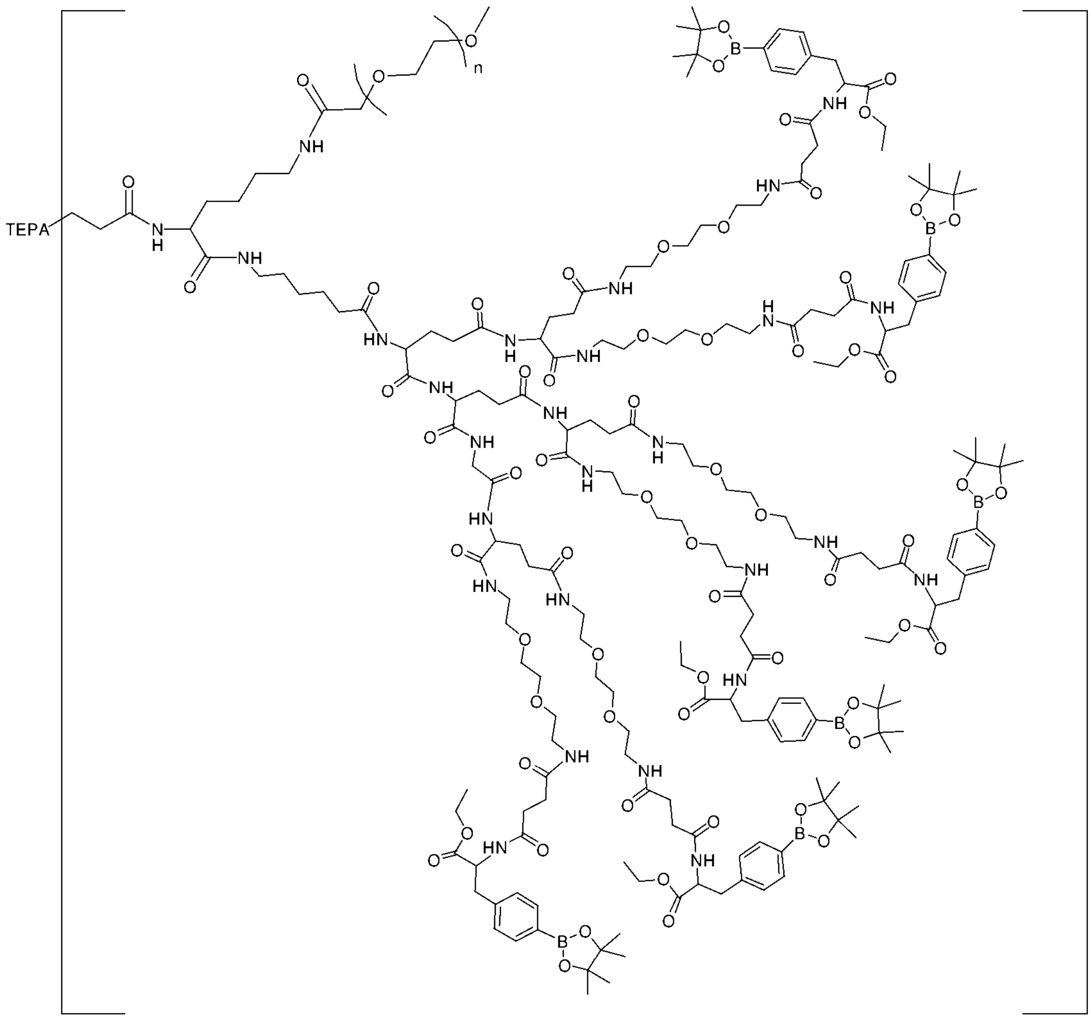 7<br>each polyethylene glycol fragment has a number-average molecular weight of 5k |

(continued)

| | |
|---|---|
| 81--118 | 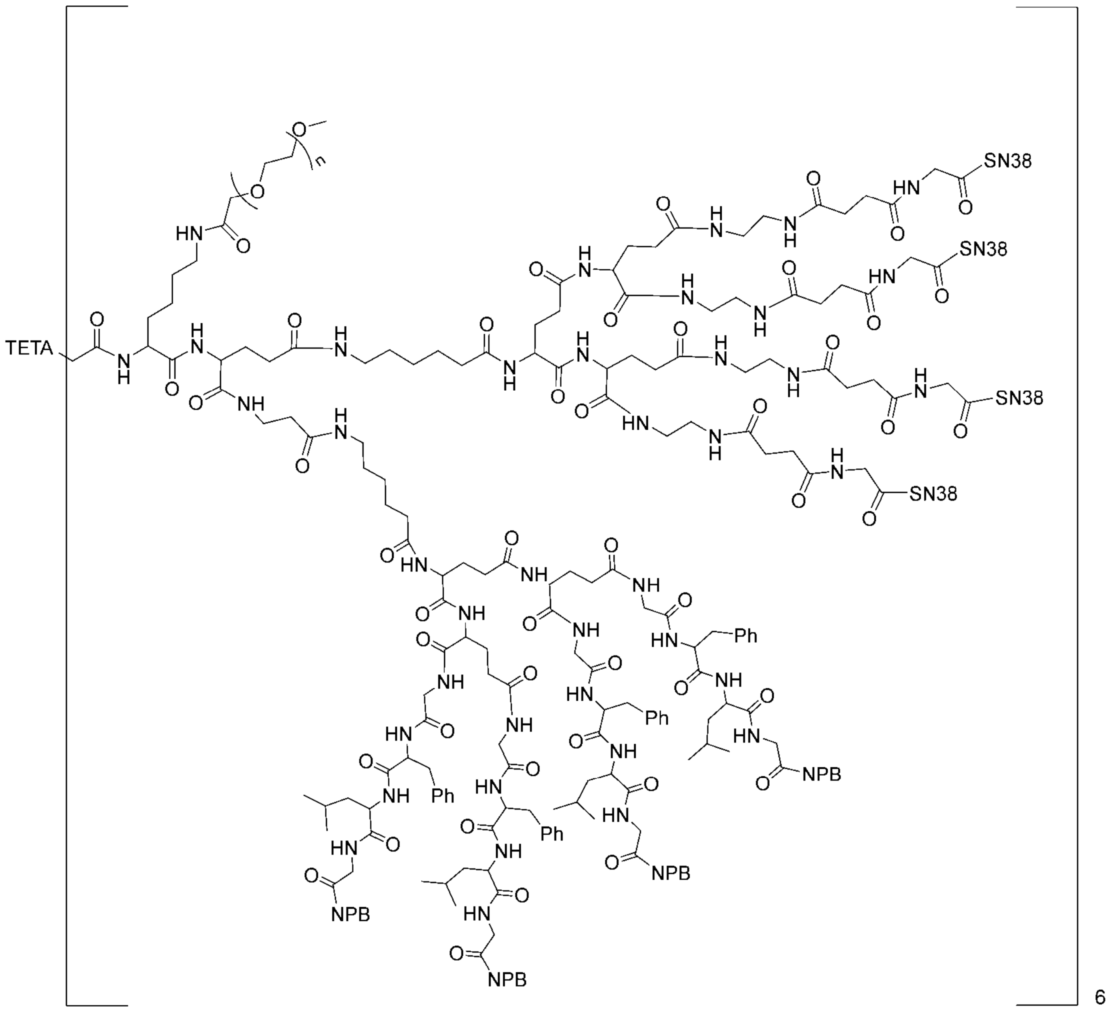<br>each polyethylene glycol fragment has a number-average molecular weight of 5k |
| 50--221 | 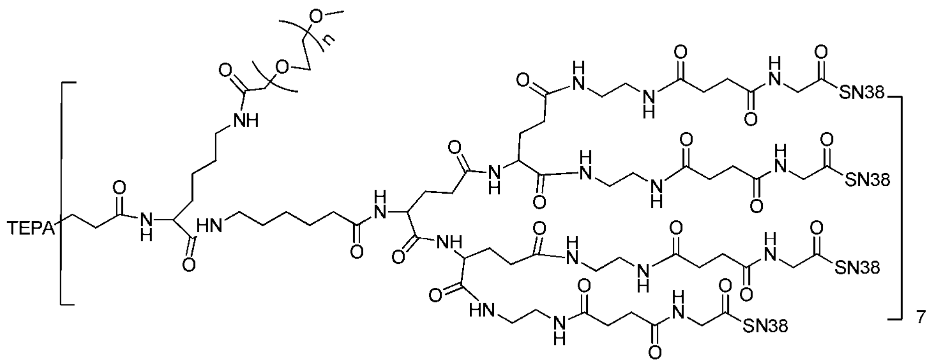<br>each polyethylene glycol fragment has a number-average molecular weight of 5k |

(continued)

| | |
|---|---|
| 69--113 | 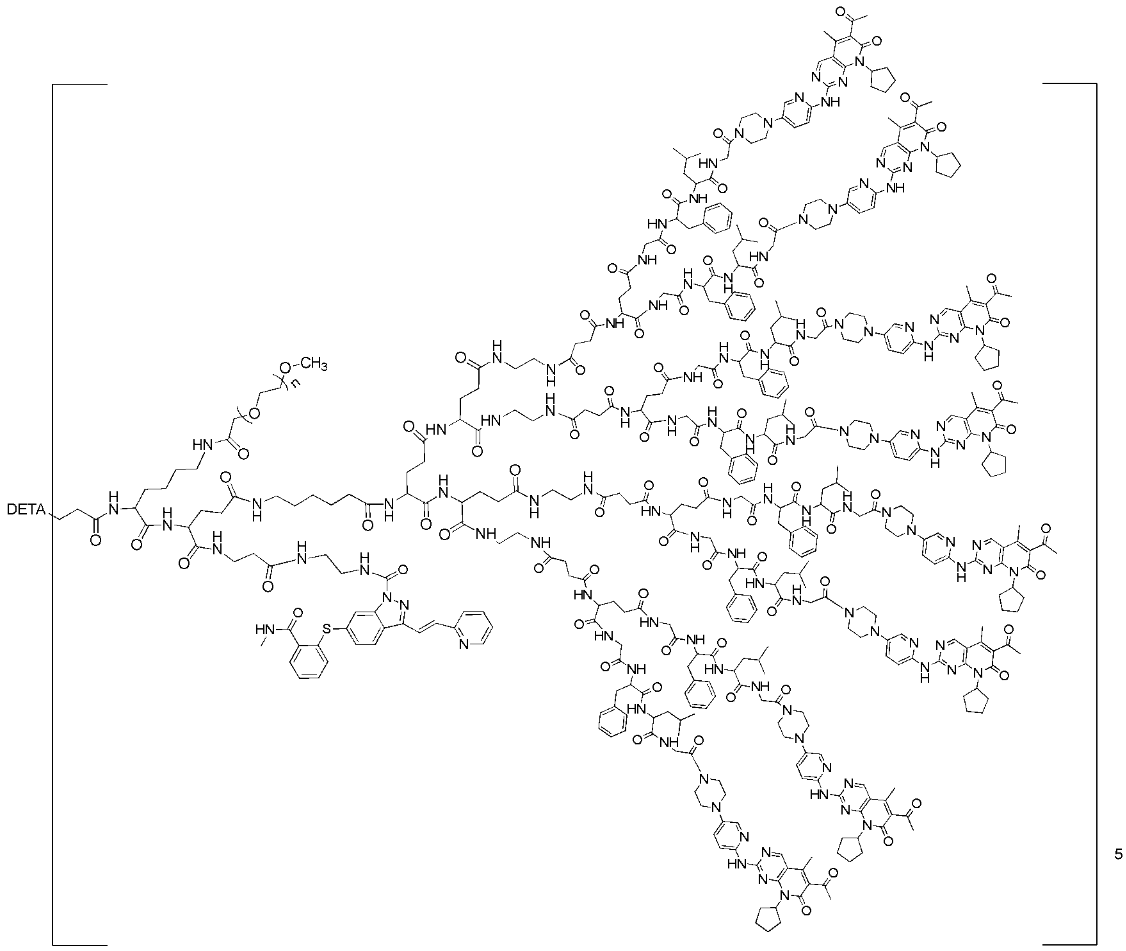 |
| | each polyethylene glycol fragment has a number-average molecular weight of 5k |

(continued)

| | |
|---|---|
| 71--246 | 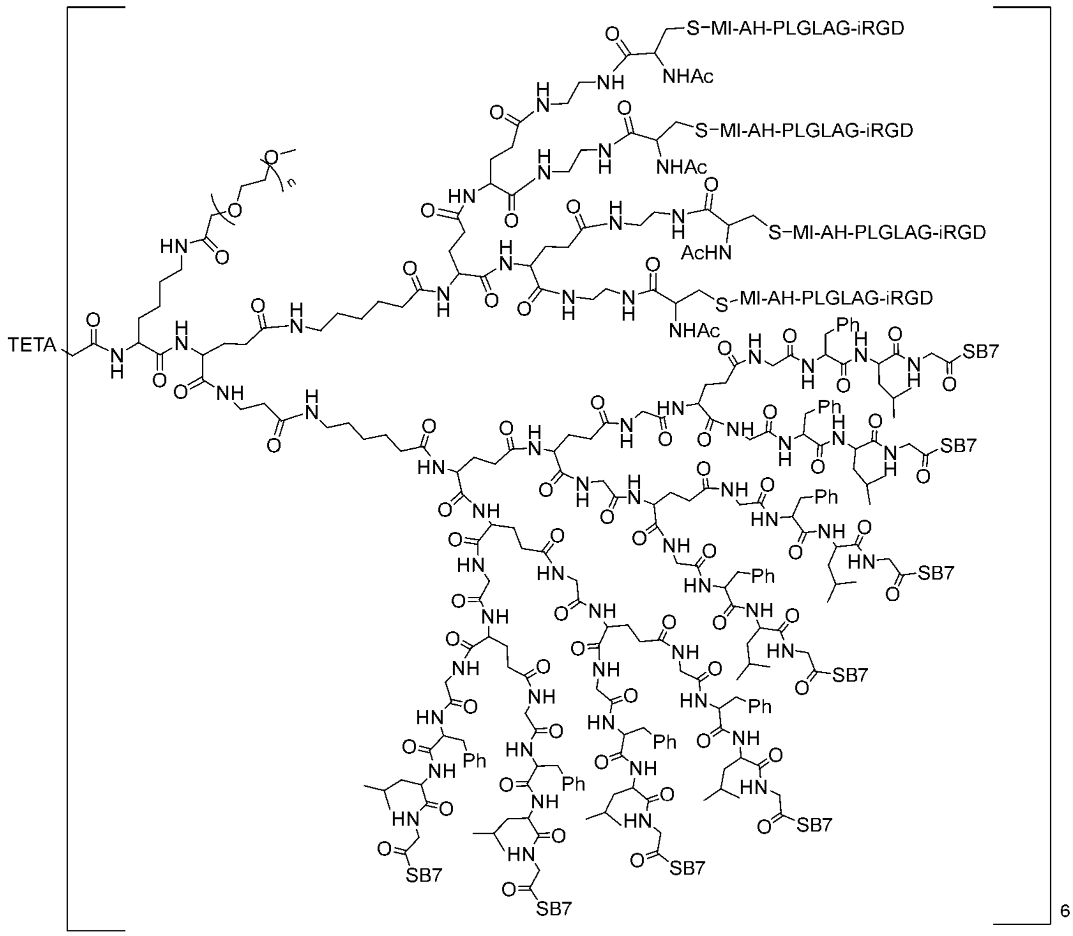 each polyethylene glycol fragment has a number-average molecular weight of 5k |
| 70--92 | 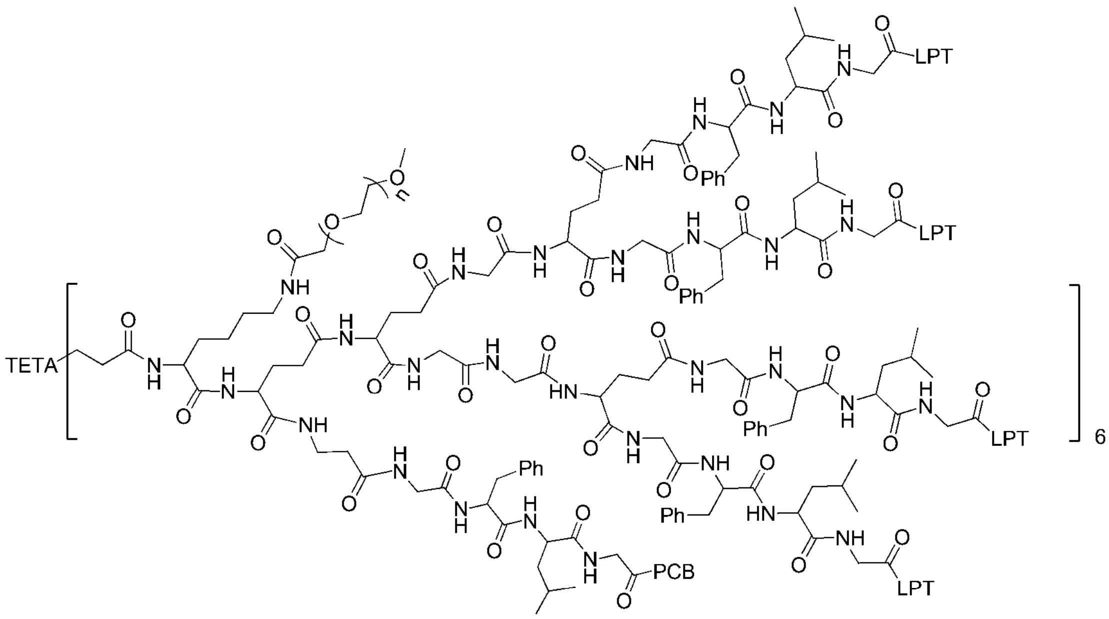 each polyethylene glycol fragment has a number-average molecular weight of 5k |

(continued)

| | |
|---|---|
| **77--231** | 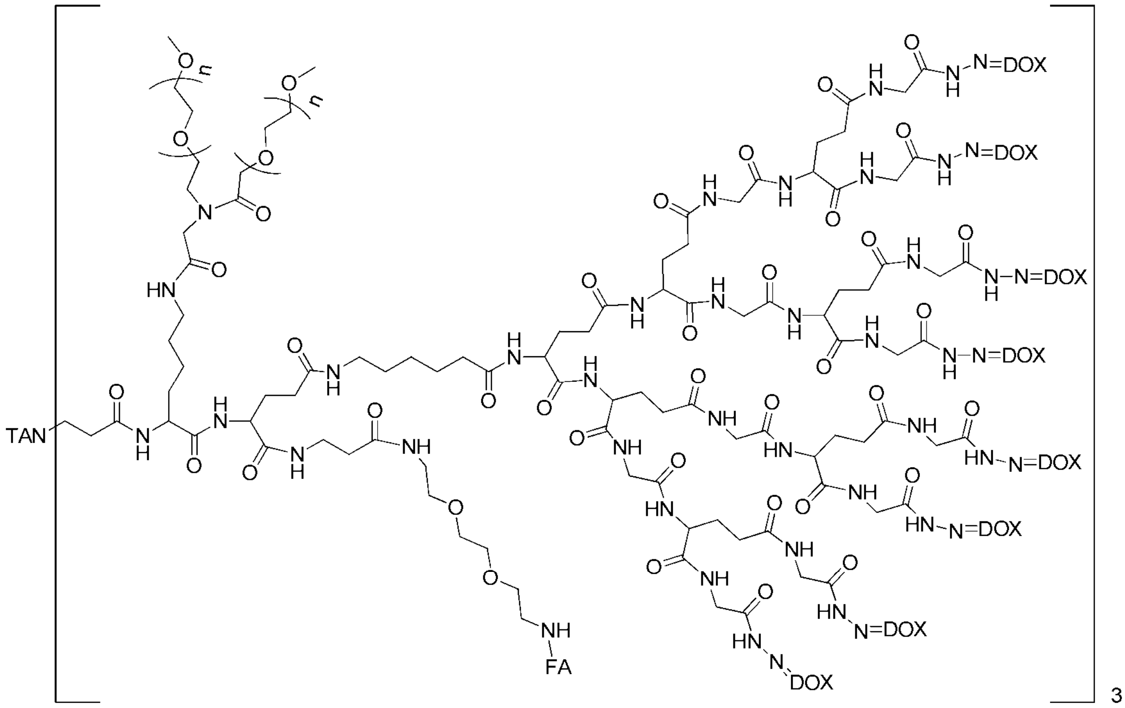<br>each polyethylene glycol fragment has a number-average molecular weight of 10k |
| **82--183** | 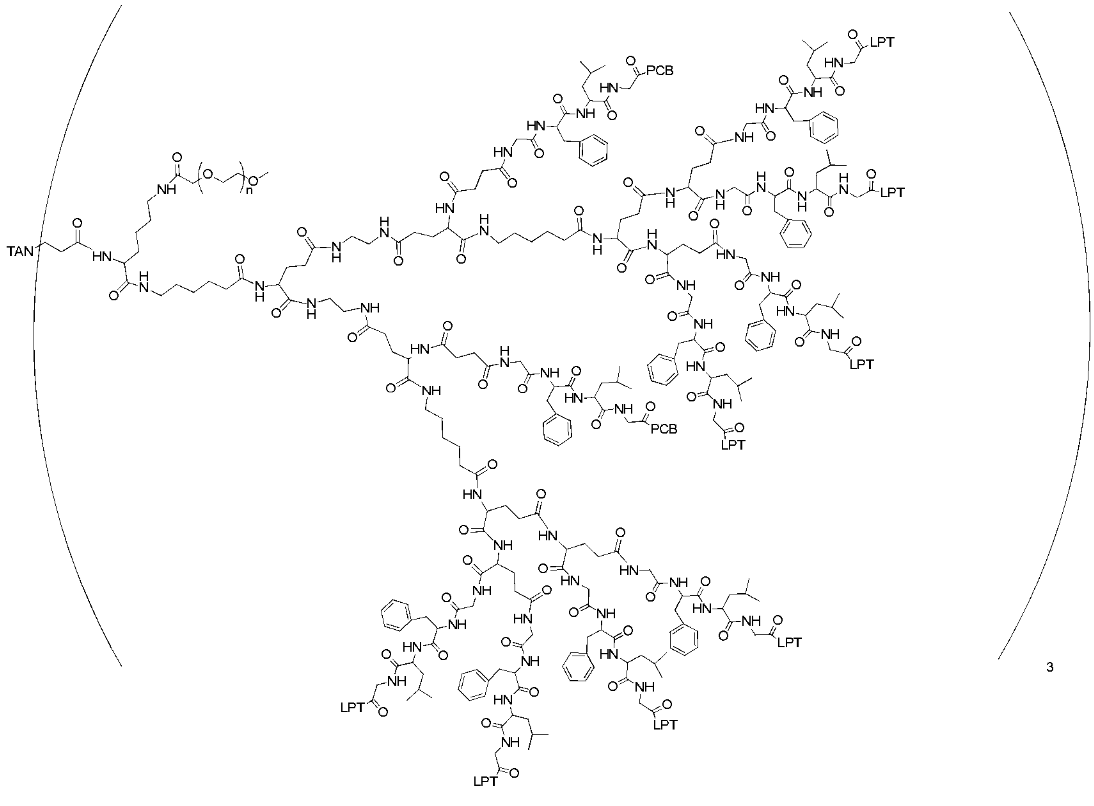<br>each polyethylene glycol fragment has a number-average molecular weight of 10k |

(continued)

| | |
|---|---|
| **82--87** | 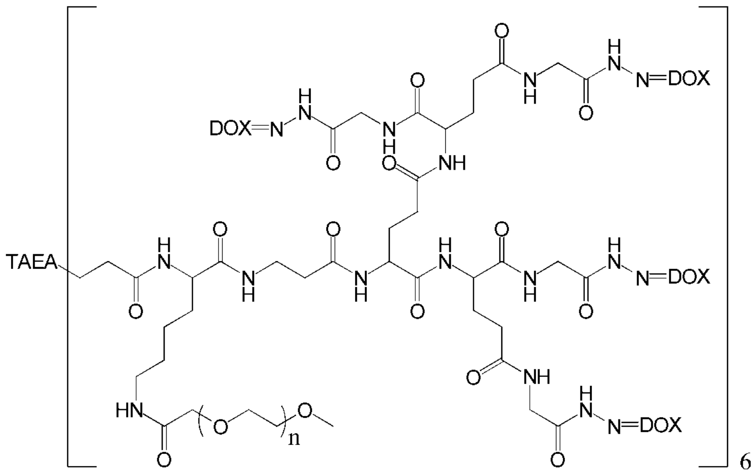<br>each polyethylene glycol fragment has a number-average molecular weight of 5k |
| **73--49** | 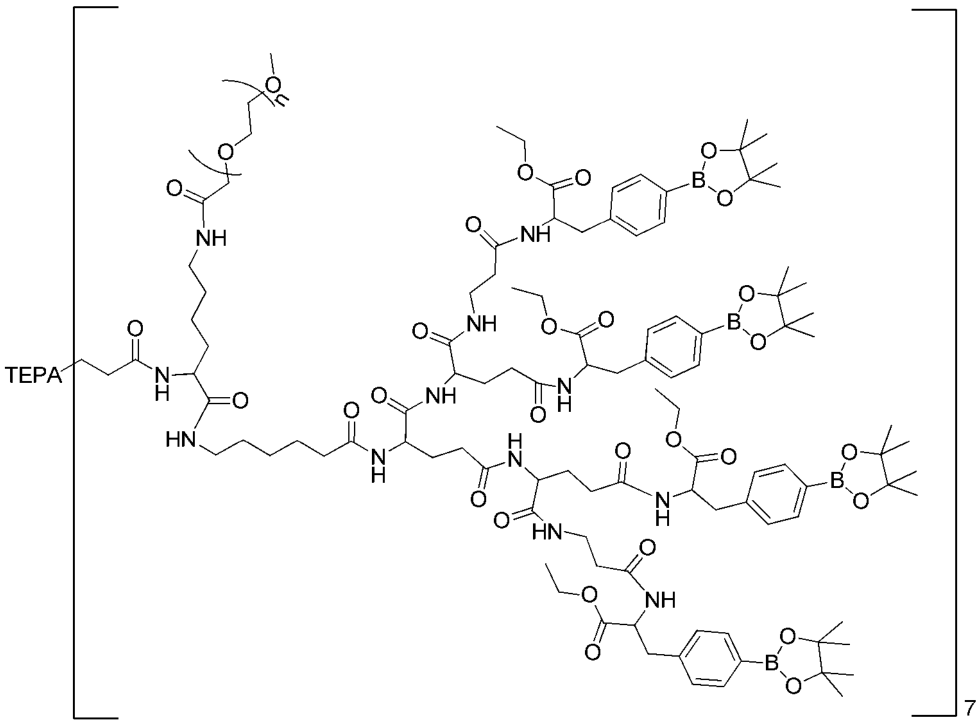<br>each polyethylene glycol fragment has a number-average molecular weight of 5k |

(continued)

| | |
|---|---|
| 82--226 | 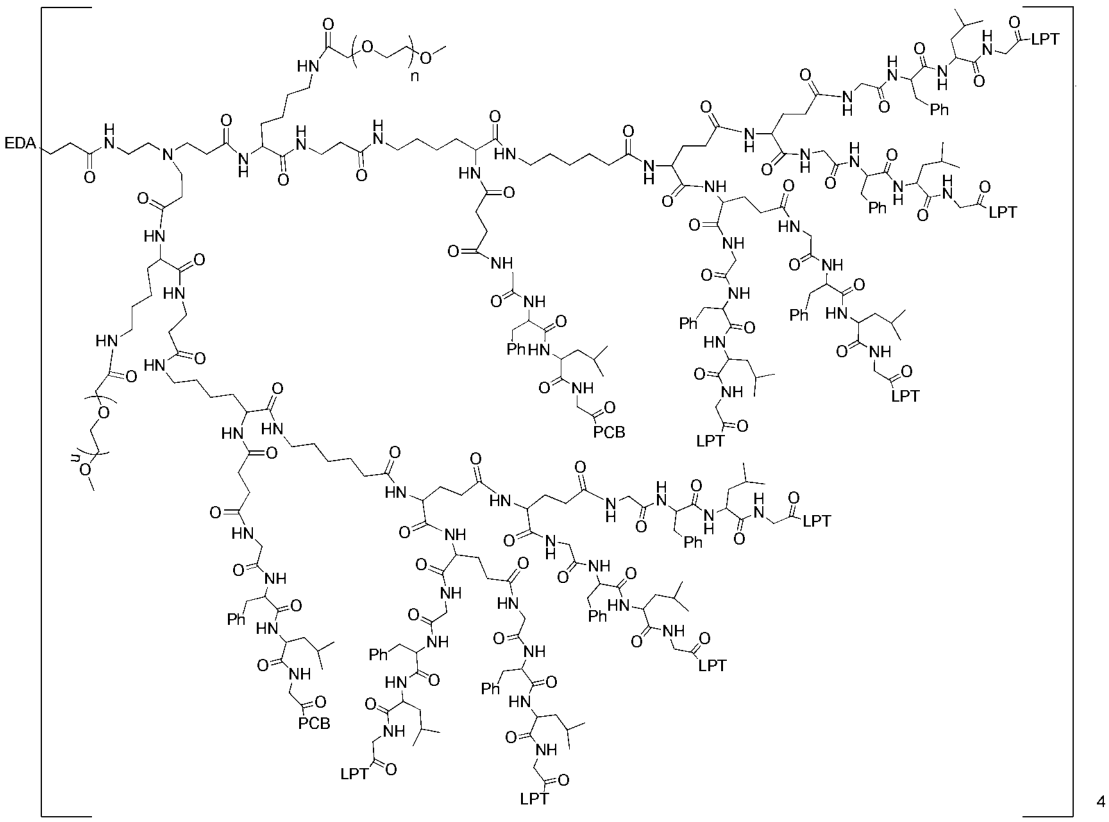<br>each polyethylene glycol fragment has a number-average molecular weight of 5k |
| 59--169 | 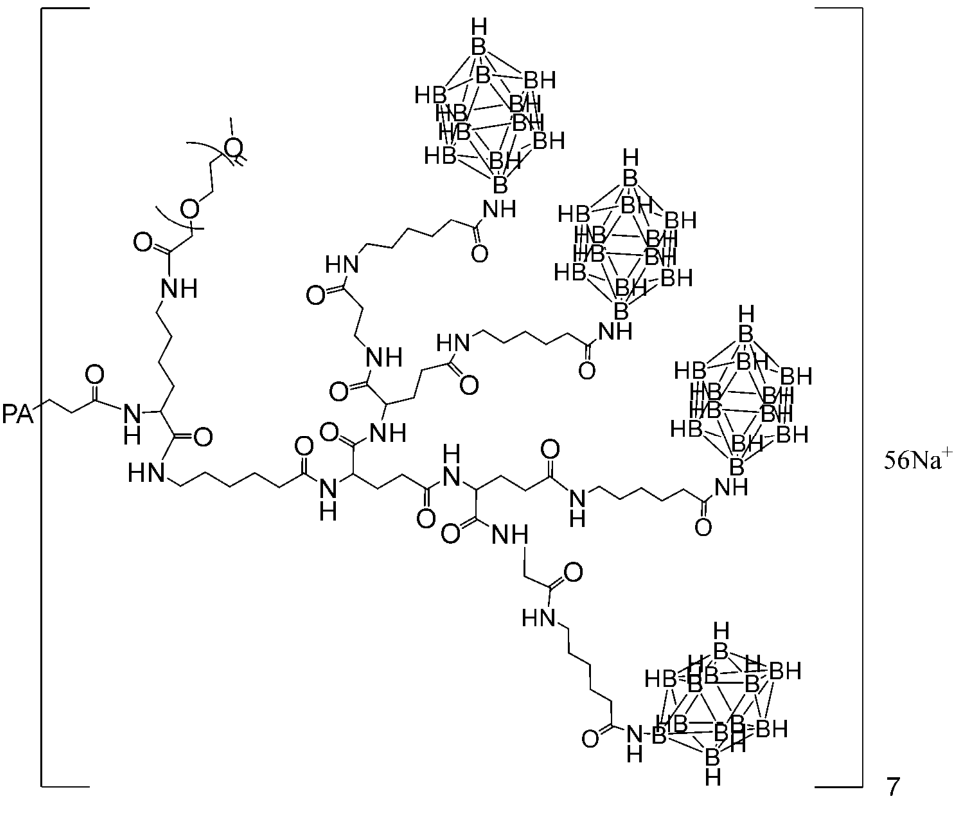<br>each polyethylene glycol fragment has a number-average molecular weight of 5k |

(continued)

| | |
|---|---|
| **67--237** | 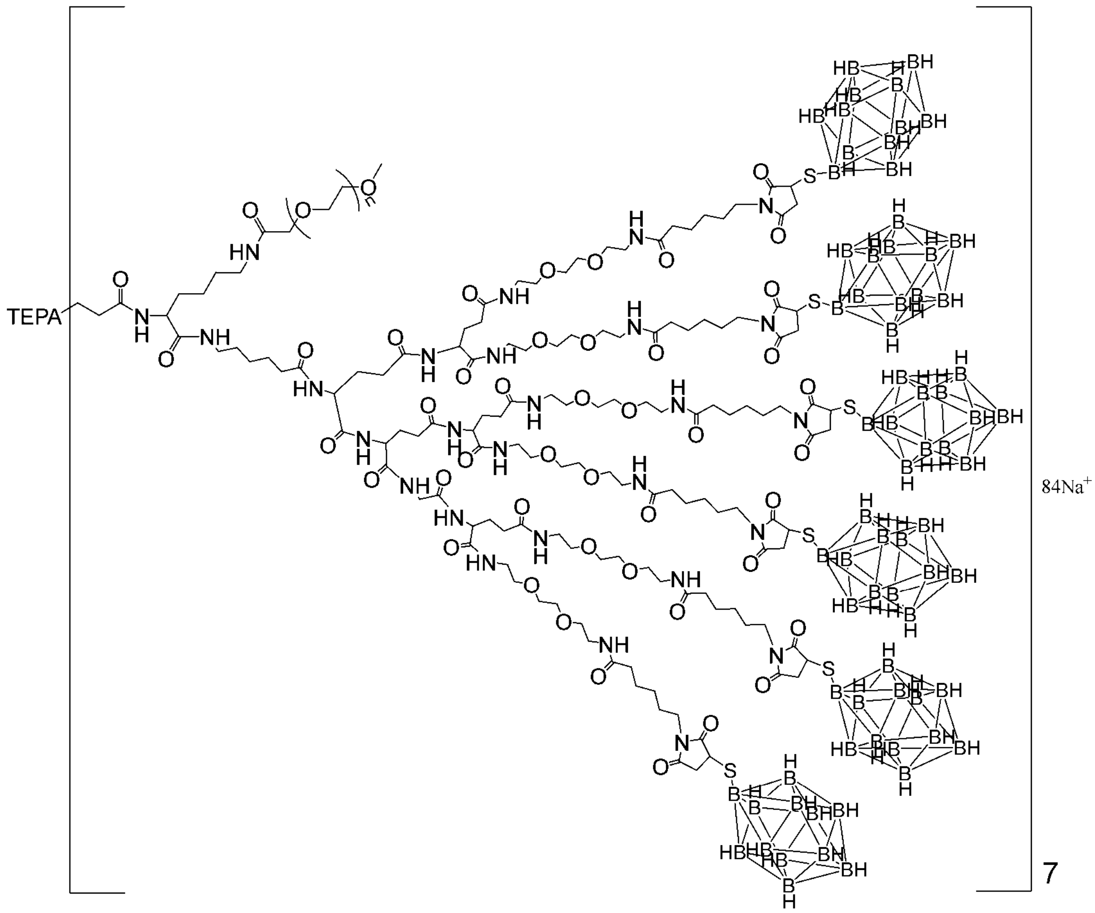 each polyethylene glycol fragment has a number-average molecular weight of 5k |
| **78--139** | 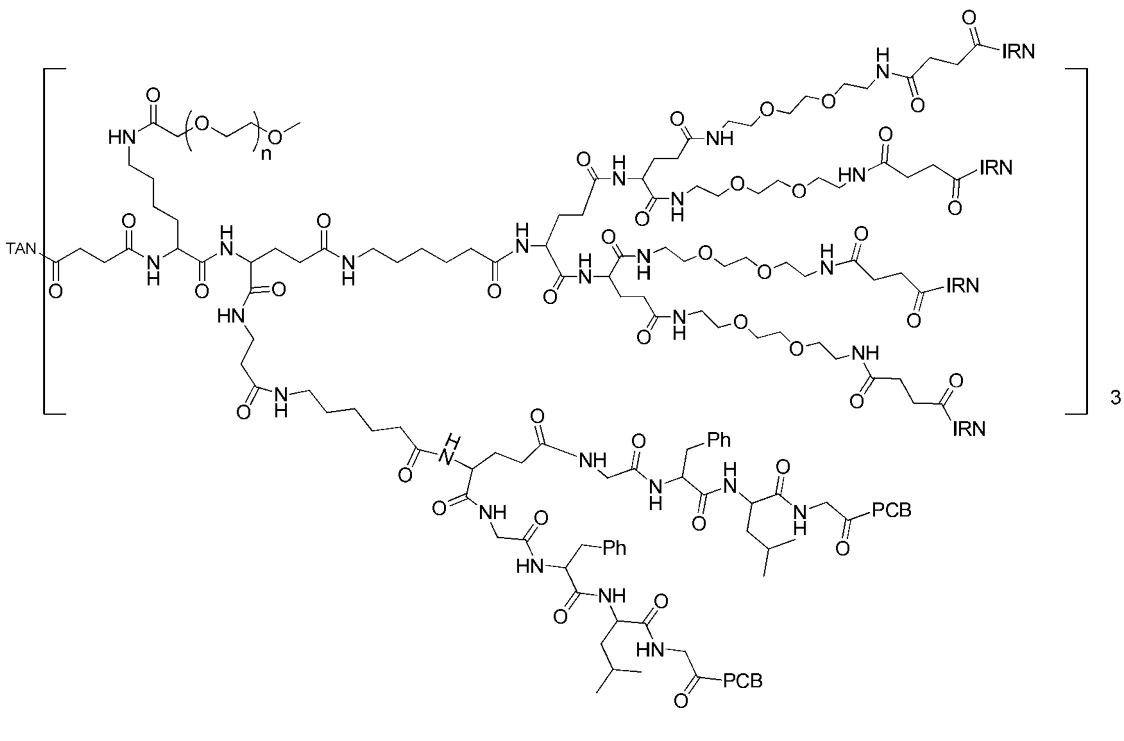 each polyethylene glycol fragment has a number-average molecular weight of 10k |

(continued)

| | |
|---|---|
| **82--178** | 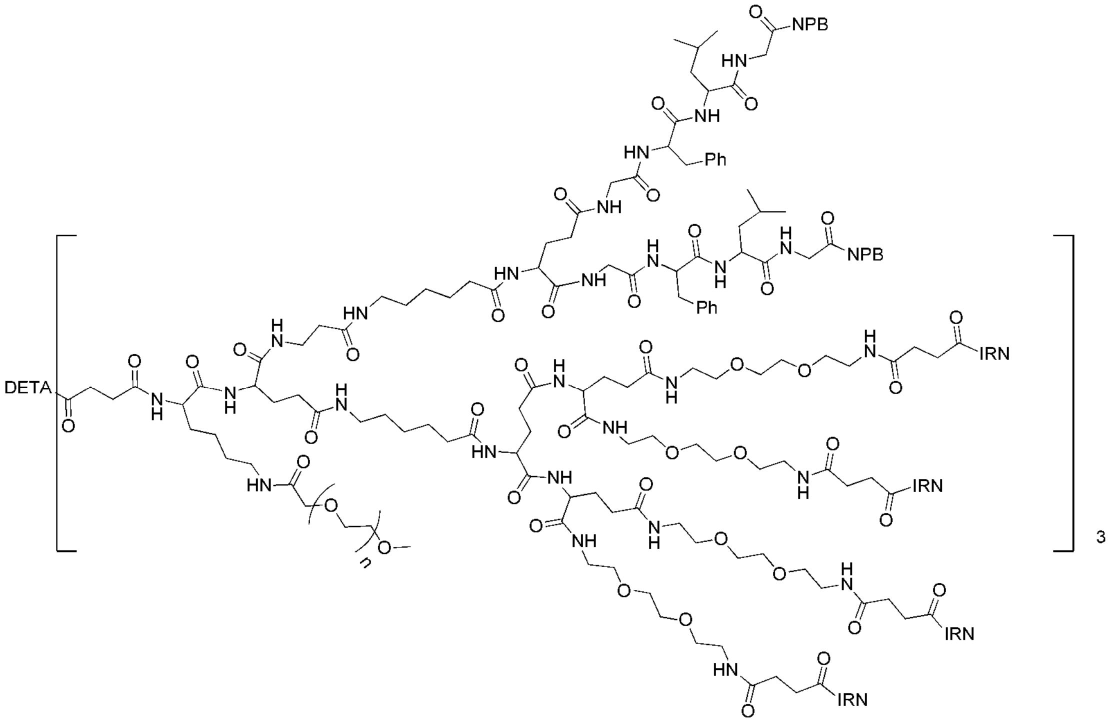<br>each polyethylene glycol fragment has a number-average molecular weight of 10k |
| **70--294** | 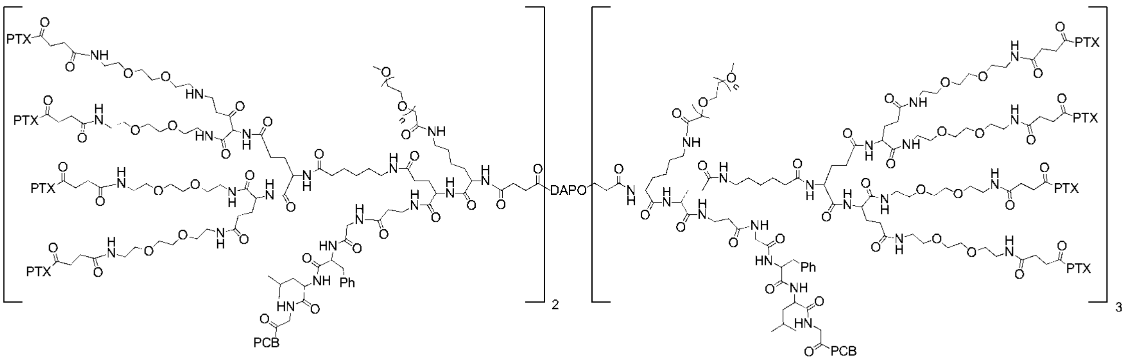<br>each polyethylene glycol fragment has a number-average molecular weight of 5k |

(continued)

| | |
|---|---|
| 81--176 | 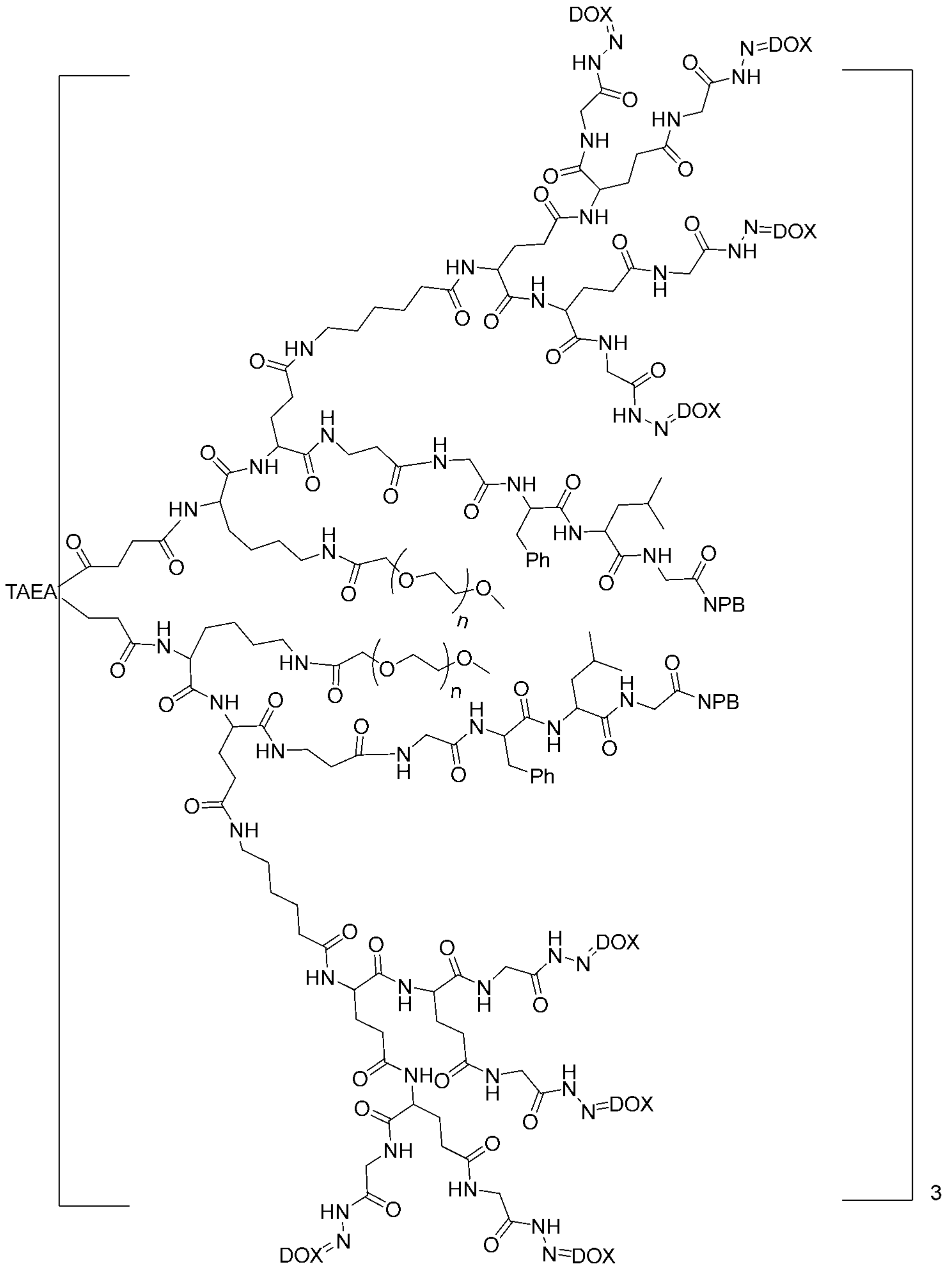<br>each polyethylene glycol fragment has a number-average molecular weight of 5k |

(continued)

| | |
|---|---|
| **85--71** | 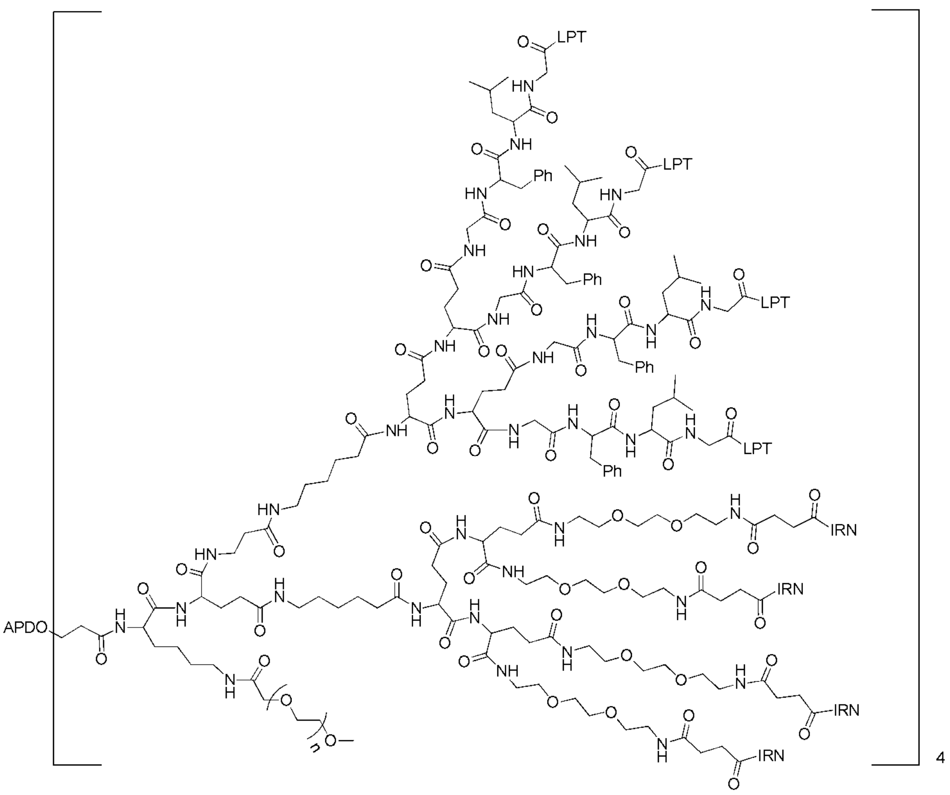<br>each polyethylene glycol fragment has a number-average molecular weight of 5k |
| **75--196** | 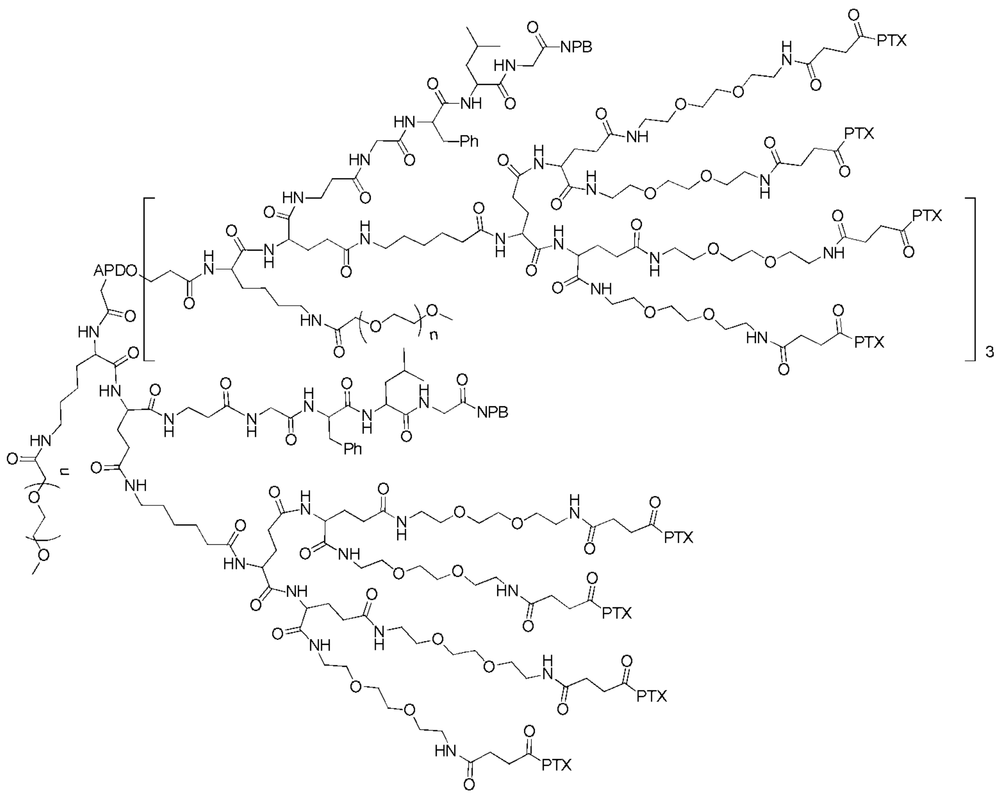<br>each polyethylene glycol fragment has a number-average molecular weight of 10k |

(continued)

| | |
|---|---|
| **86-2** | 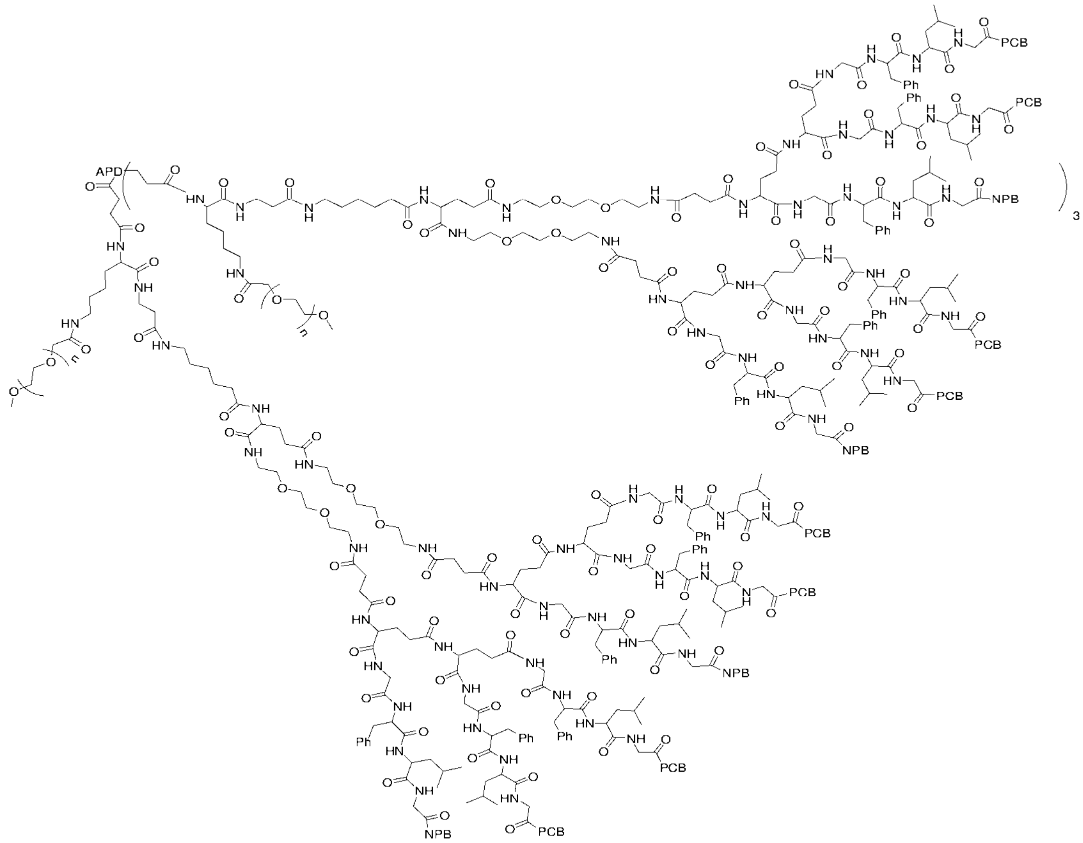 each polyethylene glycol fragment has a number-average molecular weight of 10k |
| **84--106** | 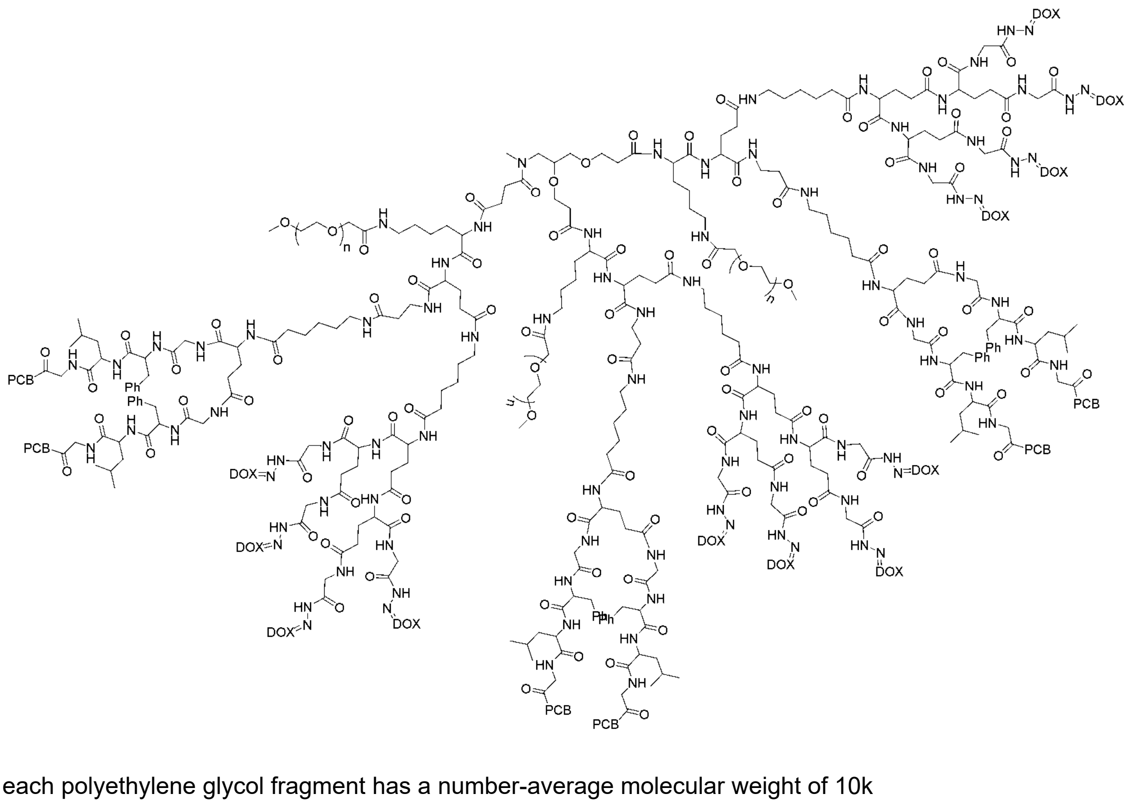 each polyethylene glycol fragment has a number-average molecular weight of 10k |

(continued)

| | |
|---|---|
| 76--207 | 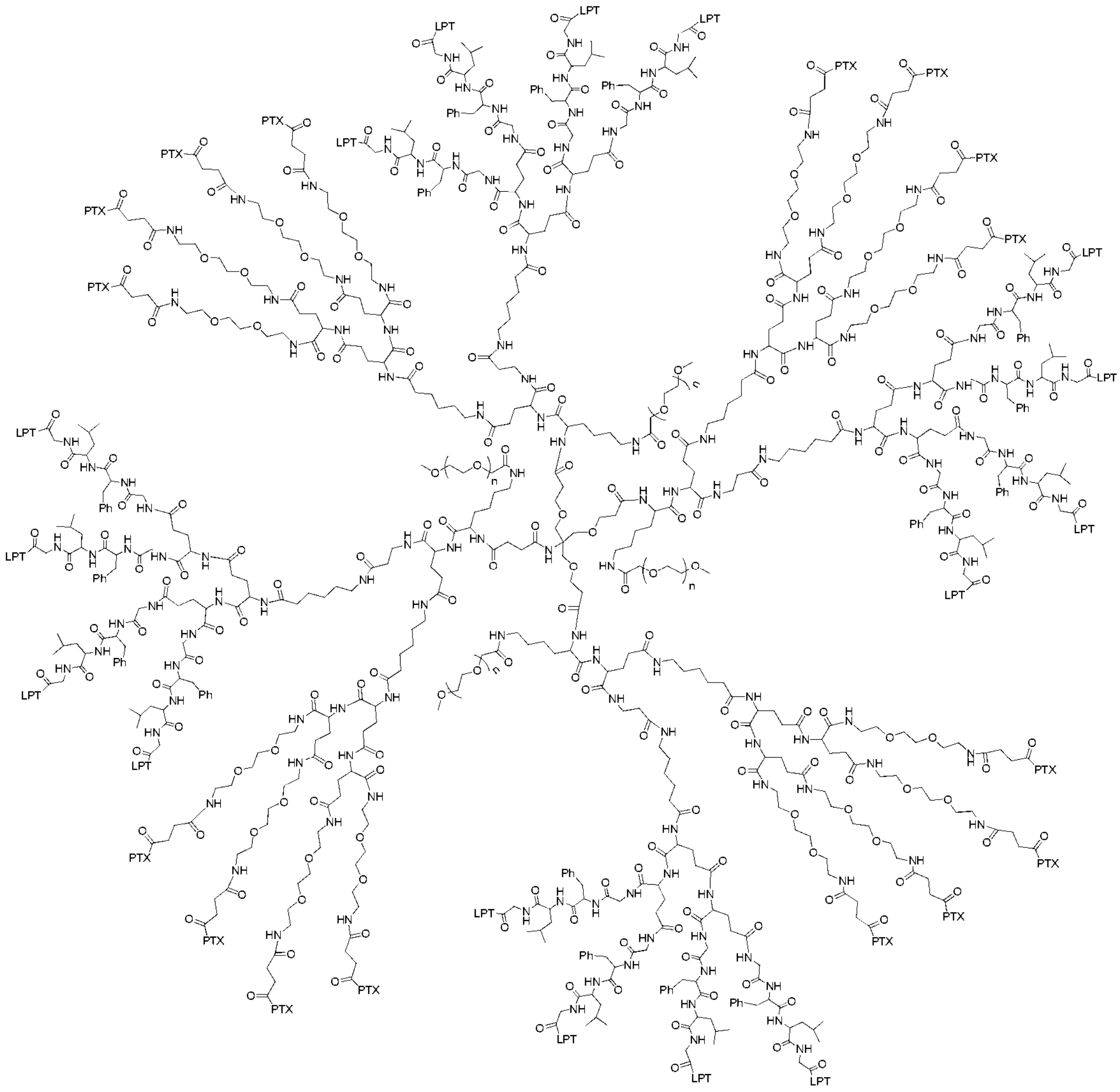 each polyethylene glycol fragment has a number-average molecular weight of 10k |

(continued)

| | |
|---|---|
| **82-- 152** | 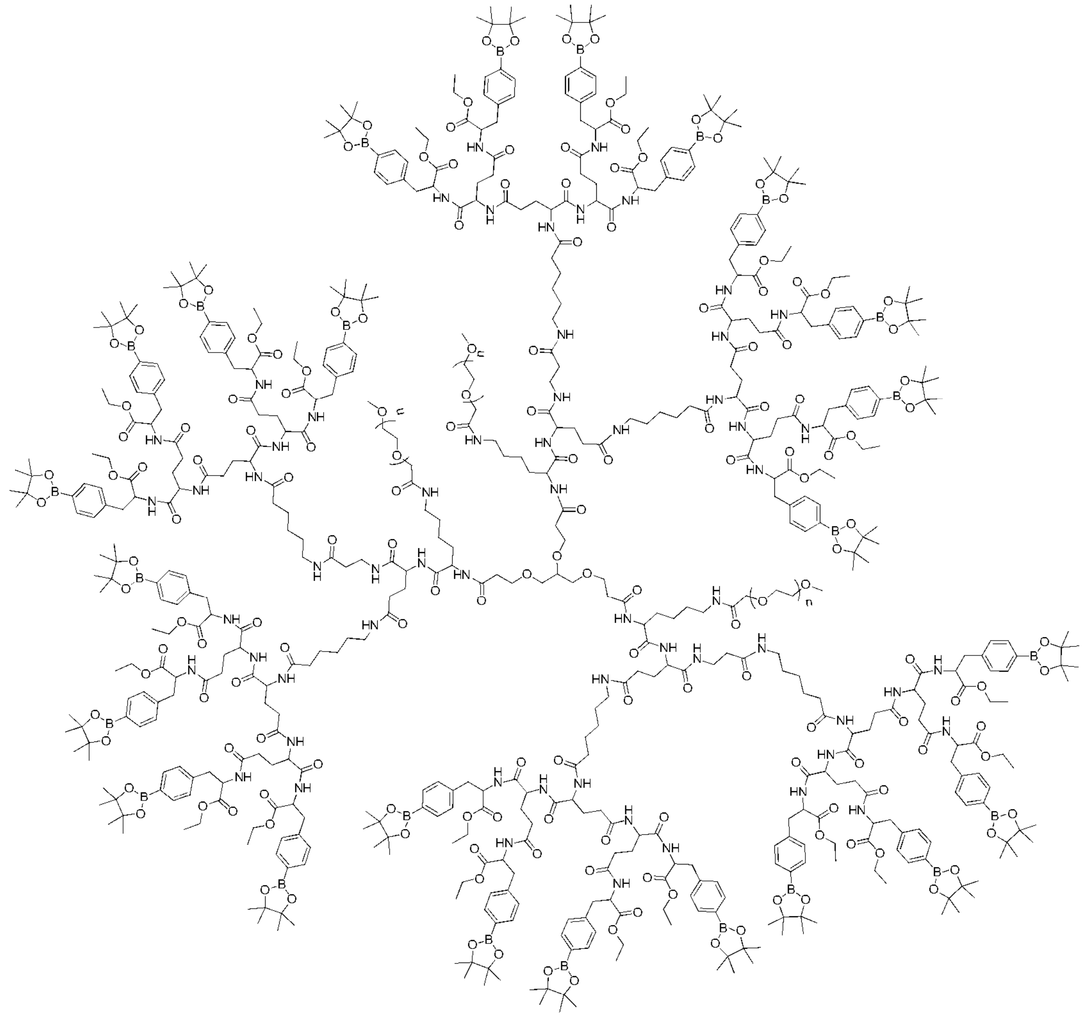 each polyethylene glycol fragment has a number-average molecular weight of 10k |

(continued)

| | |
|---|---|
| 82--132 | 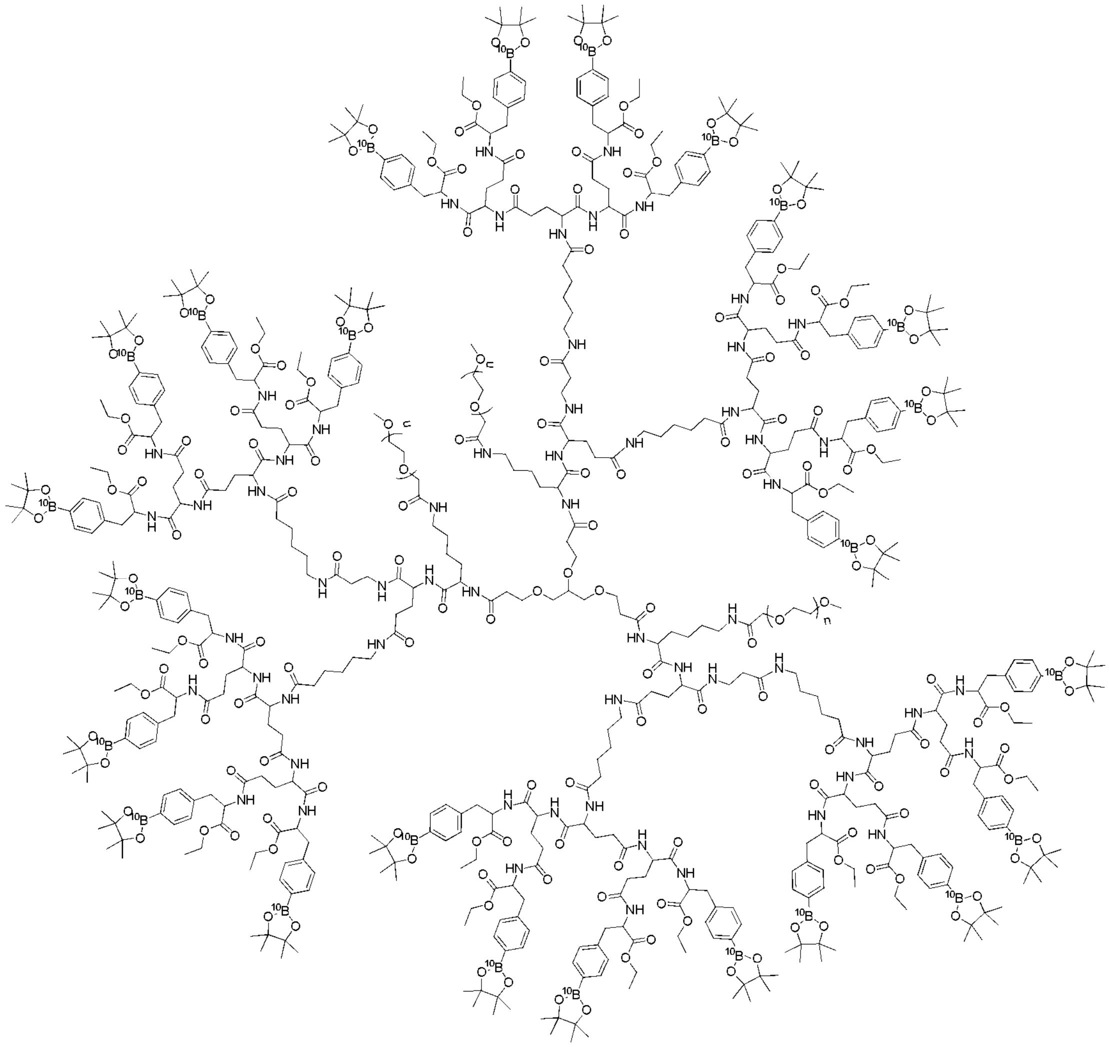 each polyethylene glycol fragment has a number-average molecular weight of 10k |

(continued)

| | |
|---|---|
| **82--208** | 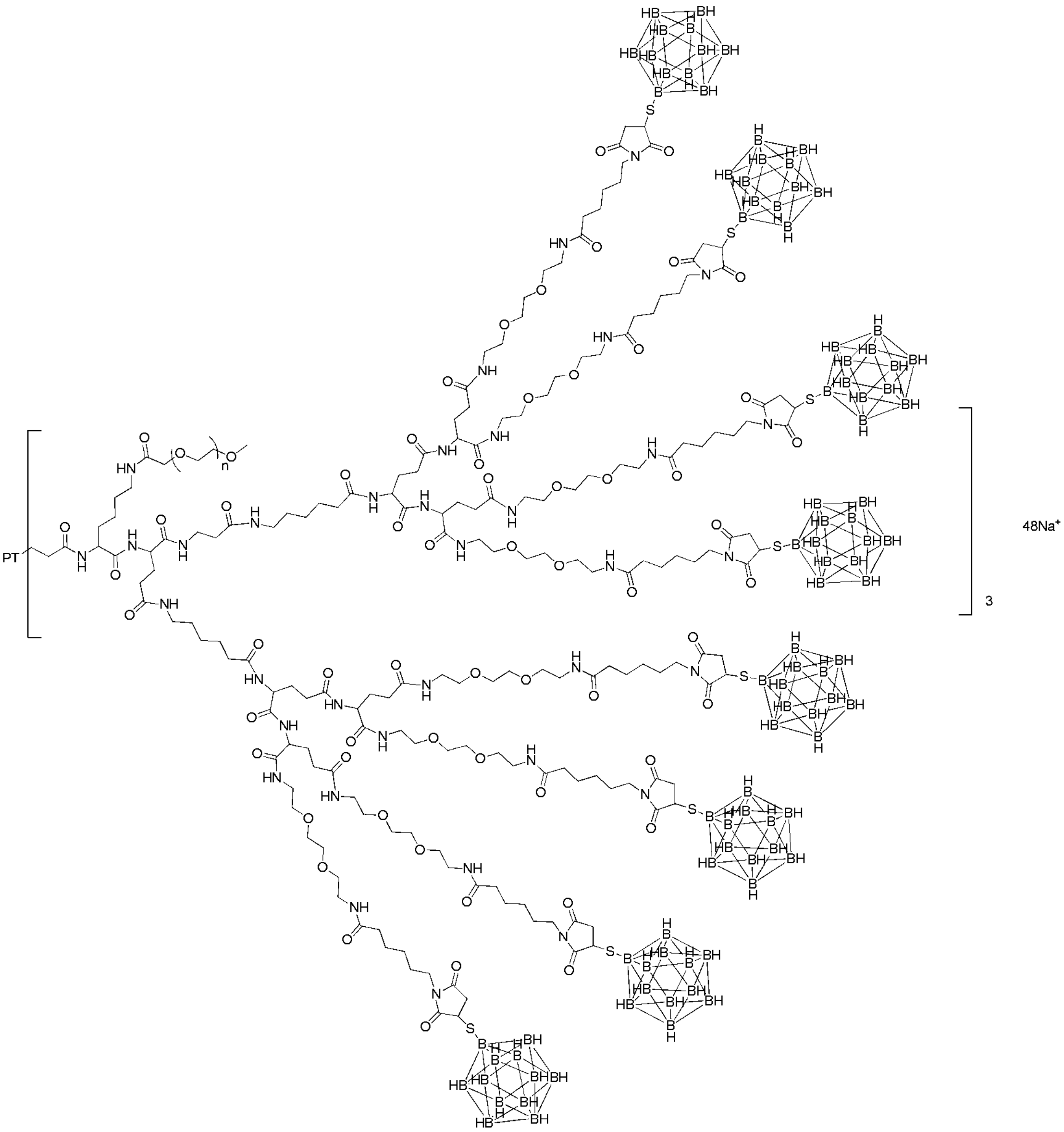 |
| | each polyethylene glycol fragment has a number-average molecular weight of 10k |

(continued)

| | |
|---|---|
| **82--207** | 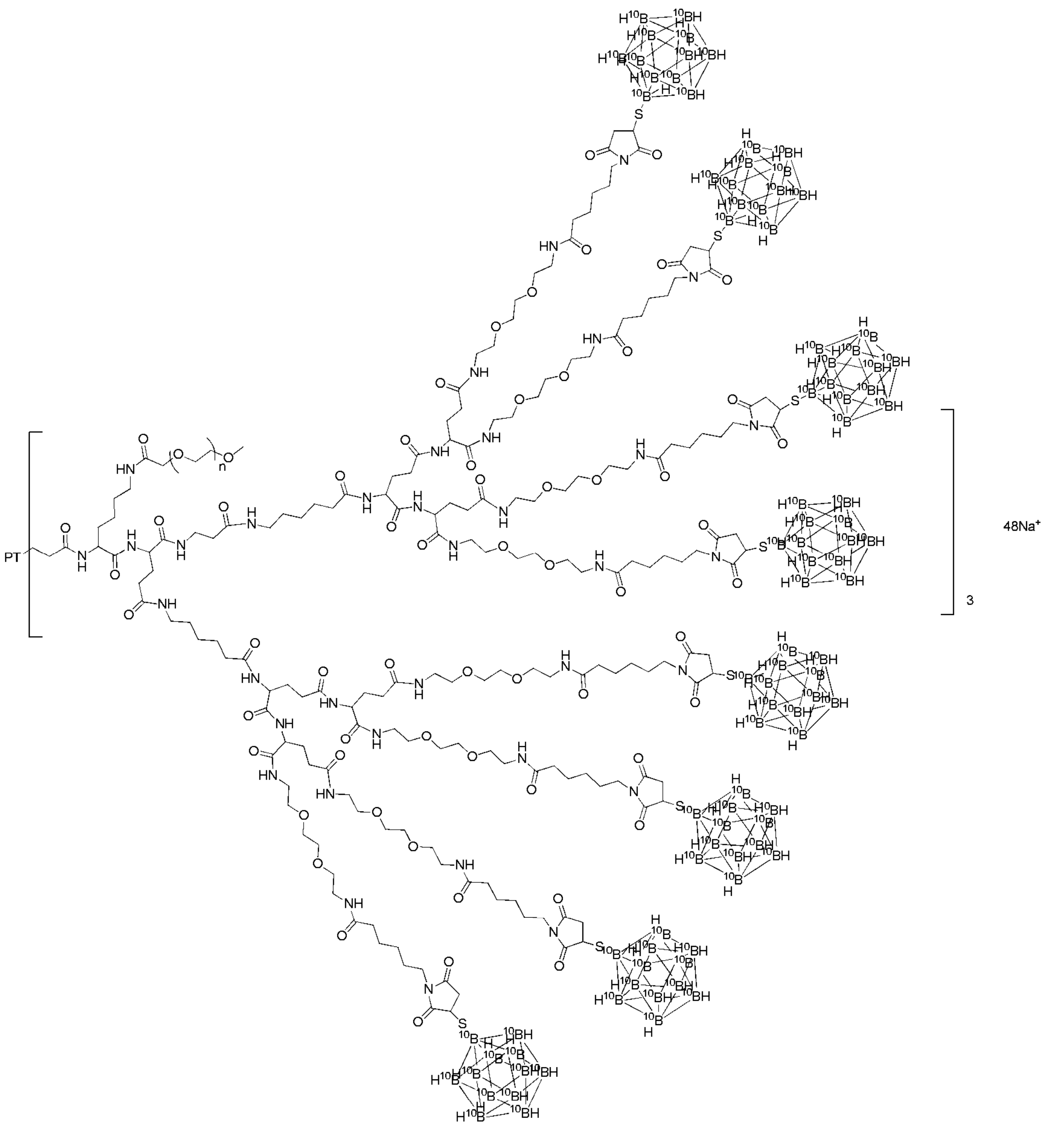 each polyethylene glycol fragment has a number-average molecular weight of 10k |

(continued)

| | |
|---|---|
| 78--127 | 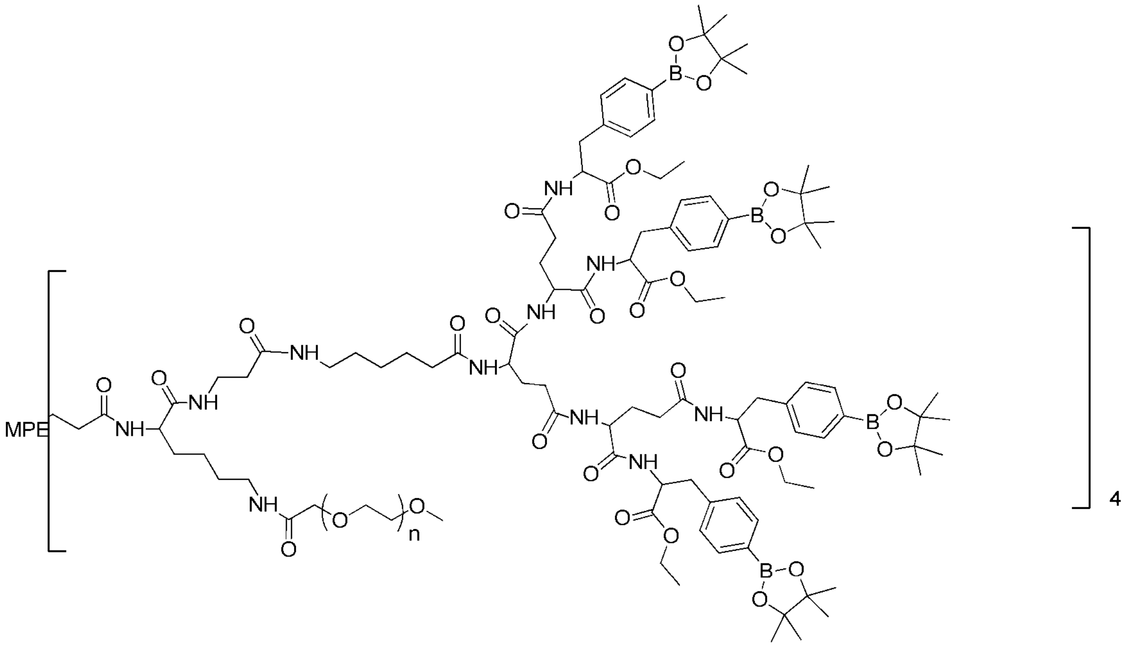 each polyethylene glycol fragment has a number-average molecular weight of 10k |
| 78--89 | 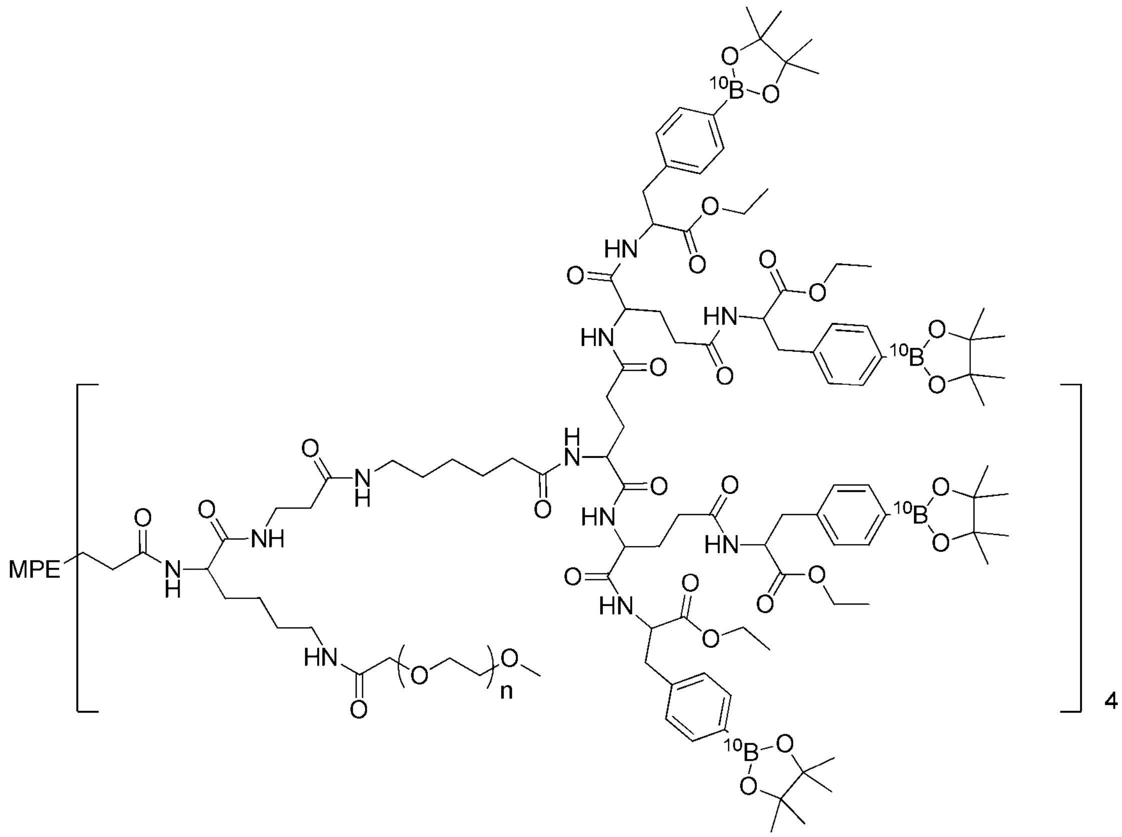 each polyethylene glycol fragment has a number-average molecular weight of 10k |

(continued)

| | |
|---|---|
| **78--152** | 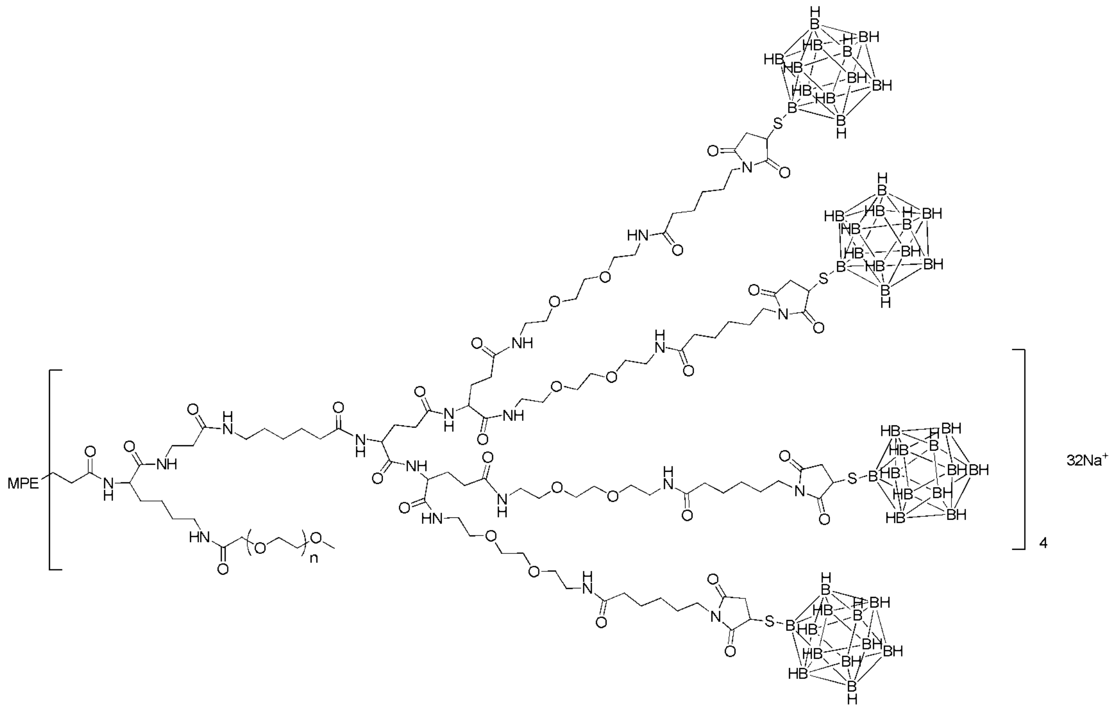<br>each polyethylene glycol fragment has a number-average molecular weight of 10k |
| **78--151** | 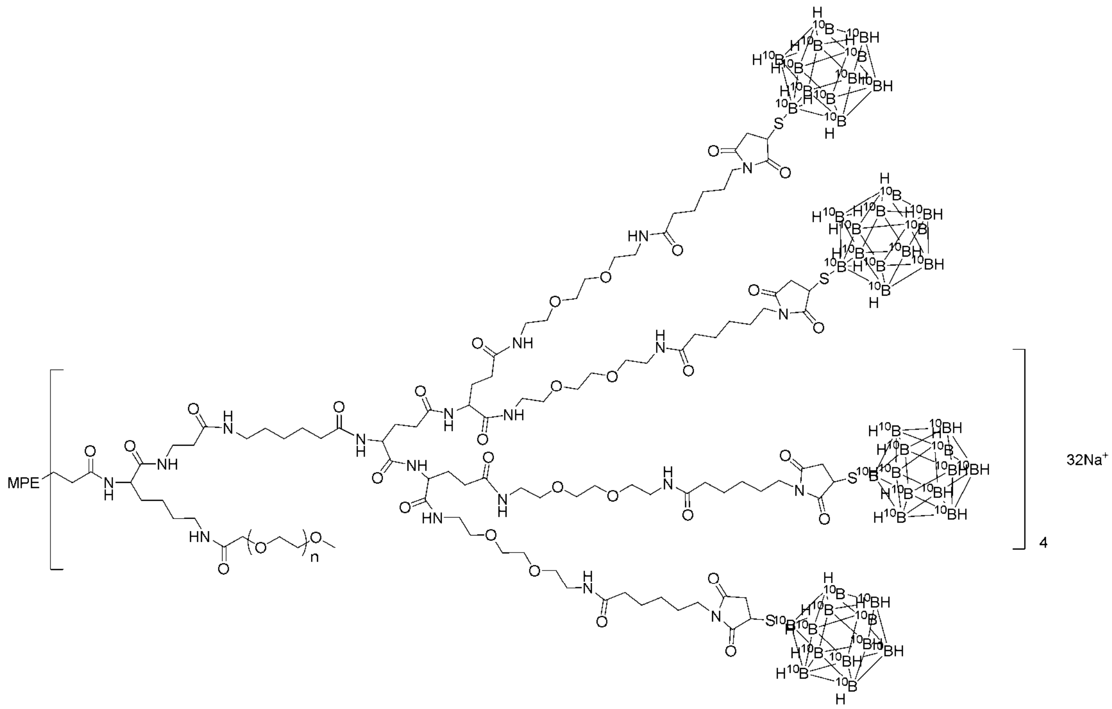<br>each polyethylene glycol fragment has a number-average molecular weight of 10k |

(continued)

| | |
|---|---|
| **72--183** | 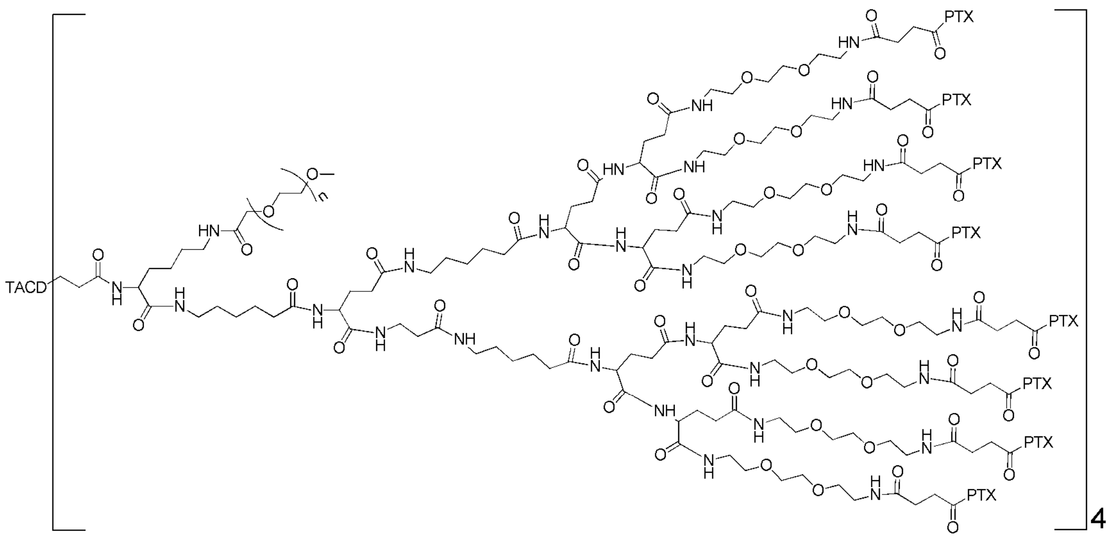 each polyethylene glycol fragment has a number-average molecular weight of 10k |

6. A compound represented by formula II or a pharmaceutically acceptable salt thereof, wherein:

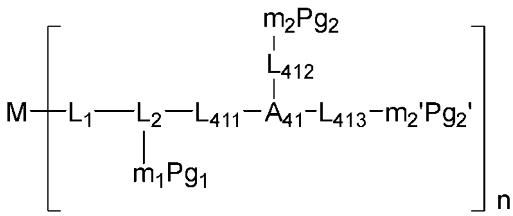

II

wherein, each of $Pg_1$ and $Pg_2$ is independently hydrogen or selected from the group consisting of amino protecting groups and carboxyl protecting groups; the amino protecting group is selected from the group consisting of alkoxycarbonyl-based protecting group, such as Boc, Fmoc, Cbz, or Teoc; and the carboxyl protecting group is selected from the group consisting of ester-based protecting group, such as methyl ester, ethyl ester, tert-butyl ester, allyl ester, or benzyl ester;
each of $m_1$ and $m_2$ is independently selected from the group consisting of 1, 2, 3, 4, 5, 6, 7, 8, 9, 10, 11, 12, 13, 14, 15, 16, 17, 18, 19, and 20;
the remaining groups are as defined in any one of claims 1 to 5.

7. The compound or pharmaceutically acceptable salt thereof according to claim 6, wherein the compound is selected from the group consisting of the following compounds:

| | |
|---|---|
| **71-56** | 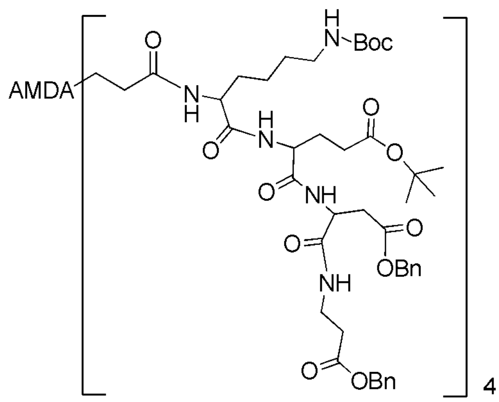 |
| **71-77** | 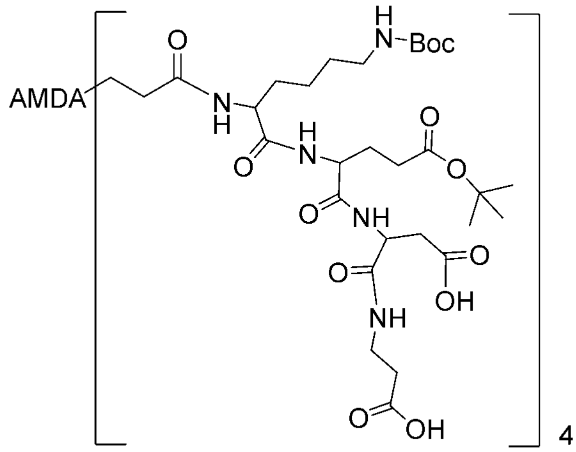 |
| **38-234** | 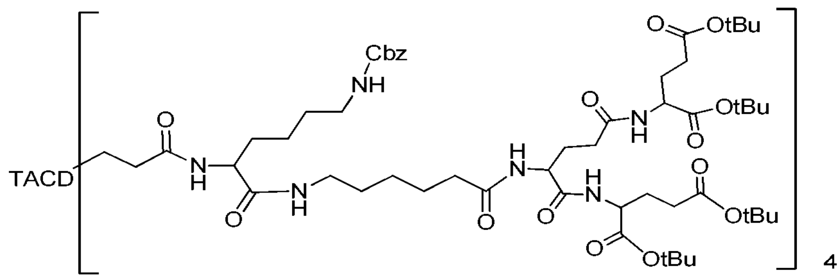 |
| **38-258** | 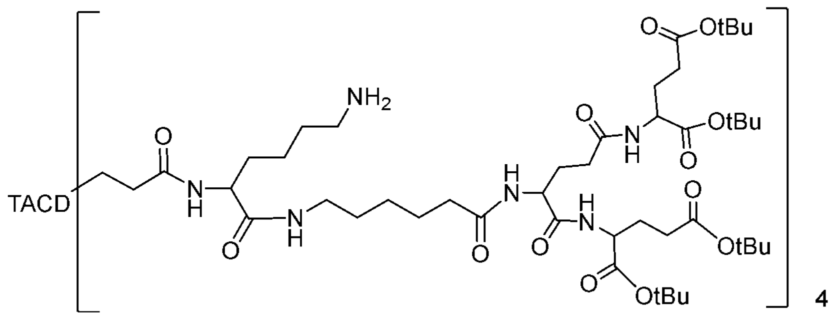 |

(continued)

| | |
|---|---|
| **67-113** | 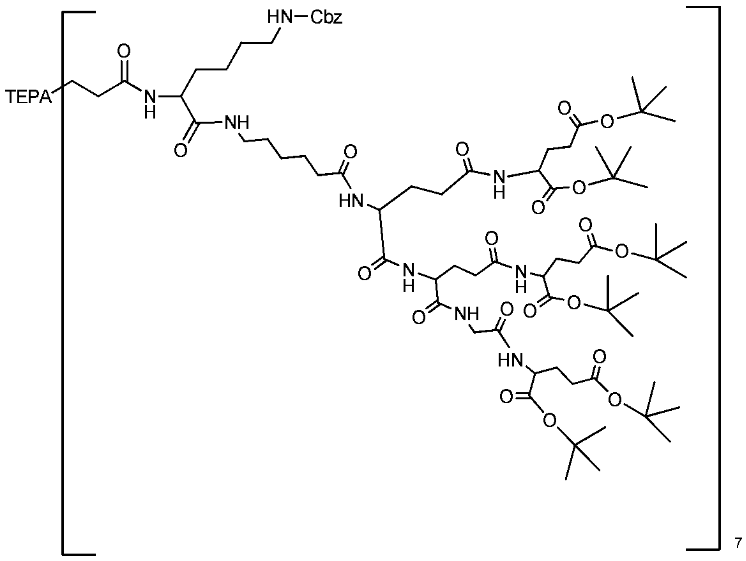 |
| **67-114** | 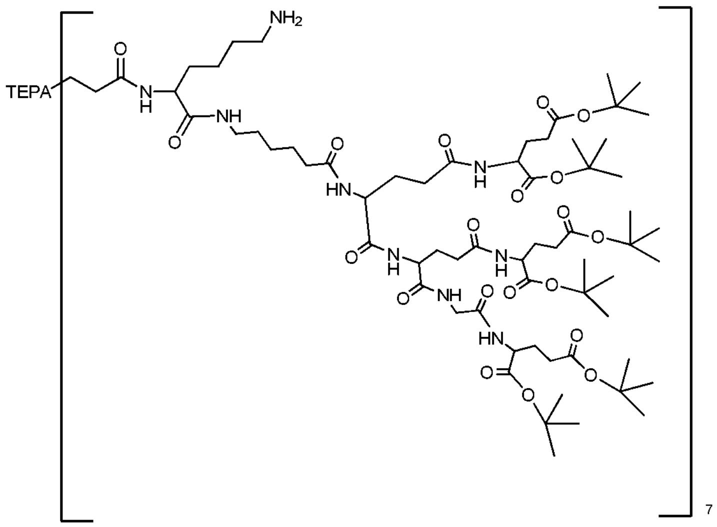 |
| **81-22** | 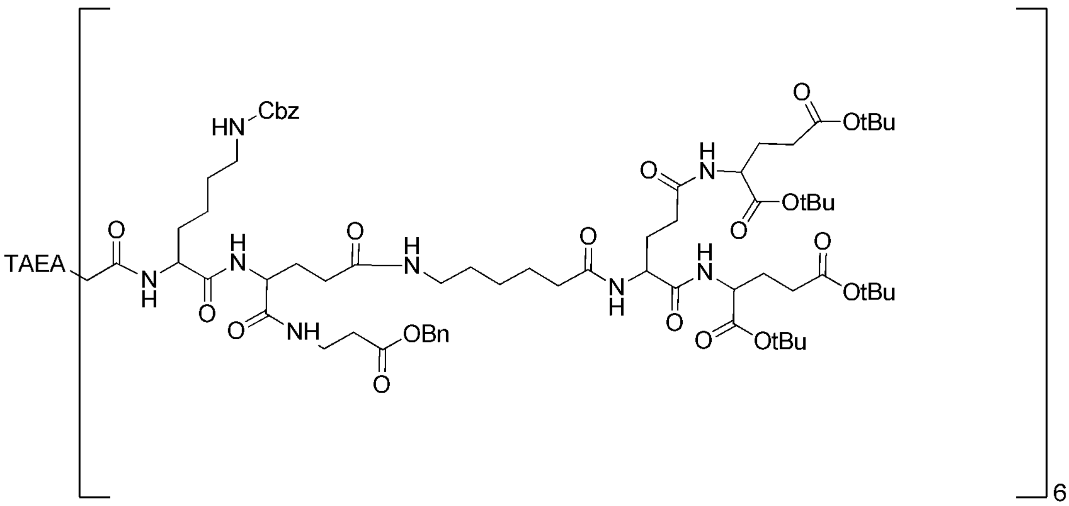 |

(continued)

| | |
|---|---|
| **81-30** | 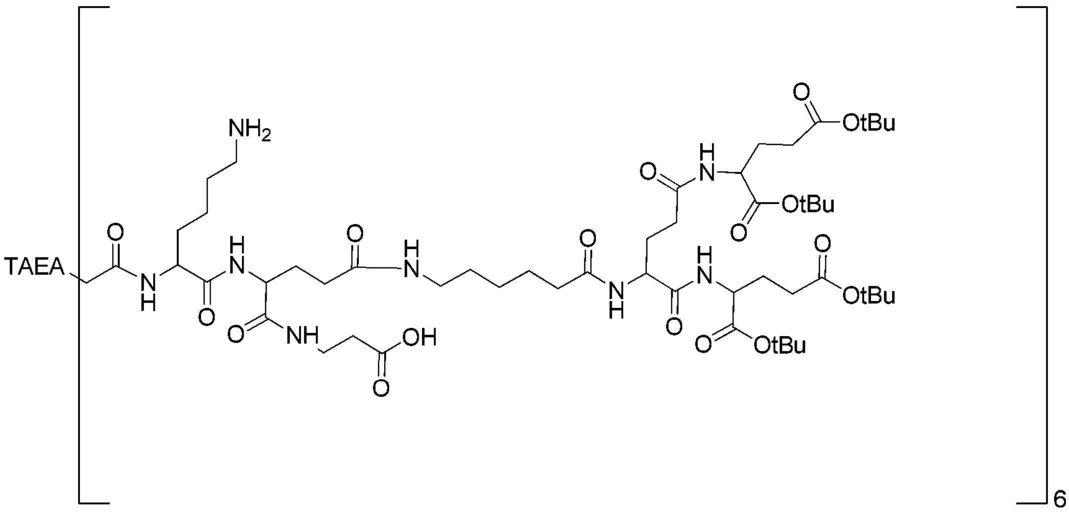 |
| **50-185** | 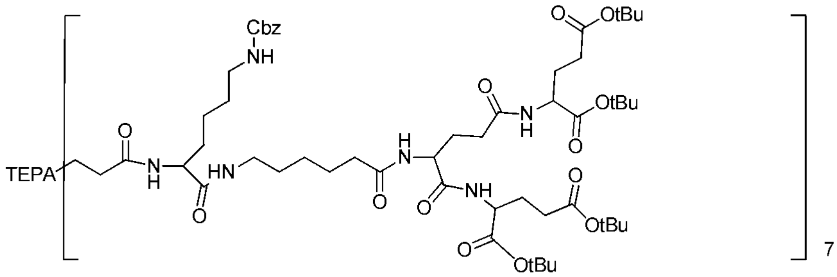 |
| **50-196** | 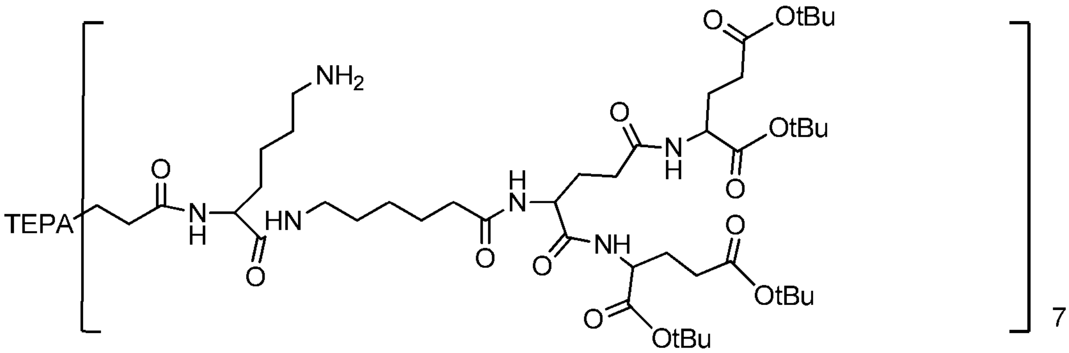 |
| **69-54** | 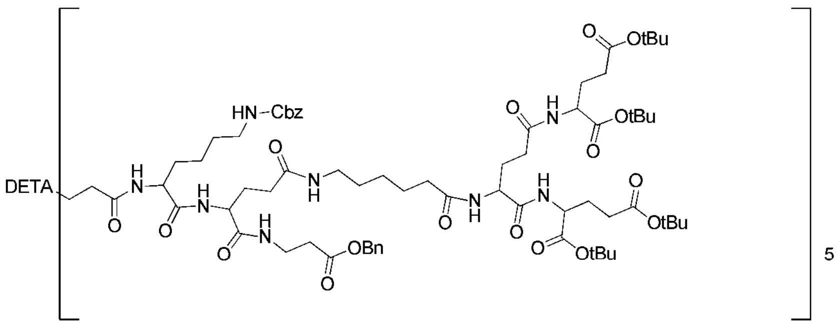 |

(continued)

| | |
|---|---|
| **69-60** | 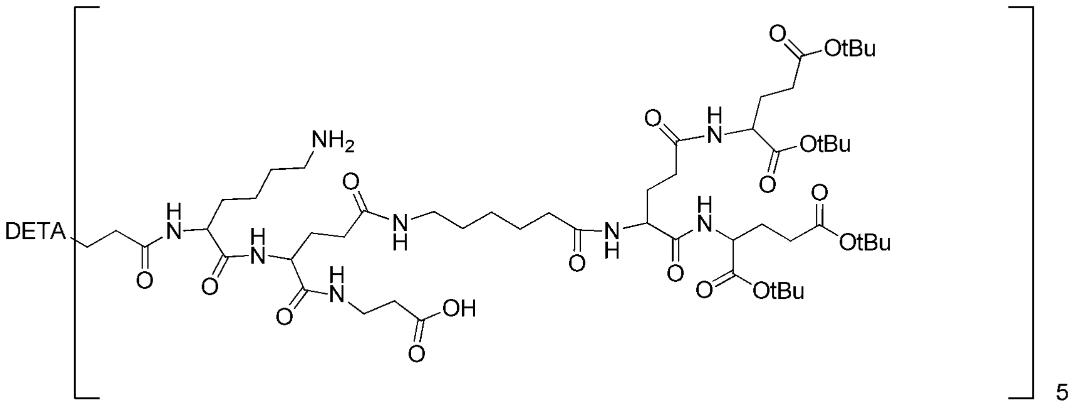 |
| **70-81** | 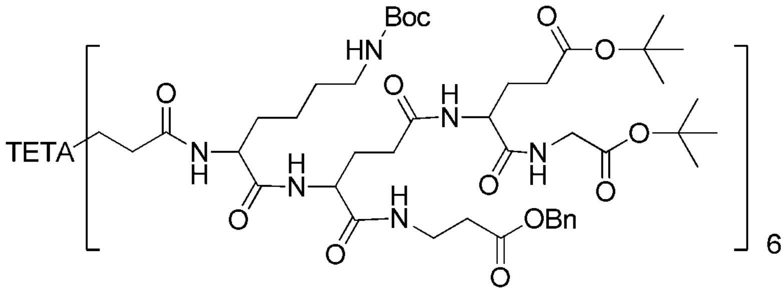 |
| **70-84** | 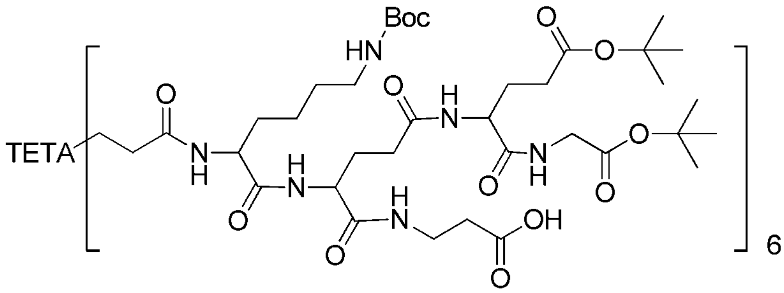 |
| **68-127** | 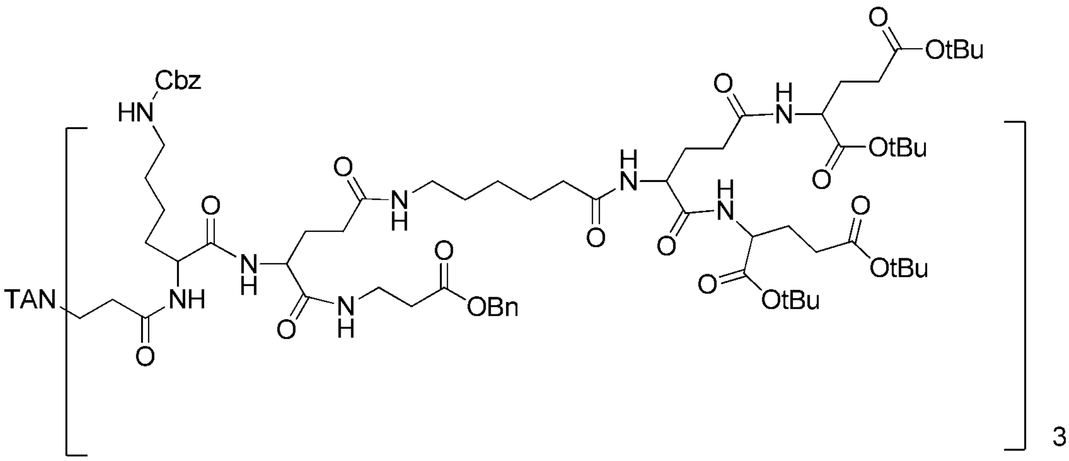 |

(continued)

| | |
|---|---|
| **68-130** | 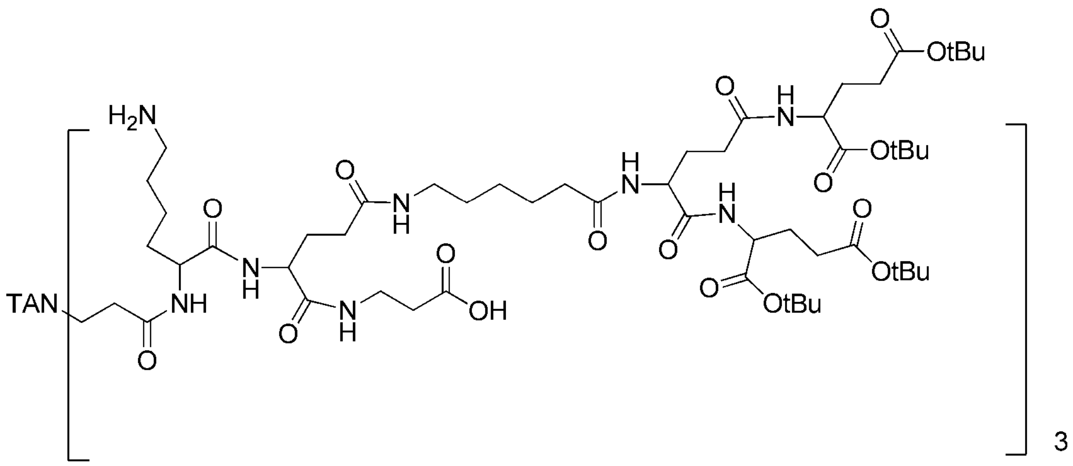<br>I-16 |
| **80-28** | 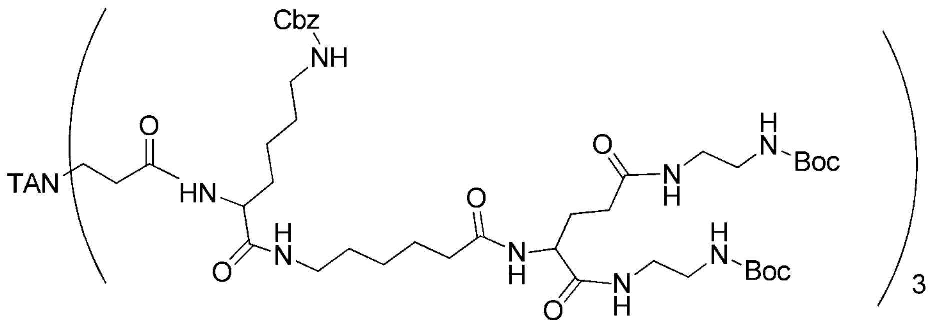 |
| **80-30** | 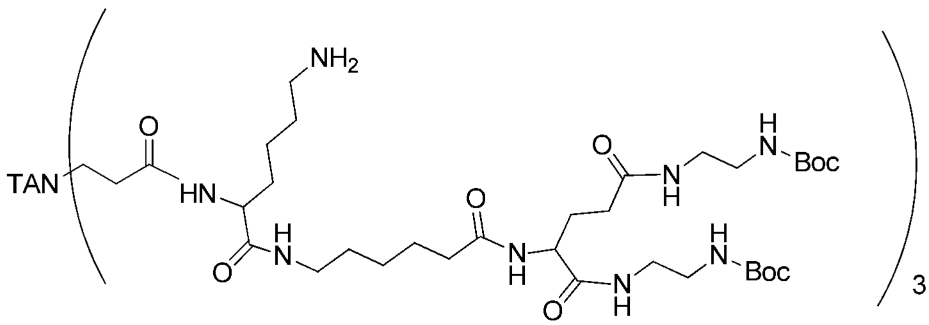 |
| **58-212** | 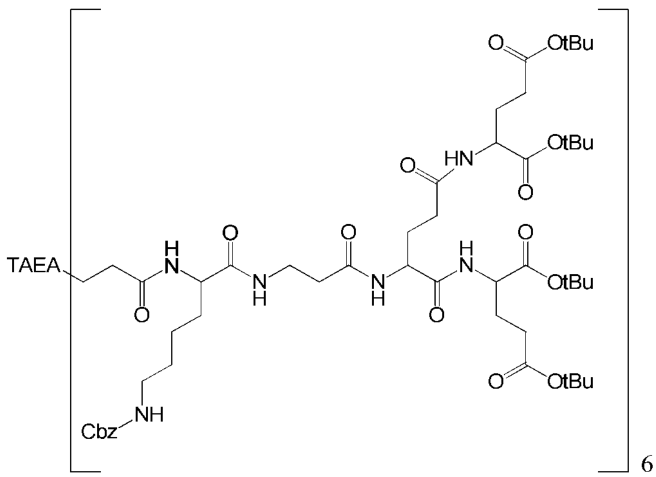 |

(continued)

| | |
|---|---|
| **58-214** | 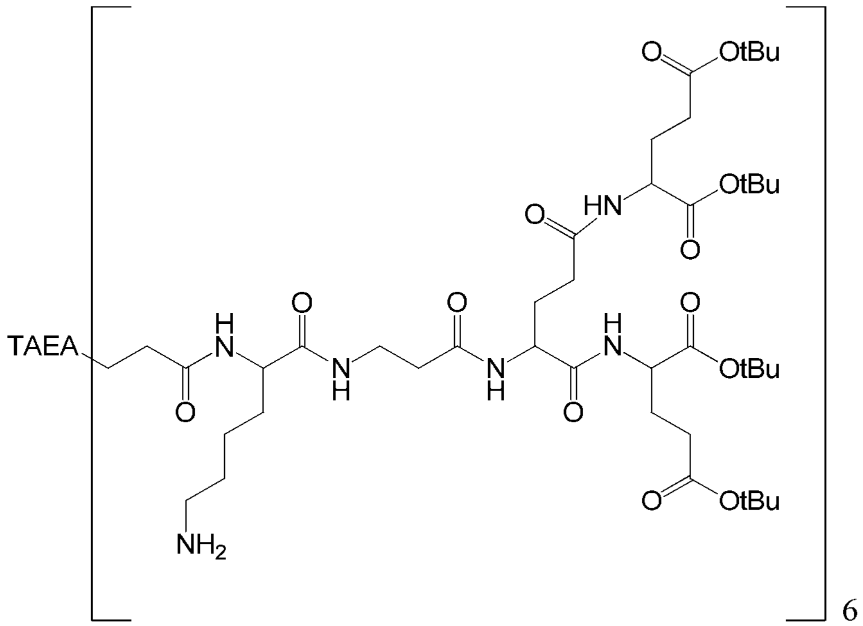 |
| **73-5** | 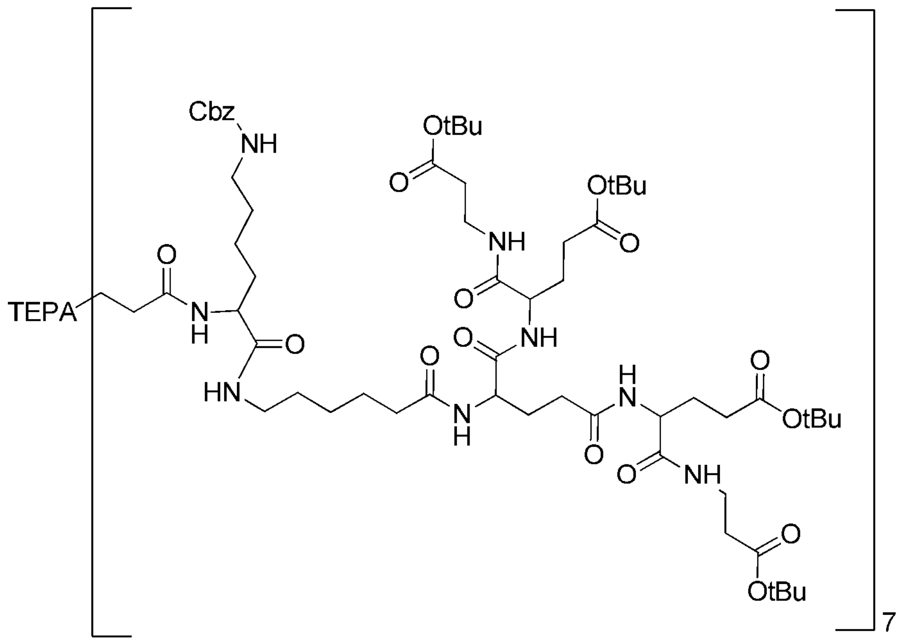 |

(continued)

| | |
|---|---|
| **73-14** | 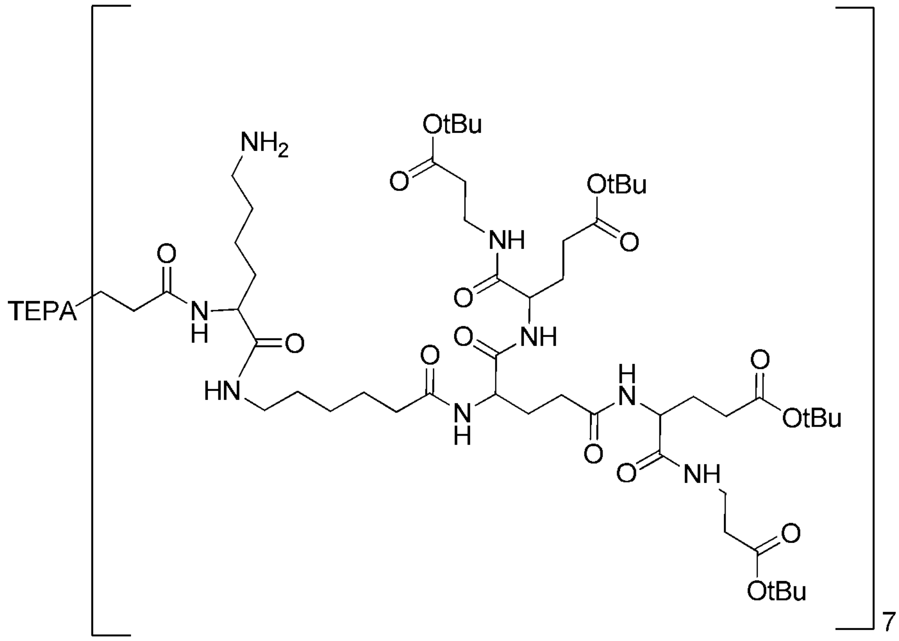 |
| **66-200** | 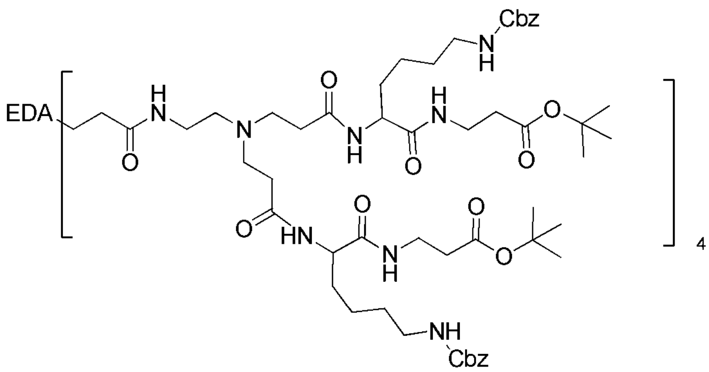 |
| **66-201** | 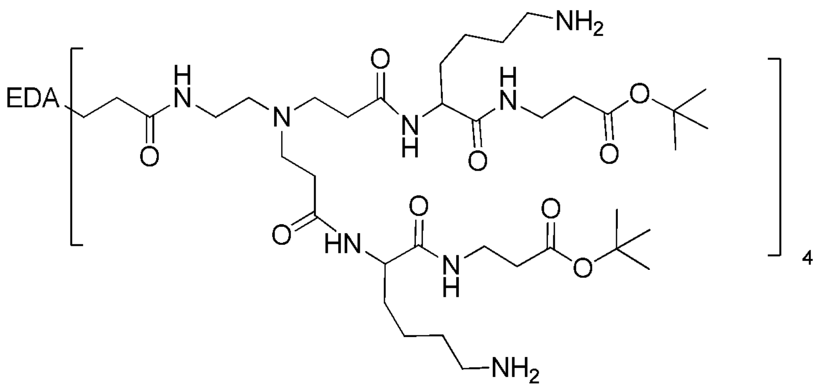 |

(continued)

| | |
|---|---|
| **78-74** | 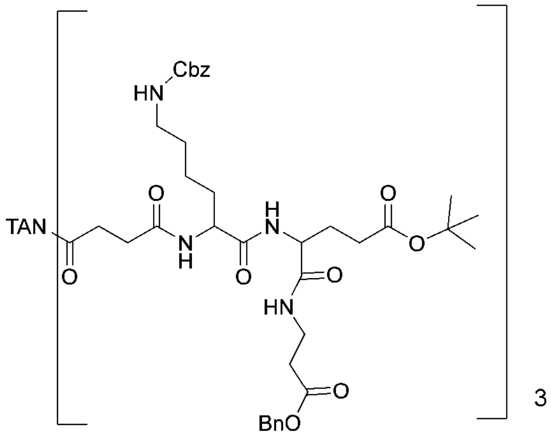 |
| **78-93** | 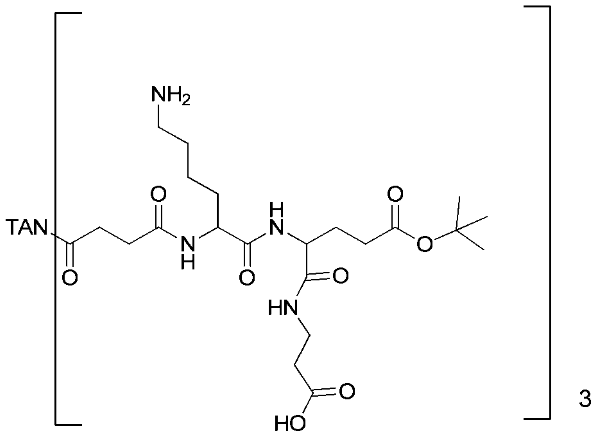 |
| **82-139** | 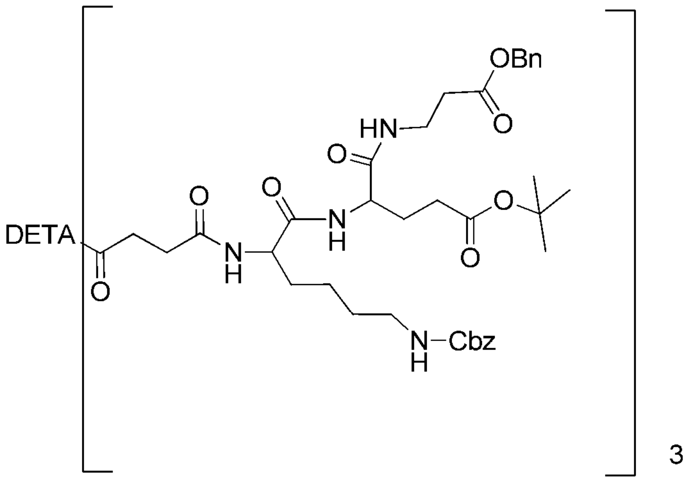 |

(continued)

| | |
|---|---|
| **82-150** | 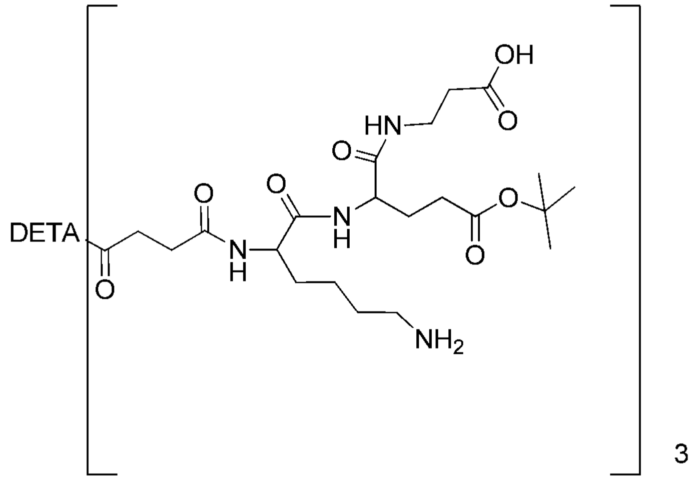 |
| **70-276** | 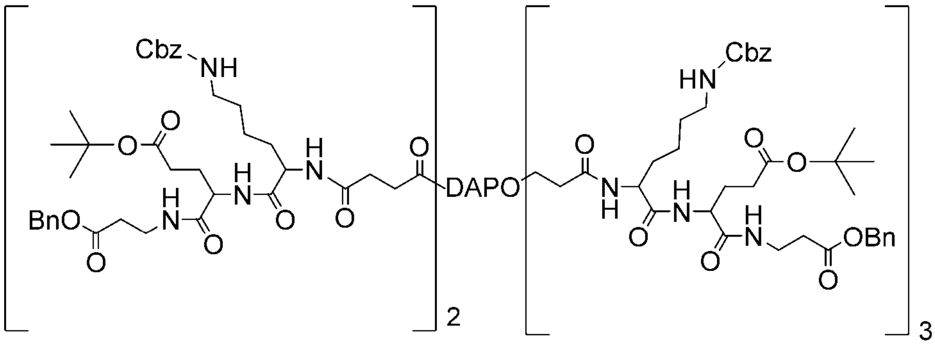 |
| **70-280** | 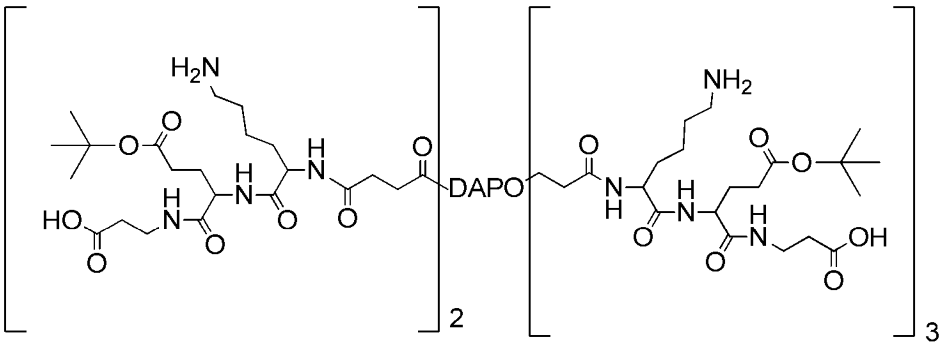 |

(continued)

| | |
|---|---|
| **81-149** | 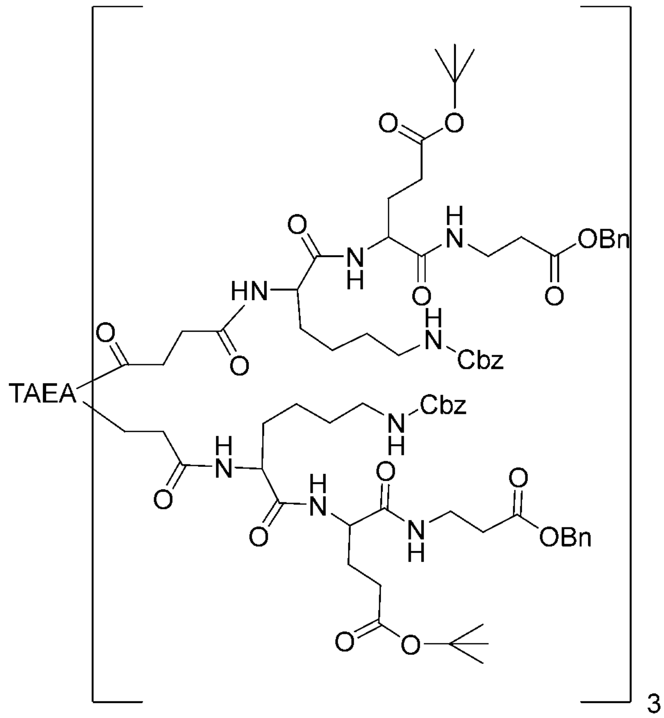 |
| **81-152** | 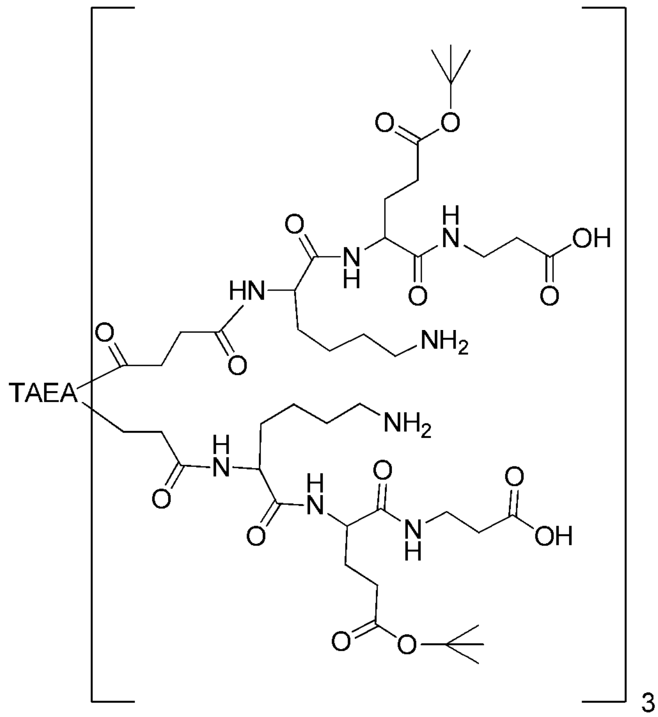 |

(continued)

| | |
|---|---|
| **71-281** | 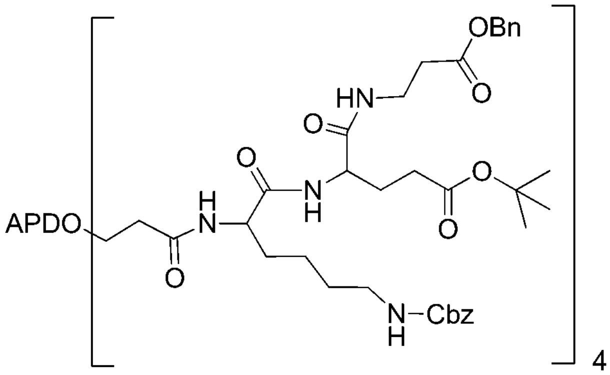 |
| **85-22** | 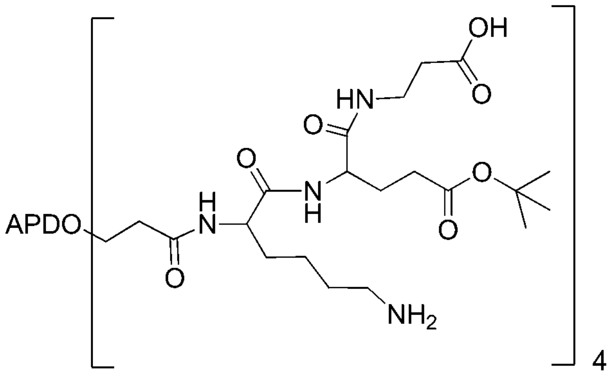 |
| **75-167** | 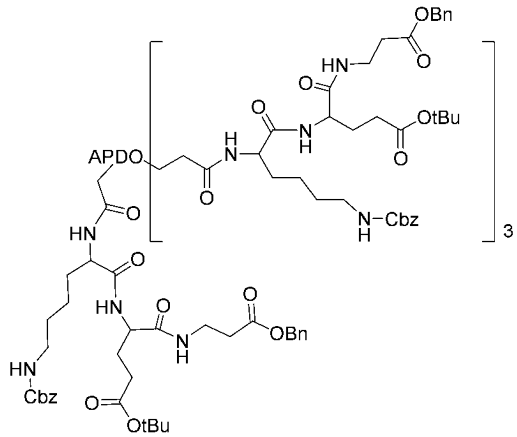 |
| **75-170** | 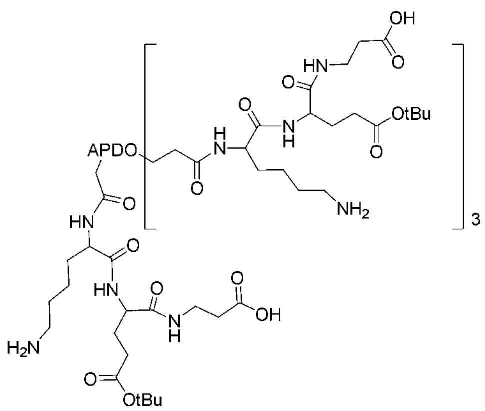 |

(continued)

| | |
|---|---|
| **69-260** | 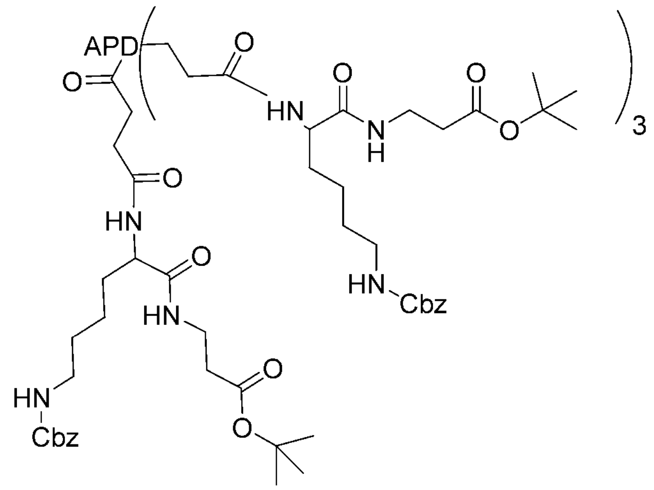 |
| **69-267** | 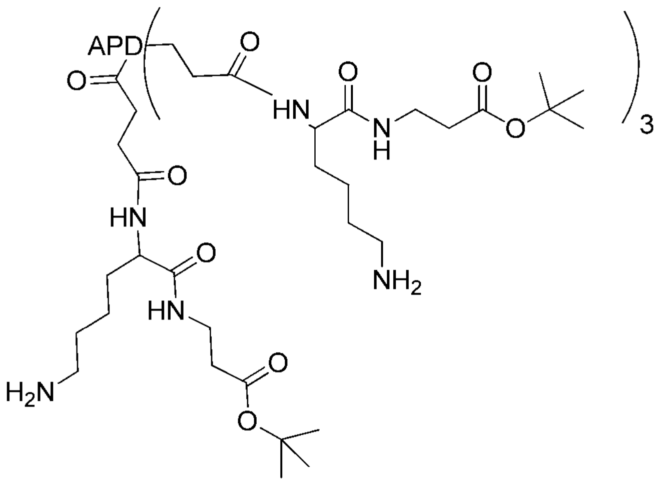 |
| **84-75** | 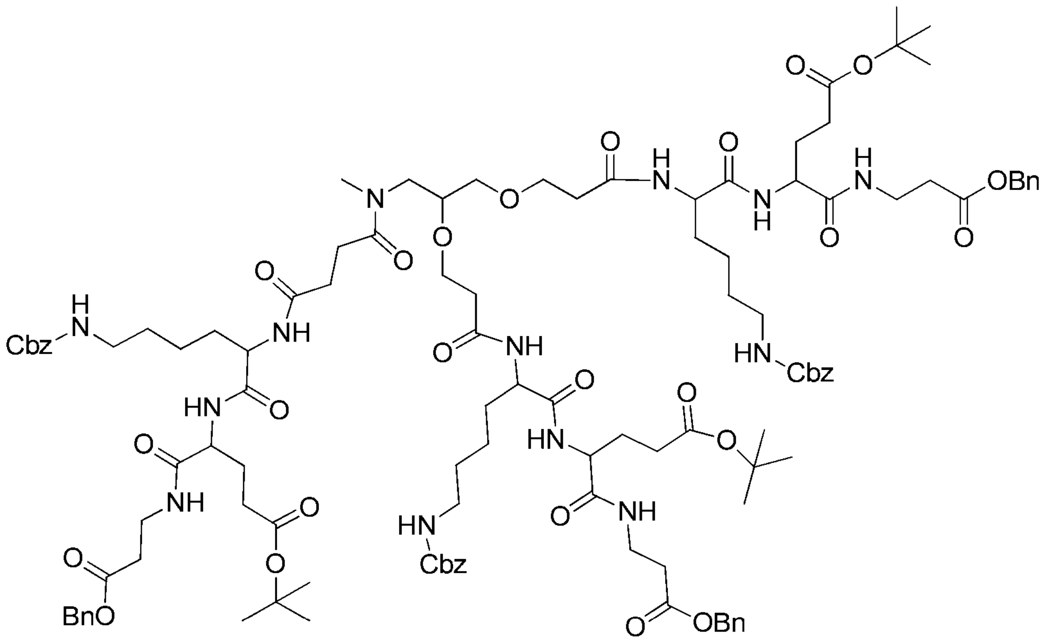 |

(continued)

| | |
|---|---|
| **84-81** | 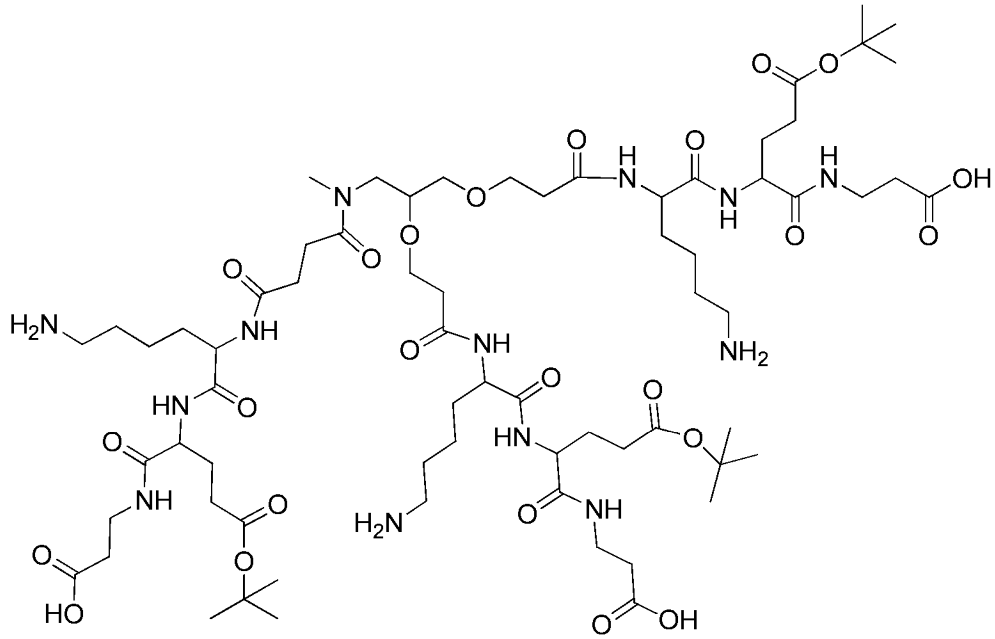 |
| **76-160** | 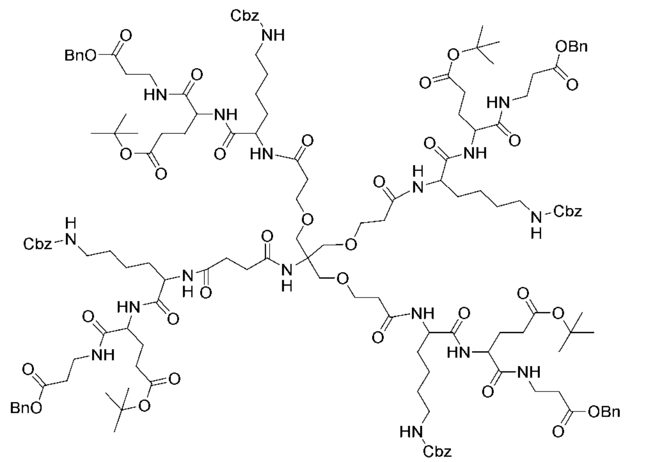 |
| **76-162** | 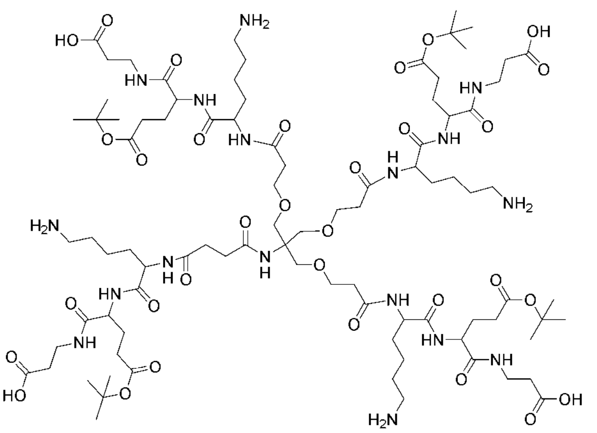 |

(continued)

| | |
|---|---|
| **82-78** | 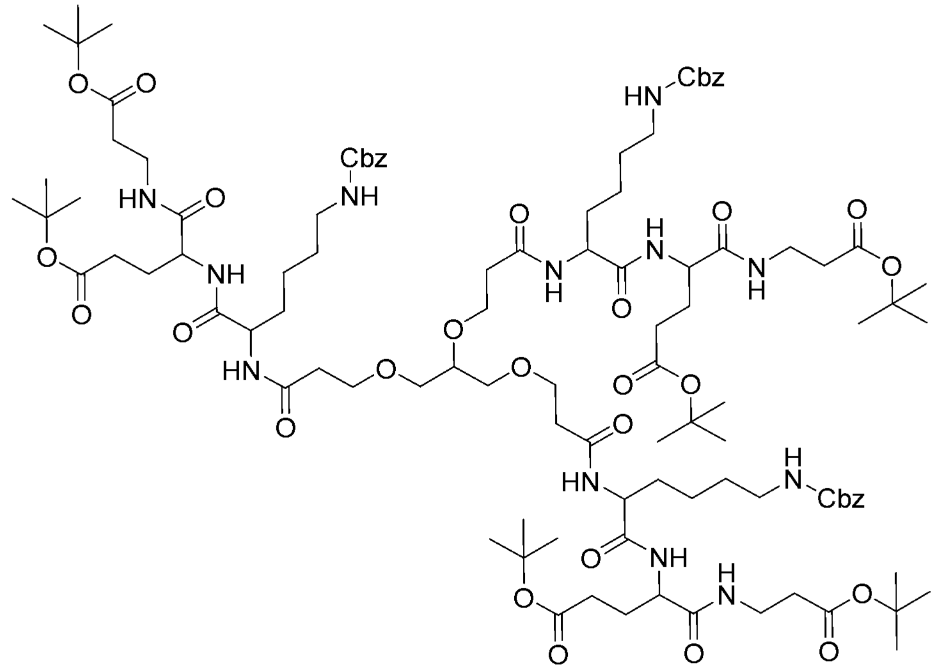 |
| **82-93** | 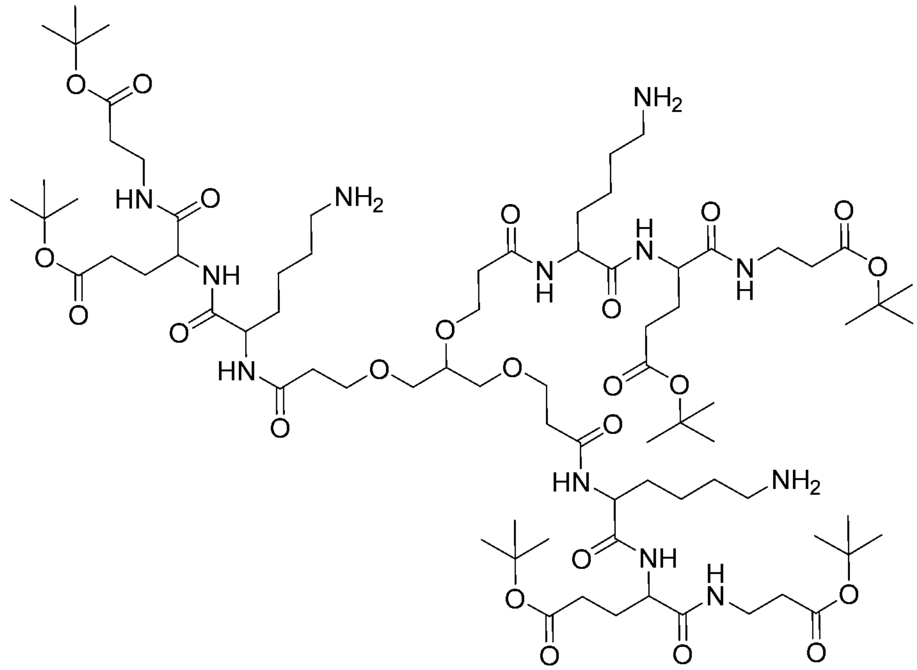 |

(continued)

| | |
|---|---|
| **80-201** | 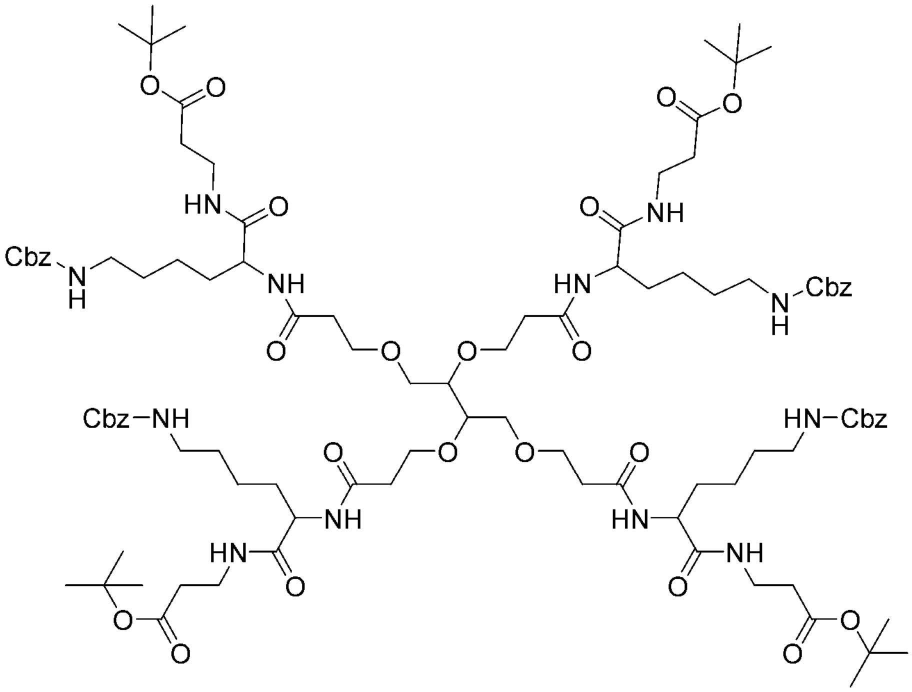 |
| **78-55** | 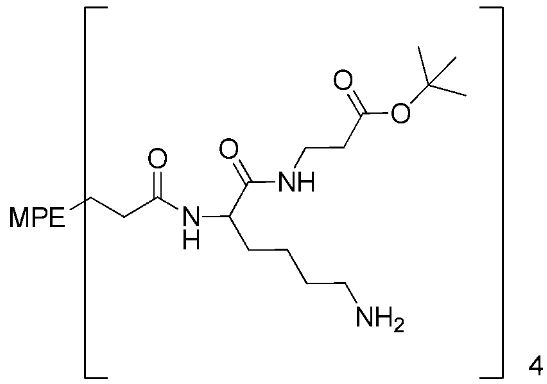 |
| **59-228** | 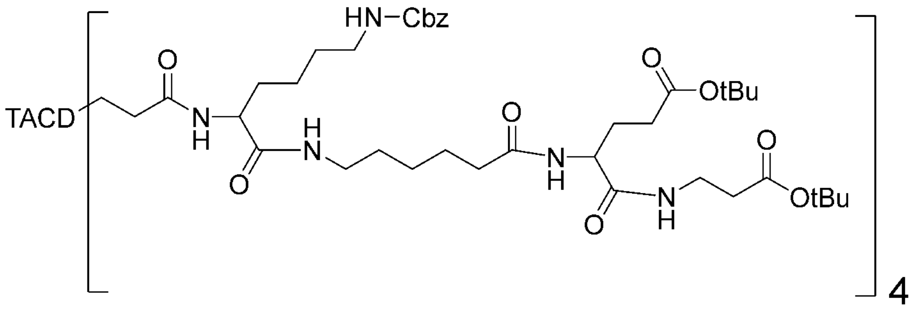 |

(continued)

| | |
|---|---|
| **59-232** | 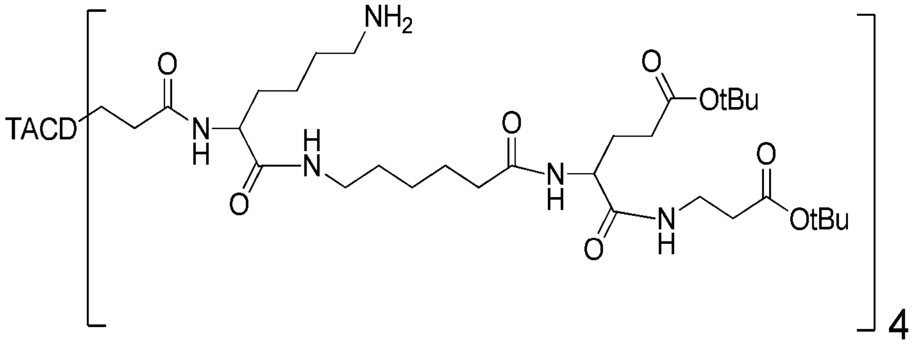 |

.

**8.** A compound represented by formula III or a pharmaceutically acceptable salt thereof:

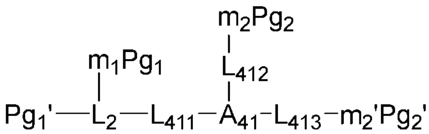

III

wherein, $Pg_1$' is hydrogen or selected from amino protecting group, such as an alkoxycarbonyl-based protecting group, further such as Boc, Fmoc, Cbz, or Teoc;
$m_1$, $m_2$, $m_2$', $Pg_1$, $Pg_2$, and $Pg_2$' are as defined in claim 6 or 7;
the remaining groups are as defined in any one of claims 1 to 7.

**9.** The compound or pharmaceutically acceptable salt thereof according to claim 8, wherein the compound is selected from the group consisting of the following compounds:

| | |
|---|---|
| **71-46** | 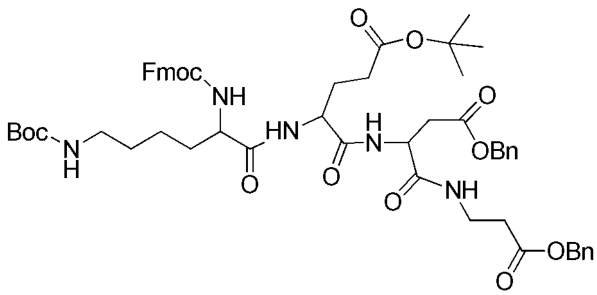 |
| **71-55** | 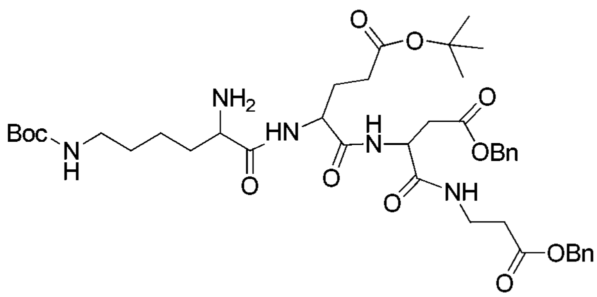 |

(continued)

| | |
|---|---|
| **38-227** | 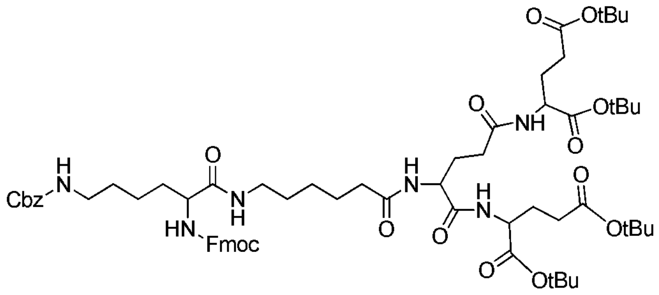 |
| **38-230** | 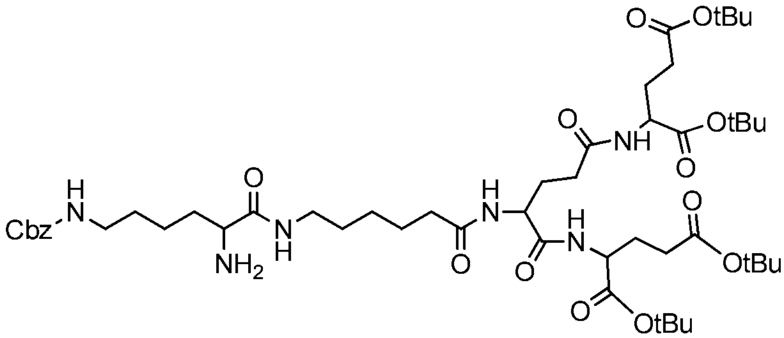 |
| **67-101** | 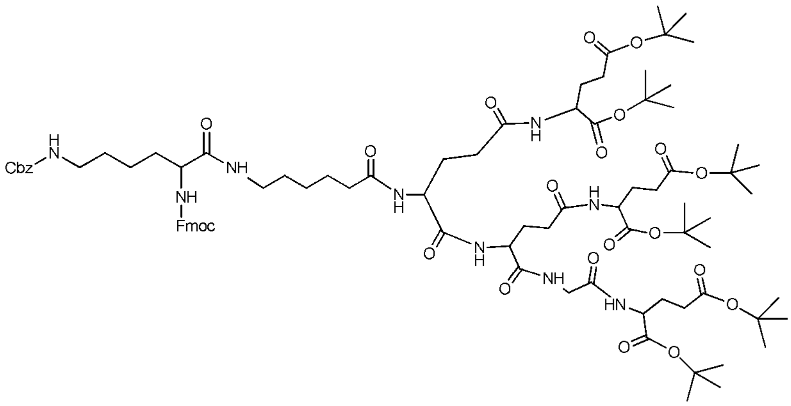 |
| **67-112** | 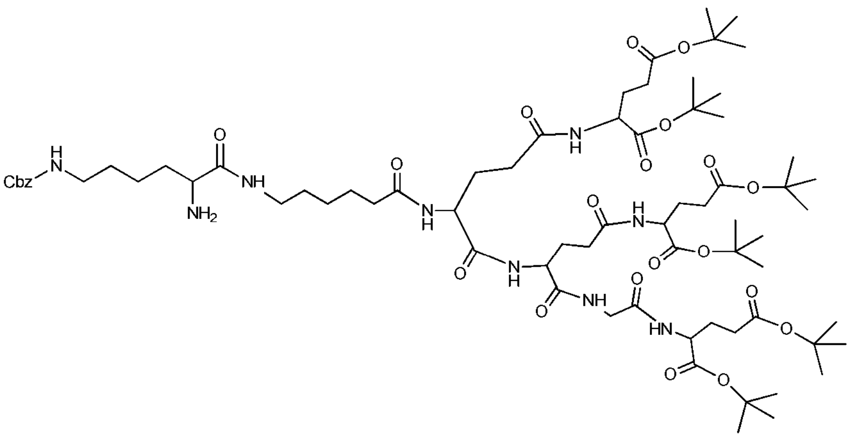 |

(continued)

| | |
|---|---|
| **69-39** | 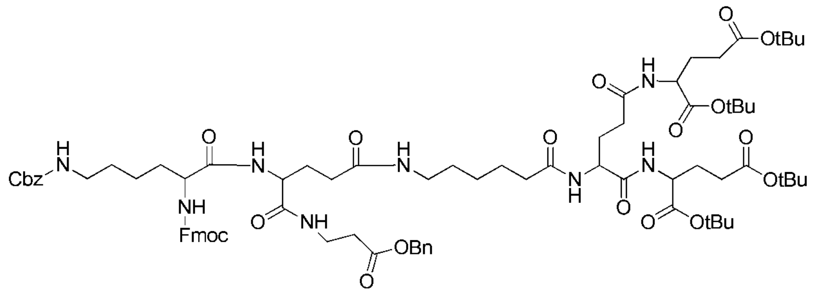 |
| **69-45** | 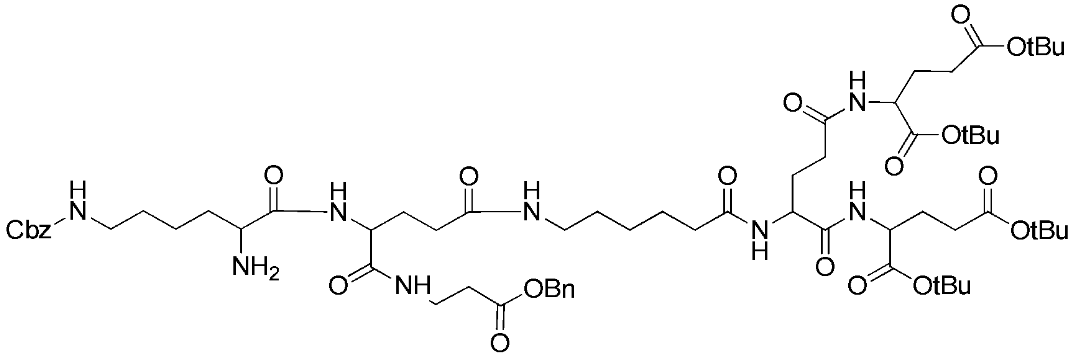 |
| **70-79** | 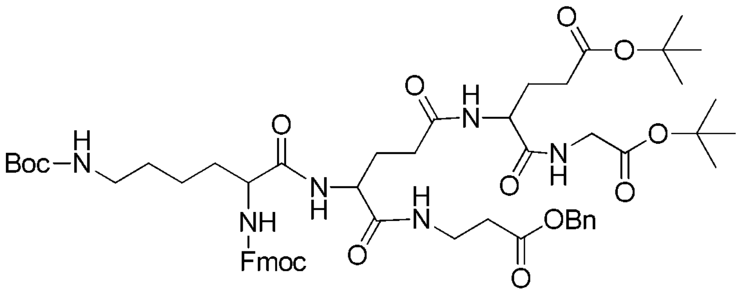 |
| **70-80** | 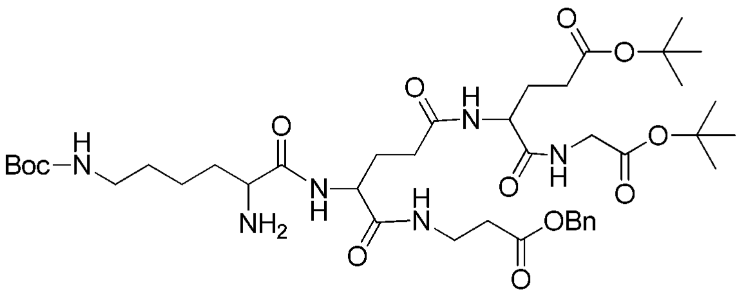 |
| **80-18** | 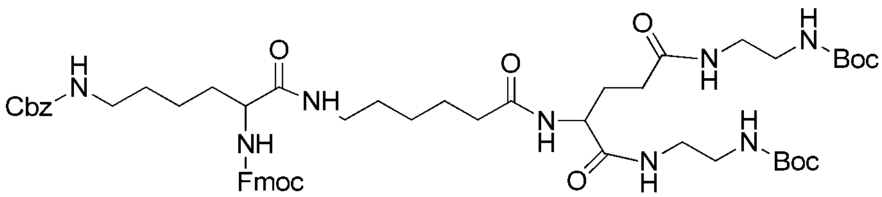 |
| **80-20** | 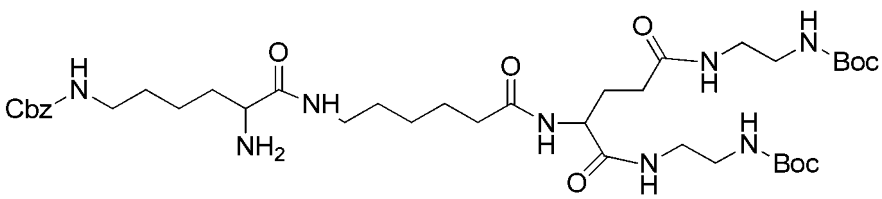 |

(continued)

| | |
|---|---|
| **58-82** | 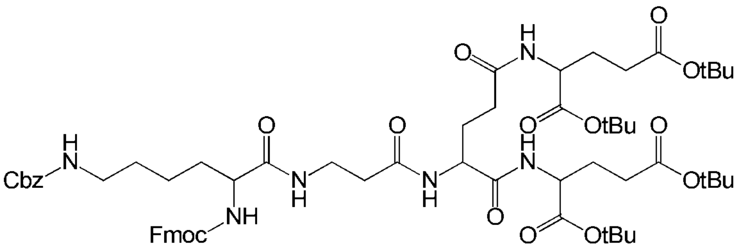 |
| **58-211** | 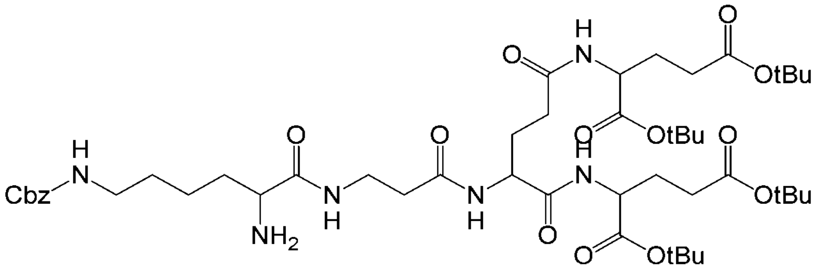 |
| **61-231** | 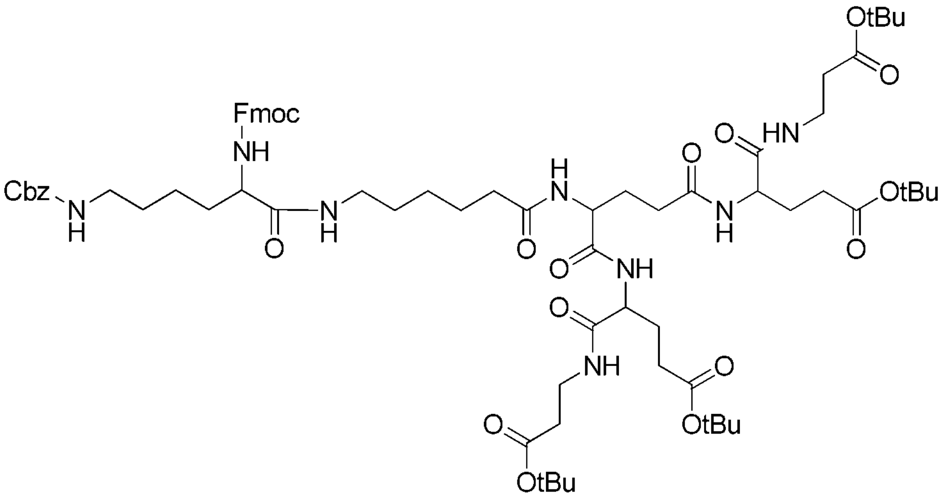 |
| **61-232** | 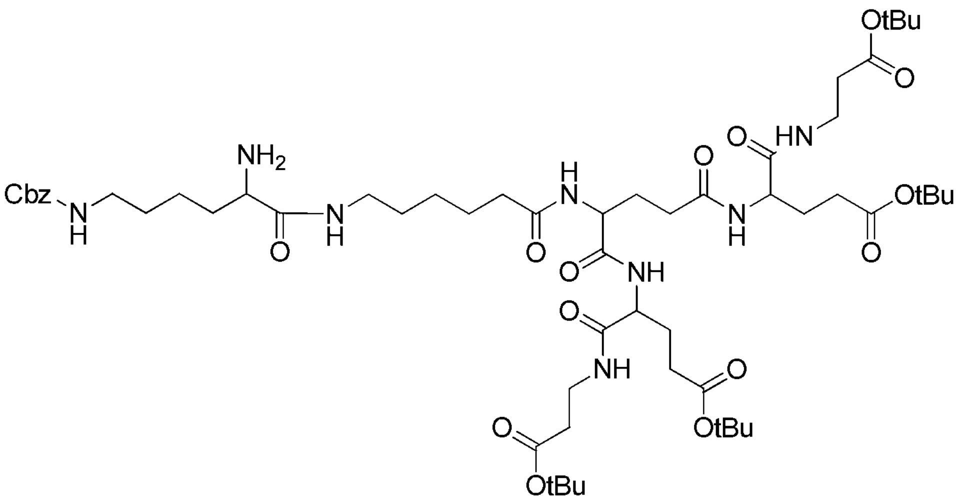 |
| **66-136** | 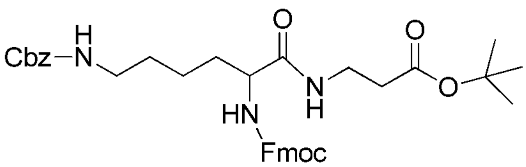 |

(continued)

| | |
|---|---|
| 66-195 | 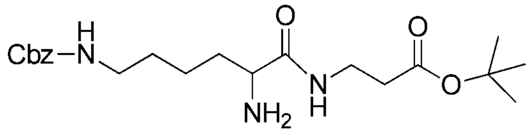 |
| 78-68 | 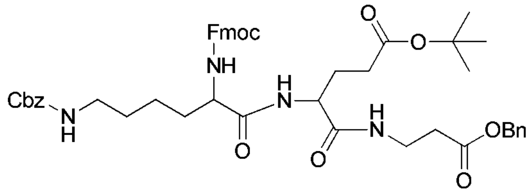 |
| 78-69 | 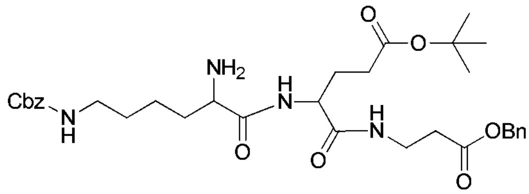 |
| 76-56 | 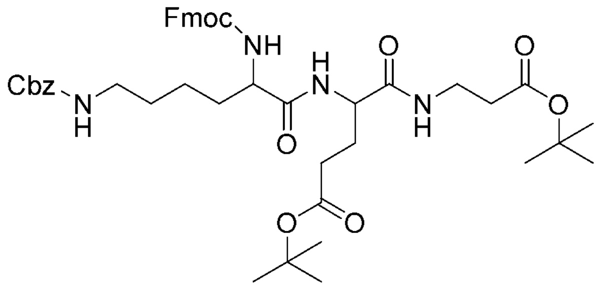 |
| 76-58 | 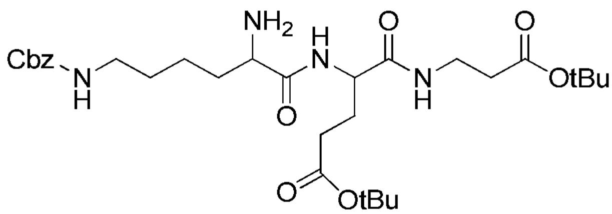 |
| 59-226 | 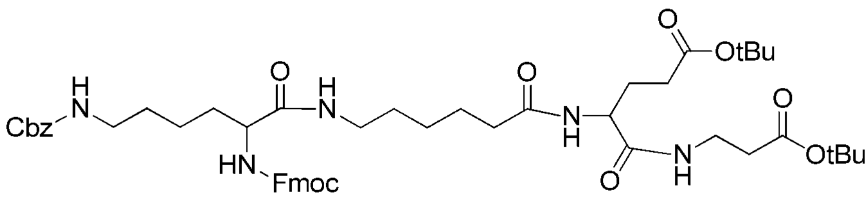 |
| 59-227 | 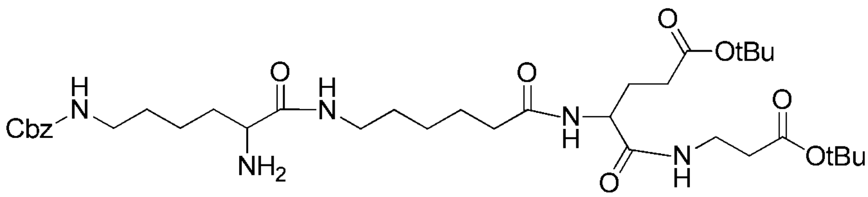 |

.

**10.** A compound represented by formula IV or a pharmaceutically acceptable salt thereof:

$M-[L_1-Pg_3]_n$ IV

wherein, each $Pg_3$ is independently hydrogen or selected from the group consisting of a carboxyl protecting group, such as an ester-based protecting group, preferably methyl ester, ethyl ester, tert-butyl ester, allyl ester, or benzyl ester;
the remaining groups are as defined in any one of claims 1 to 9.

**11.** The compound or pharmaceutically acceptable salt thereof according to claim 10, wherein the compound is selected from the group consisting of the following compounds:

| | |
|---|---|
| 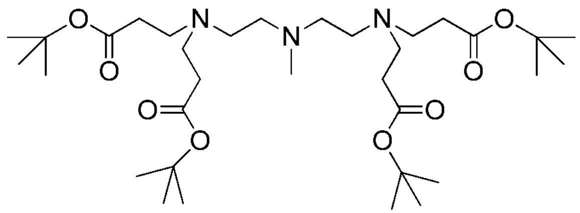 **71-6** | 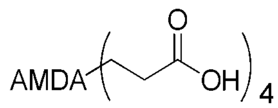 **71-54** |
| 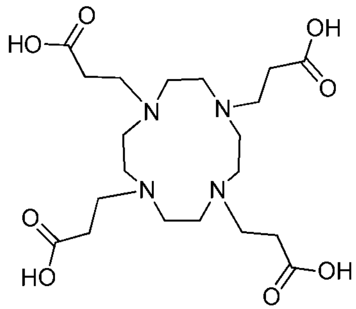 **59-108** | 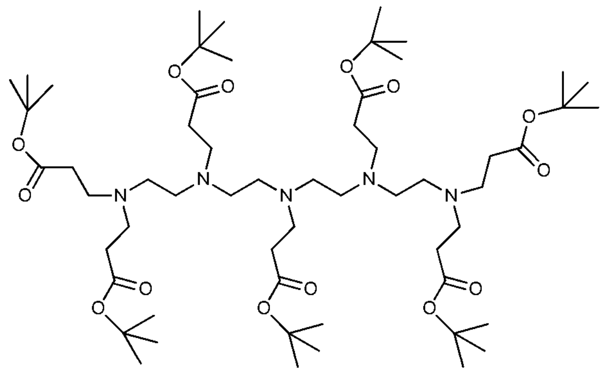 **67-34** |
| 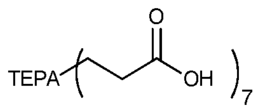 **67-53** | Triethylenetetraaminehexaacetic acid |

(continued)

| | |
|---|---|
| 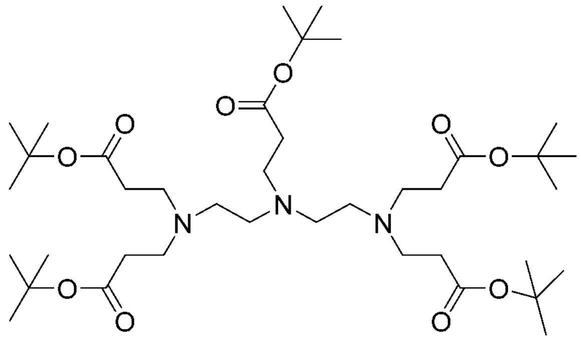 69-9 | 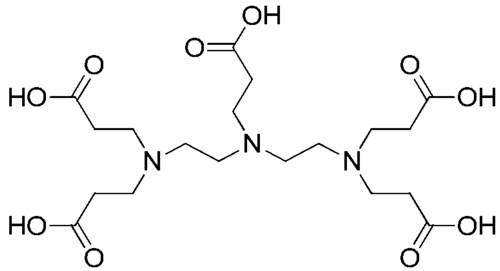 69-38 |
| 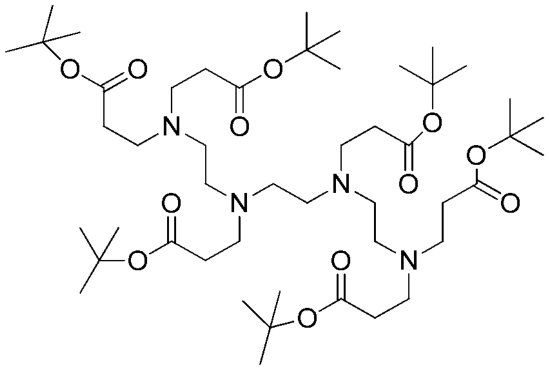 70-2 | 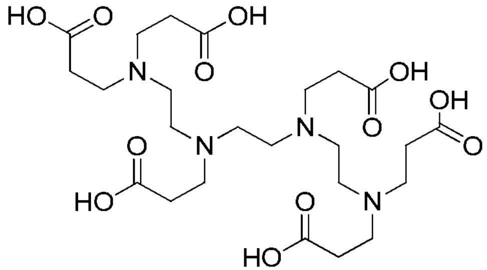 70-70 |

(continued)

| | |
|---|---|
| 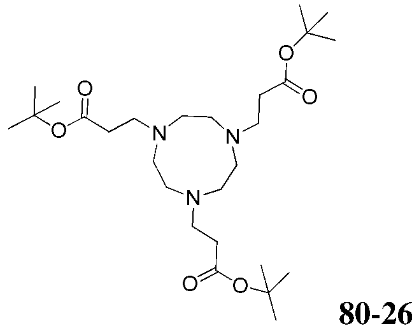 80-26 | 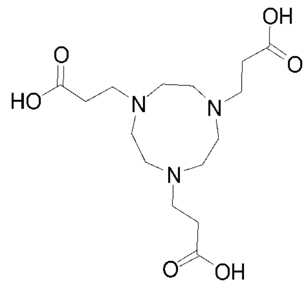 80-27 |
| 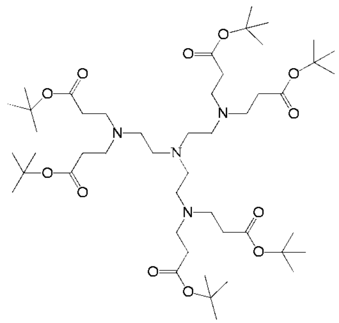 58-145 | 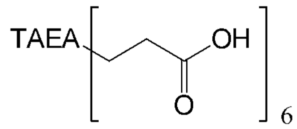 58-148 |

(continued)

| | |
|---|---|
| 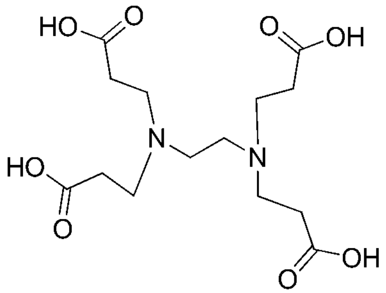 66-135 | 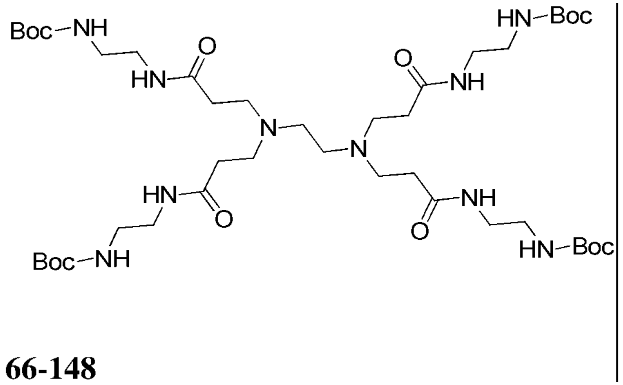 66-148 |
| 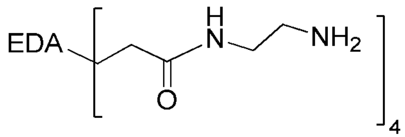 66-168 | 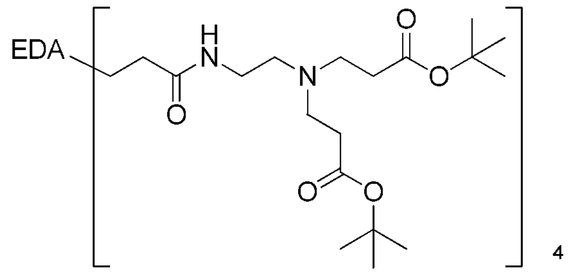 66-171 |
| 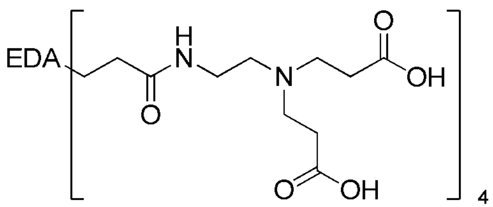 58-179 | 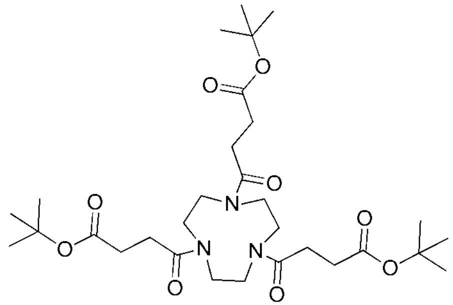 80-234 |

(continued)

| | |
|---|---|
| 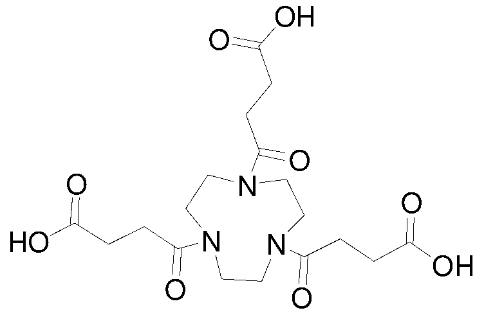 78-70 | 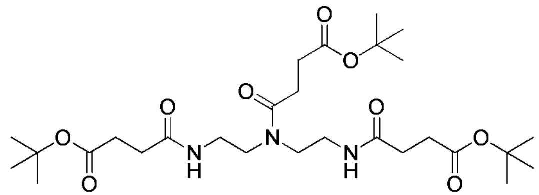 82-112 |
| 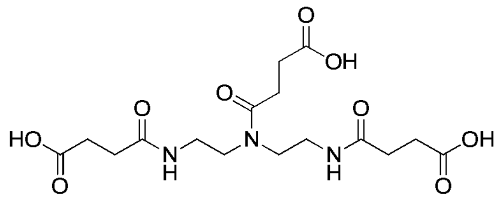 82-133 | 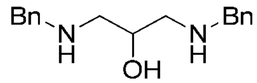 70-218 |
| 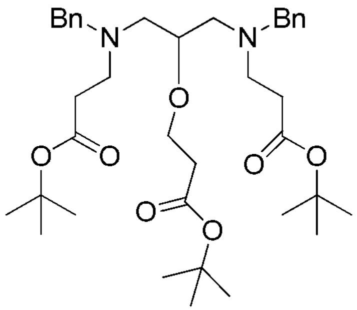 70-247 | 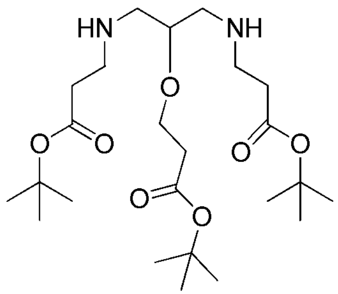 70-255 |

(continued)

| | |
|---|---|
| 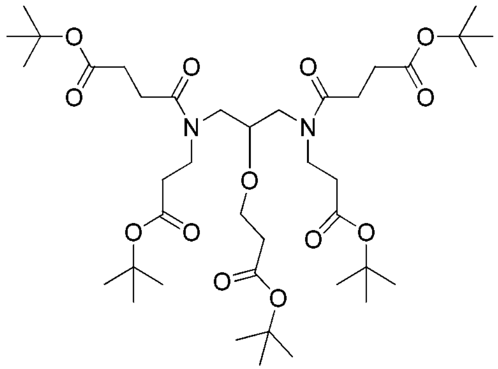 70-230 | 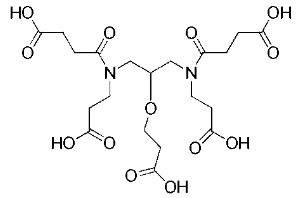 70-231 |
| 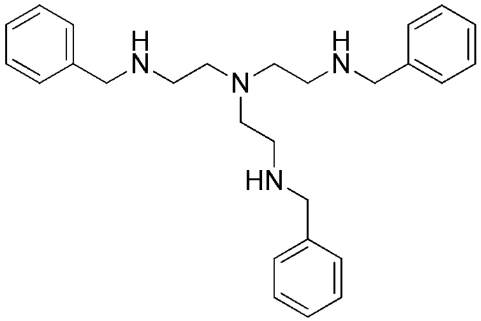 81-135 | 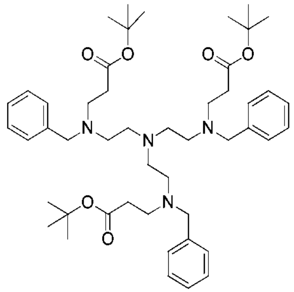 81-143 |

(continued)

| | |
|---|---|
| 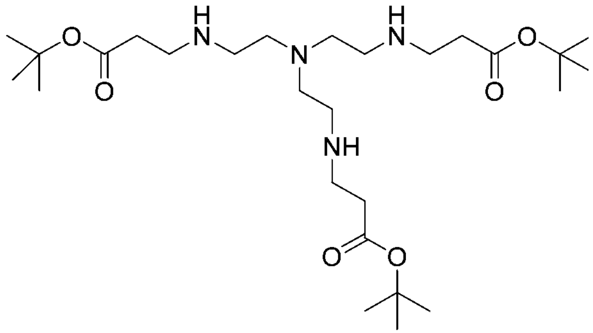 81-144 | 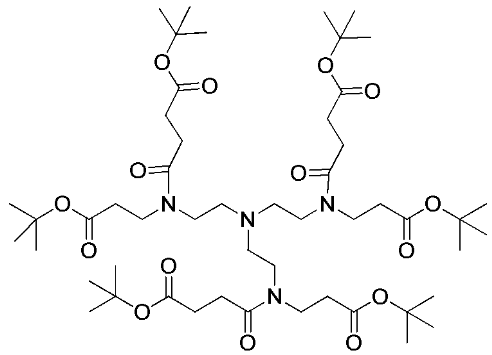 81-145 |
| 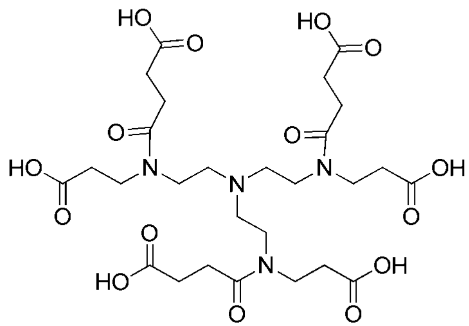 81-148 | 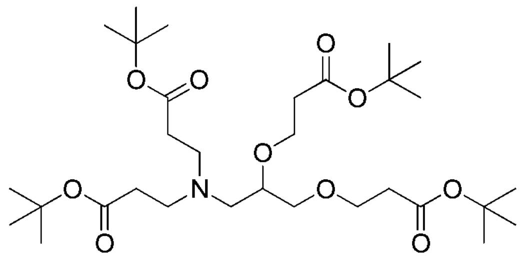 82-63 |

(continued)

| | |
|---|---|
| 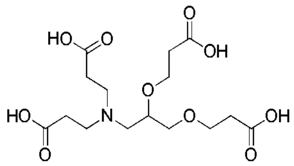 71-273 | 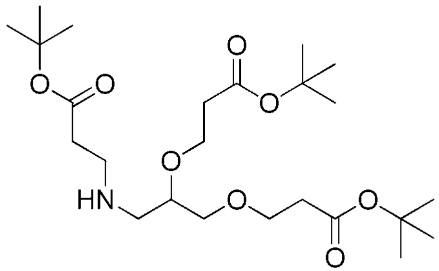 82-50 |
| 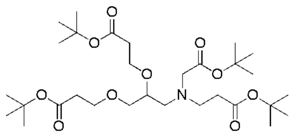 76-138 | 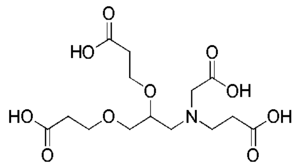 75-165 |
| 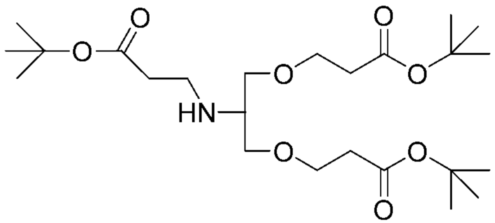 69-237 | 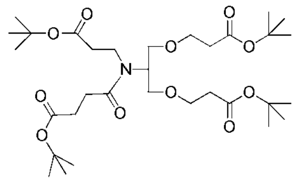 69-242 |

(continued)

| | |
|---|---|
| 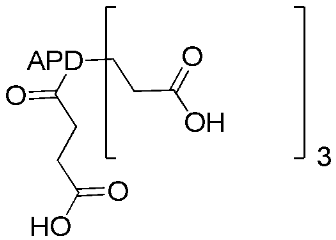 69-256 | 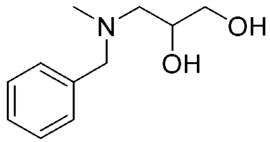 84-29 |
| 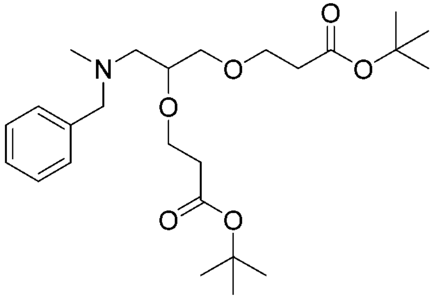 84-49 | 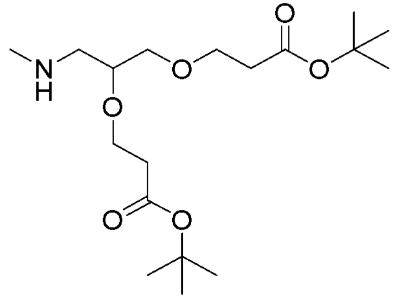 84-50 |
| 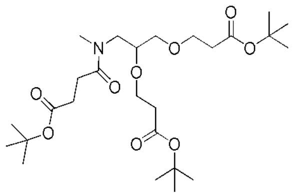 84-54 | 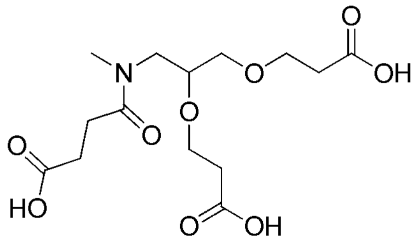 84-70 |

(continued)

| | |
|---|---|
| 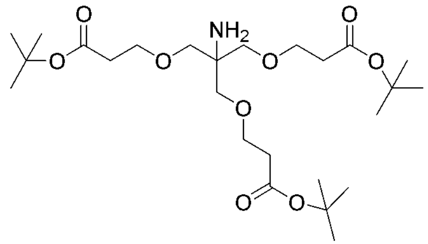 76-84 | 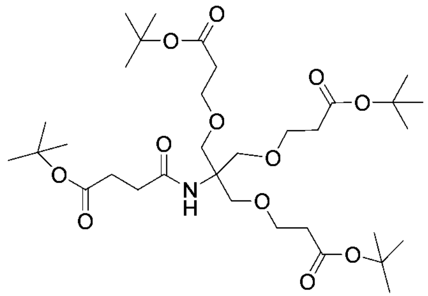 76-140 |
| 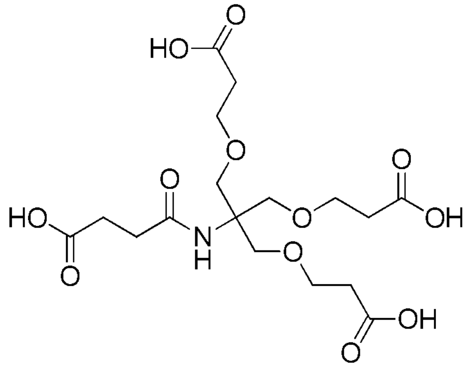 76-153 | 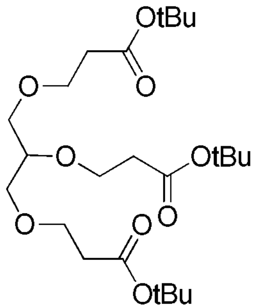 82-58 |

(continued)

| | |
|---|---|
| 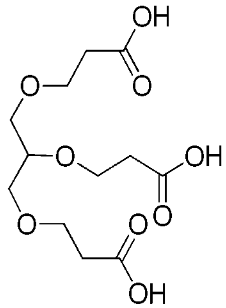 82-73 | 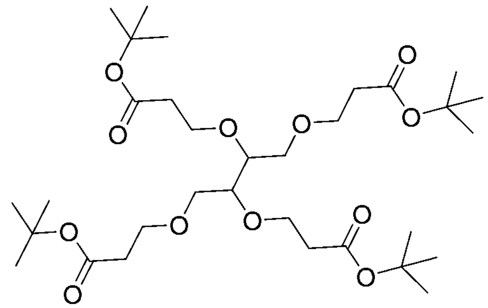 80-190 |
| 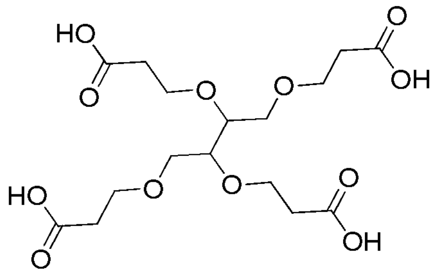 80-196 | |

.

**12.** A compound represented by formula V or a pharmaceutically acceptable salt thereof,

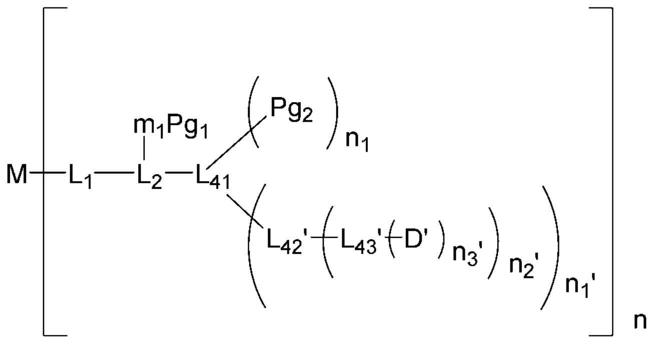

formula V

wherein each group is as defined in any one of claims 1 to 11.

**13.** The compound or pharmaceutically acceptable salt thereof according to claim 12, wherein the compound is selected from the group consisting of:

| | |
|---|---|
| **70-87** | 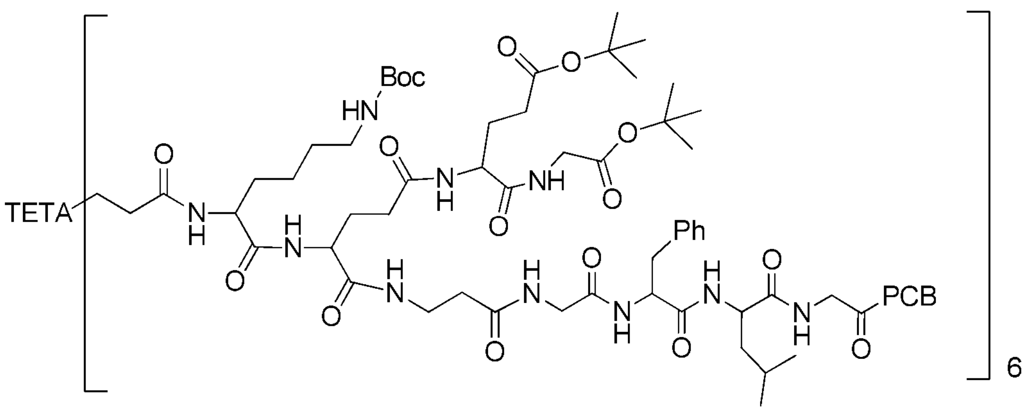 |
| **70-88** | 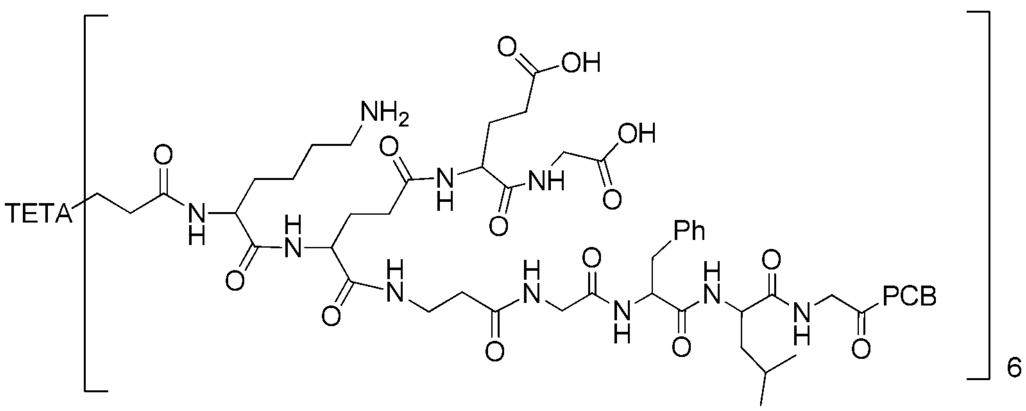 |

**14.** A pharmaceutical composition, comprising the compound or pharmaceutically acceptable salt thereof according to any one of claims 1 to 5 in an amount effective for treating and/or preventing a disease;

preferably, the composition further comprises one or more pharmaceutically acceptable excipients;
preferably, the pharmaceutical composition is formulated as an injectable formulation.

**15.** Use of the compound or pharmaceutically acceptable salt thereof according to any one of claims 1 to 5 in the manufacture of a medicament for treating and/or preventing a disease (e.g., a cancer);
preferably, the cancer is selected from the group consisting of colon cancer, leukemia, lymphoma, bladder cancer, bone cancer, brain tumor, medulloblastoma, glioma, breast cancer, adenoma/carcinoid, adrenocortical carcinoma, islet cell carcinoma, cervical cancer, endometrial cancer, ovarian cancer, colorectal cancer, skin cancer, esophageal cancer, eye cancer, gallbladder cancer, gastric cancer, head and neck cancer, liver cancer, melanoma, Kaposi's sarcoma, kidney cancer, oral cancer, lung cancer, nasopharyngeal cancer, neuroblastoma, ovarian cancer, pancreatic cancer, thyroid cancer, parathyroid gland cancer, penile cancer, prostate cancer, urethral cancer, vaginal cancer, vulvar cancer, anal cancer, sarcoma, and metastases of such cancers.

**16.** An injection, comprising the compound or pharmaceutically acceptable salt thereof according to any one of claims 1 to 5, or the pharmaceutical composition according to claim 14;
preferably, the injection uses physiological saline as a carrier.

**17.** A method for preparing the compound or pharmaceutically acceptable salt thereof according to any one of claims 1 to 5, wherein, when the $n_1$+$n_1$' side chains connected to $L_{41}$ in formula I are all the same, the method comprises the following steps:

S1-1: reacting a compound represented by formula II with PEG connected with an activating group to provide intermediate 1-1;
S1-2: subjecting intermediate 1-1 to any of the following reactions to provide the compound:

(1) reacting directly with a precursor providing fragment

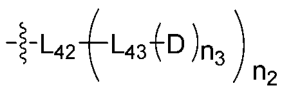

;

(2) reacting sequentially with precursors providing fragment $L_{42}$, fragment $L_{43}$ and fragment D,
(3) reacting sequentially with precursors providing fragment

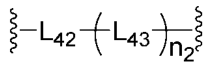

and fragment D;
(4) reacting sequentially with precursors providing fragment $L_{42}$ and fragment $L_{43}$-$n_3$D;

preferably, the step S1-1 is performed before or after S1-2, or, when S1-2 involves multiple steps, the step S1-1 is performed after any step of S1-2 is completed;
when the

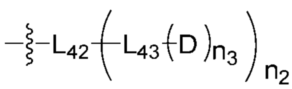

and

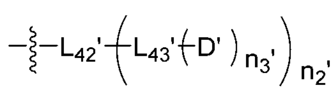

connected to $L_{41}$ in formula I are different, it comprises the following steps:

S2-1: reacting the compound represented by formula II with PEG connected with an activating group to provide intermediate 2-1;
S2-2: subjecting intermediate 2-1 to any of the following reactions to provide the compound:

(1) reacting sequentially with precursors providing fragment

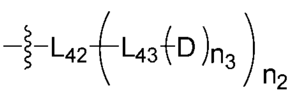

and fragment

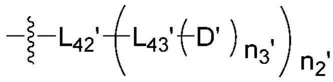
;

(2) reacting sequentially with precursors providing fragment

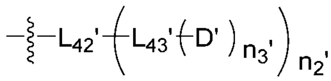

and fragment

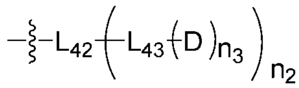
;

(3) reacting first with a precursor providing fragment

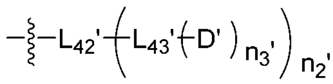
,

and then performing any of the following reactions:

1) reacting sequentially with precursors providing fragment $L_{42}$ and fragment $L_{43}$-$n_3$D;
2) reacting sequentially with precursors providing fragment $L_{42}$ , fragment $L_{43}$ and fragment D;
3) reacting sequentially with precursors providing fragment

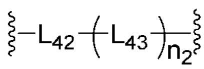

and fragment D;

preferably, the step S2-1 is performed before or after S2-2, or when S2-2 involves multiple steps, the step S2-1 is performed after any step of S2-2 is completed;
optionally, before or after any of the above steps, a step of removal of protecting group and/or activation (e.g., activation of carbonyl or amino group) is also included;
preferably, the precursor is a compound in free or activated form capable of providing the corresponding fragment;
preferably, the PEG activating group is preferably

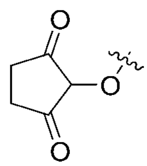
;

each of the groups is as defined in any one of claims 1 to 13.

**18.** The method according to claim 17, wherein the compound represented by formula II is obtained by reacting the compound represented by formula IV with the compound represented by formula III;
preferably, before conducting the reaction, a step of removing protecting groups is also included.

[Figure 1]

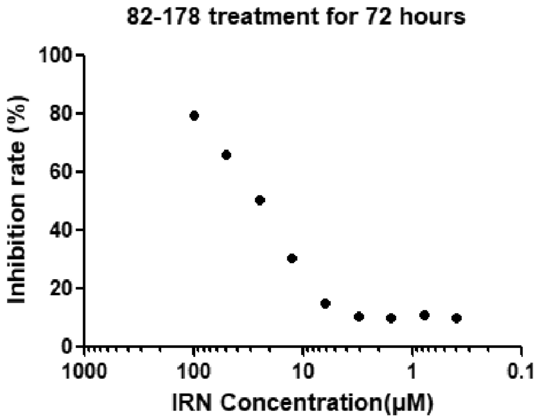


2-fold dilution results

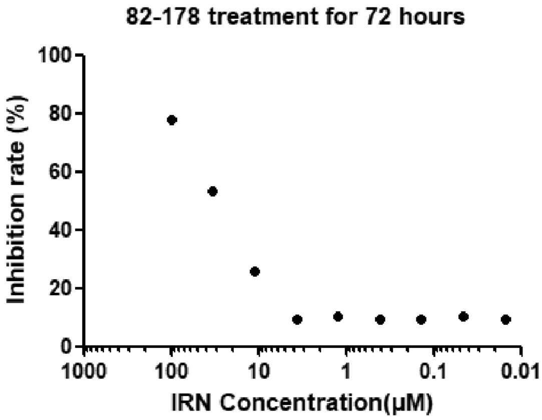


3-fold dilution results

[Figure 2]

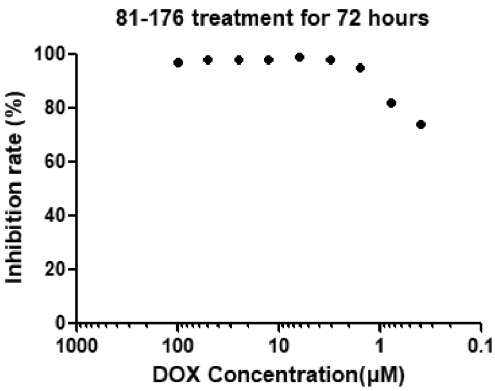


2-fold dilution results

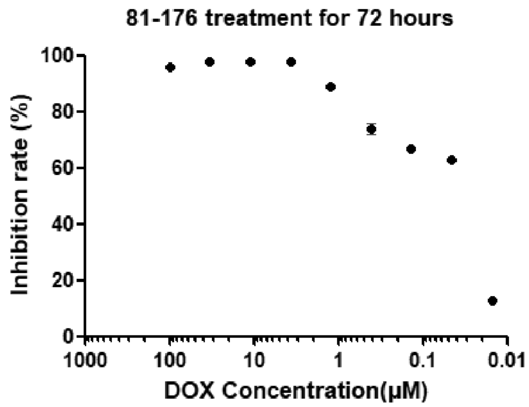


3-fold dilution results

[Figure 3]

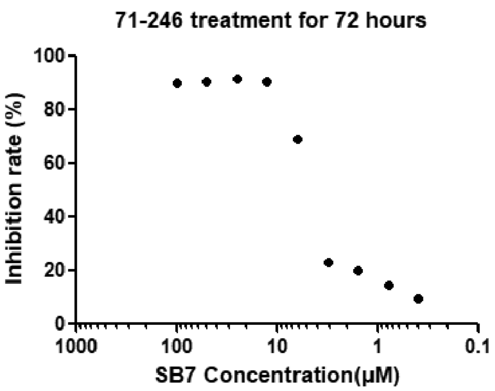


2-fold dilution results

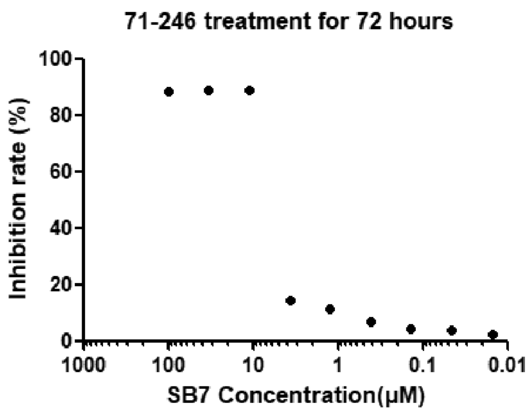


3-fold dilution results

[Figure 4]

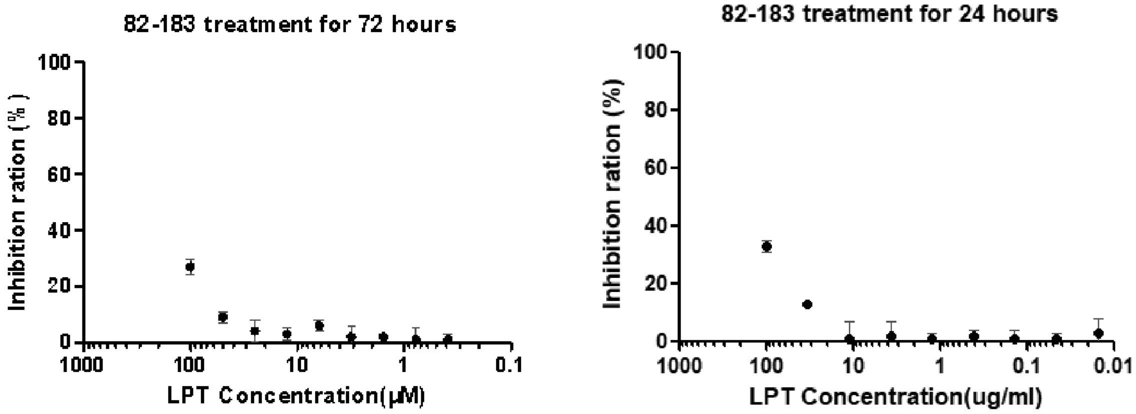


2-fold dilution results 3-fold dilution results

[Figure 5]

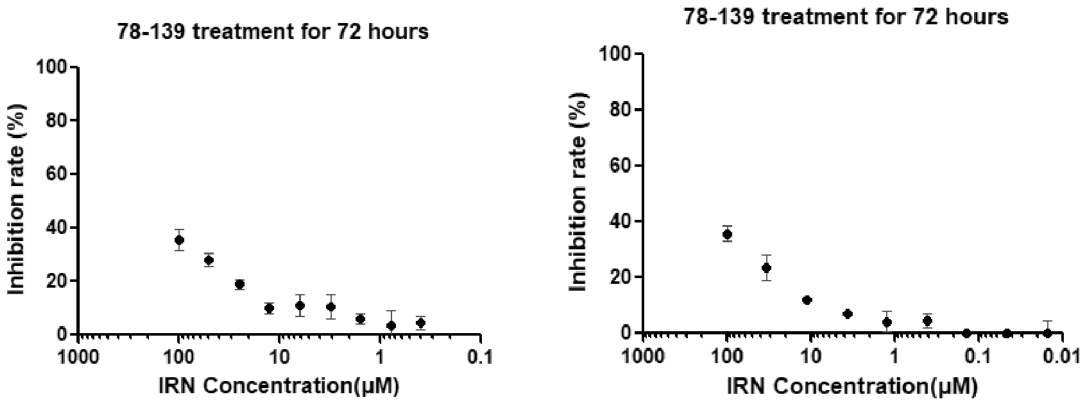


2-fold dilution results 3-fold dilution results

[Figure 6]

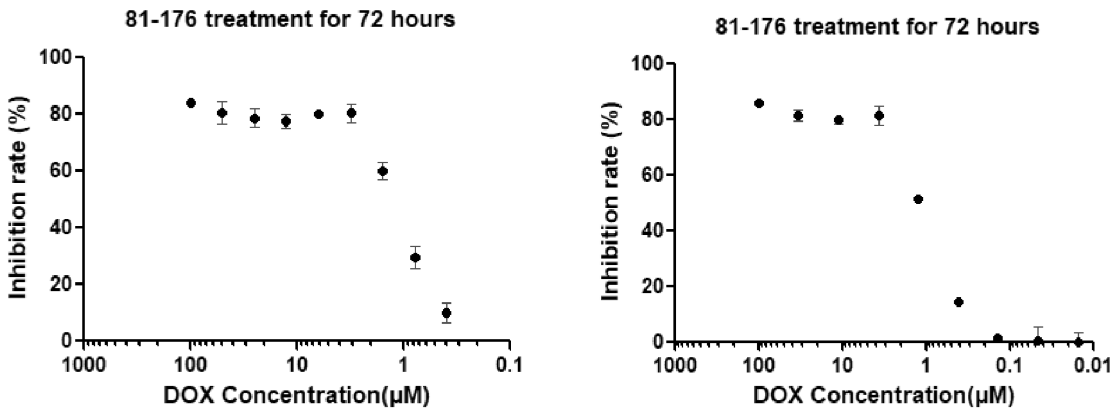


2-fold dilution results 3-fold dilution results

[Figure 7]

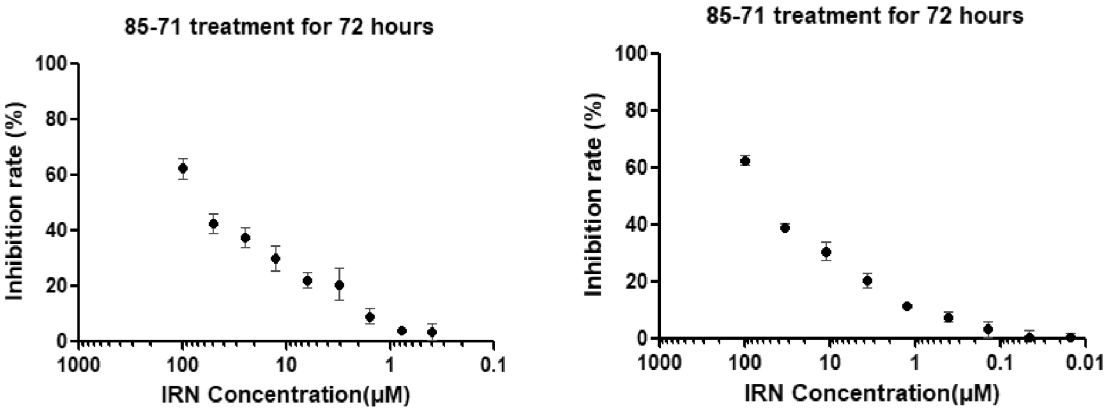


2-fold dilution results 3-fold dilution results

[Figure 8]

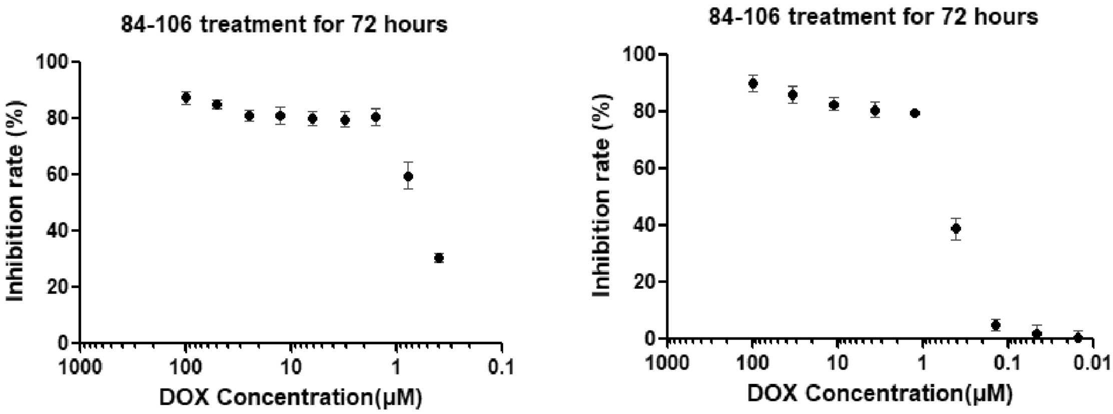


2-fold dilution results 3-fold dilution results

[Figure 9]

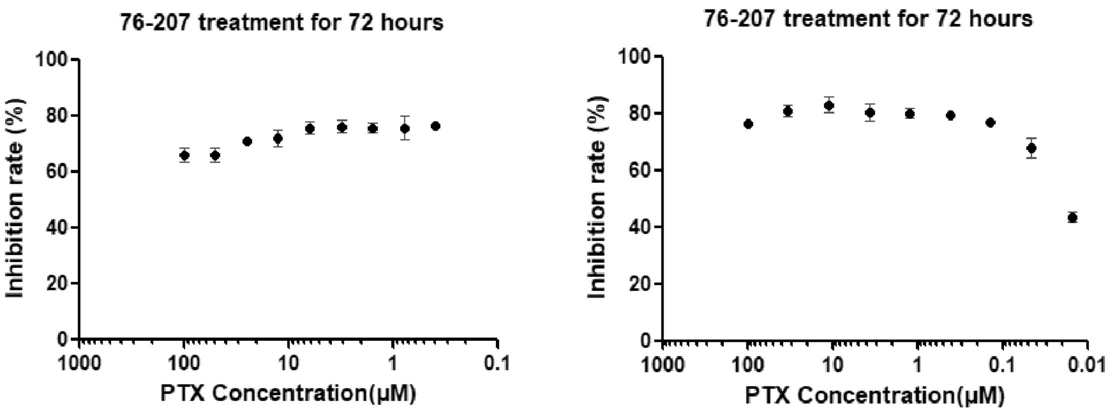


2-fold dilution results 3-fold dilution results

[Figure 10]

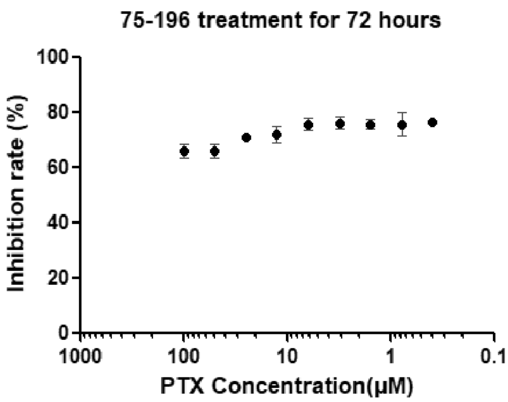


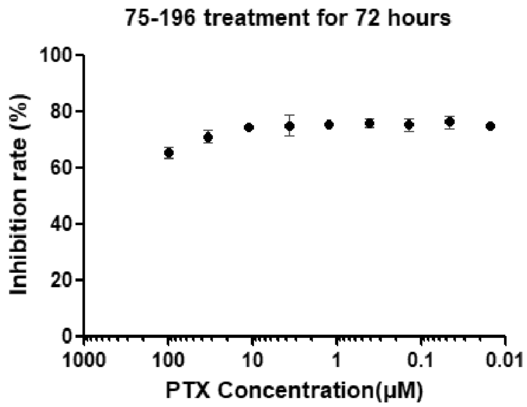


2-fold dilution results 3-fold dilution results

[Figure 11]

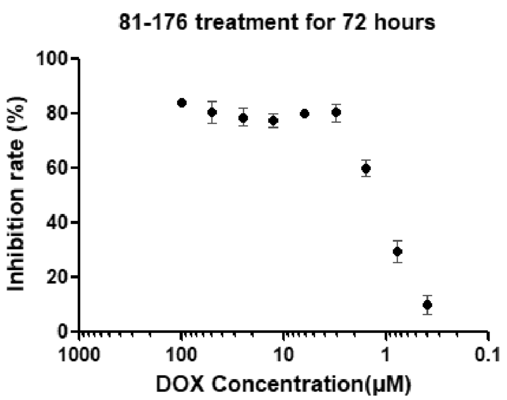


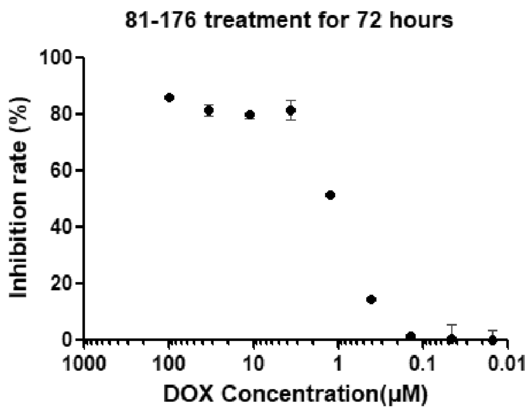


2-fold dilution results 3-fold dilution results

[Figure 12]

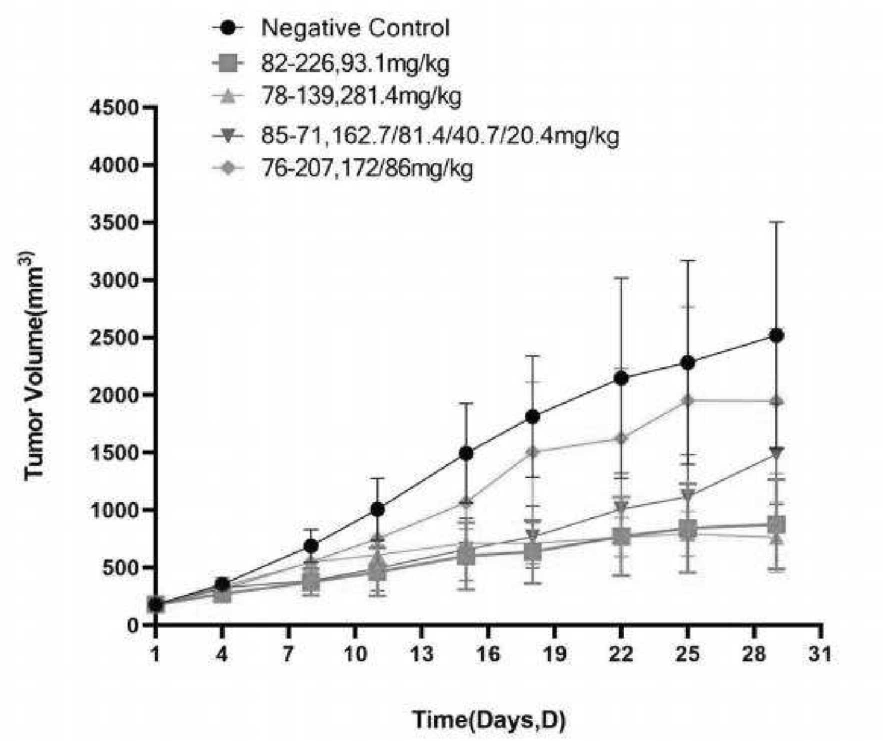

[Figure 13]

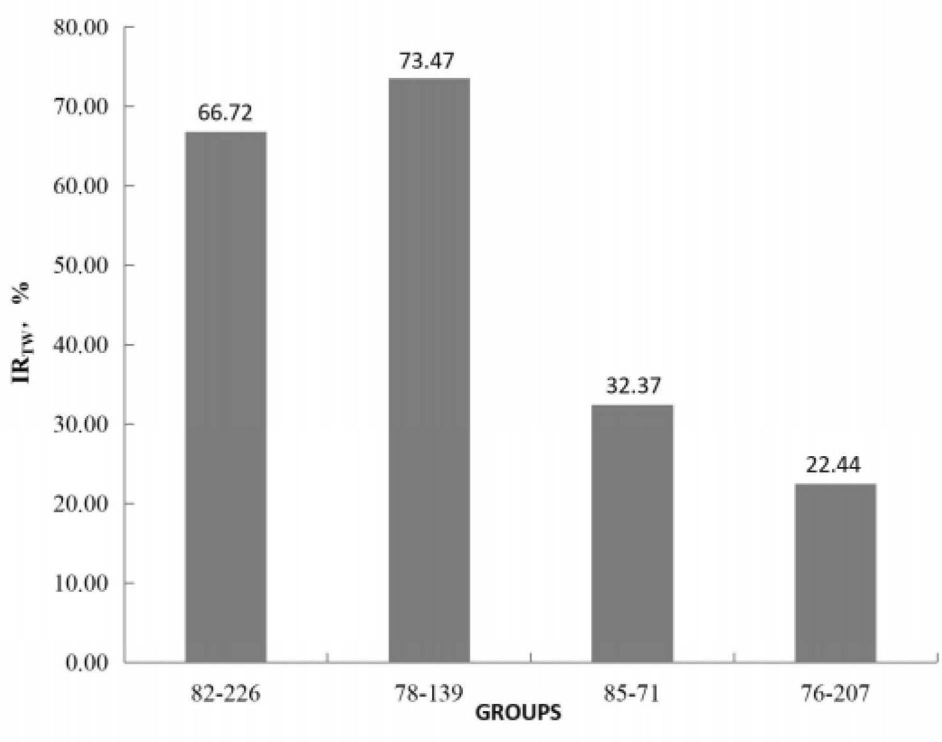


[Figure 14]

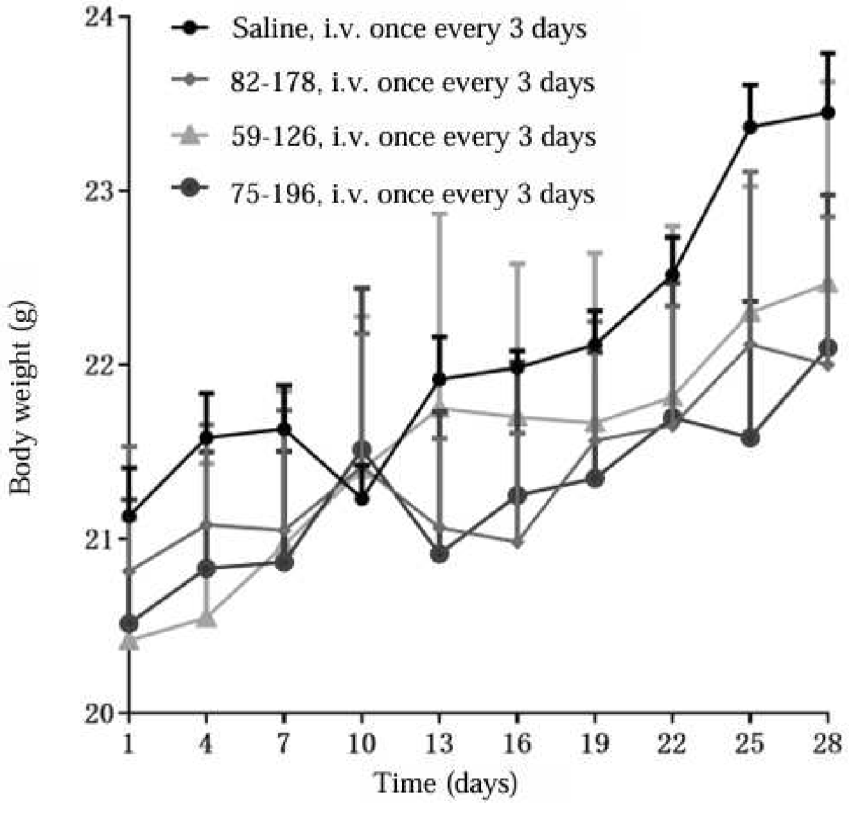

[Figure 15]

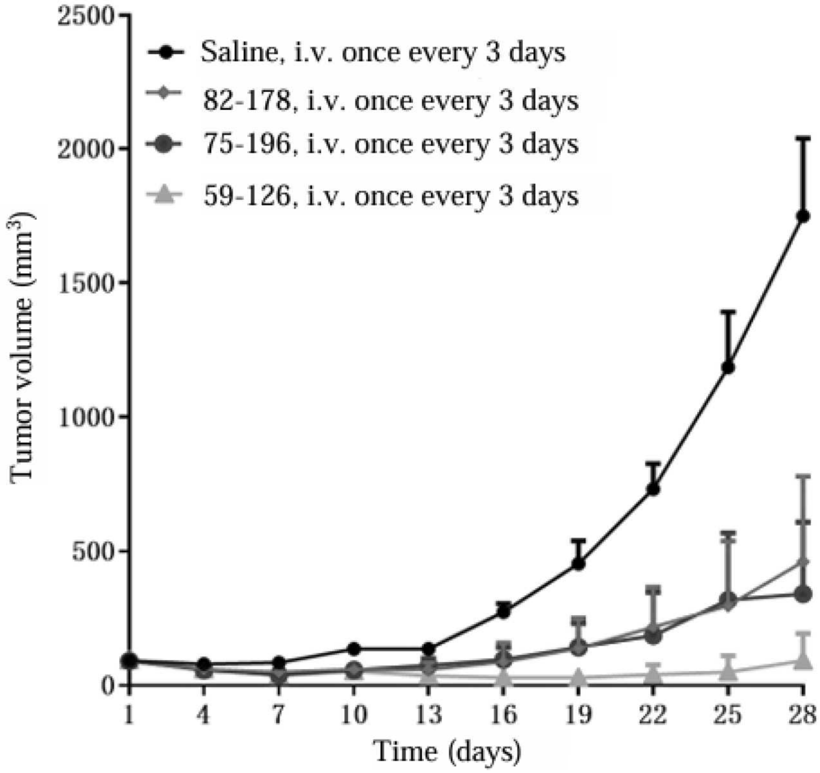


[Figure 16]

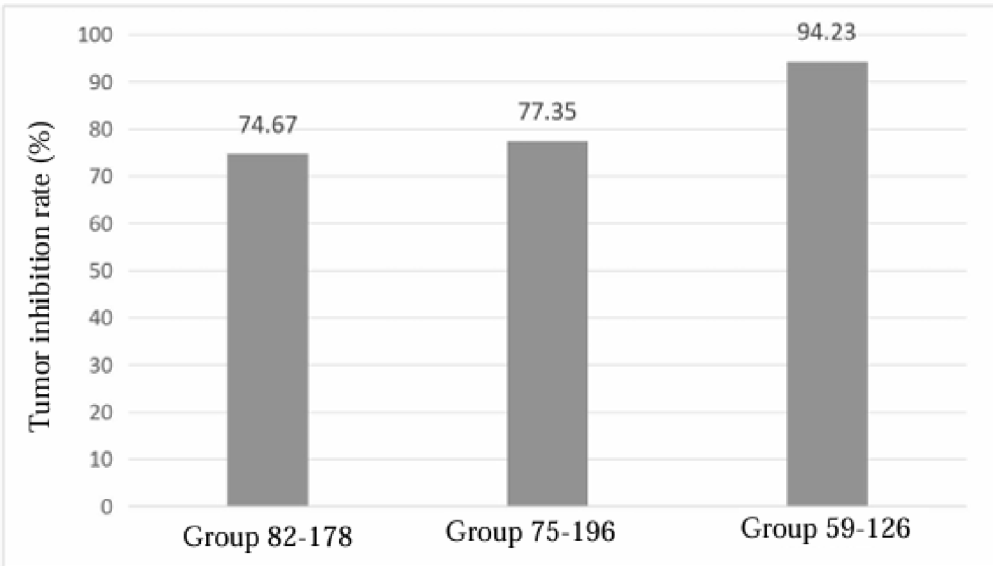

[Figure 17]

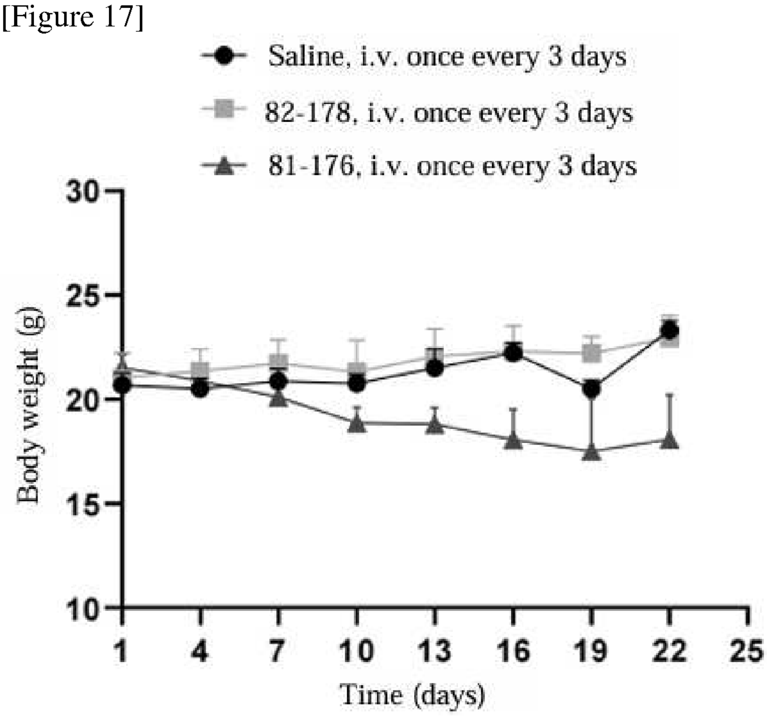


[Figure 18]

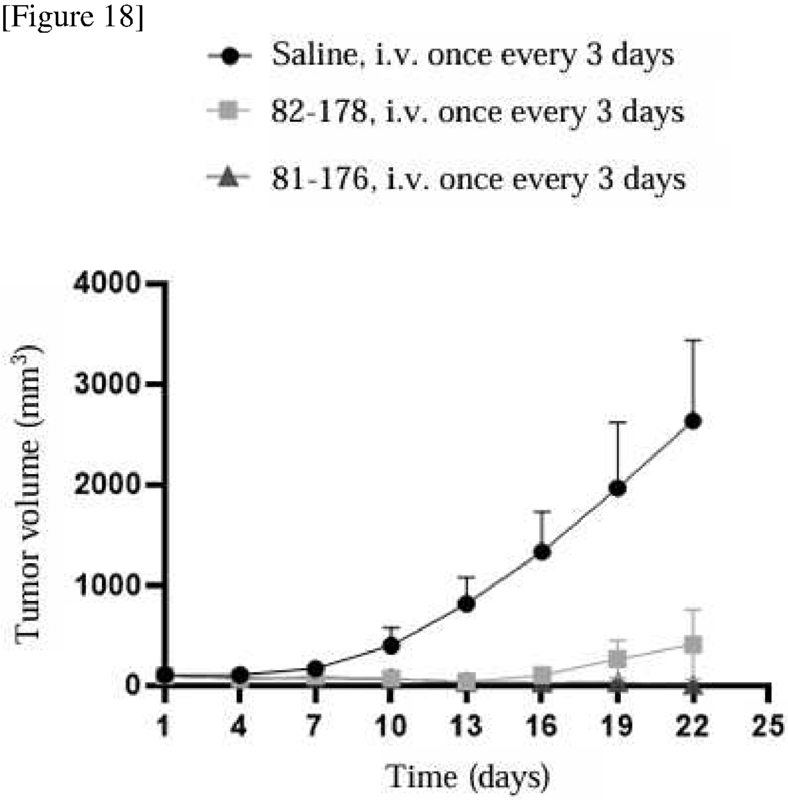

[Figure 19]

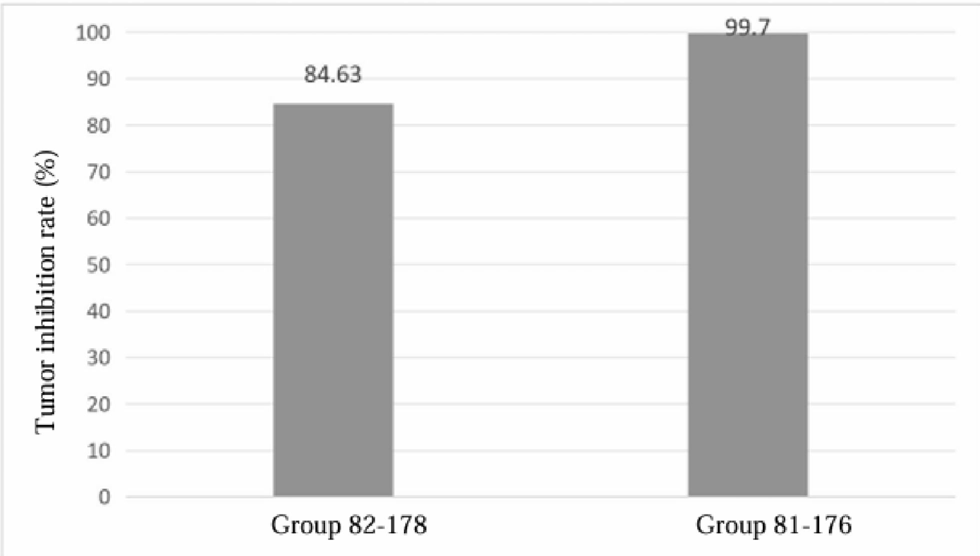
Tumor inhibition rate (%)
100
90
80
70
60
50
40
30
20
10
0
84.63
99.7
Group 82-178
Group 81-176

## INTERNATIONAL SEARCH REPORT

International application No. **PCT/CN2024/107866**

### A. CLASSIFICATION OF SUBJECT MATTER

A61K47/60(2017.01)i; A61K47/64(2017.01)i; A61K45/00(2006.01)i; A61P35/00(2006.01)i

According to International Patent Classification (IPC) or to both national classification and IPC

### B. FIELDS SEARCHED

Minimum documentation searched (classification system followed by classification symbols)

IPC: A61K, A61P

Documentation searched other than minimum documentation to the extent that such documents are included in the fields searched

Electronic data base consulted during the international search (name of data base and, where practicable, search terms used)

CNTXT; ENTXTC; WPABSC; DWPI; VEN; CNKI; 万方, WANFANG; Science Direct; 百度学术, BAIDU SCHOLAR: 重庆阿普格雷生物科技有限公司, 聚乙二醇, PEG, 氨基酸, 赖氨酸, 谷氨酸, 偶联, Polyethylene Glycol, Amino Acid, Lysine, Lys, Glutamic Acid, Glu, Coupled

### C. DOCUMENTS CONSIDERED TO BE RELEVANT

| Category* | Citation of document, with indication, where appropriate, of the relevant passages | Relevant to claim No. |
|---|---|---|
| A | WO 2022242488 A1 (CHONGQING APU GELEI BIOTECHNOLOGY CO., LTD.) 24 November 2022 (2022-11-24)<br>claim 1, and description, paragraphs 360 and 377-380 | 1-18 |
| A | CN 113995846 A (CHONGQING APU GELEI BIOTECHNOLOGY CO., LTD.) 01 February 2022 (2022-02-01)<br>claims 1-10 | 1-18 |
| A | CN 112851928 A (CHONGQING APU GELEI BIOTECHNOLOGY CO., LTD.) 28 May 2021 (2021-05-28)<br>claims 1-21 | 1-18 |
| A | WO 2020169004 A1 (BRIGHTGENE BIO-MEDICAL TECHNOLOGY CO., LTD.) 27 August 2020 (2020-08-27)<br>claims 1-17 | 1-18 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **29 October 2024** | **04 November 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2024/107866**

**C. DOCUMENTS CONSIDERED TO BE RELEVANT**

| Category* | Citation of document, with indication, where appropriate, of the relevant passages | Relevant to claim No. |
| --- | --- | --- |
| A | DENG, Chao et al. "Synthesis and Characterization of RGD Peptide Grafted Poly(ethylene glycol)-b-Poly(L-lactide)-b-Poly(L-glutamic acid) Triblock Copolymer" *Biomacromolecules,* Vol. 7, No. 2, 04 January 2006 (2006-01-04), pages 590-596 | 1-18 |

Form PCT/ISA/210 (second sheet) (July 2022)

**INTERNATIONAL SEARCH REPORT**
**Information on patent family members**

International application No.
**PCT/CN2024/107866**

| Patent document cited in search report | Publication date (day/month/year) | Patent family member(s) | Publication date (day/month/year) |
|---|---|---|---|
| WO 2022242488 A1 | 24 November 2022 | US 2024269299 A1 | 15 August 2024 |
| CN 113995846 A | 01 February 2022 | JP 2023537248 A | 31 August 2023 |
| | | EP 4190360 A1 | 07 June 2023 |
| | | WO 2022022354 A1 | 03 February 2022 |
| | | US 2023348671 A1 | 02 November 2023 |
| CN 112851928 A | 28 May 2021 | JP 2022554026 A | 27 December 2022 |
| | | AU 2020389657 A1 | 23 June 2022 |
| | | AU 2020389657 B2 | 01 August 2024 |
| | | BR 112022010360 A2 | 16 August 2022 |
| | | WO 2021104120 A1 | 03 June 2021 |
| | | US 2023088403 A1 | 23 March 2023 |
| | | US 11793881 B2 | 24 October 2023 |
| | | KR 20220102659 A | 20 July 2022 |
| | | KR 102542715 B1 | 14 June 2023 |
| | | MX 2022006519 A | 19 August 2022 |
| | | EP 4066861 A1 | 05 October 2022 |
| | | EP 4066861 A4 | 27 December 2023 |
| | | CA 3159842 A1 | 03 June 2021 |
| WO 2020169004 A1 | 27 August 2020 | US 2022096644 A1 | 31 March 2022 |
| | | TW 202031296 A | 01 September 2020 |
| | | TWI 771652 B | 21 July 2022 |
| | | AU 2020226475 A1 | 05 August 2021 |
| | | AU 2020226475 B2 | 16 February 2023 |
| | | TW 202142263 A | 16 November 2021 |
| | | TWI 792468 B | 11 February 2023 |
| | | KR 20210107083 A | 31 August 2021 |
| | | JP 2022521529 A | 08 April 2022 |
| | | JP 7353378 B2 | 29 September 2023 |
| | | CA 3126574 A1 | 27 August 2020 |
| | | CA 3126574 C | 25 June 2024 |
| | | EP 3928797 A1 | 29 December 2021 |
| | | EP 3928797 A4 | 13 April 2022 |
| | | EP 3928797 B1 | 09 October 2024 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202310959802
- EP 2012062505 W


### Non-patent literature cited in the description


- Remington's Pharmaceutical Sciences. Mack Publishing Company, 1995